# EUROPEAN PATENT APPLICATION

(11) **EP 4 700 119 A2**
(43) Date of publication of application: **25.02.2026**
(21) Application number: 25207252.5
(22) Date of filing: 14.02.2020
(51) Int. Cl.: C12N 9/00

(54) **MEANS AND METHODS FOR PREPARING ENGINEERED TARGET PROTEINS BY GENETIC CODE EXPANSION IN A TARGET PROTEIN-SELECTIVE MANNER**

(30) Priority: 14.02.2019 EP 19157257
(62) Divisional of application: 20703782.1
(71) Applicant: European Molecular Biology Laboratory, 69117 Heidelberg (DE)
(72) Inventor: LEMKE, Edward Anton, 55122 Mainz (DE); REINKEMEIER, Christopher Dieter, 69126 Heidelberg (DE); ESTRADA GIRONA, Gemma, London Greater London, N7 6ES (GB)
(74) Representative: Reitstötter Kinzebach

(57) **Abstract**

The present invention is concerned with orthogonal translation systems which allow for the site-specific introduction of non-canonical amino acid residues into a target protein (POI) in a POI-mRNA-selective manner. Specifically, the present invention relates to assembler fusion proteins which bring an RNA-targeting polypeptide (RNA-TP) segment and an orthogonal aminoacyl tRNA synthetase (O-RS) segment into spatial proximity of one another, either by direct linkage in RNA-TP/O-RS fusion proteins, or though the action of "assemblers" fused to each of these segments in assembler fusion proteins (AFPs). The invention also relates to AFP combinations and nucleic acid molecules comprising a POI-encoding sequence together with a targeting nucleotide sequence that is able to interact with an RNA-TP. The invention further relates to nucleic acid molecules, expression cassettes and expression vectors encoding said RNA-TP/O-RS fusion proteins or AFPs, cells comprising same, as well as methods and kits for translationally preparing POls.

## Description

### FIELD OF THE INVENTION

The present invention is concerned with orthogonal translation systems which allow for the site-specific introduction of non-canonical amino acid (ncAA) residues into a polypeptide of interest (POI) in a POI-mRNA-selective manner. Specifically, the present invention relates to fusion proteins which bring an RNA-targeting polypeptide (RNA-TP) segment and an orthogonal aminoacyl tRNA synthetase (O-RS) segment into spatial proximity of one another. This is achieved by combining an RNA-TP segment and an O-RS segment in one and the same fusion protein (RNA-TP/O-RS fusion protein), or by the action of one or more polypeptide segments which act as "assemblers" (APs) and facilitate a local enrichment of assembler fusion proteins (AFPs) comprising the one or more APs together with an RNA-TP segment or an O-RS segment, thus bringing said RNA-TP and O-RS segments into close proximity of one another. The invention also relates to AFP combinations and nucleic acid molecules comprising a POI-encoding sequence together with a targeting nucleotide sequence (TN) that is able to interact with an RNA-TP. The invention further relates to nucleic acid molecules, expression cassettes and expression vectors encoding said RNA-TP/O-RS fusion proteins or AFPs, cells comprising same, as well as methods and kits for translationally preparing POls.

### BACKGROUND OF THE INVENTION

The ability to engineer orthogonal (i.e. non-crossreactive) translation systems site-specifically into living cells enables the introduction of new functionality into proteins. However, this is a herculean task, as translation is a complex multistep process in which at least 20 different aminoacylated tRNAs, their cognate aminoacyl tRNA synthetases (RS), ribosomes and diverse other factors work in concert to synthesize a polypeptide chain from the RNA transcript. An ideal orthogonal system would show no cross-reactivity with factors of the host machinery, minimizing its impact on the housekeeping translational activity and normal physiology of the cell.

Towards this goal, genetic code expansion (GCE) is a method that enables reprogramming of a specific codon. With GCE, an orthogonal (suppressor) RS (O-RS) can aminoacylate its cognate suppressor tRNA with non-canonical amino acids (ncAAs). These ncAAs are typically custom designed and harbor chemical functionalities that can, for example, enable protein function to be photocontrolled, encode posttranslational modifications or allow the introduction of fluorescent labels for microscopy studies using click chemistry. To introduce ncAAs site-specifically into a polypeptide of interest (POI), the anticodon loop of the tRNA is chosen to decode and thus suppress one of the stop codons (see, e.g., Liu et al., Annu Rev Biochem 2010, 79:413-444; Lemke, ChemBioChem 2014, 15:1691-1694; Chin, Nature 2017, 550;53-60). To minimize the impact on the host cell machinery, the Amber stop codon (corresponding tRNA_{CUA}) is often utilized, owing to its particularly low abundance in E.COLI, to terminate endogenous proteins (<10%). Nevertheless, in principle any Amber codon in the genome can be suppressed, potentially leading to unwanted background suppression of non-targeted host proteins. If ncAA-modified proteins are recombinantly produced for *in vitro* applications, this background incorporation might be tolerable as long as the yields of purified full-length protein are acceptable. However, the challenge is different if the host is considered more than just a bioreactor vessel that can be sacrificed for its protein. In order to study the function of a host-cell POI *in situ,* the physiological condition of that host cell is an important factor. In that context, minimization of background incorporation of the ncAA is particularly required to ensure well-controlled experiments.

At least three elegant approaches have been developed to enable orthogonal translation in *E.coli,* that is, to decode a specific codon only for the RNA of the POI and not the entire genome. i) Orthogonal ribosomes recognizing a unique Shine-Dalgarno sequence have been developed to decode quadruplet codons, which are then used instead of stop codons to site-specifically encode an ncAA into a POI. (See, e.g., Heumann et al., Nature 2010, 464:441:444; Orelle et al., Nature 2015, 524:119-124; Fried et al., Angew Chem 2015, 54:12791-12794.) ii) Recently, genome engineering has advanced to the stage that *E.coli* strains can be depleted of selected native codons, providing a genetically clean (e.g. Amber codon free) host background for selective decoding of specific codons only in the POI. (See, e.g., Isaacs et al., Science 2011, 333:348-353; Lajoie et al., Science 2013, 342:357-360; Ostrov et al., Science 2016, 353:819-822; Wang et al., Nature 2016, 539:59-64.) iii) Unique non-canonical codons have been designed using an artificial base pair encoded only in the coding sequence of the POI. This lowers the risk of nonspecific decoding in other parts of the genome (see Zhang et al., Nature 2017, 551:644-647). However, due to genome complexity, it is not straightforward to transfer these orthogonal translation approaches to eukaryotes (see, e.g., Thompson et al., ACS Chem Biol 2018, 13:313-325), in which additionally the Amber codon is highly abundant (20% in mammalian cells).

There is therefore a high demand for strategies for POl-selective orthogonal translation which are versatile and work not only for well-characterized prokaryotes such as *E.coli,* which are relatively easy to handle and manipulate, but are also applicable to eukaryotic cells. It was therefore an object of the present invention to address this challenge.

### SUMMARY OF THE INVENTION

The inventors found that orthogonal translation systems (OT systems) which are able to selectively translate the mRNA of a POI can be created by generating spatial proximity between the mRNA of the POI and the O-RSs which allow for translationally introducing the ncAA residues into the growing polypeptide chain of the POI. The inventors demonstrated for a variety of POIs, including membrane proteins, that their OT systems allow for site-specifically introducing ncAA residues into a POI in a mammalian cell with selectivity for the mRNA of the POI compared to other mRNAs in the cytoplasm that contain the same stop codon (that is used as selector codon for encoding the ncAA residue of the POI).

In the orthogonal translation systems of the invention, the spatial proximity is achieved by including a targeting sequence (TN) in the mRNA of the POI that can selectively interact with an RNA-targeting polypeptide (RNA-TP), and linking the O-RS with such RNA-TP. Said linkage can be in a fusion protein comprising both, the O-RS and the RNA-TP (RNA-TP/O-RS fusion protein).

In another approach, this can be achieved by the action of one or more polypeptide segments which act as "assemblers" (APs) in facilitating a local enrichment of at least two assembler fusion proteins (AFPs) at least one of which comprising the one or more APs and an RNA-TP segment and at least one other AFP comprising the one or more APs and an O-RS segment, thus bringing said RNA-TP and O-RS segments (RNA-TP and O-RS also designated "effector" or "EP") into close proximity of one another. The local enrichment of the AFPs allows for the formation of assemblies (OT assemblies, also termed "OT organelles" herein) which can act as artificial orthogonally translating organelles.

The inventors demonstrated that different types of APs can be used. A first type includes APs which drive local enrichment at (previously existing) intracellular structures (such as, e.g., microtubules or the cytoplasmic side of membranes such as the cell membrane or the nuclear membrane, the ER, mitochondrial or Golgi organelles), termed intracellular targeting polypeptide (IC-TP) segments. A second type of APs generates high local AFP concentrations by self-association in the cytoplasm (in particular by phase separation) termed phase separation polypeptide (PSP) segments herein. Said AP types may also be combined with other polypeptide elements having the ability to form multimeric structures, like in particular, coiled coil heterodimers, as formed by synthetic SYNZIP polypeptide pairs. Similarly, said EP types may also be combined with other polypeptide elements having the ability to form multimeric structures, like in particular, coiled coil heterodimers, as formed by synthetic SYNZIP polypeptide pairs. Such multimer formation further improves local enrichment of AFPs.

The inventors further found that AFPs combining different AP types are particularly useful.

In still another approach, AFPs are provided encompassing in a single polypeptide, i.e. fused together, both types of EP segments, i.e. the RNA-TP and O-RS segment, one or both types of AP segments, i.e. the IC-TP and/or PSP segment, optionally supplemented by said polypeptide elements having the ability to form multimeric structures (SYNZIP polypeptide). This provides the advantage that all the elements required for generating an OT system of the invention are included in one single AFP.

Thus, in a first aspect, the present invention relates to an assembler fusion protein (AFP) comprising:
(a) at least one first polypeptide segment acting as assembler (AP) that is selected from:
   (a1) a polypeptide segment derived from an intracellular targeting polypeptide (IC-TP segment), wherein said intracellular targeting polypeptide targets, and thus becomes locally enriched at, an intracellular structural element within or directly adjacent to the cytoplasm; and
   (a2) a polypeptide segment derived from a phase separation polypeptide (PSP segment), wherein said phase separation polypeptide has the ability to undergo self-association in the cytoplasm of a cell so as to create sites of high local concentration in the cytoplasm, and
(b) at least one second polypeptide segment acting as an effector (EP) that is selected from:
   b1) an RNA-targeting polypeptide (RNA-TP) segment, and
   b2) an orthogonal aminoacyl tRNA synthetase (O-RS) segment;
wherein said polypeptide segments are functionally linked in said AFP.

In a second aspect, the present invention relates to an assembler fusion protein (AFP) combination comprising at least two AFPs of the present invention as described herein. Preferably, the AFP combination comprises at least one AFP comprising a RNA-TP segment and at least one AFP comprising an O-RS segment. Including into at least one AFP of said combination a first SYNZIP element and including in at least another AFP of said combination a second SYNZIP element, wherein said first and said second SYNZIP act together by forming a heterodimer structure, represents another advantageous form of said second aspect..

In a third aspect, the present invention relates to a fusion protein (RNA-TP/O-RS fusion protein) comprising:
(i) at least one RNA-targeting polypeptide (RNA-TP) segment; and
(ii) at least one orthogonal aminoacyl tRNA synthetase (O-RS) segment,
wherein said polypeptide segments are functionally linked in said RNA-TP/O-RS fusion protein.

In a further aspect, the present invention provides a nucleic acid molecule, or a combination of two or more nucleic acid molecules, comprising:
(i) a nucleotide sequence that encodes at least one RNA-TP/O-RS fusion protein of the present invention as described herein, or
(ii) a nucleic acid sequence complementary to (i), or
(iii) both of (i) and (ii).

In a further aspect, the present invention provides a nucleic acid molecule, or a combination of two or more nucleic acid molecules, comprising:
(i) a nucleotide sequence that encodes at least one AFP of the present invention as described herein, or
(ii) a nucleic acid sequence complementary to (i), or
(iii) both of (i) and (ii).

In a further aspect, the present invention provides a nucleic acid molecule, or a combination of two or more nucleic acid molecules, comprising:
(i) a nucleotide sequence that encodes at least one AFP combination of the present invention as described herein, or
(ii) a nucleic acid sequence complementary to (i), or
(iii) both of (i) and (ii).

In further aspects, the present invention provides an expression cassette comprising the nucleotide sequence of the nucleic acid molecule, or the combination of nucleic acid molecules, of the present invention as described herein.

In particular embodiments, the present invention provides an expression cassette comprising:
(i) a nucleotide sequence that encodes at least one RNA-TP/O-RS fusion protein of the present invention as described herein, or
(ii) a nucleic acid sequence complementary to (i), or
(iii) both of (i) and (ii).

In further particular embodiments, the present invention provides an expression cassette comprising:
(i) a nucleotide sequence that encodes at least one AFP of the present invention as described herein, or
(ii) a nucleic acid sequence complementary to (i), or
(iii) both of (i) and (ii).

In further particular embodiments, the present invention provides an expression cassette comprising:
(i) a nucleotide sequence that encodes at least one AFP combination of the present invention as described herein, or
(ii) a nucleic acid sequence complementary to (i), or
(iii) both of (i) and (ii).

In further aspects, the present invention provides an expression vector comprising at least one expression cassette of the present invention as described herein.

In further aspects, the present invention provides a cell comprising at least one nucleic acid molecule, or combination of nucleic acid molecules, of the present invention as described herein. In particular embodiments, the cell comprises at least one expression cassette or at least one expression vector of the present invention as described herein.

In a further aspect, the present invention relates to a method for preparing a polypeptide of interest (POI) comprising in its amino acid sequence one or more non-canonical amino acid (ncAA) residues. Said method comprises expressing the POI in a cell of the present invention in the presence of said one or more ncAAs, wherein the cell comprises:
(i) at least one AFP comprising a RNA-TP segment and at least one AFP comprising an O-RS segment as described herein;
(ii) a POI-encoding nucleotide sequence (CS^{POI}) wherein said one or more ncAA residues of the POI are encoded by selector codon(s),
(iii) a targeting nucleotide sequence (TN) that is functionally linked to the CS^{POI} and is able to interact with an RNA-TP segment of at least one of the AFPs in the cell;
(iv) one or more orthogonal tRNA^{ncAA} (O-tRNA^{ncAA}) molecules which carry the anticodon(s) complementary to the selector codon(s) of the CS^{POI}, and wherein said O-tRN^{ncAA} molecules together with one or more O-RS segments of the AFPs in the cell form one or more orthogonal O-RS/O-tRNA^{ncAA} pairs which allow for the introduction of said one or more ncAA residues into the amino acid sequence of the POI;
and wherein the method optionally further comprises recovering the expressed POI.

Said at least one AFP comprising a RNA-TP segment and said at least one AFP comprising an O-RS segment recited in (i) can be one and the same type of AFP, i.e. an AFP comprising both a RNA-TP segment and an O-RS segment. Alternatively, said at least one AFP comprising a RNA-TP segment and said at least one AFP comprising an O-RS segment recited in (i) can be different AFPs.

In a further aspect, the present invention relates to a method for preparing a polypeptide of interest (POI) comprising in its amino acid sequence one or more non-canonical amino acid (ncAA) residues. Said method comprises expressing the POI in a cell of the present invention in the presence of said one or more ncAAs, wherein the cell comprises:
(i) RNA-TP/O-RS fusion proteins of the present invention as described herein;
(ii) a POI-encoding nucleotide sequence (CS^{POI}) wherein said one or more ncAA residues of the POI are encoded by selector codon(s),
(iii) a targeting nucleotide sequence (TN) that is functionally linked to the CS^{POI} and is able to interact with an RNA-TP segment of at least one of the RNA-TP/O-RS fusion proteins in the cell;
(iv) one or more orthogonal tRNA^{ncAA} (O-tRNA^{ncAA}) molecules which carry the anticodon(s) complementary to the selector codon(s) of the CS^{POI}, and wherein said O-tRNA^{ncAA} molecules together with one or more O-RS segments of the RNA-TP/O-RS fusion proteins in the cell form one or more orthogonal O-RS/O-tRNA^{ncAA} pairs which allow for the introduction of said one or more ncAA residues into the amino acid sequence of the POI;
and wherein the method optionally further comprises recovering the expressed POI.

In a further aspect, the present invention relates to a method for preparing a polypeptide of interest (POI) comprising in its amino acid sequence one or more non-canonical amino acid (ncAA) residues. Said method comprises the steps of:
(a) expressing in a cell one or more AFPs comprising at least one RNA-TP segment and one or more AFPs comprising at least one O-RS segment as described herein;
(b) expressing in said cell one or more orthogonal tRNA^{ncAA} (O-tRNA^{ncAA}) molecules,
wherein
- said orthogonal tRNA^{ncAA} molecules and one or more of the O-RS segments of the AFPs in the cell form one or more orthogonal aminoacyl tRNA synthetase/tRNA^{ncAA} (O-RS/O-tRNA^{ncAA}) pairs,
- said O-RS/O-tRNA^{ncAA} pairs allow for introducing said one or more ncAA residues into the amino acid sequence of said POI,
wherein steps (a) and (b) can be concomitantly or sequentially in any order;
(c) then, expressing said POI in said cell in the presence of said one or more ncAAs, wherein
   - the POI-encoding nucleotide sequence (CS^{POI}) comprises one or more selector codons encoding said one or more ncAA residues,
   - said selector codons match the anticodons of said one or more O-tRNA^{ncAA} molecules;
   - said CS^{POI} is functionally linked to a targeting nucleotide sequence (TN), thus forming a CS^{POI}/TN fusion sequence,
   - said CS^{POI}/TN fusion sequence is able to interact, via its TN, with an RNA-TP segment of at least one of the AFPs in the cell;
      and
(d) optionally recovering the expressed POI.

In a further aspect, the present invention relates to a method for preparing a polypeptide of interest (POI) comprising in its amino acid sequence one or more non-canonical amino acid (ncAA) residues. Said method comprises the steps of:
(a) expressing in a cell RNA-TP/O-RS fusion proteins of the present invention as described herein;
(b) expressing in said cell one or more orthogonal tRNA^{ncAA} (O-tRNA^{ncAA}) molecules,
wherein
- said orthogonal tRNA^{ncAA} molecules and one or more of the O-RS segments of the RNA-TP/O-RS fusion proteins in the cell form one or more orthogonal aminoacyl tRNA synthetase/tRNA^{ncAA} (O-RS/O-tRNA^{ncAA}) pairs,
- said O-RS/O-tRNA^{ncAA} pairs allow for introducing said one or more ncAA residues into the amino acid sequence of said POI,
wherein steps (a) and (b) can be concomitantly or sequentially in any order;
(c) then, expressing said POI in said cell in the presence of said one or more ncAAs,
   wherein
   - the POI-encoding nucleotide sequence (CS^{POI}) comprises one or more selector codons encoding said one or more ncAA residues,
   - said selector codons match the anticodons of said one or more O-tRNA^{ncAA} molecules;
   - said CS^{POI} is functionally linked to a targeting nucleotide sequence (TN), thus forming a CS^{POI}/TN fusion sequence,
   - said CS^{POI}/TN fusion sequence is able to interact, via its TN, with an RNA-TP segment of at least one of the RNA-TP/O-RS fusion proteins in the cell;
      and
(d) optionally recovering the expressed POI.

In a further aspect, the present invention relates to a nucleic acid molecule comprising:
(i) a nucleotide sequence (CS^{POI}) that encodes a polypeptide of interest (POI), said POI comprising one or more, identical or different, non-canonical amino acid (ncAA) residues which are encoded in the CS^{POI} by selector codons, and
(ii) a targeting nucleotide sequence (TN), wherein an RNA molecule comprising said TN is able to interact via said TN with an RNA-targeting polypeptide (RNA-TP).

In a further aspect, the present invention relates to a kit for preparing a polypeptide of interest (POI) having at least one non-canonical amino acid (ncAA) residue, the kit comprising:
- at least one ncAA, or salt thereof, corresponding to the at least one ncAA residue of the POI, and
- at least one expression vector of the present invention as described herein.

Said expression vector comprises at least one expression cassette comprising:
(i) a nucleotide sequence that encodes at least one RNA-TP/O-RS fusion protein of the present invention, at least one AFP of the present invention, or at least one AFP combination of the present invention, or
(ii) a nucleic acid sequence complementary to (i), or
(iii) both of (i) and (ii).

### BRIEF DESCRIPTION OF THE FIGURES

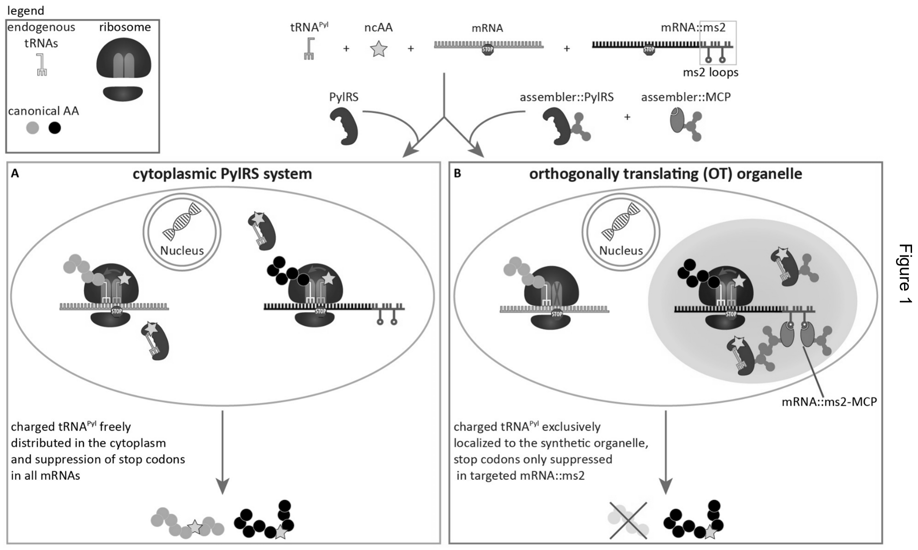

Figure 1 shows a schematic representation of the spatial separation of the components which allow for orthogonal translation so as to decode a specific stop codon in a uniquely tagged mRNA. (A) Conventional expression of the synthetase PyIRS leads to aminoacylation of its cognate stop codon suppressor tRNA^{Pyl} with a custom designed ncAA. This leads to site-specific ncAA incorporation whenever the respective stop codon occurs in mRNA of the POI. Given that many endogenous mRNAs terminate on the same stop codon, utilizing this approach in the cytoplasm potentially leads to misincorporation of the ncAA into unwanted proteins (left box). (B) To avoid this, the present invention allows that the mRNA encoding the POI and the orthogonal aminoacyl-tRNA synthetase (e.g., PyIRS) can be brought into close proximity to one another through the use of an RNA-targeting polypeptide segment (e.g., MCP) and assemblers (APs), . This allows for spatial enrichment of all components so as to create an OT assembly ("OT organelle"), including the mRNA encoding the POI, the orthogonal aminoacyl-tRNA synthetase, the tRNA, and ribosomes (right box). Here aminoacylated tRNA^{Pyl} is particularly available in direct proximity of the OT organelle, so that particularly here stop codon suppression (of the POI mRNA) can occur. This leads to a selective suppression of stop codons (and thus expression) of the POI mRNA over corresponding stop codons in mRNAs that are not targeted to the OT assembly. While in (A) GCE occurs stop codon-specific, in (B) it should occur stop codon-specific and mRNA-specific.

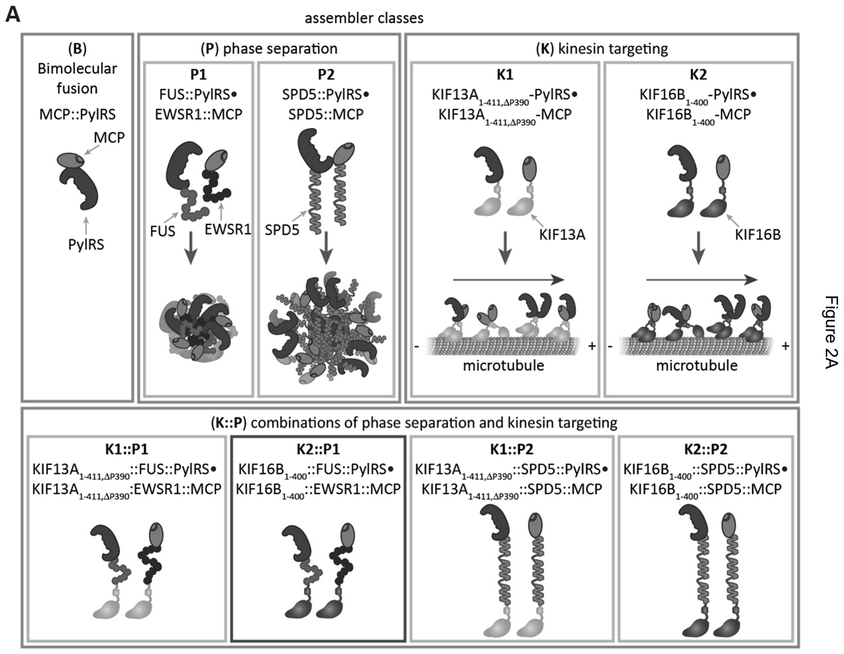

Figure 2A shows a schematic representation of different assembler classes. **B** = bimolecular MCP::PyIRS fusion, **P1** = fusions to FUS and EWSR1, **P2** = SPD5, **K1** = truncation of kinesin KIF13A (KIF13A_{1-411,ΔP390}), **K2** = truncation of kinesin KIF16B (KIF16B₁₋₄₀₀) and combinations thereof **(K1::P1, K1::P2, K2::P1, K2::P2).**

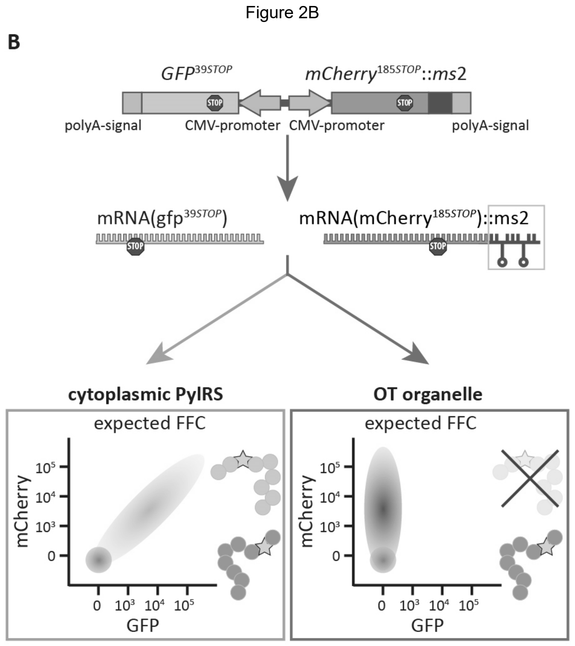

Figure 2B shows a schematic representation of the dual-color reporter. mRNAs encoding the fluorescent proteins GFP and mCherry, containing stop codons at permissive sites, are expressed from one plasmid, each with its own CMV promoter, ensuring a constant ratio of mRNA throughout each experiment. The mRNA of the mCherry reporter is tagged with two MS2 RNA stem-loops ("ms2", also referred to as MS2-tag herein), mRNA(mCherry)::ms2. In the presence of ncAA and tRNA^{Pyl}, in the case of cytoplasmic PyIRS, both GFP^{39*STOP*} and mCherry^{185*STOP*} are produced, leading to a diagonal in fluorescence flow cytometry (FFC) analysis (left box). However, under the same conditions, orthogonal translation in OT organelles enables selective stop codon suppression of mRNA(mCherry)::ms2, resulting in an mCherry-positive and GFP-negative population (drawn schematically as a vertical population in the right box). In both schemes, non-transfected HEK293T cells are represented by a gray circle at the bottom.

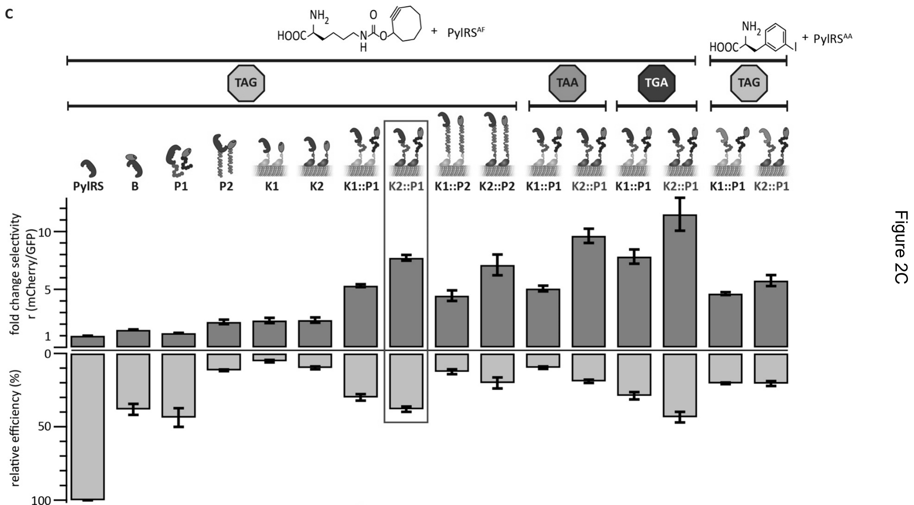

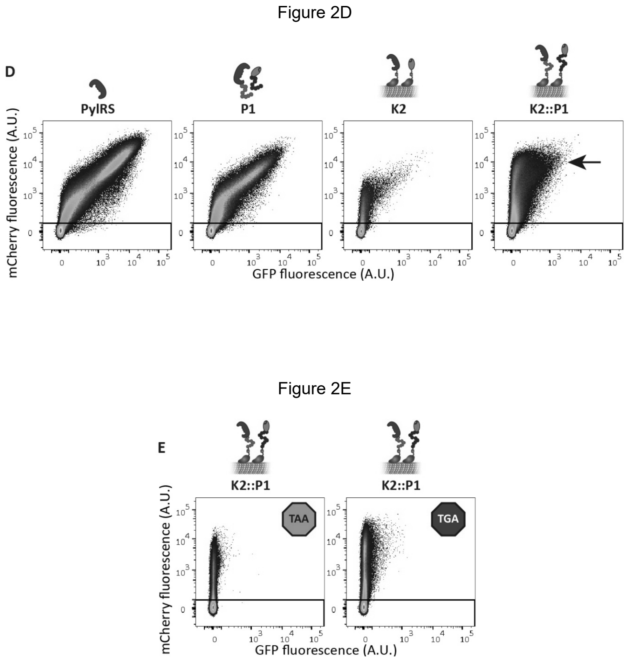

Figure 2C shows the selectivity and relative efficiency of various exemplary OT systems. For all experiments the indicated constructs were co-expressed with tRNA^{Pyl} (anticodon corresponding to the indicated codon) and the dual reporter (*GFP^{39STOP}, mCherry^{185STOP}::ms2*)*.* GCE was performed in presence of the indicated ncAAs, and cells were analyzed by FFC. The dark gray bars (normalized to cytoplasmic PyIRS) represent the fold change in the ratios *r* of the mean fluorescence intensities of mCherry versus GFP (derived from FFC, see Fig. 2D, E) for all the systems tested. The light-gray bars represent the relative efficiency as defined by the mean fluorescence intensity of mCherry for each condition divided by cytoplasmic PyIRS control (derived from FFC, see Fig. 2D, E). Shown are the mean values of at least three independent experiments; error bars represent the SEM. The box highlights the best performing OT organelle (OT^{K2::P1}).
Figure 2D shows the results of the FFC analysis of the dual-color reporter expressed with the four indicated systems in transfected HEK293T cells and tRNA^{Pyl} in the presence of the ncAA SCO, a lysine derivative with a cyclooctyne side chain. Highly selective and efficient orthogonal translation was observed for the OT assembly (the black arrow indicates a bright, highly mCherry-positive population). Shown in the dot plots are the sums of at least three independent experiments. Axes indicate fluorescence intensity in arbitrary units.
Figure 2E shows FFC plots for the OT assembly selectively translating Opal and Ochre codons only of recruited mRNA(mCherry^{185TGA})::ms2 and mRNA(mCherry^{185TAA})::m_{S2}, respectively.

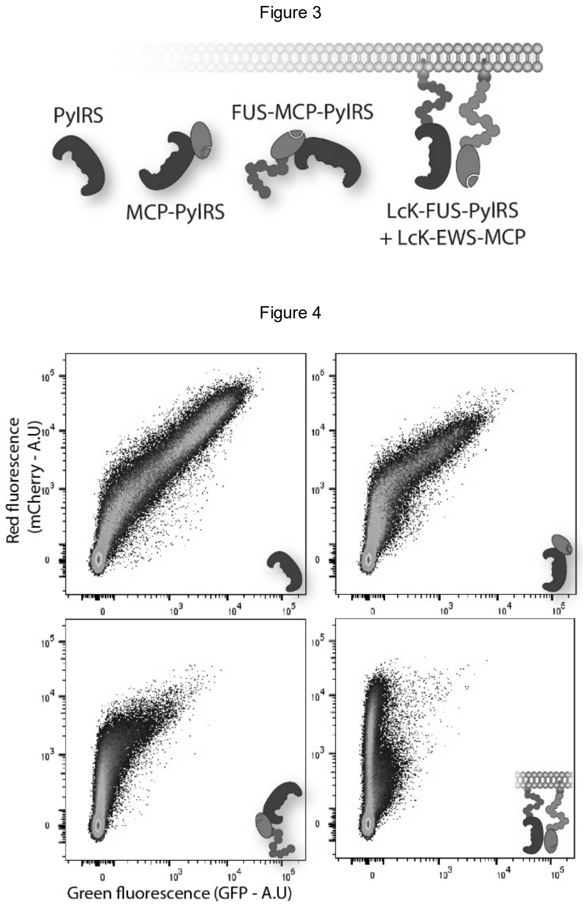

Figure 3 shows a schematic representation of the constructs composing the following systems: PyIRS, MCP::PyIRS, FUS::MCP::PyIRS and LcK::FUS::PyIRS•LcK::EWS::MCP.
Figure 4 shows the flow cytometry analysis of the dual reporter expression with the 4 different systems depicted in Figure 3. HEK293T cells were transfected with constructs encoding the dual reporter, tRNA, LcK::FUS::PyIRS and LcK::EWS::MCP or PyIRS, MCP::PyIRS, FUS::MCP::PyIRS and pcDNA3.1. Shown is the sum of at least three independent experiments. Axes indicate fluorescence intensity in arbitrary units.

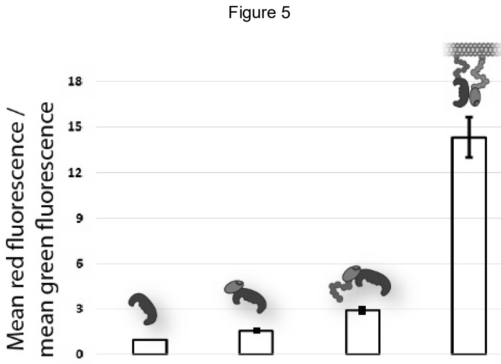

Figure 5 shows a bar plot with the ratios of the mean fluorescence intensity of mCherry vs. GFP fluorescence for all the tested systems. Plots represent mean values of at least 3 biological replicates, error bars indicate standard error of means.

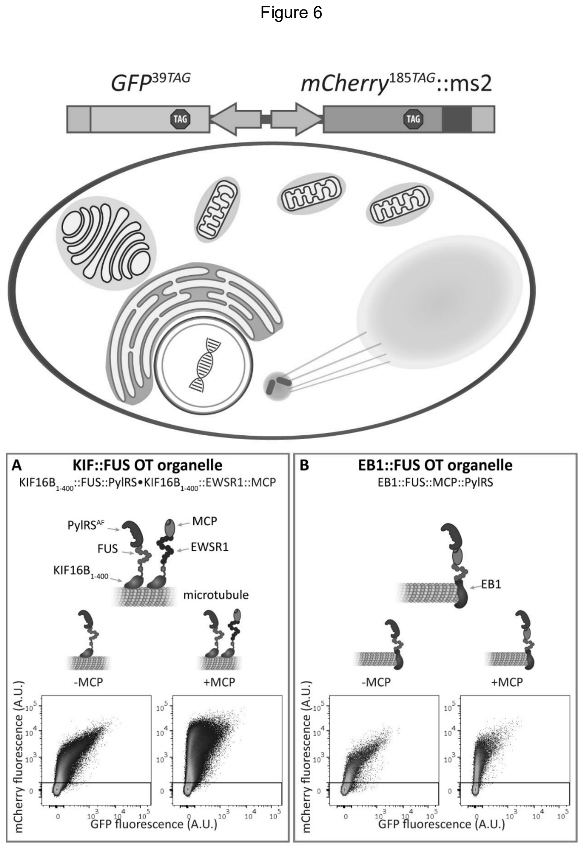

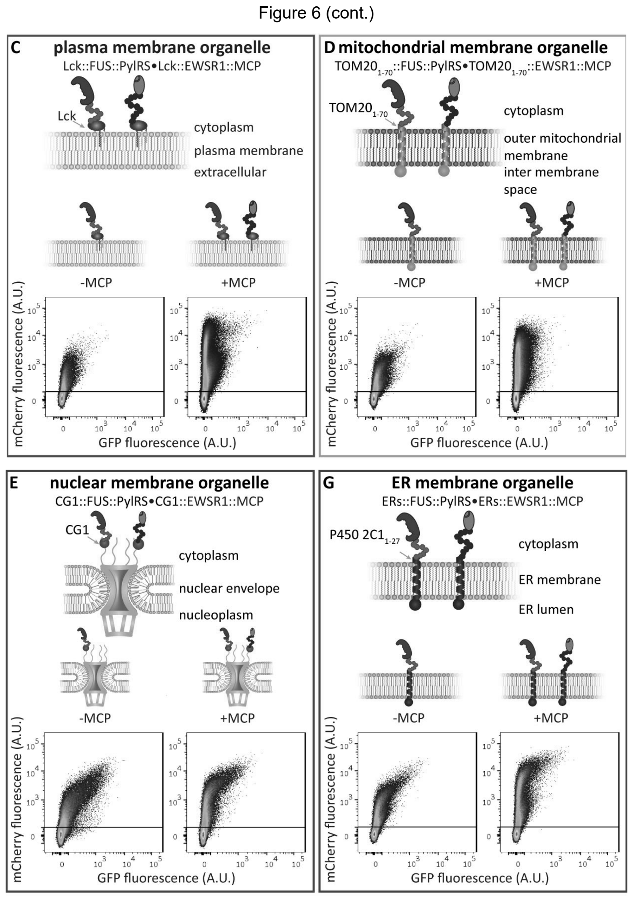

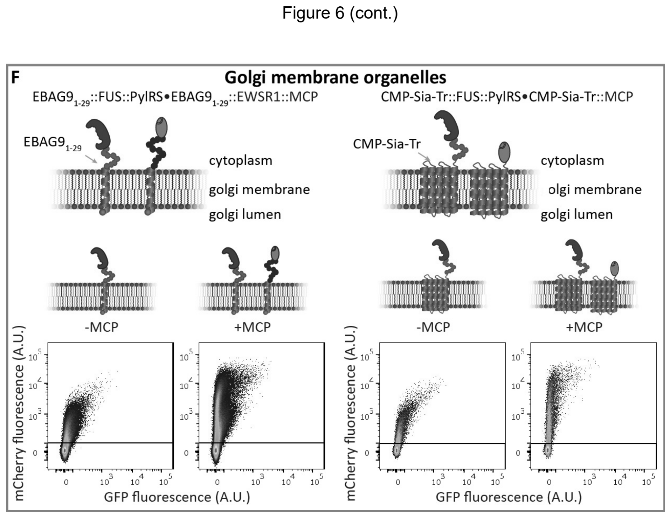

Figure 6 provides an overview of different approaches of the present invention for generating OT organelles, which target to the surface of different intra-cellular structures. Different constructs are expressed and the results of the respective fluorescence flow cytometry (FFC) analyses are shown. On top of the figure the dual color reporter construct GFP^{39TAG}•mCherry ^{185TAG}::ms2 (see also Figure 2B) as applied in each of the schematically illustrated experiments A to G is depicted and a schematic illustration of different targeted cellular compartments is shown. Control experiments performed without the effector polypeptide MCP (-MCP) are also illustrated for each of the experiments A to G:
   **A:** OT organelle targeted to microtubules and obtained by expressing the system KIF16B₁₋₄₀₀::FUS::PyIRS•KIF16B₁₋₄₀₀::EWSR1::MCP or the construct KIF16B₁₋₄₀₀::FUS::PyIRS (control);
   **B**: OT organelle targeted to microtubule plus ends and obtained by expressing the constructs EB1::FUS::MCP::PylRS or EB1::FUS::PyIRS (control).
   **C:** OT organelle targeted to plasma membrane and obtained by expressing the system LcK::FUS::PyIRS•LcK::EWSR1::MCP or the construct LcK::FUS::PyIRS (control).
   **D:** OT organelle targeted to mitochondrial membrane and obtained by expressing the system TOM20₁₋₇₀::FUS::PyIRS•TOM20₁₋₇₀::EWSR1::MCP or the construct TOM20₁₋₇₀::FUS::PyIRS (control).
   **E:** OT organelle targeted to nuclear membrane and obtained by expressing the system CG1::FUS::PyIRS•CG1::EWSR1::MCP or the construct CG1::FUS::PyIRS (control).
   **F (left side):** OT organelle targeted to Golgi membrane and obtained by expressing the system EBAG9₁₋₂₉::FUS::PylRS• EBAG9₁₋₂₉::EWSR1::MCP or the construct EBAG9₁₋₂₉::FUS::PylRS (control).
   **F (right side):** OT organelle targeted to Golgi membrane and obtained by expressing the system CMP Sia Tr::FUS::PylRS• CMP Sia Tr::MCP or the construct CMP Sia Tr::FUS::PyIRS (control).
   **G:** OT organelle targeted to ER membrane and obtained by expressing the system P450 2C1₁₋₂₇::FUS::PyIRS•P450 2C1₁₋₂₇::EWSR1::MCP or the construct P450 2C1₁₋₂₇::FUS::PyIRS (control).

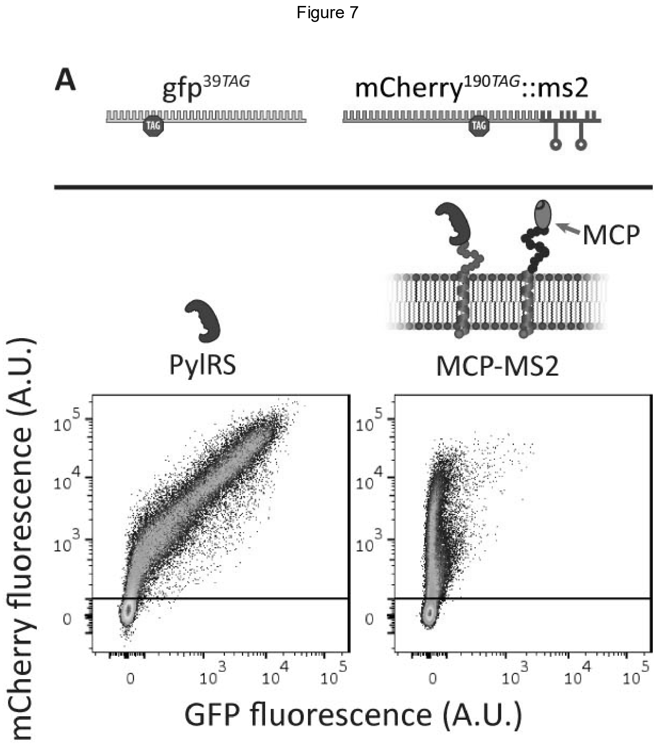

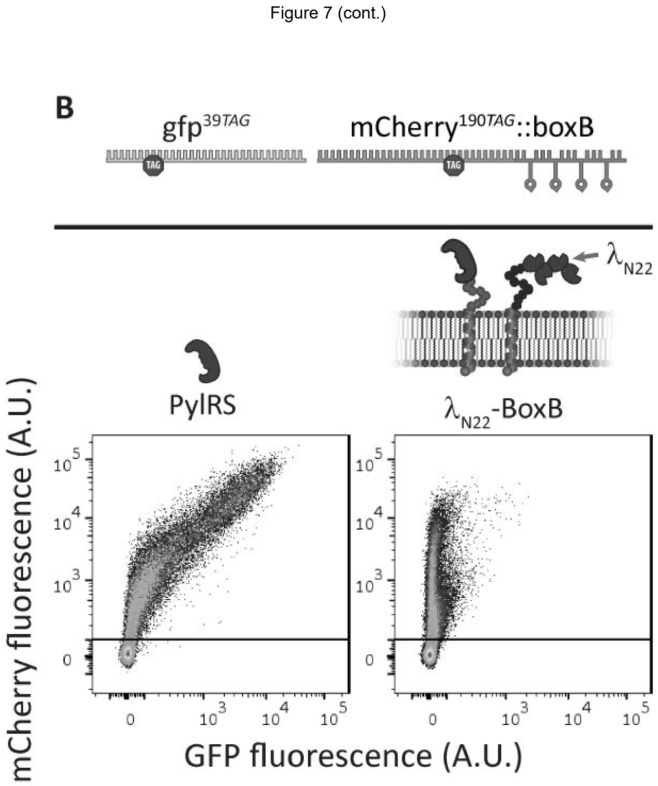

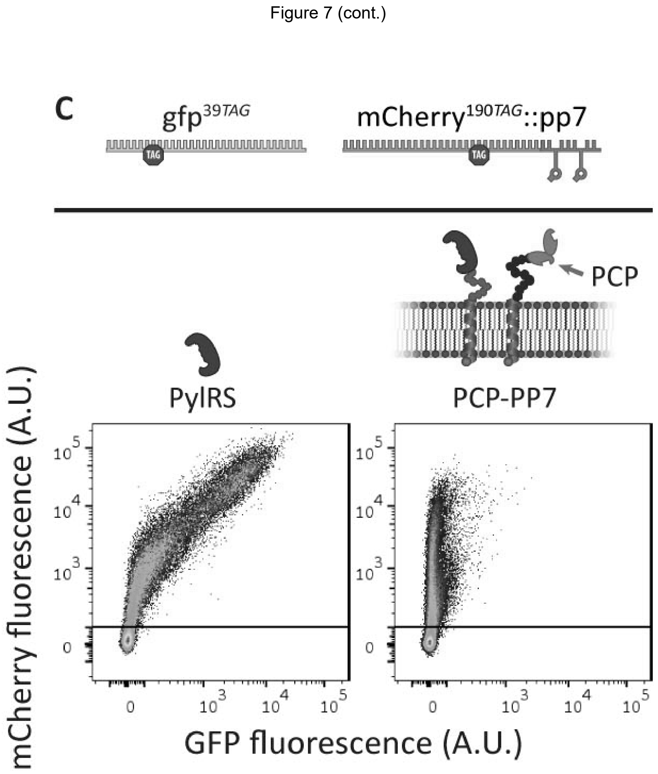

Figure 7 provides an overview of different approaches of the present invention for recruiting RNA using the interaction of different RNA loops and respective RNA targeting proteins. The results of the respective fluorescence flow cytometry (FFC) analyses are shown and compared to the respective analysis as obtained for non-targeted PyIRS alone:
   **A:** System ms-2-MCP incorporates the ms2 loops in the UTR of an mRNA molecule and recruits the mRNA with the MCP protein into the artificial organelle.
   **B**: System boxB-λ_{N22} incorporates the boxB loops in the UTR of an mRNA molecule and recruits the mRNA with the λ_{N22} protein into the artificial organelle
   **C:** System pp7-PCP incorporates the pp7 loops in the UTR of an mRNA molecule and recruits the mRNA with the PCP protein into the artificial organelle.

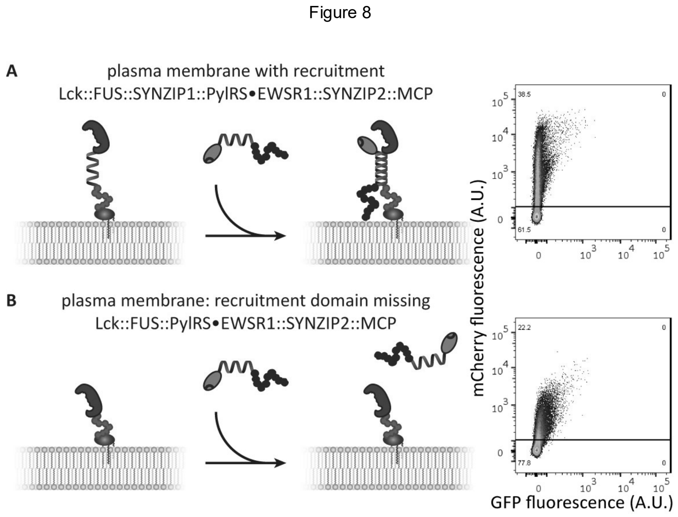

Figure 8 illustrates a further approach of the present invention for generating OT organelles which will work on the surface of different cellular structures. Here the targeting to plasma membrane is exemplified. The particular approach is characterized by the pairwise incorporation of so-called synthetic heterodimeric-coiled coil peptides SYNZIP1 and SYNZIP2 fused into the system LcK::FUS::SYNZIP1::PylRS•EWSR1::SYNZIP2::MCP; upon expression SYNZIP1 and 2 pair and recruit MCP to a plasma membrane based OT organelle which in turn enables the selective orthogonal translation of a subsequently recruited mRNA comprising the ms2 targeting nucleotide loops. Selective translation is illustrated by the results of the respective FFC analysis **(A).** In a comparative approach with the system LcK::FUS::PylRS•EWSR1::SYNZIP2::MCP, wherein SYNZIP1 is missing, no selectivity of translation could be observed **(B).**

### DETAILED DESCRIPTION OF THE INVENTION

Unless otherwise defined herein, scientific and technical terms as used in the context of the present invention shall have the meanings that are commonly understood by those of ordinary skill in the art. The meaning and scope of the terms should be clear. However, in the event of any latent ambiguity, definitions provided herein take precedent over any dictionary or extrinsic definition. Further, unless otherwise required by context, singular terms shall include pluralities and plural terms shall include the singular.

If not otherwise stated, nucleotide sequences are depicted herein in the 5' to 3' direction. If not otherwise stated, amino acid sequences are depicted herein in the direction from N-terminus to C-terminus.

If not otherwise stated, the polypeptide of interest (POI) that is translationally expressed by the OT system according to the present invention comprises one or more ncAA residues which are encoded in the nucleotide sequence encoding the POI (CS^{POI}) by selector codons.

### 1. Fusion proteins

### 1.1. General

The fusion proteins of the invention may be construed in different manner.

A first type includes fusion proteins wherein at least two types of effector polypeptides (EPs), comprising at least one RNA-TP and at least one O-RS, are comprised by one and the same fusion protein (also designated as **RNA-TP/O-RS** fusion proteins).

A second type includes fusion proteins which comprise at least one assembler polypeptide (AP) and at least one type of EP selected from RNA-TP segments and O-RS segments (also designated **AFPs).** In particular, AFPs can comprise both RNA-TP and O-RS segments, such as one or more RNA-TP segments and one or more O-RS segments in any sequential order, in addition to the at least one type of AP. Thus, AFPs in particular are selected from the following fusion protein types (segments functionally linked in any order within the polypeptide chain; one or more segments of the same type in any order within the polypeptide chain):
(RNA-TP/AP)
(O-RS/AP)
(RNA-TP/O-RS/AP)

APs are selected from IC-TPs and PSPs, and may be composed of one or more IC-TPs and/or one or more of PSPs in any sequential order. Thus, AFPs more particularly are selected from the following fusion protein types (segments functionally linked in any order within the polypeptide chain; one or more segments of the same type in any order within the polypeptide chain):
(RNA-TP/ IC-TP)
(O-RS/ IC-TP)
(RNA-TP/O-RS/ IC-TP)
(RNA-TP/PSP)
(O-RS/PSP)
(RNA-TP/O-RS/PSP)
(RNA-TP/PSP/ IC-TP)
(O-RS/ PSP/ IC-TP)
(RNA-TP/O-RS/ PSP/ IC-TP)

APs and/or EPs may also comprise (as part of the fusion protein) heterooligomer forming, in particular heterodimer forming polypeptide segments, like in particular synthetic coiled coil SYNZIP peptides. AFP combinations comprising such interacting SYNZIP pairs distributed between members of said AFP combination, so that each AFP comprises merely one member of such interacting SYNZIP pair are particular embodiments.

The term "segment" as used herein in the context of fusion proteins indicates that the thus designated element (e.g., RNA-TP, O-RS, IC-TP, PSP, SYNZIP) is part of the fusion protein, i.e. linked to the remainder of the fusion protein. The segments of the fusion proteins of the invention are functionally linked, i.e. linked such that they still function as RNA-TP, O-RS, IC-TP and PSP or SYNZIP, respectively. Said linkage is preferably covalent, and in particular is a peptidic linkage.

For example, the RNA-TP segment comprised in the fusion proteins of the present invention is a segment of the fusion protein that is derived from, and functions in the context of the fusion protein as, an RNA-TP, thus allowing the fusion protein to interact with (bind to) the targeted RNA, wherein said interaction is expediently a specific one. Thus, an RNA-TP segment may comprise the (entire) amino acid sequence, or a functional fragment, of an RNA-targeting polypeptide as described herein.

Analogously, an O-RS segment comprised by the fusion proteins of the present invention is a segment of the fusion protein that is derived from, and functions in the context of the fusion protein as, an O-RS, thus conferring to the fusion protein O-RS enzymatic activity, that is the ability to catalyze the aminoacylation of an O-tRNA with an ncAA. Thus, an O-RS segment may comprise the (entire) amino acid sequence, or a functional fragment, of an O-RS as described herein.

The assembler fusion proteins (AFPs) described herein comprise at least one polypeptide segment acting as an assembler (AP). As used herein the term AP refers to any polypeptide segment that allows for enrichment of AFPs comprising said segment at spatially distinct sites within a living cell. Expediently said spatially distinct sites are located within, or directly adjacent to, the cytoplasm of the cell and readily accessible to the translational machinery of the cell (which includes canonical aminoacylated tRNAs, translation factors, ribosomal subunits, etc.) as well as the O-tRNAs which allow for the introduction of the ncAA residues into the POI.

There are different types of polypeptide segments which can serve as APs in the present invention. One type of APs are polypeptide segments which are derived from, and function in the context of the fusion protein as, intracellular targeting polypeptides (IC-TPs). These IC-TP segments may comprise the (entire) amino acid sequence, or a function fragment, of an IC-TP. IC-TPs target, and thus become locally enriched at, intracellular structural elements within, or directly adjacent to, the cytoplasm. Examples of such structural elements include microtubules, the cytoplasmic side of membranes such as the cell membrane, the nuclear membrane, the mitochondrial membrane, the Golgi membrane, the ER membrane, etc.

Accordingly, in particular embodiments, the fusion protein of the present invention comprises at least one IC-TP segment that targets, and facilitates local enrichment of the fusion protein at, microtubules, in particular the plus end or the minus end of the microtubules). For instance, dyneins and kinesins (proteins of the dynein or kinesin family of proteins), and functional fragments and mutants thereof, can be used as IC-TPs for such function.

In further particular embodiments, the fusion protein of the present invention comprises at least one IC-TP segment that is derived from, and functions as, a membrane anchor. For example, the fusion protein of the present invention comprises at least one IC-TP segment that targets, and facilitates local enrichment of the fusion protein at, the (inner) cell membrane (in particular the cytoplasmic side of the cell membrane). In another example, the fusion protein of the present invention comprises at least one IC-TP segment that targets, and facilitates local enrichment of the fusion protein at, the (outer) nuclear membrane (in particular the cytoplasmic side of the nuclear membrane). In further particular embodiments, the fusion protein of the present invention comprises at least one IC-TP segment that targets, and facilitates local enrichment of the fusion protein at, the outer mitochondrial membrane (in particular the cytoplasmic side of the mitochondrial membrane). In further particular embodiments, the fusion protein of the present invention comprises at least one IC-TP segment that targets, and facilitates local enrichment of the fusion protein at, the outer ER membrane (in particular the cytoplasmic side of the ER membrane). In further particular embodiments, the fusion protein of the present invention comprises at least one IC-TP segment that targets, and facilitates local enrichment of the fusion protein at, the outer Golgi membrane (in particular the cytoplasmic side of the Golgi membrane). For instance, the transmembrane domain of membrane proteins, and functional fragments and mutants thereof, can be used as IC-TPs for such function.

Polypeptides which target, and thus become locally enriched at, intracellular structural elements as described above, are known in the art and are useful as IC-TPs in the present invention. Specific examples of suitable IC-TPs include, but are not limited to:
- optionally truncated kinesin polypeptides which constitutively move towards, and become locally enriched at, microtubule-plus ends in living cells, for example optionally truncated kinesin family member 16B (KIF16B), e.g. optionally truncated *Homo sapiens* KIF16B (Uniprot: Q96L93), in particular the fragment covering KIF16B amino acid residues 1-400 (KIF16B₁₋₄₀₀) comprising the amino acid sequence of SEQ ID NO:20; or optionally truncated kinesin family member 13A (KIF13A), e.g. optionally truncated *Homo sapiens* KIF13A (Uniprot: Q9H1H9), in particular the KIF13A fragment covering amino acid residues 1-411 wherein P390 is deleted (KIF13A_{1-411,Δ390}) comprising the amino acid sequence of SEQ ID NO:22; polypeptides EB1, a microtubule tip binding protein, that binds to growing microtubule plus ends (Nehlig A, Molina A, Rodrigues-Ferreira S, Honoré S, Nahmias C. Regulation of end-binding protein EB1 in the control of microtubule dynamics. Cell Mol Life Sci. 2017;74(13):2381-2393. doi:10.1007/s00018-017-2476-2) (Uniprot:Q15691) and hence targets the organelle to microtubule-plus ends and comprising the amino acid sequence of SEQ ID NO:302
- polypeptides targeting the outer mitochondrial membrane derived from transmembrane-proteins such as, e.g., optionally truncated translocase of outer mitochondrial membrane 20 (TOMM20), for example optionally truncated *Homo sapiens* TOMM20 (Uniprot: Q15388), in particular the fragment covering amino acid residues 1-70 of TOMM20 (TOMM20₁₋₇₀) comprising the amino acid sequence of SEQ ID NO:24;
- cell membrane-targeting polypeptides derived from transmembrane-proteins such as, e.g., lymphocyte-specific protein tyrosine kinase (LcK; e.g., *Mus musculus* LcK, Uniprot: P06240), CD4 (e.g., *Mus musculus* CD4, Uniprot: P06332), FRB (similar to *Homo sapiens* mTOR; Uniprot: P42345), CD28 (e.g., *Mus musculus* CD28, Uniprot: P31041) and combinations thereof, in particular polypeptides comprising the amino acid sequence of SEQ ID NO:26, SEQ ID NO:28 or SEQ ID NO:30;
- polypeptides CG1, a nucleoporin that binds to the cytoplasmic side of the nuclear pore complex (Fernandez-Martinez J, Kim SJ, Shi Y, et al. Structure and Function of the Nuclear Pore Complex Cytoplasmic mRNA Export Platform. Cell. 2016;167(5):1215-1228.e25. doi:10.1016/j.cell.2016.10.028) (also designated Nup42) (Uniprot:O15504) targeting the cytoplasmic side of the nuclear membrane comprising the amino acid sequence of SEQ ID NO:304
- polypeptides EBAG9, Golgi membrane protein with one transmembrane helix (Engelsberg A, Hermosilla R, Karsten U, Schülein R, Dörken B, Rehm A. The Golgi protein RCAS1 controls cell surface expression of tumor-associated O-linked glycan antigens. J Biol Chem. 2003;278(25):22998-23007. doi:10.1074/jbc.M301361200 (Uniprot:O00559) targeting the cytoplasmic side of the Golgi membrane comprising the amino acid sequence of SEQ ID NO:292 (full length) or comprising the first 29 N-terminal amino acid residues of SEQ ID NO:294; or polypeptides CMP Sia Tr, the CMP sialic acid transporter, a Golgi protein with 10 transmembrane helices (Eckhardt M, Gotza B, Gerardy-Schahn R. Membrane topology of the mammalian CMP-sialic acid transporter. J Biol Chem. 1999;274(13):8779-8787. doi:10.1074/jbc.274.13.8779) (Uniprot:P78382) targeting the cytoplasmic side of the Golgi membrane comprising the amino acid sequence of SEQ ID NO:296
- polypeptide fragments of P450 2C1, a endoplasmic reticulum resident protein (Fazal FM, Han S, Parker KR, et al. Atlas of Subcellular RNA Localization Revealed by APEX-Seq. Cell. 2019;178(2):473-490.e26. doi:10.1016/j.cell.2019.05.027) (Uniprot:P78382) targeting the cytoplasmic side of the ER membrane in particular a fragment comprising the N-terminal first 27 (SEQ ID NO:298); or the first 29 (SEQ ID NO:300;)amino acid residues
- The transmembrane protein stomatin-like protein 3 (SLP-3) (membrane comprising the amino acid sequence of SEQ ID NO:310; aa 1-59 (Homo sapiens, Uniprot: Q8TAV4), localizing to the plasma membrane and vesicular membranes (Lapatsina L, Jira JA, Smith ES, et al. Regulation of ASIC channels by a stomatin/STOML3 complex located in a mobile vesicle pool in sensory neurons. Open Biol. 2012;2(6):120096. doi:10.1098/rsob.120096)
as well as functional fragments and mutants of these polypeptides. Said functional fragments and mutants may have at least 60%, at least 70%, at least 80%, at least 90%, at least 95%, at least 96%, at least 97%, at least 98% or at least 99% amino acid sequence identity to the amino acid of the polypeptide they are derived from.

A further type of APs are polypeptide segments, which are derived from, and function in the context of the fusion protein as, phase separation polypeptides (PSPs). PSPs are polypeptides, which have the ability to self-assemble in the cytoplasm of a cell so as to create sites of high local concentration in the cytoplasm. Specifically, PSPs are able to drive phase separation (in particular liquid-liquid phase separation) leading to the formation of membrane-less compartments in the cytoplasm. Said compartments may take the form of droplets, aggregates, condensates or a dense phase. In particular, PSPs include intrinsically disordered proteins (IDPs) which are an important class of proteins that drive phase separation (see, e.g., Alberti et al., Bioessays 2016, 38:959-968 and references cited therein such as Patel et al., Cell 2015, 162:1066-1077; Han et al., Cell 2012, 149:768-779; Kato et al., Cell 2012, 149:753-767). There are three different classes of ICPs, proteins of each, or functional fragments or mutants thereof, can be used as PSPs in the present invention. One prominent class of IDPs contains so called prion-like domains which are devoid of charges and contain polar amino acid residues (Q, N, S, G) with interspersed aromatic residues (F, Y). See, e.g., Malinovska et al., Biochim Biophys Acta 2013, 1834:918-931; Alberti et al., 2009, Cell 137:146-158, Malinovska et al., Prion 2015, 9:339-346. Another class of IDPs is also characterized by low sequence complexity but frequently contains acidic and basic amino acid side chains, e.g. RGG repeat containing IDPs such as Ddx4. See Nott et al., Cell 2015, 57:936-947. Specific examples of suitable IC-TPs include, but are not limited to:
- spindle-defective protein 5 (SPD5) (e.g., *Caenorhabditis elegans* SPD5; Uniprot: P91349), in particular a polypeptide comprising the amino acid sequence of SEQ ID NO:32;
- fused-in sarcoma (FUS) (e.g., *Homo sapiens* FUS; Uniprot: P35637), in particular a polypeptide comprising the amino acid sequence of SEQ ID NO:34;
- Ewing sarcoma breakpoint region 1 (EWSR1) (e.g., *Homo sapiens EWSR1*; Uniprot: Q01844) , in particular a polypeptide comprising the amino acid sequence of SEQ ID NO:36;
- ATP-dependent RNA helicase laf-1 (RGG domain, 1-168, LAF-1 membrane comprising the amino acid sequence of SEQ ID NO:308;) (*Caenorhabditis elegans,* Uniprot: D0PV95), (Schuster BS, Reed EH, Parthasarathy R, et al. Controllable protein phase separation and modular recruitment to form responsive membraneless organelles. Nat Commun. 2018;9(1):2985. Published 2018 Jul 30. doi:10.1038/s41467-018-05403-1)
as well as functional fragments and mutants of these polypeptides. Said functional fragments and mutants may comprise at least 60%, at least 70%, at least 80%, at least 90%, at least 95%, at least 96%, at least 97%, at least 98% or at least 99% amino acid sequence identity to the amino acid of the polypeptide they are derived from.

The number of APs comprised by fusion proteins of the present invention is not particularly limited, i.e. a fusion protein may comprise 1, 2, 3, 4, 5, 6, 7, 8, 9, 10 or more same or different APs. Fusion proteins of the present invention which comprise at least one AP selected from IC-TP segments and at least one AP selected from PSP segments are particularly preferred. Likewise, the number of RNA-TP segments is not particularly limited and may be independently selected from 1, 2, 3, 4, 5 or more, as for example 6, 7, 8, 9 or 10, different or same RNA-TP segments. Likewise, the number of O-RS segments is not particularly limited and may be independently selected from 1, 2, 3, 4, 5 or more, as for example 6, 7, 8, 9 or 10, different or same O-RS segments. This applies to both AFPs as well as to RNA-TP/O-RS fusion proteins. The number of segments in the fusion proteins of the present invention of course influences the size of the fusion protein that is not particularly limited but typically less than 3500 amino acid residues, such as less than 3000 amino acid residues.

The order of the segments within the fusion proteins of the invention is not particularly limited either. The RNA-TP, O-RS and/or AP segments may thus be functionally linked in any order. Examples of RNA-TP/O-RS fusion protein structures (comprising both types of EP segments) include, but are not limited to,
[RNA-TP]ₓ- [O-RS]_{y}
[O-RS]_{y}- [RNA-TP]ₓ

wherein x and y, independently of each other, are integers selected from 1, 2, 3, 4 and 5; "-" designates a peptidic linkage.
[RNA-TP]ₓ for x≥2 may include the same or different RNA-TP segments. [O-RS]_{y} for y≥2 may include the same or different O-RS segments.

Examples of RNA-TP/O-RS fusion protein structures include, but are not limited to:
[IC-TP]ₘ - [EP]ₒ
[EP]ₒ - [IC-TP]ₘ
[PSP]ₙ - [EP]ₒ
[EP]ₒ - [PSP]ₙ
[IC-TP]ₘ - [EP]ₒ - [PSP]ₙ
[PSP]ₙ - [EP]ₒ - [IC-TP]ₘ
[IC-TP]ₘ - [PSP]ₙ - [EP]ₒ
[EP]ₒ - [PSP]ₙ - [IC-TP]ₘ
[PSP]ₙ - [IC-TP]ₘ - [EP]ₒ
[EP]ₒ - [IC-TP]ₘ - [PSP]ₙ
wherein m, n and o, independently of each other, are integers selected from 1, 2, 3, 4 or 5, or are selected from 1, 2, 3, 4, 5, 6 and "-" designates a peptidic linkage.

In a preferred embodiment "m" is the integer 1.

In another preferred embodiment "n" is an integer selected from 1 and 2.

In still another preferred embodiment "o" is an integer selected from 1, 2, 3, 4, 5 or 6 if EP is selected from RNA-TPs.

In still another preferred embodiment "o" is an integer selected from 1 or 2, if EP is selected from O-RSs.

In still another preferred embodiment of RNA-TP/O-RS fusion protein structures those are preferred wherein at least one ICT-TP takes a C- or N- terminal position within the polypeptide chain.

In still another preferred embodiment of RNA-TP/O-RS fusion protein structures those are preferred wherein at least one EP takes a C- or N- terminal position within the polypeptide chain.

In still another preferred embodiment of RNA-TP/O-RS fusion protein structures those are preferred wherein at least one ICT-TP takes a C- or N- terminal position within the polypeptide chain while at least one EP takes a N- or C- terminal position, respectively, within the polypeptide chain. Any PSP, if present in such structure, is positioned within the polypeptide chain.

[IC-TP]ₘ for m≥2 may include the same or different IC-TP segments. Preferably IC-TPs of the same functionality (targeting the same type of cellular structure (as for example same membrane type or type or organelle) are applied. [PSP]ₙ for n≥2 may include the same or different PSP segments. [EP]ₒ for o≥2 may include the same or different EPs. Where [EP]ₒ includes different EPs, for example at least one EP may be a RNA-TP segment and at least one may be an O-RS segment.

The fusion proteins of the present invention provide an orthogonal translation (OT) system wherein the one or more O-RS (segments) required for the introduction of the one or more ncAA residues into the POI are brought into spatial proximity to at least one RNA-targeting polypeptide (RNA-TP) segment. The mRNA of the POI comprises at least one targeting nucleotide sequence (TN) that is able to interact with an RNA-TP segment of at least one of the fusion proteins of the OT system. Said interaction is expediently a specific one. The RNA-TP segments of the fusion proteins of the invention are preferably mRNA-targeting polypeptide segments. The RNA-TP segment of the fusion protein and the TN of the POI mRNA are expediently chosen so as to specifically interact with (bind to) one another. Suitable pairs of RNA-TP segment and TN for this purpose can be selected from coat proteins of RNA viruses and the nucleic acid motifs bound by said coat proteins. Such viral coat proteins and protein-bound RNA motifs are known in the art.

Specific examples of suitable RNA-TPs include, but are not limited to:
- MCP (coat protein of *Enterobacteria* phage MS2), in particular a polypeptide comprising the amino acid sequence of SEQ ID NO:14;
- λ_{N22} (22 amino acid RNA-binding domain of lambda phage antiterminator protein N), in particular a polypeptide comprising the amino acid sequence of SEQ ID NO:16;
- PCP (coat protein of Bacteriophage PP7, Wu B, Chao JA, Singer RH. Fluorescence fluctuation spectroscopy enables quantitative imaging of single mRNAs in living cells. Biophys J. 2012;102(12):2936-2944. doi:10.1016/j.bpj.2012.05.017), in particular a polypeptide comprising the amino acid sequence of SEQ ID NO:306;
as well as functional fragments and mutants of these polypeptides. Said functional fragments and mutants may comprise at least 60%, at least 70%, at least 80%, at least 90%, at least 95%, at least 96%, at least 97%, at least 98% or at least 99% amino acid sequence identity to the amino acid of the polypeptide they are derived from.

Specific examples of suitable TNs include, but are not limited to:
- *Enterobacteria phage* MS2 RNA stem-loop, in particular a polynucleotide having an RNA sequence corresponding to (encoded by) the nucleotide (DNA) sequence of SEQ ID NO:17;
- BoxB (lambda phase RNA stem-loop, specific binding site of λ_{N22}), in particular a polynucleotide having an RNA sequence corresponding to (encoded by) the nucleotide (DNA) sequence of SEQ ID NO:18;
- Bacteriophage pp7 RNA stem loops (Wu B, Chao JA, Singer RH. Fluorescence fluctuation spectroscopy enables quantitative imaging of single mRNAs in living cells. Biophys J. 2012;102(12):2936-2944. doi:10.1016/j.bpj.2012.05.017) in particular a polynucleotide having an RNA sequence corresponding to (encoded by) the nucleotide (DNA) sequence of SEQ ID NO:289 or SEQ ID NO:290
as well as functional fragments and mutants thereof. Said functional fragments and mutants may comprise at least 60%, at least 70%, at least 80%, at least 90%, at least 95%, at least 96%, at least 97%, at least 98% or at least 99% nucleotide sequence identity to the polynucleotide sequences they are derived from.

Such TNs may be used as a single copy segment or as multiple copy segment composed of more than one, as for example two, three, four, five, six or more repetitive units of the TN.

MCP specifically interacts with MS2 RNA stem-loops. Thus, where the RNA-TP segment(s) of the fusion protein(s) comprise (consist of) segments which are derived from, and function as, MCP, the mRNA of the POI expediently comprises one or more MS2 RNA stem-loops, e.g. two, three, four, five or six MS2 RNA stem-loops. λ_{N22} specifically interacts with BoxB. Thus, where the RNA-TP segment(s) of the fusion protein(s) comprise (or consist of) segments which are derived from, and function as, λ_{N22}, the mRNA of the POI expediently comprises one or more BoxB motifs, e.g. one, two, three, four, five or six or more BoxB motifs. PCP specifically interacts with pp7 RNA stem-loops. Thus, where the RNA-TP segment(s) of the fusion protein(s) comprise (consist of) segments which are derived from, and function as, PCP, the mRNA of the POI expediently comprises one or more pp7 RNA stem-loops, e.g. two, three, four, five or six or more pp7 RNA stem-loops.

Several RSs have been used for genetic code expansion including the *Methanococcus jannaschii* tyrosyl-tRNA synthetase, *E.coli* tyrosyl-tRNA synthetase, *E.coli* leucyl-tRNA synthetase pyrrolysyl-tRNA synthetases from certain *Methanosarcina* (such as M. *mazei, M. barkeri, M. acetivorans, M. thermophila*)*, Methanococcoides* (*M. burtonii*) or *Desulfitobacterium (D. hafniense).* Corresponding orthogonal RS/tRNA pairs have been used to genetically encode a variety of functionalities in polypeptides (Chin, Annu Rev Biochem 2014, 83:379-408; Chin et al., J Am Chem Soc 2001, 124:9026; Chin et al., Science 2003, 301:964; Nguyen et al., J Am Chem Soc 2009, 131:8720; Yanagisawa et al., Chem Biol 2008, 15:1187). Depending on the cell used for the translation of the POI, these RS can be used as O-RS in the present invention.

Pyrrolysyl tRNA synthetases (PyIRSs) which can be used in methods and fusion proteins of the invention may be wildtype or genetically engineered PyIRSs. Examples for wildtype PylRSs include, but are not limited to PyIRSs from archaebacteria and eubacteria such as *Methanosarcina maize, Methanosarcina barkeri, Methanococcoides burtonii, Methanosarcina acetivorans, Methanosarcina thermophila* and *Desulfitobacterium hafniense.* Genetically engineered PyIRSs have been described, for example, by Neumann et al. (Nat Chem Biol 2008, 4:232), by Yanagisawa et al. (Chem Biol 2008, 15:1187), and in EP2192185A1. The efficiency of genetic code expansion using PyIRS can be increased by modifying the amino acid sequence of the PyIRS such that it is not directed to the nucleus. To this end, the nuclear localization signal (NLS) can be removed from the PyIRS or can be overridden by introducing a suitable nuclear export signal (NES). PylRSs which are used in the fusion proteins and methods of the present invention may be PylRSs lacking the NLS and/or comprising a NES as described, e.g., in WO 2018/069481.

Accordingly, examples of O-RS segment(s) which can be used in the fusion proteins of the present invention include, but are not limited to:
- *Methanococcus jannaschii* tyrosyl-tRNA synthetase;
- *Escherichia coli* tyrosyl-tRNA synthetase;
- *Escherichia coli* leucyl-tRNA synthetase;
- *Methanosarcina mazei* pyrrolysyl-tRNA synthetase;
- *Methanosarcina barkeri* pyrrolysyl-tRNA synthetase;
- *Methanosarcina acetivorans* pyrrolysyl-tRNA synthetase;
- *Methanosarcina thermophila* pyrrolysyl-tRNA synthetase;
- *Methanococcoides burtonii* pyrrolysyl-tRNA synthetase;
- *Desulfitobacterium hafniense* pyrrolysyl-tRNA synthetase;
as well as functional (i.e., enzymatically active) fragments and mutants of these polypeptides. Said functional fragments and mutants may comprise at least 60%, at least 70%, at least 80%, at least 90%, at least 95%, at least 96%, at least 97%, at least 98% or at least 99% amino acid sequence identity to the aminoacyl tRNA synthetase they are derived from.

Particular examples of O-RS segments useful as in the present invention which are derived from *M. mazei* pyrrolysyl-tRNA synthetases include, but are not limited to:
- O-RS segments derived from PyIRS^{AF} (*Methanosarcina mazei* pyrrolysyl tRNA synthetase double mutant: Y306A, Y384F; Uniprot: Q8PWY1), for example O-RS segments comprising the amino acid sequence of SEQ ID NO:8;
- O-RS segments derived from PyIRS^{AA} (*Methanosarcina mazei* pyrrolysyl tRNA synthetase double mutant: N346A, C348A; Uniprot: Q8PWY1), for example O-RS segments comprising the amino acid sequence of SEQ ID NO:10;
- O-RS segments derived from PyIRS^{AAAF} (*Methanosarcina mazei* pyrrolysyl tRNA synthetase quadruple mutant: Y306A, N346A, C348A, Y384F; Uniprot: Q8PWY1), for example O-RS segments comprising the amino acid sequence of SEQ ID NO:12;
- O-RS segment derived from IFRS1, a *Methanosarcina mazei* pyrrolysyl tRNA mutant (L305M, Y306L, L309S, N346S, C348M), for example O-RS segments comprising the amino acid sequence of SEQ ID NO:224
- O-RS segment derived from CbzRS, a *Methanosarcina mazei* pyrrolysyl tRNA mutant (Y306M, L309G, C348T), for example O-RS segments comprising the amino acid sequence of SEQ ID NO:226
- O-RS segment derived from CpkRS, a *Methanosarcina mazei* pyrrolysyl tRNA mutant (A302S), for example O-RS segments comprising the amino acid sequence of SEQ ID NO:228
- O-RS segment derived from OMeRS, a *Methanosarcina mazei* pyrrolysyl tRNA mutant: (A302T, Y384F, N346V, C348W, V401L), for example O-RS segments comprising the amino acid sequence of SEQ ID NO:236
as well as functional (i.e., enzymatically active) fragments and mutants of these polypeptide segments. Said functional fragments and mutants may comprise at least 60%, at least 70%, at least 80%, at least 90%, at least 95%, at least 96%, at least 97%, at least 98% or at least 99% amino acid sequence identity to the aminoacyl tRNA synthetase they are derived from.

According to particular embodiments, wild-type and mutant *M*. *mazei* PyIRSs as described herein are used for aminoacylation of tRNA with ncAAs as described in WO2012/104422 or WO2015/107064. Exemplary ncAAs for this purpose include, but are not limited to, 2-amino-6-(cyclooct-2-yn-1-yloxycarbonylamino)hexanoic acid (SCO), 2-amino-6-(cyclooct-2-yn-1-yloxyethoxycarbonylamino)hexanoid acid, 2-amino-6[(4E-cyclooct-4-en-1-yl)oxycarbonyl-amino]hexanoic acid (TCO), 2-amino-6[(2E-cyclooct-2-en-1-yl)oxycarbonylamino]hexanoic acid (TCO*), 2-amino-6-(prop-2-ynoxycarbonylamino)hexanoic acid (PrK) and 2-amino-6-(9-biocyclo[6.1.0]non-4-ynylmethoxycarbonylamino)hexanoid acid (BCN).

In another embodiment of the present invention, the above-mentioned AP (IC-TP and PSP) segments and/or the above mentioned EP (RNA-TP and O-RS) segments, independently of each other, may be further combined with natural or, more particularly, synthetic protein segments, which induce and control macromolecular interactions. In particular, such further protein segments are operably fused into the polypeptide chain of an AFP of the invention. One or more, like 2, 3, 4, 5, 6, 7, 8, 9 or 10, preferably however one such protein segment may be operably fused into a single AFP of the invention. Fusion into the AFP polypeptide chain should be such that the activity of the other polypeptide segments, AP and EP, is substantially unaffected, in particular not inhibited (i.e. AP and EP remain operable), while the ability of the additional polypeptide segment to induce and control macromolecular interactions is retained. Described in literature are so-called SYNZIP peptides, forming multimeric structures. Of particular interest in the context of the invention are SYNZIPs having the ability to form specific heterodimeric coiled-coil protein structures. Such SYNZIPs are pairs of synthetic peptides capable of interacting with each other and are used to induce and control macromolecular interactions. Non-limiting examples are the pairs SYNZIP 1:2; SYNZIP 3:4 and SYNZIP 5:6. Particularly preferred according to the invention is the heterospecific coiled-coil pair SYNZIP2:SYNZIP1 as described by Reinke, A.W., Grant, R.A., Keating, A.E. (2010) J Am Chem Soc 132 6025-6031 (SYNZIP 1: SEQ ID NO:312; SYNZIP 2: SEQ ID NO:314, SYNZIP 3: SEQ ID NO:316; SYNZIP 4: SEQ ID NO:318, as well as functional fragments and mutants of these SYNZIP polypeptides. Said functional fragments and mutants may comprise at least 60%, at least 70%, at least 80%, at least 90%, at least 95%, at least 96%, at least 97%, at least 98% or at least 99% amino acid sequence identity to the amino acid of the polypeptide they are derived from). As a pairwise use is required to induce macromolecular interaction, these SYNZIPs are preferably used pairwise in AFP combinations as described herein. By the interaction of such SYNZIP pairs integrated in different AFP fusion proteins the formation of OT organelles according to the present invention may be further supported.

In still another embodiment of the present invention a fusion protein of the invention may be further modified by introducing into (fusing of) at least one so-called "epitope tag", i.e. a short oligopeptide sequence, which serves as antibody binding sites, useful for detecting/quantifying the expressed fusion products of the invention. Non-limiting examples of such tags are the following:

| | |
|---|---|
| VSV-G: | Vesicular stomatitis virus glycoprotein epitope tag (SEQ ID NO:680) |
| HA: | Human influence hemagglutinin epitope tag (SEQ ID NO:682) |
| Myc: | Human c-Myc proto-oncogene epitope tag (SEQ ID NO:684) |

### 1.2 Particular examples of AFP constructs of the invention

Each individual exemplified construct may be construed in the N->C or C->N direction. The depicted schemes are given in the N->C direction.

In the case of segment blocks [IC-TP]ₘ, [PSP]ₙ, [O-RS]_{y} and [RNA-TP]ₓ, wherein m, n, y or x are an integer >1, the repetitive segments within such block may be identical or different, preferably identical.

The segments [IC-TP], [PSP], [O-RS], [RNA-TP]ₓ, and [SYNZIP] as applied therein may be prepared from the respective examples of segments described above in section 1.1..

### 1.2.1. Intracellular structure-targeting monofunctional AFPs

### 1.2.1.1 Intracellular structure-targeting monofunctional AFPs (i.e. comprising one type of EP)

Individually preferred examples thereof are:
[IC-TP]ₘ - [O-RS]_{y} with m =1 or 2, preferably 1; y = 1 or 2, preferably 1;
[IC-TP]ₘ - [RNA-TP]ₓ with m =1 or 2, preferably 1; x = 1, 2, 3, 4, 5 or 6 , preferably 2, 3 or 4;
[IC-TP]ₘ - [PSP]ₙ - [O-RS]_{y} with m =1 or 2, preferably 1; n =1, 2 or 3, preferably 1 or 2; y = 1 or 2, preferably 1;
[IC-TP]ₘ - [PSP]ₙ - [RNA-TP]ₓ with m =1 or 2, preferably 1; n =1, 2 or 3, preferably 1 or 2; x = 1, 2, 3, 4, 5 or 6, preferably 2, 3 or 4;
[IC-TP]ₘ - [O-RS₁]_{y} - [PSP]ₙ - [O-RS₂]_{y} with m =1 or 2, preferably 1; n =1, 2 or 3, preferably 1 or 2; y independently of each other = 1 or 2, preferably 1; and O-RS₁ and O-RS₂ identical or different, preferably identical;
[IC-TP]ₘ - [PSP₁]ₙ - [O-RS]_{y} - [PSP₂]ₙ with m =1 or 2, preferably 1; n independently of each other 1, 2 or 3, preferably 1 or 2; y independently of each other = 1 or 2, preferably 1; and PSP₁ and PSP₂ identical or different;
[IC-TP]ₘ - [RNA-TP₁]ₓ - [PSP]ₙ - [RNA-TP₂]ₓ with m =1 or 2, preferably 1; n =1, 2 or 3, preferably 1 or 2; x independently of each other = 1, 2, 3, 4, 5 or 6, preferably 2, 3 or 4; and RNA-TP₁ and RNA-TP₂ identical or different, preferably identical;
[IC-TP]ₘ - [PSP₁]ₙ - [O-RS₁]_{y} - [PSP₂]ₙ - [O-RS₂]_{y} with m =1 or 2, preferably 1; n independently of each other 1, 2 or 3, preferably 1 or 2; y independently of each other = 1 or 2, preferably 1; O-RS₁ and O-RS₂ identical or different, preferably identical; and PSP₁ and PSP₂ identical or different;
[IC-TP]ₘ - [PSP₁]ₙ - [RNA-TP₁]ₓ - [PSP₂]ₙ - [RNA-TP₂]ₓ with m =1 or 2, preferably 1; n independently of each other =1, 2 or 3, preferably 1 or 2; x independently of each other = 1, 2, 3, 4, 5 or 6, preferably 2, 3 or 4; RNA-TP₁ and RNA-TP₂ identical or different; and PSP₁ and PSP₂ identical or different.

### 1.2.1.2 Intracellular structure-targeting bifunctional AFPs (comprising two types of EP)

Individually preferred examples thereof are
[IC-TP]ₘ - [O-RS]_{y} - [RNA-TP]ₓ with m =1 or 2, preferably 1; x = 1, 2, 3, 4, 5 or 6 , preferably 2, 3 or 4; y = 1 or 2, preferably 1;
[IC-TP]ₘ - [RNA-TP]ₓ- [O-RS]_{y} with m =1 or 2, preferably 1; x = 1, 2, 3, 4, 5 or 6 , preferably 2, 3 or 4; y = 1 or 2, preferably 1;
[IC-TP]ₘ - [PSP]ₙ - [O-RS]_{y} - [RNA-TP]ₓ with m =1 or 2, preferably 1; n =1, 2 or 3, preferably 1 or 2; x = 1, 2, 3, 4, 5 or 6 , preferably 2, 3 or 4; y = 1 or 2, preferably 1;
[IC-TP]ₘ - [PSP]ₙ - [RNA-TP]ₓ- [O-RS]_{y} with m =1 or 2, preferably 1; n =1, 2 or 3, preferably 1 or 2; x = 1, 2, 3, 4, 5 or 6 , preferably 2, 3 or 4; y = 1 or 2, preferably 1;
[IC-TP]ₘ - [O-RS]_{y} - [PSP]ₙ - [RNA-TP]ₓ with m =1 or 2, preferably 1; n =1, 2 or 3, preferably 1 or 2;; x = 1, 2, 3, 4, 5 or 6 , preferably 2, 3 or 4; y = 1 or 2, preferably 1;
[IC-TP]ₘ - [RNA-TP]ₓ - [PSP]ₙ - [O-RS]_{y} with m =1 or 2, preferably 1; n =1, 2 or 3, preferably 1 or 2; x = 1, 2, 3, 4, 5 or 6 , preferably 2, 3 or 4; y = 1 or 2, preferably 1;
[IC-TP]ₘ - [PSP₁]ₙ - [O-RS]_{y} - [PSP₂]ₙ - [RNA-TP]ₓ with m =1 or 2, preferably 1; n independent of each n=1, 2 or 3, preferably 1 or 2; x = 1, 2, 3, 4, 5 or 6 , preferably 2, 3 or 4; y = 1 or 2, preferably 1; and PSP₁ and PSP₂ identical or different;
[IC-TP]ₘ - [PSP₁]ₙ - [RNA-TP]ₓ - [O-RS₁]_{y} - [PSP₂]ₙ - [O-RS₂]_{y} with m =1 or 2, preferably 1; n independent of each =1, 2 or 3, preferably 1 or 2; x = 1, 2, 3, 4, 5 or 6 , preferably 2, 3 or 4; y independently of each other = 1 or 2, preferably 1; and PSP₁ and PSP₂ identical or different; and O-RS₁ and O-RS₂ identical or different, preferably identical;
[IC-TP]ₘ - [PSP₁]ₙ - [O-RS₁]_{y} - [PSP₂]ₙ - [O-RS₂]_{y} - [RNA-TP]ₓ with m =1 or 2, preferably 1; n independent of each =1, 2 or 3, preferably 1 or 2; x = 1, 2, 3, 4, 5 or 6 , preferably 2, 3 or 4; y independently of each other = 1 or 2, preferably 1; and PSP₁ and PSP₂ identical or different; and O-RS₁ and O-RS₂ identical or different, preferably identical.

### 1.2.2. No Intracellular structure-targeting monofunctional AFPs

These are the same AFPs as listed above in section 1.2.1, with the only exception that the segments [IC-TP] is missing, while the segments [PSP] are retained.

### 1.2.3. SYNZIP Variants

These are the same AFPs as listed above in sections 1.2.1 and 1.2.2 with the only exception that at least one of the segment [IC-TP], [PSP], [O-RS₂] or [RNA-TP] is N- or C- terminally supplemented with a SYNZIP element. An AFP may contain, 1, 2, 3, 4 or 5, preferably 1 or 2, identical or different, preferably identical SYNZIPs. Non-limiting examples of such molecules are:

### 1.2.3.1 Monofunctional SYNZIP AFPs

Individually preferred examples thereof are:
[PSP]ₙ- [SYNZIP] - [O-RS]_{y} with y = 1 or 2, preferably 1; n =1, 2 or 3, preferably 1 or 2;
[PSP]ₙ- [SYNZIP]- [RNA-TP]ₓ with; x = 1, 2, 3, 4, 5 or 6 , preferably 2, 3 or 4; n =1, 2 or 3, preferably 1 or 2;
[IC-TP]ₘ- [SYNZIP] - [O-RS]_{y} with m =1 or 2, preferably 1; y = 1 or 2, preferably 1;
[IC-TP]ₘ - [SYNZIP]- [RNA-TP]ₓ with m =1 or 2, preferably 1; x = 1, 2, 3, 4, 5 or 6 , preferably 2, 3 or 4;
[IC-TP]ₘ - [PSP]ₙ- [SYNZIP] - [O-RS]_{y} with m =1 or 2, preferably 1; n =1, 2 or 3, preferably 1 or 2; y = 1 or 2, preferably 1;
[IC-TP]ₘ - [PSP]ₙ - [SYNZIP] - [RNA-TP]ₓ with m =1 or 2, preferably 1 n =1, 2 or 3, preferably 1 or 2; x = 1, 2, 3, 4, 5 or 6, preferably 2, 3 or 4.

### 1.2.3.2 Bifunctional SYNZIP AFPs

Individually preferred examples thereof are:
[IC-TP]ₘ - [O-RS]_{y}- [SYNZIP] - [RNA-TP]ₓ with m =1 or 2, preferably 1; x = 1, 2, 3, 4, 5 or 6 , preferably 2, 3 or 4; y = 1 or 2, preferably 1;
[IC-TP]ₘ - [RNA-TP]ₓ- [SYNZIP]- [O-RS]_{y} with m =1 or 2, preferably 1; x = 1, 2, 3, 4, 5 or 6 , preferably 2, 3 or 4; y = 1 or 2, preferably 1;
[IC-TP]ₘ - [PSP]ₙ- [SYNZIP] - [O-RS]_{y} - [RNA-TP]ₓ with m =1 or 2, preferably 1; n =1, 2 or 3, preferably 1 or 2; x = 1, 2, 3, 4, 5 or 6 , preferably 2, 3 or 4; y = 1 or 2, preferably 1;
[IC-TP]ₘ - [PSP]ₙ - [SYNZIP]- [RNA-TP]ₓ- [O-RS]_{y} with m =1 or 2, preferably 1; n =1, 2 or 3, preferably 1 or 2; x = 1, 2, 3, 4, 5 or 6 , preferably 2, 3 or 4; y = 1 or 2, preferably 1;
[IC-TP]ₘ - [PSP]ₙ- [SYNZIPₐ] - [O-RS]_{y} - [SYNZIP_{b}] - [RNA-TP]ₓ with m =1 or 2, preferably 1; n =1, 2 or 3, preferably 1 or 2; x = 1, 2, 3, 4, 5 or 6 , preferably 2, 3 or 4; y = 1 or 2, preferably 1;and SYNZIPₐ and SYNZIP_{b} identical or different, preferably identical
[IC-TP]ₘ - [PSP]ₙ - [SYNZIPₐ]- [RNA-TP]ₓ- [SYNZIP_{b}] - [O-RS]_{y} with m =1 or 2, preferably 1 n =1, 2 or 3, preferably 1 or 2; x = 1, 2, 3, 4, 5 or 6 , preferably 2, 3 or 4; y = 1 or 2, preferably 1; and SYNZIPₐ and SYNZIP_{b} identical or different, preferably identical
[IC-TP]ₘ - [PSP₁]ₙ - [SYNZIP]- [RNA-TP]ₓ- [O-RS₁]_{y} - [PSP₂]ₙ - [O-RS₂]_{y} with m =1 or 2, preferably 1 n =1, 2 or 3, preferably 1 or 2; x = 1, 2, 3, 4, 5 or 6 , preferably 2, 3 or 4; y = 1 or 2, preferably 1; and PSP₁ and PSP₂ identical or different; and O-RS₁ and O-RS₂ identical or different, preferably identical.

### 1.2.4. Monofunctional fusion proteins

Individually preferred examples thereof are:
[SYNZIP] - [O-RS]_{y} with y = 1 or 2, preferably 1;
[SYNZIP]- [RNA-TP]ₓ with; x = 1, 2, 3, 4, 5 or 6 , preferably 2, 3 or 4;

As IC-TP and PSP is missing here, these may be preferably used in combination with an AFP molecule containing at least one C-TP and/or PSP segment.

### 1.3 Examples of individual fusion proteins

Very specific examples of fusion protein of the inventions, and particular combinations thereof are listed below in Tables 1, 2 and 3. The content of this Tables 1, 2 and 3 also forms part of general disclosure of the specification and its content is not explicitly and literally repeated here in the general part. The disclosure of Tables 1 and 2 in the respective column designated *"Fusion protein(s) comprising O-RS and RNA-TP segments"* shall be considered as disclosed independently from the content of the other columns of Tables 1 and 2 referring to specific reports and host cell lines.

### 2. Functional fragments and mutants

Described herein are fragments and mutants of particular RNA-TPs, O-RSs, IC-TPs, PSPs, TNs, as well as SYNZIPs which are functional (i.e. have the RNA-binding activity of the parent RNA-TP, the targeting activity for intracellular structures of the parent IC-TP, the self-assembly activity of the parent PSP, the binding activity for RNA-TP of the parent TN, the enzymatic activity of the parent O-RS, or the heterodimeric coiled-coil formation ability of parent SYNZIPs, respectively). Such fragments and mutants can be characterized by a minimum degree of sequence identity as described herein. Said amino acid or nucleotide sequence identity means identity over the entire length of the thus characterized amino acid or nucleotide sequence, respectively. The percentage identity values can be determined as known in the art on the basis of BLAST alignments, blastp algorithms (protein-protein BLAST), or using the Clustal method (Higgins et al., Comput Appl. Biosci. 1989, 5(2):151-1).

Fragments and mutants of particular RNA-TPs, O-RSs, IC-TPs, SYNZIPS or PSPs which are useful in the present invention retain the relevant function (binding, self-assembly or enzymatic activity, respectively) of the parent polypeptide and can be obtained, e.g., by conservative amino acid substitution, i.e. the replacement of an amino acid residue with different amino acid residues having similar biochemical properties (e.g. charge, hydrophobicity and size) as known in the art. Typical examples are substitution of Leu by Ile or *vice versa,* substitution of Asp by Glu or *vice versa,* substitution of Asn by Gln or *vice versa,* and others.

### 3. Orthogonal translation, tRNAs and POI coding sequences

The term "translation system" generally refers to a set of components necessary to incorporate a naturally occurring amino acid in a growing polypeptide chain (protein). Components of a translation system can include, e.g., ribosomes, tRNAs, aminoacyl tRNA synthetases, mRNA and the like. An aminoacyl tRNA synthetase (RS) is an enzyme capable of aminoacylating a tRNA with an amino acid or an amino acid analog. An RS used in processes of the invention is capable of aminoacylating a tRNA with the corresponding ncAA, i.e. aminoacylating a tRNA^{ncAA}. The term "orthogonal" as used herein refers to an element of a translation system (e.g., an orthogonal tRNA (O-tRNA) and/or an orthogonal aminoacyl tRNA synthetase (O-RS)) that is used with reduced efficiency by a translation system of interest (e.g., a cell). "Orthogonal" refers to the inability or reduced efficiency, e.g., less than 20% efficient, less than 10% efficient, less than 5% efficient, or e.g., less than 1 % efficient, of an O-tRNA or an O-RS to function with the endogenous RS or endogenous tRNAs, respectively, of a translation system of interest. For example, an O-tRNA in a translation system of interest is aminoacylated by any endogenous RA of the translation system with reduced or even zero efficiency, when compared to aminoacylation of an endogenous tRNA by the endogenous RS. In another example, an O-RS aminoacylates any endogenous tRNA in the translation system of interest with reduced or even zero efficiency, as compared to aminoacylation of the endogenous tRNA by an endogenous RS. Specifically, the term "orthogonal translation system" or "OT system" is used herein to refer to a translation system using an O-RS/O-tRNA^{ncAA} pair that allows for introducing ncAA residues into a growing polypeptide chain.

O-RS/O-tRNA^{ncAA} pairs used in the invention preferably have following properties: the O-tRNA^{ncAA} is preferentially aminoacylated with the ncAA by the O-RS. In addition, the orthogonal pair functions in the translation system of interest (e.g, the cell) such that the O-tRNA^{ncAA} is used to incorporate the ncAA residue into the growing polypeptide chain of a POI. Incorporation occurs in a site specific manner. Specifically, the O-tRNA^{ncAA} recognizes a selector codon (e.g., an Amber, Ochre or Opal stop codon) in the mRNA coding for the POI.

The term "preferentially aminoacylates" refers to an efficiency of, e.g., about 50% efficient, about 70% efficient, about 75% efficient, about 85% efficient, about 90% efficient, about 95% efficient, or about 99% or more efficient, at which an O-RS aminoacylates an O-tRNA with an unnatural amino acid compared to an endogenous tRNA or amino acid of a translation system of interest (e.g., a cell). The unnatural amino acid is then incorporated into a growing polypeptide chain with high fidelity, e.g., at greater than about 75% efficiency for a given selector codon, at greater than about 80% efficiency for a given selector codon, at greater than about 90% efficiency for a given selector codon, at greater than about 95% efficiency for a given selector codon, or at greater than about 99% or more efficiency for a given selector codon.

tRNAs which can be used for being aminoacylated by a fusion protein of the present invention comprising at least one O-RS segment derived from a *M*. *mazei* pyrrolysyl tRNA synthetase include, but are not limited to pyrrolysyl tRNA of *M*. *mazei* and functional mutants thereof wherein the anticodon is the anticodon to a selector codon such as, e.g., the CUA anticodon to the Amber stop codon TAG, the anticodon UCA to the Opal stop codon TGA, and the anticodon UUA to the Ochre stop codon TAA. Examples for such pyrrolysyl tRNAs include, but are not limited to, those encoded by the nucleotide sequence of SEQ ID NO:4 (tRNA^{Pyl,CUA}), SEQ ID NO:5 (tRNA^{Pyl,UCA}) or SEQ ID NO:6 (tRNA^{Pyl,UUA}). Non-limiting examples of further suitable tRNAs are the following ones derived from pyrrolysyl tRNA of M. *mazei:*

| | |
|---|---|
| tRNA^{pyl, CGA} | Pyrrolysyl tRNA (for Serine codon), SEQ ID NO: 229 |
| tRNA^{pyl, CGG} | Pyrrolysyl tRNA (for Proline codon), SEQ ID NO: 230 |
| tRNA^{pyl, UAA} | Pyrrolysyl tRNA (for Leucine codon), SEQ ID NO: 231 |
| tRNA^{pyl, UAG} | Pyrrolysyl tRNA (for Leucine codon), SEQ ID NO: 232 |
| tRNA^{pyl, CCG} | Pyrrolysyl tRNA (for Arginine codon), SEQ ID NO: 233 |
| tRNA^{pyl, AUA} | Pyrrolysyl tRNA (for Isoleucine codon), SEQ ID NO: 234 |

The term "selector codon" as used herein refers to a codon that is recognized (i.e. bound) by the O-tRN^{ncAA} in the translation process. The term is also used for the corresponding codons in polypeptide-encoding sequences of polynucleotides which are not messenger RNAs (mRNAs), e.g. DNA plasmids. The new OT systems described herein allow for orthogonal translation of POIs in a manner that is selective for the mRNA of said POls compared to other mRNAs present in the cytoplasm of the cell. Nevertheless, it is preferable that the selector codon is a codon of low abundance in the cell chosen for expression, for example a codon of low abundance in naturally occurring eukaryotic cells. The new OT systems bring the mRNA of the POls, the O-RS and the tRNA^{ncAA} into proximity to one another, thus supporting the introduction of the ncAA (rather than the introduction of an amino acid of a different tRNA that might potentially bind to the selector codon) at the selector codon-encoded amino acid position of the POI. Thus, the selector codon can be a sense codon. Nevertheless, in preferred embodiments, the selector codon is a codon that is not recognized by endogenous tRNAs of the cell used for preparing the POI.

The anticodon of the O-tRNA^{ncAA} binds to a selector codon within an mRNA (the mRNA of the POI) and thus incorporates the ncAA site-specifically into the growing chain of the polypeptide (POI) encoded by said mRNA. Examples for selector codons which are useful in the new OT systems described herein include, but are not limited to:
- nonsense codons, such as stop codons, *e.g*., Amber (UAG), Ochre (UAA), and Opal (UGA) codons;
- codons consisting of more than three bases (e.g., four base codons);
- codons derived from natural or unnatural base pairs; and
- sense codons.

Where a selector codon is used that is a sense codon (i.e., a natural three base codon), it is preferable that the endogenous translation system of the cell used for POI expression according to a method of the present invention does not (or only scarcely) use said natural three base codon, *e.g.*, a cell that is lacking, or has a reduced abundance of, a tRNA that recognizes the natural three base codon or a cell wherein the natural three base codon is a rare codon. The use of one or more stop codons, such as one or more of Amber, Ochre and Opal, as selector codons in the present invention is particularly preferred.

A number of selector codons can be introduced into a polynucleotide encoding a desired polypeptide (target polypeptide, POI), e.g., one or more, two or more, more than three, etc. selector codons. A POI can carry two or more ncAA residues. Said ncAA residues can be the same and encoded by the same type of selector codon, or can be different and encoded by different selector codons.

An anticodon has the reverse complement sequence of the corresponding codon.

A suppressor tRNA is a tRNA (such as an O-tRNA^{ncAA}) that alters the reading of a messenger RNA (mRNA) in a given translation system (e.g., a cell). A suppressor tRNA can read through, e.g., a stop codon, a four base codon, or a rare codon.

The O-tRNA is preferentially aminoacylated by O-RS (rather than endogenous synthetases) and is capable of decoding a selector codon, as described herein. The O-RS recognizes the O-tRNA, e.g., with an extended anticodon loop, and preferentially aminoacylates the O-tRNA with an ncAA.

The O-tRNA and the O-RS used in the methods and/or fusion proteins of the invention can be naturally occurring or can be derived by mutation of a naturally occurring tRNA and/or RS from a variety of organisms. In various embodiments, the tRNA and RS are derived from at least one organism. In another embodiment, the tRNA is derived from a naturally occurring or mutated naturally occurring tRNA from a first organism and the RS is derived from naturally occurring or mutated naturally occurring RS from a second organism.

A suitable (orthogonal) tRNA/RS pair may be selected from libraries of mutant tRNA and RS, e.g.. based on the results of a library screening. Alternatively, a suitable tRNA/RS pair may be a heterologous tRNA/synthetase pair that is imported from a source species into the translation system. Preferably, the cell used as translation system is different from said source species. Methods for evolving tRNA/RS pairs are described, e.g., in WO 02/085923 and WO 02/06075.

Conventional site-directed mutagenesis can be used to introduce selector codons into the coding sequence of a POI.

### 4. Nucleic acid molecules

The invention also relates to nucleic acid molecules (single-stranded or double-stranded DNA and RNA sequences, for example cDNA, mRNA), or combinations of such nucleic acid molecules, comprising a nucleotide sequence that encodes for at least one of the fusion proteins of the present invention, and/or a nucleotide sequence complementary thereto.

Further, the invention relates to nucleic acid molecules (single-stranded or double-stranded DNA and RNA sequences, for example cDNA, mRNA), or combinations of such nucleic acid molecules, comprising (i) a nucleotide sequences (CS^{POI}) that encodes at least one POI, said POI comprising one or more ncAA residues which are encoded in the CS^{POI} by selector codons, and (ii) a targeting nucleotide sequence (TN) as described herein, wherein an RNA molecule comprising (the RNA version of) said TN is able to interact via said TN with an RNA-targeting polypeptide (RNA-TP).

The nucleic acid molecules of the invention can in addition contain untranslated sequences of the 3'- and/or 5'-end of the coding gene region. The TN is preferably located at the 3' end of the nucleic acid molecule encoding the POI(s). For example, nucleic acid molecules of the invention encoding the POI(s) can be prepared by introducing at least one TN at (in particular 3' of) the 3' untranslated region using common cloning techniques known in the art.

The nucleic acid molecules of the invention can in addition contain untranslated sequences of the 3'- and/or 5'-end of the coding gene region.

The invention further relates to, in particular recombinant, expression constructs or expression cassettes, containing, under the genetic control of regulatory nucleic acid sequences the nucleic acid sequence of the nucleic acid molecule, or combination of nucleic acid molecules, of the invention as described herein. The expression cassettes of the invention thus comprise the nucleic acid sequence coding for at least one POI (plus TN) or at least one fusion protein of the invention, and/or a nucleic acid sequence complementary thereto. The invention also relates to, in particular recombinant, vectors, comprising at least one of these expression constructs (expression vectors).

An expression cassette typically comprises a promoter sequence that is located 5' (upstream) of, and functionally linked with, the nucleic acid sequence encoding the to-be-expressed POI(s) or fusion protein(s), a terminator sequence 3' (downstream) of said encoding sequence and optionally further regulatory elements. Examples of such further regulatory elements include, but are not limited to, targeting sequences, enhancers, polyadenylation signals, selectable markers, amplification signals, replication origins and the like. Suitable regulatory sequences are described for example in Goeddel, Gene Expression Technology: Methods in Enzymology 185, Academic Press, San Diego, CA (1990).

In addition to these regulatory sequences, the natural regulation of these sequences can still be present before the actual structural genes and optionally can have been genetically altered, so that the natural regulation has been switched off and expression of the genes has been increased. The nucleic acid construct can, however, also be of simpler construction, i.e. no additional regulatory signals have been inserted before the coding sequence and the natural promoter, with its regulation, has not been removed. Instead, the natural regulatory sequence is mutated so that regulation no longer takes place and gene expression is increased.

A "functional" linkage of elements of nucleic acid molecules, such as promotor, polypeptide-encoding sequence, terminator, regulators, means that these elements are arranged such that the encoding sequence can be transcribed and the optional regulatory elements can perform their regulation of said transcription. This can be achieved by a direct linkage of the elements in one and the same nucleic acid molecule. However, such direct linkage is not necessarily required. Genetic control sequences, for example enhancer sequences, can even exert their function on the target sequence from more remote positions or even from other DNA molecules. Arrangements are preferred in which the nucleic acid sequence to be transcribed is positioned downstream (i.e. at the 3'-end of) the promoter sequence, so that the two sequences are joined together covalently. The distance between the promoter sequence and the nucleic acid sequence to be expressed can be smaller than 200 base pairs, or smaller than 100 base pairs or smaller than 50 base pairs.

For expression in a cell, the expression cassette is advantageously inserted into an expression vector. Expression vectors are chosen according to the cell to be used for expression which makes optimal expression of the encoding nucleotide sequences in the cell possible. Vectors are well known by a person skilled in the art and are given for example in "Cloning vectors" (Pouwels P. H. et al., Ed., Elsevier, Amsterdam-New York-Oxford, 1985). Examples of expression vectors include, but are not limited to, plasmids, viral vectors (phages), e.g. SV40, CMV, baculovirus and adenovirus, transposons, IS elements, phasmids, cosmids, and linear or circular DNA. See, e.g., the book "Cloning Vectors" (Eds. Pouwels P. H. et al. Elsevier, Amsterdam-New York-Oxford, 1985, ISBN 0 444 904018). These vectors can be replicated autonomously in the (host) cell or can be replicated chromosomally. Expression vectors comprising at least one expression cassette of the present invention represent a further aspect of the invention.

For the expression of a POI in a cell according to the present invention, it is possible, e.g., to introduce a nucleic acid molecule which encodes the POI (e.g. an expression vector of the invention) into the cell. Alternatively, an existing gene of the cell can be modified so as to comprise selector codons at those amino acid positions where the POI is intended to carry ncAA residues. Methods for introducing (recombinant) polypeptide-encoding nucleic acid molecules into, or for modifying existing genes of, a cell are known in the art.

The term "expression" describes, in the context of the invention, the production of polypeptides encoded by the corresponding nucleic acid sequence in a cell. The term "expression" is also used for the production of tRNA molecules encoded by nucleic acid sequences in the cell.

The nucleic acid molecules of the invention, including the expression cassettes and expression vectors of the invention can be prepared using common cloning techniques known in the art. Common recombination and cloning techniques are used, as described for example in T. Maniatis, E.F. Fritsch and J. Sambrook, Molecular Cloning: A Laboratory Manual, Cold Spring Harbor Laboratory, Cold Spring Harbor, NY (1989) and in T.J. Silhavy, M.L. Berman and L.W. Enquist, Experiments with Gene Fusions, Cold Spring Harbor Laboratory, Cold Spring Harbor, NY (1984) and in Ausubel, F.M. et al., Current Protocols in Molecular Biology, Greene Publishing Assoc. and Wiley Interscience (1987).

The nucleic acid molecules, or combinations of nucleic acid molecules, of the invention, including expression cassettes and expression vectors of the invention, can be isolated, for example by methods known in the art.

An "isolated" nucleic acid molecule is separated from other nucleic acid molecules that are present in the natural source of the nucleic acid, and moreover can be essentially free of other cellular material or culture medium, when it is produced by recombinant techniques, or free of chemical precursors or other chemicals, when it is chemically synthesized.

A nucleic acid molecule according to the invention can be isolated by standard techniques of molecular biology and the sequence information provided according to the invention. For example, cDNA can be isolated from a suitable cDNA-bank, using one of the concretely disclosed complete sequences or a segment thereof as hybridization probe and standard hybridization techniques (as described for example in Sambrook, J., Fritsch, E.F. and Maniatis, T. Molecular Cloning: A Laboratory Manual. 2nd edition, Cold Spring Harbor Laboratory, Cold Spring Harbor Laboratory Press, Cold Spring Harbor, NY, 1989). Moreover, a nucleic acid molecule, comprising one of the disclosed sequences or a segment thereof, can be isolated by polymerase chain reaction, using the oligonucleotide primers that were constructed on the basis of this sequence. The nucleic acid thus amplified can be cloned into a suitable vector and can be characterized by DNA sequence analysis. The oligonucleotides according to the invention can moreover be produced by standard methods of synthesis, e.g. with an automatic DNA synthesizer.

### 5. ncAAs and post-translational POI modifications

The abbreviation "ncAA" refers generally to any non-canonical or non-natural amino acid, or amino acid residue, that is not among the 22 naturally occurring proteinogenic amino acids. Numerous ncAAs are well known in the art (see, e.g., Liu et al., Annu Rev Biochem 2010, 79:413-444; Lemke, ChemBioChem 2014, 15:1691-1694). The term "ncAA" also refers to amino acid derivatives, for example α-hydroxy acids (rather than α-amino acids). Such derivatives have been shown to be translationally incorporable as well. See, e.g., Ohta et al., 2008, ChemBioChem 9:2773-2778. Accordingly, the meaning of terms such as "aminoacylate" or "aminoacylation" used herein is not limited to the RS-catalyzed linkage of a tRNA and an α-amino acid but also includes the RS-catalyzed linkage of a tRNA and a ncAA derivative such as an α-hydroxy acid.

Particular preferred ncAAs for use in the present invention are those which can be posttranslationally further modified, for example using click chemistry reactions. Such click reactions include strain-promoted inverse-electron-demand Diels-Alder cycloadditions (SPIEDAC; see, e.g., Devaraj et al., Angew Chem Int Ed Engl 2009, 48:7013)) as well as cycloadditions between strained cycloalkynyl groups, or strained cycloalkynyl analog groups having one or more of the ring atoms not bound by the triple bond substituted by amino groups), with azides, nitrile oxides, nitrones and diazocarbonyl reagents (see, e.g., Sanders et al., J Am Chem Soc 2010, 133:949; Agard et al., J Am Chem Soc 2004, 126:15046), for example strain promoted alkyne-azide cycloadditions (SPAAC). Such click reactions allow for ultrafast and biorthogonal covalent site-specific coupling of ncAA-labeling groups of target polypeptides with suitable groups of coupling partner molecule. Pairs of docking and labeling groups which can react via the above-mentioned click reactions are known in the art. Examples of suitable ncAAs for use in the present invention comprising docking groups include, but are not limited to, the ncAAs ("unnatural amino acids", "UAAs") described, e.g., in WO 2012/104422 and WO 2015/107064. Optionally substituted strained alkynyl groups include, but are not limited to, optionally substituted trans-cyclooctenyl groups, such as those described in. Optionally substituted strained alkenyl groups include, but are not limited to, optionally substituted cyclooctynyl groups, such as those described in WO 2012/104422 and WO 2015/107064. Optionally substituted tetrazinyl groups include, but are not limited to, those described in WO 2012/104422 and WO 2015/107064.

The ncAAs used in the context of the present invention can be used in the form of their salt. Salts of an ncAA as described herein means acid or base addition salts, especially addition salts with physiologically tolerated acids or bases. Physiologically tolerated acid addition salts can be formed by treatment of the base form of an ncAA with appropriate organic or inorganic acids. ncAAs containing an acidic proton may be converted into their non-toxic metal or amine addition salt forms by treatment with appropriate organic and inorganic bases. Salts of carboxyl groups of ncAAs can be produced in a manner known in the art and comprise inorganic salts, for example sodium, calcium, ammonium, iron and zinc salts, and salts with organic bases, for example amines, such as triethanolamine, arginine, lysine, piperidine, etc. ncAAs may also be used in the form of salts of acid addition, for example salts with mineral acids, such as hydrochloric acid or sulfuric acid and salts with organic acids, such as acetic acid and oxalic acid. The ncAAs and salts thereof which are useful in the present invention also comprise the hydrates and solvent addition forms thereof, e.g. hydrates, alcoholates and the like.

Physiologically tolerated acids or bases are in particular those which are tolerated by the translation system used for preparation of POI with ncAA residues, e.g. are substantially non-toxic to living eukaryotic cells.

ncAAs, and salts thereof, useful in the context of the present the invention can be prepared by analogy to methods which are well known in the art and are described, e.g., in the various publications cited herein.

The nature of the coupling partner molecule depends on the intended use. For example, the target polypeptide may be coupled to a molecule suitable for imaging methods or may be functionalized by coupling to a bioactive molecule. For instance, in addition to the docking group, a coupling partner molecule may comprise a group selected from, but not limited to, dyes (e.g. fluorescent, luminescent, or phosphorescent dyes, such as dansyl, coumarin, fluorescein, acridine, rhodamine, silicon-rhodamine, BODIPY, or cyanine dyes), molecules able to emit fluorescence upon contact with a reagent, chromophores (e.g., phytochrome, phycobilin, bilirubin, etc.), radiolabels (e.g. radioactive forms of hydrogen, fluorine, carbon, phosphorous, sulphur, or iodine, such as tritium, ¹⁸F,¹¹C, ¹⁴C, ³²P, ³³P, ³³S, ³⁵S, ¹¹In, ¹²⁵I, ¹²³I, ¹³¹I, ²¹²B, ⁹⁰Y or ¹⁸⁶Rh), MRI-sensitive spin labels, affinity tags (e.g. biotin, His-tag, Flag-tag, strep-tag, sugars, lipids, sterols, PEG-linkers, benzylguanines, benzylcytosines, or cofactors), polyethylene glycol groups (e.g., a branched PEG, a linear PEG, PEGs of different molecular weights, etc.), photocrosslinkers (such as p-azidoiodoacetanilide), NMR probes, X-ray probes, pH probes, IR probes, resins, solid supports and bioactive compounds (e.g. synthetic drugs). Suitable bioactive compounds include, but are not limited to, cytotoxic compounds (*e.g*., cancer chemotherapeutic compounds), antiviral compounds, biological response modifiers (*e.g*., hormones, chemokines, cytokines, interleukins, etc.), microtubule affecting agents, hormone modulators, and steroidal compounds. Specific examples of useful coupling partner molecules include, but are not limited to, a member of a receptor/ligand pair; a member of an antibody/antigen pair; a member of a lectin/carbohydrate pair; a member of an enzyme/substrate pair; biotin/avidin; biotin/streptavidin and digoxin/antidigoxin.

The ability of certain (labeling groups of) ncAA residues to be coupled covalently *in situ* to (the docking groups of) conjugation partner molecules, in particular by a click reaction as described herein, can be used for detecting a target polypeptide having such ncAA residue(s) within a eukaryotic cell or tissue expressing the target polypeptide, and for studying the distribution and fate of the target polypeptides. Specifically, the method of the present invention for preparing a POI by expression in (e.g., eukaryotic) cells can be combined with super-resolution microscopy (SRM) to detect the POI within the cell or a tissue of such cells. Several SRM methods are known in the art and can be adapted so as to utilize click chemistry for detecting a target polypeptide expressed by a eukaryotic cell of the present invention. Specific examples of such SRM methods include DNA-PAINT (DNA point accumulation for imaging in nanoscale topography; described, e.g., by Jungmann et al., Nat Methods 11:313-318, 2014), dSTORM (direct stochastic optical reconstruction microscopy) and STED (stimulated emission depletion) microscopy.

### 6. Translational preparation of POIs in cells

The OT systems provided by the invention allow for the translational preparation of a POI in a cell.

The cell used for preparing a POI according to the invention can be a prokaryotic cell. Alternatively, the cell used for preparing a POI according to the invention can be a eukaryotic cell. The cell used for preparing a POI according to the invention can be a separate cell such as, e.g., a single-cell microorganism or a cell line derived from cells of multicellular organisms. Alternatively, the cell used for preparing a POI according to the invention can be present in (and part of) a tissue, an organ, a body part or an entire multicellular organism. Thus, the methods of the invention for preparing a POI can be performed with a separate cell or a cell culture, or with a tissue or tissue culture, organ, body part or (entire multicellular) organism.

Eukaryotic cells are often more difficult to handle and manipulate compared to prokaryotes such as, e.g., *E.coli,* and therefore not or only very difficult accessible to known approaches for POI-selective orthogonal translation such as those described in the "Background of the invention" section above. The OT system and the methods of the invention are therefore particular advantageous when use for POI expression in eukaryotic cells (including, e.g., single- and multicellular eukaryotic organisms, and eukaryotic cell lines).

In principle, all prokaryotic or eukaryotic cells can be used for preparing a POI according to a method of the present invention. Microorganisms such as, e.g., bacteria, fungi or yeasts can be used, as well as eukaryotic cells, such as, e.g., mammalian cells, insect cells, yeast cells and plant cells. Eukaryotic cells and in particular mammalian cells are particularly preferred.

The cell used for preparing a POI according to the invention carries a POI-encoding nucleotide sequence (CS^{POI}) wherein the ncAA residue(s) of the POI are encoded by selector codon(s). Said CS^{POI} is functionally linked with one or more targeting sequences (TNs). Translation yields an mRNA comprising the CS^{POI} and the TN(s). The cell further comprises one or more fusion proteins of the present invention, wherein said fusion protein(s) comprise at least one O-RS segment and at least one RNA-TP segment. Said O-RS and RNA-TP can be on separate fusion proteins (e.g. AFPs) of the invention. Alternatively, said O-RS and RNA-TP can be on one and the same fusion protein (e.g. on an RNA-TP/O-RS fusion protein or an AFP) of the invention. *Via* (at least one of) its TN(s) said mRNA can interact with (bind to) at least one of the RNA-TP segments of the fusion proteins of the invention in the cell. The cell further comprises one or more orthogonal tRNA^{ncAA} molecules (O-tRNA^{ncAA}) which carry the anticodon(s) to the selector codon(s) of the CS^{POI}. Said O-tRNA^{ncAA} molecules and one or more of the O-RS segments of the fusion proteins in the cell form one or more orthogonal O-RS/O-tRNA^{ncAA} pairs which allow for introducing the ncAA residue(s) into the amino acid sequence of the (translationally prepared) POI.

The interaction of the mRNA comprising CS^{POI} and TN(s) with the RNA-TP segment(s), the aminoacylation of the O-tRNA^{ncAA} with the ncAAs by the O-RS segment(s), and the translational preparation of the POI including the introduction of the ncAA residue(s) thought to take place in the cytoplasm, more particularly in the OT assembly (OT organelle), of the cell in the presence of the ncAAs.

The mRNA comprising CS^{POI} and TN(s) (mRNA^{POI}) can be generated from a recombinant construct (e.g. expression vector) introduced into the cell. Alternatively, one or more endogenous genes of the cell can be modified so as to comprise one or more selector codons and one or more TNs. Techniques for introducing recombinant constructs into a cell as well as methods for modifying endogenous genes of a cell are well known in the art.

The TRNA^{ncAA} molecules and fusion proteins of the invention can be generated from a recombinant construct (e.g. expression vector) introduced into the cell.

Using expression vectors according to the invention, recombinant cells can be produced which can be used for preparing a POI using a method of the present invention. Advantageously, the recombinant vectors according to the invention, described above, are introduced into a suitable cell and expressed.

The cell used for preparing a POI as described herein can be prepared by introducing nucleotide sequences encoding the fusion protein(s), the tRNA^{ncAA} molecule(s) and the POI into the cell. Said nucleotide sequences can be located on separate nucleic acid molecules (vectors) or on the same nucleic acid molecule (e.g., vector), in any combination, and can be introduced into the cell in combination or sequentially.

Preferably common cloning and transfection techniques, known by a person skilled in the art, are used, for example co-precipitation, protoplast fusion, electroporation, virus-mediated gene delivery, lipofection, microinjection or others, for introducing the stated nucleic acid molecules in the respective cell. Suitable techniques are described for example in Current Protocols in Molecular Biology, F. Ausubel et al., Ed., Wiley Interscience, New York 1997, or Sambrook et al. Molecular Cloning: A Laboratory Manual. 2nd edition, Cold Spring Harbor Laboratory, Cold Spring Harbor Laboratory Press, Cold Spring Harbor, NY, 1989.

For the methods of the present invention, the cell used for POI expression is grown or cultured in a manner known by a person skilled in the art. Depending on the type of cell, a liquid medium can be used for culturing. Culture can be batchwise, semi-batchwise or continuous. Nutrients can be present at the beginning of the culturing or can be supplied later, semi-continuously or continuously.

The expressed POIs can be purified by known techniques, such as, e.g., molecular sieve chromatography (gel filtration), such as Q-sepharose chromatography, ion exchange chromatography and hydrophobic chromatography, and other common protein purification techniques such as ultrafiltration, crystallization, salting-out, dialysis and native gel electrophoresis. Suitable methods are described, for example, in Cooper, T. G., Biochemische Arbeitsmethoden [Biochemical processes], Verlag Walter de Gruyter, Berlin, New York or in Scopes, R., Protein Purification, Springer Verlag, New York, Heidelberg, Berlin.

For isolating a POI, it can be advantageous to link the POI with a tag that can serve for easier purification. This can be achieved by introducing a corresponding tag-encoding sequence into the CS^{POI}. Suitable tags for protein purification are well known in the art and include, e.g., histidine tags (e.g., His₆ tag) and epitopes that can be recognized as antigens of antibodies (described for example in Harlow, E. and Lane, D., 1988, Antibodies: A Laboratory Manual. Cold Spring Harbor (N.Y.) Press). These tags can serve for attaching the proteins to a solid carrier, for example a polymer matrix, which can for example be used as packing in a chromatography column, or can be used on a microtiter plate or on some other carrier.

A tag linked to a POI can also serve for detecting the POI. Tags for protein detection are well known in the art and include, e.g., fluorescent dyes, enzyme markers, which form a detectable reaction product after reaction with a substrate, and others.

For preparing a POI according to a method of the present invention, the expression can be achieved by culturing the cell in the presence of one or more ncAAs corresponding to the ncAA residue(s) of the POI (wherein said ncAAs may expediently be comprised in the culture medium) for a time suitable to allow translation of the POI. Depending on the nucleic acid(s) encoding the POI (and optionally the fusion proteins of the invention and/or the tRNA^{ncAA} molecules), it may be required to induce expression by adding a compound inducing transcription, such as, e.g., arabinose, isopropyl *β*-D-thiogalactoside (IPTG) or tetracycline that allows transcription.

After translation, the POI may optionally be recovered from the translation system. For this purpose, the POI can be recovered and purified, either partially or substantially to homogeneity, according to procedures known to and used by those of skill in the art. Unless the target polypeptide is secreted into the culture medium, recovery usually requires cell disruption. Methods of cell disruption are well known in the art and include physical disruption, e.g., by (ultrasound) sonication, liquid-sheer disruption (e.g., via French press), mechanical methods (such as those utilizing blenders or grinders) or freeze-thaw cycling, as well as chemical lysis using agents which disrupt lipid-lipid, protein-protein and/or protein-lipid interactions (such as detergents), and combinations of physical disruption techniques and chemical lysis. Standard procedures for purifying polypeptides from cell lysates or culture media are also well known in the art and include, *e.g*., ammonium sulfate or ethanol precipitation, acid or base extraction, column chromatography, affinity column chromatography, anion or cation exchange chromatography, phosphocellulose chromatography, hydrophobic interaction chromatography, hydroxylapatite chromatography, lectin chromatography, gel electrophoresis and the like. Protein refolding steps can be used, as desired, in making correctly folded mature proteins. High performance liquid chromatography (HPLC), affinity chromatography or other suitable methods can be employed in final purification steps where high purity is desired. Antibodies made against the polypeptides of the invention can be used as purification reagents, *i.e*. for affinity-based purification of the polypeptides. A variety of purification/protein folding methods are well known in the art, including, *e.g*., those set forth in Scopes, Protein Purification, Springer, Berlin (1993); and Deutscher, Methods in Enzymology Vol. 182: Guide to Protein Purification, Academic Press (1990); and the references cited therein.

As noted, those of skill in the art will recognize that, after synthesis, expression and/or purification, polypeptides can possess a conformation different from the desired conformations of the relevant polypeptides. For example, polypeptides produced by prokaryotic systems often are optimized by exposure to chaotropic agents to achieve proper folding. During purification from, *e.g*., cell lysates, the expressed polypeptide is optionally denatured and then renatured. This is accomplished, *e.g.,* by solubilizing the proteins in a chaotropic agent such as guanidine HCl. In general, it is occasionally desirable to denature and reduce expressed polypeptides and then to cause the polypeptides to re-fold into the preferred conformation. For example, guanidine, urea, DTT, DTE, and/or a chaperonin can be added to a translation product of interest. Methods of reducing, denaturing and renaturing proteins are well known to those of skill in the art. Polypeptides can be refolded in a redox buffer containing, *e.g*., oxidized glutathione and L-arginine.

Also described are polypeptides produced by the methods of the invention. Such polypeptides can be prepared by a method of the invention that makes use of the OT system described herein.

### 7. Kits

The present invention also provides kits for preparing a POI having at least one non-canonical amino acid (ncAA) residue. The kit of the invention may comprise at least one expression vector for at least one fusion protein of the present invention. The fusion protein(s) encoded by the expression vector(s) in the kit may comprise at least one O-RS segment and at least one RNA-TP segment. The kit may further comprise at least one ncAA, or salt thereof, corresponding to the at least one ncAA residue of the POI. Expediently said O-RS segment is capable of aminoacylating a tRNA with the at least one ncAA. The kit may further comprise at least one expression vector for an orthogonal tRNA^{ncAA} (O-tRNA^{ncAA}) molecule. Further components of the kit may include at least one expression vector comprising a multiple cloning site and a targeting nucleotide sequence (TN), wherein an RNA molecule comprising said TN is able to interact via said TN with an RNA-targeting polypeptide (RNA-TP). Expediently said TN is a sequence, which, when present in an RNA molecule, is able to interact with an RNA-TP segment of at least one of the fusion protein(s) encoded by the expression vector(s) comprised by the kit. The kit may further comprise at least one reporter construct encoding an easily detectable (e.g. fluorescent) reporter polypeptide having at least one non-canonical amino acid (ncAA) residue such that the mRNA translated from said construct comprises a TN as described herein.

The kits of the present invention can be used in methods of the invention for preparing ncAA-residue containing POls as described herein.

### PARTICULAR EMBODIMENTS

The present invention further provides the following non-limiting embodiments E1 to E50.
E1: An assembler fusion protein (AFP) comprising:
   (a) at least one first polypeptide segment acting as assembler (AP) that is selected from:
      (a1) a polypeptide segment derived from an intracellular targeting polypeptide (IC-TP segment), wherein said intracellular targeting polypeptide targets, and thus becomes locally enriched at, an intracellular structural element within or directly adjacent to the cytoplasm; and
      (a2) a polypeptide segment derived from a phase separation polypeptide (PSP segment), wherein said phase separation polypeptide has the ability to undergo self-association in the cytoplasm of a cell so as to create sites of high local concentration in the cytoplasm, and
   (b) at least one second polypeptide segment acting as an effector (EP) that is selected from:
      b1) an RNA-targeting polypeptide (RNA-TP) segment, and
      b2) an orthogonal aminoacyl tRNA synthetase (O-RS) segment;
   wherein said polypeptide segments are functionally linked in said AFP.
E2: The AFP of E1 comprising at least two APs, preferably at least one IC-TP segment and at least one PSP segment.
E3: The AFP of E1 or E2 having one of the following structures (from the N-terminus to the C-terminus):
   [IC-TP]ₘ - [EP]ₒ
   [EP]ₒ - [IC-TP]ₘ
   [PSP]ₙ - [EP]ₒ
   [EP]ₒ - [PSP]ₙ
   [IC-TP]ₘ - [EP]ₒ - [PSP]ₙ
   [PSP]ₙ - [EP]ₒ - [IC-TP]ₘ
   [IC-TP]ₘ - [PSP]ₙ - [EP]ₒ
   [EP]ₒ - [PSP]ₙ - [IC-TP]ₘ
   [PSP]ₙ - [IC-TP]ₘ - [EP]ₒ
   [EP]ₒ - [IC-TP]ₘ - [PSP]ₙ
   wherein m, n and o, independently of each other, are integers selected from 1, 2, 3, 4 or 5, and "-" designates a peptidic linkage.
E4: The AFP of any one of E1-E3, wherein the at least one EP is selected from RNA-TP segments.
E5: The AFP of any one of E1-E3, wherein the at least one EP is selected from O-RS segments.
E6: The AFP of any one of E1-E3 comprising at least one EP selected from RNA-TP segments and at least one EP selected from O-RS segments.
E7: The AFP of any one of E1-E6 comprising at least one IC-TP segment selected from dyneins and kinesins, and fragments and mutants of dyneins and kinesins, which retain the ability to target, and become enriched at, the plus or the minus end of microtubules.
E8: The AFP of any one of E1-E6 comprising at least one IC-TP segment selected from transmembrane domains of membrane proteins, and functional fragments and mutants of transmembrane domains which retain the ability to target, and become enriched at, the cytoplasmic side of membranes, in particular membranes selected from the cell membrane, nuclear membrane and mitochondrial membrane.
E9: The AFP of any one of E1-E8 comprising at least one IC-TP segment selected from:
   - KIF16B₁₋₄₀₀ comprising the amino acid sequence of SEQ ID NO:20, or a functional fragment or mutant thereof having at least 60%, at least 70%, at least 80%, at least 90%, at least 95%, at least 96%, at least 97%, at least 98% or at least 99% amino acid sequence identity to the amino acid sequence of SEQ ID NO:20;
   - KlF13A_{1-411,Δ390} comprising the amino acid sequence of SEQ ID NO:22, or a functional fragment or mutant thereof having at least 60%, at least 70%, at least 80%, at least 90%, at least 95%, at least 96%, at least 97%, at least 98% or at least 99% amino acid sequence identity to the amino acid sequence of SEQ ID NO:22;
   - TOMM20₁₋₇₀ comprising the amino acid sequence of SEQ ID NO:24, or a functional fragment or mutant thereof having at least 60%, at least 70%, at least 80%, at least 90%, at least 95%, at least 96%, at least 97%, at least 98% or at least 99% amino acid sequence identity to the amino acid sequence of SEQ ID NO:24;
   - LcK comprising the amino acid sequence of SEQ ID NO:26, or a functional fragment or mutant thereof having at least 60%, at least 70%, at least 80%, at least 90%, at least 95%, at least 96%, at least 97%, at least 98% or at least 99% amino acid sequence identity to the amino acid sequence of SEQ ID NO:26;
   - FRB-CD28 comprising the amino acid sequence of SEQ ID NO:28, or a functional fragment or mutant thereof having at least 60%, at least 70%, at least 80%, at least 90%, at least 95%, at least 96%, at least 97%, at least 98% or at least 99% amino acid sequence identity to the amino acid sequence of SEQ ID NO:28;
   - FUS-CD28 comprising the amino acid sequence of SEQ ID NO:30, or a functional fragment or mutant thereof having at least 60%, at least 70%, at least 80%, at least 90%, at least 95%, at least 96%, at least 97%, at least 98% or at least 99% amino acid sequence identity to the amino acid sequence of SEQ ID NO:30;
   - EB1 comprising the amino acid sequence of SEQ ID NO:302, or a functional fragment or mutant thereof having at least 60%, at least 70%, at least 80%, at least 90%, at least 95%, at least 96%, at least 97%, at least 98% or at least 99% amino acid sequence identity to the amino acid sequence of SEQ ID NO:303
   - CG1 comprising the amino acid sequence of SEQ ID NO:304, or a functional fragment or mutant thereof having at least 60%, at least 70%, at least 80%, at least 90%, at least 95%, at least 96%, at least 97%, at least 98% or at least 99% amino acid sequence identity to the amino acid sequence of SEQ ID NO:304
   - EBAG9 comprising the amino acid sequence of SEQ ID NO:292 (full length) or a functional fragment or mutant thereof having at least 60%, at least 70%, at least 80%, at least 90%, at least 95%, at least 96%, at least 97%, at least 98% or at least 99% amino acid sequence identity to SEQ ID NO:292 ; or comprising the first 29 N-terminal amino acid residues of SEQ ID NO:294; or a functional fragment or mutant thereof having at least 60%, at least 70%, at least 80%, at least 90%, at least 95%, at least 96%, at least 97%, at least 98% or at least 99% amino acid sequence identity to SEQ ID NO:294
   - CMP Sia Tr, comprising the amino acid sequence of SEQ ID NO:296, or a functional fragment or mutant thereof having at least 60%, at least 70%, at least 80%, at least 90%, at least 95%, at least 96%, at least 97%, at least 98% or at least 99% amino acid sequence identity to the amino acid sequence of SEQ ID NO:296; and
   - P450 2C1 targeting the cytoplasmic side of the ER membrane or a functional fragment or mutant thereof having at least 60%, at least 70%, at least 80%, at least 90%, at least 95%, at least 96%, at least 97%, at least 98% or at least 99% amino acid sequence identity thereto, in particular a fragment comprising the N-terminal first 27 (SEQ ID NO:298); or first 29 (SEQ ID NO:300) amino acid residues; or a functional fragment or mutant thereof having at least 60%, at least 70%, at least 80%, at least 90%, at least 95%, at least 96%, at least 97%, at least 98% or at least 99% amino acid sequence identity to SEQ ID NO:298 or 300.
E10: The AFP of any one of E1-E9 comprising at least one PSP segment selected from intrinsically disordered proteins (IDPs), in particular prion-like domains, and functional fragments and mutants of IDPs, or prio-like domains, which retain the ability to undergo self-association in the cytoplasm of a cell so as to create sites of high local concentration in the cytoplasm.
E11: The AFP of any one of E1-E10 comprising at least one PSP segment selected from:
   - SPD5 comprising the amino acid sequence of SEQ ID NO:32, or a functional fragment or mutant thereof having at least 60%, at least 70%, at least 80%, at least 90%, at least 95%, at least 96%, at least 97%, at least 98% or at least 99% amino acid sequence identity to the amino acid sequence of SEQ ID NO:32;
   - FUS comprising the amino acid sequence of SEQ ID NO:34, or a functional fragment or mutant thereof having at least 60%, at least 70%, at least 80%, at least 90%, at least 95%, at least 96%, at least 97%, at least 98% or at least 99% amino acid sequence identity to the amino acid sequence of SEQ ID NO:34; and
   - EWSR1 comprising the amino acid sequence of SEQ ID NO:36, or a functional fragment or mutant thereof having at least 60%, at least 70%, at least 80%, at least 90%, at least 95%, at least 96%, at least 97%, at least 98% or at least 99% amino acid sequence identity to the amino acid sequence of SEQ ID NO:36.
E12: The AFP of any one of E1-E11 comprising at least one RNA-TP segment selected from RNA-binding segments of viral coat proteins, and functional fragments and mutants of RNA-binding segments of viral coat proteins which retain the ability to interact specifically with an RNA motif of the virus.
E13: The AFP of any one of E1-E12 comprising at least one RNA-TP segment selected from:
   - MCP comprising the amino acid sequence of SEQ ID NO:14, or a functional fragment or mutant thereof having at least 60%, at least 70%, at least 80%, at least 90%, at least 95%, at least 96%, at least 97%, at least 98% or at least 99% amino acid sequence identity to the amino acid sequence of SEQ ID NO:14;
   - λ_{N22} comprising the amino acid sequence of SEQ ID NO:16, or a functional fragment or mutant thereof having at least 60%, at least 70%, at least 80%, at least 90%, at least 95%, at least 96%, at least 97%, at least 98% or at least 99% amino acid sequence identity to the amino acid sequence of SEQ ID NO:16; and
   - PCP comprising the amino acid sequence of SEQ ID NO:306, or a functional fragment or mutant thereof having at least 60% at least 70%, at least 80%, at least 90%, at least 95%, at least 96%, at least 97%, at least 98% or at least 99% amino acid sequence identity to the amino acid sequence of SEQ ID NO:306.
E14: The AFP of any one of E1-E13 comprising at least one O-RS segment selected from:
   - *Methanococcus jannaschii* tyrosyl-tRNA synthetase;
   - *Escherichia coli* tyrosyl-tRNA synthetase;
   - *Escherichia coli* leucyl-tRNA synthetase;
   - *Methanosarcina mazei* pyrrolysyl-tRNA synthetase;
   - *Methanosarcina barkeri* pyrrolysyl-tRNA synthetase;
   - *Methanosarcina acetivorans* pyrrolysyl-tRNA synthetase;
   - *Methanosarcina thermophila* pyrrolysyl-tRNA synthetase;
   - *Methanococcoides burtonii* pyrrolysyl-tRNA synthetase;
   - *Desulfitobacterium hafniense* pyrrolysyl-tRNA synthetase; and
   and functional fragments and mutants thereof which retain aminoacyl-tRNA synthetase enzymatic activity.
E15: The AFP of any one of E1-E14 comprising at least one O-RS segment selected from:
   - PyIRS^{AF} comprising the amino acid sequence of SEQ ID NO:8, or a functional fragment or mutant thereof having at least 60%, at least 70%, at least 80%, at least 90%, at least 95%, at least 96%, at least 97%, at least 98% or at least 99% amino acid sequence identity to the amino acid sequence of SEQ ID NO:8;
   - PylRS^{AA} comprising the amino acid sequence of SEQ ID NO:10, or a functional fragment or mutant thereof having at least 60%, at least 70%, at least 80%, at least 90%, at least 95%, at least 96%, at least 97%, at least 98% or at least 99% amino acid sequence identity to the amino acid sequence of SEQ ID NO:10;
   - PylRS^{AAAF} comprising the amino acid sequence of SEQ ID NO:12, or a functional fragment or mutant thereof having at least 60%, at least 70%, at least 80%, at least 90%, at least 95%, at least 96%, at least 97%, at least 98% or at least 99% amino acid sequence identity to the amino acid sequence of SEQ ID NO:12;

   - IFRS1 comprising the amino acid sequence of SEQ ID NO:224, or a functional fragment or mutant thereof having at least 60%, at least 70%, at least 80%, at least 90%, at least 95%, at least 96%, at least 97%, at least 98% or at least 99% amino acid sequence identity to the amino acid sequence of SEQ ID NO:224;
   - CbzRS comprising the amino acid sequence of SEQ ID NO:226; or a functional fragment or mutant thereof having at least 60%, at least 70%, at least 80%, at least 90%, at least 95%, at least 96%, at least 97%, at least 98% or at least 99% amino acid sequence identity to the amino acid sequence of SEQ ID NO:226;
   - CpkRS comprising the amino acid sequence of SEQ ID NO:228 or a functional fragment or mutant thereof having at least 60%, at least 70%, at least 80%, at least 90%, at least 95%, at least 96%, at least 97%, at least 98% or at least 99% amino acid sequence identity to the amino acid sequence of SEQ ID NO:228; and
   - OMeRS, comprising the amino acid sequence of SEQ ID NO:236 or a functional fragment or mutant thereof having at least 60%, at least 70%, at least 80%, at least 90%, at least 95%, at least 96%, at least 97%, at least 98% or at least 99% amino acid sequence identity to the amino acid sequence of SEQ ID NO:236.
E16: An assembler fusion protein (AFP) combination comprising at least two AFPs of any one of E1-E15.
E17: The AFP combination of E16 comprising at least one first AFP comprising at least one RNA-TP segment, and at least one second AFP comprising at least one O-RS segment.
E18: A fusion protein (RNA-TP/O-RS fusion protein) comprising:
   (i) at least one RNA-targeting polypeptide (RNA-TP) segment; and
   (ii) at least one orthogonal aminoacyl tRNA synthetase (O-RS) segment,
   wherein said polypeptide segments are functionally linked in said RNA-TP/O-RS fusion protein.
E19: The RNA-TP/O-RS fusion protein of E18 having one of the following structures (from the N-terminus to the C-terminus):
   [RNA-TP]ₓ - [O-RS]_{y}
   [O-RS]_{y} - [RNA-TP]ₓ
   wherein x and y, independently of each other, are integers selected from 1, 2, 3, 4 and 5; and "-" designates a peptidic linkage.
E20: The RNA-TP/O-RS fusion protein of E18 or E19 comprising at least one RNA-TP segment selected from RNA-binding segments of viral coat proteins, and functional fragments and mutants of RNA-binding segments of viral coat proteins which retain the ability to interact specifically with an RNA motif of the virus.
E21: The RNA-TP/O-RS fusion protein of any one of E18-E20 comprising at least one RNA-TP segment selected from:
   - MCP comprising the amino acid sequence of SEQ ID NO:14, or a functional fragment or mutant thereof having at least 60%, at least 70%, at least 80%, at least 90%, at least 95%, at least 96%, at least 97%, at least 98% or at least 99% amino acid sequence identity to the amino acid sequence of SEQ ID NO:14;
   - λ_{N22} comprising the amino acid sequence of SEQ ID NO:16, or a functional fragment or mutant thereof having at least 60%, at least 70%, at least 80%, at least 90%, at least 95%, at least 96%, at least 97%, at least 98% or at least 99% amino acid sequence identity to the amino acid sequence of SEQ ID NO:16; and
   - PCP comprising the amino acid sequence of SEQ ID NO:306, or a functional fragment or mutant thereof having at least 60% at least 70%, at least 80%, at least 90%, at least 95%, at least 96%, at least 97%, at least 98% or at least 99% amino acid sequence identity to the amino acid sequence of SEQ ID NO:306.
E22: The RNA-TP/O-RS fusion protein of any one of E18-E21 comprising at least one O-RS segment selected from:
   - *Methanococcus jannaschii* tyrosyl-tRNA synthetase;
   - *Escherichia coli* tyrosyl-tRNA synthetase;
   - *Escherichia coli* leucyl-tRNA synthetase;
   - *Methanosarcina mazei* pyrrolysyl-tRNA synthetase;
   - *Methanosarcina barkeri* pyrrolysyl-tRNA synthetase;
   - *Methanosarcina acetivorans* pyrrolysyl-tRNA synthetase;
   - *Methanosarcina thermophila* pyrrolysyl-tRNA synthetase;
   - *Methanococcoides burtonii* pyrrolysyl-tRNA synthetase;
   - *Desulfitobacterium hafniense* pyrrolysyl-tRNA synthetase; and
   and functional fragments and mutants thereof which retain aminoacyl-tRNA synthetase enzymatic activity.
E23: The RNA-TP/O-RS fusion protein of any one of E18-E22 comprising at least one O-RS segment selected from:
   - PyIRS^{AF} comprising the amino acid sequence of SEQ ID NO:8, or a functional fragment or mutant thereof having at least 60% sequence identity to the amino acid sequence of SEQ ID NO:8;
   - PylRS^{AA} comprising the amino acid sequence of SEQ ID NO:10, or a functional fragment or mutant thereof having at least 60% sequence identity to the amino acid sequence of SEQ ID NO:10;
   - PylRS^{AAAF} comprising the amino acid sequence of SEQ ID NO:12, or a functional fragment or mutant thereof having at least 60% sequence identity to the amino acid sequence of SEQ ID NO:12;
   - IFRS1 comprising the amino acid sequence of SEQ ID NO:224, or a functional fragment or mutant thereof having at least 60%, at least 70%, at least 80%, at least 90%, at least 95%, at least 96%, at least 97%, at least 98% or at least 99% amino acid sequence identity to the amino acid sequence of SEQ ID NO:224;
   - CbzRS comprising the amino acid sequence of SEQ ID NO:226; or a functional fragment or mutant thereof having at least 60%, at least 70%, at least 80%, at least 90%, at least 95%, at least 96%, at least 97%, at least 98% or at least 99% amino acid sequence identity to the amino acid sequence of SEQ ID NO:226;
   - CpkRS comprising the amino acid sequence of SEQ ID NO:228 or a functional fragment or mutant thereof having at least 60%, at least 70%, at least 80%, at least 90%, at least 95%, at least 96%, at least 97%, at least 98% or at least 99% amino acid sequence identity to the amino acid sequence of SEQ ID NO:228;and
   - OMeRS, comprising the amino acid sequence of SEQ ID NO:236 or a functional fragment or mutant thereof having at least 60%, at least 70%, at least 80%, at least 90%, at least 95%, at least 96%, at least 97%, at least 98% or at least 99% amino acid sequence identity to the amino acid sequence of SEQ ID NO:236.
E24: A nucleic acid molecule, or a combination of two or more nucleic acid molecules, comprising:
   (i) a nucleotide sequence that encodes at least one AFP of any one of E1-E15, or at least one AFP combination of E16 or E17, or
   (ii) a nucleic acid sequence complementary to the nucleotide sequence of (i).
   (iii) both of (i) and (ii).
E25: A nucleic acid molecule, or a combination of two or more nucleic acid molecules, comprising:
   (i) a nucleotide sequence that encodes at least one RNA-TP/O-RS fusion protein of any one of E18-E23, or
   (ii) a nucleic acid sequence complementary to (i), or
   (iii) both of (i) and (ii).
E26: An expression cassette comprising the nucleotide sequence of the nucleic acid molecule, or the combination of nucleic acid molecules, of E24 or E25.
E27: An expression vector comprising at least one expression cassette of E26.
E28: A cell comprising at least one nucleic acid molecule, or combination of nucleic acid molecules, of E24 or E25, at least one expression cassette of E26, or at least one expression vector of E27.
E29: The cell of E28 which is a eukaryotic cell.
E30: The cell of E28 which is a mammalian cell.
E31: The cell of any one of E28-E30 comprising at least one nucleic acid molecule, or combination of nucleic acid molecules, of E24, or at least one expression cassette comprising the nucleotide sequence of said nucleic acid molecule, or combination of nucleic acid molecules, or at least one expression vector comprising said expression cassette.
E32: The cell of E31 comprising a nucleotide sequence that encodes, or is complementary to a nucleotide sequence encoding, at least one AFP of any one of E1-E3 and E7-E15 comprising at least one EP selected from RNA-TP segments and at least one EP selected from O-RS segments.
E33: The cell of E31 comprising a nucleotide sequence that encodes, or is complementary to a nucleotide sequence encoding, at least one AFP of any one of E1-E3 and E7-E15 comprising at least one EP selected from RNA-TP segments, and at least one AFP of any one of E1-E3 and E7-E15 comprising at least one EP selected O-RS segments.
E34: The cell of any one of E28-E30 comprising at least one nucleic acid molecule, or combination of nucleic acid molecules, of E25, or at least one expression cassette comprising the nucleotide sequence of said nucleic acid molecule, or combination of nucleic acid molecules, or at least one expression vector comprising said expression cassette.
E35: The cell of any one E28-E34, wherein the cell expresses the at least one AFP, the at least one AFP combination or the at least one RNA-TP/O-RS fusion protein, respectively, that is encoded by the nucleotide sequence of said nucleic acid molecule, or combination of nucleic acid molecules.
E36: A method for preparing a polypeptide of interest (POI) comprising in its amino acid sequence one or more non-canonical amino acid (ncAA) residues, wherein the method comprises expressing the POI in a cell of any one of E31-E33 in the presence of said one or more ncAAs, wherein the cell comprises:
   (i) a POI-encoding nucleotide sequence (CS^{POI}) wherein said one or more ncAA residues of the POI are encoded by selector codon(s),
   (ii) a targeting nucleotide sequence (TN) that is functionally linked to the CS^{POI} and is able to interact with an RNA-TP segment of at least one of the AFPs in the cell;
   (iii) one or more orthogonal tRNA^{ncAA} (O-tRNA^{ncAA}) molecules which carry the anticodon(s) complementary to the selector codon(s) of the CS^{POI}, and wherein said O-tRNA^{ncAA} molecules together with one or more O-RS segments of at least one of the AFPs in the cell form one or more orthogonal O-RS/O-tRNA^{ncAA} pairs which allow for the introduction of said one or more ncAA residues into the amino acid sequence of the POI;
   and wherein the method optionally further comprises recovering the expressed POI.
E37: A method for preparing a polypeptide of interest (POI) comprising in its amino acid sequence one or more non-canonical amino acid (ncAA) residues, wherein the method comprises expressing the POI in a cell of E35 in the presence of said one or more ncAAs, wherein the cell comprises:
   (i) a POI-encoding nucleotide sequence (CS^{POI}) wherein said one or more ncAA residues of the POI are encoded by selector codon(s),
   (ii) a targeting nucleotide sequence (TN) that is functionally linked to the CS^{POI} and is able to interact with an RNA-TP segment of at least one of the RNA-TP/O-RS fusion proteins in the cell;
   (iii) one or more orthogonal tRNA^{ncAA} (O-tRNA^{ncAA}) molecules which carry the anticodon(s) complementary to the selector codon(s) of the CS^{POI}, and wherein said O-tRNA^{ncAA} molecules together with one or more O-RS segments of the RNA-TP/O-RS fusion proteins in the cell form one or more orthogonal O-RS/O-tRNA^{ncAA} pairs which allow for the introduction of said one or more ncAA residues into the amino acid sequence of the POI;
   and wherein the method optionally further comprises recovering the expressed POI.
E38: A method for preparing a polypeptide of interest (POI) comprising in its amino acid sequence one or more non-canonical amino acid (ncAA) residues, said method comprising the steps of:
   (a) expressing in a cell one or more AFPs of any one of E1-E3 and E7-E15 comprising at least one RNA-TP segment and one or more AFPs of any one of E1-E3 and E7-E15 comprising at least one O-RS segment;
   (b) expressing in said cell one or more orthogonal tRNA^{ncAA} (O-tRNA^{ncAA}) molecules,
   wherein
   - said orthogonal tRNA^{ncAA} molecules and one or more of the O-RS segments of the AFPs in the cell form one or more orthogonal aminoacyl tRNA synthetase/tRNA^{ncAA} (O-RS/O-tRNA^{ncAA}) pairs,
   - said O-RS/O-tRNA^{ncAA} pairs allow for introducing said one or more ncAA residues into the amino acid sequence of said POI,
   wherein steps (a) and (b) can be concomitantly or sequentially in any order;
   (c) then, expressing said POI in said cell in the presence of said one or more ncAAs, wherein
      - the POI-encoding nucleotide sequence (CS^{POI}) comprises one or more selector codons encoding said one or more ncAA residues,
      - said selector codons match the anticodons of said one or more O-tRNA^{ncAA} molecules;
      - said CS^{POI} is functionally linked to a targeting nucleotide sequence (TN), thus forming a CS^{POI}/TN fusion sequence,
      - said CS^{POI}/TN fusion sequence is able to interact, via its TN, with an RNA-TP segment of at least one of the AFPs in the cell;
         and
   (d) optionally recovering the expressed POI.
E39: A method for preparing a polypeptide of interest (POI) comprising in its amino acid sequence one or more non-canonical amino acid (ncAA) residues, said method comprising the steps of:
   (a) expressing in a cell RNA-TP/O-RS fusion proteins of any one of E18-E23;
   (b) expressing in said cell one or more orthogonal tRNA^{ncAA} (O-tRNA^{ncAA}) molecules,
   wherein
   - said orthogonal tRNA^{ncAA} molecules and one or more of the O-RS segments of the RNA-TP/O-RS fusion proteins in the cell form one or more orthogonal aminoacyl tRNA synthetase/tRNA^{ncAA} (O-RS/O-tRNA^{ncAA}) pairs,
   - said O-RS/O-tRNA^{ncAA} pairs allow for introducing said one or more ncAA residues into the amino acid sequence of said POI,
   wherein steps (a) and (b) can be concomitantly or sequentially in any order;
   (c) then, expressing said POI in said cell in the presence of said one or more ncAAs, wherein
      - the POI-encoding nucleotide sequence (CS^{POI}) comprises one or more selector codons encoding said one or more ncAA residues,
      - said selector codons match the anticodons of said one or more O-tRNA^{ncAA} molecules;
      - said CS^{POI} is functionally linked to a targeting nucleotide sequence (TN), thus forming a CS^{POI}/TN fusion sequence,
      - said CS^{POI}/TN fusion sequence is able to interact, via its TN, with an RNA-TP segment of at least one of the RNA-TP/O-RS fusion proteins in the cell;
         and
   (d) optionally recovering the expressed POI.
E40: The method of any one of E36-E39, wherein the TN is selected from viral RNA motifs bound by a viral coat protein, and functional fragments and mutants thereof which retain the ability to be bound by a viral coat protein.
E41: The method of any one of E36-E40, wherein the TN is selected from:
   - MS2 RNA stem-loop comprising the RNA sequence encoded by the nucleotide sequence of SEQ ID NO:17, or a functional fragment or mutant thereof having at least 60%, at least 70%, at least 80%, at least 90%, at least 95%, at least 96%, at least 97%, at least 98% or at least 99% amino acid sequence identity to the amino acid sequence of SEQ ID NO:17;
   - BoxB comprising the RNA sequence encoded by the nucleotide sequence of SEQ ID NO:18, or a functional fragment or mutant thereof having at least 60%, at least 70%, at least 80%, at least 90%, at least 95%, at least 96%, at least 97%, at least 98% or at least 99% amino acid sequence identity to the amino acid sequence of SEQ ID NO:18, and
   - pp7 RNA stem-loop existing in at least two different versions and comprising the RNA sequence encoded by the nucleotide sequence of in particular a polynucleotide having an RNA sequence corresponding to (encoded by) the nucleotide (DNA) sequence of SEQ ID NO:289 or SEQ ID NO:290 or a functional fragment or mutant thereof having at least 60%, at least 70%, at least 80%, at least 90%, at least 95%, at least 96%, at least 97%, at least 98% or at least 99% amino acid sequence identity to the amino acid sequence of SEQ ID NO:289 or 290.
E42: The method of any one of E36-E41, wherein the selector codon(s) encoding the ncAA residue(s) of the POI are selected from Amber, Ochre and Opal stop codons.
E43: A nucleic acid molecule comprising:
   (i) a nucleotide sequence (CS^{POI}) that encodes a polypeptide of interest (POI), said POI comprising one or more non-canonical amino acid (ncAA) residues which are encoded in the CS^{POI} by selector codons, and
   (ii) a targeting nucleotide sequence (TN), wherein an RNA molecule comprising said TN is able to interact via said TN with an RNA-targeting polypeptide (RNA-TP).
E44: The nucleic acid molecule of E43, wherein the TN is selected from viral RNA motifs bound by a viral coat protein, and functional fragments and mutants thereof which retain the ability to be bound by a viral coat protein.
E45: The nucleic acid molecule of E43 or E44, wherein the TN is selected from:
   - MS2 RNA stem-loop comprising the RNA sequence encoded by the nucleotide sequence of SEQ ID NO:17, or a functional fragment or mutant thereof having at least 60%,%, at least 70%, at least 80%, at least 90%, at least 95%, at least 96%, at least 97%, at least 98% or at least 99% amino acid sequence identity to the amino acid sequence of SEQ ID NO:17;
   - BoxB comprising the RNA sequence encoded by the nucleotide sequence of SEQ ID NO:18, or a functional fragment or mutant thereof having at least 60%, %, at least 70%, at least 80%, at least 90%, at least 95%, at least 96%, at least 97%, at least 98% or at least 99% amino acid sequence identity to the amino acid sequence of SEQ ID NO:18; and
   - pp7 RNA stem-loop existing in at least two different versions and comprising the RNA sequence encoded by the nucleotide sequence of in particular a polynucleotide having an RNA sequence corresponding to (encoded by) the nucleotide (DNA) sequence of SEQ ID NO:289 or SEQ ID NO:290 or a functional fragment or mutant thereof having at least 60%, at least 70%, at least 80%, at least 90%, at least 95%, at least 96%, at least 97%, at least 98% or at least 99% amino acid sequence identity to the amino acid sequence of SEQ ID NO:289 or 290.
E46: The nucleic acid molecule of any one of E43-E45, wherein the selector codon(s) encoding the ncAA residue(s) of the POI are selected from Amber, Ochre and Opal stop codons.
E47: A kit for preparing a polypeptide of interest (POI) having at least one non-canonical amino acid (ncAA) residue, the kit comprising:
   - at least one ncAA, or salt thereof, corresponding to the at least one ncAA residue of the POI, and
   - at least one expression vector of E27.
E48: The kit of E47, wherein the expression vector encodes a fusion protein comprising at least one O-RS segment and at least one RNA-TP segment.
E49: The kit of E47 or E48 further comprising at least one expression vector for an orthogonal tRNA^{ncAA} (O-tRNA^{ncAA}) molecule.
E50: The kit of any one of E47-E49 further comprising at least one expression vector comprising a multiple cloning site and a targeting nucleotide sequence (TN), wherein an RNA molecule comprising said TN is able to interact via said TN with an RNA-targeting polypeptide (RNA-TP).
Anyone of the above embodiments also encompass the following modification: The above-mentioned AP (i.e. IC-TP and PSP) segments and /or EP (RNA-TP or O-RS) segments may be further combined with synthetic protein segments, which induce and control macromolecular interactions. One or more, like 2, 3, 4, 5, 6, 7, 8, 9,or 10, preferably one such protein segment may be operably fused into a single AFP of the invention. Of particular interest in the context of the invention are SYNZIPs having the ability to form heterodimeric coiled-coil protein structures. Such SYNZIPs are pairs of synthetic peptides capable of interacting with each other and are used to induce and control macromolecular interactions. Non-limiting examples are the pairs SYNZIP 1:2; SYNZIP 3:4 and SYNZIP 5:6. Particularly preferred according to the invention is the heterospecific coiled-coil pair SYNZIP2:SYNZIP1 as described by Reinke, A.W., Grant, R.A., Keating, A.E. (2010) J Am Chem Soc 132 6025-6031 (SYNZIP 1: SEQ ID NO:312, SYNZIP 2: SEQ ID NO:314, SYNZIP 3: SEQ ID NO:316; SYNZIP 4: SEQ ID NO:318, as well as functional fragments and mutants of these SYNZIP polypeptides. Said functional fragments and mutants may comprise at least 60%, at least 70%, at least 80%, at least 90%, at least 95%, at least 96%, at least 97%, at least 98% or at least 99% amino acid sequence identity to the amino acid of the polypeptide they are derived from.

The invention is further illustrated by the following non-limiting examples.

### EXAMPLES

### Methods

### (A) Cell culture, transfections and feeding with ncAAs

HEK293T cells (ATCC CRL-3216) and COS-7 cells (ATCC, CRL-1651) were maintained in Dulbecco's modified Eagle's medium (Life Technologies, 41965-039) supplemented with 1% penicillin-streptomycin (Sigma, 10,000 U/ml penicillin, 10 mg/ml streptomycin, 0.9% NaCl), 2 mM L-glutamine (Sigma), 1 mM sodium pyruvate (Life Technologies) and 10% FBS (Sigma). Cells were cultured at 37°C in a 5% CO₂ atmosphere and passaged every 2-3 days up to 15-20 passages.

In all cases, cells were seeded 15-20h prior to transfection at a density resulting in 70-80% confluency at the time of transfection. Flow cytometry was performed using 24-well plates with plastic bottom (Nunclon Delta Surface ThermoScientific). Immunofluorescence labeling and FISH were performed on 24-well plates with glass bottom (Greiner Bio-One) or four-well Lab-Tek #1.0 borosilicate coverglass (ThermoFisher).

Transfections of HEK293T cells were performed with polyethylenimine (PEI, Sigma-Aldrich) using 3 µg PEI per 1 µg DNA. COS-7 cells were transfected using the JetPrime reagent (PeqLab) according to the manufacturer's recommendations at a ratio of 1:2.

For Amber suppression system test, cells were transfected at a ratio of a 1:1:1:1 with POI^{TAG} vectors, tRNA^{Pyl}, synthetase and MCP or mock constructs. 4-6 hours after transfection the medium to a fresh containing ncAA.

Stock and working solutions for all of the used ncAAs were prepared as described in Nikic et al. (Nat Protoc 10(5):780-791, 2015). SCO (cyclooctyne lysine, SiChem SC-8000) was used at a final concentration of 250 µM. 3-lodophenylalanine (Chem-Impex International Inc.) was used at a final concentration of 1 mM. SCO is efficiently recognized by PyIRS^{AF} (Y306A, Y384F) (see Plass et al., Angew Chem 2011, 50:3878-3881). 3-lodophenylalanine is recognized by PylRS^{AA} (C346A, N348A) (see Wang et al., ACS Chem Biol 2013, 8:405-415).

### (B) Flow cytometry

HEK293T cells were harvested after one day after transfection, resuspended in 1x PBS and passed through 100 µm nylon mesh. Co-transfections for flow cytometry were performed at a 1:1:1:1 ratio with 1.2 µg total DNA with:
- a reporter plasmid encoding the POI (a stop codon encoding the amino acid position to be occupied by the ncAA),
- a plasmid encoding the tRNA^{Pyl} having the anticodon which matches (i.e., is the reverse complement) of the stop codon in the POI-encoding sequence (hereinafter simply referred to as tRNA^{Pyl}
- a plasmid encoding the PylRS or functional mutant thereof, respectively, and
- either a plasmid encoding an MCP fusion polypeptide or a mock plasmid.

Cell culture medium was exchanged for fresh medium containing the ncAA to be incorporated into the POI 4-6 h post-transfection and left until the time of harvesting.

Data acquisition and analysis were performed using an LSRFortessa SORP Cell Analyzer (Becton, Dickinson and Company) and the FlowJo software (FlowJo). Cells were first gated by cell type using forward scatter area (FSC-A) and side scatter area (SSC-A) parameters. Subsequently, single cells were identified based on SSC-A and side scatter width (SSC-W). Each shown FFC plot is the sum of three independent biological replicates from which the mean and the SEM were calculated. At least 130,000 single cells were analyzed per condition. GFP fluorescence was acquired in the 488-530/30 channel and mCherry fluorescence in the 561-610/20 channel.

### (C) PylRS immunostaining and imaging, fluorescence in situ hybridization (FISH)

For immune-labelling experiments, the cells were rinsed with 1x PBS, fixed in 2% paraformaldehyde in 1x PBS for 10 min at RT, rinsed with 1x PBS again and then permeabilized in 0.5% Triton X in 1x PBS for 15 min at RT. After rinsing the permeabilized cell samples twice with 1x PBS, said samples were incubated for 90 min in blocking solution (3% BSA in 1x PBS for 90 min at RT), and then with 1 µg/ml primary antibody (polyclonal rat anti-PyIRS, prepared as described in Nikic et al. (Angew Chem Int Ed Engl 2016, 55(52):16172-16176) and/or polyclonal rabbit anti-MCP (Merck, ABE76) and/or monoclonal rabbit anti-RPL26L1 antibody (EPR8478, Abcam, ab137046)) in blocking solution overnight at 4°C. The next day, the cell samples were rinsed with 1x PBS and incubated with 2 µg/ml secondary antibody (chicken anti-rat IgG(H+L) cross-adsorbed Alexa Fluor 594 conjugated antibody (Thermo Fisher Scientific, A-21471) and/or goat anti-rabbit IgG(H+L) cross-adsorbed Alexa Fluor 647 conjugated F(ab')₂ (Thermo Fisher Scientific, A-21246)) in blocking solution for 60 min at RT. DNA was stained with Hoechst 33342 (1 µg/ml in 1xPBS) for 10 min at RT. If only DNA was stained, the cells were fixed and permeabilized as described above and then stained with Hoechst 33342 (1 µg/ml in 1xPBS) for 10 min at RT. Finally, the cells were rinsed twice with 1x PBS.

FISH experiments were performed one day after transfection analogously to the FISH experiments described in Nikic et al. (Angew Chem Int Ed Engl 2016, 55(52):16172-16176). The hybridization protocol was adapted for 24-well plates from Pierce et al. (Methods Cell Biol 122:415-436, 2014).

For imaging of only tRNA^{Pyl}, the hybridization probe 5'-CTAACCCGGCTGAACGGATTTAGAGTCCATTCGATC-3' (labelled at the 5' terminus with Cy5; SEQ ID NO:1) was used at 0.25 µM. After four washes with SSC and one wash with TN buffer (0.1 M TrisHCl, 150 mM NaCl), cells were incubated for 1 h at RT with 3% BSA in TN buffer prior to standard immunofluorescence labeling as described above.

For imaging of both tRNA^{Pyl} and MS2 RNA stem-loop sequence, the hybridization probe for tRNA^{Pyl} (5'-CTAACCCGGCTGAACGGATTTAGAGTCCATTCGATC-3', labelled at the 5' terminus with digoxigenin; SEQ ID NO:2) was used at 0.16 µM, and the hybridization probe for the MS2 RNA stem-loop sequence (5'-CTGCAGACATGGGTGATCCTCATGTTTTCTA-3', labelled at the 5' terminus with Alexa Fluor 647; SEQ ID NO:3) was used at 0.75 µM. After four washes with SSC, the cells were incubated for 1h at RT in blocking buffer (0.1 M TrisHCl, 150 mM NaCl, 1x blocking reagent (Sigma 11096176001). Then, the cells were incubated with fluorescein conjugated sheep anti-digoxigenin Fab (Sigma 11207741910) at a 1:200 dilution in blocking buffer overnight at 4°C. The next day, 3 washes of 5 minutes were done in Tween buffer (0.1 M TrisHCl, 150 mM NaCl, 0.5% Tween20). DNA was stained with Hoechst 33342 (1 µg/ml in 1xPBS) for 10 min at RT.

Confocal images were acquired on a Leica SP8 STED 3X microscope equipped with a 63x/1.40 oil immersion objective using the following laser lines for excitation: 405 nm for Hoechst 33342, 488 nm for fluorescein and GFP, 548 nm for mOrange, 594 nm for Alexa Fluor 594, 647 nm for Alexa Fluor 647 and Cy5. Emission light was collected with HyD detectors at 420-500 nm and 605-680 nm respectively.

Ribosomal immunofluorescence images were taken on an Olympus Fluoroview FV3000 microscope equipped with a 60x/1.40 oil immersion objective using the following laser lines for excitation: 488 nm for GFP, 594 nm for Alexa Fluor 594, 640 nm for Alexa Fluor 647.

Images were processed using FIJI software.

### (D) Constructs, cloning and mutagenesis

Two different fluorescent protein reporters (dual color reporter) were cloned into a pBI-CMV1 vector (Clontech 631630), one protein in one multiple cloning site and the other reporter in the other multiple cloning site. The CDS for one of the reporters encoded an mRNA carrying two MS2 RNA stem-loops fused to the 3' untranslated region ("MS2-tag"), while the encoded mRNA of the other reporter was not MS2-tagged.

For examination of Amber suppression, The reporters GFP^{39TAG} and mCherry^{185TAG} were used as N-terminal fusion with NLS. For examination of Ochre and Opal suppression, analogous constructs were prepared (with GFP^{39TAA} and mCherry^{185TAA}, GFP^{39TGA} and mCherry^{185TGA}, respectively).

NLS::GFP^{39TAG}::MS2-tag reporter: NLS::GFP^{39TAG} was cloned with two copies of MS2 RNA stem-loops into the pBI-CMV1 vector as a reporter for successful Amber suppression in imaging experiments.

For examination of suppression of multiple Amber codons, pBI-CMV constructs for GFP^{39,149TAG} and GFP^{39,149,182TAG} were prepared which did not contain a second (e.g. mCherry) reporter in the second multiple cloning site.

Further non-limiting examples of GFPs which are applicable in the context of the invention are:

| | |
|---|---|
| GFP^{66TAG} | GFP with Amber site (SEQ ID NO:238) |
| GFP^{66TCG} | GFP with Serine site (SEQ ID NO:240) |
| GFP^{66CCG} | GFP with Proline site (SEQ ID NO:242) |
| GFP^{66CTA} | GFP with Leucine site (SEQ ID NO:244) |
| GFP^{66TTA} | GFP with Leucine site (SEQ ID NO:246) |
| GFP^{66ATA} | GFP with Isoleucine site (SEQ ID NO:248) |
| GFP^{66CGG} | GFP with Arginine site (SEQ ID NO:250) |
| GFP^{39TCG} | GFP with Serine site (SEQ ID NO:252) |
| GFP^{39CCG} | GFP with Proline site (SEQ ID NO:254) |
| GFP^{39CTA} | GFP with Leucine site (SEQ ID NO:256) |
| GFP^{39CGG} | GFP with Arginine site (SEQ ID NO:258) |
| GFP^{39TCG} | LCK-GFP with Serine site (SEQ ID NO:278) |
| GFP^{39CCG} | LCK-GFP with Proline site (SEQ ID NO:280) |
| GFP^{39CTA} | LCK-GFP with Leucine site (SEQ ID NO:282) |
| Extended GFP^{339TCG} | ID NO:284) GFP with Serine site at position 39 genetically fused to GFP^{66CCG} (SEQ |
| Extended GFP^{339CCG} | ID NO:286) GFP with Proline site at position 39 genetically fused toGFP^{66TCC} (SEQ |
| Extended GFP^{39CTA} | ID NO:288) GFP with Leucine site at position 39 genetically fused toGFP^{66TCG} (SEQ |

Further non-limiting examples of mCherrys which are applicable in the context of the invention are:

| | |
|---|---|
| mCherry^{72TAG} | mCherry with Amber site (SEQ ID NO:260) |
| mCherry^{72TCG} | mCherry with Serine site (SEQ ID NO:262) |
| mCherry^{72CCG} | mCherry with Proline site (SEQ ID NO:264) |
| mCherry^{72CTA} | mCherry with Leucine site (SEQ ID NO:266) |
| mCherry^{72TTA} | mCherry with Leucine site (SEQ ID NO:268) |
| mCherry^{72ATA} | mCherry with Isoleucine site (SEQ ID NO:270) |
| mCherry^{185TCG} | mCherry with Serine site (SEQ ID NO:272) |
| mCherry^{185CCG} | mCherry with Proline site (SEQ ID NO:274) |
| mCherry^{185CTA} | mCherry with Leucine site (SEQ ID NO:276) |

Further non-limiting examples of mCherry constructs comprising different TN loops which are applicable in the context of the invention are:
mCherry^{190TAG}-2xPP7 mCherry with amber site and 2x pp7 loops (SEQ ID NO:216)
mCherry^{190TAG}-4xPP7 mCherry with amber site and 4x pp7 loops (SEQ ID NO:218)
mCherry^{190TAG}-6xPP7 mCherry with amber site and 6x pp7 loops (SEQ ID NO:220)
H2B-mCherry^{190TAG}-2xMS2 Human Histone H2B type 1-J (*Uniprot: P06899*) fused to mCherry with amber site and 2x ms2-loops (SEQ ID NO:222)

They may be fused into the polypeptide chain of any of the AFP molecules described herein, in particular at a position within the fusion molecule which does not inhibit the function of anyone of the other polypeptide segments (APs and EPs) of the AFP molecule. Examples of such epitope-tag containing AFP molecules are given below.

Constructs for OT assemblies were prepared as follows: tRNA^{Pyl} was cloned under the control of a human U6 promoter, and all other constructs were under CMV promoters cloned in the pcDNA3.1 (Invitrogen V86020) vector. MCP protein was cloned from the addgene plasmid #31230 and FUS from the Addgene plasmid #26374. In all FUS fusions, amino acids 1-478(S108N) were used, replacing the C-terminal NLS region by a Flag-tag. In all RS fusions the previously reported efficient NES::PylRS^{AF} (Y306A, Y384F) sequence was used (see, e.g., Nikic et al., Angew Chem Int Ed Engl 2016, 55(52):16172-16176). The PyIRS mutant PylRS^{AA} (N346A, C348A) was cloned via site-directed mutagenesis starting from wildtype PylRS. The SPD5 gene was ordered from Genewiz and fused to MCP and PylRS^{AF} via restriction cloning. KIF13A₁₋₄₁₁ and KIF16B₁₋₄₀₀ were cloned from human cDNA and inserted into pcDNA3.1 via restriction cloning. P390 of KIF13A₁₋₄₁₁ was removed via side directed mutagenesis. KIF13A₁₋₄₁₁,ΔP390 and KIF16B_{- 400} fusions with MCP, PyIRS^{AF}, EWSR1::MCP, FUS::PylRS^{AF}, FUS::PylRS^{AA}, SPD5::MCP and SPD5::PylRS^{AF} were assembled via Gibson assembly (see Gibson et al., Nat Methods 2009, 6:343-345).

Constructs for differential imaging experiments: To selectively express Nup153-EGFP^{149TAG} and Vim^{116TAG}-mOrange, one gene was first inserted together with an MS2-tag into pBI-CMV1 (compare Nikic et al., Angew Chem Int Ed Engl 2016, 55(52):16172-16176).

Subsequently, the other gene was inserted without MS2-tag. INSR^{676TAG}::mOrange was fused to an MS2-tag by replacing Vim^{116TAG}-mOrange in the pBI vector bearing Nup153::EGFP^{149TAG} and Vim^{116TAG}::mOrange::MS2-tag to yield a bicistronic vector with INSR^{676TAG}::mOrange in one and Nup153::EGFP^{149TAG} in the other cassette.

Multicistronic Amber suppression vectors for COS-7 cell experiments: As COS-7 cells have lower transfection efficiency; we generated multicistronic vectors harboring the components of an OT assembly. To assemble multicistronic Amber suppression vectors, first one copy of tRNA^{Pyl} under the control of a human U6 promoter was inserted into the pBl-CMV1 vector via Gibson assembly. Subsequently, first the AFP CDS KIF16B::FUS::PylRS^{AF} and finally the AFP CDS KIF16B::EWSR1::MCP were inserted via Gibson assembly. Alternatively, a previously published pcDNA3.1 based construct (see Nikic et al., Angew Chem Int Ed Engl 2016, 55(52):16172-16176) expressing NES::PylRS^{AF} under a CMV promoter and tRNA^{Pyl} under a human U6 promoter was used. Constructs with U6-tRNA^{Pyl}, KIF16B::FUS::PylRS^{AF} and KIF16B::EWSR1::MCP, or NES::PylRS^{AF} were alternatively inserted into a pDonor vector (GeneCopoeia).

The respective sequence information on AFPs used in the following experiments can be taken from the listing of sequences given below.

### EXAMPLE 1 - RNA-TP/O-RS fusion and AFPs comprising a single AP

An OT assembly ("OT organelle", Fig. 1) was engineered having the following components:
i) An mRNA-targeting system in which two MS2 RNA stem-loops (MS2-tag) were fused to the mRNA of choice coding for the POI, creating an mRNA::ms2 fusion. The MS2-tag binds specifically to the MS2 bacteriophage coat protein (MCP) (see Bertrand et al., Mol Cell 1998, 2:437-445), which will thus form a stable and specific mRNA::ms2-MCP complex in cells. The MS2-tag was always fused to the 3' untranslated region (3' UTR) of the mRNA, which ensures translation to yield a scar-less final POI.
ii) A tRNA/RS suppressor pair. The orthogonal tRNA/RS pair from the *Methanosarcina mazei* pyrrolysyl system (tRNA^{Pyl}/PylRS) was chosen because it has enabled the encoding of more than 200 ncAAs with diverse functionalities into proteins using GCE in a multitude of cell types and species, including *E. coli,* mammalian cells and even living mice (see, e.g., Liu et al., Annu Rev Biochem 2010, 79:413-444; Lemke, ChemBioChem 2014, 15:1691-1694; Chin, Nature 2017, 550;53-60).
iii) The assembler (AP) was the key component required to form an OT assembly. The purpose of the assembler was to create membrane-less structures in the form of a dense phase, aggregate, droplet or condensate, in which the mRNA::ms2-MCP complex is brought into close proximity of the tRNA^{Pyl}/PylRS pair.

The simplest strategy tested was the bimolecular fusion of MCP::PyIRS (termed **B,** Fig. 2). In addition, strategies were tested which were expected to yield much larger assemblies. All of those assembly systems were composed of an assembler fusion to PyIRS co-expressed with an assembler fusion to MCP. Assembler::PyIRS-assembler::MCP were expected to form large aggregates (co-expression herein denoted with "•"). One tested assembly strategy was based on phase separation of proteins and one based on the assembly of kinesins, which are abbreviated herein as **P** and **K,** respectively (Fig. 2A). Furthermore, for each **P** and **K** approach two different molecular designs (**P1**, **P2** and **K1, K2,** respectively) were tested which are summarized as follows:
**P1.** Previous studies have established the capacity of the proteins fused-in sarcoma (FUS) and Ewing sarcoma breakpoint regions 1 (EWSR1) to form mixed droplet-like structures by phase separation. They both contain a prion-like disordered domain that facilitates phase separation into liquid, gel and solid states (see, e.g., Altmeyer et al., Nat Commun 2015, 6:8088; Patel et al., Cell 2015, 162:1066-1077). In a phase-separated state, these proteins are locally highly concentrated (several orders of magnitude) compared to the remaining soluble fraction in the cytoplasm. FUS was fused to PylRS and EWSR1 was fused to MCP. It was expected that this would lead to the formation of droplets in which MCP and PylRS are highly enriched. **P1** is denoted FUS::PylRS•EWSR1::MCP.
**P2.** The *Caenorhabditis elegans* protein spindle-defective protein 5 (SPD5) has been shown to phase separate into particularly large (several micron-sized) droplets (see Woodruff et al., Cell 2017, 169:1066-1077, e1010). In a phase-separated state, SPD5 is locally highly concentrated compared to the remaining soluble fraction in the cytoplasm (by several orders of magnitude). It was expected that a protein fused to SPD5 would condense into droplets. Similarly to FUS-EWSR1 droplets, PylRS fused to SPD5 and MCP fused to SPD5 were expected to be highly enriched. **P2** is denoted SPD5::PyIRS-SPD5::MCP.
**K1.** Certain kinesin truncations constitutively move towards microtubule-plus ends in living cells (Soppina et al., Proc Natl Acad Sci U.S.A. 2014, 111:5562-5567). One such truncated kinesin is KIF13A_{1-411,ΔP390}, and it was expected that PylRS and MCP, respectively, fused to this kinesin truncation and co-expressed would be locally enriched, due to spatial targeting to microtubule-plus ends. **K1** is denoted KIF13A_{1-411,ΔP390}::PylRS•KIF13A_{1-411,ΔP390}::MCP.
**K2.** By analogy to **K1,** the truncated kinesin KIF16B₁₋₄₀₀ was also tested. **K2** is denoted KIF16B₁₋₄₀₀::PylRS•KIF16B₁₋₄₀₀::MCP.

In order to evaluate these assemblers for facilitating functional orthogonal translation of an MS2-tagged mRNA, a dual-reporter construct was designed, in which GFP and mCherry mutants are simultaneously expressed from two different expression cassettes from one plasmid, ensuring that the mRNA ratio between them is constant across all experiments. Stop codons were introduced at permissive sites into GFP at position 39 (GFP^{39STOP}) and into mCherry at position 185 (mCherry^{185STOP}; Fig. 2B). Only if stop codon suppression is successful will the corresponding green or red fluorescent protein be produced. Transfected cells (tRNA^{Pyl} and ncAA were always present unless specifically noted otherwise) were analyzed by fluorescence flow cytometry (FFC); settings were adjusted so that an approximate diagonal results in the FFC plots if GFP and mCherry are expressed from this plasmid using the conventional cytoplasmic PyIRS system, which cannot differentiate mRNAs. A selective and functional OT organelle should selectively express mCherry only if the MS2-tag is fused to the 3' UTR of the mCherry mRNA, leading to the appearance of a vertical line in the cytometry plot (Fig. 2B). Unless otherwise reported, all experiments where performed in the presence of tRNA^{Pyl} and the ncAA SCO, a widely used and well characterized lysine derivative, the side chain of which carries a cyclooctyne that can be used in a variety of click-chemistry reactions to install diverse chemical groups onto the protein. As previously reported, this ncAA is efficiently encoded by a Y306A, Y384F double mutant of PyIRS (for simplicity this mutant is designated PylRS herein, unless otherwise specified) (see Nikic et al., Angew Chem 2014, 53:2245-2249; Plass, Angew Chem 2012, 51:4166-4170; Plass et al., Angew Chem 2011, 50:3878-3881). Omission of the ncAA served as a standard negative control and lead to no expression of GFP or mCherry.

The performance of each OT system was evaluated according to its selectivity and relative efficiency. Selectivity is defined as the ratio r of the mean mCherry FFC signal divided by the mean GFP signal. Final values are expressed as fold selectivity relative to that of cytoplasmic PylRS. Relative efficiency is defined as the mean mCherry signal of each system divided by the mean mCherry signal of the cytoplasmic PyIRS system, which serves as the reference (here defined as 100%). All results on selectivity (dark-gray positive bars) and efficiency (light-gray negative bars) are summarized in the bar plot in Figure 2C. Selected FFC data is also shown in Figure 2D.

The simplest strategy **B** (MCP fused to PylRS) showed an about 1.5-fold selectivity gain (Fig. 2C). The OT system **P1** (based on phase separation of FUS/EWSR1) had a somewhat lower selectivity gain (Fig. 2C, D). The **P2** system (based on SPD5) showed an approximate twofold selectivity gain (Fig. 2C). For **K1** a twofold increase in selectivity was observed (Fig. 2C). The **K2** system behaved similarly (Fig. 2C,D). In total, the selectivity gains were relatively small, but robustly detected and distinguishable from a simple efficiency drop. The observed selectivity effect (data not shown) was robust across a titration of Amber suppression efficiencies (specifically, 0.48 ng, 2.4 ng, 12 ng, 60 ng or 300 ng tRNA^{Pyl} construct, respectively, were used), indicating that bringing the ncAA aminoacylation activity (i.e. the tRNAPyl/PyIRS in the presence of ncAA) in direct proximity of the target mRNA represents a pathway to more selective codon suppression.

### EXAMPLE 2 - AFPs comprising a combination of two APs

AFPs comprising combinations of the APs described in example 1 were tested in an analogous manner, those were:
**K1::P1** = KIF13A_{1-411,ΔP390}::FUS::PylRS•KIF13A_{1-411,ΔP390}::EWSR1::MCP,
**K2::P1** = KIF16B₁₋₄₀₀::FUS::PylRS•KIF16B₁₋₄₀₀::EWSR1::MCP,
**K1::P2** = KIF13A_{1-411,ΔP390}::SPD5::PylRS•KIF13A_{1-411,ΔP300}::SPD5::MCP,
**K2::P2** = KIF16B₁₋₄₀₀::SPD5::PylRS•KIF16B₁₋₄₀₀::SPD5::MCP.

For all combinations an at least fivefold selectivity gain was observed indicating orthogonal translation. The best performing of these systems was based on the fusion of FUS/EWSR1 with KIF16B₁₋₄₀₀, **K2::P1** and exhibited a selectivity of eightfold (box in Fig. 2C). This was also directly obvious from the FFC data, in which the bright, mCherry-positive cell population was clearly retained, whereas GFP expression was minimal (arrow in Fig. 2D).

### EXAMPLE 3 - AFPs comprising a combination of APs including a membrane-targeting AP

AFPs comprising combinations of APs derived from phase separation polypeptides (PSPs), FUS and EWSR1 (also termed EWS herein), optionally fused to SYNZIP segments, and different APs which acts as a membrane-targeting signal, LcK, EB1, CG1, EBAG9 _{full length}, EBAG9₁₋₂₉, CMP Sia Tr, P450 2C1₁₋₂₇ and P450 2C1₁₋₂₉ were tested in a manner analogous to example 2.

LcK is a cell membrane-targeting signal (Resh, Bba-Mol Cell Res 1999, 1451:1-16) that adds an amphipathic helix post translationally to the POI. For these experiments, the AFPs LcK::FUS::PyIRS and LcK::EWSR1::MCP were co-expressed in HE293T cells (see Fig. 3 and 6C). Testing of this system with the same dual reporter resulted in a dramatic shift in the signal and a strong selectivity for the expression solely of the MS2-tagged mCherry compared to the control PylRS. See Fig. 4 and Fig. 5 showing a 26-fold selectivity gain as compared to the control. IF and FISH for MCP, PylRS and tRNA show a clear membrane signal with appearance of occasional droplet-like structures and a perfect co-localization of all the components.

Without wishing to be bound by theory, it is assumed that targeting the OT system to a membrane results in a confinement of the components to a 2D surface (i.e. a film), offering an even higher spatial segregation than a cytoplasmic droplet. In accordance with such a cumulative effect of the two combined assembler strategies (LcK for membrane targeting, and FUS/EWSR1 for droplet generation), it was shown that the presence of the FUS/EWSR1 "assemblers" is not a requirement in an LcK-fused (and thus membrane-anchored system) for obtaining selective Amber suppression (data not shown). Nevertheless, the combination of the LcK-targeting with FUS/EWSR1 resulted in a higher selectivity of the system. Further, it was found that swapping the MS2-tag on the fluorescent reporters, yielded a swapped selectivity in the FFC data, underlining the selective (orthogonal) translation of the MS2-tagged mRNA.

For a further LcK based experiment, the AFP constructs LcK::FUS::SYNZIP1::PyIRS and EWSR1::SYNZIP2::MCP; were co-expressed in HE293T cells (see Fig. 8A). Testing of this system with the same dual reporter resulted in a dramatic shift in the signal and a strong selectivity for the expression solely of the MS2-tagged mCherry. Upon expression SYNZIP1 and 2 pair and recruit MCP to a plasma membrane based OT organelle. In a comparative approach co-expressing the AFP constructs LcK::FUS::PyIRS and EWSR1::SYNZIP2::MCP, wherein SYNZIP1 is missing, no selectivity of translation could be observed (see Fig. 8B)

EB1 is a microtubule plus ends-targeting signal ((Nehlig A, Molina A, Rodrigues-Ferreira S, Honoré S, Nahmias C. Regulation of end-binding protein EB1 in the control of microtubule dynamics. Cell Mol Life Sci. 2017;74(13):2381-2393. doi:10.1007/s00018-017-2476-2). For these experiments, the AFP construct EB1::PyIRS with EB1::MCP, EB1:FUS::PyIRS with EB1::EWSR1::MCP or EB1::FUS::MCP::PyIRS were expressed in HE293T cells. Testing of this system with the same dual reporter resulted in a shift in the signal and a strong selectivity for the expression solely of the MS2-tagged mCherry compared to the control PylRS. See Fig.6B.

CG1 is a nuclear membrane-targeting signal (Kim SJ, Fernandez-Martinez J, Nudelman I, et al. Integrative structure and functional anatomy of a nuclear pore complex. Nature. 2018;555(7697):475-482. doi:10.1038/nature26003) For these experiments, the AFP constructs CG1::FUS::PyIRS and CG1::EWSR1::MCP were co-expressed in HE293T cells. Testing of this system with the same dual reporter resulted in a shift in the signal and a strong selectivity for the expression solely of the MS2-tagged mCherry compared to the control PylRS. See Fig. 6E.

EBAG9 _{full length} and EBAG9₁₋₂₉ are Golgi membrane-targeting signals (Engelsberg A, Hermosilla R, Karsten U, Schülein R, Dörken B, Rehm A. The Golgi protein RCAS1 controls cell surface expression of tumor-associated O-linked glycan antigens. J Biol Chem. 2003;278(25):22998-23007. doi:10.1074/jbc.M301361200). For these experiments, the AFPconstructs EBAG9₁₋₂₉::FUS::PylRS and EBAG9₁₋₂₉::EWSR1::MCP were co-expressed in HE293T cells. Testing of this system with the same dual reporter resulted in a shift in the signal and a strong selectivity for the expression solely of the MS2-tagged mCherry compared to the control PylRS. See Fig. 6F (left side).

CMP Sia Tr is a Golgi membrane-targeting signal (Eckhardt M, Gotza B, Gerardy-Schahn R. Membrane topology of the mammalian CMP-sialic acid transporter. J Biol Chem. 1999;274(13):8779-8787. doi:10.1074/jbc.274.13.8779). For these experiments, the AFPconstructs CMP Sia Tr::FUS::PyIRS and CMP Sia Tr::MCP were co-expressed in HE293T cells. Testing of this system with the same dual reporter resulted in a shift in the signal and a strong selectivity for the expression solely of the MS2-tagged mCherry compared to the control PylRS. See Fig. 6F (right side).

P450 2C1₁₋₂₇ is an ER membrane-targeting signal (Fazal FM, Han S, Parker KR, et al. Atlas of Subcellular RNA Localization Revealed by APEX-Seq. Cell. 2019;178(2):473-490.e26. doi:10.1016/j.cell.2019.05.027). For these experiments, the AFP constructs P450 2C1₁₋₂₇::FUS::PyIRS and P450 2C1₁₋₂₇::EWSR1::MCP or P450 2C1₁₋₂₉::FUS::MCP::PylRS were co-expressed in HE293T cells. Testing of this system with the same dual reporter resulted in a shift in the signal and a strong selectivity for the expression solely of the MS2-tagged mCherry compared to the control PylRS. See Fig. 6G.

### EXAMPLE 4 - Validation of the selectivity gain being specific to the interaction of the mRNA MS2-tag and MCP

To validate that the observed selectivity gain was specific to the interaction of the MCP segment with the MS2-tag of the mRNA, all the OT systems were characterized by expressing the RS assembler fusion of each OT system without MCP. As expected, no selective orthogonal translation of MS2-tagged mRNA was observed in those cases (See Figure 6 A to G). Additionally, a reporter inversion was performed by moving the MS2-tag from the mCherry to the GFP cassette in the dual-color reporter, which as expected inverted selectivity of the system towards dominant GFP expression (data not shown). This established that the OT systems acted selectively on the MS2-tagged RNA.

### EXAMPLE 5 - Introduction of multiple ncAAs into the same POI

GCE can also be used to introduce multiple ncAAs into the same POI (see, e.g., Liu et al., Annu Rev Biochem 2010, 79:413-444; Lemke, ChemBioChem 2014, 15:1691-1694; Chin, Nature 2017, 550;53-60). However, only very few publications report on more than one, that is, two- or three-codon suppression in the same protein in eukaryotes, as yields typically suffer compared to single-codon suppression (see Xiao et al., Angew Chem 2013, 52:14080-14083; Schmied et al., J Am Chem Soc 2014, 136:15577-15583; Zhang et al., Biochem Biophys Res Co 2017, 489:490-496). Notably, even dual- and triple-Amber proteins were still suppressed with the OT organelle (data not shown).

### EXAMPLE 6 - OT with 3-iodophenylalanine

To ensure that also other ncAAs can be translated by the OT assembly, another structurally different ncAA (3-iodophenylalanine) was tested which is a phenylalanine derivative instead of a lysine derivative (such as SCO) and is encoded by a different PyIRS mutant (N346A, C348A) (see Wang et al., ACS Chem Biol 2013, 8:405-415). Consistent results were also observed for this system (Fig. 2C).

### EXAMPLE 7 - OT with different selector codons

As Opal and Ochre codons are highly abundant in eukaryotic genomes (52% Opal, 28% Ochre in the human genome), the Amber codon is by far the most used for GCE in eukaryotes. In addition, genomic approaches to orthogonal translation by removing those codons in the entire eukaryotic genome would be even more challenging then for the Amber codon and are currently beyond the state of the art. However, in the OT systems of the present invention, a simple mutation in the anticodon loop of the tRNA^{Pyl}, as well as in the respective codon in the MS2-tagged POI-encoding mRNA allows for orthogonal translation of those codons. FFC analysis revealed that the OT systems of the invention provide freedom of choice with respect to the stop (selector) codon (Fig. 2C, E). In fact, Opal suppression turned out to be the best performing system, showing an 11-fold selectivity increase. Ochre suppression still showed fivefold selectivity increase with 20% efficiency.

### EXAMPLE 8 - Orthogonal translation of proteins of various cellular compartments

To visualize the power of the OT^{K2::P1} system (the best performing Amber suppression OT system in terms of selectivity and efficiency) beyond "simple" reporters, it was intended to show differential expression of human nucleoporin 153 (Nup153) versus cytoskeletal vimentin. Nup153 locates to the nuclear pore complex and is more than 1500 amino acids long. Hence, its mRNA is approximately six-fold larger than those of the fluorescent protein reporters used above. For this experiment a previously described C-terminal GFP fusion, with an Amber mutation (Nup153::EGFP^{149TAG}) was used that gave rise to a characteristic nuclear envelope stain in confocal images only if Amber suppression was successful (see Nikic et al., Angew Chem 2016, 55:16172-16276). Said Nup153::EGFP^{149TAG} was now tagged at the mRNA level with an MS2-tag (nup153::egfp^{149TAG}::ms2) and co-expressed from the same plasmid with vimentin (a cytoskeletal protein) containing an Amber codon at position 116 fused to mOrange (Vim^{116TAG}::mOrange). Expression in HEK293T cells resulted in production of both proteins in the presence of the cytoplasmic PyIRS showing the characteristic nuclear envelope and cytoskeletal staining, respectively. Using the OT^{K2::P1} assembly only Nup153::GFP was visible (selective nuclear rim stain in confocal images of the co-transfected HEK293T cells). Consistent results were also observed in COS-7 cells. Swapping the MS-tag to vimentin inverted the effect, so that only Vim^{116TAG}::mOrange was visible (observed for both COS-7 and HEK293T cell experiments). This showed that the OT^{K2::P1} worked for dramatically different mRNAs.

### EXAMPLE 9 - Orthogonal translation of transmembrane proteins

It was also shown that transmembrane proteins can be selectively expressed using the OT^{K2::P1} assembly. Membrane protein expression represents another layer of translational complexity, as ribosomes need to bind the endoplasmic reticulum during translation, where the proteins are co-translationally inserted into the membrane. In this experiment, a fusion of insulin receptor 1 with an Amber codon at position 676 with mOrange (INSR^{676TAG}::mOrange) was used, which locates to the plasma membrane and gives rise to a characteristic plasma membrane stain in HEK293T cells (see Nikic et al., Angew Chem 2014, 53:2245-2249). This construct was tagged with an MS2-tag in the 3' UTR and cloned with Nup153::EGFP^{149TAG} into one dual-cassette plasmid. Then the construct was expressed in HEK293T cells either in the presence of the cytoplasmic PyIRS system or in the presence of the OT^{K2::P1} assembly. In the presence of the OT^{K2::P1} assembly, selective expression of the MS2-tagged protein and the expected plasma membrane localization of INSR^{676TAG}::mOrange were observed (data not shown), indicating the potential of the OT system of the present invention to participate in even more complex membrane-associated translational processes.

### EXAMPLE 10 - Spatial distribution of elements of the OT system in the cell

The spatial distribution of AFPs and particularly PylRS in cells was assessed using immunofluorescence (IF). Additionally, fluorescence *in situ* hybridization (FISH) was used for detecting tRNA^{Pyl}. In contrast to the dual color reporter used in the FFC experiments above, in all IF/FISH experiments a single color NLS-GFP^{39TAG} reporter that was fused to an MS2-tag (nis-gfp^{39TAG}::ms2) was used to identify cells active in Amber suppression (this yields a green nucleus if Amber suppression is successful and helped to optimize distinguishable color channels). IF and FISH stainings showed that in contrast to cytoplasmic PylRS, the **P1** system formed small, intracellular assembler::PyIRS droplets (data not shown). This indicated the occurrence of phase separation. The tRNA^{Pyl} co-localized well with highly dispersed assembler::PylRS droplets, indicating that it could nicely partition into the assembler::PyIRS phase. Additional stainings showed further co-localization with assembler::MCP (data hot shown). Compared to **P1,** the **P2** system showed larger but still multiple dispersed droplet-like structures (data not shown). With the combination of both assembler strategies (**K1::P1, K2::P1, K1::P2, K2::P2**) the formation of large micron-sized organelle-like structures in the cytoplasm was observed, these structures were in most cases localized to few or even a single position per cell. For the combined assemblers, mRNA::ms2, tRNA^{Pyl}, assembler::PyIRS and assembler::MCP all co-localized to organelle-like structures. The combination of the two assembler strategies, that is, phase separation paired with spatial targeting by kinesin truncations, yielded the best confinement as determined by FISH and IF and the highest selectivity increase. This is consistent with the hypothesis that the higher spatial segregation and thus higher local concentration of the tRNA^{Pyl}, PylRS and mRNA correlates with higher selectivity.

Ribosomes were stained to see whether they co-localize to the OT^{K2::P1} assembly. IF staining of the ribosomal protein RPL26L1 revealed strong co-localization with the OT^{K2::P1} organelle (data not shown) demonstrating ribosome recruitment, tentatively due to binding to mRNA::ms2 during translation. High ribosomal mobility can also explain why it was possible to successfully express the membrane protein INSR (construct: INSR^{676TAG}::mOrange::ms2).

Without wishing to be bound by theory, the experimental results strongly suggests that selective orthogonal translation happens within close proximity of, potentially even inside, the OT assemblies, by a set of recruited ribosomes that are near or fully immersed into a concentrated pool of tRNA^{Pyl}. tRNA^{Pyl} itself is recruited to the OT^{K2::P1} assembly due to its affinity for assembler::PylRS and can readily co-partition into the droplet to be aminoacylated with its cognate ncAA, while assembler::MCP recruits MS2-tagged mRNA. This in turn attracts ribosomes to co-partition into the dense phase formed by the dual assembler system (K2::P1 = KIF16B::FUS::PylRS and KIF16B::EWSR1::MCP), which maintains access to other translation factors for translation to function. Ribosomes elsewhere in the cytoplasm that are not exposed to tRNA^{Pyl} perform their canonical function to terminate translation whenever they encounter a stop codon.

### EXAMPLE 11 - Further OT systems

In addition to the OT systems described in the preceding examples, a variety of other OT systems were tested and found to allow for selective orthogonal translation of the reporter (i.e. the POI). A summary of these experiments is shown in the Table 1 below. Unless noted otherwise, the cytoplasmic NES-PyIRS system as previously described by Nikic et al. (Angew Chem Int Ed Engl 2016, 55(52):16172-16176) but with the corresponding AF, AA or AAAF mutations was used as a nonspecific reference (negative control). All experiments were performed in presence of the codon-specific tRNA^{Pyl} and PylRS mutant corresponding ncAAs.

**Table 1: Tested OT systems**

| **Fusion protein(s) comprising O-RS and RNA-TP segments** | **Reporter (POI)** | **cell lines** |
|---|---|---|
| EWSR1-MCP•FUS-PylRS^{AF} | GFP^{39TAG}-2xMS2•mCherry^{185TAG} | HEK293T/C |
| | mCherry^{185TAG}-2xMS2•GFP^{39TAG} | OS-7 |
| FUS-MCP•FUS-PylRS^{AF} | mCherry^{185TAG}-2xMS2•GFP^{39TAG} | HEK293T |
| FUS-MCP-PylRS^{AF} | GFP^{39TAG}-2xMS2•mCherry^{185TAG} | HEK293T/C |
| | mCherry^{185TAG}-2xMS2•GFP^{39TAG} | OS-7 |
| MCP-PylRS^{AF} | GFP^{39TAlG}-2xMS2•mCherry^{185TAG} | HEK293T/C |
| | mCherry^{185TAG}-2xMS2•GFP^{39TAG} | OS-7 |
| SPD5-MCP•SPD5-PylRS^{AF} | GFP^{39TAlG}-2xMS2•mCherry^{185TAG} | HEK293T |
| | mCherry^{185TAG}-2xMS2•GFP^{39TAG} | |
| SPD5-MCP-PylRS^{AF} | GFP^{39TAG}-2xMS2•mCherry^{185TAG} | HEK293T |
| | mCherry^{185TAG}-2xMS2•GFP^{39TAG} | |
| KIF16B-PylRS^{AF}•KIF16B-MCP | GFP^{39TAG}-2xMS2•mCherry^{185TAG} | HEK293T/C |
| | mCherry^{185TAG}-2xMS2•GFP^{39TAG} | OS-7 |
| KIF16B-MCP-PylRS^{AF} | GFP^{39TAG}-2xMS2•mCherry^{185TAG} | HEK293T/C |
| | mCherry^{185TAG}-2xMS2•GFP^{39TAG} | OS-7 |
| KIF16B-FUS-PylRS^{AF}•KIF16B-EWSR1- | GFP^{39TAG}-2xMS2•mCherry^{185TAG} | HEK293T/C |
| MCP | mCherry^{185TAG}-2xMS2•GFP^{39TAC} | OS-7 |
| | mCherry^{185TAA}-2xMS2•GFP^{39TAA} | |
| | mCherry^{185TGA}-2xMS2•GFP^{39TGA} | |
| | Nup153-EGFP^{149TAG}-2xMS2•Vimentin^{116TAG}-mOrange | |
| | Vimentin^{116TAG}-mOrange-2xMS2•Nup153-EGFP^{149TAG} | |
| | INSR^{676TAG}-EGFP-2xMS2•Nup153-EGFP^{149TAG} | |
| | INSR^{676TAG}-mOrange-2xMS2•Nup153-EGFP^{149TAG} | |
| | INSR^{676TAG}-INSR-2xMS2•Nup153-EGFP^{149TAG} | |
| KIF16B-SPD5-PylRS^{AF}•KIF16B-SPD5-MCP | GFP^{39TAG}-2xMS2-mCherry^{185TAG} | HEK293T |
| | mCherry^{185TAG}-2xMS2•GFP^{39TAG} | |
| KIF16B-FUS-PylRS^{AA}•KIF16B-EWSR1-MCP | mCherry^{185TAG}-2xMS2•GFP^{39TAG} | HEK293T |
| KIF16B-FUS-PylRS^{AAAF}•KIF16B-EWSR1-MCP | mCherry^{185TAG}-2xMS2•GFP^{39TAG} | HEK293T |
| KIF13A-PylRS^{AF}•KIF13A-MCP | mCherry^{185TAG}-2xMS2•GFP^{39TAG} | HEK293T |
| KIF13A-FUS-PylRS^{AF}•KIF13A-EWSR1-MCP | mCherry^{185TAG}-2xMS2•GFP^{39TAG} | HEK293T |
| | GFP^{39TAG}-2xMS2•mCherry^{185TAG} | |
| | mCherry^{185TAG}-4xBoxB•GFP^{39TAG}-2xMS2 | |
| | mCherry^{185TAA}-2xMS2•GFP^{39TAA} | |
| | mCherry^{185TGA}-2xMS2•GFP^{39TGA} | |
| KIF13A-SPD5-PylRS^{AF}•KIF13A-SPD5-MCP | mCherry^{185TAG}-2xMS2•GFP^{39TAG} | HEK293T |
| KIF13A-FUS-PylRS^{AA}•KIF13A-EWSR1-MCP | mCherry^{185TAG}-2xMS2•GFP^{39TAG} | HEK293T |
| KIF13A-FUS-PylRS^{AAAF}•KIF13A-EWSR1-MCP | mCherry^{185TAG}-2xMS2•GFP^{39TAG} | HEK293T |
| LCK-FUS-PylRS^{AF}•LCK-EWSR1-MCP | GFP^{39TAG}-2xMS2-mCherry^{185TAG} | HEK293T/C |
| | mCherry^{185TAG}-2xMS2•GFP^{39TAG} | OS-7 |
| | mCherry^{185TAG}-4xBoxB•GFP^{39TAG}-2xMS2 | |
| | mCherry^{185TAA}-2xMS2•GFP^{39TAA} | |
| | mCherry^{185TGA}-2xMS2•GFP^{39TGA} | |
| | Nup153-EGFP^{149TAG}-2xMS2•Vimentin^{116TAG}-mOrange | |
| | Vimentin^{116TAG}-mOrange-2xMS2•Nup153-EGFP^{149TAG} | |
| LCK-FUS-MCP-PylRS^{AF} | mCherry^{185TAG}-2xMS2•GFP^{39TAG} | HEK293T |
| | mCherry^{185TAG}-4xBoxB•GFP^{39TAG}-2xMS2 | |
| LCK-FUS-PylRS^{AAAF}•LCK-EWSR1-MCP | mCherry^{185TAG}-2xMS2•GFP^{39TAG} | HEK293T |
| | mCherry^{185TAG}-4xBoxB•GFP^{39TAG}-2xMS2 | |
| LCK-FUS-PylRS^{AF}•LCK-EWSR1-4xλ_{N22} | mCherry^{185TAG}-4xBoxB•GFP^{39TAG} | HEK293T |
| | mCherry^{185TAG}-4xBoxB•GFP^{39TAG}-2xMS2 | |
| LCK-PylRS^{AF}•LCK-MCP | GFP^{39TAG}-2xMS2-mCherry^{185TAG} | HEK293T/C |
| | mCherry^{185TAG}-2xMS2•GFP^{39TAG} | OS-7 |
| | mCherry^{185TAG}-4xBoxB•GFP^{39TAG}-2xMS2 | |
| LCK-PylRS^{AAAF}•LCK-MCP | mCherry^{185TAG}-2xMS2•GFP^{39TAG} | HEK293T |
| | mCherry^{185TAG}-4xBoxB•GFP^{39TAG}-2xMS2 | |
| LCK-FUS-3xMCP-PyIRS^{AF} | mCherry^{185TAG}-2xMS2•GFP^{39TAG} | HEK293T |
| | mCherry^{185TAG}-4xBoxB•GFP^{39TAG}-2xMS2 | |
| TOM20-FUS-PylRS^{AF}•TOM20-EWSR1-MCP | mCherry^{185TAG}-2xMS2•GFP^{39TAG} mCherry^{185TAG}-4xBoxB•GFP^{39TAG}-2xMS2 | HEK293T |
| TOM20-FUS-PylRS^{AF}•TOM20-EWSR1-4xλ_{N22} | mCherry^{185TAG}-4xBoxB•GFP^{39TAG} | HEK293T/C |
| | mCherry^{185TAG}-4xBoxB•GFP^{39TAG}-2xMS2 | OS-7 |
| TOM20-FUS-PylRS^{AA}•TOM20-EWSR1-MCP | mCherry^{185TAG}-2xMS2•GFP^{39TAG} | HEK293T |
| TOM20-FUS-PylRS^{AA}•TOM20-EWSR1-4xλ_{N22} | mCherry^{185TAG}-4xBoxB•GFP^{39TAG} | HEK293T |
| TOM20-FUS-PylRS^{AAAF}•TOM20-EWSR1-MCP | mCherry^{185TAG}-2xMS2•GFP^{39TAG} | HEK293T |
| TOM20-FUS-PylRS^{AAAF}•TOM20-EWSR1-4xλ_{N22} | mCherry^{185TAG}-4xBoxB•GFP^{39TAG} | HEK293T |
| | mCherry^{185TAG}-4xBoxB•GFP^{39TAG}-2xMS2 | |
| TOM20-3xMCP-PylRS^{AF} | mCherry^{185TAG}-2xMS2•GFP^{39TAG} | HEK293T |
| | mCherry^{185TAG}-4xBoxB•GFP^{39TAG}-2xMS2 | |
| TOM20-FUS-3xMCP-PylRS^{AF} | mCherry^{185TAG}-2xMS2•GFP^{39TAG} | HEK293T |
| TOM20-FUS-3xMCP-PylRS^{AAAF} | mCherry^{185TAG}-2xMS2•GFP^{39TAG} | HEK293T |
| | mCherry^{185TAG}-4xBoxB•GFP^{39TAG}-2xMS2 | |
| TOM20-FUS-4xλ_{N22}-PylRS^{AF} | mCherry^{185TAG}-4xBoxB•GFP^{39TAG}-2xMS2 | HEK293T/C OS-7 |
| TOM20-FUS-4xλ_{N22}-PylRS^{AAAF} | mCherry^{185TAG}-4xBoxB•GFP^{39TAG}-2xMS2 | HEK293T |
| KIF16B-FUS-4xλ_{N22}-PylRS^{AF} | mCherry^{185TAG}-4xBoxB•GFP^{39TAG} | HEK293T |
| | mCherry^{185TAG}-4xBoxB•GFP^{39TAG}-2xMS2 | |
| KIF16B-SPD5-4xλ_{N22}-PylRS^{AF} | mCherry^{185TAG}-4xBoxB•GFP^{39TAG} | HEK293T |
| KIF16B-SPD5-MCP-PylRS^{AF} | mCherry^{185TAG}-2xMS2•GFP^{39TAG} | HEK293T |
| FRB-CD28-FUS-PylRS^{AA}•FRB-CD28-EWSR1-MCP | mCherry^{185TAG}-2xMS2•GFP^{39TAG} | HEK293T |
| FRB-CD28-FUS-PylRS^{AA}•FRB-CD28-EWSR1-4xλ_{N22} | mCherry^{185TAG}-4xBoxB•GFP^{39TAG} | HEK293T |
| FRB-CD28-FUS-PylRS^{AF}•FRB-CD28-EWSR1-MCP | mCherry^{185TAG}-2xMS2•GFP^{39TAG} | HEK293T |
| FRB-CD28-FUS-PylRS^{AF}•FRB-CD28-EWSR1-4xλ_{N22} | mCherry^{185TAG}-4xBoxB•GFP^{39TAG} | HEK293T |
| FUS-CD28-FUS-PylRS^{AA}•FUS-CD28-EWSR1-MCP | mCherry^{185TAG}-2xMS2•GFP^{39TAG} | HEK293T |
| | mCherry^{185TAG}-4xBoxB•GFP^{39TAG}-2xMS2 | |
| FUS-CD28-FUS-PylRS^{AA}•FUS-CD28-EWSR1-4xλ_{N22} | mCherry^{185TAG}-4xBoxB•GFP^{39TAG} | HEK293T |
| | mCherry^{185TAG}-4xBoxB•GFP^{39TAG}-2xMS2 | |
| FUS-CD28-FUS-PylRS^{AF}•FUS-CD28-EWSR1-MCP | mCherry^{185TAG}-2xMS2•GFP^{39TAG} | HEK293T |
| FUS-CD28-FUS-PylRS^{AF}•FUS-CD28-EWSR1-4xλ_{N22} | mCherry^{185TAG}-4xBoxB•GFP^{39TAG} | HEK293T |
| FRB-CD28-FUS-MCP-PylRS^{AA} | mCherry^{185TAG}-2xMS2•GFP^{39TAG} | HEK293T |
| | mCherry^{185TAG}-4xBoxB•GFP^{39TAG}-2xMS2 | |
| FRB-CD28-FUS-MCP-PylRS^{AF} | mCherry^{185TAG}-2xMS2•GFP^{39TAG} | HEK293T |
| | mCherry^{185TAG}-4xBoxB•GFP^{39TAG}-2xMS2 | |
| FUS-CD28-FUS-MCP-PylRS^{AF} | mCherry^{185TAG}-2xMS2•GFP^{39TAG} | HEK293T |
| | mCherry^{185TAG}-4xBoxB•GFP^{39TAG}-2xMS2 | |
| FUS-CD28-FUS-MCP-PylRS^{AA} | mCherry^{185TAG}-2xMS2•GFP^{39TAG} | HEK293T |
| KIF16B-FUS-MCP-PylRS^{AF} | mCherry^{185TAG}-2xMS2•GFP^{39TAG} | HEK293T/C |
| | mCherry^{185TAG}-4xBoxB•GFP^{39TAG}-2xMS2 | OS-7 |
| | GFP^{39TAG} -2xMS2•mCherry^{185TAG} | |
| KIF13A-FUS-MCP-PylRS^{AF} | mCherry^{185TAG}-2xMS2•GFP^{39TAG} | HEK293T |
| TOM20-FUS-V5-PylRS^{AF}•TOM20-EWSR1-HA-MCP | mCherry^{185TAG}-2xMS2•GFP^{39TAG} | HEK293T |
| TOM20-FUS-V5-PylRS^{AF}•TOM20-EWSR1-Myc-4xλ_{N22} | mCherry^{185TAG}-4xBoxB•GFP^{39TAG} | HEK293T |
| TOM20-FUS-V5-PylRS^{AA}•TOM20-EWSR1-HA-MCP | mCherry^{185TAG}-2xMS2•GFP^{39TAG} | HEK293T |
| TOM20-FUS-V5-PylRS^{AA}•TOM20-EWSR1-Myc-4xλ_{N22} | mCherry^{185TAG}-4xBoxB•GFP^{39TAG} | HEK293T |
| TOM20-FUS-V5-PylRS^{AAAF}•TOM20-EWSR1-HA-MCP | mCherry^{185TAG}-2xMS2•GFP^{39TAG} | HEK293T |
| TOM20-FUS-V5-PylRS^{AAAF}•TOM20-EWSR1-Myc-4xλ_{N22} | mCherry^{185TAG}-4xBoxB•GFP^{39TAG} | HEK293T |
| KIF16B-VSV-G-FUS-PylRS^{AF}•KIF16B-EWSR1-MCP | mCherry^{185TAG}-2xMS2•GFP^{39TAG} | HEK293T |
| KIF16B-VSVG-FUS-PylRS^{AF}•KIF16B-EWSR1-2xPCP | mCherry^{185TAG}-2xpp7•GFP^{39TAG} | HEK293T |
| KIF16B-VSVG-FUS-PylRS^{AF}•KIF16B-EWSR1-2xPCP | mCherry^{185TAG}-4xpp7•GFP^{39TAG} | HEK293T |
| KIF16B-VSVG-FUS-PylRS^{AF}•KIF16B-EWSR1-2xPCP | mCherry^{185TAG}-6xpp7•GFP^{39TAG} | HEK293T |

### EXAMPLE 12 - Further OT systems

In addition to the OT systems described in the preceding examples, a variety of similar OT systems were tested, differing with respect to the mRNA targeting components, and were found to allow for selective orthogonal translation of the reporter (i.e. the POI). A summary of these experiments is shown in the Table 2 below. The results are shown in Figures 7A, B and C. The cytoplasmic NES-PyIRS system as previously described by Nikic *et al.* (Angew Chem Int Ed Engl 2016, 55(52):16172-16176) was used as a nonspecific reference (negative control). All experiments were performed in presence of the codon-specific tRNA^{Pyl} and PylRS mutant corresponding ncAAs.

**Table 2: Tested OT systems**

| **Fusion protein(s) comprising O-RS and RNA-TP segments** | **Reporter (POI)** | **cell lines** |
|---|---|---|
| EBAG9₁₋₂₉::FUS::PyIRS•EBAG9₁₋₂₉:: EWSR1::4xλ_{N22} | mCherry^{190TAG}-4xBoxB•GFP^{39TAG} | HEK293T |
| EBAG9₁₋₂₉::FUS::PyIRS•EBAG9₁₋₂₉:: EWSR1::MCP | mCherry^{190TAG}-2xMS2•GFP^{39TAC} | HEK293T |
| EBAG9₁₋₂₉::FUS::PyIRS • EBAG9₁₋₂₉:: EWSR1::2xPCP | mCherry^{190TAG}-2xpp7•GFP^{39TAG} | HEK293T |

The results are shown in Figures 7A, B and C.

### EXAMPLE 13 - Further OT fusion constructs tested

In addition to the OT systems described in the preceding examples, a variety of other OT fusion constructs were prepared and tested and found to allow for selective orthogonal translation of the reporter (i.e. the POI). A summary of the tested constructs is shown in the Table 3 below. Unless noted otherwise, the cytoplasmic NES-PyIRS system as previously described by Nikic et al. (Angew Chem Int Ed Engl 2016, 55(52):16172-16176) but with the corresponding AF, AA or AAAF mutations, or one of the Pyl RS mutants CpkRS, CbzRS, IFRS1 und OMeRS was used as a nonspecific reference (negative control).

All experiments were performed in the presence of the codon specific tRNA^{Pyl} and PylRS mutant corresponding noncanoncial amino acids [for example CpkRS with cyclopropene-L-Lysine, CbzRS with N(epsilon)-Benzyloxycarbonyl-L-lysine, IFRS-1 with 3-lodo-L-phenylalanine, OMeRS with 4-Methoxy-L-phenylalanine)].

All constructs were tested with a respective reporter ms2-loops for MCP, boxB-loops for λ_{N22}, pp7-loops for PCP.

In all fusion constructs the synthetases should be freely interchangeable.

For the SYNZIP constructs it is important to note that SYNZIP1 forms a pair with SYNZIP2 and SYNZIP3 forms a pair with SYNZIP4. In principle all other described SYNZIPs should work similarly (https://pubs.acs.org/doi/pdf/10.1021/ja907617a).

**Table 3: Tested OT fusion constructs**

| **Fusion proteins comprising O-RS or RNA-TP segments** | **AA SEQ ID NO:** |
|---|---|
| EBAG₉₁₋₂₉::FUS::PyIRS(AF) | 320 |
| EBAG9::PyIRS(AF) | 322 |
| EBAG9::FUS::PyIRS(AF) | 324 |
| EBAG9::MCP | 326 |
| EBAG9::EWSR1::MCP | 328 |
| EBAG9₁₋₂₉::PyIRS(AF) | 330 |
| EBAG9₁₋₂₉::FUS::PyIRS(AF) | 332 |
| EBAG9₁₋₂₉::MCP | 334 |
| EBAG9₁₋₂₉::EWSR1::MCP | 336 |
| EBAG9::EWSR1::4xλ_{N22} | 338 |
| EBAG9₁₋₂₉::EWSR1::4xλ_{N22} | 340 |
| EBAG9::PyIRS(AA) | 342 |
| EBAG9::PyIRS(AAAF) | 344 |
| EBAG9::FUS::PyIRS(AA) | 346 |
| EBAG9::FUS::PyIRS(AAAF) | 348 |
| EBAG9₁₋₂₉::FUS::PyIRS(AA) | 350 |
| EBAG9_{1.29}::FUS::PyIRS(AAAF) | 352 |
| EBAG9₁₋₂₉::FUS::MCP::PyIRS(AF) | 354 |
| CG1::PyIRS(AF) | 356 |
| CG1::PyIRS(AA) | 358 |
| CG1::PyIRS(AAAF) | 360 |
| CG1::FUS::PyIRS(AA) | 362 |
| CG1::FUS::PyIRS(AAAF) | 364 |
| CG1::MCP | 366 |
| CG1::EWSR1::MCP | 368 |
| CG1::FUS::PyIRS(AF) | 370 |
| CG1::FUS::MCP::PyIRS(AF) | 372 |
| CMP-SaTr::PyIRS(AF) | 374 |
| CMP-SaTr::PyIRS(AA) | 376 |
| CMP-SaTr::PyIRS(AAAF) | 378 |
| CMP-SaTr::FUS::PyIRS(AA) | 380 |
| CMP-SaTr::FUS::PyIRS(AAAF) | 382 |
| CMP-SaTr::MCP | 384 |
| CMP-SaTr::EWSR1::MCP | 386 |
| CMP-SaTr::PyIRS(AF)EWSR1::4xλ_{N22} | 388 |
| CMP-SaTr::FUS::PyIRS(AF) | 390 |
| P450 2C1₁₋₂₇::PyIRS(AF) | 392 |
| P450 2C1₁₋₂₇::MCP | 394 |
| P450 2C1₁₋₂₇::FUS::PyIRS(AF) | 396 |
| P450 2C1₁₋₂₇::EWSR1::MCP | 398 |
| P450 2C1₁₋₂₇::FUS::MCP::PyIRS(AF) | 400 |
| P450 2C1₁₋₂₉::FUS::MCP::PyIRS(AF) | 402 |
| EB1::PyIRS(AF) | 404 |
| EB1::PyIRS(AA) | 406 |
| EB1::PyIRS(AAAF) | 408 |
| EB1::FUS::PyIRS(AA) | 410 |
| EB1::FUS::PyIRS(AAAF) | 412 |
| EB1::MCP | 414 |
| EB1::EWSR1::MCP | 416 |
| EB1::EWSR1::4xλ_{N22} | 418 |
| EB1::FUS::PylRS(AF) | 420 |
| EB1::FUS::MCP::PyIRS(AF) | 422 |
| TOM20::FUS::PCP::PyIRS(AF) | 424 |
| TOM20::FUS::2xPCP::PyIRS(AF) | 426 |
| TOM20::FUS::4xλ_{N22}::PylRS(AF) | 428 |
| LCK::FUS::2xPCP::CbzRS | 430 |
| LCK::FUS::PCP::CbzRS | 432 |
| TOM20::FUS::CbzRS | 434 |
| TOM20::FUS::2xPCP::CbzRS | 436 |
| TOM20::FUS :4xλ_{N22}::CbzRS | 438 |
| EBAG9₁₋₂₉::FUS::PCP::PylRS(AF) | 440 |
| EBAG9₁₋₂₉::FUS::4xλ_{N22}::IFRS1 | 442 |
| KIF16B::EWSR1::Myc::2xPCP | 444 |
| KIF16B::EWSR1::HA::2xPCP | 446 |
| EBAG9₁₋₂₉::EWSR1::Myc::2xPCP | 448 |
| EBAG9₁₋₂₉::EWSR1::HA::2xPCP | 450 |
| LCK::CbzRS | 452 |
| LCK::FUS::CbzRS | 454 |
| TOM20::FUS::SYNZIP1::CpkRS | 456 |
| KIF16B::FUS::CbzRS | 458 |
| EBAG9₁₋₂₉::FUS::CpkRS | 460 |
| TOM20::FUS::CbzRS | 462 |
| EBAG9₁₋₂₉::FUS::CbzRS | 464 |
| TOM20::FUS::SYNZIP1::CbzRS | 466 |
| KIF16B::FUS::CpkRS | 468 |
| LCK::FUS::CpkRS | 470 |
| LCK::CpkRS | 472 |
| TOM20::FUS::SYNZIP3::CbzRS | 474 |
| TOM20::FUS::SYNZIP3::CpkRS | 476 |
| TOM20::EWSR1::PyIRS(AA)::FUS::PyIRS(AA) | 478 |
| LCK::PyIRS(AF)::FUS::PyIRS(AF) | 480 |
| LCK::FUS::PylRS(AF)::EWSR1::PyIRS(AF) | 482 |
| LCK::FUS::PyIRS(AF)::FUS::PyIRS(AF) | 484 |
| TOM20::FUS::PyIRS(AF)::EWSR1::PyIRS(AF) | 486 |
| TOM20::FUS::PyIRS(AF)::FUS::PyIRS(AF) | 488 |
| TOM20::EWSR1::4xλ_{N22}::PyIRS(AA)::FUS::PyIRS(AA) | 490 |
| LCK::EWSR1::MCP::PyIRS(AA)::FUS::PyIRS(AA) | 492 |
| LCK::PyIRS(AA)::FUS::PyIRS(AA) | 494 |
| LCK::PyIRS(AF)::EWSR1::PyIRS(AF) | 496 |
| TOM20::FUS::MCP::PyIRS(AF)::EWSR1::PyIRS(AF) | 498 |
| TOM20::FUS::4xλ_{N22}::PyIRS(AF)::EWSR1::PyIRS(AF) | 500 |
| TOM20::FUS::SYNZIP1::MCP::PyIRS(AF)::EWSR1::PyIRS(AF) | 502 |
| TOM20::FUS::SYNZIP2::MCP::PyIRS(AF)::EWSR1::PyIRS(AF) | 504 |
| LCK::FUS::SYNZIP1::MCP::PyIRS(AF)::EWSR1::PyIRS(AF) | 506 |
| LCK::FUS::SYNZIP2::MCP::PyIRS(AF)::EWSR1::PyIRS(AF) | 508 |
| LCK::PyIRS(AA)::EWSR1::PyIRS(AA) | 510 |
| LCK::FUS::PyIRS(AA)::EWSR1::PyIRS(AA) | 512 |
| TOM20::FUS::PyIRS(AA)::EWSR1::PyIRS(AA) | 514 |
| TOM20::FUS::MCP::PyIRS(AA)::EWSR1::PyIRS(AA) | 516 |
| TOM20::FUS::4xλ_{N22}::PyIRS(AA)::EWSR1::PyIRS(AA) | 518 |
| LCK::EWSR1::MCP::PyIRS(AF)::FUS::PyIRS(AF) | 520 |
| TOM20::EWSR1::4xλ_{N22}::PylRS(AF)::FUS::PyIRS(AF) | 522 |
| TOM20::EWSR1::MCP::PyIRS(AF)::FUS::PyIRS(AF) | 524 |
| LCK::PyIRS(AA)::FUS::PyIRS(AA) | 526 |
| EBAG9₁₋₂₉::EWSR1::SYNZIP4::4xλ_{N22} | 528 |
| KIF16B::FUS::SYNZIP1::PyIRS(AF) | 530 |
| KIF16B::FUS::SYNZIP1::PyIRS(AA) | 532 |
| EBAG9₁₋₂₉::EWSR1::SYNZIP2::MCP | 534 |
| TOM20::EWSR1::SYNZIP2::MCP | 536 |
| TOM20::FUS::SYNZIP4::4xλ_{N22}::PyIRS(AA) | 538 |
| KIF16B::EWSR1::SYNZIP4::4xλ_{N22} | 540 |
| TOM20::EWSR1::SYNZP4::4xλ_{N22} | 542 |
| TOM20::FUS::SYNZIP1::PyIRS(AF) | 544 |
| TOM20::FUS::SYNZIP::3::PyIRS(AF) | 546 |
| EBAG9₁₋₂₉::FUS::SYNZIP1::PyIRS(AF) | 548 |
| EBAG9₁₋₂₉::FUS::SYNZIP3::PyIRS(AF) | 550 |
| TOM20::FUS::SYNZIP1::PyIRS(AA) | 552 |
| TOM20::FUS::SYNZIP3::PyIRS(AA) | 554 |
| TOM20::FUS::SYNZIP3::PyIRS(AAAF) | 556 |
| LCK::FUS::SYNZIP3::PyIRS(AF) | 558 |
| LCK::SYNZIP1::PyIRS(AF) | 560 |
| LCK::SYNZIP3::PyIRS(AF) | 562 |
| SYNZIP2::MCP | 564 |
| LCK::EWSR1::SYNZIP2::MCP | 566 |
| LCK::EWSR1::SYNZIP4::4xλ_{N22} | 568 |
| LCK::SYNZIP2::MCP | 570 |
| EWSR1::SYNZIP2::MCP | 572 |
| LCK::FUS::SYNZIP1::PyIRS(AF) | 574 |
| LCK::FUS::SYNZIP3::PyIRS(AF) | 576 |
| TOM20::EWSR1::SYNZIP4::2xPCP | 578 |
| TOM20::EWSR1::SYNZIP2::2xPCP | 580 |
| KIF16B::EWSR1::SYNZIP2::MCP | 582 |
| LCK::SYNZIP1::PyIRS(AF) | 584 |
| LCK::FUS::SYNZIP1::PylRS(AF) | 586 |
| SYNZIP4::4xλN22 | 588 |
| TOM20::FUS::SYNZIP1::MCP::PyIRS(AF) | 590 |
| TOM20::FUS::SYNZIP2::MCP::PyIRS(AF) | 592 |
| TOM20::FUS::SYNZIP1::MCP::PyIRS(AA) | 594 |
| TOM20::FUS::SYNZIP2::MCP::PyIRS(AA) | 596 |
| TOM20::FUS::SYNZIP::MCP::IFRS1 | 598 |
| TOM20::FUS::SYNZIP2::MCP::IFRS1 | 600 |
| TOM20::FUS::SYNZIP3::4xλ_{N22}::CbzRS | 602 |
| EBAG9₁₋₂₉::FUS::SYNZIP3::PCP::PyIRS(AF) | 604 |
| EBAG9₁₋₂₉::FUS::SYNZIP4::PyIRS(AF) | 606 |
| EBAG9₁₋₂₉::FUS::SYNZIP3::4xλ_{N22}::IFRS1 | 608 |
| LCK::FUS::SYNZIP1::MCP::PyIRS(AF) | 610 |
| LCK::FUS::SYNZIP2::MCP::PyIRS(AF) | 612 |
| CG1::FUS::SYNZIP1::MCP::PyIRS(AF) | 614 |
| CG1::FUS::SYNZIP2::MCP::PyIRS(AF) | 616 |
| TOM20::FUS::SYNZIP4::λ_{N22}::CbzRS | 618 |
| EBAG9₁₋₂₉::FUS::SYNZIP4::4xλ_{N22}::IFRS1 | 620 |
| TOM20::FUS::SYNZIP3::4xλ_{N22}::PylRS(AA) | 622 |
| LCK::FUS::SYNZIP1::MCP::PyIRS(AA) | 624 |
| TOM20::EWSR1::SYNZIP4::4xλ_{N22}::SYNZIP4::PylRS(AF) | 626 |
| TOM20::FUS::SYNZIP1::MCP::SYNZIP1::PyIRS(AF) | 628 |
| TOM20::EWSR1::SYNZIP4::4xλ_{N22}::SYNZIP4::PyIRS(AA) | 630 |
| TOM20::FUS::SYNZIP3::4xλ_{N22}::SYNZIP3::PyIRS(AA) | 632 |
| LCK::EWSR1::SYNZIP4::4xλ_{N22}::SYNZIP4::PyIRS(AF) | 634 |
| LCK::FUS::SYNZIP3::4xλ_{N22}::SYNZIP3::PyIRS(AF) | 636 |
| TOM20::FUS::SYNZIP3::4xλ_{N22}::SYNZIP3::PyIRS(AF) | 638 |
| TOM20::EWSR1::SYNZIP2::MCP::SYNZIP2::PyIRS(AF) | 640 |
| LCK::OMeRS | 642 |
| TOM20::FUS::OMeRS | 644 |
| KIF16B::FUS::OMeRS | 646 |
| LCK::FUS::OMeRS | 648 |
| EBAG9₁-₂₉:FUS::OMeRS | 650 |
| TOM20::FUS::SYNZIP1::OMeRS | 652 |
| TOM20::FUS::SYNZIP3::OMeRS | 654 |
| SLP3::FUS::PyIRS(AF) | 656 |
| SLP3::MCP | 658 |
| SLP3::EWSR1::MCP | 660 |
| SLP3::EWSR1::4xλ_{N22} | 662 |
| SLP3::PyIRS(AF) | 664 |
| TOM20::FUS::MCP::PyIRS(AF) | 666 |
| KIF16B::1xLAF-1::PyIRS(AF) | 668 |
| KIF16B::1xLAF-1::MCP | 670 |
| KIF16B::1xLAF-1::2xPCP | 672 |
| KIF16B::2xLAF-1::2xPCP | 674 |
| KIF16B::2xLAF-1::PyIRS(AF) | 676 |
| KIF16B::2xLAF-1::MCP | 678 |

| | |
|---|---|
| AA: amino acid sequence | |

### ABBREVIATIONS

- "-" or "::": Symbols representing a peptidic linkage
- "•": Symbol representing a combination of polypeptides
- AP: polypeptide segment acting as assembler
- AFP: assembler fusion protein
- BSA: bovine serum albumin
- BoxB: lambda phase RNA stem-loop, specific binding site of λ_{N22}
- CbzRS: *Methanosarcina mazei* PyIRS (Y306M, L309G, C348T)
- CDS: (en-)coding sequence
- CG1: CG1 (Nup42) nucleoporin protein for targeting to nuclear membrane
- CMPSiaTr: CMP sialic acid transporter for targeting to Golgi membrane
- CpkRS: *Methanosarcina mazei* PyIRS (A302S
- EB1: EB1 protein for targeting to microtubule plus ends
- EBAG9: Receptor-binding cancer antigen expressed on SiSo cells
- EBAG9_{FL}: EBAG9 full length protein for targeting to Golgi membrane
- EBAG9₁₋₂₉: EBAG9 amino acid residues 1 -29 (N-terminal) for targeting to Golgi membrane
- EGFP^{149TAG}: enhanced green fluorescent protein, amino acid position 149 encoded by Amber codon (TAG)
- EP: polypeptide segment acting as an effector
- ER: Endoplasmatic Reticulum
- EWSR1: Ewing sarcoma breakpoint region 1 (also termed EWS herein)
- FBS: fetal bovine serum
- FFC: fluorescence flow cytometry
- FISH: fluorescence in situ hybridization
- FRB-CD28: synthetic membrane targeting domain derived from transmembrane proteins CD4, FRB (similar to mTOR) and CD28
- FSC-A: forward scatter area
- FUS: fused-in sarcoma
- FUS-CD28: (synthetic membrane targeting fusion polypeptide derived from CD4, FUS and CD28
- GCE: genetic code expansion
- GFP: green fluorescent protein
- GFP^{39TAA}: green fluorescent protein, amino acid position 39 encoded by Ochre codon (TAA)
- GFP^{39TAG}: green fluorescent protein, amino acid position 39 encoded by Amber codon (TAG)
- GFP^{39TGA}: green fluorescent protein, amino acid position 39 encoded by Opal codon (TGA)
- GFP^{39,149TAG}: green fluorescent protein, each of amino acid positions 39 and 149 encoded by Amber codon (TAG)
- GFP^{39,149,182TAG}: green fluorescent protein, each of amino acid positions 39, 149 and 182 encoded by Amber codon (TAG)
- IC-TP: intracellular targeting polypeptide
- IDP: intrinsically disordered protein
- IFRS1: *Methanosarcina mazei* PyIRS (L305M, Y306L, L309S, N346S, C348M)
- INSR: insulin receptor
- INSR^{676TAG}: insulin receptor, amino acid position 676 encoded by Amber codon (TAG)
- iRFP: near-infrared fluorescent protein
- KIF13A: kinesin family member 13A - Unless specified otherwise herein, "KIF13A" specifically refers to the fragment covering amino acid residues 1-411 of KIF13A wherein P390 is deleted (KIF13A_{1-411,ΔP390}).
- KIF16B: kinesin family member 16B - Unless specified otherwise herein, "KIF16B" specifically refers to the fragment covering amino acid residues 1-400 of KIF16B (KIF16B₁₋₄₀₀).
- λ_{N22}: 22 amino acid RNA-binding domain of lambda phage antiterminator protein N
- LcK: posttranslational modification site for plasma membrane targeting of lymphocyte-specific protein tyrosine kinase
- mCherry^{185TAG}: mCherry, amino acid position 185 encoded by Amber codon (TAG)
- MCP: MS2 bacteriophage coat protein
- MLC: membrane-less compartment
- MS2: Enterobacteria phage MS2
- MS2-tag: two MS2 RNA stem-loops fused to the 3' untranslated region of the mRNA (or coding sequence therefor)
- ms2: MS2-tag
- ncAA: non-canonical amino acid
- NLS: nuclear localization sequence
- Nup153: nucleoporin 153
- O-RS: orthogonal aminoacyl tRNA synthetase
- OMeRS: *Methanosarcina mazei* PyrRS (A302T, Y384F, N346V, C348W, V401L)
- OT assembly: spacially enriched components of the GCE machinery in a membrane-less assembly that is able to act as an artificial orthogonally translating (OT) organelle
- P450 2C1₁₋₂₇: P450 2C1 residues 1-27 (N-terminal) for targeting of ER membranes
- PBS: phosphate buffered saline
- PCP: Bacteriophage coat protein for targeting to pp7 loop tag
- PEI: polyethylenimine
- POI: polypeptide of interest (= target polypeptide)
- POI^{TAG}: POI comprising an Amber-(TAG-)encoded amino acid residue (or coding sequence therefor)
- pp7: pp7 loop tag from RNA bacteriophage pp7
- PSP: phase separation polypeptide
- PyIRS: pyrrolysyl tRNA synthetase
- PylRS^{AA}: mutant *M. mazei* pyrrolysyl tRNA synthetase comprising amino acid substitutions N346A and C348A
- PyIRS^{AF}: mutant *M. mazei* pyrrolysyl tRNA synthetase comprising amino acid substitutions Y306A and Y384F
- PylRS^{AAAF}: mutant *M. mazei* pyrrolysyl tRNA synthetase comprising amino acid substitutions Y306A, N346A, C348A and Y384F
- RNA-TP: RNA targeting polypeptide
- RS: aminoacyl tRNA synthetase
- RT: room temperature
- SCO: cyclooctyne lysine
- SEM: standard error of the mean
- SSC: saline-sodium citrate (buffer)
- SSC-A: side scatter area
- SSC-W: side scatter width
- SPD5: spindle-defective protein 5
- SYNZIP1: Synthetic coiled coil peptide 1
- SYNZIP2: Synthetic coiled coil peptide 2
- SYNZIP3: Synthetic coiled coil peptide 3
- SYNZIP4: Synthetic coiled coil peptide 4
- TOMM20: translocase of outer mitochondrial membrane 20
- TOMM20₁₋₇₀: fragment covering amino acid residues 1-70 of TOMM20
- tRNA^{Pyl}: tRNA that is coupled to pyrrolysyl or another non-canonical amino acid residue by a wild-type or modified PylRS and has an anticodon that, for site-specific incorporation of a (non-canonical) amino acid residue into a POI, is preferably the reverse complement of a selector codon. - The tRNA^{Pyl} used in the examples carried the anticodon against the stop codon Amber (tRNA^{Pyl,CUA}), Ochre (tRNA^{Pyl,UUA}) or Opal (tRNA^{Pyl,UCA}), depending on which of these was used as selector codon in the POI-encoding sequence.
- 3' UTR: 3' untranslated region
- Vim^{116TAG}: Vimentin, amino acid position 116 encoded by Amber codon (TAG)

### SEQUENCES

The following sections show the sequences of the polypeptides and polynucleotides described herein.

Nucleic acid sequences are stated in 5' to 3' orientation, protein sequences are stated from N- to C-terminus.

Sequences - Set 1

1. Hybridization probes

Hybridization probe for tRNA^Pyl labelled at the 5' terminus with Cy5
CTAACCCGGCTGAACGGATTTAGAGTCCATTCGATC (SEQ ID NO:1)

Hybridization probe for tRNA^Pyl labelled at the 5' terminus with digoxigenin
CTAACCCGGCTGAACGGATTTAGAGTCCATTCGATC (SEQ ID NO:2)

Hybridization probe for the MS2 RNA stem-loop sequence labelled at the 5'
terminus with Alexa Fluor 647
CTGCAGACATGGGTGATCCTCATGTTTTCTA (SEQ ID NO:3)

2. tRNAs

DNA sequence for tRNA^Pyl,CUA (pyrrolysyl tRNA of Methanosarcina mazei for
Amber codon; anticodon underlined)
GGAAACCTGATCATGTAGATCGAATGGACTCTAAATCCGTTCAGCCGGGTTAGATTCCCGGGGTTTCCG (SEQ
ID NO:4)

DNA sequence for tRNA^Pyl,UCA (pyrrolysyl tRNA of Methanosarcina mazei for Opal
codon; anticodon underlined)
GGAAACCTGATCATGTAGATCGAATGGACTTCAAATCCGTTCAGCCGGGTTAGATTCCCGGGGTTTCCG (SEQ
ID NO:5)

DNA sequence for tRNA^Pyl,UUA (pyrrolysyl tRNA of Methanosarcina mazei for
Ochre codon; anticodon underlined)
GGAAACCTGATCATGTAGATCGAATGGACTTTAAATCCGTTCAGCCGGGTTAGATTCCCGGGGTTTCCG (SEQ
ID NO:6)

3. O-RSs

PylRS^AF (Methanosarcina mazei pyrrolysyl tRNA synthetase double mutant:
Y306A, Y384F; Uniprot: Q8PWY1)
DNA:
ATGGCGTGCCCGGTGCCGCTGCAGCTGCCGCCGCTGGAACGCCTGACCCTGGATGATAAAAAACCGCTGAATACC
CTGATCTCTGCTACTGGTCTGTGGATGAGTCGTACCGGAACCATTCATAAAATCAAACACCACGAGGTTAGCCGT
TCGAAAATCTATATTGAGATGGCGTGTGGCGATCATCTGGTTGTGAACAATAGCCGCTCTTCTCGTACAGCACGT
GCACTGCGTCACCACAAATATCGTAAAACCTGTAAACGTTGCCGTGTGTCCGATGAGGATCTGAACAAATTCCTG
ACAAAAGCCAATGAGGACCAAACAAGCGTGAAAGTGAAAGTCGTTAGCGCTCCTACCCGTACTAAAAAAGCAATG
CCGAAATCCGTTGCTCGTGCCCCTAAACCACTGGAAAACACTGAAGCAGCACAGGCACAGCCGTCTGGAAGCAAA
TTCTCTCCGGCCATTCCTGTTTCTACCCAGGAGTCCGTTTCTGTTCCAGCAAGTGTGAGCACCAGCATTAGCAGT
ATTAGCACCGGTGCCACCGCTAGCGCCCTGGTTAAAGGCAATACCAATCCGATTACAAGCATGTCTGCCCCGGTT
CAAGCATCAGCTCCAGCACTGACAAAATCCCAAACCGATCGTCTGGAGGTTCTGCTGAATCCGAAAGACGAAATC
AGCCTGAATTCCGGCAAACCGTTTCGTGAACTGGAGAGCGAACTGCTGTCACGTCGTAAAAAAGACCTGCAACAA
ATCTATGCCGAAGAACGTGAGAACTATCTGGGGAAACTGGAACGTGAAATCACCCGCTTTTTCGTGGATCGTGGC
TTTCTGGAGATCAAATCCCCGATTCTGATTCCTCTGGAGTATATCGAGCGTATGGGCATCGACAATGATACCGAA
CTGAGCAAACAAATTTTCCGTGTGGATAAAAACTTCTGTCTGCGCCCTATGCTAGCACCAAATCTGGCTAACTAT
CTGCGCAAACTGGACCGTGCCCTGCCTGATCCTATCAAAATCTTCGAGATCGGCCCGTGTTATCGTAAAGAGTCC
GACGGTAAAGAACATCTGGAGGAGTTTACCATGCTGAACTTTTGCCAAATGGGTTCAGGTTGTACTCGTGAGAAC
CTGGAAAGCATCATCACCGATTTTCTGAACCACCTGGGCATTGACTTCAAAATTGTGGGCGACAGCTGTATGGTG
TTTGGCGACACCCTGGATGTCATGCACGGCGACCTGGAACTGTCTAGTGCCGTTGTGGGCCCAATCCCGCTGGAT
CGTGAGTGGGGTATCGACAAACCTTGGATCGGTGCGGGTTTTGGTCTGGAGCGTCTGCTGAAAGTAAAACACGAC
TTCAAGAACATCAAACGTGCTGCACGTTCCGAGTCCTATTACAATGGTATTTCTACTAACCTGTAA (SEQ ID
NO:7)

Protein:
MACPVPLQLPPLERLTLDDKKPLNTLISATGLWMSRTGTIHKIKHHEVSRSKIYIEMACGDHLVVNNSRSSRTAR
ALRHHKYRKTCKRCRVSDEDLNKFLTKANEDQTSVKVKVVSAPTRTKKAMPKSVARAPKPLENTEAAQAQPSGSK
FSPAIPVSTQESVSVPASVSTSISSISTGATASALVKGNTNPITSMSAPVQASAPALTKSQTDRLEVLLNPKDEI
SLNSGKPFRELESELLSRRKKDLQQIYAEERENYLGKLEREITRFFVDRGFLEIKSPILIPLEYIERMGIDNDTE
LSKQIFRVDKNFCLRPMLAPNLANYLRKLDRALPDPIKIFEIGPCYRKESDGKEHLEEFTMLNFCQMGSGCTREN
LESIITDFLNHLGIDFKIVGDSCMVFGDTLDVMHGDLELSSAVVGPIPLDREWGIDKPWIGAGFGLERLLKVKHD
FKNIKRAARSESYYNGISTNL (SEQ ID NO:8)

PylRS^AA (Methanosarcina mazei pyrrolysyl tRNA synthetase double mutant:
N346A, C348A; Uniprot: Q8PWY1)
DNA:
ATGGCGTGCCCGGTGCCGCTGCAGCTGCCGCCGCTGGAACGCCTGACCCTGGATGACAAAAAACCGCTGAATACC
CTGATCTCTGCTACTGGTCTGTGGATGAGTCGTACCGGAACCATTCATAAAATCAAACACCACGAGGTTAGCCGT
TCGAAAATCTATATTGAGATGGCGTGTGGCGATCATCTGGTTGTGAACAATAGCCGCTCTTCTCGTACAGCACGT
GCACTGCGTCACCACAAATATCGTAAAACCTGTAAACGTTGCCGTGTGTCCGATGAGGATCTGAACAAATTCCTG
ACAAAAGCCAATGAGGACCAAACAAGCGTGAAAGTGAAAGTCGTTAGCGCTCCTACCCGTACTAAAAAAGCAATG
CCGAAATCCGTTGCTCGTGCCCCTAAACCACTGGAAAACACTGAAGCAGCACAGGCACAGCCGTCTGGAAGCAAA
TTCTCTCCGGCCATTCCTGTTTCTACCCAGGAGTCCGTTTCTGTTCCAGCAAGTGTGAGCACCAGCATTAGCAGT
ATTAGCACCGGTGCCACCGCTAGCGCCCTGGTTAAAGGCAATACCAATCCGATTACAAGCATGTCTGCCCCGGTT
CAAGCATCAGCTCCAGCACTGACAAAATCCCAAACCGATCGTCTGGAGGTTCTGCTGAATCCGAAAGACGAAATC
AGCCTGAATTCCGGCAAACCGTTTCGTGAACTGGAGAGCGAACTGCTGTCACGTCGTAAAAAAGACCTGCAACAA
ATCTATGCCGAAGAACGTGAGAACTATCTGGGGAAACTGGAACGTGAAATCACCCGCTTTTTCGTGGATCGTGGC
TTTCTGGAGATCAAATCCCCGATTCTGATTCCTCTGGAGTATATCGAGCGTATGGGCATCGACAATGATACCGAA
CTGAGCAAACAAATTTTCCGTGTGGATAAAAACTTCTGTCTGCGCCCTATGCTGGCACCAAATCTGTATAACTAT
CTGCGCAAACTGGACCGTGCCCTGCCTGATCCTATCAAAATCTTCGAGATCGGCCCGTGTTATCGTAAAGAGTCC
GACGGTAAAGAACATCTGGAGGAGTTTACCATGCTGGCCTTTGCCCAAATGGGTTCAGGTTGTACTCGTGAGAAC
CTGGAAAGCATCATCACCGATTTTCTGAACCACCTGGGCATTGACTTCAAAATTGTGGGCGACAGCTGTATGGTG
TATGGCGACACCCTGGATGTCATGCACGGCGACCTGGAACTGTCTAGTGCCGTTGTTGGACCAATTCCGCTGGAC
CGTGAGTGGGGTATCGACAAACCGTGGATCGGAGCAGGATTCGGTCTGGAACGCCTGCTGAAAGTGAAACACGAC
TTCAAAAACATCAAACGTGCCGCCCGTTCTGAATCGTATTATAACGGGATCTCTACGAACCTGTAA (SEQ ID
NO:9)
Protein:
MACPVPLQLPPLERLTLDDKKPLNTLISATGLWMSRTGTIHKIKHHEVSRSKIYIEMACGDHLVVNNSRSSRTAR
ALRHHKYRKTCKRCRVSDEDLNKFLTKANEDQTSVKVKVVSAPTRTKKAMPKSVARAPKPLENTEAAQAQPSGSK
FSPAIPVSTQESVSVPASVSTSISSISTGATASALVKGNTNPITSMSAPVQASAPALTKSQTDRLEVLLNPKDEI
SLNSGKPFRELESELLSRRKKDLQQIYAEERENYLGKLEREITRFFVDRGFLEIKSPILIPLEYIERMGIDNDTE
LSKQIFRVDKNFCLRPMLAPNLYNYLRKLDRALPDPIKIFEIGPCYRKESDGKEHLEEFTMLAFAQMGSGCTREN
LESIITDFLNHLGIDFKIVGDSCMVYGDTLDVMHGDLELSSAVVGPIPLDREWGIDKPWIGAGFGLERLLKVKHD
FKNIKRAARSESYYNGISTNL (SEQ ID NO:10)

PylRS^AAAF (Methanosarcina mazei pyrrolysyl tRNA synthetase quadruple mutant:
Y306A, N346A, C348A, Y384F; Uniprot: Q8PWY1)
DNA:
GCGTGCCCGGTGCCGCTGCAGCTGCCGCCGCTGGAACGCCTGACCCTGGATGATAAAAAACCGCTGAATACCCTG
ATCTCTGCTACTGGTCTGTGGATGAGTCGTACCGGAACCATTCATAAAATCAAACACCACGAGGTTAGCCGTTCG
AAAATCTATATTGAGATGGCGTGTGGCGATCATCTGGTTGTGAACAATAGCCGCTCTTCTCGTACAGCACGTGCA
CTGCGTCACCACAAATATCGTAAAACCTGTAAACGTTGCCGTGTGTCCGATGAGGATCTGAACAAATTCCTGACA
AAAGCCAATGAGGACCAAACAAGCGTGAAAGTGAAAGTCGTTAGCGCTCCTACCCGTACTAAAAAAGCAATGCCG
AAATCCGTTGCTCGTGCCCCTAAACCACTGGAAAACACTGAAGCAGCACAGGCACAGCCGTCTGGAAGCAAATTC
TCTCCGGCCATTCCTGTTTCTACCCAGGAGTCCGTTTCTGTTCCAGCAAGTGTGAGCACCAGCATTAGCAGTATT
AGCACCGGTGCCACCGCTAGCGCCCTGGTTAAAGGCAATACCAATCCGATTACAAGCATGTCTGCCCCGGTTCAA
GCATCAGCTCCAGCACTGACAAAATCCCAAACCGATCGTCTGGAGGTTCTGCTGAATCCGAAAGACGAAATCAGC
CTGAATTCCGGCAAACCGTTTCGTGAACTGGAGAGCGAACTGCTGTCACGTCGTAAAAAAGACCTGCAACAAATC
TATGCCGAAGAACGTGAGAACTATCTGGGGAAACTGGAACGTGAAATCACCCGCTTTTTCGTGGATCGTGGCTTT
CTGGAGATCAAATCCCCGATTCTGATTCCTCTGGAGTATATCGAGCGTATGGGCATCGACAATGATACCGAACTG
AGCAAACAAATTTTCCGTGTGGATAAAAACTTCTGTCTGCGCCCTATGCTAGCACCAAATCTGGCTAACTATCTG
CGCAAACTGGACCGTGCCCTGCCTGATCCTATCAAAATCTTCGAGATCGGCCCGTGTTATCGTAAAGAGTCCGAC

GGTAAAGAACATCTGGAGGAGTTTACCATGCTGGCCTTTGCCCAAATGGGTTCAGGTTGTACTCGTGAGAACCTG
GAAAGCATCATCACCGATTTTCTGAACCACCTGGGCATTGACTTCAAAATTGTGGGCGACAGCTGTATGGTGTTT
GGCGACACCCTGGATGTCATGCACGGCGACCTGGAACTGTCTAGTGCCGTTGTGGGCCCAATCCCGCTGGATCGT
GAGTGGGGTATCGACAAACCTTGGATCGGTGCGGGTTTTGGTCTGGAGCGTCTGCTGAAAGTAAAACACGACTTC
AAGAACATCAAACGTGCTGCACGTTCCGAGTCCTATTACAATGGTATTTCTACTAACCTGTAA (SEQ ID
NO:11)
Protein:
ACPVPLQLPPLERLTLDDKKPLNTLISATGLWMSRTGTIHKIKHHEVSRSKIYIEMACGDHLVVNNSRSSRTARA
LRHHKYRKTCKRCRVSDEDLNKFLTKANEDQTSVKVKVVSAPTRTKKAMPKSVARAPKPLENTEAAQAQPSGSKF
SPAIPVSTQESVSVPASVSTSISSISTGATASALVKGNTNPITSMSAPVQASAPALTKSQTDRLEVLLNPKDEIS
LNSGKPFRELESELLSRRKKDLQQIYAEERENYLGKLEREITRFFVDRGFLEIKSPILIPLEYIERMGIDNDTEL
SKQIFRVDKNFCLRPMLAPNLANYLRKLDRALPDPIKIFEIGPCYRKESDGKEHLEEFTMLAFAQMGSGCTRENL
ESIITDFLNHLGIDFKIVGDSCMVFGDTLDVMHGDLELSSAVVGPIPLDREWGIDKPWIGAGFGLERLLKVKHDF
KNIKRAARSESYYNGISTNL (SEQ ID NO:12)

4. RNA-TPs

MCP (coat protein of Enterobacteria phage MS2)
DNA:
GCTTCTAACTTTACTCAGTTCGTTCTCGTCGACAATGGCGGAACTGGCGACGTGACTGTCGCCCCAAGCAACTTC
GCTAACGGGATCGCTGAATGGATCAGCTCTAACTCGCGTTCACAGGCTTACAAAGTAACCTGTAGCGTTCGTCAG
AGCTCTGCGCAGAATCGCAAATACACCATCAAAGTCGAGGTGCCTAAAGGCGCCTGGCGTTCGTACTTAAATATG
GAACTAACCATTCCAATTTTCGCCACGAATTCCGACTGCGAGCTTATTGTTAAGGCAATGCAAGGTCTCCTAAAA
GATGGAAACCCGATTCCCTCAGCAATCGCAGCAAACTCCGGCATCTAC (SEQ ID NO:13)
Protein:
ASNFTQFVLVDNGGTGDVTVAPSNFANGIAEWISSNSRSQAYKVTCSVRQSSAQNRKYTIKVEVPKGAWRSYLNM
ELTIPIFATNSDCELIVKAMQGLLKDGNPIPSAIAANSGIY (SEQ ID NO:14)

λN22 (22 amino acid RNA-binding domain of lambda phage antiterminator
protein N)
DNA:
ATGGACGCACAAACACGACGACGTGAGCGTCGCGCTGAGAAACAAGCTCAATGGAAAGCTGCAAAC (SEQ ID
NO:15)
Protein:
MDAQTRRRERRAEKQAQWKAAN (SEQ ID NO:16)

5. TNs

DNA sequence of Enterobacteria phage MS2 RNA stem-loop
ACATGAGGATCACCCATGT (SEQ ID NO:17)

DNA sequence of BoxB (lambda phase RNA stem-loop, specific binding site of
λN22)
GCCCTGAAAAAGGGC (SEQ ID NO:18)

6. IC-TPs

KIF16B1-400 (Homo sapiens kinesin family member 16B fragment covering amino
acid residues 1-400; Uniprot: Q96L93)
DNA:
ATGGCATCGGTCAAGGTGGCCGTGAGGGTCCGGCCCATGAATCGCAGGGAAAAGGACTTGGAGGCCAAGTTCATT
ATTCAGATGGAGAAAAGCAAAACGACAATCACAAACTTAAAGATACCAGAAGGAGGCACTGGGGACTCAGGAAGA
GAACGGACCAAGACCTTCACCTATGACTTTTCTTTTTATTCTGCTGATACAAAAAGCCCAGATTACGTTTCACAA
GAAATGGTTTTCAAAACCCTCGGCACAGATGTCGTGAAGTCTGCATTTGAAGGTTATAATGCTTGTGTCTTTGCA
TATGGGCAAACTGGATCTGGAAAGTCATACACTATGATGGGAAATTCTGGAGATTCTGGCTTAATACCTCGGATC
TGTGAAGGACTCTTCAGTCGGATAAATGAAACCACCAGATGGGATGAAGCTTCTTTTCGAACTGAAGTCAGCTAC
TTAGAAATTTATAACGAACGTGTGAGAGATCTACTTCGGCGGAAGTCATCTAAAACCTTCAATTTGAGAGTCCGT
GAGCATCCCAAAGAAGGCCCTTATGTTGAGGATTTATCCAAACATTTAGTACAGAATTATGGTGACGTAGAAGAA

CTTATGGATGCGGGCAATATCAACCGGACCACCGCAGCGACTGGGATGAACGACGTCAGTAGCAGGTCTCATGCC
ATCTTCACCATCAAGTTCACTCAGGCTAAATTTGATTCTGAAATGCCATGTGAAACCGTCAGTAAGATCCACTTG
GTTGATCTTGCCGGAAGTGAGCGTGCAGATGCCACCGGAGCCACCGGGGTTAGGCTAAAGGAAGGGGGAAATATT
AACAAGTCCCTCGTGACTCTGGGGAACGTCATTTCTGCCTTAGCTGATTTATCTCAGGATGCTGCAAATACTCTT
GCAAAGAAGAAGCAAGTTTTCGTGCCTTACAGGGATTCTGTGTTGACTTGGTTGTTAAAAGATAGCCTTGGAGGA
AACTCTAAAACTATCATGATTGCCACCATTTCACCTGCTGATGTCAATTATGGAGAAACCCTAAGTACTCTTCGC
TATGCAAATAGAGCCAAAAACATCATCAACAAGCCTACCATTAATGAGGATGCCAACGTCAAACTTATCCGTGAG
CTGCGAGCTGAAATAGCCAGACTGAAAACGCTGCTTGCTCAAGGGAATCAGATTGCCCTCTTAGACTCCCCCACA
(SEQ ID NO:19)
Protein:
MASVKVAVRVRPMNRREKDLEAKFIIQMEKSKTTITNLKIPEGGTGDSGRERTKTFTYDFSFYSADTKSPDYVSQ
EMVFKTLGTDVVKSAFEGYNACVFAYGQTGSGKSYTMMGNSGDSGLIPRICEGLFSRINETTRWDEASFRTEVSY
LEIYNERVRDLLRRKSSKTFNLRVREHPKEGPYVEDLSKHLVQNYGDVEELMDAGNINRTTAATGMNDVSSRSHA
IFTIKFTQAKFDSEMPCETVSKIHLVDLAGSERADATGATGVRLKEGGNINKSLVTLGNVISALADLSQDAANTL
AKKKQVFVPYRDSVLTWLLKDSLGGNSKTIMIATISPADVNYGETLSTLRYANRAKNIINKPTINEDANVKLIRE
LRAEIARLKTLLAQGNQIALLDSPT (SEQ ID NO:20)

KIF13A1-411,ΔP390 (Homo sapiens kinesin family member 13A fragment covering
amino acid residues 1-411 wherein P390 is deleted; Uniprot: Q9H1H9)
DNA:
ATGTCGGATACCAAGGTAAAAGTTGCCGTCCGGGTCCGGCCCATGAACCGACGAGAACTGGAACTGAACACCAAG
TGCGTGGTGGAGATGGAAGGGAATCAAACGGTCCTGCACCCTCCTCCTTCTAACACCAAACAGGGAGAAAGGAAA
CCTCCCAAGGTATTTGCCTTTGATTATTGCTTTTGGTCCATGGATGAATCTAACACTACAAAATACGCTGGTCAA
GAAGTGGTTTTCAAGTGCCTTGGGGAAGGAATTCTTGAAAAAGCCTTTCAGGGGTATAATGCGTGTATTTTTGCA
TATGGACAGACAGGTTCGGGAAAATCCTTTTCCATGATGGGCCATGCTGAGCAGCTGGGCCTTATTCCAAGGCTC
TGCTGTGCTTTATTTAAAAGGATCTCTTTGGAGCAAAATGAGTCACAGACCTTTAAAGTTGAAGTGTCCTATATG
GAAATTTATAATGAGAAAGTTCGGGATCTTTTAGACCCCAAAGGGAGTAGACAGTCTCTTAAAGTTCGAGAACAT
AAAGTTTTGGGACCATATGTAGATGGTTTATCTCAACTAGCTGTCACTAGTTTTGAGGATATTGAGTCATTGATG
TCTGAGGGAAATAAGTCTCGAACGGTAGCTGCTACCAACATGAACGAAGAAAGCAGCCGCTCCCATGCTGTGTTC
AACATCATAATCACACAGACACTTTATGACCTGCAGTCTGGGAATTCCGGGGAGAAAGTCAGTAAGGTCAGCTTG
GTAGACCTGGCGGGTAGCGAAAGAGTATCTAAAACAGGAGCTGCAGGAGAGCGACTGAAAGAAGGCAGCAACATT
AACAAATCGCTTACAACCTTGGGGTTGGTTATATCATCACTGGCTGACCAGGCAGCTGGCAAGGGTAAAAGCAAA
TTTGTGCCTTATCGAGATTCAGTCCTCACTTGGCTGCTTAAGGACAACTTGGGGGGCAACAGCCAAACCTCTATG
ATAGCCACAATCAGCCCAGCCGCAGACAACTATGAAGAGACCCTCTCCACATTAAGATATGCAGACCGAGCCAAA
AGGATTGTGAACCATGCTGTTGTGAATGAGGACCCCAACGCAAAAGTGATCCGAGAACTGCGGGAGGAAGTCGAG
AAACTGAGAGAGCAGCTCTCTCAGGCAGAGGCCATGAAGGCCGAACTGAAGGAGAAGCTCGAAGAGTCTGAAAAG
CTGATAAAAGAACTAACAGTGACTTGGGAA (SEQ ID NO:21)
Protein:
MSDTKVKVAVRVRPMNRRELELNTKCVVEMEGNQTVLHPPPSNTKQGERKPPKVFAFDYCFWSMDESNTTKYAGQ
EVVFKCLGEGILEKAFQGYNACIFAYGQTGSGKSFSMMGHAEQLGLIPRLCCALFKRISLEQNESQTFKVEVSYM
EIYNEKVRDLLDPKGSRQSLKVREHKVLGPYVDGLSQLAVTSFEDIESLMSEGNKSRTVAATNMNEESSRSHAVF
NIIITQTLYDLQSGNSGEKVSKVSLVDLAGSERVSKTGAAGERLKEGSNINKSLTTLGLVISSLADQAAGKGKSK
FVPYRDSVLTWLLKDNLGGNSQTSMIATISPAADNYEETLSTLRYADRAKRIVNHAVVNEDPNAKVIRELREEVE
KLREQLSQAEAMKAELKEKLEESEKLIKELTVTWE (SEQ ID NO:22)

TOMM201-70 (Homo sapiens translocase of outer mitochondrial membrane 20
fragment covering amino acid residues 1-70; Uniprot: Q15388)
DNA:
ATGGTGGGTCGGAACAGCGCCATCGCCGCCGGTGTATGCGGGGCCCTTTTCATTGGGTACTGCATCTACTTCGAC
CGCAAAAGACGAAGTGACCCCAACTTCAAGAACAGGCTTCGAGAACGAAGAAAGAAACAGAAGCTTGCCAAGGAG
AGAGCTGGGCTTTCCAAGTTACCTGACCTTAAAGATGCTGAAGCTGTTCAGAAATTCTTC (SEQ ID NO:23)
Protein:
MVGRNSAIAAGVCGALFIGYCIYFDRKRRSDPNFKNRLRERRKKQKLAKERAGLSKLPDLKDAEAVQKFF (SEQ
ID NO:24)

LcK (posttranslational modification site for plasma membrane targeting of
Mus musculus lymphocyte-specific protein tyrosine kinase; Uniprot: P06240)
DNA:

GGCTGCGTGTGCAGCAGCAACCCCGAGGGTACCGAGCTC (SEQ ID NO:25)
Protein: (identical part underlined P06240)
GCVCSSNPEGTEL (SEQ ID NO:26)

FRB-CD28 (synthetic membrane targeting fusion polypeptide derived from Mus musculus CD4 (Uniprot: P06332), FRB (similar to Homo sapiens mTOR; Uniprot: P42345) and Mus musculus CD28 (Uniprot: P31041)
DNA:
ATGTGCCGAGCCATCTCTCTTAGGCGCTTGCTGCTGCTGCTGCTGCAGCTGTCACAACTCCTAGCTGTCACTCAA
GGGATGCTCGAGATGTGGCATGAAGGCCTGGAAGAGGCATCTCGTTTGTACTTTGGGGAAAGGAACGTGAAAGGC
ATGTTTGAGGTGCTGGAGCCCTTGCATGCTATGATGGAACGGGGCCCCCAGACTCTGAAGGAAACATCCTTTAAT
CAGGCCTATGGTCGAGATTTAATGGAGGCCCAAGAGTGGTGCAGGAAGTACATGAAATCAGGGAATGTCAAGGAC
CTCCTCCAAGCCTGGGACCTCTATTATCATGTGTTCCGACGAATCTCAAAGACTAGAACCGGTAAGCTTTTTTGG
GCACTGGTCGTGGTTGCTGGAGTCCTGTTTTGTTATGGCTTGCTAGTGACAGTGGCTCTTTGTGTT (SEQ ID NO:27)
Protein:
MCRAISLRRLLLLLLQLSQLLAVTQGMLEMWHEGLEEASRLYFGERNVKGMFEVLEPLHAMMERGPQTLKETSFN
QAYGRDLMEAQEWCRKYMKSGNVKDLLQAWDLYYHVFRRISKTRTGKLFWALVVVAGVLFCYGLLVTVALCV
(SEQ ID NO:28)

FUS-CD28 (synthetic membrane targeting fusion polypeptide derived from Mus musculus CD4 (Uniprot: P06332), Homo sapiens fused-in sarcoma (Uniprot: P35637) and Mus musculus CD28 (Uniprot: P31041)
DNA:
ATGTGCCGAGCCATCTCTCTTAGGCGCTTGCTGCTGCTGCTGCTGCAGCTGTCACAACTCCTAGCTGTCACTCAA
GGGATGCTCATGGCCTCAAACGATTATACCCAACAAGCAACCCAAAGCTATGGGGCCTACCCCACCCAGCCCGGG
CAGGGCTATTCCCAGCAGAGCAGTCAGCCCTACGGACAGCAGAGTTACAGTGGTTATAGCCAGTCCACGGACACT
TCAGGATATGGCCAGAGCAGCTATTCTTCTTATGGCCAGAGCCAGAACACAGGCTATGGAACTCAGTCAACTCCC
CAGGGATATGGCTCGACTGGCGGCTATGGCAGTAGCCAGAGCTCCCAATCGTCTTACGGGCAGCAGTCCTCCTAC
CCTGGCTATGGCCAGCAGCCAGCTCCCAGCAGCACCTCGGGAAGTTACGGTAGCAGTTCTCAGAGCAGCAGCTAT
GGGCAGCCCCAGAGTGGGAGCTACAGCCAGCAGCCTAGCTATGGTGGACAGCAGCAAAGCTATGGACAGCAGCAA
AGCTATAATCCCCCTCAGGGCTATGGACAGCAGAACCAGTACAACAGCAGCAGTGGTGGTGGAGGTGGAGGTGGA
GGTGGAGGTAACTATGGCCAAGATCAATCCTCCATGAGTAGTGGTGGTGGCAGTGGTGGCGGTTATGGCAATCAA
GACCAGAGTGGTGGAGGTGGCAGCGGTGGCTATGGACAGCAGGACCGTGGAGGCCGCGGCAGGGGTGGCAGTGGT
GGCGGCGGCGGCGGCGGCGGTGGTGGTTACAACCGCAGCAGTGGTGGCTATGAACCCAGAGGTCGTGGAGGTGGC
CGTGGAGGCAGAGGTGGCATGGGCGGAAGTGACCGTGGTGGCTTCAATAAATTTGGTGGCCCTCGGGACCAAGGA
TCACGTCATGACTCCGAACAGGATAATTCAGACAACAACACCATCTTTGTGCAAGGCCTGGGTGAGAATGTTACA
ATTGAGTCTGTGGCTGATTACTTCAAGCAGATTGGTATTATTAAGACAAACAAGAAAACGGGACAGCCCATGATT
AATTTGTACACAGACAGGGAAACTGGCAAGCTGAAGGGAGAGGCAACGGTCTCTTTTGATGACCCACCTTCAGCT
AAAGCAGCTATTGACTGGTTTGATGGTAAAGAATTCTCCGGAAATCCTATCAAGGTCTCATTTGCTACTCGCCGG
GCAGACTTTAATCGGGGTGGTGGCAATGGTCGTGGAGGCCGAGGGCGAGGAGGACCCATGGGCCGTGGAGGCTAT
GGAGGTGGTGGCAGTGGTGGTGGTGGCCGAGGAGGATTTCCCAGTGGAGGTGGTGGCGGTGGAGGACAGCAGCGA
GCTGGTGACTGGAAGTGTCCTAATCCCACCTGTGAGAATATGAACTTCTCTTGGAGGAATGAATGCAACCAGTGT
AAGGCCCCTAAACCAGATGGCCCAGGAGGGGGACCAGGTGGCTCTCACATGGGGGGTAACTACGGGGATGATCGT
CGTGGTGGCAGAGGAGGCGGCACCGGTAAGCTTTTTTGGGCACTGGTCGTGGTTGCTGGAGTCCTGTTTTGTTAT
GGCTTGCTAGTGACAGTGGCTCTTTGTGTT (SEQ ID NO:29)
Protein:
MCRAISLRRLLLLLLQLSQLLAVTQGMLMASNDYTQQATQSYGAYPTQPGQGYSQQSSQPYGQQSYSGYSQSTDT
SGYGQSSYSSYGQSQNTGYGTQSTPQGYGSTGGYGSSQSSQSSYGQQSSYPGYGQQPAPSSTSGSYGSSSQSSSY
GQPQSGSYSQQPSYGGQQQSYGQQQSYNPPQGYGQQNQYNSSSGGGGGGGGGNYGQDQSSMSSGGGSGGGYGNQ
DQSGGGGSGGYGQQDRGGRGRGGSGGGGGGGGGGYNRSSGGYEPRGRGGGRGGRGGMGGSDRGGFNKFGGPRDQG
SRHDSEQDNSDNNTIFVQGLGENVTIESVADYFKQIGIIKTNKKTGQPMINLYTDRETGKLKGEATVSFDDPPSA
KAAIDWFDGKEFSGNPIKVSFATRRADFNRGGGNGRGGRGRGGPMGRGGYGGGSGGGGRGGFPSGGGGGGGQQR
AGDWKCPNPTCENMNFSWRNECNQCKAPKPDGPGGGPGGSHMGGNYGDDRRGGRGGGTGKLFWALVVVAGVLFCY
GLLVTVALCV (SEQ ID NO:30)

7. PSPs

SPD5 (Caenorhabditis elegans spindle-defective protein 5; Uniprot: P91349)
DNA:
ATGGAGGACAACAGCGTGCTGAACGAGGACAGCAACCTGGAGCACGTGGAGGGCCAGCCCAGAAGAAGCATGAGC
CAGCCCGTGCTGAACGTGGAGGGCGACAAGAGAACCAGCAGCACCAGCGCCACCCAGCAGCAGGTGCTGAGCGGC
GCCTTCAGCAGCGCCGACGTGAGAAGCATCCCCATCATCCAGACCTGGGAGGAGAACAAGGCCCTGAAGACCAAG
ATCACCATCCTGAGAGGCGAGCTGCAGATGTACCAGAGAAGATACAGCGAGGCCAAGGAGGCCAGCCAGAAGAGA
GTGAAGGAGGTGATGGACGACTACGTGGACCTGAAGCTGGGCCAGGAGAACGTGCAGGAGAAGATGGAGCAGTAC
AAGCTGATGGAGGAGGACCTGCTGGCCATGCAGAGCAGAATCGAGACCAGCGAGGACAACTTCGCCAGACAGATG
AAGGAGTTCGAGGCCCAGAAGCACGCCATGGAGGAGAGAATCAAGGAGCTGGAGCTGAGCGCCACCGACGCCAAC
AACACCACCGTGGGCAGCTTCAGAGGCACCCTGGACGACATCCTGAAGAAGAACGACCCCGACTTCACCCTGACC
AGCGGCTACGAGGAGAGAAAGATCAACGACCTGGAGGCCAAGCTGCTGAGCGAGATCGACAAGGTGGCCGAGCTG
GAGGACCACATCCAGCAGCTGAGACAGGAGCTGGACGACCAGAGCGCCAGACTGGCCGACAGCGAGAACGTGAGA
GCCCAGCTGGAGGCCGCCACCGGCCAGGGCATCCTGGGCGCCGCCGGCAACGCCATGGTGCCCAACAGCACCTTC
ATGATCGGCAACGGCAGAGAGAGCCAGACCAGAGACCAGCTGAACTACATCGACGACCTGGAGACCAAGCTGGCC
GACGCCAAGAAGGAGAACGACAAGGCCAGACAGGCCCTGGTGGAGTACATGAACAAGTGCAGCAAGCTGGAGCAC
GAGATCAGAACCATGGTGAAGAACAGCACCTTCGACAGCAGCAGCATGCTGCTGGGCGGCCAGACCAGCGACGAG
CTGAAGATCCAGATCGGCAAGGTGAACGGCGAGCTGAACGTGCTGAGAGCCGAGAACAGAGAGCTGAGAATCAGA
TGCGACCAGCTGACCGGCGGCGACGGCAACCTGAGCATCAGCCTGGGCCAGAGCAGACTGATGGCCGGCATCGCC
ACCAACGACGTGGACAGCATCGGCCAGGGCAACGAGACCGGCGGCACCAGCATGAGAATCCTGCCCAGAGAGAGC
CAGCTGGACGACCTGGAGGAGAGCAAGCTGCCCCTGATGGACACCAGCAGCGCCGTGAGAAACCAGCAGCAGTTC
GCCAGCATGTGGGAGGACTTCGAGAGCGTGAAGGACAGCCTGCAGAACAACCACAACGACACCCTGGAGGGCAGC
TTCAACAGCAGCATGCCCCCCCCCGGCAGAGACGCCACCCAGAGCTTCCTGAGCCAGAAGAGCTTCAAGAACAGC
CCCATCGTGATGCAGAAGCCCAAGAGCCTGCACCTGCACCTGAAGAGCCACCAGAGCGAGGGCGCCGGCGAGCAG
ATCCAGAACAACAGCTTCAGCACCAAGACCGCCAGCCCCCACGTGAGCCAGAGCCACATCCCCATCCTGCACGAC
ATGCAGCAGATCCTGGACAGCAGCGCCATGTTCCTGGAGGGCCAGCACGACGTGGCCGTGAACGTGGAGCAGATG
CAGGAGAAGATGAGCCAGATCAGAGAGGCCCTGGCCAGACTGTTCGAGAGACTGAAGAGCAGCGCCGCCCTGTTC
GAGGAGATCCTGGAGAGAATGGGCAGCAGCGACCCCAACGCCGACAAGATCAAGAAGATGAAGCTGGCCTTCGAG
ACCAGCATCAACGACAAGCTGAACGTGAGCGCCATCCTGGAGGCCGCCGAGAAGGACCTGCACAACATGAGCCTG
AACTTCAGCATCCTGGAGAAGAGCATCGTGAGCCAGGCCGCCGAGGCCAGCAGAAGATTCACCATCGCCCCCGAC
GCCGAGGACGTGGCCAGCAGCAGCCTGCTGAACGCCAGCTACAGCCCCCTGTTCAAGTTCACCAGCAACAGCGAC
ATCGTGGAGAAGCTGCAGAACGAGGTGAGCGAGCTGAAGAACGAGCTGGAGATGGCCAGAACCAGAGACATGAGA
AGCCCCCTGAACGGCAGCAGCGGCAGACTGAGCGACGTGCAGATCAACACCAACAGAATGTTCGAGGACCTGGAG
GTGAGCGAGGCCACCCTGCAGAAGGCCAAGGAGGAGAACAGCACCCTGAAGAGCCAGTTCGCCGAGCTGGAGGCC
AACCTGCACCAGGTGAACAGCAAGCTGGGCGAGGTGAGATGCGAGCTGAACGAGGCCCTGGCCAGAGTGGACGGC
GAGCAGGAGACCAGAGTGAAGGCCGAGAACGCCCTGGAGGAGGCCAGACAGCTGATCAGCAGCCTGAAGCACGAG
GAGAACGAGCTGAAGAAGACCATCACCGACATGGGCATGAGACTGAACGAGGCCAAGAAGAGCGACGAGTTCCTG
AAGAGCGAGCTGAGCACCGCCCTGGAGGAGGAGAAGAAGAGCCAGAACCTGGCCGACGAGCTGAGCGAGGAGCTG
AACGGCTGGAGAATGAGAACCAAGGAGGCCGAGAACAAGGTGGAGCACGCCAGCAGCGAGAAGAGCGAGATGCTG
GAGAGAATCGTGCACCTGGAGACCGAGATGGAGAAGCTGAGCACCAGCGAGATCGCCGCCGACTACTGCAGCACC
AAGATGACCGAGAGAAAGAAGGAGATCGAGCTGGCCAAGTACAGAGAGGACTTCGAGAACGCCGCCATCGTGGGC
CTGGAGAGAATCAGCAAGGAGATCAGCGAGCTGACCAAGAAGACCCTGAAGGCCAAGATCATCCCCAGCAACATC
AGCAGCATCCAGCTGGTGTGCGACGAGCTGTGCAGAAGACTGAGCAGAGAGAGAGAGCAGCAGCACGAGTACGCC
AAGGTGATGAGAGACGTGAACGAGAAGATCGAGAAGCTGCAGCTGGAGAAGGACGCCCTGGAGCACGAGCTGAAG
ATGATGAGCAGCAACAACGAGAACGTGCCCCCCGTGGGCACCAGCGTGAGCGGCATGCCCACCAAGACCAGCAAC
CAGAAGTGCGCCCAGCCCCACTACACCAGCCCCACCAGACAGCTGCTGCACGAGAGCACCATGGCCGTGGACGCC
ATCGTGCAGAAGCTGAAGAAGACCCACAACATGAGCGGCATGGGCCCCGAGCTGAAGGAGACCATCGGCAACGTG
ATCAACGAGAGCAGAGTGCTGAGAGACTTCCTGCACCAGAAGCTGATCCTGTTCAAGGGCATCGACATGAGCAAC
TGGAAGAACGAGACCGTGGACCAGCTGATCACCGACCTGGGCCAGCTGCACCAGGACAACCTGATGCTGGAGGAG
CAGATCAAGAAGTACAAGAAGGAGCTGAAGCTGACCAAGAGCGCCATCCCCACCCTGGGCGTGGAGTTCCAGGAC
AGAATCAAGACCGAGATCGGCAAGATCGCCACCGACATGGGCGGCGCCGTGAAGGAGATCAGAAAGAAG (SEQ
ID NO:31)
Protein:
MEDNSVLNEDSNLEHVEGQPRRSMSQPVLNVEGDKRTSSTSATQQQVLSGAFSSADVRSIPIIQTWEENKALKTK
ITILRGELQMYQRRYSEAKEASQKRVKEVMDDYVDLKLGQENVQEKMEQYKLMEEDLLAMQSRIETSEDNFARQM
KEFEAQKHAMEERIKELELSATDANNTTVGSFRGTLDDILKKNDPDFTLTSGYEERKINDLEAKLLSEIDKVAEL
EDHIQQLRQELDDQSARLADSENVRAQLEAATGQGILGAAGNAMVPNSTFMIGNGRESQTRDQLNYIDDLETKLA
DAKKENDKARQALVEYMNKCSKLEHEIRTMVKNSTFDSSSMLLGGQTSDELKIQIGKVNGELNVLRAENRELRIR
CDQLTGGDGNLSISLGQSRLMAGIATNDVDSIGQGNETGGTSMRILPRESQLDDLEESKLPLMDTSSAVRNQQQF

ASMWEDFESVKDSLQNNHNDTLEGSFNSSMPPPGRDATQSFLSQKSFKNSPIVMQKPKSLHLHLKSHQSEGAGEQ
IQNNSFSTKTASPHVSQSHIPILHDMQQILDSSAMFLEGQHDVAVNVEQMQEKMSQIREALARLFERLKSSAALF
EEILERMGSSDPNADKIKKMKLAFETSINDKLNVSAILEAAEKDLHNMSLNFSILEKSIVSQAAEASRRFTIAPD
AEDVASSSLLNASYSPLFKFTSNSDIVEKLQNEVSELKNELEMARTRDMRSPLNGSSGRLSDVQINTNRMFEDLE
VSEATLQKAKEENSTLKSQFAELEANLHQVNSKLGEVRCELNEALARVDGEQETRVKAENALEEARQLISSLKHE
ENELKKTITDMGMRLNEAKKSDEFLKSELSTALEEEKKSQNLADELSEELNGWRMRTKEAENKVEHASSEKSEML
ERIVHLETEMEKLSTSEIAADYCSTKMTERKKEIELAKYREDFENAAIVGLERISKEISELTKKTLKAKIIPSNI
SSIQLVCDELCRRLSREREQQHEYAKVMRDVNEKIEKLQLEKDALEHELKMMSSNNENVPPVGTSVSGMPTKTSN
QKCAQPHYTSPTRQLLHESTMAVDAIVQKLKKTHNMSGMGPELKETIGNVINESRVLRDFLHQKLILFKGIDMSN
WKNETVDQLITDLGQLHQDNLMLEEQIKKYKKELKLTKSAIPTLGVEFQDRIKTEIGKIATDMGGAVKEIRKK
(SEQ ID NO:32)

FUS (Homo sapiens fused-in sarcoma; Uniprot: P35637)
DNA:
ATGGCCTCAAACGATTATACCCAACAAGCAACCCAAAGCTATGGGGCCTACCCCACCCAGCCCGGGCAGGGCTAT
TCCCAGCAGAGCAGTCAGCCCTACGGACAGCAGAGTTACAGTGGTTATAGCCAGTCCACGGACACTTCAGGATAT
GGCCAGAGCAGCTATTCTTCTTATGGCCAGAGCCAGAACACAGGCTATGGAACTCAGTCAACTCCCCAGGGATAT
GGCTCGACTGGCGGCTATGGCAGTAGCCAGAGCTCCCAATCGTCTTACGGGCAGCAGTCCTCCTACCCTGGCTAT
GGCCAGCAGCCAGCTCCCAGCAGCACCTCGGGAAGTTACGGTAGCAGTTCTCAGAGCAGCAGCTATGGGCAGCCC
CAGAGTGGGAGCTACAGCCAGCAGCCTAGCTATGGTGGACAGCAGCAAAGCTATGGACAGCAGCAAAGCTATAAT
CCCCCTCAGGGCTATGGACAGCAGAACCAGTACAACAGCAGCAGTGGTGGTGGAGGTGGAGGTGGAGGTGGAGGT
AACTATGGCCAAGATCAATCCTCCATGAGTAGTGGTGGTGGCAGTGGTGGCGGTTATGGCAATCAAGACCAGAGT
GGTGGAGGTGGCAGCGGTGGCTATGGACAGCAGGACCGTGGAGGCCGCGGCAGGGGTGGCAGTGGTGGCGGCGGC
GGCGGCGGCGGTGGTGGTTACAACCGCAGCAGTGGTGGCTATGAACCCAGAGGTCGTGGAGGTGGCCGTGGAGGC
AGAGGTGGCATGGGCGGAAGTGACCGTGGTGGCTTCAATAAATTTGGTGGCCCTCGGGACCAAGGATCACGTCAT
GACTCCGAACAGGATAATTCAGACAACAACACCATCTTTGTGCAAGGCCTGGGTGAGAATGTTACAATTGAGTCT
GTGGCTGATTACTTCAAGCAGATTGGTATTATTAAGACAAACAAGAAAACGGGACAGCCCATGATTAATTTGTAC
ACAGACAGGGAAACTGGCAAGCTGAAGGGAGAGGCAACGGTCTCTTTTGATGACCCACCTTCAGCTAAAGCAGCT
ATTGACTGGTTTGATGGTAAAGAATTCTCCGGAAATCCTATCAAGGTCTCATTTGCTACTCGCCGGGCAGACTTT
AATCGGGGTGGTGGCAATGGTCGTGGAGGCCGAGGGCGAGGAGGACCCATGGGCCGTGGAGGCTATGGAGGTGGT
GGCAGTGGTGGTGGTGGCCGAGGAGGATTTCCCAGTGGAGGTGGTGGCGGTGGAGGACAGCAGCGAGCTGGTGAC
TGGAAGTGTCCTAATCCCACCTGTGAGAATATGAACTTCTCTTGGAGGAATGAATGCAACCAGTGTAAGGCCCCT
AAACCAGATGGCCCAGGAGGGGGACCAGGTGGCTCTCACATGGGGGGTAACTACGGGGATGATCGTCGTGGTGGC
AGAGGAGGC (SEQ ID NO:33)
Protein:
MASNDYTQQATQSYGAYPTQPGQGYSQQSSQPYGQQSYSGYSQSTDTSGYGQSSYSSYGQSQNTGYGTQSTPQGY
GSTGGYGSSQSSQSSYGQQSSYPGYGQQPAPSSTSGSYGSSSQSSSYGQPQSGSYSQQPSYGGQQQSYGQQQSYN
PPQGYGQQNQYNSSSGGGGGGGGGGNYGQDQSSMSSGGGSGGGYGNQDQSGGGGSGGYGQQDRGGRGRGGSGGGG
GGGGGGYNRSSGGYEPRGRGGGRGGRGGMGGSDRGGFNKFGGPRDQGSRHDSEQDNSDNNTIFVQGLGENVTIES
VADYFKQIGIIKTNKKTGQPMINLYTDRETGKLKGEATVSFDDPPSAKAAIDWFDGKEFSGNPIKVSFATRRADF
NRGGGNGRGGRGRGGPMGRGGYGGGGSGGGGRGGFPSGGGGGGGQQRAGDWKCPNPTCENMNFSWRNECNQCKAP
KPDGPGGGPGGSHMGGNYGDDRRGGRGG (SEQ ID NO:34)

EWSR1 (Homo sapiens Ewing sarcoma breakpoint region 1; Uniprot: Q01844)
DNA:
ATGGCGTCCACGGATTACAGTACCTATAGCCAAGCTGCAGCGCAGCAGGGCTACAGTGCTTACACCGCCCAGCCC
ACTCAAGGATATGCACAGACCACCCAGGCATATGGGCAACAAAGCTATGGAACCTATGGACAGCCCACTGATGTC
AGCTATACCCAGGCTCAGACCACTGCAACCTATGGGCAGACCGCCTATGCAACTTCTTATGGACAGCCTCCCACT
GGTTATACTACTCCAACTGCCCCCCAGGCATACAGCCAGCCTGTCCAGGGGTATGGCACTGGTGCTTATGATACC
ACCACTGCTACAGTCACCACCACCCAGGCCTCCTATGCAGCTCAGTCTGCATATGGCACTCAGCCTGCTTATCCA
GCCTATGGGCAGCAGCCAGCAGCCACTGCACCTACAAGACCGCAGGATGGAAACAAGCCCACTGAGACTAGTCAA
CCTCAATCTAGCACAGGGGGTTACAACCAGCCCAGCCTAGGATATGGACAGAGTAACTACAGTTATCCCCAGGTA
CCTGGGAGCTACCCCATGCAGCCAGTCACTGCACCTCCATCCTACCCTCCTACCAGCTATTCCTCTACACAGCCG
ACTAGTTATGATCAGAGCAGTTACTCTCAGCAGAACACCTATGGGCAACCGAGCAGCTATGGACAGCAGAGTAGC
TATGGTCAACAAAGCAGCTATGGGCAGCAGCCTCCCACTAGTTACCCACCCCAAACTGGATCCTACAGCCAAGCT
CCAAGTCAATATAGCCAACAGAGCAGCAGCTACGGGCAGCAGAGTTCATTCCGACAGGACCACCCCAGTAGCATG
GGTGTTTATGGGCAGGAGTCTGGAGGATTTTCCGGACCAGGAGAGAACCGGAGCATGAGTGGCCCTGATAACCGG
GGCAGGGGAAGAGGGGGATTTGATCGTGGAGGCATGAGCAGAGGTGGGCGGGGAGGAGGACGCGGTGGAATGGGC

AGCGCTGGAGAGCGAGGTGGCTTCAATAAGCCTGGTGGACCCATGGATGAAGGACCAGATCTTGATCTAGGCCCA
CCTGTAGATCCAGATGAAGACTCTGACAACAGTGCAATTTATGTACAAGGATTAAATGACAGTGTGACTCTAGAT
GATCTGGCAGACTTCTTTAAGCAGTGTGGGGTTGTTAAGATGAACAAGAGAACTGGGCAACCCATGATCCACATC
TACCTGGACAAGGAAACAGGAAAGCCCAAAGGCGATGCCACAGTGTCCTATGAAGACCCACCCACTGCCAAGGCT
GCCGTGGAATGGTTTGATGGGAAAGATTTTCAAGGGAGCAAACTTAAAGTCTCCCTTGCTCGGAAGAAGCCTCCA
ATGAACAGTATGCGGGGTGGTCTGCCACCCCGTGAGGGCAGAGGCATGCCACCACCACTCCGTGGAGGTCCAGGA
GGCCCAGGAGGTCCTGGGGGACCCATGGGTCGCATGGGAGGCCGTGGAGGAGATAGAGGAGGCTTCCCTCCAAGA
GGACCCCGGGGTTCCCGAGGGAACCCCTCTGGAGGAGGAAACGTCCAGCACCGAGCTGGAGACTGGCAGTGTCCC
AATCCGGGTTGTGGAAACCAGAACTTCGCCTGGAGAACAGAGTGCAACCAGTGTAAGGCCCCAAAGCCTGAAGGC
TTCCTCCCGCCACCCTTTCCGCCCCCGGGTGGTGATCGTGGCAGAGGTGGCCCTGGTGGCATGCGGGGAGGAAGA
GGTGGCCTCATGGATCGTGGTGGTCCCGGTGGAATGTTCAGAGGTGGCCGTGGTGGAGACAGAGGTGGCTTCCGT
GGTGGCCGGGGCATGGACCGAGGTGGCTTTGGTGGAGGAAGACGAGGTGGCCCTGGGGGGCCCCCTGGACCTTTG
ATGGAACAG (SEQ ID NO:35)
Protein:
MASTDYSTYSQAAAQQGYSAYTAQPTQGYAQTTQAYGQQSYGTYGQPTDVSYTQAQTTATYGQTAYATSYGQPPT
GYTTPTAPQAYSQPVQGYGTGAYDTTTATVTTTQASYAAQSAYGTQPAYPAYGQQPAATAPTRPQDGNKPTETSQ
PQSSTGGYNQPSLGYGQSNYSYPQVPGSYPMQPVTAPPSYPPTSYSSTQPTSYDQSSYSQQNTYGQPSSYGQQSS
YGQQSSYGQQPPTSYPPQTGSYSQAPSQYSQQSSSYGQQSSFRQDHPSSMGVYGQESGGFSGPGENRSMSGPDNR
GRGRGGFDRGGMSRGGRGGGRGGMGSAGERGGFNKPGGPMDEGPDLDLGPPVDPDEDSDNSAIYVQGLNDSVTLD
DLADFFKQCGVVKMNKRTGQPMIHIYLDKETGKPKGDATVSYEDPPTAKAAVEWFDGKDFQGSKLKVSLARKKPP
MNSMRGGLPPREGRGMPPPLRGGPGGPGGPGGPMGRMGGRGGDRGGFPPRGPRGSRGNPSGGGNVQHRAGDWQCP
NPGCGNQNFAWRTECNQCKAPKPEGFLPPPFPPPGGDRGRGGPGGMRGGRGGLMDRGGPGGMFRGGRGGDRGGFR
GGRGMDRGGFGGGRRGGPGGPPGPLMEQ (SEQ ID NO:36)

8. AFPs

EWSR1-MCP
DNA:
ATGGCGTCCACGGATTACAGTACCTATAGCCAAGCTGCAGCGCAGCAGGGCTACAGTGCTTACACCGCCCAGCCC
ACTCAAGGATATGCACAGACCACCCAGGCATATGGGCAACAAAGCTATGGAACCTATGGACAGCCCACTGATGTC
AGCTATACCCAGGCTCAGACCACTGCAACCTATGGGCAGACCGCCTATGCAACTTCTTATGGACAGCCTCCCACT
GGTTATACTACTCCAACTGCCCCCCAGGCATACAGCCAGCCTGTCCAGGGGTATGGCACTGGTGCTTATGATACC
ACCACTGCTACAGTCACCACCACCCAGGCCTCCTATGCAGCTCAGTCTGCATATGGCACTCAGCCTGCTTATCCA
GCCTATGGGCAGCAGCCAGCAGCCACTGCACCTACAAGACCGCAGGATGGAAACAAGCCCACTGAGACTAGTCAA
CCTCAATCTAGCACAGGGGGTTACAACCAGCCCAGCCTAGGATATGGACAGAGTAACTACAGTTATCCCCAGGTA
CCTGGGAGCTACCCCATGCAGCCAGTCACTGCACCTCCATCCTACCCTCCTACCAGCTATTCCTCTACACAGCCG
ACTAGTTATGATCAGAGCAGTTACTCTCAGCAGAACACCTATGGGCAACCGAGCAGCTATGGACAGCAGAGTAGC
TATGGTCAACAAAGCAGCTATGGGCAGCAGCCTCCCACTAGTTACCCACCCCAAACTGGATCCTACAGCCAAGCT
CCAAGTCAATATAGCCAACAGAGCAGCAGCTACGGGCAGCAGAGTTCATTCCGACAGGACCACCCCAGTAGCATG
GGTGTTTATGGGCAGGAGTCTGGAGGATTTTCCGGACCAGGAGAGAACCGGAGCATGAGTGGCCCTGATAACCGG
GGCAGGGGAAGAGGGGGATTTGATCGTGGAGGCATGAGCAGAGGTGGGCGGGGAGGAGGACGCGGTGGAATGGGC
AGCGCTGGAGAGCGAGGTGGCTTCAATAAGCCTGGTGGACCCATGGATGAAGGACCAGATCTTGATCTAGGCCCA
CCTGTAGATCCAGATGAAGACTCTGACAACAGTGCAATTTATGTACAAGGATTAAATGACAGTGTGACTCTAGAT
GATCTGGCAGACTTCTTTAAGCAGTGTGGGGTTGTTAAGATGAACAAGAGAACTGGGCAACCCATGATCCACATC
TACCTGGACAAGGAAACAGGAAAGCCCAAAGGCGATGCCACAGTGTCCTATGAAGACCCACCCACTGCCAAGGCT
GCCGTGGAATGGTTTGATGGGAAAGATTTTCAAGGGAGCAAACTTAAAGTCTCCCTTGCTCGGAAGAAGCCTCCA
ATGAACAGTATGCGGGGTGGTCTGCCACCCCGTGAGGGCAGAGGCATGCCACCACCACTCCGTGGAGGTCCAGGA
GGCCCAGGAGGTCCTGGGGGACCCATGGGTCGCATGGGAGGCCGTGGAGGAGATAGAGGAGGCTTCCCTCCAAGA
GGACCCCGGGGTTCCCGAGGGAACCCCTCTGGAGGAGGAAACGTCCAGCACCGAGCTGGAGACTGGCAGTGTCCC
AATCCGGGTTGTGGAAACCAGAACTTCGCCTGGAGAACAGAGTGCAACCAGTGTAAGGCCCCAAAGCCTGAAGGC
TTCCTCCCGCCACCCTTTCCGCCCCCGGGTGGTGATCGTGGCAGAGGTGGCCCTGGTGGCATGCGGGGAGGAAGA
GGTGGCCTCATGGATCGTGGTGGTCCCGGTGGAATGTTCAGAGGTGGCCGTGGTGGAGACAGAGGTGGCTTCCGT
GGTGGCCGGGGCATGGACCGAGGTGGCTTTGGTGGAGGAAGACGAGGTGGCCCTGGGGGGCCCCCTGGACCTTTG
ATGGAACAGGATTACAAGGATGACGACGATAAGGGTACCGAGCAGAAGCTGATCTCAGAGGAGGACCTGGGCGCC
CCCGGCTCCGCCGGCTCCGCCGCCGGCTCCGGCGCTTCTAACTTTACTCAGTTCGTTCTCGTCGACAATGGCGGA
ACTGGCGACGTGACTGTCGCCCCAAGCAACTTCGCTAACGGGATCGCTGAATGGATCAGCTCTAACTCGCGTTCA
CAGGCTTACAAAGTAACCTGTAGCGTTCGTCAGAGCTCTGCGCAGAATCGCAAATACACCATCAAAGTCGAGGTG
CCTAAAGGCGCCTGGCGTTCGTACTTAAATATGGAACTAACCATTCCAATTTTCGCCACGAATTCCGACTGCGAG

CTTATTGTTAAGGCAATGCAAGGTCTCCTAAAAGATGGAAACCCGATTCCCTCAGCAATCGCAGCAAACTCCGGC
ATCTACTAA (SEQ ID NO:37)
Protein:
MASTDYSTYSQAAAQQGYSAYTAQPTQGYAQTTQAYGQQSYGTYGQPTDVSYTQAQTTATYGQTAYATSYGQPPT
GYTTPTAPQAYSQPVQGYGTGAYDTTTATVTTTQASYAAQSAYGTQPAYPAYGQQPAATAPTRPQDGNKPTETSQ
PQSSTGGYNQPSLGYGQSNYSYPQVPGSYPMQPVTAPPSYPPTSYSSTQPTSYDQSSYSQQNTYGQPSSYGQQSS
YGQQSSYGQQPPTSYPPQTGSYSQAPSQYSQQSSSYGQQSSFRQDHPSSMGVYGQESGGFSGPGENRSMSGPDNR
GRGRGGFDRGGMSRGGRGGGRGGMGSAGERGGFNKPGGPMDEGPDLDLGPPVDPDEDSDNSAIYVQGLNDSVTLD
DLADFFKQCGVVKMNKRTGQPMIHIYLDKETGKPKGDATVSYEDPPTAKAAVEWFDGKDFQGSKLKVSLARKKPP
MNSMRGGLPPREGRGMPPPLRGGPGGPGGPGGPMGRMGGRGGDRGGFPPRGPRGSRGNPSGGGNVQHRAGDWQCP
NPGCGNQNFAWRTECNQCKAPKPEGFLPPPFPPPGGDRGRGGPGGMRGGRGGLMDRGGPGGMFRGGRGGDRGGFR
GGRGMDRGGFGGGRRGGPGGPPGPLMEQDYKDDDDKGTEQKLISEEDLGAPGSAGSAAGSGASNFTQFVLVDNGG
TGDVTVAPSNFANGIAEWISSNSRSQAYKVTCSVRQSSAQNRKYTIKVEVPKGAWRSYLNMELTIPIFATNSDCE
LIVKAMQGLLKDGNPIPSAIAANSGIY (SEQ ID NO:38)

FUS-MCP
DNA:
ATGGCCTCAAACGATTATACCCAACAAGCAACCCAAAGCTATGGGGCCTACCCCACCCAGCCCGGGCAGGGCTAT
TCCCAGCAGAGCAGTCAGCCCTACGGACAGCAGAGTTACAGTGGTTATAGCCAGTCCACGGACACTTCAGGATAT
GGCCAGAGCAGCTATTCTTCTTATGGCCAGAGCCAGAACACAGGCTATGGAACTCAGTCAACTCCCCAGGGATAT
GGCTCGACTGGCGGCTATGGCAGTAGCCAGAGCTCCCAATCGTCTTACGGGCAGCAGTCCTCCTACCCTGGCTAT
GGCCAGCAGCCAGCTCCCAGCAGCACCTCGGGAAGTTACGGTAGCAGTTCTCAGAGCAGCAGCTATGGGCAGCCC
CAGAGTGGGAGCTACAGCCAGCAGCCTAGCTATGGTGGACAGCAGCAAAGCTATGGACAGCAGCAAAGCTATAAT
CCCCCTCAGGGCTATGGACAGCAGAACCAGTACAACAGCAGCAGTGGTGGTGGAGGTGGAGGTGGAGGTGGAGGT
AACTATGGCCAAGATCAATCCTCCATGAGTAGTGGTGGTGGCAGTGGTGGCGGTTATGGCAATCAAGACCAGAGT
GGTGGAGGTGGCAGCGGTGGCTATGGACAGCAGGACCGTGGAGGCCGCGGCAGGGGTGGCAGTGGTGGCGGCGGC
GGCGGCGGCGGTGGTGGTTACAACCGCAGCAGTGGTGGCTATGAACCCAGAGGTCGTGGAGGTGGCCGTGGAGGC
AGAGGTGGCATGGGCGGAAGTGACCGTGGTGGCTTCAATAAATTTGGTGGCCCTCGGGACCAAGGATCACGTCAT
GACTCCGAACAGGATAATTCAGACAACAACACCATCTTTGTGCAAGGCCTGGGTGAGAATGTTACAATTGAGTCT
GTGGCTGATTACTTCAAGCAGATTGGTATTATTAAGACAAACAAGAAAACGGGACAGCCCATGATTAATTTGTAC
ACAGACAGGGAAACTGGCAAGCTGAAGGGAGAGGCAACGGTCTCTTTTGATGACCCACCTTCAGCTAAAGCAGCT
ATTGACTGGTTTGATGGTAAAGAATTCTCCGGAAATCCTATCAAGGTCTCATTTGCTACTCGCCGGGCAGACTTT
AATCGGGGTGGTGGCAATGGTCGTGGAGGCCGAGGGCGAGGAGGACCCATGGGCCGTGGAGGCTATGGAGGTGGT
GGCAGTGGTGGTGGTGGCCGAGGAGGATTTCCCAGTGGAGGTGGTGGCGGTGGAGGACAGCAGCGAGCTGGTGAC
TGGAAGTGTCCTAATCCCACCTGTGAGAATATGAACTTCTCTTGGAGGAATGAATGCAACCAGTGTAAGGCCCCT
AAACCAGATGGCCCAGGAGGGGGACCAGGTGGCTCTCACATGGGGGGTAACTACGGGGATGATCGTCGTGGTGGC
AGAGGAGGCGATTACAAGGATGACGACGATAAGGGTACCGAGCAGAAGCTGATCTCAGAGGAGGACCTGGGCGCC
CCCGGCTCCGCCGGCTCCGCCGCCGGCTCCGGCGCTTCTAACTTTACTCAGTTCGTTCTCGTCGACAATGGCGGA
ACTGGCGACGTGACTGTCGCCCCAAGCAACTTCGCTAACGGGATCGCTGAATGGATCAGCTCTAACTCGCGTTCA
CAGGCTTACAAAGTAACCTGTAGCGTTCGTCAGAGCTCTGCGCAGAATCGCAAATACACCATCAAAGTCGAGGTG
CCTAAAGGCGCCTGGCGTTCGTACTTAAATATGGAACTAACCATTCCAATTTTCGCCACGAATTCCGACTGCGAG
CTTATTGTTAAGGCAATGCAAGGTCTCCTAAAAGATGGAAACCCGATTCCCTCAGCAATCGCAGCAAACTCCGGC
ATCTACTAA (SEQ ID NO:39)
Protein:
MASNDYTQQATQSYGAYPTQPGQGYSQQSSQPYGQQSYSGYSQSTDTSGYGQSSYSSYGQSQNTGYGTQSTPQGY
GSTGGYGSSQSSQSSYGQQSSYPGYGQQPAPSSTSGSYGSSSQSSSYGQPQSGSYSQQPSYGGQQQSYGQQQSYN
PPQGYGQQNQYNSSSGGGGGGGGGNYGQDQSSMSSGGGSGGGYGNQDQSGGGGSGGYGQQDRGGRGRGGSGGGG
GGGGGGYNRSSGGYEPRGRGGGRGGRGGMGGSDRGGFNKFGGPRDQGSRHDSEQDNSDNNTIFVQGLGENVTIES
VADYFKQIGIIKTNKKTGQPMINLYTDRETGKLKGEATVSFDDPPSAKAAIDWFDGKEFSGNPIKVSFATRRADF
NRGGGNGRGGRGRGGPMGRGGYGGGGSGGGGRGGFPSGGGGGGGQQRAGDWKCPNPTCENMNFSWRNECNQCKAP
KPDGPGGGPGGSHMGGNYGDDRRGGRGGDYKDDDDKGTEQKLISEEDLGAPGSAGSAAGSGASNFTQFVLVDNGG
TGDVTVAPSNFANGIAEWISSNSRSQAYKVTCSVRQSSAQNRKYTIKVEVPKGAWRSYLNMELTIPIFATNSDCE
LIVKAMQGLLKDGNPIPSAIAANSGIY (SEQ ID NO:40)

FUS-PylRS^{AF}
DNA:
ATGGCCTCAAACGATTATACCCAACAAGCAACCCAAAGCTATGGGGCCTACCCCACCCAGCCCGGGCAGGGCTAT
TCCCAGCAGAGCAGTCAGCCCTACGGACAGCAGAGTTACAGTGGTTATAGCCAGTCCACGGACACTTCAGGATAT

GGCCAGAGCAGCTATTCTTCTTATGGCCAGAGCCAGAACACAGGCTATGGAACTCAGTCAACTCCCCAGGGATAT
GGCTCGACTGGCGGCTATGGCAGTAGCCAGAGCTCCCAATCGTCTTACGGGCAGCAGTCCTCCTACCCTGGCTAT
GGCCAGCAGCCAGCTCCCAGCAGCACCTCGGGAAGTTACGGTAGCAGTTCTCAGAGCAGCAGCTATGGGCAGCCC
CAGAGTGGGAGCTACAGCCAGCAGCCTAGCTATGGTGGACAGCAGCAAAGCTATGGACAGCAGCAAAGCTATAAT
CCCCCTCAGGGCTATGGACAGCAGAACCAGTACAACAGCAGCAGTGGTGGTGGAGGTGGAGGTGGAGGTGGAGGT
AACTATGGCCAAGATCAATCCTCCATGAGTAGTGGTGGTGGCAGTGGTGGCGGTTATGGCAATCAAGACCAGAGT
GGTGGAGGTGGCAGCGGTGGCTATGGACAGCAGGACCGTGGAGGCCGCGGCAGGGGTGGCAGTGGTGGCGGCGGC
GGCGGCGGCGGTGGTGGTTACAACCGCAGCAGTGGTGGCTATGAACCCAGAGGTCGTGGAGGTGGCCGTGGAGGC
AGAGGTGGCATGGGCGGAAGTGACCGTGGTGGCTTCAATAAATTTGGTGGCCCTCGGGACCAAGGATCACGTCAT
GACTCCGAACAGGATAATTCAGACAACAACACCATCTTTGTGCAAGGCCTGGGTGAGAATGTTACAATTGAGTCT
GTGGCTGATTACTTCAAGCAGATTGGTATTATTAAGACAAACAAGAAAACGGGACAGCCCATGATTAATTTGTAC
ACAGACAGGGAAACTGGCAAGCTGAAGGGAGAGGCAACGGTCTCTTTTGATGACCCACCTTCAGCTAAAGCAGCT
ATTGACTGGTTTGATGGTAAAGAATTCTCCGGAAATCCTATCAAGGTCTCATTTGCTACTCGCCGGGCAGACTTT
AATCGGGGTGGTGGCAATGGTCGTGGAGGCCGAGGGCGAGGAGGACCCATGGGCCGTGGAGGCTATGGAGGTGGT
GGCAGTGGTGGTGGTGGCCGAGGAGGATTTCCCAGTGGAGGTGGTGGCGGTGGAGGACAGCAGCGAGCTGGTGAC
TGGAAGTGTCCTAATCCCACCTGTGAGAATATGAACTTCTCTTGGAGGAATGAATGCAACCAGTGTAAGGCCCCT
AAACCAGATGGCCCAGGAGGGGGACCAGGTGGCTCTCACATGGGGGGTAACTACGGGGATGATCGTCGTGGTGGC
AGAGGAGGCGATTACAAGGATGACGACGATAAGGGTACCGGCGCCCCCGGCTCCGCCGGCTCCGCCGCCGGCTCC
GGCGCTTCTAACTTTACTCAGTTCGTTCTCGTCGACAATGGCGGAACTGGCGACGTGACTGTCGCCCCAAGCAAC
TTCGCTAACGGGATCGCTGAATGGATCAGCTCTAACTCGCGTTCACAGGCTTACAAAGTAACCTGTAGCGTTCGT
CAGAGCTCTGCGCAGAATCGCAAATACACCATCAAAGTCGAGGTGCCTAAAGGCGCCTGGCGTTCGTACTTAAAT
ATGGAACTAACCATTCCAATTTTCGCCACGAATTCCGACTGCGAGCTTATTGTTAAGGCAATGCAAGGTCTCCTA
AAAGATGGAAACCCGATTCCCTCAGCAATCGCAGCAAACTCCGGCATCTACGGTACCGGCGCCCCCGGCTCCGCC
GGCTCCGCCGCCGGCTCCGGCGCGTGCCCGGTGCCGCTGCAGCTGCCGCCGCTGGAACGCCTGACCCTGGATGAT
AAAAAACCGCTGAATACCCTGATCTCTGCTACTGGTCTGTGGATGAGTCGTACCGGAACCATTCATAAAATCAAA
CACCACGAGGTTAGCCGTTCGAAAATCTATATTGAGATGGCGTGTGGCGATCATCTGGTTGTGAACAATAGCCGC
TCTTCTCGTACAGCACGTGCACTGCGTCACCACAAATATCGTAAAACCTGTAAACGTTGCCGTGTGTCCGATGAG
GATCTGAACAAATTCCTGACAAAAGCCAATGAGGACCAAACAAGCGTGAAAGTGAAAGTCGTTAGCGCTCCTACC
CGTACTAAAAAAGCAATGCCGAAATCCGTTGCTCGTGCCCCTAAACCACTGGAAAACACTGAAGCAGCACAGGCA
CAGCCGTCTGGAAGCAAATTCTCTCCGGCCATTCCTGTTTCTACCCAGGAGTCCGTTTCTGTTCCAGCAAGTGTG
AGCACCAGCATTAGCAGTATTAGCACCGGTGCCACCGCTAGCGCCCTGGTTAAAGGCAATACCAATCCGATTACA
AGCATGTCTGCCCCGGTTCAAGCATCAGCTCCAGCACTGACAAAATCCCAAACCGATCGTCTGGAGGTTCTGCTG
AATCCGAAAGACGAAATCAGCCTGAATTCCGGCAAACCGTTTCGTGAACTGGAGAGCGAACTGCTGTCACGTCGT
AAAAAAGACCTGCAACAAATCTATGCCGAAGAACGTGAGAACTATCTGGGGAAACTGGAACGTGAAATCACCCGC
TTTTTCGTGGATCGTGGCTTTCTGGAGATCAAATCCCCGATTCTGATTCCTCTGGAGTATATCGAGCGTATGGGC
ATCGACAATGATACCGAACTGAGCAAACAAATTTTCCGTGTGGATAAAAACTTCTGTCTGCGCCCTATGCTAGCA
CCAAATCTGGCTAACTATCTGCGCAAACTGGACCGTGCCCTGCCTGATCCTATCAAAATCTTCGAGATCGGCCCG
TGTTATCGTAAAGAGTCCGACGGTAAAGAACATCTGGAGGAGTTTACCATGCTGAACTTTTGCCAAATGGGTTCA
GGTTGTACTCGTGAGAACCTGGAAAGCATCATCACCGATTTTCTGAACCACCTGGGCATTGACTTCAAAATTGTG
GGCGACAGCTGTATGGTGTTTGGCGACACCCTGGATGTCATGCACGGCGACCTGGAACTGTCTAGTGCCGTTGTG
GGCCCAATCCCGCTGGATCGTGAGTGGGGTATCGACAAACCTTGGATCGGTGCGGGTTTTGGTCTGGAGCGTCTG
CTGAAAGTAAAACACGACTTCAAGAACATCAAACGTGCTGCACGTTCCGAGTCCTATTACAATGGTATTTCTACT
AACCTGTAA (SEQ ID NO:41)
Protein:
MASNDYTQQATQSYGAYPTQPGQGYSQQSSQPYGQQSYSGYSQSTDTSGYGQSSYSSYGQSQNTGYGTQSTPQGY
GSTGGYGSSQSSQSSYGQQSSYPGYGQQPAPSSTSGSYGSSSQSSSYGQPQSGSYSQQPSYGGQQQSYGQQQSYN
PPQGYGQQNQYNSSSGGGGGGGGGGNYGQDQSSMSSGGGSGGGYGNQDQSGGGGSGGYGQQDRGGRGRGGSGGGG
GGGGGGYNRSSGGYEPRGRGGGRGGRGGMGGSDRGGFNKFGGPRDQGSRHDSEQDNSDNNTIFVQGLGENVTIES
VADYFKQIGIIKTNKKTGQPMINLYTDRETGKLKGEATVSFDDPPSAKAAIDWFDGKEFSGNPIKVSFATRRADF
NRGGGNGRGGRGRGGPMGRGGYGGGGSGGGGRGGFPSGGGGGGGQQRAGDWKCPNPTCENMNFSWRNECNQCKAP
KPDGPGGGPGGSHMGGNYGDDRRGGRGGDYKDDDDKGTGAPGSAGSAAGSGASNFTQFVLVDNGGTGDVTVAPSN
FANGIAEWISSNSRSQAYKVTCSVRQSSAQNRKYTIKVEVPKGAWRSYLNMELTIPIFATNSDCELIVKAMQGLL
KDGNPIPSAIAANSGIYGTGAPGSAGSAAGSGACPVPLQLPPLERLTLDDKKPLNTLISATGLWMSRTGTIHKIK
HHEVSRSKIYIEMACGDHLVVNNSRSSRTARALRHHKYRKTCKRCRVSDEDLNKFLTKANEDQTSVKVKVVSAPT
RTKKAMPKSVARAPKPLENTEAAQAQPSGSKFSPAIPVSTQESVSVPASVSTSISSISTGATASALVKGNTNPIT
SMSAPVQASAPALTKSQTDRLEVLLNPKDEISLNSGKPFRELESELLSRRKKDLQQIYAEERENYLGKLEREITR
FFVDRGFLEIKSPILIPLEYIERMGIDNDTELSKQIFRVDKNFCLRPMLAPNLANYLRKLDRALPDPIKIFEIGP

CYRKESDGKEHLEEFTMLNFCQMGSGCTRENLESIITDFLNHLGIDFKIVGDSCMVFGDTLDVMHGDLELSSAVV
GPIPLDREWGIDKPWIGAGFGLERLLKVKHDFKNIKRAARSESYYNGISTNL (SEQ ID NO:42)

MCP-PylRS^AF
DNA:
ATGGCTTCTAACTTTACTCAGTTCGTTCTCGTCGACAATGGCGGAACTGGCGACGTGACTGTCGCCCCAAGCAAC
TTCGCTAACGGGATCGCTGAATGGATCAGCTCTAACTCGCGTTCACAGGCTTACAAAGTAACCTGTAGCGTTCGT
CAGAGCTCTGCGCAGAATCGCAAATACACCATCAAAGTCGAGGTGCCTAAAGGCGCCTGGCGTTCGTACTTAAAT
ATGGAACTAACCATTCCAATTTTCGCCACGAATTCCGACTGCGAGCTTATTGTTAAGGCAATGCAAGGTCTCCTA
AAAGATGGAAACCCGATTCCCTCAGCAATCGCAGCAAACTCCGGCATCTACGGTACCGGCGCCCCCGGCTCCGCC
GGCTCCGCCGCCGGCTCCGGCGCGTGCCCGGTGCCGCTGCAGCTGCCGCCGCTGGAACGCCTGACCCTGGATGAT
AAAAAACCGCTGAATACCCTGATCTCTGCTACTGGTCTGTGGATGAGTCGTACCGGAACCATTCATAAAATCAAA
CACCACGAGGTTAGCCGTTCGAAAATCTATATTGAGATGGCGTGTGGCGATCATCTGGTTGTGAACAATAGCCGC
TCTTCTCGTACAGCACGTGCACTGCGTCACCACAAATATCGTAAAACCTGTAAACGTTGCCGTGTGTCCGATGAG
GATCTGAACAAATTCCTGACAAAAGCCAATGAGGACCAAACAAGCGTGAAAGTGAAAGTCGTTAGCGCTCCTACC
CGTACTAAAAAAGCAATGCCGAAATCCGTTGCTCGTGCCCCTAAACCACTGGAAAACACTGAAGCAGCACAGGCA
CAGCCGTCTGGAAGCAAATTCTCTCCGGCCATTCCTGTTTCTACCCAGGAGTCCGTTTCTGTTCCAGCAAGTGTG
AGCACCAGCATTAGCAGTATTAGCACCGGTGCCACCGCTAGCGCCCTGGTTAAAGGCAATACCAATCCGATTACA
AGCATGTCTGCCCCGGTTCAAGCATCAGCTCCAGCACTGACAAAATCCCAAACCGATCGTCTGGAGGTTCTGCTG
AATCCGAAAGACGAAATCAGCCTGAATTCCGGCAAACCGTTTCGTGAACTGGAGAGCGAACTGCTGTCACGTCGT
AAAAAAGACCTGCAACAAATCTATGCCGAAGAACGTGAGAACTATCTGGGGAAACTGGAACGTGAAATCACCCGC
TTTTTCGTGGATCGTGGCTTTCTGGAGATCAAATCCCCGATTCTGATTCCTCTGGAGTATATCGAGCGTATGGGC
ATCGACAATGATACCGAACTGAGCAAACAAATTTTCCGTGTGGATAAAAACTTCTGTCTGCGCCCTATGCTAGCA
CCAAATCTGGCTAACTATCTGCGCAAACTGGACCGTGCCCTGCCTGATCCTATCAAAATCTTCGAGATCGGCCCG
TGTTATCGTAAAGAGTCCGACGGTAAAGAACATCTGGAGGAGTTTACCATGCTGAACTTTTGCCAAATGGGTTCA
GGTTGTACTCGTGAGAACCTGGAAAGCATCATCACCGATTTTCTGAACCACCTGGGCATTGACTTCAAAATTGTG
GGCGACAGCTGTATGGTGTTTGGCGACACCCTGGATGTCATGCACGGCGACCTGGAACTGTCTAGTGCCGTTGTG
GGCCCAATCCCGCTGGATCGTGAGTGGGGTATCGACAAACCTTGGATCGGTGCGGGTTTTGGTCTGGAGCGTCTG
CTGAAAGTAAAACACGACTTCAAGAACATCAAACGTGCTGCACGTTCCGAGTCCTATTACAATGGTATTTCTACT
AACCTGTAA (SEQ ID NO:43)
Protein:
MASNFTQFVLVDNGGTGDVTVAPSNFANGIAEWISSNSRSQAYKVTCSVRQSSAQNRKYTIKVEVPKGAWRSYLN
MELTIPIFATNSDCELIVKAMQGLLKDGNPIPSAIAANSGIYGTGAPGSAGSAAGSGACPVPLQLPPLERLTLDD
KKPLNTLISATGLWMSRTGTIHKIKHHEVSRSKIYIEMACGDHLVVNNSRSSRTARALRHHKYRKTCKRCRVSDE
DLNKFLTKANEDQTSVKVKVVSAPTRTKKAMPKSVARAPKPLENTEAAQAQPSGSKFSPAIPVSTQESVSVPASV
STSISSISTGATASALVKGNTNPITSMSAPVQASAPALTKSQTDRLEVLLNPKDEISLNSGKPFRELESELLSRR
KKDLQQIYAEERENYLGKLEREITRFFVDRGFLEIKSPILIPLEYIERMGIDNDTELSKQIFRVDKNFCLRPMLA
PNLANYLRKLDRALPDPIKIFEIGPCYRKESDGKEHLEEFTMLNFCQMGSGCTRENLESIITDFLNHLGIDFKIV
GDSCMVFGDTLDVMHGDLELSSAVVGPIPLDREWGIDKPWIGAGFGLERLLKVKHDFKNIKRAARSESYYNGIST
NL (SEQ ID NO:44)

SPD5-MCP
DNA:
ATGGAGGACAACAGCGTGCTGAACGAGGACAGCAACCTGGAGCACGTGGAGGGCCAGCCCAGAAGAAGCATGAGC
CAGCCCGTGCTGAACGTGGAGGGCGACAAGAGAACCAGCAGCACCAGCGCCACCCAGCAGCAGGTGCTGAGCGGC
GCCTTCAGCAGCGCCGACGTGAGAAGCATCCCCATCATCCAGACCTGGGAGGAGAACAAGGCCCTGAAGACCAAG
ATCACCATCCTGAGAGGCGAGCTGCAGATGTACCAGAGAAGATACAGCGAGGCCAAGGAGGCCAGCCAGAAGAGA
GTGAAGGAGGTGATGGACGACTACGTGGACCTGAAGCTGGGCCAGGAGAACGTGCAGGAGAAGATGGAGCAGTAC
AAGCTGATGGAGGAGGACCTGCTGGCCATGCAGAGCAGAATCGAGACCAGCGAGGACAACTTCGCCAGACAGATG
AAGGAGTTCGAGGCCCAGAAGCACGCCATGGAGGAGAGAATCAAGGAGCTGGAGCTGAGCGCCACCGACGCCAAC
AACACCACCGTGGGCAGCTTCAGAGGCACCCTGGACGACATCCTGAAGAAGAACGACCCCGACTTCACCCTGACC
AGCGGCTACGAGGAGAGAAAGATCAACGACCTGGAGGCCAAGCTGCTGAGCGAGATCGACAAGGTGGCCGAGCTG
GAGGACCACATCCAGCAGCTGAGACAGGAGCTGGACGACCAGAGCGCCAGACTGGCCGACAGCGAGAACGTGAGA
GCCCAGCTGGAGGCCGCCACCGGCCAGGGCATCCTGGGCGCCGCCGGCAACGCCATGGTGCCCAACAGCACCTTC
ATGATCGGCAACGGCAGAGAGAGCCAGACCAGAGACCAGCTGAACTACATCGACGACCTGGAGACCAAGCTGGCC
GACGCCAAGAAGGAGAACGACAAGGCCAGACAGGCCCTGGTGGAGTACATGAACAAGTGCAGCAAGCTGGAGCAC
GAGATCAGAACCATGGTGAAGAACAGCACCTTCGACAGCAGCAGCATGCTGCTGGGCGGCCAGACCAGCGACGAG
CTGAAGATCCAGATCGGCAAGGTGAACGGCGAGCTGAACGTGCTGAGAGCCGAGAACAGAGAGCTGAGAATCAGA

TGCGACCAGCTGACCGGCGGCGACGGCAACCTGAGCATCAGCCTGGGCCAGAGCAGACTGATGGCCGGCATCGCC
ACCAACGACGTGGACAGCATCGGCCAGGGCAACGAGACCGGCGGCACCAGCATGAGAATCCTGCCCAGAGAGAGC
CAGCTGGACGACCTGGAGGAGAGCAAGCTGCCCCTGATGGACACCAGCAGCGCCGTGAGAAACCAGCAGCAGTTC
GCCAGCATGTGGGAGGACTTCGAGAGCGTGAAGGACAGCCTGCAGAACAACCACAACGACACCCTGGAGGGCAGC
TTCAACAGCAGCATGCCCCCCCCCGGCAGAGACGCCACCCAGAGCTTCCTGAGCCAGAAGAGCTTCAAGAACAGC
CCCATCGTGATGCAGAAGCCCAAGAGCCTGCACCTGCACCTGAAGAGCCACCAGAGCGAGGGCGCCGGCGAGCAG
ATCCAGAACAACAGCTTCAGCACCAAGACCGCCAGCCCCCACGTGAGCCAGAGCCACATCCCCATCCTGCACGAC
ATGCAGCAGATCCTGGACAGCAGCGCCATGTTCCTGGAGGGCCAGCACGACGTGGCCGTGAACGTGGAGCAGATG
CAGGAGAAGATGAGCCAGATCAGAGAGGCCCTGGCCAGACTGTTCGAGAGACTGAAGAGCAGCGCCGCCCTGTTC
GAGGAGATCCTGGAGAGAATGGGCAGCAGCGACCCCAACGCCGACAAGATCAAGAAGATGAAGCTGGCCTTCGAG
ACCAGCATCAACGACAAGCTGAACGTGAGCGCCATCCTGGAGGCCGCCGAGAAGGACCTGCACAACATGAGCCTG
AACTTCAGCATCCTGGAGAAGAGCATCGTGAGCCAGGCCGCCGAGGCCAGCAGAAGATTCACCATCGCCCCCGAC
GCCGAGGACGTGGCCAGCAGCAGCCTGCTGAACGCCAGCTACAGCCCCCTGTTCAAGTTCACCAGCAACAGCGAC
ATCGTGGAGAAGCTGCAGAACGAGGTGAGCGAGCTGAAGAACGAGCTGGAGATGGCCAGAACCAGAGACATGAGA
AGCCCCCTGAACGGCAGCAGCGGCAGACTGAGCGACGTGCAGATCAACACCAACAGAATGTTCGAGGACCTGGAG
GTGAGCGAGGCCACCCTGCAGAAGGCCAAGGAGGAGAACAGCACCCTGAAGAGCCAGTTCGCCGAGCTGGAGGCC
AACCTGCACCAGGTGAACAGCAAGCTGGGCGAGGTGAGATGCGAGCTGAACGAGGCCCTGGCCAGAGTGGACGGC
GAGCAGGAGACCAGAGTGAAGGCCGAGAACGCCCTGGAGGAGGCCAGACAGCTGATCAGCAGCCTGAAGCACGAG
GAGAACGAGCTGAAGAAGACCATCACCGACATGGGCATGAGACTGAACGAGGCCAAGAAGAGCGACGAGTTCCTG
AAGAGCGAGCTGAGCACCGCCCTGGAGGAGGAGAAGAAGAGCCAGAACCTGGCCGACGAGCTGAGCGAGGAGCTG
AACGGCTGGAGAATGAGAACCAAGGAGGCCGAGAACAAGGTGGAGCACGCCAGCAGCGAGAAGAGCGAGATGCTG
GAGAGAATCGTGCACCTGGAGACCGAGATGGAGAAGCTGAGCACCAGCGAGATCGCCGCCGACTACTGCAGCACC
AAGATGACCGAGAGAAAGAAGGAGATCGAGCTGGCCAAGTACAGAGAGGACTTCGAGAACGCCGCCATCGTGGGC
CTGGAGAGAATCAGCAAGGAGATCAGCGAGCTGACCAAGAAGACCCTGAAGGCCAAGATCATCCCCAGCAACATC
AGCAGCATCCAGCTGGTGTGCGACGAGCTGTGCAGAAGACTGAGCAGAGAGAGAGAGCAGCAGCACGAGTACGCC
AAGGTGATGAGAGACGTGAACGAGAAGATCGAGAAGCTGCAGCTGGAGAAGGACGCCCTGGAGCACGAGCTGAAG
ATGATGAGCAGCAACAACGAGAACGTGCCCCCCGTGGGCACCAGCGTGAGCGGCATGCCCACCAAGACCAGCAAC
CAGAAGTGCGCCCAGCCCCACTACACCAGCCCCACCAGACAGCTGCTGCACGAGAGCACCATGGCCGTGGACGCC
ATCGTGCAGAAGCTGAAGAAGACCCACAACATGAGCGGCATGGGCCCCGAGCTGAAGGAGACCATCGGCAACGTG
ATCAACGAGAGCAGAGTGCTGAGAGACTTCCTGCACCAGAAGCTGATCCTGTTCAAGGGCATCGACATGAGCAAC
TGGAAGAACGAGACCGTGGACCAGCTGATCACCGACCTGGGCCAGCTGCACCAGGACAACCTGATGCTGGAGGAG
CAGATCAAGAAGTACAAGAAGGAGCTGAAGCTGACCAAGAGCGCCATCCCCACCCTGGGCGTGGAGTTCCAGGAC
AGAATCAAGACCGAGATCGGCAAGATCGCCACCGACATGGGCGGCGCCGTGAAGGAGATCAGAAAGAAGGGTACC
GAGCAGAAGCTGATCTCAGAGGAGGACCTGGGCGCCCCCGGCTCCGCCGGCTCCGCCGCCGGCTCCGGCGCTTCT
AACTTTACTCAGTTCGTTCTCGTCGACAATGGCGGAACTGGCGACGTGACTGTCGCCCCAAGCAACTTCGCTAAC
GGGATCGCTGAATGGATCAGCTCTAACTCGCGTTCACAGGCTTACAAAGTAACCTGTAGCGTTCGTCAGAGCTCT
GCGCAGAATCGCAAATACACCATCAAAGTCGAGGTGCCTAAAGGCGCCTGGCGTTCGTACTTAAATATGGAACTA
ACCATTCCAATTTTCGCCACGAATTCCGACTGCGAGCTTATTGTTAAGGCAATGCAAGGTCTCCTAAAAGATGGA
AACCCGATTCCCTCAGCAATCGCAGCAAACTCCGGCATCTACTAA (SEQ ID NO:45)
Protein:
MEDNSVLNEDSNLEHVEGQPRRSMSQPVLNVEGDKRTSSTSATQQQVLSGAFSSADVRSIPIIQTWEENKALKTK
ITILRGELQMYQRRYSEAKEASQKRVKEVMDDYVDLKLGQENVQEKMEQYKLMEEDLLAMQSRIETSEDNFARQM
KEFEAQKHAMEERIKELELSATDANNTTVGSFRGTLDDILKKNDPDFTLTSGYEERKINDLEAKLLSEIDKVAEL
EDHIQQLRQELDDQSARLADSENVRAQLEAATGQGILGAAGNAMVPNSTFMIGNGRESQTRDQLNYIDDLETKLA
DAKKENDKARQALVEYMNKCSKLEHEIRTMVKNSTFDSSSMLLGGQTSDELKIQIGKVNGELNVLRAENRELRIR
CDQLTGGDGNLSISLGQSRLMAGIATNDVDSIGQGNETGGTSMRILPRESQLDDLEESKLPLMDTSSAVRNQQQF
ASMWEDFESVKDSLQNNHNDTLEGSFNSSMPPPGRDATQSFLSQKSFKNSPIVMQKPKSLHLHLKSHQSEGAGEQ
IQNNSFSTKTASPHVSQSHIPILHDMQQILDSSAMFLEGQHDVAVNVEQMQEKMSQIREALARLFERLKSSAALF
EEILERMGSSDPNADKIKKMKLAFETSINDKLNVSAILEAAEKDLHNMSLNFSILEKSIVSQAAEASRRFTIAPD
AEDVASSSLLNASYSPLFKFTSNSDIVEKLQNEVSELKNELEMARTRDMRSPLNGSSGRLSDVQINTNRMFEDLE
VSEATLQKAKEENSTLKSQFAELEANLHQVNSKLGEVRCELNEALARVDGEQETRVKAENALEEARQLISSLKHE
ENELKKTITDMGMRLNEAKKSDEFLKSELSTALEEEKKSQNLADELSEELNGWRMRTKEAENKVEHASSEKSEML
ERIVHLETEMEKLSTSEIAADYCSTKMTERKKEIELAKYREDFENAAIVGLERISKEISELTKKTLKAKIIPSNI
SSIQLVCDELCRRLSREREQQHEYAKVMRDVNEKIEKLQLEKDALEHELKMMSSNNENVPPVGTSVSGMPTKTSN
QKCAQPHYTSPTRQLLHESTMAVDAIVQKLKKTHNMSGMGPELKETIGNVINESRVLRDFLHQKLILFKGIDMSN
WKNETVDQLITDLGQLHQDNLMLEEQIKKYKKELKLTKSAIPTLGVEFQDRIKTEIGKIATDMGGAVKEIRKKGT
EQKLISEEDLGAPGSAGSAAGSGASNFTQFVLVDNGGTGDVTVAPSNFANGIAEWISSNSRSQAYKVTCSVRQSS

AQNRKYTIKVEVPKGAWRSYLNMELTIPIFATNSDCELIVKAMQGLLKDGNPIPSAIAANSGIY (SEQ ID NO:46)

SPD5-PylRS^AF
DNA:
ATGGAGGACAACAGCGTGCTGAACGAGGACAGCAACCTGGAGCACGTGGAGGGCCAGCCCAGAAGAAGCATGAGC
CAGCCCGTGCTGAACGTGGAGGGCGACAAGAGAACCAGCAGCACCAGCGCCACCCAGCAGCAGGTGCTGAGCGGC
GCCTTCAGCAGCGCCGACGTGAGAAGCATCCCCATCATCCAGACCTGGGAGGAGAACAAGGCCCTGAAGACCAAG
ATCACCATCCTGAGAGGCGAGCTGCAGATGTACCAGAGAAGATACAGCGAGGCCAAGGAGGCCAGCCAGAAGAGA
GTGAAGGAGGTGATGGACGACTACGTGGACCTGAAGCTGGGCCAGGAGAACGTGCAGGAGAAGATGGAGCAGTAC
AAGCTGATGGAGGAGGACCTGCTGGCCATGCAGAGCAGAATCGAGACCAGCGAGGACAACTTCGCCAGACAGATG
AAGGAGTTCGAGGCCCAGAAGCACGCCATGGAGGAGAGAATCAAGGAGCTGGAGCTGAGCGCCACCGACGCCAAC
AACACCACCGTGGGCAGCTTCAGAGGCACCCTGGACGACATCCTGAAGAAGAACGACCCCGACTTCACCCTGACC
AGCGGCTACGAGGAGAGAAAGATCAACGACCTGGAGGCCAAGCTGCTGAGCGAGATCGACAAGGTGGCCGAGCTG
GAGGACCACATCCAGCAGCTGAGACAGGAGCTGGACGACCAGAGCGCCAGACTGGCCGACAGCGAGAACGTGAGA
GCCCAGCTGGAGGCCGCCACCGGCCAGGGCATCCTGGGCGCCGCCGGCAACGCCATGGTGCCCAACAGCACCTTC
ATGATCGGCAACGGCAGAGAGAGCCAGACCAGAGACCAGCTGAACTACATCGACGACCTGGAGACCAAGCTGGCC
GACGCCAAGAAGGAGAACGACAAGGCCAGACAGGCCCTGGTGGAGTACATGAACAAGTGCAGCAAGCTGGAGCAC
GAGATCAGAACCATGGTGAAGAACAGCACCTTCGACAGCAGCAGCATGCTGCTGGGCGGCCAGACCAGCGACGAG
CTGAAGATCCAGATCGGCAAGGTGAACGGCGAGCTGAACGTGCTGAGAGCCGAGAACAGAGAGCTGAGAATCAGA
TGCGACCAGCTGACCGGCGGCGACGGCAACCTGAGCATCAGCCTGGGCCAGAGCAGACTGATGGCCGGCATCGCC
ACCAACGACGTGGACAGCATCGGCCAGGGCAACGAGACCGGCGGCACCAGCATGAGAATCCTGCCCAGAGAGAGC
CAGCTGGACGACCTGGAGGAGAGCAAGCTGCCCCTGATGGACACCAGCAGCGCCGTGAGAAACCAGCAGCAGTTC
GCCAGCATGTGGGAGGACTTCGAGAGCGTGAAGGACAGCCTGCAGAACAACCACAACGACACCCTGGAGGGCAGC
TTCAACAGCAGCATGCCCCCCCCCGGCAGAGACGCCACCCAGAGCTTCCTGAGCCAGAAGAGCTTCAAGAACAGC
CCCATCGTGATGCAGAAGCCCAAGAGCCTGCACCTGCACCTGAAGAGCCACCAGAGCGAGGGCGCCGGCGAGCAG
ATCCAGAACAACAGCTTCAGCACCAAGACCGCCAGCCCCCACGTGAGCCAGAGCCACATCCCCATCCTGCACGAC
ATGCAGCAGATCCTGGACAGCAGCGCCATGTTCCTGGAGGGCCAGCACGACGTGGCCGTGAACGTGGAGCAGATG
CAGGAGAAGATGAGCCAGATCAGAGAGGCCCTGGCCAGACTGTTCGAGAGACTGAAGAGCAGCGCCGCCCTGTTC
GAGGAGATCCTGGAGAGAATGGGCAGCAGCGACCCCAACGCCGACAAGATCAAGAAGATGAAGCTGGCCTTCGAG
ACCAGCATCAACGACAAGCTGAACGTGAGCGCCATCCTGGAGGCCGCCGAGAAGGACCTGCACAACATGAGCCTG
AACTTCAGCATCCTGGAGAAGAGCATCGTGAGCCAGGCCGCCGAGGCCAGCAGAAGATTCACCATCGCCCCCGAC
GCCGAGGACGTGGCCAGCAGCAGCCTGCTGAACGCCAGCTACAGCCCCCTGTTCAAGTTCACCAGCAACAGCGAC
ATCGTGGAGAAGCTGCAGAACGAGGTGAGCGAGCTGAAGAACGAGCTGGAGATGGCCAGAACCAGAGACATGAGA
AGCCCCCTGAACGGCAGCAGCGGCAGACTGAGCGACGTGCAGATCAACACCAACAGAATGTTCGAGGACCTGGAG
GTGAGCGAGGCCACCCTGCAGAAGGCCAAGGAGGAGAACAGCACCCTGAAGAGCCAGTTCGCCGAGCTGGAGGCC
AACCTGCACCAGGTGAACAGCAAGCTGGGCGAGGTGAGATGCGAGCTGAACGAGGCCCTGGCCAGAGTGGACGGC
GAGCAGGAGACCAGAGTGAAGGCCGAGAACGCCCTGGAGGAGGCCAGACAGCTGATCAGCAGCCTGAAGCACGAG
GAGAACGAGCTGAAGAAGACCATCACCGACATGGGCATGAGACTGAACGAGGCCAAGAAGAGCGACGAGTTCCTG
AAGAGCGAGCTGAGCACCGCCCTGGAGGAGGAGAAGAAGAGCCAGAACCTGGCCGACGAGCTGAGCGAGGAGCTG
AACGGCTGGAGAATGAGAACCAAGGAGGCCGAGAACAAGGTGGAGCACGCCAGCAGCGAGAAGAGCGAGATGCTG
GAGAGAATCGTGCACCTGGAGACCGAGATGGAGAAGCTGAGCACCAGCGAGATCGCCGCCGACTACTGCAGCACC
AAGATGACCGAGAGAAAGAAGGAGATCGAGCTGGCCAAGTACAGAGAGGACTTCGAGAACGCCGCCATCGTGGGC
CTGGAGAGAATCAGCAAGGAGATCAGCGAGCTGACCAAGAAGACCCTGAAGGCCAAGATCATCCCCAGCAACATC
AGCAGCATCCAGCTGGTGTGCGACGAGCTGTGCAGAAGACTGAGCAGAGAGAGAGAGCAGCAGCACGAGTACGCC
AAGGTGATGAGAGACGTGAACGAGAAGATCGAGAAGCTGCAGCTGGAGAAGGACGCCCTGGAGCACGAGCTGAAG
ATGATGAGCAGCAACAACGAGAACGTGCCCCCCGTGGGCACCAGCGTGAGCGGCATGCCCACCAAGACCAGCAAC
CAGAAGTGCGCCCAGCCCCACTACACCAGCCCCACCAGACAGCTGCTGCACGAGAGCACCATGGCCGTGGACGCC
ATCGTGCAGAAGCTGAAGAAGACCCACAACATGAGCGGCATGGGCCCCGAGCTGAAGGAGACCATCGGCAACGTG
ATCAACGAGAGCAGAGTGCTGAGAGACTTCCTGCACCAGAAGCTGATCCTGTTCAAGGGCATCGACATGAGCAAC
TGGAAGAACGAGACCGTGGACCAGCTGATCACCGACCTGGGCCAGCTGCACCAGGACAACCTGATGCTGGAGGAG
CAGATCAAGAAGTACAAGAAGGAGCTGAAGCTGACCAAGAGCGCCATCCCCACCCTGGGCGTGGAGTTCCAGGAC
AGAATCAAGACCGAGATCGGCAAGATCGCCACCGACATGGGCGGCGCCGTGAAGGAGATCAGAAAGAAGGGTACC
GGCGCCCCCGGCTCCGCCGGCTCCGCCGCCGGCTCCGGCGCGTGCCCGGTGCCGCTGCAGCTGCCGCCGCTGGAA
CGCCTGACCCTGGATGATAAAAAACCGCTGAATACCCTGATCTCTGCTACTGGTCTGTGGATGAGTCGTACCGGA
ACCATTCATAAAATCAAACACCACGAGGTTAGCCGTTCGAAAATCTATATTGAGATGGCGTGTGGCGATCATCTG
GTTGTGAACAATAGCCGCTCTTCTCGTACAGCACGTGCACTGCGTCACCACAAATATCGTAAAACCTGTAAACGT
TGCCGTGTGTCCGATGAGGATCTGAACAAATTCCTGACAAAAGCCAATGAGGACCAAACAAGCGTGAAAGTGAAA

GTCGTTAGCGCTCCTACCCGTACTAAAAAAGCAATGCCGAAATCCGTTGCTCGTGCCCCTAAACCACTGGAAAAC
ACTGAAGCAGCACAGGCACAGCCGTCTGGAAGCAAATTCTCTCCGGCCATTCCTGTTTCTACCCAGGAGTCCGTT
TCTGTTCCAGCAAGTGTGAGCACCAGCATTAGCAGTATTAGCACCGGTGCCACCGCTAGCGCCCTGGTTAAAGGC
AATACCAATCCGATTACAAGCATGTCTGCCCCGGTTCAAGCATCAGCTCCAGCACTGACAAAATCCCAAACCGAT
CGTCTGGAGGTTCTGCTGAATCCGAAAGACGAAATCAGCCTGAATTCCGGCAAACCGTTTCGTGAACTGGAGAGC
GAACTGCTGTCACGTCGTAAAAAAGACCTGCAACAAATCTATGCCGAAGAACGTGAGAACTATCTGGGGAAACTG
GAACGTGAAATCACCCGCTTTTTCGTGGATCGTGGCTTTCTGGAGATCAAATCCCCGATTCTGATTCCTCTGGAG
TATATCGAGCGTATGGGCATCGACAATGATACCGAACTGAGCAAACAAATTTTCCGTGTGGATAAAAACTTCTGT
CTGCGCCCTATGCTAGCACCAAATCTGGCTAACTATCTGCGCAAACTGGACCGTGCCCTGCCTGATCCTATCAAA
ATCTTCGAGATCGGCCCGTGTTATCGTAAAGAGTCCGACGGTAAAGAACATCTGGAGGAGTTTACCATGCTGAAC
TTTTGCCAAATGGGTTCAGGTTGTACTCGTGAGAACCTGGAAAGCATCATCACCGATTTTCTGAACCACCTGGGC
ATTGACTTCAAAATTGTGGGCGACAGCTGTATGGTGTTTGGCGACACCCTGGATGTCATGCACGGCGACCTGGAA
CTGTCTAGTGCCGTTGTGGGCCCAATCCCGCTGGATCGTGAGTGGGGTATCGACAAACCTTGGATCGGTGCGGGT
TTTGGTCTGGAGCGTCTGCTGAAAGTAAAACACGACTTCAAGAACATCAAACGTGCTGCACGTTCCGAGTCCTAT
TACAATGGTATTTCTACTAACCTGTAA (SEQ ID NO:47)
Protein:
MEDNSVLNEDSNLEHVEGQPRRSMSQPVLNVEGDKRTSSTSATQQQVLSGAFSSADVRSIPIIQTWEENKALKTK
ITILRGELQMYQRRYSEAKEASQKRVKEVMDDYVDLKLGQENVQEKMEQYKLMEEDLLAMQSRIETSEDNFARQM
KEFEAQKHAMEERIKELELSATDANNTTVGSFRGTLDDILKKNDPDFTLTSGYEERKINDLEAKLLSEIDKVAEL
EDHIQQLRQELDDQSARLADSENVRAQLEAATGQGILGAAGNAMVPNSTFMIGNGRESQTRDQLNYIDDLETKLA
DAKKENDKARQALVEYMNKCSKLEHEIRTMVKNSTFDSSSMLLGGQTSDELKIQIGKVNGELNVLRAENRELRIR
CDQLTGGDGNLSISLGQSRLMAGIATNDVDSIGQGNETGGTSMRILPRESQLDDLEESKLPLMDTSSAVRNQQQF
ASMWEDFESVKDSLQNNHNDTLEGSFNSSMPPPGRDATQSFLSQKSFKNSPIVMQKPKSLHLHLKSHQSEGAGEQ
IQNNSFSTKTASPHVSQSHIPILHDMQQILDSSAMFLEGQHDVAVNVEQMQEKMSQIREALARLFERLKSSAALF
EEILERMGSSDPNADKIKKMKLAFETSINDKLNVSAILEAAEKDLHNMSLNFSILEKSIVSQAAEASRRFTIAPD
AEDVASSSLLNASYSPLFKFTSNSDIVEKLQNEVSELKNELEMARTRDMRSPLNGSSGRLSDVQINTNRMFEDLE
VSEATLQKAKEENSTLKSQFAELEANLHQVNSKLGEVRCELNEALARVDGEQETRVKAENALEEARQLISSLKHE
ENELKKTITDMGMRLNEAKKSDEFLKSELSTALEEEKKSQNLADELSEELNGWRMRTKEAENKVEHASSEKSEML
ERIVHLETEMEKLSTSEIAADYCSTKMTERKKEIELAKYREDFENAAIVGLERISKEISELTKKTLKAKIIPSNI
SSIQLVCDELCRRLSREREQQHEYAKVMRDVNEKIEKLQLEKDALEHELKMMSSNNENVPPVGTSVSGMPTKTSN
QKCAQPHYTSPTRQLLHESTMAVDAIVQKLKKTHNMSGMGPELKETIGNVINESRVLRDFLHQKLILFKGIDMSN
WKNETVDQLITDLGQLHQDNLMLEEQIKKYKKELKLTKSAIPTLGVEFQDRIKTEIGKIATDMGGAVKEIRKKGT
GAPGSAGSAAGSGACPVPLQLPPLERLTLDDKKPLNTLISATGLWMSRTGTIHKIKHHEVSRSKIYIEMACGDHL
VVNNSRSSRTARALRHHKYRKTCKRCRVSDEDLNKFLTKANEDQTSVKVKVVSAPTRTKKAMPKSVARAPKPLEN
TEAAQAQPSGSKFSPAIPVSTQESVSVPASVSTSISSISTGATASALVKGNTNPITSMSAPVQASAPALTKSQTD
RLEVLLNPKDEISLNSGKPFRELESELLSRRKKDLQQIYAEERENYLGKLEREITRFFVDRGFLEIKSPILIPLE
YIERMGIDNDTELSKQIFRVDKNFCLRPMLAPNLANYLRKLDRALPDPIKIFEIGPCYRKESDGKEHLEEFTMLN
FCQMGSGCTRENLESIITDFLNHLGIDFKIVGDSCMVFGDTLDVMHGDLELSSAVVGPIPLDREWGIDKPWIGAG
FGLERLLKVKHDFKNIKRAARSESYYNGISTNL (SEQ ID NO:48)

KIF16B-FUS-PylRS^AF
DNA:
ATGGCATCGGTCAAGGTGGCCGTGAGGGTCCGGCCCATGAATCGCAGGGAAAAGGACTTGGAGGCCAAGTTCATT
ATTCAGATGGAGAAAAGCAAAACGACAATCACAAACTTAAAGATACCAGAAGGAGGCACTGGGGACTCAGGAAGA
GAACGGACCAAGACCTTCACCTATGACTTTTCTTTTTATTCTGCTGATACAAAAAGCCCAGATTACGTTTCACAA
GAAATGGTTTTCAAAACCCTCGGCACAGATGTCGTGAAGTCTGCATTTGAAGGTTATAATGCTTGTGTCTTTGCA
TATGGGCAAACTGGATCTGGAAAGTCATACACTATGATGGGAAATTCTGGAGATTCTGGCTTAATACCTCGGATC
TGTGAAGGACTCTTCAGTCGGATAAATGAAACCACCAGATGGGATGAAGCTTCTTTTCGAACTGAAGTCAGCTAC
TTAGAAATTTATAACGAACGTGTGAGAGATCTACTTCGGCGGAAGTCATCTAAAACCTTCAATTTGAGAGTCCGT
GAGCATCCCAAAGAAGGCCCTTATGTTGAGGATTTATCCAAACATTTAGTACAGAATTATGGTGACGTAGAAGAA
CTTATGGATGCGGGCAATATCAACCGGACCACCGCAGCGACTGGGATGAACGACGTCAGTAGCAGGTCTCATGCC
ATCTTCACCATCAAGTTCACTCAGGCTAAATTTGATTCTGAAATGCCATGTGAAACCGTCAGTAAGATCCACTTG
GTTGATCTTGCCGGAAGTGAGCGTGCAGATGCCACCGGAGCCACCGGGGTTAGGCTAAAGGAAGGGGGAAATATT
AACAAGTCCCTCGTGACTCTGGGGAACGTCATTTCTGCCTTAGCTGATTTATCTCAGGATGCTGCAAATACTCTT
GCAAAGAAGAAGCAAGTTTTCGTGCCTTACAGGGATTCTGTGTTGACTTGGTTGTTAAAAGATAGCCTTGGAGGA
AACTCTAAAACTATCATGATTGCCACCATTTCACCTGCTGATGTCAATTATGGAGAAACCCTAAGTACTCTTCGC
TATGCAAATAGAGCCAAAAACATCATCAACAAGCCTACCATTAATGAGGATGCCAACGTCAAACTTATCCGTGAG
CTGCGAGCTGAAATAGCCAGACTGAAAACGCTGCTTGCTCAAGGGAATCAGATTGCCCTCTTAGACTCCCCCACA

TATACAGATATTGAAATGAACAGATTGGGAAAGGGCGCCCCCGGCTCCGCCGGCTCCGCCGCCGGCTCCGGCATG
GCCTCAAACGATTATACCCAACAAGCAACCCAAAGCTATGGGGCCTACCCCACCCAGCCCGGGCAGGGCTATTCC
CAGCAGAGCAGTCAGCCCTACGGACAGCAGAGTTACAGTGGTTATAGCCAGTCCACGGACACTTCAGGATATGGC
CAGAGCAGCTATTCTTCTTATGGCCAGAGCCAGAACACAGGCTATGGAACTCAGTCAACTCCCCAGGGATATGGC
TCGACTGGCGGCTATGGCAGTAGCCAGAGCTCCCAATCGTCTTACGGGCAGCAGTCCTCCTACCCTGGCTATGGC
CAGCAGCCAGCTCCCAGCAGCACCTCGGGAAGTTACGGTAGCAGTTCTCAGAGCAGCAGCTATGGGCAGCCCCAG
AGTGGGAGCTACAGCCAGCAGCCTAGCTATGGTGGACAGCAGCAAAGCTATGGACAGCAGCAAAGCTATAATCCC
CCTCAGGGCTATGGACAGCAGAACCAGTACAACAGCAGCAGTGGTGGTGGAGGTGGAGGTGGAGGTGGAGGTAAC
TATGGCCAAGATCAATCCTCCATGAGTAGTGGTGGTGGCAGTGGTGGCGGTTATGGCAATCAAGACCAGAGTGGT
GGAGGTGGCAGCGGTGGCTATGGACAGCAGGACCGTGGAGGCCGCGGCAGGGGTGGCAGTGGTGGCGGCGGCGGC
GGCGGCGGTGGTGGTTACAACCGCAGCAGTGGTGGCTATGAACCCAGAGGTCGTGGAGGTGGCCGTGGAGGCAGA
GGTGGCATGGGCGGAAGTGACCGTGGTGGCTTCAATAAATTTGGTGGCCCTCGGGACCAAGGATCACGTCATGAC
TCCGAACAGGATAATTCAGACAACAACACCATCTTTGTGCAAGGCCTGGGTGAGAATGTTACAATTGAGTCTGTG
GCTGATTACTTCAAGCAGATTGGTATTATTAAGACAAACAAGAAAACGGGACAGCCCATGATTAATTTGTACACA
GACAGGGAAACTGGCAAGCTGAAGGGAGAGGCAACGGTCTCTTTTGATGACCCACCTTCAGCTAAAGCAGCTATT
GACTGGTTTGATGGTAAAGAATTCTCCGGAAATCCTATCAAGGTCTCATTTGCTACTCGCCGGGCAGACTTTAAT
CGGGGTGGTGGCAATGGTCGTGGAGGCCGAGGGCGAGGAGGACCCATGGGCCGTGGAGGCTATGGAGGTGGTGGC
AGTGGTGGTGGTGGCCGAGGAGGATTTCCCAGTGGAGGTGGTGGCGGTGGAGGACAGCAGCGAGCTGGTGACTGG
AAGTGTCCTAATCCCACCTGTGAGAATATGAACTTCTCTTGGAGGAATGAATGCAACCAGTGTAAGGCCCCTAAA
CCAGATGGCCCAGGAGGGGGACCAGGTGGCTCTCACATGGGGGGTAACTACGGGGATGATCGTCGTGGTGGCAGA
GGAGGCGATTACAAGGATGACGACGATAAGGGTACCGGCGCCCCCGGCTCCGCCGGCTCCGCCGCCGGCTCCGGC
GCGTGCCCGGTGCCGCTGCAGCTGCCGCCGCTGGAACGCCTGACCCTGGATGATAAAAAACCGCTGAATACCCTG
ATCTCTGCTACTGGTCTGTGGATGAGTCGTACCGGAACCATTCATAAAATCAAACACCACGAGGTTAGCCGTTCG
AAAATCTATATTGAGATGGCGTGTGGCGATCATCTGGTTGTGAACAATAGCCGCTCTTCTCGTACAGCACGTGCA
CTGCGTCACCACAAATATCGTAAAACCTGTAAACGTTGCCGTGTGTCCGATGAGGATCTGAACAAATTCCTGACA
AAAGCCAATGAGGACCAAACAAGCGTGAAAGTGAAAGTCGTTAGCGCTCCTACCCGTACTAAAAAAGCAATGCCG
AAATCCGTTGCTCGTGCCCCTAAACCACTGGAAAACACTGAAGCAGCACAGGCACAGCCGTCTGGAAGCAAATTC
TCTCCGGCCATTCCTGTTTCTACCCAGGAGTCCGTTTCTGTTCCAGCAAGTGTGAGCACCAGCATTAGCAGTATT
AGCACCGGTGCCACCGCTAGCGCCCTGGTTAAAGGCAATACCAATCCGATTACAAGCATGTCTGCCCCGGTTCAA
GCATCAGCTCCAGCACTGACAAAATCCCAAACCGATCGTCTGGAGGTTCTGCTGAATCCGAAAGACGAAATCAGC
CTGAATTCCGGCAAACCGTTTCGTGAACTGGAGAGCGAACTGCTGTCACGTCGTAAAAAAGACCTGCAACAAATC
TATGCCGAAGAACGTGAGAACTATCTGGGGAAACTGGAACGTGAAATCACCCGCTTTTTCGTGGATCGTGGCTTT
CTGGAGATCAAATCCCCGATTCTGATTCCTCTGGAGTATATCGAGCGTATGGGCATCGACAATGATACCGAACTG
AGCAAACAAATTTTCCGTGTGGATAAAAACTTCTGTCTGCGCCCTATGCTAGCACCAAATCTGGCTAACTATCTG
CGCAAACTGGACCGTGCCCTGCCTGATCCTATCAAAATCTTCGAGATCGGCCCGTGTTATCGTAAAGAGTCCGAC
GGTAAAGAACATCTGGAGGAGTTTACCATGCTGAACTTTTGCCAAATGGGTTCAGGTTGTACTCGTGAGAACCTG
GAAAGCATCATCACCGATTTTCTGAACCACCTGGGCATTGACTTCAAAATTGTGGGCGACAGCTGTATGGTGTTT
GGCGACACCCTGGATGTCATGCACGGCGACCTGGAACTGTCTAGTGCCGTTGTGGGCCCAATCCCGCTGGATCGT
GAGTGGGGTATCGACAAACCTTGGATCGGTGCGGGTTTTGGTCTGGAGCGTCTGCTGAAAGTAAAACACGACTTC
AAGAACATCAAACGTGCTGCACGTTCCGAGTCCTATTACAATGGTATTTCTACTAACCTGTAA (SEQ ID
NO:49)
Protein:
MASVKVAVRVRPMNRREKDLEAKFIIQMEKSKTTITNLKIPEGGTGDSGRERTKTFTYDFSFYSADTKSPDYVSQ
EMVFKTLGTDVVKSAFEGYNACVFAYGQTGSGKSYTMMGNSGDSGLIPRICEGLFSRINETTRWDEASFRTEVSY
LEIYNERVRDLLRRKSSKTFNLRVREHPKEGPYVEDLSKHLVQNYGDVEELMDAGNINRTTAATGMNDVSSRSHA
IFTIKFTQAKFDSEMPCETVSKIHLVDLAGSERADATGATGVRLKEGGNINKSLVTLGNVISALADLSQDAANTL
AKKKQVFVPYRDSVLTWLLKDSLGGNSKTIMIATISPADVNYGETLSTLRYANRAKNIINKPTINEDANVKLIRE
LRAEIARLKTLLAQGNQIALLDSPTYTDIEMNRLGKGAPGSAGSAAGSGMASNDYTQQATQSYGAYPTQPGQGYS
QQSSQPYGQQSYSGYSQSTDTSGYGQSSYSSYGQSQNTGYGTQSTPQGYGSTGGYGSSQSSQSSYGQQSSYPGYG
QQPAPSSTSGSYGSSSQSSSYGQPQSGSYSQQPSYGGQQQSYGQQQSYNPPQGYGQQNQYNSSSGGGGGGGGGGN
YGQDQSSMSSGGGSGGGYGNQDQSGGGGSGGYGQQDRGGRGRGGSGGGGGGGGGYNRSSGGYEPRGRGGGRGGR
GGMGGSDRGGFNKFGGPRDQGSRHDSEQDNSDNNTIFVQGLGENVTIESVADYFKQIGIIKTNKKTGQPMINLYT
DRETGKLKGEATVSFDDPPSAKAAIDWFDGKEFSGNPIKVSFATRRADFNRGGGNGRGGRGRGGPMGRGGYGGGG
SGGGGRGGFPSGGGGGGGQQRAGDWKCPNPTCENMNFSWRNECNQCKAPKPDGPGGGPGGSHMGGNYGDDRRGGR
GGDYKDDDDKGTGAPGSAGSAAGSGACPVPLQLPPLERLTLDDKPLNTLISATGLWMSRTGTIHKIKHHEVSRS
KIYIEMACGDHLVVNNSRSSRTARALRHHKYRKTCKRCRVSDEDLNKFLTKANEDQTSVKVKVVSAPTRTKKAMP
KSVARAPKPLENTEAAQAQPSGSKFSPAIPVSTQESVSVPASVSTSISSISTGATASALVKGNTNPITSMSAPVQ
ASAPALTKSQTDRLEVLLNPKDEISLNSGKPFRELESELLSRRKKDLQQIYAEERENYLGKLEREITRFFVDRGF

LEIKSPILIPLEYIERMGIDNDTELSKQIFRVDKNFCLRPMLAPNLANYLRKLDRALPDPIKIFEIGPCYRKESD
GKEHLEEFTMLNFCQMGSGCTRENLESIITDFLNHLGIDFKIVGDSCMVFGDTLDVMHGDLELSSAVVGPIPLDR
EWGIDKPWIGAGFGLERLLKVKHDFKNIKRAARSESYYNGISTNL (SEQ ID NO:50)

KIF16B-VSV-G-FUS-PylRS^AF
DNA:
ATGGCATCGGTCAAGGTGGCCGTGAGGGTCCGGCCCATGAATCGCAGGGAAAAGGACTTGGAGGCCAAGTTCATT
ATTCAGATGGAGAAAAGCAAAACGACAATCACAAACTTAAAGATACCAGAAGGAGGCACTGGGGACTCAGGAAGA
GAACGGACCAAGACCTTCACCTATGACTTTTCTTTTTATTCTGCTGATACAAAAAGCCCAGATTACGTTTCACAA
GAAATGGTTTTCAAAACCCTCGGCACAGATGTCGTGAAGTCTGCATTTGAAGGTTATAATGCTTGTGTCTTTGCA
TATGGGCAAACTGGATCTGGAAAGTCATACACTATGATGGGAAATTCTGGAGATTCTGGCTTAATACCTCGGATC
TGTGAAGGACTCTTCAGTCGGATAAATGAAACCACCAGATGGGATGAAGCTTCTTTTCGAACTGAAGTCAGCTAC
TTAGAAATTTATAACGAACGTGTGAGAGATCTACTTCGGCGGAAGTCATCTAAAACCTTCAATTTGAGAGTCCGT
GAGCATCCCAAAGAAGGCCCTTATGTTGAGGATTTATCCAAACATTTAGTACAGAATTATGGTGACGTAGAAGAA
CTTATGGATGCGGGCAATATCAACCGGACCACCGCAGCGACTGGGATGAACGACGTCAGTAGCAGGTCTCATGCC
ATCTTCACCATCAAGTTCACTCAGGCTAAATTTGATTCTGAAATGCCATGTGAAACCGTCAGTAAGATCCACTTG
GTTGATCTTGCCGGAAGTGAGCGTGCAGATGCCACCGGAGCCACCGGGGTTAGGCTAAAGGAAGGGGGAAATATT
AACAAGTCCCTCGTGACTCTGGGGAACGTCATTTCTGCCTTAGCTGATTTATCTCAGGATGCTGCAAATACTCTT
GCAAAGAAGAAGCAAGTTTTCGTGCCTTACAGGGATTCTGTGTTGACTTGGTTGTTAAAAGATAGCCTTGGAGGA
AACTCTAAAACTATCATGATTGCCACCATTTCACCTGCTGATGTCAATTATGGAGAAACCCTAAGTACTCTTCGC
TATGCAAATAGAGCCAAAAACATCATCAACAAGCCTACCATTAATGAGGATGCCAACGTCAAACTTATCCGTGAG
CTGCGAGCTGAAATAGCCAGACTGAAAACGCTGCTTGCTCAAGGGAATCAGATTGCCCTCTTAGACTCCCCCACA
TATACAGATATTGAAATGAACAGATTGGGAAAGGGCGCCCCCGGCTCCGCCGGCTCCGCCGCCGGCTCCGGCATG
GCCTCAAACGATTATACCCAACAAGCAACCCAAAGCTATGGGGCCTACCCCACCCAGCCCGGGCAGGGCTATTCC
CAGCAGAGCAGTCAGCCCTACGGACAGCAGAGTTACAGTGGTTATAGCCAGTCCACGGACACTTCAGGATATGGC
CAGAGCAGCTATTCTTCTTATGGCCAGAGCCAGAACACAGGCTATGGAACTCAGTCAACTCCCCAGGGATATGGC
TCGACTGGCGGCTATGGCAGTAGCCAGAGCTCCCAATCGTCTTACGGGCAGCAGTCCTCCTACCCTGGCTATGGC
CAGCAGCCAGCTCCCAGCAGCACCTCGGGAAGTTACGGTAGCAGTTCTCAGAGCAGCAGCTATGGGCAGCCCCAG
AGTGGGAGCTACAGCCAGCAGCCTAGCTATGGTGGACAGCAGCAAAGCTATGGACAGCAGCAAAGCTATAATCCC
CCTCAGGGCTATGGACAGCAGAACCAGTACAACAGCAGCAGTGGTGGTGGAGGTGGAGGTGGAGGTGGAGGTAAC
TATGGCCAAGATCAATCCTCCATGAGTAGTGGTGGTGGCAGTGGTGGCGGTTATGGCAATCAAGACCAGAGTGGT
GGAGGTGGCAGCGGTGGCTATGGACAGCAGGACCGTGGAGGCCGCGGCAGGGGTGGCAGTGGTGGCGGCGGCGGC
GGCGGCGGTGGTGGTTACAACCGCAGCAGTGGTGGCTATGAACCCAGAGGTCGTGGAGGTGGCCGTGGAGGCAGA
GGTGGCATGGGCGGAAGTGACCGTGGTGGCTTCAATAAATTTGGTGGCCCTCGGGACCAAGGATCACGTCATGAC
TCCGAACAGGATAATTCAGACAACAACACCATCTTTGTGCAAGGCCTGGGTGAGAATGTTACAATTGAGTCTGTG
GCTGATTACTTCAAGCAGATTGGTATTATTAAGACAAACAAGAAAACGGGACAGCCCATGATTAATTTGTACACA
GACAGGGAAACTGGCAAGCTGAAGGGAGAGGCAACGGTCTCTTTTGATGACCCACCTTCAGCTAAAGCAGCTATT
GACTGGTTTGATGGTAAAGAATTCTCCGGAAATCCTATCAAGGTCTCATTTGCTACTCGCCGGGCAGACTTTAAT
CGGGGTGGTGGCAATGGTCGTGGAGGCCGAGGGCGAGGAGGACCCATGGGCCGTGGAGGCTATGGAGGTGGTGGC
AGTGGTGGTGGTGGCCGAGGAGGATTTCCCAGTGGAGGTGGTGGCGGTGGAGGACAGCAGCGAGCTGGTGACTGG
AAGTGTCCTAATCCCACCTGTGAGAATATGAACTTCTCTTGGAGGAATGAATGCAACCAGTGTAAGGCCCCTAAA
CCAGATGGCCCAGGAGGGGGACCAGGTGGCTCTCACATGGGGGGTAACTACGGGGATGATCGTCGTGGTGGCAGA
GGTGGTGCGATCGCAGGAGCACCAGGAAGTGCTGGTTCTGCTGCTGGTAGTGGAGCGTGCCCGGTGCCGCTGCAG
CTGCCGCCGCTGGAACGCCTGACCCTGGATGATAAAAAACCGCTGAATACCCTGATCTCTGCTACTGGTCTGTGG
ATGAGTCGTACCGGAACCATTCATAAAATCAAACACCACGAGGTTAGCCGTTCGAAAATCTATATTGAGATGGCG
TGTGGCGATCATCTGGTTGTGAACAATAGCCGCTCTTCTCGTACAGCACGTGCACTGCGTCACCACAAATATCGT
AAAACCTGTAAACGTTGCCGTGTGTCCGATGAGGATCTGAACAAATTCCTGACAAAAGCCAATGAGGACCAAACA
AGCGTGAAAGTGAAAGTCGTTAGCGCTCCTACCCGTACTAAAAAAGCAATGCCGAAATCCGTTGCTCGTGCCCCT
AAACCACTGGAAAACACTGAAGCAGCACAGGCACAGCCGTCTGGAAGCAAATTCTCTCCGGCCATTCCTGTTTCT
ACCCAGGAGTCCGTTTCTGTTCCAGCAAGTGTGAGCACCAGCATTAGCAGTATTAGCACCGGTGCCACCGCTAGC
GCCCTGGTTAAAGGCAATACCAATCCGATTACAAGCATGTCTGCCCCGGTTCAAGCATCAGCTCCAGCACTGACA
AAATCCCAAACCGATCGTCTGGAGGTTCTGCTGAATCCGAAAGACGAAATCAGCCTGAATTCCGGCAAACCGTTT
CGTGAACTGGAGAGCGAACTGCTGTCACGTCGTAAAAAAGACCTGCAACAAATCTATGCCGAAGAACGTGAGAAC
TATCTGGGGAAACTGGAACGTGAAATCACCCGCTTTTTCGTGGATCGTGGCTTTCTGGAGATCAAATCCCCGATT
CTGATTCCTCTGGAGTATATCGAGCGTATGGGCATCGACAATGATACCGAACTGAGCAAACAAATTTTCCGTGTG
GATAAAAACTTCTGTCTGCGCCCTATGCTAGCACCAAATCTGGCTAACTATCTGCGCAAACTGGACCGTGCCCTG
CCTGATCCTATCAAAATCTTCGAGATCGGCCCGTGTTATCGTAAAGAGTCCGACGGTAAAGAACATCTGGAGGAG
TTTACCATGCTGAACTTTTGCCAAATGGGTTCAGGTTGTACTCGTGAGAACCTGGAAAGCATCATCACCGATTTT

CTGAACCACCTGGGCATTGACTTCAAAATTGTGGGCGACAGCTGTATGGTGTTTGGCGACACCCTGGATGTCATG
CACGGCGACCTGGAACTGTCTAGTGCCGTTGTGGGCCCAATCCCGCTGGATCGTGAGTGGGGTATCGACAAACCT
TGGATCGGTGCGGGTTTTGGTCTGGAGCGTCTGCTGAAAGTAAAACACGACTTCAAGAACATCAAACGTGCTGCA
CGTTCCGAGTCCTATTACAATGGTATTTCTACTAACCTGTAA (SEQ ID NO:51)
Protein:
MASVKVAVRVRPMNRREKDLEAKFIIQMEKSKTTITNLKIPEGGTGDSGRERTKTFTYDFSFYSADTKSPDYVSQ
EMVFKTLGTDVVKSAFEGYNACVFAYGQTGSGKSYTMMGNSGDSGLIPRICEGLFSRINETTRWDEASFRTEVSY
LEIYNERVRDLLRRKSSKTFNLRVREHPKEGPYVEDLSKHLVQNYGDVEELMDAGNINRTTAATGMNDVSSRSHA
IFTIKFTQAKFDSEMPCETVSKIHLVDLAGSERADATGATGVRLKEGGNINKSLVTLGNVISALADLSQDAANTL
AKKKQVFVPYRDSVLTWLLKDSLGGNSKTIMIATISPADVNYGETLSTLRYANRAKNIINKPTINEDANVKLIRE
LRAEIARLKTLLAQGNQIALLDSPTYTDIEMNRLGKGAPGSAGSAAGSGMASNDYTQQATQSYGAYPTQPGQGYS
QQSSQPYGQQSYSGYSQSTDTSGYGQSSYSSYGQSQNTGYGTQSTPQGYGSTGGYGSSQSSQSSYGQQSSYPGYG
QQPAPSSTSGSYGSSSQSSSYGQPQSGSYSQQPSYGGQQQSYGQQQSYNPPQGYGQQNQYNSSSGGGGGGGGGGN
YGQDQSSMSSGGGSGGGYGNQDQSGGGGSGGYGQQDRGGRGRGGSGGGGGGGGGGYNRSSGGYEPRGRGGGRGGR
GGMGGSDRGGFNKFGGPRDQGSRHDSEQDNSDNNTIFVQGLGENVTIESVADYFKQIGIIKTNKKTGQPMINLYT
DRETGKLKGEATVSFDDPPSAKAAIDWFDGKEFSGNPIKVSFATRRADFNRGGGNGRGGRGRGGPMGRGGYGGGG
SGGGGRGGFPSGGGGGGGGQQRAGDWKCPNPTCENMNFSWRNECNQCKAPKPDGPGGGPGGSHMGGNYGDDRRGGR
GGAIAGAPGSAGSAAGSGACPVPLQLPPLERLTLDDKKPLNTLISATGLWMSRTGTIHKIKHHEVSRSKIYIEMA
CGDHLVVNNSRSSRTARALRHHKYRKTCKRCRVSDEDLNKFLTKANEDQTSVKVKVVSAPTRTKKAMPKSVARAP
KPLENTEAAQAQPSGSKFSPAIPVSTQESVSVPASVSTSISSISTGATASALVKGNTNPITSMSAPVQASAPALT
KSQTDRLEVLLNPKDEISLNSGKPFRELESELLSRRKKDLQQIYAEERENYLGKLEREITRFFVDRGFLEIKSPI
LIPLEYIERMGIDNDTELSKQIFRVDKNFCLRPMLAPNLANYLRKLDRALPDPIKIFEIGPCYRKESDGKEHLEE
FTMLNFCQMGSGCTRENLESIITDFLNHLGIDFKIVGDSCMVFGDTLDVMHGDLELSSAVVGPIPLDREWGIDKP
WIGAGFGLERLLKVKHDFKNIKRAARSESYYNGISTNL (SEQ ID NO:52)

KIF16B-FUS-PylRS^AA
DNA:
ATGGCATCGGTCAAGGTGGCCGTGAGGGTCCGGCCCATGAATCGCAGGGAAAAGGACTTGGAGGCCAAGTTCATT
ATTCAGATGGAGAAAAGCAAAACGACAATCACAAACTTAAAGATACCAGAAGGAGGCACTGGGGACTCAGGAAGA
GAACGGACCAAGACCTTCACCTATGACTTTTCTTTTTATTCTGCTGATACAAAAAGCCCAGATTACGTTTCACAA
GAAATGGTTTTCAAAACCCTCGGCACAGATGTCGTGAAGTCTGCATTTGAAGGTTATAATGCTTGTGTCTTTGCA
TATGGGCAAACTGGATCTGGAAAGTCATACACTATGATGGGAAATTCTGGAGATTCTGGCTTAATACCTCGGATC
TGTGAAGGACTCTTCAGTCGGATAAATGAAACCACCAGATGGGATGAAGCTTCTTTTCGAACTGAAGTCAGCTAC
TTAGAAATTTATAACGAACGTGTGAGAGATCTACTTCGGCGGAAGTCATCTAAAACCTTCAATTTGAGAGTCCGT
GAGCATCCCAAAGAAGGCCCTTATGTTGAGGATTTATCCAAACATTTAGTACAGAATTATGGTGACGTAGAAGAA
CTTATGGATGCGGGCAATATCAACCGGACCACCGCAGCGACTGGGATGAACGACGTCAGTAGCAGGTCTCATGCC
ATCTTCACCATCAAGTTCACTCAGGCTAAATTTGATTCTGAAATGCCATGTGAAACCGTCAGTAAGATCCACTTG
GTTGATCTTGCCGGAAGTGAGCGTGCAGATGCCACCGGAGCCACCGGGGTTAGGCTAAAGGAAGGGGGAAATATT
AACAAGTCCCTCGTGACTCTGGGGAACGTCATTTCTGCCTTAGCTGATTTATCTCAGGATGCTGCAAATACTCTT
GCAAAGAAGAAGCAAGTTTTCGTGCCTTACAGGGATTCTGTGTTGACTTGGTTGTTAAAAGATAGCCTTGGAGGA
AACTCTAAAACTATCATGATTGCCACCATTTCACCTGCTGATGTCAATTATGGAGAAACCCTAAGTACTCTTCGC
TATGCAAATAGAGCCAAAAACATCATCAACAAGCCTACCATTAATGAGGATGCCAACGTCAAACTTATCCGTGAG
CTGCGAGCTGAAATAGCCAGACTGAAAACGCTGCTTGCTCAAGGGAATCAGATTGCCCTCTTAGACTCCCCCACA
TATACAGATATTGAAATGAACAGATTGGGAAAGGGCGCCCCCGGCTCCGCCGGCTCCGCCGCCGGCTCCGGCATG
GCCTCAAACGATTATACCCAACAAGCAACCCAAAGCTATGGGGCCTACCCCACCCAGCCCGGGCAGGGCTATTCC
CAGCAGAGCAGTCAGCCCTACGGACAGCAGAGTTACAGTGGTTATAGCCAGTCCACGGACACTTCAGGATATGGC
CAGAGCAGCTATTCTTCTTATGGCCAGAGCCAGAACACAGGCTATGGAACTCAGTCAACTCCCCAGGGATATGGC
TCGACTGGCGGCTATGGCAGTAGCCAGAGCTCCCAATCGTCTTACGGGCAGCAGTCCTCCTACCCTGGCTATGGC
CAGCAGCCAGCTCCCAGCAGCACCTCGGGAAGTTACGGTAGCAGTTCTCAGAGCAGCAGCTATGGGCAGCCCCAG
AGTGGGAGCTACAGCCAGCAGCCTAGCTATGGTGGACAGCAGCAAAGCTATGGACAGCAGCAAAGCTATAATCCC
CCTCAGGGCTATGGACAGCAGAACCAGTACAACAGCAGCAGTGGTGGTGGAGGTGGAGGTGGAGGTGGAGGTAAC
TATGGCCAAGATCAATCCTCCATGAGTAGTGGTGGTGGCAGTGGTGGCGGTTATGGCAATCAAGACCAGAGTGGT
GGAGGTGGCAGCGGTGGCTATGGACAGCAGGACCGTGGAGGCCGCGGCAGGGGTGGCAGTGGTGGCGGCGGCGGC
GGCGGCGGTGGTGGTTACAACCGCAGCAGTGGTGGCTATGAACCCAGAGGTCGTGGAGGTGGCCGTGGAGGCAGA
GGTGGCATGGGCGGAAGTGACCGTGGTGGCTTCAATAAATTTGGTGGCCCTCGGGACCAAGGATCACGTCATGAC
TCCGAACAGGATAATTCAGACAACAACACCATCTTTGTGCAAGGCCTGGGTGAGAATGTTACAATTGAGTCTGTG
GCTGATTACTTCAAGCAGATTGGTATTATTAAGACAAACAAGAAAACGGGACAGCCCATGATTAATTTGTACACA
GACAGGGAAACTGGCAAGCTGAAGGGAGAGGCAACGGTCTCTTTTGATGACCCACCTTCAGCTAAAGCAGCTATT

GACTGGTTTGATGGTAAAGAATTCTCCGGAAATCCTATCAAGGTCTCATTTGCTACTCGCCGGGCAGACTTTAAT
CGGGGTGGTGGCAATGGTCGTGGAGGCCGAGGGCGAGGAGGACCCATGGGCCGTGGAGGCTATGGAGGTGGTGGC
AGTGGTGGTGGTGGCCGAGGAGGATTTCCCAGTGGAGGTGGTGGCGGTGGAGGACAGCAGCGAGCTGGTGACTGG
AAGTGTCCTAATCCCACCTGTGAGAATATGAACTTCTCTTGGAGGAATGAATGCAACCAGTGTAAGGCCCCTAAA
CCAGATGGCCCAGGAGGGGGACCAGGTGGCTCTCACATGGGGGGTAACTACGGGGATGATCGTCGTGGTGGCAGA
GGAGGCGGCGCCCCCGGCTCCGCCGGCTCCGCCGCCGGCTCCGGCATGGCGTGCCCGGTGCCGCTGCAGCTGCCG
CCGCTGGAACGCCTGACCCTGGATGACAAAAAACCGCTGAATACCCTGATCTCTGCTACTGGTCTGTGGATGAGT
CGTACCGGAACCATTCATAAAATCAAACACCACGAGGTTAGCCGTTCGAAAATCTATATTGAGATGGCGTGTGGC
GATCATCTGGTTGTGAACAATAGCCGCTCTTCTCGTACAGCACGTGCACTGCGTCACCACAAATATCGTAAAACC
TGTAAACGTTGCCGTGTGTCCGATGAGGATCTGAACAAATTCCTGACAAAAGCCAATGAGGACCAAACAAGCGTG
AAAGTGAAAGTCGTTAGCGCTCCTACCCGTACTAAAAAAGCAATGCCGAAATCCGTTGCTCGTGCCCCTAAACCA
CTGGAAAACACTGAAGCAGCACAGGCACAGCCGTCTGGAAGCAAATTCTCTCCGGCCATTCCTGTTTCTACCCAG
GAGTCCGTTTCTGTTCCAGCAAGTGTGAGCACCAGCATTAGCAGTATTAGCACCGGTGCCACCGCTAGCGCCCTG
GTTAAAGGCAATACCAATCCGATTACAAGCATGTCTGCCCCGGTTCAAGCATCAGCTCCAGCACTGACAAAATCC
CAAACCGATCGTCTGGAGGTTCTGCTGAATCCGAAAGACGAAATCAGCCTGAATTCCGGCAAACCGTTTCGTGAA
CTGGAGAGCGAACTGCTGTCACGTCGTAAAAAAGACCTGCAACAAATCTATGCCGAAGAACGTGAGAACTATCTG
GGGAAACTGGAACGTGAAATCACCCGCTTTTTCGTGGATCGTGGCTTTCTGGAGATCAAATCCCCGATTCTGATT
CCTCTGGAGTATATCGAGCGTATGGGCATCGACAATGATACCGAACTGAGCAAACAAATTTTCCGTGTGGATAAA
AACTTCTGTCTGCGCCCTATGCTGGCACCAAATCTGTATAACTATCTGCGCAAACTGGACCGTGCCCTGCCTGAT
CCTATCAAAATCTTCGAGATCGGCCCGTGTTATCGTAAAGAGTCCGACGGTAAAGAACATCTGGAGGAGTTTACC
ATGCTGGCCTTTGCCCAAATGGGTTCAGGTTGTACTCGTGAGAACCTGGAAAGCATCATCACCGATTTTCTGAAC
CACCTGGGCATTGACTTCAAAATTGTGGGCGACAGCTGTATGGTGTATGGCGACACCCTGGATGTCATGCACGGC
GACCTGGAACTGTCTAGTGCCGTTGTTGGACCAATTCCGCTGGACCGTGAGTGGGGTATCGACAAACCGTGGATC
GGAGCAGGATTCGGTCTGGAACGCCTGCTGAAAGTGAAACACGACTTCAAAAACATCAAACGTGCCGCCCGTTCT
GAATCGTATTATAACGGGATCTCTACGAACCTGTAA (SEQ ID NO:53)
Protein:
MASVKVAVRVRPMNRREKDLEAKFIIQMEKSKTTITNLKIPEGGTGDSGRERTKTFTYDFSFYSADTKSPDYVSQ
EMVFKTLGTDVVKSAFEGYNACVFAYGQTGSGKSYTMMGNSGDSGLIPRICEGLFSRINETTRWDEASFRTEVSY
LEIYNERVRDLLRRKSSKTFNLRVREHPKEGPYVEDLSKHLVQNYGDVEELMDAGNINRTTAATGMNDVSSRSHA
IFTIKFTQAKFDSEMPCETVSKIHLVDLAGSERADATGATGVRLKEGGNINKSLVTLGNVISALADLSQDAANTL
AKKKQVFVPYRDSVLTWLLKDSLGGNSKTIMIATISPADVNYGETLSTLRYANRAKNIINKPTINEDANVKLIRE
LRAEIARLKTLLAQGNQIALLDSPTYTDIEMNRLGKGAPGSAGSAAGSGMASNDYTQQATQSYGAYPTQPGQGYS
QQSSQPYGQQSYSGYSQSTDTSGYGQSSYSSYGQSQNTGYGTQSTPQGYGSTGGYGSSQSSQSSYGQQSSYPGYG
QQPAPSSTSGSYGSSSQSSSYGQPQSGSYSQQPSYGGQQQSYGQQQSYNPPQGYGQQNQYNSSSGGGGGGGGGN
YGQDQSSMSSGGGSGGGYGNQDQSGGGGSGGYGQQDRGGRGRGGSGGGGGGGGGGYNRSSGGYEPRGRGGGRGGR
GGMGGSDRGGFNKFGGPRDQGSRHDSEQDNSDNNTIFVQGLGENVTIESVADYFKQIGIIKTNKKTGQPMINLYT
DRETGKLKGEATVSFDDPPSAKAAIDWFDGKEFSGNPIKVSFATRRADFNRGGGNGRGGRGRGGPMGRGGYGGGG
SGGGGRGGFPSGGGGGGGQQRAGDWKCPNPTCENMNFSWRNECNQCKAPKPDGPGGGPGGSHMGGNYGDDRRGGR
GGGAPGSAGSAAGSGMACPVPLQLPPLERLTLDDKKPLNTLISATGLWMSRTGTIHKIKHHEVSRSKIYIEMACG
DHLVVNNSRSSRTARALRHHKYRKTCKRCRVSDEDLNKFLTKANEDQTSVKVKVVSAPTRTKKAMPKSVARAPKP
LENTEAAQAQPSGSKFSPAIPVSTQESVSVPASVSTSISSISTGATASALVKGNTNPITSMSAPVQASAPALTKS
QTDRLEVLLNPKDEISLNSGKPFRELESELLSRRKKDLQQIYAEERENYLGKLEREITRFFVDRGFLEIKSPILI
PLEYIERMGIDNDTELSKQIFRVDKNFCLRPMLAPNLYNYLRKLDRALPDPIKIFEIGPCYRKESDGKEHLEEFT
MLAFAQMGSGCTRENLESIITDFLNHLGIDFKIVGDSCMVYGDTLDVMHGDLELSSAVVGPIPLDREWGIDKPWI
GAGFGLERLLKVKHDFKNIKRAARSESYYNGISTNL (SEQ ID NO:54)

KIF16B-FUS-PylRS^AAAF
DNA:
ATGGCATCGGTCAAGGTGGCCGTGAGGGTCCGGCCCATGAATCGCAGGGAAAAGGACTTGGAGGCCAAGTTCATT
ATTCAGATGGAGAAAAGCAAAACGACAATCACAAACTTAAAGATACCAGAAGGAGGCACTGGGGACTCAGGAAGA
GAACGGACCAAGACCTTCACCTATGACTTTTCTTTTTATTCTGCTGATACAAAAAGCCCAGATTACGTTTCACAA
GAAATGGTTTTCAAAACCCTCGGCACAGATGTCGTGAAGTCTGCATTTGAAGGTTATAATGCTTGTGTCTTTGCA
TATGGGCAAACTGGATCTGGAAAGTCATACACTATGATGGGAAATTCTGGAGATTCTGGCTTAATACCTCGGATC
TGTGAAGGACTCTTCAGTCGGATAAATGAAACCACCAGATGGGATGAAGCTTCTTTTCGAACTGAAGTCAGCTAC
TTAGAAATTTATAACGAACGTGTGAGAGATCTACTTCGGCGGAAGTCATCTAAAACCTTCAATTTGAGAGTCCGT
GAGCATCCCAAAGAAGGCCCTTATGTTGAGGATTTATCCAAACATTTAGTACAGAATTATGGTGACGTAGAAGAA
CTTATGGATGCGGGCAATATCAACCGGACCACCGCAGCGACTGGGATGAACGACGTCAGTAGCAGGTCTCATGCC
ATCTTCACCATCAAGTTCACTCAGGCTAAATTTGATTCTGAAATGCCATGTGAAACCGTCAGTAAGATCCACTTG

GTTGATCTTGCCGGAAGTGAGCGTGCAGATGCCACCGGAGCCACCGGGGTTAGGCTAAAGGAAGGGGGAAATATT
AACAAGTCCCTCGTGACTCTGGGGAACGTCATTTCTGCCTTAGCTGATTTATCTCAGGATGCTGCAAATACTCTT
GCAAAGAAGAAGCAAGTTTTCGTGCCTTACAGGGATTCTGTGTTGACTTGGTTGTTAAAAGATAGCCTTGGAGGA
AACTCTAAAACTATCATGATTGCCACCATTTCACCTGCTGATGTCAATTATGGAGAAACCCTAAGTACTCTTCGC
TATGCAAATAGAGCCAAAAACATCATCAACAAGCCTACCATTAATGAGGATGCCAACGTCAAACTTATCCGTGAG
CTGCGAGCTGAAATAGCCAGACTGAAAACGCTGCTTGCTCAAGGGAATCAGATTGCCCTCTTAGACTCCCCCACA
TATACAGATATTGAAATGAACAGATTGGGAAAGGGCGCCCCCGGCTCCGCCGGCTCCGCCGCCGGCTCCGGCATG
GCCTCAAACGATTATACCCAACAAGCAACCCAAAGCTATGGGGCCTACCCCACCCAGCCCGGGCAGGGCTATTCC
CAGCAGAGCAGTCAGCCCTACGGACAGCAGAGTTACAGTGGTTATAGCCAGTCCACGGACACTTCAGGATATGGC
CAGAGCAGCTATTCTTCTTATGGCCAGAGCCAGAACACAGGCTATGGAACTCAGTCAACTCCCCAGGGATATGGC
TCGACTGGCGGCTATGGCAGTAGCCAGAGCTCCCAATCGTCTTACGGGCAGCAGTCCTCCTACCCTGGCTATGGC
CAGCAGCCAGCTCCCAGCAGCACCTCGGGAAGTTACGGTAGCAGTTCTCAGAGCAGCAGCTATGGGCAGCCCCAG
AGTGGGAGCTACAGCCAGCAGCCTAGCTATGGTGGACAGCAGCAAAGCTATGGACAGCAGCAAAGCTATAATCCC
CCTCAGGGCTATGGACAGCAGAACCAGTACAACAGCAGCAGTGGTGGTGGAGGTGGAGGTGGAGGTGGAGGTAAC
TATGGCCAAGATCAATCCTCCATGAGTAGTGGTGGTGGCAGTGGTGGCGGTTATGGCAATCAAGACCAGAGTGGT
GGAGGTGGCAGCGGTGGCTATGGACAGCAGGACCGTGGAGGCCGCGGCAGGGGTGGCAGTGGTGGCGGCGGCGGC
GGCGGCGGTGGTGGTTACAACCGCAGCAGTGGTGGCTATGAACCCAGAGGTCGTGGAGGTGGCCGTGGAGGCAGA
GGTGGCATGGGCGGAAGTGACCGTGGTGGCTTCAATAAATTTGGTGGCCCTCGGGACCAAGGATCACGTCATGAC
TCCGAACAGGATAATTCAGACAACAACACCATCTTTGTGCAAGGCCTGGGTGAGAATGTTACAATTGAGTCTGTG
GCTGATTACTTCAAGCAGATTGGTATTATTAAGACAAACAAGAAAACGGGACAGCCCATGATTAATTTGTACACA
GACAGGGAAACTGGCAAGCTGAAGGGAGAGGCAACGGTCTCTTTTGATGACCCACCTTCAGCTAAAGCAGCTATT
GACTGGTTTGATGGTAAAGAATTCTCCGGAAATCCTATCAAGGTCTCATTTGCTACTCGCCGGGCAGACTTTAAT
CGGGGTGGTGGCAATGGTCGTGGAGGCCGAGGGCGAGGAGGACCCATGGGCCGTGGAGGCTATGGAGGTGGTGGC
AGTGGTGGTGGTGGCCGAGGAGGATTTCCCAGTGGAGGTGGTGGCGGTGGAGGACAGCAGCGAGCTGGTGACTGG
AAGTGTCCTAATCCCACCTGTGAGAATATGAACTTCTCTTGGAGGAATGAATGCAACCAGTGTAAGGCCCCTAAA
CCAGATGGCCCAGGAGGGGGACCAGGTGGCTCTCACATGGGGGGTAACTACGGGGATGATCGTCGTGGTGGCAGA
GGAGGCGATTACAAGGATGACGACGATAAGGGTACCGGCGCCCCCGGCTCCGCCGGCTCCGCCGCCGGCTCCGGC
GCGTGCCCGGTGCCGCTGCAGCTGCCGCCGCTGGAACGCCTGACCCTGGATGATAAAAAACCGCTGAATACCCTG
ATCTCTGCTACTGGTCTGTGGATGAGTCGTACCGGAACCATTCATAAAATCAAACACCACGAGGTTAGCCGTTCG
AAAATCTATATTGAGATGGCGTGTGGCGATCATCTGGTTGTGAACAATAGCCGCTCTTCTCGTACAGCACGTGCA
CTGCGTCACCACAAATATCGTAAAACCTGTAAACGTTGCCGTGTGTCCGATGAGGATCTGAACAAATTCCTGACA
AAAGCCAATGAGGACCAAACAAGCGTGAAAGTGAAAGTCGTTAGCGCTCCTACCCGTACTAAAAAAGCAATGCCG
AAATCCGTTGCTCGTGCCCCTAAACCACTGGAAAACACTGAAGCAGCACAGGCACAGCCGTCTGGAAGCAAATTC
TCTCCGGCCATTCCTGTTTCTACCCAGGAGTCCGTTTCTGTTCCAGCAAGTGTGAGCACCAGCATTAGCAGTATT
AGCACCGGTGCCACCGCTAGCGCCCTGGTTAAAGGCAATACCAATCCGATTACAAGCATGTCTGCCCCGGTTCAA
GCATCAGCTCCAGCACTGACAAAATCCCAAACCGATCGTCTGGAGGTTCTGCTGAATCCGAAAGACGAAATCAGC
CTGAATTCCGGCAAACCGTTTCGTGAACTGGAGAGCGAACTGCTGTCACGTCGTAAAAAAGACCTGCAACAAATC
TATGCCGAAGAACGTGAGAACTATCTGGGGAAACTGGAACGTGAAATCACCCGCTTTTTCGTGGATCGTGGCTTT
CTGGAGATCAAATCCCCGATTCTGATTCCTCTGGAGTATATCGAGCGTATGGGCATCGACAATGATACCGAACTG
AGCAAACAAATTTTCCGTGTGGATAAAAACTTCTGTCTGCGCCCTATGCTAGCACCAAATCTGGCTAACTATCTG
CGCAAACTGGACCGTGCCCTGCCTGATCCTATCAAAATCTTCGAGATCGGCCCGTGTTATCGTAAAGAGTCCGAC
GGTAAAGAACATCTGGAGGAGTTTACCATGCTGGCCTTTGCCCAAATGGGTTCAGGTTGTACTCGTGAGAACCTG
GAAAGCATCATCACCGATTTTCTGAACCACCTGGGCATTGACTTCAAAATTGTGGGCGACAGCTGTATGGTGTTT
GGCGACACCCTGGATGTCATGCACGGCGACCTGGAACTGTCTAGTGCCGTTGTGGGCCCAATCCCGCTGGATCGT
GAGTGGGGTATCGACAAACCTTGGATCGGTGCGGGTTTTGGTCTGGAGCGTCTGCTGAAAGTAAAACACGACTTC
AAGAACATCAAACGTGCTGCACGTTCCGAGTCCTATTACAATGGTATTTCTACTAACCTGTAA (SEQ ID
NO:55)
Protein:
MASVKVAVRVRPMNRREKDLEAKFIIQMEKSKTTITNLKIPEGGTGDSGRERTKTFTYDFSFYSADTKSPDYVSQ
EMVFKTLGTDVVKSAFEGYNACVFAYGQTGSGKSYTMMGNSGDSGLIPRICEGLFSRINETTRWDEASFRTEVSY
LEIYNERVRDLLRRKSSKTFNLRVREHPKEGPYVEDLSKHLVQNYGDVEELMDAGNINRTTAATGMNDVSSRSHA
IFTIKFTQAKFDSEMPCETVSKIHLVDLAGSERADATGATGVRLKEGGNINKSLVTLGNVISALADLSQDAANTL
AKKKQVFVPYRDSVLTWLLKDSLGGNSKTIMIATISPADVNYGETLSTLRYANRAKNIINKPTINEDANVKLIRE
LRAEIARLKTLLAQGNQIALLDSPTYTDIEMNRLGKGAPGSAGSAAGSGMASNDYTQQATQSYGAYPTQPGQGYS
QQSSQPYGQQSYSGYSQSTDTSGYGQSSYSSYGQSQNTGYGTQSTPQGYGSTGGYGSSQSSQSSYGQQSSYPGYG
QQPAPSSTSGSYGSSSQSSSYGQPQSGSYSQQPSYGGQQQSYGQQQSYNPPQGYGQQNQYNSSSGGGGGGGGGN
YGQDQSSMSSGGGSGGGYGNQDQSGGGGSGGYGQQDRGGRGRGGSGGGGGGGGGYNRSSGGYEPRGRGGGRGGR
GGMGGSDRGGFNKFGGPRDQGSRHDSEQDNSDNNTIFVQGLGENVTIESVADYFKQIGIIKTNKKTGQPMINLYT

DRETGKLKGEATVSFDDPPSAKAAIDWFDGKEFSGNPIKVSFATRRADFNRGGGNGRGGRGRGGPMGRGGYGGGG
SGGGGRGGFPSGGGGGGGQQRAGDWKCPNPTCENMNFSWRNECNQCKAPKPDGPGGGPGGSHMGGNYGDDRRGGR
GGDYKDDDDKGTGAPGSAGSAAGSGACPVPLQLPPLERLTLDDKKPLNTLISATGLWMSRTGTIHKIKHHEVSRS
KIYIEMACGDHLVVNNSRSSRTARALRHHKYRKTCKRCRVSDEDLNKFLTKANEDQTSVKVKVVSAPTRTKKAMP
KSVARAPKPLENTEAAQAQPSGSKFSPAIPVSTQESVSVPASVSTSISSISTGATASALVKGNTNPITSMSAPVQ
ASAPALTKSQTDRLEVLLNPKDEISLNSGKPFRELESELLSRRKKDLQQIYAEERENYLGKLEREITRFFVDRGF
LEIKSPILIPLEYIERMGIDNDTELSKQIFRVDKNFCLRPMLAPNLANYLRKLDRALPDPIKIFEIGPCYRKESD
GKEHLEEFTMLAFAQMGSGCTRENLESIITDFLNHLGIDFKIVGDSCMVFGDTLDVMHGDLELSSAVVGPIPLDR
EWGIDKPWIGAGFGLERLLKVKHDFKNIKRAARSESYYNGISTNL (SEQ ID NO:56)

KIF16B-EWSR1-MCP
DNA:
ATGGCATCGGTCAAGGTGGCCGTGAGGGTCCGGCCCATGAATCGCAGGGAAAAGGACTTGGAGGCCAAGTTCATT
ATTCAGATGGAGAAAAGCAAAACGACAATCACAAACTTAAAGATACCAGAAGGAGGCACTGGGGACTCAGGAAGA
GAACGGACCAAGACCTTCACCTATGACTTTTCTTTTTATTCTGCTGATACAAAAAGCCCAGATTACGTTTCACAA
GAAATGGTTTTCAAAACCCTCGGCACAGATGTCGTGAAGTCTGCATTTGAAGGTTATAATGCTTGTGTCTTTGCA
TATGGGCAAACTGGATCTGGAAAGTCATACACTATGATGGGAAATTCTGGAGATTCTGGCTTAATACCTCGGATC
TGTGAAGGACTCTTCAGTCGGATAAATGAAACCACCAGATGGGATGAAGCTTCTTTTCGAACTGAAGTCAGCTAC
TTAGAAATTTATAACGAACGTGTGAGAGATCTACTTCGGCGGAAGTCATCTAAAACCTTCAATTTGAGAGTCCGT
GAGCATCCCAAAGAAGGCCCTTATGTTGAGGATTTATCCAAACATTTAGTACAGAATTATGGTGACGTAGAAGAA
CTTATGGATGCGGGCAATATCAACCGGACCACCGCAGCGACTGGGATGAACGACGTCAGTAGCAGGTCTCATGCC
ATCTTCACCATCAAGTTCACTCAGGCTAAATTTGATTCTGAAATGCCATGTGAAACCGTCAGTAAGATCCACTTG
GTTGATCTTGCCGGAAGTGAGCGTGCAGATGCCACCGGAGCCACCGGGGTTAGGCTAAAGGAAGGGGGAAATATT
AACAAGTCCCTCGTGACTCTGGGGAACGTCATTTCTGCCTTAGCTGATTTATCTCAGGATGCTGCAAATACTCTT
GCAAAGAAGAAGCAAGTTTTCGTGCCTTACAGGGATTCTGTGTTGACTTGGTTGTTAAAAGATAGCCTTGGAGGA
AACTCTAAAACTATCATGATTGCCACCATTTCACCTGCTGATGTCAATTATGGAGAAACCCTAAGTACTCTTCGC
TATGCAAATAGAGCCAAAAACATCATCAACAAGCCTACCATTAATGAGGATGCCAACGTCAAACTTATCCGTGAG
CTGCGAGCTGAAATAGCCAGACTGAAAACGCTGCTTGCTCAAGGGAATCAGATTGCCCTCTTAGACTCCCCCACA
ATGGCGTCCACGGATTACAGTACCTATAGCCAAGCTGCAGCGCAGCAGGGCTACAGTGCTTACACCGCCCAGCCC
ACTCAAGGATATGCACAGACCACCCAGGCATATGGGCAACAAAGCTATGGAACCTATGGACAGCCCACTGATGTC
AGCTATACCCAGGCTCAGACCACTGCAACCTATGGGCAGACCGCCTATGCAACTTCTTATGGACAGCCTCCCACT
GGTTATACTACTCCAACTGCCCCCCAGGCATACAGCCAGCCTGTCCAGGGGTATGGCACTGGTGCTTATGATACC
ACCACTGCTACAGTCACCACCACCCAGGCCTCCTATGCAGCTCAGTCTGCATATGGCACTCAGCCTGCTTATCCA
GCCTATGGGCAGCAGCCAGCAGCCACTGCACCTACAAGACCGCAGGATGGAAACAAGCCCACTGAGACTAGTCAA
CCTCAATCTAGCACAGGGGGTTACAACCAGCCCAGCCTAGGATATGGACAGAGTAACTACAGTTATCCCCAGGTA
CCTGGGAGCTACCCCATGCAGCCAGTCACTGCACCTCCATCCTACCCTCCTACCAGCTATTCCTCTACACAGCCG
ACTAGTTATGATCAGAGCAGTTACTCTCAGCAGAACACCTATGGGCAACCGAGCAGCTATGGACAGCAGAGTAGC
TATGGTCAACAAAGCAGCTATGGGCAGCAGCCTCCCACTAGTTACCCACCCCAAACTGGATCCTACAGCCAAGCT
CCAAGTCAATATAGCCAACAGAGCAGCAGCTACGGGCAGCAGAGTTCATTCCGACAGGACCACCCCAGTAGCATG
GGTGTTTATGGGCAGGAGTCTGGAGGATTTTCCGGACCAGGAGAGAACCGGAGCATGAGTGGCCCTGATAACCGG
GGCAGGGGAAGAGGGGGATTTGATCGTGGAGGCATGAGCAGAGGTGGGCGGGGAGGAGGACGCGGTGGAATGGGC
AGCGCTGGAGAGCGAGGTGGCTTCAATAAGCCTGGTGGACCCATGGATGAAGGACCAGATCTTGATCTAGGCCCA
CCTGTAGATCCAGATGAAGACTCTGACAACAGTGCAATTTATGTACAAGGATTAAATGACAGTGTGACTCTAGAT
GATCTGGCAGACTTCTTTAAGCAGTGTGGGGTTGTTAAGATGAACAAGAGAACTGGGCAACCCATGATCCACATC
TACCTGGACAAGGAAACAGGAAAGCCCAAAGGCGATGCCACAGTGTCCTATGAAGACCCACCTACTGCCAAGGCT
GCCGTGGAATGGTTTGATGGGAAAGATTTTCAAGGGAGCAAACTTAAAGTCTCCCTTGCTCGGAAGAAGCCTCCA
ATGAACAGTATGCGGGGTGGTCTGCCACCCCGTGAGGGCAGAGGCATGCCACCACCACTCCGTGGAGGTCCAGGA
GGCCCAGGAGGTCCTGGGGGACCCATGGGTCGCATGGGAGGCCGTGGAGGAGATAGAGGAGGCTTCCCTCCAAGA
GGACCCCGGGGTTCCCGAGGGAACCCCTCTGGAGGAGGAAACGTCCAGCACCGAGCTGGAGACTGGCAGTGTCCC
AATCCGGGTTGTGGAAACCAGAACTTCGCCTGGAGAACAGAGTGCAACCAGTGTAAGGCCCCAAAGCCTGAAGGC
TTCCTCCCGCCACCCTTTCCGCCCCCGGGTGGTGATCGTGGCAGAGGTGGCCCTGGTGGCATGCGGGGAGGAAGA
GGTGGCCTCATGGATCGTGGTGGTCCCGGTGGAATGTTCAGAGGTGGCCGTGGTGGAGACAGAGGTGGCTTCCGT
GGTGGCCGGGGCATGGACCGAGGTGGCTTTGGTGGAGGAAGACGAGGTGGCCCTGGGGGGCCCCCTGGACCTTTG
ATGGAACAGGATTACAAGGATGACGACGATAAGGGTACCGAGCAGAAGCTGATCTCAGAGGAGGACCTGGGCGCC
CCCGGCTCCGCCGGCTCCGCCGCCGGCTCCGGCGCTTCTAACTTTACTCAGTTCGTTCTCGTCGACAATGGCGGA
ACTGGCGACGTGACTGTCGCCCCAAGCAACTTCGCTAACGGGATCGCTGAATGGATCAGCTCTAACTCGCGTTCA
CAGGCTTACAAAGTAACCTGTAGCGTTCGTCAGAGCTCTGCGCAGAATCGCAAATACACCATCAAAGTCGAGGTG
CCTAAAGGCGCCTGGCGTTCGTACTTAAATATGGAACTAACCATTCCAATTTTCGCCACGAATTCCGACTGCGAG

CTTATTGTTAAGGCAATGCAAGGTCTCCTAAAAGATGGAAACCCGATTCCCTCAGCAATCGCAGCAAACTCCGGC
ATCTACTAA (SEQ ID NO:57)
Protein:
MASVKVAVRVRPMNRREKDLEAKFIIQMEKSKTTITNLKIPEGGTGDSGRERTKTFTYDFSFYSADTKSPDYVSQ
EMVFKTLGTDVVKSAFEGYNACVFAYGQTGSGKSYTMMGNSGDSGLIPRICEGLFSRINETTRWDEASFRTEVSY
LEIYNERVRDLLRRKSSKTFNLRVREHPKEGPYVEDLSKHLVQNYGDVEELMDAGNINRTTAATGMNDVSSRSHA
IFTIKFTQAKFDSEMPCETVSKIHLVDLAGSERADATGATGVRLKEGGNINKSLVTLGNVISALADLSQDAANTL
AKKKQVFVPYRDSVLTWLLKDSLGGNSKTIMIATISPADVNYGETLSTLRYANRAKNIINKPTINEDANVKLIRE
LRAEIARLKTLLAQGNQIALLDSPTMASTDYSTYSQAAAQQGYSAYTAQPTQGYAQTTQAYGQQSYGTYGQPTDV
SYTQAQTTATYGQTAYATSYGQPPTGYTTPTAPQAYSQPVQGYGTGAYDTTTATVTTTQASYAAQSAYGTQPAYP
AYGQQPAATAPTRPQDGNKPTETSQPQSSTGGYNQPSLGYGQSNYSYPQVPGSYPMQPVTAPPSYPPTSYSSTQP
TSYDQSSYSQQNTYGQPSSYGQQSSYGQQSSYGQQPPTSYPPQTGSYSQAPSQYSQQSSSYGQQSSFRQDHPSSM
GVYGQESGGFSGPGENRSMSGPDNRGRGRGGFDRGGMSRGGRGGGRGGMGSAGERGGFNKPGGPMDEGPDLDLGP
PVDPDEDSDNSAIYVQGLNDSVTLDDLADFFKQCGVVKMNKRTGQPMIHIYLDKETGKPKGDATVSYEDPPTAKA
AVEWFDGKDFQGSKLKVSLARKKPPMNSMRGGLPPREGRGMPPPLRGGPGGPGGPGGPMGRMGGRGGDRGGFPPR
GPRGSRGNPSGGGNVQHRAGDWQCPNPGCGNQNFAWRTECNQCKAPKPEGFLPPPFPPPGGDRGRGGPGGMRGGR
GGLMDRGGPGGMFRGGRGGDRGGFRGGRGMDRGGFGGGRRGGPGGPPGPLMEQDYKDDDDKGTEQKLISEEDLGA
PGSAGSAAGSGASNFTQFVLVDNGGTGDVTVAPSNFANGIAEWISSNSRSQAYKVTCSVRQSSAQNRKYTIKVEV
PKGAWRSYLNMELTIPIFATNSDCELIVKAMQGLLKDGNPIPSAIAANSGIY (SEQ ID NO:58)

KIF16B-FUS-4xλN22-PylRSAF
DNA:
ATGGCATCGGTCAAGGTGGCCGTGAGGGTCCGGCCCATGAATCGCAGGGAAAAGGACTTGGAGGCCAAGTTCATT
ATTCAGATGGAGAAAAGCAAAACGACAATCACAAACTTAAAGATACCAGAAGGAGGCACTGGGGACTCAGGAAGA
GAACGGACCAAGACCTTCACCTATGACTTTTCTTTTTATTCTGCTGATACAAAAAGCCCAGATTACGTTTCACAA
GAAATGGTTTTCAAAACCCTCGGCACAGATGTCGTGAAGTCTGCATTTGAAGGTTATAATGCTTGTGTCTTTGCA
TATGGGCAAACTGGATCTGGAAAGTCATACACTATGATGGGAAATTCTGGAGATTCTGGCTTAATACCTCGGATC
TGTGAAGGACTCTTCAGTCGGATAAATGAAACCACCAGATGGGATGAAGCTTCTTTTCGAACTGAAGTCAGCTAC
TTAGAAATTTATAACGAACGTGTGAGAGATCTACTTCGGCGGAAGTCATCTAAAACCTTCAATTTGAGAGTCCGT
GAGCATCCCAAAGAAGGCCCTTATGTTGAGGATTTATCCAAACATTTAGTACAGAATTATGGTGACGTAGAAGAA
CTTATGGATGCGGGCAATATCAACCGGACCACCGCAGCGACTGGGATGAACGACGTCAGTAGCAGGTCTCATGCC
ATCTTCACCATCAAGTTCACTCAGGCTAAATTTGATTCTGAAATGCCATGTGAAACCGTCAGTAAGATCCACTTG
GTTGATCTTGCCGGAAGTGAGCGTGCAGATGCCACCGGAGCCACCGGGGTTAGGCTAAAGGAAGGGGGAAATATT
AACAAGTCCCTCGTGACTCTGGGGAACGTCATTTCTGCCTTAGCTGATTTATCTCAGGATGCTGCAAATACTCTT
GCAAAGAAGAAGCAAGTTTTCGTGCCTTACAGGGATTCTGTGTTGACTTGGTTGTTAAAAGATAGCCTTGGAGGA
AACTCTAAAACTATCATGATTGCCACCATTTCACCTGCTGATGTCAATTATGGAGAAACCCTAAGTACTCTTCGC
TATGCAAATAGAGCCAAAAACATCATCAACAAGCCTACCATTAATGAGGATGCCAACGTCAAACTTATCCGTGAG
CTGCGAGCTGAAATAGCCAGACTGAAAACGCTGCTTGCTCAAGGGAATCAGATTGCCCTCTTAGACTCCCCCACA
TATACAGATATTGAAATGAACAGATTGGGAAAGGGCGCCCCCGGCTCCGCCGGCTCCGCCGCCGGCTCCGGCATG
GCCTCAAACGATTATACCCAACAAGCAACCCAAAGCTATGGGGCCTACCCCACCCAGCCCGGGCAGGGCTATTCC
CAGCAGAGCAGTCAGCCCTACGGACAGCAGAGTTACAGTGGTTATAGCCAGTCCACGGACACTTCAGGATATGGC
CAGAGCAGCTATTCTTCTTATGGCCAGAGCCAGAACACAGGCTATGGAACTCAGTCAACTCCCCAGGGATATGGC
TCGACTGGCGGCTATGGCAGTAGCCAGAGCTCCCAATCGTCTTACGGGCAGCAGTCCTCCTACCCTGGCTATGGC
CAGCAGCCAGCTCCCAGCAGCACCTCGGGAAGTTACGGTAGCAGTTCTCAGAGCAGCAGCTATGGGCAGCCCCAG
AGTGGGAGCTACAGCCAGCAGCCTAGCTATGGTGGACAGCAGCAAAGCTATGGACAGCAGCAAAGCTATAATCCC
CCTCAGGGCTATGGACAGCAGAACCAGTACAACAGCAGCAGTGGTGGTGGAGGTGGAGGTGGAGGTGGAGGTAAC
TATGGCCAAGATCAATCCTCCATGAGTAGTGGTGGTGGCAGTGGTGGCGGTTATGGCAATCAAGACCAGAGTGGT
GGAGGTGGCAGCGGTGGCTATGGACAGCAGGACCGTGGAGGCCGCGGCAGGGGTGGCAGTGGTGGCGGCGGCGGC
GGCGGCGGTGGTGGTTACAACCGCAGCAGTGGTGGCTATGAACCCAGAGGTCGTGGAGGTGGCCGTGGAGGCAGA
GGTGGCATGGGCGGAAGTGACCGTGGTGGCTTCAATAAATTTGGTGGCCCTCGGGACCAAGGATCACGTCATGAC
TCCGAACAGGATAATTCAGACAACAACACCATCTTTGTGCAAGGCCTGGGTGAGAATGTTACAATTGAGTCTGTG
GCTGATTACTTCAAGCAGATTGGTATTATTAAGACAAACAAGAAAACGGGACAGCCCATGATTAATTTGTACACA
GACAGGGAAACTGGCAAGCTGAAGGGAGAGGCAACGGTCTCTTTTGATGACCCACCTTCAGCTAAAGCAGCTATT
GACTGGTTTGATGGTAAAGAATTCTCCGGAAATCCTATCAAGGTCTCATTTGCTACTCGCCGGGCAGACTTTAAT
CGGGGTGGTGGCAATGGTCGTGGAGGCCGAGGGCGAGGAGGACCCATGGGCCGTGGAGGCTATGGAGGTGGTGGC
AGTGGTGGTGGTGGCCGAGGAGGATTTCCCAGTGGAGGTGGTGGCGGTGGAGGACAGCAGCGAGCTGGTGACTGG
AAGTGTCCTAATCCCACCTGTGAGAATATGAACTTCTCTTGGAGGAATGAATGCAACCAGTGTAAGGCCCCTAAA
CCAGATGGCCCAGGAGGGGGACCAGGTGGCTCTCACATGGGGGGTAACTACGGGGATGATCGTCGTGGTGGCAGA

GGAGGCGCCACCATGGACGCACAAACACGACGACGTGAGCGTCGCGCTGAGAAACAAGCTCAATGGAAAGCTGCA
AACCCACCGCTCGACGGAGCCGGAGCTGGCGCTGGAGCTGGAGCCGGAGCTGGCGGTCTAGCCACCATGGACGCA
CAAACACGACGACGTGAGCGTCGCGCTGAGAAACAAGCTCAATGGAAAGCTGCAAACCCACCGCTCGACGGAGCC
GGAGCTGGCGCTGGAGCTGGAGCCGGAGCTGGCGGTCTAGCCACCATGGACGCACAAACACGACGACGTGAGCGT
CGCGCTGAGAAACAAGCTCAATGGAAAGCTGCAAACCCACCGCTCGACGGAGCCGGAGCTGGCGCTGGAGCTGGA
GCCGGAGCTGGCGGTCTAGCCACCATGGACGCACAAACACGACGACGTGAGCGTCGCGCTGAGAAACAAGCTCAA
TGGAAAGCTGCAAACCCACCGCTCGATTACAAGGATGACGACGATAAGGGTACCGGCGCCCCCGGCTCCGCCGGC
TCCGCCGCCGGCTCCGGCGCGTGCCCGGTGCCGCTGCAGCTGCCGCCGCTGGAACGCCTGACCCTGGATGATAAA
AAACCGCTGAATACCCTGATCTCTGCTACTGGTCTGTGGATGAGTCGTACCGGAACCATTCATAAAATCAAACAC
CACGAGGTTAGCCGTTCGAAAATCTATATTGAGATGGCGTGTGGCGATCATCTGGTTGTGAACAATAGCCGCTCT
TCTCGTACAGCACGTGCACTGCGTCACCACAAATATCGTAAAACCTGTAAACGTTGCCGTGTGTCCGATGAGGAT
CTGAACAAATTCCTGACAAAAGCCAATGAGGACCAAACAAGCGTGAAAGTGAAAGTCGTTAGCGCTCCTACCCGT
ACTAAAAAAGCAATGCCGAAATCCGTTGCTCGTGCCCCTAAACCACTGGAAAACACTGAAGCAGCACAGGCACAG
CCGTCTGGAAGCAAATTCTCTCCGGCCATTCCTGTTTCTACCCAGGAGTCCGTTTCTGTTCCAGCAAGTGTGAGC
ACCAGCATTAGCAGTATTAGCACCGGTGCCACCGCTAGCGCCCTGGTTAAAGGCAATACCAATCCGATTACAAGC
ATGTCTGCCCCGGTTCAAGCATCAGCTCCAGCACTGACAAAATCCCAAACCGATCGTCTGGAGGTTCTGCTGAAT
CCGAAAGACGAAATCAGCCTGAATTCCGGCAAACCGTTTCGTGAACTGGAGAGCGAACTGCTGTCACGTCGTAAA
AAAGACCTGCAACAAATCTATGCCGAAGAACGTGAGAACTATCTGGGGAAACTGGAACGTGAAATCACCCGCTTT
TTCGTGGATCGTGGCTTTCTGGAGATCAAATCCCCGATTCTGATTCCTCTGGAGTATATCGAGCGTATGGGCATC
GACAATGATACCGAACTGAGCAAACAAATTTTCCGTGTGGATAAAAACTTCTGTCTGCGCCCTATGCTAGCACCA
AATCTGGCTAACTATCTGCGCAAACTGGACCGTGCCCTGCCTGATCCTATCAAAATCTTCGAGATCGGCCCGTGT
TATCGTAAAGAGTCCGACGGTAAAGAACATCTGGAGGAGTTTACCATGCTGAACTTTTGCCAAATGGGTTCAGGT
TGTACTCGTGAGAACCTGGAAAGCATCATCACCGATTTTCTGAACCACCTGGGCATTGACTTCAAAATTGTGGGC
GACAGCTGTATGGTGTTTGGCGACACCCTGGATGTCATGCACGGCGACCTGGAACTGTCTAGTGCCGTTGTGGGC
CCAATCCCGCTGGATCGTGAGTGGGGTATCGACAAACCTTGGATCGGTGCGGGTTTTGGTCTGGAGCGTCTGCTG
AAAGTAAAACACGACTTCAAGAACATCAAACGTGCTGCACGTTCCGAGTCCTATTACAATGGTATTTCTACTAAC
CTGTAA (SEQ ID NO:59)
Protein:
MASVKVAVRVRPMNRREKDLEAKFIIQMEKSKTTITNLKIPEGGTGDSGRERTKTFTYDFSFYSADTKSPDYVSQ
EMVFKTLGTDVVKSAFEGYNACVFAYGQTGSGKSYTMMGNSGDSGLIPRICEGLFSRINETTRWDEASFRTEVSY
LEIYNERVRDLLRRKSSKTFNLRVREHPKEGPYVEDLSKHLVQNYGDVEELMDAGNINRTTAATGMNDVSSRSHA
IFTIKFTQAKFDSEMPCETVSKIHLVDLAGSERADATGATGVRLKEGGNINKSLVTLGNVISALADLSQDAANTL
AKKKQVFVPYRDSVLTWLLKDSLGGNSKTIMIATISPADVNYGETLSTLRYANRAKNIINKPTINEDANVKLIRE
LRAEIARLKTLLAQGNQIALLDSPTYTDIEMNRLGKGAPGSAGSAAGSGMASNDYTQQATQSYGAYPTQPGQGYS
QQSSQPYGQQSYSGYSQSTDTSGYGQSSYSSYGQSQNTGYGTQSTPQGYGSTGGYGSSQSSQSSYGQQSSYPGYG
QQPAPSSTSGSYGSSSQSSSYGQPQSGSYSQQPSYGGQQQSYGQQQSYNPPQGYGQQNQYNSSSGGGGGGGGGGN
YGQDQSSMSSGGGSGGGYGNQDQSGGGGSGGYGQQDRGGRGRGGSGGGGGGGGGGYNRSSGGYEPRGRGGGRGGR
GGMGGSDRGGFNKFGGPRDQGSRHDSEQDNSDNNTIFVQGLGENVTIESVADYFKQIGIIKTNKKTGQPMINLYT
DRETGKLKGEATVSFDDPPSAKAAIDWFDGKEFSGNPIKVSFATRRADFNRGGGNGRGGRGRGGPMGRGGYGGGG
SGGGGRGGFPSGGGGGGGQQRAGDWKCPNPTCENMNFSWRNECNQCKAPKPDGPGGGPGGSHMGGNYGDDRRGGR
GGATMDAQTRRRERRAEKQAQWKAANPPLDGAGAGAGAGAGAGGLATMDAQTRRRERRAEKQAQWKAANPPLDGA
GAGAGAGAGAGGLATMDAQTRRRERRAEKQAQWKAANPPLDGAGAGAGAGAGAGGLATMDAQTRRRERRAEKQAQ
WKAANPPLDYKDDDDKGTGAPGSAGSAAGSGACPVPLQLPPLERLTLDDKKPLNTLISATGLWMSRTGTIHKIKH
HEVSRSKIYIEMACGDHLVVNNSRSSRTARALRHHKYRKTCKRCRVSDEDLNKFLTKANEDQTSVKVKVVSAPTR
TKKAMPKSVARAPKPLENTEAAQAQPSGSKFSPAIPVSTQESVSVPASVSTSISSISTGATASALVKGNTNPITS
MSAPVQASAPALTKSQTDRLEVLLNPKDEISLNSGKPFRELESELLSRRKKDLQQIYAEERENYLGKLEREITRF
FVDRGFLEIKSPILIPLEYIERMGIDNDTELSKQIFRVDKNFCLRPMLAPNLANYLRKLDRALPDPIKIFEIGPC
YRKESDGKEHLEEFTMLNFCQMGSGCTRENLESIITDFLNHLGIDFKIVGDSCMVFGDTLDVMHGDLELSSAVVG
PIPLDREWGIDKPWIGAGFGLERLLKVKHDFKNIKRAARSESYYNGISTNL (SEQ ID NO:60)

KIF16B-FUS-MCP-PylRS^AF
DNA:
ATGGCATCGGTCAAGGTGGCCGTGAGGGTCCGGCCCATGAATCGCAGGGAAAAGGACTTGGAGGCCAAGTTCATT
ATTCAGATGGAGAAAAGCAAAACGACAATCACAAACTTAAAGATACCAGAAGGAGGCACTGGGGACTCAGGAAGA
GAACGGACCAAGACCTTCACCTATGACTTTTCTTTTTATTCTGCTGATACAAAAAGCCCAGATTACGTTTCACAA
GAAATGGTTTTCAAAACCCTCGGCACAGATGTCGTGAAGTCTGCATTTGAAGGTTATAATGCTTGTGTCTTTGCA
TATGGGCAAACTGGATCTGGAAAGTCATACACTATGATGGGAAATTCTGGAGATTCTGGCTTAATACCTCGGATC
TGTGAAGGACTCTTCAGTCGGATAAATGAAACCACCAGATGGGATGAAGCTTCTTTTCGAACTGAAGTCAGCTAC

TTAGAAATTTATAACGAACGTGTGAGAGATCTACTTCGGCGGAAGTCATCTAAAACCTTCAATTTGAGAGTCCGT
GAGCATCCCAAAGAAGGCCCTTATGTTGAGGATTTATCCAAACATTTAGTACAGAATTATGGTGACGTAGAAGAA
CTTATGGATGCGGGCAATATCAACCGGACCACCGCAGCGACTGGGATGAACGACGTCAGTAGCAGGTCTCATGCC
ATCTTCACCATCAAGTTCACTCAGGCTAAATTTGATTCTGAAATGCCATGTGAAACCGTCAGTAAGATCCACTTG
GTTGATCTTGCCGGAAGTGAGCGTGCAGATGCCACCGGAGCCACCGGGGTTAGGCTAAAGGAAGGGGGAAATATT
AACAAGTCCCTCGTGACTCTGGGGAACGTCATTTCTGCCTTAGCTGATTTATCTCAGGATGCTGCAAATACTCTT
GCAAAGAAGAAGCAAGTTTTCGTGCCTTACAGGGATTCTGTGTTGACTTGGTTGTTAAAAGATAGCCTTGGAGGA
AACTCTAAAACTATCATGATTGCCACCATTTCACCTGCTGATGTCAATTATGGAGAAACCCTAAGTACTCTTCGC
TATGCAAATAGAGCCAAAAACATCATCAACAAGCCTACCATTAATGAGGATGCCAACGTCAAACTTATCCGTGAG
CTGCGAGCTGAAATAGCCAGACTGAAAACGCTGCTTGCTCAAGGGAATCAGATTGCCCTCTTAGACTCCCCCACA
TATACAGATATTGAAATGAACAGATTGGGAAAGGGCGCCCCCGGCTCCGCCGGCTCCGCCGCCGGCTCCGGCATG
GCCTCAAACGATTATACCCAACAAGCAACCCAAAGCTATGGGGCCTACCCCACCCAGCCCGGGCAGGGCTATTCC
CAGCAGAGCAGTCAGCCCTACGGACAGCAGAGTTACAGTGGTTATAGCCAGTCCACGGACACTTCAGGATATGGC
CAGAGCAGCTATTCTTCTTATGGCCAGAGCCAGAACACAGGCTATGGAACTCAGTCAACTCCCCAGGGATATGGC
TCGACTGGCGGCTATGGCAGTAGCCAGAGCTCCCAATCGTCTTACGGGCAGCAGTCCTCCTACCCTGGCTATGGC
CAGCAGCCAGCTCCCAGCAGCACCTCGGGAAGTTACGGTAGCAGTTCTCAGAGCAGCAGCTATGGGCAGCCCCAG
AGTGGGAGCTACAGCCAGCAGCCTAGCTATGGTGGACAGCAGCAAAGCTATGGACAGCAGCAAAGCTATAATCCC
CCTCAGGGCTATGGACAGCAGAACCAGTACAACAGCAGCAGTGGTGGTGGAGGTGGAGGTGGAGGTGGAGGTAAC
TATGGCCAAGATCAATCCTCCATGAGTAGTGGTGGTGGCAGTGGTGGCGGTTATGGCAATCAAGACCAGAGTGGT
GGAGGTGGCAGCGGTGGCTATGGACAGCAGGACCGTGGAGGCCGCGGCAGGGGTGGCAGTGGTGGCGGCGGCGGC
GGCGGCGGTGGTGGTTACAACCGCAGCAGTGGTGGCTATGAACCCAGAGGTCGTGGAGGTGGCCGTGGAGGCAGA
GGTGGCATGGGCGGAAGTGACCGTGGTGGCTTCAATAAATTTGGTGGCCCTCGGGACCAAGGATCACGTCATGAC
TCCGAACAGGATAATTCAGACAACAACACCATCTTTGTGCAAGGCCTGGGTGAGAATGTTACAATTGAGTCTGTG
GCTGATTACTTCAAGCAGATTGGTATTATTAAGACAAACAAGAAAACGGGACAGCCCATGATTAATTTGTACACA
GACAGGGAAACTGGCAAGCTGAAGGGAGAGGCAACGGTCTCTTTTGATGACCCACCTTCAGCTAAAGCAGCTATT
GACTGGTTTGATGGTAAAGAATTCTCCGGAAATCCTATCAAGGTCTCATTTGCTACTCGCCGGGCAGACTTTAAT
CGGGGTGGTGGCAATGGTCGTGGAGGCCGAGGGCGAGGAGGACCCATGGGCCGTGGAGGCTATGGAGGTGGTGGC
AGTGGTGGTGGTGGCCGAGGAGGATTTCCCAGTGGAGGTGGTGGCGGTGGAGGACAGCAGCGAGCTGGTGACTGG
AAGTGTCCTAATCCCACCTGTGAGAATATGAACTTCTCTTGGAGGAATGAATGCAACCAGTGTAAGGCCCCTAAA
CCAGATGGCCCAGGAGGGGGACCAGGTGGCTCTCACATGGGGGGTAACTACGGGGATGATCGTCGTGGTGGCAGA
GGAGGCGATTACAAGGATGACGACGATAAGGGTACCGGCGCCCCCGGCTCCGCCGGCTCCGCCGCCGGCTCCGGC
GCTTCTAACTTTACTCAGTTCGTTCTCGTCGACAATGGCGGAACTGGCGACGTGACTGTCGCCCCAAGCAACTTC
GCTAACGGGATCGCTGAATGGATCAGCTCTAACTCGCGTTCACAGGCTTACAAAGTAACCTGTAGCGTTCGTCAG
AGCTCTGCGCAGAATCGCAAATACACCATCAAAGTCGAGGTGCCTAAAGGCGCCTGGCGTTCGTACTTAAATATG
GAACTAACCATTCCAATTTTCGCCACGAATTCCGACTGCGAGCTTATTGTTAAGGCAATGCAAGGTCTCCTAAAA
GATGGAAACCCGATTCCCTCAGCAATCGCAGCAAACTCCGGCATCTACGGTACCGGCGCCCCCGGCTCCGCCGGC
TCCGCCGCCGGCTCCGGCGCGTGCCCGGTGCCGCTGCAGCTGCCGCCGCTGGAACGCCTGACCCTGGATGATAAA
AAACCGCTGAATACCCTGATCTCTGCTACTGGTCTGTGGATGAGTCGTACCGGAACCATTCATAAAATCAAACAC
CACGAGGTTAGCCGTTCGAAAATCTATATTGAGATGGCGTGTGGCGATCATCTGGTTGTGAACAATAGCCGCTCT
TCTCGTACAGCACGTGCACTGCGTCACCACAAATATCGTAAAACCTGTAAACGTTGCCGTGTGTCCGATGAGGAT
CTGAACAAATTCCTGACAAAAGCCAATGAGGACCAAACAAGCGTGAAAGTGAAAGTCGTTAGCGCTCCTACCCGT
ACTAAAAAAGCAATGCCGAAATCCGTTGCTCGTGCCCCTAAACCACTGGAAAACACTGAAGCAGCACAGGCACAG
CCGTCTGGAAGCAAATTCTCTCCGGCCATTCCTGTTTCTACCCAGGAGTCCGTTTCTGTTCCAGCAAGTGTGAGC
ACCAGCATTAGCAGTATTAGCACCGGTGCCACCGCTAGCGCCCTGGTTAAAGGCAATACCAATCCGATTACAAGC
ATGTCTGCCCCGGTTCAAGCATCAGCTCCAGCACTGACAAAATCCCAAACCGATCGTCTGGAGGTTCTGCTGAAT
CCGAAAGACGAAATCAGCCTGAATTCCGGCAAACCGTTTCGTGAACTGGAGAGCGAACTGCTGTCACGTCGTAAA
AAAGACCTGCAACAAATCTATGCCGAAGAACGTGAGAACTATCTGGGGAAACTGGAACGTGAAATCACCCGCTTT
TTCGTGGATCGTGGCTTTCTGGAGATCAAATCCCCGATTCTGATTCCTCTGGAGTATATCGAGCGTATGGGCATC
GACAATGATACCGAACTGAGCAAACAAATTTTCCGTGTGGATAAAAACTTCTGTCTGCGCCCTATGCTAGCACCA
AATCTGGCTAACTATCTGCGCAAACTGGACCGTGCCCTGCCTGATCCTATCAAAATCTTCGAGATCGGCCCGTGT
TATCGTAAAGAGTCCGACGGTAAAGAACATCTGGAGGAGTTTACCATGCTGAACTTTTGCCAAATGGGTTCAGGT
TGTACTCGTGAGAACCTGGAAAGCATCATCACCGATTTTCTGAACCACCTGGGCATTGACTTCAAAATTGTGGGC
GACAGCTGTATGGTGTTTGGCGACACCCTGGATGTCATGCACGGCGACCTGGAACTGTCTAGTGCCGTTGTGGGC
CCAATCCCGCTGGATCGTGAGTGGGGTATCGACAAACCTTGGATCGGTGCGGGTTTTGGTCTGGAGCGTCTGCTG
AAAGTAAAACACGACTTCAAGAACATCAAACGTGCTGCACGTTCCGAGTCCTATTACAATGGTATTTCTACTAAC
CTGTAA (SEQ ID NO:61)
Protein:

MASVKVAVRVRPMNRREKDLEAKFIIQMEKSKTTITNLKIPEGGTGDSGRERTKTFTYDFSFYSADTKSPDYVSQ
EMVFKTLGTDVVKSAFEGYNACVFAYGQTGSGKSYTMMGNSGDSGLIPRICEGLFSRINETTRWDEASFRTEVSY
LEIYNERVRDLLRRKSSKTFNLRVREHPKEGPYVEDLSKHLVQNYGDVEELMDAGNINRTTAATGMNDVSSRSHA
IFTIKFTQAKFDSEMPCETVSKIHLVDLAGSERADATGATGVRLKEGGNINKSLVTLGNVISALADLSQDAANTL
AKKKQVFVPYRDSVLTWLLKDSLGGNSKTIMIATISPADVNYGETLSTLRYANRAKNIINKPTINEDANVKLIRE
LRAEIARLKTLLAQGNQIALLDSPTYTDIEMNRLGKGAPGSAGSAAGSGMASNDYTQQATQSYGAYPTQPGQGYS
QQSSQPYGQQSYSGYSQSTDTSGYGQSSYSSYGQSQNTGYGTQSTPQGYGSTGGYGSSQSSQSSYGQQSSYPGYG
QQPAPSSTSGSYGSSSQSSSYGQPQSGSYSQQPSYGGQQQSYGQQQSYNPPQGYGQQNQYNSSSGGGGGGGGGN
YGQDQSSMSSGGGSGGGYGNQDQSGGGGSGGYGQQDRGGRGRGGSGGGGGGGGGGYNRSSGGYEPRGRGGGRGGR
GGMGGSDRGGFNKFGGPRDQGSRHDSEQDNSDNNTIFVQGLGENVTIESVADYFKQIGIIKTNKKTGQPMINLYT
DRETGKLKGEATVSFDDPPSAKAAIDWFDGKEFSGNPIKVSFATRRADFNRGGGNGRGGRGRGGPMGRGGYGGGG
SGGGGRGGFPSGGGGGGGQQRAGDWKCPNPTCENMNFSWRNECNQCKAPKPDGPGGGPGGSHMGGNYGDDRRGGR
GGDYKDDDDKGTGAPGSAGSAAGSGASNFTQFVLVDNGGTGDVTVAPSNFANGIAEWISSNSRSQAYKVTCSVRQ
SSAQNRKYTIKVEVPKGAWRSYLNMELTIPIFATNSDCELIVKAMQGLLKDGNPIPSAIAANSGIYGTGAPGSAG
SAAGSGACPVPLQLPPLERLTLDDKKPLNTLISATGLWMSRTGTIHKIKHHEVSRSKIYIEMACGDHLVVNNSRS
SRTARALRHHKYRKTCKRCRVSDEDLNKFLTKANEDQTSVKVKVVSAPTRTKKAMPKSVARAPKPLENTEAAQAQ
PSGSKFSPAIPVSTQESVSVPASVSTSISSISTGATASALVKGNTNPITSMSAPVQASAPALTKSQTDRLEVLLN
PKDEISLNSGKPFRELESELLSRRKKDLQQIYAEERENYLGKLEREITRFFVDRGFLEIKSPILIPLEYIERMGI
DNDTELSKQIFRVDKNFCLRPMLAPNLANYLRKLDRALPDPIKIFEIGPCYRKESDGKEHLEEFTMLNFCQMGSG
CTRENLESIITDFLNHLGIDFKIVGDSCMVFGDTLDVMHGDLELSSAVVGPIPLDREWGIDKPWIGAGFGLERLL
KVKHDFKNIKRAARSESYYNGISTNL (SEQ ID NO:62)

KIF16B-PylRS^AF
DNA:
ATGGCATCGGTCAAGGTGGCCGTGAGGGTCCGGCCCATGAATCGCAGGGAAAAGGACTTGGAGGCCAAGTTCATT
ATTCAGATGGAGAAAAGCAAAACGACAATCACAAACTTAAAGATACCAGAAGGAGGCACTGGGGACTCAGGAAGA
GAACGGACCAAGACCTTCACCTATGACTTTTCTTTTTATTCTGCTGATACAAAAAGCCCAGATTACGTTTCACAA
GAAATGGTTTTCAAAACCCTCGGCACAGATGTCGTGAAGTCTGCATTTGAAGGTTATAATGCTTGTGTCTTTGCA
TATGGGCAAACTGGATCTGGAAAGTCATACACTATGATGGGAAATTCTGGAGATTCTGGCTTAATACCTCGGATC
TGTGAAGGACTCTTCAGTCGGATAAATGAAACCACCAGATGGGATGAAGCTTCTTTTCGAACTGAAGTCAGCTAC
TTAGAAATTTATAACGAACGTGTGAGAGATCTACTTCGGCGGAAGTCATCTAAAACCTTCAATTTGAGAGTCCGT
GAGCATCCCAAAGAAGGCCCTTATGTTGAGGATTTATCCAAACATTTAGTACAGAATTATGGTGACGTAGAAGAA
CTTATGGATGCGGGCAATATCAACCGGACCACCGCAGCGACTGGGATGAACGACGTCAGTAGCAGGTCTCATGCC
ATCTTCACCATCAAGTTCACTCAGGCTAAATTTGATTCTGAAATGCCATGTGAAACCGTCAGTAAGATCCACTTG
GTTGATCTTGCCGGAAGTGAGCGTGCAGATGCCACCGGAGCCACCGGGGTTAGGCTAAAGGAAGGGGGAAATATT
AACAAGTCCCTCGTGACTCTGGGGAACGTCATTTCTGCCTTAGCTGATTTATCTCAGGATGCTGCAAATACTCTT
GCAAAGAAGAAGCAAGTTTTCGTGCCTTACAGGGATTCTGTGTTGACTTGGTTGTTAAAAGATAGCCTTGGAGGA
AACTCTAAAACTATCATGATTGCCACCATTTCACCTGCTGATGTCAATTATGGAGAAACCCTAAGTACTCTTCGC
TATGCAAATAGAGCCAAAAACATCATCAACAAGCCTACCATTAATGAGGATGCCAACGTCAAACTTATCCGTGAG
CTGCGAGCTGAAATAGCCAGACTGAAAACGCTGCTTGCTCAAGGGAATCAGATTGCCCTCTTAGACTCCCCCACA
TATACAGATATTGAAATGAACAGATTGGGAAAGGGCGCCCCCGGCTCCGCCGGCTCCGCCGGCTCCGCCGCCGGC
TCCGGCATGGCGTGCCCGGTGCCGCTGCAGCTGCCGCCGCTGGAACGCCTGACCCTGGATGATAAAAAACCGCTG
AATACCCTGATCTCTGCTACTGGTCTGTGGATGAGTCGTACCGGAACCATTCATAAAATCAAACACCACGAGGTT
AGCCGTTCGAAAATCTATATTGAGATGGCGTGTGGCGATCATCTGGTTGTGAACAATAGCCGCTCTTCTCGTACA
GCACGTGCACTGCGTCACCACAAATATCGTAAAACCTGTAAACGTTGCCGTGTGTCCGATGAGGATCTGAACAAA
TTCCTGACAAAAGCCAATGAGGACCAAACAAGCGTGAAAGTGAAAGTCGTTAGCGCTCCTACCCGTACTAAAAAA
GCAATGCCGAAATCCGTTGCTCGTGCCCCTAAACCACTGGAAAACACTGAAGCAGCACAGGCACAGCCGTCTGGA
AGCAAATTCTCTCCGGCCATTCCTGTTTCTACCCAGGAGTCCGTTTCTGTTCCAGCAAGTGTGAGCACCAGCATT
AGCAGTATTAGCACCGGTGCCACCGCTAGCGCCCTGGTTAAAGGCAATACCAATCCGATTACAAGCATGTCTGCC
CCGGTTCAAGCATCAGCTCCAGCACTGACAAAATCCCAAACCGATCGTCTGGAGGTTCTGCTGAATCCGAAAGAC
GAAATCAGCCTGAATTCCGGCAAACCGTTTCGTGAACTGGAGAGCGAACTGCTGTCACGTCGTAAAAAAGACCTG
CAACAAATCTATGCCGAAGAACGTGAGAACTATCTGGGGAAACTGGAACGTGAAATCACCCGCTTTTTCGTGGAT
CGTGGCTTTCTGGAGATCAAATCCCCGATTCTGATTCCTCTGGAGTATATCGAGCGTATGGGCATCGACAATGAT
ACCGAACTGAGCAAACAAATTTTCCGTGTGGATAAAAACTTCTGTCTGCGCCCTATGCTAGCACCAAATCTGGCT
AACTATCTGCGCAAACTGGACCGTGCCCTGCCTGATCCTATCAAAATCTTCGAGATCGGCCCGTGTTATCGTAAA
GAGTCCGACGGTAAAGAACATCTGGAGGAGTTTACCATGCTGAACTTTTGCCAAATGGGTTCAGGTTGTACTCGT
GAGAACCTGGAAAGCATCATCACCGATTTTCTGAACCACCTGGGCATTGACTTCAAAATTGTGGGCGACAGCTGT
ATGGTGTTTGGCGACACCCTGGATGTCATGCACGGCGACCTGGAACTGTCTAGTGCCGTTGTGGGCCCAATCCCG

CTGGATCGTGAGTGGGGTATCGACAAACCTTGGATCGGTGCGGGTTTTGGTCTGGAGCGTCTGCTGAAAGTAAAA
CACGACTTCAAGAACATCAAACGTGCTGCACGTTCCGAGTCCTATTACAATGGTATTTCTACTAACCTGTAA
(SEQ ID NO:63)
Protein:
MASVKVAVRVRPMNRREKDLEAKFIIQMEKSKTTITNLKIPEGGTGDSGRERTKTFTYDFSFYSADTKSPDYVSQ
EMVFKTLGTDVVKSAFEGYNACVFAYGQTGSGKSYTMMGNSGDSGLIPRICEGLFSRINETTRWDEASFRTEVSY
LEIYNERVRDLLRRKSSKTFNLRVREHPKEGPYVEDLSKHLVQNYGDVEELMDAGNINRTTAATGMNDVSSRSHA
IFTIKFTQAKFDSEMPCETVSKIHLVDLAGSERADATGATGVRLKEGGNINKSLVTLGNVISALADLSQDAANTL
AKKKQVFVPYRDSVLTWLLKDSLGGNSKTIMIATISPADVNYGETLSTLRYANRAKNIINKPTINEDANVKLIRE
LRAEIARLKTLLAQGNQIALLDSPTYTDIEMNRLGKGAPGSAGSAGSAAGSGMACPVPLQLPPLERLTLDDKKPL
NTLISATGLWMSRTGTIHKIKHHEVSRSKIYIEMACGDHLVVNNSRSSRTARALRHHKYRKTCKRCRVSDEDLNK
FLTKANEDQTSVKVKVVSAPTRTKKAMPKSVARAPKPLENTEAAQAQPSGSKFSPAIPVSTQESVSVPASVSTSI
SSISTGATASALVKGNTNPITSMSAPVQASAPALTKSQTDRLEVLLNPKDEISLNSGKPFRELESELLSRRKKDL
QQIYAEERENYLGKLEREITRFFVDRGFLEIKSPILIPLEYIERMGIDNDTELSKQIFRVDKNFCLRPMLAPNLA
NYLRKLDRALPDPIKIFEIGPCYRKESDGKEHLEEFTMLNFCQMGSGCTRENLESIITDFLNHLGIDFKIVGDSC
MVFGDTLDVMHGDLELSSAVVGPIPLDREWGIDKPWIGAGFGLERLLKVKHDFKNIKRAARSESYYNGISTNL
(SEQ ID NO:64)

KIF16B-MCP
DNA:
ATGGCATCGGTCAAGGTGGCCGTGAGGGTCCGGCCCATGAATCGCAGGGAAAAGGACTTGGAGGCCAAGTTCATT
ATTCAGATGGAGAAAAGCAAAACGACAATCACAAACTTAAAGATACCAGAAGGAGGCACTGGGGACTCAGGAAGA
GAACGGACCAAGACCTTCACCTATGACTTTTCTTTTTATTCTGCTGATACAAAAAGCCCAGATTACGTTTCACAA
GAAATGGTTTTCAAAACCCTCGGCACAGATGTCGTGAAGTCTGCATTTGAAGGTTATAATGCTTGTGTCTTTGCA
TATGGGCAAACTGGATCTGGAAAGTCATACACTATGATGGGAAATTCTGGAGATTCTGGCTTAATACCTCGGATC
TGTGAAGGACTCTTCAGTCGGATAAATGAAACCACCAGATGGGATGAAGCTTCTTTTCGAACTGAAGTCAGCTAC
TTAGAAATTTATAACGAACGTGTGAGAGATCTACTTCGGCGGAAGTCATCTAAAACCTTCAATTTGAGAGTCCGT
GAGCATCCCAAAGAAGGCCCTTATGTTGAGGATTTATCCAAACATTTAGTACAGAATTATGGTGACGTAGAAGAA
CTTATGGATGCGGGCAATATCAACCGGACCACCGCAGCGACTGGGATGAACGACGTCAGTAGCAGGTCTCATGCC
ATCTTCACCATCAAGTTCACTCAGGCTAAATTTGATTCTGAAATGCCATGTGAAACCGTCAGTAAGATCCACTTG
GTTGATCTTGCCGGAAGTGAGCGTGCAGATGCCACCGGAGCCACCGGGGTTAGGCTAAAGGAAGGGGGAAATATT
AACAAGTCCCTCGTGACTCTGGGGAACGTCATTTCTGCCTTAGCTGATTTATCTCAGGATGCTGCAAATACTCTT
GCAAAGAAGAAGCAAGTTTTCGTGCCTTACAGGGATTCTGTGTTGACTTGGTTGTTAAAAGATAGCCTTGGAGGA
AACTCTAAAACTATCATGATTGCCACCATTTCACCTGCTGATGTCAATTATGGAGAAACCCTAAGTACTCTTCGC
TATGCAAATAGAGCCAAAAACATCATCAACAAGCCTACCATTAATGAGGATGCCAACGTCAAACTTATCCGTGAG
CTGCGAGCTGAAATAGCCAGACTGAAAACGCTGCTTGCTCAAGGGAATCAGATTGCCCTCTTAGACTCCCCCACA
TATACAGATATTGAAATGAACAGATTGGGAAAGGGCGCCCCCGGCTCCGCCGGCTCCGCCGCCGGCTCCGGCATG
GCTTCTAACTTTACTCAGTTCGTTCTCGTCGACAATGGCGGAACTGGCGACGTGACTGTCGCCCCAAGCAACTTC
GCTAACGGGATCGCTGAATGGATCAGCTCTAACTCGCGTTCACAGGCTTACAAAGTAACCTGTAGCGTTCGTCAG
AGCTCTGCGCAGAATCGCAAATACACCATCAAAGTCGAGGTGCCTAAAGGCGCCTGGCGTTCGTACTTAAATATG
GAACTAACCATTCCAATTTTCGCCACGAATTCCGACTGCGAGCTTATTGTTAAGGCAATGCAAGGTCTCCTAAAA
GATGGAAACCCGATTCCCTCAGCAATCGCAGCAAACTCCGGCATCTACTAA (SEQ ID NO:65)
Protein:
MASVKVAVRVRPMNRREKDLEAKFIIQMEKSKTTITNLKIPEGGTGDSGRERTKTFTYDFSFYSADTKSPDYVSQ
EMVFKTLGTDVVKSAFEGYNACVFAYGQTGSGKSYTMMGNSGDSGLIPRICEGLFSRINETTRWDEASFRTEVSY
LEIYNERVRDLLRRKSSKTFNLRVREHPKEGPYVEDLSKHLVQNYGDVEELMDAGNINRTTAATGMNDVSSRSHA
IFTIKFTQAKFDSEMPCETVSKIHLVDLAGSERADATGATGVRLKEGGNINKSLVTLGNVISALADLSQDAANTL
AKKKQVFVPYRDSVLTWLLKDSLGGNSKTIMIATISPADVNYGETLSTLRYANRAKNIINKPTINEDANVKLIRE
LRAEIARLKTLLAQGNQIALLDSPTYTDIEMNRLGKGAPGSAGSAAGSGMASNFTQFVLVDNGGTGDVTVAPSNF
ANGIAEWISSNSRSQAYKVTCSVRQSSAQNRKYTIKVEVPKGAWRSYLNMELTIPIFATNSDCELIVKAMQGLLK
DGNPIPSAIAANSGIY (SEQ ID NO:66)

KIF16B-MCP-PylRS^AF
DNA:
ATGGCATCGGTCAAGGTGGCCGTGAGGGTCCGGCCCATGAATCGCAGGGAAAAGGACTTGGAGGCCAAGTTCATT
ATTCAGATGGAGAAAAGCAAAACGACAATCACAAACTTAAAGATACCAGAAGGAGGCACTGGGGACTCAGGAAGA
GAACGGACCAAGACCTTCACCTATGACTTTTCTTTTTATTCTGCTGATACAAAAAGCCCAGATTACGTTTCACAA
GAAATGGTTTTCAAAACCCTCGGCACAGATGTCGTGAAGTCTGCATTTGAAGGTTATAATGCTTGTGTCTTTGCA

TATGGGCAAACTGGATCTGGAAAGTCATACACTATGATGGGAAATTCTGGAGATTCTGGCTTAATACCTCGGATC
TGTGAAGGACTCTTCAGTCGGATAAATGAAACCACCAGATGGGATGAAGCTTCTTTTCGAACTGAAGTCAGCTAC
TTAGAAATTTATAACGAACGTGTGAGAGATCTACTTCGGCGGAAGTCATCTAAAACCTTCAATTTGAGAGTCCGT
GAGCATCCCAAAGAAGGCCCTTATGTTGAGGATTTATCCAAACATTTAGTACAGAATTATGGTGACGTAGAAGAA
CTTATGGATGCGGGCAATATCAACCGGACCACCGCAGCGACTGGGATGAACGACGTCAGTAGCAGGTCTCATGCC
ATCTTCACCATCAAGTTCACTCAGGCTAAATTTGATTCTGAAATGCCATGTGAAACCGTCAGTAAGATCCACTTG
GTTGATCTTGCCGGAAGTGAGCGTGCAGATGCCACCGGAGCCACCGGGGTTAGGCTAAAGGAAGGGGGAAATATT
AACAAGTCCCTCGTGACTCTGGGGAACGTCATTTCTGCCTTAGCTGATTTATCTCAGGATGCTGCAAATACTCTT
GCAAAGAAGAAGCAAGTTTTCGTGCCTTACAGGGATTCTGTGTTGACTTGGTTGTTAAAAGATAGCCTTGGAGGA
AACTCTAAAACTATCATGATTGCCACCATTTCACCTGCTGATGTCAATTATGGAGAAACCCTAAGTACTCTTCGC
TATGCAAATAGAGCCAAAAACATCATCAACAAGCCTACCATTAATGAGGATGCCAACGTCAAACTTATCCGTGAG
CTGCGAGCTGAAATAGCCAGACTGAAAACGCTGCTTGCTCAAGGGAATCAGATTGCCCTCTTAGACTCCCCCACA
TATACAGATATTGAAATGAACAGATTGGGAAAGGGCGCCCCCGGCTCCGCCGGCTCCGCCGCCGGCTCCGGCATG
GCTTCTAACTTTACTCAGTTCGTTCTCGTCGACAATGGCGGAACTGGCGACGTGACTGTCGCCCCAAGCAACTTC
GCTAACGGGATCGCTGAATGGATCAGCTCTAACTCGCGTTCACAGGCTTACAAAGTAACCTGTAGCGTTCGTCAG
AGCTCTGCGCAGAATCGCAAATACACCATCAAAGTCGAGGTGCCTAAAGGCGCCTGGCGTTCGTACTTAAATATG
GAACTAACCATTCCAATTTTCGCCACGAATTCCGACTGCGAGCTTATTGTTAAGGCAATGCAAGGTCTCCTAAAA
GATGGAAACCCGATTCCCTCAGCAATCGCAGCAAACTCCGGCATCTACGGTACCGGCGCCCCCGGCTCCGCCGGC
TCCGCCGCCGGCTCCGGCGCGTGCCCGGTGCCGCTGCAGCTGCCGCCGCTGGAACGCCTGACCCTGGATGATAAA
AAACCGCTGAATACCCTGATCTCTGCTACTGGTCTGTGGATGAGTCGTACCGGAACCATTCATAAAATCAAACAC
CACGAGGTTAGCCGTTCGAAAATCTATATTGAGATGGCGTGTGGCGATCATCTGGTTGTGAACAATAGCCGCTCT
TCTCGTACAGCACGTGCACTGCGTCACCACAAATATCGTAAAACCTGTAAACGTTGCCGTGTGTCCGATGAGGAT
CTGAACAAATTCCTGACAAAAGCCAATGAGGACCAAACAAGCGTGAAAGTGAAAGTCGTTAGCGCTCCTACCCGT
ACTAAAAAAGCAATGCCGAAATCCGTTGCTCGTGCCCCTAAACCACTGGAAAACACTGAAGCAGCACAGGCACAG
CCGTCTGGAAGCAAATTCTCTCCGGCCATTCCTGTTTCTACCCAGGAGTCCGTTTCTGTTCCAGCAAGTGTGAGC
ACCAGCATTAGCAGTATTAGCACCGGTGCCACCGCTAGCGCCCTGGTTAAAGGCAATACCAATCCGATTACAAGC
ATGTCTGCCCCGGTTCAAGCATCAGCTCCAGCACTGACAAAATCCCAAACCGATCGTCTGGAGGTTCTGCTGAAT
CCGAAAGACGAAATCAGCCTGAATTCCGGCAAACCGTTTCGTGAACTGGAGAGCGAACTGCTGTCACGTCGTAAA
AAAGACCTGCAACAAATCTATGCCGAAGAACGTGAGAACTATCTGGGGAAACTGGAACGTGAAATCACCCGCTTT
TTCGTGGATCGTGGCTTTCTGGAGATCAAATCCCCGATTCTGATTCCTCTGGAGTATATCGAGCGTATGGGCATC
GACAATGATACCGAACTGAGCAAACAAATTTTCCGTGTGGATAAAAACTTCTGTCTGCGCCCTATGCTAGCACCA
AATCTGGCTAACTATCTGCGCAAACTGGACCGTGCCCTGCCTGATCCTATCAAAATCTTCGAGATCGGCCCGTGT
TATCGTAAAGAGTCCGACGGTAAAGAACATCTGGAGGAGTTTACCATGCTGAACTTTTGCCAAATGGGTTCAGGT
TGTACTCGTGAGAACCTGGAAAGCATCATCACCGATTTTCTGAACCACCTGGGCATTGACTTCAAAATTGTGGGC
GACAGCTGTATGGTGTTTGGCGACACCCTGGATGTCATGCACGGCGACCTGGAACTGTCTAGTGCCGTTGTGGGC
CCAATCCCGCTGGATCGTGAGTGGGGTATCGACAAACCTTGGATCGGTGCGGGTTTTGGTCTGGAGCGTCTGCTG
AAAGTAAAACACGACTTCAAGAACATCAAACGTGCTGCACGTTCCGAGTCCTATTACAATGGTATTTCTACTAAC
CTGTAA (SEQ ID NO:67)
Protein:
MASVKVAVRVRPMNRREKDLEAKFIIQMEKSKTTITNLKIPEGGTGDSGRERTKTFTYDFSFYSADTKSPDYVSQ
EMVFKTLGTDVVKSAFEGYNACVFAYGQTGSGKSYTMMGNSGDSGLIPRICEGLFSRINETTRWDEASFRTEVSY
LEIYNERVRDLLRRKSSKTFNLRVREHPKEGPYVEDLSKHLVQNYGDVEELMDAGNINRTTAATGMNDVSSRSHA
IFTIKFTQAKFDSEMPCETVSKIHLVDLAGSERADATGATGVRLKEGGNINKSLVTLGNVISALADLSQDAANTL
AKKKQVFVPYRDSVLTWLLKDSLGGNSKTIMIATISPADVNYGETLSTLRYANRAKNIINKPTINEDANVKLIRE
LRAEIARLKTLLAQGNQIALLDSPTYTDIEMNRLGKGAPGSAGSAAGSGMASNFTQFVLVDNGGTGDVTVAPSNF
ANGIAEWISSNSRSQAYKVTCSVRQSSAQNRKYTIKVEVPKGAWRSYLNMELTIPIFATNSDCELIVKAMQGLLK
DGNPIPSAIAANSGIYGTGAPGSAGSAAGSGACPVPLQLPPLERLTLDDKKPLNTLISATGLWMSRTGTIHKIKH
HEVSRSKIYIEMACGDHLVVNNSRSSRTARALRHHKYRKTCKRCRVSDEDLNKFLTKANEDQTSVKVKVVSAPTR
TKKAMPKSVARAPKPLENTEAAQAQPSGSKFSPAIPVSTQESVSVPASVSTSISSISTGATASALVKGNTNPITS
MSAPVQASAPALTKSQTDRLEVLLNPKDEISLNSGKPFRELESELLSRRKKDLQQIYAEERENYLGKLEREITRF
FVDRGFLEIKSPILIPLEYIERMGIDNDTELSKQIFRVDKNFCLRPMLAPNLANYLRKLDRALPDPIKIFEIGPC
YRKESDGKEHLEEFTMLNFCQMGSGCTRENLESIITDFLNHLGIDFKIVGDSCMVFGDTLDVMHGDLELSSAVVG
PIPLDREWGIDKPWIGAGFGLERLLKVKHDFKNIKRAARSESYYNGISTNL (SEQ ID NO:68)

KIF16B-SPD5-PylRS^AF
DNA:
ATGGCATCGGTCAAGGTGGCCGTGAGGGTCCGGCCCATGAATCGCAGGGAAAAGGACTTGGAGGCCAAGTTCATT
ATTCAGATGGAGAAAAGCAAAACGACAATCACAAACTTAAAGATACCAGAAGGAGGCACTGGGGACTCAGGAAGA

GAACGGACCAAGACCTTCACCTATGACTTTTCTTTTTATTCTGCTGATACAAAAAGCCCAGATTACGTTTCACAA
GAAATGGTTTTCAAAACCCTCGGCACAGATGTCGTGAAGTCTGCATTTGAAGGTTATAATGCTTGTGTCTTTGCA
TATGGGCAAACTGGATCTGGAAAGTCATACACTATGATGGGAAATTCTGGAGATTCTGGCTTAATACCTCGGATC
TGTGAAGGACTCTTCAGTCGGATAAATGAAACCACCAGATGGGATGAAGCTTCTTTTCGAACTGAAGTCAGCTAC
TTAGAAATTTATAACGAACGTGTGAGAGATCTACTTCGGCGGAAGTCATCTAAAACCTTCAATTTGAGAGTCCGT
GAGCATCCCAAAGAAGGCCCTTATGTTGAGGATTTATCCAAACATTTAGTACAGAATTATGGTGACGTAGAAGAA
CTTATGGATGCGGGCAATATCAACCGGACCACCGCAGCGACTGGGATGAACGACGTCAGTAGCAGGTCTCATGCC
ATCTTCACCATCAAGTTCACTCAGGCTAAATTTGATTCTGAAATGCCATGTGAAACCGTCAGTAAGATCCACTTG
GTTGATCTTGCCGGAAGTGAGCGTGCAGATGCCACCGGAGCCACCGGGGTTAGGCTAAAGGAAGGGGGAAATATT
AACAAGTCCCTcGTGACTCTGGGGAACGTCATTTCTGCCTTAGCTGATTTATCTCAGGATGCTGCAAATACTCTT
GCAAAGAAGAAGCAAGTTTTCGTGCCTTACAGGGATTCTGTGTTGACTTGGTTGTTAAAAGATAGCCTTGGAGGA
AACTCTAAAACTATCATGATTGCCACCATTTCACCTGCTGATGTCAATTATGGAGAAACCCTAAGTACTCTTCGC
TATGCAAATAGAGCCAAAAACATCATCAACAAGCCTACCATTAATGAGGATGCCAACGTCAAACTTATCCGTGAG
CTGCGAGCTGAAATAGCCAGACTGAAAACGCTGCTTGCTCAAGGGAATCAGATTGCCCTCTTAGACTCCCCCACA
ATGGAGGACAACAGCGTGCTGAACGAGGACAGCAACCTGGAGCACGTGGAGGGCCAGCCCAGAAGAAGCATGAGC
CAGCCCGTGCTGAACGTGGAGGGCGACAAGAGAACCAGCAGCACCAGCGCCACCCAGCAGCAGGTGCTGAGCGGC
GCCTTCAGCAGCGCCGACGTGAGAAGCATCCCCATCATCCAGACCTGGGAGGAGAACAAGGCCCTGAAGACCAAG
ATCACCATCCTGAGAGGCGAGCTGCAGATGTACCAGAGAAGATACAGCGAGGCCAAGGAGGCCAGCCAGAAGAGA
GTGAAGGAGGTGATGGACGACTACGTGGACCTGAAGCTGGGCCAGGAGAACGTGCAGGAGAAGATGGAGCAGTAC
AAGCTGATGGAGGAGGACCTGCTGGCCATGCAGAGCAGAATCGAGACCAGCGAGGACAACTTCGCCAGACAGATG
AAGGAGTTCGAGGCCCAGAAGCACGCCATGGAGGAGAGAATCAAGGAGCTGGAGCTGAGCGCCACCGACGCCAAC
AACACCACCGTGGGCAGCTTCAGAGGCACCCTGGACGACATCCTGAAGAAGAACGACCCCGACTTCACCCTGACC
AGCGGCTACGAGGAGAGAAAGATCAACGACCTGGAGGCCAAGCTGCTGAGCGAGATCGACAAGGTGGCCGAGCTG
GAGGACCACATCCAGCAGCTGAGACAGGAGCTGGACGACCAGAGCGCCAGACTGGCCGACAGCGAGAACGTGAGA
GCCCAGCTGGAGGCCGCCACCGGCCAGGGCATCCTGGGCGCCGCCGGCAACGCCATGGTGCCCAACAGCACCTTC
ATGATCGGCAACGGCAGAGAGAGCCAGACCAGAGACCAGCTGAACTACATCGACGACCTGGAGACCAAGCTGGCC
GACGCCAAGAAGGAGAACGACAAGGCCAGACAGGCCCTGGTGGAGTACATGAACAAGTGCAGCAAGCTGGAGCAC
GAGATCAGAACCATGGTGAAGAACAGCACCTTCGACAGCAGCAGCATGCTGCTGGGCGGCCAGACCAGCGACGAG
CTGAAGATCCAGATCGGCAAGGTGAACGGCGAGCTGAACGTGCTGAGAGCCGAGAACAGAGAGCTGAGAATCAGA
TGCGACCAGCTGACCGGCGGCGACGGCAACCTGAGCATCAGCCTGGGCCAGAGCAGACTGATGGCCGGCATCGCC
ACCAACGACGTGGACAGCATCGGCCAGGGCAACGAGACCGGCGGCACCAGCATGAGAATCCTGCCCAGAGAGAGC
CAGCTGGACGACCTGGAGGAGAGCAAGCTGCCCCTGATGGACACCAGCAGCGCCGTGAGAAACCAGCAGCAGTTC
GCCAGCATGTGGGAGGACTTCGAGAGCGTGAAGGACAGCCTGCAGAACAACCACAACGACACCCTGGAGGGCAGC
TTCAACAGCAGCATGCCCCCCCCCGGCAGAGACGCCACCCAGAGCTTCCTGAGCCAGAAGAGCTTCAAGAACAGC
CCCATCGTGATGCAGAAGCCCAAGAGCCTGCACCTGCACCTGAAGAGCCACCAGAGCGAGGGCGCCGGCGAGCAG
ATCCAGAACAACAGCTTCAGCACCAAGACCGCCAGCCCCCACGTGAGCCAGAGCCACATCCCCATCCTGCACGAC
ATGCAGCAGATCCTGGACAGCAGCGCCATGTTCCTGGAGGGCCAGCACGACGTGGCCGTGAACGTGGAGCAGATG
CAGGAGAAGATGAGCCAGATCAGAGAGGCCCTGGCCAGACTGTTCGAGAGACTGAAGAGCAGCGCCGCCCTGTTC
GAGGAGATCCTGGAGAGAATGGGCAGCAGCGACCCCAACGCCGACAAGATCAAGAAGATGAAGCTGGCCTTCGAG
ACCAGCATCAACGACAAGCTGAACGTGAGCGCCATCCTGGAGGCCGCCGAGAAGGACCTGCACAACATGAGCCTG
AACTTCAGCATCCTGGAGAAGAGCATCGTGAGCCAGGCCGCCGAGGCCAGCAGAAGATTCACCATCGCCCCCGAC
GCCGAGGACGTGGCCAGCAGCAGCCTGCTGAACGCCAGCTACAGCCCCCTGTTCAAGTTCACCAGCAACAGCGAC
ATCGTGGAGAAGCTGCAGAACGAGGTGAGCGAGCTGAAGAACGAGCTGGAGATGGCCAGAACCAGAGACATGAGA
AGCCCCCTGAACGGCAGCAGCGGCAGACTGAGCGACGTGCAGATCAACACCAACAGAATGTTCGAGGACCTGGAG
GTGAGCGAGGCCACCCTGCAGAAGGCCAAGGAGGAGAACAGCACCCTGAAGAGCCAGTTCGCCGAGCTGGAGGCC
AACCTGCACCAGGTGAACAGCAAGCTGGGCGAGGTGAGATGCGAGCTGAACGAGGCCCTGGCCAGAGTGGACGGC
GAGCAGGAGACCAGAGTGAAGGCCGAGAACGCCCTGGAGGAGGCCAGACAGCTGATCAGCAGCCTGAAGCACGAG
GAGAACGAGCTGAAGAAGACCATCACCGACATGGGCATGAGACTGAACGAGGCCAAGAAGAGCGACGAGTTCCTG
AAGAGCGAGCTGAGCACCGCCCTGGAGGAGGAGAAGAAGAGCCAGAACCTGGCCGACGAGCTGAGCGAGGAGCTG
AACGGCTGGAGAATGAGAACCAAGGAGGCCGAGAACAAGGTGGAGCACGCCAGCAGCGAGAAGAGCGAGATGCTG
GAGAGAATCGTGCACCTGGAGACCGAGATGGAGAAGCTGAGCACCAGCGAGATCGCCGCCGACTACTGCAGCACC
AAGATGACCGAGAGAAAGAAGGAGATCGAGCTGGCCAAGTACAGAGAGGACTTCGAGAACGCCGCCATCGTGGGC
CTGGAGAGAATCAGCAAGGAGATCAGCGAGCTGACCAAGAAGACCCTGAAGGCCAAGATCATCCCCAGCAACATC
AGCAGCATCCAGCTGGTGTGCGACGAGCTGTGCAGAAGACTGAGCAGAGAGAGAGAGCAGCAGCACGAGTACGCC
AAGGTGATGAGAGACGTGAACGAGAAGATCGAGAAGCTGCAGCTGGAGAAGGACGCCCTGGAGCACGAGCTGAAG
ATGATGAGCAGCAACAACGAGAACGTGCCCCCCGTGGGCACCAGCGTGAGCGGCATGCCCACCAAGACCAGCAAC
CAGAAGTGCGCCCAGCCCCACTACACCAGCCCCACCAGACAGCTGCTGCACGAGAGCACCATGGCCGTGGACGCC
ATCGTGCAGAAGCTGAAGAAGACCCACAACATGAGCGGCATGGGCCCCGAGCTGAAGGAGACCATCGGCAACGTG

ATCAACGAGAGCAGAGTGCTGAGAGACTTCCTGCACCAGAAGCTGATCCTGTTCAAGGGCATCGACATGAGCAAC
TGGAAGAACGAGACCGTGGACCAGCTGATCACCGACCTGGGCCAGCTGCACCAGGACAACCTGATGCTGGAGGAG
CAGATCAAGAAGTACAAGAAGGAGCTGAAGCTGACCAAGAGCGCCATCCCCACCCTGGGCGTGGAGTTCCAGGAC
AGAATCAAGACCGAGATCGGCAAGATCGCCACCGACATGGGCGGCGCCGTGAAGGAGATCAGAAAGAAGGGTACC
GGCGCCCCCGGCTCCGCCGGCTCCGCCGCCGGCTCCGGCGCGTGCCCGGTGCCGCTGCAGCTGCCGCCGCTGGAA
CGCCTGACCCTGGATGATAAAAAACCGCTGAATACCCTGATCTCTGCTACTGGTCTGTGGATGAGTCGTACCGGA
ACCATTCATAAAATCAAACACCACGAGGTTAGCCGTTCGAAAATCTATATTGAGATGGCGTGTGGCGATCATCTG
GTTGTGAACAATAGCCGCTCTTCTCGTACAGCACGTGCACTGCGTCACCACAAATATCGTAAAACCTGTAAACGT
TGCCGTGTGTCCGATGAGGATCTGAACAAATTCCTGACAAAAGCCAATGAGGACCAAACAAGCGTGAAAGTGAAA
GTCGTTAGCGCTCCTACCCGTACTAAAAAAGCAATGCCGAAATCCGTTGCTCGTGCCCCTAAACCACTGGAAAAC
ACTGAAGCAGCACAGGCACAGCCGTCTGGAAGCAAATTCTCTCCGGCCATTCCTGTTTCTACCCAGGAGTCCGTT
TCTGTTCCAGCAAGTGTGAGCACCAGCATTAGCAGTATTAGCACCGGTGCCACCGCTAGCGCCCTGGTTAAAGGC
AATACCAATCCGATTACAAGCATGTCTGCCCCGGTTCAAGCATCAGCTCCAGCACTGACAAAATCCCAAACCGAT
CGTCTGGAGGTTCTGCTGAATCCGAAAGACGAAATCAGCCTGAATTCCGGCAAACCGTTTCGTGAACTGGAGAGC
GAACTGCTGTCACGTCGTAAAAAAGACCTGCAACAAATCTATGCCGAAGAACGTGAGAACTATCTGGGGAAACTG
GAACGTGAAATCACCCGCTTTTTCGTGGATCGTGGCTTTCTGGAGATCAAATCCCCGATTCTGATTCCTCTGGAG
TATATCGAGCGTATGGGCATCGACAATGATACCGAACTGAGCAAACAAATTTTCCGTGTGGATAAAAACTTCTGT
CTGCGCCCTATGCTAGCACCAAATCTGGCTAACTATCTGCGCAAACTGGACCGTGCCCTGCCTGATCCTATCAAA
ATCTTCGAGATCGGCCCGTGTTATCGTAAAGAGTCCGACGGTAAAGAACATCTGGAGGAGTTTACCATGCTGAAC
TTTTGCCAAATGGGTTCAGGTTGTACTCGTGAGAACCTGGAAAGCATCATCACCGATTTTCTGAACCACCTGGGC
ATTGACTTCAAAATTGTGGGCGACAGCTGTATGGTGTTTGGCGACACCCTGGATGTCATGCACGGCGACCTGGAA
CTGTCTAGTGCCGTTGTGGGCCCAATCCCGCTGGATCGTGAGTGGGGTATCGACAAACCTTGGATCGGTGCGGGT
TTTGGTCTGGAGCGTCTGCTGAAAGTAAAACACGACTTCAAGAACATCAAACGTGCTGCACGTTCCGAGTCCTAT
TACAATGGTATTTCTACTAACCTGTAA (SEQ ID NO:69)
Protein:
MASVKVAVRVRPMNRREKDLEAKFIIQMEKSKTTITNLKIPEGGTGDSGRERTKTFTYDFSFYSADTKSPDYVSQ
EMVFKTLGTDVVKSAFEGYNACVFAYGQTGSGKSYTMMGNSGDSGLIPRICEGLFSRINETTRWDEASFRTEVSY
LEIYNERVRDLLRRKSSKTFNLRVREHPKEGPYVEDLSKHLVQNYGDVEELMDAGNINRTTAATGMNDVSSRSHA
IFTIKFTQAKFDSEMPCETVSKIHLVDLAGSERADATGATGVRLKEGGNINKSLVTLGNVISALADLSQDAANTL
AKKKQVFVPYRDSVLTWLLKDSLGGNSKTIMIATISPADVNYGETLSTLRYANRAKNIINKPTINEDANVKLIRE
LRAEIARLKTLLAQGNQIALLDSPTMEDNSVLNEDSNLEHVEGQPRRSMSQPVLNVEGDKRTSSTSATQQQVLSG
AFSSADVRSIPIIQTWEENKALKTKITILRGELQMYQRRYSEAKEASQKRVKEVMDDYVDLKLGQENVQEKMEQY
KLMEEDLLAMQSRIETSEDNFARQMKEFEAQKHAMEERIKELELSATDANNTTVGSFRGTLDDILKKNDPDFTLT
SGYEERKINDLEAKLLSEIDKVAELEDHIQQLRQELDDQSARLADSENVRAQLEAATGQGILGAAGNAMVPNSTF
MIGNGRESQTRDQLNYIDDLETKLADAKKENDKARQALVEYMNKCSKLEHEIRTMVKNSTFDSSSMLLGGQTSDE
LKIQIGKVNGELNVLRAENRELRIRCDQLTGGDGNLSISLGQSRLMAGIATNDVDSIGQGNETGGTSMRILPRES
QLDDLEESKLPLMDTSSAVRNQQQFASMWEDFESVKDSLQNNHNDTLEGSFNSSMPPPGRDATQSFLSQKSFKNS
PIVMQKPKSLHLHLKSHQSEGAGEQIQNNSFSTKTASPHVSQSHIPILHDMQQILDSSAMFLEGQHDVAVNVEQM
QEKMSQIREALARLFERLKSSAALFEEILERMGSSDPNADKIKKMKLAFETSINDKLNVSAILEAAEKDLHNMSL
NFSILEKSIVSQAAEASRRFTIAPDAEDVASSSLLNASYSPLFKFTSNSDIVEKLQNEVSELKNELEMARTRDMR
SPLNGSSGRLSDVQINTNRMFEDLEVSEATLQKAKEENSTLKSQFAELEANLHQVNSKLGEVRCELNEALARVDG
EQETRVKAENALEEARQLISSLKHEENELKKTITDMGMRLNEAKKSDEFLKSELSTALEEEKKSQNLADELSEEL
NGWRMRTKEAENKVEHASSEKSEMLERIVHLETEMEKLSTSEIAADYCSTKMTERKKEIELAKYREDFENAAIVG
LERISKEISELTKKTLKAKIIPSNISSIQLVCDELCRRLSREREQQHEYAKVMRDVNEKIEKLQLEKDALEHELK
MMSSNNENVPPVGTSVSGMPTKTSNQKCAQPHYTSPTRQLLHESTMAVDAIVQKLKKTHNMSGMGPELKETIGNV
INESRVLRDFLHQKLILFKGIDMSNWKNETVDQLITDLGQLHQDNLMLEEQIKKYKKELKLTKSAIPTLGVEFQD
RIKTEIGKIATDMGGAVKEIRKKGTGAPGSAGSAAGSGACPVPLQLPPLERLTLDDKKPLNTLISATGLWMSRTG
TIHKIKHHEVSRSKIYIEMACGDHLVVNNSRSSRTARALRHHKYRKTCKRCRVSDEDLNKFLTKANEDQTSVKVK
VVSAPTRTKKAMPKSVARAPKPLENTEAAQAQPSGSKFSPAIPVSTQESVSVPASVSTSISSISTGATASALVKG
NTNPITSMSAPVQASAPALTKSQTDRLEVLLNPKDEISLNSGKPFRELESELLSRRKKDLQQIYAEERENYLGKL
EREITRFFVDRGFLEIKSPILIPLEYIERMGIDNDTELSKQIFRVDKNFCLRPMLAPNLANYLRKLDRALPDPIK
IFEIGPCYRKESDGKEHLEEFTMLNFCQMGSGCTRENLESIITDFLNHLGIDFKIVGDSCMVFGDTLDVMHGDLE
LSSAVVGPIPLDREWGIDKPWIGAGFGLERLLKVKHDFKNIKRAARSESYYNGISTNL (SEQ ID NO:70)

KIF16B-SPD5-MCP
DNA:
ATGGCATCGGTCAAGGTGGCCGTGAGGGTCCGGCCCATGAATCGCAGGGAAAAGGACTTGGAGGCCAAGTTCATT
ATTCAGATGGAGAAAAGCAAAACGACAATCACAAACTTAAAGATACCAGAAGGAGGCACTGGGGACTCAGGAAGA

GAACGGACCAAGACCTTCACCTATGACTTTTCTTTTTATTCTGCTGATACAAAAAGCCCAGATTACGTTTCACAA
GAAATGGTTTTCAAAACCCTCGGCACAGATGTCGTGAAGTCTGCATTTGAAGGTTATAATGCTTGTGTCTTTGCA
TATGGGCAAACTGGATCTGGAAAGTCATACACTATGATGGGAAATTCTGGAGATTCTGGCTTAATACCTCGGATC
TGTGAAGGACTCTTCAGTCGGATAAATGAAACCACCAGATGGGATGAAGCTTCTTTTCGAACTGAAGTCAGCTAC
TTAGAAATTTATAACGAACGTGTGAGAGATCTACTTCGGCGGAAGTCATCTAAAACCTTCAATTTGAGAGTCCGT
GAGCATCCCAAAGAAGGCCCTTATGTTGAGGATTTATCCAAACATTTAGTACAGAATTATGGTGACGTAGAAGAA
CTTATGGATGCGGGCAATATCAACCGGACCACCGCAGCGACTGGGATGAACGACGTCAGTAGCAGGTCTCATGCC
ATCTTCACCATCAAGTTCACTCAGGCTAAATTTGATTCTGAAATGCCATGTGAAACCGTCAGTAAGATCCACTTG
GTTGATCTTGCCGGAAGTGAGCGTGCAGATGCCACCGGAGCCACCGGGGTTAGGCTAAAGGAAGGGGGAAATATT
AACAAGTCCCTCGTGACTCTGGGGAACGTCATTTCTGCCTTAGCTGATTTATCTCAGGATGCTGCAAATACTCTT
GCAAAGAAGAAGCAAGTTTTCGTGCCTTACAGGGATTCTGTGTTGACTTGGTTGTTAAAAGATAGCCTTGGAGGA
AACTCTAAAACTATCATGATTGCCACCATTTCACCTGCTGATGTCAATTATGGAGAAACCCTAAGTACTCTTCGC
TATGCAAATAGAGCCAAAAACATCATCAACAAGCCTACCATTAATGAGGATGCCAACGTCAAACTTATCCGTGAG
CTGCGAGCTGAAATAGCCAGACTGAAAACGCTGCTTGCTCAAGGGAATCAGATTGCCCTCTTAGACTCCCCCACA
ATGGAGGACAACAGCGTGCTGAACGAGGACAGCAACCTGGAGCACGTGGAGGGCCAGCCCAGAAGAAGCATGAGC
CAGCCCGTGCTGAACGTGGAGGGCGACAAGAGAACCAGCAGCACCAGCGCCACCCAGCAGCAGGTGCTGAGCGGC
GCCTTCAGCAGCGCCGACGTGAGAAGCATCCCCATCATCCAGACCTGGGAGGAGAACAAGGCCCTGAAGACCAAG
ATCACCATCCTGAGAGGCGAGCTGCAGATGTACCAGAGAAGATACAGCGAGGCCAAGGAGGCCAGCCAGAAGAGA
GTGAAGGAGGTGATGGACGACTACGTGGACCTGAAGCTGGGCCAGGAGAACGTGCAGGAGAAGATGGAGCAGTAC
AAGCTGATGGAGGAGGACCTGCTGGCCATGCAGAGCAGAATCGAGACCAGCGAGGACAACTTCGCCAGACAGATG
AAGGAGTTCGAGGCCCAGAAGCACGCCATGGAGGAGAGAATCAAGGAGCTGGAGCTGAGCGCCACCGACGCCAAC
AACACCACCGTGGGCAGCTTCAGAGGCACCCTGGACGACATCCTGAAGAAGAACGACCCCGACTTCACCCTGACC
AGCGGCTACGAGGAGAGAAAGATCAACGACCTGGAGGCCAAGCTGCTGAGCGAGATCGACAAGGTGGCCGAGCTG
GAGGACCACATCCAGCAGCTGAGACAGGAGCTGGACGACCAGAGCGCCAGACTGGCCGACAGCGAGAACGTGAGA
GCCCAGCTGGAGGCCGCCACCGGCCAGGGCATCCTGGGCGCCGCCGGCAACGCCATGGTGCCCAACAGCACCTTC
ATGATCGGCAACGGCAGAGAGAGCCAGACCAGAGACCAGCTGAACTACATCGACGACCTGGAGACCAAGCTGGCC
GACGCCAAGAAGGAGAACGACAAGGCCAGACAGGCCCTGGTGGAGTACATGAACAAGTGCAGCAAGCTGGAGCAC
GAGATCAGAACCATGGTGAAGAACAGCACCTTCGACAGCAGCAGCATGCTGCTGGGCGGCCAGACCAGCGACGAG
CTGAAGATCCAGATCGGCAAGGTGAACGGCGAGCTGAACGTGCTGAGAGCCGAGAACAGAGAGCTGAGAATCAGA
TGCGACCAGCTGACCGGCGGCGACGGCAACCTGAGCATCAGCCTGGGCCAGAGCAGACTGATGGCCGGCATCGCC
ACCAACGACGTGGACAGCATCGGCCAGGGCAACGAGACCGGCGGCACCAGCATGAGAATCCTGCCCAGAGAGAGC
CAGCTGGACGACCTGGAGGAGAGCAAGCTGCCCCTGATGGACACCAGCAGCGCCGTGAGAAACCAGCAGCAGTTC
GCCAGCATGTGGGAGGACTTCGAGAGCGTGAAGGACAGCCTGCAGAACAACCACAACGACACCCTGGAGGGCAGC
TTCAACAGCAGCATGCCCCCCCCCGGCAGAGACGCCACCCAGAGCTTCCTGAGCCAGAAGAGCTTCAAGAACAGC
CCCATCGTGATGCAGAAGCCCAAGAGCCTGCACCTGCACCTGAAGAGCCACCAGAGCGAGGGCGCCGGCGAGCAG
ATCCAGAACAACAGCTTCAGCACCAAGACCGCCAGCCCCCACGTGAGCCAGAGCCACATCCCCATCCTGCACGAC
ATGCAGCAGATCCTGGACAGCAGCGCCATGTTCCTGGAGGGCCAGCACGACGTGGCCGTGAACGTGGAGCAGATG
CAGGAGAAGATGAGCCAGATCAGAGAGGCCCTGGCCAGACTGTTCGAGAGACTGAAGAGCAGCGCCGCCCTGTTC
GAGGAGATCCTGGAGAGAATGGGCAGCAGCGACCCCAACGCCGACAAGATCAAGAAGATGAAGCTGGCCTTCGAG
ACCAGCATCAACGACAAGCTGAACGTGAGCGCCATCCTGGAGGCCGCCGAGAAGGACCTGCACAACATGAGCCTG
AACTTCAGCATCCTGGAGAAGAGCATCGTGAGCCAGGCCGCCGAGGCCAGCAGAAGATTCACCATCGCCCCCGAC
GCCGAGGACGTGGCCAGCAGCAGCCTGCTGAACGCCAGCTACAGCCCCCTGTTCAAGTTCACCAGCAACAGCGAC
ATCGTGGAGAAGCTGCAGAACGAGGTGAGCGAGCTGAAGAACGAGCTGGAGATGGCCAGAACCAGAGACATGAGA
AGCCCCCTGAACGGCAGCAGCGGCAGACTGAGCGACGTGCAGATCAACACCAACAGAATGTTCGAGGACCTGGAG
GTGAGCGAGGCCACCCTGCAGAAGGCCAAGGAGGAGAACAGCACCCTGAAGAGCCAGTTCGCCGAGCTGGAGGCC
AACCTGCACCAGGTGAACAGCAAGCTGGGCGAGGTGAGATGCGAGCTGAACGAGGCCCTGGCCAGAGTGGACGGC
GAGCAGGAGACCAGAGTGAAGGCCGAGAACGCCCTGGAGGAGGCCAGACAGCTGATCAGCAGCCTGAAGCACGAG
GAGAACGAGCTGAAGAAGACCATCACCGACATGGGCATGAGACTGAACGAGGCCAAGAAGAGCGACGAGTTCCTG
AAGAGCGAGCTGAGCACCGCCCTGGAGGAGGAGAAGAAGAGCCAGAACCTGGCCGACGAGCTGAGCGAGGAGCTG
AACGGCTGGAGAATGAGAACCAAGGAGGCCGAGAACAAGGTGGAGCACGCCAGCAGCGAGAAGAGCGAGATGCTG
GAGAGAATCGTGCACCTGGAGACCGAGATGGAGAAGCTGAGCACCAGCGAGATCGCCGCCGACTACTGCAGCACC
AAGATGACCGAGAGAAAGAAGGAGATCGAGCTGGCCAAGTACAGAGAGGACTTCGAGAACGCCGCCATCGTGGGC
CTGGAGAGAATCAGCAAGGAGATCAGCGAGCTGACCAAGAAGACCCTGAAGGCCAAGATCATCCCCAGCAACATC
AGCAGCATCCAGCTGGTGTGCGACGAGCTGTGCAGAAGACTGAGCAGAGAGAGAGAGCAGCAGCACGAGTACGCC
AAGGTGATGAGAGACGTGAACGAGAAGATCGAGAAGCTGCAGCTGGAGAAGGACGCCCTGGAGCACGAGCTGAAG
ATGATGAGCAGCAACAACGAGAACGTGCCCCCCGTGGGCACCAGCGTGAGCGGCATGCCCACCAAGACCAGCAAC
CAGAAGTGCGCCCAGCCCCACTACACCAGCCCCACCAGACAGCTGCTGCACGAGAGCACCATGGCCGTGGACGCC
ATCGTGCAGAAGCTGAAGAAGACCCACAACATGAGCGGCATGGGCCCCGAGCTGAAGGAGACCATCGGCAACGTG

ATCAACGAGAGCAGAGTGCTGAGAGACTTCCTGCACCAGAAGCTGATCCTGTTCAAGGGCATCGACATGAGCAAC
TGGAAGAACGAGACCGTGGACCAGCTGATCACCGACCTGGGCCAGCTGCACCAGGACAACCTGATGCTGGAGGAG
CAGATCAAGAAGTACAAGAAGGAGCTGAAGCTGACCAAGAGCGCCATCCCCACCCTGGGCGTGGAGTTCCAGGAC
AGAATCAAGACCGAGATCGGCAAGATCGCCACCGACATGGGCGGCGCCGTGAAGGAGATCAGAAAGAAGGGTACC
GAGCAGAAGCTGATCTCAGAGGAGGACCTGGGCGCCCCCGGCTCCGCCGGCTCCGCCGCCGGCTCCGGCGCTTCT
AACTTTACTCAGTTCGTTCTCGTCGACAATGGCGGAACTGGCGACGTGACTGTCGCCCCAAGCAACTTCGCTAAC
GGGATCGCTGAATGGATCAGCTCTAACTCGCGTTCACAGGCTTACAAAGTAACCTGTAGCGTTCGTCAGAGCTCT
GCGCAGAATCGCAAATACACCATCAAAGTCGAGGTGCCTAAAGGCGCCTGGCGTTCGTACTTAAATATGGAACTA
ACCATTCCAATTTTCGCCACGAATTCCGACTGCGAGCTTATTGTTAAGGCAATGCAAGGTCTCCTAAAAGATGGA
AACCCGATTCCCTCAGCAATCGCAGCAAACTCCGGCATCTACTAA (SEQ ID NO:71)
Protein:
MASVKVAVRVRPMNRREKDLEAKFIIQMEKSKTTITNLKIPEGGTGDSGRERTKTFTYDFSFYSADTKSPDYVSQ
EMVFKTLGTDVVKSAFEGYNACVFAYGQTGSGKSYTMMGNSGDSGLIPRICEGLFSRINETTRWDEASFRTEVSY
LEIYNERVRDLLRRKSSKTFNLRVREHPKEGPYVEDLSKHLVQNYGDVEELMDAGNINRTTAATGMNDVSSRSHA
IFTIKFTQAKFDSEMPCETVSKIHLVDLAGSERADATGATGVRLKEGGNINKSLVTLGNVISALADLSQDAANTL
AKKKQVFVPYRDSVLTWLLKDSLGGNSKTIMIATISPADVNYGETLSTLRYANRAKNIINKPTINEDANVKLIRE
LRAEIARLKTLLAQGNQIALLDSPTMEDNSVLNEDSNLEHVEGQPRRSMSQPVLNVEGDKRTSSTSATQQQVLSG
AFSSADVRSIPIIQTWEENKALKTKITILRGELQMYQRRYSEAKEASQKRVKEVMDDYVDLKLGQENVQEKMEQY
KLMEEDLLAMQSRIETSEDNFARQMKEFEAQKHAMEERIKELELSATDANNTTVGSFRGTLDDILKKNDPDFTLT
SGYEERKINDLEAKLLSEIDKVAELEDHIQQLRQELDDQSARLADSENVRAQLEAATGQGILGAAGNAMVPNSTF
MIGNGRESQTRDQLNYIDDLETKLADAKKENDKARQALVEYMNKCSKLEHEIRTMVKNSTFDSSSMLLGGQTSDE
LKIQIGKVNGELNVLRAENRELRIRCDQLTGGDGNLSISLGQSRLMAGIATNDVDSIGQGNETGGTSMRILPRES
QLDDLEESKLPLMDTSSAVRNQQQFASMWEDFESVKDSLQNNHNDTLEGSFNSSMPPPGRDATQSFLSQKSFKNS
PIVMQKPKSLHLHLKSHQSEGAGEQIQNNSFSTKTASPHVSQSHIPILHDMQQILDSSAMFLEGQHDVAVNVEQM
QEKMSQIREALARLFERLKSSAALFEEILERMGSSDPNADKIKKMKLAFETSINDKLNVSAILEAAEKDLHNMSL
NFSILEKSIVSQAAEASRRFTIAPDAEDVASSSLLNASYSPLFKFTSNSDIVEKLQNEVSELKNELEMARTRDMR
SPLNGSSGRLSDVQINTNRMFEDLEVSEATLQKAKEENSTLKSQFAELEANLHQVNSKLGEVRCELNEALARVDG
EQETRVKAENALEEARQLISSLKHEENELKKTITDMGMRLNEAKKSDEFLKSELSTALEEEKKSQNLADELSEEL
NGWRMRTKEAENKVEHASSEKSEMLERIVHLETEMEKLSTSEIAADYCSTKMTERKKEIELAKYREDFENAAIVG
LERISKEISELTKKTLKAKIIPSNISSIQLVCDELCRRLSREREQQHEYAKVMRDVNEKIEKLQLEKDALEHELK
MMSSNNENVPPVGTSVSGMPTKTSNQKCAQPHYTSPTRQLLHESTMAVDAIVQKLKKTHNMSGMGPELKETIGNV
INESRVLRDFLHQKLILFKGIDMSNWKNETVDQLITDLGQLHQDNLMLEEQIKKYKKELKLTKSAIPTLGVEFQD
RIKTEIGKIATDMGGAVKEIRKKGTEQKLISEEDLGAPGSAGSAAGSGASNFTQFVLVDNGGTGDVTVAPSNFAN
GIAEWISSNSRSQAYKVTCSVRQSSAQNRKYTIKVEVPKGAWRSYLNMELTIPIFATNSDCELIVKAMQGLLKDG
NPIPSAIAANSGIY (SEQ ID NO:72)

KIF16B-SPD5-MCP-PylRS^AF
DNA:
ATGGCATCGGTCAAGGTGGCCGTGAGGGTCCGGCCCATGAATCGCAGGGAAAAGGACTTGGAGGCCAAGTTCATT
ATTCAGATGGAGAAAAGCAAAACGACAATCACAAACTTAAAGATACCAGAAGGAGGCACTGGGGACTCAGGAAGA
GAACGGACCAAGACCTTCACCTATGACTTTTCTTTTTATTCTGCTGATACAAAAAGCCCAGATTACGTTTCACAA
GAAATGGTTTTCAAAACCCTCGGCACAGATGTCGTGAAGTCTGCATTTGAAGGTTATAATGCTTGTGTCTTTGCA
TATGGGCAAACTGGATCTGGAAAGTCATACACTATGATGGGAAATTCTGGAGATTCTGGCTTAATACCTCGGATC
TGTGAAGGACTCTTCAGTCGGATAAATGAAACCACCAGATGGGATGAAGCTTCTTTTCGAACTGAAGTCAGCTAC
TTAGAAATTTATAACGAACGTGTGAGAGATCTACTTCGGCGGAAGTCATCTAAAACCTTCAATTTGAGAGTCCGT
GAGCATCCCAAAGAAGGCCCTTATGTTGAGGATTTATCCAAACATTTAGTACAGAATTATGGTGACGTAGAAGAA
CTTATGGATGCGGGCAATATCAACCGGACCACCGCAGCGACTGGGATGAACGACGTCAGTAGCAGGTCTCATGCC
ATCTTCACCATCAAGTTCACTCAGGCTAAATTTGATTCTGAAATGCCATGTGAAACCGTCAGTAAGATCCACTTG
GTTGATCTTGCCGGAAGTGAGCGTGCAGATGCCACCGGAGCCACCGGGGTTAGGCTAAAGGAAGGGGGAAATATT
AACAAGTCCCTCGTGACTCTGGGGAACGTCATTTCTGCCTTAGCTGATTTATCTCAGGATGCTGCAAATACTCTT
GCAAAGAAGAAGCAAGTTTTCGTGCCTTACAGGGATTCTGTGTTGACTTGGTTGTTAAAAGATAGCCTTGGAGGA
AACTCTAAAACTATCATGATTGCCACCATTTCACCTGCTGATGTCAATTATGGAGAAACCCTAAGTACTCTTCGC
TATGCAAATAGAGCCAAAAACATCATCAACAAGCCTACCATTAATGAGGATGCCAACGTCAAACTTATCCGTGAG
CTGCGAGCTGAAATAGCCAGACTGAAAACGCTGCTTGCTCAAGGGAATCAGATTGCCCTCTTAGACTCCCCCACA
ATGGAGGACAACAGCGTGCTGAACGAGGACAGCAACCTGGAGCACGTGGAGGGCCAGCCCAGAAGAAGCATGAGC
CAGCCCGTGCTGAACGTGGAGGGCGACAAGAGAACCAGCAGCACCAGCGCCACCCAGCAGCAGGTGCTGAGCGGC
GCCTTCAGCAGCGCCGACGTGAGAAGCATCCCCATCATCCAGACCTGGGAGGAGAACAAGGCCCTGAAGACCAAG
ATCACCATCCTGAGAGGCGAGCTGCAGATGTACCAGAGAAGATACAGCGAGGCCAAGGAGGCCAGCCAGAAGAGA

GTGAAGGAGGTGATGGACGACTACGTGGACCTGAAGCTGGGCCAGGAGAACGTGCAGGAGAAGATGGAGCAGTAC
AAGCTGATGGAGGAGGACCTGCTGGCCATGCAGAGCAGAATCGAGACCAGCGAGGACAACTTCGCCAGACAGATG
AAGGAGTTCGAGGCCCAGAAGCACGCCATGGAGGAGAGAATCAAGGAGCTGGAGCTGAGCGCCACCGACGCCAAC
AACACCACCGTGGGCAGCTTCAGAGGCACCCTGGACGACATCCTGAAGAAGAACGACCCCGACTTCACCCTGACC
AGCGGCTACGAGGAGAGAAAGATCAACGACCTGGAGGCCAAGCTGCTGAGCGAGATCGACAAGGTGGCCGAGCTG
GAGGACCACATCCAGCAGCTGAGACAGGAGCTGGACGACCAGAGCGCCAGACTGGCCGACAGCGAGAACGTGAGA
GCCCAGCTGGAGGCCGCCACCGGCCAGGGCATCCTGGGCGCCGCCGGCAACGCCATGGTGCCCAACAGCACCTTC
ATGATCGGCAACGGCAGAGAGAGCCAGACCAGAGACCAGCTGAACTACATCGACGACCTGGAGACCAAGCTGGCC
GACGCCAAGAAGGAGAACGACAAGGCCAGACAGGCCCTGGTGGAGTACATGAACAAGTGCAGCAAGCTGGAGCAC
GAGATCAGAACCATGGTGAAGAACAGCACCTTCGACAGCAGCAGCATGCTGCTGGGCGGCCAGACCAGCGACGAG
CTGAAGATCCAGATCGGCAAGGTGAACGGCGAGCTGAACGTGCTGAGAGCCGAGAACAGAGAGCTGAGAATCAGA
TGCGACCAGCTGACCGGCGGCGACGGCAACCTGAGCATCAGCCTGGGCCAGAGCAGACTGATGGCCGGCATCGCC
ACCAACGACGTGGACAGCATCGGCCAGGGCAACGAGACCGGCGGCACCAGCATGAGAATCCTGCCCAGAGAGAGC
CAGCTGGACGACCTGGAGGAGAGCAAGCTGCCCCTGATGGACACCAGCAGCGCCGTGAGAAACCAGCAGCAGTTC
GCCAGCATGTGGGAGGACTTCGAGAGCGTGAAGGACAGCCTGCAGAACAACCACAACGACACCCTGGAGGGCAGC
TTCAACAGCAGCATGCCCCCCCCCGGCAGAGACGCCACCCAGAGCTTCCTGAGCCAGAAGAGCTTCAAGAACAGC
CCCATCGTGATGCAGAAGCCCAAGAGCCTGCACCTGCACCTGAAGAGCCACCAGAGCGAGGGCGCCGGCGAGCAG
ATCCAGAACAACAGCTTCAGCACCAAGACCGCCAGCCCCCACGTGAGCCAGAGCCACATCCCCATCCTGCACGAC
ATGCAGCAGATCCTGGACAGCAGCGCCATGTTCCTGGAGGGCCAGCACGACGTGGCCGTGAACGTGGAGCAGATG
CAGGAGAAGATGAGCCAGATCAGAGAGGCCCTGGCCAGACTGTTCGAGAGACTGAAGAGCAGCGCCGCCCTGTTC
GAGGAGATCCTGGAGAGAATGGGCAGCAGCGACCCCAACGCCGACAAGATCAAGAAGATGAAGCTGGCCTTCGAG
ACCAGCATCAACGACAAGCTGAACGTGAGCGCCATCCTGGAGGCCGCCGAGAAGGACCTGCACAACATGAGCCTG
AACTTCAGCATCCTGGAGAAGAGCATCGTGAGCCAGGCCGCCGAGGCCAGCAGAAGATTCACCATCGCCCCCGAC
GCCGAGGACGTGGCCAGCAGCAGCCTGCTGAACGCCAGCTACAGCCCCCTGTTCAAGTTCACCAGCAACAGCGAC
ATCGTGGAGAAGCTGCAGAACGAGGTGAGCGAGCTGAAGAACGAGCTGGAGATGGCCAGAACCAGAGACATGAGA
AGCCCCCTGAACGGCAGCAGCGGCAGACTGAGCGACGTGCAGATCAACACCAACAGAATGTTCGAGGACCTGGAG
GTGAGCGAGGCCACCCTGCAGAAGGCCAAGGAGGAGAACAGCACCCTGAAGAGCCAGTTCGCCGAGCTGGAGGCC
AACCTGCACCAGGTGAACAGCAAGCTGGGCGAGGTGAGATGCGAGCTGAACGAGGCCCTGGCCAGAGTGGACGGC
GAGCAGGAGACCAGAGTGAAGGCCGAGAACGCCCTGGAGGAGGCCAGACAGCTGATCAGCAGCCTGAAGCACGAG
GAGAACGAGCTGAAGAAGACCATCACCGACATGGGCATGAGACTGAACGAGGCCAAGAAGAGCGACGAGTTCCTG
AAGAGCGAGCTGAGCACCGCCCTGGAGGAGGAGAAGAAGAGCCAGAACCTGGCCGACGAGCTGAGCGAGGAGCTG
AACGGCTGGAGAATGAGAACCAAGGAGGCCGAGAACAAGGTGGAGCACGCCAGCAGCGAGAAGAGCGAGATGCTG
GAGAGAATCGTGCACCTGGAGACCGAGATGGAGAAGCTGAGCACCAGCGAGATCGCCGCCGACTACTGCAGCACC
AAGATGACCGAGAGAAAGAAGGAGATCGAGCTGGCCAAGTACAGAGAGGACTTCGAGAACGCCGCCATCGTGGGC
CTGGAGAGAATCAGCAAGGAGATCAGCGAGCTGACCAAGAAGACCCTGAAGGCCAAGATCATCCCCAGCAACATC
AGCAGCATCCAGCTGGTGTGCGACGAGCTGTGCAGAAGACTGAGCAGAGAGAGAGAGCAGCAGCACGAGTACGCC
AAGGTGATGAGAGACGTGAACGAGAAGATCGAGAAGCTGCAGCTGGAGAAGGACGCCCTGGAGCACGAGCTGAAG
ATGATGAGCAGCAACAACGAGAACGTGCCCCCCGTGGGCACCAGCGTGAGCGGCATGCCCACCAAGACCAGCAAC
CAGAAGTGCGCCCAGCCCCACTACACCAGCCCCACCAGACAGCTGCTGCACGAGAGCACCATGGCCGTGGACGCC
ATCGTGCAGAAGCTGAAGAAGACCCACAACATGAGCGGCATGGGCCCCGAGCTGAAGGAGACCATCGGCAACGTG
ATCAACGAGAGCAGAGTGCTGAGAGACTTCCTGCACCAGAAGCTGATCCTGTTCAAGGGCATCGACATGAGCAAC
TGGAAGAACGAGACCGTGGACCAGCTGATCACCGACCTGGGCCAGCTGCACCAGGACAACCTGATGCTGGAGGAG
CAGATCAAGAAGTACAAGAAGGAGCTGAAGCTGACCAAGAGCGCCATCCCCACCCTGGGCGTGGAGTTCCAGGAC
AGAATCAAGACCGAGATCGGCAAGATCGCCACCGACATGGGCGGCGCCGTGAAGGAGATCAGAAAGAAGGGTACC
GAGCAGAAGCTGATCTCAGAGGAGGACCTGGGCGCCCCCGGCTCCGCCGGCTCCGCCGCCGGCTCCGGCGCTTCT
AACTTTACTCAGTTCGTTCTCGTCGACAATGGCGGAACTGGCGACGTGACTGTCGCCCCAAGCAACTTCGCTAAC
GGGATCGCTGAATGGATCAGCTCTAACTCGCGTTCACAGGCTTACAAAGTAACCTGTAGCGTTCGTCAGAGCTCT
GCGCAGAATCGCAAATACACCATCAAAGTCGAGGTGCCTAAAGGCGCCTGGCGTTCGTACTTAAATATGGAACTA
ACCATTCCAATTTTCGCCACGAATTCCGACTGCGAGCTTATTGTTAAGGCAATGCAAGGTCTCCTAAAAGATGGA
AACCCGATTCCCTCAGCAATCGCAGCAAACTCCGGCATCTACGGTACCGGCGCCCCCGGCTCCGCCGGCTCCGCC
GCCGGCTCCGGCGCGTGCCCGGTGCCGCTGCAGCTGCCGCCGCTGGAACGCCTGACCCTGGATGATAAAAAACCG
CTGAATACCCTGATCTCTGCTACTGGTCTGTGGATGAGTCGTACCGGAACCATTCATAAAATCAAACACCACGAG
GTTAGCCGTTCGAAAATCTATATTGAGATGGCGTGTGGCGATCATCTGGTTGTGAACAATAGCCGCTCTTCTCGT
ACAGCACGTGCACTGCGTCACCACAAATATCGTAAAACCTGTAAACGTTGCCGTGTGTCCGATGAGGATCTGAAC
AAATTCCTGACAAAAGCCAATGAGGACCAAACAAGCGTGAAAGTGAAAGTCGTTAGCGCTCCTACCCGTACTAAA
AAAGCAATGCCGAAATCCGTTGCTCGTGCCCCTAAACCACTGGAAAACACTGAAGCAGCACAGGCACAGCCGTCT
GGAAGCAAATTCTCTCCGGCCATTCCTGTTTCTACCCAGGAGTCCGTTTCTGTTCCAGCAAGTGTGAGCACCAGC
ATTAGCAGTATTAGCACCGGTGCCACCGCTAGCGCCCTGGTTAAAGGCAATACCAATCCGATTACAAGCATGTCT

GCCCCGGTTCAAGCATCAGCTCCAGCACTGACAAAATCCCAAACCGATCGTCTGGAGGTTCTGCTGAATCCGAAA
GACGAAATCAGCCTGAATTCCGGCAAACCGTTTCGTGAACTGGAGAGCGAACTGCTGTCACGTCGTAAAAAAGAC
CTGCAACAAATCTATGCCGAAGAACGTGAGAACTATCTGGGGAAACTGGAACGTGAAATCACCCGCTTTTTCGTG
GATCGTGGCTTTCTGGAGATCAAATCCCCGATTCTGATTCCTCTGGAGTATATCGAGCGTATGGGCATCGACAAT
GATACCGAACTGAGCAAACAAATTTTCCGTGTGGATAAAAACTTCTGTCTGCGCCCTATGCTAGCACCAAATCTG
GCTAACTATCTGCGCAAACTGGACCGTGCCCTGCCTGATCCTATCAAAATCTTCGAGATCGGCCCGTGTTATCGT
AAAGAGTCCGACGGTAAAGAACATCTGGAGGAGTTTACCATGCTGAACTTTTGCCAAATGGGTTCAGGTTGTACT
CGTGAGAACCTGGAAAGCATCATCACCGATTTTCTGAACCACCTGGGCATTGACTTCAAAATTGTGGGCGACAGC
TGTATGGTGTTTGGCGACACCCTGGATGTCATGCACGGCGACCTGGAACTGTCTAGTGCCGTTGTGGGCCCAATC
CCGCTGGATCGTGAGTGGGGTATCGACAAACCTTGGATCGGTGCGGGTTTTGGTCTGGAGCGTCTGCTGAAAGTA
AAACACGACTTCAAGAACATCAAACGTGCTGCACGTTCCGAGTCCTATTACAATGGTATTTCTACTAACCTGTAA
(SEQ ID NO:73)
Protein:
MASVKVAVRVRPMNRREKDLEAKFIIQMEKSKTTITNLKIPEGGTGDSGRERTKTFTYDFSFYSADTKSPDYVSQ
EMVFKTLGTDVVKSAFEGYNACVFAYGQTGSGKSYTMMGNSGDSGLIPRICEGLFSRINETTRWDEASFRTEVSY
LEIYNERVRDLLRRKSSKTFNLRVREHPKEGPYVEDLSKHLVQNYGDVEELMDAGNINRTTAATGMNDVSSRSHA
IFTIKFTQAKFDSEMPCETVSKIHLVDLAGSERADATGATGVRLKEGGNINKSLVTLGNVISALADLSQDAANTL
AKKKQVFVPYRDSVLTWLLKDSLGGNSKTIMIATISPADVNYGETLSTLRYANRAKNIINKPTINEDANVKLIRE
LRAEIARLKTLLAQGNQIALLDSPTMEDNSVLNEDSNLEHVEGQPRRSMSQPVLNVEGDKRTSSTSATQQQVLSG
AFSSADVRSIPIIQTWEENKALKTKITILRGELQMYQRRYSEAKEASQKRVKEVMDDYVDLKLGQENVQEKMEQY
KLMEEDLLAMQSRIETSEDNFARQMKEFEAQKHAMEERIKELELSATDANNTTVGSFRGTLDDILKKNDPDFTLT
SGYEERKINDLEAKLLSEIDKVAELEDHIQQLRQELDDQSARLADSENVRAQLEAATGQGILGAAGNAMVPNSTF
MIGNGRESQTRDQLNYIDDLETKLADAKKENDKARQALVEYMNKCSKLEHEIRTMVKNSTFDSSSMLLGGQTSDE
LKIQIGKVNGELNVLRAENRELRIRCDQLTGGDGNLSISLGQSRLMAGIATNDVDSIGQGNETGGTSMRILPRES
QLDDLEESKLPLMDTSSAVRNQQQFASMWEDFESVKDSLQNNHNDTLEGSFNSSMPPPGRDATQSFLSQKSFKNS
PIVMQKPKSLHLHLKSHQSEGAGEQIQNNSFSTKTASPHVSQSHIPILHDMQQILDSSAMFLEGQHDVAVNVEQM
QEKMSQIREALARLFERLKSSAALFEEILERMGSSDPNADKIKKMKLAFETSINDKLNVSAILEAAEKDLHNMSL
NFSILEKSIVSQAAEASRRFTIAPDAEDVASSSLLNASYSPLFKFTSNSDIVEKLQNEVSELKNELEMARTRDMR
SPLNGSSGRLSDVQINTNRMFEDLEVSEATLQKAKEENSTLKSQFAELEANLHQVNSKLGEVRCELNEALARVDG
EQETRVKAENALEEARQLISSLKHEENELKKTITDMGMRLNEAKKSDEFLKSELSTALEEEKKSQNLADELSEEL
NGWRMRTKEAENKVEHASSEKSEMLERIVHLETEMEKLSTSEIAADYCSTKMTERKKEIELAKYREDFENAAIVG
LERISKEISELTKKTLKAKIIPSNISSIQLVCDELCRRLSREREQQHEYAKVMRDVNEKIEKLQLEKDALEHELK
MMSSNNENVPPVGTSVSGMPTKTSNQKCAQPHYTSPTRQLLHESTMAVDAIVQKLKKTHNMSGMGPELKETIGNV
INESRVLRDFLHQKLILFKGIDMSNWKNETVDQLITDLGQLHQDNLMLEEQIKKYKKELKLTKSAIPTLGVEFQD
RIKTEIGKIATDMGGAVKEIRKKGTEQKLISEEDLGAPGSAGSAAGSGASNFTQFVLVDNGGTGDVTVAPSNFAN
GIAEWISSNSRSQAYKVTCSVRQSSAQNRKYTIKVEVPKGAWRSYLNMELTIPIFATNSDCELIVKAMQGLLKDG
NPIPSAIAANSGIYGTGAPGSAGSAAGSGACPVPLQLPPLERLTLDDKKPLNTLISATGLWMSRTGTIHKIKHHE
VSRSKIYIEMACGDHLVVNNSRSSRTARALRHHKYRKTCKRCRVSDEDLNKFLTKANEDQTSVKVKVVSAPTRTK
KAMPKSVARAPKPLENTEAAQAQPSGSKFSPAIPVSTQESVSVPASVSTSISSISTGATASALVKGNTNPITSMS
APVQASAPALTKSQTDRLEVLLNPKDEISLNSGKPFRELESELLSRRKKDLQQIYAEERENYLGKLEREITRFFV
DRGFLEIKSPILIPLEYIERMGIDNDTELSKQIFRVDKNFCLRPMLAPNLANYLRKLDRALPDPIKIFEIGPCYR
KESDGKEHLEEFTMLNFCQMGSGCTRENLESIITDFLNHLGIDFKIVGDSCMVFGDTLDVMHGDLELSSAVVGPI
PLDREWGIDKPWIGAGFGLERLLKVKHDFKNIKRAARSESYYNGISTNL (SEQ ID NO:74)

KIF16B-SPD5-4xλN22-PylRSAF
DNA:
ATGGCATCGGTCAAGGTGGCCGTGAGGGTCCGGCCCATGAATCGCAGGGAAAAGGACTTGGAGGCCAAGTTCATT
ATTCAGATGGAGAAAAGCAAAACGACAATCACAAACTTAAAGATACCAGAAGGAGGCACTGGGGACTCAGGAAGA
GAACGGACCAAGACCTTCACCTATGACTTTTCTTTTTATTCTGCTGATACAAAAAGCCCAGATTACGTTTCACAA
GAAATGGTTTTCAAAACCCTCGGCACAGATGTCGTGAAGTCTGCATTTGAAGGTTATAATGCTTGTGTCTTTGCA
TATGGGCAAACTGGATCTGGAAAGTCATACACTATGATGGGAAATTCTGGAGATTCTGGCTTAATACCTCGGATC
TGTGAAGGACTCTTCAGTCGGATAAATGAAACCACCAGATGGGATGAAGCTTCTTTTCGAACTGAAGTCAGCTAC
TTAGAAATTTATAACGAACGTGTGAGAGATCTACTTCGGCGGAAGTCATCTAAAACCTTCAATTTGAGAGTCCGT
GAGCATCCCAAAGAAGGCCCTTATGTTGAGGATTTATCCAAACATTTAGTACAGAATTATGGTGACGTAGAAGAA
CTTATGGATGCGGGCAATATCAACCGGACCACCGCAGCGACTGGGATGAACGACGTCAGTAGCAGGTCTCATGCC
ATCTTCACCATCAAGTTCACTCAGGCTAAATTTGATTCTGAAATGCCATGTGAAACCGTCAGTAAGATCCACTTG
GTTGATCTTGCCGGAAGTGAGCGTGCAGATGCCACCGGAGCCACCGGGGTTAGGCTAAAGGAAGGGGGAAATATT
AACAAGTCCCTCGTGACTCTGGGGAACGTCATTTCTGCCTTAGCTGATTTATCTCAGGATGCTGCAAATACTCTT

GCAAAGAAGAAGCAAGTTTTCGTGCCTTACAGGGATTCTGTGTTGACTTGGTTGTTAAAAGATAGCCTTGGAGGA
AACTCTAAAACTATCATGATTGCCACCATTTCACCTGCTGATGTCAATTATGGAGAAACCCTAAGTACTCTTCGC
TATGCAAATAGAGCCAAAAACATCATCAACAAGCCTACCATTAATGAGGATGCCAACGTCAAACTTATCCGTGAG
CTGCGAGCTGAAATAGCCAGACTGAAAACGCTGCTTGCTCAAGGGAATCAGATTGCCCTCTTAGACTCCCCCACA
ATGGAGGACAACAGCGTGCTGAACGAGGACAGCAACCTGGAGCACGTGGAGGGCCAGCCCAGAAGAAGCATGAGC
CAGCCCGTGCTGAACGTGGAGGGCGACAAGAGAACCAGCAGCACCAGCGCCACCCAGCAGCAGGTGCTGAGCGGC
GCCTTCAGCAGCGCCGACGTGAGAAGCATCCCCATCATCCAGACCTGGGAGGAGAACAAGGCCCTGAAGACCAAG
ATCACCATCCTGAGAGGCGAGCTGCAGATGTACCAGAGAAGATACAGCGAGGCCAAGGAGGCCAGCCAGAAGAGA
GTGAAGGAGGTGATGGACGACTACGTGGACCTGAAGCTGGGCCAGGAGAACGTGCAGGAGAAGATGGAGCAGTAC
AAGCTGATGGAGGAGGACCTGCTGGCCATGCAGAGCAGAATCGAGACCAGCGAGGACAACTTCGCCAGACAGATG
AAGGAGTTCGAGGCCCAGAAGCACGCCATGGAGGAGAGAATCAAGGAGCTGGAGCTGAGCGCCACCGACGCCAAC
AACACCACCGTGGGCAGCTTCAGAGGCACCCTGGACGACATCCTGAAGAAGAACGACCCCGACTTCACCCTGACC
AGCGGCTACGAGGAGAGAAAGATCAACGACCTGGAGGCCAAGCTGCTGAGCGAGATCGACAAGGTGGCCGAGCTG
GAGGACCACATCCAGCAGCTGAGACAGGAGCTGGACGACCAGAGCGCCAGACTGGCCGACAGCGAGAACGTGAGA
GCCCAGCTGGAGGCCGCCACCGGCCAGGGCATCCTGGGCGCCGCCGGCAACGCCATGGTGCCCAACAGCACCTTC
ATGATCGGCAACGGCAGAGAGAGCCAGACCAGAGACCAGCTGAACTACATCGACGACCTGGAGACCAAGCTGGCC
GACGCCAAGAAGGAGAACGACAAGGCCAGACAGGCCCTGGTGGAGTACATGAACAAGTGCAGCAAGCTGGAGCAC
GAGATCAGAACCATGGTGAAGAACAGCACCTTCGACAGCAGCAGCATGCTGCTGGGCGGCCAGACCAGCGACGAG
CTGAAGATCCAGATCGGCAAGGTGAACGGCGAGCTGAACGTGCTGAGAGCCGAGAACAGAGAGCTGAGAATCAGA
TGCGACCAGCTGACCGGCGGCGACGGCAACCTGAGCATCAGCCTGGGCCAGAGCAGACTGATGGCCGGCATCGCC
ACCAACGACGTGGACAGCATCGGCCAGGGCAACGAGACCGGCGGCACCAGCATGAGAATCCTGCCCAGAGAGAGC
CAGCTGGACGACCTGGAGGAGAGCAAGCTGCCCCTGATGGACACCAGCAGCGCCGTGAGAAACCAGCAGCAGTTC
GCCAGCATGTGGGAGGACTTCGAGAGCGTGAAGGACAGCCTGCAGAACAACCACAACGACACCCTGGAGGGCAGC
TTCAACAGCAGCATGCCCCCCCCCGGCAGAGACGCCACCCAGAGCTTCCTGAGCCAGAAGAGCTTCAAGAACAGC
CCCATCGTGATGCAGAAGCCCAAGAGCCTGCACCTGCACCTGAAGAGCCACCAGAGCGAGGGCGCCGGCGAGCAG
ATCCAGAACAACAGCTTCAGCACCAAGACCGCCAGCCCCCACGTGAGCCAGAGCCACATCCCCATCCTGCACGAC
ATGCAGCAGATCCTGGACAGCAGCGCCATGTTCCTGGAGGGCCAGCACGACGTGGCCGTGAACGTGGAGCAGATG
CAGGAGAAGATGAGCCAGATCAGAGAGGCCCTGGCCAGACTGTTCGAGAGACTGAAGAGCAGCGCCGCCCTGTTC
GAGGAGATCCTGGAGAGAATGGGCAGCAGCGACCCCAACGCCGACAAGATCAAGAAGATGAAGCTGGCCTTCGAG
ACCAGCATCAACGACAAGCTGAACGTGAGCGCCATCCTGGAGGCCGCCGAGAAGGACCTGCACAACATGAGCCTG
AACTTCAGCATCCTGGAGAAGAGCATCGTGAGCCAGGCCGCCGAGGCCAGCAGAAGATTCACCATCGCCCCCGAC
GCCGAGGACGTGGCCAGCAGCAGCCTGCTGAACGCCAGCTACAGCCCCCTGTTCAAGTTCACCAGCAACAGCGAC
ATCGTGGAGAAGCTGCAGAACGAGGTGAGCGAGCTGAAGAACGAGCTGGAGATGGCCAGAACCAGAGACATGAGA
AGCCCCCTGAACGGCAGCAGCGGCAGACTGAGCGACGTGCAGATCAACACCAACAGAATGTTCGAGGACCTGGAG
GTGAGCGAGGCCACCCTGCAGAAGGCCAAGGAGGAGAACAGCACCCTGAAGAGCCAGTTCGCCGAGCTGGAGGCC
AACCTGCACCAGGTGAACAGCAAGCTGGGCGAGGTGAGATGCGAGCTGAACGAGGCCCTGGCCAGAGTGGACGGC
GAGCAGGAGACCAGAGTGAAGGCCGAGAACGCCCTGGAGGAGGCCAGACAGCTGATCAGCAGCCTGAAGCACGAG
GAGAACGAGCTGAAGAAGACCATCACCGACATGGGCATGAGACTGAACGAGGCCAAGAAGAGCGACGAGTTCCTG
AAGAGCGAGCTGAGCACCGCCCTGGAGGAGGAGAAGAAGAGCCAGAACCTGGCCGACGAGCTGAGCGAGGAGCTG
AACGGCTGGAGAATGAGAACCAAGGAGGCCGAGAACAAGGTGGAGCACGCCAGCAGCGAGAAGAGCGAGATGCTG
GAGAGAATCGTGCACCTGGAGACCGAGATGGAGAAGCTGAGCACCAGCGAGATCGCCGCCGACTACTGCAGCACC
AAGATGACCGAGAGAAAGAAGGAGATCGAGCTGGCCAAGTACAGAGAGGACTTCGAGAACGCCGCCATCGTGGGC
CTGGAGAGAATCAGCAAGGAGATCAGCGAGCTGACCAAGAAGACCCTGAAGGCCAAGATCATCCCCAGCAACATC
AGCAGCATCCAGCTGGTGTGCGACGAGCTGTGCAGAAGACTGAGCAGAGAGAGAGAGCAGCAGCACGAGTACGCC
AAGGTGATGAGAGACGTGAACGAGAAGATCGAGAAGCTGCAGCTGGAGAAGGACGCCCTGGAGCACGAGCTGAAG
ATGATGAGCAGCAACAACGAGAACGTGCCCCCCGTGGGCACCAGCGTGAGCGGCATGCCCACCAAGACCAGCAAC
CAGAAGTGCGCCCAGCCCCACTACACCAGCCCCACCAGACAGCTGCTGCACGAGAGCACCATGGCCGTGGACGCC
ATCGTGCAGAAGCTGAAGAAGACCCACAACATGAGCGGCATGGGCCCCGAGCTGAAGGAGACCATCGGCAACGTG
ATCAACGAGAGCAGAGTGCTGAGAGACTTCCTGCACCAGAAGCTGATCCTGTTCAAGGGCATCGACATGAGCAAC
TGGAAGAACGAGACCGTGGACCAGCTGATCACCGACCTGGGCCAGCTGCACCAGGACAACCTGATGCTGGAGGAG
CAGATCAAGAAGTACAAGAAGGAGCTGAAGCTGACCAAGAGCGCCATCCCCACCCTGGGCGTGGAGTTCCAGGAC
AGAATCAAGACCGAGATCGGCAAGATCGCCACCGACATGGGCGGCGCCGTGAAGGAGATCAGAAAGAAGTCCGGA
TATCCCTATGATGTGCCGGATTATGCTTCCACCATGGACGCACAAACACGACGACGTGAGCGTCGCGCTGAGAAA
CAAGCTCAATGGAAAGCTGCAAACCCACCGCTCGACGGAGCCGGAGCTGGCGCTGGAGCTGGAGCCGGAGCTGGC
GGTCTAGCCACCATGGACGCACAAACACGACGACGTGAGCGTCGCGCTGAGAAACAAGCTCAATGGAAAGCTGCA
AACCCACCGCTCGACGGAGCCGGAGCTGGCGCTGGAGCTGGAGCCGGAGCTGGCGGTCTAGCCACCATGGACGCA
CAAACACGACGACGTGAGCGTCGCGCTGAGAAACAAGCTCAATGGAAAGCTGCAAACCCACCGCTCGACGGAGCC
GGAGCTGGCGCTGGAGCTGGAGCCGGAGCTGGCGGTCTAGCCACCATGGACGCACAAACACGACGACGTGAGCGT

CGCGCTGAGAAACAAGCTCAATGGAAAGCTGCAAACCCACCGCTCGGTACCGGCGCCCCCGGCTCCGCCGGCTCC
GCCGCCGGCTCCGGCGCGTGCCCGGTGCCGCTGCAGCTGCCGCCGCTGGAACGCCTGACCCTGGATGATAAAAAA
CCGCTGAATACCCTGATCTCTGCTACTGGTCTGTGGATGAGTCGTACCGGAACCATTCATAAAATCAAACACCAC
GAGGTTAGCCGTTCGAAAATCTATATTGAGATGGCGTGTGGCGATCATCTGGTTGTGAACAATAGCCGCTCTTCT
CGTACAGCACGTGCACTGCGTCACCACAAATATCGTAAAACCTGTAAACGTTGCCGTGTGTCCGATGAGGATCTG
AACAAATTCCTGACAAAAGCCAATGAGGACCAAACAAGCGTGAAAGTGAAAGTCGTTAGCGCTCCTACCCGTACT
AAAAAAGCAATGCCGAAATCCGTTGCTCGTGCCCCTAAACCACTGGAAAACACTGAAGCAGCACAGGCACAGCCG
TCTGGAAGCAAATTCTCTCCGGCCATTCCTGTTTCTACCCAGGAGTCCGTTTCTGTTCCAGCAAGTGTGAGCACC
AGCATTAGCAGTATTAGCACCGGTGCCACCGCTAGCGCCCTGGTTAAAGGCAATACCAATCCGATTACAAGCATG
TCTGCCCCGGTTCAAGCATCAGCTCCAGCACTGACAAAATCCCAAACCGATCGTCTGGAGGTTCTGCTGAATCCG
AAAGACGAAATCAGCCTGAATTCCGGCAAACCGTTTCGTGAACTGGAGAGCGAACTGCTGTCACGTCGTAAAAAA
GACCTGCAACAAATCTATGCCGAAGAACGTGAGAACTATCTGGGGAAACTGGAACGTGAAATCACCCGCTTTTTC
GTGGATCGTGGCTTTCTGGAGATCAAATCCCCGATTCTGATTCCTCTGGAGTATATCGAGCGTATGGGCATCGAC
AATGATACCGAACTGAGCAAACAAATTTTCCGTGTGGATAAAAACTTCTGTCTGCGCCCTATGCTAGCACCAAAT
CTGGCTAACTATCTGCGCAAACTGGACCGTGCCCTGCCTGATCCTATCAAAATCTTCGAGATCGGCCCGTGTTAT
CGTAAAGAGTCCGACGGTAAAGAACATCTGGAGGAGTTTACCATGCTGAACTTTTGCCAAATGGGTTCAGGTTGT
ACTCGTGAGAACCTGGAAAGCATCATCACCGATTTTCTGAACCACCTGGGCATTGACTTCAAAATTGTGGGCGAC
AGCTGTATGGTGTTTGGCGACACCCTGGATGTCATGCACGGCGACCTGGAACTGTCTAGTGCCGTTGTGGGCCCA
ATCCCGCTGGATCGTGAGTGGGGTATCGACAAACCTTGGATCGGTGCGGGTTTTGGTCTGGAGCGTCTGCTGAAA
GTAAAACACGACTTCAAGAACATCAAACGTGCTGCACGTTCCGAGTCCTATTACAATGGTATTTCTACTAACCTG
TAA (SEQ ID NO:75)
Protein:
MASVKVAVRVRPMNRREKDLEAKFIIQMEKSKTTITNLKIPEGGTGDSGRERTKTFTYDFSFYSADTKSPDYVSQ
EMVFKTLGTDVVKSAFEGYNACVFAYGQTGSGKSYTMMGNSGDSGLIPRICEGLFSRINETTRWDEASFRTEVSY
LEIYNERVRDLLRRKSSKTFNLRVREHPKEGPYVEDLSKHLVQNYGDVEELMDAGNINRTTAATGMNDVSSRSHA
IFTIKFTQAKFDSEMPCETVSKIHLVDLAGSERADATGATGVRLKEGGNINKSLVTLGNVISALADLSQDAANTL
AKKKQVFVPYRDSVLTWLLKDSLGGNSKTIMIATISPADVNYGETLSTLRYANRAKNIINKPTINEDANVKLIRE
LRAEIARLKTLLAQGNQIALLDSPTMEDNSVLNEDSNLEHVEGQPRRSMSQPVLNVEGDKRTSSTSATQQQVLSG
AFSSADVRSIPIIQTWEENKALKTKITILRGELQMYQRRYSEAKEASQKRVKEVMDDYVDLKLGQENVQEKMEQY
KLMEEDLLAMQSRIETSEDNFARQMKEFEAQKHAMEERIKELELSATDANNTTVGSFRGTLDDILKKNDPDFTLT
SGYEERKINDLEAKLLSEIDKVAELEDHIQQLRQELDDQSARLADSENVRAQLEAATGQGILGAAGNAMVPNSTF
MIGNGRESQTRDQLNYIDDLETKLADAKKENDKARQALVEYMNKCSKLEHEIRTMVKNSTFDSSSMLLGGQTSDE
LKIQIGKVNGELNVLRAENRELRIRCDQLTGGDGNLSISLGQSRLMAGIATNDVDSIGQGNETGGTSMRILPRES
QLDDLEESKLPLMDTSSAVRNQQQFASMWEDFESVKDSLQNNHNDTLEGSFNSSMPPPGRDATQSFLSQKSFKNS
PIVMQKPKSLHLHLKSHQSEGAGEQIQNNSFSTKTASPHVSQSHIPILHDMQQILDSSAMFLEGQHDVAVNVEQM
QEKMSQIREALARLFERLKSSAALFEEILERMGSSDPNADKIKKMKLAFETSINDKLNVSAILEAAEKDLHNMSL
NFSILEKSIVSQAAEASRRFTIAPDAEDVASSSLLNASYSPLFKFTSNSDIVEKLQNEVSELKNELEMARTRDMR
SPLNGSSGRLSDVQINTNRMFEDLEVSEATLQKAKEENSTLKSQFAELEANLHQVNSKLGEVRCELNEALARVDG
EQETRVKAENALEEARQLISSLKHEENELKKTITDMGMRLNEAKKSDEFLKSELSTALEEEKKSQNLADELSEEL
NGWRMRTKEAENKVEHASSEKSEMLERIVHLETEMEKLSTSEIAADYCSTKMTERKKEIELAKYREDFENAAIVG
LERISKEISELTKKTLKAKIIPSNISSIQLVCDELCRRLSREREQQHEYAKVMRDVNEKIEKLQLEKDALEHELK
MMSSNNENVPPVGTSVSGMPTKTSNQKCAQPHYTSPTRQLLHESTMAVDAIVQKLKKTHNMSGMGPELKETIGNV
INESRVLRDFLHQKLILFKGIDMSNWKNETVDQLITDLGQLHQDNLMLEEQIKKYKKELKLTKSAIPTLGVEFQD
RIKTEIGKIATDMGGAVKEIRKKSGYPYDVPDYASTMDAQTRRRERRAEKQAQWKAANPPLDGAGAGAGAGAGAG
GLATMDAQTRRRERRAEKQAQWKAANPPLDGAGAGAGAGAGAGGLATMDAQTRRRERRAEKQAQWKAANPPLDGA
GAGAGAGAGAGGLATMDAQTRRRERRAEKQAQWKAANPPLGTGAPGSAGSAAGSGACPVPLQLPPLERLTLDDKK
PLNTLISATGLWMSRTGTIHKIKHHEVSRSKIYIEMACGDHLVVNNSRSSRTARALRHHKYRKTCKRCRVSDEDL
NKFLTKANEDQTSVKVKVVSAPTRTKKAMPKSVARAPKPLENTEAAQAQPSGSKFSPAIPVSTQESVSVPASVST
SISSISTGATASALVKGNTNPITSMSAPVQASAPALTKSQTDRLEVLLNPKDEISLNSGKPFRELESELLSRRKK
DLQQIYAEERENYLGKLEREITRFFVDRGFLEIKSPILIPLEYIERMGIDNDTELSKQIFRVDKNFCLRPMLAPN
LANYLRKLDRALPDPIKIFEIGPCYRKESDGKEHLEEFTMLNFCQMGSGCTRENLESIITDFLNHLGIDFKIVGD
SCMVFGDTLDVMHGDLELSSAVVGPIPLDREWGIDKPWIGAGFGLERLLKVKHDFKNIKRAARSESYYNGISTNL
(SEQ ID NO:76)

KIF13A-FUS-PylRS^AF
DNA:
ATGTCGGATACCAAGGTAAAAGTTGCCGTCCGGGTCCGGCCCATGAACCGACGAGAACTGGAACTGAACACCAAG
TGCGTGGTGGAGATGGAAGGGAATCAAACGGTCCTGCACCCTCCTCCTTCTAACACCAAACAGGGAGAAAGGAAA

CCTCCCAAGGTATTTGCCTTTGATTATTGCTTTTGGTCCATGGATGAATCTAACACTACAAAATACGCTGGTCAA
GAAGTGGTTTTCAAGTGCCTTGGGGAAGGAATTCTTGAAAAAGCCTTTCAGGGGTATAATGCGTGTATTTTTGCA
TATGGACAGACAGGTTCGGGAAAATCCTTTTCCATGATGGGCCATGCTGAGCAGCTGGGCCTTATTCCAAGGCTC
TGCTGTGCTTTATTTAAAAGGATCTCTTTGGAGCAAAATGAGTCACAGACCTTTAAAGTTGAAGTGTCCTATATG
GAAATTTATAATGAGAAAGTTCGGGATCTTTTAGACCCCAAAGGGAGTAGACAGTCTCTTAAAGTTCGAGAACAT
AAAGTTTTGGGACCATATGTAGATGGTTTATCTCAACTAGCTGTCACTAGTTTTGAGGATATTGAGTCATTGATG
TCTGAGGGAAATAAGTCTCGAACGGTAGCTGCTACCAACATGAACGAAGAAAGCAGCCGCTCCCATGCTGTGTTC
AACATCATAATCACACAGACACTTTATGACCTGCAGTCTGGGAATTCCGGGGAGAAAGTCAGTAAGGTCAGCTTG
GTAGACCTGGCGGGTAGCGAAAGAGTATCTAAAACAGGAGCTGCAGGAGAGCGACTGAAAGAAGGCAGCAACATT
AACAAATCGCTTACAACCTTGGGGTTGGTTATATCATCACTGGCTGACCAGGCAGCTGGCAAGGGTAAAAGCAAA
TTTGTGCCTTATCGAGATTCAGTCCTCACTTGGCTGCTTAAGGACAACTTGGGGGGCAACAGCCAAACCTCTATG
ATAGCCACAATCAGCCCAGCCGCAGACAACTATGAAGAGACCCTCTCCACATTAAGATATGCAGACCGAGCCAAA
AGGATTGTGAACCATGCTGTTGTGAATGAGGACCCCAACGCAAAAGTGATCCGAGAACTGCGGGAGGAAGTCGAG
AAACTGAGAGAGCAGCTCTCTCAGGCAGAGGCCATGAAGGCCGAACTGAAGGAGAAGCTCGAAGAGTCTGAAAAG
CTGATAAAAGAACTAACAGTGACTTGGGAATATACAGATATTGAAATGAACAGATTGGGAAAGGGCGCCCCCGGC
TCCGCCGGCTCCGCCGCCGGCTCCGGCATGGCCTCAAACGATTATACCCAACAAGCAACCCAAAGCTATGGGGCC
TACCCCACCCAGCCCGGGCAGGGCTATTCCCAGCAGAGCAGTCAGCCCTACGGACAGCAGAGTTACAGTGGTTAT
AGCCAGTCCACGGACACTTCAGGATATGGCCAGAGCAGCTATTCTTCTTATGGCCAGAGCCAGAACACAGGCTAT
GGAACTCAGTCAACTCCCCAGGGATATGGCTCGACTGGCGGCTATGGCAGTAGCCAGAGCTCCCAATCGTCTTAC
GGGCAGCAGTCCTCCTACCCTGGCTATGGCCAGCAGCCAGCTCCCAGCAGCACCTCGGGAAGTTACGGTAGCAGT
TCTCAGAGCAGCAGCTATGGGCAGCCCCAGAGTGGGAGCTACAGCCAGCAGCCTAGCTATGGTGGACAGCAGCAA
AGCTATGGACAGCAGCAAAGCTATAATCCCCCTCAGGGCTATGGACAGCAGAACCAGTACAACAGCAGCAGTGGT
GGTGGAGGTGGAGGTGGAGGTGGAGGTAACTATGGCCAAGATCAATCCTCCATGAGTAGTGGTGGTGGCAGTGGT
GGCGGTTATGGCAATCAAGACCAGAGTGGTGGAGGTGGCAGCGGTGGCTATGGACAGCAGGACCGTGGAGGCCGC
GGCAGGGGTGGCAGTGGTGGCGGCGGCGGCGGCGGTGGTGGTTACAACCGCAGCAGTGGTGGCTATGAACCC
AGAGGTCGTGGAGGTGGCCGTGGAGGCAGAGGTGGCATGGGCGGAAGTGACCGTGGTGGCTTCAATAAATTTGGT
GGCCCTCGGGACCAAGGATCACGTCATGACTCCGAACAGGATAATTCAGACAACAACACCATCTTTGTGCAAGGC
CTGGGTGAGAATGTTACAATTGAGTCTGTGGCTGATTACTTCAAGCAGATTGGTATTATTAAGACAAACAAGAAA
ACGGGACAGCCCATGATTAATTTGTACACAGACAGGGAAACTGGCAAGCTGAAGGGAGAGGCAACGGTCTCTTTT
GATGACCCACCTTCAGCTAAAGCAGCTATTGACTGGTTTGATGGTAAAGAATTCTCCGGAAATCCTATCAAGGTC
TCATTTGCTACTCGCCGGGCAGACTTTAATCGGGGTGGTGGCAATGGTCGTGGAGGCCGAGGGCGAGGAGGACCC
ATGGGCCGTGGAGGCTATGGAGGTGGTGGCAGTGGTGGTGGTGGCCGAGGAGGATTTCCCAGTGGAGGTGGTGGC
GGTGGAGGACAGCAGCGAGCTGGTGACTGGAAGTGTCCTAATCCCACCTGTGAGAATATGAACTTCTCTTGGAGG
AATGAATGCAACCAGTGTAAGGCCCCTAAACCAGATGGCCCAGGAGGGGGACCAGGTGGCTCTCACATGGGGGGT
AACTACGGGGATGATCGTCGTGGTGGCAGAGGAGGCGATTACAAGGATGACGACGATAAGGGTACCGGCGCCCCC
GGCTCCGCCGGCTCCGCCGCCGGCTCCGGCGCGTGCCCGGTGCCGCTGCAGCTGCCGCCGCTGGAACGCCTGACC
CTGGATGATAAAAAACCGCTGAATACCCTGATCTCTGCTACTGGTCTGTGGATGAGTCGTACCGGAACCATTCAT
AAAATCAAACACCACGAGGTTAGCCGTTCGAAAATCTATATTGAGATGGCGTGTGGCGATCATCTGGTTGTGAAC
AATAGCCGCTCTTCTCGTACAGCACGTGCACTGCGTCACCACAAATATCGTAAAACCTGTAAACGTTGCCGTGTG
TCCGATGAGGATCTGAACAAATTCCTGACAAAAGCCAATGAGGACCAAACAAGCGTGAAAGTGAAAGTCGTTAGC
GCTCCTACCCGTACTAAAAAAGCAATGCCGAAATCCGTTGCTCGTGCCCCTAAACCACTGGAAAACACTGAAGCA
GCACAGGCACAGCCGTCTGGAAGCAAATTCTCTCCGGCCATTCCTGTTTCTACCCAGGAGTCCGTTTCTGTTCCA
GCAAGTGTGAGCACCAGCATTAGCAGTATTAGCACCGGTGCCACCGCTAGCGCCCTGGTTAAAGGCAATACCAAT
CCGATTACAAGCATGTCTGCCCCGGTTCAAGCATCAGCTCCAGCACTGACAAAATCCCAAACCGATCGTCTGGAG
GTTCTGCTGAATCCGAAAGACGAAATCAGCCTGAATTCCGGCAAACCGTTTCGTGAACTGGAGAGCGAACTGCTG
TCACGTCGTAAAAAAGACCTGCAACAAATCTATGCCGAAGAACGTGAGAACTATCTGGGGAAACTGGAACGTGAA
ATCACCCGCTTTTTCGTGGATCGTGGCTTTCTGGAGATCAAATCCCCGATTCTGATTCCTCTGGAGTATATCGAG
CGTATGGGCATCGACAATGATACCGAACTGAGCAAACAAATTTTCCGTGTGGATAAAAACTTCTGTCTGCGCCCT
ATGCTAGCACCAAATCTGGCTAACTATCTGCGCAAACTGGACCGTGCCCTGCCTGATCCTATCAAAATCTTCGAG
ATCGGCCCGTGTTATCGTAAAGAGTCCGACGGTAAAGAACATCTGGAGGAGTTTACCATGCTGAACTTTTGCCAA
ATGGGTTCAGGTTGTACTCGTGAGAACCTGGAAAGCATCATCACCGATTTTCTGAACCACCTGGGCATTGACTTC
AAAATTGTGGGCGACAGCTGTATGGTGTTTGGCGACACCCTGGATGTCATGCACGGCGACCTGGAACTGTCTAGT
GCCGTTGTGGGCCCAATCCCGCTGGATCGTGAGTGGGGTATCGACAAACCTTGGATCGGTGCGGGTTTTGGTCTG
GAGCGTCTGCTGAAAGTAAAACACGACTTCAAGAACATCAAACGTGCTGCACGTTCCGAGTCCTATTACAATGGT
ATTTCTACTAACCTGTAA (SEQ ID NO:77)
Protein:
MSDTKVKVAVRVRPMNRRELELNTKCVVEMEGNQTVLHPPPSNTKQGERKPPKVFAFDYCFWSMDESNTTKYAGQ
EVVFKCLGEGILEKAFQGYNACIFAYGQTGSGKSFSMMGHAEQLGLIPRLCCALFKRISLEQNESQTFKVEVSYM

EIYNEKVRDLLDPKGSRQSLKVREHKVLGPYVDGLSQLAVTSFEDIESLMSEGNKSRTVAATNMNEESSRSHAVF
NIIITQTLYDLQSGNSGEKVSKVSLVDLAGSERVSKTGAAGERLKEGSNINKSLTTLGLVISSLADQAAGKGKSK
FVPYRDSVLTWLLKDNLGGNSQTSMIATISPAADNYEETLSTLRYADRAKRIVNHAVVNEDPNAKVIRELREEVE
KLREQLSQAEAMKAELKEKLEESEKLIKELTVTWEYTDIEMNRLGKGAPGSAGSAAGSGMASNDYTQQATQSYGA
YPTQPGQGYSQQSSQPYGQQSYSGYSQSTDTSGYGQSSYSSYGQSQNTGYGTQSTPQGYGSTGGYGSSQSSQSSY
GQQSSYPGYGQQPAPSSTSGSYGSSSQSSSYGQPQSGSYSQQPSYGGQQQSYGQQQSYNPPQGYGQQNQYNSSSG
GGGGGGGGGNYGQDQSSMSSGGGSGGGYGNQDQSGGGGSGGYGQQDRGGRGRGGSGGGGGGGGGGYNRSSGGYEP
RGRGGGRGGRGGMGGSDRGGFNKFGGPRDQGSRHDSEQDNSDNNTIFVQGLGENVTIESVADYFKQIGIIKTNKK
TGQPMINLYTDRETGKLKGEATVSFDDPPSAKAAIDWFDGKEFSGNPIKVSFATRRADFNRGGGNGRGGRGRGGP
MGRGGYGGGGSGGGGRGGFPSGGGGGGGQQRAGDWKCPNPTCENMNFSWRNECNQCKAPKPDGPGGGPGGSHMGG
NYGDDRRGGRGGDYKDDDDKGTGAPGSAGSAAGSGACPVPLQLPPLERLTLDDKKPLNTLISATGLWMSRTGTIH
KIKHHEVSRSKIYIEMACGDHLVVNNSRSSRTARALRHHKYRKTCKRCRVSDEDLNKFLTKANEDQTSVKVKVVS
APTRTKKAMPKSVARAPKPLENTEAAQAQPSGSKFSPAIPVSTQESVSVPASVSTSISSISTGATASALVKGNTN
PITSMSAPVQASAPALTKSQTDRLEVLLNPKDEISLNSGKPFRELESELLSRRKKDLQQIYAEERENYLGKLERE
ITRFFVDRGFLEIKSPILIPLEYIERMGIDNDTELSKQIFRVDKNFCLRPMLAPNLANYLRKLDRALPDPIKIFE
IGPCYRKESDGKEHLEEFTMLNFCQMGSGCTRENLESIITDFLNHLGIDFKIVGDSCMVFGDTLDVMHGDLELSS
AVVGPIPLDREWGIDKPWIGAGFGLERLLKVKHDFKNIKRAARSESYYNGISTNL (SEQ ID NO:78)

KIF13A-FUS-PylRS^AA
DNA:
ATGTCGGATACCAAGGTAAAAGTTGCCGTCCGGGTCCGGCCCATGAACCGACGAGAACTGGAACTGAACACCAAG
TGCGTGGTGGAGATGGAAGGGAATCAAACGGTCCTGCACCCTCCTCCTTCTAACACCAAACAGGGAGAAAGGAAA
CCTCCCAAGGTATTTGCCTTTGATTATTGCTTTTGGTCCATGGATGAATCTAACACTACAAAATACGCTGGTCAA
GAAGTGGTTTTCAAGTGCCTTGGGGAAGGAATTCTTGAAAAAGCCTTTCAGGGGTATAATGCGTGTATTTTTGCA
TATGGACAGACAGGTTCGGGAAAATCCTTTTCCATGATGGGCCATGCTGAGCAGCTGGGCCTTATTCCAAGGCTC
TGCTGTGCTTTATTTAAAAGGATCTCTTTGGAGCAAAATGAGTCACAGACCTTTAAAGTTGAAGTGTCCTATATG
GAAATTTATAATGAGAAAGTTCGGGATCTTTTAGACCCCAAAGGGAGTAGACAGTCTCTTAAAGTTCGAGAACAT
AAAGTTTTGGGACCATATGTAGATGGTTTATCTCAACTAGCTGTCACTAGTTTTGAGGATATTGAGTCATTGATG
TCTGAGGGAAATAAGTCTCGAACGGTAGCTGCTACCAACATGAACGAAGAAAGCAGCCGCTCCCATGCTGTGTTC
AACATCATAATCACACAGACACTTTATGACCTGCAGTCTGGGAATTCCGGGGAGAAAGTCAGTAAGGTCAGCTTG
GTAGACCTGGCGGGTAGCGAAAGAGTATCTAAAACAGGAGCTGCAGGAGAGCGACTGAAAGAAGGCAGCAACATT
AACAAATCGCTTACAACCTTGGGGTTGGTTATATCATCACTGGCTGACCAGGCAGCTGGCAAGGGTAAAAGCAAA
TTTGTGCCTTATCGAGATTCAGTCCTCACTTGGCTGCTTAAGGACAACTTGGGGGGCAACAGCCAAACCTCTATG
ATAGCCACAATCAGCCCAGCCGCAGACAACTATGAAGAGACCCTCTCCACATTAAGATATGCAGACCGAGCCAAA
AGGATTGTGAACCATGCTGTTGTGAATGAGGACCCCAACGCAAAAGTGATCCGAGAACTGCGGGAGGAAGTCGAG
AAACTGAGAGAGCAGCTCTCTCAGGCAGAGGCCATGAAGGCCGAACTGAAGGAGAAGCTCGAAGAGTCTGAAAAG
CTGATAAAAGAACTAACAGTGACTTGGGAATATACAGATATTGAAATGAACAGATTGGGAAAGGGCGCCCCCGGC
TCCGCCGGCTCCGCCGCCGGCTCCGGCATGGCCTCAAACGATTATACCCAACAAGCAACCCAAAGCTATGGGGCC
TACCCCACCCAGCCCGGGCAGGGCTATTCCCAGCAGAGCAGTCAGCCCTACGGACAGCAGAGTTACAGTGGTTAT
AGCCAGTCCACGGACACTTCAGGATATGGCCAGAGCAGCTATTCTTCTTATGGCCAGAGCCAGAACACAGGCTAT
GGAACTCAGTCAACTCCCCAGGGATATGGCTCGACTGGCGGCTATGGCAGTAGCCAGAGCTCCCAATCGTCTTAC
GGGCAGCAGTCCTCCTACCCTGGCTATGGCCAGCAGCCAGCTCCCAGCAGCACCTCGGGAAGTTACGGTAGCAGT
TCTCAGAGCAGCAGCTATGGGCAGCCCCAGAGTGGGAGCTACAGCCAGCAGCCTAGCTATGGTGGACAGCAGCAA
AGCTATGGACAGCAGCAAAGCTATAATCCCCCTCAGGGCTATGGACAGCAGAACCAGTACAACAGCAGCAGTGGT
GGTGGAGGTGGAGGTGGAGGTGGAGGTAACTATGGCCAAGATCAATCCTCCATGAGTAGTGGTGGTGGCAGTGGT
GGCGGTTATGGCAATCAAGACCAGAGTGGTGGAGGTGGCAGCGGTGGCTATGGACAGCAGGACCGTGGAGGCCGC
GGCAGGGGTGGCAGTGGTGGCGGCGGCGGCGGCGGCGGTGGTGGTTACAACCGCAGCAGTGGTGGCTATGAACCC
AGAGGTCGTGGAGGTGGCCGTGGAGGCAGAGGTGGCATGGGCGGAAGTGACCGTGGTGGCTTCAATAAATTTGGT
GGCCCTCGGGACCAAGGATCACGTCATGACTCCGAACAGGATAATTCAGACAACAACACCATCTTTGTGCAAGGC
CTGGGTGAGAATGTTACAATTGAGTCTGTGGCTGATTACTTCAAGCAGATTGGTATTATTAAGACAAACAAGAAA
ACGGGACAGCCCATGATTAATTTGTACACAGACAGGGAAACTGGCAAGCTGAAGGGAGAGGCAACGGTCTCTTTT
GATGACCCACCTTCAGCTAAAGCAGCTATTGACTGGTTTGATGGTAAAGAATTCTCCGGAAATCCTATCAAGGTC
TCATTTGCTACTCGCCGGGCAGACTTTAATCGGGGTGGTGGCAATGGTCGTGGAGGCCGAGGGCGAGGAGGACCC
ATGGGCCGTGGAGGCTATGGAGGTGGTGGCAGTGGTGGTGGTGGCCGAGGAGGATTTCCCAGTGGAGGTGGTGGC
GGTGGAGGACAGCAGCGAGCTGGTGACTGGAAGTGTCCTAATCCCACCTGTGAGAATATGAACTTCTCTTGGAGG
AATGAATGCAACCAGTGTAAGGCCCCTAAACCAGATGGCCCAGGAGGGGGACCAGGTGGCTCTCACATGGGGGGT
AACTACGGGGATGATCGTCGTGGTGGCAGAGGAGGCGGCGCCCCCGGCTCCGCCGGCTCCGCCGCCGGCTCCGGC
ATGGCGTGCCCGGTGCCGCTGCAGCTGCCGCCGCTGGAACGCCTGACCCTGGATGACAAAAAACCGCTGAATACC

CTGATCTCTGCTACTGGTCTGTGGATGAGTCGTACCGGAACCATTCATAAAATCAAACACCACGAGGTTAGCCGT
TCGAAAATCTATATTGAGATGGCGTGTGGCGATCATCTGGTTGTGAACAATAGCCGCTCTTCTCGTACAGCACGT
GCACTGCGTCACCACAAATATCGTAAAACCTGTAAACGTTGCCGTGTGTCCGATGAGGATCTGAACAAATTCCTG
ACAAAAGCCAATGAGGACCAAACAAGCGTGAAAGTGAAAGTCGTTAGCGCTCCTACCCGTACTAAAAAAGCAATG
CCGAAATCCGTTGCTCGTGCCCCTAAACCACTGGAAAACACTGAAGCAGCACAGGCACAGCCGTCTGGAAGCAAA
TTCTCTCCGGCCATTCCTGTTTCTACCCAGGAGTCCGTTTCTGTTCCAGCAAGTGTGAGCACCAGCATTAGCAGT
ATTAGCACCGGTGCCACCGCTAGCGCCCTGGTTAAAGGCAATACCAATCCGATTACAAGCATGTCTGCCCCGGTT
CAAGCATCAGCTCCAGCACTGACAAAATCCCAAACCGATCGTCTGGAGGTTCTGCTGAATCCGAAAGACGAAATC
AGCCTGAATTCCGGCAAACCGTTTCGTGAACTGGAGAGCGAACTGCTGTCACGTCGTAAAAAAGACCTGCAACAA
ATCTATGCCGAAGAACGTGAGAACTATCTGGGGAAACTGGAACGTGAAATCACCCGCTTTTTCGTGGATCGTGGC
TTTCTGGAGATCAAATCCCCGATTCTGATTCCTCTGGAGTATATCGAGCGTATGGGCATCGACAATGATACCGAA
CTGAGCAAACAAATTTTCCGTGTGGATAAAAACTTCTGTCTGCGCCCTATGCTGGCACCAAATCTGTATAACTAT
CTGCGCAAACTGGACCGTGCCCTGCCTGATCCTATCAAAATCTTCGAGATCGGCCCGTGTTATCGTAAAGAGTCC
GACGGTAAAGAACATCTGGAGGAGTTTACCATGCTGGCCTTTGCCCAAATGGGTTCAGGTTGTACTCGTGAGAAC
CTGGAAAGCATCATCACCGATTTTCTGAACCACCTGGGCATTGACTTCAAAATTGTGGGCGACAGCTGTATGGTG
TATGGCGACACCCTGGATGTCATGCACGGCGACCTGGAACTGTCTAGTGCCGTTGTTGGACCAATTCCGCTGGAC
CGTGAGTGGGGTATCGACAAACCGTGGATCGGAGCAGGATTCGGTCTGGAACGCCTGCTGAAAGTGAAACACGAC
TTCAAAAACATCAAACGTGCCGCCCGTTCTGAATCGTATTATAACGGGATCTCTACGAACCTGTAA (SEQ ID
NO:79)
Protein:
MSDTKVKVAVRVRPMNRRELELNTKCVVEMEGNQTVLHPPPSNTKQGERKPPKVFAFDYCFWSMDESNTTKYAGQ
EVVFKCLGEGILEKAFQGYNACIFAYGQTGSGKSFSMMGHAEQLGLIPRLCCALFKRISLEQNESQTFKVEVSYM
EIYNEKVRDLLDPKGSRQSLKVREHKVLGPYVDGLSQLAVTSFEDIESLMSEGNKSRTVAATNMNEESSRSHAVF
NIIITQTLYDLQSGNSGEKVSKVSLVDLAGSERVSKTGAAGERLKEGSNINKSLTTLGLVISSLADQAAGKGKSK
FVPYRDSVLTWLLKDNLGGNSQTSMIATISPAADNYEETLSTLRYADRAKRIVNHAVVNEDPNAKVIRELREEVE
KLREQLSQAEAMKAELKEKLEESEKLIKELTVTWEYTDIEMNRLGKGAPGSAGSAAGSGMASNDYTQQATQSYGA
YPTQPGQGYSQQSSQPYGQQSYSGYSQSTDTSGYGQSSYSSYGQSQNTGYGTQSTPQGYGSTGGYGSSQSSQSSY
GQQSSYPGYGQQPAPSSTSGSYGSSSQSSSYGQPQSGSYSQQPSYGGQQQSYGQQQSYNPPQGYGQQNQYNSSSG
GGGGGGGGGNYGQDQSSMSSGGGSGGGYGNQDQSGGGGSGGYGQQDRGGRGRGGSGGGGGGGGGGYNRSSGGYEP
RGRGGGRGGRGGMGGSDRGGFNKFGGPRDQGSRHDSEQDNSDNNTIFVQGLGENVTIESVADYFKQIGIIKTNKK
TGQPMINLYTDRETGKLKGEATVSFDDPPSAKAAIDWFDGKEFSGNPIKVSFATRRADFNRGGGNGRGGRGRGGP
MGRGGYGGGGSGGGGRGGFPSGGGGGGGQQRAGDWKCPNPTCENMNFSWRNECNQCKAPKPDGPGGGPGGSHMGG
NYGDDRRGGRGGGAPGSAGSAAGSGMACPVPLQLPPLERLTLDDKKPLNTLISATGLWMSRTGTIHKIKHHEVSR
SKIYIEMACGDHLVVNNSRSSRTARALRHHKYRKTCKRCRVSDEDLNKFLTKANEDQTSVKVKVVSAPTRTKKAM
PKSVARAPKPLENTEAAQAQPSGSKFSPAIPVSTQESVSVPASVSTSISSISTGATASALVKGNTNPITSMSAPV
QASAPALTKSQTDRLEVLLNPKDEISLNSGKPFRELESELLSRRKKDLQQIYAEERENYLGKLEREITRFFVDRG
FLEIKSPILIPLEYIERMGIDNDTELSKQIFRVDKNFCLRPMLAPNLYNYLRKLDRALPDPIKIFEIGPCYRKES
DGKEHLEEFTMLAFAQMGSGCTRENLESIITDFLNHLGIDFKIVGDSCMVYGDTLDVMHGDLELSSAVVGPIPLD
REWGIDKPWIGAGFGLERLLKVKHDFKNIKRAARSESYYNGISTNL (SEQ ID NO:80)

KIF13A-FUS-PylRS^AAAF
DNA:
ATGTCGGATACCAAGGTAAAAGTTGCCGTCCGGGTCCGGCCCATGAACCGACGAGAACTGGAACTGAACACCAAG
TGCGTGGTGGAGATGGAAGGGAATCAAACGGTCCTGCACCCTCCTCCTTCTAACACCAAACAGGGAGAAAGGAAA
CCTCCCAAGGTATTTGCCTTTGATTATTGCTTTTGGTCCATGGATGAATCTAACACTACAAAATACGCTGGTCAA
GAAGTGGTTTTCAAGTGCCTTGGGGAAGGAATTCTTGAAAAAGCCTTTCAGGGGTATAATGCGTGTATTTTTGCA
TATGGACAGACAGGTTCGGGAAAATCCTTTTCCATGATGGGCCATGCTGAGCAGCTGGGCCTTATTCCAAGGCTC
TGCTGTGCTTTATTTAAAAGGATCTCTTTGGAGCAAAATGAGTCACAGACCTTTAAAGTTGAAGTGTCCTATATG
GAAATTTATAATGAGAAAGTTCGGGATCTTTTAGACCCCAAAGGGAGTAGACAGTCTCTTAAAGTTCGAGAACAT
AAAGTTTTGGGACCATATGTAGATGGTTTATCTCAACTAGCTGTCACTAGTTTTGAGGATATTGAGTCATTGATG
TCTGAGGGAAATAAGTCTCGAACGGTAGCTGCTACCAACATGAACGAAGAAAGCAGCCGCTCCCATGCTGTGTTC
AACATCATAATCACACAGACACTTTATGACCTGCAGTCTGGGAATTCCGGGGAGAAAGTCAGTAAGGTCAGCTTG
GTAGACCTGGCGGGTAGCGAAAGAGTATCTAAAACAGGAGCTGCAGGAGAGCGACTGAAAGAAGGCAGCAACATT
AACAAATCGCTTACAACCTTGGGGTTGGTTATATCATCACTGGCTGACCAGGCAGCTGGCAAGGGTAAAAGCAAA
TTTGTGCCTTATCGAGATTCAGTCCTCACTTGGCTGCTTAAGGACAACTTGGGGGGCAACAGCCAAACCTCTATG
ATAGCCACAATCAGCCCAGCCGCAGACAACTATGAAGAGACCCTCTCCACATTAAGATATGCAGACCGAGCCAAA
AGGATTGTGAACCATGCTGTTGTGAATGAGGACCCCAACGCAAAAGTGATCCGAGAACTGCGGGAGGAAGTCGAG
AAACTGAGAGAGCAGCTCTCTCAGGCAGAGGCCATGAAGGCCGAACTGAAGGAGAAGCTCGAAGAGTCTGAAAAG

CTGATAAAAGAACTAACAGTGACTTGGGAATATACAGATATTGAAATGAACAGATTGGGAAAGGGCGCCCCCGGC
TCCGCCGGCTCCGCCGCCGGCTCCGGCATGGCCTCAAACGATTATACCCAACAAGCAACCCAAAGCTATGGGGCC
TACCCCACCCAGCCCGGGCAGGGCTATTCCCAGCAGAGCAGTCAGCCCTACGGACAGCAGAGTTACAGTGGTTAT
AGCCAGTCCACGGACACTTCAGGATATGGCCAGAGCAGCTATTCTTCTTATGGCCAGAGCCAGAACACAGGCTAT
GGAACTCAGTCAACTCCCCAGGGATATGGCTCGACTGGCGGCTATGGCAGTAGCCAGAGCTCCCAATCGTCTTAC
GGGCAGCAGTCCTCCTACCCTGGCTATGGCCAGCAGCCAGCTCCCAGCAGCACCTCGGGAAGTTACGGTAGCAGT
TCTCAGAGCAGCAGCTATGGGCAGCCCCAGAGTGGGAGCTACAGCCAGCAGCCTAGCTATGGTGGACAGCAGCAA
AGCTATGGACAGCAGCAAAGCTATAATCCCCCTCAGGGCTATGGACAGCAGAACCAGTACAACAGCAGCAGTGGT
GGTGGAGGTGGAGGTGGAGGTGGAGGTAACTATGGCCAAGATCAATCCTCCATGAGTAGTGGTGGTGGCAGTGGT
GGCGGTTATGGCAATCAAGACCAGAGTGGTGGAGGTGGCAGCGGTGGCTATGGACAGCAGGACCGTGGAGGCCGC
GGCAGGGGTGGCAGTGGTGGCGGCGGCGGCGGCGGCGGTGGTGGTTACAACCGCAGCAGTGGTGGCTATGAACCC
AGAGGTCGTGGAGGTGGCCGTGGAGGCAGAGGTGGCATGGGCGGAAGTGACCGTGGTGGCTTCAATAAATTTGGT
GGCCCTCGGGACCAAGGATCACGTCATGACTCCGAACAGGATAATTCAGACAACAACACCATCTTTGTGCAAGGC
CTGGGTGAGAATGTTACAATTGAGTCTGTGGCTGATTACTTCAAGCAGATTGGTATTATTAAGACAAACAAGAAA
ACGGGACAGCCCATGATTAATTTGTACACAGACAGGGAAACTGGCAAGCTGAAGGGAGAGGCAACGGTCTCTTTT
GATGACCCACCTTCAGCTAAAGCAGCTATTGACTGGTTTGATGGTAAAGAATTCTCCGGAAATCCTATCAAGGTC
TCATTTGCTACTCGCCGGGCAGACTTTAATCGGGGTGGTGGCAATGGTCGTGGAGGCCGAGGGCGAGGAGGACCC
ATGGGCCGTGGAGGCTATGGAGGTGGTGGCAGTGGTGGTGGTGGCCGAGGAGGATTTCCCAGTGGAGGTGGTGGC
GGTGGAGGACAGCAGCGAGCTGGTGACTGGAAGTGTCCTAATCCCACCTGTGAGAATATGAACTTCTCTTGGAGG
AATGAATGCAACCAGTGTAAGGCCCCTAAACCAGATGGCCCAGGAGGGGGACCAGGTGGCTCTCACATGGGGGGT
AACTACGGGGATGATCGTCGTGGTGGCAGAGGAGGCGATTACAAGGATGACGACGATAAGGGTACCGGCGCCCCC
GGCTCCGCCGGCTCCGCCGCCGGCTCCGGCGCGTGCCCGGTGCCGCTGCAGCTGCCGCCGCTGGAACGCCTGACC
CTGGATGATAAAAAACCGCTGAATACCCTGATCTCTGCTACTGGTCTGTGGATGAGTCGTACCGGAACCATTCAT
AAAATCAAACACCACGAGGTTAGCCGTTCGAAAATCTATATTGAGATGGCGTGTGGCGATCATCTGGTTGTGAAC
AATAGCCGCTCTTCTCGTACAGCACGTGCACTGCGTCACCACAAATATCGTAAAACCTGTAAACGTTGCCGTGTG
TCCGATGAGGATCTGAACAAATTCCTGACAAAAGCCAATGAGGACCAAACAAGCGTGAAAGTGAAAGTCGTTAGC
GCTCCTACCCGTACTAAAAAAGCAATGCCGAAATCCGTTGCTCGTGCCCCTAAACCACTGGAAAACACTGAAGCA
GCACAGGCACAGCCGTCTGGAAGCAAATTCTCTCCGGCCATTCCTGTTTCTACCCAGGAGTCCGTTTCTGTTCCA
GCAAGTGTGAGCACCAGCATTAGCAGTATTAGCACCGGTGCCACCGCTAGCGCCCTGGTTAAAGGCAATACCAAT
CCGATTACAAGCATGTCTGCCCCGGTTCAAGCATCAGCTCCAGCACTGACAAAATCCCAAACCGATCGTCTGGAG
GTTCTGCTGAATCCGAAAGACGAAATCAGCCTGAATTCCGGCAAACCGTTTCGTGAACTGGAGAGCGAACTGCTG
TCACGTCGTAAAAAAGACCTGCAACAAATCTATGCCGAAGAACGTGAGAACTATCTGGGGAAACTGGAACGTGAA
ATCACCCGCTTTTTCGTGGATCGTGGCTTTCTGGAGATCAAATCCCCGATTCTGATTCCTCTGGAGTATATCGAG
CGTATGGGCATCGACAATGATACCGAACTGAGCAAACAAATTTTCCGTGTGGATAAAAACTTCTGTCTGCGCCCT
ATGCTAGCACCAAATCTGGCTAACTATCTGCGCAAACTGGACCGTGCCCTGCCTGATCCTATCAAAATCTTCGAG
ATCGGCCCGTGTTATCGTAAAGAGTCCGACGGTAAAGAACATCTGGAGGAGTTTACCATGCTGGCCTTTGCCCAA
ATGGGTTCAGGTTGTACTCGTGAGAACCTGGAAAGCATCATCACCGATTTTCTGAACCACCTGGGCATTGACTTC
AAAATTGTGGGCGACAGCTGTATGGTGTTTGGCGACACCCTGGATGTCATGCACGGCGACCTGGAACTGTCTAGT
GCCGTTGTGGGCCCAATCCCGCTGGATCGTGAGTGGGGTATCGACAAACCTTGGATCGGTGCGGGTTTTGGTCTG
GAGCGTCTGCTGAAAGTAAAACACGACTTCAAGAACATCAAACGTGCTGCACGTTCCGAGTCCTATTACAATGGT
ATTTCTACTAACCTGTAA (SEQ ID NO:81)
Protein:
MSDTKVKVAVRVRPMNRRELELNTKCVVEMEGNQTVLHPPPSNTKQGERKPPKVFAFDYCFWSMDESNTTKYAGQ
EVVFKCLGEGILEKAFQGYNACIFAYGQTGSGKSFSMMGHAEQLGLIPRLCCALFKRISLEQNESQTFKVEVSYM
EIYNEKVRDLLDPKGSRQSLKVREHKVLGPYVDGLSQLAVTSFEDIESLMSEGNKSRTVAATNMNEESSRSHAVF
NIIITQTLYDLQSGNSGEKVSKVSLVDLAGSERVSKTGAAGERLKEGSNINKSLTTLGLVISSLADQAAGKGKSK
FVPYRDSVLTWLLKDNLGGNSQTSMIATISPAADNYEETLSTLRYADRAKRIVNHAVVNEDPNAKVIRELREEVE
KLREQLSQAEAMKAELKEKLEESEKLIKELTVTWEYTDIEMNRLGKGAPGSAGSAAGSGMASNDYTQQATQSYGA
YPTQPGQGYSQQSSQPYGQQSYSGYSQSTDTSGYGQSSYSSYGQSQNTGYGTQSTPQGYGSTGGYGSSQSSQSSY
GQQSSYPGYGQQPAPSSTSGSYGSSSQSSSYGQPQSGSYSQQPSYGGQQQSYGQQQSYNPPQGYGQQNQYNSSSG
GGGGGGGGGNYGQDQSSMSSGGGSGGGYGNQDQSGGGGSGGYGQQDRGGRGRGGSGGGGGGGGGYNRSSGGYEP
RGRGGGRGGRGGMGGSDRGGFNKFGGPRDQGSRHDSEQDNSDNNTIFVQGLGENVTIESVADYFKQIGIIKTNKK
TGQPMINLYTDRETGKLKGEATVSFDDPPSAKAAIDWFDGKEFSGNPIKVSFATRRADFNRGGGNGRGGRGRGGP
MGRGGYGGGGSGGGGRGGFPSGGGGGGGQQRAGDWKCPNPTCENMNFSWRNECNQCKAPKPDGPGGGPGGSHMGG
NYGDDRRGGRGGDYKDDDDKGTGAPGSAGSAAGSGACPVPLQLPPLERLTLDDKKPLNTLISATGLWMSRTGTIH
KIKHHEVSRSKIYIEMACGDHLVVNNSRSSRTARALRHHKYRKTCKRCRVSDEDLNKFLTKANEDQTSVKVKVVS
APTRTKKAMPKSVARAPKPLENTEAAQAQPSGSKFSPAIPVSTQESVSVPASVSTSISSISTGATASALVKGNTN
PITSMSAPVQASAPALTKSQTDRLEVLLNPKDEISLNSGKPFRELESELLSRRKKDLQQIYAEERENYLGKLERE

ITRFFVDRGFLEIKSPILIPLEYIERMGIDNDTELSKQIFRVDKNFCLRPMLAPNLANYLRKLDRALPDPIKIFE
IGPCYRKESDGKEHLEEFTMLAFAQMGSGCTRENLESIITDFLNHLGIDFKIVGDSCMVFGDTLDVMHGDLELSS
AVVGPIPLDREWGIDKPWIGAGFGLERLLKVKHDFKNIKRAARSESYYNGISTNL (SEQ ID NO:82)

KIF13A-EWSR1-MCP
DNA:
ATGTCGGATACCAAGGTAAAAGTTGCCGTCCGGGTCCGGCCCATGAACCGACGAGAACTGGAACTGAACACCAAG
TGCGTGGTGGAGATGGAAGGGAATCAAACGGTCCTGCACCCTCCTCCTTCTAACACCAAACAGGGAGAAAGGAAA
CCTCCCAAGGTATTTGCCTTTGATTATTGCTTTTGGTCCATGGATGAATCTAACACTACAAAATACGCTGGTCAA
GAAGTGGTTTTCAAGTGCCTTGGGGAAGGAATTCTTGAAAAAGCCTTTCAGGGGTATAATGCGTGTATTTTTGCA
TATGGACAGACAGGTTCGGGAAAATCCTTTTCCATGATGGGCCATGCTGAGCAGCTGGGCCTTATTCCAAGGCTC
TGCTGTGCTTTATTTAAAAGGATCTCTTTGGAGCAAAATGAGTCACAGACCTTTAAAGTTGAAGTGTCCTATATG
GAAATTTATAATGAGAAAGTTCGGGATCTTTTAGACCCCAAAGGGAGTAGACAGTCTCTTAAAGTTCGAGAACAT
AAAGTTTTGGGACCATATGTAGATGGTTTATCTCAACTAGCTGTCACTAGTTTTGAGGATATTGAGTCATTGATG
TCTGAGGGAAATAAGTCTCGAACGGTAGCTGCTACCAACATGAACGAAGAAAGCAGCCGCTCCCATGCTGTGTTC
AACATCATAATCACACAGACACTTTATGACCTGCAGTCTGGGAATTCCGGGGAGAAAGTCAGTAAGGTCAGCTTG
GTAGACCTGGCGGGTAGCGAAAGAGTATCTAAAACAGGAGCTGCAGGAGAGCGACTGAAAGAAGGCAGCAACATT
AACAAATCGCTTACAACCTTGGGGTTGGTTATATCATCACTGGCTGACCAGGCAGCTGGCAAGGGTAAAAGCAAA
TTTGTGCCTTATCGAGATTCAGTCCTCACTTGGCTGCTTAAGGACAACTTGGGGGGCAACAGCCAAACCTCTATG
ATAGCCACAATCAGCCCAGCCGCAGACAACTATGAAGAGACCCTCTCCACATTAAGATATGCAGACCGAGCCAAA
AGGATTGTGAACCATGCTGTTGTGAATGAGGACCCCAACGCAAAAGTGATCCGAGAACTGCGGGAGGAAGTCGAG
AAACTGAGAGAGCAGCTCTCTCAGGCAGAGGCCATGAAGGCCGAACTGAAGGAGAAGCTCGAAGAGTCTGAAAAG
CTGATAAAAGAACTAACAGTGACTTGGGAAATGGCGTCCACGGATTACAGTACCTATAGCCAAGCTGCAGCGCAG
CAGGGCTACAGTGCTTACACCGCCCAGCCCACTCAAGGATATGCACAGACCACCCAGGCATATGGGCAACAAAGC
TATGGAACCTATGGACAGCCCACTGATGTCAGCTATACCCAGGCTCAGACCACTGCAACCTATGGGCAGACCGCC
TATGCAACTTCTTATGGACAGCCTCCCACTGGTTATACTACTCCAACTGCCCCCCAGGCATACAGCCAGCCTGTC
CAGGGGTATGGCACTGGTGCTTATGATACCACCACTGCTACAGTCACCACCACCCAGGCCTCCTATGCAGCTCAG
TCTGCATATGGCACTCAGCCTGCTTATCCAGCCTATGGGCAGCAGCCAGCAGCCACTGCACCTACAAGACCGCAG
GATGGAAACAAGCCCACTGAGACTAGTCAACCTCAATCTAGCACAGGGGGTTACAACCAGCCCAGCCTAGGATAT
GGACAGAGTAACTACAGTTATCCCCAGGTACCTGGGAGCTACCCCATGCAGCCAGTCACTGCACCTCCATCCTAC
CCTCCTACCAGCTATTCCTCTACACAGCCGACTAGTTATGATCAGAGCAGTTACTCTCAGCAGAACACCTATGGG
CAACCGAGCAGCTATGGACAGCAGAGTAGCTATGGTCAACAAAGCAGCTATGGGCAGCAGCCTCCCACTAGTTAC
CCACCCCAAACTGGATCCTACAGCCAAGCTCCAAGTCAATATAGCCAACAGAGCAGCAGCTACGGGCAGCAGAGT
TCATTCCGACAGGACCACCCCAGTAGCATGGGTGTTTATGGGCAGGAGTCTGGAGGATTTTCCGGACCAGGAGAG
AACCGGAGCATGAGTGGCCCTGATAACCGGGGCAGGGGAAGAGGGGGATTTGATCGTGGAGGCATGAGCAGAGGT
GGGCGGGGAGGAGGACGCGGTGGAATGGGCAGCGCTGGAGAGCGAGGTGGCTTCAATAAGCCTGGTGGACCCATG
GATGAAGGACCAGATCTTGATCTAGGCCCACCTGTAGATCCAGATGAAGACTCTGACAACAGTGCAATTTATGTA
CAAGGATTAAATGACAGTGTGACTCTAGATGATCTGGCAGACTTCTTTAAGCAGTGTGGGGTTGTTAAGATGAAC
AAGAGAACTGGGCAACCCATGATCCACATCTACCTGGACAAGGAAACAGGAAAGCCCAAAGGCGATGCCACAGTG
TCCTATGAAGACCCACCTACTGCCAAGGCTGCCGTGGAATGGTTTGATGGGAAAGATTTTCAAGGGAGCAAACTT
AAAGTCTCCCTTGCTCGGAAGAAGCCTCCAATGAACAGTATGCGGGGTGGTCTGCCACCCCGTGAGGGCAGAGGC
ATGCCACCACCACTCCGTGGAGGTCCAGGAGGCCCAGGAGGTCCTGGGGGACCCATGGGTCGCATGGGAGGCCGT
GGAGGAGATAGAGGAGGCTTCCCTCCAAGAGGACCCCGGGGTTCCCGAGGGAACCCCTCTGGAGGAGGAAACGTC
CAGCACCGAGCTGGAGACTGGCAGTGTCCCAATCCGGGTTGTGGAAACCAGAACTTCGCCTGGAGAACAGAGTGC
AACCAGTGTAAGGCCCCAAAGCCTGAAGGCTTCCTCCCGCCACCCTTTCCGCCCCCGGGTGGTGATCGTGGCAGA
GGTGGCCCTGGTGGCATGCGGGGAGGAAGAGGTGGCCTCATGGATCGTGGTGGTCCCGGTGGAATGTTCAGAGGT
GGCCGTGGTGGAGACAGAGGTGGCTTCCGTGGTGGCCGGGGCATGGACCGAGGTGGCTTTGGTGGAGGAAGACGA
GGTGGCCCTGGGGGGCCCCCTGGACCTTTGATGGAACAGGATTACAAGGATGACGACGATAAGGGTACCGAGCAG
AAGCTGATCTCAGAGGAGGACCTGGGCGCCCCCGGCTCCGCCGGCTCCGCCGCCGGCTCCGGCGCTTCTAACTTT
ACTCAGTTCGTTCTCGTCGACAATGGCGGAACTGGCGACGTGACTGTCGCCCCAAGCAACTTCGCTAACGGGATC
GCTGAATGGATCAGCTCTAACTCGCGTTCACAGGCTTACAAAGTAACCTGTAGCGTTCGTCAGAGCTCTGCGCAG
AATCGCAAATACACCATCAAAGTCGAGGTGCCTAAAGGCGCCTGGCGTTCGTACTTAAATATGGAACTAACCATT
CCAATTTTCGCCACGAATTCCGACTGCGAGCTTATTGTTAAGGCAATGCAAGGTCTCCTAAAAGATGGAAACCCG
ATTCCCTCAGCAATCGCAGCAAACTCCGGCATCTACTAA (SEQ ID NO:83)
Protein:
MSDTKVKVAVRVRPMNRRELELNTKCVVEMEGNQTVLHPPPSNTKQGERKPPKVFAFDYCFWSMDESNTTKYAGQ
EVVFKCLGEGILEKAFQGYNACIFAYGQTGSGKSFSMMGHAEQLGLIPRLCCALFKRISLEQNESQTFKVEVSYM
EIYNEKVRDLLDPKGSRQSLKVREHKVLGPYVDGLSQLAVTSFEDIESLMSEGNKSRTVAATNMNEESSRSHAVF

NIIITQTLYDLQSGNSGEKVSKVSLVDLAGSERVSKTGAAGERLKEGSNINKSLTTLGLVISSLADQAAGKGKSK
FVPYRDSVLTWLLKDNLGGNSQTSMIATISPAADNYEETLSTLRYADRAKRIVNHAVVNEDPNAKVIRELREEVE
KLREQLSQAEAMKAELKEKLEESEKLIKELTVTWEMASTDYSTYSQAAAQQGYSAYTAQPTQGYAQTTQAYGQQS
YGTYGQPTDVSYTQAQTTATYGQTAYATSYGQPPTGYTTPTAPQAYSQPVQGYGTGAYDTTTATVTTTQASYAAQ
SAYGTQPAYPAYGQQPAATAPTRPQDGNKPTETSQPQSSTGGYNQPSLGYGQSNYSYPQVPGSYPMQPVTAPPSY
PPTSYSSTQPTSYDQSSYSQQNTYGQPSSYGQQSSYGQQSSYGQQPPTSYPPQTGSYSQAPSQYSQQSSSYGQQS
SFRQDHPSSMGVYGQESGGFSGPGENRSMSGPDNRGRGRGGFDRGGMSRGGRGGGRGGMGSAGERGGFNKPGGPM
DEGPDLDLGPPVDPDEDSDNSAIYVQGLNDSVTLDDLADFFKQCGVVKMNKRTGQPMIHIYLDKETGKPKGDATV
SYEDPPTAKAAVEWFDGKDFQGSKLKVSLARKKPPMNSMRGGLPPREGRGMPPPLRGGPGGPGGPGGPMGRMGGR
GGDRGGFPPRGPRGSRGNPSGGGNVQHRAGDWQCPNPGCGNQNFAWRTECNQCKAPKPEGFLPPPFPPPGGDRGR
GGPGGMRGGRGGLMDRGGPGGMFRGGRGGDRGGFRGGRGMDRGGFGGGRRGGPGGPPGPLMEQDYKDDDDKGTEQ
KLISEEDLGAPGSAGSAAGSGASNFTQFVLVDNGGTGDVTVAPSNFANGIAEWISSNSRSQAYKVTCSVRQSSAQ
NRKYTIKVEVPKGAWRSYLNMELTIPIFATNSDCELIVKAMQGLLKDGNPIPSAIAANSGIY (SEQ ID
NO:84)

KIF13A-SPD5-PylRS^AF
DNA:
ATGTCGGATACCAAGGTAAAAGTTGCCGTCCGGGTCCGGCCCATGAACCGACGAGAACTGGAACTGAACACCAAG
TGCGTGGTGGAGATGGAAGGGAATCAAACGGTCCTGCACCCTCCTCCTTCTAACACCAAACAGGGAGAAAGGAAA
CCTCCCAAGGTATTTGCCTTTGATTATTGCTTTTGGTCCATGGATGAATCTAACACTACAAAATACGCTGGTCAA
GAAGTGGTTTTCAAGTGCCTTGGGGAAGGAATTCTTGAAAAAGCCTTTCAGGGGTATAATGCGTGTATTTTTGCA
TATGGACAGACAGGTTCGGGAAAATCCTTTTCCATGATGGGCCATGCTGAGCAGCTGGGCCTTATTCCAAGGCTC
TGCTGTGCTTTATTTAAAAGGATCTCTTTGGAGCAAAATGAGTCACAGACCTTTAAAGTTGAAGTGTCCTATATG
GAAATTTATAATGAGAAAGTTCGGGATCTTTTAGACCCCAAAGGGAGTAGACAGTCTCTTAAAGTTCGAGAACAT
AAAGTTTTGGGACCATATGTAGATGGTTTATCTCAACTAGCTGTCACTAGTTTTGAGGATATTGAGTCATTGATG
TCTGAGGGAAATAAGTCTCGAACGGTAGCTGCTACCAACATGAACGAAGAAAGCAGCCGCTCCCATGCTGTGTTC
AACATCATAATCACACAGACACTTTATGACCTGCAGTCTGGGAATTCCGGGGAGAAAGTCAGTAAGGTCAGCTTG
GTAGACCTGGCGGGTAGCGAAAGAGTATCTAAAACAGGAGCTGCAGGAGAGCGACTGAAAGAAGGCAGCAACATT
AACAAATCGCTTACAACCTTGGGGTTGGTTATATCATCACTGGCTGACCAGGCAGCTGGCAAGGGTAAAAGCAAA
TTTGTGCCTTATCGAGATTCAGTCCTCACTTGGCTGCTTAAGGACAACTTGGGGGGCAACAGCCAAACCTCTATG
ATAGCCACAATCAGCCCAGCCGCAGACAACTATGAAGAGACCCTCTCCACATTAAGATATGCAGACCGAGCCAAA
AGGATTGTGAACCATGCTGTTGTGAATGAGGACCCCAACGCAAAAGTGATCCGAGAACTGCGGGAGGAAGTCGAG
AAACTGAGAGAGCAGCTCTCTCAGGCAGAGGCCATGAAGGCCGAACTGAAGGAGAAGCTCGAAGAGTCTGAAAAG
CTGATAAAAGAACTAACAGTGACTTGGGAAATGGAGGACAACAGCGTGCTGAACGAGGACAGCAACCTGGAGCAC
GTGGAGGGCCAGCCCAGAAGAAGCATGAGCCAGCCCGTGCTGAACGTGGAGGGCGACAAGAGAACCAGCAGCACC
AGCGCCACCCAGCAGCAGGTGCTGAGCGGCGCCTTCAGCAGCGCCGACGTGAGAAGCATCCCCATCATCCAGACC
TGGGAGGAGAACAAGGCCCTGAAGACCAAGATCACCATCCTGAGAGGCGAGCTGCAGATGTACCAGAGAAGATAC
AGCGAGGCCAAGGAGGCCAGCCAGAAGAGAGTGAAGGAGGTGATGGACGACTACGTGGACCTGAAGCTGGGCCAG
GAGAACGTGCAGGAGAAGATGGAGCAGTACAAGCTGATGGAGGAGGACCTGCTGGCCATGCAGAGCAGAATCGAG
ACCAGCGAGGACAACTTCGCCAGACAGATGAAGGAGTTCGAGGCCCAGAAGCACGCCATGGAGGAGAGAATCAAG
GAGCTGGAGCTGAGCGCCACCGACGCCAACAACACCACCGTGGGCAGCTTCAGAGGCACCCTGGACGACATCCTG
AAGAAGAACGACCCCGACTTCACCCTGACCAGCGGCTACGAGGAGAGAAAGATCAACGACCTGGAGGCCAAGCTG
CTGAGCGAGATCGACAAGGTGGCCGAGCTGGAGGACCACATCCAGCAGCTGAGACAGGAGCTGGACGACCAGAGC
GCCAGACTGGCCGACAGCGAGAACGTGAGAGCCCAGCTGGAGGCCGCCACCGGCCAGGGCATCCTGGGCGCCGCC
GGCAACGCCATGGTGCCCAACAGCACCTTCATGATCGGCAACGGCAGAGAGAGCCAGACCAGAGACCAGCTGAAC
TACATCGACGACCTGGAGACCAAGCTGGCCGACGCCAAGAAGGAGAACGACAAGGCCAGACAGGCCCTGGTGGAG
TACATGAACAAGTGCAGCAAGCTGGAGCACGAGATCAGAACCATGGTGAAGAACAGCACCTTCGACAGCAGCAGC
ATGCTGCTGGGCGGCCAGACCAGCGACGAGCTGAAGATCCAGATCGGCAAGGTGAACGGCGAGCTGAACGTGCTG
AGAGCCGAGAACAGAGAGCTGAGAATCAGATGCGACCAGCTGACCGGCGGCGACGGCAACCTGAGCATCAGCCTG
GGCCAGAGCAGACTGATGGCCGGCATCGCCACCAACGACGTGGACAGCATCGGCCAGGGCAACGAGACCGGCGGC
ACCAGCATGAGAATCCTGCCCAGAGAGAGCCAGCTGGACGACCTGGAGGAGAGCAAGCTGCCCCTGATGGACACC
AGCAGCGCCGTGAGAAACCAGCAGCAGTTCGCCAGCATGTGGGAGGACTTCGAGAGCGTGAAGGACAGCCTGCAG
AACAACCACAACGACACCCTGGAGGGCAGCTTCAACAGCAGCATGCCCCCCCCCGGCAGAGACGCCACCCAGAGC
TTCCTGAGCCAGAAGAGCTTCAAGAACAGCCCCATCGTGATGCAGAAGCCCAAGAGCCTGCACCTGCACCTGAAG
AGCCACCAGAGCGAGGGCGCCGGCGAGCAGATCCAGAACAACAGCTTCAGCACCAAGACCGCCAGCCCCCACGTG
AGCCAGAGCCACATCCCCATCCTGCACGACATGCAGCAGATCCTGGACAGCAGCGCCATGTTCCTGGAGGGCCAG
CACGACGTGGCCGTGAACGTGGAGCAGATGCAGGAGAAGATGAGCCAGATCAGAGAGGCCCTGGCCAGACTGTTC
GAGAGACTGAAGAGCAGCGCCGCCCTGTTCGAGGAGATCCTGGAGAGAATGGGCAGCAGCGACCCCAACGCCGAC

AAGATCAAGAAGATGAAGCTGGCCTTCGAGACCAGCATCAACGACAAGCTGAACGTGAGCGCCATCCTGGAGGCC
GCCGAGAAGGACCTGCACAACATGAGCCTGAACTTCAGCATCCTGGAGAAGAGCATCGTGAGCCAGGCCGCCGAG
GCCAGCAGAAGATTCACCATCGCCCCCGACGCCGAGGACGTGGCCAGCAGCAGCCTGCTGAACGCCAGCTACAGC
CCCCTGTTCAAGTTCACCAGCAACAGCGACATCGTGGAGAAGCTGCAGAACGAGGTGAGCGAGCTGAAGAACGAG
CTGGAGATGGCCAGAACCAGAGACATGAGAAGCCCCCTGAACGGCAGCAGCGGCAGACTGAGCGACGTGCAGATC
AACACCAACAGAATGTTCGAGGACCTGGAGGTGAGCGAGGCCACCCTGCAGAAGGCCAAGGAGGAGAACAGCACC
CTGAAGAGCCAGTTCGCCGAGCTGGAGGCCAACCTGCACCAGGTGAACAGCAAGCTGGGCGAGGTGAGATGCGAG
CTGAACGAGGCCCTGGCCAGAGTGGACGGCGAGCAGGAGACCAGAGTGAAGGCCGAGAACGCCCTGGAGGAGGCC
AGACAGCTGATCAGCAGCCTGAAGCACGAGGAGAACGAGCTGAAGAAGACCATCACCGACATGGGCATGAGACTG
AACGAGGCCAAGAAGAGCGACGAGTTCCTGAAGAGCGAGCTGAGCACCGCCCTGGAGGAGGAGAAGAAGAGCCAG
AACCTGGCCGACGAGCTGAGCGAGGAGCTGAACGGCTGGAGAATGAGAACCAAGGAGGCCGAGAACAAGGTGGAG
CACGCCAGCAGCGAGAAGAGCGAGATGCTGGAGAGAATCGTGCACCTGGAGACCGAGATGGAGAAGCTGAGCACC
AGCGAGATCGCCGCCGACTACTGCAGCACCAAGATGACCGAGAGAAAGAAGGAGATCGAGCTGGCCAAGTACAGA
GAGGACTTCGAGAACGCCGCCATCGTGGGCCTGGAGAGAATCAGCAAGGAGATCAGCGAGCTGACCAAGAAGACC
CTGAAGGCCAAGATCATCCCCAGCAACATCAGCAGCATCCAGCTGGTGTGCGACGAGCTGTGCAGAAGACTGAGC
AGAGAGAGAGAGCAGCAGCACGAGTACGCCAAGGTGATGAGAGACGTGAACGAGAAGATCGAGAAGCTGCAGCTG
GAGAAGGACGCCCTGGAGCACGAGCTGAAGATGATGAGCAGCAACAACGAGAACGTGCCCCCCGTGGGCACCAGC
GTGAGCGGCATGCCCACCAAGACCAGCAACCAGAAGTGCGCCCAGCCCCACTACACCAGCCCCACCAGACAGCTG
CTGCACGAGAGCACCATGGCCGTGGACGCCATCGTGCAGAAGCTGAAGAAGACCCACAACATGAGCGGCATGGGC
CCCGAGCTGAAGGAGACCATCGGCAACGTGATCAACGAGAGCAGAGTGCTGAGAGACTTCCTGCACCAGAAGCTG
ATCCTGTTCAAGGGCATCGACATGAGCAACTGGAAGAACGAGACCGTGGACCAGCTGATCACCGACCTGGGCCAG
CTGCACCAGGACAACCTGATGCTGGAGGAGCAGATCAAGAAGTACAAGAAGGAGCTGAAGCTGACCAAGAGCGCC
ATCCCCACCCTGGGCGTGGAGTTCCAGGACAGAATCAAGACCGAGATCGGCAAGATCGCCACCGACATGGGCGGC
GCCGTGAAGGAGATCAGAAAGAAGGGTACCGGCGCCCCCGGCTCCGCCGGCTCCGCCGCCGGCTCCGGCGCGTGC
CCGGTGCCGCTGCAGCTGCCGCCGCTGGAACGCCTGACCCTGGATGATAAAAAACCGCTGAATACCCTGATCTCT
GCTACTGGTCTGTGGATGAGTCGTACCGGAACCATTCATAAAATCAAACACCACGAGGTTAGCCGTTCGAAAATC
TATATTGAGATGGCGTGTGGCGATCATCTGGTTGTGAACAATAGCCGCTCTTCTCGTACAGCACGTGCACTGCGT
CACCACAAATATCGTAAAACCTGTAAACGTTGCCGTGTGTCCGATGAGGATCTGAACAAATTCCTGACAAAAGCC
AATGAGGACCAAACAAGCGTGAAAGTGAAAGTCGTTAGCGCTCCTACCCGTACTAAAAAAGCAATGCCGAAATCC
GTTGCTCGTGCCCCTAAACCACTGGAAAACACTGAAGCAGCACAGGCACAGCCGTCTGGAAGCAAATTCTCTCCG
GCCATTCCTGTTTCTACCCAGGAGTCCGTTTCTGTTCCAGCAAGTGTGAGCACCAGCATTAGCAGTATTAGCACC
GGTGCCACCGCTAGCGCCCTGGTTAAAGGCAATACCAATCCGATTACAAGCATGTCTGCCCCGGTTCAAGCATCA
GCTCCAGCACTGACAAAATCCCAAACCGATCGTCTGGAGGTTCTGCTGAATCCGAAAGACGAAATCAGCCTGAAT
TCCGGCAAACCGTTTCGTGAACTGGAGAGCGAACTGCTGTCACGTCGTAAAAAAGACCTGCAACAAATCTATGCC
GAAGAACGTGAGAACTATCTGGGGAAACTGGAACGTGAAATCACCCGCTTTTTCGTGGATCGTGGCTTTCTGGAG
ATCAAATCCCCGATTCTGATTCCTCTGGAGTATATCGAGCGTATGGGCATCGACAATGATACCGAACTGAGCAAA
CAAATTTTCCGTGTGGATAAAAACTTCTGTCTGCGCCCTATGCTAGCACCAAATCTGGCTAACTATCTGCGCAAA
CTGGACCGTGCCCTGCCTGATCCTATCAAAATCTTCGAGATCGGCCCGTGTTATCGTAAAGAGTCCGACGGTAAA
GAACATCTGGAGGAGTTTACCATGCTGAACTTTTGCCAAATGGGTTCAGGTTGTACTCGTGAGAACCTGGAAAGC
ATCATCACCGATTTTCTGAACCACCTGGGCATTGACTTCAAAATTGTGGGCGACAGCTGTATGGTGTTTGGCGAC
ACCCTGGATGTCATGCACGGCGACCTGGAACTGTCTAGTGCCGTTGTGGGCCCAATCCCGCTGGATCGTGAGTGG
GGTATCGACAAACCTTGGATCGGTGCGGGTTTTGGTCTGGAGCGTCTGCTGAAAGTAAAACACGACTTCAAGAAC
ATCAAACGTGCTGCACGTTCCGAGTCCTATTACAATGGTATTTCTACTAACCTGTAA (SEQ ID NO:85)
Protein:
MSDTKVKVAVRVRPMNRRELELNTKCVVEMEGNQTVLHPPPSNTKQGERKPPKVFAFDYCFWSMDESNTTKYAGQ
EVVFKCLGEGILEKAFQGYNACIFAYGQTGSGKSFSMMGHAEQLGLIPRLCCALFKRISLEQNESQTFKVEVSYM
EIYNEKVRDLLDPKGSRQSLKVREHKVLGPYVDGLSQLAVTSFEDIESLMSEGNKSRTVAATNMNEESSRSHAVF
NIIITQTLYDLQSGNSGEKVSKVSLVDLAGSERVSKTGAAGERLKEGSNINKSLTTLGLVISSLADQAAGKGKSK
FVPYRDSVLTWLLKDNLGGNSQTSMIATISPAADNYEETLSTLRYADRAKRIVNHAVVNEDPNAKVIRELREEVE
KLREQLSQAEAMKAELKEKLEESEKLIKELTVTWEMEDNSVLNEDSNLEHVEGQPRRSMSQPVLNVEGDKRTSST
SATQQQVLSGAFSSADVRSIPIIQTWEENKALKTKITILRGELQMYQRRYSEAKEASQKRVKEVMDDYVDLKLGQ
ENVQEKMEQYKLMEEDLLAMQSRIETSEDNFARQMKEFEAQKHAMEERIKELELSATDANNTTVGSFRGTLDDIL
KKNDPDFTLTSGYEERKINDLEAKLLSEIDKVAELEDHIQQLRQELDDQSARLADSENVRAQLEAATGQGILGAA
GNAMVPNSTFMIGNGRESQTRDQLNYIDDLETKLADAKKENDKARQALVEYMNKCSKLEHEIRTMVKNSTFDSSS
MLLGGQTSDELKIQIGKVNGELNVLRAENRELRIRCDQLTGGDGNLSISLGQSRLMAGIATNDVDSIGQGNETGG
TSMRILPRESQLDDLEESKLPLMDTSSAVRNQQQFASMWEDFESVKDSLQNNHNDTLEGSFNSSMPPPGRDATQS
FLSQKSFKNSPIVMQKPKSLHLHLKSHQSEGAGEQIQNNSFSTKTASPHVSQSHIPILHDMQQILDSSAMFLEGQ
HDVAVNVEQMQEKMSQIREALARLFERLKSSAALFEEILERMGSSDPNADKIKKMKLAFETSINDKLNVSAILEA

AEKDLHNMSLNFSILEKSIVSQAAEASRRFTIAPDAEDVASSSLLNASYSPLFKFTSNSDIVEKLQNEVSELKNE
LEMARTRDMRSPLNGSSGRLSDVQINTNRMFEDLEVSEATLQKAKEENSTLKSQFAELEANLHQVNSKLGEVRCE
LNEALARVDGEQETRVKAENALEEARQLISSLKHEENELKKTITDMGMRLNEAKKSDEFLKSELSTALEEEKKSQ
NLADELSEELNGWRMRTKEAENKVEHASSEKSEMLERIVHLETEMEKLSTSEIAADYCSTKMTERKKEIELAKYR
EDFENAAIVGLERISKEISELTKKTLKAKIIPSNISSIQLVCDELCRRLSREREQQHEYAKVMRDVNEKIEKLQL
EKDALEHELKMMSSNNENVPPVGTSVSGMPTKTSNQKCAQPHYTSPTRQLLHESTMAVDAIVQKLKKTHNMSGMG
PELKETIGNVINESRVLRDFLHQKLILFKGIDMSNWKNETVDQLITDLGQLHQDNLMLEEQIKKYKKELKLTKSA
IPTLGVEFQDRIKTEIGKIATDMGGAVKEIRKKGTGAPGSAGSAAGSGACPVPLQLPPLERLTLDDKKPLNTLIS
ATGLWMSRTGTIHKIKHHEVSRSKIYIEMACGDHLVVNNSRSSRTARALRHHKYRKTCKRCRVSDEDLNKFLTKA
NEDQTSVKVKVVSAPTRTKKAMPKSVARAPKPLENTEAAQAQPSGSKFSPAIPVSTQESVSVPASVSTSISSIST
GATASALVKGNTNPITSMSAPVQASAPALTKSQTDRLEVLLNPKDEISLNSGKPFRELESELLSRRKKDLQQIYA
EERENYLGKLEREITRFFVDRGFLEIKSPILIPLEYIERMGIDNDTELSKQIFRVDKNFCLRPMLAPNLANYLRK
LDRALPDPIKIFEIGPCYRKESDGKEHLEEFTMLNFCQMGSGCTRENLESIITDFLNHLGIDFKIVGDSCMVFGD
TLDVMHGDLELSSAVVGPIPLDREWGIDKPWIGAGFGLERLLKVKHDFKNIKRAARSESYYNGISTNL    (SEQ
ID NO:86)

KIF13A-SPD5-MCP
DNA:
ATGTCGGATACCAAGGTAAAAGTTGCCGTCCGGGTCCGGCCCATGAACCGACGAGAACTGGAACTGAACACCAAG
TGCGTGGTGGAGATGGAAGGGAATCAAACGGTCCTGCACCCTCCTCCTTCTAACACCAAACAGGGAGAAAGGAAA
CCTCCCAAGGTATTTGCCTTTGATTATTGCTTTTGGTCCATGGATGAATCTAACACTACAAAATACGCTGGTCAA
GAAGTGGTTTTCAAGTGCCTTGGGGAAGGAATTCTTGAAAAAGCCTTTCAGGGGTATAATGCGTGTATTTTTGCA
TATGGACAGACAGGTTCGGGAAAATCCTTTTCCATGATGGGCCATGCTGAGCAGCTGGGCCTTATTCCAAGGCTC
TGCTGTGCTTTATTTAAAAGGATCTCTTTGGAGCAAAATGAGTCACAGACCTTTAAAGTTGAAGTGTCCTATATG
GAAATTTATAATGAGAAAGTTCGGGATCTTTTAGACCCCAAAGGGAGTAGACAGTCTCTTAAAGTTCGAGAACAT
AAAGTTTTGGGACCATATGTAGATGGTTTATCTCAACTAGCTGTCACTAGTTTTGAGGATATTGAGTCATTGATG
TCTGAGGGAAATAAGTCTCGAACGGTAGCTGCTACCAACATGAACGAAGAAAGCAGCCGCTCCCATGCTGTGTTC
AACATCATAATCACACAGACACTTTATGACCTGCAGTCTGGGAATTCCGGGGAGAAAGTCAGTAAGGTCAGCTTG
GTAGACCTGGCGGGTAGCGAAAGAGTATCTAAAACAGGAGCTGCAGGAGAGCGACTGAAAGAAGGCAGCAACATT
AACAAATCGCTTACAACCTTGGGGTTGGTTATATCATCACTGGCTGACCAGGCAGCTGGCAAGGGTAAAAGCAAA
TTTGTGCCTTATCGAGATTCAGTCCTCACTTGGCTGCTTAAGGACAACTTGGGGGGCAACAGCCAAACCTCTATG
ATAGCCACAATCAGCCCAGCCGCAGACAACTATGAAGAGACCCTCTCCACATTAAGATATGCAGACCGAGCCAAA
AGGATTGTGAACCATGCTGTTGTGAATGAGGACCCCAACGCAAAAGTGATCCGAGAACTGCGGGAGGAAGTCGAG
AAACTGAGAGAGCAGCTCTCTCAGGCAGAGGCCATGAAGGCCGAACTGAAGGAGAAGCTCGAAGAGTCTGAAAAG
CTGATAAAAGAACTAACAGTGACTTGGGAAATGGAGGACAACAGCGTGCTGAACGAGGACAGCAACCTGGAGCAC
GTGGAGGGCCAGCCCAGAAGAAGCATGAGCCAGCCCGTGCTGAACGTGGAGGGCGACAAGAGAACCAGCAGCACC
AGCGCCACCCAGCAGCAGGTGCTGAGCGGCGCCTTCAGCAGCGCCGACGTGAGAAGCATCCCCATCATCCAGACC
TGGGAGGAGAACAAGGCCCTGAAGACCAAGATCACCATCCTGAGAGGCGAGCTGCAGATGTACCAGAGAAGATAC
AGCGAGGCCAAGGAGGCCAGCCAGAAGAGAGTGAAGGAGGTGATGGACGACTACGTGGACCTGAAGCTGGGCCAG
GAGAACGTGCAGGAGAAGATGGAGCAGTACAAGCTGATGGAGGAGGACCTGCTGGCCATGCAGAGCAGAATCGAG
ACCAGCGAGGACAACTTCGCCAGACAGATGAAGGAGTTCGAGGCCCAGAAGCACGCCATGGAGGAGAGAATCAAG
GAGCTGGAGCTGAGCGCCACCGACGCCAACAACACCACCGTGGGCAGCTTCAGAGGCACCCTGGACGACATCCTG
AAGAAGAACGACCCCGACTTCACCCTGACCAGCGGCTACGAGGAGAGAAAGATCAACGACCTGGAGGCCAAGCTG
CTGAGCGAGATCGACAAGGTGGCCGAGCTGGAGGACCACATCCAGCAGCTGAGACAGGAGCTGGACGACCAGAGC
GCCAGACTGGCCGACAGCGAGAACGTGAGAGCCCAGCTGGAGGCCGCCACCGGCCAGGGCATCCTGGGCGCCGCC
GGCAACGCCATGGTGCCCAACAGCACCTTCATGATCGGCAACGGCAGAGAGAGCCAGACCAGAGACCAGCTGAAC
TACATCGACGACCTGGAGACCAAGCTGGCCGACGCCAAGAAGGAGAACGACAAGGCCAGACAGGCCCTGGTGGAG
TACATGAACAAGTGCAGCAAGCTGGAGCACGAGATCAGAACCATGGTGAAGAACAGCACCTTCGACAGCAGCAGC
ATGCTGCTGGGCGGCCAGACCAGCGACGAGCTGAAGATCCAGATCGGCAAGGTGAACGGCGAGCTGAACGTGCTG
AGAGCCGAGAACAGAGAGCTGAGAATCAGATGCGACCAGCTGACCGGCGGCGACGGCAACCTGAGCATCAGCCTG
GGCCAGAGCAGACTGATGGCCGGCATCGCCACCAACGACGTGGACAGCATCGGCCAGGGCAACGAGACCGGCGGC
ACCAGCATGAGAATCCTGCCCAGAGAGAGCCAGCTGGACGACCTGGAGGAGAGCAAGCTGCCCCTGATGGACACC
AGCAGCGCCGTGAGAAACCAGCAGCAGTTCGCCAGCATGTGGGAGGACTTCGAGAGCGTGAAGGACAGCCTGCAG
AACAACCACAACGACACCCTGGAGGGCAGCTTCAACAGCAGCATGCCCCCCCCCGGCAGAGACGCCACCCAGAGC
TTCCTGAGCCAGAAGAGCTTCAAGAACAGCCCCATCGTGATGCAGAAGCCCAAGAGCCTGCACCTGCACCTGAAG
AGCCACCAGAGCGAGGGCGCCGGCGAGCAGATCCAGAACAACAGCTTCAGCACCAAGACCGCCAGCCCCCACGTG
AGCCAGAGCCACATCCCCATCCTGCACGACATGCAGCAGATCCTGGACAGCAGCGCCATGTTCCTGGAGGGCCAG
CACGACGTGGCCGTGAACGTGGAGCAGATGCAGGAGAAGATGAGCCAGATCAGAGAGGCCCTGGCCAGACTGTTC

GAGAGACTGAAGAGCAGCGCCGCCCTGTTCGAGGAGATCCTGGAGAGAATGGGCAGCAGCGACCCCAACGCCGAC
AAGATCAAGAAGATGAAGCTGGCCTTCGAGACCAGCATCAACGACAAGCTGAACGTGAGCGCCATCCTGGAGGCC
GCCGAGAAGGACCTGCACAACATGAGCCTGAACTTCAGCATCCTGGAGAAGAGCATCGTGAGCCAGGCCGCCGAG
GCCAGCAGAAGATTCACCATCGCCCCCGACGCCGAGGACGTGGCCAGCAGCAGCCTGCTGAACGCCAGCTACAGC
CCCCTGTTCAAGTTCACCAGCAACAGCGACATCGTGGAGAAGCTGCAGAACGAGGTGAGCGAGCTGAAGAACGAG
CTGGAGATGGCCAGAACCAGAGACATGAGAAGCCCCCTGAACGGCAGCAGCGGCAGACTGAGCGACGTGCAGATC
AACACCAACAGAATGTTCGAGGACCTGGAGGTGAGCGAGGCCACCCTGCAGAAGGCCAAGGAGGAGAACAGCACC
CTGAAGAGCCAGTTCGCCGAGCTGGAGGCCAACCTGCACCAGGTGAACAGCAAGCTGGGCGAGGTGAGATGCGAG
CTGAACGAGGCCCTGGCCAGAGTGGACGGCGAGCAGGAGACCAGAGTGAAGGCCGAGAACGCCCTGGAGGAGGCC
AGACAGCTGATCAGCAGCCTGAAGCACGAGGAGAACGAGCTGAAGAAGACCATCACCGACATGGGCATGAGACTG
AACGAGGCCAAGAAGAGCGACGAGTTCCTGAAGAGCGAGCTGAGCACCGCCCTGGAGGAGGAGAAGAAGAGCCAG
AACCTGGCCGACGAGCTGAGCGAGGAGCTGAACGGCTGGAGAATGAGAACCAAGGAGGCCGAGAACAAGGTGGAG
CACGCCAGCAGCGAGAAGAGCGAGATGCTGGAGAGAATCGTGCACCTGGAGACCGAGATGGAGAAGCTGAGCACC
AGCGAGATCGCCGCCGACTACTGCAGCACCAAGATGACCGAGAGAAAGAAGGAGATCGAGCTGGCCAAGTACAGA
GAGGACTTCGAGAACGCCGCCATCGTGGGCCTGGAGAGAATCAGCAAGGAGATCAGCGAGCTGACCAAGAAGACC
CTGAAGGCCAAGATCATCCCCAGCAACATCAGCAGCATCCAGCTGGTGTGCGACGAGCTGTGCAGAAGACTGAGC
AGAGAGAGAGAGCAGCAGCACGAGTACGCCAAGGTGATGAGAGACGTGAACGAGAAGATCGAGAAGCTGCAGCTG
GAGAAGGACGCCCTGGAGCACGAGCTGAAGATGATGAGCAGCAACAACGAGAACGTGCCCCCCGTGGGCACCAGC
GTGAGCGGCATGCCCACCAAGACCAGCAACCAGAAGTGCGCCCAGCCCCACTACACCAGCCCCACCAGACAGCTG
CTGCACGAGAGCACCATGGCCGTGGACGCCATCGTGCAGAAGCTGAAGAAGACCCACAACATGAGCGGCATGGGC
CCCGAGCTGAAGGAGACCATCGGCAACGTGATCAACGAGAGCAGAGTGCTGAGAGACTTCCTGCACCAGAAGCTG
ATCCTGTTCAAGGGCATCGACATGAGCAACTGGAAGAACGAGACCGTGGACCAGCTGATCACCGACCTGGGCCAG
CTGCACCAGGACAACCTGATGCTGGAGGAGCAGATCAAGAAGTACAAGAAGGAGCTGAAGCTGACCAAGAGCGCC
ATCCCCACCCTGGGCGTGGAGTTCCAGGACAGAATCAAGACCGAGATCGGCAAGATCGCCACCGACATGGGCGGC
GCCGTGAAGGAGATCAGAAAGAAGGGTACCGAGCAGAAGCTGATCTCAGAGGAGGACCTGGGCGCCCCCGGCTCC
GCCGGCTCCGCCGCCGGCTCCGGCGCTTCTAACTTTACTCAGTTCGTTCTCGTCGACAATGGCGGAACTGGCGAC
GTGACTGTCGCCCCAAGCAACTTCGCTAACGGGATCGCTGAATGGATCAGCTCTAACTCGCGTTCACAGGCTTAC
AAAGTAACCTGTAGCGTTCGTCAGAGCTCTGCGCAGAATCGCAAATACACCATCAAAGTCGAGGTGCCTAAAGGC
GCCTGGCGTTCGTACTTAAATATGGAACTAACCATTCCAATTTTCGCCACGAATTCCGACTGCGAGCTTATTGTT
AAGGCAATGCAAGGTCTCCTAAAAGATGGAAACCCGATTCCCTCAGCAATCGCAGCAAACTCCGGCATCTACTAA
(SEQ ID NO:87)
Protein:
MSDTKVKVAVRVRPMNRRELELNTKCVVEMEGNQTVLHPPPSNTKQGERKPPKVFAFDYCFWSMDESNTTKYAGQ
EVVFKCLGEGILEKAFQGYNACIFAYGQTGSGKSFSMMGHAEQLGLIPRLCCALFKRISLEQNESQTFKVEVSYM
EIYNEKVRDLLDPKGSRQSLKVREHKVLGPYVDGLSQLAVTSFEDIESLMSEGNKSRTVAATNMNEESSRSHAVF
NIIITQTLYDLQSGNSGEKVSKVSLVDLAGSERVSKTGAAGERLKEGSNINKSLTTLGLVISSLADQAAGKGKSK
FVPYRDSVLTWLLKDNLGGNSQTSMIATISPAADNYEETLSTLRYADRAKRIVNHAVVNEDPNAKVIRELREEVE
KLREQLSQAEAMKAELKEKLEESEKLIKELTVTWEMEDNSVLNEDSNLEHVEGQPRRSMSQPVLNVEGDKRTSST
SATQQQVLSGAFSSADVRSIPIIQTWEENKALKTKITILRGELQMYQRRYSEAKEASQKRVKEVMDDYVDLKLGQ
ENVQEKMEQYKLMEEDLLAMQSRIETSEDNFARQMKEFEAQKHAMEERIKELELSATDANNTTVGSFRGTLDDIL
KKNDPDFTLTSGYEERKINDLEAKLLSEIDKVAELEDHIQQLRQELDDQSARLADSENVRAQLEAATGQGILGAA
GNAMVPNSTFMIGNGRESQTRDQLNYIDDLETKLADAKKENDKARQALVEYMNKCSKLEHEIRTMVKNSTFDSSS
MLLGGQTSDELKIQIGKVNGELNVLRAENRELRIRCDQLTGGDGNLSISLGQSRLMAGIATNDVDSIGQGNETGG
TSMRILPRESQLDDLEESKLPLMDTSSAVRNQQQFASMWEDFESVKDSLQNNHNDTLEGSFNSSMPPPGRDATQS
FLSQKSFKNSPIVMQKPKSLHLHLKSHQSEGAGEQIQNNSFSTKTASPHVSQSHIPILHDMQQILDSSAMFLEGQ
HDVAVNVEQMQEKMSQIREALARLFERLKSSAALFEEILERMGSSDPNADKIKKMKLAFETSINDKLNVSAILEA
AEKDLHNMSLNFSILEKSIVSQAAEASRRFTIAPDAEDVASSSLLNASYSPLFKFTSNSDIVEKLQNEVSELKNE
LEMARTRDMRSPLNGSSGRLSDVQINTNRMFEDLEVSEATLQKAKEENSTLKSQFAELEANLHQVNSKLGEVRCE
LNEALARVDGEQETRVKAENALEEARQLISSLKHEENELKKTITDMGMRLNEAKKSDEFLKSELSTALEEEKKSQ
NLADELSEELNGWRMRTKEAENKVEHASSEKSEMLERIVHLETEMEKLSTSEIAADYCSTKMTERKKEIELAKYR
EDFENAAIVGLERISKEISELTKKTLKAKIIPSNISSIQLVCDELCRRLSREREQQHEYAKVMRDVNEKIEKLQL
EKDALEHELKMMSSNNENVPPVGTSVSGMPTKTSNQKCAQPHYTSPTRQLLHESTMAVDAIVQKLKKTHNMSGMG
PELKETIGNVINESRVLRDFLHQKLILFKGIDMSNWKNETVDQLITDLGQLHQDNLMLEEQIKKYKKELKLTKSA
IPTLGVEFQDRIKTEIGKIATDMGGAVKEIRKKGTEQKLISEEDLGAPGSAGSAAGSGASNFTQFVLVDNGGTGD
VTVAPSNFANGIAEWISSNSRSQAYKVTCSVRQSSAQNRKYTIKVEVPKGAWRSYLNMELTIPIFATNSDCELIV
KAMQGLLKDGNPIPSAIAANSGIY (SEQ ID NO:88)

KIF13A-FUS-MCP-PylRS^AF

DNA:
ATGTCGGATACCAAGGTAAAAGTTGCCGTCCGGGTCCGGCCCATGAACCGACGAGAACTGGAACTGAACACCAAG
TGCGTGGTGGAGATGGAAGGGAATCAAACGGTCCTGCACCCTCCTCCTTCTAACACCAAACAGGGAGAAAGGAAA
CCTCCCAAGGTATTTGCCTTTGATTATTGCTTTTGGTCCATGGATGAATCTAACACTACAAAATACGCTGGTCAA
GAAGTGGTTTTCAAGTGCCTTGGGGAAGGAATTCTTGAAAAAGCCTTTCAGGGGTATAATGCGTGTATTTTTGCA
TATGGACAGACAGGTTCGGGAAAATCCTTTTCCATGATGGGCCATGCTGAGCAGCTGGGCCTTATTCCAAGGCTC
TGCTGTGCTTTATTTAAAAGGATCTCTTTGGAGCAAAATGAGTCACAGACCTTTAAAGTTGAAGTGTCCTATATG
GAAATTTATAATGAGAAAGTTCGGGATCTTTTAGACCCCAAAGGGAGTAGACAGTCTCTTAAAGTTCGAGAACAT
AAAGTTTTGGGACCATATGTAGATGGTTTATCTCAACTAGCTGTCACTAGTTTTGAGGATATTGAGTCATTGATG
TCTGAGGGAAATAAGTCTCGAACGGTAGCTGCTACCAACATGAACGAAGAAAGCAGCCGCTCCCATGCTGTGTTC
AACATCATAATCACACAGACACTTTATGACCTGCAGTCTGGGAATTCCGGGGAGAAAGTCAGTAAGGTCAGCTTG
GTAGACCTGGCGGGTAGCGAAAGAGTATCTAAAACAGGAGCTGCAGGAGAGCGACTGAAAGAAGGCAGCAACATT
AACAAATCGCTTACAACCTTGGGGTTGGTTATATCATCACTGGCTGACCAGGCAGCTGGCAAGGGTAAAAGCAAA
TTTGTGCCTTATCGAGATTCAGTCCTCACTTGGCTGCTTAAGGACAACTTGGGGGGCAACAGCCAAACCTCTATG
ATAGCCACAATCAGCCCAGCCGCAGACAACTATGAAGAGACCCTCTCCACATTAAGATATGCAGACCGAGCCAAA
AGGATTGTGAACCATGCTGTTGTGAATGAGGACCCCAACGCAAAAGTGATCCGAGAACTGCGGGAGGAAGTCGAG
AAACTGAGAGAGCAGCTCTCTCAGGCAGAGGCCATGAAGGCCGAACTGAAGGAGAAGCTCGAAGAGTCTGAAAAG
CTGATAAAAGAACTAACAGTGACTTGGGAATATACAGATATTGAAATGAACAGATTGGGAAAGGGCGCCCCCGGC
TCCGCCGGCTCCGCCGCCGGCTCCGGCATGGCCTCAAACGATTATACCCAACAAGCAACCCAAAGCTATGGGGCC
TACCCCACCCAGCCCGGGCAGGGCTATTCCCAGCAGAGCAGTCAGCCCTACGGACAGCAGAGTTACAGTGGTTAT
AGCCAGTCCACGGACACTTCAGGATATGGCCAGAGCAGCTATTCTTCTTATGGCCAGAGCCAGAACACAGGCTAT
GGAACTCAGTCAACTCCCCAGGGATATGGCTCGACTGGCGGCTATGGCAGTAGCCAGAGCTCCCAATCGTCTTAC
GGGCAGCAGTCCTCCTACCCTGGCTATGGCCAGCAGCCAGCTCCCAGCAGCACCTCGGGAAGTTACGGTAGCAGT
TCTCAGAGCAGCAGCTATGGGCAGCCCCAGAGTGGGAGCTACAGCCAGCAGCCTAGCTATGGTGGACAGCAGCAA
AGCTATGGACAGCAGCAAAGCTATAATCCCCCTCAGGGCTATGGACAGCAGAACCAGTACAACAGCAGCAGTGGT
GGTGGAGGTGGAGGTGGAGGTGGAGGTAACTATGGCCAAGATCAATCCTCCATGAGTAGTGGTGGTGGCAGTGGT
GGCGGTTATGGCAATCAAGACCAGAGTGGTGGAGGTGGCAGCGGTGGCTATGGACAGCAGGACCGTGGAGGCCGC
GGCAGGGGTGGCAGTGGTGGCGGCGGCGGCGGCGGTGGTGGTTACAACCGCAGCAGTGGTGGCTATGAACCC
AGAGGTCGTGGAGGTGGCCGTGGAGGCAGAGGTGGCATGGGCGGAAGTGACCGTGGTGGCTTCAATAAATTTGGT
GGCCCTCGGGACCAAGGATCACGTCATGACTCCGAACAGGATAATTCAGACAACAACACCATCTTTGTGCAAGGC
CTGGGTGAGAATGTTACAATTGAGTCTGTGGCTGATTACTTCAAGCAGATTGGTATTATTAAGACAAACAAGAAA
ACGGGACAGCCCATGATTAATTTGTACACAGACAGGGAAACTGGCAAGCTGAAGGGAGAGGCAACGGTCTCTTTT
GATGACCCACCTTCAGCTAAAGCAGCTATTGACTGGTTTGATGGTAAAGAATTCTCCGGAAATCCTATCAAGGTC
TCATTTGCTACTCGCCGGGCAGACTTTAATCGGGGTGGTGGCAATGGTCGTGGAGGCCGAGGGCGAGGAGGACCC
ATGGGCCGTGGAGGCTATGGAGGTGGTGGCAGTGGTGGTGGTGGCCGAGGAGGATTTCCCAGTGGAGGTGGTGGC
GGTGGAGGACAGCAGCGAGCTGGTGACTGGAAGTGTCCTAATCCCACCTGTGAGAATATGAACTTCTCTTGGAGG
AATGAATGCAACCAGTGTAAGGCCCCTAAACCAGATGGCCCAGGAGGGGGACCAGGTGGCTCTCACATGGGGGGT
AACTACGGGGATGATCGTCGTGGTGGCAGAGGAGGCGATTACAAGGATGACGACGATAAGGGTACCGGCGCCCCC
GGCTCCGCCGGCTCCGCCGCCGGCTCCGGCGCTTCTAACTTTACTCAGTTCGTTCTCGTCGACAATGGCGGAACT
GGCGACGTGACTGTCGCCCCAAGCAACTTCGCTAACGGGATCGCTGAATGGATCAGCTCTAACTCGCGTTCACAG
GCTTACAAAGTAACCTGTAGCGTTCGTCAGAGCTCTGCGCAGAATCGCAAATACACCATCAAAGTCGAGGTGCCT
AAAGGCGCCTGGCGTTCGTACTTAAATATGGAACTAACCATTCCAATTTTCGCCACGAATTCCGACTGCGAGCTT
ATTGTTAAGGCAATGCAAGGTCTCCTAAAAGATGGAAACCCGATTCCCTCAGCAATCGCAGCAAACTCCGGCATC
TACGGTACCGGCGCCCCCGGCTCCGCCGGCTCCGCCGCCGGCTCCGGCGCGTGCCCGGTGCCGCTGCAGCTGCCG
CCGCTGGAACGCCTGACCCTGGATGATAAAAAACCGCTGAATACCCTGATCTCTGCTACTGGTCTGTGGATGAGT
CGTACCGGAACCATTCATAAAATCAAACACCACGAGGTTAGCCGTTCGAAAATCTATATTGAGATGGCGTGTGGC
GATCATCTGGTTGTGAACAATAGCCGCTCTTCTCGTACAGCACGTGCACTGCGTCACCACAAATATCGTAAAACC
TGTAAACGTTGCCGTGTGTCCGATGAGGATCTGAACAAATTCCTGACAAAAGCCAATGAGGACCAAACAAGCGTG
AAAGTGAAAGTCGTTAGCGCTCCTACCCGTACTAAAAAAGCAATGCCGAAATCCGTTGCTCGTGCCCCTAAACCA
CTGGAAAACACTGAAGCAGCACAGGCACAGCCGTCTGGAAGCAAATTCTCTCCGGCCATTCCTGTTTCTACCCAG
GAGTCCGTTTCTGTTCCAGCAAGTGTGAGCACCAGCATTAGCAGTATTAGCACCGGTGCCACCGCTAGCGCCCTG
GTTAAAGGCAATACCAATCCGATTACAAGCATGTCTGCCCCGGTTCAAGCATCAGCTCCAGCACTGACAAAATCC
CAAACCGATCGTCTGGAGGTTCTGCTGAATCCGAAAGACGAAATCAGCCTGAATTCCGGCAAACCGTTTCGTGAA
CTGGAGAGCGAACTGCTGTCACGTCGTAAAAAAGACCTGCAACAAATCTATGCCGAAGAACGTGAGAACTATCTG
GGGAAACTGGAACGTGAAATCACCCGCTTTTTCGTGGATCGTGGCTTTCTGGAGATCAAATCCCCGATTCTGATT
CCTCTGGAGTATATCGAGCGTATGGGCATCGACAATGATACCGAACTGAGCAAACAAATTTTCCGTGTGGATAAA
AACTTCTGTCTGCGCCCTATGCTAGCACCAAATCTGGCTAACTATCTGCGCAAACTGGACCGTGCCCTGCCTGAT
CCTATCAAAATCTTCGAGATCGGCCCGTGTTATCGTAAAGAGTCCGACGGTAAAGAACATCTGGAGGAGTTTACC

ATGCTGAACTTTTGCCAAATGGGTTCAGGTTGTACTCGTGAGAACCTGGAAAGCATCATCACCGATTTTCTGAAC
CACCTGGGCATTGACTTCAAAATTGTGGGCGACAGCTGTATGGTGTTTGGCGACACCCTGGATGTCATGCACGGC
GACCTGGAACTGTCTAGTGCCGTTGTGGGCCCAATCCCGCTGGATCGTGAGTGGGGTATCGACAAACCTTGGATC
GGTGCGGGTTTTGGTCTGGAGCGTCTGCTGAAAGTAAAACACGACTTCAAGAACATCAAACGTGCTGCACGTTCC
GAGTCCTATTACAATGGTATTTCTACTAACCTGTAA (SEQ ID NO:89)
Protein:
MSDTKVKVAVRVRPMNRRELELNTKCVVEMEGNQTVLHPPPSNTKQGERKPPKVFAFDYCFWSMDESNTTKYAGQ
EVVFKCLGEGILEKAFQGYNACIFAYGQTGSGKSFSMMGHAEQLGLIPRLCCALFKRISLEQNESQTFKVEVSYM
EIYNEKVRDLLDPKGSRQSLKVREHKVLGPYVDGLSQLAVTSFEDIESLMSEGNKSRTVAATNMNEESSRSHAVF
NIIITQTLYDLQSGNSGEKVSKVSLVDLAGSERVSKTGAAGERLKEGSNINKSLTTLGLVISSLADQAAGKGKSK
FVPYRDSVLTWLLKDNLGGNSQTSMIATISPAADNYEETLSTLRYADRAKRIVNHAVVNEDPNAKVIRELREEVE
KLREQLSQAEAMKAELKEKLEESEKLIKELTVTWEYTDIEMNRLGKGAPGSAGSAAGSGMASNDYTQQATQSYGA
YPTQPGQGYSQQSSQPYGQQSYSGYSQSTDTSGYGQSSYSSYGQSQNTGYGTQSTPQGYGSTGGYGSSQSSQSSY
GQQSSYPGYGQQPAPSSTSGSYGSSSQSSSYGQPQSGSYSQQPSYGGQQQSYGQQQSYNPPQGYGQQNQYNSSSG
GGGGGGGGGNYGQDQSSMSSGGGSGGGYGNQDQSGGGGSGGYGQQDRGGRGRGGSGGGGGGGGGGYNRSSGGYEP
RGRGGGRGGRGGMGGSDRGGFNKFGGPRDQGSRHDSEQDNSDNNTIFVQGLGENVTIESVADYFKQIGIIKTNKK
TGQPMINLYTDRETGKLKGEATVSFDDPPSAKAAIDWFDGKEFSGNPIKVSFATRRADFNRGGGNGRGGRGRGGP
MGRGGYGGGGSGGGGRGGFPSGGGGGGGQQRAGDWKCPNPTCENMNFSWRNECNQCKAPKPDGPGGGPGGSHMGG
NYGDDRRGGRGGDYKDDDDKGTGAPGSAGSAAGSGASNFTQFVLVDNGGTGDVTVAPSNFANGIAEWISSNSRSQ
AYKVTCSVRQSSAQNRKYTIKVEVPKGAWRSYLNMELTIPIFATNSDCELIVKAMQGLLKDGNPIPSAIAANSGI
YGTGAPGSAGSAAGSGACPVPLQLPPLERLTLDDKKPLNTLISATGLWMSRTGTIHKIKHHEVSRSKIYIEMACG
DHLVVNNSRSSRTARALRHHKYRKTCKRCRVSDEDLNKFLTKANEDQTSVKVKVVSAPTRTKKAMPKSVARAPKP
LENTEAAQAQPSGSKFSPAIPVSTQESVSVPASVSTSISSISTGATASALVKGNTNPITSMSAPVQASAPALTKS
QTDRLEVLLNPKDEISLNSGKPFRELESELLSRRKKDLQQIYAEERENYLGKLEREITRFFVDRGFLEIKSPILI
PLEYIERMGIDNDTELSKQIFRVDKNFCLRPMLAPNLANYLRKLDRALPDPIKIFEIGPCYRKESDGKEHLEEFT
MLNFCQMGSGCTRENLESIITDFLNHLGIDFKIVGDSCMVFGDTLDVMHGDLELSSAVVGPIPLDREWGIDKPWI
GAGFGLERLLKVKHDFKNIKRAARSESYYNGISTNL (SEQ ID NO:90)

KIF13A-PylRS^AF
DNA:
ATGTCGGATACCAAGGTAAAAGTTGCCGTCCGGGTCCGGCCCATGAACCGACGAGAACTGGAACTGAACACCAAG
TGCGTGGTGGAGATGGAAGGGAATCAAACGGTCCTGCACCCTCCTCCTTCTAACACCAAACAGGGAGAAAGGAAA
CCTCCCAAGGTATTTGCCTTTGATTATTGCTTTTGGTCCATGGATGAATCTAACACTACAAAATACGCTGGTCAA
GAAGTGGTTTTCAAGTGCCTTGGGGAAGGAATTCTTGAAAAAGCCTTTCAGGGGTATAATGCGTGTATTTTTGCA
TATGGACAGACAGGTTCGGGAAAATCCTTTTCCATGATGGGCCATGCTGAGCAGCTGGGCCTTATTCCAAGGCTC
TGCTGTGCTTTATTTAAAAGGATCTCTTTGGAGCAAAATGAGTCACAGACCTTTAAAGTTGAAGTGTCCTATATG
GAAATTTATAATGAGAAAGTTCGGGATCTTTTAGACCCCAAAGGGAGTAGACAGTCTCTTAAAGTTCGAGAACAT
AAAGTTTTGGGACCATATGTAGATGGTTTATCTCAACTAGCTGTCACTAGTTTTGAGGATATTGAGTCATTGATG
TCTGAGGGAAATAAGTCTCGAACGGTAGCTGCTACCAACATGAACGAAGAAAGCAGCCGCTCCCATGCTGTGTTC
AACATCATAATCACACAGACACTTTATGACCTGCAGTCTGGGAATTCCGGGGAGAAAGTCAGTAAGGTCAGCTTG
GTAGACCTGGCGGGTAGCGAAAGAGTATCTAAAACAGGAGCTGCAGGAGAGCGACTGAAAGAAGGCAGCAACATT
AACAAATCGCTTACAACCTTGGGGTTGGTTATATCATCACTGGCTGACCAGGCAGCTGGCAAGGGTAAAAGCAAA
TTTGTGCCTTATCGAGATTCAGTCCTCACTTGGCTGCTTAAGGACAACTTGGGGGGCAACAGCCAAACCTCTATG
ATAGCCACAATCAGCCCAGCCGCAGACAACTATGAAGAGACCCTCTCCACATTAAGATATGCAGACCGAGCCAAA
AGGATTGTGAACCATGCTGTTGTGAATGAGGACCCCAACGCAAAAGTGATCCGAGAACTGCGGGAGGAAGTCGAG
AAACTGAGAGAGCAGCTCTCTCAGGCAGAGGCCATGAAGGCCGAACTGAAGGAGAAGCTCGAAGAGTCTGAAAAG
CTGATAAAAGAACTAACAGTGACTTGGGAATATACAGATATTGAAATGAACAGATTGGGAAAGGGCGCCCCCGGC
TCCGCCGGCTCCGCCGCCGGCTCCGGCATGGCGTGCCCGGTGCCGCTGCAGCTGCCGCCGCTGGAACGCCTGACC
CTGGATGATAAAAAACCGCTGAATACCCTGATCTCTGCTACTGGTCTGTGGATGAGTCGTACCGGAACCATTCAT
AAAATCAAACACCACGAGGTTAGCCGTTCGAAAATCTATATTGAGATGGCGTGTGGCGATCATCTGGTTGTGAAC
AATAGCCGCTCTTCTCGTACAGCACGTGCACTGCGTCACCACAAATATCGTAAAACCTGTAAACGTTGCCGTGTG
TCCGATGAGGATCTGAACAAATTCCTGACAAAAGCCAATGAGGACCAAACAAGCGTGAAAGTGAAAGTCGTTAGC
GCTCCTACCCGTACTAAAAAAGCAATGCCGAAATCCGTTGCTCGTGCCCCTAAACCACTGGAAAACACTGAAGCA
GCACAGGCACAGCCGTCTGGAAGCAAATTCTCTCCGGCCATTCCTGTTTCTACCCAGGAGTCCGTTTCTGTTCCA
GCAAGTGTGAGCACCAGCATTAGCAGTATTAGCACCGGTGCCACCGCTAGCGCCCTGGTTAAAGGCAATACCAAT
CCGATTACAAGCATGTCTGCCCCGGTTCAAGCATCAGCTCCAGCACTGACAAAATCCCAAACCGATCGTCTGGAG
GTTCTGCTGAATCCGAAAGACGAAATCAGCCTGAATTCCGGCAAACCGTTTCGTGAACTGGAGAGCGAACTGCTG
TCACGTCGTAAAAAAGACCTGCAACAAATCTATGCCGAAGAACGTGAGAACTATCTGGGGAAACTGGAACGTGAA

ATCACCCGCTTTTTCGTGGATCGTGGCTTTCTGGAGATCAAATCCCCGATTCTGATTCCTCTGGAGTATATCGAG
CGTATGGGCATCGACAATGATACCGAACTGAGCAAACAAATTTTCCGTGTGGATAAAAACTTCTGTCTGCGCCCT
ATGCTAGCACCAAATCTGGCTAACTATCTGCGCAAACTGGACCGTGCCCTGCCTGATCCTATCAAAATCTTCGAG
ATCGGCCCGTGTTATCGTAAAGAGTCCGACGGTAAAGAACATCTGGAGGAGTTTACCATGCTGAACTTTTGCCAA
ATGGGTTCAGGTTGTACTCGTGAGAACCTGGAAAGCATCATCACCGATTTTCTGAACCACCTGGGCATTGACTTC
AAAATTGTGGGCGACAGCTGTATGGTGTTTGGCGACACCCTGGATGTCATGCACGGCGACCTGGAACTGTCTAGT
GCCGTTGTGGGCCCAATCCCGCTGGATCGTGAGTGGGGTATCGACAAACCTTGGATCGGTGCGGGTTTTGGTCTG
GAGCGTCTGCTGAAAGTAAAACACGACTTCAAGAACATCAAACGTGCTGCACGTTCCGAGTCCTATTACAATGGT
ATTTCTACTAACCTGTAA (SEQ ID NO:91)
Protein:
MSDTKVKVAVRVRPMNRRELELNTKCVVEMEGNQTVLHPPPSNTKQGERKPPKVFAFDYCFWSMDESNTTKYAGQ
EVVFKCLGEGILEKAFQGYNACIFAYGQTGSGKSFSMMGHAEQLGLIPRLCCALFKRISLEQNESQTFKVEVSYM
EIYNEKVRDLLDPKGSRQSLKVREHKVLGPYVDGLSQLAVTSFEDIESLMSEGNKSRTVAATNMNEESSRSHAVF
NIIITQTLYDLQSGNSGEKVSKVSLVDLAGSERVSKTGAAGERLKEGSNINKSLTTLGLVISSLADQAAGKGKSK
FVPYRDSVLTWLLKDNLGGNSQTSMIATISPAADNYEETLSTLRYADRAKRIVNHAVVNEDPNAKVIRELREEVE
KLREQLSQAEAMKAELKEKLEESEKLIKELTVTWEYTDIEMNRLGKGAPGSAGSAAGSGMACPVPLQLPPLERLT
LDDKKPLNTLISATGLWMSRTGTIHKIKHHEVSRSKIYIEMACGDHLVVNNSRSSRTARALRHHKYRKTCKRCRV
SDEDLNKFLTKANEDQTSVKVKVVSAPTRTKKAMPKSVARAPKPLENTEAAQAQPSGSKFSPAIPVSTQESVSVP
ASVSTSISSISTGATASALVKGNTNPITSMSAPVQASAPALTKSQTDRLEVLLNPKDEISLNSGKPFRELESELL
SRRKKDLQQIYAEERENYLGKLEREITRFFVDRGFLEIKSPILIPLEYIERMGIDNDTELSKQIFRVDKNFCLRP
MLAPNLANYLRKLDRALPDPIKIFEIGPCYRKESDGKEHLEEFTMLNFCQMGSGCTRENLESIITDFLNHLGIDF
KIVGDSCMVFGDTLDVMHGDLELSSAVVGPIPLDREWGIDKPWIGAGFGLERLLKVKHDFKNIKRAARSESYYNG
ISTNL (SEQ ID NO:92)

KIF13A-MCP
DNA:
ATGTCGGATACCAAGGTAAAAGTTGCCGTCCGGGTCCGGCCCATGAACCGACGAGAACTGGAACTGAACACCAAG
TGCGTGGTGGAGATGGAAGGGAATCAAACGGTCCTGCACCCTCCTCCTTCTAACACCAAACAGGGAGAAAGGAAA
CCTCCCAAGGTATTTGCCTTTGATTATTGCTTTTGGTCCATGGATGAATCTAACACTACAAAATACGCTGGTCAA
GAAGTGGTTTTCAAGTGCCTTGGGGAAGGAATTCTTGAAAAAGCCTTTCAGGGGTATAATGCGTGTATTTTTGCA
TATGGACAGACAGGTTCGGGAAAATCCTTTTCCATGATGGGCCATGCTGAGCAGCTGGGCCTTATTCCAAGGCTC
TGCTGTGCTTTATTTAAAAGGATCTCTTTGGAGCAAAATGAGTCACAGACCTTTAAAGTTGAAGTGTCCTATATG
GAAATTTATAATGAGAAAGTTCGGGATCTTTTAGACCCCAAAGGGAGTAGACAGTCTCTTAAAGTTCGAGAACAT
AAAGTTTTGGGACCATATGTAGATGGTTTATCTCAACTAGCTGTCACTAGTTTTGAGGATATTGAGTCATTGATG
TCTGAGGGAAATAAGTCTCGAACGGTAGCTGCTACCAACATGAACGAAGAAAGCAGCCGCTCCCATGCTGTGTTC
AACATCATAATCACACAGACACTTTATGACCTGCAGTCTGGGAATTCCGGGGAGAAAGTCAGTAAGGTCAGCTTG
GTAGACCTGGCGGGTAGCGAAAGAGTATCTAAAACAGGAGCTGCAGGAGAGCGACTGAAAGAAGGCAGCAACATT
AACAAATCGCTTACAACCTTGGGGTTGGTTATATCATCACTGGCTGACCAGGCAGCTGGCAAGGGTAAAAGCAAA
TTTGTGCCTTATCGAGATTCAGTCCTCACTTGGCTGCTTAAGGACAACTTGGGGGGCAACAGCCAAACCTCTATG
ATAGCCACAATCAGCCCAGCCGCAGACAACTATGAAGAGACCCTCTCCACATTAAGATATGCAGACCGAGCCAAA
AGGATTGTGAACCATGCTGTTGTGAATGAGGACCCCAACGCAAAAGTGATCCGAGAACTGCGGGAGGAAGTCGAG
AAACTGAGAGAGCAGCTCTCTCAGGCAGAGGCCATGAAGGCCGAACTGAAGGAGAAGCTCGAAGAGTCTGAAAAG
CTGATAAAAGAACTAACAGTGACTTGGGAATATACAGATATTGAAATGAACAGATTGGGAAAGGGCGCCCCCGGC
TCCGCCGGCTCCGCCGCCGGCTCCGGCATGGCTTCTAACTTTACTCAGTTCGTTCTCGTCGACAATGGCGGAACT
GGCGACGTGACTGTCGCCCCAAGCAACTTCGCTAACGGGATCGCTGAATGGATCAGCTCTAACTCGCGTTCACAG
GCTTACAAAGTAACCTGTAGCGTTCGTCAGAGCTCTGCGCAGAATCGCAAATACACCATCAAAGTCGAGGTGCCT
AAAGGCGCCTGGCGTTCGTACTTAAATATGGAACTAACCATTCCAATTTTCGCCACGAATTCCGACTGCGAGCTT
ATTGTTAAGGCAATGCAAGGTCTCCTAAAAGATGGAAACCCGATTCCCTCAGCAATCGCAGCAAACTCCGGCATC
TACTAA (SEQ ID NO:93)
Protein:
MSDTKVKVAVRVRPMNRRELELNTKCVVEMEGNQTVLHPPPSNTKQGERKPPKVFAFDYCFWSMDESNTTKYAGQ
EVVFKCLGEGILEKAFQGYNACIFAYGQTGSGKSFSMMGHAEQLGLIPRLCCALFKRISLEQNESQTFKVEVSYM
EIYNEKVRDLLDPKGSRQSLKVREHKVLGPYVDGLSQLAVTSFEDIESLMSEGNKSRTVAATNMNEESSRSHAVF
NIIITQTLYDLQSGNSGEKVSKVSLVDLAGSERVSKTGAAGERLKEGSNINKSLTTLGLVISSLADQAAGKGKSK
FVPYRDSVLTWLLKDNLGGNSQTSMIATISPAADNYEETLSTLRYADRAKRIVNHAVVNEDPNAKVIRELREEVE
KLREQLSQAEAMKAELKEKLEESEKLIKELTVTWEYTDIEMNRLGKGAPGSAGSAAGSGMASNFTQFVLVDNGGT
GDVTVAPSNFANGIAEWISSNSRSQAYKVTCSVRQSSAQNRKYTIKVEVPKGAWRSYLNMELTIPIFATNSDCEL
IVKAMQGLLKDGNPIPSAIAANSGIY (SEQ ID NO:94)

TOMM20-EWSR1-MCP
DNA:
ATGGTGGGTCGGAACAGCGCCATCGCCGCCGGTGTATGCGGGGCCCTTTTCATTGGGTACTGCATCTACTTCGAC
CGCAAAAGACGAAGTGACCCCAACTTCAAGAACAGGCTTCGAGAACGAAGAAAGAAACAGAAGCTTGCCAAGGAG
AGAGCTGGGCTTTCCAAGTTACCTGACCTTAAAGATGCTGAAGCTGTTCAGAAGTTCTTCATGGCGTCCACGGAT
TACAGTACCTATAGCCAAGCTGCAGCGCAGCAGGGCTACAGTGCTTACACCGCCCAGCCCACTCAAGGATATGCA
CAGACCACCCAGGCATATGGGCAACAAAGCTATGGAACCTATGGACAGCCCACTGATGTCAGCTATACCCAGGCT
CAGACCACTGCAACCTATGGGCAGACCGCCTATGCAACTTCTTATGGACAGCCTCCCACTGGTTATACTACTCCA
ACTGCCCCCCAGGCATACAGCCAGCCTGTCCAGGGGTATGGCACTGGTGCTTATGATACCACCACTGCTACAGTC
ACCACCACCCAGGCCTCCTATGCAGCTCAGTCTGCATATGGCACTCAGCCTGCTTATCCAGCCTATGGGCAGCAG
CCAGCAGCCACTGCACCTACAAGACCGCAGGATGGAAACAAGCCCACTGAGACTAGTCAACCTCAATCTAGCACA
GGGGGTTACAACCAGCCCAGCCTAGGATATGGACAGAGTAACTACAGTTATCCCCAGGTACCTGGGAGCTACCCC
ATGCAGCCAGTCACTGCACCTCCATCCTACCCTCCTACCAGCTATTCCTCTACACAGCCGACTAGTTATGATCAG
AGCAGTTACTCTCAGCAGAACACCTATGGGCAACCGAGCAGCTATGGACAGCAGAGTAGCTATGGTCAACAAAGC
AGCTATGGGCAGCAGCCTCCCACTAGTTACCCACCCCAAACTGGATCCTACAGCCAAGCTCCAAGTCAATATAGC
CAACAGAGCAGCAGCTACGGGCAGCAGAGTTCATTCCGACAGGACCACCCCAGTAGCATGGGTGTTTATGGGCAG
GAGTCTGGAGGATTTTCCGGACCAGGAGAGAACCGGAGCATGAGTGGCCCTGATAACCGGGGCAGGGGAAGAGGG
GGATTTGATCGTGGAGGCATGAGCAGAGGTGGGCGGGGAGGAGGACGCGGTGGAATGGGCAGCGCTGGAGAGCGA
GGTGGCTTCAATAAGCCTGGTGGACCCATGGATGAAGGACCAGATCTTGATCTAGGCCCACCTGTAGATCCAGAT
GAAGACTCTGACAACAGTGCAATTTATGTACAAGGATTAAATGACAGTGTGACTCTAGATGATCTGGCAGACTTC
TTTAAGCAGTGTGGGGTTGTTAAGATGAACAAGAGAACTGGGCAACCCATGATCCACATCTACCTGGACAAGGAA
ACAGGAAAGCCCAAAGGCGATGCCACAGTGTCCTATGAAGACCCACCTACTGCCAAGGCTGCCGTGGAATGGTTT
GATGGGAAAGATTTTCAAGGGAGCAAACTTAAAGTCTCCCTTGCTCGGAAGAAGCCTCCAATGAACAGTATGCGG
GGTGGTCTGCCACCCCGTGAGGGCAGAGGCATGCCACCACCACTCCGTGGAGGTCCAGGAGGCCCAGGAGGTCCT
GGGGGACCCATGGGTCGCATGGGAGGCCGTGGAGGAGATAGAGGAGGCTTCCCTCCAAGAGGACCCCGGGGTTCC
CGAGGGAACCCCTCTGGAGGAGGAAACGTCCAGCACCGAGCTGGAGACTGGCAGTGTCCCAATCCGGGTTGTGGA
AACCAGAACTTCGCCTGGAGAACAGAGTGCAACCAGTGTAAGGCCCCAAAGCCTGAAGGCTTCCTCCCGCCACCC
TTTCCGCCCCCGGGTGGTGATCGTGGCAGAGGTGGCCCTGGTGGCATGCGGGGAGGAAGAGGTGGCCTCATGGAT
CGTGGTGGTCCCGGTGGAATGTTCAGAGGTGGCCGTGGTGGAGACAGAGGTGGCTTCCGTGGTGGCCGGGGCATG
GACCGAGGTGGCTTTGGTGGAGGAAGACGAGGTGGCCCTGGGGGGCCCCCTGGACCTTTGATGGAACAGGATTAC
AAGGATGACGACGATAAGGGTACCGAGCAGAAGCTGATCTCAGAGGAGGACCTGGGCGCCCCCGGCTCCGCCGGC
TCCGCCGCCGGCTCCGGCGCTTCTAACTTTACTCAGTTCGTTCTCGTCGACAATGGCGGAACTGGCGACGTGACT
GTCGCCCCAAGCAACTTCGCTAACGGGATCGCTGAATGGATCAGCTCTAACTCGCGTTCACAGGCTTACAAAGTA
ACCTGTAGCGTTCGTCAGAGCTCTGCGCAGAATCGCAAATACACCATCAAAGTCGAGGTGCCTAAAGGCGCCTGG
CGTTCGTACTTAAATATGGAACTAACCATTCCAATTTTCGCCACGAATTCCGACTGCGAGCTTATTGTTAAGGCA
ATGCAAGGTCTCCTAAAAGATGGAAACCCGATTCCCTCAGCAATCGCAGCAAACTCCGGCATCTACTAA (SEQ
ID NO:95)
Protein:
MVGRNSAIAAGVCGALFIGYCIYFDRKRRSDPNFKNRLRERRKKQKLAKERAGLSKLPDLKDAEAVQKFFMASTD
YSTYSQAAAQQGYSAYTAQPTQGYAQTTQAYGQQSYGTYGQPTDVSYTQAQTTATYGQTAYATSYGQPPTGYTTP
TAPQAYSQPVQGYGTGAYDTTTATVTTTQASYAAQSAYGTQPAYPAYGQQPAATAPTRPQDGNKPTETSQPQSST
GGYNQPSLGYGQSNYSYPQVPGSYPMQPVTAPPSYPPTSYSSTQPTSYDQSSYSQQNTYGQPSSYGQQSSYGQQS
SYGQQPPTSYPPQTGSYSQAPSQYSQQSSSYGQQSSFRQDHPSSMGVYGQESGGFSGPGENRSMSGPDNRGRGRG
GFDRGGMSRGGRGGGRGGMGSAGERGGFNKPGGPMDEGPDLDLGPPVDPDEDSDNSAIYVQGLNDSVTLDDLADF
FKQCGVVKMNKRTGQPMIHIYLDKETGKPKGDATVSYEDPPTAKAAVEWFDGKDFQGSKLKVSLARKKPPMNSMR
GGLPPREGRGMPPPLRGGPGGPGGPGGPMGRMGGRGGDRGGFPPRGPRGSRGNPSGGGNVQHRAGDWQCPNPGCG
NQNFAWRTECNQCKAPKPEGFLPPPFPPPGGDRGRGGPGGMRGGRGGLMDRGGPGGMFRGGRGGDRGGFRGGRGM
DRGGFGGGRRGGPGGPPGPLMEQDYKDDDDKGTEQKLISEEDLGAPGSAGSAAGSGASNFTQFVLVDNGGTGDVT
VAPSNFANGIAEWISSNSRSQAYKVTCSVRQSSAQNRKYTIKVEVPKGAWRSYLNMELTIPIFATNSDCELIVKA
MQGLLKDGNPIPSAIAANSGIY (SEQ ID NO:96)

TOMM20-EWSR1-HA-MCP
DNA:
ATGGTGGGTCGGAACAGCGCCATCGCCGCCGGTGTATGCGGGGCCCTTTTCATTGGGTACTGCATCTACTTCGAC
CGCAAAAGACGAAGTGACCCCAACTTCAAGAACAGGCTTCGAGAACGAAGAAAGAAACAGAAGCTTGCCAAGGAG
AGAGCTGGGCTTTCCAAGTTACCTGACCTTAAAGATGCTGAAGCTGTTCAGAAGTTCTTCATGGCGTCCACGGAT
TACAGTACCTATAGCCAAGCTGCAGCGCAGCAGGGCTACAGTGCTTACACCGCCCAGCCCACTCAAGGATATGCA

CAGACCACCCAGGCATATGGGCAACAAAGCTATGGAACCTATGGACAGCCCACTGATGTCAGCTATACCCAGGCT
CAGACCACTGCAACCTATGGGCAGACCGCCTATGCAACTTCTTATGGACAGCCTCCCACTGGTTATACTACTCCA
ACTGCCCCCCAGGCATACAGCCAGCCTGTCCAGGGGTATGGCACTGGTGCTTATGATACCACCACTGCTACAGTC
ACCACCACCCAGGCCTCCTATGCAGCTCAGTCTGCATATGGCACTCAGCCTGCTTATCCAGCCTATGGGCAGCAG
CCAGCAGCCACTGCACCTACAAGACCGCAGGATGGAAACAAGCCCACTGAGACTAGTCAACCTCAATCTAGCACA
GGGGGTTACAACCAGCCCAGCCTAGGATATGGACAGAGTAACTACAGTTATCCCCAGGTACCTGGGAGCTACCCC
ATGCAGCCAGTCACTGCACCTCCATCCTACCCTCCTACCAGCTATTCCTCTACACAGCCGACTAGTTATGATCAG
AGCAGTTACTCTCAGCAGAACACCTATGGGCAACCGAGCAGCTATGGACAGCAGAGTAGCTATGGTCAACAAAGC
AGCTATGGGCAGCAGCCTCCCACTAGTTACCCACCCCAAACTGGATCCTACAGCCAAGCTCCAAGTCAATATAGC
CAACAGAGCAGCAGCAGCTACGGGCAGCAGAGTTCATTCCGACAGGACCACCCCAGTAGCATGGGTGTTTATGGGCAG
GAGTCTGGAGGATTTTCCGGACCAGGAGAGAACCGGAGCATGAGTGGCCCTGATAACCGGGGCAGGGGAAGAGGG
GGATTTGATCGTGGAGGCATGAGCAGAGGTGGGCGGGGAGGAGGACGCGGTGGAATGGGCAGCGCTGGAGAGCGA
GGTGGCTTCAATAAGCCTGGTGGACCCATGGATGAAGGACCAGATCTTGATCTAGGCCCACCTGTAGATCCAGAT
GAAGACTCTGACAACAGTGCAATTTATGTACAAGGATTAAATGACAGTGTGACTCTAGATGATCTGGCAGACTTC
TTTAAGCAGTGTGGGGTTGTTAAGATGAACAAGAGAACTGGGCAACCCATGATCCACATCTACCTGGACAAGGAA
ACAGGAAAGCCCAAAGGCGATGCCACAGTGTCCTATGAAGACCCACCTACTGCCAAGGCTGCCGTGGAATGGTTT
GATGGGAAAGATTTTCAAGGGAGCAAACTTAAAGTCTCCCTTGCTCGGAAGAAGCCTCCAATGAACAGTATGCGG
GGTGGTCTGCCACCCCGTGAGGGCAGAGGCATGCCACCACCACTCCGTGGAGGTCCAGGAGGCCCAGGAGGTCCT
GGGGGACCCATGGGTCGCATGGGAGGCCGTGGAGGAGATAGAGGAGGCTTCCCTCCAAGAGGACCCCGGGGTTCC
CGAGGGAACCCCTCTGGAGGAGGAAACGTCCAGCACCGAGCTGGAGACTGGCAGTGTCCCAATCCGGGTTGTGGA
AACCAGAACTTCGCCTGGAGAACAGAGTGCAACCAGTGTAAGGCCCCAAAGCCTGAAGGCTTCCTCCCGCCACCC
TTTCCGCCCCCGGGTGGTGATCGTGGCAGAGGTGGCCCTGGTGGCATGCGGGGAGGAAGAGGTGGCCTCATGGAT
CGTGGTGGTCCCGGTGGAATGTTCAGAGGTGGCCGTGGTGGAGACAGAGGTGGCTTCCGTGGTGGCCGGGGCATG
GACCGAGGTGGCTTTGGTGGAGGAAGACGAGGTGGCCCTGGGGGGCCCCCTGGACCTTTGATGGAACAGGCGATC
GCATATCCCTATGATGTGCCGGATTATGCTGGAGCACCAGGAAGTGCTGGTTCTGCTGCTGGTAGTGGAGCTTCT
AACTTTACTCAGTTCGTTCTCGTCGACAATGGCGGAACTGGCGACGTGACTGTCGCCCCAAGCAACTTCGCTAAC
GGGATCGCTGAATGGATCAGCTCTAACTCGCGTTCACAGGCTTACAAAGTAACCTGTAGCGTTCGTCAGAGCTCT
GCGCAGAATCGCAAATACACCATCAAAGTCGAGGTGCCTAAAGGCGCCTGGCGTTCGTACTTAAATATGGAACTA
ACCATTCCAATTTTCGCCACGAATTCCGACTGCGAGCTTATTGTTAAGGCAATGCAAGGTCTCCTAAAAGATGGA
AACCCGATTCCCTCAGCAATCGCAGCAAACTCCGGCATCTACTAA (SEQ ID NO:97)
Protein:
MVGRNSAIAAGVCGALFIGYCIYFDRKRRSDPNFKNRLRERRKKQKLAKERAGLSKLPDLKDAEAVQKFFMASTD
YSTYSQAAAQQGYSAYTAQPTQGYAQTTQAYGQQSYGTYGQPTDVSYTQAQTTATYGQTAYATSYGQPPTGYTTP
TAPQAYSQPVQGYGTGAYDTTTATVTTTQASYAAQSAYGTQPAYPAYGQQPAATAPTRPQDGNKPTETSQPQSST
GGYNQPSLGYGQSNYSYPQVPGSYPMQPVTAPPSYPPTSYSSTQPTSYDQSSYSQQNTYGQPSSYGQQSSYGQQS
SYGQQPPTSYPPQTGSYSQAPSQYSQQSSSYGQQSSFRQDHPSSMGVYGQESGGFSGPGENRSMSGPDNRGRGRG
GFDRGGMSRGGRGGGRGGMGSAGERGGFNKPGGPMDEGPDLDLGPPVDPDEDSDNSAIYVQGLNDSVTLDDLADF
FKQCGVVKMNKRTGQPMIHIYLDKETGKPKGDATVSYEDPPTAKAAVEWFDGKDFQGSKLKVSLARKKPPMNSMR
GGLPPREGRGMPPPLRGGPGGPGGPGGPMGRMGGRGGDRGGFPPRGPRGSRGNPSGGGNVQHRAGDWQCPNPGCG
NQNFAWRTECNQCKAPKPEGFLPPPFPPPGGDRGRGGPGGMRGGRGGLMDRGGPGGMFRGGRGGDRGGFRGGRGM
DRGGFGGGRRGGPGGPPGPLMEQAIAYPYDVPDYAGAPGSAGSAAGSGASNFTQFVLVDNGGTGDVTVAPSNFAN
GIAEWISSNSRSQAYKVTCSVRQSSAQNRKYTIKVEVPKGAWRSYLNMELTIPIFATNSDCELIVKAMQGLLKDG
NPIPSAIAANSGIY (SEQ ID NO:98)

TOMM20-FUS-PylRS^AF
DNA:
ATGGTGGGTCGGAACAGCGCCATCGCCGCCGGTGTATGCGGGGCCCTTTTCATTGGGTACTGCATCTACTTCGAC
CGCAAAAGACGAAGTGACCCCAACTTCAAGAACAGGCTTCGAGAACGAAGAAAGAAACAGAAGCTTGCCAAGGAG
AGAGCTGGGCTTTCCAAGTTACCTGACCTTAAAGATGCTGAAGCTGTTCAGAAATTCTTCATGGCCTCAAACGAT
TATACCCAACAAGCAACCCAAAGCTATGGGGCCTACCCCACCCAGCCCGGGCAGGGCTATTCCCAGCAGAGCAGT
CAGCCCTACGGACAGCAGAGTTACAGTGGTTATAGCCAGTCCACGGACACTTCAGGATATGGCCAGAGCAGCTAT
TCTTCTTATGGCCAGAGCCAGAACACAGGCTATGGAACTCAGTCAACTCCCCAGGGATATGGCTCGACTGGCGGC
TATGGCAGTAGCCAGAGCTCCCAATCGTCTTACGGGCAGCAGTCCTCCTACCCTGGCTATGGCCAGCAGCCAGCT
CCCAGCAACACCTCGGGAAGTTACGGTAGCAGTTCTCAGAGCAGCAGCTATGGGCAGCCCCAGAGTGGGAGCTAC
AGCCAGCAGCCTAGCTATGGTGGACAGCAGCAAAGCTATGGACAGCAGCAAAGCTATAATCCCCCTCAGGGCTAT
GGACAGCAGAACCAGTACAACAGCAGCAGTGGTGGTGGAGGTGGAGGTGGAGGTGGAGGTAACTATGGCCAAGAT
CAATCCTCCATGAGTAGTGGTGGTGGCAGTGGTGGCGGTTATGGCAATCAAGACCAGAGTGGTGGAGGTGGCAGC
GGTGGCTATGGACAGCAGGACCGTGGAGGCCGCGGCAGGGGTGGCAGTGGTGGCGGCGGCGGCGGCGGCGGTGGT

GGTTACAACCGCAGCAGTGGTGGCTATGAACCCAGAGGTCGTGGAGGTGGCCGTGGAGGCAGAGGTGGCATGGGC
GGAAGTGACCGTGGTGGCTTCAATAAATTTGGTGGCCCTCGGGACCAAGGATCACGTCATGACTCCGAACAGGAT
AATTCAGACAACAACACCATCTTTGTGCAAGGCCTGGGTGAGAATGTTACAATTGAGTCTGTGGCTGATTACTTC
AAGCAGATTGGTATTATTAAGACAAACAAGAAAACGGGACAGCCCATGATTAATTTGTACACAGACAGGGAAACT
GGCAAGCTGAAGGGAGAGGCAACGGTCTCTTTTGATGACCCACCTTCAGCTAAAGCAGCTATTGACTGGTTTGAT
GGTAAAGAATTCTCCGGAAATCCTATCAAGGTCTCATTTGCTACTCGCCGGGCAGACTTTAATCGGGGTGGTGGC
AATGGTCGTGGAGGCCGAGGGCGAGGAGGACCCATGGGCCGTGGAGGCTATGGAGGTGGTGGCAGTGGTGGTGGT
GGCCGAGGAGGATTTCCCAGTGGAGGTGGTGGCGGTGGAGGACAGCAGCGAGCTGGTGACTGGAAGTGTCCTAAT
CCCACCTGTGAGAATATGAACTTCTCTTGGAGGAATGAATGCAACCAGTGTAAGGCCCCTAAACCAGATGGCCCA
GGAGGGGGACCAGGTGGCTCTCACATGGGGGGTAACTACGGGGATGATCGTCGTGGTGGCAGAGGAGGCGATTAC
AAGGATGACGACGATAAGGGTACCGGCGCCCCCGGCTCCGCCGGCTCCGCCGCCGGCTCCGGCGCGTGCCCGGTG
CCGCTGCAGCTGCCGCCGCTGGAACGCCTGACCCTGGATGATAAAAAACCGCTGAATACCCTGATCTCTGCTACT
GGTCTGTGGATGAGTCGTACCGGAACCATTCATAAAATCAAACACCACGAGGTTAGCCGTTCGAAAATCTATATT
GAGATGGCGTGTGGCGATCATCTGGTTGTGAACAATAGCCGCTCTTCTCGTACAGCACGTGCACTGCGTCACCAC
AAATATCGTAAAACCTGTAAACGTTGCCGTGTGTCCGATGAGGATCTGAACAAATTCCTGACAAAAGCCAATGAG
GACCAAACAAGCGTGAAAGTGAAAGTCGTTAGCGCTCCTACCCGTACTAAAAAAGCAATGCCGAAATCCGTTGCT
CGTGCCCCTAAACCACTGGAAAACACTGAAGCAGCACAGGCACAGCCGTCTGGAAGCAAATTCTCTCCGGCCATT
CCTGTTTCTACCCAGGAGTCCGTTTCTGTTCCAGCAAGTGTGAGCACCAGCATTAGCAGTATTAGCACCGGTGCC
ACCGCTAGCGCCCTGGTTAAAGGCAATACCAATCCGATTACAAGCATGTCTGCCCCGGTTCAAGCATCAGCTCCA
GCACTGACAAAATCCCAAACCGATCGTCTGGAGGTTCTGCTGAATCCGAAAGACGAAATCAGCCTGAATTCCGGC
AAACCGTTTCGTGAACTGGAGAGCGAACTGCTGTCACGTCGTAAAAAAGACCTGCAACAAATCTATGCCGAAGAA
CGTGAGAACTATCTGGGGAAACTGGAACGTGAAATCACCCGCTTTTTCGTGGATCGTGGCTTTCTGGAGATCAAA
TCCCCGATTCTGATTCCTCTGGAGTATATCGAGCGTATGGGCATCGACAATGATACCGAACTGAGCAAACAAATT
TTCCGTGTGGATAAAAACTTCTGTCTGCGCCCTATGCTAGCACCAAATCTGGCTAACTATCTGCGCAAACTGGAC
CGTGCCCTGCCTGATCCTATCAAAATCTTCGAGATCGGCCCGTGTTATCGTAAAGAGTCCGACGGTAAAGAACAT
CTGGAGGAGTTTACCATGCTGAACTTTTGCCAAATGGGTTCAGGTTGTACTCGTGAGAACCTGGAAAGCATCATC
ACCGATTTTCTGAACCACCTGGGCATTGACTTCAAAATTGTGGGCGACAGCTGTATGGTGTTTGGCGACACCCTG
GATGTCATGCACGGCGACCTGGAACTGTCTAGTGCCGTTGTGGGCCCAATCCCGCTGGATCGTGAGTGGGGTATC
GACAAACCTTGGATCGGTGCGGGTTTTGGTCTGGAGCGTCTGCTGAAAGTAAAACACGACTTCAAGAACATCAAA
CGTGCTGCACGTTCCGAGTCCTATTACAATGGTATTTCTACTAACCTGTAA (SEQ ID NO:99)
Protein:
MVGRNSAIAAGVCGALFIGYCIYFDRKRRSDPNFKNRLRERRKKQKLAKERAGLSKLPDLKDAEAVQKFFMASND
YTQQATQSYGAYPTQPGQGYSQQSSQPYGQQSYSGYSQSTDTSGYGQSSYSSYGQSQNTGYGTQSTPQGYGSTGG
YGSSQSSQSSYGQQSSYPGYGQQPAPSNTSGSYGSSSQSSSYGQPQSGSYSQQPSYGGQQQSYGQQQSYNPPQGY
GQQNQYNSSSGGGGGGGGGGNYGQDQSSMSSGGGSGGGYGNQDQSGGGGSGGYGQQDRGGRGRGGSGGGGGGGGG
GYNRSSGGYEPRGRGGGRGGRGGMGGSDRGGFNKFGGPRDQGSRHDSEQDNSDNNTIFVQGLGENVTIESVADYF
KQIGIIKTNKKTGQPMINLYTDRETGKLKGEATVSFDDPPSAKAAIDWFDGKEFSGNPIKVSFATRRADFNRGGG
NGRGGRGRGGPMGRGGYGGGGSGGGGRGGFPSGGGGGGGQQRAGDWKCPNPTCENMNFSWRNECNQCKAPKPDGP
GGGPGGSHMGGNYGDDRRGGRGGDYKDDDDKGTGAPGSAGSAAGSGACPVPLQLPPLERLTLDDKKPLNTLISAT
GLWMSRTGTIHKIKHHEVSRSKIYIEMACGDHLVVNNSRSSRTARALRHHKYRKTCKRCRVSDEDLNKFLTKANE
DQTSVKVKVVSAPTRTKKAMPKSVARAPKPLENTEAAQAQPSGSKFSPAIPVSTQESVSVPASVSTSISSISTGA
TASALVKGNTNPITSMSAPVQASAPALTKSQTDRLEVLLNPKDEISLNSGKPFRELESELLSRRKKDLQQIYAEE
RENYLGKLEREITRFFVDRGFLEIKSPILIPLEYIERMGIDNDTELSKQIFRVDKNFCLRPMLAPNLANYLRKLD
RALPDPIKIFEIGPCYRKESDGKEHLEEFTMLNFCQMGSGCTRENLESIITDFLNHLGIDFKIVGDSCMVFGDTL
DVMHGDLELSSAVVGPIPLDREWGIDKPWIGAGFGLERLLKVKHDFKNIKRAARSESYYNGISTNL (SEQ ID
NO:100)

TOMM20-FUS-V5-PylRS^AF
DNA:
ATGGTGGGTCGGAACAGCGCCATCGCCGCCGGTGTATGCGGGGCCCTTTTCATTGGGTACTGCATCTACTTCGAC
CGCAAAAGACGAAGTGACCCCAACTTCAAGAACAGGCTTCGAGAACGAAGAAAGAAACAGAAGCTTGCCAAGGAG
AGAGCTGGGCTTTCCAAGTTACCTGACCTTAAAGATGCTGAAGCTGTTCAGAAATTCTTCATGGCCTCAAACGAT
TATACCCAACAAGCAACCCAAAGCTATGGGGCCTACCCCACCCAGCCCGGGCAGGGCTATTCCCAGCAGAGCAGT
CAGCCCTACGGACAGCAGAGTTACAGTGGTTATAGCCAGTCCACGGACACTTCAGGATATGGCCAGAGCAGCTAT
TCTTCTTATGGCCAGAGCCAGAACACAGGCTATGGAACTCAGTCAACTCCCCAGGGATATGGCTCGACTGGCGGC
TATGGCAGTAGCCAGAGCTCCCAATCGTCTTACGGGCAGCAGTCCTCCTACCCTGGCTATGGCCAGCAGCCAGCT
CCCAGCAACACCTCGGGAAGTTACGGTAGCAGTTCTCAGAGCAGCAGCTATGGGCAGCCCCAGAGTGGGAGCTAC
AGCCAGCAGCCTAGCTATGGTGGACAGCAGCAAAGCTATGGACAGCAGCAAAGCTATAATCCCCCTCAGGGCTAT

GGACAGCAGAACCAGTACAACAGCAGCAGTGGTGGTGGAGGTGGAGGTGGAGGTGGAGGTAACTATGGCCAAGAT
CAATCCTCCATGAGTAGTGGTGGTGGCAGTGGTGGCGGTTATGGCAATCAAGACCAGAGTGGTGGAGGTGGCAGC
GGTGGCTATGGACAGCAGGACCGTGGAGGCCGCGGCAGGGGTGGCAGTGGTGGCGGCGGCGGCGGCGGTGGT
GGTTACAACCGCAGCAGTGGTGGCTATGAACCCAGAGGTCGTGGAGGTGGCCGTGGAGGCAGAGGTGGCATGGGC
GGAAGTGACCGTGGTGGCTTCAATAAATTTGGTGGCCCTCGGGACCAAGGATCACGTCATGACTCCGAACAGGAT
AATTCAGACAACAACACCATCTTTGTGCAAGGCCTGGGTGAGAATGTTACAATTGAGTCTGTGGCTGATTACTTC
AAGCAGATTGGTATTATTAAGACAAACAAGAAAACGGGACAGCCCATGATTAATTTGTACACAGACAGGGAAACT
GGCAAGCTGAAGGGAGAGGCAACGGTCTCTTTTGATGACCCACCTTCAGCTAAAGCAGCTATTGACTGGTTTGAT
GGTAAAGAATTCTCCGGAAATCCTATCAAGGTCTCATTTGCTACTCGCCGGGCAGACTTTAATCGGGGTGGTGGC
AATGGTCGTGGAGGCCGAGGGCGAGGAGGACCCATGGGCCGTGGAGGCTATGGAGGTGGTGGCAGTGGTGGTGGT
GGCCGAGGAGGATTTCCCAGTGGAGGTGGTGGCGGTGGAGGACAGCAGCGAGCTGGTGACTGGAAGTGTCCTAAT
CCCACCTGTGAGAATATGAACTTCTCTTGGAGGAATGAATGCAACCAGTGTAAGGCCCCTAAACCAGATGGCCCA
GGAGGGGGACCAGGTGGCTCTCACATGGGGGGTAACTACGGGGATGATCGTCGTGGTGGCAGAGGAGGCGCGATC
GCAGGCAAGCCTATTCCCAACCCCCTGCTGGGCCTGGATAGCACCGGAGCACCAGGAAGTGCTGGTTCTGCTGCT
GGTAGTGGAGCGTGCCCGGTGCCGCTGCAGCTGCCGCCGCTGGAACGCCTGACCCTGGATGATAAAAAACCGCTG
AATACCCTGATCTCTGCTACTGGTCTGTGGATGAGTCGTACCGGAACCATTCATAAAATCAAACACCACGAGGTT
AGCCGTTCGAAAATCTATATTGAGATGGCGTGTGGCGATCATCTGGTTGTGAACAATAGCCGCTCTTCTCGTACA
GCACGTGCACTGCGTCACCACAAATATCGTAAAACCTGTAAACGTTGCCGTGTGTCCGATGAGGATCTGAACAAA
TTCCTGACAAAAGCCAATGAGGACCAAACAAGCGTGAAAGTGAAAGTCGTTAGCGCTCCTACCCGTACTAAAAAA
GCAATGCCGAAATCCGTTGCTCGTGCCCCTAAACCACTGGAAAACACTGAAGCAGCACAGGCACAGCCGTCTGGA
AGCAAATTCTCTCCGGCCATTCCTGTTTCTACCCAGGAGTCCGTTTCTGTTCCAGCAAGTGTGAGCACCAGCATT
AGCAGTATTAGCACCGGTGCCACCGCTAGCGCCCTGGTTAAAGGCAATACCAATCCGATTACAAGCATGTCTGCC
CCGGTTCAAGCATCAGCTCCAGCACTGACAAAATCCCAAACCGATCGTCTGGAGGTTCTGCTGAATCCGAAAGAC
GAAATCAGCCTGAATTCCGGCAAACCGTTTCGTGAACTGGAGAGCGAACTGCTGTCACGTCGTAAAAAAGACCTG
CAACAAATCTATGCCGAAGAACGTGAGAACTATCTGGGGAAACTGGAACGTGAAATCACCCGCTTTTTCGTGGAT
CGTGGCTTTCTGGAGATCAAATCCCCGATTCTGATTCCTCTGGAGTATATCGAGCGTATGGGCATCGACAATGAT
ACCGAACTGAGCAAACAAATTTTCCGTGTGGATAAAAACTTCTGTCTGCGCCCTATGCTAGCACCAAATCTGGCT
AACTATCTGCGCAAACTGGACCGTGCCCTGCCTGATCCTATCAAAATCTTCGAGATCGGCCCGTGTTATCGTAAA
GAGTCCGACGGTAAAGAACATCTGGAGGAGTTTACCATGCTGAACTTTTGCCAAATGGGTTCAGGTTGTACTCGT
GAGAACCTGGAAAGCATCATCACCGATTTTCTGAACCACCTGGGCATTGACTTCAAAATTGTGGGCGACAGCTGT
ATGGTGTTTGGCGACACCCTGGATGTCATGCACGGCGACCTGGAACTGTCTAGTGCCGTTGTGGGCCCAATCCCG
CTGGATCGTGAGTGGGGTATCGACAAACCTTGGATCGGTGCGGGTTTTGGTCTGGAGCGTCTGCTGAAAGTAAAA
CACGACTTCAAGAACATCAAACGTGCTGCACGTTCCGAGTCCTATTACAATGGTATTTCTACTAACCTGTAA
(SEQ ID NO:101)
Protein:
MVGRNSAIAAGVCGALFIGYCIYFDRKRRSDPNFKNRLRERRKKQKLAKERAGLSKLPDLKDAEAVQKFFMASND
YTQQATQSYGAYPTQPGQGYSQQSSQPYGQQSYSGYSQSTDTSGYGQSSYSSYGQSQNTGYGTQSTPQGYGSTGG
YGSSQSSQSSYGQQSSYPGYGQQPAPSNTSGSYGSSSQSSSYGQPQSGSYSQQPSYGGQQQSYGQQQSYNPPQGY
GQQNQYNSSSGGGGGGGGGGNYGQDQSSMSSGGGSGGGYGNQDQSGGGGSGGYGQQDRGGRGRGGSGGGGGGGG
GYNRSSGGYEPRGRGGGRGGRGGMGGSDRGGFNKFGGPRDQGSRHDSEQDNSDNNTIFVQGLGENVTIESVADYF
KQIGIIKTNKKTGQPMINLYTDRETGKLKGEATVSFDDPPSAKAAIDWFDGKEFSGNPIKVSFATRRADFNRGGG
NGRGGRGRGGPMGRGGYGGGGSGGGGRGGFPSGGGGGGGQQRAGDWKCPNPTCENMNFSWRNECNQCKAPKPDGP
GGGPGGSHMGGNYGDDRRGGRGGAIAGKPIPNPLLGLDSTGAPGSAGSAAGSGACPVPLQLPPLERLTLDDKKPL
NTLISATGLWMSRTGTIHKIKHHEVSRSKIYIEMACGDHLVVNNSRSSRTARALRHHKYRKTCKRCRVSDEDLNK
FLTKANEDQTSVKVKVVSAPTRTKKAMPKSVARAPKPLENTEAAQAQPSGSKFSPAIPVSTQESVSVPASVSTSI
SSISTGATASALVKGNTNPITSMSAPVQASAPALTKSQTDRLEVLLNPKDEISLNSGKPFRELESELLSRRKKDL
QQIYAEERENYLGKLEREITRFFVDRGFLEIKSPILIPLEYIERMGIDNDTELSKQIFRVDKNFCLRPMLAPNLA
NYLRKLDRALPDPIKIFEIGPCYRKESDGKEHLEEFTMLNFCQMGSGCTRENLESIITDFLNHLGIDFKIVGDSC
MVFGDTLDVMHGDLELSSAVVGPIPLDREWGIDKPWIGAGFGLERLLKVKHDFKNIKRAARSESYYNGISTNL
(SEQ ID NO:102)

TOMM20-FUS-PylRS^AA
DNA:
ATGGTGGGTCGGAACAGCGCCATCGCCGCCGGTGTATGCGGGGCCCTTTTCATTGGGTACTGCATCTACTTCGAC
CGCAAAAGACGAAGTGACCCCAACTTCAAGAACAGGCTTCGAGAACGAAGAAAGAAACAGAAGCTTGCCAAGGAG
AGAGCTGGGCTTTCCAAGTTACCTGACCTTAAAGATGCTGAAGCTGTTCAGAAGTTCTTCATGGCCTCAAACGAT
TATACCCAACAAGCAACCCAAAGCTATGGGGCCTACCCCACCCAGCCCGGGCAGGGCTATTCCCAGCAGAGCAGT
CAGCCCTACGGACAGCAGAGTTACAGTGGTTATAGCCAGTCCACGGACACTTCAGGATATGGCCAGAGCAGCTAT

TCTTCTTATGGCCAGAGCCAGAACACAGGCTATGGAACTCAGTCAACTCCCCAGGGATATGGCTCGACTGGCGGC
TATGGCAGTAGCCAGAGCTCCCAATCGTCTTACGGGCAGCAGTCCTCCTACCCTGGCTATGGCCAGCAGCCAGCT
CCCAGCAGCACCTCGGGAAGTTACGGTAGCAGTTCTCAGAGCAGCAGCTATGGGCAGCCCCAGAGTGGGAGCTAC
AGCCAGCAGCCTAGCTATGGTGGACAGCAGCAAAGCTATGGACAGCAGCAAAGCTATAATCCCCCTCAGGGCTAT
GGACAGCAGAACCAGTACAACAGCAGCAGTGGTGGTGGAGGTGGAGGTGGAGGTGGAGGTAACTATGGCCAAGAT
CAATCCTCCATGAGTAGTGGTGGTGGCAGTGGTGGCGGTTATGGCAATCAAGACCAGAGTGGTGGAGGTGGCAGC
GGTGGCTATGGACAGCAGGACCGTGGAGGCCGCGGCAGGGGTGGCAGTGGTGGCGGCGGCGGCGGCGGCGGTGGT
GGTTACAACCGCAGCAGTGGTGGCTATGAACCCAGAGGTCGTGGAGGTGGCCGTGGAGGCAGAGGTGGCATGGGC
GGAAGTGACCGTGGTGGCTTCAATAAATTTGGTGGCCCTCGGGACCAAGGATCACGTCATGACTCCGAACAGGAT
AATTCAGACAACAACACCATCTTTGTGCAAGGCCTGGGTGAGAATGTTACAATTGAGTCTGTGGCTGATTACTTC
AAGCAGATTGGTATTATTAAGACAAACAAGAAAACGGGACAGCCCATGATTAATTTGTACACAGACAGGGAAACT
GGCAAGCTGAAGGGAGAGGCAACGGTCTCTTTTGATGACCCACCTTCAGCTAAAGCAGCTATTGACTGGTTTGAT
GGTAAAGAATTCTCCGGAAATCCTATCAAGGTCTCATTTGCTACTCGCCGGGCAGACTTTAATCGGGGTGGTGGC
AATGGTCGTGGAGGCCGAGGGCGAGGAGGACCCATGGGCCGTGGAGGCTATGGAGGTGGTGGCAGTGGTGGTGGT
GGCCGAGGAGGATTTCCCAGTGGAGGTGGTGGCGGTGGAGGACAGCAGCGAGCTGGTGACTGGAAGTGTCCTAAT
CCCACCTGTGAGAATATGAACTTCTCTTGGAGGAATGAATGCAACCAGTGTAAGGCCCCTAAACCAGATGGCCCA
GGAGGGGGACCAGGTGGCTCTCACATGGGGGGTAACTACGGGGATGATCGTCGTGGTGGCAGAGGAGGCGGCGCC
CCCGGCTCCGCCGGCTCCGCCGCCGGCTCCGGCATGGCGTGCCCGGTGCCGCTGCAGCTGCCGCCGCTGGAACGC
CTGACCCTGGATGACAAAAAACCGCTGAATACCCTGATCTCTGCTACTGGTCTGTGGATGAGTCGTACCGGAACC
ATTCATAAAATCAAACACCACGAGGTTAGCCGTTCGAAAATCTATATTGAGATGGCGTGTGGCGATCATCTGGTT
GTGAACAATAGCCGCTCTTCTCGTACAGCACGTGCACTGCGTCACCACAAATATCGTAAAACCTGTAAACGTTGC
CGTGTGTCCGATGAGGATCTGAACAAATTCCTGACAAAAGCCAATGAGGACCAAACAAGCGTGAAAGTGAAAGTC
GTTAGCGCTCCTACCCGTACTAAAAAAGCAATGCCGAAATCCGTTGCTCGTGCCCCTAAACCACTGGAAAACACT
GAAGCAGCACAGGCACAGCCGTCTGGAAGCAAATTCTCTCCGGCCATTCCTGTTTCTACCCAGGAGTCCGTTTCT
GTTCCAGCAAGTGTGAGCACCAGCATTAGCAGTATTAGCACCGGTGCCACCGCTAGCGCCCTGGTTAAAGGCAAT
ACCAATCCGATTACAAGCATGTCTGCCCCGGTTCAAGCATCAGCTCCAGCACTGACAAAATCCCAAACCGATCGT
CTGGAGGTTCTGCTGAATCCGAAAGACGAAATCAGCCTGAATTCCGGCAAACCGTTTCGTGAACTGGAGAGCGAA
CTGCTGTCACGTCGTAAAAAAGACCTGCAACAAATCTATGCCGAAGAACGTGAGAACTATCTGGGGAAACTGGAA
CGTGAAATCACCCGCTTTTTCGTGGATCGTGGCTTTCTGGAGATCAAATCCCCGATTCTGATTCCTCTGGAGTAT
ATCGAGCGTATGGGCATCGACAATGATACCGAACTGAGCAAACAAATTTTCCGTGTGGATAAAAACTTCTGTCTG
CGCCCTATGCTGGCACCAAATCTGTATAACTATCTGCGCAAACTGGACCGTGCCCTGCCTGATCCTATCAAAATC
TTCGAGATCGGCCCGTGTTATCGTAAAGAGTCCGACGGTAAAGAACATCTGGAGGAGTTTACCATGCTGGCCTTT
GCCCAAATGGGTTCAGGTTGTACTCGTGAGAACCTGGAAAGCATCATCACCGATTTTCTGAACCACCTGGGCATT
GACTTCAAAATTGTGGGCGACAGCTGTATGGTGTATGGCGACACCCTGGATGTCATGCACGGCGACCTGGAACTG
TCTAGTGCCGTTGTTGGACCAATTCCGCTGGACCGTGAGTGGGGTATCGACAAACCGTGGATCGGAGCAGGATTC
GGTCTGGAACGCCTGCTGAAAGTGAAACACGACTTCAAAAACATCAAACGTGCCGCCCGTTCTGAATCGTATTAT
AACGGGATCTCTACGAACCTGTAA (SEQ ID NO:103)
Protein:
MVGRNSAIAAGVCGALFIGYCIYFDRKRRSDPNFKNRLRERRKKQKLAKERAGLSKLPDLKDAEAVQKFFMASND
YTQQATQSYGAYPTQPGQGYSQQSSQPYGQQSYSGYSQSTDTSGYGQSSYSSYGQSQNTGYGTQSTPQGYGSTGG
YGSSQSSQSSYGQQSSYPGYGQQPAPSSTSGSYGSSSQSSSYGQPQSGSYSQQPSYGGQQQSYGQQQSYNPPQGY
GQQNQYNSSSGGGGGGGGGGNYGQDQSSMSSGGGSGGGYGNQDQSGGGGSGGYGQQDRGGRGRGGSGGGGGGGGG
GYNRSSGGYEPRGRGGGRGGRGGMGGSDRGGFNKFGGPRDQGSRHDSEQDNSDNNTIFVQGLGENVTIESVADYF
KQIGIIKTNKKTGQPMINLYTDRETGKLKGEATVSFDDPPSAKAAIDWFDGKEFSGNPIKVSFATRRADFNRGGG
NGRGGRGRGGPMGRGGYGGGGSGGGGRGGFPSGGGGGGGQQRAGDWKCPNPTCENMNFSWRNECNQCKAPKPDGP
GGGPGGSHMGGNYGDDRRGGRGGGAPGSAGSAAGSGMACPVPLQLPPLERLTLDDKKPLNTLISATGLWMSRTGT
IHKIKHHEVSRSKIYIEMACGDHLVVNNSRSSRTARALRHHKYRKTCKRCRVSDEDLNKFLTKANEDQTSVKVKV
VSAPTRTKKAMPKSVARAPKPLENTEAAQAQPSGSKFSPAIPVSTQESVSVPASVSTSISSISTGATASALVKGN
TNPITSMSAPVQASAPALTKSQTDRLEVLLNPKDEISLNSGKPFRELESELLSRRKKDLQQIYAEERENYLGKLE
REITRFFVDRGFLEIKSPILIPLEYIERMGIDNDTELSKQIFRVDKNFCLRPMLAPNLYNYLRKLDRALPDPIKI
FEIGPCYRKESDGKEHLEEFTMLAFAQMGSGCTRENLESIITDFLNHLGIDFKIVGDSCMVYGDTLDVMHGDLEL
SSAVVGPIPLDREWGIDKPWIGAGFGLERLLKVKHDFKNIKRAARSESYYNGISTNL (SEQ ID NO:104)

TOMM20-FUS-V5-PylRS^AA
DNA:
ATGGTGGGTCGGAACAGCGCCATCGCCGCCGGTGTATGCGGGGCCCTTTTCATTGGGTACTGCATCTACTTCGAC
CGCAAAAGACGAAGTGACCCCAACTTCAAGAACAGGCTTCGAGAACGAAGAAAGAAACAGAAGCTTGCCAAGGAG
AGAGCTGGGCTTTCCAAGTTACCTGACCTTAAAGATGCTGAAGCTGTTCAGAAATTCTTCATGGCCTCAAACGAT

TATACCCAACAAGCAACCCAAAGCTATGGGGCCTACCCCACCCAGCCCGGGCAGGGCTATTCCCAGCAGAGCAGT
CAGCCCTACGGACAGCAGAGTTACAGTGGTTATAGCCAGTCCACGGACACTTCAGGATATGGCCAGAGCAGCTAT
TCTTCTTATGGCCAGAGCCAGAACACAGGCTATGGAACTCAGTCAACTCCCCAGGGATATGGCTCGACTGGCGGC
TATGGCAGTAGCCAGAGCTCCCAATCGTCTTACGGGCAGCAGTCCTCCTACCCTGGCTATGGCCAGCAGCCAGCT
CCCAGCAACACCTCGGGAAGTTACGGTAGCAGTTCTCAGAGCAGCAGCTATGGGCAGCCCCAGAGTGGGAGCTAC
AGCCAGCAGCCTAGCTATGGTGGACAGCAGCAAAGCTATGGACAGCAGCAAAGCTATAATCCCCCTCAGGGCTAT
GGACAGCAGAACCAGTACAACAGCAGCAGTGGTGGTGGAGGTGGAGGTGGAGGTGGAGGTAACTATGGCCAAGAT
CAATCCTCCATGAGTAGTGGTGGTGGCAGTGGTGGCGGTTATGGCAATCAAGACCAGAGTGGTGGAGGTGGCAGC
GGTGGCTATGGACAGCAGGACCGTGGAGGCCGCGGCAGGGGTGGCAGTGGTGGCGGCGGCGGCGGCGGTGGT
GGTTACAACCGCAGCAGTGGTGGCTATGAACCCAGAGGTCGTGGAGGTGGCCGTGGAGGCAGAGGTGGCATGGGC
GGAAGTGACCGTGGTGGCTTCAATAAATTTGGTGGCCCTCGGGACCAAGGATCACGTCATGACTCCGAACAGGAT
AATTCAGACAACAACACCATCTTTGTGCAAGGCCTGGGTGAGAATGTTACAATTGAGTCTGTGGCTGATTACTTC
AAGCAGATTGGTATTATTAAGACAAACAAGAAAACGGGACAGCCCATGATTAATTTGTACACAGACAGGGAAACT
GGCAAGCTGAAGGGAGAGGCAACGGTCTCTTTTGATGACCCACCTTCAGCTAAAGCAGCTATTGACTGGTTTGAT
GGTAAAGAATTCTCCGGAAATCCTATCAAGGTCTCATTTGCTACTCGCCGGGCAGACTTTAATCGGGGTGGTGGC
AATGGTCGTGGAGGCCGAGGGCGAGGAGGACCCATGGGCCGTGGAGGCTATGGAGGTGGTGGCAGTGGTGGTGGT
GGCCGAGGAGGATTTCCCAGTGGAGGTGGTGGCGGTGGAGGACAGCAGCGAGCTGGTGACTGGAAGTGTCCTAAT
CCCACCTGTGAGAATATGAACTTCTCTTGGAGGAATGAATGCAACCAGTGTAAGGCCCCTAAACCAGATGGCCCA
GGAGGGGGACCAGGTGGCTCTCACATGGGGGGTAACTACGGGGATGATCGTCGTGGTGGCAGAGGAGGCGCGATC
GCAGGCAAGCCTATTCCCAACCCCCTGCTGGGCCTGGATAGCACCGGAGCACCAGGAAGTGCTGGTTCTGCTGCT
GGTAGTGGAGCGTGCCCGGTGCCGCTGCAGCTGCCGCCGCTGGAACGCCTGACCCTGGATGACAAAAAACCGCTG
AATACCCTGATCTCTGCTACTGGTCTGTGGATGAGTCGTACCGGAACCATTCATAAAATCAAACACCACGAGGTT
AGCCGTTCGAAAATCTATATTGAGATGGCGTGTGGCGATCATCTGGTTGTGAACAATAGCCGCTCTTCTCGTACA
GCACGTGCACTGCGTCACCACAAATATCGTAAAACCTGTAAACGTTGCCGTGTGTCCGATGAGGATCTGAACAAA
TTCCTGACAAAAGCCAATGAGGACCAAACAAGCGTGAAAGTGAAAGTCGTTAGCGCTCCTACCCGTACTAAAAAA
GCAATGCCGAAATCCGTTGCTCGTGCCCCTAAACCACTGGAAAACACTGAAGCAGCACAGGCACAGCCGTCTGGA
AGCAAATTCTCTCCGGCCATTCCTGTTTCTACCCAGGAGTCCGTTTCTGTTCCAGCAAGTGTGAGCACCAGCATT
AGCAGTATTAGCACCGGTGCCACCGCTAGCGCCCTGGTTAAAGGCAATACCAATCCGATTACAAGCATGTCTGCC
CCGGTTCAAGCATCAGCTCCAGCACTGACAAAATCCCAAACCGATCGTCTGGAGGTTCTGCTGAATCCGAAAGAC
GAAATCAGCCTGAATTCCGGCAAACCGTTTCGTGAACTGGAGAGCGAACTGCTGTCACGTCGTAAAAAAGACCTG
CAACAAATCTATGCCGAAGAACGTGAGAACTATCTGGGGAAACTGGAACGTGAAATCACCCGCTTTTTCGTGGAT
CGTGGCTTTCTGGAGATCAAATCCCCGATTCTGATTCCTCTGGAGTATATCGAGCGTATGGGCATCGACAATGAT
ACCGAACTGAGCAAACAAATTTTCCGTGTGGATAAAAACTTCTGTCTGCGCCCTATGCTGGCACCAAATCTGTAT
AACTATCTGCGCAAACTGGACCGTGCCCTGCCTGATCCTATCAAAATCTTCGAGATCGGCCCGTGTTATCGTAAA
GAGTCCGACGGTAAAGAACATCTGGAGGAGTTTACCATGCTGGCCTTTTGCCAAATGGGTTCAGGTTGTACTCGT
GAGAACCTGGAAAGCATCATCACCGATTTTCTGAACCACCTGGGCATTGACTTCAAAATTGTGGGCGACAGCTGT
ATGGTGTATGGCGACACCCTGGATGTCATGCACGGCGACCTGGAACTGTCTAGTGCCGTTGTGGACCAATTCCG
CTGGACCGTGAGTGGGGTATCGACAAACCGTGGATCGGAGCAGGATTCGGTCTGGAACGCCTGCTGAAAGTGAAA
CACGACTTCAAAAACATCAAACGTGCCGCCCGTTCTGAATCGTATTATAACGGGATCTCTACGAACCTGTAA
(SEQ ID NO:105)
Protein:
MVGRNSAIAAGVCGALFIGYCIYFDRKRRSDPNFKNRLRERRKKQKLAKERAGLSKLPDLKDAEAVQKFFMASND
YTQQATQSYGAYPTQPGQGYSQQSSQPYGQQSYSGYSQSTDTSGYGQSSYSSYGQSQNTGYGTQSTPQGYGSTGG
YGSSQSSQSSYGQQSSYPGYGQQPAPSNTSGSYGSSSQSSSYGQPQSGSYSQQPSYGGQQQSYGQQQSYNPPQGY
GQQNQYNSSSGGGGGGGGGGNYGQDQSSMSSGGGSGGGYGNQDQSGGGGSGGYGQQDRGGRGRGGSGGGGGGGGG
GYNRSSGGYEPRGRGGGRGGRGGMGGSDRGGFNKFGGPRDQGSRHDSEQDNSDNNTIFVQGLGENVTIESVADYF
KQIGIIKTNKKTGQPMINLYTDRETGKLKGEATVSFDDPPSAKAAIDWFDGKEFSGNPIKVSFATRRADFNRGGG
NGRGGRGRGGPMGRGGYGGGGSGGGGRGGFPSGGGGGGGQQRAGDWKCPNPTCENMNFSWRNECNQCKAPKPDGP
GGGPGGSHMGGNYGDDRRGGRGGAIAGKPIPNPLLGLDSTGAPGSAGSAAGSGACPVPLQLPPLERLTLDDKKPL
NTLISATGLWMSRTGTIHKIKHHEVSRSKIYIEMACGDHLVVNNSRSSRTARALRHHKYRKTCKRCRVSDEDLNK
FLTKANEDQTSVKVKVVSAPTRTKKAMPKSVARAPKPLENTEAAQAQPSGSKFSPAIPVSTQESVSVPASVSTSI
SSISTGATASALVKGNTNPITSMSAPVQASAPALTKSQTDRLEVLLNPKDEISLNSGKPFRELESELLSRRKKDL
QQIYAEERENYLGKLEREITRFFVDRGFLEIKSPILIPLEYIERMGIDNDTELSKQIFRVDKNFCLRPMLAPNLY
NYLRKLDRALPDPIKIFEIGPCYRKESDGKEHLEEFTMLAFAQMGSGCTRENLESIITDFLNHLGIDFKIVGDSC
MVYGDTLDVMHGDLELSSAVVGPIPLDREWGIDKPWIGAGFGLERLLKVKHDFKNIKRAARSESYYNGISTNL
(SEQ ID NO:106)

TOMM20-FUS-PylRS^AAAF

DNA:
ATGGTGGGTCGGAACAGCGCCATCGCCGCCGGTGTATGCGGGGCCCTTTTCATTGGGTACTGCATCTACTTCGAC
CGCAAAAGACGAAGTGACCCCAACTTCAAGAACAGGCTTCGAGAACGAAGAAAGAAACAGAAGCTTGCCAAGGAG
AGAGCTGGGCTTTCCAAGTTACCTGACCTTAAAGATGCTGAAGCTGTTCAGAAATTCTTCATGGCCTCAAACGAT
TATACCCAACAAGCAACCCAAAGCTATGGGGCCTACCCCACCCAGCCCGGGCAGGGCTATTCCCAGCAGAGCAGT
CAGCCCTACGGACAGCAGAGTTACAGTGGTTATAGCCAGTCCACGGACACTTCAGGATATGGCCAGAGCAGCTAT
TCTTCTTATGGCCAGAGCCAGAACACAGGCTATGGAACTCAGTCAACTCCCCAGGGATATGGCTCGACTGGCGGC
TATGGCAGTAGCCAGAGCTCCCAATCGTCTTACGGGCAGCAGTCCTCCTACCCTGGCTATGGCCAGCAGCCAGCT
CCCAGCAACACCTCGGGAAGTTACGGTAGCAGTTCTCAGAGCAGCAGCTATGGGCAGCCCCAGAGTGGGAGCTAC
AGCCAGCAGCCTAGCTATGGTGGACAGCAGCAAAGCTATGGACAGCAGCAAAGCTATAATCCCCCTCAGGGCTAT
GGACAGCAGAACCAGTACAACAGCAGCAGTGGTGGTGGAGGTGGAGGTGGAGGTGGAGGTAACTATGGCCAAGAT
CAATCCTCCATGAGTAGTGGTGGTGGCAGTGGTGGCGGTTATGGCAATCAAGACCAGAGTGGTGGAGGTGGCAGC
GGTGGCTATGGACAGCAGGACCGTGGAGGCCGCGGCAGGGGTGGCAGTGGTGGCGGCGGCGGCGGCGGTGGT
GGTTACAACCGCAGCAGTGGTGGCTATGAACCCAGAGGTCGTGGAGGTGGCCGTGGAGGCAGAGGTGGCATGGGC
GGAAGTGACCGTGGTGGCTTCAATAAATTTGGTGGCCCTCGGGACCAAGGATCACGTCATGACTCCGAACAGGAT
AATTCAGACAACAACACCATCTTTGTGCAAGGCCTGGGTGAGAATGTTACAATTGAGTCTGTGGCTGATTACTTC
AAGCAGATTGGTATTATTAAGACAAACAAGAAAACGGGACAGCCCATGATTAATTTGTACACAGACAGGGAAACT
GGCAAGCTGAAGGGAGAGGCAACGGTCTCTTTTGATGACCCACCTTCAGCTAAAGCAGCTATTGACTGGTTTGAT
GGTAAAGAATTCTCCGGAAATCCTATCAAGGTCTCATTTGCTACTCGCCGGGCAGACTTTAATCGGGGTGGTGGC
AATGGTCGTGGAGGCCGAGGGCGAGGAGGACCCATGGGCCGTGGAGGCTATGGAGGTGGTGGCAGTGGTGGTGGT
GGCCGAGGAGGATTTCCCAGTGGAGGTGGTGGCGGTGGAGGACAGCAGCGAGCTGGTGACTGGAAGTGTCCTAAT
CCCACCTGTGAGAATATGAACTTCTCTTGGAGGAATGAATGCAACCAGTGTAAGGCCCCTAAACCAGATGGCCCA
GGAGGGGGACCAGGTGGCTCTCACATGGGGGGTAACTACGGGGATGATCGTCGTGGTGGCAGAGGAGGCGATTAC
AAGGATGACGACGATAAGGGTACCGGCGCCCCCGGCTCCGCCGGCTCCGCCGCCGGCTCCGGCGCGTGCCCGGTG
CCGCTGCAGCTGCCGCCGCTGGAACGCCTGACCCTGGATGATAAAAAACCGCTGAATACCCTGATCTCTGCTACT
GGTCTGTGGATGAGTCGTACCGGAACCATTCATAAAATCAAACACCACGAGGTTAGCCGTTCGAAAATCTATATT
GAGATGGCGTGTGGCGATCATCTGGTTGTGAACAATAGCCGCTCTTCTCGTACAGCACGTGCACTGCGTCACCAC
AAATATCGTAAAACCTGTAAACGTTGCCGTGTGTCCGATGAGGATCTGAACAAATTCCTGACAAAAGCCAATGAG
GACCAAACAAGCGTGAAAGTGAAAGTCGTTAGCGCTCCTACCCGTACTAAAAAAGCAATGCCGAAATCCGTTGCT
CGTGCCCCTAAACCACTGGAAAACACTGAAGCAGCACAGGCACAGCCGTCTGGAAGCAAATTCTCTCCGGCCATT
CCTGTTTCTACCCAGGAGTCCGTTTCTGTTCCAGCAAGTGTGAGCACCAGCATTAGCAGTATTAGCACCGGTGCC
ACCGCTAGCGCCCTGGTTAAAGGCAATACCAATCCGATTACAAGCATGTCTGCCCCGGTTCAAGCATCAGCTCCA
GCACTGACAAAATCCCAAACCGATCGTCTGGAGGTTCTGCTGAATCCGAAAGACGAAATCAGCCTGAATTCCGGC
AAACCGTTTCGTGAACTGGAGAGCGAACTGCTGTCACGTCGTAAAAAAGACCTGCAACAAATCTATGCCGAAGAA
CGTGAGAACTATCTGGGGAAACTGGAACGTGAAATCACCCGCTTTTTCGTGGATCGTGGCTTTCTGGAGATCAAA
TCCCCGATTCTGATTCCTCTGGAGTATATCGAGCGTATGGGCATCGACAATGATACCGAACTGAGCAAACAAATT
TTCCGTGTGGATAAAAACTTCTGTCTGCGCCCTATGCTAGCACCAAATCTGGCTAACTATCTGCGCAAACTGGAC
CGTGCCCTGCCTGATCCTATCAAAATCTTCGAGATCGGCCCGTGTTATCGTAAAGAGTCCGACGGTAAAGAACAT
CTGGAGGAGTTTACCATGCTGGCCTTTGCCCAAATGGGTTCAGGTTGTACTCGTGAGAACCTGGAAAGCATCATC
ACCGATTTTCTGAACCACCTGGGCATTGACTTCAAAATTGTGGGCGACAGCTGTATGGTGTTTGGCGACACCCTG
GATGTCATGCACGGCGACCTGGAACTGTCTAGTGCCGTTGTGGGCCCAATCCCGCTGGATCGTGAGTGGGGTATC
GACAAACCTTGGATCGGTGCGGGTTTTGGTCTGGAGCGTCTGCTGAAAGTAAAACACGACTTCAAGAACATCAAA
CGTGCTGCACGTTCCGAGTCCTATTACAATGGTATTTCTACTAACCTGTAA (SEQ ID NO:107)
Protein:
MVGRNSAIAAGVCGALFIGYCIYFDRKRRSDPNFKNRLRERRKKQKLAKERAGLSKLPDLKDAEAVQKFFMASND
YTQQATQSYGAYPTQPGQGYSQQSSQPYGQQSYSGYSQSTDTSGYGQSSYSSYGQSQNTGYGTQSTPQGYGSTGG
YGSSQSSQSSYGQQSSYPGYGQQPAPSNTSGSYGSSSQSSSYGQPQSGSYSQQPSYGGQQQSYGQQQSYNPPQGY
GQQNQYNSSSGGGGGGGGGGNYGQDQSSMSSGGGSGGGYGNQDQSGGGGSGGYGQQDRGGRGRGGSGGGGGGGG
GYNRSSGGYEPRGRGGGRGGRGGMGGSDRGGFNKFGGPRDQGSRHDSEQDNSDNNTIFVQGLGENVTIESVADYF
KQIGIIKTNKKTGQPMINLYTDRETGKLKGEATVSFDDPPSAKAAIDWFDGKEFSGNPIKVSFATRRADFNRGGG
NGRGGRGRGGPMGRGGYGGGGSGGGGRGGFPSGGGGGGGQQRAGDWKCPNPTCENMNFSWRNECNQCKAPKPDGP
GGGPGGSHMGGNYGDDRRGGRGGDYKDDDDKGTGAPGSAGSAAGSGACPVPLQLPPLERLTLDDKKPLNTLISAT
GLWMSRTGTIHKIKHHEVSRSKIYIEMACGDHLVVNNSRSSRTARALRHHKYRKTCKRCRVSDEDLNKFLTKANE
DQTSVKVKVVSAPTRTKKAMPKSVARAPKPLENTEAAQAQPSGSKFSPAIPVSTQESVSVPASVSTSISSISTGA
TASALVKGNTNPITSMSAPVQASAPALTKSQTDRLEVLLNPKDEISLNSGKPFRELESELLSRRKKDLQQIYAEE
RENYLGKLEREITRFFVDRGFLEIKSPILIPLEYIERMGIDNDTELSKQIFRVDKNFCLRPMLAPNLANYLRKLD
RALPDPIKIFEIGPCYRKESDGKEHLEEFTMLAFAQMGSGCTRENLESIITDFLNHLGIDFKIVGDSCMVFGDTL

DVMHGDLELSSAVVGPIPLDREWGIDKPWIGAGFGLERLLKVKHDFKNIKRAARSESYYNGISTNL (SEQ ID NO:108)

TOMM20-FUS-V5-PylRS^AAAF
DNA:
ATGGTGGGTCGGAACAGCGCCATCGCCGCCGGTGTATGCGGGGCCCTTTTCATTGGGTACTGCATCTACTTCGAC
CGCAAAAGACGAAGTGACCCCAACTTCAAGAACAGGCTTCGAGAACGAAGAAAGAAACAGAAGCTTGCCAAGGAG
AGAGCTGGGCTTTCCAAGTTACCTGACCTTAAAGATGCTGAAGCTGTTCAGAAATTCTTCATGGCCTCAAACGAT
TATACCCAACAAGCAACCCAAAGCTATGGGGCCTACCCCACCCAGCCCGGGCAGGGCTATTCCCAGCAGAGCAGT
CAGCCCTACGGACAGCAGAGTTACAGTGGTTATAGCCAGTCCACGGACACTTCAGGATATGGCCAGAGCAGCTAT
TCTTCTTATGGCCAGAGCCAGAACACAGGCTATGGAACTCAGTCAACTCCCCAGGGATATGGCTCGACTGGCGGC
TATGGCAGTAGCCAGAGCTCCCAATCGTCTTACGGGCAGCAGTCCTCCTACCCTGGCTATGGCCAGCAGCCAGCT
CCCAGCAACACCTCGGGAAGTTACGGTAGCAGTTCTCAGAGCAGCAGCTATGGGCAGCCCCAGAGTGGGAGCTAC
AGCCAGCAGCCTAGCTATGGTGGACAGCAGCAAAGCTATGGACAGCAGCAAAGCTATAATCCCCCTCAGGGCTAT
GGACAGCAGAACCAGTACAACAGCAGCAGTGGTGGTGGAGGTGGAGGTGGAGGTGGAGGTAACTATGGCCAAGAT
CAATCCTCCATGAGTAGTGGTGGTGGCAGTGGTGGCGGTTATGGCAATCAAGACCAGAGTGGTGGAGGTGGCAGC
GGTGGCTATGGACAGCAGGACCGTGGAGGCCGCGGCAGGGGTGGCAGTGGTGGCGGCGGCGGCGGCGGCGGTGGT
GGTTACAACCGCAGCAGTGGTGGCTATGAACCCAGAGGTCGTGGAGGTGGCCGTGGAGGCAGAGGTGGCATGGGC
GGAAGTGACCGTGGTGGCTTCAATAAATTTGGTGGCCCTCGGGACCAAGGATCACGTCATGACTCCGAACAGGAT
AATTCAGACAACAACACCATCTTTGTGCAAGGCCTGGGTGAGAATGTTACAATTGAGTCTGTGGCTGATTACTTC
AAGCAGATTGGTATTATTAAGACAAACAAGAAAACGGGACAGCCCATGATTAATTTGTACACAGACAGGGAAACT
GGCAAGCTGAAGGGAGAGGCAACGGTCTCTTTTGATGACCCACCTTCAGCTAAAGCAGCTATTGACTGGTTTGAT
GGTAAAGAATTCTCCGGAAATCCTATCAAGGTCTCATTTGCTACTCGCCGGGCAGACTTTAATCGGGGTGGTGGC
AATGGTCGTGGAGGCCGAGGGCGAGGAGGACCCATGGGCCGTGGAGGCTATGGAGGTGGTGGCAGTGGTGGTGGT
GGCCGAGGAGGATTTCCCAGTGGAGGTGGTGGCGGTGGAGGACAGCAGCGAGCTGGTGACTGGAAGTGTCCTAAT
CCCACCTGTGAGAATATGAACTTCTCTTGGAGGAATGAATGCAACCAGTGTAAGGCCCCTAAACCAGATGGCCCA
GGAGGGGGACCAGGTGGCTCTCACATGGGGGGTAACTACGGGGATGATCGTCGTGGTGGCAGAGGAGGCGCGATC
GCAGGCAAGCCTATTCCCAACCCCCTGCTGGGCCTGGATAGCACCGGAGCACCAGGAAGTGCTGGTTCTGCTGCT
GGTAGTGGAGCGTGCCCGGTGCCGCTGCAGCTGCCGCCGCTGGAACGCCTGACCCTGGATGATAAAAAACCGCTG
AATACCCTGATCTCTGCTACTGGTCTGTGGATGAGTCGTACCGGAACCATTCATAAAATCAAACACCACGAGGTT
AGCCGTTCGAAAATCTATATTGAGATGGCGTGTGGCGATCATCTGGTTGTGAACAATAGCCGCTCTTCTCGTACA
GCACGTGCACTGCGTCACCACAAATATCGTAAAACCTGTAAACGTTGCCGTGTGTCCGATGAGGATCTGAACAAA
TTCCTGACAAAAGCCAATGAGGACCAAACAAGCGTGAAAGTGAAAGTCGTTAGCGCTCCTACCCGTACTAAAAAA
GCAATGCCGAAATCCGTTGCTCGTGCCCCTAAACCACTGGAAAACACTGAAGCAGCACAGGCACAGCCGTCTGGA
AGCAAATTCTCTCCGGCCATTCCTGTTTCTACCCAGGAGTCCGTTTCTGTTCCAGCAAGTGTGAGCACCAGCATT
AGCAGTATTAGCACCGGTGCCACCGCTAGCGCCCTGGTTAAAGGCAATACCAATCCGATTACAAGCATGTCTGCC
CCGGTTCAAGCATCAGCTCCAGCACTGACAAAATCCCAAACCGATCGTCTGGAGGTTCTGCTGAATCCGAAAGAC
GAAATCAGCCTGAATTCCGGCAAACCGTTTCGTGAACTGGAGAGCGAACTGCTGTCACGTCGTAAAAAAGACCTG
CAACAAATCTATGCCGAAGAACGTGAGAACTATCTGGGGAAACTGGAACGTGAAATCACCCGCTTTTTCGTGGAT
CGTGGCTTTCTGGAGATCAAATCCCCGATTCTGATTCCTCTGGAGTATATCGAGCGTATGGGCATCGACAATGAT
ACCGAACTGAGCAAACAAATTTTCCGTGTGGATAAAAACTTCTGTCTGCGCCCTATGCTAGCACCAAATCTGGCT
AACTATCTGCGCAAACTGGACCGTGCCCTGCCTGATCCTATCAAAATCTTCGAGATCGGCCCGTGTTATCGTAAA
GAGTCCGACGGTAAAGAACATCTGGAGGAGTTTACCATGCTGGCCTTTGCCCAAATGGGTTCAGGTTGTACTCGT
GAGAACCTGGAAAGCATCATCACCGATTTTCTGAACCACCTGGGCATTGACTTCAAAATTGTGGGCGACAGCTGT
ATGGTGTTTGGCGACACCCTGGATGTCATGCACGGCGACCTGGAACTGTCTAGTGCCGTTGTGGGCCCAATCCCG
CTGGATCGTGAGTGGGGTATCGACAAACCTTGGATCGGTGCGGGTTTTGGTCTGGAGCGTCTGCTGAAAGTAAAA
CACGACTTCAAGAACATCAAACGTGCTGCACGTTCCGAGTCCTATTACAATGGTATTTCTACTAACCTGTAA
(SEQ ID NO:109)
Protein:
MVGRNSAIAAGVCGALFIGYCIYFDRKRRSDPNFKNRLRERRKKQKLAKERAGLSKLPDLKDAEAVQKFFMASND
YTQQATQSYGAYPTQPGQGYSQQSSQPYGQQSYSGYSQSTDTSGYGQSSYSSYGQSQNTGYGTQSTPQGYGSTGG
YGSSQSSQSSYGQQSSYPGYGQQPAPSNTSGSYGSSSQSSSYGQPQSGSYSQQPSYGGQQQSYGQQQSYNPPQGY
GQQNQYNSSSGGGGGGGGGGNYGQDQSSMSSGGGSGGGYGNQDQSGGGGSGGYGQQDRGGRGRGGSGGGGGGGGG
GYNRSSGGYEPRGRGGGRGGRGGMGGSDRGGFNKFGGPRDQGSRHDSEQDNSDNNTIFVQGLGENVTIESVADYF
KQIGIIKTNKKTGQPMINLYTDRETGKLKGEATVSFDDPPSAKAAIDWFDGKEFSGNPIKVSFATRRADFNRGGG
NGRGGRGRGGPMGRGGYGGGGSGGGGRGGFPSGGGGGGGQQRAGDWKCPNPTCENMNFSWRNECNQCKAPKPDGP
GGGPGGSHMGGNYGDDRRGGRGGAIAGKPIPNPLLGLDSTGAPGSAGSAAGSGACPVPLQLPPLERLTLDDKKPL
NTLISATGLWMSRTGTIHKIKHHEVSRSKIYIEMACGDHLVVNNSRSSRTARALRHHKYRKTCKRCRVSDEDLNK

FLTKANEDQTSVKVKVVSAPTRTKKAMPKSVARAPKPLENTEAAQAQPSGSKFSPAIPVSTQESVSVPASVSTSI
SSISTGATASALVKGNTNPITSMSAPVQASAPALTKSQTDRLEVLLNPKDEISLNSGKPFRELESELLSRRKKDL
QQIYAEERENYLGKLEREITRFFVDRGFLEIKSPILIPLEYIERMGIDNDTELSKQIFRVDKNFCLRPMLAPNLA
NYLRKLDRALPDPIKIFEIGPCYRKESDGKEHLEEFTMLAFAQMGSGCTRENLESIITDFLNHLGIDFKIVGDSC
MVFGDTLDVMHGDLELSSAVVGPIPLDREWGIDKPWIGAGFGLERLLKVKHDFKNIKRAARSESYYNGISTNL
(SEQ ID NO:110)

TOMM20-EWSR1-λN22
DNA:
ATGGTGGGTCGGAACAGCGCCATCGCCGCCGGTGTATGCGGGGCCCTTTTCATTGGGTACTGCATCTACTTCGAC
CGCAAAAGACGAAGTGACCCCAACTTCAAGAACAGGCTTCGAGAACGAAGAAAGAAACAGAAGCTTGCCAAGGAG
AGAGCTGGGCTTTCCAAGTTACCTGACCTTAAAGATGCTGAAGCTGTTCAGAAGTTCTTCATGGCGTCCACGGAT
TACAGTACCTATAGCCAAGCTGCAGCGCAGCAGGGCTACAGTGCTTACACCGCCCAGCCCACTCAAGGATATGCA
CAGACCACCCAGGCATATGGGCAACAAAGCTATGGAACCTATGGACAGCCCACTGATGTCAGCTATACCCAGGCT
CAGACCACTGCAACCTATGGGCAGACCGCCTATGCAACTTCTTATGGACAGCCTCCCACTGGTTATACTACTCCA
ACTGCCCCCCAGGCATACAGCCAGCCTGTCCAGGGGTATGGCACTGGTGCTTATGATACCACCACTGCTACAGTC
ACCACCACCCAGGCCTCCTATGCAGCTCAGTCTGCATATGGCACTCAGCCTGCTTATCCAGCCTATGGGCAGCAG
CCAGCAGCCACTGCACCTACAAGACCGCAGGATGGAAACAAGCCCACTGAGACTAGTCAACCTCAATCTAGCACA
GGGGGTTACAACCAGCCCAGCCTAGGATATGGACAGAGTAACTACAGTTATCCCCAGGTACCTGGGAGCTACCCC
ATGCAGCCAGTCACTGCACCTCCATCCTACCCTCCTACCAGCTATTCCTCTACACAGCCGACTAGTTATGATCAG
AGCAGTTACTCTCAGCAGAACACCTATGGGCAACCGAGCAGCTATGGACAGCAGAGTAGCTATGGTCAACAAAGC
AGCTATGGGCAGCAGCCTCCCACTAGTTACCCACCCCAAACTGGATCCTACAGCCAAGCTCCAAGTCAATATAGC
CAACAGAGCAGCAGCTACGGGCAGCAGAGTTCATTCCGACAGGACCACCCCAGTAGCATGGGTGTTTATGGGCAG
GAGTCTGGAGGATTTTCCGGACCAGGAGAGAACCGGAGCATGAGTGGCCCTGATAACCGGGGCAGGGGAAGAGGG
GGATTTGATCGTGGAGGCATGAGCAGAGGTGGGCGGGGAGGAGGACGCGGTGGAATGGGCAGCGCTGGAGAGCGA
GGTGGCTTCAATAAGCCTGGTGGACCCATGGATGAAGGACCAGATCTTGATCTAGGCCCACCTGTAGATCCAGAT
GAAGACTCTGACAACAGTGCAATTTATGTACAAGGATTAAATGACAGTGTGACTCTAGATGATCTGGCAGACTTC
TTTAAGCAGTGTGGGGTTGTTAAGATGAACAAGAGAACTGGGCAACCCATGATCCACATCTACCTGGACAAGGAA
ACAGGAAAGCCCAAAGGCGATGCCACAGTGTCCTATGAAGACCCACCTACTGCCAAGGCTGCCGTGGAATGGTTT
GATGGGAAAGATTTTCAAGGGAGCAAACTTAAAGTCTCCCTTGCTCGGAAGAAGCCTCCAATGAACAGTATGCGG
GGTGGTCTGCCACCCCGTGAGGGCAGAGGCATGCCACCACCACTCCGTGGAGGTCCAGGAGGCCCAGGAGGTCCT
GGGGGACCCATGGGTCGCATGGGAGGCCGTGGAGGAGATAGAGGAGGCTTCCCTCCAAGAGGACCCCGGGGTTCC
CGAGGGAACCCCTCTGGAGGAGGAAACGTCCAGCACCGAGCTGGAGACTGGCAGTGTCCCAATCCGGGTTGTGGA
AACCAGAACTTCGCCTGGAGAACAGAGTGCAACCAGTGTAAGGCCCCAAAGCCTGAAGGCTTCCTCCCGCCACCC
TTTCCGCCCCCGGGTGGTGATCGTGGCAGAGGTGGCCCTGGTGGCATGCGGGGAGGAAGAGGTGGCCTCATGGAT
CGTGGTGGTCCCGGTGGAATGTTCAGAGGTGGCCGTGGTGGAGACAGAGGTGGCTTCCGTGGTGGCCGGGGCATG
GACCGAGGTGGCTTTGGTGGAGGAAGACGAGGTGGCCCTGGGGGGCCCCCTGGACCTTTGATGGAACAGGCGATC
GCAGGAGCACCAGGAAGTGCTGGTTCTGCTGCTGGTAGTGGAGAGCAGAAGCTGATCTCAGAGGAGGACCTGCTA
GCCACCATGGACGCACAAACACGACGACGTGAGCGTCGCGCTGAGAAACAAGCTCAATGGAAAGCTGCAAACCCA
CCGCTCGACGGAGCCGGAGCTGGCGCTGGAGCTGGAGCCGGAGCTGGCGGTCTAGCCACCATGGACGCACAAACA
CGACGACGTGAGCGTCGCGCTGAGAAACAAGCTCAATGGAAAGCTGCAAACCCACCGCTCGACGGAGCCGGAGCT
GGCGCTGGAGCTGGAGCCGGAGCTGGCGGTCTAGCCACCATGGACGCACAAACACGACGACGTGAGCGTCGCGCT
GAGAAACAAGCTCAATGGAAAGCTGCAAACCCACCGCTCGACGGAGCCGGAGCTGGCGCTGGAGCTGGAGCCGGA
GCTGGCGGTCTAGCCACCATGGACGCACAAACACGACGACGTGAGCGTCGCGCTGAGAAACAAGCTCAATGGAAA
GCTGCAAACCCACCGCTCGAGTCTAGAGGGCCCGTTTAA (SEQ ID NO:111)
Protein:
MVGRNSAIAAGVCGALFIGYCIYFDRKRRSDPNFKNRLRERRKKQKLAKERAGLSKLPDLKDAEAVQKFFMASTD
YSTYSQAAAQQGYSAYTAQPTQGYAQTTQAYGQQSYGTYGQPTDVSYTQAQTTATYGQTAYATSYGQPPTGYTTP
TAPQAYSQPVQGYGTGAYDTTTATVTTTQASYAAQSAYGTQPAYPAYGQQPAATAPTRPQDGNKPTETSQPQSST
GGYNQPSLGYGQSNYSYPQVPGSYPMQPVTAPPSYPPTSYSSTQPTSYDQSSYSQQNTYGQPSSYGQQSSYGQQS
SYGQQPPTSYPPQTGSYSQAPSQYSQQSSSYGQQSSFRQDHPSSMGVYGQESGGFSGPGENRSMSGPDNRGRGRG
GFDRGGMSRGGRGGGRGGMGSAGERGGFNKPGGPMDEGPDLDLGPPVDPDEDSDNSAIYVQGLNDSVTLDDLADF
FKQCGVVKMNKRTGQPMIHIYLDKETGKPKGDATVSYEDPPTAKAAVEWFDGKDFQGSKLKVSLARKKPPMNSMR
GGLPPREGRGMPPPLRGGPGGPGGPGGPMGRMGGRGGDRGGFPPRGPRGSRGNPSGGGNVQHRAGDWQCPNPGCG
NQNFAWRTECNQCKAPKPEGFLPPPFPPPGGDRGRGGPGGMRGGRGGLMDRGGPGGMFRGGRGGDRGGFRGGRGM
DRGGFGGGRRGGPGGPPGPLMEQAIAGAPGSAGSAAGSGEQKLISEEDLLATMDAQTRRRERRAEKQAQWKAANP
PLDGAGAGAGAGAGAGGLATMDAQTRRRERRAEKQAQWKAANPPLDGAGAGAGAGAGAGGLATMDAQTRRRERRA

EKQAQWKAANPPLDGAGAGAGAGAGAGGLATMDAQTRRRERRAEKQAQWKAANPPLESRGPV (SEQ ID NO:112)

TOMM20-EWSR1-Myc-λN22
DNA:
ATGGTGGGTCGGAACAGCGCCATCGCCGCCGGTGTATGCGGGGCCCTTTTCATTGGGTACTGCATCTACTTCGAC
CGCAAAAGACGAAGTGACCCCAACTTCAAGAACAGGCTTCGAGAACGAAGAAAGAAACAGAAGCTTGCCAAGGAG
AGAGCTGGGCTTTCCAAGTTACCTGACCTTAAAGATGCTGAAGCTGTTCAGAAGTTCTTCATGGCGTCCACGGAT
TACAGTACCTATAGCCAAGCTGCAGCGCAGCAGGGCTACAGTGCTTACACCGCCCAGCCCACTCAAGGATATGCA
CAGACCACCCAGGCATATGGGCAACAAAGCTATGGAACCTATGGACAGCCCACTGATGTCAGCTATACCCAGGCT
CAGACCACTGCAACCTATGGGCAGACCGCCTATGCAACTTCTTATGGACAGCCTCCCACTGGTTATACTACTCCA
ACTGCCCCCCAGGCATACAGCCAGCCTGTCCAGGGGTATGGCACTGGTGCTTATGATACCACCACTGCTACAGTC
ACCACCACCCAGGCCTCCTATGCAGCTCAGTCTGCATATGGCACTCAGCCTGCTTATCCAGCCTATGGGCAGCAG
CCAGCAGCCACTGCACCTACAAGACCGCAGGATGGAAACAAGCCCACTGAGACTAGTCAACCTCAATCTAGCACA
GGGGGTTACAACCAGCCCAGCCTAGGATATGGACAGAGTAACTACAGTTATCCCCAGGTACCTGGGAGCTACCCC
ATGCAGCCAGTCACTGCACCTCCATCCTACCCTCCTACCAGCTATTCCTCTACACAGCCGACTAGTTATGATCAG
AGCAGTTACTCTCAGCAGAACACCTATGGGCAACCGAGCAGCTATGGACAGCAGAGTAGCTATGGTCAACAAAGC
AGCTATGGGCAGCAGCCTCCCACTAGTTACCCACCCCAAACTGGATCCTACAGCCAAGCTCCAAGTCAATATAGC
CAACAGAGCAGCAGCTACGGGCAGCAGAGTTCATTCCGACAGGACCACCCCAGTAGCATGGGTGTTTATGGGCAG
GAGTCTGGAGGATTTTCCGGACCAGGAGAGAACCGGAGCATGAGTGGCCCTGATAACCGGGGCAGGGGAAGAGGG
GGATTTGATCGTGGAGGCATGAGCAGAGGTGGGCGGGGAGGAGGACGCGGTGGAATGGGCAGCGCTGGAGAGCGA
GGTGGCTTCAATAAGCCTGGTGGACCCATGGATGAAGGACCAGATCTTGATCTAGGCCCACCTGTAGATCCAGAT
GAAGACTCTGACAACAGTGCAATTTATGTACAAGGATTAAATGACAGTGTGACTCTAGATGATCTGGCAGACTTC
TTTAAGCAGTGTGGGGTTGTTAAGATGAACAAGAGAACTGGGCAACCCATGATCCACATCTACCTGGACAAGGAA
ACAGGAAAGCCCAAAGGCGATGCCACAGTGTCCTATGAAGACCCACCTACTGCCAAGGCTGCCGTGGAATGGTTT
GATGGGAAAGATTTTCAAGGGAGCAAACTTAAAGTCTCCCTTGCTCGGAAGAAGCCTCCAATGAACAGTATGCGG
GGTGGTCTGCCACCCCGTGAGGGCAGAGGCATGCCACCACCACTCCGTGGAGGTCCAGGAGGCCCAGGAGGTCCT
GGGGGACCCATGGGTCGCATGGGAGGCCGTGGAGGAGATAGAGGAGGCTTCCCTCCAAGAGGACCCCGGGGTTCC
CGAGGGAACCCCTCTGGAGGAGGAAACGTCCAGCACCGAGCTGGAGACTGGCAGTGTCCCAATCCGGGTTGTGGA
AACCAGAACTTCGCCTGGAGAACAGAGTGCAACCAGTGTAAGGCCCCAAAGCCTGAAGGCTTCCTCCCGCCACCC
TTTCCGCCCCCGGGTGGTGATCGTGGCAGAGGTGGCCCTGGTGGCATGCGGGGAGGAAGAGGTGGCCTCATGGAT
CGTGGTGGTCCCGGTGGAATGTTCAGAGGTGGCCGTGGTGGAGACAGAGGTGGCTTCCGTGGTGGCCGGGGCATG
GACCGAGGTGGCTTTGGTGGAGGAAGACGAGGTGGCCCTGGGGGGCCCCCTGGACCTTTGATGGAACAGGCGATC
GCAGGAGCACCAGGAAGTGCTGGTTCTGCTGCTGGTAGTGGAGAGCAGAAGCTGATCTCAGAGGAGGACCTGCTA
GCCACCATGGACGCACAAACACGACGACGTGAGCGTCGCGCTGAGAAACAAGCTCAATGGAAAGCTGCAAACCCA
CCGCTCGACGGAGCCGGAGCTGGCGCTGGAGCTGGAGCCGGAGCTGGCGGTCTAGCCACCATGGACGCACAAACA
CGACGACGTGAGCGTCGCGCTGAGAAACAAGCTCAATGGAAAGCTGCAAACCCACCGCTCGACGGAGCCGGAGCT
GGCGCTGGAGCTGGAGCCGGAGCTGGCGGTCTAGCCACCATGGACGCACAAACACGACGACGTGAGCGTCGCGCT
GAGAAACAAGCTCAATGGAAAGCTGCAAACCCACCGCTCGACGGAGCCGGAGCTGGCGCTGGAGCTGGAGCCGGA
GCTGGCGGTCTAGCCACCATGGACGCACAAACACGACGACGTGAGCGTCGCGCTGAGAAACAAGCTCAATGGAAA
GCTGCAAACCCACCGCTCGAGTCTAGAGGGCCCGTTTAA (SEQ ID NO:113)
Protein:
MVGRNSAIAAGVCGALFIGYCIYFDRKRRSDPNFKNRLRERRKKQKLAKERAGLSKLPDLKDAEAVQKFFMASTD
YSTYSQAAAQQGYSAYTAQPTQGYAQTTQAYGQQSYGTYGQPTDVSYTQAQTTATYGQTAYATSYGQPPTGYTTP
TAPQAYSQPVQGYGTGAYDTTTATVTTTQASYAAQSAYGTQPAYPAYGQQPAATAPTRPQDGNKPTETSQPQSST
GGYNQPSLGYGQSNYSYPQVPGSYPMQPVTAPPSYPPTSYSSTQPTSYDQSSYSQQNTYGQPSSYGQQSSYGQQS
SYGQQPPTSYPPQTGSYSQAPSQYSQQSSSYGQQSSFRQDHPSSMGVYGQESGGFSGPGENRSMSGPDNRGRGRG
GFDRGGMSRGGRGGGRGGMGSAGERGGFNKPGGPMDEGPDLDLGPPVDPDEDSDNSAIYVQGLNDSVTLDDLADF
FKQCGVVKMNKRTGQPMIHIYLDKETGKPKGDATVSYEDPPTAKAAVEWFDGKDFQGSKLKVSLARKKPPMNSMR
GGLPPREGRGMPPPLRGGPGGPGGPGGPMGRMGGRGGDRGGFPPRGPRGSRGNPSGGGNVQHRAGDWQCPNPGCG
NQNFAWRTECNQCKAPKPEGFLPPPFPPPGGDRGRGGPGGMRGGRGGLMDRGGPGGMFRGGRGGDRGGFRGGRGM
DRGGFGGGRRGGPGGPPGPLMEQAIAGAPGSAGSAAGSGEQKLISEEDLLATMDAQTRRRERRAEKQAQWKAANP
PLDGAGAGAGAGAGAGGLATMDAQTRRRERRAEKQAQWKAANPPLDGAGAGAGAGAGAGGLATMDAQTRRRERRA
EKQAQWKAANPPLDGAGAGAGAGAGAGGLATMDAQTRRRERRAEKQAQWKAANPPLESRGPV (SEQ ID NO:114)

TOMM20-3xMCP-PylRSAF
DNA:

ATGGTGGGTCGGAACAGCGCCATCGCCGCCGGTGTATGCGGGGCCCTTTTCATTGGGTACTGCATCTACTTCGAC
CGCAAAAGACGAAGTGACCCCAACTTCAAGAACAGGCTTCGAGAACGAAGAAAGAAACAGAAGCTTGCCAAGGAG
AGAGCTGGGCTTTCCAAGTTACCTGACCTTAAAGATGCTGAAGCTGTTCAGAAGTTCTTCTATACAGATATTGAA
ATGAACAGATTGGGAAAGGAGCAGAAGCTGATCTCAGAGGAGGACCTGGGCGCCCCCGGCTCCGCCGGCTCCGCC
GCCGGCTCCGGCGCTTCTAACTTTACTCAGTTCGTTCTCGTCGACAATGGCGGAACTGGCGACGTGACTGTCGCC
CCAAGCAACTTCGCTAACGGGATCGCTGAATGGATCAGCTCTAACTCGCGTTCACAGGCTTACAAAGTAACCTGT
AGCGTTCGTCAGAGCTCTGCGCAGAATCGCAAATACACCATCAAAGTCGAGGTGCCTAAAGGCGCCTGGCGTTCG
TACTTAAATATGGAACTAACCATTCCAATTTTCGCCACGAATTCCGACTGCGAGCTTATTGTTAAGGCAATGCAA
GGTCTCCTAAAAGATGGAAACCCGATTCCCTCAGCAATCGCAGCAAACTCCGGCATCTACGGTGCCCCAGGCTCC
GCAGGAAGCGCAGCGGGGTCCGGAGCTTCTAACTTTACTCAGTTCGTTCTCGTCGACAATGGCGGAACTGGCGAC
GTGACTGTCGCCCCAAGCAACTTCGCTAACGGGATCGCTGAATGGATCAGCTCTAACTCGCGTTCACAGGCTTAC
AAAGTAACCTGTAGCGTTCGTCAGAGCTCTGCGCAGAATCGCAAATACACCATCAAAGTCGAGGTGCCTAAAGGC
GCCTGGCGTTCGTACTTAAATATGGAACTAACCATTCCAATTTTCGCCACGAATTCCGACTGCGAGCTTATTGTT
AAGGCAATGCAAGGTCTCCTAAAAGATGGAAACCCGATTCCCTCAGCAATCGCAGCAAACTCCGGCATCTACGGC
GCACCTGGTAGTGCTGGTTCTGCTGCTGGATCAGGTGCTTCTAACTTTACTCAGTTCGTTCTCGTCGACAATGGC
GGAACTGGCGACGTGACTGTCGCCCCAAGCAACTTCGCTAACGGGATCGCTGAATGGATCAGCTCTAACTCGCGT
TCACAGGCTTACAAAGTAACCTGTAGCGTTCGTCAGAGCTCTGCGCAGAATCGCAAATACACCATCAAAGTCGAG
GTGCCTAAAGGCGCCTGGCGTTCGTACTTAAATATGGAACTAACCATTCCAATTTTCGCCACGAATTCCGACTGC
GAGCTTATTGTTAAGGCAATGCAAGGTCTCCTAAAAGATGGAAACCCGATTCCCTCAGCAATCGCAGCAAACTCC
GGCATCTACGGTACCGGCGCCCCCGGCTCCGCCGGCTCCGCCGCCGGCTCCGGCGCGTGCCCGGTGCCGCTGCAG
CTGCCGCCGCTGGAACGCCTGACCCTGGATGATAAAAAACCGCTGAATACCCTGATCTCTGCTACTGGTCTGTGG
ATGAGTCGTACCGGAACCATTCATAAAATCAAACACCACGAGGTTAGCCGTTCGAAAATCTATATTGAGATGGCG
TGTGGCGATCATCTGGTTGTGAACAATAGCCGCTCTTCTCGTACAGCACGTGCACTGCGTCACCACAAATATCGT
AAAACCTGTAAACGTTGCCGTGTGTCCGATGAGGATCTGAACAAATTCCTGACAAAAGCCAATGAGGACCAAACA
AGCGTGAAAGTGAAAGTCGTTAGCGCTCCTACCCGTACTAAAAAAGCAATGCCGAAATCCGTTGCTCGTGCCCCT
AAACCACTGGAAAACACTGAAGCAGCACAGGCACAGCCGTCTGGAAGCAAATTCTCTCCGGCCATTCCTGTTTCT
ACCCAGGAGTCCGTTTCTGTTCCAGCAAGTGTGAGCACCAGCATTAGCAGTATTAGCACCGGTGCCACCGCTAGC
GCCCTGGTTAAAGGCAATACCAATCCGATTACAAGCATGTCTGCCCCGGTTCAAGCATCAGCTCCAGCACTGACA
AAATCCCAAACCGATCGTCTGGAGGTTCTGCTGAATCCGAAAGACGAAATCAGCCTGAATTCCGGCAAACCGTTT
CGTGAACTGGAGAGCGAACTGCTGTCACGTCGTAAAAAAGACCTGCAACAAATCTATGCCGAAGAACGTGAGAAC
TATCTGGGGAAACTGGAACGTGAAATCACCCGCTTTTTCGTGGATCGTGGCTTTCTGGAGATCAAATCCCCGATT
CTGATTCCTCTGGAGTATATCGAGCGTATGGGCATCGACAATGATACCGAACTGAGCAAACAAATTTTCCGTGTG
GATAAAAACTTCTGTCTGCGCCCTATGCTAGCACCAAATCTGGCTAACTATCTGCGCAAACTGGACCGTGCCCTG
CCTGATCCTATCAAAATCTTCGAGATCGGCCCGTGTTATCGTAAAGAGTCCGACGGTAAAGAACATCTGGAGGAG
TTTACCATGCTGAACTTTTGCCAAATGGGTTCAGGTTGTACTCGTGAGAACCTGGAAAGCATCATCACCGATTTT
CTGAACCACCTGGGCATTGACTTCAAAATTGTGGGCGACAGCTGTATGGTGTTTGGCGACACCCTGGATGTCATG
CACGGCGACCTGGAACTGTCTAGTGCCGTTGTGGGCCCAATCCCGCTGGATCGTGAGTGGGGTATCGACAAACCT
TGGATCGGTGCGGGTTTTGGTCTGGAGCGTCTGCTGAAAGTAAAACACGACTTCAAGAACATCAAACGTGCTGCA
CGTTCCGAGTCCTATTACAATGGTATTTCTACTAACCTGTAA (SEQ ID NO:115)
Protein:
MVGRNSAIAAGVCGALFIGYCIYFDRKRRSDPNFKNRLRERRKKQKLAKERAGLSKLPDLKDAEAVQKFFYTDIE
MNRLGKEQKLISEEDLGAPGSAGSAAGSGASNFTQFVLVDNGGTGDVTVAPSNFANGIAEWISSNSRSQAYKVTC
SVRQSSAQNRKYTIKVEVPKGAWRSYLNMELTIPIFATNSDCELIVKAMQGLLKDGNPIPSAIAANSGIYGAPGS
AGSAAGSGASNFTQFVLVDNGGTGDVTVAPSNFANGIAEWISSNSRSQAYKVTCSVRQSSAQNRKYTIKVEVPKG
AWRSYLNMELTIPIFATNSDCELIVKAMQGLLKDGNPIPSAIAANSGIYGAPGSAGSAAGSGASNFTQFVLVDNG
GTGDVTVAPSNFANGIAEWISSNSRSQAYKVTCSVRQSSAQNRKYTIKVEVPKGAWRSYLNMELTIPIFATNSDC
ELIVKAMQGLLKDGNPIPSAIAANSGIYGTGAPGSAGSAAGSGACPVPLQLPPLERLTLDDKKPLNTLISATGLW
MSRTGTIHKIKHHEVSRSKIYIEMACGDHLVVNNSRSSRTARALRHHKYRKTCKRCRVSDEDLNKFLTKANEDQT
SVKVKVVSAPTRTKKAMPKSVARAPKPLENTEAAQAQPSGSKFSPAIPVSTQESVSVPASVSTSISSISTGATAS
ALVKGNTNPITSMSAPVQASAPALTKSQTDRLEVLLNPKDEISLNSGKPFRELESELLSRRKKDLQQIYAEEREN
YLGKLEREITRFFVDRGFLEIKSPILIPLEYIERMGIDNDTELSKQIFRVDKNFCLRPMLAPNLANYLRKLDRAL
PDPIKIFEIGPCYRKESDGKEHLEEFTMLNFCQMGSGCTRENLESIITDFLNHLGIDFKIVGDSCMVFGDTLDVM
HGDLELSSAVVGPIPLDREWGIDKPWIGAGFGLERLLKVKHDFKNIKRAARSESYYNGISTNL (SEQ ID
NO:116)

TOMM20-FUS-3xMCP-PylRS^AF
DNA:

ATGGTGGGTCGGAACAGCGCCATCGCCGCCGGTGTATGCGGGGCCCTTTTCATTGGGTACTGCATCTACTTCGAC
CGCAAAAGACGAAGTGACCCCAACTTCAAGAACAGGCTTCGAGAACGAAGAAAGAAACAGAAGCTTGCCAAGGAG
AGAGCTGGGCTTTCCAAGTTACCTGACCTTAAAGATGCTGAAGCTGTTCAGAAATTCTTCATGGCCTCAAACGAT
TATACCCAACAAGCAACCCAAAGCTATGGGGCCTACCCCACCCAGCCCGGGCAGGGCTATTCCCAGCAGAGCAGT
CAGCCCTACGGACAGCAGAGTTACAGTGGTTATAGCCAGTCCACGGACACTTCAGGATATGGCCAGAGCAGCTAT
TCTTCTTATGGCCAGAGCCAGAACACAGGCTATGGAACTCAGTCAACTCCCCAGGGATATGGCTCGACTGGCGGC
TATGGCAGTAGCCAGAGCTCCCAATCGTCTTACGGGCAGCAGTCCTCCTACCCTGGCTATGGCCAGCAGCCAGCT
CCCAGCAACACCTCGGGAAGTTACGGTAGCAGTTCTCAGAGCAGCAGCTATGGGCAGCCCCAGAGTGGGAGCTAC
AGCCAGCAGCCTAGCTATGGTGGACAGCAGCAAAGCTATGGACAGCAGCAAAGCTATAATCCCCCTCAGGGCTAT
GGACAGCAGAACCAGTACAACAGCAGCAGTGGTGGTGGAGGTGGAGGTGGAGGTGGAGGTAACTATGGCCAAGAT
CAATCCTCCATGAGTAGTGGTGGTGGCAGTGGTGGCGGTTATGGCAATCAAGACCAGAGTGGTGGAGGTGGCAGC
GGTGGCTATGGACAGCAGGACCGTGGAGGCCGCGGCAGGGGTGGCAGTGGTGGCGGCGGCGGCGGCGGCGGTGGT
GGTTACAACCGCAGCAGTGGTGGCTATGAACCCAGAGGTCGTGGAGGTGGCCGTGGAGGCAGAGGTGGCATGGGC
GGAAGTGACCGTGGTGGCTTCAATAAATTTGGTGGCCCTCGGGACCAAGGATCACGTCATGACTCCGAACAGGAT
AATTCAGACAACAACACCATCTTTGTGCAAGGCCTGGGTGAGAATGTTACAATTGAGTCTGTGGCTGATTACTTC
AAGCAGATTGGTATTATTAAGACAAACAAGAAAACGGGACAGCCCATGATTAATTTGTACACAGACAGGGAAACT
GGCAAGCTGAAGGGAGAGGCAACGGTCTCTTTTGATGACCCACCTTCAGCTAAAGCAGCTATTGACTGGTTTGAT
GGTAAAGAATTCTCCGGAAATCCTATCAAGGTCTCATTTGCTACTCGCCGGGCAGACTTTAATCGGGGTGGTGGC
AATGGTCGTGGAGGCCGAGGGCGAGGAGGACCCATGGGCCGTGGAGGCTATGGAGGTGGTGGCAGTGGTGGTGGT
GGCCGAGGAGGATTTCCCAGTGGAGGTGGTGGCGGTGGAGGACAGCAGCGAGCTGGTGACTGGAAGTGTCCTAAT
CCCACCTGTGAGAATATGAACTTCTCTTGGAGGAATGAATGCAACCAGTGTAAGGCCCCTAAACCAGATGGCCCA
GGAGGGGGACCAGGTGGCTCTCACATGGGGGGTAACTACGGGGATGATCGTCGTGGTGGCAGAGGAGGCGAGCAG
AAGCTGATCTCAGAGGAGGACCTGGGCGCCCCCGGCTCCGCCGGCTCCGCCGCCGGCTCCGGCGCTTCTAACTTT
ACTCAGTTCGTTCTCGTCGACAATGGCGGAACTGGCGACGTGACTGTCGCCCCAAGCAACTTCGCTAACGGGATC
GCTGAATGGATCAGCTCTAACTCGCGTTCACAGGCTTACAAAGTAACCTGTAGCGTTCGTCAGAGCTCTGCGCAG
AATCGCAAATACACCATCAAAGTCGAGGTGCCTAAAGGCGCCTGGCGTTCGTACTTAAATATGGAACTAACCATT
CCAATTTTCGCCACGAATTCCGACTGCGAGCTTATTGTTAAGGCAATGCAAGGTCTCCTAAAAGATGGAAACCCG
ATTCCCTCAGCAATCGCAGCAAACTCCGGCATCTACGGTGCCCCAGGCTCCGCAGGAAGCGCAGCGGGGTCCGGA
GCTTCTAACTTTACTCAGTTCGTTCTCGTCGACAATGGCGGAACTGGCGACGTGACTGTCGCCCCAAGCAACTTC
GCTAACGGGATCGCTGAATGGATCAGCTCTAACTCGCGTTCACAGGCTTACAAAGTAACCTGTAGCGTTCGTCAG
AGCTCTGCGCAGAATCGCAAATACACCATCAAAGTCGAGGTGCCTAAAGGCGCCTGGCGTTCGTACTTAAATATG
GAACTAACCATTCCAATTTTCGCCACGAATTCCGACTGCGAGCTTATTGTTAAGGCAATGCAAGGTCTCCTAAAA
GATGGAAACCCGATTCCCTCAGCAATCGCAGCAAACTCCGGCATCTACGGCGCACCTGGTAGTGCTGGTTCTGCT
GCTGGATCAGGTGCTTCTAACTTTACTCAGTTCGTTCTCGTCGACAATGGCGGAACTGGCGACGTGACTGTCGCC
CCAAGCAACTTCGCTAACGGGATCGCTGAATGGATCAGCTCTAACTCGCGTTCACAGGCTTACAAAGTAACCTGT
AGCGTTCGTCAGAGCTCTGCGCAGAATCGCAAATACACCATCAAAGTCGAGGTGCCTAAAGGCGCCTGGCGTTCG
TACTTAAATATGGAACTAACCATTCCAATTTTCGCCACGAATTCCGACTGCGAGCTTATTGTTAAGGCAATGCAA
GGTCTCCTAAAAGATGGAAACCCGATTCCCTCAGCAATCGCAGCAAACTCCGGCATCTACGGTACCGGCGCCCCC
GGCTCCGCCGGCTCCGCCGCCGGCTCCGGCGCGTGCCCGGTGCCGCTGCAGCTGCCGCCGCTGGAACGCCTGACC
CTGGATGATAAAAAACCGCTGAATACCCTGATCTCTGCTACTGGTCTGTGGATGAGTCGTACCGGAACCATTCAT
AAAATCAAACACCACGAGGTTAGCCGTTCGAAAATCTATATTGAGATGGCGTGTGGCGATCATCTGGTTGTGAAC
AATAGCCGCTCTTCTCGTACAGCACGTGCACTGCGTCACCACAAATATCGTAAAACCTGTAAACGTTGCCGTGTG
TCCGATGAGGATCTGAACAAATTCCTGACAAAAGCCAATGAGGACCAAACAAGCGTGAAAGTGAAAGTCGTTAGC
GCTCCTACCCGTACTAAAAAAGCAATGCCGAAATCCGTTGCTCGTGCCCCTAAACCACTGGAAAACACTGAAGCA
GCACAGGCACAGCCGTCTGGAAGCAAATTCTCTCCGGCCATTCCTGTTTCTACCCAGGAGTCCGTTTCTGTTCCA
GCAAGTGTGAGCACCAGCATTAGCAGTATTAGCACCGGTGCCACCGCTAGCGCCCTGGTTAAAGGCAATACCAAT
CCGATTACAAGCATGTCTGCCCCGGTTCAAGCATCAGCTCCAGCACTGACAAAATCCCAAACCGATCGTCTGGAG
GTTCTGCTGAATCCGAAAGACGAAATCAGCCTGAATTCCGGCAAACCGTTTCGTGAACTGGAGAGCGAACTGCTG
TCACGTCGTAAAAAAGACCTGCAACAAATCTATGCCGAAGAACGTGAGAACTATCTGGGGAAACTGGAACGTGAA
ATCACCCGCTTTTTCGTGGATCGTGGCTTTCTGGAGATCAAATCCCCGATTCTGATTCCTCTGGAGTATATCGAG
CGTATGGGCATCGACAATGATACCGAACTGAGCAAACAAATTTTCCGTGTGGATAAAAACTTCTGTCTGCGCCCT
ATGCTAGCACCAAATCTGGCTAACTATCTGCGCAAACTGGACCGTGCCCTGCCTGATCCTATCAAAATCTTCGAG
ATCGGCCCGTGTTATCGTAAAGAGTCCGACGGTAAAGAACATCTGGAGGAGTTTACCATGCTGAACTTTTGCCAA
ATGGGTTCAGGTTGTACTCGTGAGAACCTGGAAAGCATCATCACCGATTTTCTGAACCACCTGGGCATTGACTTC
AAAATTGTGGGCGACAGCTGTATGGTGTTTGGCGACACCCTGGATGTCATGCACGGCGACCTGGAACTGTCTAGT
GCCGTTGTGGGCCCAATCCCGCTGGATCGTGAGTGGGGTATCGACAAACCTTGGATCGGTGCGGGTTTTGGTCTG
GAGCGTCTGCTGAAAGTAAAACACGACTTCAAGAACATCAAACGTGCTGCACGTTCCGAGTCCTATTACAATGGT
ATTTCTACTAACCTGTAA (SEQ ID NO:117)

Protein:
MVGRNSAIAAGVCGALFIGYCIYFDRKRRSDPNFKNRLRERRKKQKLAKERAGLSKLPDLKDAEAVQKFFMASND
YTQQATQSYGAYPTQPGQGYSQQSSQPYGQQSYSGYSQSTDTSGYGQSSYSSYGQSQNTGYGTQSTPQGYGSTGG
YGSSQSSQSSYGQQSSYPGYGQQPAPSNTSGSYGSSSQSSSYGQPQSGSYSQQPSYGGQQQSYGQQQSYNPPQGY
GQQNQYNSSSGGGGGGGGGGNYGQDQSSMSSGGGSGGGYGNQDQSGGGGSGGYGQQDRGGRGRGGSGGGGGGGG
GYNRSSGGYEPRGRGGGRGGRGGMGGSDRGGFNKFGGPRDQGSRHDSEQDNSDNNTIFVQGLGENVTIESVADYF
KQIGIIKTNKKTGQPMINLYTDRETGKLKGEATVSFDDPPSAKAAIDWFDGKEFSGNPIKVSFATRRADFNRGGG
NGRGGRGRGGPMGRGGYGGGGSGGGGRGGFPSGGGGGGGQQRAGDWKCPNPTCENMNFSWRNECNQCKAPKPDGP
GGGPGGSHMGGNYGDDRRGGRGGEQKLISEEDLGAPGSAGSAAGSGASNFTQFVLVDNGGTGDVTVAPSNFANGI
AEWISSNSRSQAYKVTCSVRQSSAQNRKYTIKVEVPKGAWRSYLNMELTIPIFATNSDCELIVKAMQGLLKDGNP
IPSAIAANSGIYGAPGSAGSAAGSGASNFTQFVLVDNGGTGDVTVAPSNFANGIAEWISSNSRSQAYKVTCSVRQ
SSAQNRKYTIKVEVPKGAWRSYLNMELTIPIFATNSDCELIVKAMQGLLKDGNPIPSAIAANSGIYGAPGSAGSA
AGSGASNFTQFVLVDNGGTGDVTVAPSNFANGIAEWISSNSRSQAYKVTCSVRQSSAQNRKYTIKVEVPKGAWRS
YLNMELTIPIFATNSDCELIVKAMQGLLKDGNPIPSAIAANSGIYGTGAPGSAGSAAGSGACPVPLQLPPLERLT
LDDKKPLNTLISATGLWMSRTGTIHKIKHHEVSRSKIYIEMACGDHLVVNNSRSSRTARALRHHKYRKTCKRCRV
SDEDLNKFLTKANEDQTSVKVKVVSAPTRTKKAMPKSVARAPKPLENTEAAQAQPSGSKFSPAIPVSTQESVSVP
ASVSTSISSISTGATASALVKGNTNPITSMSAPVQASAPALTKSQTDRLEVLLNPKDEISLNSGKPFRELESELL
SRRKKDLQQIYAEERENYLGKLEREITRFFVDRGFLEIKSPILIPLEYIERMGIDNDTELSKQIFRVDKNFCLRP
MLAPNLANYLRKLDRALPDPIKIFEIGPCYRKESDGKEHLEEFTMLNFCQMGSGCTRENLESIITDFLNHLGIDF
KIVGDSCMVFGDTLDVMHGDLELSSAVVGPIPLDREWGIDKPWIGAGFGLERLLKVKHDFKNIKRAARSESYYNG
ISTNL  (SEQ ID NO:118)

TOMM20-FUS-3xMCP-PylRS^AAAF
DNA:
ATGGTGGGTCGGAACAGCGCCATCGCCGCCGGTGTATGCGGGGCCCTTTTCATTGGGTACTGCATCTACTTCGAC
CGCAAAAGACGAAGTGACCCCAACTTCAAGAACAGGCTTCGAGAACGAAGAAAGAAACAGAAGCTTGCCAAGGAG
AGAGCTGGGCTTTCCAAGTTACCTGACCTTAAAGATGCTGAAGCTGTTCAGAAATTCTTCATGGCCTCAAACGAT
TATACCCAACAAGCAACCCAAAGCTATGGGGCCTACCCCACCCAGCCCGGGCAGGGCTATTCCCAGCAGAGCAGT
CAGCCCTACGGACAGCAGAGTTACAGTGGTTATAGCCAGTCCACGGACACTTCAGGATATGGCCAGAGCAGCTAT
TCTTCTTATGGCCAGAGCCAGAACACAGGCTATGGAACTCAGTCAACTCCCCAGGGATATGGCTCGACTGGCGGC
TATGGCAGTAGCCAGAGCTCCCAATCGTCTTACGGGCAGCAGTCCTCCTACCCTGGCTATGGCCAGCAGCCAGCT
CCCAGCAACACCTCGGGAAGTTACGGTAGCAGTTCTCAGAGCAGCAGCTATGGGCAGCCCCAGAGTGGGAGCTAC
AGCCAGCAGCCTAGCTATGGTGGACAGCAGCAAAGCTATGGACAGCAGCAAAGCTATAATCCCCCTCAGGGCTAT
GGACAGCAGAACCAGTACAACAGCAGCAGTGGTGGTGGAGGTGGAGGTGGAGGTGGAGGTAACTATGGCCAAGAT
CAATCCTCCATGAGTAGTGGTGGTGGCAGTGGTGGCGGTTATGGCAATCAAGACCAGAGTGGTGGAGGTGGCAGC
GGTGGCTATGGACAGCAGGACCGTGGAGGCCGCGGCAGGGGTGGCAGTGGTGGCGGCGGCGGCGGCGGCGGTGGT
GGTTACAACCGCAGCAGTGGTGGCTATGAACCCAGAGGTCGTGGAGGTGGCCGTGGAGGCAGAGGTGGCATGGGC
GGAAGTGACCGTGGTGGCTTCAATAAATTTGGTGGCCCTCGGGACCAAGGATCACGTCATGACTCCGAACAGGAT
AATTCAGACAACAACACCATCTTTGTGCAAGGCCTGGGTGAGAATGTTACAATTGAGTCTGTGGCTGATTACTTC
AAGCAGATTGGTATTATTAAGACAAACAAGAAAACGGGACAGCCCATGATTAATTTGTACACAGACAGGGAAACT
GGCAAGCTGAAGGGAGAGGCAACGGTCTCTTTTGATGACCCACCTTCAGCTAAAGCAGCTATTGACTGGTTTGAT
GGTAAAGAATTCTCCGGAAATCCTATCAAGGTCTCATTTGCTACTCGCCGGGCAGACTTTAATCGGGGTGGTGGC
AATGGTCGTGGAGGCCGAGGGCGAGGAGGACCCATGGGCCGTGGAGGCTATGGAGGTGGTGGCAGTGGTGGTGGT
GGCCGAGGAGGATTTCCCAGTGGAGGTGGTGGCGGTGGAGGACAGCAGCGAGCTGGTGACTGGAAGTGTCCTAAT
CCCACCTGTGAGAATATGAACTTCTCTTGGAGGAATGAATGCAACCAGTGTAAGGCCCCTAAACCAGATGGCCCA
GGAGGGGGACCAGGTGGCTCTCACATGGGGGGTAACTACGGGGATGATCGTCGTGGTGGCAGAGGAGGCGAGCAG
AAGCTGATCTCAGAGGAGGACCTGGGCGCCCCCGGCTCCGCCGGCTCCGCCGCCGGCTCCGGCGCTTCTAACTTT
ACTCAGTTCGTTCTCGTCGACAATGGCGGAACTGGCGACGTGACTGTCGCCCCAAGCAACTTCGCTAACGGGATC
GCTGAATGGATCAGCTCTAACTCGCGTTCACAGGCTTACAAAGTAACCTGTAGCGTTCGTCAGAGCTCTGCGCAG
AATCGCAAATACACCATCAAAGTCGAGGTGCCTAAAGGCGCCTGGCGTTCGTACTTAAATATGGAACTAACCATT
CCAATTTTCGCCACGAATTCCGACTGCGAGCTTATTGTTAAGGCAATGCAAGGTCTCCTAAAAGATGGAAACCCG
ATTCCCTCAGCAATCGCAGCAAACTCCGGCATCTACGGTGCCCCAGGCTCCGCAGGAAGCGCAGCGGGGTCCGGA
GCTTCTAACTTTACTCAGTTCGTTCTCGTCGACAATGGCGGAACTGGCGACGTGACTGTCGCCCCAAGCAACTTC
GCTAACGGGATCGCTGAATGGATCAGCTCTAACTCGCGTTCACAGGCTTACAAAGTAACCTGTAGCGTTCGTCAG
AGCTCTGCGCAGAATCGCAAATACACCATCAAAGTCGAGGTGCCTAAAGGCGCCTGGCGTTCGTACTTAAATATG
GAACTAACCATTCCAATTTTCGCCACGAATTCCGACTGCGAGCTTATTGTTAAGGCAATGCAAGGTCTCCTAAAA
GATGGAAACCCGATTCCCTCAGCAATCGCAGCAAACTCCGGCATCTACGGCGCACCTGGTAGTGCTGGTTCTGCT
GCTGGATCAGGTGCTTCTAACTTTACTCAGTTCGTTCTCGTCGACAATGGCGGAACTGGCGACGTGACTGTCGCC

CCAAGCAACTTCGCTAACGGGATCGCTGAATGGATCAGCTCTAACTCGCGTTCACAGGCTTACAAAGTAACCTGT
AGCGTTCGTCAGAGCTCTGCGCAGAATCGCAAATACACCATCAAAGTCGAGGTGCCTAAAGGCGCCTGGCGTTCG
TACTTAAATATGGAACTAACCATTCCAATTTTCGCCACGAATTCCGACTGCGAGCTTATTGTTAAGGCAATGCAA
GGTCTCCTAAAAGATGGAAACCCGATTCCCTCAGCAATCGCAGCAAACTCCGGCATCTACGGTACCGGCGCCCCC
GGCTCCGCCGGCTCCGCCGCCGGCTCCGGCGCGTGCCCGGTGCCGCTGCAGCTGCCGCCGCTGGAACGCCTGACC
CTGGATGATAAAAAACCGCTGAATACCCTGATCTCTGCTACTGGTCTGTGGATGAGTCGTACCGGAACCATTCAT
AAAATCAAACACCACGAGGTTAGCCGTTCGAAAATCTATATTGAGATGGCGTGTGGCGATCATCTGGTTGTGAAC
AATAGCCGCTCTTCTCGTACAGCACGTGCACTGCGTCACCACAAATATCGTAAAACCTGTAAACGTTGCCGTGTG
TCCGATGAGGATCTGAACAAATTCCTGACAAAAGCCAATGAGGACCAAACAAGCGTGAAAGTGAAAGTCGTTAGC
GCTCCTACCCGTACTAAAAAAGCAATGCCGAAATCCGTTGCTCGTGCCCCTAAACCACTGGAAAACACTGAAGCA
GCACAGGCACAGCCGTCTGGAAGCAAATTCTCTCCGGCCATTCCTGTTTCTACCCAGGAGTCCGTTTCTGTTCCA
GCAAGTGTGAGCACCAGCATTAGCAGTATTAGCACCGGTGCCACCGCTAGCGCCCTGGTTAAAGGCAATACCAAT
CCGATTACAAGCATGTCTGCCCCGGTTCAAGCATCAGCTCCAGCACTGACAAAATCCCAAACCGATCGTCTGGAG
GTTCTGCTGAATCCGAAAGACGAAATCAGCCTGAATTCCGGCAAACCGTTTCGTGAACTGGAGAGCGAACTGCTG
TCACGTCGTAAAAAAGACCTGCAACAAATCTATGCCGAAGAACGTGAGAACTATCTGGGGAAACTGGAACGTGAA
ATCACCCGCTTTTTCGTGGATCGTGGCTTTCTGGAGATCAAATCCCCGATTCTGATTCCTCTGGAGTATATCGAG
CGTATGGGCATCGACAATGATACCGAACTGAGCAAACAAATTTTCCGTGTGGATAAAAACTTCTGTCTGCGCCCT
ATGCTAGCACCAAATCTGGCTAACTATCTGCGCAAACTGGACCGTGCCCTGCCTGATCCTATCAAAATCTTCGAG
ATCGGCCCGTGTTATCGTAAAGAGTCCGACGGTAAAGAACATCTGGAGGAGTTTACCATGCTGGCCTTTGCCCAA
ATGGGTTCAGGTTGTACTCGTGAGAACCTGGAAAGCATCATCACCGATTTTCTGAACCACCTGGGCATTGACTTC
AAAATTGTGGGCGACAGCTGTATGGTGTTTGGCGACACCCTGGATGTCATGCACGGCGACCTGGAACTGTCTAGT
GCCGTTGTGGGCCCAATCCCGCTGGATCGTGAGTGGGGTATCGACAAACCTTGGATCGGTGCGGGTTTTGGTCTG
GAGCGTCTGCTGAAAGTAAAACACGACTTCAAGAACATCAAACGTGCTGCACGTTCCGAGTCCTATTACAATGGT
ATTTCTACTAACCTGTAA (SEQ ID NO:119)
Protein:
MVGRNSAIAAGVCGALFIGYCIYFDRKRRSDPNFKNRLRERRKKQKLAKERAGLSKLPDLKDAEAVQKFFMASND
YTQQATQSYGAYPTQPGQGYSQQSSQPYGQQSYSGYSQSTDTSGYGQSSYSSYGQSQNTGYGTQSTPQGYGSTGG
YGSSQSSQSSYGQQSSYPGYGQQPAPSNTSGSYGSSSQSSSYGQPQSGSYSQQPSYGGQQQSYGQQQSYNPPQGY
GQQNQYNSSSGGGGGGGGGGNYGQDQSSMSSGGGSGGGYGNQDQSGGGGSGGYGQQDRGGRGRGGSGGGGGGGGG
GYNRSSGGYEPRGRGGGRGGRGGMGGSDRGGFNKFGGPRDQGSRHDSEQDNSDNNTIFVQGLGENVTIESVADYF
KQIGIIKTNKKTGQPMINLYTDRETGKLKGEATVSFDDPPSAKAAIDWFDGKEFSGNPIKVSFATRRADFNRGGG
NGRGGRGRGGPMGRGGYGGGGSGGGGRGGFPSGGGGGGGQQRAGDWKCPNPTCENMNFSWRNECNQCKAPKPDGP
GGGPGGSHMGGNYGDDRRGGRGGEQKLISEEDLGAPGSAGSAAGSGASNFTQFVLVDNGGTGDVTVAPSNFANGI
AEWISSNSRSQAYKVTCSVRQSSAQNRKYTIKVEVPKGAWRSYLNMELTIPIFATNSDCELIVKAMQGLLKDGNP
IPSAIAANSGIYGAPGSAGSAAGSGASNFTQFVLVDNGGTGDVTVAPSNFANGIAEWISSNSRSQAYKVTCSVRQ
SSAQNRKYTIKVEVPKGAWRSYLNMELTIPIFATNSDCELIVKAMQGLLKDGNPIPSAIAANSGIYGAPGSAGSA
AGSGASNFTQFVLVDNGGTGDVTVAPSNFANGIAEWISSNSRSQAYKVTCSVRQSSAQNRKYTIKVEVPKGAWRS
YLNMELTIPIFATNSDCELIVKAMQGLLKDGNPIPSAIAANSGIYGTGAPGSAGSAAGSGACPVPLQLPPLERLT
LDDKKPLNTLISATGLWMSRTGTIHKIKHHEVSRSKIYIEMACGDHLVVNNSRSSRTARALRHHKYRKTCKRCRV
SDEDLNKFLTKANEDQTSVKVKVVSAPTRTKKAMPKSVARAPKPLENTEAAQAQPSGSKFSPAIPVSTQESVSVP
ASVSTSISSISTGATASALVKGNTNPITSMSAPVQASAPALTKSQTDRLEVLLNPKDEISLNSGKPFRELESELL
SRRKKDLQQIYAEERENYLGKLEREITRFFVDRGFLEIKSPILIPLEYIERMGIDNDTELSKQIFRVDKNFCLRP
MLAPNLANYLRKLDRALPDPIKIFEIGPCYRKESDGKEHLEEFTMLAFAQMGSGCTRENLESIITDFLNHLGIDF
KIVGDSCMVFGDTLDVMHGDLELSSAVVGPIPLDREWGIDKPWIGAGFGLERLLKVKHDFKNIKRAARSESYYNG
ISTNL (SEQ ID NO:120)

TOMM20-FUS-4xλN22-PylRSAF
DNA:
ATGGTGGGTCGGAACAGCGCCATCGCCGCCGGTGTATGCGGGGCCCTTTTCATTGGGTACTGCATCTACTTCGAC
CGCAAAAGACGAAGTGACCCCAACTTCAAGAACAGGCTTCGAGAACGAAGAAAGAAACAGAAGCTTGCCAAGGAG
AGAGCTGGGCTTTCCAAGTTACCTGACCTTAAAGATGCTGAAGCTGTTCAGAAATTCTTCATGGCCTCAAACGAT
TATACCCAACAAGCAACCCAAAGCTATGGGGCCTACCCCACCCAGCCCGGGCAGGGCTATTCCCAGCAGAGCAGT
CAGCCCTACGGACAGCAGAGTTACAGTGGTTATAGCCAGTCCACGGACACTTCAGGATATGGCCAGAGCAGCTAT
TCTTCTTATGGCCAGAGCCAGAACACAGGCTATGGAACTCAGTCAACTCCCCAGGGATATGGCTCGACTGGCGGC
TATGGCAGTAGCCAGAGCTCCCAATCGTCTTACGGGCAGCAGTCCTCCTACCCTGGCTATGGCCAGCAGCCAGCT
CCCAGCAACACCTCGGGAAGTTACGGTAGCAGTTCTCAGAGCAGCAGCTATGGGCAGCCCCAGAGTGGGAGCTAC
AGCCAGCAGCCTAGCTATGGTGGACAGCAGCAAAGCTATGGACAGCAGCAAAGCTATAATCCCCCTCAGGGCTAT
GGACAGCAGAACCAGTACAACAGCAGCAGTGGTGGTGGAGGTGGAGGTGGAGGTGGAGGTAACTATGGCCAAGAT

CAATCCTCCATGAGTAGTGGTGGTGGCAGTGGTGGCGGTTATGGCAATCAAGACCAGAGTGGTGGAGGTGGCAGC
GGTGGCTATGGACAGCAGGACCGTGGAGGCCGCGGCAGGGGTGGCAGTGGTGGCGGCGGCGGCGGCGGTGGT
GGTTACAACCGCAGCAGTGGTGGCTATGAACCCAGAGGTCGTGGAGGTGGCCGTGGAGGCAGAGGTGGCATGGGC
GGAAGTGACCGTGGTGGCTTCAATAAATTTGGTGGCCCTCGGGACCAAGGATCACGTCATGACTCCGAACAGGAT
AATTCAGACAACAACACCATCTTTGTGCAAGGCCTGGGTGAGAATGTTACAATTGAGTCTGTGGCTGATTACTTC
AAGCAGATTGGTATTATTAAGACAAACAAGAAAACGGGACAGCCCATGATTAATTTGTACACAGACAGGGAAACT
GGCAAGCTGAAGGGAGAGGCAACGGTCTCTTTTGATGACCCACCTTCAGCTAAAGCAGCTATTGACTGGTTTGAT
GGTAAAGAATTCTCCGGAAATCCTATCAAGGTCTCATTTGCTACTCGCCGGGCAGACTTTAATCGGGGTGGTGGC
AATGGTCGTGGAGGCCGAGGGCGAGGAGGACCCATGGGCCGTGGAGGCTATGGAGGTGGTGGCAGTGGTGGTGGT
GGCCGAGGAGGATTTCCCAGTGGAGGTGGTGGCGGTGGAGGACAGCAGCGAGCTGGTGACTGGAAGTGTCCTAAT
CCCACCTGTGAGAATATGAACTTCTCTTGGAGGAATGAATGCAACCAGTGTAAGGCCCCTAAACCAGATGGCCCA
GGAGGGGGACCAGGTGGCTCTCACATGGGGGGTAACTACGGGGATGATCGTCGTGGTGGCAGAGGAGGCGCCACC
ATGGACGCACAAACACGACGACGTGAGCGTCGCGCTGAGAAACAAGCTCAATGGAAAGCTGCAAACCCACCGCTC
GACGGAGCCGGAGCTGGCGCTGGAGCTGGAGCCGGAGCTGGCGGTCTAGCCACCATGGACGCACAAACACGACGA
CGTGAGCGTCGCGCTGAGAAACAAGCTCAATGGAAAGCTGCAAACCCACCGCTCGACGGAGCCGGAGCTGGCGCT
GGAGCTGGAGCCGGAGCTGGCGGTCTAGCCACCATGGACGCACAAACACGACGACGTGAGCGTCGCGCTGAGAAA
CAAGCTCAATGGAAAGCTGCAAACCCACCGCTCGACGGAGCCGGAGCTGGCGCTGGAGCTGGAGCCGGAGCTGGC
GGTCTAGCCACCATGGACGCACAAACACGACGACGTGAGCGTCGCGCTGAGAAACAAGCTCAATGGAAAGCTGCA
AACCCACCGCTCGATTACAAGGATGACGACGATAAGGGTACCGGCGCCCCCGGCTCCGCCGGCTCCGCCGCCGGC
TCCGGCGCGTGCCCGGTGCCGCTGCAGCTGCCGCCGCTGGAACGCCTGACCCTGGATGATAAAAAACCGCTGAAT
ACCCTGATCTCTGCTACTGGTCTGTGGATGAGTCGTACCGGAACCATTCATAAAATCAAACACCACGAGGTTAGC
CGTTCGAAAATCTATATTGAGATGGCGTGTGGCGATCATCTGGTTGTGAACAATAGCCGCTCTTCTCGTACAGCA
CGTGCACTGCGTCACCACAAATATCGTAAAACCTGTAAACGTTGCCGTGTGTCCGATGAGGATCTGAACAAATTC
CTGACAAAAGCCAATGAGGACCAAACAAGCGTGAAAGTGAAAGTCGTTAGCGCTCCTACCCGTACTAAAAAAGCA
ATGCCGAAATCCGTTGCTCGTGCCCCTAAACCACTGGAAAACACTGAAGCAGCACAGGCACAGCCGTCTGGAAGC
AAATTCTCTCCGGCCATTCCTGTTTCTACCCAGGAGTCCGTTTCTGTTCCAGCAAGTGTGAGCACCAGCATTAGC
AGTATTAGCACCGGTGCCACCGCTAGCGCCCTGGTTAAAGGCAATACCAATCCGATTACAAGCATGTCTGCCCCG
GTTCAAGCATCAGCTCCAGCACTGACAAAATCCCAAACCGATCGTCTGGAGGTTCTGCTGAATCCGAAAGACGAA
ATCAGCCTGAATTCCGGCAAACCGTTTCGTGAACTGGAGAGCGAACTGCTGTCACGTCGTAAAAAAGACCTGCAA
CAAATCTATGCCGAAGAACGTGAGAACTATCTGGGGAAACTGGAACGTGAAATCACCCGCTTTTTCGTGGATCGT
GGCTTTCTGGAGATCAAATCCCCGATTCTGATTCCTCTGGAGTATATCGAGCGTATGGGCATCGACAATGATACC
GAACTGAGCAAACAAATTTTCCGTGTGGATAAAAACTTCTGTCTGCGCCCTATGCTAGCACCAAATCTGGCTAAC
TATCTGCGCAAACTGGACCGTGCCCTGCCTGATCCTATCAAAATCTTCGAGATCGGCCCGTGTTATCGTAAAGAG
TCCGACGGTAAAGAACATCTGGAGGAGTTTACCATGCTGAACTTTTGCCAAATGGGTTCAGGTTGTACTCGTGAG
AACCTGGAAAGCATCATCACCGATTTTCTGAACCACCTGGGCATTGACTTCAAAATTGTGGGCGACAGCTGTATG
GTGTTTGGCGACACCCTGGATGTCATGCACGGCGACCTGGAACTGTCTAGTGCCGTTGTGGGCCCAATCCCGCTG
GATCGTGAGTGGGGTATCGACAAACCTTGGATCGGTGCGGGTTTTGGTCTGGAGCGTCTGCTGAAAGTAAAACAC
GACTTCAAGAACATCAAACGTGCTGCACGTTCCGAGTCCTATTACAATGGTATTTCTACTAACCTGTAA (SEQ
ID NO:121)
Protein:
MVGRNSAIAAGVCGALFIGYCIYFDRKRRSDPNFKNRLRERRKKQKLAKERAGLSKLPDLKDAEAVQKFFMASND
YTQQATQSYGAYPTQPGQGYSQQSSQPYGQQSYSGYSQSTDTSGYGQSSYSSYGQSQNTGYGTQSTPQGYGSTGG
YGSSQSSQSSYGQQSSYPGYGQQPAPSNTSGSYGSSSQSSSYGQPQSGSYSQQPSYGGQQQSYGQQQSYNPPQGY
GQQNQYNSSSGGGGGGGGGGNYGQDQSSMSSGGGSGGGYGNQDQSGGGGSGGYGQQDRGGRGRGGSGGGGGGGGG
GYNRSSGGYEPRGRGGGRGGRGGMGGSDRGGFNKFGGPRDQGSRHDSEQDNSDNNTIFVQGLGENVTIESVADYF
KQIGIIKTNKKTGQPMINLYTDRETGKLKGEATVSFDDPPSAKAAIDWFDGKEFSGNPIKVSFATRRADFNRGGG
NGRGGRGRGGPMGRGGYGGGGSGGGGRGGFPSGGGGGGGQQRAGDWKCPNPTCENMNFSWRNECNQCKAPKPDGP
GGGPGGSHMGGNYGDDRRGGRGGATMDAQTRRRERRAEKQAQWKAANPPLDGAGAGAGAGAGGLATMDAQTRR
RERRAEKQAQWKAANPPLDGAGAGAGAGAGGLATMDAQTRRRERRAEKQAQWKAANPPLDGAGAGAGAGAGAG
GLATMDAQTRRRERRAEKQAQWKAANPPLDYKDDDDKGTGAPGSAGSAAGSGACPVPLQLPPLERLTLDDKKPLN
TLISATGLWMSRTGTIHKIKHHEVSRSKIYIEMACGDHLVVNNSRSSRTARALRHHKYRKTCKRCRVSDEDLNKF
LTKANEDQTSVKVKVVSAPTRTKKAMPKSVARAPKPLENTEAAQAQPSGSKFSPAIPVSTQESVSVPASVSTSIS
SISTGATASALVKGNTNPITSMSAPVQASAPALTKSQTDRLEVLLNPKDEISLNSGKPFRELESELLSRRKKDLQ
QIYAEERENYLGKLEREITRFFVDRGFLEIKSPILIPLEYIERMGIDNDTELSKQIFRVDKNFCLRPMLAPNLAN
YLRKLDRALPDPIKIFEIGPCYRKESDGKEHLEEFTMLNFCQMGSGCTRENLESIITDFLNHLGIDFKIVGDSCM
VFGDTLDVMHGDLELSSAVVGPIPLDREWGIDKPWIGAGFGLERLLKVKHDFKNIKRAARSESYYNGISTNL
(SEQ ID NO:122)

TOMM20-FUS-4xλN22-PylRSAAAF
DNA:
ATGGTGGGTCGGAACAGCGCCATCGCCGCCGGTGTATGCGGGGCCCTTTTCATTGGGTACTGCATCTACTTCGAC
CGCAAAAGACGAAGTGACCCCAACTTCAAGAACAGGCTTCGAGAACGAAGAAAGAAACAGAAGCTTGCCAAGGAG
AGAGCTGGGCTTTCCAAGTTACCTGACCTTAAAGATGCTGAAGCTGTTCAGAAATTCTTCATGGCCTCAAACGAT
TATACCCAACAAGCAACCCAAAGCTATGGGGCCTACCCCACCCAGCCCGGGCAGGGCTATTCCCAGCAGAGCAGT
CAGCCCTACGGACAGCAGAGTTACAGTGGTTATAGCCAGTCCACGGACACTTCAGGATATGGCCAGAGCAGCTAT
TCTTCTTATGGCCAGAGCCAGAACACAGGCTATGGAACTCAGTCAACTCCCCAGGGATATGGCTCGACTGGCGGC
TATGGCAGTAGCCAGAGCTCCCAATCGTCTTACGGGCAGCAGTCCTCCTACCCTGGCTATGGCCAGCAGCCAGCT
CCCAGCAACACCTCGGGAAGTTACGGTAGCAGTTCTCAGAGCAGCAGCTATGGGCAGCCCCAGAGTGGGAGCTAC
AGCCAGCAGCCTAGCTATGGTGGACAGCAGCAAAGCTATGGACAGCAGCAAAGCTATAATCCCCCTCAGGGCTAT
GGACAGCAGAACCAGTACAACAGCAGCAGTGGTGGTGGAGGTGGAGGTGGAGGTGGAGGTAACTATGGCCAAGAT
CAATCCTCCATGAGTAGTGGTGGTGGCAGTGGTGGCGGTTATGGCAATCAAGACCAGAGTGGTGGAGGTGGCAGC
GGTGGCTATGGACAGCAGGACCGTGGAGGCCGCGGCAGGGGTGGCAGTGGTGGCGGCGGCGGCGGCGGCGGTGGT
GGTTACAACCGCAGCAGTGGTGGCTATGAACCCAGAGGTCGTGGAGGTGGCCGTGGAGGCAGAGGTGGCATGGGC
GGAAGTGACCGTGGTGGCTTCAATAAATTTGGTGGCCCTCGGGACCAAGGATCACGTCATGACTCCGAACAGGAT
AATTCAGACAACAACACCATCTTTGTGCAAGGCCTGGGTGAGAATGTTACAATTGAGTCTGTGGCTGATTACTTC
AAGCAGATTGGTATTATTAAGACAAACAAGAAAACGGGACAGCCCATGATTAATTTGTACACAGACAGGGAAACT
GGCAAGCTGAAGGGAGAGGCAACGGTCTCTTTTGATGACCCACCTTCAGCTAAAGCAGCTATTGACTGGTTTGAT
GGTAAAGAATTCTCCGGAAATCCTATCAAGGTCTCATTTGCTACTCGCCGGGCAGACTTTAATCGGGGTGGTGGC
AATGGTCGTGGAGGCCGAGGGCGAGGAGGACCCATGGGCCGTGGAGGCTATGGAGGTGGTGGCAGTGGTGGTGGT
GGCCGAGGAGGATTTCCCAGTGGAGGTGGTGGCGGTGGAGGACAGCAGCGAGCTGGTGACTGGAAGTGTCCTAAT
CCCACCTGTGAGAATATGAACTTCTCTTGGAGGAATGAATGCAACCAGTGTAAGGCCCCTAAACCAGATGGCCCA
GGAGGGGGACCAGGTGGCTCTCACATGGGGGGTAACTACGGGGATGATCGTCGTGGTGGCAGAGGAGGCGCCACC
ATGGACGCACAAACACGACGACGTGAGCGTCGCGCTGAGAAACAAGCTCAATGGAAAGCTGCAAACCCACCGCTC
GACGGAGCCGGAGCTGGCGCTGGAGCTGGAGCCGGAGCTGGCGGTCTAGCCACCATGGACGCACAAACACGACGA
CGTGAGCGTCGCGCTGAGAAACAAGCTCAATGGAAAGCTGCAAACCCACCGCTCGACGGAGCCGGAGCTGGCGCT
GGAGCTGGAGCCGGAGCTGGCGGTCTAGCCACCATGGACGCACAAACACGACGACGTGAGCGTCGCGCTGAGAAA
CAAGCTCAATGGAAAGCTGCAAACCCACCGCTCGACGGAGCCGGAGCTGGCGCTGGAGCTGGAGCCGGAGCTGGC
GGTCTAGCCACCATGGACGCACAAACACGACGACGTGAGCGTCGCGCTGAGAAACAAGCTCAATGGAAAGCTGCA
AACCCACCGCTCGATTACAAGGATGACGACGATAAGGGTACCGGCGCCCCCGGCTCCGCCGGCTCCGCCGCCGGC
TCCGGCGCGTGCCCGGTGCCGCTGCAGCTGCCGCCGCTGGAACGCCTGACCCTGGATGATAAAAAACCGCTGAAT
ACCCTGATCTCTGCTACTGGTCTGTGGATGAGTCGTACCGGAACCATTCATAAAATCAAACACCACGAGGTTAGC
CGTTCGAAAATCTATATTGAGATGGCGTGTGGCGATCATCTGGTTGTGAACAATAGCCGCTCTTCTCGTACAGCA
CGTGCACTGCGTCACCACAAATATCGTAAAACCTGTAAACGTTGCCGTGTGTCCGATGAGGATCTGAACAAATTC
CTGACAAAAGCCAATGAGGACCAAACAAGCGTGAAAGTGAAAGTCGTTAGCGCTCCTACCCGTACTAAAAAAGCA
ATGCCGAAATCCGTTGCTCGTGCCCCTAAACCACTGGAAAACACTGAAGCAGCACAGGCACAGCCGTCTGGAAGC
AAATTCTCTCCGGCCATTCCTGTTTCTACCCAGGAGTCCGTTTCTGTTCCAGCAAGTGTGAGCACCAGCATTAGC
AGTATTAGCACCGGTGCCACCGCTAGCGCCCTGGTTAAAGGCAATACCAATCCGATTACAAGCATGTCTGCCCCG
GTTCAAGCATCAGCTCCAGCACTGACAAAATCCCAAACCGATCGTCTGGAGGTTCTGCTGAATCCGAAAGACGAA
ATCAGCCTGAATTCCGGCAAACCGTTTCGTGAACTGGAGAGCGAACTGCTGTCACGTCGTAAAAAAGACCTGCAA
CAAATCTATGCCGAAGAACGTGAGAACTATCTGGGGAAACTGGAACGTGAAATCACCCGCTTTTTCGTGGATCGT
GGCTTTCTGGAGATCAAATCCCCGATTCTGATTCCTCTGGAGTATATCGAGCGTATGGGCATCGACAATGATACC
GAACTGAGCAAACAAATTTTCCGTGTGGATAAAAACTTCTGTCTGCGCCCTATGCTAGCACCAAATCTGGCTAAC
TATCTGCGCAAACTGGACCGTGCCCTGCCTGATCCTATCAAAATCTTCGAGATCGGCCCGTGTTATCGTAAAGAG
TCCGACGGTAAAGAACATCTGGAGGAGTTTACCATGCTGGCCTTTGCCCAAATGGGTTCAGGTTGTACTCGTGAG
AACCTGGAAAGCATCATCACCGATTTTCTGAACCACCTGGGCATTGACTTCAAAATTGTGGGCGACAGCTGTATG
GTGTTTGGCGACACCCTGGATGTCATGCACGGCGACCTGGAACTGTCTAGTGCCGTTGTGGGCCCAATCCCGCTG
GATCGTGAGTGGGGTATCGACAAACCTTGGATCGGTGCGGGTTTTGGTCTGGAGCGTCTGCTGAAAGTAAAACAC
GACTTCAAGAACATCAAACGTGCTGCACGTTCCGAGTCCTATTACAATGGTATTTCTACTAACCTGTAA (SEQ
ID NO:123)
Protein:
MVGRNSAIAAGVCGALFIGYCIYFDRKRRSDPNFKNRLRERRKKQKLAKERAGLSKLPDLKDAEAVQKFFMASND
YTQQATQSYGAYPTQPGQGYSQQSSQPYGQQSYSGYSQSTDTSGYGQSSYSSYGQSQNTGYGTQSTPQGYGSTGG
YGSSQSSQSSYGQQSSYPGYGQQPAPSNTSGSYGSSSQSSSYGQPQSGSYSQQPSYGGQQQSYGQQQSYNPPQGY
GQQNQYNSSSGGGGGGGGGNYGQDQSSMSSGGGSGGGYGNQDQSGGGGSGGYGQQDRGGRGRGGSGGGGGGGG
GYNRSSGGYEPRGRGGGRGGRGGMGGSDRGGFNKFGGPRDQGSRHDSEQDNSDNNTIFVQGLGENVTIESVADYF
KQIGIIKTNKKTGQPMINLYTDRETGKLKGEATVSFDDPPSAKAAIDWFDGKEFSGNPIKVSFATRRADFNRGGG

NGRGGRGRGGPMGRGGYGGGGSGGGGRGGFPSGGGGGGGQQRAGDWKCPNPTCENMNFSWRNECNQCKAPKPDGP
GGGPGGSHMGGNYGDDRRGGRGGATMDAQTRRRERRAEKQAQWKAANPPLDGAGAGAGAGAGAGGLATMDAQTRR
RERRAEKQAQWKAANPPLDGAGAGAGAGAGAGGLATMDAQTRRRERRAEKQAQWKAANPPLDGAGAGAGAGAGAG
GLATMDAQTRRRERRAEKQAQWKAANPPLDYKDDDDKGTGAPGSAGSAAGSGACPVPLQLPPLERLTLDDKKPLN
TLISATGLWMSRTGTIHKIKHHEVSRSKIYIEMACGDHLVVNNSRSSRTARALRHHKYRKTCKRCRVSDEDLNKF
LTKANEDQTSVKVKVVSAPTRTKKAMPKSVARAPKPLENTEAAQAQPSGSKFSPAIPVSTQESVSVPASVSTSIS
SISTGATASALVKGNTNPITSMSAPVQASAPALTKSQTDRLEVLLNPKDEISLNSGKPFRELESELLSRRKKDLQ
QIYAEERENYLGKLEREITRFFVDRGFLEIKSPILIPLEYIERMGIDNDTELSKQIFRVDKNFCLRPMLAPNLAN
YLRKLDRALPDPIKIFEIGPCYRKESDGKEHLEEFTMLAFAQMGSGCTRENLESIITDFLNHLGIDFKIVGDSCM
VFGDTLDVMHGDLELSSAVVGPIPLDREWGIDKPWIGAGFGLERLLKVKHDFKNIKRAARSESYYNGISTNL
(SEQ ID NO:124)

LcK-EWSR1-MCP
DNA:
ATGGGCTGCGTGTGCAGCAGCAACCCCGAGGGTACCGAGCTCGCGTCCACGGATTACAGTACCTATAGCCAAGCT
GCAGCGCAGCAGGGCTACAGTGCTTACACCGCCCAGCCCACTCAAGGATATGCACAGACCACCCAGGCATATGGG
CAACAAAGCTATGGAACCTATGGACAGCCCACTGATGTCAGCTATACCCAGGCTCAGACCACTGCAACCTATGGG
CAGACCGCCTATGCAACTTCTTATGGACAGCCTCCCACTGGTTATACTACTCCAACTGCCCCCCAGGCATACAGC
CAGCCTGTCCAGGGGTATGGCACTGGTGCTTATGATACCACCACTGCTACAGTCACCACCACCCAGGCCTCCTAT
GCAGCTCAGTCTGCATATGGCACTCAGCCTGCTTATCCAGCCTATGGGCAGCAGCCAGCAGCCACTGCACCTACA
AGACCGCAGGATGGAAACAAGCCCACTGAGACTAGTCAACCTCAATCTAGCACAGGGGGTTACAACCAGCCCAGC
CTAGGATATGGACAGAGTAACTACAGTTATCCCCAGGTACCTGGGAGCTACCCCATGCAGCCAGTCACTGCACCT
CCATCCTACCCTCCTACCAGCTATTCCTCTACACAGCCGACTAGTTATGATCAGAGCAGTTACTCTCAGCAGAAC
ACCTATGGGCAACCGAGCAGCTATGGACAGCAGAGTAGCTATGGTCAACAAAGCAGCTATGGGCAGCAGCCTCCC
ACTAGTTACCCACCCCAAACTGGATCCTACAGCCAAGCTCCAAGTCAATATAGCCAACAGAGCAGCAGCTACGGG
CAGCAGAGTTCATTCCGACAGGACCACCCCAGTAGCATGGGTGTTTATGGGCAGGAGTCTGGAGGATTTTCCGGA
CCAGGAGAGAACCGGAGCATGAGTGGCCCTGATAACCGGGGCAGGGGAAGAGGGGGATTTGATCGTGGAGGCATG
AGCAGAGGTGGGCGGGGAGGAGGACGCGGTGGAATGGGCAGCGCTGGAGAGCGAGGTGGCTTCAATAAGCCTGGT
GGACCCATGGATGAAGGACCAGATCTTGATCTAGGCCCACCTGTAGATCCAGATGAAGACTCTGACAACAGTGCA
ATTTATGTACAAGGATTAAATGACAGTGTGACTCTAGATGATCTGGCAGACTTCTTTAAGCAGTGTGGGGTTGTT
AAGATGAACAAGAGAACTGGGCAACCCATGATCCACATCTACCTGGACAAGGAAACAGGAAAGCCCAAAGGCGAT
GCCACAGTGTCCTATGAAGACCCACCCACTGCCAAGGCTGCCGTGGAATGGTTTGATGGGAAAGATTTTCAAGGG
AGCAAACTTAAAGTCTCCCTTGCTCGGAAGAAGCCTCCAATGAACAGTATGCGGGGTGGTCTGCCACCCCGTGAG
GGCAGAGGCATGCCACCACCACTCCGTGGAGGTCCAGGAGGCCCAGGAGGTCCTGGGGGACCCATGGGTCGCATG
GGAGGCCGTGGAGGAGATAGAGGAGGCTTCCCTCCAAGAGGACCCCGGGGTTCCCGAGGGAACCCCTCTGGAGGA
GGAAACGTCCAGCACCGAGCTGGAGACTGGCAGTGTCCCAATCCGGGTTGTGGAAACCAGAACTTCGCCTGGAGA
ACAGAGTGCAACCAGTGTAAGGCCCCAAAGCCTGAAGGCTTCCTCCCGCCACCCTTTCCGCCCCCGGGTGGTGAT
CGTGGCAGAGGTGGCCCTGGTGGCATGCGGGGAGGAAGAGGTGGCCTCATGGATCGTGGTGGTCCCGGTGGAATG
TTCAGAGGTGGCCGTGGTGGAGACAGAGGTGGCTTCCGTGGTGGCCGGGGCATGGACCGAGGTGGCTTTGGTGGA
GGAAGACGAGGTGGCCCTGGGGGGCCCCCTGGACCTTTGATGGAACAGGATTACAAGGATGACGACGATAAGGGT
ACCGAGCAGAAGCTGATCTCAGAGGAGGACCTGGGCGCCCCCGGCTCCGCCGGCTCCGCCGCCGGCTCCGGCGCT
TCTAACTTTACTCAGTTCGTTCTCGTCGACAATGGCGGAACTGGCGACGTGACTGTCGCCCCAAGCAACTTCGCT
AACGGGATCGCTGAATGGATCAGCTCTAACTCGCGTTCACAGGCTTACAAAGTAACCTGTAGCGTTCGTCAGAGC
TCTGCGCAGAATCGCAAATACACCATCAAAGTCGAGGTGCCTAAAGGCGCCTGGCGTTCGTACTTAAATATGGAA
CTAACCATTCCAATTTTCGCCACGAATTCCGACTGCGAGCTTATTGTTAAGGCAATGCAAGGTCTCCTAAAAGAT
GGAAACCCGATTCCCTCAGCAATCGCAGCAAACTCCGGCATCTACTAA (SEQ ID NO:125)
Protein:
MGCVCSSNPEGTELASTDYSTYSQAAAQQGYSAYTAQPTQGYAQTTQAYGQQSYGTYGQPTDVSYTQAQTTATYG
QTAYATSYGQPPTGYTTPTAPQAYSQPVQGYGTGAYDTTTATVTTTQASYAAQSAYGTQPAYPAYGQQPAATAPT
RPQDGNKPTETSQPQSSTGGYNQPSLGYGQSNYSYPQVPGSYPMQPVTAPPSYPPTSYSSTQPTSYDQSSYSQQN
TYGQPSSYGQQSSYGQQSSYGQQPPTSYPPQTGSYSQAPSQYSQQSSSYGQQSSFRQDHPSSMGVYGQESGGFSG
PGENRSMSGPDNRGRGRGGFDRGGMSRGGRGGGRGGMGSAGERGGFNKPGGPMDEGPDLDLGPPVDPDEDSDNSA
IYVQGLNDSVTLDDLADFFKQCGVVKMNKRTGQPMIHIYLDKETGKPKGDATVSYEDPPTAKAAVEWFDGKDFQG
SKLKVSLARKKPPMNSMRGGLPPREGRGMPPPLRGGPGGPGGPGGPMGRMGGRGGDRGGFPPRGPRGSRGNPSGG
GNVQHRAGDWQCPNPGCGNQNFAWRTECNQCKAPKPEGFLPPPFPPPGGDRGRGGPGGMRGGRGGLMDRGGPGGM
FRGGRGGDRGGFRGGRGMDRGGFGGGRRGGPGGPPGPLMEQDYKDDDDKGTEQKLISEEDLGAPGSAGSAAGSGA
SNFTQFVLVDNGGTGDVTVAPSNFANGIAEWISSNSRSQAYKVTCSVRQSSAQNRKYTIKVEVPKGAWRSYLNME
LTIPIFATNSDCELIVKAMQGLLKDGNPIPSAIAANSGIY (SEQ ID NO:126)

LcK-EWSR1-4xλN22
DNA:
ATGGGCTGCGTGTGCAGCAGCAACCCCGAGGGTACCGAGCTCGCGTCCACGGATTACAGTACCTATAGCCAAGCT
GCAGCGCAGCAGGGCTACAGTGCTTACACCGCCCAGCCCACTCAAGGATATGCACAGACCACCCAGGCATATGGG
CAACAAAGCTATGGAACCTATGGACAGCCCACTGATGTCAGCTATACCCAGGCTCAGACCACTGCAACCTATGGG
CAGACCGCCTATGCAACTTCTTATGGACAGCCTCCCACTGGTTATACTACTCCAACTGCCCCCCAGGCATACAGC
CAGCCTGTCCAGGGGTATGGCACTGGTGCTTATGATACCACCACTGCTACAGTCACCACCACCCAGGCCTCCTAT
GCAGCTCAGTCTGCATATGGCACTCAGCCTGCTTATCCAGCCTATGGGCAGCAGCCAGCAGCCACTGCACCTACA
AGACCGCAGGATGGAAACAAGCCCACTGAGACTAGTCAACCTCAATCTAGCACAGGGGGTTACAACCAGCCCAGC
CTAGGATATGGACAGAGTAACTACAGTTATCCCCAGGTACCTGGGAGCTACCCCATGCAGCCAGTCACTGCACCT
CCATCCTACCCTCCTACCAGCTATTCCTCTACACAGCCGACTAGTTATGATCAGAGCAGTTACTCTCAGCAGAAC
ACCTATGGGCAACCGAGCAGCTATGGACAGCAGAGTAGCTATGGTCAACAAAGCAGCTATGGGCAGCAGCCTCCC
ACTAGTTACCCACCCCAAACTGGATCCTACAGCCAAGCTCCAAGTCAATATAGCCAACAGAGCAGCAGCTACGGG
CAGCAGAGTTCATTCCGACAGGACCACCCCAGTAGCATGGGTGTTTATGGGCAGGAGTCTGGAGGATTTTCCGGA
CCAGGAGAGAACCGGAGCATGAGTGGCCCTGATAACCGGGGCAGGGGAAGAGGGGGATTTGATCGTGGAGGCATG
AGCAGAGGTGGGCGGGGAGGAGGACGCGGTGGAATGGGCAGCGCTGGAGAGCGAGGTGGCTTCAATAAGCCTGGT
GGACCCATGGATGAAGGACCAGATCTTGATCTAGGCCCACCTGTAGATCCAGATGAAGACTCTGACAACAGTGCA
ATTTATGTACAAGGATTAAATGACAGTGTGACTCTAGATGATCTGGCAGACTTCTTTAAGCAGTGTGGGGTTGTT
AAGATGAACAAGAGAACTGGGCAACCCATGATCCACATCTACCTGGACAAGGAAACAGGAAAGCCCAAAGGCGAT
GCCACAGTGTCCTATGAAGACCCACCTACTGCCAAGGCTGCCGTGGAATGGTTTGATGGGAAAGATTTTCAAGGG
AGCAAACTTAAAGTCTCCCTTGCTCGGAAGAAGCCTCCAATGAACAGTATGCGGGGTGGTCTGCCACCCCGTGAG
GGCAGAGGCATGCCACCACCACTCCGTGGAGGTCCAGGAGGCCCAGGAGGTCCTGGGGGACCCATGGGTCGCATG
GGAGGCCGTGGAGGAGATAGAGGAGGCTTCCCTCCAAGAGGACCCCGGGGTTCCCGAGGGAACCCCTCTGGAGGA
GGAAACGTCCAGCACCGAGCTGGAGACTGGCAGTGTCCCAATCCGGGTTGTGGAAACCAGAACTTCGCCTGGAGA
ACAGAGTGCAACCAGTGTAAGGCCCCAAAGCCTGAAGGCTTCCTCCCGCCACCCTTTCCGCCCCCGGGTGGTGAT
CGTGGCAGAGGTGGCCCTGGTGGCATGCGGGGAGGAAGAGGTGGCCTCATGGATCGTGGTGGTCCCGGTGGAATG
TTCAGAGGTGGCCGTGGTGGAGACAGAGGTGGCTTCCGTGGTGGCCGGGGCATGGACCGAGGTGGCTTTGGTGGA
GGAAGACGAGGTGGCCCTGGGGGGCCCCCTGGACCTTTGATGGAACAGGATTACAAGGATGACGACGATAAGGGT
ACCGAGCAGAAGCTGATCTCAGAGGAGGACCTGCTAGCCACCATGGACGCACAAACACGACGACGTGAGCGTCGC
GCTGAGAAACAAGCTCAATGGAAAGCTGCAAACCCACCGCTCGACGGAGCCGGAGCTGGCGCTGGAGCTGGAGCC
GGAGCTGGCGGTCTAGCCACCATGGACGCACAAACACGACGACGTGAGCGTCGCGCTGAGAAACAAGCTCAATGG
AAAGCTGCAAACCCACCGCTCGACGGAGCCGGAGCTGGCGCTGGAGCTGGAGCCGGAGCTGGCGGTCTAGCCACC
ATGGACGCACAAACACGACGACGTGAGCGTCGCGCTGAGAAACAAGCTCAATGGAAAGCTGCAAACCCACCGCTC
GACGGAGCCGGAGCTGGCGCTGGAGCTGGAGCCGGAGCTGGCGGTCTAGCCACCATGGACGCACAAACACGACGA
CGTGAGCGTCGCGCTGAGAAACAAGCTCAATGGAAAGCTGCAAACCCACCGCTCGAGTCTAGAGGGCCCGTTTAAA
CCCGCTGATCAGCCTCGACTGTGCCTTCTAGTTGCCAGCCATCTGTTGTTTGCCCCTCCCCCGTGCCTTCCTTGA
(SEQ ID NO:127)
Protein:
MGCVCSSNPEGTELASTDYSTYSQAAAQQGYSAYTAQPTQGYAQTTQAYGQQSYGTYGQPTDVSYTQAQTTATYG
QTAYATSYGQPPTGYTTPTAPQAYSQPVQGYGTGAYDTTTATVTTTQASYAAQSAYGTQPAYPAYGQQPAATAPT
RPQDGNKPTETSQPQSSTGGYNQPSLGYGQSNYSYPQVPGSYPMQPVTAPPSYPPTSYSSTQPTSYDQSSYSQQN
TYGQPSSYGQQSSYGQQSSYGQQPPTSYPPQTGSYSQAPSQYSQQSSSYGQQSSFRQDHPSSMGVYGQESGGFSG
PGENRSMSGPDNRGRGRGGFDRGGMSRGGRGGGRGGMGSAGERGGFNKPGGPMDEGPDLDLGPPVDPDEDSDNSA
IYVQGLNDSVTLDDLADFFKQCGVVKMNKRTGQPMIHIYLDKETGKPKGDATVSYEDPPTAKAAVEWFDGKDFQG
SKLKVSLARKKPPMNSMRGGLPPREGRGMPPPLRGGPGGPGGPGGPMGRMGGRGGDRGGFPPRGPRGSRGNPSGG
GNVQHRAGDWQCPNPGCGNQNFAWRTECNQCKAPKPEGFLPPPFPPPGGDRGRGGPGGMRGGRGGLMDRGGPGGM
FRGGRGGDRGGFRGGRGMDRGGFGGGRRGGPGGPPGPLMEQDYKDDDDKGTEQKLISEEDLLATMDAQTRRRERR
AEKQAQWKAANPPLDGAGAGAGAGAGAGGLATMDAQTRRRERRAEKQAQWKAANPPLDGAGAGAGAGAGAGGLAT
MDAQTRRRERRAEKQAQWKAANPPLDGAGAGAGAGAGAGGLATMDAQTRRRERRAEKQAQWKAANPPLESRGPVK
PADQPRLCLLVASHLLFAPPPCLP  (SEQ ID NO:128)

LcK-FUS-PylRSAF
DNA:
ATGGGCTGCGTGTGCAGCAGCAACCCCGAGGGTACCGAGCTCGCCTCAAACGATTATACCCAACAAGCAACCCAA
AGCTATGGGGCCTACCCCACCCAGCCCGGGCAGGGCTATTCCCAGCAGAGCAGTCAGCCCTACGGACAGCAGAGT
TACAGTGGTTATAGCCAGTCCACGGACACTTCAGGATATGGCCAGAGCAGCTATTCTTCTTATGGCCAGAGCCAG
AACACAGGCTATGGAACTCAGTCAACTCCCCAGGGATATGGCTCGACTGGCGGCTATGGCAGTAGCCAGAGCTCC

CAATCGTCTTACGGGCAGCAGTCCTCCTACCCTGGCTATGGCCAGCAGCCAGCTCCCAGCAACACCTCGGGAAGT
TACGGTAGCAGTTCTCAGAGCAGCAGCTATGGGCAGCCCCAGAGTGGGAGCTACAGCCAGCAGCCTAGCTATGGT
GGACAGCAGCAAAGCTATGGACAGCAGCAAAGCTATAATCCCCCTCAGGGCTATGGACAGCAGAACCAGTACAAC
AGCAGCAGTGGTGGTGGAGGTGGAGGTGGAGGTGGAGGTAACTATGGCCAAGATCAATCCTCCATGAGTAGTGGT
GGTGGCAGTGGTGGCGGTTATGGCAATCAAGACCAGAGTGGTGGAGGTGGCAGCGGTGGCTATGGACAGCAGGAC
CGTGGAGGCCGCGGCAGGGGTGGCAGTGGTGGCGGCGGCGGCGGCGGCGGTGGTGGTTACAACCGCAGCAGTGGT
GGCTATGAACCCAGAGGTCGTGGAGGTGGCCGTGGAGGCAGAGGTGGCATGGGCGGAAGTGACCGTGGTGGCTTC
AATAAATTTGGTGGCCCTCGGGACCAAGGATCACGTCATGACTCCGAACAGGATAATTCAGACAACAACACCATC
TTTGTGCAAGGCCTGGGTGAGAATGTTACAATTGAGTCTGTGGCTGATTACTTCAAGCAGATTGGTATTATTAAG
ACAAACAAGAAAACGGGACAGCCCATGATTAATTTGTACACAGACAGGGAAACTGGCAAGCTGAAGGGAGAGGCA
ACGGTCTCTTTTGATGACCCACCTTCAGCTAAAGCAGCTATTGACTGGTTTGATGGTAAAGAATTCTCCGGAAAT
CCTATCAAGGTCTCATTTGCTACTCGCCGGGCAGACTTTAATCGGGGTGGTGGCAATGGTCGTGGAGGCCGAGGG
CGAGGAGGACCCATGGGCCGTGGAGGCTATGGAGGTGGTGGCAGTGGTGGTGGTGGCCGAGGAGGATTTCCCAGT
GGAGGTGGTGGCGGTGGAGGACAGCAGCGAGCTGGTGACTGGAAGTGTCCTAATCCCACCTGTGAGAATATGAAC
TTCTCTTGGAGGAATGAATGCAACCAGTGTAAGGCCCCTAAACCAGATGGCCCAGGAGGGGGACCAGGTGGCTCT
CACATGGGGGGTAACTACGGGGATGATCGTCGTGGTGGCAGAGGAGGCGATTACAAGGATGACGACGATAAGGGT
ACCGGCGCCCCCGGCTCCGCCGGCTCCGCCGCCGGCTCCGGCGCGTGCCCGGTGCCGCTGCAGCTGCCGCCGCTG
GAACGCCTGACCCTGGATGATAAAAAACCGCTGAATACCCTGATCTCTGCTACTGGTCTGTGGATGAGTCGTACC
GGAACCATTCATAAAATCAAACACCACGAGGTTAGCCGTTCGAAAATCTATATTGAGATGGCGTGTGGCGATCAT
CTGGTTGTGAACAATAGCCGCTCTTCTCGTACAGCACGTGCACTGCGTCACCACAAATATCGTAAAACCTGTAAA
CGTTGCCGTGTGTCCGATGAGGATCTGAACAAATTCCTGACAAAAGCCAATGAGGACCAAACAAGCGTGAAAGTG
AAAGTCGTTAGCGCTCCTACCCGTACTAAAAAAGCAATGCCGAAATCCGTTGCTCGTGCCCCTAAACCACTGGAA
AACACTGAAGCAGCACAGGCACAGCCGTCTGGAAGCAAATTCTCTCCGGCCATTCCTGTTTCTACCCAGGAGTCC
GTTTCTGTTCCAGCAAGTGTGAGCACCAGCATTAGCAGTATTAGCACCGGTGCCACCGCTAGCGCCCTGGTTAAA
GGCAATACCAATCCGATTACAAGCATGTCTGCCCCGGTTCAAGCATCAGCTCCAGCACTGACAAAATCCCAAACC
GATCGTCTGGAGGTTCTGCTGAATCCGAAAGACGAAATCAGCCTGAATTCCGGCAAACCGTTTCGTGAACTGGAG
AGCGAACTGCTGTCACGTCGTAAAAAAGACCTGCAACAAATCTATGCCGAAGAACGTGAGAACTATCTGGGGAAA
CTGGAACGTGAAATCACCCGCTTTTTCGTGGATCGTGGCTTTCTGGAGATCAAATCCCCGATTCTGATTCCTCTG
GAGTATATCGAGCGTATGGGCATCGACAATGATACCGAACTGAGCAAACAAATTTTCCGTGTGGATAAAAACTTC
TGTCTGCGCCCTATGCTAGCACCAAATCTGGCTAACTATCTGCGCAAACTGGACCGTGCCCTGCCTGATCCTATC
AAAATCTTCGAGATCGGCCCGTGTTATCGTAAAGAGTCCGACGGTAAAGAACATCTGGAGGAGTTTACCATGCTG
AACTTTTGCCAAATGGGTTCAGGTTGTACTCGTGAGAACCTGGAAAGCATCATCACCGATTTTCTGAACCACCTG
GGCATTGACTTCAAAATTGTGGGCGACAGCTGTATGGTGTTTGGCGACACCCTGGATGTCATGCACGGCGACCTG
GAACTGTCTAGTGCCGTTGTGGGCCCAATCCCGCTGGATCGTGAGTGGGGTATCGACAAACCTTGGATCGGTGCG
GGTTTTGGTCTGGAGCGTCTGCTGAAAGTAAAACACGACTTCAAGAACATCAAACGTGCTGCACGTTCCGAGTCC
TATTACAATGGTATTTCTACTAACCTGTAA  (SEQ ID NO:129)
Protein:
MGCVCSSNPEGTELASNDYTQQATQSYGAYPTQPGQGYSQQSSQPYGQQSYSGYSQSTDTSGYGQSSYSSYGQSQ
NTGYGTQSTPQGYGSTGGYGSSQSSQSSYGQQSSYPGYGQQPAPSNTSGSYGSSSQSSSYGQPQSGSYSQQPSYG
GQQQSYGQQQSYNPPQGYGQQNQYNSSSGGGGGGGGGNYGQDQSSMSSGGGSGGGYGNQDQSGGGGSGGYGQQD
RGGRGRGGSGGGGGGGGGYNRSSGGYEPRGRGGGRGGRGGMGGSDRGGFNKFGGPRDQGSRHDSEQDNSDNNTI
FVQGLGENVTIESVADYFKQIGIIKTNKKTGQPMINLYTDRETGKLKGEATVSFDDPPSAKAAIDWFDGKEFSGN
PIKVSFATRRADFNRGGGNGRGGRGRGGPMGRGGYGGGGSGGGGRGGFPSGGGGGGGQQRAGDWKCPNPTCENMN
FSWRNECNQCKAPKPDGPGGGPGGSHMGGNYGDDRRGGRGGDYKDDDDKGTGAPGSAGSAAGSGACPVPLQLPPL
ERLTLDDKKPLNTLISATGLWMSRTGTIHKIKHHEVSRSKIYIEMACGDHLVVNNSRSSRTARALRHHKYRKTCK
RCRVSDEDLNKFLTKANEDQTSVKVKVVSAPTRTKKAMPKSVARAPKPLENTEAAQAQPSGSKFSPAIPVSTQES
VSVPASVSTSISSISTGATASALVKGNTNPITSMSAPVQASAPALTKSQTDRLEVLLNPKDEISLNSGKPFRELE
SELLSRRKKDLQQIYAEERENYLGKLEREITRFFVDRGFLEIKSPILIPLEYIERMGIDNDTELSKQIFRVDKNF
CLRPMLAPNLANYLRKLDRALPDPIKIFEIGPCYRKESDGKEHLEEFTMLNFCQMGSGCTRENLESIITDFLNHL
GIDFKIVGDSCMVFGDTLDVMHGDLELSSAVVGPIPLDREWGIDKPWIGAGFGLERLLKVKHDFKNIKRAARSES
YYNGISTNL  (SEQ ID NO:130)

LcK-FUS-MCP-PylRS^AF
DNA:
ATGGGCTGCGTGTGCAGCAGCAACCCCGAGGGTACCGAGCTCGCCTCAAACGATTATACCCAACAAGCAACCCAA
AGCTATGGGGCCTACCCCACCCAGCCCGGGCAGGGCTATTCCCAGCAGAGCAGTCAGCCCTACGGACAGCAGAGT
TACAGTGGTTATAGCCAGTCCACGGACACTTCAGGATATGGCCAGAGCAGCTATTCTTCTTATGGCCAGAGCCAG
AACACAGGCTATGGAACTCAGTCAACTCCCCAGGGATATGGCTCGACTGGCGGCTATGGCAGTAGCCAGAGCTCC

CAATCGTCTTACGGGCAGCAGTCCTCCTACCCTGGCTATGGCCAGCAGCCAGCTCCCAGCAGCACCTCGGGAAGT
TACGGTAGCAGTTCTCAGAGCAGCAGCTATGGGCAGCCCCAGAGTGGGAGCTACAGCCAGCAGCCTAGCTATGGT
GGACAGCAGCAAAGCTATGGACAGCAGCAAAGCTATAATCCCCCTCAGGGCTATGGACAGCAGAACCAGTACAAC
AGCAGCAGTGGTGGTGGAGGTGGAGGTGGAGGTGGAGGTAACTATGGCCAAGATCAATCCTCCATGAGTAGTGGT
GGTGGCAGTGGTGGCGGTTATGGCAATCAAGACCAGAGTGGTGGAGGTGGCAGCGGTGGCTATGGACAGCAGGAC
CGTGGAGGCCGCGGCAGGGGTGGCAGTGGTGGCGGCGGCGGCGGCGGTGGTGGTTACAACCGCAGCAGTGGT
GGCTATGAACCCAGAGGTCGTGGAGGTGGCCGTGGAGGCAGAGGTGGCATGGGCGGAAGTGACCGTGGTGGCTTC
AATAAATTTGGTGGCCCTCGGGACCAAGGATCACGTCATGACTCCGAACAGGATAATTCAGACAACAACACCATC
TTTGTGCAAGGCCTGGGTGAGAATGTTACAATTGAGTCTGTGGCTGATTACTTCAAGCAGATTGGTATTATTAAG
ACAAACAAGAAAACGGGACAGCCCATGATTAATTTGTACACAGACAGGGAAACTGGCAAGCTGAAGGGAGAGGCA
ACGGTCTCTTTTGATGACCCACCTTCAGCTAAAGCAGCTATTGACTGGTTTGATGGTAAAGAATTCTCCGGAAAT
CCTATCAAGGTCTCATTTGCTACTCGCCGGGCAGACTTTAATCGGGGTGGTGGCAATGGTCGTGGAGGCCGAGGG
CGAGGAGGACCCATGGGCCGTGGAGGCTATGGAGGTGGTGGCAGTGGTGGTGGTGGCCGAGGAGGATTTCCCAGT
GGAGGTGGTGGCGGTGGAGGACAGCAGCGAGCTGGTGACTGGAAGTGTCCTAATCCCACCTGTGAGAATATGAAC
TTCTCTTGGAGGAATGAATGCAACCAGTGTAAGGCCCCTAAACCAGATGGCCCAGGAGGGGGACCAGGTGGCTCT
CACATGGGGGGTAACTACGGGGATGATCGTCGTGGTGGCAGAGGAGGCGATTACAAGGATGACGACGATAAGGGT
ACCGGCGCCCCCGGCTCCGCCGGCTCCGCCGCCGGCTCCGGCGCTTCTAACTTTACTCAGTTCGTTCTCGTCGAC
AATGGCGGAACTGGCGACGTGACTGTCGCCCCAAGCAACTTCGCTAACGGGATCGCTGAATGGATCAGCTCTAAC
TCGCGTTCACAGGCTTACAAAGTAACCTGTAGCGTTCGTCAGAGCTCTGCGCAGAATCGCAAATACACCATCAAA
GTCGAGGTGCCTAAAGGCGCCTGGCGTTCGTACTTAAATATGGAACTAACCATTCCAATTTTCGCCACGAATTCC
GACTGCGAGCTTATTGTTAAGGCAATGCAAGGTCTCCTAAAAGATGGAAACCCGATTCCCTCAGCAATCGCAGCA
AACTCCGGCATCTACGGTACCGGCGCCCCCGGCTCCGCCGGCTCCGCCGCCGGCTCCGGCGCGTGCCCGGTGCCG
CTGCAGCTGCCGCCGCTGGAACGCCTGACCCTGGATGATAAAAAACCGCTGAATACCCTGATCTCTGCTACTGGT
CTGTGGATGAGTCGTACCGGAACCATTCATAAAATCAAACACCACGAGGTTAGCCGTTCGAAAATCTATATTGAG
ATGGCGTGTGGCGATCATCTGGTTGTGAACAATAGCCGCTCTTCTCGTACAGCACGTGCACTGCGTCACCACAAA
TATCGTAAAACCTGTAAACGTTGCCGTGTGTCCGATGAGGATCTGAACAAATTCCTGACAAAAGCCAATGAGGAC
CAAACAAGCGTGAAAGTGAAAGTCGTTAGCGCTCCTACCCGTACTAAAAAAGCAATGCCGAAATCCGTTGCTCGT
GCCCCTAAACCACTGGAAAACACTGAAGCAGCACAGGCACAGCCGTCTGGAAGCAAATTCTCTCCGGCCATTCCT
GTTTCTACCCAGGAGTCCGTTTCTGTTCCAGCAAGTGTGAGCACCAGCATTAGCAGTATTAGCACCGGTGCCACC
GCTAGCGCCCTGGTTAAAGGCAATACCAATCCGATTACAAGCATGTCTGCCCCGGTTCAAGCATCAGCTCCAGCA
CTGACAAAATCCCAAACCGATCGTCTGGAGGTTCTGCTGAATCCGAAAGACGAAATCAGCCTGAATTCCGGCAAA
CCGTTTCGTGAACTGGAGAGCGAACTGCTGTCACGTCGTAAAAAAGACCTGCAACAAATCTATGCCGAAGAACGT
GAGAACTATCTGGGGAAACTGGAACGTGAAATCACCCGCTTTTTCGTGGATCGTGGCTTTCTGGAGATCAAATCC
CCGATTCTGATTCCTCTGGAGTATATCGAGCGTATGGGCATCGACAATGATACCGAACTGAGCAAACAAATTTTC
CGTGTGGATAAAAACTTCTGTCTGCGCCCTATGCTAGCACCAAATCTGGCTAACTATCTGCGCAAACTGGACCGT
GCCCTGCCTGATCCTATCAAAATCTTCGAGATCGGCCCGTGTTATCGTAAAGAGTCCGACGGTAAAGAACATCTG
GAGGAGTTTACCATGCTGAACTTTTGCCAAATGGGTTCAGGTTGTACTCGTGAGAACCTGGAAAGCATCATCACC
GATTTTCTGAACCACCTGGGCATTGACTTCAAAATTGTGGGCGACAGCTGTATGGTGTTTGGCGACACCCTGGAT
GTCATGCACGGCGACCTGGAACTGTCTAGTGCCGTTGTGGGCCCAATCCCGCTGGATCGTGAGTGGGGTATCGAC
AAACCTTGGATCGGTGCGGGTTTTGGTCTGGAGCGTCTGCTGAAAGTAAAACACGACTTCAAGAACATCAAACGT
GCTGCACGTTCCGAGTCCTATTACAATGGTATTTCTACTAACCTGTAA (SEQ ID NO:131)
Protein:
MGCVCSSNPEGTELASNDYTQQATQSYGAYPTQPGQGYSQQSSQPYGQQSYSGYSQSTDTSGYGQSSYSSYGQSQ
NTGYGTQSTPQGYGSTGGYGSSQSSQSSYGQQSSYPGYGQQPAPSSTSGSYGSSSQSSSYGQPQSGSYSQQPSYG
GQQQSYGQQQSYNPPQGYGQQNQYNSSSGGGGGGGGGNYGQDQSSMSSGGGSGGGYGNQDQSGGGGSGGYGQQD
RGGRGRGGSGGGGGGGGGYNRSSGGYEPRGRGGGRGGRGGMGGSDRGGFNKFGGPRDQGSRHDSEQDNSDNNTI
FVQGLGENVTIESVADYFKQIGIIKTNKKTGQPMINLYTDRETGKLKGEATVSFDDPPSAKAAIDWFDGKEFSGN
PIKVSFATRRADFNRGGGNGRGGRGRGGPMGRGGYGGGGSGGGGRGGFPSGGGGGGGQQRAGDWKCPNPTCENMN
FSWRNECNQCKAPKPDGPGGGPGGSHMGGNYGDDRRGGRGGDYKDDDDKGTGAPGSAGSAAGSGASNFTQFVLVD
NGGTGDVTVAPSNFANGIAEWISSNSRSQAYKVTCSVRQSSAQNRKYTIKVEVPKGAWRSYLNMELTIPIFATNS
DCELIVKAMQGLLKDGNPIPSAIAANSGIYGTGAPGSAGSAAGSGACPVPLQLPPLERLTLDDKKPLNTLISATG
LWMSRTGTIHKIKHHEVSRSKIYIEMACGDHLVVNNSRSSRTARALRHHKYRKTCKRCRVSDEDLNKFLTKANED
QTSVKVKVVSAPTRTKKAMPKSVARAPKPLENTEAAQAQPSGSKFSPAIPVSTQESVSVPASVSTSISSISTGAT
ASALVKGNTNPITSMSAPVQASAPALTKSQTDRLEVLLNPKDEISLNSGKPFRELESELLSRRKKDLQQIYAEER
ENYLGKLEREITRFFVDRGFLEIKSPILIPLEYIERMGIDNDTELSKQIFRVDKNFCLRPMLAPNLANYLRKLDR
ALPDPIKIFEIGPCYRKESDGKEHLEEFTMLNFCQMGSGCTRENLESIITDFLNHLGIDFKIVGDSCMVFGDTLD
VMHGDLELSSAVVGPIPLDREWGIDKPWIGAGFGLERLLKVKHDFKNIKRAARSESYYNGISTNL (SEQ ID
NO:132)

LCK-FUS-3xMCP-PylRS^AF
DNA:
ATGGGCTGCGTGTGCAGCAGCAACCCCGAGGGTACCGAGCTCGCCTCAAACGATTATACCCAACAAGCAACCCAA
AGCTATGGGGCCTACCCCACCCAGCCCGGGCAGGGCTATTCCCAGCAGAGCAGTCAGCCCTACGGACAGCAGAGT
TACAGTGGTTATAGCCAGTCCACGGACACTTCAGGATATGGCCAGAGCAGCTATTCTTCTTATGGCCAGAGCCAG
AACACAGGCTATGGAACTCAGTCAACTCCCCAGGGATATGGCTCGACTGGCGGCTATGGCAGTAGCCAGAGCTCC
CAATCGTCTTACGGGCAGCAGTCCTCCTACCCTGGCTATGGCCAGCAGCCAGCTCCCAGCAACACCTCGGGAAGT
TACGGTAGCAGTTCTCAGAGCAGCAGCTATGGGCAGCCCCAGAGTGGGAGCTACAGCCAGCAGCCTAGCTATGGT
GGACAGCAGCAAAGCTATGGACAGCAGCAAAGCTATAATCCCCCTCAGGGCTATGGACAGCAGAACCAGTACAAC
AGCAGCAGTGGTGGTGGAGGTGGAGGTGGAGGTGGAGGTAACTATGGCCAAGATCAATCCTCCATGAGTAGTGGT
GGTGGCAGTGGTGGCGGTTATGGCAATCAAGACCAGAGTGGTGGAGGTGGCAGCGGTGGCTATGGACAGCAGGAC
CGTGGAGGCCGCGGCAGGGGTGGCAGTGGTGGCGGCGGCGGCGGCGGTGGTGGTTACAACCGCAGCAGTGGT
GGCTATGAACCCAGAGGTCGTGGAGGTGGCCGTGGAGGCAGAGGTGGCATGGGCGGAAGTGACCGTGGTGGCTTC
AATAAATTTGGTGGCCCTCGGGACCAAGGATCACGTCATGACTCCGAACAGGATAATTCAGACAACAACACCATC
TTTGTGCAAGGCCTGGGTGAGAATGTTACAATTGAGTCTGTGGCTGATTACTTCAAGCAGATTGGTATTATTAAG
ACAAACAAGAAAACGGGACAGCCCATGATTAATTTGTACACAGACAGGGAAACTGGCAAGCTGAAGGGAGAGGCA
ACGGTCTCTTTTGATGACCCACCTTCAGCTAAAGCAGCTATTGACTGGTTTGATGGTAAAGAATTCTCCGGAAAT
CCTATCAAGGTCTCATTTGCTACTCGCCGGGCAGACTTTAATCGGGGTGGTGGCAATGGTCGTGGAGGCCGAGGG
CGAGGAGGACCCATGGGCCGTGGAGGCTATGGAGGTGGTGGCAGTGGTGGTGGTGGCCGAGGAGGATTTCCCAGT
GGAGGTGGTGGCGGTGGAGGACAGCAGCGAGCTGGTGACTGGAAGTGTCCTAATCCCACCTGTGAGAATATGAAC
TTCTCTTGGAGGAATGAATGCAACCAGTGTAAGGCCCCTAAACCAGATGGCCCAGGAGGGGGACCAGGTGGCTCT
CACATGGGGGGTAACTACGGGGATGATCGTCGTGGTGGCAGAGGAGGCGAGCAGAAGCTGATCTCAGAGGAGGAC
CTGGGCGCCCCCGGCTCCGCCGGCTCCGCCGCCGGCTCCGGCGCTTCTAACTTTACTCAGTTCGTTCTCGTCGAC
AATGGCGGAACTGGCGACGTGACTGTCGCCCCAAGCAACTTCGCTAACGGGATCGCTGAATGGATCAGCTCTAAC
TCGCGTTCACAGGCTTACAAAGTAACCTGTAGCGTTCGTCAGAGCTCTGCGCAGAATCGCAAATACACCATCAAA
GTCGAGGTGCCTAAAGGCGCCTGGCGTTCGTACTTAAATATGGAACTAACCATTCCAATTTTCGCCACGAATTCC
GACTGCGAGCTTATTGTTAAGGCAATGCAAGGTCTCCTAAAAGATGGAAACCCGATTCCCTCAGCAATCGCAGCA
AACTCCGGCATCTACGGTGCCCCAGGCTCCGCAGGAAGCGCAGCGGGGTCCGGAGCTTCTAACTTTACTCAGTTC
GTTCTCGTCGACAATGGCGGAACTGGCGACGTGACTGTCGCCCCAAGCAACTTCGCTAACGGGATCGCTGAATGG
ATCAGCTCTAACTCGCGTTCACAGGCTTACAAAGTAACCTGTAGCGTTCGTCAGAGCTCTGCGCAGAATCGCAAA
TACACCATCAAAGTCGAGGTGCCTAAAGGCGCCTGGCGTTCGTACTTAAATATGGAACTAACCATTCCAATTTTC
GCCACGAATTCCGACTGCGAGCTTATTGTTAAGGCAATGCAAGGTCTCCTAAAAGATGGAAACCCGATTCCCTCA
GCAATCGCAGCAAACTCCGGCATCTACGGCGCACCTGGTAGTGCTGGTTCTGCTGCTGGATCAGGTGCTTCTAAC
TTTACTCAGTTCGTTCTCGTCGACAATGGCGGAACTGGCGACGTGACTGTCGCCCCAAGCAACTTCGCTAACGGG
ATCGCTGAATGGATCAGCTCTAACTCGCGTTCACAGGCTTACAAAGTAACCTGTAGCGTTCGTCAGAGCTCTGCG
CAGAATCGCAAATACACCATCAAAGTCGAGGTGCCTAAAGGCGCCTGGCGTTCGTACTTAAATATGGAACTAACC
ATTCCAATTTTCGCCACGAATTCCGACTGCGAGCTTATTGTTAAGGCAATGCAAGGTCTCCTAAAAGATGGAAAC
CCGATTCCCTCAGCAATCGCAGCAAACTCCGGCATCTACGGTACCGGCGCCCCCGGCTCCGCCGGCTCCGCCGCC
GGCTCCGGCGCGTGCCCGGTGCCGCTGCAGCTGCCGCCGCTGGAACGCCTGACCCTGGATGATAAAAAACCGCTG
AATACCCTGATCTCTGCTACTGGTCTGTGGATGAGTCGTACCGGAACCATTCATAAAATCAAACACCACGAGGTT
AGCCGTTCGAAAATCTATATTGAGATGGCGTGTGGCGATCATCTGGTTGTGAACAATAGCCGCTCTTCTCGTACA
GCACGTGCACTGCGTCACCACAAATATCGTAAAACCTGTAAACGTTGCCGTGTGTCCGATGAGGATCTGAACAAA
TTCCTGACAAAAGCCAATGAGGACCAAACAAGCGTGAAAGTGAAAGTCGTTAGCGCTCCTACCCGTACTAAAAAA
GCAATGCCGAAATCCGTTGCTCGTGCCCCTAAACCACTGGAAAACACTGAAGCAGCACAGGCACAGCCGTCTGGA
AGCAAATTCTCTCCGGCCATTCCTGTTTCTACCCAGGAGTCCGTTTCTGTTCCAGCAAGTGTGAGCACCAGCATT
AGCAGTATTAGCACCGGTGCCACCGCTAGCGCCCTGGTTAAAGGCAATACCAATCCGATTACAAGCATGTCTGCC
CCGGTTCAAGCATCAGCTCCAGCACTGACAAAATCCCAAACCGATCGTCTGGAGGTTCTGCTGAATCCGAAAGAC
GAAATCAGCCTGAATTCCGGCAAACCGTTTCGTGAACTGGAGAGCGAACTGCTGTCACGTCGTAAAAAAGACCTG
CAACAAATCTATGCCGAAGAACGTGAGAACTATCTGGGGAAACTGGAACGTGAAATCACCCGCTTTTTCGTGGAT
CGTGGCTTTCTGGAGATCAAATCCCCGATTCTGATTCCTCTGGAGTATATCGAGCGTATGGGCATCGACAATGAT
ACCGAACTGAGCAAACAAATTTTCCGTGTGGATAAAAACTTCTGTCTGCGCCCTATGCTAGCACCAAATCTGGCT
AACTATCTGCGCAAACTGGACCGTGCCCTGCCTGATCCTATCAAAATCTTCGAGATCGGCCCGTGTTATCGTAAA
GAGTCCGACGGTAAAGAACATCTGGAGGAGTTTACCATGCTGAACTTTTGCCAAATGGGTTCAGGTTGTACTCGT
GAGAACCTGGAAAGCATCATCACCGATTTTCTGAACCACCTGGGCATTGACTTCAAAATTGTGGGCGACAGCTGT
ATGGTGTTTGGCGACACCCTGGATGTCATGCACGGCGACCTGGAACTGTCTAGTGCCGTTGTGGGCCCAATCCCG
CTGGATCGTGAGTGGGGTATCGACAAACCTTGGATCGGTGCGGGTTTTGGTCTGGAGCGTCTGCTGAAAGTAAAA

CACGACTTCAAGAACATCAAACGTGCTGCACGTTCCGAGTCCTATTACAATGGTATTTCTACTAACCTGTAA
(SEQ ID NO:133)
Protein:
MGCVCSSNPEGTELASNDYTQQATQSYGAYPTQPGQGYSQQSSQPYGQQSYSGYSQSTDTSGYGQSSYSSYGQSQ
NTGYGTQSTPQGYGSTGGYGSSQSSQSSYGQQSSYPGYGQQPAPSNTSGSYGSSSQSSSYGQPQSGSYSQQPSYG
GQQQSYGQQQSYNPPQGYGQQNQYNSSSGGGGGGGGGGNYGQDQSSMSSGGGSGGGYGNQDQSGGGGSGGYGQQD
RGGRGRGGSGGGGGGGGGGYNRSSGGYEPRGRGGGRGGRGGMGGSDRGGFNKFGGPRDQGSRHDSEQDNSDNNTI
FVQGLGENVTIESVADYFKQIGIIKTNKKTGQPMINLYTDRETGKLKGEATVSFDDPPSAKAAIDWFDGKEFSGN
PIKVSFATRRADFNRGGGNGRGGRGRGGPMGRGGYGGGGSGGGGRGGFPSGGGGGGGQQRAGDWKCPNPTCENMN
FSWRNECNQCKAPKPDGPGGGPGGSHMGGNYGDDRRGGRGGEQKLISEEDLGAPGSAGSAAGSGASNFTQFVLVD
NGGTGDVTVAPSNFANGIAEWISSNSRSQAYKVTCSVRQSSAQNRKYTIKVEVPKGAWRSYLNMELTIPIFATNS
DCELIVKAMQGLLKDGNPIPSAIAANSGIYGAPGSAGSAAGSGASNFTQFVLVDNGGTGDVTVAPSNFANGIAEW
ISSNSRSQAYKVTCSVRQSSAQNRKYTIKVEVPKGAWRSYLNMELTIPIFATNSDCELIVKAMQGLLKDGNPIPS
AIAANSGIYGAPGSAGSAAGSGASNFTQFVLVDNGGTGDVTVAPSNFANGIAEWISSNSRSQAYKVTCSVRQSSA
QNRKYTIKVEVPKGAWRSYLNMELTIPIFATNSDCELIVKAMQGLLKDGNPIPSAIAANSGIYGTGAPGSAGSAA
GSGACPVPLQLPPLERLTLDDKKPLNTLISATGLWMSRTGTIHKIKHHEVSRSKIYIEMACGDHLVVNNSRSSRT
ARALRHHKYRKTCKRCRVSDEDLNKFLTKANEDQTSVKVKVVSAPTRTKKAMPKSVARAPKPLENTEAAQAQPSG
SKFSPAIPVSTQESVSVPASVSTSISSISTGATASALVKGNTNPITSMSAPVQASAPALTKSQTDRLEVLLNPKD
EISLNSGKPFRELESELLSRRKKDLQQIYAEERENYLGKLEREITRFFVDRGFLEIKSPILIPLEYIERMGIDND
TELSKQIFRVDKNFCLRPMLAPNLANYLRKLDRALPDPIKIFEIGPCYRKESDGKEHLEEFTMLNFCQMGSGCTR
ENLESIITDFLNHLGIDFKIVGDSCMVFGDTLDVMHGDLELSSAVVGPIPLDREWGIDKPWIGAGFGLERLLKVK
HDFKNIKRAARSESYYNGISTNL (SEQ ID NO:134)

LcK-FUS-PylRS^AAAF
DNA:
ATGGGCTGCGTGTGCAGCAGCAACCCCGAGGGTACCGAGCTCGCCTCAAACGATTATACCCAACAAGCAACCCAA
AGCTATGGGGCCTACCCCACCCAGCCCGGGCAGGGCTATTCCCAGCAGAGCAGTCAGCCCTACGGACAGCAGAGT
TACAGTGGTTATAGCCAGTCCACGGACACTTCAGGATATGGCCAGAGCAGCTATTCTTCTTATGGCCAGAGCCAG
AACACAGGCTATGGAACTCAGTCAACTCCCCAGGGATATGGCTCGACTGGCGGCTATGGCAGTAGCCAGAGCTCC
CAATCGTCTTACGGGCAGCAGTCCTCCTACCCTGGCTATGGCCAGCAGCCAGCTCCCAGCAACACCTCGGGAAGT
TACGGTAGCAGTTCTCAGAGCAGCAGCTATGGGCAGCCCCAGAGTGGGAGCTACAGCCAGCAGCCTAGCTATGGT
GGACAGCAGCAAAGCTATGGACAGCAGCAAAGCTATAATCCCCCTCAGGGCTATGGACAGCAGAACCAGTACAAC
AGCAGCAGTGGTGGTGGAGGTGGAGGTGGAGGTGGAGGTAACTATGGCCAAGATCAATCCTCCATGAGTAGTGGT
GGTGGCAGTGGTGGCGGTTATGGCAATCAAGACCAGAGTGGTGGAGGTGGCAGCGGTGGCTATGGACAGCAGGAC
CGTGGAGGCCGCGGCAGGGGTGGCAGTGGTGGCGGCGGCGGCGGCGGTGGTGGTTACAACCGCAGCAGTGGT
GGCTATGAACCCAGAGGTCGTGGAGGTGGCCGTGGAGGCAGAGGTGGCATGGGCGGAAGTGACCGTGGTGGCTTC
AATAAATTTGGTGGCCCTCGGGACCAAGGATCACGTCATGACTCCGAACAGGATAATTCAGACAACAACACCATC
TTTGTGCAAGGCCTGGGTGAGAATGTTACAATTGAGTCTGTGGCTGATTACTTCAAGCAGATTGGTATTATTAAG
ACAAACAAGAAAACGGGACAGCCCATGATTAATTTGTACACAGACAGGGAAACTGGCAAGCTGAAGGGAGAGGCA
ACGGTCTCTTTTGATGACCCACCTTCAGCTAAAGCAGCTATTGACTGGTTTGATGGTAAAGAATTCTCCGGAAAT
CCTATCAAGGTCTCATTTGCTACTCGCCGGGCAGACTTTAATCGGGGTGGTGGCAATGGTCGTGGAGGCCGAGGG
CGAGGAGGACCCATGGGCCGTGGAGGCTATGGAGGTGGTGGCAGTGGTGGTGGTGGCCGAGGAGGATTTCCCAGT
GGAGGTGGTGGCGGTGGAGGACAGCAGCGAGCTGGTGACTGGAAGTGTCCTAATCCCACCTGTGAGAATATGAAC
TTCTCTTGGAGGAATGAATGCAACCAGTGTAAGGCCCCTAAACCAGATGGCCCAGGAGGGGGACCAGGTGGCTCT
CACATGGGGGGTAACTACGGGGATGATCGTCGTGGTGGCAGAGGAGGCGATTACAAGGATGACGACGATAAGGGT
ACCGGCGCCCCCGGCTCCGCCGGCTCCGCCGCCGGCTCCGGCGCGTGCCCGGTGCCGCTGCAGCTGCCGCCGCTG
GAACGCCTGACCCTGGATGATAAAAAACCGCTGAATACCCTGATCTCTGCTACTGGTCTGTGGATGAGTCGTACC
GGAACCATTCATAAAATCAAACACCACGAGGTTAGCCGTTCGAAAATCTATATTGAGATGGCGTGTGGCGATCAT
CTGGTTGTGAACAATAGCCGCTCTTCTCGTACAGCACGTGCACTGCGTCACCACAAATATCGTAAAACCTGTAAA
CGTTGCCGTGTGTCCGATGAGGATCTGAACAAATTCCTGACAAAAGCCAATGAGGACCAAACAAGCGTGAAAGTG
AAAGTCGTTAGCGCTCCTACCCGTACTAAAAAAGCAATGCCGAAATCCGTTGCTCGTGCCCCTAAACCACTGGAA
AACACTGAAGCAGCACAGGCACAGCCGTCTGGAAGCAAATTCTCTCCGGCCATTCCTGTTTCTACCCAGGAGTCC
GTTTCTGTTCCAGCAAGTGTGAGCACCAGCATTAGCAGTATTAGCACCGGTGCCACCGCTAGCGCCCTGGTTAAA
GGCAATACCAATCCGATTACAAGCATGTCTGCCCCGGTTCAAGCATCAGCTCCAGCACTGACAAAATCCCAAACC
GATCGTCTGGAGGTTCTGCTGAATCCGAAAGACGAAATCAGCCTGAATTCCGGCAAACCGTTTCGTGAACTGGAG
AGCGAACTGCTGTCACGTCGTAAAAAAGACCTGCAACAAATCTATGCCGAAGAACGTGAGAACTATCTGGGGAAA
CTGGAACGTGAAATCACCCGCTTTTTCGTGGATCGTGGCTTTCTGGAGATCAAATCCCCGATTCTGATTCCTCTG
GAGTATATCGAGCGTATGGGCATCGACAATGATACCGAACTGAGCAAACAAATTTTCCGTGTGGATAAAAACTTC

TGTCTGCGCCCTATGCTAGCACCAAATCTGGCTAACTATCTGCGCAAACTGGACCGTGCCCTGCCTGATCCTATC
AAAATCTTCGAGATCGGCCCGTGTTATCGTAAAGAGTCCGACGGTAAAGAACATCTGGAGGAGTTTACCATGCTG
GCCTTTGCCCAAATGGGTTCAGGTTGTACTCGTGAGAACCTGGAAAGCATCATCACCGATTTTCTGAACCACCTG
GGCATTGACTTCAAAATTGTGGGCGACAGCTGTATGGTGTTTGGCGACACCCTGGATGTCATGCACGGCGACCTG
GAACTGTCTAGTGCCGTTGTGGGCCCAATCCCGCTGGATCGTGAGTGGGGTATCGACAAACCTTGGATCGGTGCG
GGTTTTGGTCTGGAGCGTCTGCTGAAAGTAAAACACGACTTCAAGAACATCAAACGTGCTGCACGTTCCGAGTCC
TATTACAATGGTATTTCTACTAACCTGTAA  (SEQ ID NO:135)
Protein:
MGCVCSSNPEGTELASNDYTQQATQSYGAYPTQPGQGYSQQSSQPYGQQSYSGYSQSTDTSGYGQSSYSSYGQSQ
NTGYGTQSTPQGYGSTGGYGSSQSSQSSYGQQSSYPGYGQQPAPSNTSGSYGSSSQSSSYGQPQSGSYSQQPSYG
GQQQSYGQQQSYNPPQGYGQQNQYNSSSGGGGGGGGGGNYGQDQSSMSSGGGSGGGYGNQDQSGGGGSGGYGQQD
RGGRGRGGSGGGGGGGGGGYNRSSGGYEPRGRGGGRGGRGGMGGSDRGGFNKFGGPRDQGSRHDSEQDNSDNNTI
FVQGLGENVTIESVADYFKQIGIIKTNKKTGQPMINLYTDRETGKLKGEATVSFDDPPSAKAAIDWFDGKEFSGN
PIKVSFATRRADFNRGGGNGRGGRGRGGPMGRGGYGGGGSGGGGRGGFPSGGGGGGGQQRAGDWKCPNPTCENMN
FSWRNECNQCKAPKPDGPGGGPGGSHMGGNYGDDRRGGRGGDYKDDDDKGTGAPGSAGSAAGSGACPVPLQLPPL
ERLTLDDKKPLNTLISATGLWMSRTGTIHKIKHHEVSRSKIYIEMACGDHLVVNNSRSSRTARALRHHKYRKTCK
RCRVSDEDLNKFLTKANEDQTSVKVKVVSAPTRTKKAMPKSVARAPKPLENTEAAQAQPSGSKFSPAIPVSTQES
VSVPASVSTSISSISTGATASALVKGNTNPITSMSAPVQASAPALTKSQTDRLEVLLNPKDEISLNSGKPFRELE
SELLSRRKKDLQQIYAEERENYLGKLEREITRFFVDRGFLEIKSPILIPLEYIERMGIDNDTELSKQIFRVDKNF
CLRPMLAPNLANYLRKLDRALPDPIKIFEIGPCYRKESDGKEHLEEFTMLAFAQMGSGCTRENLESIITDFLNHL
GIDFKIVGDSCMVFGDTLDVMHGDLELSSAVVGPIPLDREWGIDKPWIGAGFGLERLLKVKHDFKNIKRAARSES
YYNGISTNL  (SEQ ID NO:136)

LcK-PylRS^AF
DNA:
ATGGGCTGCGTGTGCAGCAGCAACCCCGAGGGTACCGAGCTCGCGTGCCCGGTGCCGCTGCAGCTGCCGCCGCTG
GAACGCCTGACCCTGGATGATAAAAAACCGCTGAATACCCTGATCTCTGCTACTGGTCTGTGGATGAGTCGTACC
GGAACCATTCATAAAATCAAACACCACGAGGTTAGCCGTTCGAAAATCTATATTGAGATGGCGTGTGGCGATCAT
CTGGTTGTGAACAATAGCCGCTCTTCTCGTACAGCACGTGCACTGCGTCACCACAAATATCGTAAAACCTGTAAA
CGTTGCCGTGTGTCCGATGAGGATCTGAACAAATTCCTGACAAAAGCCAATGAGGACCAAACAAGCGTGAAAGTG
AAAGTCGTTAGCGCTCCTACCCGTACTAAAAAAGCAATGCCGAAATCCGTTGCTCGTGCCCCTAAACCACTGGAA
AACACTGAAGCAGCACAGGCACAGCCGTCTGGAAGCAAATTCTCTCCGGCCATTCCTGTTTCTACCCAGGAGTCC
GTTTCTGTTCCAGCAAGTGTGAGCACCAGCATTAGCAGTATTAGCACCGGTGCCACCGCTAGCGCCCTGGTTAAA
GGCAATACCAATCCGATTACAAGCATGTCTGCCCCGGTTCAAGCATCAGCTCCAGCACTGACAAAATCCCAAACC
GATCGTCTGGAGGTTCTGCTGAATCCGAAAGACGAAATCAGCCTGAATTCCGGCAAACCGTTCCGTGAACTGGAG
AGCGAACTGCTGTCACGTCGTAAAAAAGACCTGCAACAAATCTATGCCGAAGAACGTGAGAACTATCTGGGGAAA
CTGGAACGTGAAATCACCCGCTTTTTCGTGGATCGTGGCTTTCTGGAGATCAAATCCCCGATTCTGATTCCTCTG
GAGTATATCGAGCGTATGGGCATCGACAATGATACCGAACTGAGCAAACAAATTTTCCGTGTGGATAAAAACTTC
TGTCTGCGCCCTATGCTAGCACCAAATCTGGCTAACTATCTGCGCAAACTGGACCGTGCCCTGCCTGATCCTATC
AAAATCTTCGAGATCGGCCCGTGTTATCGTAAAGAGTCCGACGGTAAAGAACATCTGGAGGAGTTTACCATGCTG
AACTTTTGCCAAATGGGTTCAGGTTGTACTCGTGAGAACCTGGAAAGCATCATCACCGATTTTCTGAACCACCTG
GGCATTGACTTCAAAATTGTGGGCGACAGCTGTATGGTGTTTGGCGACACCCTGGATGTCATGCACGGCGACCTG
GAACTGTCTAGTGCCGTTGTGGGCCCAATCCCGCTGGATCGTGAGTGGGGTATCGACAAACCTTGGATCGGTGCG
GGTTTTGGTCTGGAGCGTCTGCTGAAAGTAAAACACGACTTCAAGAACATCAAACGTGCTGCACGTTCCGAGTCC
TATTACAATGGTATTTCTACTAACCTGTAA (SEQ ID NO:137)
Protein:
MGCVCSSNPEGTELACPVPLQLPPLERLTLDDKKPLNTLISATGLWMSRTGTIHKIKHHEVSRSKIYIEMACGDH
LVVNNSRSSRTARALRHHKYRKTCKRCRVSDEDLNKFLTKANEDQTSVKVKVVSAPTRTKKAMPKSVARAPKPLE
NTEAAQAQPSGSKFSPAIPVSTQESVSVPASVSTSISSISTGATASALVKGNTNPITSMSAPVQASAPALTKSQT
DRLEVLLNPKDEISLNSGKPFRELESELLSRRKKDLQQIYAEERENYLGKLEREITRFFVDRGFLEIKSPILIPL
EYIERMGIDNDTELSKQIFRVDKNFCLRPMLAPNLANYLRKLDRALPDPIKIFEIGPCYRKESDGKEHLEEFTML
NFCQMGSGCTRENLESIITDFLNHLGIDFKIVGDSCMVFGDTLDVMHGDLELSSAVVGPIPLDREWGIDKPWIGA
GFGLERLLKVKHDFKNIKRAARSESYYNGISTNL  (SEQ ID NO:138)

LcK-PylRS^AAAF
DNA:
ATGGGCTGCGTGTGCAGCAGCAACCCCGAGGGTACCGAGCTCGCGTGCCCGGTGCCGCTGCAGCTGCCGCCGCTG
GAACGCCTGACCCTGGATGATAAAAAACCGCTGAATACCCTGATCTCTGCTACTGGTCTGTGGATGAGTCGTACC

GGAACCATTCATAAAATCAAACACCACGAGGTTAGCCGTTCGAAAATCTATATTGAGATGGCGTGTGGCGATCAT
CTGGTTGTGAACAATAGCCGCTCTTCTCGTACAGCACGTGCACTGCGTCACCACAAATATCGTAAAACCTGTAAA
CGTTGCCGTGTGTCCGATGAGGATCTGAACAAATTCCTGACAAAAGCCAATGAGGACCAAACAAGCGTGAAAGTG
AAAGTCGTTAGCGCTCCTACCCGTACTAAAAAAGCAATGCCGAAATCCGTTGCTCGTGCCCCTAAACCACTGGAA
AACACTGAAGCAGCACAGGCACAGCCGTCTGGAAGCAAATTCTCTCCGGCCATTCCTGTTTCTACCCAGGAGTCC
GTTTCTGTTCCAGCAAGTGTGAGCACCAGCATTAGCAGTATTAGCACCGGTGCCACCGCTAGCGCCCTGGTTAAA
GGCAATACCAATCCGATTACAAGCATGTCTGCCCCGGTTCAAGCATCAGCTCCAGCACTGACAAAATCCCAAACC
GATCGTCTGGAGGTTCTGCTGAATCCGAAAGACGAAATCAGCCTGAATTCCGGCAAACCGTTTCGTGAACTGGAG
AGCGAACTGCTGTCACGTCGTAAAAAAGACCTGCAACAAATCTATGCCGAAGAACGTGAGAACTATCTGGGGAAA
CTGGAACGTGAAATCACCCGCTTTTTCGTGGATCGTGGCTTTCTGGAGATCAAATCCCCGATTCTGATTCCTCTG
GAGTATATCGAGCGTATGGGCATCGACAATGATACCGAACTGAGCAAACAAATTTTCCGTGTGGATAAAAACTTC
TGTCTGCGCCCTATGCTAGCACCAAATCTGGCTAACTATCTGCGCAAACTGGACCGTGCCCTGCCTGATCCTATC
AAAATCTTCGAGATCGGCCCGTGTTATCGTAAAGAGTCCGACGGTAAAGAACATCTGGAGGAGTTTACCATGCTG
GCCTTTGCCCAAATGGGTTCAGGTTGTACTCGTGAGAACCTGGAAAGCATCATCACCGATTTTCTGAACCACCTG
GGCATTGACTTCAAAATTGTGGGCGACAGCTGTATGGTGTTTGGCGACACCCTGGATGTCATGCACGGCGACCTG
GAACTGTCTAGTGCCGTTGTGGGCCCAATCCCGCTGGATCGTGAGTGGGGTATCGACAAACCTTGGATCGGTGCG
GGTTTTGGTCTGGAGCGTCTGCTGAAAGTAAAACACGACTTCAAGAACATCAAACGTGCTGCACGTTCCGAGTCC
TATTACAATGGTATTTCTACTAACCTGTAA (SEQ ID NO:139)
Protein:
MGCVCSSNPEGTELACPVPLQLPPLERLTLDDKKPLNTLISATGLWMSRTGTIHKIKHHEVSRSKIYIEMACGDH
LVVNNSRSSRTARALRHHKYRKTCKRCRVSDEDLNKFLTKANEDQTSVKVKVVSAPTRTKKAMPKSVARAPKPLE
NTEAAQAQPSGSKFSPAIPVSTQESVSVPASVSTSISSISTGATASALVKGNTNPITSMSAPVQASAPALTKSQT
DRLEVLLNPKDEISLNSGKPFRELESELLSRRKKDLQQIYAEERENYLGKLEREITRFFVDRGFLEIKSPILIPL
EYIERMGIDNDTELSKQIFRVDKNFCLRPMLAPNLANYLRKLDRALPDPIKIFEIGPCYRKESDGKEHLEEFTML
AFAQMGSGCTRENLESIITDFLNHLGIDFKIVGDSCMVFGDTLDVMHGDLELSSAVVGPIPLDREWGIDKPWIGA
GFGLERLLKVKHDFKNIKRAARSESYYNGISTNL (SEQ ID NO:140)

LcK-MCP
DNA:
ATGGGCTGCGTGTGCAGCAGCAACCCCGAGGGTACCGAGCTCGAGCAGAAGCTGATCTCAGAGGAGGACCTGGGC
GCCCCCGGCTCCGCCGGCTCCGCCGCCGGCTCCGGCGCTTCTAACTTTACTCAGTTCGTTCTCGTCGACAATGGC
GGAACTGGCGACGTGACTGTCGCCCCAAGCAACTTCGCTAACGGGATCGCTGAATGGATCAGCTCTAACTCGCGT
TCACAGGCTTACAAAGTAACCTGTAGCGTTCGTCAGAGCTCTGCGCAGAATCGCAAATACACCATCAAAGTCGAG
GTGCCTAAAGGCGCCTGGCGTTCGTACTTAAATATGGAACTAACCATTCCAATTTTCGCCACGAATTCCGACTGC
GAGCTTATTGTTAAGGCAATGCAAGGTCTCCTAAAAGATGGAAACCCGATTCCCTCAGCAATCGCAGCAAACTCC
GGCATCTACTAA (SEQ ID NO:141)
Protein:
MGCVCSSNPEGTELEQKLISEEDLGAPGSAGSAAGSGASNFTQFVLVDNGGTGDVTVAPSNFANGIAEWISSNSR
SQAYKVTCSVRQSSAQNRKYTIKVEVPKGAWRSYLNMELTIPIFATNSDCELIVKAMQGLLKDGNPIPSAIAANS
GIY (SEQ ID NO:142)

FRB-CD28-FUS-PylRS^AF
DNA:
ATGTGCCGAGCCATCTCTCTTAGGCGCTTGCTGCTGCTGCTGCAGCTGTCACAACTCCTAGCTGTCACTCAA
GGGATGCTCGAGATGTGGCATGAAGGCCTGGAAGAGGCATCTCGTTTGTACTTTGGGGAAAGGAACGTGAAAGGC
ATGTTTGAGGTGCTGGAGCCCTTGCATGCTATGATGGAACGGGGCCCCCAGACTCTGAAGGAAACATCCTTTAAT
CAGGCCTATGGTCGAGATTTAATGGAGGCCCAAGAGTGGTGCAGGAAGTACATGAAATCAGGGAATGTCAAGGAC
CTCCTCCAAGCCTGGGACCTCTATTATCATGTGTTCCGACGAATCTCAAAGACTAGAACCGGTAAGCTTTTTTGG
GCACTGGTCGTGGTTGCTGGAGTCCTGTTTTGTTATGGCTTGCTAGTGACAGTGGCTCTTTGTGTTATCTGGGTA
AGATCTGGTATGGCCTCAAACGATTATACCCAACAAGCAACCCAAAGCTATGGGGCCTACCCCACCCAGCCCGGG
CAGGGCTATTCCCAGCAGAGCAGTCAGCCCTACGGACAGCAGAGTTACAGTGGTTATAGCCAGTCCACGGACACT
TCAGGATATGGCCAGAGCAGCTATTCTTCTTATGGCCAGAGCCAGAACACAGGCTATGGAACTCAGTCAACTCCC
CAGGGATATGGCTCGACTGGCGGCTATGGCAGTAGCCAGAGCTCCCAATCGTCTTACGGGCAGCAGTCCTCCTAC
CCTGGCTATGGCCAGCAGCCAGCTCCCAGCAGCACCTCGGGAAGTTACGGTAGCAGTTCTCAGAGCAGCAGCTAT
GGGCAGCCCCAGAGTGGGAGCTACAGCCAGCAGCCTAGCTATGGTGGACAGCAGCAAAGCTATGGACAGCAGCAA
AGCTATAATCCCCCTCAGGGCTATGGACAGCAGAACCAGTACAACAGCAGCAGTGGTGGTGGAGGTGGAGGTGGA
GGTGGAGGTAACTATGGCCAAGATCAATCCTCCATGAGTAGTGGTGGTGGCAGTGGTGGCGGTTATGGCAATCAA
GACCAGAGTGGTGGAGGTGGCAGCGGTGGCTATGGACAGCAGGACCGTGGAGGCCGCGGCAGGGGTGGCAGTGGT

GGCGGCGGCGGCGGCGGCGGTGGTGGTTACAACCGCAGCAGTGGTGGCTATGAACCCAGAGGTCGTGGAGGTGGC
CGTGGAGGCAGAGGTGGCATGGGCGGAAGTGACCGTGGTGGCTTCAATAAATTTGGTGGCCCTCGGGACCAAGGA
TCACGTCATGACTCCGAACAGGATAATTCAGACAACAACACCATCTTTGTGCAAGGCCTGGGTGAGAATGTTACA
ATTGAGTCTGTGGCTGATTACTTCAAGCAGATTGGTATTATTAAGACAAACAAGAAAACGGGACAGCCCATGATT
AATTTGTACACAGACAGGGAAACTGGCAAGCTGAAGGGAGAGGCAACGGTCTCTTTTGATGACCCACCTTCAGCT
AAAGCAGCTATTGACTGGTTTGATGGTAAAGAATTCTCCGGAAATCCTATCAAGGTCTCATTTGCTACTCGCCGG
GCAGACTTTAATCGGGGTGGTGGCAATGGTCGTGGAGGCCGAGGGCGAGGAGGACCCATGGGCCGTGGAGGCTAT
GGAGGTGGTGGCAGTGGTGGTGGTGGCCGAGGAGGATTTCCCAGTGGAGGTGGTGGCGGTGGAGGACAGCAGCGA
GCTGGTGACTGGAAGTGTCCTAATCCCACCTGTGAGAATATGAACTTCTCTTGGAGGAATGAATGCAACCAGTGT
AAGGCCCCTAAACCAGATGGCCCAGGAGGGGGACCAGGTGGCTCTCACATGGGGGGTAACTACGGGGATGATCGT
CGTGGTGGCAGAGGAGGCGGCGCCCCCGGCTCCGCCGGCTCCGCCGCCGGCTCCGGCATGGCGTGCCCGGTGCCG
CTGCAGCTGCCGCCGCTGGAACGCCTGACCCTGGATGATAAAAAACCGCTGAATACCCTGATCTCTGCTACTGGT
CTGTGGATGAGTCGTACCGGAACCATTCATAAAATCAAACACCACGAGGTTAGCCGTTCGAAAATCTATATTGAG
ATGGCGTGTGGCGATCATCTGGTTGTGAACAATAGCCGCTCTTCTCGTACAGCACGTGCACTGCGTCACCACAAA
TATCGTAAAACCTGTAAACGTTGCCGTGTGTCCGATGAGGATCTGAACAAATTCCTGACAAAAGCCAATGAGGAC
CAAACAAGCGTGAAAGTGAAAGTCGTTAGCGCTCCTACCCGTACTAAAAAAGCAATGCCGAAATCCGTTGCTCGT
GCCCCTAAACCACTGGAAAACACTGAAGCAGCACAGGCACAGCCGTCTGGAAGCAAATTCTCTCCGGCCATTCCT
GTTTCTACCCAGGAGTCCGTTTCTGTTCCAGCAAGTGTGAGCACCAGCATTAGCAGTATTAGCACCGGTGCCACC
GCTAGCGCCCTGGTTAAAGGCAATACCAATCCGATTACAAGCATGTCTGCCCCGGTTCAAGCATCAGCTCCAGCA
CTGACAAAATCCCAAACCGATCGTCTGGAGGTTCTGCTGAATCCGAAAGACGAAATCAGCCTGAATTCCGGCAAA
CCGTTTCGTGAACTGGAGAGCGAACTGCTGTCACGTCGTAAAAAAGACCTGCAACAAATCTATGCCGAAGAACGT
GAGAACTATCTGGGGAAACTGGAACGTGAAATCACCCGCTTTTTCGTGGATCGTGGCTTTCTGGAGATCAAATCC
CCGATTCTGATTCCTCTGGAGTATATCGAGCGTATGGGCATCGACAATGATACCGAACTGAGCAAACAAATTTTC
CGTGTGGATAAAAACTTCTGTCTGCGCCCTATGCTAGCACCAAATCTGGCTAACTATCTGCGCAAACTGGACCGT
GCCCTGCCTGATCCTATCAAAATCTTCGAGATCGGCCCGTGTTATCGTAAAGAGTCCGACGGTAAAGAACATCTG
GAGGAGTTTACCATGCTGAACTTTTGCCAAATGGGTTCAGGTTGTACTCGTGAGAACCTGGAAAGCATCATCACC
GATTTTCTGAACCACCTGGGCATTGACTTCAAAATTGTGGGCGACAGCTGTATGGTGTTTGGCGACACCCTGGAT
GTCATGCACGGCGACCTGGAACTGTCTAGTGCCGTTGTGGGCCCAATCCCGCTGGATCGTGAGTGGGGTATCGAC
AAACCTTGGATCGGTGCGGGTTTTGGTCTGGAGCGTCTGCTGAAAGTAAAACACGACTTCAAGAACATCAAACGT
GCTGCACGTTCCGAGTCCTATTACAATGGTATTTCTACTAACCTGTAA (SEQ ID NO:143)
Protein:
MCRAISLRRLLLLLLQLSQLLAVTQGMLEMWHEGLEEASRLYFGERNVKGMFEVLEPLHAMMERGPQTLKETSFN
QAYGRDLMEAQEWCRKYMKSGNVKDLLQAWDLYYHVFRRISKTRTGKLFWALVVVAGVLFCYGLLVTVALCVIWV
RSGMASNDYTQQATQSYGAYPTQPGQGYSQQSSQPYGQQSYSGYSQSTDTSGYGQSSYSSYGQSQNTGYGTQSTP
QGYGSTGGYGSSQSSQSSYGQQSSYPGYGQQPAPSSTSGSYGSSSQSSSYGQPQSGSYSQQPSYGGQQQSYGQQQ
SYNPPQGYGQQNQYNSSSGGGGGGGGGGNYGQDQSSMSSGGGSGGGYGNQDQSGGGGSGGYGQQDRGGRGRGGSG
GGGGGGGGGYNRSSGGYEPRGRGGGRGGRGGMGGSDRGGFNKFGGPRDQGSRHDSEQDNSDNNTIFVQGLGENVT
IESVADYFKQIGIIKTNKKTGQPMINLYTDRETGKLKGEATVSFDDPPSAKAAIDWFDGKEFSGNPIKVSFATRR
ADFNRGGGNGRGGRGRGGPMGRGGYGGGGSGGGGRGGFPSGGGGGGGQQRAGDWKCPNPTCENMNFSWRNECNQC
KAPKPDGPGGGPGGSHMGGNYGDDRRGGRGGGAPGSAGSAAGSGMACPVPLQLPPLERLTLDDKKPLNTLISATG
LWMSRTGTIHKIKHHEVSRSKIYIEMACGDHLVVNNSRSSRTARALRHHKYRKTCKRCRVSDEDLNKFLTKANED
QTSVKVKVVSAPTRTKKAMPKSVARAPKPLENTEAAQAQPSGSKFSPAIPVSTQESVSVPASVSTSISSISTGAT
ASALVKGNTNPITSMSAPVQASAPALTKSQTDRLEVLLNPKDEISLNSGKPFRELESELLSRRKKDLQQIYAEER
ENYLGKLEREITRFFVDRGFLEIKSPILIPLEYIERMGIDNDTELSKQIFRVDKNFCLRPMLAPNLANYLRKLDR
ALPDPIKIFEIGPCYRKESDGKEHLEEFTMLNFCQMGSGCTRENLESIITDFLNHLGIDFKIVGDSCMVFGDTLD
VMHGDLELSSAVVGPIPLDREWGIDKPWIGAGFGLERLLKVKHDFKNIKRAARSESYYNGISTNL (SEQ ID
NO:144)

FRB-CD28-FUS-PylRS^AA
DNA:
ATGTGCCGAGCCATCTCTCTTAGGCGCTTGCTGCTGCTGCTGCTGCAGCTGTCACAACTCCTAGCTGTCACTCAA
GGGATGCTCGAGATGTGGCATGAAGGCCTGGAAGAGGCATCTCGTTTGTACTTTGGGGAAAGGAACGTGAAAGGC
ATGTTTGAGGTGCTGGAGCCCTTGCATGCTATGATGGAACGGGGCCCCCAGACTCTGAAGGAAACATCCTTTAAT
CAGGCCTATGGTCGAGATTTAATGGAGGCCCAAGAGTGGTGCAGGAAGTACATGAAATCAGGGAATGTCAAGGAC
CTCCTCCAAGCCTGGGACCTCTATTATCATGTGTTCCGACGAATCTCAAAGACTAGAACCGGTAAGCTTTTTTGG
GCACTGGTCGTGGTTGCTGGAGTCCTGTTTTGTTATGGCTTGCTAGTGACAGTGGCTCTTTGTGTTATCTGGGTA
AGATCTGGTATGGCCTCAAACGATTATACCCAACAAGCAACCCAAAGCTATGGGGCCTACCCCACCCAGCCCGGG
CAGGGCTATTCCCAGCAGAGCAGTCAGCCCTACGGACAGCAGAGTTACAGTGGTTATAGCCAGTCCACGGACACT

TCAGGATATGGCCAGAGCAGCTATTCTTCTTATGGCCAGAGCCAGAACACAGGCTATGGAACTCAGTCAACTCCC
CAGGGATATGGCTCGACTGGCGGCTATGGCAGTAGCCAGAGCTCCCAATCGTCTTACGGGCAGCAGTCCTCCTAC
CCTGGCTATGGCCAGCAGCCAGCTCCCAGCAGCACCTCGGGAAGTTACGGTAGCAGTTCTCAGAGCAGCAGCTAT
GGGCAGCCCCAGAGTGGGAGCTACAGCCAGCAGCCTAGCTATGGTGGACAGCAGCAAAGCTATGGACAGCAGCAA
AGCTATAATCCCCCTCAGGGCTATGGACAGCAGAACCAGTACAACAGCAGCAGTGGTGGTGGAGGTGGAGGTGGA
GGTGGAGGTAACTATGGCCAAGATCAATCCTCCATGAGTAGTGGTGGTGGCAGTGGTGGCGGTTATGGCAATCAA
GACCAGAGTGGTGGAGGTGGCAGCGGTGGCTATGGACAGCAGGACCGTGGAGGCCGCGGCAGGGGTGGCAGTGGT
GGCGGCGGCGGCGGCGGCGGTGGTGGTTACAACCGCAGCAGTGGTGGCTATGAACCCAGAGGTCGTGGAGGTGGC
CGTGGAGGCAGAGGTGGCATGGGCGGAAGTGACCGTGGTGGCTTCAATAAATTTGGTGGCCCTCGGGACCAAGGA
TCACGTCATGACTCCGAACAGGATAATTCAGACAACAACACCATCTTTGTGCAAGGCCTGGGTGAGAATGTTACA
ATTGAGTCTGTGGCTGATTACTTCAAGCAGATTGGTATTATTAAGACAAACAAGAAAACGGGACAGCCCATGATT
AATTTGTACACAGACAGGGAAACTGGCAAGCTGAAGGGAGAGGCAACGGTCTCTTTTGATGACCCACCTTCAGCT
AAAGCAGCTATTGACTGGTTTGATGGTAAAGAATTCTCCGGAAATCCTATCAAGGTCTCATTTGCTACTCGCCGG
GCAGACTTTAATCGGGGTGGTGGCAATGGTCGTGGAGGCCGAGGGCGAGGAGGACCCATGGGCCGTGGAGGCTAT
GGAGGTGGTGGCAGTGGTGGTGGTGGCCGAGGAGGATTTCCCAGTGGAGGTGGTGGCGGTGGAGGACAGCAGCGA
GCTGGTGACTGGAAGTGTCCTAATCCCACCTGTGAGAATATGAACTTCTCTTGGAGGAATGAATGCAACCAGTGT
AAGGCCCCTAAACCAGATGGCCCAGGAGGGGGACCAGGTGGCTCTCACATGGGGGGTAACTACGGGGATGATCGT
CGTGGTGGCAGAGGAGGCGGCGCCCCCGGCTCCGCCGGCTCCGCCGCCGGCTCCGGCATGGCGTGCCCGGTGCCG
CTGCAGCTGCCGCCGCTGGAACGCCTGACCCTGGATGACAAAAAACCGCTGAATACCCTGATCTCTGCTACTGGT
CTGTGGATGAGTCGTACCGGAACCATTCATAAAATCAAACACCACGAGGTTAGCCGTTCGAAAATCTATATTGAG
ATGGCGTGTGGCGATCATCTGGTTGTGAACAATAGCCGCTCTTCTCGTACAGCACGTGCACTGCGTCACCACAAA
TATCGTAAAACCTGTAAACGTTGCCGTGTGTCCGATGAGGATCTGAACAAATTCCTGACAAAAGCCAATGAGGAC
CAAACAAGCGTGAAAGTGAAAGTCGTTAGCGCTCCTACCCGTACTAAAAAAGCAATGCCGAAATCCGTTGCTCGT
GCCCCTAAACCACTGGAAAACACTGAAGCAGCACAGGCACAGCCGTCTGGAAGCAAATTCTCTCCGGCCATTCCT
GTTTCTACCCAGGAGTCCGTTTCTGTTCCAGCAAGTGTGAGCACCAGCATTAGCAGTATTAGCACCGGTGCCACC
GCTAGCGCCCTGGTTAAAGGCAATACCAATCCGATTACAAGCATGTCTGCCCCGGTTCAAGCATCAGCTCCAGCA
CTGACAAAATCCCAAACCGATCGTCTGGAGGTTCTGCTGAATCCGAAAGACGAAATCAGCCTGAATTCCGGCAAA
CCGTTTCGTGAACTGGAGAGCGAACTGCTGTCACGTCGTAAAAAAGACCTGCAACAAATCTATGCCGAAGAACGT
GAGAACTATCTGGGGAAACTGGAACGTGAAATCACCCGCTTTTTCGTGGATCGTGGCTTTCTGGAGATCAAATCC
CCGATTCTGATTCCTCTGGAGTATATCGAGCGTATGGGCATCGACAATGATACCGAACTGAGCAAACAAATTTTC
CGTGTGGATAAAAACTTCTGTCTTCGCCCTATGCTGGCACCAAATCTGTATAACTATCTGCGCAAACTGGACCGT
GCCCTGCCTGATCCTATCAAAATCTTCGAGATCGGCCCGTGTTATCGTAAAGAGTCCGACGGTAAAGAACATCTG
GAGGAGTTTACCATGCTGGCCTTTGCCCAAATGGGTTCAGGTTGTACTCGTGAGAACCTGGAAAGCATCATCACC
GATTTTCTGAACCACCTGGGCATTGACTTCAAAATTGTGGGCGACAGCTGTATGGTGTATGGCGACACCCTGGAT
GTCATGCACGGCGACCTGGAACTGTCTAGTGCCGTTGTTGGACCAATTCCGCTGGACCGTGAGTGGGGTATCGAC
AAACCGTGGATCGGAGCAGGATTCGGTCTGGAACGCCTGCTGAAAGTGAAACACGACTTCAAAAACATCAAACGT
GCCGCCCGTTCTGAATCGTATTATAACGGGATCTCTACGAACCTGTAA (SEQ ID NO:145)
Protein:
MCRAISLRRLLLLLLQLSQLLAVTQGMLEMWHEGLEEASRLYFGERNVKGMFEVLEPLHAMMERGPQTLKETSFN
QAYGRDLMEAQEWCRKYMKSGNVKDLLQAWDLYYHVFRRISKTRTGKLFWALVVVAGVLFCYGLLVTVALCVIWV
RSGMASNDYTQQATQSYGAYPTQPGQGYSQQSSQPYGQQSYSGYSQSTDTSGYGQSSYSSYGQSQNTGYGTQSTP
QGYGSTGGYGSSQSSQSSYGQQSSYPGYGQQPAPSSTSGSYGSSSQSSSYGQPQSGSYSQQPSYGGQQQSYGQQQ
SYNPPQGYGQQNQYNSSSGGGGGGGGGNYGQDQSSMSSGGGSGGGYGNQDQSGGGGSGGYGQQDRGGRGRGGSG
GGGGGGGGGYNRSSGGYEPRGRGGGRGGRGGMGGSDRGGFNKFGGPRDQGSRHDSEQDNSDNNTIFVQGLGENVT
IESVADYFKQIGIIKTNKKTGQPMINLYTDRETGKLKGEATVSFDDPPSAKAAIDWFDGKEFSGNPIKVSFATRR
ADFNRGGGNGRGGRGRGGPMGRGGYGGGGSGGGGRGGFPSGGGGGGGQQRAGDWKCPNPTCENMNFSWRNECNQC
KAPKPDGPGGGPGGSHMGGNYGDDRRGGRGGGAPGSAGSAAGSGMACPVPLQLPPLERLTLDDKKPLNTLISATG
LWMSRTGTIHKIKHHEVSRSKIYIEMACGDHLVVNNSRSSRTARALRHHKYRKTCKRCRVSDEDLNKFLTKANED
QTSVKVKVVSAPTRTKKAMPKSVARAPKPLENTEAAQAQPSGSKFSPAIPVSTQESVSVPASVSTSISSISTGAT
ASALVKGNTNPITSMSAPVQASAPALTKSQTDRLEVLLNPKDEISLNSGKPFRELESELLSRRKKDLQQIYAEER
ENYLGKLEREITRFFVDRGFLEIKSPILIPLEYIERMGIDNDTELSKQIFRVDKNFCLRPMLAPNLYNYLRKLDR
ALPDPIKIFEIGPCYRKESDGKEHLEEFTMLAFAQMGSGCTRENLESIITDFLNHLGIDFKIVGDSCMVYGDTLD
VMHGDLELSSAVVGPIPLDREWGIDKPWIGAGFGLERLLKVKHDFKNIKRAARSESYYNGISTNL (SEQ ID
NO:146)

FRB-CD28-EWSR1-MCP
DNA:

ATGTGCCGAGCCATCTCTCTTAGGCGCTTGCTGCTGCTGCTGCAGCTGTCACAACTCCTAGCTGTCACTCAA
GGGATGCTCGAGATGTGGCATGAAGGCCTGGAAGAGGCATCTCGTTTGTACTTTGGGGAAAGGAACGTGAAAGGC
ATGTTTGAGGTGCTGGAGCCCTTGCATGCTATGATGGAACGGGGCCCCCAGACTCTGAAGGAAACATCCTTTAAT
CAGGCCTATGGTCGAGATTTAATGGAGGCCCAAGAGTGGTGCAGGAAGTACATGAAATCAGGGAATGTCAAGGAC
CTCCTCCAAGCCTGGGACCTCTATTATCATGTGTTCCGACGAATCTCAAAGACTAGAACCGGTAAGCTTTTTTGG
GCACTGGTCGTGGTTGCTGGAGTCCTGTTTTGTTATGGCTTGCTAGTGACAGTGGCTCTTTGTGTTATCTGGGTA
AGATCTATGGCGTCCACGGATTACAGTACCTATAGCCAAGCTGCAGCGCAGCAGGGCTACAGTGCTTACACCGCC
CAGCCCACTCAAGGATATGCACAGACCACCCAGGCATATGGGCAACAAAGCTATGGAACCTATGGACAGCCCACT
GATGTCAGCTATACCCAGGCTCAGACCACTGCAACCTATGGGCAGACCGCCTATGCAACTTCTTATGGACAGCCT
CCCACTGGTTATACTACTCCAACTGCCCCCCAGGCATACAGCCAGCCTGTCCAGGGGTATGGCACTGGTGCTTAT
GATACCACCACTGCTACAGTCACCACCACCCAGGCCTCCTATGCAGCTCAGTCTGCATATGGCACTCAGCCTGCT
TATCCAGCCTATGGGCAGCAGCCAGCAGCCACTGCACCTACAAGACCGCAGGATGGAAACAAGCCCACTGAGACT
AGTCAACCTCAATCTAGCACAGGGGGTTACAACCAGCCCAGCCTAGGATATGGACAGAGTAACTACAGTTATCCC
CAGGTACCTGGGAGCTACCCCATGCAGCCAGTCACTGCACCTCCATCCTACCCTCCTACCAGCTATTCCTCTACA
CAGCCGACTAGTTATGATCAGAGCAGTTACTCTCAGCAGAACACCTATGGGCAACCGAGCAGCTATGGACAGCAG
AGTAGCTATGGTCAACAAAGCAGCTATGGGCAGCAGCCTCCCACTAGTTACCCACCCCAAACTGGATCCTACAGC
CAAGCTCCAAGTCAATATAGCCAACAGAGCAGCAGCTACGGGCAGCAGAGTTCATTCCGACAGGACCACCCCAGT
AGCATGGGTGTTTATGGGCAGGAGTCTGGAGGATTTTCCGGACCAGGAGAGAACCGGAGCATGAGTGGCCCTGAT
AACCGGGGCAGGGGAAGAGGGGGATTTGATCGTGGAGGCATGAGCAGAGGTGGGCGGGGAGGAGGACGCGGTGGA
ATGGGCAGCGCTGGAGAGCGAGGTGGCTTCAATAAGCCTGGTGGACCCATGGATGAAGGACCAGATCTTGATCTA
GGCCCACCTGTAGATCCAGATGAAGACTCTGACAACAGTGCAATTTATGTACAAGGATTAAATGACAGTGTGACT
CTAGATGATCTGGCAGACTTCTTTAAGCAGTGTGGGGTTGTTAAGATGAACAAGAGAACTGGGCAACCCATGATC
CACATCTACCTGGACAAGGAAACAGGAAAGCCCAAAGGCGATGCCACAGTGTCCTATGAAGACCCACCTACTGCC
AAGGCTGCCGTGGAATGGTTTGATGGGAAAGATTTTCAAGGGAGCAAACTTAAAGTCTCCCTTGCTCGGAAGAAG
CCTCCAATGAACAGTATGCGGGGTGGTCTGCCACCCCGTGAGGGCAGAGGCATGCCACCACCACTCCGTGGAGGT
CCAGGAGGCCCAGGAGGTCCTGGGGGACCCATGGGTCGCATGGGAGGCCGTGGAGGAGATAGAGGAGGCTTCCCT
CCAAGAGGACCCCGGGGTTCCCGAGGGAACCCCTCTGGAGGAGGAAACGTCCAGCACCGAGCTGGAGACTGGCAG
TGTCCCAATCCGGGTTGTGGAAACCAGAACTTCGCCTGGAGAACAGAGTGCAACCAGTGTAAGGCCCCAAAGCCT
GAAGGCTTCCTCCCGCCACCCTTTCCGCCCCCGGGTGGTGATCGTGGCAGAGGTGGCCCTGGTGGCATGCGGGGA
GGAAGAGGTGGCCTCATGGATCGTGGTGGTCCCGGTGGAATGTTCAGAGGTGGCCGTGGTGGAGACAGAGGTGGC
TTCCGTGGTGGCCGGGGCATGGACCGAGGTGGCTTTGGTGGAGGAAGACGAGGTGGCCCTGGGGGGCCCCCTGGA
CCTTTGATGGAACAGGATTACAAGGATGACGACGATAAGGGTACCGAGCAGAAGCTGATCTCAGAGGAGGACCTG
GGCGCCCCCGGCTCCGCCGGCTCCGCCGCCGGCTCCGGCGCTTCTAACTTTACTCAGTTCGTTCTCGTCGACAAT
GGCGGAACTGGCGACGTGACTGTCGCCCCAAGCAACTTCGCTAACGGGATCGCTGAATGGATCAGCTCTAACTCG
CGTTCACAGGCTTACAAAGTAACCTGTAGCGTTCGTCAGAGCTCTGCGCAGAATCGCAAATACACCATCAAAGTC
GAGGTGCCTAAAGGCGCCTGGCGTTCGTACTTAAATATGGAACTAACCATTCCAATTTTCGCCACGAATTCCGAC
TGCGAGCTTATTGTTAAGGCAATGCAAGGTCTCCTAAAAGATGGAAACCCGATTCCCTCAGCAATCGCAGCAAAC
TCCGGCATCTACTAA (SEQ ID NO:147)
Protein:
MCRAISLRRLLLLLLQLSQLLAVTQGMLEMWHEGLEEASRLYFGERNVKGMFEVLEPLHAMMERGPQTLKETSFN
QAYGRDLMEAQEWCRKYMKSGNVKDLLQAWDLYYHVFRRISKTRTGKLFWALVVVAGVLFCYGLLVTVALCVIWV
RSMASTDYSTYSQAAAQQGYSAYTAQPTQGYAQTTQAYGQQSYGTYGQPTDVSYTQAQTTATYGQTAYATSYGQP
PTGYTTPTAPQAYSQPVQGYGTGAYDTTTATVTTTQASYAAQSAYGTQPAYPAYGQQPAATAPTRPQDGNKPTET
SQPQSSTGGYNQPSLGYGQSNYSYPQVPGSYPMQPVTAPPSYPPTSYSSTQPTSYDQSSYSQQNTYGQPSSYGQQ
SSYGQQSSYGQQPPTSYPPQTGSYSQAPSQYSQQSSSYGQQSSFRQDHPSSMGVYGQESGGFSGPGENRSMSGPD
NRGRGRGGFDRGGMSRGGRGGGRGGMGSAGERGGFNKPGGPMDEGPDLDLGPPVDPDEDSDNSAIYVQGLNDSVT
LDDLADFFKQCGVVKMNKRTGQPMIHIYLDKETGKPKGDATVSYEDPPTAKAAVEWFDGKDFQGSKLKVSLARKK
PPMNSMRGGLPPREGRGMPPPLRGGPGGPGGPGGPMGRMGGRGGDRGGFPPRGPRGSRGNPSGGGNVQHRAGDWQ
CPNPGCGNQNFAWRTECNQCKAPKPEGFLPPPFPPPGGDRGRGGPGGMRGGRGGLMDRGGPGGMFRGGRGGDRGG
FRGGRGMDRGGFGGGRRGGPGGPPGPLMEQDYKDDDDKGTEQKLISEEDLGAPGSAGSAAGSGASNFTQFVLVDN
GGTGDVTVAPSNFANGIAEWISSNSRSQAYKVTCSVRQSSAQNRKYTIKVEVPKGAWRSYLNMELTIPIFATNSD
CELIVKAMQGLLKDGNPIPSAIAANSGIY (SEQ ID NO:148)

FRB-CD28-EWSR1-4xλN22
DNA:
ATGTGCCGAGCCATCTCTCTTAGGCGCTTGCTGCTGCTGCTGCAGCTGTCACAACTCCTAGCTGTCACTCAA
GGGATGCTCGAGATGTGGCATGAAGGCCTGGAAGAGGCATCTCGTTTGTACTTTGGGGAAAGGAACGTGAAAGGC
ATGTTTGAGGTGCTGGAGCCCTTGCATGCTATGATGGAACGGGGCCCCCAGACTCTGAAGGAAACATCCTTTAAT

CAGGCCTATGGTCGAGATTTAATGGAGGCCCAAGAGTGGTGCAGGAAGTACATGAAATCAGGGAATGTCAAGGAC
CTCCTCCAAGCCTGGGACCTCTATTATCATGTGTTCCGACGAATCTCAAAGACTAGAACCGGTAAGCTTTTTTGG
GCACTGGTCGTGGTTGCTGGAGTCCTGTTTTGTTATGGCTTGCTAGTGACAGTGGCTCTTTGTGTTATCTGGGTA
AGATCTATGGCGTCCACGGATTACAGTACCTATAGCCAAGCTGCAGCGCAGCAGGGCTACAGTGCTTACACCGCC
CAGCCCACTCAAGGATATGCACAGACCACCCAGGCATATGGGCAACAAAGCTATGGAACCTATGGACAGCCCACT
GATGTCAGCTATACCCAGGCTCAGACCACTGCAACCTATGGGCAGACCGCCTATGCAACTTCTTATGGACAGCCT
CCCACTGGTTATACTACTCCAACTGCCCCCCAGGCATACAGCCAGCCTGTCCAGGGGTATGGCACTGGTGCTTAT
GATACCACCACTGCTACAGTCACCACCACCCAGGCCTCCTATGCAGCTCAGTCTGCATATGGCACTCAGCCTGCT
TATCCAGCCTATGGGCAGCAGCCAGCAGCCACTGCACCTACAAGACCGCAGGATGGAAACAAGCCCACTGAGACT
AGTCAACCTCAATCTAGCACAGGGGGTTACAACCAGCCCAGCCTAGGATATGGACAGAGTAACTACAGTTATCCC
CAGGTACCTGGGAGCTACCCCATGCAGCCAGTCACTGCACCTCCATCCTACCCTCCTACCAGCTATTCCTCTACA
CAGCCGACTAGTTATGATCAGAGCAGTTACTCTCAGCAGAACACCTATGGGCAACCGAGCAGCTATGGACAGCAG
AGTAGCTATGGTCAACAAAGCAGCTATGGGCAGCAGCCTCCCACTAGTTACCCACCCCAAACTGGATCCTACAGC
CAAGCTCCAAGTCAATATAGCCAACAGAGCAGCAGCTACGGGCAGCAGAGTTCATTCCGACAGGACCACCCCAGT
AGCATGGGTGTTTATGGGCAGGAGTCTGGAGGATTTTCCGGACCAGGAGAGAACCGGAGCATGAGTGGCCCTGAT
AACCGGGGCAGGGGAAGAGGGGGATTTGATCGTGGAGGCATGAGCAGAGGTGGGCGGGGAGGAGGACGCGGTGGA
ATGGGCAGCGCTGGAGAGCGAGGTGGCTTCAATAAGCCTGGTGGACCCATGGATGAAGGACCAGATCTTGATCTA
GGCCCACCTGTAGATCCAGATGAAGACTCTGACAACAGTGCAATTTATGTACAAGGATTAAATGACAGTGTGACT
CTAGATGATCTGGCAGACTTCTTTAAGCAGTGTGGGGTTGTTAAGATGAACAAGAGAACTGGGCAACCCATGATC
CACATCTACCTGGACAAGGAAACAGGAAAGCCCAAAGGCGATGCCACAGTGTCCTATGAAGACCCACCTACTGCC
AAGGCTGCCGTGGAATGGTTTGATGGGAAAGATTTTCAAGGGAGCAAACTTAAAGTCTCCCTTGCTCGGAAGAAG
CCTCCAATGAACAGTATGCGGGGTGGTCTGCCACCCCGTGAGGGCAGAGGCATGCCACCACCACTCCGTGGAGGT
CCAGGAGGCCCAGGAGGTCCTGGGGGACCCATGGGTCGCATGGGAGGCCGTGGAGGAGATAGAGGAGGCTTCCCT
CCAAGAGGACCCCGGGGTTCCCGAGGGAACCCCTCTGGAGGAGGAAACGTCCAGCACCGAGCTGGAGACTGGCAG
TGTCCCAATCCGGGTTGTGGAAACCAGAACTTCGCCTGGAGAACAGAGTGCAACCAGTGTAAGGCCCCAAAGCCT
GAAGGCTTCCTCCCGCCACCCTTTCCGCCCCCGGGTGGTGATCGTGGCAGAGGTGGCCCTGGTGGCATGCGGGGA
GGAAGAGGTGGCCTCATGGATCGTGGTGGTCCCGGTGGAATGTTCAGAGGTGGCCGTGGTGGAGACAGAGGTGGC
TTCCGTGGTGGCCGGGGCATGGACCGAGGTGGCTTTGGTGGAGGAAGACGAGGTGGCCCTGGGGGGCCCCCTGGA
CCTTTGATGGAACAGGATTACAAGGATGACGACGATAAGGGTACCGAGCAGAAGCTGATCTCAGAGGAGGACCTG
CTAGCCACCATGGACGCACAAACACGACGACGTGAGCGTCGCGCTGAGAAACAAGCTCAATGGAAAGCTGCAAAC
CCACCGCTCGACGGAGCCGGAGCTGGCGCTGGAGCTGGAGCCGGAGCTGGCGGTCTAGCCACCATGGACGCACAA
ACACGACGACGTGAGCGTCGCGCTGAGAAACAAGCTCAATGGAAAGCTGCAAACCCACCGCTCGACGGAGCCGGA
GCTGGCGCTGGAGCTGGAGCCGGAGCTGGCGGTCTAGCCACCATGGACGCACAAACACGACGACGTGAGCGTCGC
GCTGAGAAACAAGCTCAATGGAAAGCTGCAAACCCACCGCTCGACGGAGCCGGAGCTGGCGCTGGAGCTGGAGCC
GGAGCTGGCGGTCTAGCCACCATGGACGCACAAACACGACGACGTGAGCGTCGCGCTGAGAAACAAGCTCAATGG
AAAGCTGCAAACCCACCGCTCGAGTCTAGAGGGCCCGTTTAA   (SEQ ID NO:149)
Protein:
MCRAISLRRLLLLLLQLSQLLAVTQGMLEMWHEGLEEASRLYFGERNVKGMFEVLEPLHAMMERGPQTLKETSFN
QAYGRDLMEAQEWCRKYMKSGNVKDLLQAWDLYYHVFRRISKTRTGKLFWALVVVAGVLFCYGLLVTVALCVIWV
RSMASTDYSTYSQAAAQQGYSAYTAQPTQGYAQTTQAYGQQSYGTYGQPTDVSYTQAQTTATYGQTAYATSYGQP
PTGYTTPTAPQAYSQPVQGYGTGAYDTTTATVTTTQASYAAQSAYGTQPAYPAYGQQPAATAPTRPQDGNKPTET
SQPQSSTGGYNQPSLGYGQSNYSYPQVPGSYPMQPVTAPPSYPPTSYSSTQPTSYDQSSYSQQNTYGQPSSYGQQ
SSYGQQSSYGQQPPTSYPPQTGSYSQAPSQYSQQSSSYGQQSSFRQDHPSSMGVYGQESGGFSGPGENRSMSGPD
NRGRGRGGFDRGGMSRGGRGGGRGGMGSAGERGGFNKPGGPMDEGPDLDLGPPVDPDEDSDNSAIYVQGLNDSVT
LDDLADFFKQCGVVKMNKRTGQPMIHIYLDKETGKPKGDATVSYEDPPTAKAAVEWFDGKDFQGSKLKVSLARKK
PPMNSMRGGLPPREGRGMPPPLRGGPGGPGGPGGPMGRMGGRGGDRGGFPPRGPRGSRGNPSGGGNVQHRAGDWQ
CPNPGCGNQNFAWRTECNQCKAPKPEGFLPPPFPPPGGDRGRGGPGGMRGGRGGLMDRGGPGGMFRGGRGGDRGG
FRGGRGMDRGGFGGGRRGGPGGPPGPLMEQDYKDDDDKGTEQKLISEEDLLATMDAQTRRRERRAEKQAQWKAAN
PPLDGAGAGAGAGAGGLATMDAQTRRRERRAEKQAQWKAANPPLDGAGAGAGAGAGGLATMDAQTRRRERR
AEKQAQWKAANPPLDGAGAGAGAGAGGLATMDAQTRRRERRAEKQAQWKAANPPLESRGPV   (SEQ ID
NO:150)

FUS-CD28-FUS-PylRS^AF
DNA:
ATGTGCCGAGCCATCTCTCTTAGGCGCTTGCTGCTGCTGCTGCTGCAGCTGTCACAACTCCTAGCTGTCACTCAA
GGGATGCTCATGGCCTCAAACGATTATACCCAACAAGCAACCCAAAGCTATGGGGCCTACCCCACCCAGCCCGGG
CAGGGCTATTCCCAGCAGAGCAGTCAGCCCTACGGACAGCAGAGTTACAGTGGTTATAGCCAGTCCACGGACACT
TCAGGATATGGCCAGAGCAGCTATTCTTCTTATGGCCAGAGCCAGAACACAGGCTATGGAACTCAGTCAACTCCC

CAGGGATATGGCTCGACTGGCGGCTATGGCAGTAGCCAGAGCTCCCAATCGTCTTACGGGCAGCAGTCCTCCTAC
CCTGGCTATGGCCAGCAGCCAGCTCCCAGCAGCACCTCGGGAAGTTACGGTAGCAGTTCTCAGAGCAGCAGCTAT
GGGCAGCCCCAGAGTGGGAGCTACAGCCAGCAGCCTAGCTATGGTGGACAGCAGCAAAGCTATGGACAGCAGCAA
AGCTATAATCCCCCTCAGGGCTATGGACAGCAGAACCAGTACAACAGCAGCAGTGGTGGTGGAGGTGGAGGTGGA
GGTGGAGGTAACTATGGCCAAGATCAATCCTCCATGAGTAGTGGTGGTGGCAGTGGTGGCGGTTATGGCAATCAA
GACCAGAGTGGTGGAGGTGGCAGCGGTGGCTATGGACAGCAGGACCGTGGAGGCCGCGGCAGGGGTGGCAGTGGT
GGCGGCGGCGGCGGCGGTGGTGGTTACAACCGCAGCAGTGGTGGCTATGAACCCAGAGGTCGTGGAGGTGGC
CGTGGAGGCAGAGGTGGCATGGGCGGAAGTGACCGTGGTGGCTTCAATAAATTTGGTGGCCCTCGGGACCAAGGA
TCACGTCATGACTCCGAACAGGATAATTCAGACAACAACACCATCTTTGTGCAAGGCCTGGGTGAGAATGTTACA
ATTGAGTCTGTGGCTGATTACTTCAAGCAGATTGGTATTATTAAGACAAACAAGAAAACGGGACAGCCCATGATT
AATTTGTACACAGACAGGGAAACTGGCAAGCTGAAGGGAGAGGCAACGGTCTCTTTTGATGACCCACCTTCAGCT
AAAGCAGCTATTGACTGGTTTGATGGTAAAGAATTCTCCGGAAATCCTATCAAGGTCTCATTTGCTACTCGCCGG
GCAGACTTTAATCGGGGTGGTGGCAATGGTCGTGGAGGCCGAGGGCGAGGAGGACCCATGGGCCGTGGAGGCTAT
GGAGGTGGTGGCAGTGGTGGTGGTGGCCGAGGAGGATTTCCCAGTGGAGGTGGTGGCGGTGGAGGACAGCAGCGA
GCTGGTGACTGGAAGTGTCCTAATCCCACCTGTGAGAATATGAACTTCTCTTGGAGGAATGAATGCAACCAGTGT
AAGGCCCCTAAACCAGATGGCCCAGGAGGGGGACCAGGTGGCTCTCACATGGGGGGTAACTACGGGGATGATCGT
CGTGGTGGCAGAGGAGGCGGCACCGGTAAGCTTTTTTGGGCACTGGTCGTGGTTGCTGGAGTCCTGTTTTGTTAT
GGCTTGCTAGTGACAGTGGCTCTTTGTGTTATCTGGGTAAGATCTGGTATGGCCTCAAACGATTATACCCAACAA
GCAACCCAAAGCTATGGGGCCTACCCCACCCAGCCCGGGCAGGGCTATTCCCAGCAGAGCAGTCAGCCCTACGGA
CAGCAGAGTTACAGTGGTTATAGCCAGTCCACGGACACTTCAGGATATGGCCAGAGCAGCTATTCTTCTTATGGC
CAGAGCCAGAACACAGGCTATGGAACTCAGTCAACTCCCCAGGGATATGGCTCGACTGGCGGCTATGGCAGTAGC
CAGAGCTCCCAATCGTCTTACGGGCAGCAGTCCTCCTACCCTGGCTATGGCCAGCAGCCAGCTCCCAGCAGCACC
TCGGGAAGTTACGGTAGCAGTTCTCAGAGCAGCAGCTATGGGCAGCCCCAGAGTGGGAGCTACAGCCAGCAGCCT
AGCTATGGTGGACAGCAGCAAAGCTATGGACAGCAGCAAAGCTATAATCCCCCTCAGGGCTATGGACAGCAGAAC
CAGTACAACAGCAGCAGTGGTGGTGGAGGTGGAGGTGGAGGTGGAGGTAACTATGGCCAAGATCAATCCTCCATG
AGTAGTGGTGGTGGCAGTGGTGGCGGTTATGGCAATCAAGACCAGAGTGGTGGAGGTGGCAGCGGTGGCTATGGA
CAGCAGGACCGTGGAGGCCGCGGCAGGGGTGGCAGTGGTGGCGGCGGCGGCGGCGGTGGTGGTTACAACCGC
AGCAGTGGTGGCTATGAACCCAGAGGTCGTGGAGGTGGCCGTGGAGGCAGAGGTGGCATGGGCGGAAGTGACCGT
GGTGGCTTCAATAAATTTGGTGGCCCTCGGGACCAAGGATCACGTCATGACTCCGAACAGGATAATTCAGACAAC
AACACCATCTTTGTGCAAGGCCTGGGTGAGAATGTTACAATTGAGTCTGTGGCTGATTACTTCAAGCAGATTGGT
ATTATTAAGACAAACAAGAAAACGGGACAGCCCATGATTAATTTGTACACAGACAGGGAAACTGGCAAGCTGAAG
GGAGAGGCAACGGTCTCTTTTGATGACCCACCTTCAGCTAAAGCAGCTATTGACTGGTTTGATGGTAAAGAATTC
TCCGGAAATCCTATCAAGGTCTCATTTGCTACTCGCCGGGCAGACTTTAATCGGGGTGGTGGCAATGGTCGTGGA
GGCCGAGGGCGAGGAGGACCCATGGGCCGTGGAGGCTATGGAGGTGGTGGCAGTGGTGGTGGTGGCCGAGGAGGA
TTTCCCAGTGGAGGTGGTGGCGGTGGAGGACAGCAGCGAGCTGGTGACTGGAAGTGTCCTAATCCCACCTGTGAG
AATATGAACTTCTCTTGGAGGAATGAATGCAACCAGTGTAAGGCCCCTAAACCAGATGGCCCAGGAGGGGGACCA
GGTGGCTCTCACATGGGGGGTAACTACGGGGATGATCGTCGTGGTGGCAGAGGAGGCGGCGCCCCCGGCTCCGCC
GGCTCCGCCGCCGGCTCCGGCATGGCGTGCCCGGTGCCGCTGCAGCTGCCGCCGCTGGAACGCCTGACCCTGGAT
GATAAAAAACCGCTGAATACCCTGATCTCTGCTACTGGTCTGTGGATGAGTCGTACCGGAACCATTCATAAAATC
AAACACCACGAGGTTAGCCGTTCGAAAATCTATATTGAGATGGCGTGTGGCGATCATCTGGTTGTGAACAATAGC
CGCTCTTCTCGTACAGCACGTGCACTGCGTCACCACAAATATCGTAAAACCTGTAAACGTTGCCGTGTGTCCGAT
GAGGATCTGAACAAATTCCTGACAAAAGCCAATGAGGACCAAACAAGCGTGAAAGTGAAAGTCGTTAGCGCTCCT
ACCCGTACTAAAAAAGCAATGCCGAAATCCGTTGCTCGTGCCCCTAAACCACTGGAAAACACTGAAGCAGCACAG
GCACAGCCGTCTGGAAGCAAATTCTCTCCGGCCATTCCTGTTTCTACCCAGGAGTCCGTTTCTGTTCCAGCAAGT
GTGAGCACCAGCATTAGCAGTATTAGCACCGGTGCCACCGCTAGCGCCCTGGTTAAAGGCAATACCAATCCGATT
ACAAGCATGTCTGCCCCGGTTCAAGCATCAGCTCCAGCACTGACAAAATCCCAAACCGATCGTCTGGAGGTTCTG
CTGAATCCGAAAGACGAAATCAGCCTGAATTCCGGCAAACCGTTTCGTGAACTGGAGAGCGAACTGCTGTCACGT
CGTAAAAAAGACCTGCAACAAATCTATGCCGAAGAACGTGAGAACTATCTGGGGAAACTGGAACGTGAAATCACC
CGCTTTTTCGTGGATCGTGGCTTTCTGGAGATCAAATCCCCGATTCTGATTCCTCTGGAGTATATCGAGCGTATG
GGCATCGACAATGATACCGAACTGAGCAAACAAATTTTCCGTGTGGATAAAAACTTCTGTCTGCGCCCTATGCTA
GCACCAAATCTGGCTAACTATCTGCGCAAACTGGACCGTGCCCTGCCTGATCCTATCAAAATCTTCGAGATCGGC
CCGTGTTATCGTAAAGAGTCCGACGGTAAAGAACATCTGGAGGAGTTTACCATGCTGAACTTTTGCCAAATGGGT
TCAGGTTGTACTCGTGAGAACCTGGAAAGCATCATCACCGATTTTCTGAACCACCTGGGCATTGACTTCAAAATT
GTGGGCGACAGCTGTATGGTGTTTGGCGACACCCTGGATGTCATGCACGGCGACCTGGAACTGTCTAGTGCCGTT
GTGGGCCCAATCCCGCTGGATCGTGAGTGGGGTATCGACAAACCTTGGATCGGTGCGGGTTTTGGTCTGGAGCGT
CTGCTGAAAGTAAAACACGACTTCAAGAACATCAAACGTGCTGCACGTTCCGAGTCCTATTACAATGGTATTTCT
ACTAACCTGTAA (SEQ ID NO:151)
Protein:

MCRAISLRRLLLLLLQLSQLLAVTQGMLMASNDYTQQATQSYGAYPTQPGQGYSQQSSQPYGQQSYSGYSQSTDT
SGYGQSSYSSYGQSQNTGYGTQSTPQGYGSTGGYGSSQSSQSSYGQQSSYPGYGQQPAPSSTSGSYGSSSQSSSY
GQPQSGSYSQQPSYGGQQQSYGQQQSYNPPQGYGQQNQYNSSSGGGGGGGGGGNYGQDQSSMSSGGGSGGGYGNQ
DQSGGGGSGGYGQQDRGGRGRGGSGGGGGGGGGGYNRSSGGYEPRGRGGGRGGRGGMGGSDRGGFNKFGGPRDQG
SRHDSEQDNSDNNTIFVQGLGENVTIESVADYFKQIGIIKTNKKTGQPMINLYTDRETGKLKGEATVSFDDPPSA
KAAIDWFDGKEFSGNPIKVSFATRRADFNRGGGNGRGGRGRGGPMGRGGYGGGGSGGGGRGGFPSGGGGGGGQQR
AGDWKCPNPTCENMNFSWRNECNQCKAPKPDGPGGGPGGSHMGGNYGDDRRGGRGGGTGKLFWALVVVAGVLFCY
GLLVTVALCVIWVRSGMASNDYTQQATQSYGAYPTQPGQGYSQQSSQPYGQQSYSGYSQSTDTSGYGQSSYSSYG
QSQNTGYGTQSTPQGYGSTGGYGSSQSSQSSYGQQSSYPGYGQQPAPSSTSGSYGSSSQSSSYGQPQSGSYSQQP
SYGGQQQSYGQQQSYNPPQGYGQQNQYNSSSGGGGGGGGGGNYGQDQSSMSSGGGSGGGYGNQDQSGGGGSGGYG
QQDRGGRGRGGSGGGGGGGGGGYNRSSGGYEPRGRGGGRGGRGGMGGSDRGGFNKFGGPRDQGSRHDSEQDNSDN
NTIFVQGLGENVTIESVADYFKQIGIIKTNKKTGQPMINLYTDRETGKLKGEATVSFDDPPSAKAAIDWFDGKEF
SGNPIKVSFATRRADFNRGGGNGRGGRGRGGPMGRGGYGGGGSGGGGRGGFPSGGGGGGGQQRAGDWKCPNPTCE
NMNFSWRNECNQCKAPKPDGPGGGPGGSHMGGNYGDDRRGGRGGGAPGSAGSAAGSGMACPVPLQLPPLERLTLD
DKKPLNTLISATGLWMSRTGTIHKIKHHEVSRSKIYIEMACGDHLVVNNSRSSRTARALRHHKYRKTCKRCRVSD
EDLNKFLTKANEDQTSVKVKVVSAPTRTKKAMPKSVARAPKPLENTEAAQAQPSGSKFSPAIPVSTQESVSVPAS
VSTSISSISTGATASALVKGNTNPITSMSAPVQASAPALTKSQTDRLEVLLNPKDEISLNSGKPFRELESELLSR
RKKDLQQIYAEERENYLGKLEREITRFFVDRGFLEIKSPILIPLEYIERMGIDNDTELSKQIFRVDKNFCLRPML
APNLANYLRKLDRALPDPIKIFEIGPCYRKESDGKEHLEEFTMLNFCQMGSGCTRENLESIITDFLNHLGIDFKI
VGDSCMVFGDTLDVMHGDLELSSAVVGPIPLDREWGIDKPWIGAGFGLERLLKVKHDFKNIKRAARSESYYNGIS
TNL (SEQ ID NO:152)

FUS-CD28-FUS-PylRS^AA
DNA:
ATGTGCCGAGCCATCTCTCTTAGGCGCTTGCTGCTGCTGCTGCTGCAGCTGTCACAACTCCTAGCTGTCACTCAA
GGGATGCTCATGGCCTCAAACGATTATACCCAACAAGCAACCCAAAGCTATGGGGCCTACCCCACCCAGCCCGGG
CAGGGCTATTCCCAGCAGAGCAGTCAGCCCTACGGACAGCAGAGTTACAGTGGTTATAGCCAGTCCACGGACACT
TCAGGATATGGCCAGAGCAGCTATTCTTCTTATGGCCAGAGCCAGAACACAGGCTATGGAACTCAGTCAACTCCC
CAGGGATATGGCTCGACTGGCGGCTATGGCAGTAGCCAGAGCTCCCAATCGTCTTACGGGCAGCAGTCCTCCTAC
CCTGGCTATGGCCAGCAGCCAGCTCCCAGCAGCACCTCGGGAAGTTACGGTAGCAGTTCTCAGAGCAGCAGCTAT
GGGCAGCCCCAGAGTGGGAGCTACAGCCAGCAGCCTAGCTATGGTGGACAGCAGCAAAGCTATGGACAGCAGCAA
AGCTATAATCCCCCTCAGGGCTATGGACAGCAGAACCAGTACAACAGCAGCAGTGGTGGTGGAGGTGGAGGTGGA
GGTGGAGGTAACTATGGCCAAGATCAATCCTCCATGAGTAGTGGTGGTGGCAGTGGTGGCGGTTATGGCAATCAA
GACCAGAGTGGTGGAGGTGGCAGCGGTGGCTATGGACAGCAGGACCGTGGAGGCCGCGGCAGGGGTGGCAGTGGT
GGCGGCGGCGGCGGCGGCGGTGGTGGTTACAACCGCAGCAGTGGTGGCTATGAACCCAGAGGTCGTGGAGGTGGC
CGTGGAGGCAGAGGTGGCATGGGCGGAAGTGACCGTGGTGGCTTCAATAAATTTGGTGGCCCTCGGGACCAAGGA
TCACGTCATGACTCCGAACAGGATAATTCAGACAACAACACCATCTTTGTGCAAGGCCTGGGTGAGAATGTTACA
ATTGAGTCTGTGGCTGATTACTTCAAGCAGATTGGTATTATTAAGACAAACAAGAAAACGGGACAGCCCATGATT
AATTTGTACACAGACAGGGAAACTGGCAAGCTGAAGGGAGAGGCAACGGTCTCTTTTGATGACCCACCTTCAGCT
AAAGCAGCTATTGACTGGTTTGATGGTAAAGAATTCTCCGGAAATCCTATCAAGGTCTCATTTGCTACTCGCCGG
GCAGACTTTAATCGGGGTGGTGGCAATGGTCGTGGAGGCCGAGGGCGAGGAGGACCCATGGGCCGTGGAGGCTAT
GGAGGTGGTGGCAGTGGTGGTGGTGGCCGAGGAGGATTTCCCAGTGGAGGTGGTGGCGGTGGAGGACAGCAGCGA
GCTGGTGACTGGAAGTGTCCTAATCCCACCTGTGAGAATATGAACTTCTCTTGGAGGAATGAATGCAACCAGTGT
AAGGCCCCTAAACCAGATGGCCCAGGAGGGGGACCAGGTGGCTCTCACATGGGGGGTAACTACGGGGATGATCGT
CGTGGTGGCAGAGGAGGCGGCACCGGTAAGCTTTTTTGGGCACTGGTCGTGGTTGCTGGAGTCCTGTTTTGTTAT
GGCTTGCTAGTGACAGTGGCTCTTTGTGTTATCTGGGTAAGATCTGGTATGGCCTCAAACGATTATACCCAACAA
GCAACCCAAAGCTATGGGGCCTACCCCACCCAGCCCGGGCAGGGCTATTCCCAGCAGAGCAGTCAGCCCTACGGA
CAGCAGAGTTACAGTGGTTATAGCCAGTCCACGGACACTTCAGGATATGGCCAGAGCAGCTATTCTTCTTATGGC
CAGAGCCAGAACACAGGCTATGGAACTCAGTCAACTCCCCAGGGATATGGCTCGACTGGCGGCTATGGCAGTAGC
CAGAGCTCCCAATCGTCTTACGGGCAGCAGTCCTCCTACCCTGGCTATGGCCAGCAGCCAGCTCCCAGCAGCACC
TCGGGAAGTTACGGTAGCAGTTCTCAGAGCAGCAGCTATGGGCAGCCCCAGAGTGGGAGCTACAGCCAGCAGCCT
AGCTATGGTGGACAGCAGCAAAGCTATGGACAGCAGCAAAGCTATAATCCCCCTCAGGGCTATGGACAGCAGAAC
CAGTACAACAGCAGCAGTGGTGGTGGAGGTGGAGGTGGAGGTGGAGGTAACTATGGCCAAGATCAATCCTCCATG
AGTAGTGGTGGTGGCAGTGGTGGCGGTTATGGCAATCAAGACCAGAGTGGTGGAGGTGGCAGCGGTGGCTATGGA
CAGCAGGACCGTGGAGGCCGCGGCAGGGGTGGCAGTGGTGGCGGCGGCGGCGGCGGCGGTGGTGGTTACAACCGC
AGCAGTGGTGGCTATGAACCCAGAGGTCGTGGAGGTGGCCGTGGAGGCAGAGGTGGCATGGGCGGAAGTGACCGT
GGTGGCTTCAATAAATTTGGTGGCCCTCGGGACCAAGGATCACGTCATGACTCCGAACAGGATAATTCAGACAAC
AACACCATCTTTGTGCAAGGCCTGGGTGAGAATGTTACAATTGAGTCTGTGGCTGATTACTTCAAGCAGATTGGT

ATTATTAAGACAAACAAGAAAACGGGACAGCCCATGATTAATTTGTACACAGACAGGGAAACTGGCAAGCTGAAG
GGAGAGGCAACGGTCTCTTTTGATGACCCACCTTCAGCTAAAGCAGCTATTGACTGGTTTGATGGTAAAGAATTC
TCCGGAAATCCTATCAAGGTCTCATTTGCTACTCGCCGGGCAGACTTTAATCGGGGTGGTGGCAATGGTCGTGGA
GGCCGAGGGCGAGGAGGACCCATGGGCCGTGGAGGCTATGGAGGTGGTGGCAGTGGTGGTGGTGGCCGAGGAGGA
TTTCCCAGTGGAGGTGGTGGCGGTGGAGGACAGCAGCGAGCTGGTGACTGGAAGTGTCCTAATCCCACCTGTGAG
AATATGAACTTCTCTTGGAGGAATGAATGCAACCAGTGTAAGGCCCCTAAACCAGATGGCCCAGGAGGGGGACCA
GGTGGCTCTCACATGGGGGGTAACTACGGGGATGATCGTCGTGGTGGCAGAGGAGGCGGCGCCCCCGGCTCCGCC
GGCTCCGCCGCCGGCTCCGGCATGGCGTGCCCGGTGCCGCTGCAGCTGCCGCCGCTGGAACGCCTGACCCTGGAT
GACAAAAAACCGCTGAATACCCTGATCTCTGCTACTGGTCTGTGGATGAGTCGTACCGGAACCATTCATAAAATC
AAACACCACGAGGTTAGCCGTTCGAAAATCTATATTGAGATGGCGTGTGGCGATCATCTGGTTGTGAACAATAGC
CGCTCTTCTCGTACAGCACGTGCACTGCGTCACCACAAATATCGTAAAACCTGTAAACGTTGCCGTGTGTCCGAT
GAGGATCTGAACAAATTCCTGACAAAAGCCAATGAGGACCAAACAAGCGTGAAAGTGAAAGTCGTTAGCGCTCCT
ACCCGTACTAAAAAAGCAATGCCGAAATCCGTTGCTCGTGCCCCTAAACCACTGGAAAACACTGAAGCAGCACAG
GCACAGCCGTCTGGAAGCAAATTCTCTCCGGCCATTCCTGTTTCTACCCAGGAGTCCGTTTCTGTTCCAGCAAGT
GTGAGCACCAGCATTAGCAGTATTAGCACCGGTGCCACCGCTAGCGCCCTGGTTAAAGGCAATACCAATCCGATT
ACAAGCATGTCTGCCCCGGTTCAAGCATCAGCTCCAGCACTGACAAAATCCCAAACCGATCGTCTGGAGGTTCTG
CTGAATCCGAAAGACGAAATCAGCCTGAATTCCGGCAAACCGTTTCGTGAACTGGAGAGCGAACTGCTGTCACGT
CGTAAAAAAGACCTGCAACAAATCTATGCCGAAGAACGTGAGAACTATCTGGGGAAACTGGAACGTGAAATCACC
CGCTTTTTCGTGGATCGTGGCTTTCTGGAGATCAAATCCCCGATTCTGATTCCTCTGGAGTATATCGAGCGTATG
GGCATCGACAATGATACCGAACTGAGCAAACAAATTTTCCGTGTGGATAAAAACTTCTGTCTGCGCCCTATGCTG
GCACCAAATCTGTATAACTATCTGCGCAAACTGGACCGTGCCCTGCCTGATCCTATCAAAATCTTCGAGATCGGC
CCGTGTTATCGTAAAGAGTCCGACGGTAAAGAACATCTGGAGGAGTTTACCATGCTGGCCTTTGCCCAAATGGGT
TCAGGTTGTACTCGTGAGAACCTGGAAAGCATCATCACCGATTTTCTGAACCACCTGGGCATTGACTTCAAAATT
GTGGGCGACAGCTGTATGGTGTATGGCGACACCCTGGATGTCATGCACGGCGACCTGGAACTGTCTAGTGCCGTT
GTTGGACCAATTCCGCTGGACCGTGAGTGGGGTATCGACAAACCGTGGATCGGAGCAGGATTCGGTCTGGAACGC
CTGCTGAAAGTGAAACACGACTTCAAAAACATCAAACGTGCCGCCCGTTCTGAATCGTATTATAACGGGATCTCT
ACGAACCTGTAA  (SEQ ID NO:153)
Protein:
MCRAISLRRLLLLLLQLSQLLAVTQGMLMASNDYTQQATQSYGAYPTQPGQGYSQQSSQPYGQQSYSGYSQSTDT
SGYGQSSYSSYGQSQNTGYGTQSTPQGYGSTGGYGSSQSSQSSYGQQSSYPGYGQQPAPSSTSGSYGSSSQSSSY
GQPQSGSYSQQPSYGGQQQSYGQQQSYNPPQGYGQQNQYNSSSGGGGGGGGGNYGQDQSSMSSGGGSGGGYGNQ
DQSGGGGSGGYGQQDRGGRGRGGSGGGGGGGGGGYNRSSGGYEPRGRGGGRGGRGGMGGSDRGGFNKFGGPRDQG
SRHDSEQDNSDNNTIFVQGLGENVTIESVADYFKQIGIIKTNKKTGQPMINLYTDRETGKLKGEATVSFDDPPSA
KAAIDWFDGKEFSGNPIKVSFATRRADFNRGGGNGRGGRGRGGPMGRGGYGGGGSGGGGRGGFPSGGGGGGGQQR
AGDWKCPNPTCENMNFSWRNECNQCKAPKPDGPGGGPGGSHMGGNYGDDRRGGRGGGTGKLFWALVVVAGVLFCY
GLLVTVALCVIWVRSGMASNDYTQQATQSYGAYPTQPGQGYSQQSSQPYGQQSYSGYSQSTDTSGYGQSSYSSYG
QSQNTGYGTQSTPQGYGSTGGYGSSQSSQSSYGQQSSYPGYGQQPAPSSTSGSYGSSSQSSSYGQPQSGSYSQQP
SYGGQQQSYGQQQSYNPPQGYGQQNQYNSSSGGGGGGGGGNYGQDQSSMSSGGGSGGGYGNQDQSGGGGSGGYG
QQDRGGRGRGGSGGGGGGGGGGYNRSSGGYEPRGRGGGRGGRGGMGGSDRGGFNKFGGPRDQGSRHDSEQDNSDN
NTIFVQGLGENVTIESVADYFKQIGIIKTNKKTGQPMINLYTDRETGKLKGEATVSFDDPPSAKAAIDWFDGKEF
SGNPIKVSFATRRADFNRGGGNGRGGRGRGGPMGRGGYGGGGSGGGGRGGFPSGGGGGGGQQRAGDWKCPNPTCE
NMNFSWRNECNQCKAPKPDGPGGGPGGSHMGGNYGDDRRGGRGGGAPGSAGSAAGSGMACPVPLQLPPLERLTLD
DKKPLNTLISATGLWMSRTGTIHKIKHHEVSRSKIYIEMACGDHLVVNNSRSSRTARALRHHKYRKTCKRCRVSD
EDLNKFLTKANEDQTSVKVKVVSAPTRTKKAMPKSVARAPKPLENTEAAQAQPSGSKFSPAIPVSTQESVSVPAS
VSTSISSISTGATASALVKGNTNPITSMSAPVQASAPALTKSQTDRLEVLLNPKDEISLNSGKPFRELESELLSR
RKKDLQQIYAEERENYLGKLEREITRFFVDRGFLEIKSPILIPLEYIERMGIDNDTELSKQIFRVDKNFCLRPML
APNLYNYLRKLDRALPDPIKIFEIGPCYRKESDGKEHLEEFTMLAFAQMGSGCTRENLESIITDFLNHLGIDFKI
VGDSCMVYGDTLDVMHGDLELSSAVVGPIPLDREWGIDKPWIGAGFGLERLLKVKHDFKNIKRAARSESYYNGIS
TNL  (SEQ ID NO:154)

FUS-CD28-FUS-MCP-PylRS^AF
DNA:
ATGTGCCGAGCCATCTCTCTTAGGCGCTTGCTGCTGCTGCTGCTGCAGCTGTCACAACTCCTAGCTGTCACTCAA
GGGATGCTCATGGCCTCAAACGATTATACCCAACAAGCAACCCAAAGCTATGGGGCCTACCCCACCCAGCCCGGG
CAGGGCTATTCCCAGCAGAGCAGTCAGCCCTACGGACAGCAGAGTTACAGTGGTTATAGCCAGTCCACGGACACT
TCAGGATATGGCCAGAGCAGCTATTCTTCTTATGGCCAGAGCCAGAACACAGGCTATGGAACTCAGTCAACTCCC
CAGGGATATGGCTCGACTGGCGGCTATGGCAGTAGCCAGAGCTCCCAATCGTCTTACGGGCAGCAGTCCTCCTAC
CCTGGCTATGGCCAGCAGCCAGCTCCCAGCAGCACCTCGGGAAGTTACGGTAGCAGTTCTCAGAGCAGCAGCTAT

GGGCAGCCCCAGAGTGGGAGCTACAGCCAGCAGCCTAGCTATGGTGGACAGCAGCAAAGCTATGGACAGCAGCAA
AGCTATAATCCCCCTCAGGGCTATGGACAGCAGAACCAGTACAACAGCAGCAGTGGTGGTGGAGGTGGAGGTGGA
GGTGGAGGTAACTATGGCCAAGATCAATCCTCCATGAGTAGTGGTGGTGGCAGTGGTGGCGGTTATGGCAATCAA
GACCAGAGTGGTGGAGGTGGCAGCGGTGGCTATGGACAGCAGGACCGTGGAGGCCGCGGCAGGGGTGGCAGTGGT
GGCGGCGGCGGCGGCGGCGGTGGTGGTTACAACCGCAGCAGTGGTGGCTATGAACCCAGAGGTCGTGGAGGTGGC
CGTGGAGGCAGAGGTGGCATGGGCGGAAGTGACCGTGGTGGCTTCAATAAATTTGGTGGCCCTCGGGACCAAGGA
TCACGTCATGACTCCGAACAGGATAATTCAGACAACAACACCATCTTTGTGCAAGGCCTGGGTGAGAATGTTACA
ATTGAGTCTGTGGCTGATTACTTCAAGCAGATTGGTATTATTAAGACAAACAAGAAAACGGGACAGCCCATGATT
AATTTGTACACAGACAGGGAAACTGGCAAGCTGAAGGGAGAGGCAACGGTCTCTTTTGATGACCCACCTTCAGCT
AAAGCAGCTATTGACTGGTTTGATGGTAAAGAATTCTCCGGAAATCCTATCAAGGTCTCATTTGCTACTCGCCGG
GCAGACTTTAATCGGGGTGGTGGCAATGGTCGTGGAGGCCGAGGGCGAGGAGGACCCATGGGCCGTGGAGGCTAT
GGAGGTGGTGGCAGTGGTGGTGGTGGCCGAGGAGGATTTCCCAGTGGAGGTGGTGGCGGTGGAGGACAGCAGCGA
GCTGGTGACTGGAAGTGTCCTAATCCCACCTGTGAGAATATGAACTTCTCTTGGAGGAATGAATGCAACCAGTGT
AAGGCCCCTAAACCAGATGGCCCAGGAGGGGGACCAGGTGGCTCTCACATGGGGGGTAACTACGGGGATGATCGT
CGTGGTGGCAGAGGAGGCGGCACCGGTAAGCTTTTTTGGGCACTGGTCGTGGTTGCTGGAGTCCTGTTTTGTTAT
GGCTTGCTAGTGACAGTGGCTCTTTGTGTTATCTGGGTAAGATCTGGTATGGCCTCAAACGATTATACCCAACAA
GCAACCCAAAGCTATGGGGCCTACCCCACCCAGCCCGGGCAGGGCTATTCCCAGCAGAGCAGTCAGCCCTACGGA
CAGCAGAGTTACAGTGGTTATAGCCAGTCCACGGACACTTCAGGATATGGCCAGAGCAGCTATTCTTCTTATGGC
CAGAGCCAGAACACAGGCTATGGAACTCAGTCAACTCCCCAGGGATATGGCTCGACTGGCGGCTATGGCAGTAGC
CAGAGCTCCCAATCGTCTTACGGGCAGCAGTCCTCCTACCCTGGCTATGGCCAGCAGCCAGCTCCCAGCAGCACC
TCGGGAAGTTACGGTAGCAGTTCTCAGAGCAGCAGCTATGGGCAGCCCCAGAGTGGGAGCTACAGCCAGCAGCCT
AGCTATGGTGGACAGCAGCAAAGCTATGGACAGCAGCAAAGCTATAATCCCCCTCAGGGCTATGGACAGCAGAAC
CAGTACAACAGCAGCAGTGGTGGTGGAGGTGGAGGTGGAGGTGGAGGTAACTATGGCCAAGATCAATCCTCCATG
AGTAGTGGTGGTGGCAGTGGTGGCGGTTATGGCAATCAAGACCAGAGTGGTGGAGGTGGCAGCGGTGGCTATGGA
CAGCAGGACCGTGGAGGCCGCGGCAGGGGTGGCAGTGGTGGCGGCGGCGGCGGCGGCGGTGGTGGTTACAACCGC
AGCAGTGGTGGCTATGAACCCAGAGGTCGTGGAGGTGGCCGTGGAGGCAGAGGTGGCATGGGCGGAAGTGACCGT
GGTGGCTTCAATAAATTTGGTGGCCCTCGGGACCAAGGATCACGTCATGACTCCGAACAGGATAATTCAGACAAC
AACACCATCTTTGTGCAAGGCCTGGGTGAGAATGTTACAATTGAGTCTGTGGCTGATTACTTCAAGCAGATTGGT
ATTATTAAGACAAACAAGAAAACGGGACAGCCCATGATTAATTTGTACACAGACAGGGAAACTGGCAAGCTGAAG
GGAGAGGCAACGGTCTCTTTTGATGACCCACCTTCAGCTAAAGCAGCTATTGACTGGTTTGATGGTAAAGAATTC
TCCGGAAATCCTATCAAGGTCTCATTTGCTACTCGCCGGGCAGACTTTAATCGGGGTGGTGGCAATGGTCGTGGA
GGCCGAGGGCGAGGAGGACCCATGGGCCGTGGAGGCTATGGAGGTGGTGGCAGTGGTGGTGGTGGCCGAGGAGGA
TTTCCCAGTGGAGGTGGTGGCGGTGGAGGACAGCAGCGAGCTGGTGACTGGAAGTGTCCTAATCCCACCTGTGAG
AATATGAACTTCTCTTGGAGGAATGAATGCAACCAGTGTAAGGCCCCTAAACCAGATGGCCCAGGAGGGGGACCA
GGTGGCTCTCACATGGGGGGTAACTACGGGGATGATCGTCGTGGTGGCAGAGGAGGCGATTACAAGGATGACGAC
GATAAGGGTACCGGCGCCCCCGGCTCCGCCGGCTCCGCCGCCGGCTCCGGCGCTTCTAACTTTACTCAGTTCGTT
CTCGTCGACAATGGCGGAACTGGCGACGTGACTGTCGCCCCAAGCAACTTCGCTAACGGGATCGCTGAATGGATC
AGCTCTAACTCGCGTTCACAGGCTTACAAAGTAACCTGTAGCGTTCGTCAGAGCTCTGCGCAGAATCGCAAATAC
ACCATCAAAGTCGAGGTGCCTAAAGGCGCCTGGCGTTCGTACTTAAATATGGAACTAACCATTCCAATTTTCGCC
ACGAATTCCGACTGCGAGCTTATTGTTAAGGCAATGCAAGGTCTCCTAAAAGATGGAAACCCGATTCCCTCAGCA
ATCGCAGCAAACTCCGGCATCTACGGTACCGGCGCCCCCGGCTCCGCCGGCTCCGCCGCCGGCTCCGGCGCGTGC
CCGGTGCCGCTGCAGCTGCCGCCGCTGGAACGCCTGACCCTGGATGATAAAAAACCGCTGAATACCCTGATCTCT
GCTACTGGTCTGTGGATGAGTCGTACCGGAACCATTCATAAAATCAAACACCACGAGGTTAGCCGTTCGAAAATC
TATATTGAGATGGCGTGTGGCGATCATCTGGTTGTGAACAATAGCCGCTCTTCTCGTACAGCACGTGCACTGCGT
CACCACAAATATCGTAAAACCTGTAAACGTTGCCGTGTGTCCGATGAGGATCTGAACAAATTCCTGACAAAAGCC
AATGAGGACCAAACAAGCGTGAAAGTGAAAGTCGTTAGCGCTCCTACCCGTACTAAAAAAGCAATGCCGAAATCC
GTTGCTCGTGCCCCTAAACCACTGGAAAACACTGAAGCAGCACAGGCACAGCCGTCTGGAAGCAAATTCTCTCCG
GCCATTCCTGTTTCTACCCAGGAGTCCGTTTCTGTTCCAGCAAGTGTGAGCACCAGCATTAGCAGTATTAGCACC
GGTGCCACCGCTAGCGCCCTGGTTAAAGGCAATACCAATCCGATTACAAGCATGTCTGCCCCGGTTCAAGCATCA
GCTCCAGCACTGACAAAATCCCAAACCGATCGTCTGGAGGTTCTGCTGAATCCGAAAGACGAAATCAGCCTGAAT
TCCGGCAAACCGTTTCGTGAACTGGAGAGCGAACTGCTGTCACGTCGTAAAAAAGACCTGCAACAAATCTATGCC
GAAGAACGTGAGAACTATCTGGGGAAACTGGAACGTGAAATCACCCGCTTTTTCGTGGATCGTGGCTTTCTGGAG
ATCAAATCCCCGATTCTGATTCCTCTGGAGTATATCGAGCGTATGGGCATCGACAATGATACCGAACTGAGCAAA
CAAATTTTCCGTGTGGATAAAAACTTCTGTCTGCGCCCTATGCTAGCACCAAATCTGGCTAACTATCTGCGCAAA
CTGGACCGTGCCCTGCCTGATCCTATCAAAATCTTCGAGATCGGCCCGTGTTATCGTAAAGAGTCCGACGGTAAA
GAACATCTGGAGGAGTTTACCATGCTGAACTTTTGCCAAATGGGTTCAGGTTGTACTCGTGAGAACCTGGAAAGC
ATCATCACCGATTTTCTGAACCACCTGGGCATTGACTTCAAAATTGTGGGCGACAGCTGTATGGTGTTTGGCGAC
ACCCTGGATGTCATGCACGGCGACCTGGAACTGTCTAGTGCCGTTGTGGGCCCAATCCCGCTGGATCGTGAGTGG

GGTATCGACAAACCTTGGATCGGTGCGGGTTTTGGTCTGGAGCGTCTGCTGAAAGTAAAACACGACTTCAAGAAC
ATCAAACGTGCTGCACGTTCCGAGTCCTATTACAATGGTATTTCTACTAACCTGTAA (SEQ ID NO:155)
Protein:
MCRAISLRRLLLLLLQLSQLLAVTQGMLMASNDYTQQATQSYGAYPTQPGQGYSQQSSQPYGQQSYSGYSQSTDT
SGYGQSSYSSYGQSQNTGYGTQSTPQGYGSTGGYGSSQSSQSSYGQQSSYPGYGQQPAPSSTSGSYGSSSQSSSY
GQPQSGSYSQQPSYGGQQQSYGQQQSYNPPQGYGQQNQYNSSSGGGGGGGGGNYGQDQSSMSSGGGSGGGYGNQ
DQSGGGGSGGYGQQDRGGRGRGGSGGGGGGGGGGYNRSSGGYEPRGRGGGRGGRGGMGGSDRGGFNKFGGPRDQG
SRHDSEQDNSDNNTIFVQGLGENVTIESVADYFKQIGIIKTNKKTGQPMINLYTDRETGKLKGEATVSFDDPPSA
KAAIDWFDGKEFSGNPIKVSFATRRADFNRGGGNGRGGRGRGGPMGRGGYGGGGSGGGGRGGFPSGGGGGGGQQR
AGDWKCPNPTCENMNFSWRNECNQCKAPKPDGPGGGPGGSHMGGNYGDDRRGGRGGGTGKLFWALVVVAGVLFCY
GLLVTVALCVIWVRSGMASNDYTQQATQSYGAYPTQPGQGYSQQSSQPYGQQSYSGYSQSTDTSGYGQSSYSSYG
QSQNTGYGTQSTPQGYGSTGGYGSSQSSQSSYGQQSSYPGYGQQPAPSSTSGSYGSSSQSSSYGQPQSGSYSQQP
SYGGQQQSYGQQQSYNPPQGYGQQNQYNSSSGGGGGGGGGNYGQDQSSMSSGGGSGGGYGNQDQSGGGGSGGYG
QQDRGGRGRGGSGGGGGGGGGGYNRSSGGYEPRGRGGGRGGRGGMGGSDRGGFNKFGGPRDQGSRHDSEQDNSDN
NTIFVQGLGENVTIESVADYFKQIGIIKTNKKTGQPMINLYTDRETGKLKGEATVSFDDPPSAKAAIDWFDGKEF
SGNPIKVSFATRRADFNRGGGNGRGGRGRGGPMGRGGYGGGGSGGGGRGGFPSGGGGGGGQQRAGDWKCPNPTCE
NMNFSWRNECNQCKAPKPDGPGGGPGGSHMGGNYGDDRRGGRGGDYKDDDDKGTGAPGSAGSAAGSGASNFTQFV
LVDNGGTGDVTVAPSNFANGIAEWISSNSRSQAYKVTCSVRQSSAQNRKYTIKVEVPKGAWRSYLNMELTIPIFA
TNSDCELIVKAMQGLLKDGNPIPSAIAANSGIYGTGAPGSAGSAAGSGACPVPLQLPPLERLTLDDKKPLNTLIS
ATGLWMSRTGTIHKIKHHEVSRSKIYIEMACGDHLVVNNSRSSRTARALRHHKYRKTCKRCRVSDEDLNKFLTKA
NEDQTSVKVKVVSAPTRTKKAMPKSVARAPKPLENTEAAQAQPSGSKFSPAIPVSTQESVSVPASVSTSISSIST
GATASALVKGNTNPITSMSAPVQASAPALTKSQTDRLEVLLNPKDEISLNSGKPFRELESELLSRRKKDLQQIYA
EERENYLGKLEREITRFFVDRGFLEIKSPILIPLEYIERMGIDNDTELSKQIFRVDKNFCLRPMLAPNLANYLRK
LDRALPDPIKIFEIGPCYRKESDGKEHLEEFTMLNFCQMGSGCTRENLESIITDFLNHLGIDFKIVGDSCMVFGD
TLDVMHGDLELSSAVVGPIPLDREWGIDKPWIGAGFGLERLLKVKHDFKNIKRAARSESYYNGISTNL (SEQ
ID NO:156)

FUS-CD28-FUS-MCP-PylRS^AA
DNA:
ATGTGCCGAGCCATCTCTCTTAGGCGCTTGCTGCTGCTGCTGCTGCAGCTGTCACAACTCCTAGCTGTCACTCAA
GGGATGCTCATGGCCTCAAACGATTATACCCAACAAGCAACCCAAAGCTATGGGGCCTACCCCACCCAGCCCGGG
CAGGGCTATTCCCAGCAGAGCAGTCAGCCCTACGGACAGCAGAGTTACAGTGGTTATAGCCAGTCCACGGACACT
TCAGGATATGGCCAGAGCAGCTATTCTTCTTATGGCCAGAGCCAGAACACAGGCTATGGAACTCAGTCAACTCCC
CAGGGATATGGCTCGACTGGCGGCTATGGCAGTAGCCAGAGCTCCCAATCGTCTTACGGGCAGCAGTCCTCCTAC
CCTGGCTATGGCCAGCAGCCAGCTCCCAGCAGCACCTCGGGAAGTTACGGTAGCAGTTCTCAGAGCAGCAGCTAT
GGGCAGCCCCAGAGTGGGAGCTACAGCCAGCAGCCTAGCTATGGTGGACAGCAGCAAAGCTATGGACAGCAGCAA
AGCTATAATCCCCCTCAGGGCTATGGACAGCAGAACCAGTACAACAGCAGCAGTGGTGGTGGAGGTGGAGGTGGA
GGTGGAGGTAACTATGGCCAAGATCAATCCTCCATGAGTAGTGGTGGTGGCAGTGGTGGCGGTTATGGCAATCAA
GACCAGAGTGGTGGAGGTGGCAGCGGTGGCTATGGACAGCAGGACCGTGGAGGCCGCGGCAGGGGTGGCAGTGGT
GGCGGCGGCGGCGGCGGTGGTGGTTACAACCGCAGCAGTGGTGGCTATGAACCCAGAGGTCGTGGAGGTGGC
CGTGGAGGCAGAGGTGGCATGGGCGGAAGTGACCGTGGTGGCTTCAATAAATTTGGTGGCCCTCGGGACCAAGGA
TCACGTCATGACTCCGAACAGGATAATTCAGACAACAACACCATCTTTGTGCAAGGCCTGGGTGAGAATGTTACA
ATTGAGTCTGTGGCTGATTACTTCAAGCAGATTGGTATTATTAAGACAAACAAGAAAACGGGACAGCCCATGATT
AATTTGTACACAGACAGGGAAACTGGCAAGCTGAAGGGAGAGGCAACGGTCTCTTTTGATGACCCACCTTCAGCT
AAAGCAGCTATTGACTGGTTTGATGGTAAAGAATTCTCCGGAAATCCTATCAAGGTCTCATTTGCTACTCGCCGG
GCAGACTTTAATCGGGGTGGTGGCAATGGTCGTGGAGGCCGAGGGCGAGGAGGACCCATGGGCCGTGGAGGCTAT
GGAGGTGGTGGCAGTGGTGGTGGTGGCCGAGGAGGATTTCCCAGTGGAGGTGGTGGCGGTGGAGGACAGCAGCGA
GCTGGTGACTGGAAGTGTCCTAATCCCACCTGTGAGAATATGAACTTCTCTTGGAGGAATGAATGCAACCAGTGT
AAGGCCCCTAAACCAGATGGCCCAGGAGGGGGACCAGGTGGCTCTCACATGGGGGGTAACTACGGGGATGATCGT
CGTGGTGGCAGAGGAGGCGGCACCGGTAAGCTTTTTTGGGCACTGGTCGTGGTTGCTGGAGTCCTGTTTTGTTAT
GGCTTGCTAGTGACAGTGGCTCTTTGTGTTATCTGGGTAAGATCTGGTATGGCCTCAAACGATTATACCCAACAA
GCAACCCAAAGCTATGGGGCCTACCCCACCCAGCCCGGGCAGGGCTATTCCCAGCAGAGCAGTCAGCCCTACGGA
CAGCAGAGTTACAGTGGTTATAGCCAGTCCACGGACACTTCAGGATATGGCCAGAGCAGCTATTCTTCTTATGGC
CAGAGCCAGAACACAGGCTATGGAACTCAGTCAACTCCCCAGGGATATGGCTCGACTGGCGGCTATGGCAGTAGC
CAGAGCTCCCAATCGTCTTACGGGCAGCAGTCCTCCTACCCTGGCTATGGCCAGCAGCCAGCTCCCAGCAGCACC
TCGGGAAGTTACGGTAGCAGTTCTCAGAGCAGCAGCTATGGGCAGCCCCAGAGTGGGAGCTACAGCCAGCAGCCT
AGCTATGGTGGACAGCAGCAAAGCTATGGACAGCAGCAAAGCTATAATCCCCCTCAGGGCTATGGACAGCAGAAC
CAGTACAACAGCAGCAGTGGTGGTGGAGGTGGAGGTGGAGGTGGAGGTAACTATGGCCAAGATCAATCCTCCATG

AGTAGTGGTGGTGGCAGTGGTGGCGGTTATGGCAATCAAGACCAGAGTGGTGGAGGTGGCAGCGGTGGCTATGGA
CAGCAGGACCGTGGAGGCCGCGGCAGGGGTGGCAGTGGTGGCGGCGGCGGCGGCGGTGGTGGTTACAACCGC
AGCAGTGGTGGCTATGAACCCAGAGGTCGTGGAGGTGGCCGTGGAGGCAGAGGTGGCATGGGCGGAAGTGACCGT
GGTGGCTTCAATAAATTTGGTGGCCCTCGGGACCAAGGATCACGTCATGACTCCGAACAGGATAATTCAGACAAC
AACACCATCTTTGTGCAAGGCCTGGGTGAGAATGTTACAATTGAGTCTGTGGCTGATTACTTCAAGCAGATTGGT
ATTATTAAGACAAACAAGAAAACGGGACAGCCCATGATTAATTTGTACACAGACAGGGAAACTGGCAAGCTGAAG
GGAGAGGCAACGGTCTCTTTTGATGACCCACCTTCAGCTAAAGCAGCTATTGACTGGTTTGATGGTAAAGAATTC
TCCGGAAATCCTATCAAGGTCTCATTTGCTACTCGCCGGGCAGACTTTAATCGGGGTGGTGGCAATGGTCGTGGA
GGCCGAGGGCGAGGAGGACCCATGGGCCGTGGAGGCTATGGAGGTGGTGGCAGTGGTGGTGGTGGCCGAGGAGGA
TTTCCCAGTGGAGGTGGTGGCGGTGGAGGACAGCAGCGAGCTGGTGACTGGAAGTGTCCTAATCCCACCTGTGAG
AATATGAACTTCTCTTGGAGGAATGAATGCAACCAGTGTAAGGCCCCTAAACCAGATGGCCCAGGAGGGGGACCA
GGTGGCTCTCACATGGGGGGTAACTACGGGGATGATCGTCGTGGTGGCAGAGGAGGCGGCGCCCCCGGCTCCGCC
GGCTCCGCCGCCGGCTCCGGCATGGCGTGCCCGGTGCCGCTGCAGCTGCCGCCGCTGGAACGCCTGACCCTGGAT
GACAAAAAACCGCTGAATACCCTGATCTCTGCTACTGGTCTGTGGATGAGTCGTACCGGAACCATTCATAAAATC
AAACACCACGAGGTTAGCCGTTCGAAAATCTATATTGAGATGGCGTGTGGCGATCATCTGGTTGTGAACAATAGC
CGCTCTTCTCGTACAGCACGTGCACTGCGTCACCACAAATATCGTAAAACCTGTAAACGTTGCCGTGTGTCCGAT
GAGGATCTGAACAAATTCCTGACAAAAGCCAATGAGGACCAAACAAGCGTGAAAGTGAAAGTCGTTAGCGCTCCT
ACCCGTACTAAAAAAGCAATGCCGAAATCCGTTGCTCGTGCCCCTAAACCACTGGAAAACACTGAAGCAGCACAG
GCACAGCCGTCTGGAAGCAAATTCTCTCCGGCCATTCCTGTTTCTACCCAGGAGTCCGTTTCTGTTCCAGCAAGT
GTGAGCACCAGCATTAGCAGTATTAGCACCGGTGCCACCGCTAGCGCCCTGGTTAAAGGCAATACCAATCCGATT
ACAAGCATGTCTGCCCCGGTTCAAGCATCAGCTCCAGCACTGACAAAATCCCAAACCGATCGTCTGGAGGTTCTG
CTGAATCCGAAAGACGAAATCAGCCTGAATTCCGGCAAACCGTTTCGTGAACTGGAGAGCGAACTGCTGTCACGT
CGTAAAAAAGACCTGCAACAAATCTATGCCGAAGAACGTGAGAACTATCTGGGGAAACTGGAACGTGAAATCACC
CGCTTTTTCGTGGATCGTGGCTTTCTGGAGATCAAATCCCCGATTCTGATTCCTCTGGAGTATATCGAGCGTATG
GGCATCGACAATGATACCGAACTGAGCAAACAAATTTTCCGTGTGGATAAAAACTTCTGTCTGCGCCCTATGCTG
GCACCAAATCTGTATAACTATCTGCGCAAACTGGACCGTGCCCTGCCTGATCCTATCAAAATCTTCGAGATCGGC
CCGTGTTATCGTAAAGAGTCCGACGGTAAAGAACATCTGGAGGAGTTTACCATGCTGGCCTTTGCCCAAATGGGT
TCAGGTTGTACTCGTGAGAACCTGGAAAGCATCATCACCGATTTTCTGAACCACCTGGGCATTGACTTCAAAATT
GTGGGCGACAGCTGTATGGTGTATGGCGACACCCTGGATGTCATGCACGGCGACCTGGAACTGTCTAGTGCCGTT
GTTGGACCAATTCCGCTGGACCGTGAGTGGGGTATCGACAAACCGTGGATCGGAGCAGGATTCGGTCTGGAACGC
CTGCTGAAAGTGAAACACGACTTCAAAAACATCAAACGTGCCGCCCGTTCTGAATCGTATTATAACGGGATCTCT
ACGAACCTGTAA  (SEQ ID NO:157)
Protein:
MCRAISLRRLLLLLLQLSQLLAVTQGMLMASNDYTQQATQSYGAYPTQPGQGYSQQSSQPYGQQSYSGYSQSTDT
SGYGQSSYSSYGQSQNTGYGTQSTPQGYGSTGGYGSSQSSQSSYGQQSSYPGYGQQPAPSSTSGSYGSSSQSSSY
GQPQSGSYSQQPSYGGQQQSYGQQQSYNPPQGYGQQNQYNSSSGGGGGGGGGNYGQDQSSMSSGGGSGGGYGNQ
DQSGGGGSGGYGQQDRGGRGRGGSGGGGGGGGGGYNRSSGGYEPRGRGGGRGGRGGMGGSDRGGFNKFGGPRDQG
SRHDSEQDNSDNNTIFVQGLGENVTIESVADYFKQIGIIKTNKKTGQPMINLYTDRETGKLKGEATVSFDDPPSA
KAAIDWFDGKEFSGNPIKVSFATRRADFNRGGGNGRGGRGRGGPMGRGGYGGGGSGGGGRGGFPSGGGGGGGQQR
AGDWKCPNPTCENMNFSWRNECNQCKAPKPDGPGGGPGGSHMGGNYGDDRRGGRGGGTGKLFWALVVVAGVLFCY
GLLVTVALCVIWVRSGMASNDYTQQATQSYGAYPTQPGQGYSQQSSQPYGQQSYSGYSQSTDTSGYGQSSYSSYG
QSQNTGYGTQSTPQGYGSTGGYGSSQSSQSSYGQQSSYPGYGQQPAPSSTSGSYGSSSQSSSYGQPQSGSYSQQP
SYGGQQQSYGQQQSYNPPQGYGQQNQYNSSSGGGGGGGGGNYGQDQSSMSSGGGSGGGYGNQDQSGGGGSGGYG
QQDRGGRGRGGSGGGGGGGGGGYNRSSGGYEPRGRGGGRGGRGGMGGSDRGGFNKFGGPRDQGSRHDSEQDNSDN
NTIFVQGLGENVTIESVADYFKQIGIIKTNKKTGQPMINLYTDRETGKLKGEATVSFDDPPSAKAAIDWFDGKEF
SGNPIKVSFATRRADFNRGGGNGRGGRGRGGPMGRGGYGGGGSGGGGRGGFPSGGGGGGGQQRAGDWKCPNPTCE
NMNFSWRNECNQCKAPKPDGPGGGPGGSHMGGNYGDDRRGGRGGGAPGSAGSAAGSGMACPVPLQLPPLERLTLD
DKKPLNTLISATGLWMSRTGTIHKIKHHEVSRSKIYIEMACGDHLVVNNSRSSRTARALRHHKYRKTCKRCRVSD
EDLNKFLTKANEDQTSVKVKVVSAPTRTKKAMPKSVARAPKPLENTEAAQAQPSGSKFSPAIPVSTQESVSVPAS
VSTSISSISTGATASALVKGNTNPITSMSAPVQASAPALTKSQTDRLEVLLNPKDEISLNSGKPFRELESELLSR
RKKDLQQIYAEERENYLGKLEREITRFFVDRGFLEIKSPILIPLEYIERMGIDNDTELSKQIFRVDKNFCLRPML
APNLYNYLRKLDRALPDPIKIFEIGPCYRKESDGKEHLEEFTMLAFAQMGSGCTRENLESIITDFLNHLGIDFKI
VGDSCMVYGDTLDVMHGDLELSSAVVGPIPLDREWGIDKPWIGAGFGLERLLKVKHDFKNIKRAARSESYYNGIS
TNL  (SEQ ID NO:158)

FUS-CD28-EWSR1-MCP
DNA:

ATGTGCCGAGCCATCTCTCTTAGGCGCTTGCTGCTGCTGCTGCAGCTGTCACAACTCCTAGCTGTCACTCAA
GGGATGCTCATGGCCTCAAACGATTATACCCAACAAGCAACCCAAAGCTATGGGGCCTACCCCACCCAGCCCGGG
CAGGGCTATTCCCAGCAGAGCAGTCAGCCCTACGGACAGCAGAGTTACAGTGGTTATAGCCAGTCCACGGACACT
TCAGGATATGGCCAGAGCAGCTATTCTTCTTATGGCCAGAGCCAGAACACAGGCTATGGAACTCAGTCAACTCCC
CAGGGATATGGCTCGACTGGCGGCTATGGCAGTAGCCAGAGCTCCCAATCGTCTTACGGGCAGCAGTCCTCCTAC
CCTGGCTATGGCCAGCAGCCAGCTCCCAGCAGCACCTCGGGAAGTTACGGTAGCAGTTCTCAGAGCAGCAGCTAT
GGGCAGCCCCAGAGTGGGAGCTACAGCCAGCAGCCTAGCTATGGTGGACAGCAGCAAAGCTATGGACAGCAGCAA
AGCTATAATCCCCCTCAGGGCTATGGACAGCAGAACCAGTACAACAGCAGCAGTGGTGGTGGAGGTGGAGGTGGA
GGTGGAGGTAACTATGGCCAAGATCAATCCTCCATGAGTAGTGGTGGTGGCAGTGGTGGCGGTTATGGCAATCAA
GACCAGAGTGGTGGAGGTGGCAGCGGTGGCTATGGACAGCAGGACCGTGGAGGCCGCGGCAGGGGTGGCAGTGGT
GGCGGCGGCGGCGGCGGCGGTGGTGGTTACAACCGCAGCAGTGGTGGCTATGAACCCAGAGGTCGTGGAGGTGGC
CGTGGAGGCAGAGGTGGCATGGGCGGAAGTGACCGTGGTGGCTTCAATAAATTTGGTGGCCCTCGGGACCAAGGA
TCACGTCATGACTCCGAACAGGATAATTCAGACAACAACACCATCTTTGTGCAAGGCCTGGGTGAGAATGTTACA
ATTGAGTCTGTGGCTGATTACTTCAAGCAGATTGGTATTATTAAGACAAACAAGAAAACGGGACAGCCCATGATT
AATTTGTACACAGACAGGGAAACTGGCAAGCTGAAGGGAGAGGCAACGGTCTCTTTTGATGACCCACCTTCAGCT
AAAGCAGCTATTGACTGGTTTGATGGTAAAGAATTCTCCGGAAATCCTATCAAGGTCTCATTTGCTACTCGCCGG
GCAGACTTTAATCGGGGTGGTGGCAATGGTCGTGGAGGCCGAGGGCGAGGAGGACCCATGGGCCGTGGAGGCTAT
GGAGGTGGTGGCAGTGGTGGTGGTGGCCGAGGAGGATTTCCCAGTGGAGGTGGTGGCGGTGGAGGACAGCAGCGA
GCTGGTGACTGGAAGTGTCCTAATCCCACCTGTGAGAATATGAACTTCTCTTGGAGGAATGAATGCAACCAGTGT
AAGGCCCCTAAACCAGATGGCCCAGGAGGGGGACCAGGTGGCTCTCACATGGGGGGTAACTACGGGGATGATCGT
CGTGGTGGCAGAGGAGGCGGCACCGGTAAGCTTTTTTGGGCACTGGTCGTGGTTGCTGGAGTCCTGTTTTGTTAT
GGCTTGCTAGTGACAGTGGCTCTTTGTGTTATCTGGGTAAGATCTATGGCGTCCACGGATTACAGTACCTATAGC
CAAGCTGCAGCGCAGCAGGGCTACAGTGCTTACACCGCCCAGCCCACTCAAGGATATGCACAGACCACCCAGGCA
TATGGGCAACAAAGCTATGGAACCTATGGACAGCCCACTGATGTCAGCTATACCCAGGCTCAGACCACTGCAACC
TATGGGCAGACCGCCTATGCAACTTCTTATGGACAGCCTCCCACTGGTTATACTACTCCAACTGCCCCCCAGGCA
TACAGCCAGCCTGTCCAGGGGTATGGCACTGGTGCTTATGATACCACCACTGCTACAGTCACCACCACCCAGGCC
TCCTATGCAGCTCAGTCTGCATATGGCACTCAGCCTGCTTATCCAGCCTATGGGCAGCAGCCAGCAGCCACTGCA
CCTACAAGACCGCAGGATGGAAACAAGCCCACTGAGACTAGTCAACCTCAATCTAGCACAGGGGGTTACAACCAG
CCCAGCCTAGGATATGGACAGAGTAACTACAGTTATCCCCAGGTACCTGGGAGCTACCCCATGCAGCCAGTCACT
GCACCTCCATCCTACCCTCCTACCAGCTATTCCTCTACACAGCCGACTAGTTATGATCAGAGCAGTTACTCTCAG
CAGAACACCTATGGGCAACCGAGCAGCTATGGACAGCAGAGTAGCTATGGTCAACAAAGCAGCTATGGGCAGCAG
CCTCCCACTAGTTACCCACCCCAAACTGGATCCTACAGCCAAGCTCCAAGTCAATATAGCCAACAGAGCAGCAGC
TACGGGCAGCAGAGTTCATTCCGACAGGACCACCCCAGTAGCATGGGTGTTTATGGGCAGGAGTCTGGAGGATTT
TCCGGACCAGGAGAGAACCGGAGCATGAGTGGCCCTGATAACCGGGGCAGGGGAAGAGGGGGATTTGATCGTGGA
GGCATGAGCAGAGGTGGGCGGGGAGGAGGACGCGGTGGAATGGGCAGCGCTGGAGAGCGAGGTGGCTTCAATAAG
CCTGGTGGACCCATGGATGAAGGACCAGATCTTGATCTAGGCCCACCTGTAGATCCAGATGAAGACTCTGACAAC
AGTGCAATTTATGTACAAGGATTAAATGACAGTGTGACTCTAGATGATCTGGCAGACTTCTTTAAGCAGTGTGGG
GTTGTTAAGATGAACAAGAGAACTGGGCAACCCATGATCCACATCTACCTGGACAAGGAAACAGGAAAGCCCAAA
GGCGATGCCACAGTGTCCTATGAAGACCCACCCACTGCCAAGGCTGCCGTGGAATGGTTTGATGGGAAAGATTTT
CAAGGGAGCAAACTTAAAGTCTCCCTTGCTCGGAAGAAGCCTCCAATGAACAGTATGCGGGGTGGTCTGCCACCC
CGTGAGGGCAGAGGCATGCCACCACCACTCCGTGGAGGTCCAGGAGGCCCAGGAGGTCCTGGGGGACCCATGGGT
CGCATGGGAGGCCGTGGAGGAGATAGAGGAGGCTTCCCTCCAAGAGGACCCCGGGGTTCCCGAGGGAACCCCTCT
GGAGGAGGAAACGTCCAGCACCGAGCTGGAGACTGGCAGTGTCCCAATCCGGGTTGTGGAAACCAGAACTTCGCC
TGGAGAACAGAGTGCAACCAGTGTAAGGCCCCAAAGCCTGAAGGCTTCCTCCCGCCACCCTTTCCGCCCCCGGGT
GGTGATCGTGGCAGAGGTGGCCCTGGTGGCATGCGGGGAGGAAGAGGTGGCCTCATGGATCGTGGTGGTCCCGGT
GGAATGTTCAGAGGTGGCCGTGGTGGAGACAGAGGTGGCTTCCGTGGTGGCCGGGGCATGGACCGAGGTGGCTTT
GGTGGAGGAAGACGAGGTGGCCCTGGGGGGCCCCCTGGACCTTTGATGGAACAGGATTACAAGGATGACGACGAT
AAGGGTACCGAGCAGAAGCTGATCTCAGAGGAGGACCTGGGCGCCCCCGGCTCCGCCGGCTCCGCCGCCGGCTCC
GGCGCTTCTAACTTTACTCAGTTCGTTCTCGTCGACAATGGCGGAACTGGCGACGTGACTGTCGCCCCAAGCAAC
TTCGCTAACGGGATCGCTGAATGGATCAGCTCTAACTCGCGTTCACAGGCTTACAAAGTAACCTGTAGCGTTCGT
CAGAGCTCTGCGCAGAATCGCAAATACACCATCAAAGTCGAGGTGCCTAAAGGCGCCTGGCGTTCGTACTTAAAT
ATGGAACTAACCATTCCAATTTTCGCCACGAATTCCGACTGCGAGCTTATTGTTAAGGCAATGCAAGGTCTCCTA
AAAGATGGAAACCCGATTCCCTCAGCAATCGCAGCAAACTCCGGCATCTACTAA   (SEQ ID NO:159)
Protein:
MCRAISLRRLLLLLLQLSQLLAVTQGMLMASNDYTQQATQSYGAYPTQPGQGYSQQSSQPYGQQSYSGYSQSTDT
SGYGQSSYSSYGQSQNTGYGTQSTPQGYGSTGGYGSSQSSQSSYGQQSSYPGYGQQPAPSSTSGSYGSSSQSSSY
GQPQSGSYSQQPSYGGQQQSYGQQQSYNPPQGYGQQNQYNSSSGGGGGGGGGNYGQDQSSMSSGGGSGGGYGNQ
DQSGGGGSGGYGQQDRGGRGRGGSGGGGGGGGGYNRSSGGYEPRGRGGGRGGRGGMGGSDRGGFNKFGGPRDQG

SRHDSEQDNSDNNTIFVQGLGENVTIESVADYFKQIGIIKTNKKTGQPMINLYTDRETGKLKGEATVSFDDPPSA
KAAIDWFDGKEFSGNPIKVSFATRRADFNRGGGNGRGGRGRGGPMGRGGYGGGGSGGGGRGGFPSGGGGGGGQQR
AGDWKCPNPTCENMNFSWRNECNQCKAPKPDGPGGGPGGSHMGGNYGDDRRGGRGGGTGKLFWALVVVAGVLFCY
GLLVTVALCVIWVRSMASTDYSTYSQAAAQQGYSAYTAQPTQGYAQTTQAYGQQSYGTYGQPTDVSYTQAQTTAT
YGQTAYATSYGQPPTGYTTPTAPQAYSQPVQGYGTGAYDTTTATVTTTQASYAAQSAYGTQPAYPAYGQQPAATA
PTRPQDGNKPTETSQPQSSTGGYNQPSLGYGQSNYSYPQVPGSYPMQPVTAPPSYPPTSYSSTQPTSYDQSSYSQ
QNTYGQPSSYGQQSSYGQQSSYGQQPPTSYPPQTGSYSQAPSQYSQQSSSYGQQSSFRQDHPSSMGVYGQESGGF
SGPGENRSMSGPDNRGRGRGGFDRGGMSRGGRGGGRGGMGSAGERGGFNKPGGPMDEGPDLDLGPPVDPDEDSDN
SAIYVQGLNDSVTLDDLADFFKQCGVVKMNKRTGQPMIHIYLDKETGKPKGDATVSYEDPPTAKAAVEWFDGKDF
QGSKLKVSLARKKPPMNSMRGGLPPREGRGMPPPLRGGPGGPGGPGGPMGRMGGRGGDRGGFPPRGPRGSRGNPS
GGGNVQHRAGDWQCPNPGCGNQNFAWRTECNQCKAPKPEGFLPPPFPPPGGDRGRGGPGGMRGGRGGLMDRGGPG
GMFRGGRGGDRGGFRGGRGMDRGGFGGGRRGGPGGPPGPLMEQDYKDDDDKGTEQKLISEEDLGAPGSAGSAAGS
GASNFTQFVLVDNGGTGDVTVAPSNFANGIAEWISSNSRSQAYKVTCSVRQSSAQNRKYTIKVEVPKGAWRSYLN
MELTIPIFATNSDCELIVKAMQGLLKDGNPIPSAIAANSGIY  (SEQ ID NO:160)

FUS-CD28-EWSR1-4xλN22
DNA:
ATGTGCCGAGCCATCTCTCTTAGGCGCTTGCTGCTGCTGCTGCAGCTGTCACAACTCCTAGCTGTCACTCAA
GGGATGCTCATGGCCTCAAACGATTATACCCAACAAGCAACCCAAAGCTATGGGGCCTACCCCACCCAGCCCGGG
CAGGGCTATTCCCAGCAGAGCAGTCAGCCCTACGGACAGCAGAGTTACAGTGGTTATAGCCAGTCCACGGACACT
TCAGGATATGGCCAGAGCAGCTATTCTTCTTATGGCCAGAGCCAGAACACAGGCTATGGAACTCAGTCAACTCCC
CAGGGATATGGCTCGACTGGCGGCTATGGCAGTAGCCAGAGCTCCCAATCGTCTTACGGGCAGCAGTCCTCCTAC
CCTGGCTATGGCCAGCAGCCAGCTCCCAGCAGCACCTCGGGAAGTTACGGTAGCAGTTCTCAGAGCAGCAGCTAT
GGGCAGCCCCAGAGTGGGAGCTACAGCCAGCAGCCTAGCTATGGTGGACAGCAGCAAAGCTATGGACAGCAGCAA
AGCTATAATCCCCCTCAGGGCTATGGACAGCAGAACCAGTACAACAGCAGCAGTGGTGGTGGAGGTGGAGGTGGA
GGTGGAGGTAACTATGGCCAAGATCAATCCTCCATGAGTAGTGGTGGTGGCAGTGGTGGCGGTTATGGCAATCAA
GACCAGAGTGGTGGAGGTGGCAGCGGTGGCTATGGACAGCAGGACCGTGGAGGCCGCGGCAGGGGTGGCAGTGGT
GGCGGCGGCGGCGGCGGTGGTGGTTACAACCGCAGCAGTGGTGGCTATGAACCCAGAGGTCGTGGAGGTGGC
CGTGGAGGCAGAGGTGGCATGGGCGGAAGTGACCGTGGTGGCTTCAATAAATTTGGTGGCCCTCGGGACCAAGGA
TCACGTCATGACTCCGAACAGGATAATTCAGACAACAACACCATCTTTGTGCAAGGCCTGGGTGAGAATGTTACA
ATTGAGTCTGTGGCTGATTACTTCAAGCAGATTGGTATTATTAAGACAAACAAGAAAACGGGACAGCCCATGATT
AATTTGTACACAGACAGGGAAACTGGCAAGCTGAAGGGAGAGGCAACGGTCTCTTTTGATGACCCACCTTCAGCT
AAAGCAGCTATTGACTGGTTTGATGGTAAAGAATTCTCCGGAAATCCTATCAAGGTCTCATTTGCTACTCGCCGG
GCAGACTTTAATCGGGGTGGTGGCAATGGTCGTGGAGGCCGAGGGCGAGGAGGACCCATGGGCCGTGGAGGCTAT
GGAGGTGGTGGCAGTGGTGGTGGTGGCCGAGGAGGATTTCCCAGTGGAGGTGGTGGCGGTGGAGGACAGCAGCGA
GCTGGTGACTGGAAGTGTCCTAATCCCACCTGTGAGAATATGAACTTCTCTTGGAGGAATGAATGCAACCAGTGT
AAGGCCCCTAAACCAGATGGCCCAGGAGGGGGACCAGGTGGCTCTCACATGGGGGGTAACTACGGGGATGATCGT
CGTGGTGGCAGAGGAGGCGGCACCGGTAAGCTTTTTTGGGCACTGGTCGTGGTTGCTGGAGTCCTGTTTTGTTAT
GGCTTGCTAGTGACAGTGGCTCTTTGTGTTATCTGGGTAAGATCTATGGCGTCCACGGATTACAGTACCTATAGC
CAAGCTGCAGCGCAGCAGGGCTACAGTGCTTACACCGCCCAGCCCACTCAAGGATATGCACAGACCACCCAGGCA
TATGGGCAACAAAGCTATGGAACCTATGGACAGCCCACTGATGTCAGCTATACCCAGGCTCAGACCACTGCAACC
TATGGGCAGACCGCCTATGCAACTTCTTATGGACAGCCTCCCACTGGTTATACTACTCCAACTGCCCCCCAGGCA
TACAGCCAGCCTGTCCAGGGGTATGGCACTGGTGCTTATGATACCACCACTGCTACAGTCACCACCACCCAGGCC
TCCTATGCAGCTCAGTCTGCATATGGCACTCAGCCTGCTTATCCAGCCTATGGGCAGCAGCCAGCAGCCACTGCA
CCTACAAGACCGCAGGATGGAAACAAGCCCACTGAGACTAGTCAACCTCAATCTAGCACAGGGGGTTACAACCAG
CCCAGCCTAGGATATGGACAGAGTAACTACAGTTATCCCCAGGTACCTGGGAGCTACCCCATGCAGCCAGTCACT
GCACCTCCATCCTACCCTCCTACCAGCTATTCCTCTACACAGCCGACTAGTTATGATCAGAGCAGTTACTCTCAG
CAGAACACCTATGGGCAACCGAGCAGCTATGGACAGCAGAGTAGCTATGGTCAACAAAGCAGCTATGGGCAGCAG
CCTCCCACTAGTTACCCACCCCAAACTGGATCCTACAGCCAAGCTCCAAGTCAATATAGCCAACAGAGCAGCAGC
TACGGGCAGCAGAGTTCATTCCGACAGGACCACCCCAGTAGCATGGGTGTTTATGGGCAGGAGTCTGGAGGATTT
TCCGGACCAGGAGAGAACCGGAGCATGAGTGGCCCTGATAACCGGGGCAGGGGAAGAGGGGGATTTGATCGTGGA
GGCATGAGCAGAGGTGGGCGGGGAGGAGGACGCGGTGGAATGGGCAGCGCTGGAGAGCGAGGTGGCTTCAATAAG
CCTGGTGGACCCATGGATGAAGGACCAGATCTTGATCTAGGCCCACCTGTAGATCCAGATGAAGACTCTGACAAC
AGTGCAATTTATGTACAAGGATTAAATGACAGTGTGACTCTAGATGATCTGGCAGACTTCTTTAAGCAGTGTGGG
GTTGTTAAGATGAACAAGAGAACTGGGCAACCCATGATCCACATCTACCTGGACAAGGAAACAGGAAAGCCCAAA
GGCGATGCCACAGTGTCCTATGAAGACCCACCTACTGCCAAGGCTGCCGTGGAATGGTTTGATGGGAAAGATTTT
CAAGGGAGCAAACTTAAAGTCTCCCTTGCTCGGAAGAAGCCTCCAATGAACAGTATGCGGGGTGGTCTGCCACCC
CGTGAGGGCAGAGGCATGCCACCACCACTCCGTGGAGGTCCAGGAGGCCCAGGAGGTCCTGGGGGACCCATGGGT

CGCATGGGAGGCCGTGGAGGAGATAGAGGAGGCTTCCCTCCAAGAGGACCCCGGGGTTCCCGAGGGAACCCCTCT
GGAGGAGGAAACGTCCAGCACCGAGCTGGAGACTGGCAGTGTCCCAATCCGGGTTGTGGAAACCAGAACTTCGCC
TGGAGAACAGAGTGCAACCAGTGTAAGGCCCCAAAGCCTGAAGGCTTCCTCCCGCCACCCTTTCCGCCCCCGGGT
GGTGATCGTGGCAGAGGTGGCCCTGGTGGCATGCGGGGAGGAAGAGGTGGCCTCATGGATCGTGGTGGTCCCGGT
GGAATGTTCAGAGGTGGCCGTGGTGGAGACAGAGGTGGCTTCCGTGGTGGCCGGGGCATGGACCGAGGTGGCTTT
GGTGGAGGAAGACGAGGTGGCCCTGGGGGGCCCCCTGGACCTTTGATGGAACAGGATTACAAGGATGACGACGAT
AAGGGTACCGAGCAGAAGCTGATCTCAGAGGAGGACCTGCTAGCCACCATGGACGCACAAACACGACGACGTGAG
CGTCGCGCTGAGAAACAAGCTCAATGGAAAGCTGCAAACCCACCGCTCGACGGAGCCGGAGCTGGCGCTGGAGCT
GGAGCCGGAGCTGGCGGTCTAGCCACCATGGACGCACAAACACGACGACGTGAGCGTCGCGCTGAGAAACAAGCT
CAATGGAAAGCTGCAAACCCACCGCTCGACGGAGCCGGAGCTGGCGCTGGAGCTGGAGCCGGAGCTGGCGGTCTA
GCCACCATGGACGCACAAACACGACGACGTGAGCGTCGCGCTGAGAAACAAGCTCAATGGAAAGCTGCAAACCCA
CCGCTCGACGGAGCCGGAGCTGGCGCTGGAGCTGGAGCCGGAGCTGGCGGTCTAGCCACCATGGACGCACAAACA
CGACGACGTGAGCGTCGCGCTGAGAAACAAGCTCAATGGAAAGCTGCAAACCCACCGCTCGAGTCTAGAGGGCCC
GTTTAA  (SEQ ID NO:161)
Protein:
MCRAISLRRLLLLLLQLSQLLAVTQGMLMASNDYTQQATQSYGAYPTQPGQGYSQQSSQPYGQQSYSGYSQSTDT
SGYGQSSYSSYGQSQNTGYGTQSTPQGYGSTGGYGSSQSSQSSYGQQSSYPGYGQQPAPSSTSGSYGSSSQSSSY
GQPQSGSYSQQPSYGGQQQSYGQQQSYNPPQGYGQQNQYNSSSGGGGGGGGGGNYGQDQSSMSSGGGSGGGYGNQ
DQSGGGGSGGYGQQDRGGRGRGGSGGGGGGGGGGYNRSSGGYEPRGRGGGRGGRGGMGGSDRGGFNKFGGPRDQG
SRHDSEQDNSDNNTIFVQGLGENVTIESVADYFKQIGIIKTNKKTGQPMINLYTDRETGKLKGEATVSFDDPPSA
KAAIDWFDGKEFSGNPIKVSFATRRADFNRGGGNGRGGRGRGGPMGRGGYGGGGSGGGGRGGFPSGGGGGGGQQR
AGDWKCPNPTCENMNFSWRNECNQCKAPKPDGPGGGPGGSHMGGNYGDDRRGGRGGGTGKLFWALVVVAGVLFCY
GLLVTVALCVIWVRSMASTDYSTYSQAAAQQGYSAYTAQPTQGYAQTTQAYGQQSYGTYGQPTDVSYTQAQTTAT
YGQTAYATSYGQPPTGYTTPTAPQAYSQPVQGYGTGAYDTTTATVTTTQASYAAQSAYGTQPAYPAYGQQPAATA
PTRPQDGNKPTETSQPQSSTGGYNQPSLGYGQSNYSYPQVPGSYPMQPVTAPPSYPPTSYSSTQPTSYDQSSYSQ
QNTYGQPSSYGQQSSYGQQSSYGQQPPTSYPPQTGSYSQAPSQYSQQSSSYGQQSSFRQDHPSSMGVYGQESGGF
SGPGENRSMSGPDNRGRGRGGFDRGGMSRGGRGGGRGGMGSAGERGGFNKPGGPMDEGPDLDLGPPVDPDEDSDN
SAIYVQGLNDSVTLDDLADFFKQCGVVKMNKRTGQPMIHIYLDKETGKPKGDATVSYEDPPTAKAAVEWFDGKDF
QGSKLKVSLARKKPPMNSMRGGLPPREGRGMPPPLRGGPGGPGGPGGPMGRMGGRGGDRGGFPPRGPRGSRGNPS
GGGNVQHRAGDWQCPNPGCGNQNFAWRTECNQCKAPKPEGFLPPPFPPPGGDRGRGGPGGMRGGRGGLMDRGGPG
GMFRGGRGGDRGGFRGGRGMDRGGFGGGRRGGPGGPPGPLMEQDYKDDDDKGTEQKLISEEDLLATMDAQTRRRE
RRAEKQAQWKAANPPLDGAGAGAGAGAGAGGLATMDAQTRRRERRAEKQAQWKAANPPLDGAGAGAGAGAGAGGL
ATMDAQTRRRERRAEKQAQWKAANPPLDGAGAGAGAGAGAGGLATMDAQTRRRERRAEKQAQWKAANPPLESRGP
V  (SEQ ID NO:162)

FRB-CD28-FUS-MCP-PylRS^AA
DNA:
ATGTGCCGAGCCATCTCTCTTAGGCGCTTGCTGCTGCTGCTGCTGCAGCTGTCACAACTCCTAGCTGTCACTCAA
GGGATGCTCGAGATGTGGCATGAAGGCCTGGAAGAGGCATCTCGTTTGTACTTTGGGGAAAGGAACGTGAAAGGC
ATGTTTGAGGTGCTGGAGCCCTTGCATGCTATGATGGAACGGGGCCCCCAGACTCTGAAGGAAACATCCTTTAAT
CAGGCCTATGGTCGAGATTTAATGGAGGCCCAAGAGTGGTGCAGGAAGTACATGAAATCAGGGAATGTCAAGGAC
CTCCTCCAAGCCTGGGACCTCTATTATCATGTGTTCCGACGAATCTCAAAGACTAGAACCGGTAAGCTTTTTTGG
GCACTGGTCGTGGTTGCTGGAGTCCTGTTTTGTTATGGCTTGCTAGTGACAGTGGCTCTTTGTGTTATCTGGGTA
AGATCTGGTATGGCCTCAAACGATTATACCCAACAAGCAACCCAAAGCTATGGGGCCTACCCCACCCAGCCCGGG
CAGGGCTATTCCCAGCAGAGCAGTCAGCCCTACGGACAGCAGAGTTACAGTGGTTATAGCCAGTCCACGGACACT
TCAGGATATGGCCAGAGCAGCTATTCTTCTTATGGCCAGAGCCAGAACACAGGCTATGGAACTCAGTCAACTCCC
CAGGGATATGGCTCGACTGGCGGCTATGGCAGTAGCCAGAGCTCCCAATCGTCTTACGGGCAGCAGTCCTCCTAC
CCTGGCTATGGCCAGCAGCCAGCTCCCAGCAGCACCTCGGGAAGTTACGGTAGCAGTTCTCAGAGCAGCAGCTAT
GGGCAGCCCCAGAGTGGGAGCTACAGCCAGCAGCCTAGCTATGGTGGACAGCAGCAAAGCTATGGACAGCAGCAA
AGCTATAATCCCCCTCAGGGCTATGGACAGCAGAACCAGTACAACAGCAGCAGTGGTGGTGGAGGTGGAGGTGGA
GGTGGAGGTAACTATGGCCAAGATCAATCCTCCATGAGTAGTGGTGGTGGCAGTGGTGGCGGTTATGGCAATCAA
GACCAGAGTGGTGGAGGTGGCAGCGGTGGCTATGGACAGCAGGACCGTGGAGGCCGCGGCAGGGGTGGCAGTGGT
GGCGGCGGCGGCGGCGGCGGTGGTGGTTACAACCGCAGCAGTGGTGGCTATGAACCCAGAGGTCGTGGAGGTGGC
CGTGGAGGCAGAGGTGGCATGGGCGGAAGTGACCGTGGTGGCTTCAATAAATTTGGTGGCCCTCGGGACCAAGGA
TCACGTCATGACTCCGAACAGGATAATTCAGACAACAACACCATCTTTGTGCAAGGCCTGGGTGAGAATGTTACA
ATTGAGTCTGTGGCTGATTACTTCAAGCAGATTGGTATTATTAAGACAAACAAGAAAACGGGACAGCCCATGATT
AATTTGTACACAGACAGGGAAACTGGCAAGCTGAAGGGAGAGGCAACGGTCTCTTTTGATGACCCACCTTCAGCT
AAAGCAGCTATTGACTGGTTTGATGGTAAAGAATTCTCCGGAAATCCTATCAAGGTCTCATTTGCTACTCGCCGG

GCAGACTTTAATCGGGGTGGTGGCAATGGTCGTGGAGGCCGAGGGCGAGGAGGACCCATGGGCCGTGGAGGCTAT
GGAGGTGGTGGCAGTGGTGGTGGTGGCCGAGGAGGATTTCCCAGTGGAGGTGGTGGCGGTGGAGGACAGCAGCGA
GCTGGTGACTGGAAGTGTCCTAATCCCACCTGTGAGAATATGAACTTCTCTTGGAGGAATGAATGCAACCAGTGT
AAGGCCCCTAAACCAGATGGCCCAGGAGGGGGACCAGGTGGCTCTCACATGGGGGGTAACTACGGGGATGATCGT
CGTGGTGGCAGAGGAGGCGATTACAAGGATGACGACGATAAGGGTACCGGCGCCCCCGGCTCCGCCGGCTCCGCC
GCCGGCTCCGGCGCTTCTAACTTTACTCAGTTCGTTCTCGTCGACAATGGCGGAACTGGCGACGTGACTGTCGCC
CCAAGCAACTTCGCTAACGGGATCGCTGAATGGATCAGCTCTAACTCGCGTTCACAGGCTTACAAAGTAACCTGT
AGCGTTCGTCAGAGCTCTGCGCAGAATCGCAAATACACCATCAAAGTCGAGGTGCCTAAAGGCGCCTGGCGTTCG
TACTTAAATATGGAACTAACCATTCCAATTTTCGCCACGAATTCCGACTGCGAGCTTATTGTTAAGGCAATGCAA
GGTCTCCTAAAAGATGGAAACCCGATTCCCTCAGCAATCGCAGCAAACTCCGGCATCTACGGTACCGGCGCCCCC
GGCTCCGCCGGCTCCGCCGCCGGCTCCGGCGCGTGCCCGGTGCCGCTGCAGCTGCCGCCGCTGGAACGCCTGACC
CTGGATGATAAAAAACCGCTGAATACCCTGATCTCTGCTACTGGTCTGTGGATGAGTCGTACCGGAACCATTCAT
AAAATCAAACACCACGAGGTTAGCCGTTCGAAAATCTATATTGAGATGGCGTGTGGCGATCATCTGGTTGTGAAC
AATAGCCGCTCTTCTCGTACAGCACGTGCACTGCGTCACCACAAATATCGTAAAACCTGTAAACGTTGCCGTGTG
TCCGATGAGGATCTGAACAAATTCCTGACAAAAGCCAATGAGGACCAAACAAGCGTGAAAGTGAAAGTCGTTAGC
GCTCCTACCCGTACTAAAAAAGCAATGCCGAAATCCGTTGCTCGTGCCCCTAAACCACTGGAAAACACTGAAGCA
GCACAGGCACAGCCGTCTGGAAGCAAATTCTCTCCGGCCATTCCTGTTTCTACCCAGGAGTCCGTTTCTGTTCCA
GCAAGTGTGAGCACCAGCATTAGCAGTATTAGCACCGGTGCCACCGCTAGCGCCCTGGTTAAAGGCAATACCAAT
CCGATTACAAGCATGTCTGCCCCGGTTCAAGCATCAGCTCCAGCACTGACAAAATCCCAAACCGATCGTCTGGAG
GTTCTGCTGAATCCGAAAGACGAAATCAGCCTGAATTCCGGCAAACCGTTTCGTGAACTGGAGAGCGAACTGCTG
TCACGTCGTAAAAAAGACCTGCAACAAATCTATGCCGAAGAACGTGAGAACTATCTGGGGAAACTGGAACGTGAA
ATCACCCGCTTTTTCGTGGATCGTGGCTTTCTGGAGATCAAATCCCCGATTCTGATTCCTCTGGAGTATATCGAG
CGTATGGGCATCGACAATGATACCGAACTGAGCAAACAAATTTTCCGTGTGGATAAAAACTTCTGTCTGCGCCCT
ATGCTGGCACCAAATCTGTATAACTATCTGCGCAAACTGGACCGTGCCCTGCCTGATCCTATCAAAATCTTCGAG
ATCGGCCCGTGTTATCGTAAAGAGTCCGACGGTAAAGAACATCTGGAGGAGTTTACCATGCTGGCCTTTGCCCAA
ATGGGTTCAGGTTGTACTCGTGAGAACCTGGAAAGCATCATCACCGATTTTCTGAACCACCTGGGCATTGACTTC
AAAATTGTGGGCGACAGCTGTATGGTGTATGGCGACACCCTGGATGTCATGCACGGCGACCTGGAACTGTCTAGT
GCCGTTGTTGGACCAATTCCGCTGGACCGTGAGTGGGGTATCGACAAACCGTGGATCGGAGCAGGATTCGGTCTG
GAACGCCTGCTGAAAGTGAAACACGACTTCAAAAACATCAAACGTGCCGCCCGTTCTGAATCGTATTATAACGGG
ATCTCTACGAACCTGTAA (SEQ ID NO:163)
Protein:
MCRAISLRRLLLLLLQLSQLLAVTQGMLEMWHEGLEEASRLYFGERNVKGMFEVLEPLHAMMERGPQTLKETSFN
QAYGRDLMEAQEWCRKYMKSGNVKDLLQAWDLYYHVFRRISKTRTGKLFWALVVVAGVLFCYGLLVTVALCVIWV
RSGMASNDYTQQATQSYGAYPTQPGQGYSQQSSQPYGQQSYSGYSQSTDTSGYGQSSYSSYGQSQNTGYGTQSTP
QGYGSTGGYGSSQSSQSSYGQQSSYPGYGQQPAPSSTSGSYGSSSQSSSYGQPQSGSYSQQPSYGGQQQSYGQQQ
SYNPPQGYGQQNQYNSSSGGGGGGGGGGNYGQDQSSMSSGGGSGGGYGNQDQSGGGGSGGYGQQDRGGRGRGGSG
GGGGGGGGGYNRSSGGYEPRGRGGGRGGRGGMGGSDRGGFNKFGGPRDQGSRHDSEQDNSDNNTIFVQGLGENVT
IESVADYFKQIGIIKTNKKTGQPMINLYTDRETGKLKGEATVSFDDPPSAKAAIDWFDGKEFSGNPIKVSFATRR
ADFNRGGGNGRGGRGRGGPMGRGGYGGGGSGGGGRGGFPSGGGGGGGQQRAGDWKCPNPTCENMNFSWRNECNQC
KAPKPDGPGGGPGGSHMGGNYGDDRRGGRGGDYKDDDDKGTGAPGSAGSAAGSGASNFTQFVLVDNGGTGDVTVA
PSNFANGIAEWISSNSRSQAYKVTCSVRQSSAQNRKYTIKVEVPKGAWRSYLNMELTIPIFATNSDCELIVKAMQ
GLLKDGNPIPSAIAANSGIYGTGAPGSAGSAAGSGACPVPLQLPPLERLTLDDKKPLNTLISATGLWMSRTGTIH
KIKHHEVSRSKIYIEMACGDHLVVNNSRSSRTARALRHHKYRKTCKRCRVSDEDLNKFLTKANEDQTSVKVKVVS
APTRTKKAMPKSVARAPKPLENTEAAQAQPSGSKFSPAIPVSTQESVSVPASVSTSISSISTGATASALVKGNTN
PITSMSAPVQASAPALTKSQTDRLEVLLNPKDEISLNSGKPFRELESELLSRRKKDLQQIYAEERENYLGKLERE
ITRFFVDRGFLEIKSPILIPLEYIERMGIDNDTELSKQIFRVDKNFCLRPMLAPNLYNYLRKLDRALPDPIKIFE
IGPCYRKESDGKEHLEEFTMLAFAQMGSGCTRENLESIITDFLNHLGIDFKIVGDSCMVYGDTLDVMHGDLELSS
AVVGPIPLDREWGIDKPWIGAGFGLERLLKVKHDFKNIKRAARSESYYNGISTNL (SEQ ID NO:164)

FRB-CD28-FUS-MCP-PylRS^AF
DNA:
ATGTGCCGAGCCATCTCTCTTAGGCGCTTGCTGCTGCTGCTGCTGCAGCTGTCACAACTCCTAGCTGTCACTCAA
GGGATGCTCGAGATGTGGCATGAAGGCCTGGAAGAGGCATCTCGTTTGTACTTTGGGGAAAGGAACGTGAAAGGC
ATGTTTGAGGTGCTGGAGCCCTTGCATGCTATGATGGAACGGGGCCCCCAGACTCTGAAGGAAACATCCTTTAAT
CAGGCCTATGGTCGAGATTTAATGGAGGCCCAAGAGTGGTGCAGGAAGTACATGAAATCAGGGAATGTCAAGGAC
CTCCTCCAAGCCTGGGACCTCTATTATCATGTGTTCCGACGAATCTCAAAGACTAGAACCGGTAAGCTTTTTTGG
GCACTGGTCGTGGTTGCTGGAGTCCTGTTTTGTTATGGCTTGCTAGTGACAGTGGCTCTTTGTGTTATCTGGGTA
AGATCTGGTATGGCCTCAAACGATTATACCCAACAAGCAACCCAAAGCTATGGGGCCTACCCCACCCAGCCCGGG

CAGGGCTATTCCCAGCAGAGCAGTCAGCCCTACGGACAGCAGAGTTACAGTGGTTATAGCCAGTCCACGGACACT
TCAGGATATGGCCAGAGCAGCTATTCTTCTTATGGCCAGAGCCAGAACACAGGCTATGGAACTCAGTCAACTCCC
CAGGGATATGGCTCGACTGGCGGCTATGGCAGTAGCCAGAGCTCCCAATCGTCTTACGGGCAGCAGTCCTCCTAC
CCTGGCTATGGCCAGCAGCCAGCTCCCAGCAGCACCTCGGGAAGTTACGGTAGCAGTTCTCAGAGCAGCAGCTAT
GGGCAGCCCCAGAGTGGGAGCTACAGCCAGCAGCCTAGCTATGGTGGACAGCAGCAAAGCTATGGACAGCAGCAA
AGCTATAATCCCCCTCAGGGCTATGGACAGCAGAACCAGTACAACAGCAGCAGTGGTGGTGGAGGTGGAGGTGGA
GGTGGAGGTAACTATGGCCAAGATCAATCCTCCATGAGTAGTGGTGGTGGCAGTGGTGGCGGTTATGGCAATCAA
GACCAGAGTGGTGGAGGTGGCAGCGGTGGCTATGGACAGCAGGACCGTGGAGGCCGCGGCAGGGGTGGCAGTGGT
GGCGGCGGCGGCGGCGGTGGTGGTTACAACCGCAGCAGTGGTGGCTATGAACCCAGAGGTCGTGGAGGTGGC
CGTGGAGGCAGAGGTGGCATGGGCGGAAGTGACCGTGGTGGCTTCAATAAATTTGGTGGCCCTCGGGACCAAGGA
TCACGTCATGACTCCGAACAGGATAATTCAGACAACAACACCATCTTTGTGCAAGGCCTGGGTGAGAATGTTACA
ATTGAGTCTGTGGCTGATTACTTCAAGCAGATTGGTATTATTAAGACAAACAAGAAAACGGGACAGCCCATGATT
AATTTGTACACAGACAGGGAAACTGGCAAGCTGAAGGGAGAGGCAACGGTCTCTTTTGATGACCCACCTTCAGCT
AAAGCAGCTATTGACTGGTTTGATGGTAAAGAATTCTCCGGAAATCCTATCAAGGTCTCATTTGCTACTCGCCGG
GCAGACTTTAATCGGGGTGGTGGCAATGGTCGTGGAGGCCGAGGGCGAGGAGGACCCATGGGCCGTGGAGGCTAT
GGAGGTGGTGGCAGTGGTGGTGGTGGCCGAGGAGGATTTCCCAGTGGAGGTGGTGGCGGTGGAGGACAGCAGCGA
GCTGGTGACTGGAAGTGTCCTAATCCCACCTGTGAGAATATGAACTTCTCTTGGAGGAATGAATGCAACCAGTGT
AAGGCCCCTAAACCAGATGGCCCAGGAGGGGGACCAGGTGGCTCTCACATGGGGGGTAACTACGGGGATGATCGT
CGTGGTGGCAGAGGAGGCGATTACAAGGATGACGACGATAAGGGTACCGGCGCCCCCGGCTCCGCCGGCTCCGCC
GCCGGCTCCGGCGCTTCTAACTTTACTCAGTTCGTTCTCGTCGACAATGGCGGAACTGGCGACGTGACTGTCGCC
CCAAGCAACTTCGCTAACGGGATCGCTGAATGGATCAGCTCTAACTCGCGTTCACAGGCTTACAAAGTAACCTGT
AGCGTTCGTCAGAGCTCTGCGCAGAATCGCAAATACACCATCAAAGTCGAGGTGCCTAAAGGCGCCTGGCGTTCG
TACTTAAATATGGAACTAACCATTCCAATTTTCGCCACGAATTCCGACTGCGAGCTTATTGTTAAGGCAATGCAA
GGTCTCCTAAAAGATGGAAACCCGATTCCCTCAGCAATCGCAGCAAACTCCGGCATCTACGGTACCGGCGCCCCC
GGCTCCGCCGGCTCCGCCGCCGGCTCCGGCGCGTGCCCGGTGCCGCTGCAGCTGCCGCCGCTGGAACGCCTGACC
CTGGATGATAAAAAACCGCTGAATACCCTGATCTCTGCTACTGGTCTGTGGATGAGTCGTACCGGAACCATTCAT
AAAATCAAACACCACGAGGTTAGCCGTTCGAAAATCTATATTGAGATGGCGTGTGGCGATCATCTGGTTGTGAAC
AATAGCCGCTCTTCTCGTACAGCACGTGCACTGCGTCACCACAAATATCGTAAAACCTGTAAACGTTGCCGTGTG
TCCGATGAGGATCTGAACAAATTCCTGACAAAAGCCAATGAGGACCAAACAAGCGTGAAAGTGAAAGTCGTTAGC
GCTCCTACCCGTACTAAAAAAGCAATGCCGAAATCCGTTGCTCGTGCCCCTAAACCACTGGAAAACACTGAAGCA
GCACAGGCACAGCCGTCTGGAAGCAAATTCTCTCCGGCCATTCCTGTTTCTACCCAGGAGTCCGTTTCTGTTCCA
GCAAGTGTGAGCACCAGCATTAGCAGTATTAGCACCGGTGCCACCGCTAGCGCCCTGGTTAAAGGCAATACCAAT
CCGATTACAAGCATGTCTGCCCCGGTTCAAGCATCAGCTCCAGCACTGACAAAATCCCAAACCGATCGTCTGGAG
GTTCTGCTGAATCCGAAAGACGAAATCAGCCTGAATTCCGGCAAACCGTTTCGTGAACTGGAGAGCGAACTGCTG
TCACGTCGTAAAAAAGACCTGCAACAAATCTATGCCGAAGAACGTGAGAACTATCTGGGGAAACTGGAACGTGAA
ATCACCCGCTTTTTCGTGGATCGTGGCTTTCTGGAGATCAAATCCCCGATTCTGATTCCTCTGGAGTATATCGAG
CGTATGGGCATCGACAATGATACCGAACTGAGCAAACAAATTTTCCGTGTGGATAAAAACTTCTGTCTGCGCCCT
ATGCTAGCACCAAATCTGGCTAACTATCTGCGCAAACTGGACCGTGCCCTGCCTGATCCTATCAAAATCTTCGAG
ATCGGCCCGTGTTATCGTAAAGAGTCCGACGGTAAAGAACATCTGGAGGAGTTTACCATGCTGAACTTTTGCCAA
ATGGGTTCAGGTTGTACTCGTGAGAACCTGGAAAGCATCATCACCGATTTTCTGAACCACCTGGGCATTGACTTC
AAAATTGTGGGCGACAGCTGTATGGTGTTTGGCGACACCCTGGATGTCATGCACGGCGACCTGGAACTGTCTAGT
GCCGTTGTGGGCCCAATCCCGCTGGATCGTGAGTGGGGTATCGACAAACCTTGGATCGGTGCGGGTTTTGGTCTG
GAGCGTCTGCTGAAAGTAAAACACGACTTCAAGAACATCAAACGTGCTGCACGTTCCGAGTCCTATTACAATGGT
ATTTCTACTAACCTGTAA (SEQ ID NO:165)
Protein:
MCRAISLRRLLLLLLQLSQLLAVTQGMLEMWHEGLEEASRLYFGERNVKGMFEVLEPLHAMMERGPQTLKETSFN
QAYGRDLMEAQEWCRKYMKSGNVKDLLQAWDLYYHVFRRISKTRTGKLFWALVVVAGVLFCYGLLVTVALCVIWV
RSGMASNDYTQQATQSYGAYPTQPGQGYSQQSSQPYGQQSYSGYSQSTDTSGYGQSSYSSYGQSQNTGYGTQSTP
QGYGSTGGYGSSQSSQSSYGQQSSYPGYGQQPAPSSTSGSYGSSSQSSSYGQPQSGSYSQQPSYGGQQQSYGQQQ
SYNPPQGYGQQNQYNSSSGGGGGGGGGGNYGQDQSSMSSGGGSGGGYGNQDQSGGGGSGGYGQQDRGGRGRGGSG
GGGGGGGGYNRSSGGYEPRGRGGGRGGRGGMGGSDRGGFNKFGGPRDQGSRHDSEQDNSDNNTIFVQGLGENVT
IESVADYFKQIGIIKTNKKTGQPMINLYTDRETGKLKGEATVSFDDPPSAKAAIDWFDGKEFSGNPIKVSFATRR
ADFNRGGGNGRGGRGRGGPMGRGGYGGGGSGGGGRGGFPSGGGGGGQQRAGDWKCPNPTCENMNFSWRNECNQC
KAPKPDGPGGGPGGSHMGGNYGDDRRGGRGGDYKDDDDKGTGAPGSAGSAAGSGASNFTQFVLVDNGGTGDVTVA
PSNFANGIAEWISSNSRSQAYKVTCSVRQSSAQNRKYTIKVEVPKGAWRSYLNMELTIPIFATNSDCELIVKAMQ
GLLKDGNPIPSAIAANSGIYGTGAPGSAGSAAGSGACPVPLQLPPLERLTLDDKKPLNTLISATGLWMSRTGTIH
KIKHHEVSRSKIYIEMACGDHLVVNNSRSSRTARALRHHKYRKTCKRCRVSDEDLNKFLTKANEDQTSVKVKVVS
APTRTKKAMPKSVARAPKPLENTEAAQAQPSGSKFSPAIPVSTQESVSVPASVSTSISSISTGATASALVKGNTN

PITSMSAPVQASAPALTKSQTDRLEVLLNPKDEISLNSGKPFRELESELLSRRKKDLQQIYAEERENYLGKLERE
ITRFFVDRGFLEIKSPILIPLEYIERMGIDNDTELSKQIFRVDKNFCLRPMLAPNLANYLRKLDRALPDPIKIFE
IGPCYRKESDGKEHLEEFTMLNFCQMGSGCTRENLESIITDFLNHLGIDFKIVGDSCMVFGDTLDVMHGDLELSS
AVVGPIPLDREWGIDKPWIGAGFGLERLLKVKHDFKNIKRAARSESYYNGISTNL  (SEQ ID NO:166)

FUS-MCP-PylRS^AF
DNA:
ATGGCCTCAAACGATTATACCCAACAAGCAACCCAAAGCTATGGGGCCTACCCCACCCAGCCCGGGCAGGGCTAT
TCCCAGCAGAGCAGTCAGCCCTACGGACAGCAGAGTTACAGTGGTTATAGCCAGTCCACGGACACTTCAGGATAT
GGCCAGAGCAGCTATTCTTCTTATGGCCAGAGCCAGAACACAGGCTATGGAACTCAGTCAACTCCCCAGGGATAT
GGCTCGACTGGCGGCTATGGCAGTAGCCAGAGCTCCCAATCGTCTTACGGGCAGCAGTCCTCCTACCCTGGCTAT
GGCCAGCAGCCAGCTCCCAGCAGCACCTCGGGAAGTTACGGTAGCAGTTCTCAGAGCAGCAGCTATGGGCAGCCC
CAGAGTGGGAGCTACAGCCAGCAGCCTAGCTATGGTGGACAGCAGCAAAGCTATGGACAGCAGCAAAGCTATAAT
CCCCCTCAGGGCTATGGACAGCAGAACCAGTACAACAGCAGCAGTGGTGGTGGAGGTGGAGGTGGAGGTGGAGGT
AACTATGGCCAAGATCAATCCTCCATGAGTAGTGGTGGTGGCAGTGGTGGCGGTTATGGCAATCAAGACCAGAGT
GGTGGAGGTGGCAGCGGTGGCTATGGACAGCAGGACCGTGGAGGCCGCGGCAGGGGTGGCAGTGGTGGCGGCGGC
GGCGGCGGCGGTGGTGGTTACAACCGCAGCAGTGGTGGCTATGAACCCAGAGGTCGTGGAGGTGGCCGTGGAGGC
AGAGGTGGCATGGGCGGAAGTGACCGTGGTGGCTTCAATAAATTTGGTGGCCCTCGGGACCAAGGATCACGTCAT
GACTCCGAACAGGATAATTCAGACAACAACACCATCTTTGTGCAAGGCCTGGGTGAGAATGTTACAATTGAGTCT
GTGGCTGATTACTTCAAGCAGATTGGTATTATTAAGACAAACAAGAAAACGGGACAGCCCATGATTAATTTGTAC
ACAGACAGGGAAACTGGCAAGCTGAAGGGAGAGGCAACGGTCTCTTTTGATGACCCACCTTCAGCTAAAGCAGCT
ATTGACTGGTTTGATGGTAAAGAATTCTCCGGAAATCCTATCAAGGTCTCATTTGCTACTCGCCGGGCAGACTTT
AATCGGGGTGGTGGCAATGGTCGTGGAGGCCGAGGGCGAGGAGGACCCATGGGCCGTGGAGGCTATGGAGGTGGT
GGCAGTGGTGGTGGTGGCCGAGGAGGATTTCCCAGTGGAGGTGGTGGCGGTGGAGGACAGCAGCGAGCTGGTGAC
TGGAAGTGTCCTAATCCCACCTGTGAGAATATGAACTTCTCTTGGAGGAATGAATGCAACCAGTGTAAGGCCCCT
AAACCAGATGGCCCAGGAGGGGGACCAGGTGGCTCTCACATGGGGGGTAACTACGGGGATGATCGTCGTGGTGGC
AGAGGAGGCGATTACAAGGATGACGACGATAAGGGTACCGGCGCCCCCGGCTCCGCCGGCTCCGCCGCCGGCTCC
GGCGCTTCTAACTTTACTCAGTTCGTTCTCGTCGACAATGGCGGAACTGGCGACGTGACTGTCGCCCCAAGCAAC
TTCGCTAACGGGATCGCTGAATGGATCAGCTCTAACTCGCGTTCACAGGCTTACAAAGTAACCTGTAGCGTTCGT
CAGAGCTCTGCGCAGAATCGCAAATACACCATCAAAGTCGAGGTGCCTAAAGGCGCCTGGCGTTCGTACTTAAAT
ATGGAACTAACCATTCCAATTTTCGCCACGAATTCCGACTGCGAGCTTATTGTTAAGGCAATGCAAGGTCTCCTA
AAAGATGGAAACCCGATTCCCTCAGCAATCGCAGCAAACTCCGGCATCTACGGTACCGGCGCCCCCGGCTCCGCC
GGCTCCGCCGCCGGCTCCGGCGCGTGCCCGGTGCCGCTGCAGCTGCCGCCGCTGGAACGCCTGACCCTGGATGAT
AAAAAACCGCTGAATACCCTGATCTCTGCTACTGGTCTGTGGATGAGTCGTACCGGAACCATTCATAAAATCAAA
CACCACGAGGTTAGCCGTTCGAAAATCTATATTGAGATGGCGTGTGGCGATCATCTGGTTGTGAACAATAGCCGC
TCTTCTCGTACAGCACGTGCACTGCGTCACCACAAATATCGTAAAACCTGTAAACGTTGCCGTGTGTCCGATGAG
GATCTGAACAAATTCCTGACAAAAGCCAATGAGGACCAAACAAGCGTGAAAGTGAAAGTCGTTAGCGCTCCTACC
CGTACTAAAAAAGCAATGCCGAAATCCGTTGCTCGTGCCCCTAAACCACTGGAAAACACTGAAGCAGCACAGGCA
CAGCCGTCTGGAAGCAAATTCTCTCCGGCCATTCCTGTTTCTACCCAGGAGTCCGTTTCTGTTCCAGCAAGTGTG
AGCACCAGCATTAGCAGTATTAGCACCGGTGCCACCGCTAGCGCCCTGGTTAAAGGCAATACCAATCCGATTACA
AGCATGTCTGCCCCGGTTCAAGCATCAGCTCCAGCACTGACAAAATCCCAAACCGATCGTCTGGAGGTTCTGCTG
AATCCGAAAGACGAAATCAGCCTGAATTCCGGCAAACCGTTTCGTGAACTGGAGAGCGAACTGCTGTCACGTCGT
AAAAAAGACCTGCAACAAATCTATGCCGAAGAACGTGAGAACTATCTGGGGAAACTGGAACGTGAAATCACCCGC
TTTTTCGTGGATCGTGGCTTTCTGGAGATCAAATCCCCGATTCTGATTCCTCTGGAGTATATCGAGCGTATGGGC
ATCGACAATGATACCGAACTGAGCAAACAAATTTTCCGTGTGGATAAAAACTTCTGTCTGCGCCCTATGCTAGCA
CCAAATCTGGCTAACTATCTGCGCAAACTGGACCGTGCCCTGCCTGATCCTATCAAAATCTTCGAGATCGGCCCG
TGTTATCGTAAAGAGTCCGACGGTAAAGAACATCTGGAGGAGTTTACCATGCTGAACTTTTGCCAAATGGGTTCA
GGTTGTACTCGTGAGAACCTGGAAAGCATCATCACCGATTTTCTGAACCACCTGGGCATTGACTTCAAAATTGTG
GGCGACAGCTGTATGGTGTTTGGCGACACCCTGGATGTCATGCACGGCGACCTGGAACTGTCTAGTGCCGTTGTG
GGCCCAATCCCGCTGGATCGTGAGTGGGGTATCGACAAACCTTGGATCGGTGCGGGTTTTGGTCTGGAGCGTCTG
CTGAAAGTAAAACACGACTTCAAGAACATCAAACGTGCTGCACGTTCCGAGTCCTATTACAATGGTATTTCTACT
AACCTGTAA  (SEQ ID NO:167)
Protein:
MASNDYTQQATQSYGAYPTQPGQGYSQQSSQPYGQQSYSGYSQSTDTSGYGQSSYSSYGQSQNTGYGTQSTPQGY
GSTGGYGSSQSSQSSYGQQSSYPGYGQQPAPSSTSGSYGSSSQSSSYGQPQSGSYSQQPSYGGQQQSYGQQQSYN
PPQGYGQQNQYNSSSGGGGGGGGGNYGQDQSSMSSGGGSGGGYGNQDQSGGGGSGGYGQQDRGGRGRGGSGGGG
GGGGGGYNRSSGGYEPRGRGGGRGGRGGMGGSDRGGFNKFGGPRDQGSRHDSEQDNSDNNTIFVQGLGENVTIES
VADYFKQIGIIKTNKKTGQPMINLYTDRETGKLKGEATVSFDDPPSAKAAIDWFDGKEFSGNPIKVSFATRRADF

NRGGGNGRGGRGRGGPMGRGGYGGGGSGGGGRGGFPSGGGGGGQQRAGDWKCPNPTCENMNFSWRNECNQCKAP
KPDGPGGGPGGSHMGGNYGDDRRGGRGGDYKDDDDKGTGAPGSAGSAAGSGASNFTQFVLVDNGGTGDVTVAPSN
FANGIAEWISSNSRSQAYKVTCSVRQSSAQNRKYTIKVEVPKGAWRSYLNMELTIPIFATNSDCELIVKAMQGLL
KDGNPIPSAIAANSGIYGTGAPGSAGSAAGSGACPVPLQLPPLERLTLDDKKPLNTLISATGLWMSRTGTIHKIK
HHEVSRSKIYIEMACGDHLVVNNSRSSRTARALRHHKYRKTCKRCRVSDEDLNKFLTKANEDQTSVKVKVVSAPT
RTKKAMPKSVARAPKPLENTEAAQAQPSGSKFSPAIPVSTQESVSVPASVSTSISSISTGATASALVKGNTNPIT
SMSAPVQASAPALTKSQTDRLEVLLNPKDEISLNSGKPFRELESELLSRRKKDLQQIYAEERENYLGKLEREITR
FFVDRGFLEIKSPILIPLEYIERMGIDNDTELSKQIFRVDKNFCLRPMLAPNLANYLRKLDRALPDPIKIFEIGP
CYRKESDGKEHLEEFTMLNFCQMGSGCTRENLESIITDFLNHLGIDFKIVGDSCMVFGDTLDVMHGDLELSSAVV
GPIPLDREWGIDKPWIGAGFGLERLLKVKHDFKNIKRAARSESYYNGISTNL  (SEQ ID NO:168)

SPD5-MCP-PylRS^AF
DNA:
ATGGAGGACAACAGCGTGCTGAACGAGGACAGCAACCTGGAGCACGTGGAGGGCCAGCCCAGAAGAAGCATGAGC
CAGCCCGTGCTGAACGTGGAGGGCGACAAGAGAACCAGCAGCACCAGCGCCACCCAGCAGCAGGTGCTGAGCGGC
GCCTTCAGCAGCGCCGACGTGAGAAGCATCCCCATCATCCAGACCTGGGAGGAGAACAAGGCCCTGAAGACCAAG
ATCACCATCCTGAGAGGCGAGCTGCAGATGTACCAGAGAAGATACAGCGAGGCCAAGGAGGCCAGCCAGAAGAGA
GTGAAGGAGGTGATGGACGACTACGTGGACCTGAAGCTGGGCCAGGAGAACGTGCAGGAGAAGATGGAGCAGTAC
AAGCTGATGGAGGAGGACCTGCTGGCCATGCAGAGCAGAATCGAGACCAGCGAGGACAACTTCGCCAGACAGATG
AAGGAGTTCGAGGCCCAGAAGCACGCCATGGAGGAGAGAATCAAGGAGCTGGAGCTGAGCGCCACCGACGCCAAC
AACACCACCGTGGGCAGCTTCAGAGGCACCCTGGACGACATCCTGAAGAAGAACGACCCCGACTTCACCCTGACC
AGCGGCTACGAGGAGAGAAAGATCAACGACCTGGAGGCCAAGCTGCTGAGCGAGATCGACAAGGTGGCCGAGCTG
GAGGACCACATCCAGCAGCTGAGACAGGAGCTGGACGACCAGAGCGCCAGACTGGCCGACAGCGAGAACGTGAGA
GCCCAGCTGGAGGCCGCCACCGGCCAGGGCATCCTGGGCGCCGCCGGCAACGCCATGGTGCCCAACAGCACCTTC
ATGATCGGCAACGGCAGAGAGAGCCAGACCAGAGACCAGCTGAACTACATCGACGACCTGGAGACCAAGCTGGCC
GACGCCAAGAAGGAGAACGACAAGGCCAGACAGGCCCTGGTGGAGTACATGAACAAGTGCAGCAAGCTGGAGCAC
GAGATCAGAACCATGGTGAAGAACAGCACCTTCGACAGCAGCAGCATGCTGCTGGGCGGCCAGACCAGCGACGAG
CTGAAGATCCAGATCGGCAAGGTGAACGGCGAGCTGAACGTGCTGAGAGCCGAGAACAGAGAGCTGAGAATCAGA
TGCGACCAGCTGACCGGCGGCGACGGCAACCTGAGCATCAGCCTGGGCCAGAGCAGACTGATGGCCGGCATCGCC
ACCAACGACGTGGACAGCATCGGCCAGGGCAACGAGACCGGCGGCACCAGCATGAGAATCCTGCCCAGAGAGAGC
CAGCTGGACGACCTGGAGGAGAGCAAGCTGCCCCTGATGGACACCAGCAGCGCCGTGAGAAACCAGCAGCAGTTC
GCCAGCATGTGGGAGGACTTCGAGAGCGTGAAGGACAGCCTGCAGAACAACCACAACGACACCCTGGAGGGCAGC
TTCAACAGCAGCATGCCCCCCCCCGGCAGAGACGCCACCCAGAGCTTCCTGAGCCAGAAGAGCTTCAAGAACAGC
CCCATCGTGATGCAGAAGCCCAAGAGCCTGCACCTGCACCTGAAGAGCCACCAGAGCGAGGGCGCCGGCGAGCAG
ATCCAGAACAACAGCTTCAGCACCAAGACCGCCAGCCCCCACGTGAGCCAGAGCCACATCCCCATCCTGCACGAC
ATGCAGCAGATCCTGGACAGCAGCGCCATGTTCCTGGAGGGCCAGCACGACGTGGCCGTGAACGTGGAGCAGATG
CAGGAGAAGATGAGCCAGATCAGAGAGGCCCTGGCCAGACTGTTCGAGAGACTGAAGAGCAGCGCCGCCCTGTTC
GAGGAGATCCTGGAGAGAATGGGCAGCAGCGACCCCAACGCCGACAAGATCAAGAAGATGAAGCTGGCCTTCGAG
ACCAGCATCAACGACAAGCTGAACGTGAGCGCCATCCTGGAGGCCGCCGAGAAGGACCTGCACAACATGAGCCTG
AACTTCAGCATCCTGGAGAAGAGCATCGTGAGCCAGGCCGCCGAGGCCAGCAGAAGATTCACCATCGCCCCCGAC
GCCGAGGACGTGGCCAGCAGCAGCCTGCTGAACGCCAGCTACAGCCCCCTGTTCAAGTTCACCAGCAACAGCGAC
ATCGTGGAGAAGCTGCAGAACGAGGTGAGCGAGCTGAAGAACGAGCTGGAGATGGCCAGAACCAGAGACATGAGA
AGCCCCCTGAACGGCAGCAGCGGCAGACTGAGCGACGTGCAGATCAACACCAACAGAATGTTCGAGGACCTGGAG
GTGAGCGAGGCCACCCTGCAGAAGGCCAAGGAGGAGAACAGCACCCTGAAGAGCCAGTTCGCCGAGCTGGAGGCC
AACCTGCACCAGGTGAACAGCAAGCTGGGCGAGGTGAGATGCGAGCTGAACGAGGCCCTGGCCAGAGTGGACGGC
GAGCAGGAGACCAGAGTGAAGGCCGAGAACGCCCTGGAGGAGGCCAGACAGCTGATCAGCAGCCTGAAGCACGAG
GAGAACGAGCTGAAGAAGACCATCACCGACATGGGCATGAGACTGAACGAGGCCAAGAAGAGCGACGAGTTCCTG
AAGAGCGAGCTGAGCACCGCCCTGGAGGAGGAGAAGAAGAGCCAGAACCTGGCCGACGAGCTGAGCGAGGAGCTG
AACGGCTGGAGAATGAGAACCAAGGAGGCCGAGAACAAGGTGGAGCACGCCAGCAGCGAGAAGAGCGAGATGCTG
GAGAGAATCGTGCACCTGGAGACCGAGATGGAGAAGCTGAGCACCAGCGAGATCGCCGCCGACTACTGCAGCACC
AAGATGACCGAGAGAAAGAAGGAGATCGAGCTGGCCAAGTACAGAGAGGACTTCGAGAACGCCGCCATCGTGGGC
CTGGAGAGAATCAGCAAGGAGATCAGCGAGCTGACCAAGAAGACCCTGAAGGCCAAGATCATCCCCAGCAACATC
AGCAGCATCCAGCTGGTGTGCGACGAGCTGTGCAGAAGACTGAGCAGAGAGAGAGAGCAGCAGCACGAGTACGCC
AAGGTGATGAGAGACGTGAACGAGAAGATCGAGAAGCTGCAGCTGGAGAAGGACGCCCTGGAGCACGAGCTGAAG
ATGATGAGCAGCAACAACGAGAACGTGCCCCCCGTGGGCACCAGCGTGAGCGGCATGCCCACCAAGACCAGCAAC
CAGAAGTGCGCCCAGCCCCACTACACCAGCCCCACCAGACAGCTGCTGCACGAGAGCACCATGGCCGTGGACGCC
ATCGTGCAGAAGCTGAAGAAGACCCACAACATGAGCGGCATGGGCCCCGAGCTGAAGGAGACCATCGGCAACGTG
ATCAACGAGAGCAGAGTGCTGAGAGACTTCCTGCACCAGAAGCTGATCCTGTTCAAGGGCATCGACATGAGCAAC

TGGAAGAACGAGACCGTGGACCAGCTGATCACCGACCTGGGCCAGCTGCACCAGGACAACCTGATGCTGGAGGAG
CAGATCAAGAAGTACAAGAAGGAGCTGAAGCTGACCAAGAGCGCCATCCCCACCCTGGGCGTGGAGTTCCAGGAC
AGAATCAAGACCGAGATCGGCAAGATCGCCACCGACATGGGCGGCGCCGTGAAGGAGATCAGAAAGAAGGGTACC
GAGCAGAAGCTGATCTCAGAGGAGGACCTGGGCGCCCCCGGCTCCGCCGGCTCCGCCGCCGGCTCCGGCGCTTCT
AACTTTACTCAGTTCGTTCTCGTCGACAATGGCGGAACTGGCGACGTGACTGTCGCCCCAAGCAACTTCGCTAAC
GGGATCGCTGAATGGATCAGCTCTAACTCGCGTTCACAGGCTTACAAAGTAACCTGTAGCGTTCGTCAGAGCTCT
GCGCAGAATCGCAAATACACCATCAAAGTCGAGGTGCCTAAAGGCGCCTGGCGTTCGTACTTAAATATGGAACTA
ACCATTCCAATTTTCGCCACGAATTCCGACTGCGAGCTTATTGTTAAGGCAATGCAAGGTCTCCTAAAAGATGGA
AACCCGATTCCCTCAGCAATCGCAGCAAACTCCGGCATCTACGGTACCGGCGCCCCCGGCTCCGCCGGCTCCGCC
GCCGGCTCCGGCGCGTGCCCGGTGCCGCTGCAGCTGCCGCCGCTGGAACGCCTGACCCTGGATGATAAAAAACCG
CTGAATACCCTGATCTCTGCTACTGGTCTGTGGATGAGTCGTACCGGAACCATTCATAAAATCAAACACCACGAG
GTTAGCCGTTCGAAAATCTATATTGAGATGGCGTGTGGCGATCATCTGGTTGTGAACAATAGCCGCTCTTCTCGT
ACAGCACGTGCACTGCGTCACCACAAATATCGTAAAACCTGTAAACGTTGCCGTGTGTCCGATGAGGATCTGAAC
AAATTCCTGACAAAAGCCAATGAGGACCAAACAAGCGTGAAAGTGAAAGTCGTTAGCGCTCCTACCCGTACTAAA
AAAGCAATGCCGAAATCCGTTGCTCGTGCCCCTAAACCACTGGAAAACACTGAAGCAGCACAGGCACAGCCGTCT
GGAAGCAAATTCTCTCCGGCCATTCCTGTTTCTACCCAGGAGTCCGTTTCTGTTCCAGCAAGTGTGAGCACCAGC
ATTAGCAGTATTAGCACCGGTGCCACCGCTAGCGCCCTGGTTAAAGGCAATACCAATCCGATTACAAGCATGTCT
GCCCCGGTTCAAGCATCAGCTCCAGCACTGACAAAATCCCAAACCGATCGTCTGGAGGTTCTGCTGAATCCGAAA
GACGAAATCAGCCTGAATTCCGGCAAACCGTTTCGTGAACTGGAGAGCGAACTGCTGTCACGTCGTAAAAAAGAC
CTGCAACAAATCTATGCCGAAGAACGTGAGAACTATCTGGGGAAACTGGAACGTGAAATCACCCGCTTTTTCGTG
GATCGTGGCTTTCTGGAGATCAAATCCCCGATTCTGATTCCTCTGGAGTATATCGAGCGTATGGGCATCGACAAT
GATACCGAACTGAGCAAACAAATTTTCCGTGTGGATAAAAACTTCTGTCTGCGCCCTATGCTAGCACCAAATCTG
GCTAACTATCTGCGCAAACTGGACCGTGCCCTGCCTGATCCTATCAAAATCTTCGAGATCGGCCCGTGTTATCGT
AAAGAGTCCGACGGTAAAGAACATCTGGAGGAGTTTACCATGCTGAACTTTTGCCAAATGGGTTCAGGTTGTACT
CGTGAGAACCTGGAAAGCATCATCACCGATTTTCTGAACCACCTGGGCATTGACTTCAAAATTGTGGGCGACAGC
TGTATGGTGTTTGGCGACACCCTGGATGTCATGCACGGCGACCTGGAACTGTCTAGTGCCGTTGTGGGCCCAATC
CCGCTGGATCGTGAGTGGGGTATCGACAAACCTTGGATCGGTGCGGGTTTTGGTCTGGAGCGTCTGCTGAAAGTA
AAACACGACTTCAAGAACATCAAACGTGCTGCACGTTCCGAGTCCTATTACAATGGTATTTCTACTAACCTGTAA
(SEQ ID NO:169)
Protein:
MEDNSVLNEDSNLEHVEGQPRRSMSQPVLNVEGDKRTSSTSATQQQVLSGAFSSADVRSIPIIQTWEENKALKTK
ITILRGELQMYQRRYSEAKEASQKRVKEVMDDYVDLKLGQENVQEKMEQYKLMEEDLLAMQSRIETSEDNFARQM
KEFEAQKHAMEERIKELELSATDANNTTVGSFRGTLDDILKKNDPDFTLTSGYEERKINDLEAKLLSEIDKVAEL
EDHIQQLRQELDDQSARLADSENVRAQLEAATGQGILGAAGNAMVPNSTFMIGNGRESQTRDQLNYIDDLETKLA
DAKKENDKARQALVEYMNKCSKLEHEIRTMVKNSTFDSSSMLLGGQTSDELKIQIGKVNGELNVLRAENRELRIR
CDQLTGGDGNLSISLGQSRLMAGIATNDVDSIGQGNETGGTSMRILPRESQLDDLEESKLPLMDTSSAVRNQQQF
ASMWEDFESVKDSLQNNHNDTLEGSFNSSMPPPGRDATQSFLSQKSFKNSPIVMQKPKSLHLHLKSHQSEGAGEQ
IQNNSFSTKTASPHVSQSHIPILHDMQQILDSSAMFLEGQHDVAVNVEQMQEKMSQIREALARLFERLKSSAALF
EEILERMGSSDPNADKIKKMKLAFETSINDKLNVSAILEAAEKDLHNMSLNFSILEKSIVSQAAEASRRFTIAPD
AEDVASSSLLNASYSPLFKFTSNSDIVEKLQNEVSELKNELEMARTRDMRSPLNGSSGRLSDVQINTNRMFEDLE
VSEATLQKAKEENSTLKSQFAELEANLHQVNSKLGEVRCELNEALARVDGEQETRVKAENALEEARQLISSLKHE
ENELKKTITDMGMRLNEAKKSDEFLKSELSTALEEEKKSQNLADELSEELNGWRMRTKEAENKVEHASSEKSEML
ERIVHLETEMEKLSTSEIAADYCSTKMTERKKEIELAKYREDFENAAIVGLERISKEISELTKKTLKAKIIPSNI
SSIQLVCDELCRRLSREREQQHEYAKVMRDVNEKIEKLQLEKDALEHELKMMSSNNENVPPVGTSVSGMPTKTSN
QKCAQPHYTSPTRQLLHESTMAVDAIVQKLKKTHNMSGMGPELKETIGNVINESRVLRDFLHQKLILFKGIDMSN
WKNETVDQLITDLGQLHQDNLMLEEQIKKYKKELKLTKSAIPTLGVEFQDRIKTEIGKIATDMGGAVKEIRKKGT
EQKLISEEDLGAPGSAGSAAGSGASNFTQFVLVDNGGTGDVTVAPSNFANGIAEWISSNSRSQAYKVTCSVRQSS
AQNRKYTIKVEVPKGAWRSYLNMELTIPIFATNSDCELIVKAMQGLLKDGNPIPSAIAANSGIYGTGAPGSAGSA
AGSGACPVPLQLPPLERLTLDDKKPLNTLISATGLWMSRTGTIHKIKHHEVSRSKIYIEMACGDHLVVNNSRSSR
TARALRHHKYRKTCKRCRVSDEDLNKFLTKANEDQTSVKVKVVSAPTRTKKAMPKSVARAPKPLENTEAAQAQPS
GSKFSPAIPVSTQESVSVPASVSTSISSISTGATASALVKGNTNPITSMSAPVQASAPALTKSQTDRLEVLLNPK
DEISLNSGKPFRELESELLSRRKKDLQQIYAEERENYLGKLEREITRFFVDRGFLEIKSPILIPLEYIERMGIDN
DTELSKQIFRVDKNFCLRPMLAPNLANYLRKLDRALPDPIKIFEIGPCYRKESDGKEHLEEFTMLNFCQMGSGCT
RENLESIITDFLNHLGIDFKIVGDSCMVFGDTLDVMHGDLELSSAVVGPIPLDREWGIDKPWIGAGFGLERLLKV
KHDFKNIKRAARSESYYNGISTNL  (SEQ ID NO:170)

9. POIs (reporters) and controls

GFP^{39TAG} (GFP with Amber-encoded amino acid position 39)
DNA: (Amber codon underlined)
ATGGGCCGCCTGGAAAGCACCCCGCCGAAAAAAAAACGCAAAGTGGAAGATAGCGCGAGCGATTACAAAGATGAT
GATGATAAAGTGAGCAAGGGCGAGGAGCTGTTCACCGGGGTGGTGCCCATCCTGGTCGAGCTGGACGGCGACGTA
AACGGCCACAAGTTCAGCGTGTCCGGCGAGGGCGAGGGCGATGCCACCTAGGGCAAGCTGACCCTGAAGTTCATC
TGCACCACCGGCAAGCTGCCCGTGCCCTGGCCCACCCTCGTGACCACCCTGACCTACGGCGTGCAGTGCTTCAGC
CGCTACCCCGACCACATGAAGCAGCACGACTTCTTCAAGTCCGCCATGCCCGAAGGCTACGTCCAGGAGCGCACC
ATCTTCTTCAAGGACGACGGCAACTACAAGACCCGCGCCGAGGTGAAGTTCGAGGGCGACACCCTGGTGAACCGC
ATCGAGCTGAAGGGCATCGACTTCAAGGAGGACGGCAACATCCTGGGGCACAAGCTGGAGTACAACTACAACAGC
CACAACGTCTATATCATGGCCGACAAGCAGAAGAACGGCATCAAGGCCAACTTCAAGATCCGCCACAACATCGAG
GACGGCAGCGTGCAGCTCGCCGACCACTACCAGCAGAACACCCCCATCGGCGACGGCCCCGTGCTGCTGCCCGAC
AACCACTACCTGAGCACCCAGTCCGCCCTGAGCAAAGACCCCAACGAGAAGCGCGATCACATGGTCCTGCTGGAG
TTCGTGACCGCCGCCGGGATCACTCTCGGCATGGACGAGCTGTACAAGCATCACCATCACCATCACTAA (SEQ
ID NO:171)
Protein: (X indicates non-canonical amino acid)
MGRLESTPPKKKRKVEDSASDYKDDDDKVSKGEELFTGVVPILVELDGDVNGHKFSVSGEGEGDATXGKLTLKFI
CTTGKLPVPWPTLVTTLTYGVQCFSRYPDHMKQHDFFKSAMPEGYVQERTIFFKDDGNYKTRAEVKFEGDTLVNR
IELKGIDFKEDGNILGHKLEYNYNSHNVYIMADKQKNGIKANFKIRHNIEDGSVQLADHYQQNTPIGDGPVLLPD
NHYLSTQSALSKDPNEKRDHMVLLEFVTAAGITLGMDELYKHHHHHH (SEQ ID NO:172)

GFP^{39TAG}-2xMS2 (GFP^{39TAG} with 2 MS2 stem-loops)
DNA: (MS2 stem-loops and Amber codon underlined)
ATGGGCCGCCTGGAAAGCACCCCGCCGAAAAAAAAACGCAAAGTGGAAGATAGCGCGAGCGATTACAAAGATGAT
GATGATAAAGTGAGCAAGGGCGAGGAGCTGTTCACCGGGGTGGTGCCCATCCTGGTCGAGCTGGACGGCGACGTA
AACGGCCACAAGTTCAGCGTGTCCGGCGAGGGCGAGGGCGATGCCACCTAGGGCAAGCTGACCCTGAAGTTCATC
TGCACCACCGGCAAGCTGCCCGTGCCCTGGCCCACCCTCGTGACCACCCTGACCTACGGCGTGCAGTGCTTCAGC
CGCTACCCCGACCACATGAAGCAGCACGACTTCTTCAAGTCCGCCATGCCCGAAGGCTACGTCCAGGAGCGCACC
ATCTTCTTCAAGGACGACGGCAACTACAAGACCCGCGCCGAGGTGAAGTTCGAGGGCGACACCCTGGTGAACCGC
ATCGAGCTGAAGGGCATCGACTTCAAGGAGGACGGCAACATCCTGGGGCACAAGCTGGAGTACAACTACAACAGC
CACAACGTCTATATCATGGCCGACAAGCAGAAGAACGGCATCAAGGCCAACTTCAAGATCCGCCACAACATCGAG
GACGGCAGCGTGCAGCTCGCCGACCACTACCAGCAGAACACCCCCATCGGCGACGGCCCCGTGCTGCTGCCCGAC
AACCACTACCTGAGCACCCAGTCCGCCCTGAGCAAAGACCCCAACGAGAAGCGCGATCACATGGTCCTGCTGGAG
TTCGTGACCGCCGCCGGGATCACTCTCGGCATGGACGAGCTGTACAAGCATCACCATCACCATCACTAAGGATCC
TAAGGTACCTAATTGCCTAGAAAACATGAGGATCACCCATGTCTGCAGGTCGACTCTAGAAAACATGAGGATCAC
CCATGT (SEQ ID NO:173)

Protein: (X indicates non-canonical amino acid)
MGRLESTPPKKKRKVEDSASDYKDDDDKVSKGEELFTGVVPILVELDGDVNGHKFSVSGEGEGDATXGKLTLKFI
CTTGKLPVPWPTLVTTLTYGVQCFSRYPDHMKQHDFFKSAMPEGYVQERTIFFKDDGNYKTRAEVKFEGDTLVNR
IELKGIDFKEDGNILGHKLEYNYNSHNVYIMADKQKNGIKANFKIRHNIEDGSVQLADHYQQNTPIGDGPVLLPD
NHYLSTQSALSKDPNEKRDHMVLLEFVTAAGITLGMDELYKHHHHHH (SEQ ID NO:174)

mOrange
DNA:
ATGGTGAGCAAGGGCGAGGAGAATAATATGGCCATCATCAAGGAGTTCATGCGCTTCAAGGTGCGCATGGAGGGC
ACCGTGAACGGCCACGAGTTCGAGATCGAGGGCGAGGGCGAGGGCCGCCCCTACGAGGGCTTTCAGACCGCTAAG
CTGAAGGTGACCAAGGGCGGCCCCCTGCCCTTCGCCTGGGACATCCTGTCCCCTCTCTTCACCTACGGCTCCAAG
GCCTACGTGAAGCACCCCGCCGACATCCCCGACTACTTCAAGCTGTCCTTCCCCGAGGGCTTCAAGTGGGAGCGC
GTGATGAACTACGAGGACGGCGGCGTGGTGACCGTGACCCAGGACTCCTCACTGCAGGACGGCGAGTTCATCTAC
AAGGTGAAGATGCGCGGCACCAACTTCCCCTCCGACGGCCCCGTGATGCAGAAGAAGACCATGGGCTGGGAGGCC
TCCTCCGAGCGGATGTACCCCGAGGACGGCGCCCTGAAGGGCGAGATCAGGATGAGGCTGAAGCTGAAGGACGGC
GGCCACTACACCTCCGAGGTCAAGACCACCTACAAGGCCAAGAAGTCCGTGCAGCTGCCCGGCGCCTACATCGTC
GGCATCAAGCTGGACATCACCTCCCACAACGAGGACTACACCATCGTGGAACAGTACGAACGCGCCGAGGGCCGC
CACTCCACCGGCGGCATGGACGAGCTGTACAAGTAA (SEQ ID NO:175)
Protein:
MVSKGEENNMAIIKEFMRFKVRMEGTVNGHEFEIEGEGEGRPYEGFQTAKLKVTKGGPLPFAWDILSPLFTYGSK
AYVKHPADIPDYFKLSFPEGFKWERVMNYEDGGVVTVTQDSSLQDGEFIYKVKMRGTNFPSDGPVMQKKTMGWEA

SSERMYPEDGALKGEIRMRLKLKDGGHYTSEVKTTYKAKKSVQLPGAYIVGIKLDITSHNEDYTIVEQYERAEGR
HSTGGMDELYK (SEQ ID NO:176)

iRFP (near-infrared fluorescent protein)
DNA:
GAAGGATCCGTCGCCAGGCAGCCTGACCTCTTGACCTGCGACGATGAGCCGATCCATATCCCCGGTGCCATCCAA
CCGCATGGACTGCTGCTCGCCCTCGCCGCCGACATGACGATCGTTGCCGGCAGCGACAACCTTCCCGAACTCACC
GGACTGGCGATCGGCGCCCTGATCGGCCGCTCTGCGGCCGATGTCTTCGACTCGGAGACGCACAACCGTCTGACG
ATCGCCTTGGCCGAGCCCGGGGCGGCCGTCGGAGCACCGATCACTGTCGGCTTCACGATGCGAAAGGACGCAGGC
TTCATCGGCTCCTGGCATCGCCATGATCAGCTCATCTTCCTCGAGCTCGAGCCTCCCCAGCGGGACGTCGCCGAG
CCGCAGGCGTTCTTCCGCCGCACCAACAGCGCCATCCGCCGCCTGCAGGCCGCCGAAACCTTGGAAAGCGCCTGC
GCCGCCGCGGCGCAAGAGGTGCGGAAGATTACCGGCTTCGATCGGGTGATGATCTATCGCTTCGCCTCCGACTTC
AGCGGCGAAGTGATCGCAGAGGATCGGTGCGCCGAGGTCGAGTCAAAACTAGGCCTGCACTATCCTGCCTCAACC
GTGCCGGCGCAGGCCCGTCGGCTCTATACCATCAACCCGGTACGGATCATTCCCGATATCAATTATCGGCCGGTG
CCGGTCACCCCAGACCTCAATCCGGTCACCGGGCGGCCGATTGATCTTAGCTTCGCCATCCTGCGCAGCGTCTCG
CCCGTCCATCTGGAATTCATGCGCAACATAGGCATGCACGGCACGATGTCGATCTCGATTTTGCGCGGCGAGCGA
CTGTGGGGATTGATCGTTTGCCATCACCGAACGCCGTACTACGTCGATCTCGATGGCCGCCAAGCCTGCGAGCTA
GTCGCCCAGGTTCTGGCCTGGCAGATCGGCGTGATGGAAGAG (SEQ ID NO:177)
Protein:
EGSVARQPDLLTCDDEPIHIPGAIQPHGLLLALAADMTIVAGSDNLPELTGLAIGALIGRSAADVFDSETHNRLT
IALAEPGAAVGAPITVGFTMRKDAGFIGSWHRHDQLIFLELEPPQRDVAEPQAFFRRTNSAIRRLQAAETLESAC
AAAAQEVRKITGFDRVMIYRFASDFSGEVIAEDRCAEVESKLGLHYPASTVPAQARRLYTINPVRIIPDINYRPV
PVTPDLNPVTGRPIDLSFAILRSVSPVHLEFMRNIGMHGTMSISILRGERLWGLIVCHHRTPYYVDLDGRQACEL
VAQVLAWQIGVMEE (SEQ ID NO:178)

mCherry185TAG (mCherry with Amber-encoded amino acid position 185)
DNA: (Amber codon underlined)
ATGGGCCGCCTGGAAAGCACCCCGCCGAAAAAAAAACGCAAAGTGGAAGATAGCGCGAGCGTGAGCAAGGGCGAG
GAGGATAACATGGCCATCATCAAGGAGTTCATGCGCTTCAAGGTGCACATGGAGGGCTCCGTGAACGGCCACGAG
TTCGAGATCGAGGGCGAGGGCGAGGGCCGCCCCTACGAGGGCACCCAGACCGCCAAGCTGAAGGTGACCAAGGGT
GGCCCCCTGCCCTTCGCCTGGGACATCCTGTCCCCTCAGTTCATGTACGGCTCCAAGGCCTACGTGAAGCACCCC
GCCGACATCCCCGACTACTTGAAGCTGTCCTTCCCCGAGGGCTTCAAGTGGGAGCGCGTGATGAACTTCGAGGAC
GGCGGCGTGGTGACCGTGACCCAGGACTCCTCCCTGCAGGACGGCGAGTTCATCTACAAGGTGAAGCTGCGCGGC
ACCAACTTCCCCTCCGACGGCCCCGTAATGCAGAAGAAGACGATGGGCTGGGAGGCCTCCTCCGAGCGGATGTAC
CCCGAGGACGGCGCCCTGAAGGGCGAGATCAAGCAGAGGCTGAAGCTGAAGGACGGCGGCCACTACGACGCTGAG
GTCAAGACCACCTACAAGGCCAAGTAGCCCGTGCAGCTGCCCGGCGCCTACAACGTCAACATCAAGTTGGACATC
ACCTCCCACAACGAGGACTACACCATCGTGGAACAGTACGAACGCGCCGAGGGCCGCCACTCCACCGGCGGCATG
GACGAGCTGTACAAGCATCATCATCATCATCATTAA (SEQ ID NO:179)
Protein: (X non-canonical amino acid)
MGRLESTPPKKKRKVEDSASVSKGEEDNMAIIKEFMRFKVHMEGSVNGHEFEIEGEGEGRPYEGTQTAKLKVTKG
GPLPFAWDILSPQFMYGSKAYVKHPADIPDYLKLSFPEGFKWERVMNFEDGGVVTVTQDSSLQDGEFIYKVKLRG
TNFPSDGPVMQKKTMGWEASSERMYPEDGALKGEIKQRLKLKDGGHYDAEVKTTYKAKXPVQLPGAYNVNIKLDI
TSHNEDYTIVEQYERAEGRHSTGGMDELYKHHHHHH (SEQ ID NO:180)

mCherry185TAG-2xMS2(mCherry185TAG with 2 MS2 RNA stem-loops)
DNA: (MS2 stem-loops and Amber codon underlined)
ATGGGCCGCCTGGAAAGCACCCCGCCGAAAAAAAAACGCAAAGTGGAAGATAGCGCGAGCGTGAGCAAGGGCGAG
GAGGATAACATGGCCATCATCAAGGAGTTCATGCGCTTCAAGGTGCACATGGAGGGCTCCGTGAACGGCCACGAG
TTCGAGATCGAGGGCGAGGGCGAGGGCCGCCCCTACGAGGGCACCCAGACCGCCAAGCTGAAGGTGACCAAGGGT
GGCCCCCTGCCCTTCGCCTGGGACATCCTGTCCCCTCAGTTCATGTACGGCTCCAAGGCCTACGTGAAGCACCCC
GCCGACATCCCCGACTACTTGAAGCTGTCCTTCCCCGAGGGCTTCAAGTGGGAGCGCGTGATGAACTTCGAGGAC
GGCGGCGTGGTGACCGTGACCCAGGACTCCTCCCTGCAGGACGGCGAGTTCATCTACAAGGTGAAGCTGCGCGGC
ACCAACTTCCCCTCCGACGGCCCCGTAATGCAGAAGAAGACGATGGGCTGGGAGGCCTCCTCCGAGCGGATGTAC
CCCGAGGACGGCGCCCTGAAGGGCGAGATCAAGCAGAGGCTGAAGCTGAAGGACGGCGGCCACTACGACGCTGAG
GTCAAGACCACCTACAAGGCCAAGTAGCCCGTGCAGCTGCCCGGCGCCTACAACGTCAACATCAAGTTGGACATC
ACCTCCCACAACGAGGACTACACCATCGTGGAACAGTACGAACGCGCCGAGGGCCGCCACTCCACCGGCGGCATG
GACGAGCTGTACAAGCATCATCATCATCATCATTAAAGATCCTAAGGTACCTAATTGCCTAGAAAACATGAGGAT
CACCCATGTCTGCAGGTCGACTCTAGAAAACATGAGGATCACCCATGT (SEQ ID NO:181)

Protein: (X indicates non-canonical amino acid)
MGRLESTPPKKKRKVEDSASVSKGEEDNMAIIKEFMRFKVHMEGSVNGHEFEIEGEGEGRPYEGTQTAKLKVTKG
GPLPFAWDILSPQFMYGSKAYVKHPADIPDYLKLSFPEGFKWERVMNFEDGGVVTVTQDSSLQDGEFIYKVKLRG
TNFPSDGPVMQKKTMGWEASSERMYPEDGALKGEIKQRLKLKDGGHYDAEVKTTYKAKXPVQLPGAYNVNIKLDI
TSHNEDYTIVEQYERAEGRHSTGGMDELYKHHHHHH (SEQ ID NO:182)

mCherry185TAG-4xBoxB (mCherry185TAG with 4 BoxB-loops)
DNA: (BoxB-stem loops and Amber codon underlined)
ATGGGCCGCCTGGAAAGCACCCCGCCGAAAAAAAAACGCAAAGTGGAAGATAGCGCGAGCGTGAGCAAGGGCGAG
GAGGATAACATGGCCATCATCAAGGAGTTCATGCGCTTCAAGGTGCACATGGAGGGCTCCGTGAACGGCCACGAG
TTCGAGATCGAGGGCGAGGGCGAGGGCCGCCCCTACGAGGGCACCCAGACCGCCAAGCTGAAGGTGACCAAGGGT
GGCCCCCTGCCCTTCGCCTGGGACATCCTGTCCCCTCAGTTCATGTACGGCTCCAAGGCCTACGTGAAGCACCCC
GCCGACATCCCCGACTACTTGAAGCTGTCCTTCCCCGAGGGCTTCAAGTGGGAGCGCGTGATGAACTTCGAGGAC
GGCGGCGTGGTGACCGTGACCCAGGACTCCTCCCTGCAGGACGGCGAGTTCATCTACAAGGTGAAGCTGCGCGGC
ACCAACTTCCCCTCCGACGGCCCCGTAATGCAGAAGAAGACGATGGGCTGGGAGGCCTCCTCCGAGCGGATGTAC
CCCGAGGACGGCGCCCTGAAGGGCGAGATCAAGCAGAGGCTGAAGCTGAAGGACGGCGGCCACTACGACGCTGAG
GTCAAGACCACCTACAAGGCCAAGTAGCCCGTGCAGCTGCCCGGCGCCTACAACGTCAACATCAAGTTGGACATC
ACCTCCCACAACGAGGACTACACCATCGTGGAACAGTACGAACGCGCCGAGGGCCGCCACTCCACCGGCGGCATG
GACGAGCTGTACAAGCATCATCATCATCATCATTAAAGATCCTAAGGTACCGCCCTGAAAAAGGGCTCGAGCCCT
GAAAAAGGGCAATTGCCCTGAAAAAGGGCGTCGACGCCCTGAAAAAGGGC (SEQ ID NO:183)
Protein: (X indicates non-canonical amino acid)
MGRLESTPPKKKRKVEDSASVSKGEEDNMAIIKEFMRFKVHMEGSVNGHEFEIEGEGEGRPYEGTQTAKLKVTKG
GPLPFAWDILSPQFMYGSKAYVKHPADIPDYLKLSFPEGFKWERVMNFEDGGVVTVTQDSSLQDGEFIYKVKLRG
TNFPSDGPVMQKKTMGWEASSERMYPEDGALKGEIKQRLKLKDGGHYDAEVKTTYKAKXPVQLPGAYNVNIKLDI
TSHNEDYTIVEQYERAEGRHSTGGMDELYKHHHHHH (SEQ ID NO:184)

GFP39TAA-2xMS2 (GFP with Ochre-encoded amino acid position 39 and 2 MS2 RNA
stem-loops)
DNA: (MS2 stem-loops and Ochre codon underlined)
ATGGGCCGCCTGGAAAGCACCCCGCCGAAAAAAAAACGCAAAGTGGAAGATAGCGCGAGCGATTACAAAGATGAT
GATGATAAAGTGAGCAAGGGCGAGGAGCTGTTCACCGGGGTGGTGCCCATCCTGGTCGAGCTGGACGGCGACGTA
AACGGCCACAAGTTCAGCGTGTCCGGCGAGGGCGAGGGCGATGCCACCTAAGGCAAGCTGACCCTGAAGTTCATC
TGCACCACCGGCAAGCTGCCCGTGCCCTGGCCCACCCTCGTGACCACCCTGACCTACGGCGTGCAGTGCTTCAGC
CGCTACCCCGACCACATGAAGCAGCACGACTTCTTCAAGTCCGCCATGCCCGAAGGCTACGTCCAGGAGCGCACC
ATCTTCTTCAAGGACGACGGCAACTACAAGACCCGCGCCGAGGTGAAGTTCGAGGGCGACACCCTGGTGAACCGC
ATCGAGCTGAAGGGCATCGACTTCAAGGAGGACGGCAACATCCTGGGGCACAAGCTGGAGTACAACTACAACAGC
CACAACGTCTATATCATGGCCGACAAGCAGAAGAACGGCATCAAGGCCAACTTCAAGATCCGCCACAACATCGAG
GACGGCAGCGTGCAGCTCGCCGACCACTACCAGCAGAACACCCCCATCGGCGACGGCCCCGTGCTGCTGCCCGAC
AACCACTACCTGAGCACCCAGTCCGCCCTGAGCAAAGACCCCAACGAGAAGCGCGATCACATGGTCCTGCTGGAG
TTCGTGACCGCCGCCGGGATCACTCTCGGCATGGACGAGCTGTACAAGCATCACCATCACCATCACTGAGGATCC
TAAGGTACCTAATTGCCTAGAAAACATGAGGATCACCCATGTCTGCAGGTCGACTCTAGAAAACATGAGGATCAC
CCATGT (SEQ ID NO:185)
Protein: (X indicates non-canonical amino acid)
MGRLESTPPKKKRKVEDSASDYKDDDDKVSKGEELFTGVVPILVELDGDVNGHKFSVSGEGEGDATXGKLTLKFI
CTTGKLPVPWPTLVTTLTYGVQCFSRYPDHMKQHDFFKSAMPEGYVQERTIFFKDDGNYKTRAEVKFEGDTLVNR
IELKGIDFKEDGNILGHKLEYNYNSHNVYIMADKQKNGIKANFKIRHNIEDGSVQLADHYQQNTPIGDGPVLLPD
NHYLSTQSALSKDPNEKRDHMVLLEFVTAAGITLGMDELYKHHHHHH (SEQ ID NO:186)

mCherry185TAA-2xMS2 (mCherry with Ochre-encoded amino acid position 185 and 2
MS2 RNA stem-loops)
DNA: (MS2 stem-loops and Ochre codon underlined)
ATGGGCCGCCTGGAAAGCACCCCGCCGAAAAAAAAACGCAAAGTGGAAGATAGCGCGAGCGTGAGCAAGGGCGAG
GAGGATAACATGGCCATCATCAAGGAGTTCATGCGCTTCAAGGTGCACATGGAGGGCTCCGTGAACGGCCACGAG
TTCGAGATCGAGGGCGAGGGCGAGGGCCGCCCCTACGAGGGCACCCAGACCGCCAAGCTGAAGGTGACCAAGGGT
GGCCCCCTGCCCTTCGCCTGGGACATCCTGTCCCCTCAGTTCATGTACGGCTCCAAGGCCTACGTGAAGCACCCC
GCCGACATCCCCGACTACTTGAAGCTGTCCTTCCCCGAGGGCTTCAAGTGGGAGCGCGTGATGAACTTCGAGGAC
GGCGGCGTGGTGACCGTGACCCAGGACTCCTCCCTGCAGGACGGCGAGTTCATCTACAAGGTGAAGCTGCGCGGC
ACCAACTTCCCCTCCGACGGCCCCGTAATGCAGAAGAAGACGATGGGCTGGGAGGCCTCCTCCGAGCGGATGTAC

CCCGAGGACGGCGCCCTGAAGGGCGAGATCAAGCAGAGGCTGAAGCTGAAGGACGGCGGCCACTACGACGCTGAG
GTCAAGACCACCTACAAGGCCAAGTAACCCGTGCAGCTGCCCGGCGCCTACAACGTCAACATCAAGTTGGACATC
ACCTCCCACAACGAGGACTACACCATCGTGGAACAGTACGAACGCGCCGAGGGCCGCCACTCCACCGGCGGCATG
GACGAGCTGTACAAGCATCATCATCATCATCATTGAAGATCCTAAGGTACCTAATTGCCTAGAAAACATGAGGAT
CACCCATGTCTGCAGGTCGACTCTAGAAAACATGAGGATCACCCATGT (SEQ ID NO:187)
Protein: (X indicates non-canonical amino acid)
MGRLESTPPKKKRKVEDSASVSKGEEDNMAIIKEFMRFKVHMEGSVNGHEFEIEGEGEGRPYEGTQTAKLKVTKG
GPLPFAWDILSPQFMYGSKAYVKHPADIPDYLKLSFPEGFKWERVMNFEDGGVVTVTQDSSLQDGEFIYKVKLRG
TNFPSDGPVMQKKTMGWEASSERMYPEDGALKGEIKQRLKLKDGGHYDAEVKTTYKAKXPVQLPGAYNVNIKLDI
TSHNEDYTIVEQYERAEGRHSTGGMDELYKHHHHHH (SEQ ID NO:188)

GFP39TGA-2xMS2 (GFP with Opal-encoded amino acid position 39 and 2 MS2 RNA
stem-loops)
DNA: (MS2 stem-loops and Opal codon underlined)
ATGGGCCGCCTGGAAAGCACCCCGCCGAAAAAAAAACGCAAAGTGGAAGATAGCGCGAGCGATTACAAAGATGAT
GATGATAAAGTGAGCAAGGGCGAGGAGCTGTTCACCGGGGTGGTGCCCATCCTGGTCGAGCTGGACGGCGACGTA
AACGGCCACAAGTTCAGCGTGTCCGGCGAGGGCGAGGGCGATGCCACCTGAGGCAAGCTGACCCTGAAGTTCATC
TGCACCACCGGCAAGCTGCCCGTGCCCTGGCCCACCCTCGTGACCACCCTGACCTACGGCGTGCAGTGCTTCAGC
CGCTACCCCGACCACATGAAGCAGCACGACTTCTTCAAGTCCGCCATGCCCGAAGGCTACGTCCAGGAGCGCACC
ATCTTCTTCAAGGACGACGGCAACTACAAGACCCGCGCCGAGGTGAAGTTCGAGGGCGACACCCTGGTGAACCGC
ATCGAGCTGAAGGGCATCGACTTCAAGGAGGACGGCAACATCCTGGGGCACAAGCTGGAGTACAACTACAACAGC
CACAACGTCTATATCATGGCCGACAAGCAGAAGAACGGCATCAAGGCCAACTTCAAGATCCGCCACAACATCGAG
GACGGCAGCGTGCAGCTCGCCGACCACTACCAGCAGAACACCCCCATCGGCGACGGCCCCGTGCTGCTGCCCGAC
AACCACTACCTGAGCACCCAGTCCGCCCTGAGCAAAGACCCCAACGAGAAGCGCGATCACATGGTCCTGCTGGAG
TTCGTGACCGCCGCCGGGATCACTCTCGGCATGGACGAGCTGTACAAGCATCACCATCACCATCACTAAGGATCC
TAAGGTACCTAATTGCCTAGAAAACATGAGGATCACCCATGTCTGCAGGTCGACTCTAGAAAACATGAGGATCAC
CCATGT (SEQ ID NO:189)
Protein: (X indicates non-canonical amino acid)
MGRLESTPPKKKRKVEDSASDYKDDDDKVSKGEELFTGVVPILVELDGDVNGHKFSVSGEGEGDATXGKLTLKFI
CTTGKLPVPWPTLVTTLTYGVQCFSRYPDHMKQHDFFKSAMPEGYVQERTIFFKDDGNYKTRAEVKFEGDTLVNR
IELKGIDFKEDGNILGHKLEYNYNSHNVYIMADKQKNGIKANFKIRHNIEDGSVQLADHYQQNTPIGDGPVLLPD
NHYLSTQSALSKDPNEKRDHMVLLEFVTAAGITLGMDELYKHHHHHH (SEQ ID NO:190)

mCherry185TGA-2xMS2 (mCherry with Opal-encoded amino acid position 185 and 2
MS2 RNA stem-loops)
DNA: (MS2 stem-loops and Opal codon underlined)
ATGGGCCGCCTGGAAAGCACCCCGCCGAAAAAAAAACGCAAAGTGGAAGATAGCGCGAGCGTGAGCAAGGGCGAG
GAGGATAACATGGCCATCATCAAGGAGTTCATGCGCTTCAAGGTGCACATGGAGGGCTCCGTGAACGGCCACGAG
TTCGAGATCGAGGGCGAGGGCGAGGGCCGCCCCTACGAGGGCACCCAGACCGCCAAGCTGAAGGTGACCAAGGGT
GGCCCCCTGCCCTTCGCCTGGGACATCCTGTCCCCTCAGTTCATGTACGGCTCCAAGGCCTACGTGAAGCACCCC
GCCGACATCCCCGACTACTTGAAGCTGTCCTTCCCCGAGGGCTTCAAGTGGGAGCGCGTGATGAACTTCGAGGAC
GGCGGCGTGGTGACCGTGACCCAGGACTCCTCCCTGCAGGACGGCGAGTTCATCTACAAGGTGAAGCTGCGCGGC
ACCAACTTCCCCTCCGACGGCCCCGTAATGCAGAAGAAGACGATGGGCTGGGAGGCCTCCTCCGAGCGGATGTAC
CCCGAGGACGGCGCCCTGAAGGGCGAGATCAAGCAGAGGCTGAAGCTGAAGGACGGCGGCCACTACGACGCTGAG
GTCAAGACCACCTACAAGGCCAAGTGACCCGTGCAGCTGCCCGGCGCCTACAACGTCAACATCAAGTTGGACATC
ACCTCCCACAACGAGGACTACACCATCGTGGAACAGTACGAACGCGCCGAGGGCCGCCACTCCACCGGCGGCATG
GACGAGCTGTACAAGCATCATCATCATCATCATTAAAGATCCTAAGGTACCTAATTGCCTAGAAAACATGAGGAT
CACCCATGTCTGCAGGTCGACTCTAGAAAACATGAGGATCACCCATGT (SEQ ID NO:191)
Protein: (X indicates non-canonical amino acid)
MGRLESTPPKKKRKVEDSASVSKGEEDNMAIIKEFMRFKVHMEGSVNGHEFEIEGEGEGRPYEGTQTAKLKVTKG
GPLPFAWDILSPQFMYGSKAYVKHPADIPDYLKLSFPEGFKWERVMNFEDGGVVTVTQDSSLQDGEFIYKVKLRG
TNFPSDGPVMQKKTMGWEASSERMYPEDGALKGEIKQRLKLKDGGHYDAEVKTTYKAKXPVQLPGAYNVNIKLDI
TSHNEDYTIVEQYERAEGRHSTGGMDELYKHHHHHH (SEQ ID NO:192)

Nup153 (Homo sapiens Nucleoporin 153; Uniprot: P49790)
DNA:
ATGGCGTCTGGTGCTGGCGGTGTTGGTGGAGGAGGTGGGGGTAAAATTCGTACTCGTCGCTGTCATCAAGGTCCG
ATTAAACCGTATCAGCAGGGACGTCAGCAACATCAGGGTATTCTGAGCCGTGTGACCGAAAGCGTGAAAAACATT

GTGCCGGGTTGGCTGCAACGTTATTTCAACAAAAATGAGGATGTGTGTTCGTGTTCTACCGATACCAGTGAAGTT
CCTCGTTGGCCGGAAAACAAAGAAGATCACCTGGTGTATGCCGATGAAGAATCGAGCAATATCACCGATGGCCGT
ATTACTCCTGAACCGGCGGTTAGTAACACTGAAGAACCGTCAACCACAAGCACAGCATCGAACTATCCAGATGTC
CTGACTCGCCCTTCTCTGCACCGTTCTCACCTGAACTTTAGCATGCTGGAATCACCAGCTCTGCATTGTCAGCCG
TCTACCAGTAGTGCCTTCCCGATTGGCTCTAGTGGCTTTTCGCTGGTCAAAGAGATCAAAGACTCGACCTCTCAA
CATGACGATGATAACATTAGCACGACCTCGGGTTTTAGTAGCCGTGCCTCCGATAAAGACATTACCGTGAGCAAA
AACACCTCTCTGCCGCCTCTGTGGAGTCCTGAAGCCGAACGCTCTCATAGTCTGTCTCAGCACACAGCCACCAGT
TCCAAAAAACCAGCCTTCAACCTGAGCGCCTTTGGTACACTGTCACCGAGCCTGGGAAATTCCTCTATCCTGAAA
ACATCACAGCTGGGCGATAGTCCGTTTTATCCGGGCAAAACGACGTATGGTGGTGCCGCTGCTGCTGTTCGCCAG
TCTAAACTGCGTAACACTCCGTATCAAGCTCCAGTCCGTCGCCAAATGAAAGCAAAACAACTGTCGGCCCAGTCT
TATGGTGTGACAAGCTCTACAGCTCGTCGTATCCTGCAAAGTCTGGAGAAAATGTCATCTCCGCTGGCAGATGCC
AAACGTATTCCGTCCATTGTGAGCAGTCCGCTGAATAGCCCGCTGGACCGTAGTGGGATCGATATCACCGACTTC
CAAGCCAAACGTGAGAAAGTGGATAGCCAGTATCCGCCTGTACAACGTCTGATGACCCCGAAACCGGTTTCAATT
GCCACGAATCGTAGCGTGTATTTCAAACCGTCACTGACCCCTAGTGGTGAGTTTCGTAAAACAAATCAGCGTATC
GACAACAAATGCTCTACCGGGTATGAAAAAAACATGACGCCGGGACAGAATCGTGAACAACGTGAATCTGGCTTC
TCTTATCCGAACTTTAGTCTGCCGGCAGCAAATGGTCTGAGTAGCGGTGTAGGAGGTGGTGGGGGCAAAATGCGC
CGTGAACGTCACGCCTTTGTGGCCTCTAAACCTCTGGAAGAAGAAGAGATGGAGGTTCCTGTACTGCCGAAAATC
AGTCTGCCTATCACCTCTTCAAGTCTGCCGACCTTCAACTTTTCTAGTCCGGAAATCACAACCTCTAGCCCGTCA
CCGATTAATAGCAGTCAAGCACTGACGAATAAAGTCCAAATGACCTCACCGAGTTCTACGGGTTCTCCGATGTTC
AAATTCTCTAGTCCTATCGTGAAATCAACCGAAGCGAACGTCCTGCCTCCTTCTAGTATTGGGTTCACCTTTAGC
GTCCCAGTGGCCAAAACAGCTGAACTGAGCGGTAGCAGTAGTACTCTGGAACCGATTATCAGCTCAAGCGCCCAT
CATGTCACTACCGTGAATAGCACAAACTGTAAAAAAACGCCGCCTGAGGACTGTGAAGGACCGTTTCGTCCTGCC
GAAATCCTGAAAGAAGGTTCCGTCCTGGACATTCTGAAATCTCCGGGATTTGCCTCTCCTAAAATCGACTCTGTT
GCCGCTCAACCAACTGCCACATCACCGGTGGTTTATACTCGTCCGGCGATTAGCAGTTTTAGCAGTAGTGGCATC
GGTTTTGGTGAATCCCTGAAAGCTGGCTCATCTTGGCAGTGTGACACCTGCCTGCTGCAAAACAAAGTGACCGAT
AACAAATGTATTGCCTGTCAGGCCGCCAAACTGTCTCCTCGTGATACAGCCAAACAGACCGGCATCGAAACCCCT
AATAAAAGCGGGAAAACGACCCTGTCAGCAAGTGGTACGGGATTTGGGGACAAATTCAAACCTGTGATCGGCACA
TGGGACTGTGACACTTGTCTGGTACAGAACAAACCAGAAGCGATCAAATGTGTGGCCTGTGAAACGCCTAAACCT
GGAACATGTGTGAAACGTGCCCTGACTCTGACTGTTGTGTCAGAAAGCGCCGAAACCATGACGGCAAGCAGCTCA
TCCTGTACTGTGACTACCGGGACTCTGGGATTTGGTGACAAATTCAAACGCCCGATTGGTTCCTGGGAATGCTCC
GTGTGTTGTGTGAGCAATAATGCCGAGGACAACAAATGTGTGTCCTGTATGAGCGAGAAACCTGGCAGCTCTGTT
CCTGCTAGCAGCTCTAGCACAGTTCCTGTTAGTCTGCCTAGTGGTGGTTCTCTGGGTCTGGAAAAATTCAAAAAA
CCTGAAGGAAGCTGGGATTGTGAGCTGTGCCTGGTACAGAATAAAGCGGATAGCACGAAATGTCTGGCCTGTGAG
TCAGCCAAACCAGGGACTAAAAGCGGCTTTAAAGGCTTCGACACGTCGAGCAGTTCTAGTAACAGCGCCGCCTCA
TCATCTTTCAAATTTGGGGTGAGCAGCTCCTCTAGTGGTCCTAGTCAAACACTGACCTCTACCGGAAACTTCAAA
TTCGGCGATCAGGGTGGCTTCAAAATTGGTGTCTCCTCTGATTCGGGTAGCATTAACCCGATGAGTGAGGGGTTC
AAATTCAGCAAACCAATTGGCGATTTCAAATTCGGTGTGTCGTCTGAATCCAAACCTGAAGAAGTCAAAAAAGAC
AGCAAAAACGACAATTTCAAATTCGGCCTGTCTAGTGGTCTGTCTAATCCGGTTAGCCTGACCCCGTTTCAGTTC
GGGGTGTCTAATCTGGGTCAGGAAGAGAAAAAAGAGGAGCTGCCTAAAAGTTCATCTGCCGGGTTCAGTTTTGGT
ACAGGCGTGATCAATAGCACTCCAGCACCAGCCAATACAATCGTGACGAGCGAGAACAAATCGAGCTTCAACCTG
GGGACAATCGAAACGAAAAGCGCCAGTGTAGCGCCATTCACGTGTAAAACCTCCGAGGCAAAAAAAGAAGAGATG
CCGGCCACAAAAGGTGGATTCTCATTCGGCAACGTGGAACCGGCTAGCCTGCCATCAGCAAGCGTGTTTGTACTG
GGCCGTACCGAGGAGAAACAGCAGGAACCTGTTACTAGCACCAGTCTGGTCTTTGGTAAAAAAGCCGACAACGAA
GAACCGAAATGTCAGCCAGTGTTCAGCTTCGGCAATAGCGAACAGACGAAAGACGAAAACAGCAGCAAATCGACG
TTCAGCTTCAGTATGACGAAACCGAGCGAAAAAGAAAGTGAGCAGCCAGCAAAAGCAACGTTCGCCTTTGGAGCA
CAGACATCAACCACAGCCGATCAAGGAGCAGCGAAACCAGTTTTCAGTTTTCTGAATAACAGCTCAAGCAGCAGT
TCTACACCAGCAACCTCAGCAGGTGGTGGGATCTTTGGATCAAGCACCTCATCCAGCAATCCGCCAGTGGCAACA
TTCGTGTTTGGCCAGAGCAGTAATCCGGTGTCATCTTCAGCATTTGGGAATACCGCCGAGAGTAGCACATCACAG
TCTCTGCTGTTCTCACAGGACTCTAAACTGGCAACCACCTCTTCTACTGGTACAGCGGTTACCCCGTTTGTGTTC
GGTCCGGGAGCATCATCCAATAATACCACGACGTCGGGCTTTGGGTTTGGTGCCACGACAACAAGCAGTAGCGCT
GGTAGCAGCTTTGTCTTTGGCACAGGTCCTTCAGCACCTTCTGCTTCACCAGCTTTCGGAGCCAATCAGACTCCG
ACATTCGGACAGTCACAGGGTGCCTCTCAACCAAATCCTCCGGGTTTTGGCAGTATTAGCAGTAGTACCGCCCTG
TTCCCGACCGGTAGTCAACCGGCACCGCCAACATTTGGAACGGTTAGCAGTAGTAGTCAGCCTCCGGTGTTTGGA
CAACAACCGAGCCAGAGCGCCTTCGGATCAGGAACGACCCCTAATAGTAGCAGTGCCTTCCAGTTCGGTAGCAGT
ACCACCAACTTCAACTTCACGAACAATAGCCCGTCAGGTGTGTTCACGTTTGGCGCCAATTCTTCTACCCCAGCG
GCAAGTGCTCAACCTTCAGGCTCAGGTGGATTTCCTTTCAACCAGTCACCAGCAGCGTTTACTGTTGGTTCTAAC
GGGAAAAACGTTTTCAGTAGCAGCGGCACCTCGTTTTCTGGTCGTAAAATCAAAACGGCCGTTCGTCGCCGTAAA
(SEQ ID NO:193)

Protein:



















 (SEQ ID NO:194)

Vim116TAG (Homo sapiens Vimentin with Amber-encoded amino acid position 116;
Uniprot: P08670)
DNA: (Amber codon underlined)
ATGTCCACCAGGTCCGTGTCCTCGTCCTCCTACCGCAGGATGTTCGGCGGCCCGGGCACCGCGAGCCGGCCGAGC
TCCAGCCGGAGCTACGTGACTACGTCCACCCGCACCTACAGCCTGGGCAGCGCGCTGCGCCCCAGCACCAGCCGC
AGCCTCTACGCCTCGTCCCCGGGCGGCGTGTATGCCACGCGCTCCTCTGCCGTGCGCCTGCGGAGCAGCGTGCCC
GGGGTGCGGCTCCTGCAGGACTCGGTGGACTTCTCGCTGGCCGACGCCATCAACACCGAGTTCAAGAACACCCGC
ACCAACGAGAAGGTGGAGCTGCAGGAGCTGAATGACCGCTTCGCCTAGTACATCGACAAGGTGCGCTTCCTGGAG
CAGCAGAATAAGATCCTGCTGGCCGAGCTCGAGCAGCTCAAGGGCCAAGGCAAGTCGCGCCTGGGGGACCTCTAC
GAGGAGGAGATGCGGGAGCTGCGCCGGCAGGTGGACCAGCTAACCAACGACAAAGCCCGCGTCGAGGTGGAGCGC
GACAACCTGGCCGAGGACATCATGCGCCTCCGGGAGAAATTGCAGGAGGAGATGCTTCAGAGAGAGGAAGCCGAA
AACACCCTGCAATCTTTCAGACAGGATGTTGACAATGCGTCTCTGGCACGTCTTGACCTTGAACGCAAAGTGGAA
TCTTTGCAAGAAGAGATTGCCTTTTTGAAGAAACTCCACGAAGAGGAAATCCAGGAGCTGCAGGCTCAGATTCAG
GAACAGCATGTCCAAATCGATGTGGATGTTTCCAAGCCTGACCTCACGGCTGCCCTGCGTGACGTACGTCAGCAA
TATGAAAGTGTGGCTGCCAAGAACCTGCAGGAGGCAGAAGAATGGTACAAATCCAAGTTTGCTGACCTCTCTGAG
GCTGCCAACCGGAACAATGACGCCCTGCGCCAGGCAAAGCAGGAGTCCACTGAGTACCGGAGACAGGTGCAGTCC
CTCACCTGTGAAGTGGATGCCCTTAAAGGAACCAATGAGTCCCTGGAACGCCAGATGCGTGAAATGGAAGAGAAC
TTTGCCGTTGAAGCTGCTAACTACCAAGACACTATTGGCCGCCTGCAGGATGAGATTCAGAATATGAAGGAGGAA
ATGGCTCGTCACCTTCGTGAATACCAAGACCTGCTCAATGTTAAGATGGCCCTTGACATTGAGATTGCCACCTAC
AGGAAGCTGCTGGAAGGCGAGGAGAGCAGGATTTCTCTGCCTCTTCCAAACTTTTCCTCCCTGAACCTGAGGGAA
ACTAATCTGGATTCACTCCCTCTGGTTGATACCCACTCAAAAAGGACACTTCTGATTAAGACGGTTGAAACTAGA
GATGGACAGGTTATCAACGAAACTTCTCAGCATCACGATGACCTTGAA (SEQ ID NO:195)
Protein: (X indicates non-canonical amino acid)
MSTRSVSSSSYRRMFGGPGTASRPSSSRSYVTTSTRTYSLGSALRPSTSRSLYASSPGGVYATRSSAVRLRSSVP
GVRLLQDSVDFSLADAINTEFKNTRTNEKVELQELNDRFAXYIDKVRFLEQQNKILLAELEQLKGQGKSRLGDLY
EEEMRELRRQVDQLTNDKARVEVERDNLAEDIMRLREKLQEEMLQREEAENTLQSFRQDVDNASLARLDLERKVE
SLQEEIAFLKKLHEEEIQELQAQIQEQHVQIDVDVSKPDLTAALRDVRQQYESVAAKNLQEAEEWYKSKFADLSE
AANRNNDALRQAKQESTEYRRQVQSLTCEVDALKGTNESLERQMREMEENFAVEAANYQDTIGRLQDEIQNMKEE
MARHLREYQDLLNVKMALDIEIATYRKLLEGEESRISLPLPNFSSLNLRETNLDSLPLVDTHSKRTLLIKTVETR
DGQVINETSQHHDDLE (SEQ ID NO:196)

INSR676TAG (Homo sapiens insulin receptor; Uniprot: P06213)
DNA: (Amber codon underlined)
ATGGGCACCGGGGGCCGGCGGGGGGCGGCGGCCGCGCCGCTGCTGGTGGCGGTGGCCGCGCTGCTACTGGGCGCC
GCGGGCCACCTGTACCCCGGAGAGGTGTGTCCCGGCATGGATATCCGGAACAACCTCACTAGGTTGCATGAGCTG
GAGAATTGCTCTGTCATCGAAGGACACTTGCAGATACTCTTGATGTTCAAAACGAGGCCCGAAGATTTCCGAGAC

CTCAGTTTCCCCAAACTCATCATGATCACTGATTACTTGCTGCTCTTCCGGGTCTATGGGCTCGAGAGCCTGAAG
GACCTGTTCCCCAACCTCACGGTCATCCGGGGATCACGACTGTTCTTTAACTACGCGCTGGTCATCTTCGAGATG
GTTCACCTCAAGGAACTCGGCCTCTACAACCTGATGAACATCACCCGGGGTTCTGTCCGCATCGAGAAGAACAAT
GAGCTCTGTTACTTGGCCACTATCGACTGGTCCCGTATCCTGGATTCCGTGGAGGATAATTACATCGTGTTGAAC
AAAGATGACAACGAGGAGTGTGGAGACATCTGTCCGGGTACCGCGAAGGGCAAGACCAACTGCCCCGCCACCGTC
ATCAACGGGCAGTTTGTCGAACGATGTTGGACTCATAGTCACTGCCAGAAAGTTTGCCCGACCATCTGTAAGTCA
CACGGCTGCACCGCCGAAGGCCTCTGTTGCCACAGCGAGTGCCTGGGCAACTGTTCTCAGCCCGACGACCCCACC
AAGTGCGTGGCCTGCCGCAACTTCTACCTGGATGGCAGGTGTGTGGAGACCTGCCCGCCCCCGTACTACCACTTC
CAGGACTGGCGCTGTGTGAACTTCAGCTTCTGCCAGGACCTGCACCACAAATGCAAGAACTCGCGGAGGCAGGGC
TGCCACCAGTACGTCATTCACAACAACAAGTGCATCCCTGAGTGTCCCTCCGGGTACACGATGAATTCCAGCAAC
TTGCTGTGCACCCCATGCCTGGGTCCCTGTCCCAAGGTGTGCCACCTCCTAGAAGGCGAGAAGACCATCGACTCG
GTGACGTCTGCCCAGGAGCTCCGAGGATGCACCGTCATCAACGGGAGTCTGATCATCAACATTCGAGGAGGCAAC
AATCTGGCAGCTGAGCTAGAAGCCAACCTCGGCCTCATTGAAGAAATTTCAGGGTATCTAAAAATCCGCCGATCC
TACGCTCTGGTGTCACTTTCCTTCTTCCGGAAGTTACGTCTGATTCGAGGAGAGACCTTGGAAATTGGGAACTAC
TCCTTCTATGCCTTGGACAACCAGAACCTAAGGCAGCTCTGGGACTGGAGCAAACACAACCTCACCATCACTCAG
GGGAAACTCTTCTTCCACTATAACCCCAAACTCTGCTTGTCAGAAATCCACAAGATGGAAGAAGTTTCAGGAACC
AAGGGGCGCCAGGAGAGAAACGACATTGCCCTGAAGACCAATGGGGACCAGGCATCCTGTGAAAATGAGTTACTT
AAATTTTCTTACATTCGGACATCTTTTGACAAGATCTTGCTGAGATGGGAGCCGTACTGGCCCCCCGACTTCCGA
GACCTCTTGGGGTTCATGCTGTTCTACAAAGAGGCCCCTTATCAGAATGTGACGGAGTTCGACGGGCAGGATGCA
TGTGGTTCCAACAGTTGGACGGTGGTAGACATTGACCCACCCCTGAGGTCCAACGACCCCAAATCACAGAACCAC
CCAGGGTGGCTGATGCGGGGTCTCAAGCCCTGGACCCAGTATGCCATCTTTGTGAAGACCCTGGTCACCTTTTCG
GATGAACGCCGGACCTATGGGGCCAAGAGTGACATCATTTATGTCCAGACAGATGCCACCAACCCCTCTGTGCCC
CTGGATCCAATCTCAGTGTCTAACTCATCATCCCAGATTATTCTGAAGTGGAAACCACCCTCCGACCCCAATGGC
AACATCACCCACTACCTGGTTTTCTGGGAGAGGCAGGCGGAAGACAGTGAGCTGTTCGAGCTGGATTATTGCCTC
TAGGGGCTGAAGCTGCCCTCGAGGACCTGGTCTCCACCATTCGAGTCTGAAGATTCTCAGAAGCACAACCAGAGT
GAGTATGAGGATTCGGCCGGCGAATGCTGCTCCTGTCCAAAGACAGACTCTCAGATCCTGAAGGAGCTGGAGGAG
TCCTCGTTTAGGAAGACGTTTGAGGATTACCTGCACAACGTGGTTTTCGTCCCCAGGCCATCTCGGAAACGCAGG
TCCCTTGGCGATGTTGGGAATGTGACGGTGGCCGTGCCCACGGTGGCAGCTTTCCCCAACACTTCCTCGACCAGC
GTGCCCACGAGTCCGGAGGAGCACAGGCCTTTTGAGAAGGTGGTGAACAAGGAGTCGCTGGTCATCTCCGGCTTG
CGACACTTCACGGGCTATCGCATCGAGCTGCAGGCTTGCAACCAGGACACCCCTGAGGAACGGTGCAGTGTGGCA
GCCTACGTCAGTGCGAGGACCATGCCTGAAGCCAAGGCTGATGACATTGTTGGCCCTGTGACGCATGAAATCTTT
GAGAACAACGTCGTCCACTTGATGTGGCAGGAGCCGAAGGAGCCCAATGGTCTGATCGTGCTGTATGAAGTGAGT
TATCGGCGATATGGTGATGAGGAGCTGCATCTCTGCGTCTCCCGCAAGCACTTCGCTCTGGAACGGGGCTGCAGG
CTGCGTGGGCTGTCACCGGGGAACTACAGCGTGCGAATCCGGGCCACCTCCCTTGCGGGCAACGGCTCTTGGACG
GAACCCACCTATTTCTACGTGACAGACTATTTAGACGTCCCGTCAAATATTGCAAAAATTATCATCGGCCCCCTC
ATCTTTGTCTTTCTCTTCAGTGTTGTGATTGGAAGTATTTATCTATTCCTGAGAAAGAGGCAGCCAGATGGGCCG
CTGGGACCGCTTTACGCTTCTTCAAACCCTGAGTATCTCAGTGCCAGTGATGTGTTTCCATGCTCTGTGTACGTG
CCGGACGAGTGGGAGGTGTCTCGAGAGAAGATCACCCTCCTTCGAGAGCTGGGGCAGGGCTCCTTCGGCATGGTG
TATGAGGGCAATGCCAGGGACATCATCAAGGGTGAGGCAGAGACCCGCGTGGCGGTGAAGACGGTCAACGAGTCA
GCCAGTCTCCGAGAGCGGATTGAGTTCCTCAATGAGGCCTCGGTCATGAAGGGCTTCACCTGCCATCACGTGGTG
CGCCTCCTGGGAGTGGTGTCCAAGGGCCAGCCCACGCTGGTGGTGATGGAGCTGATGGCTCACGGAGACCTGAAG
AGCTACCTCCGTTCTCTGCGGCCAGAGGCTGAGAATAATCCTGGCCGCCCTCCCCCTACCCTTCAAGAGATGATT
CAGATGGCGGCAGAGATTGCTGACGGGATGGCCTACCTGAACGCCAAGAAGTTTGTGCATCGGGACCTGGCAGCG
AGAAACTGCATGGTCGCCCATGATTTTACTGTCAAAATTGGAGACTTTGGAATGACCAGAGACATCTATGAAACG
GATTACTACCGGAAAGGGGGCAAGGGTCTGCTCCCTGTACGGTGGATGGCACCGGAGTCCCTGAAGGATGGGGTC
TTCACCACTTCTTCTGACATGTGGTCCTTTGGCGTGGTCCTTTGGGAAATCACCAGCTTGGCAGAACAGCCTTAC
CAAGGCCTGTCTAATGAACAGGTGTTGAAATTTGTCATGGATGGAGGGTATCTGGATCAACCCGACAACTGTCCA
GAGAGAGTCACTGACCTCATGCGCATGTGCTGGCAATTCAACCCCAACATGAGGCCAACCTTCCTGGAGATTGTC
AACCTGCTCAAGGACGACCTGCACCCCAGCTTTCCAGAGGTGTCGTTCTTCCACAGCGAGGAGAACAAGGCTCCC
GAGAGTGAGGAGCTGGAGATGGAGTTTGAGGACATGGAGAATGTGCCCCTGGACCGTTCCTCGCACTGTCAGAGG
GAGGAGGCGGGGGGCCGGGATGGAGGGTCCTCGCTGGGTTTCAAGCGGAGCTACGAGGAACACATCCCTTACACA
CACATGAACGGAGGCAAGAAAAACGGGCGGATTCTGACCTTGCCTCGGTCCAATCCTTCCT (SEQ ID
NO:197)
Protein: (X indicates non-canonical amino acid)
MGTGGRRGAAAAPLLVAVAALLLGAAGHLYPGEVCPGMDIRNNLTRLHELENCSVIEGHLQILLMFKTRPEDFRD
LSFPKLIMITDYLLLFRVYGLESLKDLFPNLTVIRGSRLFFNYALVIFEMVHLKELGLYNLMNITRGSVRIEKNN
ELCYLATIDWSRILDSVEDNYIVLNKDDNEECGDICPGTAKGKTNCPATVINGQFVERCWTHSHCQKVCPTICKS
HGCTAEGLCCHSECLGNCSQPDDPTKCVACRNFYLDGRCVETCPPPYYHFQDWRCVNFSFCQDLHHKCKNSRRQG

CHQYVIHNNKCIPECPSGYTMNSSNLLCTPCLGPCPKVCHLLEGEKTIDSVTSAQELRGCTVINGSLIINIRGGN
NLAAELEANLGLIEEISGYLKIRRSYALVSLSFFRKLRLIRGETLEIGNYSFYALDNQNLRQLWDWSKHNLTITQ
GKLFFHYNPKLCLSEIHKMEEVSGTKGRQERNDIALKTNGDQASCENELLKFSYIRTSFDKILLRWEPYWPPDFR
DLLGFMLFYKEAPYQNVTEFDGQDACGSNSWTVVDIDPPLRSNDPKSQNHPGWLMRGLKPWTQYAIFVKTLVTFS
DERRTYGAKSDIIYVQTDATNPSVPLDPISVSNSSSQIILKWKPPSDPNGNITHYLVFWERQAEDSELFELDYCL
XGLKLPSRTWSPPFESEDSQKHNQSEYEDSAGECCSCPKTDSQILKELEESSFRKTFEDYLHNVVFVPRPSRKRR
SLGDVGNVTVAVPTVAAFPNTSSTSVPTSPEEHRPFEKVVNKESLVISGLRHFTGYRIELQACNQDTPEERCSVA
AYVSARTMPEAKADDIVGPVTHEIFENNVVHLMWQEPKEPNGLIVLYEVSYRRYGDEELHLCVSRKHFALERGCR
LRGLSPGNYSVRIRATSLAGNGSWTEPTYFYVTDYLDVPSNIAKIIIGPLIFVFLFSVVIGSIYLFLRKRQPDGP
LGPLYASSNPEYLSASDVFPCSVYVPDEWEVSREKITLLRELGQGSFGMVYEGNARDIIKGEAETRVAVKTVNES
ASLRERIEFLNEASVMKGFTCHHVVRLLGVVSKGQPTLVVMELMAHGDLKSYLRSLRPEAENNPGRPPPTLQEMI
QMAAEIADGMAYLNAKKFVHRDLAARNCMVAHDFTVKIGDFGMTRDIYETDYYRKGGKGLLPVRWMAPESLKDGV
FTTSSDMWSFGVVLWEITSLAEQPYQGLSNEQVLKFVMDGGYLDQPDNCPERVTDLMRMCWQFNPNMRPTFLEIV
NLLKDDLHPSFPEVSFFHSEENKAPESEELEMEFEDMENVPLDRSSHCQREEAGGRDGGSSLGFKRSYEEHIPYT
HMNGGKKNGRILTLPRSNPS (SEQ ID NO:198)

Nup153-EGFP149TAG
DNA: (Amber codon underlined)
ATGGCGTCTGGTGCTGGCGGTGTTGGTGGAGGAGGTGGGGGTAAAATTCGTACTCGTCGCTGTCATCAAGGTCCG
ATTAAACCGTATCAGCAGGGACGTCAGCAACATCAGGGTATTCTGAGCCGTGTGACCGAAAGCGTGAAAAACATT
GTGCCGGGTTGGCTGCAACGTTATTTCAACAAAAATGAGGATGTGTGTTCGTGTTCTACCGATACCAGTGAAGTT
CCTCGTTGGCCGGAAAACAAAGAAGATCACCTGGTGTATGCCGATGAAGAATCGAGCAATATCACCGATGGCCGT
ATTACTCCTGAACCGGCGGTTAGTAACACTGAAGAACCGTCAACCACAAGCACAGCATCGAACTATCCAGATGTC
CTGACTCGCCCTTCTCTGCACCGTTCTCACCTGAACTTTAGCATGCTGGAATCACCAGCTCTGCATTGTCAGCCG
TCTACCAGTAGTGCCTTCCCGATTGGCTCTAGTGGCTTTTCGCTGGTCAAAGAGATCAAAGACTCGACCTCTCAA
CATGACGATGATAACATTAGCACGACCTCGGGTTTTAGTAGCCGTGCCTCCGATAAAGACATTACCGTGAGCAAA
AACACCTCTCTGCCGCCTCTGTGGAGTCCTGAAGCCGAACGCTCTCATAGTCTGTCTCAGCACACAGCCACCAGT
TCCAAAAAACCAGCCTTCAACCTGAGCGCCTTTGGTACACTGTCACCGAGCCTGGGAAATTCCTCTATCCTGAAA
ACATCACAGCTGGGCGATAGTCCGTTTTATCCGGGCAAAACGACGTATGGTGGTGCCGCTGCTGCTGTTCGCCAG
TCTAAACTGCGTAACACTCCGTATCAAGCTCCAGTCCGTCGCCAAATGAAAGCAAAACAACTGTCGGCCCAGTCT
TATGGTGTGACAAGCTCTACAGCTCGTCGTATCCTGCAAAGTCTGGAGAAAATGTCATCTCCGCTGGCAGATGCC
AAACGTATTCCGTCCATTGTGAGCAGTCCGCTGAATAGCCCGCTGGACCGTAGTGGGATCGATATCACCGACTTC
CAAGCCAAACGTGAGAAAGTGGATAGCCAGTATCCGCCTGTACAACGTCTGATGACCCCGAAACCGGTTTCAATT
GCCACGAATCGTAGCGTGTATTTCAAACCGTCACTGACCCCTAGTGGTGAGTTTCGTAAAACAAATCAGCGTATC
GACAACAAATGCTCTACCGGGTATGAAAAAAACATGACGCCGGGACAGAATCGTGAACAACGTGAATCTGGCTTC
TCTTATCCGAACTTTAGTCTGCCGGCAGCAAATGGTCTGAGTAGCGGTGTAGGAGGTGGTGGGGGCAAAATGCGC
CGTGAACGTCACGCCTTTGTGGCCTCTAAACCTCTGGAAGAAGAAGAGATGGAGGTTCCTGTACTGCCGAAAATC
AGTCTGCCTATCACCTCTTCAAGTCTGCCGACCTTCAACTTTTCTAGTCCGGAAATCACAACCTCTAGCCCGTCA
CCGATTAATAGCAGTCAAGCACTGACGAATAAAGTCCAAATGACCTCACCGAGTTCTACGGGTTCTCCGATGTTC
AAATTCTCTAGTCCTATCGTGAAATCAACCGAAGCGAACGTCCTGCCTCCTTCTAGTATTGGGTTCACCTTTAGC
GTCCCAGTGGCCAAAACAGCTGAACTGAGCGGTAGCAGTAGTACTCTGGAACCGATTATCAGCTCAAGCGCCCAT
CATGTCACTACCGTGAATAGCACAAACTGTAAAAAAACGCCGCCTGAGGACTGTGAAGGACCGTTTCGTCCTGCC
GAAATCCTGAAAGAAGGTTCCGTCCTGGACATTCTGAAATCTCCGGGATTTGCCTCTCCTAAAATCGACTCTGTT
GCCGCTCAACCAACTGCCACATCACCGGTGGTTTATACTCGTCCGGCGATTAGCAGTTTTAGCAGTAGTGGCATC
GGTTTTGGTGAATCCCTGAAAGCTGGCTCATCTTGGCAGTGTGACACCTGCCTGCTGCAAAACAAAGTGACCGAT
AACAAATGTATTGCCTGTCAGGCCGCCAAACTGTCTCCTCGTGATACAGCCAAACAGACCGGCATCGAAACCCCT
AATAAAAGCGGGAAAACGACCCTGTCAGCAAGTGGTACGGGATTTGGGGACAAATTCAAACCTGTGATCGGCACA
TGGGACTGTGACACTTGTCTGGTACAGAACAAACCAGAAGCGATCAAATGTGTGGCCTGTGAAACGCCTAAACCT
GGAACATGTGTGAAACGTGCCCTGACTCTGACTGTTGTGTCAGAAAGCGCCGAAACCATGACGGCAAGCAGCTCA
TCCTGTACTGTGACTACCGGGACTCTGGGATTTGGTGACAAATTCAAACGCCCGATTGGTTCCTGGGAATGCTCC
GTGTGTTGTGTGAGCAATAATGCCGAGGACAACAAATGTGTGTCCTGTATGAGCGAGAAACCTGGCAGCTCTGTT
CCTGCTAGCAGCTCTAGCACAGTTCCTGTTAGTCTGCCTAGTGGTGGTTCTCTGGGTCTGGAAAAATTCAAAAAA
CCTGAAGGAAGCTGGGATTGTGAGCTGTGCCTGGTACAGAATAAAGCGGATAGCACGAAATGTCTGGCCTGTGAG
TCAGCCAAACCAGGGACTAAAAGCGGCTTTAAAGGCTTCGACACGTCGAGCAGTTCTAGTAACAGCGCCGCCTCA
TCATCTTTCAAATTTGGGGTGAGCAGCTCCTCTAGTGGTCCTAGTCAAACACTGACCTCTACCGGAAACTTCAAA
TTCGGCGATCAGGGTGGCTTCAAAATTGGTGTCTCCTCTGATTCGGGTAGCATTAACCCGATGAGTGAGGGGTTC
AAATTCAGCAAACCAATTGGCGATTTCAAATTCGGTGTGTCGTCTGAATCCAAACCTGAAGAAGTCAAAAAAGAC
AGCAAAAACGACAATTTCAAATTCGGCCTGTCTAGTGGTCTGTCTAATCCGGTTAGCCTGACCCCGTTTCAGTTC

GGGGTGTCTAATCTGGGTCAGGAAGAGAAAAAAGAGGAGCTGCCTAAAAGTTCATCTGCCGGGTTCAGTTTTGGT
ACAGGCGTGATCAATAGCACTCCAGCACCAGCCAATACAATCGTGACGAGCGAGAACAAATCGAGCTTCAACCTG
GGGACAATCGAAACGAAAAGCGCCAGTGTAGCGCCATTCACGTGTAAAACCTCCGAGGCAAAAAAAGAAGAGATG
CCGGCCACAAAAGGTGGATTCTCATTCGGCAACGTGGAACCGGCTAGCCTGCCATCAGCAAGCGTGTTTGTACTG
GGCCGTACCGAGGAGAAACAGCAGGAACCTGTTACTAGCACCAGTCTGGTCTTTGGTAAAAAAGCCGACAACGAA
GAACCGAAATGTCAGCCAGTGTTCAGCTTCGGCAATAGCGAACAGACGAAAGACGAAAACAGCAGCAAATCGACG
TTCAGCTTCAGTATGACGAAACCGAGCGAAAAAGAAAGTGAGCAGCCAGCAAAAGCAACGTTCGCCTTTGGAGCA
CAGACATCAACCACAGCCGATCAAGGAGCAGCGAAACCAGTTTTCAGTTTTCTGAATAACAGCTCAAGCAGCAGT
TCTACACCAGCAACCTCAGCAGGTGGTGGGATCTTTGGATCAAGCACCTCATCCAGCAATCCGCCAGTGGCAACA
TTCGTGTTTGGCCAGAGCAGTAATCCGGTGTCATCTTCAGCATTTGGGAATACCGCCGAGAGTAGCACATCACAG
TCTCTGCTGTTCTCACAGGACTCTAAACTGGCAACCACCTCTTCTACTGGTACAGCGGTTACCCCGTTTGTGTTC
GGTCCGGGAGCATCATCCAATAATACCACGACGTCGGGCTTTGGGTTTGGTGCCACGACAACAAGCAGTAGCGCT
GGTAGCAGCTTTGTCTTTGGCACAGGTCCTTCAGCACCTTCTGCTTCACCAGCTTTCGGAGCCAATCAGACTCCG
ACATTCGGACAGTCACAGGGTGCCTCTCAACCAAATCCTCCGGGTTTTGGCAGTATTAGCAGTAGTACCGCCCTG
TTCCCGACCGGTAGTCAACCGGCACCGCCAACATTTGGAACGGTTAGCAGTAGTAGTCAGCCTCCGGTGTTTGGA
CAACAACCGAGCCAGAGCGCCTTCGGATCAGGAACGACCCCTAATAGTAGCAGTGCCTTCCAGTTCGGTAGCAGT
ACCACCAACTTCAACTTCACGAACAATAGCCCGTCAGGTGTGTTCACGTTTGGCGCCAATTCTTCTACCCCAGCG
GCAAGTGCTCAACCTTCAGGCTCAGGTGGATTTCCTTTCAACCAGTCACCAGCAGCGTTTACTGTTGGTTCTAAC
GGGAAAAACGTTTTCAGTAGCAGCGGCACCTCGTTTTCTGGTCGTAAAATCAAAACGGCCGTTCGTCGCCGTAAA
GCGGATCCACCGGTCGCCACGAGAGTGAGCAAGGGCGAGGAGCTGTTCACCGGGGTGGTGCCCATCCTGGTCGAG
CTGGACGGCGACGTAAACGGCCACAAGTTCAGCGTGTCCGGCGAGGGCGAGGGCGATGCCACCTACGGCAAGCTG
ACCCTGAAGTTCATCTGCACCACCGGCAAGCTGCCCGTGCCCTGGCCCACCCTCGTGACCACCCTGACCTACGGC
GTGCAGTGCTTCAGCCGCTACCCCGACCACATGAAGCAGCACGACTTCTTCAAGTCCGCCATGCCCGAAGGCTAC
GTCCAGGAGCGCACCATCTTCTTCAAGGACGACGGCAACTACAAGACCCGCGCCGAGGTGAAGTTCGAGGGCGAC
ACCCTGGTGAACCGCATCGAGCTGAAGGGCATCGACTTCAAGGAGGACGGCAACATCCTGGGGCACAAGCTGGAG
TACAACTACAACAGCCACTAGGTCTATATCATGGCCGACAAGCAGAAGAACGGCATCAAGGTGAACTTCAAGATC
CGCCACAACATCGAGGACGGCAGCGTGCAGCTCGCCGACCACTACCAGCAGAACACCCCCATCGGCGACGGCCCC
GTGCTGCTGCCCGACAACCACTACCTGAGCACCCAGTCCGCCCTGAGCAAAGACCCCAACGAGAAGCGCGATCAC
ATGGTCCTGCTGGAGTTCGTGACCGCCGCCGGGATCACTCTCGGCATGGACGAGCTGTACAAGTAA (SEQ ID
NO:199)
Protein: (X indicates non-canonical amino acid)
MASGAGGVGGGGGGKIRTRRCHQGPIKPYQQGRQQHQGILSRVTESVKNIVPGWLQRYFNKNEDVCSCSTDTSEV
PRWPENKEDHLVYADEESSNITDGRITPEPAVSNTEEPSTTSTASNYPDVLTRPSLHRSHLNFSMLESPALHCQP
STSSAFPIGSSGFSLVKEIKDSTSQHDDDNISTTSGFSSRASDKDITVSKNTSLPPLWSPEAERSHSLSQHTATS
SKKPAFNLSAFGTLSPSLGNSSILKTSQLGDSPFYPGKTTYGGAAAAVRQSKLRNTPYQAPVRRQMKAKQLSAQS
YGVTSSTARRILQSLEKMSSPLADAKRIPSIVSSPLNSPLDRSGIDITDFQAKREKVDSQYPPVQRLMTPKPVSI
ATNRSVYFKPSLTPSGEFRKTNQRIDNKCSTGYEKNMTPGQNREQRESGFSYPNFSLPAANGLSSGVGGGGGKMR
RERHAFVASKPLEEEEMEVPVLPKISLPITSSSLPTFNFSSPEITTSSPSPINSSQALTNKVQMTSPSSTGSPMF
KFSSPIVKSTEANVLPPSSIGFTFSVPVAKTAELSGSSSTLEPIISSSAHHVTTVNSTNCKKTPPEDCEGPFRPA
EILKEGSVLDILKSPGFASPKIDSVAAQPTATSPVVYTRPAISSFSSSGIGFGESLKAGSSWQCDTCLLQNKVTD
NKCIACQAAKLSPRDTAKQTGIETPNKSGKTTLSASGTGFGDKFKPVIGTWDCDTCLVQNKPEAIKCVACETPKP
GTCVKRALTLTVVSESAETMTASSSSCTVTTGTLGFGDKFKRPIGSWECSVCCVSNNAEDNKCVSCMSEKPGSSV
PASSSSTVPVSLPSGGSLGLEKFKKPEGSWDCELCLVQNKADSTKCLACESAKPGTKSGFKGFDTSSSSSNSAAS
SSFKFGVSSSSSGPSQTLTSTGNFKFGDQGGFKIGVSSDSGSINPMSEGFKFSKPIGDFKFGVSSESKPEEVKKD
SKNDNFKFGLSSGLSNPVSLTPFQFGVSNLGQEEKKEELPKSSSAGFSFGTGVINSTPAPANTIVTSENKSSFNL
GTIETKSASVAPFTCKTSEAKKEEMPATKGGFSFGNVEPASLPSASVFVLGRTEEKQQEPVTSTSLVFGKKADNE
EPKCQPVFSFGNSEQTKDENSSKSTFSFSMTKPSEKESEQPAKATFAFGAQTSTTADQGAAKPVFSFLNNSSSSS
STPATSAGGGIFGSSTSSSNPPVATFVFGQSSNPVSSSAFGNTAESSTSQSLLFSQDSKLATTSSTGTAVTPFVF
GPGASSNNTTTSGFGFGATTTSSSAGSSFVFGTGPSAPSASPAFGANQTPTFGQSQGASQPNPPGFGSISSSTAL
FPTGSQPAPPTFGTVSSSSQPPVFGQQPSQSAFGSGTTPNSSSAFQFGSSTTNFNFTNNSPSGVFTFGANSSTPA
ASAQPSGSGGFPFNQSPAAFTVGSNGKNVFSSSGTSFSGRKIKTAVRRRKADPPVATRVSKGEELFTGVVPILVE
LDGDVNGHKFSVSGEGEGDATYGKLTLKFICTTGKLPVPWPTLVTTLTYGVQCFSRYPDHMKQHDFFKSAMPEGY
VQERTIFFKDDGNYKTRAEVKFEGDTLVNRIELKGIDFKEDGNILGHKLEYNYNSHXVYIMADKQKNGIKVNFKI
RHNIEDGSVQLADHYQQNTPIGDGPVLLPDNHYLSTQSALSKDPNEKRDHMVLLEFVTAAGITLGMDELYK
(SEQ ID NO:200)

Nup153-EGFP149TAG-MS2
DNA: (MS2stem-loops and Amber codon underlined)

ATGGCGTCTGGTGCTGGCGGTGTTGGTGGAGGAGGTGGGGGTAAAATTCGTACTCGTCGCTGTCATCAAGGTCCG
ATTAAACCGTATCAGCAGGGACGTCAGCAACATCAGGGTATTCTGAGCCGTGTGACCGAAAGCGTGAAAAACATT
GTGCCGGGTTGGCTGCAACGTTATTTCAACAAAAATGAGGATGTGTGTTCGTGTTCTACCGATACCAGTGAAGTT
CCTCGTTGGCCGGAAAACAAAGAAGATCACCTGGTGTATGCCGATGAAGAATCGAGCAATATCACCGATGGCCGT
ATTACTCCTGAACCGGCGGTTAGTAACACTGAAGAACCGTCAACCACAAGCACAGCATCGAACTATCCAGATGTC
CTGACTCGCCCTTCTCTGCACCGTTCTCACCTGAACTTTAGCATGCTGGAATCACCAGCTCTGCATTGTCAGCCG
TCTACCAGTAGTGCCTTCCCGATTGGCTCTAGTGGCTTTTCGCTGGTCAAAGAGATCAAAGACTCGACCTCTCAA
CATGACGATGATAACATTAGCACGACCTCGGGTTTTAGTAGCCGTGCCTCCGATAAAGACATTACCGTGAGCAAA
AACACCTCTCTGCCGCCTCTGTGGAGTCCTGAAGCCGAACGCTCTCATAGTCTGTCTCAGCACACAGCCACCAGT
TCCAAAAAACCAGCCTTCAACCTGAGCGCCTTTGGTACACTGTCACCGAGCCTGGGAAATTCCTCTATCCTGAAA
ACATCACAGCTGGGCGATAGTCCGTTTTATCCGGGCAAAACGACGTATGGTGGTGCCGCTGCTGCTGTTCGCCAG
TCTAAACTGCGTAACACTCCGTATCAAGCTCCAGTCCGTCGCCAAATGAAAGCAAAACAACTGTCGGCCCAGTCT
TATGGTGTGACAAGCTCTACAGCTCGTCGTATCCTGCAAAGTCTGGAGAAAATGTCATCTCCGCTGGCAGATGCC
AAACGTATTCCGTCCATTGTGAGCAGTCCGCTGAATAGCCCGCTGGACCGTAGTGGGATCGATATCACCGACTTC
CAAGCCAAACGTGAGAAAGTGGATAGCCAGTATCCGCCTGTACAACGTCTGATGACCCCGAAACCGGTTTCAATT
GCCACGAATCGTAGCGTGTATTTCAAACCGTCACTGACCCCTAGTGGTGAGTTTCGTAAAACAAATCAGCGTATC
GACAACAAATGCTCTACCGGGTATGAAAAAAACATGACGCCGGGACAGAATCGTGAACAACGTGAATCTGGCTTC
TCTTATCCGAACTTTAGTCTGCCGGCAGCAAATGGTCTGAGTAGCGGTGTAGGAGGTGGTGGGGGCAAAATGCGC
CGTGAACGTCACGCCTTTGTGGCCTCTAAACCTCTGGAAGAAGAAGAGATGGAGGTTCCTGTACTGCCGAAAATC
AGTCTGCCTATCACCTCTTCAAGTCTGCCGACCTTCAACTTTTCTAGTCCGGAAATCACAACCTCTAGCCCGTCA
CCGATTAATAGCAGTCAAGCACTGACGAATAAAGTCCAAATGACCTCACCGAGTTCTACGGGTTCTCCGATGTTC
AAATTCTCTAGTCCTATCGTGAAATCAACCGAAGCGAACGTCCTGCCTCCTTCTAGTATTGGGTTCACCTTTAGC
GTCCCAGTGGCCAAAACAGCTGAACTGAGCGGTAGCAGTAGTACTCTGGAACCGATTATCAGCTCAAGCGCCCAT
CATGTCACTACCGTGAATAGCACAAACTGTAAAAAAACGCCGCCTGAGGACTGTGAAGGACCGTTTCGTCCTGCC
GAAATCCTGAAAGAAGGTTCCGTCCTGGACATTCTGAAATCTCCGGGATTTGCCTCTCCTAAAATCGACTCTGTT
GCCGCTCAACCAACTGCCACATCACCGGTGGTTTATACTCGTCCGGCGATTAGCAGTTTTAGCAGTAGTGGCATC
GGTTTTGGTGAATCCCTGAAAGCTGGCTCATCTTGGCAGTGTGACACCTGCCTGCTGCAAAACAAAGTGACCGAT
AACAAATGTATTGCCTGTCAGGCCGCCAAACTGTCTCCTCGTGATACAGCCAAACAGACCGGCATCGAAACCCCT
AATAAAAGCGGGAAAACGACCCTGTCAGCAAGTGGTACGGGATTTGGGGACAAATTCAAACCTGTGATCGGCACA
TGGGACTGTGACACTTGTCTGGTACAGAACAAACCAGAAGCGATCAAATGTGTGGCCTGTGAAACGCCTAAACCT
GGAACATGTGTGAAACGTGCCCTGACTCTGACTGTTGTGTCAGAAAGCGCCGAAACCATGACGGCAAGCAGCTCA
TCCTGTACTGTGACTACCGGGACTCTGGGATTTGGTGACAAATTCAAACGCCCGATTGGTTCCTGGGAATGCTCC
GTGTGTTGTGTGAGCAATAATGCCGAGGACAACAAATGTGTGTCCTGTATGAGCGAGAAACCTGGCAGCTCTGTT
CCTGCTAGCAGCTCTAGCACAGTTCCTGTTAGTCTGCCTAGTGGTGGTTCTCTGGGTCTGGAAAAATTCAAAAAA
CCTGAAGGAAGCTGGGATTGTGAGCTGTGCCTGGTACAGAATAAAGCGGATAGCACGAAATGTCTGGCCTGTGAG
TCAGCCAAACCAGGGACTAAAAGCGGCTTTAAAGGCTTCGACACGTCGAGCAGTTCTAGTAACAGCGCCGCCTCA
TCATCTTTCAAATTTGGGGTGAGCAGCTCCTCTAGTGGTCCTAGTCAAACACTGACCTCTACCGGAAACTTCAAA
TTCGGCGATCAGGGTGGCTTCAAAATTGGTGTCTCCTCTGATTCGGGTAGCATTAACCCGATGAGTGAGGGGTTC
AAATTCAGCAAACCAATTGGCGATTTCAAATTCGGTGTGTCGTCTGAATCCAAACCTGAAGAAGTCAAAAAAGAC
AGCAAAAACGACAATTTCAAATTCGGCCTGTCTAGTGGTCTGTCTAATCCGGTTAGCCTGACCCCGTTTCAGTTC
GGGGTGTCTAATCTGGGTCAGGAAGAGAAAAAAGAGGAGCTGCCTAAAAGTTCATCTGCCGGGTTCAGTTTTGGT
ACAGGCGTGATCAATAGCACTCCAGCACCAGCCAATACAATCGTGACGAGCGAGAACAAATCGAGCTTCAACCTG
GGGACAATCGAAACGAAAAGCGCCAGTGTAGCGCCATTCACGTGTAAAACCTCCGAGGCAAAAAAAGAAGAGATG
CCGGCCACAAAAGGTGGATTCTCATTCGGCAACGTGGAACCGGCTAGCCTGCCATCAGCAAGCGTGTTTGTACTG
GGCCGTACCGAGGAGAAACAGCAGGAACCTGTTACTAGCACCAGTCTGGTCTTTGGTAAAAAAGCCGACAACGAA
GAACCGAAATGTCAGCCAGTGTTCAGCTTCGGCAATAGCGAACAGACGAAAGACGAAAACAGCAGCAAATCGACG
TTCAGCTTCAGTATGACGAAACCGAGCGAAAAAGAAAGTGAGCAGCCAGCAAAAGCAACGTTCGCCTTTGGAGCA
CAGACATCAACCACAGCCGATCAAGGAGCAGCGAAACCAGTTTTCAGTTTTCTGAATAACAGCTCAAGCAGCAGT
TCTACACCAGCAACCTCAGCAGGTGGTGGGATCTTTGGATCAAGCACCTCATCCAGCAATCCGCCAGTGGCAACA
TTCGTGTTTGGCCAGAGCAGTAATCCGGTGTCATCTTCAGCATTTGGGAATACCGCCGAGAGTAGCACATCACAG
TCTCTGCTGTTCTCACAGGACTCTAAACTGGCAACCACCTCTTCTACTGGTACAGCGGTTACCCCGTTTGTGTTC
GGTCCGGGAGCATCATCCAATAATACCACGACGTCGGGCTTTGGGTTTGGTGCCACGACAACAAGCAGTAGCGCT
GGTAGCAGCTTTGTCTTTGGCACAGGTCCTTCAGCACCTTCTGCTTCACCAGCTTTCGGAGCCAATCAGACTCCG
ACATTCGGACAGTCACAGGGTGCCTCTCAACCAAATCCTCCGGGTTTTGGCAGTATTAGCAGTAGTACCGCCCTG
TTCCCGACCGGTAGTCAACCGGCACCGCCAACATTTGGAACGGTTAGCAGTAGTAGTCAGCCTCCGGTGTTTGGA
CAACAACCGAGCCAGAGCGCCTTCGGATCAGGAACGACCCCTAATAGTAGCAGTGCCTTCCAGTTCGGTAGCAGT
ACCACCAACTTCAACTTCACGAACAATAGCCCGTCAGGTGTGTTCACGTTTGGCGCCAATTCTTCTACCCCAGCG
GCAAGTGCTCAACCTTCAGGCTCAGGTGGATTTCCTTTCAACCAGTCACCAGCAGCGTTTACTGTTGGTTCTAAC

GGGAAAAACGTTTTCAGTAGCAGCGGCACCTCGTTTTCTGGTCGTAAAATCAAAACGGCCGTTCGTCGCCGTAAA
GCGGATCCACCGGTCGCCACGAGAGTGAGCAAGGGCGAGGAGCTGTTCACCGGGGTGGTGCCCATCCTGGTCGAG
CTGGACGGCGACGTAAACGGCCACAAGTTCAGCGTGTCCGGCGAGGGCGAGGGCGATGCCACCTACGGCAAGCTG
ACCCTGAAGTTCATCTGCACCACCGGCAAGCTGCCCGTGCCCTGGCCCACCCTCGTGACCACCCTGACCTACGGC
GTGCAGTGCTTCAGCCGCTACCCCGACCACATGAAGCAGCACGACTTCTTCAAGTCCGCCATGCCCGAAGGCTAC
GTCCAGGAGCGCACCATCTTCTTCAAGGACGACGGCAACTACAAGACCCGCGCCGAGGTGAAGTTCGAGGGCGAC
ACCCTGGTGAACCGCATCGAGCTGAAGGGCATCGACTTCAAGGAGGACGGCAACATCCTGGGGCACAAGCTGGAG
TACAACTACAACAGCCACTAGGTCTATATCATGGCCGACAAGCAGAAGAACGGCATCAAGGTGAACTTCAAGATC
CGCCACAACATCGAGGACGGCAGCGTGCAGCTCGCCGACCACTACCAGCAGAACACCCCCATCGGCGACGGCCCC
GTGCTGCTGCCCGACAACCACTACCTGAGCACCCAGTCCGCCCTGAGCAAAGACCCCAACGAGAAGCGCGATCAC
ATGGTCCTGCTGGAGTTCGTGACCGCCGCCGGGATCACTCTCGGCATGGACGAGCTGTACAAGTAAAGCGGCCGC
GACTCTAGATCATAATCAGCACATGAGGATCACCCATGTCTGCAGGTCGACTCTAGAAAACATGAGGATCACCCA
TGT (SEQ ID NO:201)
Protein: (X indicates non-canonical amino acid)
MASGAGGVGGGGGGKIRTRRCHQGPIKPYQQGRQQHQGILSRVTESVKNIVPGWLQRYFNKNEDVCSCSTDTSEV
PRWPENKEDHLVYADEESSNITDGRITPEPAVSNTEEPSTTSTASNYPDVLTRPSLHRSHLNFSMLESPALHCQP
STSSAFPIGSSGFSLVKEIKDSTSQHDDDNISTTSGFSSRASDKDITVSKNTSLPPLWSPEAERSHSLSQHTATS
SKKPAFNLSAFGTLSPSLGNSSILKTSQLGDSPFYPGKTTYGGAAAAVRQSKLRNTPYQAPVRRQMKAKQLSAQS
YGVTSSTARRILQSLEKMSSPLADAKRIPSIVSSPLNSPLDRSGIDITDFQAKREKVDSQYPPVQRLMTPKPVSI
ATNRSVYFKPSLTPSGEFRKTNQRIDNKCSTGYEKNMTPGQNREQRESGFSYPNFSLPAANGLSSGVGGGGKMR
RERHAFVASKPLEEEEMEVPVLPKISLPITSSSLPTFNFSSPEITTSSPSPINSSQALTNKVQMTSPSSTGSPMF
KFSSPIVKSTEANVLPPSSIGFTFSVPVAKTAELSGSSSTLEPIISSSAHHVTTVNSTNCKKTPPEDCEGPFRPA
EILKEGSVLDILKSPGFASPKIDSVAAQPTATSPVVYTRPAISSFSSSGIGFGESLKAGSSWQCDTCLLQNKVTD
NKCIACQAAKLSPRDTAKQTGIETPNKSGKTTLSASGTGFGDKFKPVIGTWDCDTCLVQNKPEAIKCVACETPKP
GTCVKRALTLTVVSESAETMTASSSSCTVTTGTLGFGDKFKRPIGSWECSVCCVSNNAEDNKCVSCMSEKPGSSV
PASSSSTVPVSLPSGGSLGLEKFKKPEGSWDCELCLVQNKADSTKCLACESAKPGTKSGFKGFDTSSSSSNSAAS
SSFKFGVSSSSSGPSQTLTSTGNFKFGDQGGFKIGVSSDSGSINPMSEGFKFSKPIGDFKFGVSSESKPEEVKKD
SKNDNFKFGLSSGLSNPVSLTPFQFGVSNLGQEEKKEELPKSSSAGFSFGTGVINSTPAPANTIVTSENKSSFNL
GTIETKSASVAPFTCKTSEAKKEEMPATKGGFSFGNVEPASLPSASVFVLGRTEEKQQEPVTSTSLVFGKKADNE
EPKCQPVFSFGNSEQTKDENSSKSTFSFSMTKPSEKESEQPAKATFAFGAQTSTTADQGAAKPVFSFLNNSSSSS
STPATSAGGGIFGSSTSSSNPPVATFVFGQSSNPVSSSAFGNTAESSTSQSLLFSQDSKLATTSSTGTAVTPFVF
GPGASSNNTTTSGFGFGATTTSSSAGSSFVFGTGPSAPSASPAFGANQTPTFGQSQGASQPNPPGFGSISSSTAL
FPTGSQPAPPTFGTVSSSSQPPVFGQQPSQSAFGSGTTPNSSSAFQFGSSTTNFNFTNNSPSGVFTFGANSSTPA
ASAQPSGSGGFPFNQSPAAFTVGSNGKNVFSSSGTSFSGRKIKTAVRRRKADPPVATRVSKGEELFTGVVPILVE
LDGDVNGHKFSVSGEGEGDATYGKLTLKFICTTGKLPVPWPTLVTTLTYGVQCFSRYPDHMKQHDFFKSAMPEGY
VQERTIFFKDDGNYKTRAEVKFEGDTLVNRIELKGIDFKEDGNILGHKLEYNYNSHXVYIMADKQKNGIKVNFKI
RHNIEDGSVQLADHYQQNTPIGDGPVLLPDNHYLSTQSALSKDPNEKRDHMVLLEFVTAAGITLGMDELYK
(SEQ ID NO:202)

Vim116TAG-mOrange
DNA: (Amber codon underlined)
ATGTCCACCAGGTCCGTGTCCTCGTCCTCCTACCGCAGGATGTTCGGCGGCCCGGGCACCGCGAGCCGGCCGAGC
TCCAGCCGGAGCTACGTGACTACGTCCACCCGCACCTACAGCCTGGGCAGCGCGCTGCGCCCCAGCACCAGCCGC
AGCCTCTACGCCTCGTCCCCGGGCGGCGTGTATGCCACGCGCTCCTCTGCCGTGCGCCTGCGGAGCAGCGTGCCC
GGGGTGCGGCTCCTGCAGGACTCGGTGGACTTCTCGCTGGCCGACGCCATCAACACCGAGTTCAAGAACACCCGC
ACCAACGAGAAGGTGGAGCTGCAGGAGCTGAATGACCGCTTCGCCTAGTACATCGACAAGGTGCGCTTCCTGGAG
CAGCAGAATAAGATCCTGCTGGCCGAGCTCGAGCAGCTCAAGGGCCAAGGCAAGTCGCGCCTGGGGGACCTCTAC
GAGGAGGAGATGCGGGAGCTGCGCCGGCAGGTGGACCAGCTAACCAACGACAAAGCCCGCGTCGAGGTGGAGCGC
GACAACCTGGCCGAGGACATCATGCGCCTCCGGGAGAAATTGCAGGAGGAGATGCTTCAGAGAGAGGAAGCCGAA
AACACCCTGCAATCTTTCAGACAGGATGTTGACAATGCGTCTCTGGCACGTCTTGACCTTGAACGCAAAGTGGAA
TCTTTGCAAGAAGAGATTGCCTTTTTGAAGAAACTCCACGAAGAGGAAATCCAGGAGCTGCAGGCTCAGATTCAG
GAACAGCATGTCCAAATCGATGTGGATGTTTCCAAGCCTGACCTCACGGCTGCCCTGCGTGACGTACGTCAGCAA
TATGAAAGTGTGGCTGCCAAGAACCTGCAGGAGGCAGAAGAATGGTACAAATCCAAGTTTGCTGACCTCTCTGAG
GCTGCCAACCGGAACAATGACGCCCTGCGCCAGGCAAAGCAGGAGTCCACTGAGTACCGGAGACAGGTGCAGTCC
CTCACCTGTGAAGTGGATGCCCTTAAAGGAACCAATGAGTCCCTGGAACGCCAGATGCGTGAAATGGAAGAGAAC
TTTGCCGTTGAAGCTGCTAACTACCAAGACACTATTGGCCGCCTGCAGGATGAGATTCAGAATATGAAGGAGGAA
ATGGCTCGTCACCTTCGTGAATACCAAGACCTGCTCAATGTTAAGATGGCCCTTGACATTGAGATTGCCACCTAC
AGGAAGCTGCTGGAAGGCGAGGAGAGCAGGATTTCTCTGCCTCTTCCAAACTTTTCCTCCCTGAACCTGAGGGAA

ACTAATCTGGATTCACTCCCTCTGGTTGATACCCACTCAAAAAGGACACTTCTGATTAAGACGGTTGAAACTAGA
GATGGACAGGTTATCAACGAAACTTCTCAGCATCACGATGACCTTGAAGGGGATCCACCGGTCGCCACCATGGTG
AGCAAGGGCGAGGAGAATAATATGGCCATCATCAAGGAGTTCATGCGCTTCAAGGTGCGCATGGAGGGCACCGTG
AACGGCCACGAGTTCGAGATCGAGGGCGAGGGCGAGGGCCGCCCCTACGAGGGCTTTCAGACCGCTAAGCTGAAG
GTGACCAAGGGCGGCCCCCTGCCCTTCGCCTGGGACATCCTGTCCCCTCTCTTCACCTACGGCTCCAAGGCCTAC
GTGAAGCACCCCGCCGACATCCCCGACTACTTCAAGCTGTCCTTCCCCGAGGGCTTCAAGTGGGAGCGCGTGATG
AACTACGAGGACGGCGGCGTGGTGACCGTGACCCAGGACTCCTCACTGCAGGACGGCGAGTTCATCTACAAGGTG
AAGATGCGCGGCACCAACTTCCCCTCCGACGGCCCCGTGATGCAGAAGAAGACCATGGGCTGGGAGGCCTCCTCC
GAGCGGATGTACCCCGAGGACGGCGCCCTGAAGGGCGAGATCAGGATGAGGCTGAAGCTGAAGGACGGCGGCCAC
TACACCTCCGAGGTCAAGACCACCTACAAGGCCAAGAAGTCCGTGCAGCTGCCCGGCGCCTACATCGTCGGCATC
AAGCTGGACATCACCTCCCACAACGAGGACTACACCATCGTGGAACAGTACGAACGCGCCGAGGGCCGCCACTCC
ACCGGCGGCATGGACGAGCTGTACAAGTAA (SEQ ID NO:203)
Protein: (X indicates non-canonical amino acid)
MSTRSVSSSSYRRMFGGPGTASRPSSSRSYVTTSTRTYSLGSALRPSTSRSLYASSPGGVYATRSSAVRLRSSVP
GVRLLQDSVDFSLADAINTEFKNTRTNEKVELQELNDRFAXYIDKVRFLEQQNKILLAELEQLKGQGKSRLGDLY
EEEMRELRRQVDQLTNDKARVEVERDNLAEDIMRLREKLQEEMLQREEAENTLQSFRQDVDNASLARLDLERKVE
SLQEEIAFLKKLHEEEIQELQAQIQEQHVQIDVDVSKPDLTAALRDVRQQYESVAAKNLQEAEEWYKSKFADLSE
AANRNNDALRQAKQESTEYRRQVQSLTCEVDALKGTNESLERQMREMEENFAVEAANYQDTIGRLQDEIQNMKEE
MARHLREYQDLLNVKMALDIEIATYRKLLEGEESRISLPLPNFSSLNLRETNLDSLPLVDTHSKRTLLIKTVETR
DGQVINETSQHHDDLEGDPPVATMVSKGEENNMAIIKEFMRFKVRMEGTVNGHEFEIEGEGEGRPYEGFQTAKLK
VTKGGPLPFAWDILSPLFTYGSKAYVKHPADIPDYFKLSFPEGFKWERVMNYEDGGVVTVTQDSSLQDGEFIYKV
KMRGTNFPSDGPVMQKKTMGWEASSERMYPEDGALKGEIRMRLKLKDGGHYTSEVKTTYKAKKSVQLPGAYIVGI
KLDITSHNEDYTIVEQYERAEGRHSTGGMDELYK (SEQ ID NO:204)

Vim116TAG-mOrange-MS2
DNA: (MS2 stem-loops and Amber codon underlined)
ATGTCCACCAGGTCCGTGTCCTCGTCCTCCTACCGCAGGATGTTCGGCGGCCCGGGCACCGCGAGCCGGCCGAGC
TCCAGCCGGAGCTACGTGACTACGTCCACCCGCACCTACAGCCTGGGCAGCGCGCTGCGCCCCAGCACCAGCCGC
AGCCTCTACGCCTCGTCCCCGGGCGGCGTGTATGCCACGCGCTCCTCTGCCGTGCGCCTGCGGAGCAGCGTGCCC
GGGGTGCGGCTCCTGCAGGACTCGGTGGACTTCTCGCTGGCCGACGCCATCAACACCGAGTTCAAGAACACCCGC
ACCAACGAGAAGGTGGAGCTGCAGGAGCTGAATGACCGCTTCGCCTAGTACATCGACAAGGTGCGCTTCCTGGAG
CAGCAGAATAAGATCCTGCTGGCCGAGCTCGAGCAGCTCAAGGGCCAAGGCAAGTCGCGCCTGGGGGACCTCTAC
GAGGAGGAGATGCGGGAGCTGCGCCGGCAGGTGGACCAGCTAACCAACGACAAAGCCCGCGTCGAGGTGGAGCGC
GACAACCTGGCCGAGGACATCATGCGCCTCCGGGAGAAATTGCAGGAGGAGATGCTTCAGAGAGAGGAAGCCGAA
AACACCCTGCAATCTTTCAGACAGGATGTTGACAATGCGTCTCTGGCACGTCTTGACCTTGAACGCAAAGTGGAA
TCTTTGCAAGAAGAGATTGCCTTTTTGAAGAAACTCCACGAAGAGGAAATCCAGGAGCTGCAGGCTCAGATTCAG
GAACAGCATGTCCAAATCGATGTGGATGTTTCCAAGCCTGACCTCACGGCTGCCCTGCGTGACGTACGTCAGCAA
TATGAAAGTGTGGCTGCCAAGAACCTGCAGGAGGCAGAAGAATGGTACAAATCCAAGTTTGCTGACCTCTCTGAG
GCTGCCAACCGGAACAATGACGCCCTGCGCCAGGCAAAGCAGGAGTCCACTGAGTACCGGAGACAGGTGCAGTCC
CTCACCTGTGAAGTGGATGCCCTTAAAGGAACCAATGAGTCCCTGGAACGCCAGATGCGTGAAATGGAAGAGAAC
TTTGCCGTTGAAGCTGCTAACTACCAAGACACTATTGGCCGCCTGCAGGATGAGATTCAGAATATGAAGGAGGAA
ATGGCTCGTCACCTTCGTGAATACCAAGACCTGCTCAATGTTAAGATGGCCCTTGACATTGAGATTGCCACCTAC
AGGAAGCTGCTGGAAGGCGAGGAGAGCAGGATTTCTCTGCCTCTTCCAAACTTTTCCTCCCTGAACCTGAGGGAA
ACTAATCTGGATTCACTCCCTCTGGTTGATACCCACTCAAAAAGGACACTTCTGATTAAGACGGTTGAAACTAGA
GATGGACAGGTTATCAACGAAACTTCTCAGCATCACGATGACCTTGAAGGGGATCCACCGGTCGCCACCATGGTG
AGCAAGGGCGAGGAGAATAATATGGCCATCATCAAGGAGTTCATGCGCTTCAAGGTGCGCATGGAGGGCACCGTG
AACGGCCACGAGTTCGAGATCGAGGGCGAGGGCGAGGGCCGCCCCTACGAGGGCTTTCAGACCGCTAAGCTGAAG
GTGACCAAGGGCGGCCCCCTGCCCTTCGCCTGGGACATCCTGTCCCCTCTCTTCACCTACGGCTCCAAGGCCTAC
GTGAAGCACCCCGCCGACATCCCCGACTACTTCAAGCTGTCCTTCCCCGAGGGCTTCAAGTGGGAGCGCGTGATG
AACTACGAGGACGGCGGCGTGGTGACCGTGACCCAGGACTCCTCACTGCAGGACGGCGAGTTCATCTACAAGGTG
AAGATGCGCGGCACCAACTTCCCCTCCGACGGCCCCGTGATGCAGAAGAAGACCATGGGCTGGGAGGCCTCCTCC
GAGCGGATGTACCCCGAGGACGGCGCCCTGAAGGGCGAGATCAGGATGAGGCTGAAGCTGAAGGACGGCGGCCAC
TACACCTCCGAGGTCAAGACCACCTACAAGGCCAAGAAGTCCGTGCAGCTGCCCGGCGCCTACATCGTCGGCATC
AAGCTGGACATCACCTCCCACAACGAGGACTACACCATCGTGGAACAGTACGAACGCGCCGAGGGCCGCCACTCC
ACCGGCGGCATGGACGAGCTGTACAAGTAAAGCGGCCGCGACTCTAGATCATAATCAGCACATGAGGATCACCCA
TGTCTGCAGGTCGACTCTAGAAAACATGAGGATCACCCATGT (SEQ ID NO:205)
Protein: (X indicates non-canonical amino acid)

MSTRSVSSSSYRRMFGGPGTASRPSSSRSYVTTSTRTYSLGSALRPSTSRSLYASSPGGVYATRSSAVRLRSSVP
GVRLLQDSVDFSLADAINTEFKNTRTNEKVELQELNDRFAXYIDKVRFLEQQNKILLAELEQLKGQGKSRLGDLY
EEEMRELRRQVDQLTNDKARVEVERDNLAEDIMRLREKLQEEMLQREEAENTLQSFRQDVDNASLARLDLERKVE
SLQEEIAFLKKLHEEEIQELQAQIQEQHVQIDVDVSKPDLTAALRDVRQQYESVAAKNLQEAEEWYKSKFADLSE
AANRNNDALRQAKQESTEYRRQVQSLTCEVDALKGTNESLERQMREMEENFAVEAANYQDTIGRLQDEIQNMKEE
MARHLREYQDLLNVKMALDIEIATYRKLLEGEESRISLPLPNFSSLNLRETNLDSLPLVDTHSKRTLLIKTVETR
DGQVINETSQHHDDLEGDPPVATMVSKGEENNMAIIKEFMRFKVRMEGTVNGHEFEIEGEGEGRPYEGFQTAKLK
VTKGGPLPFAWDILSPLFTYGSKAYVKHPADIPDYFKLSFPEGFKWERVMNYEDGGVVTVTQDSSLQDGEFIYKV
KMRGTNFPSDGPVMQKKTMGWEASSERMYPEDGALKGEIRMRLKLKDGGHYTSEVKTTYKAKKSVQLPGAYIVGI
KLDITSHNEDYTIVEQYERAEGRHSTGGMDELYK (SEQ ID NO:206)

INSR676TAG-EGFP
DNA: (Amber codon underlined)
ATGGGCACCGGGGGCCGGCGGGGGGCGGCGGCCGCGCCGCTGCTGGTGGCGGTGGCCGCGCTGCTACTGGGCGCC
GCGGGCCACCTGTACCCCGGAGAGGTGTGTCCCGGCATGGATATCCGGAACAACCTCACTAGGTTGCATGAGCTG
GAGAATTGCTCTGTCATCGAAGGACACTTGCAGATACTCTTGATGTTCAAAACGAGGCCCGAAGATTTCCGAGAC
CTCAGTTTCCCCAAACTCATCATGATCACTGATTACTTGCTGCTCTTCCGGGTCTATGGGCTCGAGAGCCTGAAG
GACCTGTTCCCCAACCTCACGGTCATCCGGGGATCACGACTGTTCTTTAACTACGCGCTGGTCATCTTCGAGATG
GTTCACCTCAAGGAACTCGGCCTCTACAACCTGATGAACATCACCCGGGGTTCTGTCCGCATCGAGAAGAACAAT
GAGCTCTGTTACTTGGCCACTATCGACTGGTCCCGTATCCTGGATTCCGTGGAGGATAATTACATCGTGTTGAAC
AAAGATGACAACGAGGAGTGTGGAGACATCTGTCCGGGTACCGCGAAGGGCAAGACCAACTGCCCCGCCACCGTC
ATCAACGGGCAGTTTGTCGAACGATGTTGGACTCATAGTCACTGCCAGAAAGTTTGCCCGACCATCTGTAAGTCA
CACGGCTGCACCGCCGAAGGCCTCTGTTGCCACAGCGAGTGCCTGGGCAACTGTTCTCAGCCCGACGACCCCACC
AAGTGCGTGGCCTGCCGCAACTTCTACCTGGATGGCAGGTGTGTGGAGACCTGCCCGCCCCCGTACTACCACTTC
CAGGACTGGCGCTGTGTGAACTTCAGCTTCTGCCAGGACCTGCACCACAAATGCAAGAACTCGCGGAGGCAGGGC
TGCCACCAGTACGTCATTCACAACAACAAGTGCATCCCTGAGTGTCCCTCCGGGTACACGATGAATTCCAGCAAC
TTGCTGTGCACCCCATGCCTGGGTCCCTGTCCCAAGGTGTGCCACCTCCTAGAAGGCGAGAAGACCATCGACTCG
GTGACGTCTGCCCAGGAGCTCCGAGGATGCACCGTCATCAACGGGAGTCTGATCATCAACATTCGAGGAGGCAAC
AATCTGGCAGCTGAGCTAGAAGCCAACCTCGGCCTCATTGAAGAAATTTCAGGGTATCTAAAAATCCGCCGATCC
TACGCTCTGGTGTCACTTTCCTTCTTCCGGAAGTTACGTCTGATTCGAGGAGAGACCTTGGAAATTGGGAACTAC
TCCTTCTATGCCTTGGACAACCAGAACCTAAGGCAGCTCTGGGACTGGAGCAAACACAACCTCACCATCACTCAG
GGGAAACTCTTCTTCCACTATAACCCCAAACTCTGCTTGTCAGAAATCCACAAGATGGAAGAAGTTTCAGGAACC
AAGGGGCGCCAGGAGAGAAACGACATTGCCCTGAAGACCAATGGGGACCAGGCATCCTGTGAAAATGAGTTACTT
AAATTTTCTTACATTCGGACATCTTTTGACAAGATCTTGCTGAGATGGGAGCCGTACTGGCCCCCCGACTTCCGA
GACCTCTTGGGGTTCATGCTGTTCTACAAAGAGGCCCCTTATCAGAATGTGACGGAGTTCGACGGGCAGGATGCA
TGTGGTTCCAACAGTTGGACGGTGGTAGACATTGACCCACCCCTGAGGTCCAACGACCCCAAATCACAGAACCAC
CCAGGGTGGCTGATGCGGGGTCTCAAGCCCTGGACCCAGTATGCCATCTTTGTGAAGACCCTGGTCACCTTTTCG
GATGAACGCCGGACCTATGGGGCCAAGAGTGACATCATTTATGTCCAGACAGATGCCACCAACCCCTCTGTGCCC
CTGGATCCAATCTCAGTGTCTAACTCATCATCCCAGATTATTCTGAAGTGGAAACCACCCTCCGACCCCAATGGC
AACATCACCCACTACCTGGTTTTCTGGGAGAGGCAGGCGGAAGACAGTGAGCTGTTCGAGCTGGATTATTGCCTC
TAGGGGCTGAAGCTGCCCTCGAGGACCTGGTCTCCACCATTCGAGTCTGAAGATTCTCAGAAGCACAACCAGAGT
GAGTATGAGGATTCGGCCGGCGAATGCTGCTCCTGTCCAAAGACAGACTCTCAGATCCTGAAGGAGCTGGAGGAG
TCCTCGTTTAGGAAGACGTTTGAGGATTACCTGCACAACGTGGTTTTCGTCCCCAGAAAAACCTCTTCAGGCACT
GGTGCCGAGGACCCTAGGCCATCTCGGAAACGCAGGTCCCTTGGCGATGTTGGGAATGTGACGGTGGCCGTGCCC
ACGGTGGCAGCTTTCCCCAACACTTCCTCGACCAGCGTGCCCACGAGTCCGGAGGAGCACAGGCCTTTTGAGAAG
GTGGTGAACAAGGAGTCGCTGGTCATCTCCGGCTTGCGACACTTCACGGGCTATCGCATCGAGCTGCAGGCTTGC
AACCAGGACACCCCTGAGGAACGGTGCAGTGTGGCAGCCTACGTCAGTGCGAGGACCATGCCTGAAGCCAAGGCT
GATGACATTGTTGGCCCTGTGACGCATGAAATCTTTGAGAACAACGTCGTCCACTTGATGTGGCAGGAGCCGAAG
GAGCCCAATGGTCTGATCGTGCTGTATGAAGTGAGTTATCGGCGATATGGTGATGAGGAGCTGCATCTCTGCGTC
TCCCGCAAGCACTTCGCTCTGGAACGGGGCTGCAGGCTGCGTGGGCTGTCACCGGGGAACTACAGCGTGCGAATC
CGGGCCACCTCCCTTGCGGGCAACGGCTCTTGGACGGAACCCACCTATTTCTACGTGACAGACTATTTAGACGTC
CCGTCAAATATTGCAAAAATTATCATCGGCCCCCTCATCTTTGTCTTTCTCTTCAGTGTTGTGATTGGAAGTATT
TATCTATTCCTGAGAAAGAGGCAGCCAGATGGGCCGCTGGGACCGCTTTACGCTTCTTCAAACCCTGAGTATCTC
AGTGCCAGTGATGTGTTTCCATGCTCTGTGTACGTGCCGGACGAGTGGGAGGTGTCTCGAGAGAAGATCACCCTC
CTTCGAGAGCTGGGGCAGGGCTCCTTCGGCATGGTGTATGAGGGCAATGCCAGGGACATCATCAAGGGTGAGGCA
GAGACCCGCGTGGCGGTGAAGACGGTCAACGAGTCAGCCAGTCTCCGAGAGCGGATTGAGTTCCTCAATGAGGCC
TCGGTCATGAAGGGCTTCACCTGCCATCACGTGGTGCGCCTCCTGGGAGTGGTGTCCAAGGGCCAGCCCACGCTG
GTGGTGATGGAGCTGATGGCTCACGGAGACCTGAAGAGCTACCTCCGTTCTCTGCGGCCAGAGGCTGAGAATAAT

CCTGGCCGCCCTCCCCCTACCCTTCAAGAGATGATTCAGATGGCGGCAGAGATTGCTGACGGGATGGCCTACCTG
AACGCCAAGAAGTTTGTGCATCGGGACCTGGCAGCGAGAAACTGCATGGTCGCCCATGATTTTACTGTCAAAATT
GGAGACTTTGGAATGACCAGAGACATCTATGAAACGGATTACTACCGGAAAGGGGGCAAGGGTCTGCTCCCTGTA
CGGTGGATGGCACCGGAGTCCCTGAAGGATGGGGTCTTCACCACTTCTTCTGACATGTGGTCCTTTGGCGTGGTC
CTTTGGGAAATCACCAGCTTGGCAGAACAGCCTTACCAAGGCCTGTCTAATGAACAGGTGTTGAAATTTGTCATG
GATGGAGGGTATCTGGATCAACCCGACAACTGTCCAGAGAGAGTCACTGACCTCATGCGCATGTGCTGGCAATTC
AACCCCAACATGAGGCCAACCTTCCTGGAGATTGTCAACCTGCTCAAGGACGACCTGCACCCCAGCTTTCCAGAG
GTGTCGTTCTTCCACAGCGAGGAGAACAAGGCTCCCGAGAGTGAGGAGCTGGAGATGGAGTTTGAGGACATGGAG
AATGTGCCCCTGGACCGTTCCTCGCACTGTCAGAGGGAGGAGGCGGGGGGCCGGGATGGAGGGTCCTCGCTGGGT
TTCAAGCGGAGCTACGAGGAACACATCCCTTACACACACATGAACGGAGGCAAGAAAAACGGGCGGATTCTGACC
TTGCCTCGGTCCAATCCTTCCTGGGCCCGGGATCCACCGGTCGCCACCATGGTGAGCAAGGGCGAGGAGCTGTTC
ACCGGGGTGGTGCCCATCCTGGTCGAGCTGGACGGCGACGTAAACGGCCACAAGTTCAGCGTGTCCGGCGAGGGC
GAGGGCGATGCCACCTACGGCAAGCTGACCCTGAAGTTCATCTGCACCACCGGCAAGCTGCCCGTGCCCTGGCCC
ACCCTCGTGACCACCCTGACCTACGGCGTGCAGTGCTTCAGCCGCTACCCCGACCACATGAAGCAGCACGACTTC
TTCAAGTCCGCCATGCCCGAAGGCTACGTCCAGGAGCGCACCATCTTCTTCAAGGACGACGGCAACTACAAGACC
CGCGCCGAGGTGAAGTTCGAGGGCGACACCCTGGTGAACCGCATCGAGCTGAAGGGCATCGACTTCAAGGAGGAC
GGCAACATCCTGGGGCACAAGCTGGAGTACAACTACAACAGCCACAACGTCTATATCATGGCCGACAAGCAGAAG
AACGGCATCAAGGTGAACTTCAAGATCCGCCACAACATCGAGGACGGCAGCGTGCAGCTCGCCGACCACTACCAG
CAGAACACCCCCATCGGCGACGGCCCCGTGCTGCTGCCCGACAACCACTACCTGAGCACCCAGTCCGCCCTGAGC
AAAGACCCCAACGAGAAGCGCGATCACATGGTCCTGCTGGAGTTCGTGACCGCCGCCGGGATCACTCTCGGCATG
GACGAGCTGTACAAGTAA (SEQ ID NO:207)
Protein: (X indicates non-canonical amino acid)
MGTGGRRGAAAAPLLVAVAALLLGAAGHLYPGEVCPGMDIRNNLTRLHELENCSVIEGHLQILLMFKTRPEDFRD
LSFPKLIMITDYLLLFRVYGLESLKDLFPNLTVIRGSRLFFNYALVIFEMVHLKELGLYNLMNITRGSVRIEKNN
ELCYLATIDWSRILDSVEDNYIVLNKDDNEECGDICPGTAKGKTNCPATVINGQFVERCWTHSHCQKVCPTICKS
HGCTAEGLCCHSECLGNCSQPDDPTKCVACRNFYLDGRCVETCPPPYYHFQDWRCVNFSFCQDLHHKCKNSRRQG
CHQYVIHNNKCIPECPSGYTMNSSNLLCTPCLGPCPKVCHLLEGEKTIDSVTSAQELRGCTVINGSLIINIRGGN
NLAAELEANLGLIEEISGYLKIRRSYALVSLSFFRKLRLIRGETLEIGNYSFYALDNQNLRQLWDWSKHNLTITQ
GKLFFHYNPKLCLSEIHKMEEVSGTKGRQERNDIALKTNGDQASCENELLKFSYIRTSFDKILLRWEPYWPPDFR
DLLGFMLFYKEAPYQNVTEFDGQDACGSNSWTVVDIDPPLRSNDPKSQNHPGWLMRGLKPWTQYAIFVKTLVTFS
DERRTYGAKSDIIYVQTDATNPSVPLDPISVSNSSSQIILKWKPPSDPNGNITHYLVFWERQAEDSELFELDYCL
XGLKLPSRTWSPPFESEDSQKHNQSEYEDSAGECCSCPKTDSQILKELEESSFRKTFEDYLHNVVFVPRKTSSGT
GAEDPRPSRKRRSLGDVGNVTVAVPTVAAFPNTSSTSVPTSPEEHRPFEKVVNKESLVISGLRHFTGYRIELQAC
NQDTPEERCSVAAYVSARTMPEAKADDIVGPVTHEIFENNVVHLMWQEPKEPNGLIVLYEVSYRRYGDEELHLCV
SRKHFALERGCRLRGLSPGNYSVRIRATSLAGNGSWTEPTYFYVTDYLDVPSNIAKIIIGPLIFVFLFSVVIGSI
YLFLRKRQPDGPLGPLYASSNPEYLSASDVFPCSVYVPDEWEVSREKITLLRELGQGSFGMVYEGNARDIIKGEA
ETRVAVKTVNESASLRERIEFLNEASVMKGFTCHHVVRLLGVVSKGQPTLVVMELMAHGDLKSYLRSLRPEAENN
PGRPPPTLQEMIQMAAEIADGMAYLNAKKFVHRDLAARNCMVAHDFTVKIGDFGMTRDIYETDYYRKGGKGLLPV
RWMAPESLKDGVFTTSSDMWSFGVVLWEITSLAEQPYQGLSNEQVLKFVMDGGYLDQPDNCPERVTDLMRMCWQF
NPNMRPTFLEIVNLLKDDLHPSFPEVSFFHSEENKAPESEELEMEFEDMENVPLDRSSHCQREEAGGRDGGSSLG
FKRSYEEHIPYTHMNGGKKNGRILTLPRSNPSWARDPPVATMVSKGEELFTGVVPILVELDGDVNGHKFSVSGEG
EGDATYGKLTLKFICTTGKLPVPWPTLVTTLTYGVQCFSRYPDHMKQHDFFKSAMPEGYVQERTIFFKDDGNYKT
RAEVKFEGDTLVNRIELKGIDFKEDGNILGHKLEYNYNSHNVYIMADKQKNGIKVNFKIRHNIEDGSVQLADHYQ
QNTPIGDGPVLLPDNHYLSTQSALSKDPNEKRDHMVLLEFVTAAGITLGMDELYK (SEQ ID NO:208)

INSR676TAG-EGFP-MS2
DNA: (MS2 stem-loops and Amber codon underlined)
ATGGGCACCGGGGGCCGGCGGGGGGCGGCGGCCGCGCCGCTGCTGGTGGCGGTGGCCGCGCTGCTACTGGGCGCC
GCGGGCCACCTGTACCCCGGAGAGGTGTGTCCCGGCATGGATATCCGGAACAACCTCACTAGGTTGCATGAGCTG
GAGAATTGCTCTGTCATCGAAGGACACTTGCAGATACTCTTGATGTTCAAAACGAGGCCCGAAGATTTCCGAGAC
CTCAGTTTCCCCAAACTCATCATGATCACTGATTACTTGCTGCTCTTCCGGGTCTATGGGCTCGAGAGCCTGAAG
GACCTGTTCCCCAACCTCACGGTCATCCGGGGATCACGACTGTTCTTTAACTACGCGCTGGTCATCTTCGAGATG
GTTCACCTCAAGGAACTCGGCCTCTACAACCTGATGAACATCACCCGGGGTTCTGTCCGCATCGAGAAGAACAAT
GAGCTCTGTTACTTGGCCACTATCGACTGGTCCCGTATCCTGGATTCCGTGGAGGATAATTACATCGTGTTGAAC
AAAGATGACAACGAGGAGTGTGGAGACATCTGTCCGGGTACCGCGAAGGGCAAGACCAACTGCCCCGCCACCGTC
ATCAACGGGCAGTTTGTCGAACGATGTTGGACTCATAGTCACTGCCAGAAAGTTTGCCCGACCATCTGTAAGTCA
CACGGCTGCACCGCCGAAGGCCTCTGTTGCCACAGCGAGTGCCTGGGCAACTGTTCTCAGCCCGACGACCCCACC
AAGTGCGTGGCCTGCCGCAACTTCTACCTGGATGGCAGGTGTGTGGAGACCTGCCCGCCCCCGTACTACCACTTC

CAGGACTGGCGCTGTGTGAACTTCAGCTTCTGCCAGGACCTGCACCACAAATGCAAGAACTCGCGGAGGCAGGGC
TGCCACCAGTACGTCATTCACAACAACAAGTGCATCCCTGAGTGTCCCTCCGGGTACACGATGAATTCCAGCAAC
TTGCTGTGCACCCCATGCCTGGGTCCCTGTCCCAAGGTGTGCCACCTCCTAGAAGGCGAGAAGACCATCGACTCG
GTGACGTCTGCCCAGGAGCTCCGAGGATGCACCGTCATCAACGGGAGTCTGATCATCAACATTCGAGGAGGCAAC
AATCTGGCAGCTGAGCTAGAAGCCAACCTCGGCCTCATTGAAGAAATTTCAGGGTATCTAAAAATCCGCCGATCC
TACGCTCTGGTGTCACTTTCCTTCTTCCGGAAGTTACGTCTGATTCGAGGAGAGACCTTGGAAATTGGGAACTAC
TCCTTCTATGCCTTGGACAACCAGAACCTAAGGCAGCTCTGGGACTGGAGCAAACACAACCTCACCATCACTCAG
GGGAAACTCTTCTTCCACTATAACCCCAAACTCTGCTTGTCAGAAATCCACAAGATGGAAGAAGTTTCAGGAACC
AAGGGGCGCCAGGAGAGAAACGACATTGCCCTGAAGACCAATGGGGACCAGGCATCCTGTGAAAATGAGTTACTT
AAATTTTCTTACATTCGGACATCTTTTGACAAGATCTTGCTGAGATGGGAGCCGTACTGGCCCCCCGACTTCCGA
GACCTCTTGGGGTTCATGCTGTTCTACAAAGAGGCCCCTTATCAGAATGTGACGGAGTTCGACGGGCAGGATGCA
TGTGGTTCCAACAGTTGGACGGTGGTAGACATTGACCCACCCCTGAGGTCCAACGACCCCAAATCACAGAACCAC
CCAGGGTGGCTGATGCGGGGTCTCAAGCCCTGGACCCAGTATGCCATCTTTGTGAAGACCCTGGTCACCTTTTCG
GATGAACGCCGGACCTATGGGGCCAAGAGTGACATCATTTATGTCCAGACAGATGCCACCAACCCCTCTGTGCCC
CTGGATCCAATCTCAGTGTCTAACTCATCATCCCAGATTATTCTGAAGTGGAAACCACCCTCCGACCCCAATGGC
AACATCACCCACTACCTGGTTTTCTGGGAGAGGCAGGCGGAAGACAGTGAGCTGTTCGAGCTGGATTATTGCCTC
TAGGGGCTGAAGCTGCCCTCGAGGACCTGGTCTCCACCATTCGAGTCTGAAGATTCTCAGAAGCACAACCAGAGT
GAGTATGAGGATTCGGCCGGCGAATGCTGCTCCTGTCCAAAGACAGACTCTCAGATCCTGAAGGAGCTGGAGGAG
TCCTCGTTTAGGAAGACGTTTGAGGATTACCTGCACAACGTGGTTTTCGTCCCCAGAAAAACCTCTTCAGGCACT
GGTGCCGAGGACCCTAGGCCATCTCGGAAACGCAGGTCCCTTGGCGATGTTGGGAATGTGACGGTGGCCGTGCCC
ACGGTGGCAGCTTTCCCCAACACTTCCTCGACCAGCGTGCCCACGAGTCCGGAGGAGCACAGGCCTTTTGAGAAG
GTGGTGAACAAGGAGTCGCTGGTCATCTCCGGCTTGCGACACTTCACGGGCTATCGCATCGAGCTGCAGGCTTGC
AACCAGGACACCCCTGAGGAACGGTGCAGTGTGGCAGCCTACGTCAGTGCGAGGACCATGCCTGAAGCCAAGGCT
GATGACATTGTTGGCCCTGTGACGCATGAAATCTTTGAGAACAACGTCGTCCACTTGATGTGGCAGGAGCCGAAG
GAGCCCAATGGTCTGATCGTGCTGTATGAAGTGAGTTATCGGCGATATGGTGATGAGGAGCTGCATCTCTGCGTC
TCCCGCAAGCACTTCGCTCTGGAACGGGGCTGCAGGCTGCGTGGGCTGTCACCGGGGAACTACAGCGTGCGAATC
CGGGCCACCTCCCTTGCGGGCAACGGCTCTTGGACGGAACCCACCTATTTCTACGTGACAGACTATTTAGACGTC
CCGTCAAATATTGCAAAAATTATCATCGGCCCCCTCATCTTTGTCTTTCTCTTCAGTGTTGTGATTGGAAGTATT
TATCTATTCCTGAGAAAGAGGCAGCCAGATGGGCCGCTGGGACCGCTTTACGCTTCTTCAAACCCTGAGTATCTC
AGTGCCAGTGATGTGTTTCCATGCTCTGTGTACGTGCCGGACGAGTGGGAGGTGTCTCGAGAGAAGATCACCCTC
CTTCGAGAGCTGGGGCAGGGCTCCTTCGGCATGGTGTATGAGGGCAATGCCAGGGACATCATCAAGGGTGAGGCA
GAGACCCGCGTGGCGGTGAAGACGGTCAACGAGTCAGCCAGTCTCCGAGAGCGGATTGAGTTCCTCAATGAGGCC
TCGGTCATGAAGGGCTTCACCTGCCATCACGTGGTGCGCCTCCTGGGAGTGGTGTCCAAGGGCCAGCCCACGCTG
GTGGTGATGGAGCTGATGGCTCACGGAGACCTGAAGAGCTACCTCCGTTCTCTGCGGCCAGAGGCTGAGAATAAT
CCTGGCCGCCCTCCCCCTACCCTTCAAGAGATGATTCAGATGGCGGCAGAGATTGCTGACGGGATGGCCTACCTG
AACGCCAAGAAGTTTGTGCATCGGGACCTGGCAGCGAGAAACTGCATGGTCGCCCATGATTTTACTGTCAAAATT
GGAGACTTTGGAATGACCAGAGACATCTATGAAACGGATTACTACCGGAAAGGGGGCAAGGGTCTGCTCCCTGTA
CGGTGGATGGCACCGGAGTCCCTGAAGGATGGGGTCTTCACCACTTCTTCTGACATGTGGTCCTTTGGCGTGGTC
CTTTGGGAAATCACCAGCTTGGCAGAACAGCCTTACCAAGGCCTGTCTAATGAACAGGTGTTGAAATTTGTCATG
GATGGAGGGTATCTGGATCAACCCGACAACTGTCCAGAGAGAGTCACTGACCTCATGCGCATGTGCTGGCAATTC
AACCCCAACATGAGGCCAACCTTCCTGGAGATTGTCAACCTGCTCAAGGACGACCTGCACCCCAGCTTTCCAGAG
GTGTCGTTCTTCCACAGCGAGGAGAACAAGGCTCCCGAGAGTGAGGAGCTGGAGATGGAGTTTGAGGACATGGAG
AATGTGCCCCTGGACCGTTCCTCGCACTGTCAGAGGGAGGAGGCGGGGGGCCGGGATGGAGGGTCCTCGCTGGGT
TTCAAGCGGAGCTACGAGGAACACATCCCTTACACACACATGAACGGAGGCAAGAAAAACGGGCGGATTCTGACC
TTGCCTCGGTCCAATCCTTCCTGGGCCCGGGATCCACCGGTCGCCACCATGGTGAGCAAGGGCGAGGAGCTGTTC
ACCGGGGTGGTGCCCATCCTGGTCGAGCTGGACGGCGACGTAAACGGCCACAAGTTCAGCGTGTCCGGCGAGGGC
GAGGGCGATGCCACCTACGGCAAGCTGACCCTGAAGTTCATCTGCACCACCGGCAAGCTGCCCGTGCCCTGGCCC
ACCCTCGTGACCACCCTGACCTACGGCGTGCAGTGCTTCAGCCGCTACCCCGACCACATGAAGCAGCACGACTTC
TTCAAGTCCGCCATGCCCGAAGGCTACGTCCAGGAGCGCACCATCTTCTTCAAGGACGACGGCAACTACAAGACC
CGCGCCGAGGTGAAGTTCGAGGGCGACACCCTGGTGAACCGCATCGAGCTGAAGGGCATCGACTTCAAGGAGGAC
GGCAACATCCTGGGGCACAAGCTGGAGTACAACTACAACAGCCACAACGTCTATATCATGGCCGACAAGCAGAAG
AACGGCATCAAGGTGAACTTCAAGATCCGCCACAACATCGAGGACGGCAGCGTGCAGCTCGCCGACCACTACCAG
CAGAACACCCCCATCGGCGACGGCCCCGTGCTGCTGCCCGACAACCACTACCTGAGCACCCAGTCCGCCCTGAGC
AAAGACCCCAACGAGAAGCGCGATCACATGGTCCTGCTGGAGTTCGTGACCGCCGCCGGGATCACTCTCGGCATG
GACGAGCTGTACAAGTAAAGCGGCCGCGCGGCCGCGACTCTAGATCATAATCAGCACATGAGGATCACCCATGTC
TGCAGGTCGACTCTAGAAAACATGAGGATCACCCATGT (SEQ ID NO:209)
Protein: (X indicates non-canonical amino acid)

MGTGGRRGAAAAPLLVAVAALLLGAAGHLYPGEVCPGMDIRNNLTRLHELENCSVIEGHLQILLMFKTRPEDFRD
LSFPKLIMITDYLLLFRVYGLESLKDLFPNLTVIRGSRLFFNYALVIFEMVHLKELGLYNLMNITRGSVRIEKNN
ELCYLATIDWSRILDSVEDNYIVLNKDDNEECGDICPGTAKGKTNCPATVINGQFVERCWTHSHCQKVCPTICKS
HGCTAEGLCCHSECLGNCSQPDDPTKCVACRNFYLDGRCVETCPPPYYHFQDWRCVNFSFCQDLHHKCKNSRRQG
CHQYVIHNNKCIPECPSGYTMNSSNLLCTPCLGPCPKVCHLLEGEKTIDSVTSAQELRGCTVINGSLIINIRGGN
NLAAELEANLGLIEEISGYLKIRRSYALVSLSFFRKLRLIRGETLEIGNYSFYALDNQNLRQLWDWSKHNLTITQ
GKLFFHYNPKLCLSEIHKMEEVSGTKGRQERNDIALKTNGDQASCENELLKFSYIRTSFDKILLRWEPYWPPDFR
DLLGFMLFYKEAPYQNVTEFDGQDACGSNSWTVVDIDPPLRSNDPKSQNHPGWLMRGLKPWTQYAIFVKTLVTFS
DERRTYGAKSDIIYVQTDATNPSVPLDPISVSNSSSQIILKWKPPSDPNGNITHYLVFWERQAEDSELFELDYCL
XGLKLPSRTWSPPFESEDSQKHNQSEYEDSAGECCSCPKTDSQILKELEESSFRKTFEDYLHNVVFVPRKTSSGT
GAEDPRPSRKRRSLGDVGNVTVAVPTVAAFPNTSSTSVPTSPEEHRPFEKVVNKESLVISGLRHFTGYRIELQAC
NQDTPEERCSVAAYVSARTMPEAKADDIVGPVTHEIFENNVVHLMWQEPKEPNGLIVLYEVSYRRYGDEELHLCV
SRKHFALERGCRLRGLSPGNYSVRIRATSLAGNGSWTEPTYFYVTDYLDVPSNIAKIIIGPLIFVFLFSVVIGSI
YLFLRKRQPDGPLGPLYASSNPEYLSASDVFPCSVYVPDEWEVSREKITLLRELGQGSFGMVYEGNARDIIKGEA
ETRVAVKTVNESASLRERIEFLNEASVMKGFTCHHVVRLLGVVSKGQPTLVVMELMAHGDLKSYLRSLRPEAENN
PGRPPPTLQEMIQMAAEIADGMAYLNAKKFVHRDLAARNCMVAHDFTVKIGDFGMTRDIYETDYYRKGGKGLLPV
RWMAPESLKDGVFTTSSDMWSFGVVLWEITSLAEQPYQGLSNEQVLKFVMDGGYLDQPDNCPERVTDLMRMCWQF
NPNMRPTFLEIVNLLKDDLHPSFPEVSFFHSEENKAPESEELEMEFEDMENVPLDRSSHCQREEAGGRDGGSSLG
FKRSYEEHIPYTHMNGGKKNGRILTLPRSNPSWARDPPVATMVSKGEELFTGVVPILVELDGDVNGHKFSVSGEG
EGDATYGKLTLKFICTTGKLPVPWPTLVTTLTYGVQCFSRYPDHMKQHDFFKSAMPEGYVQERTIFFKDDGNYKT
RAEVKFEGDTLVNRIELKGIDFKEDGNILGHKLEYNYNSHNVYIMADKQKNGIKVNFKIRHNIEDGSVQLADHYQ
QNTPIGDGPVLLPDNHYLSTQSALSKDPNEKRDHMVLLEFVTAAGITLGMDELYK (SEQ ID NO:210)

INSR676TAG-mOrange-MS2
DNA: (MS2 stem-loops and Amber codon underlined)
ATGGGCACCGGGGGCCGGCGGGGGGCGGCGGCCGCGCCGCTGCTGGTGGCGGTGGCCGCGCTGCTACTGGGCGCC
GCGGGCCACCTGTACCCCGGAGAGGTGTGTCCCGGCATGGATATCCGGAACAACCTCACTAGGTTGCATGAGCTG
GAGAATTGCTCTGTCATCGAAGGACACTTGCAGATACTCTTGATGTTCAAAACGAGGCCCGAAGATTTCCGAGAC
CTCAGTTTCCCCAAACTCATCATGATCACTGATTACTTGCTGCTCTTCCGGGTCTATGGGCTCGAGAGCCTGAAG
GACCTGTTCCCCAACCTCACGGTCATCCGGGGATCACGACTGTTCTTTAACTACGCGCTGGTCATCTTCGAGATG
GTTCACCTCAAGGAACTCGGCCTCTACAACCTGATGAACATCACCCGGGGTTCTGTCCGCATCGAGAAGAACAAT
GAGCTCTGTTACTTGGCCACTATCGACTGGTCCCGTATCCTGGATTCCGTGGAGGATAATTACATCGTGTTGAAC
AAAGATGACAACGAGGAGTGTGGAGACATCTGTCCGGGTACCGCGAAGGGCAAGACCAACTGCCCCGCCACCGTC
ATCAACGGGCAGTTTGTCGAACGATGTTGGACTCATAGTCACTGCCAGAAAGTTTGCCCGACCATCTGTAAGTCA
CACGGCTGCACCGCCGAAGGCCTCTGTTGCCACAGCGAGTGCCTGGGCAACTGTTCTCAGCCCGACGACCCCACC
AAGTGCGTGGCCTGCCGCAACTTCTACCTGGATGGCAGGTGTGTGGAGACCTGCCCGCCCCCGTACTACCACTTC
CAGGACTGGCGCTGTGTGAACTTCAGCTTCTGCCAGGACCTGCACCACAAATGCAAGAACTCGCGGAGGCAGGGC
TGCCACCAGTACGTCATTCACAACAACAAGTGCATCCCTGAGTGTCCCTCCGGGTACACGATGAATTCCAGCAAC
TTGCTGTGCACCCCATGCCTGGGTCCCTGTCCCAAGGTGTGCCACCTCCTAGAAGGCGAGAAGACCATCGACTCG
GTGACGTCTGCCCAGGAGCTCCGAGGATGCACCGTCATCAACGGGAGTCTGATCATCAACATTCGAGGAGGCAAC
AATCTGGCAGCTGAGCTAGAAGCCAACCTCGGCCTCATTGAAGAAATTTCAGGGTATCTAAAAATCCGCCGATCC
TACGCTCTGGTGTCACTTTCCTTCTTCCGGAAGTTACGTCTGATTCGAGGAGAGACCTTGGAAATTGGGAACTAC
TCCTTCTATGCCTTGGACAACCAGAACCTAAGGCAGCTCTGGGACTGGAGCAAACACAACCTCACCATCACTCAG
GGGAAACTCTTCTTCCACTATAACCCCAAACTCTGCTTGTCAGAAATCCACAAGATGGAAGAAGTTTCAGGAACC
AAGGGGCGCCAGGAGAGAAACGACATTGCCCTGAAGACCAATGGGGACCAGGCATCCTGTGAAAATGAGTTACTT
AAATTTTCTTACATTCGGACATCTTTTGACAAGATCTTGCTGAGATGGGAGCCGTACTGGCCCCCCGACTTCCGA
GACCTCTTGGGGTTCATGCTGTTCTACAAAGAGGCCCCTTATCAGAATGTGACGGAGTTCGACGGGCAGGATGCA
TGTGGTTCCAACAGTTGGACGGTGGTAGACATTGACCCACCCCTGAGGTCCAACGACCCCAAATCACAGAACCAC
CCAGGGTGGCTGATGCGGGGTCTCAAGCCCTGGACCCAGTATGCCATCTTTGTGAAGACCCTGGTCACCTTTTCG
GATGAACGCCGGACCTATGGGGCCAAGAGTGACATCATTTATGTCCAGACAGATGCCACCAACCCCTCTGTGCCC
CTGGATCCAATCTCAGTGTCTAACTCATCATCCCAGATTATTCTGAAGTGGAAACCACCCTCCGACCCCAATGGC
AACATCACCCACTACCTGGTTTTCTGGGAGAGGCAGGCGGAAGACAGTGAGCTGTTCGAGCTGGATTATTGCCTC
TAGGGGCTGAAGCTGCCCTCGAGGACCTGGTCTCCACCATTCGAGTCTGAAGATTCTCAGAAGCACAACCAGAGT
GAGTATGAGGATTCGGCCGGCGAATGCTGCTCCTGTCCAAAGACAGACTCTCAGATCCTGAAGGAGCTGGAGGAG
TCCTCGTTTAGGAAGACGTTTGAGGATTACCTGCACAACGTGGTTTTCGTCCCCAGGCCATCTCGGAAACGCAGG
TCCCTTGGCGATGTTGGGAATGTGACGGTGGCCGTGCCCACGGTGGCAGCTTTCCCCAACACTTCCTCGACCAGC
GTGCCCACGAGTCCGGAGGAGCACAGGCCTTTTGAGAAGGTGGTGAACAAGGAGTCGCTGGTCATCTCCGGCTTG
CGACACTTCACGGGCTATCGCATCGAGCTGCAGGCTTGCAACCAGGACACCCCTGAGGAACGGTGCAGTGTGGCA

GCCTACGTCAGTGCGAGGACCATGCCTGAAGCCAAGGCTGATGACATTGTTGGCCCTGTGACGCATGAAATCTTT
GAGAACAACGTCGTCCACTTGATGTGGCAGGAGCCGAAGGAGCCCAATGGTCTGATCGTGCTGTATGAAGTGAGT
TATCGGCGATATGGTGATGAGGAGCTGCATCTCTGCGTCTCCCGCAAGCACTTCGCTCTGGAACGGGGCTGCAGG
CTGCGTGGGCTGTCACCGGGGAACTACAGCGTGCGAATCCGGGCCACCTCCCTTGCGGGCAACGGCTCTTGGACG
GAACCCACCTATTTCTACGTGACAGACTATTTAGACGTCCCGTCAAATATTGCAAAAATTATCATCGGCCCCCTC
ATCTTTGTCTTTCTCTTCAGTGTTGTGATTGGAAGTATTTATCTATTCCTGAGAAAGAGGCAGCCAGATGGGCCG
CTGGGACCGCTTTACGCTTCTTCAAACCCTGAGTATCTCAGTGCCAGTGATGTGTTTCCATGCTCTGTGTACGTG
CCGGACGAGTGGGAGGTGTCTCGAGAGAAGATCACCCTCCTTCGAGAGCTGGGGCAGGGCTCCTTCGGCATGGTG
TATGAGGGCAATGCCAGGGACATCATCAAGGGTGAGGCAGAGACCCGCGTGGCGGTGAAGACGGTCAACGAGTCA
GCCAGTCTCCGAGAGCGGATTGAGTTCCTCAATGAGGCCTCGGTCATGAAGGGCTTCACCTGCCATCACGTGGTG
CGCCTCCTGGGAGTGGTGTCCAAGGGCCAGCCCACGCTGGTGGTGATGGAGCTGATGGCTCACGGAGACCTGAAG
AGCTACCTCCGTTCTCTGCGGCCAGAGGCTGAGAATAATCCTGGCCGCCCTCCCCCTACCCTTCAAGAGATGATT
CAGATGGCGGCAGAGATTGCTGACGGGATGGCCTACCTGAACGCCAAGAAGTTTGTGCATCGGGACCTGGCAGCG
AGAAACTGCATGGTCGCCCATGATTTTACTGTCAAAATTGGAGACTTTGGAATGACCAGAGACATCTATGAAACG
GATTACTACCGGAAAGGGGGCAAGGGTCTGCTCCCTGTACGGTGGATGGCACCGGAGTCCCTGAAGGATGGGGTC
TTCACCACTTCTTCTGACATGTGGTCCTTTGGCGTGGTCCTTTGGGAAATCACCAGCTTGGCAGAACAGCCTTAC
CAAGGCCTGTCTAATGAACAGGTGTTGAAATTTGTCATGGATGGAGGGTATCTGGATCAACCCGACAACTGTCCA
GAGAGAGTCACTGACCTCATGCGCATGTGCTGGCAATTCAACCCCAACATGAGGCCAACCTTCCTGGAGATTGTC
AACCTGCTCAAGGACGACCTGCACCCCAGCTTTCCAGAGGTGTCGTTCTTCCACAGCGAGGAGAACAAGGCTCCC
GAGAGTGAGGAGCTGGAGATGGAGTTTGAGGACATGGAGAATGTGCCCCTGGACCGTTCCTCGCACTGTCAGAGG
GAGGAGGCGGGGGGCCGGGATGGAGGGTCCTCGCTGGGTTTCAAGCGGAGCTACGAGGAACACATCCCTTACACA
CACATGAACGGAGGCAAGAAAAACGGGCGGATTCTGACCTTGCCTCGGTCCAATCCTTCCTGGGCCCGGGATCCA
CCGGTCGCCACCGTGAGCAAGGGCGAGGAGAATAATATGGCCATCATCAAGGAGTTCATGCGCTTCAAGGTGCGC
ATGGAGGGCACCGTGAACGGCCACGAGTTCGAGATCGAGGGCGAGGGCGAGGGCCGCCCCTACGAGGGCTTTCAG
ACCGCTAAGCTGAAGGTGACCAAGGGCGGCCCCCTGCCCTTCGCCTGGGACATCCTGTCCCCTCTCTTCACCTAC
GGCTCCAAGGCCTACGTGAAGCACCCCGCCGACATCCCCGACTACTTCAAGCTGTCCTTCCCCGAGGGCTTCAAG
TGGGAGCGCGTGATGAACTACGAGGACGGCGGCGTGGTGACCGTGACCCAGGACTCCTCACTGCAGGACGGCGAG
TTCATCTACAAGGTGAAGATGCGCGGCACCAACTTCCCCTCCGACGGCCCCGTGATGCAGAAGAAGACCATGGGC
TGGGAGGCCTCCTCCGAGCGGATGTACCCCGAGGACGGCGCCCTGAAGGGCGAGATCAGGATGAGGCTGAAGCTG
AAGGACGGCGGCCACTACACCTCCGAGGTCAAGACCACCTACAAGGCCAAGAAGTCCGTGCAGCTGCCCGGCGCC
TACATCGTCGGCATCAAGCTGGACATCACCTCCCACAACGAGGACTACACCATCGTGGAACAGTACGAACGCGCC
GAGGGCCGCCACTCCACCGGCGGCATGGACGAGCTGTACAAGTAAAGCGGCCGCGACTCTAGATCATAATCAGCA
CATGAGGATCACCCATGTCTGCAGGTCGACTCTAGAAAACATGAGGATCACCCATGT (SEQ ID NO:211)
Protein: (X indicates non-canonical amino acid)
MGTGGRRGAAAAPLLVAVAALLLGAAGHLYPGEVCPGMDIRNNLTRLHELENCSVIEGHLQILLMFKTRPEDFRD
LSFPKLIMITDYLLLFRVYGLESLKDLFPNLTVIRGSRLFFNYALVIFEMVHLKELGLYNLMNITRGSVRIEKNN
ELCYLATIDWSRILDSVEDNYIVLNKDDNEECGDICPGTAKGKTNCPATVINGQFVERCWTHSHCQKVCPTICKS
HGCTAEGLCCHSECLGNCSQPDDPTKCVACRNFYLDGRCVETCPPPYYHFQDWRCVNFSFCQDLHHKCKNSRRQG
CHQYVIHNNKCIPECPSGYTMNSSNLLCTPCLGPCPKVCHLLEGEKTIDSVTSAQELRGCTVINGSLIINIRGGN
NLAAELEANLGLIEEISGYLKIRRSYALVSLSFFRKLRLIRGETLEIGNYSFYALDNQNLRQLWDWSKHNLTITQ
GKLFFHYNPKLCLSEIHKMEEVSGTKGRQERNDIALKTNGDQASCENELLKFSYIRTSFDKILLRWEPYWPPDFR
DLLGFMLFYKEAPYQNVTEFDGQDACGSNSWTVVDIDPPLRSNDPKSQNHPGWLMRGLKPWTQYAIFVKTLVTFS
DERRTYGAKSDIIYVQTDATNPSVPLDPISVSNSSSQIILKWKPPSDPNGNITHYLVFWERQAEDSELFELDYCL
XGLKLPSRTWSPPFESEDSQKHNQSEYEDSAGECCSCPKTDSQILKELEESSFRKTFEDYLHNVVFVPRPSRKRR
SLGDVGNVTVAVPTVAAFPNTSSTSVPTSPEEHRPFEKVVNKESLVISGLRHFTGYRIELQACNQDTPEERCSVA
AYVSARTMPEAKADDIVGPVTHEIFENNVVHLMWQEPKEPNGLIVLYEVSYRRYGDEELHLCVSRKHFALERGCR
LRGLSPGNYSVRIRATSLAGNGSWTEPTYFYVTDYLDVPSNIAKIIIGPLIFVFLFSVVIGSIYLFLRKRQPDGP
LGPLYASSNPEYLSASDVFPCSVYVPDEWEVSREKITLLRELGQGSFGMVYEGNARDIIKGEAETRVAVKTVNES
ASLRERIEFLNEASVMKGFTCHHVVRLLGVVSKGQPTLVVMELMAHGDLKSYLRSLRPEAENNPGRPPPTLQEMI
QMAAEIADGMAYLNAKKFVHRDLAARNCMVAHDFTVKIGDFGMTRDIYETDYYRKGGKGLLPVRWMAPESLKDGV
FTTSSDMWSFGVVLWEITSLAEQPYQGLSNEQVLKFVMDGGYLDQPDNCPERVTDLMRMCWQFNPNMRPTFLEIV
NLLKDDLHPSFPEVSFFHSEENKAPESEELEMEFEDMENVPLDRSSHCQREEAGGRDGGSSLGFKRSYEEHIPYT
HMNGGKKNGRILTLPRSNPSWARDPPVATVSKGEENNMAIIKEFMRFKVRMEGTVNGHEFEIEGEGEGRPYEGFQ
TAKLKVTKGGPLPFAWDILSPLFTYGSKAYVKHPADIPDYFKLSFPEGFKWERVMNYEDGGVVTVTQDSSLQDGE
FIYKVKMRGTNFPSDGPVMQKKTMGWEASSERMYPEDGALKGEIRMRLKLKDGGHYTSEVKTTYKAKKSVQLPGA
YIVGIKLDITSHNEDYTIVEQYERAEGRHSTGGMDELYK (SEQ ID NO:212)

INSR676TAG-iRFP-MS2

DNA: (MS2 stem-loops and Amber codon underlined)
ATGGGCACCGGGGGCCGGCGGGGGGCGGCGGCCGCGCCGCTGCTGGTGGCGGTGGCCGCGCTGCTACTGGGCGCC
GCGGGCCACCTGTACCCCGGAGAGGTGTGTCCCGGCATGGATATCCGGAACAACCTCACTAGGTTGCATGAGCTG
GAGAATTGCTCTGTCATCGAAGGACACTTGCAGATACTCTTGATGTTCAAAACGAGGCCCGAAGATTTCCGAGAC
CTCAGTTTCCCCAAACTCATCATGATCACTGATTACTTGCTGCTCTTCCGGGTCTATGGGCTCGAGAGCCTGAAG
GACCTGTTCCCCAACCTCACGGTCATCCGGGGATCACGACTGTTCTTTAACTACGCGCTGGTCATCTTCGAGATG
GTTCACCTCAAGGAACTCGGCCTCTACAACCTGATGAACATCACCCGGGGTTCTGTCCGCATCGAGAAGAACAAT
GAGCTCTGTTACTTGGCCACTATCGACTGGTCCCGTATCCTGGATTCCGTGGAGGATAATTACATCGTGTTGAAC
AAAGATGACAACGAGGAGTGTGGAGACATCTGTCCGGGTACCGCGAAGGGCAAGACCAACTGCCCCGCCACCGTC
ATCAACGGGCAGTTTGTCGAACGATGTTGGACTCATAGTCACTGCCAGAAAGTTTGCCCGACCATCTGTAAGTCA
CACGGCTGCACCGCCGAAGGCCTCTGTTGCCACAGCGAGTGCCTGGGCAACTGTTCTCAGCCCGACGACCCCACC
AAGTGCGTGGCCTGCCGCAACTTCTACCTGGATGGCAGGTGTGTGGAGACCTGCCCGCCCCCGTACTACCACTTC
CAGGACTGGCGCTGTGTGAACTTCAGCTTCTGCCAGGACCTGCACCACAAATGCAAGAACTCGCGGAGGCAGGGC
TGCCACCAGTACGTCATTCACAACAACAAGTGCATCCCTGAGTGTCCCTCCGGGTACACGATGAATTCCAGCAAC
TTGCTGTGCACCCCATGCCTGGGTCCCTGTCCCAAGGTGTGCCACCTCCTAGAAGGCGAGAAGACCATCGACTCG
GTGACGTCTGCCCAGGAGCTCCGAGGATGCACCGTCATCAACGGGAGTCTGATCATCAACATTCGAGGAGGCAAC
AATCTGGCAGCTGAGCTAGAAGCCAACCTCGGCCTCATTGAAGAAATTTCAGGGTATCTAAAAATCCGCCGATCC
TACGCTCTGGTGTCACTTTCCTTCTTCCGGAAGTTACGTCTGATTCGAGGAGAGACCTTGGAAATTGGGAACTAC
TCCTTCTATGCCTTGGACAACCAGAACCTAAGGCAGCTCTGGGACTGGAGCAAACACAACCTCACCATCACTCAG
GGGAAACTCTTCTTCCACTATAACCCCAAACTCTGCTTGTCAGAAATCCACAAGATGGAAGAAGTTTCAGGAACC
AAGGGGCGCCAGGAGAGAAACGACATTGCCCTGAAGACCAATGGGGACCAGGCATCCTGTGAAAATGAGTTACTT
AAATTTTCTTACATTCGGACATCTTTTGACAAGATCTTGCTGAGATGGGAGCCGTACTGGCCCCCCGACTTCCGA
GACCTCTTGGGGTTCATGCTGTTCTACAAAGAGGCCCCTTATCAGAATGTGACGGAGTTCGACGGGCAGGATGCA
TGTGGTTCCAACAGTTGGACGGTGGTAGACATTGACCCACCCCTGAGGTCCAACGACCCCAAATCACAGAACCAC
CCAGGGTGGCTGATGCGGGGTCTCAAGCCCTGGACCCAGTATGCCATCTTTGTGAAGACCCTGGTCACCTTTTCG
GATGAACGCCGGACCTATGGGGCCAAGAGTGACATCATTTATGTCCAGACAGATGCCACCAACCCCTCTGTGCCC
CTGGATCCAATCTCAGTGTCTAACTCATCATCCCAGATTATTCTGAAGTGGAAACCACCCTCCGACCCCAATGGC
AACATCACCCACTACCTGGTTTTCTGGGAGAGGCAGGCGGAAGACAGTGAGCTGTTCGAGCTGGATTATTGCCTC
TAGGGGCTGAAGCTGCCCTCGAGGACCTGGTCTCCACCATTCGAGTCTGAAGATTCTCAGAAGCACAACCAGAGT
GAGTATGAGGATTCGGCCGGCGAATGCTGCTCCTGTCCAAAGACAGACTCTCAGATCCTGAAGGAGCTGGAGGAG
TCCTCGTTTAGGAAGACGTTTGAGGATTACCTGCACAACGTGGTTTTCGTCCCCAGGCCATCTCGGAAACGCAGG
TCCCTTGGCGATGTTGGGAATGTGACGGTGGCCGTGCCCACGGTGGCAGCTTTCCCCAACACTTCCTCGACCAGC
GTGCCCACGAGTCCGGAGGAGCACAGGCCTTTTGAGAAGGTGGTGAACAAGGAGTCGCTGGTCATCTCCGGCTTG
CGACACTTCACGGGCTATCGCATCGAGCTGCAGGCTTGCAACCAGGACACCCCTGAGGAACGGTGCAGTGTGGCA
GCCTACGTCAGTGCGAGGACCATGCCTGAAGCCAAGGCTGATGACATTGTTGGCCCTGTGACGCATGAAATCTTT
GAGAACAACGTCGTCCACTTGATGTGGCAGGAGCCGAAGGAGCCCAATGGTCTGATCGTGCTGTATGAAGTGAGT
TATCGGCGATATGGTGATGAGGAGCTGCATCTCTGCGTCTCCCGCAAGCACTTCGCTCTGGAACGGGGCTGCAGG
CTGCGTGGGCTGTCACCGGGGAACTACAGCGTGCGAATCCGGGCCACCTCCCTTGCGGGCAACGGCTCTTGGACG
GAACCCACCTATTTCTACGTGACAGACTATTTAGACGTCCCGTCAAATATTGCAAAAATTATCATCGGCCCCCTC
ATCTTTGTCTTTCTCTTCAGTGTTGTGATTGGAAGTATTTATCTATTCCTGAGAAAGAGGCAGCCAGATGGGCCG
CTGGGACCGCTTTACGCTTCTTCAAACCCTGAGTATCTCAGTGCCAGTGATGTGTTTCCATGCTCTGTGTACGTG
CCGGACGAGTGGGAGGTGTCTCGAGAGAAGATCACCCTCCTTCGAGAGCTGGGGCAGGGCTCCTTCGGCATGGTG
TATGAGGGCAATGCCAGGGACATCATCAAGGGTGAGGCAGAGACCCGCGTGGCGGTGAAGACGGTCAACGAGTCA
GCCAGTCTCCGAGAGCGGATTGAGTTCCTCAATGAGGCCTCGGTCATGAAGGGCTTCACCTGCCATCACGTGGTG
CGCCTCCTGGGAGTGGTGTCCAAGGGCCAGCCCACGCTGGTGGTGATGGAGCTGATGGCTCACGGAGACCTGAAG
AGCTACCTCCGTTCTCTGCGGCCAGAGGCTGAGAATAATCCTGGCCGCCCTCCCCCTACCCTTCAAGAGATGATT
CAGATGGCGGCAGAGATTGCTGACGGGATGGCCTACCTGAACGCCAAGAAGTTTGTGCATCGGGACCTGGCAGCG
AGAAACTGCATGGTCGCCCATGATTTTACTGTCAAAATTGGAGACTTTGGAATGACCAGAGACATCTATGAAACG
GATTACTACCGGAAAGGGGGCAAGGGTCTGCTCCCTGTACGGTGGATGGCACCGGAGTCCCTGAAGGATGGGGTC
TTCACCACTTCTTCTGACATGTGGTCCTTTGGCGTGGTCCTTTGGGAAATCACCAGCTTGGCAGAACAGCCTTAC
CAAGGCCTGTCTAATGAACAGGTGTTGAAATTTGTCATGGATGGAGGGTATCTGGATCAACCCGACAACTGTCCA
GAGAGAGTCACTGACCTCATGCGCATGTGCTGGCAATTCAACCCCAACATGAGGCCAACCTTCCTGGAGATTGTC
AACCTGCTCAAGGACGACCTGCACCCCAGCTTTCCAGAGGTGTCGTTCTTCCACAGCGAGGAGAACAAGGCTCCC
GAGAGTGAGGAGCTGGAGATGGAGTTTGAGGACATGGAGAATGTGCCCCTGGACCGTTCCTCGCACTGTCAGAGG
GAGGAGGCGGGGGGCCGGGATGGAGGGTCCTCGCTGGGTTTCAAGCGGAGCTACGAGGAACACATCCCTTACACA
CACATGAACGGAGGCAAGAAAAACGGGCGGATTCTGACCTTGCCTCGGTCCAATCCTTCCTGGGCCCGGGATCCA
CCGGTCGCCACCGCGGAAGGATCCGTCGCCAGGCAGCCTGACCTCTTGACCTGCGACGATGAGCCGATCCATATC
CCCGGTGCCATCCAACCGCATGGACTGCTGCTCGCCCTCGCCGCCGACATGACGATCGTTGCCGGCAGCGACAAC

CTTCCCGAACTCACCGGACTGGCGATCGGCGCCCTGATCGGCCGCTCTGCGGCCGATGTCTTCGACTCGGAGACG
CACAACCGTCTGACGATCGCCTTGGCCGAGCCCGGGGCGGCCGTCGGAGCACCGATCACTGTCGGCTTCACGATG
CGAAAGGACGCAGGCTTCATCGGCTCCTGGCATCGCCATGATCAGCTCATCTTCCTCGAGCTCGAGCCTCCCCAG
CGGGACGTCGCCGAGCCGCAGGCGTTCTTCCGCCGCACCAACAGCGCCATCCGCCGCCTGCAGGCCGCCGAAACC
TTGGAAAGCGCCTGCGCCGCCGCGGCGCAAGAGGTGCGGAAGATTACCGGCTTCGATCGGGTGATGATCTATCGC
TTCGCCTCCGACTTCAGCGGCGAAGTGATCGCAGAGGATCGGTGCGCCGAGGTCGAGTCAAAACTAGGCCTGCAC
TATCCTGCCTCAACCGTGCCGGCGCAGGCCCGTCGGCTCTATACCATCAACCCGGTACGGATCATTCCCGATATC
AATTATCGGCCGGTGCCGGTCACCCCAGACCTCAATCCGGTCACCGGGCGGCCGATTGATCTTAGCTTCGCCATC
CTGCGCAGCGTCTCGCCCGTCCATCTGGAATTCATGCGCAACATAGGCATGCACGGCACGATGTCGATCTCGATT
TTGCGCGGCGAGCGACTGTGGGGATTGATCGTTTGCCATCACCGAACGCCGTACTACGTCGATCTCGATGGCCGC
CAAGCCTGCGAGCTAGTCGCCCAGGTTCTGGCCTGGCAGATCGGCGTGATGGAAGAGTAAGCGGCCGCGACTCTA
GATCATAATCAGCACATGAGGATCACCCATGTCTGCAGGTCGACTCTAGAAAACATGAGGATCACCCATGT
(SEQ ID NO:213)

Protein: (X indicates non-canonical amino acid)
MGTGGRRGAAAAPLLVAVAALLLGAAGHLYPGEVCPGMDIRNNLTRLHELENCSVIEGHLQILLMFKTRPEDFRD
LSFPKLIMITDYLLLFRVYGLESLKDLFPNLTVIRGSRLFFNYALVIFEMVHLKELGLYNLMNITRGSVRIEKNN
ELCYLATIDWSRILDSVEDNYIVLNKDDNEECGDICPGTAKGKTNCPATVINGQFVERCWTHSHCQKVCPTICKS
HGCTAEGLCCHSECLGNCSQPDDPTKCVACRNFYLDGRCVETCPPPYYHFQDWRCVNFSFCQDLHHKCKNSRRQG
CHQYVIHNNKCIPECPSGYTMNSSNLLCTPCLGPCPKVCHLLEGEKTIDSVTSAQELRGCTVINGSLIINIRGGN
NLAAELEANLGLIEEISGYLKIRRSYALVSLSFFRKLRLIRGETLEIGNYSFYALDNQNLRQLWDWSKHNLTITQ
GKLFFHYNPKLCLSEIHKMEEVSGTKGRQERNDIALKTNGDQASCENELLKFSYIRTSFDKILLRWEPYWPPDFR
DLLGFMLFYKEAPYQNVTEFDGQDACGSNSWTVVDIDPPLRSNDPKSQNHPGWLMRGLKPWTQYAIFVKTLVTFS
DERRTYGAKSDIIYVQTDATNPSVPLDPISVSNSSSQIILKWKPPSDPNGNITHYLVFWERQAEDSELFELDYCL
XGLKLPSRTWSPPFESEDSQKHNQSEYEDSAGECCSCPKTDSQILKELEESSFRKTFEDYLHNVVFVPRPSRKRR
SLGDVGNVTVAVPTVAAFPNTSSTSVPTSPEEHRPFEKVVNKESLVISGLRHFTGYRIELQACNQDTPEERCSVA
AYVSARTMPEAKADDIVGPVTHEIFENNVVHLMWQEPKEPNGLIVLYEVSYRRYGDEELHLCVSRKHFALERGCR
LRGLSPGNYSVRIRATSLAGNGSWTEPTYFYVTDYLDVPSNIAKIIIGPLIFVFLFSVVIGSIYLFLRKRQPDGP
LGPLYASSNPEYLSASDVFPCSVYVPDEWEVSREKITLLRELGQGSFGMVYEGNARDIIKGEAETRVAVKTVNES
ASLRERIEFLNEASVMKGFTCHHVVRLLGVVSKGQPTLVVMELMAHGDLKSYLRSLRPEAENNPGRPPPTLQEMI
QMAAEIADGMAYLNAKKFVHRDLAARNCMVAHDFTVKIGDFGMTRDIYETDYYRKGGKGLLPVRWMAPESLKDGV
FTTSSDMWSFGVVLWEITSLAEQPYQGLSNEQVLKFVMDGGYLDQPDNCPERVTDLMRMCWQFNPNMRPTFLEIV
NLLKDDLHPSFPEVSFFHSEENKAPESEELEMEFEDMENVPLDRSSHCQREEAGGRDGGSSLGFKRSYEEHIPYT
HMNGGKKNGRILTLPRSNPSWARDPPVATAEGSVARQPDLLTCDDEPIHIPGAIQPHGLLLALAADMTIVAGSDN
LPELTGLAIGALIGRSAADVFDSETHNRLTIALAEPGAAVGAPITVGFTMRKDAGFIGSWHRHDQLIFLELEPPQ
RDVAEPQAFFRRTNSAIRRLQAAETLESACAAAAQEVRKITGFDRVMIYRFASDFSGEVIAEDRCAEVESKLGLH
YPASTVPAQARRLYTINPVRIIPDINYRPVPVTPDLNPVTGRPIDLSFAILRSVSPVHLEFMRNIGMHGTMSISI
LRGERLWGLIVCHHRTPYYVDLDGRQACELVAQVLAWQIGVMEE (SEQ ID NO:214)

Sequences - Set 2

1. Further Components:

mCherry190TAG-2xPP7 mCherry with amber site and 2 PP7 loops (TAG codon,PP7 loops)
DNA:
ATGGGCCGCCTGGAAAGCACCCCGCCGAAAAAAAAACGCAAAGTGGAAGATAGCGCGAGCGTGAGCAAGGGCGAG
GAGGATAACATGGCCATCATCAAGGAGTTCATGCGCTTCAAGGTGCACATGGAGGGCTCCGTGAACGGCCACGAG
TTCGAGATCGAGGGCGAGGGCGAGGGCCGCCCCTACGAGGGCACCCAGACCGCCAAGCTGAAGGTGACCAAGGGT
GGCCCCCTGCCCTTCGCCTGGGACATCCTGTCCCCTCAGTTCATGTACGGCTCCAAGGCCTACGTGAAGCACCCC
GCCGACATCCCCGACTACTTGAAGCTGTCCTTCCCCGAGGGCTTCAAGTGGGAGCGCGTGATGAACTTCGAGGAC
GGCGGCGTGGTGACCGTGACCCAGGACTCCTCCCTGCAGGACGGCGAGTTCATCTACAAGGTGAAGCTGCGCGGC
ACCAACTTCCCCTCCGACGGCCCCGTAATGCAGAAGAAGACGATGGGCTGGGAGGCCTCCTCCGAGCGGATGTAC
CCCGAGGACGGCGCCCTGAAGGGCGAGATCAAGCAGAGGCTGAAGCTGAAGGACGGCGGCCACTACGACGCTGAG
GTCAAGACCACCTACAAGGCCAAGTAGCCCGTGCAGCTGCCCGGCGCCTACAACGTCAACATCAAGTTGGACATC
ACCTCCCACAACGAGGACTACACCATCGTGGAACAGTACGAACGCGCCGAGGGCCGCCACTCCACCGGCGGCATG
GACGAGCTGTACAAGCATCATCATCATCATCATTAAAGATCCTAAGGTACAATTGCCTAGAAAGGAGCAGACGAT
ATGGCGTCGCTCCCTGCAGGTCGACTCTAGAAACCAGCAGAGCATATGGGCTCGCTGG (SEQ ID NO: 215)

Protein:
MGRLESTPPKKKRKVEDSASVSKGEEDNMAIIKEFMRFKVHMEGSVNGHEFEIEGEGEGRPYEGTQTAKLKVTKG
GPLPFAWDILSPQFMYGSKAYVKHPADIPDYLKLSFPEGFKWERVMNFEDGGVVTVTQDSSLQDGEFIYKVKLRG
TNFPSDGPVMQKKTMGWEASSERMYPEDGALKGEIKQRLKLKDGGHYDAEVKTTYKAKXPVQLPGAYNVNIKLDI
TSHNEDYTIVEQYERAEGRHSTGGMDELYKHHHHHH*(SEQ ID NO: 216)

mCherry190TAG-4xPP7 mCherry with amber site and 4 PP7 loops (TAG codon,PP7 loops)
DNA:
ATGGGCCGCCTGGAAAGCACCCCGCCGAAAAAAAAACGCAAAGTGGAAGATAGCGCGAGCGTGAGCAAGGGCGAG
GAGGATAACATGGCCATCATCAAGGAGTTCATGCGCTTCAAGGTGCACATGGAGGGCTCCGTGAACGGCCACGAG
TTCGAGATCGAGGGCGAGGGCGAGGGCCGCCCCTACGAGGGCACCCAGACCGCCAAGCTGAAGGTGACCAAGGGT
GGCCCCCTGCCCTTCGCCTGGGACATCCTGTCCCCTCAGTTCATGTACGGCTCCAAGGCCTACGTGAAGCACCCC
GCCGACATCCCCGACTACTTGAAGCTGTCCTTCCCCGAGGGCTTCAAGTGGGAGCGCGTGATGAACTTCGAGGAC
GGCGGCGTGGTGACCGTGACCCAGGACTCCTCCCTGCAGGACGGCGAGTTCATCTACAAGGTGAAGCTGCGCGGC
ACCAACTTCCCCTCCGACGGCCCCGTAATGCAGAAGAAGACGATGGGCTGGGAGGCCTCCTCCGAGCGGATGTAC
CCCGAGGACGGCGCCCTGAAGGGCGAGATCAAGCAGAGGCTGAAGCTGAAGGACGGCGGCCACTACGACGCTGAG
GTCAAGACCACCTACAAGGCCAAGTAGCCCGTGCAGCTGCCCGGCGCCTACAACGTCAACATCAAGTTGGACATC
ACCTCCCACAACGAGGACTACACCATCGTGGAACAGTACGAACGCGCCGAGGGCCGCCACTCCACCGGCGGCATG
GACGAGCTGTACAAGCATCATCATCATCATCATTAAAGATCCTAAGGTACAATTGCCTAGAAAGGAGCAGACGAT
ATGGCGTCGCTCCCTGCAGGTCGACTCTAGAAACCAGCAGAGCATATGGGCTCGCTGGCTGGGTACAATTGCCTA
GAAAGGAGCAGACGATATGGCGTCGCTCCCTGCAGGTCGACTCTAGAAACCAGCAGAGCATATGGGCTCGCTGG
(SEQ ID NO: 217)

Protein:
MGRLESTPPKKKRKVEDSASVSKGEEDNMAIIKEFMRFKVHMEGSVNGHEFEIEGEGEGRPYEGTQTAKLKVTKG
GPLPFAWDILSPQFMYGSKAYVKHPADIPDYLKLSFPEGFKWERVMNFEDGGVVTVTQDSSLQDGEFIYKVKLRG
TNFPSDGPVMQKKTMGWEASSERMYPEDGALKGEIKQRLKLKDGGHYDAEVKTTYKAKXPVQLPGAYNVNIKLDI
TSHNEDYTIVEQYERAEGRHSTGGMDELYKHHHHHH*(SEQ ID NO: 218)

mCherry190TAG-6xPP7 mCherry with amber site and 6 PP7 loops (TAG codon,PP7 loops)
DNA:
ATGGGCCGCCTGGAAAGCACCCCGCCGAAAAAAAAACGCAAAGTGGAAGATAGCGCGAGCGTGAGCAAGGGCGAG
GAGGATAACATGGCCATCATCAAGGAGTTCATGCGCTTCAAGGTGCACATGGAGGGCTCCGTGAACGGCCACGAG
TTCGAGATCGAGGGCGAGGGCGAGGGCCGCCCCTACGAGGGCACCCAGACCGCCAAGCTGAAGGTGACCAAGGGT

GGCCCCCTGCCCTTCGCCTGGGACATCCTGTCCCCTCAGTTCATGTACGGCTCCAAGGCCTACGTGAAGCACCCC
GCCGACATCCCCGACTACTTGAAGCTGTCCTTCCCCGAGGGCTTCAAGTGGGAGCGCGTGATGAACTTCGAGGAC
GGCGGCGTGGTGACCGTGACCCAGGACTCCTCCCTGCAGGACGGCGAGTTCATCTACAAGGTGAAGCTGCGCGGC
ACCAACTTCCCCTCCGACGGCCCCGTAATGCAGAAGAAGACGATGGGCTGGGAGGCCTCCTCCGAGCGGATGTAC
CCCGAGGACGGCGCCCTGAAGGGCGAGATCAAGCAGAGGCTGAAGCTGAAGGACGGCGGCCACTACGACGCTGAG
GTCAAGACCACCTACAAGGCCAAGTAGCCCGTGCAGCTGCCCGGCGCCTACAACGTCAACATCAAGTTGGACATC
ACCTCCCACAACGAGGACTACACCATCGTGGAACAGTACGAACGCGCCGAGGGCCGCCACTCCACCGGCGGCATG
GACGAGCTGTACAAGCATCATCATCATCATCATTAAAGATCCTAAGGTACAATTGCCTAGAAAGGAGCAGACGAT
ATGGCGTCGCTCCCTGCAGGTCGACTCTAGAAACCAGCAGAGCATATGGGCTCGCTGGCTGGGTACAATTGCCTA
GAAAGGAGCAGACGATATGGCGTCGCTCCCTGCAGGTCGACTCTAGAAACCAGCAGAGCATATGGGCTCGCTGGC
TGGGTACAATTGCCTAGAAAGGAGCAGACGATATGGCGTCGCTCCCTGCAGGTCGACTCTAGAAACCAGCAGAGC
ATATGGGCTCGCTGG (SEQ ID NO: 219)

Protein:
MGRLESTPPKKKRKVEDSASVSKGEEDNMAIIKEFMRFKVHMEGSVNGHEFEIEGEGEGRPYEGTQTAKLKVTKG
GPLPFAWDILSPQFMYGSKAYVKHPADIPDYLKLSFPEGFKWERVMNFEDGGVVTVTQDSSLQDGEFIYKVKLRG
TNFPSDGPVMQKKTMGWEASSERMYPEDGALKGEIKQRLKLKDGGHYDAEVKTTYKAKXPVQLPGAYNVNIKLDI
TSHNEDYTIVEQYERAEGRHSTGGMDELYKHHHHHH*(SEQ ID NO: 220)

H2B-mCherry190TAG-2xMS2 Human Histone H2B type 1-J (Uniprot: P06899) fused
to mCherry with amber site and 2x ms2-loops (TAG codon, ms2 loops)
DNA:
ATGCCAGAGCCAGCGAAGTCTGCTCCCGCCCCGAAAAAGGGCTCCAAGAAGGCGGTGACTAAGGCGCAGAAGAAA
GGCGGCAAGAAGCGCAAGCGCAGCCGCAAGGAGAGCTATTCCATCTATGTGTACAAGGTTCTGAAGCAGGTCCAC
CCTGACACCGGCATTTCGTCCAAGGCCATGGGCATCATGAATTCGTTTGTGAACGACATTTTCGAGCGCATCGCA
GGTGAGGCTTCCCGCCTGGCGCATTACAACAAGCGCTCGACCATCACCTCCAGGGAGATCCAGACGGCCGTGCGC
CTGCTGCTGCCTGGGGAGTTGGCCAAGCACGCCGTGTCCGAGGGTACTAAGGCCATCACCAAGTACACCAGCGCT
AAGGATCCACCGGTCGCCACCATGGTGAGCAAGGGCGAGGAGGATAACATGGCCATCATCAAGGAGTTCATGCGC
TTCAAGGTGCACATGGAGGGCTCCGTGAACGGCCACGAGTTCGAGATCGAGGGCGAGGGCGAGGGCCGCCCCTAC
GAGGGCACCCAGACCGCCAAGCTGAAGGTGACCAAGGGTGGCCCCCTGCCCTTCGCCTGGGACATCCTGTCCCCT
CAGTTCATGTACGGCTCCAAGGCCTACGTGAAGCACCCCGCCGACATCCCCGACTACTTGAAGCTGTCCTTCCCC
GAGGGCTTCAAGTGGGAGCGCGTGATGAACTTCGAGGACGGCGGCGTGGTGACCGTGACCCAGGACTCCTCCCTG
CAGGACGGCGAGTTCATCTACAAGGTGAAGCTGCGCGGCACCAACTTCCCCTCCGACGGCCCCGTAATGCAGAAG
AAGACGATGGGCTGGGAGGCCTCCTCCGAGCGGATGTACCCCGAGGACGGCGCCCTGAAGGGCGAGATCAAGCAG
AGGCTGAAGCTGAAGGACGGCGGCCACTACGACGCTGAGGTCAAGACCACCTACAAGGCCAAGTAGCCCGTGCAG
CTGCCCGGCGCCTACAACGTCAACATCAAGTTGGACATCACCTCCCACAACGAGGACTACACCATCGTGGAACAG
TACGAACGCGCCGAGGGCCGCCACTCCACCGGCGGCATGGACGAGCTGTACAAGCATCATCATCATCATCATTAA
GGGCCCAGATCTCCTGAGGCTGCAGCCTACTAGTCCTAGAAAACATGAGGATCACCCATGTCTGCACCTCGACAC
TAGAAAACATGAGGATCACCCATGT(SEQ ID NO: 221)

Protein:
MPEPAKSAPAPKKGSKKAVTKAQKKGGKKRKRSRKESYSIYVYKVLKQVHPDTGISSKAMGIMNSFVNDIFERIA
GEASRLAHYNKRSTITSREIQTAVRLLLPGELAKHAVSEGTKAITKYTSAKDPPVATMVSKGEEDNMAIIKEFMR
FKVHMEGSVNGHEFEIEGEGEGRPYEGTQTAKLKVTKGGPLPFAWDILSPQFMYGSKAYVKHPADIPDYLKLSFP
EGFKWERVMNFEDGGVVTVTQDSSLQDGEFIYKVKLRGTNFPSDGPVMQKKTMGWEASSERMYPEDGALKGEIKQ
RLKLKDGGHYDAEVKTTYKAKXPVQLPGAYNVNIKLDITSHNEDYTIVEQYERAEGRHSTGGMDELYKHHHHHH*
(SEQ ID NO: 222)

IFRS1 Methanosarcina mazei PylRS (L305M, Y306L, L309S, N346S, C348M)
DNA:
GACAAAAAACCGCTGAATACCCTGATCTCTGCTACTGGTCTGTGGATGAGTCGTACCGGAACCATTCATAAAATC
AAACACCACGAGGTTAGCCGTTCGAAAATCTATATTGAGATGGCGTGTGGCGATCATCTGGTTGTGAACAATAGC
CGCTCTTCTCGTACAGCACGTGCACTGCGTCACCACAAATATCGTAAAACCTGTAAACGTTGCCGTGTGTCCGAT
GAGGATCTGAACAAATTCCTGACAAAAGCCAATGAGGACCAAACAAGCGTGAAAGTGAAAGTCGTTAGCGCTCCT
ACCCGTACTAAAAAAGCAATGCCGAAATCCGTTGCTCGTGCCCCTAAACCACTGGAAAACACTGAAGCAGCACAG
GCACAGCCGTCTGGAAGCAAATTCTCTCCGGCCATTCCTGTTTCTACCCAGGAGTCCGTTTCTGTTCCAGCAAGT
GTGAGCACCAGCATTAGCAGTATTAGCACCGGTGCCACCGCTAGCGCCCTGGTTAAAGGCAATACCAATCCGATT
ACAAGCATGTCTGCCCCGGTTCAAGCATCAGCTCCAGCACTGACAAAATCCCAAACCGATCGTCTGGAGGTTCTG

CTGAATCCGAAAGACGAAATCAGCCTGAATTCCGGCAAACCGTTTCGTGAACTGGAGAGCGAACTGCTGTCACGT
CGTAAAAAAGACCTGCAACAAATCTATGCCGAAGAACGTGAGAACTATCTGGGGAAACTGGAACGTGAAATCACC
CGCTTTTTCGTGGATCGTGGCTTTCTGGAGATCAAATCCCCGATTCTGATTCCTCTGGAGTATATCGAGCGTATG
GGCATCGACAATGATACCGAACTGAGCAAACAAATTTTCCGTGTGGATAAAAACTTCTGTCTGCGCCCTATGCTT
GCACCAAATATGCTGAACTATAGCCGCAAACTGGACCGTGCCCTGCCTGATCCTATCAAAATCTTCGAGATCGGC
CCGTGTTATCGTAAAGAGTCCGACGGTAAAGAACATCTGGAGGAGTTTACCATGCTGTCTTTTATGCAAATGGGT
TCAGGTTGTACTCGTGAGAACCTGGAAAGCATCATCACCGATTTTCTGAACCACCTGGGCATTGACTTCAAAATT
GTGGGCGACAGCTGTATGGTGTATGGCGACACCCTGGATGTCATGCACGGCGACCTGGAACTGTCTAGTGCCGTT
GTTGGACCAATTCCGCTGGACCGTGAGTGGGGTATCGACAAACCGTGGATCGGAGCAGGATTCGGTCTGGAACGC
CTGCTGAAAGTGAAACACGACTTCAAAAACATCAAACGTGCCGCCCGTTCTGAATCGTATTATAACGGGATCTCT
ACGAACCTGTAA (SEQ ID NO: 223)

Protein:
DKKPLNTLISATGLWMSRTGTIHKIKHHEVSRSKIYIEMACGDHLVVNNSRSSRTARALRHHKYRKTCKRCRVSD
EDLNKFLTKANEDQTSVKVKVVSAPTRTKKAMPKSVARAPKPLENTEAAQAQPSGSKFSPAIPVSTQESVSVPAS
VSTSISSISTGATASALVKGNTNPITSMSAPVQASAPALTKSQTDRLEVLLNPKDEISLNSGKPFRELESELLSR
RKKDLQQIYAEERENYLGKLEREITRFFVDRGFLEIKSPILIPLEYIERMGIDNDTELSKQIFRVDKNFCLRPML
APNMLNYSRKLDRALPDPIKIFEIGPCYRKESDGKEHLEEFTMLSFMQMGSGCTRENLESIITDFLNHLGIDFKI
VGDSCMVYGDTLDVMHGDLELSSAVVGPIPLDREWGIDKPWIGAGFGLERLLKVKHDFKNIKRAARSESYYNGIS
TNL*(SEQ ID NO: 224)

CbzRS Methanosarcina mazei PylRS (Y306M, L309G, C348T)
DNA:
GACAAAAAACCGCTGAATACCCTGATCTCTGCTACTGGTCTGTGGATGAGTCGTACCGGAACCATTCATAAAATC
AAACACCACGAGGTTAGCCGTTCGAAAATCTATATTGAGATGGCGTGTGGCGATCATCTGGTTGTGAACAATAGC
CGCTCTTCTCGTACAGCACGTGCACTGCGTCACCACAAATATCGTAAAACCTGTAAACGTTGCCGTGTGTCCGAT
GAGGATCTGAACAAATTCCTGACAAAAGCCAATGAGGACCAAACAAGCGTGAAAGTGAAAGTCGTTAGCGCTCCT
ACCCGTACTAAAAAAGCAATGCCGAAATCCGTTGCTCGTGCCCCTAAACCACTGGAAAACACTGAAGCAGCACAG
GCACAGCCGTCTGGAAGCAAATTCTCTCCGGCCATTCCTGTTTCTACCCAGGAGTCCGTTTCTGTTCCAGCAAGT
GTGAGCACCAGCATTAGCAGTATTAGCACCGGTGCCACCGCTAGCGCCCTGGTTAAAGGCAATACCAATCCGATT
ACAAGCATGTCTGCCCCGGTTCAAGCATCAGCTCCAGCACTGACAAAATCCCAAACCGATCGTCTGGAGGTTCTG
CTGAATCCGAAAGACGAAATCAGCCTGAATTCCGGCAAACCGTTTCGTGAACTGGAGAGCGAACTGCTGTCACGT
CGTAAAAAAGACCTGCAACAAATCTATGCCGAAGAACGTGAGAACTATCTGGGGAAACTGGAACGTGAAATCACC
CGCTTTTTCGTGGATCGTGGCTTTCTGGAGATCAAATCCCCGATTCTGATTCCTCTGGAGTATATCGAGCGTATG
GGCATCGACAATGATACCGAACTGAGCAAACAAATTTTCCGTGTGGATAAAAACTTCTGTCTGCGCCCTATGCTT
GCACCAAATCTGATGAACTATGGACGCAAACTGGACCGTGCCCTGCCTGATCCTATCAAAATCTTCGAGATCGGC
CCGTGTTATCGTAAAGAGTCCGACGGTAAAGAACATCTGGAGGAGTTTACCATGCTGAACTTTACACAAATGGGT
TCAGGTTGTACTCGTGAGAACCTGGAAAGCATCATCACCGATTTTCTGAACCACCTGGGCATTGACTTCAAAATT
GTGGGCGACAGCTGTATGGTGTATGGCGACACCCTGGATGTCATGCACGGCGACCTGGAACTGTCTAGTGCCGTT
GTTGGACCAATTCCGCTGGACCGTGAGTGGGGTATCGACAAACCGTGGATCGGAGCAGGATTCGGTCTGGAACGC
CTGCTGAAAGTGAAACACGACTTCAAAAACATCAAACGTGCCGCCCGTTCTGAATCGTATTATAACGGGATCTCT
ACGAACCTGTAA (SEQ ID NO: 225)

Protein:
DKKPLNTLISATGLWMSRTGTIHKIKHHEVSRSKIYIEMACGDHLVVNNSRSSRTARALRHHKYRKTCKRCRVSD
EDLNKFLTKANEDQTSVKVKVVSAPTRTKKAMPKSVARAPKPLENTEAAQAQPSGSKFSPAIPVSTQESVSVPAS
VSTSISSISTGATASALVKGNTNPITSMSAPVQASAPALTKSQTDRLEVLLNPKDEISLNSGKPFRELESELLSR
RKKDLQQIYAEERENYLGKLEREITRFFVDRGFLEIKSPILIPLEYIERMGIDNDTELSKQIFRVDKNFCLRPML
APNLMNYGRKLDRALPDPIKIFEIGPCYRKESDGKEHLEEFTMLNFTQMGSGCTRENLESIITDFLNHLGIDFKI
VGDSCMVYGDTLDVMHGDLELSSAVVGPIPLDREWGIDKPWIGAGFGLERLLKVKHDFKNIKRAARSESYYNGIS
TNL*(SEQ ID NO: 226)

CpkRS Methanosarcina mazei PylRS (A302S)

DNA:
GATAAAAAACCGCTGAATACCCTGATCTCTGCTACTGGTCTGTGGATGAGTCGTACCGGAACCATTCATAAAATC
AAACACCACGAGGTTAGCCGTTCGAAAATCTATATTGAGATGGCGTGTGGCGATCATCTGGTTGTGAACAATAGC

CGCTCTTCTCGTACAGCACGTGCACTGCGTCACCACAAATATCGTAAAACCTGTAAACGTTGCCGTGTGTCCGAT
GAGGATCTGAACAAATTCCTGACAAAAGCCAATGAGGACCAAACAAGCGTGAAAGTGAAAGTCGTTAGCGCTCCT
ACCCGTACTAAAAAAGCAATGCCGAAATCCGTTGCTCGTGCCCCTAAACCACTGGAAAACACTGAAGCAGCACAG
GCACAGCCGTCTGGAAGCAAATTCTCTCCGGCCATTCCTGTTTCTACCCAGGAGTCCGTTTCTGTTCCAGCAAGT
GTGAGCACCAGCATTAGCAGTATTAGCACCGGTGCCACCGCTAGCGCCCTGGTTAAAGGCAATACCAATCCGATT
ACAAGCATGTCTGCCCCGGTTCAAGCATCAGCTCCAGCACTGACAAAATCCCAAACCGATCGTCTGGAGGTTCTG
CTGAATCCGAAAGACGAAATCAGCCTGAATTCCGGCAAACCGTTTCGTGAACTGGAGAGCGAACTGCTGTCACGT
CGTAAAAAAGACCTGCAACAAATCTATGCCGAAGAACGTGAGAACTATCTGGGGAAACTGGAACGTGAAATCACC
CGCTTTTTCGTGGATCGTGGCTTTCTGGAGATCAAATCCCCGATTCTGATTCCTCTGGAGTATATCGAGCGTATG
GGCATCGACAATGATACCGAACTGAGCAAACAAATTTTCCGTGTGGATAAAAACTTCTGTCTGCGCCCTATGCTT
TCACCAAATCTGTATAACTATCTGCGCAAACTGGACCGTGCCCTGCCTGATCCTATCAAAATCTTCGAGATCGGC
CCGTGTTATCGTAAAGAGTCCGACGGTAAAGAACATCTGGAGGAGTTTACCATGCTGAACTTTTGCCAAATGGGT
TCAGGTTGTACTCGTGAGAACCTGGAAAGCATCATCACCGATTTTCTGAACCACCTGGGCATTGACTTCAAAATT
GTGGGCGACAGCTGTATGGTGTATGGCGACACCCTGGATGTCATGCACGGCGACCTGGAACTGTCTAGTGCCGTT
GTTGGACCAATTCCGCTGGACCGTGAGTGGGGTATCGACAAACCGTGGATCGGAGCAGGATTCGGTCTGGAACGC
CTGCTGAAAGTGAAACACGACTTCAAAAACATCAAACGTGCCGCCCGTTCTGAATCGTATTATAACGGGATCTCT
ACGAACCTGTAA (SEQ ID NO: 227)

Protein:
DKKPLNTLISATGLWMSRTGTIHKIKHHEVSRSKIYIEMACGDHLVVNNSRSSRTARALRHHKYRKTCKRCRVSD
EDLNKFLTKANEDQTSVKVKVVSAPTRTKKAMPKSVARAPKPLENTEAAQAQPSGSKFSPAIPVSTQESVSVPAS
VSTSISSISTGATASALVKGNTNPITSMSAPVQASAPALTKSQTDRLEVLLNPKDEISLNSGKPFRELESELLSR
RKKDLQQIYAEERENYLGKLEREITRFFVDRGFLEIKSPILIPLEYIERMGIDNDTELSKQIFRVDKNFCLRPML
SPNLYNYLRKLDRALPDPIKIFEIGPCYRKESDGKEHLEEFTMLNFCQMGSGCTRENLESIITDFLNHLGIDFKI
VGDSCMVYGDTLDVMHGDLELSSAVVGPIPLDREWGIDKPWIGAGFGLERLLKVKHDFKNIKRAARSESYYNGIS
TNL*(SEQ ID NO: 228)

tRNA^Pyl, CGA Pyrrolysyl tRNA (for Serine codon, anticodon bold)
(Methanosarcina mazei)
DNA:GGAAACCTGATCATGTAGATCGAATGGACTCGAAATCCGTTCAGCCGGGTTAGATTCCCGGGGTTTCCG
(SEQ ID NO: 229)

tRNA^Pyl, CGG Pyrrolysyl tRNA (for Proline codon, anticodon in bold)
(Methanosarcina mazei)
DNA:GGAAACCTGATCATGTAGATCGAATGGACTCGGAATCCGTTCAGCCGGGTTAGATTCCCGGGGTTTCCG
(SEQ ID NO: 230)

tRNA^Pyl, UAA Pyrrolysyl tRNA (for Leucine codon, anticodon in bold)
(Methanosarcina mazei)
DNA: GGAAACCTGATCATGTAGATCGAATGGACTTAAAATCCGTTCAGCCGGGTTAGATTCCCGGGGTTTCCG
(SEQ ID NO: 231)

tRNA^Pyl, UAG Pyrrolysyl tRNA (for Leucine codon, anticodon in bold)
(Methanosarcina mazei)
DNA: GGAAACCTGATCATGTAGATCGAATGGACTTAGAATCCGTTCAGCCGGGTTAGATTCCCGGGGTTTCCG
(SEQ ID NO: 232)

tRNA^Pyl, CCG Pyrrolysyl tRNA (for Arginine codon, anticodon in bold)
(Methanosarcina mazei)
DNA: GGAAACCTGATCATGTAGATCGAATGGACTCCGAATCCGTTCAGCCGGGTTAGATTCCCGGGGTTTCCG
(SEQ ID NO: 233)

tRNA^Pyl, AUA Pyrrolysyl tRNA (for Isoleucine codon, anticodon in purple)
(Methanosarcina mazei)
DNA: GGAAACCTGATCATGTAGATCGAATGGACTATAAATCCGTTCAGCCGGGTTAGATTCCCGGGGTTTCCG
(SEQ ID NO: 234)

OMeRS        Pyrrolysyl tRNA Synthetase mutant: A302T, Y384F, N346V, C348W,
V401L (Methanosarcina mazei)
DNA:
ATGGCGTGCCCGGTGCCGCTGCAGCTGCCGCCGCTGGAACGCCTGACCCTGGATGATAAAAAACCGCTGAATACC
CTGATCTCTGCTACTGGTCTGTGGATGAGTCGTACCGGAACCATTCATAAAATCAAACACCACGAGGTTAGCCGT
TCGAAAATCTATATTGAGATGGCGTGTGGCGATCATCTGGTTGTGAACAATAGCCGCTCTTCTCGTACAGCACGT
GCACTGCGTCACCACAAATATCGTAAAACCTGTAAACGTTGCCGTGTGTCCGATGAGGATCTGAACAAATTCCTG
ACAAAAGCCAATGAGGACCAAACAAGCGTGAAAGTGAAAGTCGTTAGCGCTCCTACCCGTACTAAAAAAGCAATG
CCGAAATCCGTTGCTCGTGCCCCTAAACCACTGGAAAACACTGAAGCAGCACAGGCACAGCCGTCTGGAAGCAAA
TTCTCTCCGGCCATTCCTGTTTCTACCCAGGAGTCCGTTTCTGTTCCAGCAAGTGTGAGCACCAGCATTAGCAGT
ATTAGCACCGGTGCCACCGCTAGCGCCCTGGTTAAAGGCAATACCAATCCGATTACAAGCATGTCTGCCCCGGTT
CAAGCATCAGCTCCAGCACTGACAAAATCCCAAACCGATCGTCTGGAGGTTCTGCTGAATCCGAAAGACGAAATC
AGCCTGAATTCCGGCAAACCGTTTCGTGAACTGGAGAGCGAACTGCTGTCACGTCGTAAAAAAGACCTGCAACAA
ATCTATGCCGAAGAACGTGAGAACTATCTGGGGAAACTGGAACGTGAAATCACCCGCTTTTTCGTGGATCGTGGC
TTTCTGGAGATCAAATCCCCGATTCTGATTCCTCTGGAGTATATCGAGCGTATGGGCATCGACAATGATACCGAA
CTGAGCAAACAAATTTTCCGTGTGGATAAAAACTTCTGTCTGCGCCCTATGCTAACACCAAATCTGTATAACTAT
CTGCGCAAACTGGACCGTGCCCTGCCTGATCCTATCAAAATCTTCGAGATCGGCCCGTGTTATCGTAAAGAGTCC
GACGGTAAAGAACATCTGGAGGAGTTTACCATGCTGGTCTTTTGGCAAATGGGTTCAGGTTGTACTCGTGAGAAC
CTGGAAAGCATCATCACCGATTTTCTGAACCACCTGGGCATTGACTTCAAAATTGTGGGCGACAGCTGTATGGTG
TTTGGCGACACCCTGGATGTCATGCACGGCGACCTGGAACTGTCTAGTGCCCTTGTGGGCCCAATCCCGCTGGAT
CGTGAGTGGGGTATCGACAAACCTTGGATCGGTGCGGGTTTTGGTCTGGAGCGTCTGCTGAAAGTAAAACACGAC
TTCAAGAACATCAAACGTGCTGCACGTTCCGAGTCCTATTACAATGGTATTTCTACTAACCTGTAA  (SEQ ID
NO: 235)

Protein:
MACPVPLQLPPLERLTLDDKKPLNTLISATGLWMSRTGTIHKIKHHEVSRSKIYIEMACGDHLVVNNSRSSRTAR
ALRHHKYRKTCKRCRVSDEDLNKFLTKANEDQTSVKVKVVSAPTRTKKAMPKSVARAPKPLENTEAAQAQPSGSK
FSPAIPVSTQESVSVPASVSTSISSISTGATASALVKGNTNPITSMSAPVQASAPALTKSQTDRLEVLLNPKDEI
SLNSGKPFRELESELLSRRKKDLQQIYAEERENYLGKLEREITRFFVDRGFLEIKSPILIPLEYIERMGIDNDTE
LSKQIFRVDKNFCLRPMLTPNLYNYLRKLDRALPDPIKIFEIGPCYRKESDGKEHLEEFTMLVFWQMGSGCTREN
LESIITDFLNHLGIDFKIVGDSCMVFGDTLDVMHGDLELSSALVGPIPLDREWGIDKPWIGAGFGLERLLKVKHD
FKNIKRAARSESYYNGISTNL (SEQ ID NO: 236)

GFP66TAG        GFP with Amber site
DNA: (MS2-stem loops, Amber codon)
ATGGGCCGCCTGGAGAGCACCCCCCCCAAGAAGAAGCGCAAGGTGGAGGACAGCGCCAGCGACTACAAGGACGAC
GACGACAAGGTGAGCAAGGGCGAGGAGCTGTTCACCGGCGTGGTGCCCATCCTGGTGGAGCTGGACGGCGACGTG
AACGGCCACAAGTTCAGCGTGAGCGGCGAGGGCGAGGGCGACGCCACCTATGGCAAGCTGACCCTGAAGTTCATC
TGTACCACCGGCAAGCTGCCCGTGCCCTGGCCCACCCTGGTGACCACCCTGACCTAGGGCGTGCAGTGTTTCAGC
CGCTACCCCGACCACATGAAGCAGCACGACTTCTTCAAGAGCGCCATGCCCGAGGGCTACGTGCAGGAGCGCACC
ATCTTCTTCAAGGACGACGGCAACTACAAGACCCGCGCCGAGGTGAAGTTCGAGGGCGACACCCTGGTGAACCGC
ATCGAGCTGAAGGGCATCGACTTCAAGGAGGACGGCAACATCCTGGGCCACAAGCTGGAGTACAACTACAACAGC
CACAACGTGTACATCATGGCCGACAAGCAGAAGAACGGCATCAAGGCCAACTTCAAGATCCGCCACAACATCGAG
GACGGCAGCGTGCAGCTGGCCGACCACTACCAGCAGAACACCCCCATCGGCGACGGCCCCGTGCTGCTGCCCGAC
AACCACTACCTGAGCACCCAGAGCGCCCTGAGCAAGGACCCCAACGAGAAGCGCGACCACATGGTGCTGCTGGAG
TTCGTGACCGCCGCCGGCATCACCCTGGGCATGGACGAGCTGTACAAGCATCACCATCACCATCACTAAGCGGCC
GCATCGATTATAAGCTTTGTACACCTAGAAAACATGAGGATCACCCATGTCTGCACCTCGACACTAGAAAACATG
AGGATCACCCATGT (SEQ ID NO: 237)

Protein: X indicates non-canonical amino acid
MGRLESTPPKKKRKVEDSASDYKDDDDKVSKGEELFTGVVPILVELDGDVNGHKFSVSGEGEGDATYGKLTLKFI
CTTGKLPVPWPTLVTTLTXGVQCFSRYPDHMKQHDFFKSAMPEGYVQERTIFFKDDGNYKTRAEVKFEGDTLVNR
IELKGIDFKEDGNILGHKLEYNYNSHNVYIMADKQKNGIKANFKIRHNIEDGSVQLADHYQQNTPIGDGPVLLPD
NHYLSTQSALSKDPNEKRDHMVLLEFVTAAGITLGMDELYKHHHHHH(SEQ ID NO: 238)

GFP66TCG        GFP with Serine site
DNA: (MS2-stem loops, Serine codon)

ATGGGCCGCCTGGAGAGCACCCCCCCCAAGAAGAAGCGCAAGGTGGAGGACAGCGCCAGCGACTACAAGGACGAC
GACGACAAGGTGAGCAAGGGCGAGGAGCTGTTCACCGGCGTGGTGCCCATCCTGGTGGAGCTGGACGGCGACGTG
AACGGCCACAAGTTCAGCGTGAGCGGCGAGGGCGAGGGCGACGCCACCTATGGCAAGCTGACCCTGAAGTTCATC
TGTACCACCGGCAAGCTGCCCGTGCCCTGGCCCACCCTGGTGACCACCCTGACCTCGGGCGTGCAGTGTTTCAGC
CGCTACCCCGACCACATGAAGCAGCACGACTTCTTCAAGAGCGCCATGCCCGAGGGCTACGTGCAGGAGCGCACC
ATCTTCTTCAAGGACGACGGCAACTACAAGACCCGCGCCGAGGTGAAGTTCGAGGGCGACACCCTGGTGAACCGC
ATCGAGCTGAAGGGCATCGACTTCAAGGAGGACGGCAACATCCTGGGCCACAAGCTGGAGTACAACTACAACAGC
CACAACGTGTACATCATGGCCGACAAGCAGAAGAACGGCATCAAGGCCAACTTCAAGATCCGCCACAACATCGAG
GACGGCAGCGTGCAGCTGGCCGACCACTACCAGCAGAACACCCCCATCGGCGACGGCCCCGTGCTGCTGCCCGAC
AACCACTACCTGAGCACCCAGAGCGCCCTGAGCAAGGACCCCAACGAGAAGCGCGACCACATGGTGCTGCTGGAG
TTCGTGACCGCCGCCGGCATCACCCTGGGCATGGACGAGCTGTACAAGCATCACCATCACCATCACTAAGCGGCC
GCATCGATTATAAGCTTTGTACACCTAGAAAACATGAGGATCACCCATGTCTGCACCTCGACACTAGAAAACATG
AGGATCACCCATGT (SEQ ID NO: 239)

Protein: X indicates non-canonical amino acid
MGRLESTPPKKKRKVEDSASDYKDDDDKVSKGEELFTGVVPILVELDGDVNGHKFSVSGEGEGDATYGKLTLKFI
CTTGKLPVPWPTLVTTLTXGVQCFSRYPDHMKQHDFFKSAMPEGYVQERTIFFKDDGNYKTRAEVKFEGDTLVNR
IELKGIDFKEDGNILGHKLEYNYNSHNVYIMADKQKNGIKANFKIRHNIEDGSVQLADHYQQNTPIGDGPVLLPD
NHYLSTQSALSKDPNEKRDHMVLLEFVTAAGITLGMDELYKHHHHHH(SEQ ID NO: 240)

GFP66CCG GFP with Proline site
DNA: (MS2-stem loops, Proline codon)
ATGGGCCGCCTGGAGAGCACCCCCCCCAAGAAGAAGCGCAAGGTGGAGGACAGCGCCAGCGACTACAAGGACGAC
GACGACAAGGTGAGCAAGGGCGAGGAGCTGTTCACCGGCGTGGTGCCCATCCTGGTGGAGCTGGACGGCGACGTG
AACGGCCACAAGTTCAGCGTGAGCGGCGAGGGCGAGGGCGACGCCACCTATGGCAAGCTGACCCTGAAGTTCATC
TGTACCACCGGCAAGCTGCCCGTGCCCTGGCCCACCCTGGTGACCACCCTGACCCCGGGCGTGCAGTGTTTCAGC
CGCTACCCCGACCACATGAAGCAGCACGACTTCTTCAAGAGCGCCATGCCCGAGGGCTACGTGCAGGAGCGCACC
ATCTTCTTCAAGGACGACGGCAACTACAAGACCCGCGCCGAGGTGAAGTTCGAGGGCGACACCCTGGTGAACCGC
ATCGAGCTGAAGGGCATCGACTTCAAGGAGGACGGCAACATCCTGGGCCACAAGCTGGAGTACAACTACAACAGC
CACAACGTGTACATCATGGCCGACAAGCAGAAGAACGGCATCAAGGCCAACTTCAAGATCCGCCACAACATCGAG
GACGGCAGCGTGCAGCTGGCCGACCACTACCAGCAGAACACCCCCATCGGCGACGGCCCCGTGCTGCTGCCCGAC
AACCACTACCTGAGCACCCAGAGCGCCCTGAGCAAGGACCCCAACGAGAAGCGCGACCACATGGTGCTGCTGGAG
TTCGTGACCGCCGCCGGCATCACCCTGGGCATGGACGAGCTGTACAAGCATCACCATCACCATCACTAAGCGGCC
GCATCGATTATAAGCTTTGTACACCTAGAAAACATGAGGATCACCCATGTCTGCACCTCGACACTAGAAAACATG
AGGATCACCCATGT (SEQ ID NO: 241)

Protein: X indicates non-canonical amino acid
MGRLESTPPKKKRKVEDSASDYKDDDDKVSKGEELFTGVVPILVELDGDVNGHKFSVSGEGEGDATYGKLTLKFI
CTTGKLPVPWPTLVTTLTXGVQCFSRYPDHMKQHDFFKSAMPEGYVQERTIFFKDDGNYKTRAEVKFEGDTLVNR
IELKGIDFKEDGNILGHKLEYNYNSHNVYIMADKQKNGIKANFKIRHNIEDGSVQLADHYQQNTPIGDGPVLLPD
NHYLSTQSALSKDPNEKRDHMVLLEFVTAAGITLGMDELYKHHHHHH (SEQ ID NO: 242)

GFP66CTA GFP with Leucine site
DNA: (MS2-stem loops, Leucine codon)
ATGGGCCGCCTGGAGAGCACCCCCCCCAAGAAGAAGCGCAAGGTGGAGGACAGCGCCAGCGACTACAAGGACGAC
GACGACAAGGTGAGCAAGGGCGAGGAGCTGTTCACCGGCGTGGTGCCCATCCTGGTGGAGCTGGACGGCGACGTG
AACGGCCACAAGTTCAGCGTGAGCGGCGAGGGCGAGGGCGACGCCACCTATGGCAAGCTGACCCTGAAGTTCATC
TGTACCACCGGCAAGCTGCCCGTGCCCTGGCCCACCCTGGTGACCACCCTGACCCTAGGCGTGCAGTGTTTCAGC
CGCTACCCCGACCACATGAAGCAGCACGACTTCTTCAAGAGCGCCATGCCCGAGGGCTACGTGCAGGAGCGCACC
ATCTTCTTCAAGGACGACGGCAACTACAAGACCCGCGCCGAGGTGAAGTTCGAGGGCGACACCCTGGTGAACCGC
ATCGAGCTGAAGGGCATCGACTTCAAGGAGGACGGCAACATCCTGGGCCACAAGCTGGAGTACAACTACAACAGC
CACAACGTGTACATCATGGCCGACAAGCAGAAGAACGGCATCAAGGCCAACTTCAAGATCCGCCACAACATCGAG
GACGGCAGCGTGCAGCTGGCCGACCACTACCAGCAGAACACCCCCATCGGCGACGGCCCCGTGCTGCTGCCCGAC
AACCACTACCTGAGCACCCAGAGCGCCCTGAGCAAGGACCCCAACGAGAAGCGCGACCACATGGTGCTGCTGGAG
TTCGTGACCGCCGCCGGCATCACCCTGGGCATGGACGAGCTGTACAAGCATCACCATCACCATCACTAAGCGGCC
GCATCGATTATAAGCTTTGTACACCTAGAAAACATGAGGATCACCCATGTCTGCACCTCGACACTAGAAAACATG
AGGATCACCCATGT (SEQ ID NO: 243)

Protein: X indicates non-canonical amino acid
MGRLESTPPKKKRKVEDSASDYKDDDDKVSKGEELFTGVVPILVELDGDVNGHKFSVSGEGEGDATYGKLTLKFI
CTTGKLPVPWPTLVTTLTXGVQCFSRYPDHMKQHDFFKSAMPEGYVQERTIFFKDDGNYKTRAEVKFEGDTLVNR
IELKGIDFKEDGNILGHKLEYNYNSHNVYIMADKQKNGIKANFKIRHNIEDGSVQLADHYQQNTPIGDGPVLLPD
NHYLSTQSALSKDPNEKRDHMVLLEFVTAAGITLGMDELYKHHHHHH (SEQ ID NO: 244)

GFP66TTA GFP with Leucine site
DNA: (MS2-stem loops, Leucine codon)
ATGGGCCGCCTGGAGAGCACCCCCCCCAAGAAGAAGCGCAAGGTGGAGGACAGCGCCAGCGACTACAAGGACGAC
GACGACAAGGTGAGCAAGGGCGAGGAGCTGTTCACCGGCGTGGTGCCCATCCTGGTGGAGCTGGACGGCGACGTG
AACGGCCACAAGTTCAGCGTGAGCGGCGAGGGCGAGGGCGACGCCACCTATGGCAAGCTGACCCTGAAGTTCATC
TGTACCACCGGCAAGCTGCCCGTGCCCTGGCCCACCCTGGTGACCACCCTGACCTTAGGCGTGCAGTGTTTCAGC
CGCTACCCCGACCACATGAAGCAGCACGACTTCTTCAAGAGCGCCATGCCCGAGGGCTACGTGCAGGAGCGCACC
ATCTTCTTCAAGGACGACGGCAACTACAAGACCCGCGCCGAGGTGAAGTTCGAGGGCGACACCCTGGTGAACCGC
ATCGAGCTGAAGGGCATCGACTTCAAGGAGGACGGCAACATCCTGGGCCACAAGCTGGAGTACAACTACAACAGC
CACAACGTGTACATCATGGCCGACAAGCAGAAGAACGGCATCAAGGCCAACTTCAAGATCCGCCACAACATCGAG
GACGGCAGCGTGCAGCTGGCCGACCACTACCAGCAGAACACCCCCATCGGCGACGGCCCCGTGCTGCTGCCCGAC
AACCACTACCTGAGCACCCAGAGCGCCCTGAGCAAGGACCCCAACGAGAAGCGCGACCACATGGTGCTGCTGGAG
TTCGTGACCGCCGCCGGCATCACCCTGGGCATGGACGAGCTGTACAAGCATCACCATCACCATCACTAAGCGGCC
GCATCGATTATAAGCTTTGTACACCTAGAAAACATGAGGATCACCCATGTCTGCACCTCGACACTAGAAAACATG
AGGATCACCCATGT (SEQ ID NO: 245)

Protein: X indicates non-canonical amino acid
MGRLESTPPKKKRKVEDSASDYKDDDDKVSKGEELFTGVVPILVELDGDVNGHKFSVSGEGEGDATYGKLTLKFI
CTTGKLPVPWPTLVTTLTXGVQCFSRYPDHMKQHDFFKSAMPEGYVQERTIFFKDDGNYKTRAEVKFEGDTLVNR
IELKGIDFKEDGNILGHKLEYNYNSHNVYIMADKQKNGIKANFKIRHNIEDGSVQLADHYQQNTPIGDGPVLLPD
NHYLSTQSALSKDPNEKRDHMVLLEFVTAAGITLGMDELYKHHHHHH (SEQ ID NO: 246)

GFP66ATA GFP with Isoleucine site
DNA: (MS2-stem loops, Isoleucine codon)
ATGGGCCGCCTGGAAAGCACCCCGCCGAAAAAAAAACGCAAAGTGGAAGATAGCGCGAGCGATTACAAAGATGAT
GATGATAAAGTGAGCAAGGGCGAGGAGCTGTTCACCGGGGTGGTGCCCATCCTGGTCGAGCTGGACGGCGACGTA
AACGGCCACAAGTTCAGCGTGTCCGGCGAGGGCGAGGGCGATGCCACCTATGGCAAGCTGACCCTGAAGTTCATC
TGCACCACCGGCAAGCTGCCCGTGCCCTGGCCCACCCTCGTGACCACCCTGACCATAGGCGTGCAGTGCTTCAGC
CGCTACCCCGACCACATGAAGCAGCACGACTTCTTCAAGTCCGCCATGCCCGAAGGCTACGTCCAGGAGCGCACC
ATCTTCTTCAAGGACGACGGCAACTACAAGACCCGCGCCGAGGTGAAGTTCGAGGGCGACACCCTGGTGAACCGC
ATCGAGCTGAAGGGCATCGACTTCAAGGAGGACGGCAACATCCTGGGGCACAAGCTGGAGTACAACTACAACAGC
CACAACGTCTATATCATGGCCGACAAGCAGAAGAACGGCATCAAGGCCAACTTCAAGATCCGCCACAACATCGAG
GACGGCAGCGTGCAGCTCGCCGACCACTACCAGCAGAACACCCCCATCGGCGACGGCCCCGTGCTGCTGCCCGAC
AACCACTACCTGAGCACCCAGTCCGCCCTGAGCAAAGACCCCAACGAGAAGCGCGATCACATGGTCCTGCTGGAG
TTCGTGACCGCCGCCGGGATCACTCTCGGCATGGACGAGCTGTACAAGCATCACCATCACCATCACTAAGCGGCC
GCATCGATTATAAGCTTTGTACACCTAGAAAACATGAGGATCACCCATGTCTGCACCTCGACACTAGAAAACATG
AGGATCACCCATGT (SEQ ID NO: 247)

Protein: X indicates non-canonical amino acid
MGRLESTPPKKKRKVEDSASDYKDDDDKVSKGEELFTGVVPILVELDGDVNGHKFSVSGEGEGDATYGKLTLKFI
CTTGKLPVPWPTLVTTLTXGVQCFSRYPDHMKQHDFFKSAMPEGYVQERTIFFKDDGNYKTRAEVKFEGDTLVNR
IELKGIDFKEDGNILGHKLEYNYNSHNVYIMADKQKNGIKANFKIRHNIEDGSVQLADHYQQNTPIGDGPVLLPD
NHYLSTQSALSKDPNEKRDHMVLLEFVTAAGITLGMDELYKHHHHHH (SEQ ID NO: 248)

GFP66CGG GFP with Arginine site
DNA: (MS2-stem loops, Arginine codon)
ATGGGCCGCCTGGAGAGCACCCCCCCCAAGAAGAAGCGCAAGGTGGAGGACAGCGCCAGCGACTACAAGGACGAC
GACGACAAGGTGAGCAAGGGCGAGGAGCTGTTCACCGGCGTGGTGCCCATCCTGGTGGAGCTGGACGGCGACGTG
AACGGCCACAAGTTCAGCGTGAGCGGCGAGGGCGAGGGCGACGCCACCTATGGCAAGCTGACCCTGAAGTTCATC
TGTACCACCGGCAAGCTGCCCGTGCCCTGGCCCACCCTGGTGACCACCCTGACCCGGGGCGTGCAGTGTTTCAGC
CGCTACCCCGACCACATGAAGCAGCACGACTTCTTCAAGAGCGCCATGCCCGAGGGCTACGTGCAGGAGCGCACC
ATCTTCTTCAAGGACGACGGCAACTACAAGACCCGCGCCGAGGTGAAGTTCGAGGGCGACACCCTGGTGAACCGC

ATCGAGCTGAAGGGCATCGACTTCAAGGAGGACGGCAACATCCTGGGCCACAAGCTGGAGTACAACTACAACAGC
CACAACGTGTACATCATGGCCGACAAGCAGAAGAACGGCATCAAGGCCAACTTCAAGATCCGCCACAACATCGAG
GACGGCAGCGTGCAGCTGGCCGACCACTACCAGCAGAACACCCCCATCGGCGACGGCCCCGTGCTGCTGCCCGAC
AACCACTACCTGAGCACCCAGAGCGCCCTGAGCAAGGACCCCAACGAGAAGCGCGACCACATGGTGCTGCTGGAG
TTCGTGACCGCCGCCGGCATCACCCTGGGCATGGACGAGCTGTACAAGCACCACCACCACCACCACTAAAAGCTT
TGTACACCTAGAAAACATGAGGATCACCCATGTCTGCACCTCGACACTAGAAAACATGAGGATCACCCATGT
(SEQ ID NO: 249)

Protein: X indicates non-canonical amino acid
MGRLESTPPKKKRKVEDSASDYKDDDDKVSKGEELFTGVVPILVELDGDVNGHKFSVSGEGEGDATYGKLTLKFI
CTTGKLPVPWPTLVTTLTXGVQCFSRYPDHMKQHDFFKSAMPEGYVQERTIFFKDDGNYKTRAEVKFEGDTLVNR
IELKGIDFKEDGNILGHKLEYNYNSHNVYIMADKQKNGIKANFKIRHNIEDGSVQLADHYQQNTPIGDGPVLLPD
NHYLSTQSALSKDPNEKRDHMVLLEFVTAAGITLGMDELYKHHHHHH (SEQ ID NO: 250)

GFP39TCG GFP with Serine site
DNA: (MS2-stem loops, Serine codon)
ATGGGCCGCCTGGAGAGCACCCCCCCCAAGAAGAAGCGCAAGGTGGAGGACAGCGCCAGCGACTACAAGGACGAC
GACGACAAGGTGAGCAAGGGCGAGGAGCTGTTCACCGGCGTGGTGCCCATCCTGGTGGAGCTGGACGGCGACGTG
AACGGCCACAAGTTCAGCGTGAGCGGCGAGGGCGAGGGCGACGCCACCTCGGGCAAGCTGACCCTGAAGTTCATC
TGTACCACCGGCAAGCTGCCCGTGCCCTGGCCCACCCTGGTGACCACCCTGACCTACGGCGTGCAGTGTTTCAGC
CGCTACCCCGACCACATGAAGCAGCACGACTTCTTCAAGAGCGCCATGCCCGAGGGCTACGTGCAGGAGCGCACC
ATCTTCTTCAAGGACGACGGCAACTACAAGACCCGCGCCGAGGTGAAGTTCGAGGGCGACACCCTGGTGAACCGC
ATCGAGCTGAAGGGCATCGACTTCAAGGAGGACGGCAACATCCTGGGCCACAAGCTGGAGTACAACTACAACAGC
CACAACGTGTACATCATGGCCGACAAGCAGAAGAACGGCATCAAGGCCAACTTCAAGATCCGCCACAACATCGAG
GACGGCAGCGTGCAGCTGGCCGACCACTACCAGCAGAACACCCCCATCGGCGACGGCCCCGTGCTGCTGCCCGAC
AACCACTACCTGAGCACCCAGAGCGCCCTGAGCAAGGACCCCAACGAGAAGCGCGACCACATGGTGCTGCTGGAG
TTCGTGACCGCCGCCGGCATCACCCTGGGCATGGACGAGCTGTACAAGCACCACCACCACCACCATAAGGATCCT
AAGGATCCTAATTGCCTAGAAAACATGAGGATCACCCATGTCTGCAGGTCGACTCTAGAAAACATGAGGATCACC
CATGT (SEQ ID NO: 251)

Protein: X indicates non-canonical amino acid
MGRLESTPPKKKRKVEDSASDYKDDDDKVSKGEELFTGVVPILVELDGDVNGHKFSVSGEGEGDATXGKLTLKFI
CTTGKLPVPWPTLVTTLTYGVQCFSRYPDHMKQHDFFKSAMPEGYVQERTIFFKDDGNYKTRAEVKFEGDTLVNR
IELKGIDFKEDGNILGHKLEYNYNSHNVYIMADKQKNGIKANFKIRHNIEDGSVQLADHYQQNTPIGDGPVLLPD
NHYLSTQSALSKDPNEKRDHMVLLEFVTAAGITLGMDELYKHHHHHHK (SEQ ID NO: 252)

GFP39CCG GFP with Proline site
DNA: (MS2-stem loops, Proline codon
ATGGGCCGCCTGGAGAGCACCCCCCCCAAGAAGAAGCGCAAGGTGGAGGACAGCGCCAGCGACTACAAGGACGAC
GACGACAAGGTGAGCAAGGGCGAGGAGCTGTTCACCGGCGTGGTGCCCATCCTGGTGGAGCTGGACGGCGACGTG
AACGGCCACAAGTTCAGCGTGAGCGGCGAGGGCGAGGGCGACGCCACCCCGGGCAAGCTGACCCTGAAGTTCATC
TGTACCACCGGCAAGCTGCCCGTGCCCTGGCCCACCCTGGTGACCACCCTGACCTACGGCGTGCAGTGTTTCAGC
CGCTACCCCGACCACATGAAGCAGCACGACTTCTTCAAGAGCGCCATGCCCGAGGGCTACGTGCAGGAGCGCACC
ATCTTCTTCAAGGACGACGGCAACTACAAGACCCGCGCCGAGGTGAAGTTCGAGGGCGACACCCTGGTGAACCGC
ATCGAGCTGAAGGGCATCGACTTCAAGGAGGACGGCAACATCCTGGGCCACAAGCTGGAGTACAACTACAACAGC
CACAACGTGTACATCATGGCCGACAAGCAGAAGAACGGCATCAAGGCCAACTTCAAGATCCGCCACAACATCGAG
GACGGCAGCGTGCAGCTGGCCGACCACTACCAGCAGAACACCCCCATCGGCGACGGCCCCGTGCTGCTGCCCGAC
AACCACTACCTGAGCACCCAGAGCGCCCTGAGCAAGGACCCCAACGAGAAGCGCGACCACATGGTGCTGCTGGAG
TTCGTGACCGCCGCCGGCATCACCCTGGGCATGGACGAGCTGTACAAGCACCACCACCACCACCACTAAAGATCC
TAAGGATCCTAATTGCCTAGAAAACATGAGGATCACCCATGTCTGCAGGTCGACTCTAGAAAACATGAGGATCAC
CCATGT (SEQ ID NO: 253)

Protein: X indicates non-canonical amino acid
MGRLESTPPKKKRKVEDSASDYKDDDDKVSKGEELFTGVVPILVELDGDVNGHKFSVSGEGEGDATXGKLTLKFI
CTTGKLPVPWPTLVTTLTYGVQCFSRYPDHMKQHDFFKSAMPEGYVQERTIFFKDDGNYKTRAEVKFEGDTLVNR
IELKGIDFKEDGNILGHKLEYNYNSHNVYIMADKQKNGIKANFKIRHNIEDGSVQLADHYQQNTPIGDGPVLLPD
NHYLSTQSALSKDPNEKRDHMVLLEFVTAAGITLGMDELYKHHHHHHK (SEQ ID NO: 254)

GFP^{39CTA} GFP with Leucine site
DNA: (MS2-stem loops, Leucine codon)
ATGGGCCGCCTGGAGAGCACCCCCCCCAAGAAGAAGCGCAAGGTGGAGGACAGCGCCAGCGACTACAAGGACGAC
GACGACAAGGTGAGCAAGGGCGAGGAGCTGTTCACCGGCGTGGTGCCCATCCTGGTGGAGCTGGACGGCGACGTG
AACGGCCACAAGTTCAGCGTGAGCGGCGAGGGCGAGGGCGACGCCACCCTAGGCAAGCTGACCCTGAAGTTCATC
TGTACCACCGGCAAGCTGCCCGTGCCCTGGCCCACCCTGGTGACCACCCTGACCTACGGCGTGCAGTGTTTCAGC
CGCTACCCCGACCACATGAAGCAGCACGACTTCTTCAAGAGCGCCATGCCCGAGGGCTACGTGCAGGAGCGCACC
ATCTTCTTCAAGGACGACGGCAACTACAAGACCCGCGCCGAGGTGAAGTTCGAGGGCGACACCCTGGTGAACCGC
ATCGAGCTGAAGGGCATCGACTTCAAGGAGGACGGCAACATCCTGGGCCACAAGCTGGAGTACAACTACAACAGC
CACAACGTGTACATCATGGCCGACAAGCAGAAGAACGGCATCAAGGCCAACTTCAAGATCCGCCACAACATCGAG
GACGGCAGCGTGCAGCTGGCCGACCACTACCAGCAGAACACCCCCATCGGCGACGGCCCCGTGCTGCTGCCCGAC
AACCACTACCTGAGCACCCAGAGCGCCCTGAGCAAGGACCCCAACGAGAAGCGCGACCACATGGTGCTGCTGGAG
TTCGTGACCGCCGCCGGCATCACCCTGGGCATGGACGAGCTGTACAAGCACCACCACCACCACCACTAAAGATCC
TAAGGATCCTAATTGCCTAGAAAACATGAGGATCACCCATGTCTGCAGGTCGACTCTAGAAAACATGAGGATCAC
CCATGT (SEQ ID NO: 255)

Protein: X indicates non-canonical amino acid
MGRLESTPPKKKRKVEDSASDYKDDDDKVSKGEELFTGVVPILVELDGDVNGHKFSVSGEGEGDATXGKLTLKFI
CTTGKLPVPWPTLVTTLTYGVQCFSRYPDHMKQHDFFKSAMPEGYVQERTIFFKDDGNYKTRAEVKFEGDTLVNR
IELKGIDFKEDGNILGHKLEYNYNSHNVYIMADKQKNGIKANFKIRHNIEDGSVQLADHYQQNTPIGDGPVLLPD
NHYLSTQSALSKDPNEKRDHMVLLEFVTAAGITLGMDELYKHHHHHHK (SEQ ID NO: 256)

GFP^{39CGG} GFP with Arginine site
DNA: (MS2-stem loops, Arginine codon)
ATGGGCCGCCTGGAGAGCACCCCCCCCAAGAAGAAGCGCAAGGTGGAGGACAGCGCCAGCGACTACAAGGACGAC
GACGACAAGGTGAGCAAGGGCGAGGAGCTGTTCACCGGCGTGGTGCCCATCCTGGTGGAGCTGGACGGCGACGTG
AACGGCCACAAGTTCAGCGTGAGCGGCGAGGGCGAGGGCGACGCCACCCGGGGCAAGCTGACCCTGAAGTTCATC
TGTACCACCGGCAAGCTGCCCGTGCCCTGGCCCACCCTGGTGACCACCCTGACCTACGGCGTGCAGTGTTTCAGC
CGCTACCCCGACCACATGAAGCAGCACGACTTCTTCAAGAGCGCCATGCCCGAGGGCTACGTGCAGGAGCGCACC
ATCTTCTTCAAGGACGACGGCAACTACAAGACCCGCGCCGAGGTGAAGTTCGAGGGCGACACCCTGGTGAACCGC
ATCGAGCTGAAGGGCATCGACTTCAAGGAGGACGGCAACATCCTGGGCCACAAGCTGGAGTACAACTACAACAGC
CACAACGTGTACATCATGGCCGACAAGCAGAAGAACGGCATCAAGGCCAACTTCAAGATCCGCCACAACATCGAG
GACGGCAGCGTGCAGCTGGCCGACCACTACCAGCAGAACACCCCCATCGGCGACGGCCCCGTGCTGCTGCCCGAC
AACCACTACCTGAGCACCCAGAGCGCCCTGAGCAAGGACCCCAACGAGAAGCGCGACCACATGGTGCTGCTGGAG
TTCGTGACCGCCGCCGGCATCACCCTGGGCATGGACGAGCTGTACAAGCACCACCACCACCACCACTAAAGATCC
TAAGGATCCTAATTGCCTAGAAAACATGAGGATCACCCATGTCTGCAGGTCGACTCTAGAAAACATGAGGATCAC
CCATGTC (SEQ ID NO: 257)

Protein: X indicates non-canonical amino acid
MGRLESTPPKKKRKVEDSASDYKDDDDKVSKGEELFTGVVPILVELDGDVNGHKFSVSGEGEGDATXGKLTLKFI
CTTGKLPVPWPTLVTTLTYGVQCFSRYPDHMKQHDFFKSAMPEGYVQERTIFFKDDGNYKTRAEVKFEGDTLVNR
IELKGIDFKEDGNILGHKLEYNYNSHNVYIMADKQKNGIKANFKIRHNIEDGSVQLADHYQQNTPIGDGPVLLPD
NHYLSTQSALSKDPNEKRDHMVLLEFVTAAGITLGMDELYKHHHHHHK (SEQ ID NO: 258)

mCherry^{72TAG} mCherry with Amber site
DNA: (MS2-stem loops, Amber codon)
ATGGGCCGCCTGGAAAGCACCCCGCCGAAAAAAAAACGCAAAGTGGAAGATAGCGCGAGCGATTACAAAGATGAT
GATGATAAAGTGGCGTACGCGGTGAGCAAGGGCGAGGAGGATAACATGGCCATCATCAAGGAGTTCATGCGCTTC
AAGGTGCACATGGAGGGCTCCGTGAACGGCCACGAGTTCGAGATCGAGGGCGAGGGCGAGGGCCGCCCCTACGAG
GGCACCCAGACCGCCAAGCTGAAGGTGACCAAGGGTGGCCCCCTGCCCTTCGCCTGGGACATCCTGTCCCCTCAG
TTCATGTAGGGCTCCAAGGCCTACGTGAAGCACCCCGCCGACATCCCCGACTACTTGAAGCTGTCCTTCCCCGAG
GGCTTCAAGTGGGAGCGCGTGATGAACTTCGAGGACGGCGGCGTGGTGACCGTGACCCAGGACTCCTCCCTGCAG
GACGGCGAGTTCATCTACAAGGTGAAGCTGCGCGGCACCAACTTCCCCTCCGACGGCCCCGTAATGCAGAAGAAG
ACGATGGGCTGGGAGGCCTCCTCCGAGCGGATGTACCCCGAGGACGGCGCCCTGAAGGGCGAGATCAAGCAGAGG
CTGAAGCTGAAGGACGGCGGCCACTACGACGCTGAGGTCAAGACCACCTACAAGGCCAAGAAGCCCGTGCAGCTG
CCCGGCGCCTACAACGTCAACATCAAGTTGGACATCACCTCCCACAACGAGGACTACACCATCGTGGAACAGTAC
GAACGCGCCGAGGGCCGCCACTCCACCGGCGGCATGGACGAGCTGTACAAGCATCACCATCACCATCACTAAGCG

GCCGCATCGATTATAAGCTTTGTACACCTAGAAAACATGAGGATCACCCATGTCTGCACCTCGACACTAGAAAAC
ATGAGGATCACCCATGT (SEQ ID NO: 259)

Protein: X indicates non-canonical amino acid
MGRLESTPPKKKRKVEDSASDYKDDDDKVAYAVSKGEEDNMAIIKEFMRFKVHMEGSVNGHEFEIEGEGEGRPYE
GTQTAKLKVTKGGPLPFAWDILSPQFMXGSKAYVKHPADIPDYLKLSFPEGFKWERVMNFEDGGVVTVTQDSSLQ
DGEFIYKVKLRGTNFPSDGPVMQKKTMGWEASSERMYPEDGALKGEIKQRLKLKDGGHYDAEVKTTYKAKKPVQL
PGAYNVNIKLDITSHNEDYTIVEQYERAEGRHSTGGMDELYKHHHHHH (SEQ ID NO: 260)

mCherry72TCG mCherry with Serine site
DNA: (MS2-stem loops, Serine codon)
ATGGGCCGCCTGGAAAGCACCCCGCCGAAAAAAAAACGCAAAGTGGAAGATAGCGCGAGCGATTACAAAGATGAT
GATGATAAAGTGGCGTACGCGGTGAGCAAGGGCGAGGAGGATAACATGGCCATCATCAAGGAGTTCATGCGCTTC
AAGGTGCACATGGAGGGCTCCGTGAACGGCCACGAGTTCGAGATCGAGGGCGAGGGCGAGGGCCGCCCCTACGAG
GGCACCCAGACCGCCAAGCTGAAGGTGACCAAGGGTGGCCCCCTGCCCTTCGCCTGGGACATCCTGTCCCCTCAG
TTCATGTCGGGCTCCAAGGCCTACGTGAAGCACCCCGCCGACATCCCCGACTACTTGAAGCTGTCCTTCCCCGAG
GGCTTCAAGTGGGAGCGCGTGATGAACTTCGAGGACGGCGGCGTGGTGACCGTGACCCAGGACTCCTCCCTGCAG
GACGGCGAGTTCATCTACAAGGTGAAGCTGCGCGGCACCAACTTCCCCTCCGACGGCCCCGTAATGCAGAAGAAG
ACGATGGGCTGGGAGGCCTCCTCCGAGCGGATGTACCCCGAGGACGGCGCCCTGAAGGGCGAGATCAAGCAGAGG
CTGAAGCTGAAGGACGGCGGCCACTACGACGCTGAGGTCAAGACCACCTACAAGGCCAAGAAGCCCGTGCAGCTG
CCCGGCGCCTACAACGTCAACATCAAGTTGGACATCACCTCCCACAACGAGGACTACACCATCGTGGAACAGTAC
GAACGCGCCGAGGGCCGCCACTCCACCGGCGGCATGGACGAGCTGTACAAGCATCACCATCACCATCACTAAGCG
GCCGCATCGATTATAAGCTTTGTACACCTAGAAAACATGAGGATCACCCATGTCTGCACCTCGACACTAGAAAAC
ATGAGGATCACCCATGT (SEQ ID NO:261)

Protein: X indicates non-canonical amino acid
MGRLESTPPKKKRKVEDSASDYKDDDDKVAYAVSKGEEDNMAIIKEFMRFKVHMEGSVNGHEFEIEGEGEGRPYE
GTQTAKLKVTKGGPLPFAWDILSPQFMXGSKAYVKHPADIPDYLKLSFPEGFKWERVMNFEDGGVVTVTQDSSLQ
DGEFIYKVKLRGTNFPSDGPVMQKKTMGWEASSERMYPEDGALKGEIKQRLKLKDGGHYDAEVKTTYKAKKPVQL
PGAYNVNIKLDITSHNEDYTIVEQYERAEGRHSTGGMDELYKHHHHHH (SEQ ID NO:262)

mCherry72CCG mCherry with Proline site
DNA: (MS2-stem loops, Proline codon)
ATGGGCCGCCTGGAAAGCACCCCGCCGAAAAAAAAACGCAAAGTGGAAGATAGCGCGAGCGATTACAAAGATGAT
GATGATAAAGTGGCGTACGCGGTGAGCAAGGGCGAGGAGGATAACATGGCCATCATCAAGGAGTTCATGCGCTTC
AAGGTGCACATGGAGGGCTCCGTGAACGGCCACGAGTTCGAGATCGAGGGCGAGGGCGAGGGCCGCCCCTACGAG
GGCACCCAGACCGCCAAGCTGAAGGTGACCAAGGGTGGCCCCCTGCCCTTCGCCTGGGACATCCTGTCCCCTCAG
TTCATGCCGGGCTCCAAGGCCTACGTGAAGCACCCCGCCGACATCCCCGACTACTTGAAGCTGTCCTTCCCCGAG
GGCTTCAAGTGGGAGCGCGTGATGAACTTCGAGGACGGCGGCGTGGTGACCGTGACCCAGGACTCCTCCCTGCAG
GACGGCGAGTTCATCTACAAGGTGAAGCTGCGCGGCACCAACTTCCCCTCCGACGGCCCCGTAATGCAGAAGAAG
ACGATGGGCTGGGAGGCCTCCTCCGAGCGGATGTACCCCGAGGACGGCGCCCTGAAGGGCGAGATCAAGCAGAGG
CTGAAGCTGAAGGACGGCGGCCACTACGACGCTGAGGTCAAGACCACCTACAAGGCCAAGAAGCCCGTGCAGCTG
CCCGGCGCCTACAACGTCAACATCAAGTTGGACATCACCTCCCACAACGAGGACTACACCATCGTGGAACAGTAC
GAACGCGCCGAGGGCCGCCACTCCACCGGCGGCATGGACGAGCTGTACAAGCATCACCATCACCATCACTAAGCG
GCCGCATCGATTATAAGCTTTGTACACCTAGAAAACATGAGGATCACCCATGTCTGCACCTCGACACTAGAAAAC
ATGAGGATCACCCATGT (SEQ ID NO: 263)

Protein: X indicates non-canonical amino acid
MGRLESTPPKKKRKVEDSASDYKDDDDKVAYAVSKGEEDNMAIIKEFMRFKVHMEGSVNGHEFEIEGEGEGRPYE
GTQTAKLKVTKGGPLPFAWDILSPQFMXGSKAYVKHPADIPDYLKLSFPEGFKWERVMNFEDGGVVTVTQDSSLQ
DGEFIYKVKLRGTNFPSDGPVMQKKTMGWEASSERMYPEDGALKGEIKQRLKLKDGGHYDAEVKTTYKAKKPVQL
PGAYNVNIKLDITSHNEDYTIVEQYERAEGRHSTGGMDELYKHHHHHH (SEQ ID NO: 264)

mCherry72CTA mCherry with Leucine site
DNA: (MS2-stem loops, Leucine codon)
ATGGGCCGCCTGGAAAGCACCCCGCCGAAAAAAAAACGCAAAGTGGAAGATAGCGCGAGCGATTACAAAGATGAT
GATGATAAAGTGGCGTACGCGGTGAGCAAGGGCGAGGAGGATAACATGGCCATCATCAAGGAGTTCATGCGCTTC
AAGGTGCACATGGAGGGCTCCGTGAACGGCCACGAGTTCGAGATCGAGGGCGAGGGCGAGGGCCGCCCCTACGAG

GGCACCCAGACCGCCAAGCTGAAGGTGACCAAGGGTGGCCCCCTGCCCTTCGCCTGGGACATCCTGTCCCCTCAG
TTCATGCTAGGCTCCAAGGCCTACGTGAAGCACCCCGCCGACATCCCCGACTACTTGAAGCTGTCCTTCCCCGAG
GGCTTCAAGTGGGAGCGCGTGATGAACTTCGAGGACGGCGGCGTGGTGACCGTGACCCAGGACTCCTCCCTGCAG
GACGGCGAGTTCATCTACAAGGTGAAGCTGCGCGGCACCAACTTCCCCTCCGACGGCCCCGTAATGCAGAAGAAG
ACGATGGGCTGGGAGGCCTCCTCCGAGCGGATGTACCCCGAGGACGGCGCCCTGAAGGGCGAGATCAAGCAGAGG
CTGAAGCTGAAGGACGGCGGCCACTACGACGCTGAGGTCAAGACCACCTACAAGGCCAAGAAGCCCGTGCAGCTG
CCCGGCGCCTACAACGTCAACATCAAGTTGGACATCACCTCCCACAACGAGGACTACACCATCGTGGAACAGTAC
GAACGCGCCGAGGGCCGCCACTCCACCGGCGGCATGGACGAGCTGTACAAGCATCACCATCACCATCACTAAGCG
GCCGCATCGATTATAAGCTTTGTACACCTAGAAAACATGAGGATCACCCATGTCTGCACCTCGACACTAGAAAAC
ATGAGGATCACCCATGT (SEQ ID NO: 265)

Protein: X indicates non-canonical amino acid
MGRLESTPPKKKRKVEDSASDYKDDDDKVAYAVSKGEEDNMAIIKEFMRFKVHMEGSVNGHEFEIEGEGEGRPYE
GTQTAKLKVTKGGPLPFAWDILSPQFMXGSKAYVKHPADIPDYLKLSFPEGFKWERVMNFEDGGVVTVTQDSSLQ
DGEFIYKVKLRGTNFPSDGPVMQKKTMGWEASSERMYPEDGALKGEIKQRLKLKDGGHYDAEVKTTYKAKKPVQL
PGAYNVNIKLDITSHNEDYTIVEQYERAEGRHSTGGMDELYKHHHHHH (SEQ ID NO: 266)

mCherry72TTA mCherry with Leucine site
DNA: (MS2-stem loops, Leucine codon)
ATGGGCCGCCTGGAAAGCACCCCGCCGAAAAAAAAACGCAAAGTGGAAGATAGCGCGAGCGATTACAAAGATGAT
GATGATAAAGTGGCGTACGCGGTGAGCAAGGGCGAGGAGGATAACATGGCCATCATCAAGGAGTTCATGCGCTTC
AAGGTGCACATGGAGGGCTCCGTGAACGGCCACGAGTTCGAGATCGAGGGCGAGGGCGAGGGCCGCCCCTACGAG
GGCACCCAGACCGCCAAGCTGAAGGTGACCAAGGGTGGCCCCCTGCCCTTCGCCTGGGACATCCTGTCCCCTCAG
TTCATGTTAGGCTCCAAGGCCTACGTGAAGCACCCCGCCGACATCCCCGACTACTTGAAGCTGTCCTTCCCCGAG
GGCTTCAAGTGGGAGCGCGTGATGAACTTCGAGGACGGCGGCGTGGTGACCGTGACCCAGGACTCCTCCCTGCAG
GACGGCGAGTTCATCTACAAGGTGAAGCTGCGCGGCACCAACTTCCCCTCCGACGGCCCCGTAATGCAGAAGAAG
ACGATGGGCTGGGAGGCCTCCTCCGAGCGGATGTACCCCGAGGACGGCGCCCTGAAGGGCGAGATCAAGCAGAGG
CTGAAGCTGAAGGACGGCGGCCACTACGACGCTGAGGTCAAGACCACCTACAAGGCCAAGAAGCCCGTGCAGCTG
CCCGGCGCCTACAACGTCAACATCAAGTTGGACATCACCTCCCACAACGAGGACTACACCATCGTGGAACAGTAC
GAACGCGCCGAGGGCCGCCACTCCACCGGCGGCATGGACGAGCTGTACAAGCATCACCATCACCATCACTAAGCG
GCCGCATCGATTATAAGCTTTGTACACCTAGAAAACATGAGGATCACCCATGTCTGCACCTCGACACTAGAAAAC
ATGAGGATCACCCATGT (SEQ ID NO: 267)

Protein: X indicates non-canonical amino acid
MGRLESTPPKKKRKVEDSASDYKDDDDKVAYAVSKGEEDNMAIIKEFMRFKVHMEGSVNGHEFEIEGEGEGRPYE
GTQTAKLKVTKGGPLPFAWDILSPQFMXGSKAYVKHPADIPDYLKLSFPEGFKWERVMNFEDGGVVTVTQDSSLQ
DGEFIYKVKLRGTNFPSDGPVMQKKTMGWEASSERMYPEDGALKGEIKQRLKLKDGGHYDAEVKTTYKAKKPVQL
PGAYNVNIKLDITSHNEDYTIVEQYERAEGRHSTGGMDELYKHHHHHH (SEQ ID NO: 268)

mCherry72ATA mCherry with Isoleucine site
DNA: (MS2-stem loops, Isoleucine codon)
ATGGGCCGCCTGGAAAGCACCCCGCCGAAAAAAAAACGCAAAGTGGAAGATAGCGCGAGCGATTACAAAGATGAT
GATGATAAAGTGGCGTACGCGGTGAGCAAGGGCGAGGAGGATAACATGGCCATCATCAAGGAGTTCATGCGCTTC
AAGGTGCACATGGAGGGCTCCGTGAACGGCCACGAGTTCGAGATCGAGGGCGAGGGCGAGGGCCGCCCCTACGAG
GGCACCCAGACCGCCAAGCTGAAGGTGACCAAGGGTGGCCCCCTGCCCTTCGCCTGGGACATCCTGTCCCCTCAG
TTCATGATAGGCTCCAAGGCCTACGTGAAGCACCCCGCCGACATCCCCGACTACTTGAAGCTGTCCTTCCCCGAG
GGCTTCAAGTGGGAGCGCGTGATGAACTTCGAGGACGGCGGCGTGGTGACCGTGACCCAGGACTCCTCCCTGCAG
GACGGCGAGTTCATCTACAAGGTGAAGCTGCGCGGCACCAACTTCCCCTCCGACGGCCCCGTAATGCAGAAGAAG
ACGATGGGCTGGGAGGCCTCCTCCGAGCGGATGTACCCCGAGGACGGCGCCCTGAAGGGCGAGATCAAGCAGAGG
CTGAAGCTGAAGGACGGCGGCCACTACGACGCTGAGGTCAAGACCACCTACAAGGCCAAGAAGCCCGTGCAGCTG
CCCGGCGCCTACAACGTCAACATCAAGTTGGACATCACCTCCCACAACGAGGACTACACCATCGTGGAACAGTAC
GAACGCGCCGAGGGCCGCCACTCCACCGGCGGCATGGACGAGCTGTACAAGCATCACCATCACCATCACTAAGCG
GCCGCATCGATTATAAGCTTTGTACACCTAGAAAACATGAGGATCACCCATGTCTGCACCTCGACACTAGAAAAC
ATGAGGATCACCCATGT (SEQ ID NO: 269)

Protein: X indicates non-canonical amino acid
MGRLESTPPKKKRKVEDSASDYKDDDDKVAYAVSKGEEDNMAIIKEFMRFKVHMEGSVNGHEFEIEGEGEGRPYE
GTQTAKLKVTKGGPLPFAWDILSPQFMXGSKAYVKHPADIPDYLKLSFPEGFKWERVMNFEDGGVVTVTQDSSLQ

DGEFIYKVKLRGTNFPSDGPVMQKKTMGWEASSERMYPEDGALKGEIKQRLKLKDGGHYDAEVKTTYKAKKPVQL
PGAYNVNIKLDITSHNEDYTIVEQYERAEGRHSTGGMDELYKHHHHHH (SEQ ID NO: 270)

mCherry^{185TCG} mCherry with Serine site
DNA: (Serine codon)
ATGGGCCGCCTGGAGAGCACCCCCCCCAAGAAGAAGCGCAAGGTGGAGGACAGCGCCAGCGTGAGCAAGGGCGAG
GAGGACAACATGGCCATCATCAAGGAGTTCATGCGCTTCAAGGTGCACATGGAGGGCAGCGTGAACGGCCACGAG
TTCGAGATCGAGGGCGAGGGCGAGGGCCGCCCCTACGAGGGCACCCAGACCGCCAAGCTGAAGGTGACCAAGGGC
GGCCCCCTGCCCTTCGCCTGGGACATCCTGAGCCCCCAGTTCATGTACGGCAGCAAGGCCTACGTGAAGCACCCC
GCCGACATCCCCGACTACCTGAAGCTGAGCTTCCCCGAGGGCTTCAAGTGGGAGCGCGTGATGAACTTCGAGGAC
GGCGGCGTGGTGACCGTGACCCAGGACAGCAGCCTGCAGGACGGCGAGTTCATCTACAAGGTGAAGCTGCGCGGC
ACCAACTTCCCCAGCGACGGCCCCGTGATGCAGAAGAAGACCATGGGCTGGGAGGCCAGCAGCGAGCGCATGTAC
CCCGAGGACGGCGCCCTGAAGGGCGAGATCAAGCAGCGCCTGAAGCTGAAGGACGGCGGCCACTACGACGCCGAG
GTGAAGACCACCTACAAGGCCAAGTCGCCCGTGCAGCTGCCCGGCGCCTACAACGTGAACATCAAGCTGGACATC
ACCAGCCACAACGAGGACTACACCATCGTGGAGCAGTACGAGCGCGCCGAGGGCCGCCACAGCACCGGCGGCATG
GACGAGCTGTACAAGCACCACCACCACCACCACTAA (SEQ ID NO: 271)

Protein: X indicates non-canonical amino acid
MGRLESTPPKKKRKVEDSASVSKGEEDNMAIIKEFMRFKVHMEGSVNGHEFEIEGEGEGRPYEGTQTAKLKVTKG
GPLPFAWDILSPQFMYGSKAYVKHPADIPDYLKLSFPEGFKWERVMNFEDGGVVTVTQDSSLQDGEFIYKVKLRG
TNFPSDGPVMQKKTMGWEASSERMYPEDGALKGEIKQRLKLKDGGHYDAEVKTTYKAKXPVQLPGAYNVNIKLDI
TSHNEDYTIVEQYERAEGRHSTGGMDELYKHHHHHH (SEQ ID NO: 272)

mCherry^{185CCG} mCherry with Proline site
DNA: (Proline codon)
ATGGGCCGCCTGGAGAGCACCCCCCCCAAGAAGAAGCGCAAGGTGGAGGACAGCGCCAGCGTGAGCAAGGGCGAG
GAGGACAACATGGCCATCATCAAGGAGTTCATGCGCTTCAAGGTGCACATGGAGGGCAGCGTGAACGGCCACGAG
TTCGAGATCGAGGGCGAGGGCGAGGGCCGCCCCTACGAGGGCACCCAGACCGCCAAGCTGAAGGTGACCAAGGGC
GGCCCCCTGCCCTTCGCCTGGGACATCCTGAGCCCCCAGTTCATGTACGGCAGCAAGGCCTACGTGAAGCACCCC
GCCGACATCCCCGACTACCTGAAGCTGAGCTTCCCCGAGGGCTTCAAGTGGGAGCGCGTGATGAACTTCGAGGAC
GGCGGCGTGGTGACCGTGACCCAGGACAGCAGCCTGCAGGACGGCGAGTTCATCTACAAGGTGAAGCTGCGCGGC
ACCAACTTCCCCAGCGACGGCCCCGTGATGCAGAAGAAGACCATGGGCTGGGAGGCCAGCAGCGAGCGCATGTAC
CCCGAGGACGGCGCCCTGAAGGGCGAGATCAAGCAGCGCCTGAAGCTGAAGGACGGCGGCCACTACGACGCCGAG
GTGAAGACCACCTACAAGGCCAAGCCGCCCGTGCAGCTGCCCGGCGCCTACAACGTGAACATCAAGCTGGACATC
ACCAGCCACAACGAGGACTACACCATCGTGGAGCAGTACGAGCGCGCCGAGGGCCGCCACAGCACCGGCGGCATG
GACGAGCTGTACAAGCACCACCACCACCACCACTAA (SEQ ID NO: 273)

Protein: X indicates non-canonical amino acid
MGRLESTPPKKKRKVEDSASVSKGEEDNMAIIKEFMRFKVHMEGSVNGHEFEIEGEGEGRPYEGTQTAKLKVTKG
GPLPFAWDILSPQFMYGSKAYVKHPADIPDYLKLSFPEGFKWERVMNFEDGGVVTVTQDSSLQDGEFIYKVKLRG
TNFPSDGPVMQKKTMGWEASSERMYPEDGALKGEIKQRLKLKDGGHYDAEVKTTYKAKXPVQLPGAYNVNIKLDI
TSHNEDYTIVEQYERAEGRHSTGGMDELYKHHHHHH (SEQ ID NO: 274)

mCherry^{185CTA} mCherry with Leucine site
DNA: (Leucine codon)
ATGGGCCGCCTGGAGAGCACCCCCCCCAAGAAGAAGCGCAAGGTGGAGGACAGCGCCAGCGTGAGCAAGGGCGAG
GAGGACAACATGGCCATCATCAAGGAGTTCATGCGCTTCAAGGTGCACATGGAGGGCAGCGTGAACGGCCACGAG
TTCGAGATCGAGGGCGAGGGCGAGGGCCGCCCCTACGAGGGCACCCAGACCGCCAAGCTGAAGGTGACCAAGGGC
GGCCCCCTGCCCTTCGCCTGGGACATCCTGAGCCCCCAGTTCATGTACGGCAGCAAGGCCTACGTGAAGCACCCC
GCCGACATCCCCGACTACCTGAAGCTGAGCTTCCCCGAGGGCTTCAAGTGGGAGCGCGTGATGAACTTCGAGGAC
GGCGGCGTGGTGACCGTGACCCAGGACAGCAGCCTGCAGGACGGCGAGTTCATCTACAAGGTGAAGCTGCGCGGC
ACCAACTTCCCCAGCGACGGCCCCGTGATGCAGAAGAAGACCATGGGCTGGGAGGCCAGCAGCGAGCGCATGTAC
CCCGAGGACGGCGCCCTGAAGGGCGAGATCAAGCAGCGCCTGAAGCTGAAGGACGGCGGCCACTACGACGCCGAG
GTGAAGACCACCTACAAGGCCAAGCTACCCGTGCAGCTGCCCGGCGCCTACAACGTGAACATCAAGCTGGACATC
ACCAGCCACAACGAGGACTACACCATCGTGGAGCAGTACGAGCGCGCCGAGGGCCGCCACAGCACCGGCGGCATG
GACGAGCTGTACAAGCACCACCACCACCACCACTAA (SEQ ID NO: 275)

Protein: X indicates non-canonical amino acid

MGRLESTPPKKKRKVEDSASVSKGEEDNMAIIKEFMRFKVHMEGSVNGHEFEIEGEGEGRPYEGTQTAKLKVTKG
GPLPFAWDILSPQFMYGSKAYVKHPADIPDYLKLSFPEGFKWERVMNFEDGGVVTVTQDSSLQDGEFIYKVKLRG
TNFPSDGPVMQKKTMGWEASSERMYPEDGALKGEIKQRLKLKDGGHYDAEVKTTYKAKXPVQLPGAYNVNIKLDI
TSHNEDYTIVEQYERAEGRHSTGGMDELYKHHHHHH (SEQ ID NO: 276)

GFP^{39TCG}      LCK-GFP with Serine site
DNA: (MS2-stem loops, Serine codon in red, LCK in blue)
ATGGGCTGCGTGTGCAGCAGCAACCCCGAGGGTACCGAGCTCGTGAGCAAGGGCGAGGAGCTGTTCACCGGCGTG
GTGCCCATCCTGGTGGAGCTGGACGGCGACGTGAACGGCCACAAGTTCAGCGTGAGCGGCGAGGGCGAGGGCGAC
GCCACCTCGGGCAAGCTGACCCTGAAGTTCATCTGTACCACCGGCAAGCTGCCCGTGCCCTGGCCCACCCTGGTG
ACCACCCTGACCTACGGCGTGCAGTGTTTCAGCCGCTACCCCGACCACATGAAGCAGCACGACTTCTTCAAGAGC
GCCATGCCCGAGGGCTACGTGCAGGAGCGCACCATCTTCTTCAAGGACGACGGCAACTACAAGACCCGCGCCGAG
GTGAAGTTCGAGGGCGACACCCTGGTGAACCGCATCGAGCTGAAGGGCATCGACTTCAAGGAGGACGGCAACATC
CTGGGCCACAAGCTGGAGTACAACTACAACAGCCACAACGTGTACATCATGGCCGACAAGCAGAAGAACGGCATC
AAGGCCAACTTCAAGATCCGCCACAACATCGAGGACGGCAGCGTGCAGCTGGCCGACCACTACCAGCAGAACACC
CCCATCGGCGACGGCCCCGTGCTGCTGCCCGACAACCACTACCTGAGCACCCAGAGCGCCCTGAGCAAGGACCCC
AACGAGAAGCGCGACCACATGGTGCTGCTGGAGTTCGTGACCGCCGCCGGCATCACCCTGGGCATGGACGAGCTG
TACAAGCACCACCACCACCACCACTAAAAGCTTTGTACACCTAGAAAACATGAGGATCACCCATGTCTGCACCTC
GACACTAGAAAACATGAGGATCACCCATGT (SEQ ID NO: 277)

Protein: X indicates non-canonical amino acid
MGCVCSSNPEGTELVSKGEELFTGVVPILVELDGDVNGHKFSVSGEGEGDATXGKLTLKFICTTGKLPVPWPTLV
TTLTYGVQCFSRYPDHMKQHDFFKSAMPEGYVQERTIFFKDDGNYKTRAEVKFEGDTLVNRIELKGIDFKEDGNI
LGHKLEYNYNSHNVYIMADKQKNGIKANFKIRHNIEDGSVQLADHYQQNTPIGDGPVLLPDNHYLSTQSALSKDP
NEKRDHMVLLEFVTAAGITLGMDELYKHHHHHH (SEQ ID NO: 278)

GFP^{39CCG}      LCK-GFP with Proline site
DNA: (MS2-stem loops, Proline codon, LCK)
ATGGGCTGCGTGTGCAGCAGCAACCCCGAGGGTACCGAGCTCGTGAGCAAGGGCGAGGAGCTGTTCACCGGCGTG
GTGCCCATCCTGGTGGAGCTGGACGGCGACGTGAACGGCCACAAGTTCAGCGTGAGCGGCGAGGGCGAGGGCGAC
GCCACCCCGGGCAAGCTGACCCTGAAGTTCATCTGTACCACCGGCAAGCTGCCCGTGCCCTGGCCCACCCTGGTG
ACCACCCTGACCTACGGCGTGCAGTGTTTCAGCCGCTACCCCGACCACATGAAGCAGCACGACTTCTTCAAGAGC
GCCATGCCCGAGGGCTACGTGCAGGAGCGCACCATCTTCTTCAAGGACGACGGCAACTACAAGACCCGCGCCGAG
GTGAAGTTCGAGGGCGACACCCTGGTGAACCGCATCGAGCTGAAGGGCATCGACTTCAAGGAGGACGGCAACATC
CTGGGCCACAAGCTGGAGTACAACTACAACAGCCACAACGTGTACATCATGGCCGACAAGCAGAAGAACGGCATC
AAGGCCAACTTCAAGATCCGCCACAACATCGAGGACGGCAGCGTGCAGCTGGCCGACCACTACCAGCAGAACACC
CCCATCGGCGACGGCCCCGTGCTGCTGCCCGACAACCACTACCTGAGCACCCAGAGCGCCCTGAGCAAGGACCCC
AACGAGAAGCGCGACCACATGGTGCTGCTGGAGTTCGTGACCGCCGCCGGCATCACCCTGGGCATGGACGAGCTG
TACAAGCACCACCACCACCACCACTAAAAGCTTTGTACACCTAGAAAACATGAGGATCACCCATGTCTGCACCTC
GACACTAGAAAACATGAGGATCACCCATGT (SEQ ID NO: 279)

Protein: X indicates non-canonical amino acid
MGCVCSSNPEGTELVSKGEELFTGVVPILVELDGDVNGHKFSVSGEGEGDATXGKLTLKFICTTGKLPVPWPTLV
TTLTYGVQCFSRYPDHMKQHDFFKSAMPEGYVQERTIFFKDDGNYKTRAEVKFEGDTLVNRIELKGIDFKEDGNI
LGHKLEYNYNSHNVYIMADKQKNGIKANFKIRHNIEDGSVQLADHYQQNTPIGDGPVLLPDNHYLSTQSALSKDP
NEKRDHMVLLEFVTAAGITLGMDELYKHHHHHH (SEQ ID NO: 280)

GFP^{39CTA}      LCK-GFP with Leucine site
DNA: (MS2-stem loops, Leucine codon, LCK)
ATGGGCTGCGTGTGCAGCAGCAACCCCGAGGGTACCGAGCTCGTGAGCAAGGGCGAGGAGCTGTTCACCGGCGTG
GTGCCCATCCTGGTGGAGCTGGACGGCGACGTGAACGGCCACAAGTTCAGCGTGAGCGGCGAGGGCGAGGGCGAC
GCCACCCTAGGCAAGCTGACCCTGAAGTTCATCTGTACCACCGGCAAGCTGCCCGTGCCCTGGCCCACCCTGGTG
ACCACCCTGACCTACGGCGTGCAGTGTTTCAGCCGCTACCCCGACCACATGAAGCAGCACGACTTCTTCAAGAGC
GCCATGCCCGAGGGCTACGTGCAGGAGCGCACCATCTTCTTCAAGGACGACGGCAACTACAAGACCCGCGCCGAG
GTGAAGTTCGAGGGCGACACCCTGGTGAACCGCATCGAGCTGAAGGGCATCGACTTCAAGGAGGACGGCAACATC
CTGGGCCACAAGCTGGAGTACAACTACAACAGCCACAACGTGTACATCATGGCCGACAAGCAGAAGAACGGCATC
AAGGCCAACTTCAAGATCCGCCACAACATCGAGGACGGCAGCGTGCAGCTGGCCGACCACTACCAGCAGAACACC
CCCATCGGCGACGGCCCCGTGCTGCTGCCCGACAACCACTACCTGAGCACCCAGAGCGCCCTGAGCAAGGACCCC

AACGAGAAGCGCGACCACATGGTGCTGCTGGAGTTCGTGACCGCCGCCGGCATCACCCTGGGCATGGACGAGCTG
TACAAGCACCACCACCACCACCACTAAAAGCTTTGTACACCTAGAAAACATGAGGATCACCCATGTCTGCACCTC
GACACTAGAAAACATGAGGATCACCCATGT (SEQ ID NO: 281)

Protein: X indicates non-canonical amino acid
MGCVCSSNPEGTELVSKGEELFTGVVPILVELDGDVNGHKFSVSGEGEGDATXGKLTLKFICTTGKLPVPWPTLV
TTLTYGVQCFSRYPDHMKQHDFFKSAMPEGYVQERTIFFKDDGNYKTRAEVKFEGDTLVNRIELKGIDFKEDGNI
LGHKLEYNYNSHNVYIMADKQKNGIKANFKIRHNIEDGSVQLADHYQQNTPIGDGPVLLPDNHYLSTQSALSKDP
NEKRDHMVLLEFVTAAGITLGMDELYKHHHHHH (SEQ ID NO: 282)

Extended GFP39TCG GFP with Serine site at position 39 genetically fused to
GFP66CCG
DNA: (MS2-stem loops, Serine codon , )
ATGGGCCGCCTGGAGAGCACCCCCCCCAAGAAGAAGCGCAAGGTGGAGGACAGCGCCAGCGACTACAAGGACGAC
GACGACAAGGTGAGCAAGGGCGAGGAGCTGTTCACCGGCGTGGTGCCCATCCTGGTGGAGCTGGACGGCGACGTG
AACGGCCACAAGTTCAGCGTGAGCGGCGAGGGCGAGGGCGACGCCACCTCGGGCAAGCTGACCCTGAAGTTCATC
TGTACCACCGGCAAGCTGCCCGTGCCCTGGCCCACCCTGGTGACCACCCTGACCTACGGCGTGCAGTGTTTCAGC
CGCTACCCCGACCACATGAAGCAGCACGACTTCTTCAAGAGCGCCATGCCCGAGGGCTACGTGCAGGAGCGCACC
ATCTTCTTCAAGGACGACGGCAACTACAAGACCCGCGCCGAGGTGAAGTTCGAGGGCGACACCCTGGTGAACCGC
ATCGAGCTGAAGGGCATCGACTTCAAGGAGGACGGCAACATCCTGGGCCACAAGCTGGAGTACAACTACAACAGC
CACAACGTGTACATCATGGCCGACAAGCAGAAGAACGGCATCAAGGCCAACTTCAAGATCCGCCACAACATCGAG
GACGGCAGCGTGCAGCTGGCCGACCACTACCAGCAGAACACCCCCATCGGCGACGGCCCCGTGCTGCTGCCCGAC
AACCACTACCTGAGCACCCAGAGCGCCCTGAGCAAGGACCCCAACGAGAAGCGCGACCACATGGTGCTGCTGGAG
TTCGTGACCGCCGCCGGCATCACCCTGGGCATGGACGAGCTGTACAAGGGAGCACCAGGAAGTGCTGGTTCTGCT
GCTGGTAGTGGAGTGAGCAAGGGCGAGGAGCTGTTCACCGGCGTGGTGCCCATCCTGGTGGAGCTGGACGGCGAC
GTGAACGGCCACAAGTTCAGCGTGAGCGGCGAGGGCGAGGGCGACGCCACCTATGGCAAGCTGACCCTGAAGTTC
ATCTGTACCACCGGCAAGCTGCCCGTGCCCTGGCCCACCCTGGTGACCACCCTGACC   GGCGTGCAGTGTTTC
AGCCGCTACCCCGACCACATGAAGCAGCACGACTTCTTCAAGAGCGCCATGCCCGAGGGCTACGTGCAGGAGCGC
ACCATCTTCTTCAAGGACGACGGCAACTACAAGACCCGCGCCGAGGTGAAGTTCGAGGGCGACACCCTGGTGAAC
CGCATCGAGCTGAAGGGCATCGACTTCAAGGAGGACGGCAACATCCTGGGCCACAAGCTGGAGTACAACTACAAC
AGCCACAACGTGTACATCATGGCCGACAAGCAGAAGAACGGCATCAAGGCCAACTTCAAGATCCGCCACAACATC
GAGGACGGCAGCGTGCAGCTGGCCGACCACTACCAGCAGAACACCCCCATCGGCGACGGCCCCGTGCTGCTGCCC
GACAACCACTACCTGAGCACCCAGAGCGCCCTGAGCAAGGACCCCAACGAGAAGCGCGACCACATGGTGCTGCTG
GAGTTCGTGACCGCCGCCGGCATCACCCTGGGCATGGACGAGCTGTACAAGCACCACCACCACCACCACTAAAAG
CTTTGTACACCTAGAAAACATGAGGATCACCCATGTCTGCACCTCGACACTAGAAAACATGAGGATCACCCATGT
(SEQ ID NO: 283)

Protein: X indicates non-canonical amino acid
MGRLESTPPKKKRKVEDSASDYKDDDDKVSKGEELFTGVVPILVELDGDVNGHKFSVSGEGEGDATXGKLTLKFI
CTTGKLPVPWPTLVTTLTYGVQCFSRYPDHMKQHDFFKSAMPEGYVQERTIFFKDDGNYKTRAEVKFEGDTLVNR
IELKGIDFKEDGNILGHKLEYNYNSHNVYIMADKQKNGIKANFKIRHNIEDGSVQLADHYQQNTPIGDGPVLLPD
NHYLSTQSALSKDPNEKRDHMVLLEFVTAAGITLGMDELYKGAPGSAGSAAGSGVSKGEELFTGVVPILVELDGD
VNGHKFSVSGEGEGDATYGKLTLKFICTTGKLPVPWPTLVTTLTPGVQCFSRYPDHMKQHDFFKSAMPEGYVQER
TIFFKDDGNYKTRAEVKFEGDTLVNRIELKGIDFKEDGNILGHKLEYNYNSHNVYIMADKQKNGIKANFKIRHNI
EDGSVQLADHYQQNTPIGDGPVLLPDNHYLSTQSALSKDPNEKRDHMVLLEFVTAAGITLGMDELYKHHHHHH
(SEQ ID NO: 284)

Extended GFP39CCG GFP with Proline site at position 39 genetically fused
toGFP66TCG

DNA: (MS2-stem loops, Proline codon, )
ATGGGCCGCCTGGAGAGCACCCCCCCCAAGAAGAAGCGCAAGGTGGAGGACAGCGCCAGCGACTACAAGGACGAC
GACGACAAGGTGAGCAAGGGCGAGGAGCTGTTCACCGGCGTGGTGCCCATCCTGGTGGAGCTGGACGGCGACGTG
AACGGCCACAAGTTCAGCGTGAGCGGCGAGGGCGAGGGCGACGCCACCCCGGGCAAGCTGACCCTGAAGTTCATC
TGTACCACCGGCAAGCTGCCCGTGCCCTGGCCCACCCTGGTGACCACCCTGACCTACGGCGTGCAGTGTTTCAGC
CGCTACCCCGACCACATGAAGCAGCACGACTTCTTCAAGAGCGCCATGCCCGAGGGCTACGTGCAGGAGCGCACC
ATCTTCTTCAAGGACGACGGCAACTACAAGACCCGCGCCGAGGTGAAGTTCGAGGGCGACACCCTGGTGAACCGC
ATCGAGCTGAAGGGCATCGACTTCAAGGAGGACGGCAACATCCTGGGCCACAAGCTGGAGTACAACTACAACAGC

CACAACGTGTACATCATGGCCGACAAGCAGAAGAACGGCATCAAGGCCAACTTCAAGATCCGCCACAACATCGAG
GACGGCAGCGTGCAGCTGGCCGACCACTACCAGCAGAACACCCCCATCGGCGACGGCCCCGTGCTGCTGCCCGAC
AACCACTACCTGAGCACCCAGAGCGCCCTGAGCAAGGACCCCAACGAGAAGCGCGACCACATGGTGCTGCTGGAG
TTCGTGACCGCCGCCGGCATCACCCTGGGCATGGACGAGCTGTACAAGGGAGCACCAGGAAGTGCTGGTTCTGCT
GCTGGTAGTGGAGTGAGCAAGGGCGAGGAGCTGTTCACCGGCGTGGTGCCCATCCTGGTGGAGCTGGACGGCGAC
GTGAACGGCCACAAGTTCAGCGTGAGCGGCGAGGGCGAGGGCGACGCCACCTATGGCAAGCTGACCCTGAAGTTC
ATCTGTACCACCGGCAAGCTGCCCGTGCCCTGGCCCACCCTGGTGACCACCCTGACC    GGCGTGCAGTGTTTC
AGCCGCTACCCCGACCACATGAAGCAGCACGACTTCTTCAAGAGCGCCATGCCCGAGGGCTACGTGCAGGAGCGC
ACCATCTTCTTCAAGGACGACGGCAACTACAAGACCCGCGCCGAGGTGAAGTTCGAGGGCGACACCCTGGTGAAC
CGCATCGAGCTGAAGGGCATCGACTTCAAGGAGGACGGCAACATCCTGGGCCACAAGCTGGAGTACAACTACAAC
AGCCACAACGTGTACATCATGGCCGACAAGCAGAAGAACGGCATCAAGGCCAACTTCAAGATCCGCCACAACATC
GAGGACGGCAGCGTGCAGCTGGCCGACCACTACCAGCAGAACACCCCCATCGGCGACGGCCCCGTGCTGCTGCCC
GACAACCACTACCTGAGCACCCAGAGCGCCCTGAGCAAGGACCCCAACGAGAAGCGCGACCACATGGTGCTGCTG
GAGTTCGTGACCGCCGCCGGCATCACCCTGGGCATGGACGAGCTGTACAAGCATCACCATCACCATCACTAAGCG
GCCGCATCGATTATAAGCTTTGTACACCTAGAAAACATGAGGATCACCCATGTCTGCACCTCGACACTAGAAAAC
ATGAGGATCACCCATGT (SEQ ID NO: 285)

Protein: X indicates non-canonical amino acid
MGRLESTPPKKKRKVEDSASDYKDDDDKVSKGEELFTGVVPILVELDGDVNGHKFSVSGEGEGDATXGKLTLKFI
CTTGKLPVPWPTLVTTLTYGVQCFSRYPDHMKQHDFFKSAMPEGYVQERTIFFKDDGNYKTRAEVKFEGDTLVNR
IELKGIDFKEDGNILGHKLEYNYNSHNVYIMADKQKNGIKANFKIRHNIEDGSVQLADHYQQNTPIGDGPVLLPD
NHYLSTQSALSKDPNEKRDHMVLLEFVTAAGITLGMDELYKGAPGSAGSAAGSGVSKGEELFTGVVPILVELDGD
VNGHKFSVSGEGEGDATYGKLTLKFICTTGKLPVPWPTLVTTLTSGVQCFSRYPDHMKQHDFFKSAMPEGYVQER
TIFFKDDGNYKTRAEVKFEGDTLVNRIELKGIDFKEDGNILGHKLEYNYNSHNVYIMADKQKNGIKANFKIRHNI
EDGSVQLADHYQQNTPIGDGPVLLPDNHYLSTQSALSKDPNEKRDHMVLLEFVTAAGITLGMDELYKHHHHHH
(SEQ ID NO: 286)

Extended GFP39CTA GFP with Leucine site at position 39 genetically fused toGFP66TCG
DNA: (MS2-stem loops, Leucine codon,            )
ATGGGCCGCCTGGAGAGCACCCCCCCCAAGAAGAAGCGCAAGGTGGAGGACAGCGCCAGCGACTACAAGGACGAC
GACGACAAGGTGAGCAAGGGCGAGGAGCTGTTCACCGGCGTGGTGCCCATCCTGGTGGAGCTGGACGGCGACGTG
AACGGCCACAAGTTCAGCGTGAGCGGCGAGGGCGAGGGCGACGCCACCCTAGGCAAGCTGACCCTGAAGTTCATC
TGTACCACCGGCAAGCTGCCCGTGCCCTGGCCCACCCTGGTGACCACCCTGACCTACGGCGTGCAGTGTTTCAGC
CGCTACCCCGACCACATGAAGCAGCACGACTTCTTCAAGAGCGCCATGCCCGAGGGCTACGTGCAGGAGCGCACC
ATCTTCTTCAAGGACGACGGCAACTACAAGACCCGCGCCGAGGTGAAGTTCGAGGGCGACACCCTGGTGAACCGC
ATCGAGCTGAAGGGCATCGACTTCAAGGAGGACGGCAACATCCTGGGCCACAAGCTGGAGTACAACTACAACAGC
CACAACGTGTACATCATGGCCGACAAGCAGAAGAACGGCATCAAGGCCAACTTCAAGATCCGCCACAACATCGAG
GACGGCAGCGTGCAGCTGGCCGACCACTACCAGCAGAACACCCCCATCGGCGACGGCCCCGTGCTGCTGCCCGAC
AACCACTACCTGAGCACCCAGAGCGCCCTGAGCAAGGACCCCAACGAGAAGCGCGACCACATGGTGCTGCTGGAG
TTCGTGACCGCCGCCGGCATCACCCTGGGCATGGACGAGCTGTACAAGGGAGCACCAGGAAGTGCTGGTTCTGCT
GCTGGTAGTGGAGTGAGCAAGGGCGAGGAGCTGTTCACCGGCGTGGTGCCCATCCTGGTGGAGCTGGACGGCGAC
GTGAACGGCCACAAGTTCAGCGTGAGCGGCGAGGGCGAGGGCGACGCCACCTATGGCAAGCTGACCCTGAAGTTC
ATCTGTACCACCGGCAAGCTGCCCGTGCCCTGGCCCACCCTGGTGACCACCCTGACC    GGCGTGCAGTGTTTC
AGCCGCTACCCCGACCACATGAAGCAGCACGACTTCTTCAAGAGCGCCATGCCCGAGGGCTACGTGCAGGAGCGC
ACCATCTTCTTCAAGGACGACGGCAACTACAAGACCCGCGCCGAGGTGAAGTTCGAGGGCGACACCCTGGTGAAC
CGCATCGAGCTGAAGGGCATCGACTTCAAGGAGGACGGCAACATCCTGGGCCACAAGCTGGAGTACAACTACAAC
AGCCACAACGTGTACATCATGGCCGACAAGCAGAAGAACGGCATCAAGGCCAACTTCAAGATCCGCCACAACATC
GAGGACGGCAGCGTGCAGCTGGCCGACCACTACCAGCAGAACACCCCCATCGGCGACGGCCCCGTGCTGCTGCCC
GACAACCACTACCTGAGCACCCAGAGCGCCCTGAGCAAGGACCCCAACGAGAAGCGCGACCACATGGTGCTGCTG
GAGTTCGTGACCGCCGCCGGCATCACCCTGGGCATGGACGAGCTGTACAAGCATCACCATCACCATCACTAAGCG
GCCGCATCGATTATAAGCTTTGTACACCTAGAAAACATGAGGATCACCCATGTCTGCACCTCGACACTAGAAAAC
ATGAGGATCACCCATGT (SEQ ID NO: 287)

Protein: X indicates non-canonical amino acid
MGRLESTPPKKKRKVEDSASDYKDDDDKVSKGEELFTGVVPILVELDGDVNGHKFSVSGEGEGDATXGKLTLKFI
CTTGKLPVPWPTLVTTLTYGVQCFSRYPDHMKQHDFFKSAMPEGYVQERTIFFKDDGNYKTRAEVKFEGDTLVNR
IELKGIDFKEDGNILGHKLEYNYNSHNVYIMADKQKNGIKANFKIRHNIEDGSVQLADHYQQNTPIGDGPVLLPD

NHYLSTQSALSKDPNEKRDHMVLLEFVTAAGITLGMDELYKGAPGSAGSAAGSGVSKGEELFTGVVPILVELDGD
VNGHKFSVSGEGEGDATYGKLTLKFICTTGKLPVPWPTLVTTLTSGVQCFSRYPDHMKQHDFFKSAMPEGYVQER
TIFFKDDGNYKTRAEVKFEGDTLVNRIELKGIDFKEDGNILGHKLEYNYNSHNVYIMADKQKNGIKANFKIRHNI
EDGSVQLADHYQQNTPIGDGPVLLPDNHYLSTQSALSKDPNEKRDHMVLLEFVTAAGITLGMDELYKHHHHHH
(SEQ ID NO: 288)

pp7 Bacteriophage PP7 RNA stem-loops: (version 1)
DNA:

GGAGCAGACGATATGGCGTCGCTCC (SEQ ID NO: 289)

pp7 Bacteriophage PP7 RNA stem-loops: (version 2)
DNA sequence of

DNA:

CCAGCAGAGCATATGGGCTCGCTGG (SEQ ID NO: 290)

EBAG9: Receptor-binding cancer antigen expressed on SiSo cells (Homo
sapiens, Uniprot: O00559)
DNA:
ATGGCCATCACCCAGTTTCGGTTATTTAAATTTTGTACCTGCCTAGCAACAGTATTCTCATTCCTAAAGAGATTA
ATATGCAGATCTGGCAGAGGACGGAAATTAAGTGGAGACCAAATAACTTTGCCAACTACAGTTGATTATTCATCA
GTTCCTAAGCAGACAGATGTTGAAGAGTGGACTTCCTGGGATGAAGATGCACCCACCAGTGTAAAGATCGAAGGA
GGGAATGGGAATGTGGCAACACAACAAAATTCTTTGGAACAACTGGAACCTGACTATTTTAAGGACATGACACCA
ACTATTAGGAAAACTCAGAAAATTGTTATTAAGAAGAGAGAACCATTGAATTTTGGCATCCCAGATGGGAGCACA
GGTTTCTCTAGTAGATTAGCAGCTACACAAGATCTGCCTTTTATTCATCAGTCTTCTGAATTAGGTGACTTAGAT
ACCTGGCAGGAAAATACCAATGCATGGGAAGAAGAAGAAGATGCAGCCTGGCAAGCAGAAGAAGTTCTGAGACAG
CAGAAACTAGCAGACAGAGAAAAGAGAGCAGCCGAACAACAAAGGAAGAAAATGGAAAAGGAAGCACAACGGCTA
ATGAAGAAGGAACAAAACAAAATTGGTGTGAAACTTTCA (SEQ ID NO: 291)
Protein:
MAITQFRLFKFCTCLATVFSFLKRLICRSGRGRKLSGDQITLPTTVDYSSVPKQTDVEEWTSWDEDAPTSVKIEG
GNGNVATQQNSLEQLEPDYFKDMTPTIRKTQKIVIKKREPLNFGIPDGSTGFSSRLAATQDLPFIHQSSELGDLD
TWQENTNAWEEEEDAAWQAEEVLRQQKLADREKRAAEQQRKKMEKEAQRLMKKEQNKIGVKLS (SEQ ID NO:
292)

EBAG91-29: Receptor-binding cancer antigen expressed on SiSo cells (Homo
sapiens, Uniprot: O00559)
DNA:
ATGGCCATCACCCAGTTTCGGTTATTTAAATTTTGTACCTGCCTAGCAACAGTATTCTCATTCCTAAAGAGATTA
ATATGCAGATCT (SEQ ID NO: 293)

Protein:
MAITQFRLFKFCTCLATVFSFLKRLICRS (SEQ ID NO: 294)

CMP-SaTr/SLC35A1: CMP-sialic acid transporter (Homo sapiens, Uniprot:
P78382)
DNA:
ATGGCTGCCCCGAGAGACAATGTCACTTTATTATTCAAGTTATACTGCTTGGCAGTGATGACCCTGATGGCTGCA
GTCTATACCATAGCTTTAAGATACACAAGGACATCAGACAAAGAACTCTACTTTTCAACGACAGCCGTGTGTATC
ACAGAAGTTATAAAGTTATTGCTAAGTGTGGGAATTTTAGCTAAAGAAACTGGTAGTCTGGGTAGATTCAAAGCA
TCTTTAAGAGAAAATGTCTTGGGGAGCCCCAAGGAACTGTTGAAGTTAAGTGTGCCATCGTTAGTGTATGCTGTT
CAGAACAACATGGCTTTCCTAGCTCTTAGCAATCTGGATGCAGCAGTGTACCAGGTGACCTACCAGTTGAAGATT
CCGTGTACTGCTTTATGCACTGTTTTAATGTTAAATCGGACACTCAGCAAATTACAGTGGGTTTCAGTTTTTATG
CTGTGTGCTGGAGTTACGCTTGTACAGTGGAAACCAGCCCAAGCTACAAAAGTGGTGGTGGAACAAAATCCATTA

TTAGGGTTTGGCGCTATAGCTATTGCTGTATTGTGCTCAGGATTTGCAGGAGTATATTTTGAAAAAGTTTTAAAG
AGTTCAGATACTTCTCTTTGGGTGAGAAACATTCAAATGTATCTATCAGGGATTATTGTGACATTAGCTGGCGTC
TACTTGTCAGATGGAGCTGAAATTAAAGAAAAAGGATTTTTCTATGGTTACACATATTATGTCTGGTTTGTCATC
TTTCTTGCAAGTGTTGGTGGCCTCTACACTTCTGTTGTGGTTAAGTACACAGACAACATCATGAAAGGCTTTTCT
GCAGCAGCGGCCATTGTCCTTTCCACCATTGCTTCAGTAATGCTGTTTGGATTACAGATAACACTCACCTTTGCC
CTGGGTACTCTTCTTGTATGTGTTTCCATATATCTCTATGGATTACCCAGACAAGACACTACATCCATCCAACAA
GGAGAAACAGCTTCAAAGGAGAGAGTTATTGGTGTG (SEQ ID NO: 295)

Protein:
MAAPRDNVTLLFKLYCLAVMTLMAAVYTIALRYTRTSDKELYFSTTAVCITEVIKLLLSVGILAKETGSLGRFKA
SLRENVLGSPKELLKLSVPSLVYAVQNNMAFLALSNLDAAVYQVTYQLKIPCTALCTVLMLNRTLSKLQWVSVFM
LCAGVTLVQWKPAQATKVVVEQNPLLGFGAIAIAVLCSGFAGVYFEKVLKSSDTSLWVRNIQMYLSGIIVTLAGV
YLSDGAEIKEKGFFYGYTYYVWFVIFLASVGGLYTSVVVKYTDNIMKGFSAAAAIVLSTIASVMLFGLQITLTFA
LGTLLVCVSIYLYGLPRQDTTSIQQGETASKERVIGV (SEQ ID NO: 296)

P450 2C1₁₋₂₇: Cytochrome P450 2C1 (Oryctolagus cuniculus, Uniprot: P00180)
DNA:
ATGGACCCCGTGGTCGTGCTGGGCCTGTGCCTGTCATGCCTGCTGCTGCTGAGCCTGTGGAAGCAGAGCTACGGC
GGAGGC (SEQ ID NO: 297)

Protein:
MDPVVVLGLCLSCLLLLSLWKQSYGGG (SEQ ID NO: 298)

P450 2C1₁₋₂₉: Cytochrome P450 2C1 (Oryctolagus cuniculus, Uniprot: P00180)
DNA:
ATGGACCCCGTGGTCGTGCTGGGCCTGTGCCTGTCATGCCTGCTGCTGCTGAGCCTGTGGAAGCAGAGCTACGGC
GGAGGCAAGCTG (SEQ ID NO:299)

Protein:
MDPVVVLGLCLSCLLLLSLWKQSYGGGKL (SEQ ID NO:300)

EB1: Microtubule-associated protein RP/EB family member 1 (Homo sapiens,
Uniprot: Q8WQ86)
DNA:
ATGGCAGTGAACGTATACTCAACGTCAGTGACCAGTGATAACCTAAGTCGACATGACATGCTGGCCTGGATCAAT
GAGTCTCTGCAGTTGAATCTGACAAAGATCGAACAGTTGTGCTCAGGGGCTGCGTATTGTCAGTTTATGGACATG
CTGTTCCCTGGCTCCATTGCCTTGAAGAAAGTGAAATTCCAAGCTAAGCTAGAACACGAGTACATCCAGAACTTC
AAAATACTACAAGCAGGTTTTAAGAGAATGGGTGTTGACAAAATAATTCCTGTGGACAAATTAGTAAAAGGAAAG
TTTCAGGACAATTTTGAATTCGTTCAGTGGTTCAAGAAGTTTTTCGATGCAAACTATGATGGAAAAGACTATGAC
CCTGTGGCTGCCAGACAAGGTCAAGAAACTGCAGTGGCTCCTTCCCTTGTTGCTCCAGCTCTGAATAAACCGAAG
AAACCTCTCACTTCTAGCAGTGCAGCTCCCCAGAGGCCCATCTCAACACAGAGAACCGCTGCGGCTCCTAAGGCT
GGCCCTGGTGTGGTGCGAAAGAACCCTGGTGTGGGCAACGGAGATGACGAGGCAGCTGAGTTGATGCAGCAGGTC
AACGTATTGAAACTTACTGTTGAAGACTTGGAGAAAGAGAGGGATTTCTACTTCGGAAAGCTACGGAACATTGAA
TTGATTTGCCAGGAGAACGAGGGGGAAAACGACCCTGTATTGCAGAGGATTGTAGACATTCTGTATGCCACAGAT
GAAGGCTTTGTGATACCTGATGAAGGGGGCCCACAGGAGGAGCAAGAAGAGTAT (SEQ ID NO: 301)

Protein:
MAVNVYSTSVTSDNLSRHDMLAWINESLQLNLTKIEQLCSGAAYCQFMDMLFPGSIALKKVKFQAKLEHEYIQNF
KILQAGFKRMGVDKIIPVDKLVKGKFQDNFEFVQWFKKFFDANYDGKDYDPVAARQGQETAVAPSLVAPALNKPK
KPLTSSSAAPQRPISTQRTAAAPKAGPGVVRKNPGVGNGDDEAAELMQQVNVLKLTVEDLEKERDFYFGKLRNIE
LICQENEGENDPVLQRIVDILYATDEGFVIPDEGGPQEEQEEY (SEQ ID NO: 302)

CG1: Nucleoporin NUP42 (Homo sapiens, Uniprot: O15504)
DNA:

ATGGCCATTTGTCAATTCTTCCTTCAAGGCCGGTGCCGCTTTGGAGATCGGTGCTGGAACGAACATCCCGGTGCT
AGGGGTGCAGGAGGAGGACGGCAGCAACCGCAGCAGCAGCCTTCAGGTAATAATAGACGTGGATGGAATACAACT
AGCCAGAGATATTCCAATGTCATCCAGCCATCCAGTTTCTCCAAATCCACACCATGGGGGGGCAGCAGAGATCAA
GAAAAGCCATATTTCAGTTCTTTTGATTCTGGAGCTTCAACTAACAGGAAGGAAGGCTTTGGATTGTCTGAGAAC
CCATTTGCTTCACTTAGTCCTGATGAGCAGAAAGATGAAAAGAAACTTCTGGAAGGAATTGTAAAAGATATGGAG
GTTTGGGAATCATCAGGGCAGTGGATGTTTTCTGTTTATTCACCAGTGAAAAAGAAACCTAATATTTCAGGTTTT
ACAGACATTTCACCAGAGGAATTGAGGCTTGAATACCATAACTTCTTAACCAGCAATAACTTACAGAGTTATCTA
AATTCTGTCCAACGTTTAATAAATCAATGGAGGAACAGGGTAAATGAACTGAAAAGTCTAAATATATCAACTAAA
GTAGCTTTGCTCTCTGATGTAAAGGATGGAGTAAATCAAGCAGCACCTGCATTTGGATTTGGCAGCAGTCAAGCA
GCAACATTTATGTCGCCAGGCTTTCCAGTCAATAACAGCAGCAGTGATAATGCTCAGAACTTTAGTTTTAAAACA
AACTCTGGATTTGCTGCTGCCTCTTCTGGAAGCCCTGCTGGTTTTGGGAGTTCCCCAGCATTTGGAGCTGCAGCC
TCTACCAGTTCAGGTATCTCTACTTCTGCTCCAGCTTTTGGATTTGGGAAGCCTGAAGTCACATCGGCTGCATCA
TTTTCATTCAAAAGCCCTGCAGCTTCCAGTTTTGGATCACCTGGATTTTCAGGACTTCCAGCTTCCTTGGCAACA
GGTCCTGTCAGAGCTCCAGTGGCCCCAGCCTTTGGAGGTGGCAGTTCTGTGGCTGGTTTTGGTAGTCCGGGCTCA
CATTCTCACACTGCTTTTTCTAAGCCATCCAGTGACACTTTTGGAAATAGCAGCATATCCACTTCTCTGTCAGCC
TCAAGCAGCATCATTGCAACAGATAATGTGTTATTCACACCCAGAGATAAACTAACAGTAGAAGAACTGGAACAA
TTTCAATCCAAGAAATTTACTCTGGGAAAAATTCCATTAAAGCCTCCACCTCTGGAACTTCTAAATGTT (SEQ
ID NO: 303)

Protein:
MAICQFFLQGRCRFGDRCWNEHPGARGAGGGRQQPQQQPSGNNRRGWNTTSQRYSNVIQPSSFSKSTPWGGSRDQ
EKPYFSSFDSGASTNRKEGFGLSENPFASLSPDEQKDEKKLLEGIVKDMEVWESSGQWMFSVYSPVKKKPNISGF
TDISPEELRLEYHNFLTSNNLQSYLNSVQRLINQWRNRVNELKSLNISTKVALLSDVKDGVNQAAPAFGFGSSQA
ATFMSPGFPVNNSSSDNAQNFSFKTNSGFAAASSGSPAGFGSSPAFGAAASTSSGISTSAPAFGFGKPEVTSAAS
FSFKSPAASSFGSPGFSGLPASLATGPVRAPVAPAFGGGSSVAGFGSPGSHSHTAFSKPSSDTFGNSSISTSLSA
SSSIIATDNVLFTPRDKLTVEELEQFQSKKFTLGKIPLKPPPLELLNV (SEQ ID NO: 304)

PCP: Cytochrome P450 2C1 (Oryctolagus cuniculus, Uniprot: P00180)
DNA:
TCCAAAACCATCGTTCTTTCGGTCGGCGAGGCTACTCGCACTCTGACTGAGATCCAGTCCACCGCAGACCGTCAG
ATCTTCGAAGAGAAGGTCGGGCCTCTGGTGGGTCGGCTGCGCCTCACGGCTTCGCTCCGTCAAAACGGAGCCAAG
ACCGCGTATCGCGTCAACCTAAAACTGGATCAGGCGGACGTCGTTGATTCCGGACTTCCGAAAGTGCGCTACACT
CAGGTATGGTCGCACGACGTGACAATCGTTGCGAATAGCACCGAGGCCTCGCGCAAATCGTTGTACGATTTGACC
AAGTCCCTCGTCGCGACCTCGCAGGTCGAAGATCTTGTCGTCAACCTTGTGCCGCTGGGCCGT (SEQ ID NO:
305)

Protein:
SKTIVLSVGEATRTLTEIQSTADRQIFEEKVGPLVGRLRLTASLRQNGAKTAYRVNLKLDQADVVDSGLPKVRYT
QVWSHDVTIVANSTEASRKSLYDLTKSLVATSQVEDLVVNLVPLGR (SEQ ID NO: 306)

LAF-1: ATP-dependent RNA helicase laf-1 (RGG domain, 1-168)
(Caenorhabditis elegans, Uniprot: D0PV95)
DNA:
ATGGAAAGCAACCAGAGCAACAACGGCGGCTCTGGCAACGCCGCTCTGAACAGAGGCGGCAGATACGTGCCCCCC
CACCTGAGAGGAGGCGACGGCGGCGCCGCCGCCGCTGCATCTGCCGGCGGAGATGACAGAAGAGGCGGAGCCGGA
GGCGGCGGCTATAGACGGGGAGGCGGAAACAGCGGCGGCGGAGGCGGAGGCGGCTACGACAGAGGCTACAACGAC
AACCGGGACGACCGGGACAACAGAGGCGGCAGCGGCGGATACGGCAGAGATCGAAACTACGAGGACAGAGGCTAC
AATGGCGGAGGCGGAGGCGGCGGCAACCGGGGCTACAACAACAACAGAGGAGGCGGCGGCGGCGGCTACAACCGC
CAGGACAGAGGCGATGGCGGATCTAGCAATTTCAGCAGAGGCGGCTACAACAACCGGGACGAGGGCAGCGACAAC
AGAGGCAGCGGAAGAAGCTACAACAATGACCGGAGAGATAATGGCGGAGATGGC (SEQ ID NO: 307)

Protein:
MESNQSNNGGSGNAALNRGGRYVPPHLRGGDGGAAAAASAGGDDRRGGAGGGGYRRGGGNSGGGGGGGYDRGYND
NRDDRDNRGGSGGYGRDRNYEDRGYNGGGGGGGNRGYNNNRGGGGGGYNRQDRGDGGSSNFSRGGYNNRDEGSDN
RGSGRSYNNDRRDNGGDG (SEQ ID NO:308)

SLP3: Stomatin-like protein 3, aa 1-59 (Homo sapiens, Uniprot: Q8TAV4)
DNA:
ATGGATTCTAGGGTGTCTTCACCTGAGAAGCAAGATAAAGAGAATTTCGTGGGTGTCAACAATAAACGGCTTGGT
GTATGTGGCTGGATCCTGTTTTCCCTCTCTTTCCTGTTGGTGATCATTACCTTCCCCATCTCCATATGGATGTGC
TTGAAGATCATTAAGGAGTATGAACGT (SEQ ID NO: 309)

Protein:
MDSRVSSPEKQDKENFVGVNNKRLGVCGWILFSLSFLLVIITFPISIWMCLKIIKEYER (SEQ ID NO:
310)

SYNZIP1: Synthetic coiled-coil peptide 1 (Synthetic)
DNA:
AATCTGGTGGCCCAGCTGGAAAACGAGGTGGCCAGCCTGGAAAACGAGAACGAAACCCTGAAGAAAAAGAACCTG
CACAAGAAGGACCTGATCGCCTACCTGGAAAAGGAAATCGCCAACCTGAGAAAGAAGATCGAGGAA (SEQ ID
NO: 311)

Protein:
NLVAQLENEVASLENENETLKKKNLHKKDLIAYLEKEIANLRKKIEE (SEQ ID NO: 312)

SYNZIP2: Synthetic coiled-coil peptide 1 (Synthetic)
DNA:
GCTAGAAACGCCTACCTGAGAAAGAAAATCGCCAGACTGAAGAAGGACAACCTGCAGCTGGAAAGAGACGAGCAG
AACCTGGAAAAGATCATCGCCAACCTCAGAGATGAGATCGCCAGACTGGAAAACGAGGTGGCCAGCCACGAGCAG
(SEQ ID NO: 313)

Protein:
ARNAYLRKKIARLKKDNLQLERDEQNLEKIIANLRDEIARLENEVASHEQ (SEQ ID NO: 314)

SYNZIP3: Synthetic coiled-coil peptide 1 (Synthetic)
DNA:
AATGAGGTGACCACCCTGGAAAACGACGCCGCCTTCATCGAGAACGAGAACGCCTACCTGGAAAAAGAGATCGCC
AGACTGAGAAAGGAAAAGGCCGCTCTGCGGAACAGACTGGCCCACAAGAAG (SEQ ID NO: 315)

Protein:
NEVTTLENDAAFIENENAYLEKEIARLRKEKAALRNRLAHKK (SEQ ID NO: 316)

SYNZIP4: Synthetic coiled-coil peptide 1 (Synthetic)
DNA:
CAAAAGGTGGCTGAACTGAAAAATAGAGTGGCCGTGAAGCTGAACCGGAACGAGCAGCTGAAGAACAAGGTGGAA
GAGCTGAAGAACAGAAACGCCTACCTGAAGAATGAGCTGGCCACCCTGGAAAACGAGGTGGCCAGACTGGAAAAC
GACGTGGCCGAG (SEQ ID NO: 317)

Protein:
QKVAELKNRVAVKLNRNEQLKNKVEELKNRNAYLKNELATLENEVARLENDVAE (SEQ ID NO: 318)

2. Further Fusion proteis:

EBAG91-29::FUS::PylRS(AF)
DNA:
ATGGCCATCACCCAGTTTCGGTTATTTAAATTTTGTACCTGCCTAGCAACAGTATTCTCATTCCTAAAGAGATTA
ATATGCAGATCTGGAGCACCCGGCTCCGCCGGCTCCGCCGCCGGCTCCGGCATGGCCTCAAACGATTATACCCAA
CAAGCAACCCAAAGCTATGGGGCCTACCCCACCCAGCCCGGGCAGGGCTATTCCCAGCAGAGCAGTCAGCCCTAC

GGACAGCAGAGTTACAGTGGTTATAGCCAGTCCACGGACACTTCAGGATATGGCCAGAGCAGCTATTCTTCTTAT
GGCCAGAGCCAGAACACAGGCTATGGAACTCAGTCAACTCCCCAGGGATATGGCTCGACTGGCGGCTATGGCAGT
AGCCAGAGCTCCCAATCGTCTTACGGGCAGCAGTCCTCCTACCCTGGCTATGGCCAGCAGCCAGCTCCCAGCAGC
ACCTCGGGAAGTTACGGTAGCAGTTCTCAGAGCAGCAGCTATGGGCAGCCCCAGAGTGGGAGCTACAGCCAGCAG
CCTAGCTATGGTGGACAGCAGCAAAGCTATGGACAGCAGCAAAGCTATAATCCCCCTCAGGGCTATGGACAGCAG
AACCAGTACAACAGCAGCAGTGGTGGTGGAGGTGGAGGTGGAGGTGGAGGTAACTATGGCCAAGATCAATCCTCC
ATGAGTAGTGGTGGTGGCAGTGGTGGCGGTTATGGCAATCAAGACCAGAGTGGTGGAGGTGGCAGCGGTGGCTAT
GGACAGCAGGACCGTGGAGGCCGCGGCAGGGGTGGCAGTGGTGGCGGCGGCGGCGGTGGTGGTTACAAC
CGCAGCAGTGGTGGCTATGAACCCAGAGGTCGTGGAGGTGGCCGTGGAGGCAGAGGTGGCATGGGCGGAAGTGAC
CGTGGTGGCTTCAATAAATTTGGTGGCCCTCGGGACCAAGGATCACGTCATGACTCCGAACAGGATAATTCAGAC
AACAACACCATCTTTGTGCAAGGCCTGGGTGAGAATGTTACAATTGAGTCTGTGGCTGATTACTTCAAGCAGATT
GGTATTATTAAGACAAACAAGAAAACGGGACAGCCCATGATTAATTTGTACACAGACAGGGAAACTGGCAAGCTG
AAGGGAGAGGCAACGGTCTCTTTTGATGACCCACCTTCAGCTAAAGCAGCTATTGACTGGTTTGATGGTAAAGAA
TTCTCCGGAAATCCTATCAAGGTCTCATTTGCTACTCGCCGGGCAGACTTTAATCGGGGTGGTGGCAATGGTCGT
GGAGGCCGAGGGCGAGGAGGACCCATGGGCCGTGGAGGCTATGGAGGTGGTGGCAGTGGTGGTGGCCGAGGA
GGATTTCCCAGTGGAGGTGGTGGCGGTGGAGGACAGCAGCGAGCTGGTGACTGGAAGTGTCCTAATCCCACCTGT
GAGAATATGAACTTCTCTTGGAGGAATGAATGCAACCAGTGTAAGGCCCCTAAACCAGATGGCCCAGGAGGGGGA
CCAGGTGGCTCTCACATGGGGGGTAACTACGGGGATGATCGTCGTGGTGGCAGAGGTGGTGCGATCGCAGGCGCC
GATTACAAGGACGATGATGACAAGGGAGCACCAGGAAGTGCTGGTTCTGCTGCTGGTAGTGGAGCGTGCCCGGTG
CCGCTGCAGCTGCCGCCGCTGGAACGCCTGACCCTGGATGATAAAAAACCGCTGAATACCCTGATCTCTGCTACT
GGTCTGTGGATGAGTCGTACCGGAACCATTCATAAAATCAAACACCACGAGGTTAGCCGTTCGAAAATCTATATT
GAGATGGCGTGTGGCGATCATCTGGTTGTGAACAATAGCCGCTCTTCTCGTACAGCACGTGCACTGCGTCACCAC
AAATATCGTAAAACCTGTAAACGTTGCCGTGTGTCCGATGAGGATCTGAACAAATTCCTGACAAAAGCCAATGAG
GACCAAACAAGCGTGAAAGTGAAAGTCGTTAGCGCTCCTACCCGTACTAAAAAAGCAATGCCGAAATCCGTTGCT
CGTGCCCCTAAACCACTGGAAAACACTGAAGCAGCACAGGCACAGCCGTCTGGAAGCAAATTCTCTCCGGCCATT
CCTGTTTCTACCCAGGAGTCCGTTTCTGTTCCAGCAAGTGTGAGCACCAGCATTAGCAGTATTAGCACCGGTGCC
ACCGCTAGCGCCCTGGTTAAAGGCAATACCAATCCGATTACAAGCATGTCTGCCCCGGTTCAAGCATCAGCTCCA
GCACTGACAAAATCCCAAACCGATCGTCTGGAGGTTCTGCTGAATCCGAAAGACGAAATCAGCCTGAATTCCGGC
AAACCGTTTCGTGAACTGGAGAGCGAACTGCTGTCACGTCGTAAAAAAGACCTGCAACAAATCTATGCCGAAGAA
CGTGAGAACTATCTGGGGAAACTGGAACGTGAAATCACCCGCTTTTTCGTGGATCGTGGCTTTCTGGAGATCAAA
TCCCCGATTCTGATTCCTCTGGAGTATATCGAGCGTATGGGCATCGACAATGATACCGAACTGAGCAAACAAATT
TTCCGTGTGGATAAAAACTTCTGTCTGCGCCCTATGCTAGCACCAAATCTGGCTAACTATCTGCGCAAACTGGAC
CGTGCCCTGCCTGATCCTATCAAAATCTTCGAGATCGGCCCGTGTTATCGTAAAGAGTCCGACGGTAAAGAACAT
CTGGAGGAGTTTACCATGCTGAACTTTTGCCAAATGGGTTCAGGTTGTACTCGTGAGAACCTGGAAAGCATCATC
ACCGATTTTCTGAACCACCTGGGCATTGACTTCAAAATTGTGGGCGACAGCTGTATGGTGTTTGGCGACACCCTG
GATGTCATGCACGGCGACCTGGAACTGTCTAGTGCCGTTGTGGGCCCAATCCCGCTGGATCGTGAGTGGGGTATC
GACAAACCTTGGATCGGTGCGGGTTTTGGTCTGGAGCGTCTGCTGAAAGTAAAACACGACTTCAAGAACATCAAA
CGTGCTGCACGTTCCGAGTCCTATTACAATGGTATTTCTACTAACCTGTAA (SEQ ID NO: 319)

Protein:
MAITQFRLFKFCTCLATVFSFLKRLICRSGAPGSAGSAAGSGMASNDYTQQATQSYGAYPTQPGQGYSQQSSQPY
GQQSYSGYSQSTDTSGYGQSSYSSYGQSQNTGYGTQSTPQGYGSTGGYGSSQSSQSSYGQQSSYPGYGQQPAPSS
TSGSYGSSSQSSSYGQPQSGSYSQQPSYGGQQQSYGQQQSYNPPQGYGQQNQYNSSSGGGGGGGGGNYGQDQSS
MSSGGGSGGGYGNQDQSGGGGSGGYGQQDRGGRGRGGSGGGGGGGGGYNRSSGGYEPRGRGGGRGGRGGMGGSD
RGGFNKFGGPRDQGSRHDSEQDNSDNNTIFVQGLGENVTIESVADYFKQIGIIKTNKKTGQPMINLYTDRETGKL
KGEATVSFDDPPSAKAAIDWFDGKEFSGNPIKVSFATRRADFNRGGGNGRGGRGRGGPMGRGGYGGGGSGGGGRG
GFPSGGGGGGGQQRAGDWKCPNPTCENMNFSWRNECNQCKAPKPDGPGGGPGGSHMGGNYGDDRRGGRGGAIAGA
DYKDDDDKGAPGSAGSAAGSGACPVPLQLPPLERLTLDDKKPLNTLISATGLWMSRTGTIHKIKHHEVSRSKIYI
EMACGDHLVVNNSRSSRTARALRHHKYRKTCKRCRVSDEDLNKFLTKANEDQTSVKVKVVSAPTRTKKAMPKSVA
RAPKPLENTEAAQAQPSGSKFSPAIPVSTQESVSVPASVSTSISSISTGATASALVKGNTNPITSMSAPVQASAP
ALTKSQTDRLEVLLNPKDEISLNSGKPFRELESELLSRRKKDLQQIYAEERENYLGKLEREITRFFVDRGFLEIK
SPILIPLEYIERMGIDNDTELSKQIFRVDKNFCLRPMLAPNLANYLRKLDRALPDPIKIFEIGPCYRKESDGKEH
LEEFTMLNFCQMGSGCTRENLESIITDFLNHLGIDFKIVGDSCMVFGDTLDVMHGDLELSSAVVGPIPLDREWGI
DKPWIGAGFGLERLLKVKHDFKNIKRAARSESYYNGISTNL* (SEQ ID NO: 320)

EBAG9::PylRS(AF)
DNA:

ATGGCCATCACCCAGTTTCGGTTATTTAAATTTTGTACCTGCCTAGCAACAGTATTCTCATTCCTAAAGAGATTA
ATATGCAGATCTGGCAGAGGACGGAAATTAAGTGGAGACCAAATAACTTTGCCAACTACAGTTGATTATTCATCA
GTTCCTAAGCAGACAGATGTTGAAGAGTGGACTTCCTGGGATGAAGATGCACCCACCAGTGTAAAGATCGAAGGA
GGGAATGGGAATGTGGCAACACAACAAAATTCTTTGGAACAACTGGAACCTGACTATTTTAAGGACATGACACCA
ACTATTAGGAAAACTCAGAAAATTGTTATTAAGAAGAGAGAACCATTGAATTTTGGCATCCCAGATGGGAGCACA
GGTTTCTCTAGTAGATTAGCAGCTACACAAGATCTGCCTTTTATTCATCAGTCTTCTGAATTAGGTGACTTAGAT
ACCTGGCAGGAAAATACCAATGCATGGGAAGAAGAAGAAGATGCAGCCTGGCAAGCAGAAGAAGTTCTGAGACAG
CAGAAACTAGCAGACAGAGAAAAGAGAGCAGCCGAACAACAAAGGAAGAAAATGGAAAAGGAAGCACAACGGCTA
ATGAAGAAGGAACAAAACAAAATTGGTGTGAAACTTTCAGGCGCCGATTACAAGGACGATGATGACAAGGGAGCA
CCAGGAAGTGCTGGTTCTGCTGCTGGTAGTGGAGCGTGCCCGGTGCCGCTGCAGCTGCCGCCGCTGGAACGCCTG
ACCCTGGATGATAAAAAACCGCTGAATACCCTGATCTCTGCTACTGGTCTGTGGATGAGTCGTACCGGAACCATT
CATAAAATCAAACACCACGAGGTTAGCCGTTCGAAAATCTATATTGAGATGGCGTGTGGCGATCATCTGGTTGTG
AACAATAGCCGCTCTTCTCGTACAGCACGTGCACTGCGTCACCACAAATATCGTAAAACCTGTAAACGTTGCCGT
GTGTCCGATGAGGATCTGAACAAATTCCTGACAAAAGCCAATGAGGACCAAACAAGCGTGAAAGTGAAAGTCGTT
AGCGCTCCTACCCGTACTAAAAAAGCAATGCCGAAATCCGTTGCTCGTGCCCCTAAACCACTGGAAAACACTGAA
GCAGCACAGGCACAGCCGTCTGGAAGCAAATTCTCTCCGGCCATTCCTGTTTCTACCCAGGAGTCCGTTTCTGTT
CCAGCAAGTGTGAGCACCAGCATTAGCAGTATTAGCACCGGTGCCACCGCTAGCGCCCTGGTTAAAGGCAATACC
AATCCGATTACAAGCATGTCTGCCCCGGTTCAAGCATCAGCTCCAGCACTGACAAAATCCCAAACCGATCGTCTG
GAGGTTCTGCTGAATCCGAAAGACGAAATCAGCCTGAATTCCGGCAAACCGTTTCGTGAACTGGAGAGCGAACTG
CTGTCACGTCGTAAAAAAGACCTGCAACAAATCTATGCCGAAGAACGTGAGAACTATCTGGGGAAACTGGAACGT
GAAATCACCCGCTTTTTCGTGGATCGTGGCTTTCTGGAGATCAAATCCCCGATTCTGATTCCTCTGGAGTATATC
GAGCGTATGGGCATCGACAATGATACCGAACTGAGCAAACAAATTTTCCGTGTGGATAAAAACTTCTGTCTGCGC
CCTATGCTAGCACCAAATCTGGCTAACTATCTGCGCAAACTGGACCGTGCCCTGCCTGATCCTATCAAAATCTTC
GAGATCGGCCCGTGTTATCGTAAAGAGTCCGACGGTAAAGAACATCTGGAGGAGTTTACCATGCTGAACTTTTGC
CAAATGGGTTCAGGTTGTACTCGTGAGAACCTGGAAAGCATCATCACCGATTTTCTGAACCACCTGGGCATTGAC
TTCAAAATTGTGGGCGACAGCTGTATGGTGTTTGGCGACACCCTGGATGTCATGCACGGCGACCTGGAACTGTCT
AGTGCCGTTGTGGGCCCAATCCCGCTGGATCGTGAGTGGGGTATCGACAAACCTTGGATCGGTGCGGGTTTTGGT
CTGGAGCGTCTGCTGAAAGTAAAACACGACTTCAAGAACATCAAACGTGCTGCACGTTCCGAGTCCTATTACAAT
GGTATTTCTACTAACCTGTAA (SEQ ID NO: 321)

Protein:
MAITQFRLFKFCTCLATVFSFLKRLICRSGRGRKLSGDQITLPTTVDYSSVPKQTDVEEWTSWDEDAPTSVKIEG
GNGNVATQQNSLEQLEPDYFKDMTPTIRKTQKIVIKKREPLNFGIPDGSTGFSSRLAATQDLPFIHQSSELGDLD
TWQENTNAWEEEEDAAWQAEEVLRQQKLADREKRAAEQQRKKMEKEAQRLMKKEQNKIGVKLSGADYKDDDDKGA
PGSAGSAAGSGACPVPLQLPPLERLTLDDKKPLNTLISATGLWMSRTGTIHKIKHHEVSRSKIYIEMACGDHLVV
NNSRSSRTARALRHHKYRKTCKRCRVSDEDLNKFLTKANEDQTSVKVKVVSAPTRTKKAMPKSVARAPKPLENTE
AAQAQPSGSKFSPAIPVSTQESVSVPASVSTSISSISTGATASALVKGNTNPITSMSAPVQASAPALTKSQTDRL
EVLLNPKDEISLNSGKPFRELESELLSRRKKDLQQIYAEERENYLGKLEREITRFFVDRGFLEIKSPILIPLEYI
ERMGIDNDTELSKQIFRVDKNFCLRPMLAPNLANYLRKLDRALPDPIKIFEIGPCYRKESDGKEHLEEFTMLNFC
QMGSGCTRENLESIITDFLNHLGIDFKIVGDSCMVFGDTLDVMHGDLELSSAVVGPIPLDREWGIDKPWIGAGFG
LERLLKVKHDFKNIKRAARSESYYNGISTNL* (SEQ ID NO: 322)

EBAG9::FUS::PylRS(AF)
DNA:
ATGGCCATCACCCAGTTTCGGTTATTTAAATTTTGTACCTGCCTAGCAACAGTATTCTCATTCCTAAAGAGATTA
ATATGCAGATCTGGCAGAGGACGGAAATTAAGTGGAGACCAAATAACTTTGCCAACTACAGTTGATTATTCATCA
GTTCCTAAGCAGACAGATGTTGAAGAGTGGACTTCCTGGGATGAAGATGCACCCACCAGTGTAAAGATCGAAGGA
GGGAATGGGAATGTGGCAACACAACAAAATTCTTTGGAACAACTGGAACCTGACTATTTTAAGGACATGACACCA
ACTATTAGGAAAACTCAGAAAATTGTTATTAAGAAGAGAGAACCATTGAATTTTGGCATCCCAGATGGGAGCACA
GGTTTCTCTAGTAGATTAGCAGCTACACAAGATCTGCCTTTTATTCATCAGTCTTCTGAATTAGGTGACTTAGAT
ACCTGGCAGGAAAATACCAATGCATGGGAAGAAGAAGAAGATGCAGCCTGGCAAGCAGAAGAAGTTCTGAGACAG
CAGAAACTAGCAGACAGAGAAAAGAGAGCAGCCGAACAACAAAGGAAGAAAATGGAAAAGGAAGCACAACGGCTA
ATGAAGAAGGAACAAAACAAAATTGGTGTGAAACTTTCAGGAGCACCCGGCTCCGCCGGCTCCGCCGCCGGCTCC
GGCATGGCCTCAAACGATTATACCCAACAAGCAACCCAAAGCTATGGGGCCTACCCCACCCAGCCCGGGCAGGGC
TATTCCCAGCAGAGCAGTCAGCCCTACGGACAGCAGAGTTACAGTGGTTATAGCCAGTCCACGGACACTTCAGGA
TATGGCCAGAGCAGCTATTCTTCTTATGGCCAGAGCCAGAACACAGGCTATGGAACTCAGTCAACTCCCCAGGGA
TATGGCTCGACTGGCGGCTATGGCAGTAGCCAGAGCTCCCAATCGTCTTACGGGCAGCAGTCCTCCTACCCTGGC
TATGGCCAGCAGCCAGCTCCCAGCAGCACCTCGGGAAGTTACGGTAGCAGTTCTCAGAGCAGCAGCTATGGGCAG

CCCCAGAGTGGGAGCTACAGCCAGCAGCCTAGCTATGGTGGACAGCAGCAAAGCTATGGACAGCAGCAAAGCTAT
AATCCCCCTCAGGGCTATGGACAGCAGAACCAGTACAACAGCAGCAGTGGTGGTGGAGGTGGAGGTGGAGGTGGA
GGTAACTATGGCCAAGATCAATCCTCCATGAGTAGTGGTGGTGGCAGTGGTGGCGGTTATGGCAATCAAGACCAG
AGTGGTGGAGGTGGCAGCGGTGGCTATGGACAGCAGGACCGTGGAGGCCGCGGCAGGGGTGGCAGTGGTGGCGGC
GGCGGCGGCGGCGGTGGTGGTTACAACCGCAGCAGTGGTGGCTATGAACCCAGAGGTCGTGGAGGTGGCCGTGGA
GGCAGAGGTGGCATGGGCGGAAGTGACCGTGGTGGCTTCAATAAATTTGGTGGCCCTCGGGACCAAGGATCACGT
CATGACTCCGAACAGGATAATTCAGACAACAACACCATCTTTGTGCAAGGCCTGGGTGAGAATGTTACAATTGAG
TCTGTGGCTGATTACTTCAAGCAGATTGGTATTATTAAGACAAACAAGAAAACGGGACAGCCCATGATTAATTTG
TACACAGACAGGGAAACTGGCAAGCTGAAGGGAGAGGCAACGGTCTCTTTTGATGACCCACCTTCAGCTAAAGCA
GCTATTGACTGGTTTGATGGTAAAGAATTCTCCGGAAATCCTATCAAGGTCTCATTTGCTACTCGCCGGGCAGAC
TTTAATCGGGGTGGTGGCAATGGTCGTGGAGGCCGAGGGCGAGGAGGACCCATGGGCCGTGGAGGCTATGGAGGT
GGTGGCAGTGGTGGTGGTGGCCGAGGAGGATTTCCCAGTGGAGGTGGTGGCGGTGGAGGACAGCAGCGAGCTGGT
GACTGGAAGTGTCCTAATCCCACCTGTGAGAATATGAACTTCTCTTGGAGGAATGAATGCAACCAGTGTAAGGCC
CCTAAACCAGATGGCCCAGGAGGGGGACCAGGTGGCTCTCACATGGGGGGTAACTACGGGGATGATCGTCGTGGT
GGCAGAGGTGGTGCGATCGCAGGCGCCGATTACAAGGACGATGATGACAAGGGAGCACCAGGAAGTGCTGGTTCT
GCTGCTGGTAGTGGAGCGTGCCCGGTGCCGCTGCAGCTGCCGCCGCTGGAACGCCTGACCCTGGATGATAAAAAA
CCGCTGAATACCCTGATCTCTGCTACTGGTCTGTGGATGAGTCGTACCGGAACCATTCATAAAATCAAACACCAC
GAGGTTAGCCGTTCGAAAATCTATATTGAGATGGCGTGTGGCGATCATCTGGTTGTGAACAATAGCCGCTCTTCT
CGTACAGCACGTGCACTGCGTCACCACAAATATCGTAAAACCTGTAAACGTTGCCGTGTGTCCGATGAGGATCTG
AACAAATTCCTGACAAAAGCCAATGAGGACCAAACAAGCGTGAAAGTGAAAGTCGTTAGCGCTCCTACCCGTACT
AAAAAAGCAATGCCGAAATCCGTTGCTCGTGCCCCTAAACCACTGGAAAACACTGAAGCAGCACAGGCACAGCCG
TCTGGAAGCAAATTCTCTCCGGCCATTCCTGTTTCTACCCAGGAGTCCGTTTCTGTTCCAGCAAGTGTGAGCACC
AGCATTAGCAGTATTAGCACCGGTGCCACCGCTAGCGCCCTGGTTAAAGGCAATACCAATCCGATTACAAGCATG
TCTGCCCCGGTTCAAGCATCAGCTCCAGCACTGACAAAATCCCAAACCGATCGTCTGGAGGTTCTGCTGAATCCG
AAAGACGAAATCAGCCTGAATTCCGGCAAACCGTTTCGTGAACTGGAGAGCGAACTGCTGTCACGTCGTAAAAAA
GACCTGCAACAAATCTATGCCGAAGAACGTGAGAACTATCTGGGGAAACTGGAACGTGAAATCACCCGCTTTTTC
GTGGATCGTGGCTTTCTGGAGATCAAATCCCCGATTCTGATTCCTCTGGAGTATATCGAGCGTATGGGCATCGAC
AATGATACCGAACTGAGCAAACAAATTTTCCGTGTGGATAAAAACTTCTGTCTGCGCCCTATGCTAGCACCAAAT
CTGGCTAACTATCTGCGCAAACTGGACCGTGCCCTGCCTGATCCTATCAAAATCTTCGAGATCGGCCCGTGTTAT
CGTAAAGAGTCCGACGGTAAAGAACATCTGGAGGAGTTTACCATGCTGAACTTTTGCCAAATGGGTTCAGGTTGT
ACTCGTGAGAACCTGGAAAGCATCATCACCGATTTTCTGAACCACCTGGGCATTGACTTCAAAATTGTGGGCGAC
AGCTGTATGGTGTTTGGCGACACCCTGGATGTCATGCACGGCGACCTGGAACTGTCTAGTGCCGTTGTGGGCCCA
ATCCCGCTGGATCGTGAGTGGGGTATCGACAAACCTTGGATCGGTGCGGGTTTTGGTCTGGAGCGTCTGCTGAAA
GTAAAACACGACTTCAAGAACATCAAACGTGCTGCACGTTCCGAGTCCTATTACAATGGTATTTCTACTAACCTG
TAA (SEQ ID NO: 323)

Protein:
MAITQFRLFKFCTCLATVFSFLKRLICRSGRGRKLSGDQITLPTTVDYSSVPKQTDVEEWTSWDEDAPTSVKIEG
GNGNVATQQNSLEQLEPDYFKDMTPTIRKTQKIVIKKREPLNFGIPDGSTGFSSRLAATQDLPFIHQSSELGDLD
TWQENTNAWEEEEDAAWQAEEVLRQQKLADREKRAAEQQRKKMEKEAQRLMKKEQNKIGVKLSGAPGSAGSAAGS
GMASNDYTQQATQSYGAYPTQPGQGYSQQSSQPYGQQSYSGYSQSTDTSGYGQSSYSSYGQSQNTGYGTQSTPQG
YGSTGGYGSSQSSQSSYGQQSSYPGYGQQPAPSSTSGSYGSSSQSSSYGQPQSGSYSQQPSYGGQQQSYGQQQSY
NPPQGYGQQNQYNSSSGGGGGGGGGGNYGQDQSSMSSGGGSGGGYGNQDQSGGGGSGGYGQQDRGGRGRGGSGGG
GGGGGGGYNRSSGGYEPRGRGGGRGGRGGMGGSDRGGFNKFGGPRDQGSRHDSEQDNSDNNTIFVQGLGENVTIE
SVADYFKQIGIIKTNKKTGQPMINLYTDRETGKLKGEATVSFDDPPSAKAAIDWFDGKEFSGNPIKVSFATRRAD
FNRGGGNGRGGRGRGGPMGRGGYGGGGSGGGGRGGFPSGGGGGGGQQRAGDWKCPNPTCENMNFSWRNECNQCKA
PKPDGPGGGPGGSHMGGNYGDDRRGGRGGAIAGADYKDDDDKGAPGSAGSAAGSGACPVPLQLPPLERLTLDDKK
PLNTLISATGLWMSRTGTIHKIKHHEVSRSKIYIEMACGDHLVVNNSRSSRTARALRHHKYRKTCKRCRVSDEDL
NKFLTKANEDQTSVKVKVVSAPTRTKKAMPKSVARAPKPLENTEAAQAQPSGSKFSPAIPVSTQESVSVPASVST
SISSISTGATASALVKGNTNPITSMSAPVQASAPALTKSQTDRLEVLLNPKDEISLNSGKPFRELESELLSRRKK
DLQQIYAEERENYLGKLEREITRFFVDRGFLEIKSPILIPLEYIERMGIDNDTELSKQIFRVDKNFCLRPMLAPN
LANYLRKLDRALPDPIKIFEIGPCYRKESDGKEHLEEFTMLNFCQMGSGCTRENLESIITDFLNHLGIDFKIVGD
SCMVFGDTLDVMHGDLELSSAVVGPIPLDREWGIDKPWIGAGFGLERLLKVKHDFKNIKRAARSESYYNGISTNL
* (SEQ ID NO: 324)

EBAG9::MCP
DNA:

ATGGCCATCACCCAGTTTCGGTTATTTAAATTTTGTACCTGCCTAGCAACAGTATTCTCATTCCTAAAGAGATTA
ATATGCAGATCTGGCAGAGGACGGAAATTAAGTGGAGACCAAATAACTTTGCCAACTACAGTTGATTATTCATCA
GTTCCTAAGCAGACAGATGTTGAAGAGTGGACTTCCTGGGATGAAGATGCACCCACCAGTGTAAAGATCGAAGGA
GGGAATGGGAATGTGGCAACACAACAAAATTCTTTGGAACAACTGGAACCTGACTATTTTAAGGACATGACACCA
ACTATTAGGAAAACTCAGAAAATTGTTATTAAGAAGAGAGAACCATTGAATTTTGGCATCCCAGATGGGAGCACA
GGTTTCTCTAGTAGATTAGCAGCTACACAAGATCTGCCTTTTATTCATCAGTCTTCTGAATTAGGTGACTTAGAT
ACCTGGCAGGAAAATACCAATGCATGGGAAGAAGAAGAAGATGCAGCCTGGCAAGCAGAAGAAGTTCTGAGACAG
CAGAAACTAGCAGACAGAGAAAAGAGAGCAGCCGAACAACAAAGGAAGAAAATGGAAAAGGAAGCACAACGGCTA
ATGAAGAAGGAACAAAACAAAATTGGTGTGAAACTTTCAGCGATCGCATATCCCTATGATGTGCCGGATTATGCT
GGAGCACCAGGAAGTGCTGGTTCTGCTGCTGGTAGTGGAGCTTCTAACTTTACTCAGTTCGTTCTCGTCGACAAT
GGCGGAACTGGCGACGTGACTGTCGCCCCAAGCAACTTCGCTAACGGGATCGCTGAATGGATCAGCTCTAACTCG
CGTTCACAGGCTTACAAAGTAACCTGTAGCGTTCGTCAGAGCTCTGCGCAGAATCGCAAATACACCATCAAAGTC
GAGGTGCCTAAAGGCGCCTGGCGTTCGTACTTAAATATGGAACTAACCATTCCAATTTTCGCCACGAATTCCGAC
TGCGAGCTTATTGTTAAGGCAATGCAAGGTCTCCTAAAAGATGGAAACCCGATTCCCTCAGCAATCGCAGCAAAC
TCCGGCATCTACTAA (SEQ ID NO: 325)

Protein:
MAITQFRLFKFCTCLATVFSFLKRLICRSGRGRKLSGDQITLPTTVDYSSVPKQTDVEEWTSWDEDAPTSVKIEG
GNGNVATQQNSLEQLEPDYFKDMTPTIRKTQKIVIKKREPLNFGIPDGSTGFSSRLAATQDLPFIHQSSELGDLD
TWQENTNAWEEEEDAAWQAEEVLRQQKLADREKRAAEQQRKKMEKEAQRLMKKEQNKIGVKLSAIAYPYDVPDYA
GAPGSAGSAAGSGASNFTQFVLVDNGGTGDVTVAPSNFANGIAEWISSNSRSQAYKVTCSVRQSSAQNRKYTIKV
EVPKGAWRSYLNMELTIPIFATNSDCELIVKAMQGLLKDGNPIPSAIAANSGIY* (SEQ ID NO: 326)

EBAG9::EWSR1::MCP
DNA:
ATGGCCATCACCCAGTTTCGGTTATTTAAATTTTGTACCTGCCTAGCAACAGTATTCTCATTCCTAAAGAGATTA
ATATGCAGATCTGGCAGAGGACGGAAATTAAGTGGAGACCAAATAACTTTGCCAACTACAGTTGATTATTCATCA
GTTCCTAAGCAGACAGATGTTGAAGAGTGGACTTCCTGGGATGAAGATGCACCCACCAGTGTAAAGATCGAAGGA
GGGAATGGGAATGTGGCAACACAACAAAATTCTTTGGAACAACTGGAACCTGACTATTTTAAGGACATGACACCA
ACTATTAGGAAAACTCAGAAAATTGTTATTAAGAAGAGAGAACCATTGAATTTTGGCATCCCAGATGGGAGCACA
GGTTTCTCTAGTAGATTAGCAGCTACACAAGATCTGCCTTTTATTCATCAGTCTTCTGAATTAGGTGACTTAGAT
ACCTGGCAGGAAAATACCAATGCATGGGAAGAAGAAGAAGATGCAGCCTGGCAAGCAGAAGAAGTTCTGAGACAG
CAGAAACTAGCAGACAGAGAAAAGAGAGCAGCCGAACAACAAAGGAAGAAAATGGAAAAGGAAGCACAACGGCTA
ATGAAGAAGGAACAAAACAAAATTGGTGTGAAACTTTCAATGGCGTCCACGGATTACAGTACCTATAGCCAAGCT
GCAGCGCAGCAGGGCTACAGTGCTTACACCGCCCAGCCCACTCAAGGATATGCACAGACCACCCAGGCATATGGG
CAACAAAGCTATGGAACCTATGGACAGCCCACTGATGTCAGCTATACCCAGGCTCAGACCACTGCAACCTATGGG
CAGACCGCCTATGCAACTTCTTATGGACAGCCTCCCACTGGTTATACTACTCCAACTGCCCCCCAGGCATACAGC
CAGCCTGTCCAGGGGTATGGCACTGGTGCTTATGATACCACCACTGCTACAGTCACCACCACCCAGGCCTCCTAT
GCAGCTCAGTCTGCATATGGCACTCAGCCTGCTTATCCAGCCTATGGGCAGCAGCCAGCAGCCACTGCACCTACA
AGACCGCAGGATGGAAACAAGCCCACTGAGACTAGTCAACCTCAATCTAGCACAGGGGGTTACAACCAGCCCAGC
CTAGGATATGGACAGAGTAACTACAGTTATCCCCAGGTACCTGGGAGCTACCCCATGCAGCCAGTCACTGCACCT
CCATCCTACCCTCCTACCAGCTATTCCTCTACACAGCCGACTAGTTATGATCAGAGCAGTTACTCTCAGCAGAAC
ACCTATGGGCAACCGAGCAGCTATGGACAGCAGAGTAGCTATGGTCAACAAAGCAGCTATGGGCAGCAGCCTCCC
ACTAGTTACCCACCCCAAACTGGATCCTACAGCCAAGCTCCAAGTCAATATAGCCAACAGAGCAGCAGCTACGGG
CAGCAGAGTTCATTCCGACAGGACCACCCCAGTAGCATGGGTGTTTATGGGCAGGAGTCTGGAGGATTTTCCGGA
CCAGGAGAGAACCGGAGCATGAGTGGCCCTGATAACCGGGGCAGGGGAAGAGGGGGATTTGATCGTGGAGGCATG
AGCAGAGGTGGGCGGGGAGGAGGACGCGGTGGAATGGGCAGCGCTGGAGAGCGAGGTGGCTTCAATAAGCCTGGT
GGACCCATGGATGAAGGACCAGATCTTGATCTAGGCCCACCTGTAGATCCAGATGAAGACTCTGACAACAGTGCA
ATTTATGTACAAGGATTAAATGACAGTGTGACTCTAGATGATCTGGCAGACTTCTTTAAGCAGTGTGGGGTTGTT
AAGATGAACAAGAGAACTGGGCAACCCATGATCCACATCTACCTGGACAAGGAAACAGGAAAGCCCAAAGGCGAT
GCCACAGTGTCCTATGAAGACCCACCTACTGCCAAGGCTGCCGTGGAATGGTTTGATGGGAAAGATTTTCAAGGG
AGCAAACTTAAAGTCTCCCTTGCTCGGAAGAAGCCTCCAATGAACAGTATGCGGGGTGGTCTGCCACCCCGTGAG
GGCAGAGGCATGCCACCACCACTCCGTGGAGGTCCAGGAGGCCCAGGAGGTCCTGGGGGACCCATGGGTCGCATG
GGAGGCCGTGGAGGAGATAGAGGAGGCTTCCCTCCAAGAGGACCCCGGGGTTCCCGAGGGAACCCCTCTGGAGGA
GGAAACGTCCAGCACCGAGCTGGAGACTGGCAGTGTCCCAATCCGGGTTGTGGAAACCAGAACTTCGCCTGGAGA
ACAGAGTGCAACCAGTGTAAGGCCCCAAAGCCTGAAGGCTTCCTCCCGCCACCCTTTCCGCCCCCGGGTGGTGAT
CGTGGCAGAGGTGGCCCTGGTGGCATGCGGGGAGGAAGAGGTGGCCTCATGGATCGTGGTGGTCCCGGTGGAATG
TTCAGAGGTGGCCGTGGTGGAGACAGAGGTGGCTTCCGTGGTGGCCGGGGCATGGACCGAGGTGGCTTTGGTGGA

GGAAGACGAGGTGGCCCTGGGGGGCCCCCTGGACCTTTGATGGAACAGGCGATCGCATATCCCTATGATGTGCCG
GATTATGCTGGAGCACCAGGAAGTGCTGGTTCTGCTGCTGGTAGTGGAGCTTCTAACTTTACTCAGTTCGTTCTC
GTCGACAATGGCGGAACTGGCGACGTGACTGTCGCCCCAAGCAACTTCGCTAACGGGATCGCTGAATGGATCAGC
TCTAACTCGCGTTCACAGGCTTACAAAGTAACCTGTAGCGTTCGTCAGAGCTCTGCGCAGAATCGCAAATACACC
ATCAAAGTCGAGGTGCCTAAAGGCGCCTGGCGTTCGTACTTAAATATGGAACTAACCATTCCAATTTTCGCCACG
AATTCCGACTGCGAGCTTATTGTTAAGGCAATGCAAGGTCTCCTAAAAGATGGAAACCCGATTCCCTCAGCAATC
GCAGCAAACTCCGGCATCTACTAA (SEQ ID NO: 327)

Protein:

MAITQFRLFKFCTCLATVFSFLKRLICRSGRGRKLSGDQITLPTTVDYSSVPKQTDVEEWTSWDEDAPTSVKIEG
GNGNVATQQNSLEQLEPDYFKDMTPTIRKTQKIVIKKREPLNFGIPDGSTGFSSRLAATQDLPFIHQSSELGDLD
TWQENTNAWEEEEDAAWQAEEVLRQQKLADREKRAAEQQRKKMEKEAQRLMKKEQNKIGVKLSMASTDYSTYSQA
AAQQGYSAYTAQPTQGYAQTTQAYGQQSYGTYGQPTDVSYTQAQTTATYGQTAYATSYGQPPTGYTTPTAPQAYS
QPVQGYGTGAYDTTTATVTTTQASYAAQSAYGTQPAYPAYGQQPAATAPTRPQDGNKPTETSQPQSSTGGYNQPS
LGYGQSNYSYPQVPGSYPMQPVTAPPSYPPTSYSSTQPTSYDQSSYSQQNTYGQPSSYGQQSSYGQQSSYGQQPP
TSYPPQTGSYSQAPSQYSQQSSSYGQQSSFRQDHPSSMGVYGQESGGFSGPGENRSMSGPDNRGRGRGGFDRGGM
SRGGRGGGRGGMGSAGERGGFNKPGGPMDEGPDLDLGPPVDPDEDSDNSAIYVQGLNDSVTLDDLADFFKQCGVV
KMNKRTGQPMIHIYLDKETGKPKGDATVSYEDPPTAKAAVEWFDGKDFQGSKLKVSLARKKPPMNSMRGGLPPRE
GRGMPPPLRGGPGGPGGPGGPMGRMGGRGGDRGGFPPRGPRGSRGNPSGGGNVQHRAGDWQCPNPGCGNQNFAWR
TECNQCKAPKPEGFLPPPFPPPGGDRGRGGPGGMRGGRGGLMDRGGPGGMFRGGRGGDRGGFRGGRGMDRGGFGG
GRRGGPGGPPGPLMEQAIAYPYDVPDYAGAPGSAGSAAGSGASNFTQFVLVDNGGTGDVTVAPSNFANGIAEWIS
SNSRSQAYKVTCSVRQSSAQNRKYTIKVEVPKGAWRSYLNMELTIPIFATNSDCELIVKAMQGLLKDGNPIPSAI
AANSGIY* (SEQ ID NO: 328)

EBAG9₁₋₂₉::PylRS(AF)
DNA:
ATGGCCATCACCCAGTTTCGGTTATTTAAATTTTGTACCTGCCTAGCAACAGTATTCTCATTCCTAAAGAGATTA
ATATGCAGATCTGGCGCCGATTACAAGGACGATGATGACAAGGGAGCACCAGGAAGTGCTGGTTCTGCTGCTGGT
AGTGGAGCGTGCCCGGTGCCGCTGCAGCTGCCGCCGCTGGAACGCCTGACCCTGGATGATAAAAAACCGCTGAAT
ACCCTGATCTCTGCTACTGGTCTGTGGATGAGTCGTACCGGAACCATTCATAAAATCAAACACCACGAGGTTAGC
CGTTCGAAAATCTATATTGAGATGGCGTGTGGCGATCATCTGGTTGTGAACAATAGCCGCTCTTCTCGTACAGCA
CGTGCACTGCGTCACCACAAATATCGTAAAACCTGTAAACGTTGCCGTGTGTCCGATGAGGATCTGAACAAATTC
CTGACAAAAGCCAATGAGGACCAAACAAGCGTGAAAGTGAAAGTCGTTAGCGCTCCTACCCGTACTAAAAAAGCA
ATGCCGAAATCCGTTGCTCGTGCCCCTAAACCACTGGAAAACACTGAAGCAGCACAGGCACAGCCGTCTGGAAGC
AAATTCTCTCCGGCCATTCCTGTTTCTACCCAGGAGTCCGTTTCTGTTCCAGCAAGTGTGAGCACCAGCATTAGC
AGTATTAGCACCGGTGCCACCGCTAGCGCCCTGGTTAAAGGCAATACCAATCCGATTACAAGCATGTCTGCCCCG
GTTCAAGCATCAGCTCCAGCACTGACAAAATCCCAAACCGATCGTCTGGAGGTTCTGCTGAATCCGAAAGACGAA
ATCAGCCTGAATTCCGGCAAACCGTTTCGTGAACTGGAGAGCGAACTGCTGTCACGTCGTAAAAAAGACCTGCAA
CAAATCTATGCCGAAGAACGTGAGAACTATCTGGGGAAACTGGAACGTGAAATCACCCGCTTTTTCGTGGATCGT
GGCTTTCTGGAGATCAAATCCCCGATTCTGATTCCTCTGGAGTATATCGAGCGTATGGGCATCGACAATGATACC
GAACTGAGCAAACAAATTTTCCGTGTGGATAAAAACTTCTGTCTGCGCCCTATGCTAGCACCAAATCTGGCTAAC
TATCTGCGCAAACTGGACCGTGCCCTGCCTGATCCTATCAAAATCTTCGAGATCGGCCCGTGTTATCGTAAAGAG
TCCGACGGTAAAGAACATCTGGAGGAGTTTACCATGCTGAACTTTTGCCAAATGGGTTCAGGTTGTACTCGTGAG
AACCTGGAAAGCATCATCACCGATTTTCTGAACCACCTGGGCATTGACTTCAAAATTGTGGGCGACAGCTGTATG
GTGTTTGGCGACACCCTGGATGTCATGCACGGCGACCTGGAACTGTCTAGTGCCGTTGTGGGCCCAATCCCGCTG
GATCGTGAGTGGGGTATCGACAAACCTTGGATCGGTGCGGGTTTTGGTCTGGAGCGTCTGCTGAAAGTAAAACAC
GACTTCAAGAACATCAAACGTGCTGCACGTTCCGAGTCCTATTACAATGGTATTTCTACTAACCTGTAA (SEQ
ID NO: 329)

Protein:
MAITQFRLFKFCTCLATVFSFLKRLICRSGADYKDDDDKGAPGSAGSAAGSGACPVPLQLPPLERLTLDDKKPLN
TLISATGLWMSRTGTIHKIKHHEVSRSKIYIEMACGDHLVVNNSRSSRTARALRHHKYRKTCKRCRVSDEDLNKF
LTKANEDQTSVKVKVVSAPTRTKKAMPKSVARAPKPLENTEAAQAQPSGSKFSPAIPVSTQESVSVPASVSTSIS
SISTGATASALVKGNTNPITSMSAPVQASAPALTKSQTDRLEVLLNPKDEISLNSGKPFRELESELLSRRKKDLQ
QIYAEERENYLGKLEREITRFFVDRGFLEIKSPILIPLEYIERMGIDNDTELSKQIFRVDKNFCLRPMLAPNLAN
YLRKLDRALPDPIKIFEIGPCYRKESDGKEHLEEFTMLNFCQMGSGCTRENLESIITDFLNHLGIDFKIVGDSCM

VFGDTLDVMHGDLELSSAVVGPIPLDREWGIDKPWIGAGFGLERLLKVKHDFKNIKRAARSESYYNGISTNL*
(SEQ ID NO: 330)

EBAG9₁₋₂₉::FUS::PylRS(AF)
DNA:
ATGGCCATCACCCAGTTTCGGTTATTTAAATTTTGTACCTGCCTAGCAACAGTATTCTCATTCCTAAAGAGATTA
ATATGCAGATCTGGAGCACCCGGCTCCGCCGGCTCCGCCGCCGGCTCCGGCATGGCCTCAAACGATTATACCCAA
CAAGCAACCCAAAGCTATGGGGCCTACCCCACCCAGCCCGGGCAGGGCTATTCCCAGCAGAGCAGTCAGCCCTAC
GGACAGCAGAGTTACAGTGGTTATAGCCAGTCCACGGACACTTCAGGATATGGCCAGAGCAGCTATTCTTCTTAT
GGCCAGAGCCAGAACACAGGCTATGGAACTCAGTCAACTCCCCAGGGATATGGCTCGACTGGCGGCTATGGCAGT
AGCCAGAGCTCCCAATCGTCTTACGGGCAGCAGTCCTCCTACCCTGGCTATGGCCAGCAGCCAGCTCCCAGCAGC
ACCTCGGGAAGTTACGGTAGCAGTTCTCAGAGCAGCAGCTATGGGCAGCCCCAGAGTGGGAGCTACAGCCAGCAG
CCTAGCTATGGTGGACAGCAGCAAAGCTATGGACAGCAGCAAAGCTATAATCCCCCTCAGGGCTATGGACAGCAG
AACCAGTACAACAGCAGCAGTGGTGGTGGAGGTGGAGGTGGAGGTGGAGGTAACTATGGCCAAGATCAATCCTCC
ATGAGTAGTGGTGGTGGCAGTGGTGGCGGTTATGGCAATCAAGACCAGAGTGGTGGAGGTGGCAGCGGTGGCTAT
GGACAGCAGGACCGTGGAGGCCGCGGCAGGGGTGGCAGTGGTGGCGGCGGCGGCGGCGGTGGTGGTTACAAC
CGCAGCAGTGGTGGCTATGAACCCAGAGGTCGTGGAGGTGGCCGTGGAGGCAGAGGTGGCATGGGCGGAAGTGAC
CGTGGTGGCTTCAATAAATTTGGTGGCCCTCGGGACCAAGGATCACGTCATGACTCCGAACAGGATAATTCAGAC
AACAACACCATCTTTGTGCAAGGCCTGGGTGAGAATGTTACAATTGAGTCTGTGGCTGATTACTTCAAGCAGATT
GGTATTATTAAGACAAACAAGAAAACGGGACAGCCCATGATTAATTTGTACACAGACAGGGAAACTGGCAAGCTG
AAGGGAGAGGCAACGGTCTCTTTTGATGACCCACCTTCAGCTAAAGCAGCTATTGACTGGTTTGATGGTAAAGAA
TTCTCCGGAAATCCTATCAAGGTCTCATTTGCTACTCGCCGGGCAGACTTTAATCGGGGTGGTGGCAATGGTCGT
GGAGGCCGAGGGCGAGGAGGACCCATGGGCCGTGGAGGCTATGGAGGTGGTGGCAGTGGTGGTGGTGGCCGAGGA
GGATTTCCCAGTGGAGGTGGTGGCGGTGGAGGACAGCAGCGAGCTGGTGACTGGAAGTGTCCTAATCCCACCTGT
GAGAATATGAACTTCTCTTGGAGGAATGAATGCAACCAGTGTAAGGCCCCTAAACCAGATGGCCCAGGAGGGGGA
CCAGGTGGCTCTCACATGGGGGGTAACTACGGGGATGATCGTCGTGGTGGCAGAGGTGGTGCGATCGCAGGCGCC
GATTACAAGGACGATGATGACAAGGGAGCACCAGGAAGTGCTGGTTCTGCTGCTGGTAGTGGAGCGTGCCCGGTG
CCGCTGCAGCTGCCGCCGCTGGAACGCCTGACCCTGGATGATAAAAAACCGCTGAATACCCTGATCTCTGCTACT
GGTCTGTGGATGAGTCGTACCGGAACCATTCATAAAATCAAACACCACGAGGTTAGCCGTTCGAAAATCTATATT
GAGATGGCGTGTGGCGATCATCTGGTTGTGAACAATAGCCGCTCTTCTCGTACAGCACGTGCACTGCGTCACCAC
AAATATCGTAAAACCTGTAAACGTTGCCGTGTGTCCGATGAGGATCTGAACAAATTCCTGACAAAAGCCAATGAG
GACCAAACAAGCGTGAAAGTGAAAGTCGTTAGCGCTCCTACCCGTACTAAAAAAGCAATGCCGAAATCCGTTGCT
CGTGCCCCTAAACCACTGGAAAACACTGAAGCAGCACAGGCACAGCCGTCTGGAAGCAAATTCTCTCCGGCCATT
CCTGTTTCTACCCAGGAGTCCGTTTCTGTTCCAGCAAGTGTGAGCACCAGCATTAGCAGTATTAGCACCGGTGCC
ACCGCTAGCGCCCTGGTTAAAGGCAATACCAATCCGATTACAAGCATGTCTGCCCCGGTTCAAGCATCAGCTCCA
GCACTGACAAAATCCCAAACCGATCGTCTGGAGGTTCTGCTGAATCCGAAAGACGAAATCAGCCTGAATTCCGGC
AAACCGTTTCGTGAACTGGAGAGCGAACTGCTGTCACGTCGTAAAAAAGACCTGCAACAAATCTATGCCGAAGAA
CGTGAGAACTATCTGGGGAAACTGGAACGTGAAATCACCCGCTTTTTCGTGGATCGTGGCTTTCTGGAGATCAAA
TCCCCGATTCTGATTCCTCTGGAGTATATCGAGCGTATGGGCATCGACAATGATACCGAACTGAGCAAACAAATT
TTCCGTGTGGATAAAAACTTCTGTCTGCGCCCTATGCTAGCACCAAATCTGGCTAACTATCTGCGCAAACTGGAC
CGTGCCCTGCCTGATCCTATCAAAATCTTCGAGATCGGCCCGTGTTATCGTAAAGAGTCCGACGGTAAAGAACAT
CTGGAGGAGTTTACCATGCTGAACTTTTGCCAAATGGGTTCAGGTTGTACTCGTGAGAACCTGGAAAGCATCATC
ACCGATTTTCTGAACCACCTGGGCATTGACTTCAAAATTGTGGGCGACAGCTGTATGGTGTTTGGCGACACCCTG
GATGTCATGCACGGCGACCTGGAACTGTCTAGTGCCGTTGTGGGCCCAATCCCGCTGGATCGTGAGTGGGGTATC
GACAAACCTTGGATCGGTGCGGGTTTTGGTCTGGAGCGTCTGCTGAAAGTAAAACACGACTTCAAGAACATCAAA
CGTGCTGCACGTTCCGAGTCCTATTACAATGGTATTTCTACTAACCTGTAA (SEQ ID NO: 331)

Protein:
MAITQFRLFKFCTCLATVFSFLKRLICRSGAPGSAGSAAGSGMASNDYTQQATQSYGAYPTQPGQGYSQQSSQPY
GQQSYSGYSQSTDTSGYGQSSYSSYGQSQNTGYGTQSTPQGYGSTGGYGSSQSSQSSYGQQSSYPGYGQQPAPSS
TSGSYGSSSQSSSYGQPQSGSYSQQPSYGGQQQSYGQQQSYNPPQGYGQQNQYNSSSGGGGGGGGGNYGQDQSS
MSSGGGSGGGYGNQDQSGGGGSGGYGQQDRGGRGRGGSGGGGGGGGGGYNRSSGGYEPRGRGGGRGGRGGMGGSD
RGGFNKFGGPRDQGSRHDSEQDNSDNNTIFVQGLGENVTIESVADYFKQIGIIKTNKKTGQPMINLYTDRETGKL
KGEATVSFDDPPSAKAAIDWFDGKEFSGNPIKVSFATRRADFNRGGGNGRGGRGRGGPMGRGGYGGGGSGGGGRG
GFPSGGGGGGGGQQRAGDWKCPNPTCENMNFSWRNECNQCKAPKPDGPGGGPGGSHMGGNYGDDRRGGRGGAIAGA
DYKDDDDKGAPGSAGSAAGSGACPVPLQLPPLERLTLDDKKPLNTLISATGLWMSRTGTIHKIKHHEVSRSKIYI
EMACGDHLVVNNSRSSRTARALRHHKYRKTCKRCRVSDEDLNKFLTKANEDQTSVKVKVVSAPTRTKKAMPKSVA
RAPKPLENTEAAQAQPSGSKFSPAIPVSTQESVSVPASVSTSISSISTGATASALVKGNTNPITSMSAPVQASAP

ALTKSQTDRLEVLLNPKDEISLNSGKPFRELESELLSRRKKDLQQIYAEERENYLGKLEREITRFFVDRGFLEIK
SPILIPLEYIERMGIDNDTELSKQIFRVDKNFCLRPMLAPNLANYLRKLDRALPDPIKIFEIGPCYRKESDGKEH
LEEFTMLNFCQMGSGCTRENLESIITDFLNHLGIDFKIVGDSCMVFGDTLDVMHGDLELSSAVVGPIPLDREWGI
DKPWIGAGFGLERLLKVKHDFKNIKRAARSESYYNGISTNL* (SEQ ID NO: 332)

EBAG9₁₋₂₉::MCP
DNA:
ATGGCCATCACCCAGTTTCGGTTATTTAAATTTTGTACCTGCCTAGCAACAGTATTCTCATTCCTAAAGAGATTA
ATATGCAGATCTGCGATCGCATATCCCTATGATGTGCCGGATTATGCTGGAGCACCAGGAAGTGCTGGTTCTGCT
GCTGGTAGTGGAGCTTCTAACTTTACTCAGTTCGTTCTCGTCGACAATGGCGGAACTGGCGACGTGACTGTCGCC
CCAAGCAACTTCGCTAACGGGATCGCTGAATGGATCAGCTCTAACTCGCGTTCACAGGCTTACAAAGTAACCTGT
AGCGTTCGTCAGAGCTCTGCGCAGAATCGCAAATACACCATCAAAGTCGAGGTGCCTAAAGGCGCCTGGCGTTCG
TACTTAAATATGGAACTAACCATTCCAATTTTCGCCACGAATTCCGACTGCGAGCTTATTGTTAAGGCAATGCAA
GGTCTCCTAAAAGATGGAAACCCGATTCCCTCAGCAATCGCAGCAAACTCCGGCATCTACTAA (SEQ ID NO:
333)

Protein:
MAITQFRLFKFCTCLATVFSFLKRLICRSAIAYPYDVPDYAGAPGSAGSAAGSGASNFTQFVLVDNGGTGDVTVA
PSNFANGIAEWISSNSRSQAYKVTCSVRQSSAQNRKYTIKVEVPKGAWRSYLNMELTIPIFATNSDCELIVKAMQ
GLLKDGNPIPSAIAANSGIY* (SEQ ID NO: 334)

EBAG9₁₋₂₉::EWSR1::MCP
DNA:
ATGGCCATCACCCAGTTTCGGTTATTTAAATTTTGTACCTGCCTAGCAACAGTATTCTCATTCCTAAAGAGATTA
ATATGCAGATCTATGGCGTCCACGGATTACAGTACCTATAGCCAAGCTGCAGCGCAGCAGGGCTACAGTGCTTAC
ACCGCCCAGCCCACTCAAGGATATGCACAGACCACCCAGGCATATGGGCAACAAAGCTATGGAACCTATGGACAG
CCCACTGATGTCAGCTATACCCAGGCTCAGACCACTGCAACCTATGGGCAGACCGCCTATGCAACTTCTTATGGA
CAGCCTCCCACTGGTTATACTACTCCAACTGCCCCCCAGGCATACAGCCAGCCTGTCCAGGGGTATGGCACTGGT
GCTTATGATACCACCACTGCTACAGTCACCACCACCCAGGCCTCCTATGCAGCTCAGTCTGCATATGGCACTCAG
CCTGCTTATCCAGCCTATGGGCAGCAGCCAGCAGCCACTGCACCTACAAGACCGCAGGATGGAAACAAGCCCACT
GAGACTAGTCAACCTCAATCTAGCACAGGGGGTTACAACCAGCCCAGCCTAGGATATGGACAGAGTAACTACAGT
TATCCCCAGGTACCTGGGAGCTACCCCATGCAGCCAGTCACTGCACCTCCATCCTACCCTCCTACCAGCTATTCC
TCTACACAGCCGACTAGTTATGATCAGAGCAGTTACTCTCAGCAGAACACCTATGGGCAACCGAGCAGCTATGGA
CAGCAGAGTAGCTATGGTCAACAAAGCAGCTATGGGCAGCAGCCTCCCACTAGTTACCCACCCCAAACTGGATCC
TACAGCCAAGCTCCAAGTCAATATAGCCAACAGAGCAGCAGCTACGGGCAGCAGAGTTCATTCCGACAGGACCAC
CCCAGTAGCATGGGTGTTTATGGGCAGGAGTCTGGAGGATTTTCCGGACCAGGAGAGAACCGGAGCATGAGTGGC
CCTGATAACCGGGGCAGGGGAAGAGGGGGATTTGATCGTGGAGGCATGAGCAGAGGTGGGCGGGGAGGAGGACGC
GGTGGAATGGGCAGCGCTGGAGAGCGAGGTGGCTTCAATAAGCCTGGTGGACCCATGGATGAAGGACCAGATCTT
GATCTAGGCCCACCTGTAGATCCAGATGAAGACTCTGACAACAGTGCAATTTATGTACAAGGATTAAATGACAGT
GTGACTCTAGATGATCTGGCAGACTTCTTTAAGCAGTGTGGGGTTGTTAAGATGAACAAGAGAACTGGGCAACCC
ATGATCCACATCTACCTGGACAAGGAAACAGGAAAGCCCAAAGGCGATGCCACAGTGTCCTATGAAGACCCACCT
ACTGCCAAGGCTGCCGTGGAATGGTTTGATGGGAAAGATTTTCAAGGGAGCAAACTTAAAGTCTCCCTTGCTCGG
AAGAAGCCTCCAATGAACAGTATGCGGGGTGGTCTGCCACCCCGTGAGGGCAGAGGCATGCCACCACCACTCCGT
GGAGGTCCAGGAGGCCCAGGAGGTCCTGGGGGACCCATGGGTCGCATGGGAGGCCGTGGAGGAGATAGAGGAGGC
TTCCCTCCAAGAGGACCCCGGGGTTCCCGAGGGAACCCCTCTGGAGGAGGAAACGTCCAGCACCGAGCTGGAGAC
TGGCAGTGTCCCAATCCGGGTTGTGGAAACCAGAACTTCGCCTGGAGAACAGAGTGCAACCAGTGTAAGGCCCCA
AAGCCTGAAGGCTTCCTCCCGCCACCCTTTCCGCCCCCGGGTGGTGATCGTGGCAGAGGTGGCCCTGGTGGCATG
CGGGGAGGAAGAGGTGGCCTCATGGATCGTGGTGGTCCCGGTGGAATGTTCAGAGGTGGCCGTGGTGGAGACAGA
GGTGGCTTCCGTGGTGGCCGGGGCATGGACCGAGGTGGCTTTGGTGGAGGAAGACGAGGTGGCCCTGGGGGGCCC
CCTGGACCTTTGATGGAACAGGCGATCGCATATCCCTATGATGTGCCGGATTATGCTGGAGCACCAGGAAGTGCT
GGTTCTGCTGCTGGTAGTGGAGCTTCTAACTTTACTCAGTTCGTTCTCGTCGACAATGGCGGAACTGGCGACGTG
ACTGTCGCCCCAAGCAACTTCGCTAACGGGATCGCTGAATGGATCAGCTCTAACTCGCGTTCACAGGCTTACAAA
GTAACCTGTAGCGTTCGTCAGAGCTCTGCGCAGAATCGCAAATACACCATCAAAGTCGAGGTGCCTAAAGGCGCC
TGGCGTTCGTACTTAAATATGGAACTAACCATTCCAATTTTCGCCACGAATTCCGACTGCGAGCTTATTGTTAAG
GCAATGCAAGGTCTCCTAAAAGATGGAAACCCGATTCCCTCAGCAATCGCAGCAAACTCCGGCATCTACTAA
(SEQ ID NO: 335)

Protein:
MAITQFRLFKFCTCLATVFSFLKRLICRSMASTDYSTYSQAAAQQGYSAYTAQPTQGYAQTTQAYGQQSYGTYGQ
PTDVSYTQAQTTATYGQTAYATSYGQPPTGYTTPTAPQAYSQPVQGYGTGAYDTTTATVTTTQASYAAQSAYGTQ
PAYPAYGQQPAATAPTRPQDGNKPTETSQPQSSTGGYNQPSLGYGQSNYSYPQVPGSYPMQPVTAPPSYPPTSYS
STQPTSYDQSSYSQQNTYGQPSSYGQQSSYGQQSSYGQQPPTSYPPQTGSYSQAPSQYSQQSSSYGQQSSFRQDH
PSSMGVYGQESGGFSGPGENRSMSGPDNRGRGRGGFDRGGMSRGGRGGGRGGMGSAGERGGFNKPGGPMDEGPDL
DLGPPVDPDEDSDNSAIYVQGLNDSVTLDDLADFFKQCGVVKMNKRTGQPMIHIYLDKETGKPKGDATVSYEDPP
TAKAAVEWFDGKDFQGSKLKVSLARKKPPMNSMRGGLPPREGRGMPPPLRGGPGGPGGPGGPMGRMGGRGGDRGG
FPPRGPRGSRGNPSGGGNVQHRAGDWQCPNPGCGNQNFAWRTECNQCKAPKPEGFLPPPFPPPGGDRGRGGPGGM
RGGRGGLMDRGGPGGMFRGGRGGDRGGFRGGRGMDRGGFGGGRRGGPGGPPGPLMEQAIAYPYDVPDYAGAPGSA
GSAAGSGASNFTQFVLVDNGGTGDVTVAPSNFANGIAEWISSNSRSQAYKVTCSVRQSSAQNRKYTIKVEVPKGA
WRSYLNMELTIPIFATNSDCELIVKAMQGLLKDGNPIPSAIAANSGIY* (SEQ ID NO: 336)

EBAG9::EWSR1::4xλN22
DNA:
ATGGCCATCACCCAGTTTCGGTTATTTAAATTTTGTACCTGCCTAGCAACAGTATTCTCATTCCTAAAGAGATTA
ATATGCAGATCTGGCAGAGGACGGAAATTAAGTGGAGACCAAATAACTTTGCCAACTACAGTTGATTATTCATCA
GTTCCTAAGCAGACAGATGTTGAAGAGTGGACTTCCTGGGATGAAGATGCACCCACCAGTGTAAAGATCGAAGGA
GGGAATGGGAATGTGGCAACACAACAAAATTCTTTGGAACAACTGGAACCTGACTATTTTAAGGACATGACACCA
ACTATTAGGAAAACTCAGAAAATTGTTATTAAGAAGAGAGAACCATTGAATTTTGGCATCCCAGATGGGAGCACA
GGTTTCTCTAGTAGATTAGCAGCTACACAAGATCTGCCTTTTATTCATCAGTCTTCTGAATTAGGTGACTTAGAT
ACCTGGCAGGAAAATACCAATGCATGGGAAGAAGAAGAAGATGCAGCCTGGCAAGCAGAAGAAGTTCTGAGACAG
CAGAAACTAGCAGACAGAGAAAAGAGAGCAGCCGAACAACAAAGGAAGAAAATGGAAAAGGAAGCACAACGGCTA
ATGAAGAAGGAACAAAACAAAATTGGTGTGAAACTTTCAATGGCGTCCACGGATTACAGTACCTATAGCCAAGCT
GCAGCGCAGCAGGGCTACAGTGCTTACACCGCCCAGCCCACTCAAGGATATGCACAGACCACCCAGGCATATGGG
CAACAAAGCTATGGAACCTATGGACAGCCCACTGATGTCAGCTATACCCAGGCTCAGACCACTGCAACCTATGGG
CAGACCGCCTATGCAACTTCTTATGGACAGCCTCCCACTGGTTATACTACTCCAACTGCCCCCCAGGCATACAGC
CAGCCTGTCCAGGGGTATGGCACTGGTGCTTATGATACCACCACTGCTACAGTCACCACCACCCAGGCCTCCTAT
GCAGCTCAGTCTGCATATGGCACTCAGCCTGCTTATCCAGCCTATGGGCAGCAGCCAGCAGCCACTGCACCTACA
AGACCGCAGGATGGAAACAAGCCCACTGAGACTAGTCAACCTCAATCTAGCACAGGGGGTTACAACCAGCCCAGC
CTAGGATATGGACAGAGTAACTACAGTTATCCCCAGGTACCTGGGAGCTACCCCATGCAGCCAGTCACTGCACCT
CCATCCTACCCTCCTACCAGCTATTCCTCTACACAGCCGACTAGTTATGATCAGAGCAGTTACTCTCAGCAGAAC
ACCTATGGGCAACCGAGCAGCTATGGACAGCAGAGTAGCTATGGTCAACAAAGCAGCTATGGGCAGCAGCCTCCC
ACTAGTTACCCACCCCAAACTGGATCCTACAGCCAAGCTCCAAGTCAATATAGCCAACAGAGCAGCAGCTACGGG
CAGCAGAGTTCATTCCGACAGGACCACCCCAGTAGCATGGGTGTTTATGGGCAGGAGTCTGGAGGATTTTCCGGA
CCAGGAGAGAACCGGAGCATGAGTGGCCCTGATAACCGGGGCAGGGGAAGAGGGGGATTTGATCGTGGAGGCATG
AGCAGAGGTGGGCGGGGAGGAGGACGCGGTGGAATGGGCAGCGCTGGAGAGCGAGGTGGCTTCAATAAGCCTGGT
GGACCCATGGATGAAGGACCAGATCTTGATCTAGGCCCACCTGTAGATCCAGATGAAGACTCTGACAACAGTGCA
ATTTATGTACAAGGATTAAATGACAGTGTGACTCTAGATGATCTGGCAGACTTCTTTAAGCAGTGTGGGGTTGTT
AAGATGAACAAGAGAACTGGGCAACCCATGATCCACATCTACCTGGACAAGGAAACAGGAAAGCCCAAAGGCGAT
GCCACAGTGTCCTATGAAGACCCACCTACTGCCAAGGCTGCCGTGGAATGGTTTGATGGGAAAGATTTTCAAGGG
AGCAAACTTAAAGTCTCCCTTGCTCGGAAGAAGCCTCCAATGAACAGTATGCGGGGTGGTCTGCCACCCCGTGAG
GGCAGAGGCATGCCACCACCACTCCGTGGAGGTCCAGGAGGCCCAGGAGGTCCTGGGGGACCCATGGGTCGCATG
GGAGGCCGTGGAGGAGATAGAGGAGGCTTCCCTCCAAGAGGACCCCGGGGTTCCCGAGGGAACCCCTCTGGAGGA
GGAAACGTCCAGCACCGAGCTGGAGACTGGCAGTGTCCCAATCCGGGTTGTGGAAACCAGAACTTCGCCTGGAGA
ACAGAGTGCAACCAGTGTAAGGCCCCAAAGCCTGAAGGCTTCCTCCCGCCACCCTTTCCGCCCCCGGGTGGTGAT
CGTGGCAGAGGTGGCCCTGGTGGCATGCGGGGAGGAAGAGGTGGCCTCATGGATCGTGGTGGTCCCGGTGGAATG
TTCAGAGGTGGCCGTGGTGGAGACAGAGGTGGCTTCCGTGGTGGCCGGGGCATGGACCGAGGTGGCTTTGGTGGA
GGAAGACGAGGTGGCCCTGGGGGGCCCCCTGGACCTTTGATGGAACAGGCGATCGCAGGAGCACCAGGAAGTGCT
GGTTCTGCTGCTGGTAGTGGAGAGCAGAAGCTGATCTCAGAGGAGGACCTGCTAGCCACCATGGACGCACAAACA
CGACGACGTGAGCGTCGCGCTGAGAAACAAGCTCAATGGAAAGCTGCAAACCCACCGCTCGACGGAGCCGGAGCT
GGCGCTGGAGCTGGAGCCGGAGCTGGCGGTCTAGCCACCATGGACGCACAAACACGACGACGTGAGCGTCGCGCT
GAGAAACAAGCTCAATGGAAAGCTGCAAACCCACCGCTCGACGGAGCCGGAGCTGGCGCTGGAGCTGGAGCCGGA
GCTGGCGGTCTAGCCACCATGGACGCACAAACACGACGACGTGAGCGTCGCGCTGAGAAACAAGCTCAATGGAAA
GCTGCAAACCCACCGCTCGACGGAGCCGGAGCTGGCGCTGGAGCTGGAGCCGGAGCTGGCGGTCTAGCCACCATG

GACGCACAAACACGACGACGTGAGCGTCGCGCTGAGAAACAAGCTCAATGGAAAGCTGCAAACCCACCGCTCGAG
TCTAGAGGGCCCGTTTAA (SEQ ID NO: 337)

Protein:
MAITQFRLFKFCTCLATVFSFLKRLICRSGRGRKLSGDQITLPTTVDYSSVPKQTDVEEWTSWDEDAPTSVKIEG
GNGNVATQQNSLEQLEPDYFKDMTPTIRKTQKIVIKKREPLNFGIPDGSTGFSSRLAATQDLPFIHQSSELGDLD
TWQENTNAWEEEEDAAWQAEEVLRQQKLADREKRAAEQQRKKMEKEAQRLMKKEQNKIGVKLSMASTDYSTYSQA
AAQQGYSAYTAQPTQGYAQTTQAYGQQSYGTYGQPTDVSYTQAQTTATYGQTAYATSYGQPPTGYTTPTAPQAYS
QPVQGYGTGAYDTTTATVTTTQASYAAQSAYGTQPAYPAYGQQPAATAPTRPQDGNKPTETSQPQSSTGGYNQPS
LGYGQSNYSYPQVPGSYPMQPVTAPPSYPPTSYSSTQPTSYDQSSYSQQNTYGQPSSYGQQSSYGQQSSYGQQPP
TSYPPQTGSYSQAPSQYSQQSSSYGQQSSFRQDHPSSMGVYGQESGGFSGPGENRSMSGPDNRGRGRGGFDRGGM
SRGGRGGGRGGMGSAGERGGFNKPGGPMDEGPDLDLGPPVDPDEDSDNSAIYVQGLNDSVTLDDLADFFKQCGVV
KMNKRTGQPMIHIYLDKETGKPKGDATVSYEDPPTAKAAVEWFDGKDFQGSKLKVSLARKKPPMNSMRGGLPPRE
GRGMPPPLRGGPGGPGGPGGPMGRMGGRGGDRGGFPPRGPRGSRGNPSGGGNVQHRAGDWQCPNPGCGNQNFAWR
TECNQCKAPKPEGFLPPPFPPPGGDRGRGGPGGMRGGRGGLMDRGGPGGMFRGGRGGDRGGFRGGRGMDRGGFGG
GRRGGPGGPPGPLMEQAIAGAPGSAGSAAGSGEQKLISEEDLLATMDAQTRRRERRAEKQAQWKAANPPLDGAGA
GAGAGAGAGGLATMDAQTRRRERRAEKQAQWKAANPPLDGAGAGAGAGAGAGGLATMDAQTRRRERRAEKQAQWK
AANPPLDGAGAGAGAGAGAGGLATMDAQTRRRERRAEKQAQWKAANPPLESRGPV* (SEQ ID NO: 338)

EBAG9₁₋₂₉::EWSR1::4xλN22
DNA:
ATGGCCATCACCCAGTTTCGGTTATTTAAATTTTGTACCTGCCTAGCAACAGTATTCTCATTCCTAAAGAGATT
AATATGCAGATCTATGGCGTCCACGGATTACAGTACCTATAGCCAAGCTGCAGCGCAGCAGGGCTACAGTGCTTAC
ACCGCCCAGCCCACTCAAGGATATGCACAGACCACCCAGGCATATGGGCAACAAAGCTATGGAACCTATGGACAG
CCCACTGATGTCAGCTATACCCAGGCTCAGACCACTGCAACCTATGGGCAGACCGCCTATGCAACTTCTTATGGA
CAGCCTCCCACTGGTTATACTACTCCAACTGCCCCCCAGGCATACAGCCAGCCTGTCCAGGGGTATGGCACTGGT
GCTTATGATACCACCACTGCTACAGTCACCACCACCCAGGCCTCCTATGCAGCTCAGTCTGCATATGGCACTCAG
CCTGCTTATCCAGCCTATGGGCAGCAGCCAGCAGCCACTGCACCTACAAGACCGCAGGATGGAAACAAGCCCACT
GAGACTAGTCAACCTCAATCTAGCACAGGGGGTTACAACCAGCCCAGCCTAGGATATGGACAGAGTAACTACAGT
TATCCCCAGGTACCTGGGAGCTACCCCATGCAGCCAGTCACTGCACCTCCATCCTACCCTCCTACCAGCTATTCC
TCTACACAGCCGACTAGTTATGATCAGAGCAGTTACTCTCAGCAGAACACCTATGGGCAACCGAGCAGCTATGGA
CAGCAGAGTAGCTATGGTCAACAAAGCAGCTATGGGCAGCAGCCTCCCACTAGTTACCCACCCCAAACTGGATCC
TACAGCCAAGCTCCAAGTCAATATAGCCAACAGAGCAGCAGCTACGGGCAGCAGAGTTCATTCCGACAGGACCAC
CCCAGTAGCATGGGTGTTTATGGGCAGGAGTCTGGAGGATTTTCCGGACCAGGAGAGAACCGGAGCATGAGTGGC
CCTGATAACCGGGGCAGGGGAAGAGGGGGATTTGATCGTGGAGGCATGAGCAGAGGTGGGCGGGGAGGAGGACGC
GGTGGAATGGGCAGCGCTGGAGAGCGAGGTGGCTTCAATAAGCCTGGTGGACCCATGGATGAAGGACCAGATCTT
GATCTAGGCCCACCTGTAGATCCAGATGAAGACTCTGACAACAGTGCAATTTATGTACAAGGATTAAATGACAGT
GTGACTCTAGATGATCTGGCAGACTTCTTTAAGCAGTGTGGGGTTGTTAAGATGAACAAGAGAACTGGGCAACCC
ATGATCCACATCTACCTGGACAAGGAAACAGGAAAGCCCAAAGGCGATGCCACAGTGTCCTATGAAGACCCACCT
ACTGCCAAGGCTGCCGTGGAATGGTTTGATGGGAAAGATTTTCAAGGGAGCAAACTTAAAGTCTCCCTTGCTCGG
AAGAAGCCTCCAATGAACAGTATGCGGGGTGGTCTGCCACCCCGTGAGGGCAGAGGCATGCCACCACCACTCCGT
GGAGGTCCAGGAGGCCCAGGAGGTCCTGGGGGACCCATGGGTCGCATGGGAGGCCGTGGAGGAGATAGAGGAGGC
TTCCCTCCAAGAGGACCCCGGGGTTCCCGAGGGAACCCCTCTGGAGGAGGAAACGTCCAGCACCGAGCTGGAGAC
TGGCAGTGTCCCAATCCGGGTTGTGGAAACCAGAACTTCGCCTGGAGAACAGAGTGCAACCAGTGTAAGGCCCCA
AAGCCTGAAGGCTTCCTCCCGCCACCCTTTCCGCCCCCGGGTGGTGATCGTGGCAGAGGTGGCCCTGGTGGCATG
CGGGGAGGAAGAGGTGGCCTCATGGATCGTGGTGGTCCCGGTGGAATGTTCAGAGGTGGCCGTGGTGGAGACAGA
GGTGGCTTCCGTGGTGGCCGGGGCATGGACCGAGGTGGCTTTGGTGGAGGAAGACGAGGTGGCCCTGGGGGGCCC
CCTGGACCTTTGATGGAACAGGCGATCGCAGGAGCACCAGGAAGTGCTGGTTCTGCTGCTGGTAGTGGAGAGCAG
AAGCTGATCTCAGAGGAGGACCTGCTAGCCACCATGGACGCACAAACACGACGACGTGAGCGTCGCGCTGAGAAA
CAAGCTCAATGGAAAGCTGCAAACCCACCGCTCGACGGAGCCGGAGCTGGCGCTGGAGCTGGAGCCGGAGCTGGC
GGTCTAGCCACCATGGACGCACAAACACGACGACGTGAGCGTCGCGCTGAGAAACAAGCTCAATGGAAAGCTGCA
AACCCACCGCTCGACGGAGCCGGAGCTGGCGCTGGAGCTGGAGCCGGAGCTGGCGGTCTAGCCACCATGGACGCA
CAAACACGACGACGTGAGCGTCGCGCTGAGAAACAAGCTCAATGGAAAGCTGCAAACCCACCGCTCGACGGAGCC
GGAGCTGGCGCTGGAGCTGGAGCCGGAGCTGGCGGTCTAGCCACCATGGACGCACAAACACGACGACGTGAGCGT
CGCGCTGAGAAACAAGCTCAATGGAAAGCTGCAAACCCACCGCTCGAGTCTAGAGGGCCCGTTTAA (SEQ ID
NO: 339)

Protein:

MAITQFRLFKFCTCLATVFSFLKRLICRSMASTDYSTYSQAAAQQGYSAYTAQPTQGYAQTTQAYGQQSYGTYGQ
PTDVSYTQAQTTATYGQTAYATSYGQPPTGYTTPTAPQAYSQPVQGYGTGAYDTTTATVTTTQASYAAQSAYGTQ
PAYPAYGQQPAATAPTRPQDGNKPTETSQPQSSTGGYNQPSLGYGQSNYSYPQVPGSYPMQPVTAPPSYPPTSYS
STQPTSYDQSSYSQQNTYGQPSSYGQQSSYGQQSSYGQQPPTSYPPQTGSYSQAPSQYSQQSSSYGQQSSFRQDH
PSSMGVYGQESGGFSGPGENRSMSGPDNRGRGRGGFDRGGMSRGGRGGGRGGMGSAGERGGFNKPGGPMDEGPDL
DLGPPVDPDEDSDNSAIYVQGLNDSVTLDDLADFFKQCGVVKMNKRTGQPMIHIYLDKETGKPKGDATVSYEDPP
TAKAAVEWFDGKDFQGSKLKVSLARKKPPMNSMRGGLPPREGRGMPPPLRGGPGGPGGPGGPMGRMGGRGGDRGG
FPPRGPRGSRGNPSGGGNVQHRAGDWQCPNPGCGNQNFAWRTECNQCKAPKPEGFLPPPFPPPGGDRGRGGPGGM
RGGRGGLMDRGGPGGMFRGGRGGDRGGFRGGRGMDRGGFGGGRRGGPGGPPGPLMEQAIAGAPGSAGSAAGSGEQ
KLISEEDLLATMDAQTRRRERRAEKQAQWKAANPPLDGAGAGAGAGAGAGGLATMDAQTRRRERRAEKQAQWKAA
NPPLDGAGAGAGAGAGAGGLATMDAQTRRRERRAEKQAQWKAANPPLDGAGAGAGAGAGAGGLATMDAQTRRRER
RAEKQAQWKAANPPLESRGPV* (SEQ ID NO: 340)

**EBAG9::PylRS(AA)**
DNA:
ATGGCCATCACCCAGTTTCGGTTATTTAAATTTTGTACCTGCCTAGCAACAGTATTCTCATTCCTAAAGAGATTA
ATATGCAGATCTGGCAGAGGACGGAAATTAAGTGGAGACCAAATAACTTTGCCAACTACAGTTGATTATTCATCA
GTTCCTAAGCAGACAGATGTTGAAGAGTGGACTTCCTGGGATGAAGATGCACCCACCAGTGTAAAGATCGAAGGA
GGGAATGGGAATGTGGCAACACAACAAAATTCTTTGGAACAACTGGAACCTGACTATTTTAAGGACATGACACCA
ACTATTAGGAAAACTCAGAAAATTGTTATTAAGAAGAGAGAACCATTGAATTTTGGCATCCCAGATGGGAGCACA
GGTTTCTCTAGTAGATTAGCAGCTACACAAGATCTGCCTTTTATTCATCAGTCTTCTGAATTAGGTGACTTAGAT
ACCTGGCAGGAAAATACCAATGCATGGGAAGAAGAAGAAGATGCAGCCTGGCAAGCAGAAGAAGTTCTGAGACAG
CAGAAACTAGCAGACAGAGAAAAGAGAGCAGCCGAACAACAAAGGAAGAAAATGGAAAAGGAAGCACAACGGCTA
ATGAAGAAGGAACAAAACAAAATTGGTGTGAAACTTTCAGGCGCCGATTACAAGGACGATGATGACAAGGGAGCA
CCAGGAAGTGCTGGTTCTGCTGCTGGTAGTGGAGCGTGCCCGGTGCCGCTGCAGCTGCCGCCGCTGGAACGCCTG
ACCCTGGATGACAAAAAACCGCTGAATACCCTGATCTCTGCTACTGGTCTGTGGATGAGTCGTACCGGAACCATT
CATAAAATCAAACACCACGAGGTTAGCCGTTCGAAAATCTATATTGAGATGGCGTGTGGCGATCATCTGGTTGTG
AACAATAGCCGCTCTTCTCGTACAGCACGTGCACTGCGTCACCACAAATATCGTAAAACCTGTAAACGTTGCCGT
GTGTCCGATGAGGATCTGAACAAATTCCTGACAAAAGCCAATGAGGACCAAACAAGCGTGAAAGTGAAAGTCGTT
AGCGCTCCTACCCGTACTAAAAAAGCAATGCCGAAATCCGTTGCTCGTGCCCCTAAACCACTGGAAAACACTGAA
GCAGCACAGGCACAGCCGTCTGGAAGCAAATTCTCTCCGGCCATTCCTGTTTCTACCCAGGAGTCCGTTTCTGTT
CCAGCAAGTGTGAGCACCAGCATTAGCAGTATTAGCACCGGTGCCACCGCTAGCGCCCTGGTTAAAGGCAATACC
AATCCGATTACAAGCATGTCTGCCCCGGTTCAAGCATCAGCTCCAGCACTGACAAAATCCCAAACCGATCGTCTG
GAGGTTCTGCTGAATCCGAAAGACGAAATCAGCCTGAATTCCGGCAAACCGTTTCGTGAACTGGAGAGCGAACTG
CTGTCACGTCGTAAAAAAGACCTGCAACAAATCTATGCCGAAGAACGTGAGAACTATCTGGGGAAACTGGAACGT
GAAATCACCCGCTTTTTCGTGGATCGTGGCTTTCTGGAGATCAAATCCCCGATTCTGATTCCTCTGGAGTATATC
GAGCGTATGGGCATCGACAATGATACCGAACTGAGCAAACAAATTTTCCGTGTGGATAAAAACTTCTGTCTGCGC
CCTATGCTGGCACCAAATCTGTATAACTATCTGCGCAAACTGGACCGTGCCCTGCCTGATCCTATCAAAATCTTC
GAGATCGGCCCGTGTTATCGTAAAGAGTCCGACGGTAAAGAACATCTGGAGGAGTTTACCATGCTGGCCTTTGCC
CAAATGGGTTCAGGTTGTACTCGTGAGAACCTGGAAAGCATCATCACCGATTTTCTGAACCACCTGGGCATTGAC
TTCAAAATTGTGGGCGACAGCTGTATGGTGTATGGCGACACCCTGGATGTCATGCACGGCGACCTGGAACTGTCT
AGTGCCGTTGTTGGACCAATTCCGCTGGACCGTGAGTGGGGTATCGACAAACCGTGGATCGGAGCAGGATTCGGT
CTGGAACGCCTGCTGAAAGTGAAACACGACTTCAAAAACATCAAACGTGCCGCCCGTTCTGAATCGTATTATAAC
GGGATCTCTACGAACCTGTAA (SEQ ID NO: 341)

Protein:
MAITQFRLFKFCTCLATVFSFLKRLICRSGRGRKLSGDQITLPTTVDYSSVPKQTDVEEWTSWDEDAPTSVKIEG
GNGNVATQQNSLEQLEPDYFKDMTPTIRKTQKIVIKKREPLNFGIPDGSTGFSSRLAATQDLPFIHQSSELGDLD
TWQENTNAWEEEEDAAWQAEEVLRQQKLADREKRAAEQQRKKMEKEAQRLMKKEQNKIGVKLSGADYKDDDDKGA
PGSAGSAAGSGACPVPLQLPPLERLTLDDKKPLNTLISATGLWMSRTGTIHKIKHHEVSRSKIYIEMACGDHLVV
NNSRSSRTARALRHHKYRKTCKRCRVSDEDLNKFLTKANEDQTSVKVKVVSAPTRTKKAMPKSVARAPKPLENTE
AAQAQPSGSKFSPAIPVSTQESVSVPASVSTSISSISTGATASALVKGNTNPITSMSAPVQASAPALTKSQTDRL
EVLLNPKDEISLNSGKPFRELESELLSRRKKDLQQIYAEERENYLGKLEREITRFFVDRGFLEIKSPILIPLEYI
ERMGIDNDTELSKQIFRVDKNFCLRPMLAPNLYNYLRKLDRALPDPIKIFEIGPCYRKESDGKEHLEEFTMLAFA
QMGSGCTRENLESIITDFLNHLGIDFKIVGDSCMVYGDTLDVMHGDLELSSAVVGPIPLDREWGIDKPWIGAGFG
LERLLKVKHDFKNIKRAARSESYYNGISTNL* (SEQ ID NO: 342)

EBAG9::PylRS(AAAF)
DNA:
ATGGCCATCACCCAGTTTCGGTTATTTAAATTTTGTACCTGCCTAGCAACAGTATTCTCATTCCTAAAGAGATTA
ATATGCAGATCTGGCAGAGGACGGAAATTAAGTGGAGACCAAATAACTTTGCCAACTACAGTTGATTATTCATCA
GTTCCTAAGCAGACAGATGTTGAAGAGTGGACTTCCTGGGATGAAGATGCACCCACCAGTGTAAAGATCGAAGGA
GGGAATGGGAATGTGGCAACACAACAAAATTCTTTGGAACAACTGGAACCTGACTATTTTAAGGACATGACACCA
ACTATTAGGAAAACTCAGAAAATTGTTATTAAGAAGAGAGAACCATTGAATTTTGGCATCCCAGATGGGAGCACA
GGTTTCTCTAGTAGATTAGCAGCTACACAAGATCTGCCTTTTATTCATCAGTCTTCTGAATTAGGTGACTTAGAT
ACCTGGCAGGAAAATACCAATGCATGGGAAGAAGAAGAAGATGCAGCCTGGCAAGCAGAAGAAGTTCTGAGACAG
CAGAAACTAGCAGACAGAGAAAAGAGAGCAGCCGAACAACAAAGGAAGAAAATGGAAAAGGAAGCACAACGGCTA
ATGAAGAAGGAACAAAACAAAATTGGTGTGAAACTTTCAGGCGCCGATTACAAGGACGATGATGACAAGGGAGCA
CCAGGAAGTGCTGGTTCTGCTGCTGGTAGTGGAGCGTGCCCGGTGCCGCTGCAGCTGCCGCCGCTGGAACGCCTG
ACCCTGGATGATAAAAAACCGCTGAATACCCTGATCTCTGCTACTGGTCTGTGGATGAGTCGTACCGGAACCATT
CATAAAATCAAACACCACGAGGTTAGCCGTTCGAAAATCTATATTGAGATGGCGTGTGGCGATCATCTGGTTGTG
AACAATAGCCGCTCTTCTCGTACAGCACGTGCACTGCGTCACCACAAATATCGTAAAACCTGTAAACGTTGCCGT
GTGTCCGATGAGGATCTGAACAAATTCCTGACAAAAGCCAATGAGGACCAAACAAGCGTGAAAGTGAAAGTCGTT
AGCGCTCCTACCCGTACTAAAAAAGCAATGCCGAAATCCGTTGCTCGTGCCCCTAAACCACTGGAAAACACTGAA
GCAGCACAGGCACAGCCGTCTGGAAGCAAATTCTCTCCGGCCATTCCTGTTTCTACCCAGGAGTCCGTTTCTGTT
CCAGCAAGTGTGAGCACCAGCATTAGCAGTATTAGCACCGGTGCCACCGCTAGCGCCCTGGTTAAAGGCAATACC
AATCCGATTACAAGCATGTCTGCCCCGGTTCAAGCATCAGCTCCAGCACTGACAAAATCCCAAACCGATCGTCTG
GAGGTTCTGCTGAATCCGAAAGACGAAATCAGCCTGAATTCCGGCAAACCGTTTCGTGAACTGGAGAGCGAACTG
CTGTCACGTCGTAAAAAAGACCTGCAACAAATCTATGCCGAAGAACGTGAGAACTATCTGGGGAAACTGGAACGT
GAAATCACCCGCTTTTTCGTGGATCGTGGCTTTCTGGAGATCAAATCCCCGATTCTGATTCCTCTGGAGTATATC
GAGCGTATGGGCATCGACAATGATACCGAACTGAGCAAACAAATTTTCCGTGTGGATAAAAACTTCTGTCTGCGC
CCTATGCTAGCACCAAATCTGGCTAACTATCTGCGCAAACTGGACCGTGCCCTGCCTGATCCTATCAAAATCTTC
GAGATCGGCCCGTGTTATCGTAAAGAGTCCGACGGTAAAGAACATCTGGAGGAGTTTACCATGCTGGCCTTTGCC
CAAATGGGTTCAGGTTGTACTCGTGAGAACCTGGAAAGCATCATCACCGATTTTCTGAACCACCTGGGCATTGAC
TTCAAAATTGTGGGCGACAGCTGTATGGTGTTTGGCGACACCCTGGATGTCATGCACGGCGACCTGGAACTGTCT
AGTGCCGTTGTGGGCCCAATCCCGCTGGATCGTGAGTGGGGTATCGACAAACCTTGGATCGGTGCGGGTTTTGGT
CTGGAGCGTCTGCTGAAAGTAAAACACGACTTCAAGAACATCAAACGTGCTGCACGTTCCGAGTCCTATTACAAT
GGTATTTCTACTAACCTGTAA (SEQ ID NO: 343)

Protein:
MAITQFRLFKFCTCLATVFSFLKRLICRSGRGRKLSGDQITLPTTVDYSSVPKQTDVEEWTSWDEDAPTSVKIEG
GNGNVATQQNSLEQLEPDYFKDMTPTIRKTQKIVIKKREPLNFGIPDGSTGFSSRLAATQDLPFIHQSSELGDLD
TWQENTNAWEEEEDAAWQAEEVLRQQKLADREKRAAEQQRKKMEKEAQRLMKKEQNKIGVKLSGADYKDDDDKGA
PGSAGSAAGSGACPVPLQLPPLERLTLDDKKPLNTLISATGLWMSRTGTIHKIKHHEVSRSKIYIEMACGDHLVV
NNSRSSRTARALRHHKYRKTCKRCRVSDEDLNKFLTKANEDQTSVKVKVVSAPTRTKKAMPKSVARAPKPLENTE
AAQAQPSGSKFSPAIPVSTQESVSVPASVSTSISSISTGATASALVKGNTNPITSMSAPVQASAPALTKSQTDRL
EVLLNPKDEISLNSGKPFRELESELLSRRKKDLQQIYAEERENYLGKLEREITRFFVDRGFLEIKSPILIPLEYI
ERMGIDNDTELSKQIFRVDKNFCLRPMLAPNLANYLRKLDRALPDPIKIFEIGPCYRKESDGKEHLEEFTMLAFA
QMGSGCTRENLESIITDFLNHLGIDFKIVGDSCMVFGDTLDVMHGDLELSSAVVGPIPLDREWGIDKPWIGAGFG
LERLLKVKHDFKNIKRAARSESYYNGISTNL* (SEQ ID NO: 344)

EBAG9::FUS::PylRS(AA)
DNA:
ATGGCCATCACCCAGTTTCGGTTATTTAAATTTTGTACCTGCCTAGCAACAGTATTCTCATTCCTAAAGAGATTA
ATATGCAGATCTGGCAGAGGACGGAAATTAAGTGGAGACCAAATAACTTTGCCAACTACAGTTGATTATTCATCA
GTTCCTAAGCAGACAGATGTTGAAGAGTGGACTTCCTGGGATGAAGATGCACCCACCAGTGTAAAGATCGAAGGA
GGGAATGGGAATGTGGCAACACAACAAAATTCTTTGGAACAACTGGAACCTGACTATTTTAAGGACATGACACCA
ACTATTAGGAAAACTCAGAAAATTGTTATTAAGAAGAGAGAACCATTGAATTTTGGCATCCCAGATGGGAGCACA
GGTTTCTCTAGTAGATTAGCAGCTACACAAGATCTGCCTTTTATTCATCAGTCTTCTGAATTAGGTGACTTAGAT
ACCTGGCAGGAAAATACCAATGCATGGGAAGAAGAAGAAGATGCAGCCTGGCAAGCAGAAGAAGTTCTGAGACAG
CAGAAACTAGCAGACAGAGAAAAGAGAGCAGCCGAACAACAAAGGAAGAAAATGGAAAAGGAAGCACAACGGCTA
ATGAAGAAGGAACAAAACAAAATTGGTGTGAAACTTTCAGGAGCACCCGGCTCCGCCGGCTCCGCCGCCGGCTCC
GGCATGGCCTCAAACGATTATACCCAACAAGCAACCCAAAGCTATGGGGCCTACCCCACCCAGCCCGGGCAGGGC

TATTCCCAGCAGAGCAGTCAGCCCTACGGACAGCAGAGTTACAGTGGTTATAGCCAGTCCACGGACACTTCAGGA
TATGGCCAGAGCAGCTATTCTTCTTATGGCCAGAGCCAGAACACAGGCTATGGAACTCAGTCAACTCCCCAGGGA
TATGGCTCGACTGGCGGCTATGGCAGTAGCCAGAGCTCCCAATCGTCTTACGGGCAGCAGTCCTCCTACCCTGGC
TATGGCCAGCAGCCAGCTCCCAGCAGCACCTCGGGAAGTTACGGTAGCAGTTCTCAGAGCAGCAGCTATGGGCAG
CCCCAGAGTGGGAGCTACAGCCAGCAGCCTAGCTATGGTGGACAGCAGCAAAGCTATGGACAGCAGCAAAGCTAT
AATCCCCCTCAGGGCTATGGACAGCAGAACCAGTACAACAGCAGCAGTGGTGGTGGAGGTGGAGGTGGAGGTGGA
GGTAACTATGGCCAAGATCAATCCTCCATGAGTAGTGGTGGTGGCAGTGGTGGCGGTTATGGCAATCAAGACCAG
AGTGGTGGAGGTGGCAGCGGTGGCTATGGACAGCAGGACCGTGGAGGCCGCGGCAGGGGTGGCAGTGGTGGCGGC
GGCGGCGGCGGCGGTGGTGGTTACAACCGCAGCAGTGGTGGCTATGAACCCAGAGGTCGTGGAGGTGGCCGTGGA
GGCAGAGGTGGCATGGGCGGAAGTGACCGTGGTGGCTTCAATAAATTTGGTGGCCCTCGGGACCAAGGATCACGT
CATGACTCCGAACAGGATAATTCAGACAACAACACCATCTTTGTGCAAGGCCTGGGTGAGAATGTTACAATTGAG
TCTGTGGCTGATTACTTCAAGCAGATTGGTATTATTAAGACAAACAAGAAAACGGGACAGCCCATGATTAATTTG
TACACAGACAGGGAAACTGGCAAGCTGAAGGGAGAGGCAACGGTCTCTTTTGATGACCCACCTTCAGCTAAAGCA
GCTATTGACTGGTTTGATGGTAAAGAATTCTCCGGAAATCCTATCAAGGTCTCATTTGCTACTCGCCGGGCAGAC
TTTAATCGGGGTGGTGGCAATGGTCGTGGAGGCCGAGGGCGAGGAGGACCCATGGGCCGTGGAGGCTATGGAGGT
GGTGGCAGTGGTGGTGGTGGCCGAGGAGGATTTCCCAGTGGAGGTGGTGGCGGTGGAGGACAGCAGCGAGCTGGT
GACTGGAAGTGTCCTAATCCCACCTGTGAGAATATGAACTTCTCTTGGAGGAATGAATGCAACCAGTGTAAGGCC
CCTAAACCAGATGGCCCAGGAGGGGGACCAGGTGGCTCTCACATGGGGGGTAACTACGGGGATGATCGTCGTGGT
GGCAGAGGTGGTGCGATCGCAGGCGCCGATTACAAGGACGATGATGACAAGGGAGCACCAGGAAGTGCTGGTTCT
GCTGCTGGTAGTGGAGCGTGCCCGGTGCCGCTGCAGCTGCCGCCGCTGGAACGCCTGACCCTGGATGACAAAAAA
CCGCTGAATACCCTGATCTCTGCTACTGGTCTGTGGATGAGTCGTACCGGAACCATTCATAAAATCAAACACCAC
GAGGTTAGCCGTTCGAAAATCTATATTGAGATGGCGTGTGGCGATCATCTGGTTGTGAACAATAGCCGCTCTTCT
CGTACAGCACGTGCACTGCGTCACCACAAATATCGTAAAACCTGTAAACGTTGCCGTGTGTCCGATGAGGATCTG
AACAAATTCCTGACAAAAGCCAATGAGGACCAAACAAGCGTGAAAGTGAAAGTCGTTAGCGCTCCTACCCGTACT
AAAAAAGCAATGCCGAAATCCGTTGCTCGTGCCCCTAAACCACTGGAAAACACTGAAGCAGCACAGGCACAGCCG
TCTGGAAGCAAATTCTCTCCGGCCATTCCTGTTTCTACCCAGGAGTCCGTTTCTGTTCCAGCAAGTGTGAGCACC
AGCATTAGCAGTATTAGCACCGGTGCCACCGCTAGCGCCCTGGTTAAAGGCAATACCAATCCGATTACAAGCATG
TCTGCCCCGGTTCAAGCATCAGCTCCAGCACTGACAAAATCCCAAACCGATCGTCTGGAGGTTCTGCTGAATCCG
AAAGACGAAATCAGCCTGAATTCCGGCAAACCGTTTCGTGAACTGGAGAGCGAACTGCTGTCACGTCGTAAAAAA
GACCTGCAACAAATCTATGCCGAAGAACGTGAGAACTATCTGGGGAAACTGGAACGTGAAATCACCCGCTTTTTC
GTGGATCGTGGCTTTCTGGAGATCAAATCCCCGATTCTGATTCCTCTGGAGTATATCGAGCGTATGGGCATCGAC
AATGATACCGAACTGAGCAAACAAATTTTCCGTGTGGATAAAAACTTCTGTCTGCGCCCTATGCTGGCACCAAAT
CTGTATAACTATCTGCGCAAACTGGACCGTGCCCTGCCTGATCCTATCAAAATCTTCGAGATCGGCCCGTGTTAT
CGTAAAGAGTCCGACGGTAAAGAACATCTGGAGGAGTTTACCATGCTGGCCTTTGCCCAAATGGGTTCAGGTTGT
ACTCGTGAGAACCTGGAAAGCATCATCACCGATTTTCTGAACCACCTGGGCATTGACTTCAAAATTGTGGGCGAC
AGCTGTATGGTGTATGGCGACACCCTGGATGTCATGCACGGCGACCTGGAACTGTCTAGTGCCGTTGTTGGACCA
ATTCCGCTGGACCGTGAGTGGGGTATCGACAAACCGTGGATCGGAGCAGGATTCGGTCTGGAACGCCTGCTGAAA
GTGAAACACGACTTCAAAAACATCAAACGTGCCGCCCGTTCTGAATCGTATTATAACGGGATCTCTACGAACCTG
TAA (SEQ ID NO: 345)

Protein:
MAITQFRLFKFCTCLATVFSFLKRLICRSGRGRKLSGDQITLPTTVDYSSVPKQTDVEEWTSWDEDAPTSVKIEG
GNGNVATQQNSLEQLEPDYFKDMTPTIRKTQKIVIKKREPLNFGIPDGSTGFSSRLAATQDLPFIHQSSELGDLD
TWQENTNAWEEEEDAAWQAEEVLRQQKLADREKRAAEQQRKKMEKEAQRLMKKEQNKIGVKLSGAPGSAGSAAGS
GMASNDYTQQATQSYGAYPTQPGQGYSQQSSQPYGQQSYSGYSQSTDTSGYGQSSYSSYGQSQNTGYGTQSTPQG
YGSTGGYGSSQSSQSSYGQQSSYPGYGQQPAPSSTSGSYGSSSQSSSYGQPQSGSYSQQPSYGGQQQSYGQQQSY
NPPQGYGQQNQYNSSSGGGGGGGGGGNYGQDQSSMSSGGGSGGGYGNQDQSGGGGSGGYGQQDRGGRGRGGSGGG
GGGGGGGYNRSSGGYEPRGRGGGRGGRGGMGGSDRGGFNKFGGPRDQGSRHDSEQDNSDNNTIFVQGLGENVTIE
SVADYFKQIGIIKTNKKTGQPMINLYTDRETGKLKGEATVSFDDPPSAKAAIDWFDGKEFSGNPIKVSFATRRAD
FNRGGGNGRGGRGRGGPMGRGGYGGGGSGGGGRGGFPSGGGGGGGQQRAGDWKCPNPTCENMNFSWRNECNQCKA
PKPDGPGGGPGGSHMGGNYGDDRRGGRGGAIAGADYKDDDDKGAPGSAGSAAGSGACPVPLQLPPLERLTLDDKK
PLNTLISATGLWMSRTGTIHKIKHHEVSRSKIYIEMACGDHLVVNNSRSSRTARALRHHKYRKTCKRCRVSDEDL
NKFLTKANEDQTSVKVKVVSAPTRTKKAMPKSVARAPKPLENTEAAQAQPSGSKFSPAIPVSTQESVSVPASVST
SISSISTGATASALVKGNTNPITSMSAPVQASAPALTKSQTDRLEVLLNPKDEISLNSGKPFRELESELLSRRKK
DLQQIYAEERENYLGKLEREITRFFVDRGFLEIKSPILIPLEYIERMGIDNDTELSKQIFRVDKNFCLRPMLAPN
LYNYLRKLDRALPDPIKIFEIGPCYRKESDGKEHLEEFTMLAFAQMGSGCTRENLESIITDFLNHLGIDFKIVGD
SCMVYGDTLDVMHGDLELSSAVVGPIPLDREWGIDKPWIGAGFGLERLLKVKHDFKNIKRAARSESYYNGISTNL
* (SEQ ID NO: 346)

EBAG9::FUS::PylRS(AAAF)
DNA:
ATGGCCATCACCCAGTTTCGGTTATTTAAATTTTGTACCTGCCTAGCAACAGTATTCTCATTCCTAAAGAGATTA
ATATGCAGATCTGGCAGAGGACGGAAATTAAGTGGAGACCAAATAACTTTGCCAACTACAGTTGATTATTCATCA
GTTCCTAAGCAGACAGATGTTGAAGAGTGGACTTCCTGGGATGAAGATGCACCCACCAGTGTAAAGATCGAAGGA
GGGAATGGGAATGTGGCAACACAACAAAATTCTTTGGAACAACTGGAACCTGACTATTTTAAGGACATGACACCA
ACTATTAGGAAAACTCAGAAAATTGTTATTAAGAAGAGAGAACCATTGAATTTTGGCATCCCAGATGGGAGCACA
GGTTTCTCTAGTAGATTAGCAGCTACACAAGATCTGCCTTTTATTCATCAGTCTTCTGAATTAGGTGACTTAGAT
ACCTGGCAGGAAAATACCAATGCATGGGAAGAAGAAGAAGATGCAGCCTGGCAAGCAGAAGAAGTTCTGAGACAG
CAGAAACTAGCAGACAGAGAAAAGAGAGCAGCCGAACAACAAAGGAAGAAAATGGAAAAGGAAGCACAACGGCTA
ATGAAGAAGGAACAAAACAAAATTGGTGTGAAACTTTCAGGAGCACCCGGCTCCGCCGGCTCCGCCGCCGGCTCC
GGCATGGCCTCAAACGATTATACCCAACAAGCAACCCAAAGCTATGGGGCCTACCCCACCCAGCCCGGGCAGGGC
TATTCCCAGCAGAGCAGTCAGCCCTACGGACAGCAGAGTTACAGTGGTTATAGCCAGTCCACGGACACTTCAGGA
TATGGCCAGAGCAGCTATTCTTCTTATGGCCAGAGCCAGAACACAGGCTATGGAACTCAGTCAACTCCCCAGGGA
TATGGCTCGACTGGCGGCTATGGCAGTAGCCAGAGCTCCCAATCGTCTTACGGGCAGCAGTCCTCCTACCCTGGC
TATGGCCAGCAGCCAGCTCCCAGCAGCACCTCGGGAAGTTACGGTAGCAGTTCTCAGAGCAGCAGCTATGGGCAG
CCCCAGAGTGGGAGCTACAGCCAGCAGCCTAGCTATGGTGGACAGCAGCAAAGCTATGGACAGCAGCAAAGCTAT
AATCCCCCTCAGGGCTATGGACAGCAGAACCAGTACAACAGCAGCAGTGGTGGTGGAGGTGGAGGTGGAGGTGGA
GGTAACTATGGCCAAGATCAATCCTCCATGAGTAGTGGTGGTGGCAGTGGTGGCGGTTATGGCAATCAAGACCAG
AGTGGTGGAGGTGGCAGCGGTGGCTATGGACAGCAGGACCGTGGAGGCCGCGGCAGGGGTGGCAGTGGTGGCGGC
GGCGGCGGCGGCGGTGGTGGTTACAACCGCAGCAGTGGTGGCTATGAACCCAGAGGTCGTGGAGGTGGCCGTGGA
GGCAGAGGTGGCATGGGCGGAAGTGACCGTGGTGGCTTCAATAAATTTGGTGGCCCTCGGGACCAAGGATCACGT
CATGACTCCGAACAGGATAATTCAGACAACAACACCATCTTTGTGCAAGGCCTGGGTGAGAATGTTACAATTGAG
TCTGTGGCTGATTACTTCAAGCAGATTGGTATTATTAAGACAAACAAGAAAACGGGACAGCCCATGATTAATTTG
TACACAGACAGGGAAACTGGCAAGCTGAAGGGAGAGGCAACGGTCTCTTTTGATGACCCACCTTCAGCTAAAGCA
GCTATTGACTGGTTTGATGGTAAAGAATTCTCCGGAAATCCTATCAAGGTCTCATTTGCTACTCGCCGGGCAGAC
TTTAATCGGGGTGGTGGCAATGGTCGTGGAGGCCGAGGGCGAGGAGGACCCATGGGCCGTGGAGGCTATGGAGGT
GGTGGCAGTGGTGGTGGTGGCCGAGGAGGATTTCCCAGTGGAGGTGGTGGCGGTGGAGGACAGCAGCGAGCTGGT
GACTGGAAGTGTCCTAATCCCACCTGTGAGAATATGAACTTCTCTTGGAGGAATGAATGCAACCAGTGTAAGGCC
CCTAAACCAGATGGCCCAGGAGGGGGACCAGGTGGCTCTCACATGGGGGGTAACTACGGGGATGATCGTCGTGGT
GGCAGAGGTGGTGCGATCGCAGGCGCCGATTACAAGGACGATGATGACAAGGGAGCACCAGGAAGTGCTGGTTCT
GCTGCTGGTAGTGGAGCGTGCCCGGTGCCGCTGCAGCTGCCGCCGCTGGAACGCCTGACCCTGGATGATAAAAAA
CCGCTGAATACCCTGATCTCTGCTACTGGTCTGTGGATGAGTCGTACCGGAACCATTCATAAAATCAAACACCAC
GAGGTTAGCCGTTCGAAAATCTATATTGAGATGGCGTGTGGCGATCATCTGGTTGTGAACAATAGCCGCTCTTCT
CGTACAGCACGTGCACTGCGTCACCACAAATATCGTAAAACCTGTAAACGTTGCCGTGTGTCCGATGAGGATCTG
AACAAATTCCTGACAAAAGCCAATGAGGACCAAACAAGCGTGAAAGTGAAAGTCGTTAGCGCTCCTACCCGTACT
AAAAAAGCAATGCCGAAATCCGTTGCTCGTGCCCCTAAACCACTGGAAAACACTGAAGCAGCACAGGCACAGCCG
TCTGGAAGCAAATTCTCTCCGGCCATTCCTGTTTCTACCCAGGAGTCCGTTTCTGTTCCAGCAAGTGTGAGCACC
AGCATTAGCAGTATTAGCACCGGTGCCACCGCTAGCGCCCTGGTTAAAGGCAATACCAATCCGATTACAAGCATG
TCTGCCCCGGTTCAAGCATCAGCTCCAGCACTGACAAAATCCCAAACCGATCGTCTGGAGGTTCTGCTGAATCCG
AAAGACGAAATCAGCCTGAATTCCGGCAAACCGTTTCGTGAACTGGAGAGCGAACTGCTGTCACGTCGTAAAAAA
GACCTGCAACAAATCTATGCCGAAGAACGTGAGAACTATCTGGGGAAACTGGAACGTGAAATCACCCGCTTTTTC
GTGGATCGTGGCTTTCTGGAGATCAAATCCCCGATTCTGATTCCTCTGGAGTATATCGAGCGTATGGGCATCGAC
AATGATACCGAACTGAGCAAACAAATTTTCCGTGTGGATAAAAACTTCTGTCTGCGCCCTATGCTAGCACCAAAT
CTGGCTAACTATCTGCGCAAACTGGACCGTGCCCTGCCTGATCCTATCAAAATCTTCGAGATCGGCCCGTGTTAT
CGTAAAGAGTCCGACGGTAAAGAACATCTGGAGGAGTTTACCATGCTGGCCTTTGCCCAAATGGGTTCAGGTTGT
ACTCGTGAGAACCTGGAAAGCATCATCACCGATTTTCTGAACCACCTGGGCATTGACTTCAAAATTGTGGGCGAC
AGCTGTATGGTGTTTGGCGACACCCTGGATGTCATGCACGGCGACCTGGAACTGTCTAGTGCCGTTGTGGGCCCA
ATCCCGCTGGATCGTGAGTGGGGTATCGACAAACCTTGGATCGGTGCGGGTTTTGGTCTGGAGCGTCTGCTGAAA
GTAAAACACGACTTCAAGAACATCAAACGTGCTGCACGTTCCGAGTCCTATTACAATGGTATTTCTACTAACCTG
TAA (SEQ ID NO: 347)

Protein:
MAITQFRLFKFCTCLATVFSFLKRLICRSGRGRKLSGDQITLPTTVDYSSVPKQTDVEEWTSWDEDAPTSVKIEG
GNGNVATQQNSLEQLEPDYFKDMTPTIRKTQKIVIKKREPLNFGIPDGSTGFSSRLAATQDLPFIHQSSELGDLD
TWQENTNAWEEEEDAAWQAEEVLRQQKLADREKRAAEQQRKKMEKEAQRLMKKEQNKIGVKLSGAPGSAGSAAGS

GMASNDYTQQATQSYGAYPTQPGQGYSQQSSQPYGQQSYSGYSQSTDTSGYGQSSYSSYGQSQNTGYGTQSTPQG
YGSTGGYGSSQSSQSSYGQQSSYPGYGQQPAPSSTSGSYGSSSQSSSYGQPQSGSYSQQPSYGGQQQSYGQQQSY
NPPQGYGQQNQYNSSSGGGGGGGGGGNYGQDQSSMSSGGGSGGGYGNQDQSGGGGSGGYGQQDRGGRGRGGSGGG
GGGGGGGYNRSSGGYEPRGRGGGRGGRGGMGGSDRGGFNKFGGPRDQGSRHDSEQDNSDNNTIFVQGLGENVTIE
SVADYFKQIGIIKTNKKTGQPMINLYTDRETGKLKGEATVSFDDPPSAKAAIDWFDGKEFSGNPIKVSFATRRAD
FNRGGGNGRGGRGRGGPMGRGGYGGGGSGGGGRGGFPSGGGGGGGQQRAGDWKCPNPTCENMNFSWRNECNQCKA
PKPDGPGGGPGGSHMGGNYGDDRRGGRGGAIAGADYKDDDDKGAPGSAGSAAGSGACPVPLQLPPLERLTLDDKK
PLNTLISATGLWMSRTGTIHKIKHHEVSRSKIYIEMACGDHLVVNNSRSSRTARALRHHKYRKTCKRCRVSDEDL
NKFLTKANEDQTSVKVKVVSAPTRTKKAMPKSVARAPKPLENTEAAQAQPSGSKFSPAIPVSTQESVSVPASVST
SISSISTGATASALVKGNTNPITSMSAPVQASAPALTKSQTDRLEVLLNPKDEISLNSGKPFRELESELLSRRKK
DLQQIYAEERENYLGKLEREITRFFVDRGFLEIKSPILIPLEYIERMGIDNDTELSKQIFRVDKNFCLRPMLAPN
LANYLRKLDRALPDPIKIFEIGPCYRKESDGKEHLEEFTMLAFAQMGSGCTRENLESIITDFLNHLGIDFKIVGD
SCMVFGDTLDVMHGDLELSSAVVGPIPLDREWGIDKPWIGAGFGLERLLKVKHDFKNIKRAARSESYYNGISTNL
* (SEQ ID NO: 348)

EBAG9₁₋₂₉::FUS::PylRS(AA)
DNA:
ATGGCCATCACCCAGTTTCGGTTATTTAAATTTTGTACCTGCCTAGCAACAGTATTCTCATTCCTAAAGAGATTA
ATATGCAGATCTGGAGCACCCGGCTCCGCCGGCTCCGCCGCCGGCTCCGGCATGGCCTCAAACGATTATACCCAA
CAAGCAACCCAAAGCTATGGGGCCTACCCCACCCAGCCCGGGCAGGGCTATTCCCAGCAGAGCAGTCAGCCCTAC
GGACAGCAGAGTTACAGTGGTTATAGCCAGTCCACGGACACTTCAGGATATGGCCAGAGCAGCTATTCTTCTTAT
GGCCAGAGCCAGAACACAGGCTATGGAACTCAGTCAACTCCCCAGGGATATGGCTCGACTGGCGGCTATGGCAGT
AGCCAGAGCTCCCAATCGTCTTACGGGCAGCAGTCCTCCTACCCTGGCTATGGCCAGCAGCCAGCTCCCAGCAGC
ACCTCGGGAAGTTACGGTAGCAGTTCTCAGAGCAGCAGCTATGGGCAGCCCCAGAGTGGGAGCTACAGCCAGCAG
CCTAGCTATGGTGGACAGCAGCAAAGCTATGGACAGCAGCAAAGCTATAATCCCCCTCAGGGCTATGGACAGCAG
AACCAGTACAACAGCAGCAGTGGTGGTGGAGGTGGAGGTGGAGGTGGAGGTAACTATGGCCAAGATCAATCCTCC
ATGAGTAGTGGTGGTGGCAGTGGTGGCGGTTATGGCAATCAAGACCAGAGTGGTGGAGGTGGCAGCGGTGGCTAT
GGACAGCAGGACCGTGGAGGCCGCGGCAGGGGTGGCAGTGGTGGCGGCGGCGGCGGCGGTGGTGGTTACAAC
CGCAGCAGTGGTGGCTATGAACCCAGAGGTCGTGGAGGTGGCCGTGGAGGCAGAGGTGGCATGGGCGGAAGTGAC
CGTGGTGGCTTCAATAAATTTGGTGGCCCTCGGGACCAAGGATCACGTCATGACTCCGAACAGGATAATTCAGAC
AACAACACCATCTTTGTGCAAGGCCTGGGTGAGAATGTTACAATTGAGTCTGTGGCTGATTACTTCAAGCAGATT
GGTATTATTAAGACAAACAAGAAAACGGGACAGCCCATGATTAATTTGTACACAGACAGGGAAACTGGCAAGCTG
AAGGGAGAGGCAACGGTCTCTTTTGATGACCCACCTTCAGCTAAAGCAGCTATTGACTGGTTTGATGGTAAAGAA
TTCTCCGGAAATCCTATCAAGGTCTCATTTGCTACTCGCCGGGCAGACTTTAATCGGGGTGGTGGCAATGGTCGT
GGAGGCCGAGGGCGAGGAGGACCCATGGGCCGTGGAGGCTATGGAGGTGGTGGCAGTGGTGGTGGTGGCCGAGGA
GGATTTCCCAGTGGAGGTGGTGGCGGTGGAGGACAGCAGCGAGCTGGTGACTGGAAGTGTCCTAATCCCACCTGT
GAGAATATGAACTTCTCTTGGAGGAATGAATGCAACCAGTGTAAGGCCCCTAAACCAGATGGCCCAGGAGGGGGA
CCAGGTGGCTCTCACATGGGGGGTAACTACGGGGATGATCGTCGTGGTGGCAGAGGTGGTGCGATCGCAGGCGCC
GATTACAAGGACGATGATGACAAGGGAGCACCAGGAAGTGCTGGTTCTGCTGCTGGTAGTGGAGCGTGCCCGGTG
CCGCTGCAGCTGCCGCCGCTGGAACGCCTGACCCTGGATGACAAAAAACCGCTGAATACCCTGATCTCTGCTACT
GGTCTGTGGATGAGTCGTACCGGAACCATTCATAAAATCAAACACCACGAGGTTAGCCGTTCGAAAATCTATATT
GAGATGGCGTGTGGCGATCATCTGGTTGTGAACAATAGCCGCTCTTCTCGTACAGCACGTGCACTGCGTCACCAC
AAATATCGTAAAACCTGTAAACGTTGCCGTGTGTCCGATGAGGATCTGAACAAATTCCTGACAAAAGCCAATGAG
GACCAAACAAGCGTGAAAGTGAAAGTCGTTAGCGCTCCTACCCGTACTAAAAAAGCAATGCCGAAATCCGTTGCT
CGTGCCCCTAAACCACTGGAAAACACTGAAGCAGCACAGGCACAGCCGTCTGGAAGCAAATTCTCTCCGGCCATT
CCTGTTTCTACCCAGGAGTCCGTTTCTGTTCCAGCAAGTGTGAGCACCAGCATTAGCAGTATTAGCACCGGTGCC
ACCGCTAGCGCCCTGGTTAAAGGCAATACCAATCCGATTACAAGCATGTCTGCCCCGGTTCAAGCATCAGCTCCA
GCACTGACAAAATCCCAAACCGATCGTCTGGAGGTTCTGCTGAATCCGAAAGACGAAATCAGCCTGAATTCCGGC
AAACCGTTTCGTGAACTGGAGAGCGAACTGCTGTCACGTCGTAAAAAAGACCTGCAACAAATCTATGCCGAAGAA
CGTGAGAACTATCTGGGGAAACTGGAACGTGAAATCACCCGCTTTTTCGTGGATCGTGGCTTTCTGGAGATCAAA
TCCCCGATTCTGATTCCTCTGGAGTATATCGAGCGTATGGGCATCGACAATGATACCGAACTGAGCAAACAAATT
TTCCGTGTGGATAAAAACTTCTGTCTGCGCCCTATGCTGGCACCAAATCTGTATAACTATCTGCGCAAACTGGAC
CGTGCCCTGCCTGATCCTATCAAAATCTTCGAGATCGGCCCGTGTTATCGTAAAGAGTCCGACGGTAAAGAACAT
CTGGAGGAGTTTACCATGCTGGCCTTTGCCCAAATGGGTTCAGGTTGTACTCGTGAGAACCTGGAAAGCATCATC
ACCGATTTTCTGAACCACCTGGGCATTGACTTCAAAATTGTGGGCGACAGCTGTATGGTGTATGGCGACACCCTG
GATGTCATGCACGGCGACCTGGAACTGTCTAGTGCCGTTGTTGGACCAATTCCGCTGGACCGTGAGTGGGGTATC

GACAAACCGTGGATCGGAGCAGGATTCGGTCTGGAACGCCTGCTGAAAGTGAAACACGACTTCAAAAACATCAAA
CGTGCCGCCCGTTCTGAATCGTATTATAACGGGATCTCTACGAACCTGTAA (SEQ ID NO: 349)

Protein:
MAITQFRLFKFCTCLATVFSFLKRLICRSGAPGSAGSAAGSGMASNDYTQQATQSYGAYPTQPGQGYSQQSSQPY
GQQSYSGYSQSTDTSGYGQSSYSSYGQSQNTGYGTQSTPQGYGSTGGYGSSQSSQSSYGQQSSYPGYGQQPAPSS
TSGSYGSSSQSSSYGQPQSGSYSQQPSYGGQQQSYGQQQSYNPPQGYGQQNQYNSSSGGGGGGGGGGNYGQDQSS
MSSGGGSGGGYGNQDQSGGGGSGGYGQQDRGGRGRGGSGGGGGGGGGGYNRSSGGYEPRGRGGGRGGRGGMGGSD
RGGFNKFGGPRDQGSRHDSEQDNSDNNTIFVQGLGENVTIESVADYFKQIGIIKTNKKTGQPMINLYTDRETGKL
KGEATVSFDDPPSAKAAIDWFDGKEFSGNPIKVSFATRRADFNRGGGNGRGGRGRGGPMGRGGYGGGGSGGGGRG
GFPSGGGGGGGQQRAGDWKCPNPTCENMNFSWRNECNQCKAPKPDGPGGGPGGSHMGGNYGDDRRGGRGGAIAGA
DYKDDDDKGAPGSAGSAAGSGACPVPLQLPPLERLTLDDKKPLNTLISATGLWMSRTGTIHKIKHHEVSRSKIYI
EMACGDHLVVNNSRSSRTARALRHHKYRKTCKRCRVSDEDLNKFLTKANEDQTSVKVKVVSAPTRTKKAMPKSVA
RAPKPLENTEAAQAQPSGSKFSPAIPVSTQESVSVPASVSTSISSISTGATASALVKGNTNPITSMSAPVQASAP
ALTKSQTDRLEVLLNPKDEISLNSGKPFRELESELLSRRKKDLQQIYAEERENYLGKLEREITRFFVDRGFLEIK
SPILIPLEYIERMGIDNDTELSKQIFRVDKNFCLRPMLAPNLYNYLRKLDRALPDPIKIFEIGPCYRKESDGKEH
LEEFTMLAFAQMGSGCTRENLESIITDFLNHLGIDFKIVGDSCMVYGDTLDVMHGDLELSSAVVGPIPLDREWGI
DKPWIGAGFGLERLLKVKHDFKNIKRAARSESYYNGISTNL* (SEQ ID NO: 350)

EBAG9₁₋₂₉::FUS::PylRS(AAAF)
DNA:
ATGGCCATCACCCAGTTTCGGTTATTTAAATTTTGTACCTGCCTAGCAACAGTATTCTCATTCCTAAAGAGATTA
ATATGCAGATCTGGAGCACCCGGCTCCGCCGGCTCCGCCGCCGGCTCCGGCATGGCCTCAAACGATTATACCCAA
CAAGCAACCCAAAGCTATGGGGCCTACCCCACCCAGCCCGGGCAGGGCTATTCCCAGCAGAGCAGTCAGCCCTAC
GGACAGCAGAGTTACAGTGGTTATAGCCAGTCCACGGACACTTCAGGATATGGCCAGAGCAGCTATTCTTCTTAT
GGCCAGAGCCAGAACACAGGCTATGGAACTCAGTCAACTCCCCAGGGATATGGCTCGACTGGCGGCTATGGCAGT
AGCCAGAGCTCCCAATCGTCTTACGGGCAGCAGTCCTCCTACCCTGGCTATGGCCAGCAGCCAGCTCCCAGCAGC
ACCTCGGGAAGTTACGGTAGCAGTTCTCAGAGCAGCAGCTATGGGCAGCCCCAGAGTGGGAGCTACAGCCAGCAG
CCTAGCTATGGTGGACAGCAGCAAAGCTATGGACAGCAGCAAAGCTATAATCCCCCTCAGGGCTATGGACAGCAG
AACCAGTACAACAGCAGCAGTGGTGGTGGAGGTGGAGGTGGAGGTGGAGGTAACTATGGCCAAGATCAATCCTCC
ATGAGTAGTGGTGGTGGCAGTGGTGGCGGTTATGGCAATCAAGACCAGAGTGGTGGAGGTGGCAGCGGTGGCTAT
GGACAGCAGGACCGTGGAGGCCGCGGCAGGGGTGGCAGTGGTGGCGGCGGCGGCGGCGGCGGTGGTGGTTACAAC
CGCAGCAGTGGTGGCTATGAACCCAGAGGTCGTGGAGGTGGCCGTGGAGGCAGAGGTGGCATGGGCGGAAGTGAC
CGTGGTGGCTTCAATAAATTTGGTGGCCCTCGGGACCAAGGATCACGTCATGACTCCGAACAGGATAATTCAGAC
AACAACACCATCTTTGTGCAAGGCCTGGGTGAGAATGTTACAATTGAGTCTGTGGCTGATTACTTCAAGCAGATT
GGTATTATTAAGACAAACAAGAAAACGGGACAGCCCATGATTAATTTGTACACAGACAGGGAAACTGGCAAGCTG
AAGGGAGAGGCAACGGTCTCTTTTGATGACCCACCTTCAGCTAAAGCAGCTATTGACTGGTTTGATGGTAAAGAA
TTCTCCGGAAATCCTATCAAGGTCTCATTTGCTACTCGCCGGGCAGACTTTAATCGGGGTGGTGGCAATGGTCGT
GGAGGCCGAGGGCGAGGAGGACCCATGGGCCGTGGAGGCTATGGAGGTGGTGGCAGTGGTGGTGGTGGCCGAGGA
GGATTTCCCAGTGGAGGTGGTGGCGGTGGAGGACAGCAGCGAGCTGGTGACTGGAAGTGTCCTAATCCCACCTGT
GAGAATATGAACTTCTCTTGGAGGAATGAATGCAACCAGTGTAAGGCCCCTAAACCAGATGGCCCAGGAGGGGGA
CCAGGTGGCTCTCACATGGGGGGTAACTACGGGGATGATCGTCGTGGTGGCAGAGGTGGTGCGATCGCAGGCGCC
GATTACAAGGACGATGATGACAAGGGAGCACCAGGAAGTGCTGGTTCTGCTGCTGGTAGTGGAGCGTGCCCGGTG
CCGCTGCAGCTGCCGCCGCTGGAACGCCTGACCCTGGATGATAAAAAACCGCTGAATACCCTGATCTCTGCTACT
GGTCTGTGGATGAGTCGTACCGGAACCATTCATAAAATCAAACACCACGAGGTTAGCCGTTCGAAAATCTATATT
GAGATGGCGTGTGGCGATCATCTGGTTGTGAACAATAGCCGCTCTTCTCGTACAGCACGTGCACTGCGTCACCAC
AAATATCGTAAAACCTGTAAACGTTGCCGTGTGTCCGATGAGGATCTGAACAAATTCCTGACAAAAGCCAATGAG
GACCAAACAAGCGTGAAAGTGAAAGTCGTTAGCGCTCCTACCCGTACTAAAAAAGCAATGCCGAAATCCGTTGCT
CGTGCCCCTAAACCACTGGAAAACACTGAAGCAGCACAGGCACAGCCGTCTGGAAGCAAATTCTCTCCGGCCATT
CCTGTTTCTACCCAGGAGTCCGTTTCTGTTCCAGCAAGTGTGAGCACCAGCATTAGCAGTATTAGCACCGGTGCC
ACCGCTAGCGCCCTGGTTAAAGGCAATACCAATCCGATTACAAGCATGTCTGCCCCGGTTCAAGCATCAGCTCCA
GCACTGACAAAATCCCAAACCGATCGTCTGGAGGTTCTGCTGAATCCGAAAGACGAAATCAGCCTGAATTCCGGC
AAACCGTTTCGTGAACTGGAGAGCGAACTGCTGTCACGTCGTAAAAAAGACCTGCAACAAATCTATGCCGAAGAA
CGTGAGAACTATCTGGGGAAACTGGAACGTGAAATCACCCGCTTTTTCGTGGATCGTGGCTTTCTGGAGATCAAA
TCCCCGATTCTGATTCCTCTGGAGTATATCGAGCGTATGGGCATCGACAATGATACCGAACTGAGCAAACAAATT
TTCCGTGTGGATAAAAACTTCTGTCTGCGCCCTATGCTAGCACCAAATCTGGCTAACTATCTGCGCAAACTGGAC
CGTGCCCTGCCTGATCCTATCAAAATCTTCGAGATCGGCCCGTGTTATCGTAAAGAGTCCGACGGTAAAGAACAT

CTGGAGGAGTTTACCATGCTGGCCTTTGCCCAAATGGGTTCAGGTTGTACTCGTGAGAACCTGGAAAGCATCATC
ACCGATTTTCTGAACCACCTGGGCATTGACTTCAAAATTGTGGGCGACAGCTGTATGGTGTTTGGCGACACCCTG
GATGTCATGCACGGCGACCTGGAACTGTCTAGTGCCGTTGTGGGCCCAATCCCGCTGGATCGTGAGTGGGGTATC
GACAAACCTTGGATCGGTGCGGGTTTTGGTCTGGAGCGTCTGCTGAAAGTAAAACACGACTTCAAGAACATCAAA
CGTGCTGCACGTTCCGAGTCCTATTACAATGGTATTTCTACTAACCTGTAA (SEQ ID NO: 351)

Protein:
MAITQFRLFKFCTCLATVFSFLKRLICRSGAPGSAGSAAGSGMASNDYTQQATQSYGAYPTQPGQGYSQQSSQPY
GQQSYSGYSQSTDTSGYGQSSYSSYGQSQNTGYGTQSTPQGYGSTGGYGSSQSSQSSYGQQSSYPGYGQQPAPSS
TSGSYGSSSQSSSYGQPQSGSYSQQPSYGGQQQSYGQQQSYNPPQGYGQQNQYNSSSGGGGGGGGGGNYGQDQSS
MSSGGGSGGGYGNQDQSGGGGSGGYGQQDRGGRGRGGSGGGGGGGGGGYNRSSGGYEPRGRGGGRGGRGGMGGSD
RGGFNKFGGPRDQGSRHDSEQDNSDNNTIFVQGLGENVTIESVADYFKQIGIIKTNKKTGQPMINLYTDRETGKL
KGEATVSFDDPPSAKAAIDWFDGKEFSGNPIKVSFATRRADFNRGGGNGRGGRGRGGPMGRGGYGGGGSGGGGRG
GFPSGGGGGGGGQQRAGDWKCPNPTCENMNFSWRNECNQCKAPKPDGPGGGPGGSHMGGNYGDDRRGGRGGAIAGA
DYKDDDDKGAPGSAGSAAGSGACPVPLQLPPLERLTLDDKKPLNTLISATGLWMSRTGTIHKIKHHEVSRSKIYI
EMACGDHLVVNNSRSSRTARALRHHKYRKTCKRCRVSDEDLNKFLTKANEDQTSVKVKVVSAPTRTKKAMPKSVA
RAPKPLENTEAAQAQPSGSKFSPAIPVSTQESVSVPASVSTSISSISTGATASALVKGNTNPITSMSAPVQASAP
ALTKSQTDRLEVLLNPKDEISLNSGKPFRELESELLSRRKKDLQQIYAEERENYLGKLEREITRFFVDRGFLEIK
SPILIPLEYIERMGIDNDTELSKQIFRVDKNFCLRPMLAPNLANYLRKLDRALPDPIKIFEIGPCYRKESDGKEH
LEEFTMLAFAQMGSGCTRENLESIITDFLNHLGIDFKIVGDSCMVFGDTLDVMHGDLELSSAVVGPIPLDREWGI
DKPWIGAGFGLERLLKVKHDFKNIKRAARSESYYNGISTNL* (SEQ ID NO: 352)

EBAG9₁₋₂₉::FUS::MCP::PylRS(AF)
DNA:
ATGGCCATCACCCAGTTTCGGTTATTTAAATTTTGTACCTGCCTAGCAACAGTATTCTCATTCCTAAAGAGATTA
ATATGCAGATCTGGAGCACCCGGCTCCGCCGGCTCCGCCGCCGGCTCCGGCATGGCCTCAAACGATTATACCCAA
CAAGCAACCCAAAGCTATGGGGCCTACCCCACCCAGCCCGGGCAGGGCTATTCCCAGCAGAGCAGTCAGCCCTAC
GGACAGCAGAGTTACAGTGGTTATAGCCAGTCCACGGACACTTCAGGATATGGCCAGAGCAGCTATTCTTCTTAT
GGCCAGAGCCAGAACACAGGCTATGGAACTCAGTCAACTCCCCAGGGATATGGCTCGACTGGCGGCTATGGCAGT
AGCCAGAGCTCCCAATCGTCTTACGGGCAGCAGTCCTCCTACCCTGGCTATGGCCAGCAGCCAGCTCCCAGCAGC
ACCTCGGGAAGTTACGGTAGCAGTTCTCAGAGCAGCAGCTATGGGCAGCCCCAGAGTGGGAGCTACAGCCAGCAG
CCTAGCTATGGTGGACAGCAGCAAAGCTATGGACAGCAGCAAAGCTATAATCCCCCTCAGGGCTATGGACAGCAG
AACCAGTACAACAGCAGCAGTGGTGGTGGAGGTGGAGGTGGAGGTGGAGGTAACTATGGCCAAGATCAATCCTCC
ATGAGTAGTGGTGGTGGCAGTGGTGGCGGTTATGGCAATCAAGACCAGAGTGGTGGAGGTGGCAGCGGTGGCTAT
GGACAGCAGGACCGTGGAGGCCGCGGCAGGGGTGGCAGTGGTGGCGGCGGCGGCGGCGGCGGTGGTGGTTACAAC
CGCAGCAGTGGTGGCTATGAACCCAGAGGTCGTGGAGGTGGCCGTGGAGGCAGAGGTGGCATGGGCGGAAGTGAC
CGTGGTGGCTTCAATAAATTTGGTGGCCCTCGGGACCAAGGATCACGTCATGACTCCGAACAGGATAATTCAGAC
AACAACACCATCTTTGTGCAAGGCCTGGGTGAGAATGTTACAATTGAGTCTGTGGCTGATTACTTCAAGCAGATT
GGTATTATTAAGACAAACAAGAAAACGGGACAGCCCATGATTAATTTGTACACAGACAGGGAAACTGGCAAGCTG
AAGGGAGAGGCAACGGTCTCTTTTGATGACCCACCTTCAGCTAAAGCAGCTATTGACTGGTTTGATGGTAAAGAA
TTCTCCGGAAATCCTATCAAGGTCTCATTTGCTACTCGCCGGGCAGACTTTAATCGGGGTGGTGGCAATGGTCGT
GGAGGCCGAGGGCGAGGAGGACCCATGGGCCGTGGAGGCTATGGAGGTGGTGGCAGTGGTGGTGGTGGCCGAGGA
GGATTTCCCAGTGGAGGTGGTGGCGGTGGAGGACAGCAGCGAGCTGGTGACTGGAAGTGTCCTAATCCCACCTGT
GAGAATATGAACTTCTCTTGGAGGAATGAATGCAACCAGTGTAAGGCCCCTAAACCAGATGGCCCAGGAGGGGGA
CCAGGTGGCTCTCACATGGGGGGTAACTACGGGGATGATCGTCGTGGTGGCAGAGGTGGTGCGATCGCATATCCC
TATGATGTGCCGGATTATGCTGGAGCACCAGGAAGTGCTGGTTCTGCTGCTGGTAGTGGAGCTTCTAACTTTACT
CAGTTCGTTCTCGTCGACAATGGCGGAACTGGCGACGTGACTGTCGCCCCAAGCAACTTCGCTAACGGGATCGCT
GAATGGATCAGCTCTAACTCGCGTTCACAGGCTTACAAAGTAACCTGTAGCGTTCGTCAGAGCTCTGCGCAGAAT
CGCAAATACACCATCAAAGTCGAGGTGCCTAAAGGCGCCTGGCGTTCGTACTTAAATATGGAACTAACCATTCCA
ATTTTCGCCACGAATTCCGACTGCGAGCTTATTGTTAAGGCAATGCAAGGTCTCCTAAAAGATGGAAACCCGATT
CCCTCAGCAATCGCAGCAAACTCCGGCATCTACGGCGCCGATTACAAGGACGATGATGACAAGGGAGCACCAGGA
AGTGCTGGTTCTGCTGCTGGTAGTGGAGCGTGCCCGGTGCCGCTGCAGCTGCCGCCGCTGGAACGCCTGACCCTG
GATGATAAAAAACCGCTGAATACCCTGATCTCTGCTACTGGTCTGTGGATGAGTCGTACCGGAACCATTCATAAA
ATCAAACACCACGAGGTTAGCCGTTCGAAAATCTATATTGAGATGGCGTGTGGCGATCATCTGGTTGTGAACAAT
AGCCGCTCTTCTCGTACAGCACGTGCACTGCGTCACCACAAATATCGTAAAACCTGTAAACGTTGCCGTGTGTCC
GATGAGGATCTGAACAAATTCCTGACAAAAGCCAATGAGGACCAAACAAGCGTGAAAGTGAAAGTCGTTAGCGCT
CCTACCCGTACTAAAAAAGCAATGCCGAAATCCGTTGCTCGTGCCCCTAAACCACTGGAAAACACTGAAGCAGCA

CAGGCACAGCCGTCTGGAAGCAAATTCTCTCCGGCCATTCCTGTTTCTACCCAGGAGTCCGTTTCTGTTCCAGCA
AGTGTGAGCACCAGCATTAGCAGTATTAGCACCGGTGCCACCGCTAGCGCCCTGGTTAAAGGCAATACCAATCCG
ATTACAAGCATGTCTGCCCCGGTTCAAGCATCAGCTCCAGCACTGACAAAATCCCAAACCGATCGTCTGGAGGTT
CTGCTGAATCCGAAAGACGAAATCAGCCTGAATTCCGGCAAACCGTTTCGTGAACTGGAGAGCGAACTGCTGTCA
CGTCGTAAAAAAGACCTGCAACAAATCTATGCCGAAGAACGTGAGAACTATCTGGGGAAACTGGAACGTGAAATC
ACCCGCTTTTTCGTGGATCGTGGCTTTCTGGAGATCAAATCCCCGATTCTGATTCCTCTGGAGTATATCGAGCGT
ATGGGCATCGACAATGATACCGAACTGAGCAAACAAATTTTCCGTGTGGATAAAAACTTCTGTCTGCGCCCTATG
CTAGCACCAAATCTGGCTAACTATCTGCGCAAACTGGACCGTGCCCTGCCTGATCCTATCAAAATCTTCGAGATC
GGCCCGTGTTATCGTAAAGAGTCCGACGGTAAAGAACATCTGGAGGAGTTTACCATGCTGAACTTTTGCCAAATG
GGTTCAGGTTGTACTCGTGAGAACCTGGAAAGCATCATCACCGATTTTCTGAACCACCTGGGCATTGACTTCAAA
ATTGTGGGCGACAGCTGTATGGTGTTTGGCGACACCCTGGATGTCATGCACGGCGACCTGGAACTGTCTAGTGCC
GTTGTGGGCCCAATCCCGCTGGATCGTGAGTGGGGTATCGACAAACCTTGGATCGGTGCGGGTTTTGGTCTGGAG
CGTCTGCTGAAAGTAAAACACGACTTCAAGAACATCAAACGTGCTGCACGTTCCGAGTCCTATTACAATGGTATT
TCTACTAACCTGTAA (SEQ ID NO: 353)

Protein:
MAITQFRLFKFCTCLATVFSFLKRLICRSGAPGSAGSAAGSGMASNDYTQQATQSYGAYPTQPGQGYSQQSSQPY
GQQSYSGYSQSTDTSGYGQSSYSSYGQSQNTGYGTQSTPQGYGSTGGYGSSQSSQSSYGQQSSYPGYGQQPAPSS
TSGSYGSSSQSSSYGQPQSGSYSQQPSYGGQQQSYGQQQSYNPPQGYGQQNQYNSSSGGGGGGGGGGNYGQDQSS
MSSGGGSGGGYGNQDQSGGGGSGGYGQQDRGGRGRGGSGGGGGGGGGGYNRSSGGYEPRGRGGGRGGRGGMGGSD
RGGFNKFGGPRDQGSRHDSEQDNSDNNTIFVQGLGENVTIESVADYFKQIGIIKTNKKTGQPMINLYTDRETGKL
KGEATVSFDDPPSAKAAIDWFDGKEFSGNPIKVSFATRRADFNRGGGNGRGGRGRGGPMGRGGYGGGGSGGGGRG
GFPSGGGGGGGGQQRAGDWKCPNPTCENMNFSWRNECNQCKAPKPDGPGGGPGGSHMGGNYGDDRRGGRGGAIAYP
YDVPDYAGAPGSAGSAAGSGASNFTQFVLVDNGGTGDVTVAPSNFANGIAEWISSNSRSQAYKVTCSVRQSSAQN
RKYTIKVEVPKGAWRSYLNMELTIPIFATNSDCELIVKAMQGLLKDGNPIPSAIAANSGIYGADYKDDDDKGAPG
SAGSAAGSGACPVPLQLPPLERLTLDDKKPLNTLISATGLWMSRTGTIHKIKHHEVSRSKIYIEMACGDHLVVNN
SRSSRTARALRHHKYRKTCKRCRVSDEDLNKFLTKANEDQTSVKVKVVSAPTRTKKAMPKSVARAPKPLENTEAA
QAQPSGSKFSPAIPVSTQESVSVPASVSTSISSISTGATASALVKGNTNPITSMSAPVQASAPALTKSQTDRLEV
LLNPKDEISLNSGKPFRELESELLSRRKKDLQQIYAEERENYLGKLEREITRFFVDRGFLEIKSPILIPLEYIER
MGIDNDTELSKQIFRVDKNFCLRPMLAPNLANYLRKLDRALPDPIKIFEIGPCYRKESDGKEHLEEFTMLNFCQM
GSGCTRENLESIITDFLNHLGIDFKIVGDSCMVFGDTLDVMHGDLELSSAVVGPIPLDREWGIDKPWIGAGFGLE
RLLKVKHDFKNIKRAARSESYYNGISTNL* (SEQ ID NO: 354)

CG1::PylRS(AF)
DNA:
ATGGCCATTTGTCAATTCTTCCTTCAAGGCCGGTGCCGCTTTGGAGATCGGTGCTGGAACGAACATCCCGGTGCT
AGGGGTGCAGGAGGAGGACGGCAGCAACCGCAGCAGCAGCCTTCAGGTAATAATAGACGTGGATGGAATACAACT
AGCCAGAGATATTCCAATGTCATCCAGCCATCCAGTTTCTCCAAATCCACACCATGGGGGGGCAGCAGAGATCAA
GAAAAGCCATATTTCAGTTCTTTTGATTCTGGAGCTTCAACTAACAGGAAGGAAGGCTTTGGATTGTCTGAGAAC
CCATTTGCTTCACTTAGTCCTGATGAGCAGAAAGATGAAAAGAAACTTCTGGAAGGAATTGTAAAAGATATGGAG
GTTTGGGAATCATCAGGGCAGTGGATGTTTTCTGTTTATTCACCAGTGAAAAAGAAACCTAATATTTCAGGTTTT
ACAGACATTTCACCAGAGGAATTGAGGCTTGAATACCATAACTTCTTAACCAGCAATAACTTACAGAGTTATCTA
AATTCTGTCCAACGTTTAATAAATCAATGGAGGAACAGGGTAAATGAACTGAAAAGTCTAAATATATCAACTAAA
GTAGCTTTGCTCTCTGATGTAAAGGATGGAGTAAATCAAGCAGCACCTGCATTTGGATTTGGCAGCAGTCAAGCA
GCAACATTTATGTCGCCAGGCTTTCCAGTCAATAACAGCAGCAGTGATAATGCTCAGAACTTTAGTTTTAAAACA
AACTCTGGATTTGCTGCTGCCTCTTCTGGAAGCCCTGCTGGTTTTGGGAGTTCCCCAGCATTTGGAGCTGCAGCC
TCTACCAGTTCAGGTATCTCTACTTCTGCTCCAGCTTTTGGATTTGGGAAGCCTGAAGTCACATCGGCTGCATCA
TTTTCATTCAAAAGCCCTGCAGCTTCCAGTTTTGGATCACCTGGATTTTCAGGACTTCCAGCTTCCTTGGCAACA
GGTCCTGTCAGAGCTCCAGTGGCCCCAGCCTTTGGAGGTGGCAGTTCTGTGGCTGGTTTTGGTAGTCCGGGCTCA
CATTCTCACACTGCTTTTTCTAAGCCATCCAGTGACACTTTTGGAAATAGCAGCATATCCACTTCTCTGTCAGCC
TCAAGCAGCATCATTGCAACAGATAATGTGTTATTCACACCCAGAGATAAACTAACAGTAGAAGAACTGGAACAA
TTTCAATCCAAGAAATTTACTCTGGGAAAAATTCCATTAAAGCCTCCACCTCTGGAACTTCTAAATGTTGGCGCC
GATTACAAGGACGATGATGACAAGGGAGCACCAGGAAGTGCTGGTTCTGCTGCTGGTAGTGGAGCGTGCCCGGTG
CCGCTGCAGCTGCCGCCGCTGGAACGCCTGACCCTGGATGATAAAAAACCGCTGAATACCCTGATCTCTGCTACT
GGTCTGTGGATGAGTCGTACCGGAACCATTCATAAAATCAAACACCACGAGGTTAGCCGTTCGAAAATCTATATT
GAGATGGCGTGTGGCGATCATCTGGTTGTGAACAATAGCCGCTCTTCTCGTACAGCACGTGCACTGCGTCACCAC
AAATATCGTAAAACCTGTAAACGTTGCCGTGTGTCCGATGAGGATCTGAACAAATTCCTGACAAAAGCCAATGAG

GACCAAACAAGCGTGAAAGTGAAAGTCGTTAGCGCTCCTACCCGTACTAAAAAAGCAATGCCGAAATCCGTTGCT
CGTGCCCCTAAACCACTGGAAAACACTGAAGCAGCACAGGCACAGCCGTCTGGAAGCAAATTCTCTCCGGCCATT
CCTGTTTCTACCCAGGAGTCCGTTTCTGTTCCAGCAAGTGTGAGCACCAGCATTAGCAGTATTAGCACCGGTGCC
ACCGCTAGCGCCCTGGTTAAAGGCAATACCAATCCGATTACAAGCATGTCTGCCCCGGTTCAAGCATCAGCTCCA
GCACTGACAAAATCCCAAACCGATCGTCTGGAGGTTCTGCTGAATCCGAAAGACGAAATCAGCCTGAATTCCGGC
AAACCGTTTCGTGAACTGGAGAGCGAACTGCTGTCACGTCGTAAAAAAGACCTGCAACAAATCTATGCCGAAGAA
CGTGAGAACTATCTGGGGAAACTGGAACGTGAAATCACCCGCTTTTTCGTGGATCGTGGCTTTCTGGAGATCAAA
TCCCCGATTCTGATTCCTCTGGAGTATATCGAGCGTATGGGCATCGACAATGATACCGAACTGAGCAAACAAATT
TTCCGTGTGGATAAAAACTTCTGTCTGCGCCCTATGCTAGCACCAAATCTGGCTAACTATCTGCGCAAACTGGAC
CGTGCCCTGCCTGATCCTATCAAAATCTTCGAGATCGGCCCGTGTTATCGTAAAGAGTCCGACGGTAAAGAACAT
CTGGAGGAGTTTACCATGCTGAACTTTTGCCAAATGGGTTCAGGTTGTACTCGTGAGAACCTGGAAAGCATCATC
ACCGATTTTCTGAACCACCTGGGCATTGACTTCAAAATTGTGGGCGACAGCTGTATGGTGTTTGGCGACACCCTG
GATGTCATGCACGGCGACCTGGAACTGTCTAGTGCCGTTGTGGGCCCAATCCCGCTGGATCGTGAGTGGGGTATC
GACAAACCTTGGATCGGTGCGGGTTTTGGTCTGGAGCGTCTGCTGAAAGTAAAACACGACTTCAAGAACATCAAA
CGTGCTGCACGTTCCGAGTCCTATTACAATGGTATTTCTACTAACCTGTAA (SEQ ID NO: 355)

Protein:
MAICQFFLQGRCRFGDRCWNEHPGARGAGGGRQQPQQQPSGNNRRGWNTTSQRYSNVIQPSSFSKSTPWGGSRDQ
EKPYFSSFDSGASTNRKEGFGLSENPFASLSPDEQKDEKKLLEGIVKDMEVWESSGQWMFSVYSPVKKKPNISGF
TDISPEELRLEYHNFLTSNNLQSYLNSVQRLINQWRNRVNELKSLNISTKVALLSDVKDGVNQAAPAFGFGSSQA
ATFMSPGFPVNNSSSDNAQNFSFKTNSGFAAASSGSPAGFGSSPAFGAAASTSSGISTSAPAFGFGKPEVTSAAS
FSFKSPAASSFGSPGFSGLPASLATGPVRAPVAPAFGGGSSVAGFGSPGSHSHTAFSKPSSDTFGNSSISTSLSA
SSSIIATDNVLFTPRDKLTVEELEQFQSKKFTLGKIPLKPPPLELLNVGADYKDDDDKGAPGSAGSAAGSGACPV
PLQLPPLERLTLDDKKPLNTLISATGLWMSRTGTIHKIKHHEVSRSKIYIEMACGDHLVVNNSRSSRTARALRHH
KYRKTCKRCRVSDEDLNKFLTKANEDQTSVKVKVVSAPTRTKKAMPKSVARAPKPLENTEAAQAQPSGSKFSPAI
PVSTQESVSVPASVSTSISSISTGATASALVKGNTNPITSMSAPVQASAPALTKSQTDRLEVLLNPKDEISLNSG
KPFRELESELLSRRKKDLQQIYAEERENYLGKLEREITRFFVDRGFLEIKSPILIPLEYIERMGIDNDTELSKQI
FRVDKNFCLRPMLAPNLANYLRKLDRALPDPIKIFEIGPCYRKESDGKEHLEEFTMLNFCQMGSGCTRENLESII
TDFLNHLGIDFKIVGDSCMVFGDTLDVMHGDLELSSAVVGPIPLDREWGIDKPWIGAGFGLERLLKVKHDFKNIK
RAARSESYYNGISTNL* (SEQ ID NO: 356)

CG1::PylRS(AA)
DNA:
ATGGCCATTTGTCAATTCTTCCTTCAAGGCCGGTGCCGCTTTGGAGATCGGTGCTGGAACGAACATCCCGGTGCT
AGGGGTGCAGGAGGAGGACGGCAGCAACCGCAGCAGCAGCCTTCAGGTAATAATAGACGTGGATGGAATACAACT
AGCCAGAGATATTCCAATGTCATCCAGCCATCCAGTTTCTCCAAATCCACACCATGGGGGGGCAGCAGAGATCAA
GAAAAGCCATATTTCAGTTCTTTTGATTCTGGAGCTTCAACTAACAGGAAGGAAGGCTTTGGATTGTCTGAGAAC
CCATTTGCTTCACTTAGTCCTGATGAGCAGAAAGATGAAAAGAAACTTCTGGAAGGAATTGTAAAAGATATGGAG
GTTTGGGAATCATCAGGGCAGTGGATGTTTTCTGTTTATTCACCAGTGAAAAAGAAACCTAATATTTCAGGTTTT
ACAGACATTTCACCAGAGGAATTGAGGCTTGAATACCATAACTTCTTAACCAGCAATAACTTACAGAGTTATCTA
AATTCTGTCCAACGTTTAATAAATCAATGGAGGAACAGGGTAAATGAACTGAAAAGTCTAAATATATCAACTAAA
GTAGCTTTGCTCTCTGATGTAAAGGATGGAGTAAATCAAGCAGCACCTGCATTTGGATTTGGCAGCAGTCAAGCA
GCAACATTTATGTCGCCAGGCTTTCCAGTCAATAACAGCAGCAGTGATAATGCTCAGAACTTTAGTTTTAAAACA
AACTCTGGATTTGCTGCTGCCTCTTCTGGAAGCCCTGCTGGTTTTGGGAGTTCCCCAGCATTTGGAGCTGCAGCC
TCTACCAGTTCAGGTATCTCTACTTCTGCTCCAGCTTTTGGATTTGGGAAGCCTGAAGTCACATCGGCTGCATCA
TTTTCATTCAAAAGCCCTGCAGCTTCCAGTTTTGGATCACCTGGATTTTCAGGACTTCCAGCTTCCTTGGCAACA
GGTCCTGTCAGAGCTCCAGTGGCCCCAGCCTTTGGAGGTGGCAGTTCTGTGGCTGGTTTTGGTAGTCCGGGCTCA
CATTCTCACACTGCTTTTTCTAAGCCATCCAGTGACACTTTTGGAAATAGCAGCATATCCACTTCTCTGTCAGCC
TCAAGCAGCATCATTGCAACAGATAATGTGTTATTCACACCCAGAGATAAACTAACAGTAGAAGAACTGGAACAA
TTTCAATCCAAGAAATTTACTCTGGGAAAAATTCCATTAAAGCCTCCACCTCTGGAACTTCTAAATGTTGGCGCC
GATTACAAGGACGATGATGACAAGGGAGCACCAGGAAGTGCTGGTTCTGCTGCTGGTAGTGGAGCGTGCCCGGTG
CCGCTGCAGCTGCCGCCGCTGGAACGCCTGACCCTGGATGACAAAAAACCGCTGAATACCCTGATCTCTGCTACT
GGTCTGTGGATGAGTCGTACCGGAACCATTCATAAAATCAAACACCACGAGGTTAGCCGTTCGAAAATCTATATT
GAGATGGCGTGTGGCGATCATCTGGTTGTGAACAATAGCCGCTCTTCTCGTACAGCACGTGCACTGCGTCACCAC
AAATATCGTAAAACCTGTAAACGTTGCCGTGTGTCCGATGAGGATCTGAACAAATTCCTGACAAAAGCCAATGAG
GACCAAACAAGCGTGAAAGTGAAAGTCGTTAGCGCTCCTACCCGTACTAAAAAAGCAATGCCGAAATCCGTTGCT
CGTGCCCCTAAACCACTGGAAAACACTGAAGCAGCACAGGCACAGCCGTCTGGAAGCAAATTCTCTCCGGCCATT

CCTGTTTCTACCCAGGAGTCCGTTTCTGTTCCAGCAAGTGTGAGCACCAGCATTAGCAGTATTAGCACCGGTGCC
ACCGCTAGCGCCCTGGTTAAAGGCAATACCAATCCGATTACAAGCATGTCTGCCCCGGTTCAAGCATCAGCTCCA
GCACTGACAAAATCCCAAACCGATCGTCTGGAGGTTCTGCTGAATCCGAAAGACGAAATCAGCCTGAATTCCGGC
AAACCGTTTCGTGAACTGGAGAGCGAACTGCTGTCACGTCGTAAAAAAGACCTGCAACAAATCTATGCCGAAGAA
CGTGAGAACTATCTGGGGAAACTGGAACGTGAAATCACCCGCTTTTTCGTGGATCGTGGCTTTCTGGAGATCAAA
TCCCCGATTCTGATTCCTCTGGAGTATATCGAGCGTATGGGCATCGACAATGATACCGAACTGAGCAAACAAATT
TTCCGTGTGGATAAAAACTTCTGTCTGCGCCCTATGCTGGCACCAAATCTGTATAACTATCTGCGCAAACTGGAC
CGTGCCCTGCCTGATCCTATCAAAATCTTCGAGATCGGCCCGTGTTATCGTAAAGAGTCCGACGGTAAAGAACAT
CTGGAGGAGTTTACCATGCTGGCCTTTGCCCAAATGGGTTCAGGTTGTACTCGTGAGAACCTGGAAAGCATCATC
ACCGATTTTCTGAACCACCTGGGCATTGACTTCAAAATTGTGGGCGACAGCTGTATGGTGTATGGCGACACCCTG
GATGTCATGCACGGCGACCTGGAACTGTCTAGTGCCGTTGTTGGACCAATTCCGCTGGACCGTGAGTGGGGTATC
GACAAACCGTGGATCGGAGCAGGATTCGGTCTGGAACGCCTGCTGAAAGTGAAACACGACTTCAAAAACATCAAA
CGTGCCGCCCGTTCTGAATCGTATTATAACGGGATCTCTACGAACCTGTAA (SEQ ID NO: 357)

Protein:
MAICQFFLQGRCRFGDRCWNEHPGARGAGGGRQQPQQQPSGNNRRGWNTTSQRYSNVIQPSSFSKSTPWGGSRDQ
EKPYFSSFDSGASTNRKEGFGLSENPFASLSPDEQKDEKKLLEGIVKDMEVWESSGQWMFSVYSPVKKKPNISGF
TDISPEELRLEYHNFLTSNNLQSYLNSVQRLINQWRNRVNELKSLNISTKVALLSDVKDGVNQAAPAFGFGSSQA
ATFMSPGFPVNNSSSDNAQNFSFKTNSGFAAASSGSPAGFGSSPAFGAAASTSSGISTSAPAFGFGKPEVTSAAS
FSFKSPAASSFGSPGFSGLPASLATGPVRAPVAPAFGGGSSVAGFGSPGSHSHTAFSKPSSDTFGNSSISTSLSA
SSSIIATDNVLFTPRDKLTVEELEQFQSKKFTLGKIPLKPPPLELLNVGADYKDDDDKGAPGSAGSAAGSGACPV
PLQLPPLERLTLDDKKPLNTLISATGLWMSRTGTIHKIKHHEVSRSKIYIEMACGDHLVVNNSRSSRTARALRHH
KYRKTCKRCRVSDEDLNKFLTKANEDQTSVKVKVVSAPTRTKKAMPKSVARAPKPLENTEAAQAQPSGSKFSPAI
PVSTQESVSVPASVSTSISSISTGATASALVKGNTNPITSMSAPVQASAPALTKSQTDRLEVLLNPKDEISLNSG
KPFRELESELLSRRKKDLQQIYAEERENYLGKLEREITRFFVDRGFLEIKSPILIPLEYIERMGIDNDTELSKQI
FRVDKNFCLRPMLAPNLYNYLRKLDRALPDPIKIFEIGPCYRKESDGKEHLEEFTMLAFAQMGSGCTRENLESII
TDFLNHLGIDFKIVGDSCMVYGDTLDVMHGDLELSSAVVGPIPLDREWGIDKPWIGAGFGLERLLKVKHDFKNIK
RAARSESYYNGISTNL* (SEQ ID NO: 358)

CG1::PylRS(AAAF)
DNA:
ATGGCCATTTGTCAATTCTTCCTTCAAGGCCGGTGCCGCTTTGGAGATCGGTGCTGGAACGAACATCCCGGTGCT
AGGGGTGCAGGAGGAGGACGGCAGCAACCGCAGCAGCAGCCTTCAGGTAATAATAGACGTGGATGGAATACAACT
AGCCAGAGATATTCCAATGTCATCCAGCCATCCAGTTTCTCCAAATCCACACCATGGGGGGGCAGCAGAGATCAA
GAAAAGCCATATTTCAGTTCTTTTGATTCTGGAGCTTCAACTAACAGGAAGGAAGGCTTTGGATTGTCTGAGAAC
CCATTTGCTTCACTTAGTCCTGATGAGCAGAAAGATGAAAAGAAACTTCTGGAAGGAATTGTAAAAGATATGGAG
GTTTGGGAATCATCAGGGCAGTGGATGTTTTCTGTTTATTCACCAGTGAAAAAGAAACCTAATATTTCAGGTTTT
ACAGACATTTCACCAGAGGAATTGAGGCTTGAATACCATAACTTCTTAACCAGCAATAACTTACAGAGTTATCTA
AATTCTGTCCAACGTTTAATAAATCAATGGAGGAACAGGGTAAATGAACTGAAAAGTCTAAATATATCAACTAAA
GTAGCTTTGCTCTCTGATGTAAAGGATGGAGTAAATCAAGCAGCACCTGCATTTGGATTTGGCAGCAGTCAAGCA
GCAACATTTATGTCGCCAGGCTTTCCAGTCAATAACAGCAGCAGTGATAATGCTCAGAACTTTAGTTTTAAAACA
AACTCTGGATTTGCTGCTGCCTCTTCTGGAAGCCCTGCTGGTTTTGGGAGTTCCCCAGCATTTGGAGCTGCAGCC
TCTACCAGTTCAGGTATCTCTACTTCTGCTCCAGCTTTTGGATTTGGGAAGCCTGAAGTCACATCGGCTGCATCA
TTTTCATTCAAAAGCCCTGCAGCTTCCAGTTTTGGATCACCTGGATTTTCAGGACTTCCAGCTTCCTTGGCAACA
GGTCCTGTCAGAGCTCCAGTGGCCCCAGCCTTTGGAGGTGGCAGTTCTGTGGCTGGTTTTGGTAGTCCGGGCTCA
CATTCTCACACTGCTTTTTCTAAGCCATCCAGTGACACTTTTGGAAATAGCAGCATATCCACTTCTCTGTCAGCC
TCAAGCAGCATCATTGCAACAGATAATGTGTTATTCACACCCAGAGATAAACTAACAGTAGAAGAACTGGAACAA
TTTCAATCCAAGAAATTTACTCTGGGAAAAATTCCATTAAAGCCTCCACCTCTGGAACTTCTAAATGTTGGCGCC
GATTACAAGGACGATGATGACAAGGGAGCACCAGGAAGTGCTGGTTCTGCTGCTGGTAGTGGAGCGTGCCCGGTG
CCGCTGCAGCTGCCGCCGCTGGAACGCCTGACCCTGGATGATAAAAAACCGCTGAATACCCTGATCTCTGCTACT
GGTCTGTGGATGAGTCGTACCGGAACCATTCATAAAATCAAACACCACGAGGTTAGCCGTTCGAAAATCTATATT
GAGATGGCGTGTGGCGATCATCTGGTTGTGAACAATAGCCGCTCTTCTCGTACAGCACGTGCACTGCGTCACCAC
AAATATCGTAAAACCTGTAAACGTTGCCGTGTGTCCGATGAGGATCTGAACAAATTCCTGACAAAAGCCAATGAG
GACCAAACAAGCGTGAAAGTGAAAGTCGTTAGCGCTCCTACCCGTACTAAAAAAGCAATGCCGAAATCCGTTGCT
CGTGCCCCTAAACCACTGGAAAACACTGAAGCAGCACAGGCACAGCCGTCTGGAAGCAAATTCTCTCCGGCCATT
CCTGTTTCTACCCAGGAGTCCGTTTCTGTTCCAGCAAGTGTGAGCACCAGCATTAGCAGTATTAGCACCGGTGCC
ACCGCTAGCGCCCTGGTTAAAGGCAATACCAATCCGATTACAAGCATGTCTGCCCCGGTTCAAGCATCAGCTCCA

GCACTGACAAAATCCCAAACCGATCGTCTGGAGGTTCTGCTGAATCCGAAAGACGAAATCAGCCTGAATTCCGGC
AAACCGTTTCGTGAACTGGAGAGCGAACTGCTGTCACGTCGTAAAAAAGACCTGCAACAAATCTATGCCGAAGAA
CGTGAGAACTATCTGGGGAAACTGGAACGTGAAATCACCCGCTTTTTCGTGGATCGTGGCTTTCTGGAGATCAAA
TCCCCGATTCTGATTCCTCTGGAGTATATCGAGCGTATGGGCATCGACAATGATACCGAACTGAGCAAACAAATT
TTCCGTGTGGATAAAAACTTCTGTCTGCGCCCTATGCTAGCACCAAATCTGGCTAACTATCTGCGCAAACTGGAC
CGTGCCCTGCCTGATCCTATCAAAATCTTCGAGATCGGCCCGTGTTATCGTAAAGAGTCCGACGGTAAAGAACAT
CTGGAGGAGTTTACCATGCTGGCCTTTGCCCAAATGGGTTCAGGTTGTACTCGTGAGAACCTGGAAAGCATCATC
ACCGATTTTCTGAACCACCTGGGCATTGACTTCAAAATTGTGGGCGACAGCTGTATGGTGTTTGGCGACACCCTG
GATGTCATGCACGGCGACCTGGAACTGTCTAGTGCCGTTGTGGGCCCAATCCCGCTGGATCGTGAGTGGGGTATC
GACAAACCTTGGATCGGTGCGGGTTTTGGTCTGGAGCGTCTGCTGAAAGTAAAACACGACTTCAAGAACATCAAA
CGTGCTGCACGTTCCGAGTCCTATTACAATGGTATTTCTACTAACCTGTAA (SEQ ID NO: 359)

Protein:
MAICQFFLQGRCRFGDRCWNEHPGARGAGGGRQQPQQQPSGNNRRGWNTTSQRYSNVIQPSSFSKSTPWGGSRDQ
EKPYFSSFDSGASTNRKEGFGLSENPFASLSPDEQKDEKKLLEGIVKDMEVWESSGQWMFSVYSPVKKKPNISGF
TDISPEELRLEYHNFLTSNNLQSYLNSVQRLINQWRNRVNELKSLNISTKVALLSDVKDGVNQAAPAFGFGSSQA
ATFMSPGFPVNNSSSDNAQNFSFKTNSGFAAASSGSPAGFGSSPAFGAAASTSSGISTSAPAFGFGKPEVTSAAS
FSFKSPAASSFGSPGFSGLPASLATGPVRAPVAPAFGGGSSVAGFGSPGSHSHTAFSKPSSDTFGNSSISTSLSA
SSSIIATDNVLFTPRDKLTVEELEQFQSKKFTLGKIPLKPPPLELLNVGADYKDDDDKGAPGSAGSAAGSGACPV
PLQLPPLERLTLDDKKPLNTLISATGLWMSRTGTIHKIKHHEVSRSKIYIEMACGDHLVVNNSRSSRTARALRHH
KYRKTCKRCRVSDEDLNKFLTKANEDQTSVKVKVVSAPTRTKKAMPKSVARAPKPLENTEAAQAQPSGSKFSPAI
PVSTQESVSVPASVSTSISSISTGATASALVKGNTNPITSMSAPVQASAPALTKSQTDRLEVLLNPKDEISLNSG
KPFRELESELLSRRKKDLQQIYAEERENYLGKLEREITRFFVDRGFLEIKSPILIPLEYIERMGIDNDTELSKQI
FRVDKNFCLRPMLAPNLANYLRKLDRALPDPIKIFEIGPCYRKESDGKEHLEEFTMLAFAQMGSGCTRENLESII
TDFLNHLGIDFKIVGDSCMVFGDTLDVMHGDLELSSAVVGPIPLDREWGIDKPWIGAGFGLERLLKVKHDFKNIK
RAARSESYYNGISTNL* (SEQ ID NO: 360)

CG1::FUS::PylRS(AA)
DNA:
ATGGCCATTTGTCAATTCTTCCTTCAAGGCCGGTGCCGCTTTGGAGATCGGTGCTGGAACGAACATCCCGGTGCT
AGGGGTGCAGGAGGAGGACGGCAGCAACCGCAGCAGCAGCCTTCAGGTAATAATAGACGTGGATGGAATACAACT
AGCCAGAGATATTCCAATGTCATCCAGCCATCCAGTTTCTCCAAATCCACACCATGGGGGGGCAGCAGAGATCAA
GAAAAGCCATATTTCAGTTCTTTTGATTCTGGAGCTTCAACTAACAGGAAGGAAGGCTTTGGATTGTCTGAGAAC
CCATTTGCTTCACTTAGTCCTGATGAGCAGAAAGATGAAAAGAAACTTCTGGAAGGAATTGTAAAAGATATGGAG
GTTTGGGAATCATCAGGGCAGTGGATGTTTTCTGTTTATTCACCAGTGAAAAAGAAACCTAATATTTCAGGTTTT
ACAGACATTTCACCAGAGGAATTGAGGCTTGAATACCATAACTTCTTAACCAGCAATAACTTACAGAGTTATCTA
AATTCTGTCCAACGTTTAATAAATCAATGGAGGAACAGGGTAAATGAACTGAAAAGTCTAAATATATCAACTAAA
GTAGCTTTGCTCTCTGATGTAAAGGATGGAGTAAATCAAGCAGCACCTGCATTTGGATTTGGCAGCAGTCAAGCA
GCAACATTTATGTCGCCAGGCTTTCCAGTCAATAACAGCAGCAGTGATAATGCTCAGAACTTTAGTTTTAAAACA
AACTCTGGATTTGCTGCTGCCTCTTCTGGAAGCCCTGCTGGTTTTGGGAGTTCCCCAGCATTTGGAGCTGCAGCC
TCTACCAGTTCAGGTATCTCTACTTCTGCTCCAGCTTTTGGATTTGGGAAGCCTGAAGTCACATCGGCTGCATCA
TTTTCATTCAAAAGCCCTGCAGCTTCCAGTTTTGGATCACCTGGATTTTCAGGACTTCCAGCTTCCTTGGCAACA
GGTCCTGTCAGAGCTCCAGTGGCCCCAGCCTTTGGAGGTGGCAGTTCTGTGGCTGGTTTTGGTAGTCCGGGCTCA
CATTCTCACACTGCTTTTTCTAAGCCATCCAGTGACACTTTTGGAAATAGCAGCATATCCACTTCTCTGTCAGCC
TCAAGCAGCATCATTGCAACAGATAATGTGTTATTCACACCCAGAGATAAACTAACAGTAGAAGAACTGGAACAA
TTTCAATCCAAGAAATTTACTCTGGGAAAAATTCCATTAAAGCCTCCACCTCTGGAACTTCTAAATGTTGGAGCA
CCCGGCTCCGCCGGCTCCGCCGCCGGCTCCGGCATGGCCTCAAACGATTATACCCAACAAGCAACCCAAAGCTAT
GGGGCCTACCCCACCCAGCCCGGGCAGGGCTATTCCCAGCAGAGCAGTCAGCCCTACGGACAGCAGAGTTACAGT
GGTTATAGCCAGTCCACGGACACTTCAGGATATGGCCAGAGCAGCTATTCTTCTTATGGCCAGAGCCAGAACACA
GGCTATGGAACTCAGTCAACTCCCCAGGGATATGGCTCGACTGGCGGCTATGGCAGTAGCCAGAGCTCCCAATCG
TCTTACGGGCAGCAGTCCTCCTACCCTGGCTATGGCCAGCAGCCAGCTCCCAGCAGCACCTCGGGAAGTTACGGT
AGCAGTTCTCAGAGCAGCAGCTATGGGCAGCCCCAGAGTGGGAGCTACAGCCAGCAGCCTAGCTATGGTGGACAG
CAGCAAAGCTATGGACAGCAGCAAAGCTATAATCCCCCTCAGGGCTATGGACAGCAGAACCAGTACAACAGCAGC
AGTGGTGGTGGAGGTGGAGGTGGAGGTGGAGGTAACTATGGCCAAGATCAATCCTCCATGAGTAGTGGTGGTGGC
AGTGGTGGCGGTTATGGCAATCAAGACCAGAGTGGTGGAGGTGGCAGCGGTGGCTATGGACAGCAGGACCGTGGA
GGCCGCGGCAGGGGTGGCAGTGGTGGCGGCGGCGGCGGCGGTGGTGGTTACAACCGCAGCAGTGGTGGCTAT
GAACCCAGAGGTCGTGGAGGTGGCCGTGGAGGCAGAGGTGGCATGGGCGGAAGTGACCGTGGTGGCTTCAATAAA

TTTGGTGGCCCTCGGGACCAAGGATCACGTCATGACTCCGAACAGGATAATTCAGACAACAACACCATCTTTGTG
CAAGGCCTGGGTGAGAATGTTACAATTGAGTCTGTGGCTGATTACTTCAAGCAGATTGGTATTATTAAGACAAAC
AAGAAAACGGGACAGCCCATGATTAATTTGTACACAGACAGGGAAACTGGCAAGCTGAAGGGAGAGGCAACGGTC
TCTTTTGATGACCCACCTTCAGCTAAAGCAGCTATTGACTGGTTTGATGGTAAAGAATTCTCCGGAAATCCTATC
AAGGTCTCATTTGCTACTCGCCGGGCAGACTTTAATCGGGGTGGTGGCAATGGTCGTGGAGGCCGAGGGCGAGGA
GGACCCATGGGCCGTGGAGGCTATGGAGGTGGTGGCAGTGGTGGTGGTGGCCGAGGAGGATTTCCCAGTGGAGGT
GGTGGCGGTGGAGGACAGCAGCGAGCTGGTGACTGGAAGTGTCCTAATCCCACCTGTGAGAATATGAACTTCTCT
TGGAGGAATGAATGCAACCAGTGTAAGGCCCCTAAACCAGATGGCCCAGGAGGGGGACCAGGTGGCTCTCACATG
GGGGGTAACTACGGGGATGATCGTCGTGGTGGCAGAGGTGGTGCGATCGCAGGCGCCGATTACAAGGACGATGAT
GACAAGGGAGCACCAGGAAGTGCTGGTTCTGCTGCTGGTAGTGGAGCGTGCCCGGTGCCGCTGCAGCTGCCGCCG
CTGGAACGCCTGACCCTGGATGACAAAAAACCGCTGAATACCCTGATCTCTGCTACTGGTCTGTGGATGAGTCGT
ACCGGAACCATTCATAAAATCAAACACCACGAGGTTAGCCGTTCGAAAATCTATATTGAGATGGCGTGTGGCGAT
CATCTGGTTGTGAACAATAGCCGCTCTTCTCGTACAGCACGTGCACTGCGTCACCACAAATATCGTAAAACCTGT
AAACGTTGCCGTGTGTCCGATGAGGATCTGAACAAATTCCTGACAAAAGCCAATGAGGACCAAACAAGCGTGAAA
GTGAAAGTCGTTAGCGCTCCTACCCGTACTAAAAAAGCAATGCCGAAATCCGTTGCTCGTGCCCCTAAACCACTG
GAAAACACTGAAGCAGCACAGGCACAGCCGTCTGGAAGCAAATTCTCTCCGGCCATTCCTGTTTCTACCCAGGAG
TCCGTTTCTGTTCCAGCAAGTGTGAGCACCAGCATTAGCAGTATTAGCACCGGTGCCACCGCTAGCGCCCTGGTT
AAAGGCAATACCAATCCGATTACAAGCATGTCTGCCCCGGTTCAAGCATCAGCTCCAGCACTGACAAAATCCCAA
ACCGATCGTCTGGAGGTTCTGCTGAATCCGAAAGACGAAATCAGCCTGAATTCCGGCAAACCGTTTCGTGAACTG
GAGAGCGAACTGCTGTCACGTCGTAAAAAAGACCTGCAACAAATCTATGCCGAAGAACGTGAGAACTATCTGGGG
AAACTGGAACGTGAAATCACCCGCTTTTTCGTGGATCGTGGCTTTCTGGAGATCAAATCCCCGATTCTGATTCCT
CTGGAGTATATCGAGCGTATGGGCATCGACAATGATACCGAACTGAGCAAACAAATTTTCCGTGTGGATAAAAAC
TTCTGTCTGCGCCCTATGCTGGCACCAAATCTGTATAACTATCTGCGCAAACTGGACCGTGCCCTGCCTGATCCT
ATCAAAATCTTCGAGATCGGCCCGTGTTATCGTAAAGAGTCCGACGGTAAAGAACATCTGGAGGAGTTTACCATG
CTGGCCTTTGCCCAAATGGGTTCAGGTTGTACTCGTGAGAACCTGGAAAGCATCATCACCGATTTTCTGAACCAC
CTGGGCATTGACTTCAAAATTGTGGGCGACAGCTGTATGGTGTATGGCGACACCCTGGATGTCATGCACGGCGAC
CTGGAACTGTCTAGTGCCGTTGTTGGACCAATTCCGCTGGACCGTGAGTGGGGTATCGACAAACCGTGGATCGGA
GCAGGATTCGGTCTGGAACGCCTGCTGAAAGTGAAACACGACTTCAAAAACATCAAACGTGCCGCCCGTTCTGAA
TCGTATTATAACGGGATCTCTACGAACCTGTAA (SEQ ID NO: 361)

Protein:
MAICQFFLQGRCRFGDRCWNEHPGARGAGGGRQQPQQQPSGNNRRGWNTTSQRYSNVIQPSSFSKSTPWGGSRDQ
EKPYFSSFDSGASTNRKEGFGLSENPFASLSPDEQKDEKKLLEGIVKDMEVWESSGQWMFSVYSPVKKKPNISGF
TDISPEELRLEYHNFLTSNNLQSYLNSVQRLINQWRNRVNELKSLNISTKVALLSDVKDGVNQAAPAFGFGSSQA
ATFMSPGFPVNNSSSDNAQNFSFKTNSGFAAASSGSPAGFGSSPAFGAAASTSSGISTSAPAFGFGKPEVTSAAS
FSFKSPAASSFGSPGFSGLPASLATGPVRAPVAPAFGGGSSVAGFGSPGSHSHTAFSKPSSDTFGNSSISTSLSA
SSSIIATDNVLFTPRDKLTVEELEQFQSKKFTLGKIPLKPPPLELLNVGAPGSAGSAAGSGMASNDYTQQATQSY
GAYPTQPGQGYSQQSSQPYGQQSYSGYSQSTDTSGYGQSSYSSYGQSQNTGYGTQSTPQGYGSTGGYGSSQSSQS
SYGQQSSYPGYGQQPAPSSTSGSYGSSSQSSSYGQPQSGSYSQQPSYGGQQQSYGQQQSYNPPQGYGQQNQYNSS
SGGGGGGGGGGNYGQDQSSMSSGGGSGGGYGNQDQSGGGGSGGYGQQDRGGRGRGGSGGGGGGGGGGYNRSSGGY
EPRGRGGGGRGGRGGMGGSDRGGFNKFGGPRDQGSRHDSEQDNSDNNTIFVQGLGENVTIESVADYFKQIGIIKTN
KKTGQPMINLYTDRETGKLKGEATVSFDDPPSAKAAIDWFDGKEFSGNPIKVSFATRRADFNRGGGNGRGGRGRG
GPMGRGGYGGGGSGGGGRGGFPSGGGGGGGQQRAGDWKCPNPTCENMNFSWRNECNQCKAPKPDGPGGGPGGSHM
GGNYGDDRRGGRGGAIAGADYKDDDDKGAPGSAGSAAGSGACPVPLQLPPLERLTLDDKKPLNTLISATGLWMSR
TGTIHKIKHHEVSRSKIYIEMACGDHLVVNNSRSSRTARALRHHKYRKTCKRCRVSDEDLNKFLTKANEDQTSVK
VKVVSAPTRTKKAMPKSVARAPKPLENTEAAQAQPSGSKFSPAIPVSTQESVSVPASVSTSISSISTGATASALV
KGNTNPITSMSAPVQASAPALTKSQTDRLEVLLNPKDEISLNSGKPFRELESELLSRRKKDLQQIYAEERENYLG
KLEREITRFFVDRGFLEIKSPILIPLEYIERMGIDNDTELSKQIFRVDKNFCLRPMLAPNLYNYLRKLDRALPDP
IKIFEIGPCYRKESDGKEHLEEFTMLAFAQMGSGCTRENLESIITDFLNHLGIDFKIVGDSCMVYGDTLDVMHGD
LELSSAVVGPIPLDREWGIDKPWIGAGFGLERLLKVKHDFKNIKRAARSESYYNGISTNL* (SEQ ID NO:
362)

CG1::FUS::PylRS(AAAF)
DNA:
ATGGCCATTTGTCAATTCTTCCTTCAAGGCCGGTGCCGCTTTGGAGATCGGTGCTGGAACGAACATCCCGGTGCT
AGGGGTGCAGGAGGAGGACGGCAGCAACCGCAGCAGCAGCCTTCAGGTAATAATAGACGTGGATGGAATACAACT
AGCCAGAGATATTCCAATGTCATCCAGCCATCCAGTTTCTCCAAATCCACACCATGGGGGGGCAGCAGAGATCAA

GAAAAGCCATATTTCAGTTCTTTTGATTCTGGAGCTTCAACTAACAGGAAGGAAGGCTTTGGATTGTCTGAGAAC
CCATTTGCTTCACTTAGTCCTGATGAGCAGAAAGATGAAAAGAAACTTCTGGAAGGAATTGTAAAAGATATGGAG
GTTTGGGAATCATCAGGGCAGTGGATGTTTTCTGTTTATTCACCAGTGAAAAAGAAACCTAATATTTCAGGTTTT
ACAGACATTTCACCAGAGGAATTGAGGCTTGAATACCATAACTTCTTAACCAGCAATAACTTACAGAGTTATCTA
AATTCTGTCCAACGTTTAATAAATCAATGGAGGAACAGGGTAAATGAACTGAAAAGTCTAAATATATCAACTAAA
GTAGCTTTGCTCTCTGATGTAAAGGATGGAGTAAATCAAGCAGCACCTGCATTTGGATTTGGCAGCAGTCAAGCA
GCAACATTTATGTCGCCAGGCTTTCCAGTCAATAACAGCAGCAGTGATAATGCTCAGAACTTTAGTTTTAAAACA
AACTCTGGATTTGCTGCTGCCTCTTCTGGAAGCCCTGCTGGTTTTGGGAGTTCCCCAGCATTTGGAGCTGCAGCC
TCTACCAGTTCAGGTATCTCTACTTCTGCTCCAGCTTTTGGATTTGGGAAGCCTGAAGTCACATCGGCTGCATCA
TTTTCATTCAAAAGCCCTGCAGCTTCCAGTTTTGGATCACCTGGATTTTCAGGACTTCCAGCTTCCTTGGCAACA
GGTCCTGTCAGAGCTCCAGTGGCCCCAGCCTTTGGAGGTGGCAGTTCTGTGGCTGGTTTTGGTAGTCCGGGCTCA
CATTCTCACACTGCTTTTTCTAAGCCATCCAGTGACACTTTTGGAAATAGCAGCATATCCACTTCTCTGTCAGCC
TCAAGCAGCATCATTGCAACAGATAATGTGTTATTCACACCCAGAGATAAACTAACAGTAGAAGAACTGGAACAA
TTTCAATCCAAGAAATTTACTCTGGGAAAAATTCCATTAAAGCCTCCACCTCTGGAACTTCTAAATGTTGGAGCA
CCCGGCTCCGCCGGCTCCGCCGCCGGCTCCGGCATGGCCTCAAACGATTATACCCAACAAGCAACCCAAAGCTAT
GGGGCCTACCCCACCCAGCCCGGGCAGGGCTATTCCCAGCAGAGCAGTCAGCCCTACGGACAGCAGAGTTACAGT
GGTTATAGCCAGTCCACGGACACTTCAGGATATGGCCAGAGCAGCTATTCTTCTTATGGCCAGAGCCAGAACACA
GGCTATGGAACTCAGTCAACTCCCCAGGGATATGGCTCGACTGGCGGCTATGGCAGTAGCCAGAGCTCCCAATCG
TCTTACGGGCAGCAGTCCTCCTACCCTGGCTATGGCCAGCAGCCAGCTCCCAGCAGCACCTCGGGAAGTTACGGT
AGCAGTTCTCAGAGCAGCAGCTATGGGCAGCCCCAGAGTGGGAGCTACAGCCAGCAGCCTAGCTATGGTGGACAG
CAGCAAAGCTATGGACAGCAGCAAAGCTATAATCCCCCTCAGGGCTATGGACAGCAGAACCAGTACAACAGCAGC
AGTGGTGGTGGAGGTGGAGGTGGAGGTGGAGGTAACTATGGCCAAGATCAATCCTCCATGAGTAGTGGTGGTGGC
AGTGGTGGCGGTTATGGCAATCAAGACCAGAGTGGTGGAGGTGGCAGCGGTGGCTATGGACAGCAGGACCGTGGA
GGCCGCGGCAGGGGTGGCAGTGGTGGCGGCGGCGGCGGCGGCGGTGGTGGTTACAACCGCAGCAGTGGTGGCTAT
GAACCCAGAGGTCGTGGAGGTGGCCGTGGAGGCAGAGGTGGCATGGGCGGAAGTGACCGTGGTGGCTTCAATAAA
TTTGGTGGCCCTCGGGACCAAGGATCACGTCATGACTCCGAACAGGATAATTCAGACAACAACACCATCTTTGTG
CAAGGCCTGGGTGAGAATGTTACAATTGAGTCTGTGGCTGATTACTTCAAGCAGATTGGTATTATTAAGACAAAC
AAGAAAACGGGACAGCCCATGATTAATTTGTACACAGACAGGGAAACTGGCAAGCTGAAGGGAGAGGCAACGGTC
TCTTTTGATGACCCACCTTCAGCTAAAGCAGCTATTGACTGGTTTGATGGTAAAGAATTCTCCGGAAATCCTATC
AAGGTCTCATTTGCTACTCGCCGGGCAGACTTTAATCGGGGTGGTGGCAATGGTCGTGGAGGCCGAGGGCGAGGA
GGACCCATGGGCCGTGGAGGCTATGGAGGTGGTGGCAGTGGTGGTGGTGGCCGAGGAGGATTTCCCAGTGGAGGT
GGTGGCGGTGGAGGACAGCAGCGAGCTGGTGACTGGAAGTGTCCTAATCCCACCTGTGAGAATATGAACTTCTCT
TGGAGGAATGAATGCAACCAGTGTAAGGCCCCTAAACCAGATGGCCCAGGAGGGGGACCAGGTGGCTCTCACATG
GGGGGTAACTACGGGGATGATCGTCGTGGTGGCAGAGGTGGTGCGATCGCAGGCGCCGATTACAAGGACGATGAT
GACAAGGGAGCACCAGGAAGTGCTGGTTCTGCTGCTGGTAGTGGAGCGTGCCCGGTGCCGCTGCAGCTGCCGCCG
CTGGAACGCCTGACCCTGGATGATAAAAAACCGCTGAATACCCTGATCTCTGCTACTGGTCTGTGGATGAGTCGT
ACCGGAACCATTCATAAAATCAAACACCACGAGGTTAGCCGTTCGAAAATCTATATTGAGATGGCGTGTGGCGAT
CATCTGGTTGTGAACAATAGCCGCTCTTCTCGTACAGCACGTGCACTGCGTCACCACAAATATCGTAAAACCTGT
AAACGTTGCCGTGTGTCCGATGAGGATCTGAACAAATTCCTGACAAAAGCCAATGAGGACCAAACAAGCGTGAAA
GTGAAAGTCGTTAGCGCTCCTACCCGTACTAAAAAAGCAATGCCGAAATCCGTTGCTCGTGCCCCTAAACCACTG
GAAAACACTGAAGCAGCACAGGCACAGCCGTCTGGAAGCAAATTCTCTCCGGCCATTCCTGTTTCTACCCAGGAG
TCCGTTTCTGTTCCAGCAAGTGTGAGCACCAGCATTAGCAGTATTAGCACCGGTGCCACCGCTAGCGCCCTGGTT
AAAGGCAATACCAATCCGATTACAAGCATGTCTGCCCCGGTTCAAGCATCAGCTCCAGCACTGACAAAATCCCAA
ACCGATCGTCTGGAGGTTCTGCTGAATCCGAAAGACGAAATCAGCCTGAATTCCGGCAAACCGTTTCGTGAACTG
GAGAGCGAACTGCTGTCACGTCGTAAAAAAGACCTGCAACAAATCTATGCCGAAGAACGTGAGAACTATCTGGGG
AAACTGGAACGTGAAATCACCCGCTTTTTCGTGGATCGTGGCTTTCTGGAGATCAAATCCCCGATTCTGATTCCT
CTGGAGTATATCGAGCGTATGGGCATCGACAATGATACCGAACTGAGCAAACAAATTTTCCGTGTGGATAAAAAC
TTCTGTCTGCGCCCTATGCTAGCACCAAATCTGGCTAACTATCTGCGCAAACTGGACCGTGCCCTGCCTGATCCT
ATCAAAATCTTCGAGATCGGCCCGTGTTATCGTAAAGAGTCCGACGGTAAAGAACATCTGGAGGAGTTTACCATG
CTGGCCTTTGCCCAAATGGGTTCAGGTTGTACTCGTGAGAACCTGGAAAGCATCATCACCGATTTTCTGAACCAC
CTGGGCATTGACTTCAAAATTGTGGGCGACAGCTGTATGGTGTTTGGCGACACCCTGGATGTCATGCACGGCGAC
CTGGAACTGTCTAGTGCCGTTGTGGGCCCAATCCCGCTGGATCGTGAGTGGGGTATCGACAAACCTTGGATCGGT
GCGGGTTTTGGTCTGGAGCGTCTGCTGAAAGTAAAACACGACTTCAAGAACATCAAACGTGCTGCACGTTCCGAG
TCCTATTACAATGGTATTTCTACTAACCTGTAA (SEQ ID NO: 363)

Protein:
MAICQFFLQGRCRFGDRCWNEHPGARGAGGGRQQPQQQPSGNNRRGWNTTSQRYSNVIQPSSFSKSTPWGGSRDQ
EKPYFSSFDSGASTNRKEGFGLSENPFASLSPDEQKDEKKLLEGIVKDMEVWESSGQWMFSVYSPVKKKPNISGF

TDISPEELRLEYHNFLTSNNLQSYLNSVQRLINQWRNRVNELKSLNISTKVALLSDVKDGVNQAAPAFGFGSSQA
ATFMSPGFPVNNSSSDNAQNFSFKTNSGFAAASSGSPAGFGSSPAFGAAASTSSGISTSAPAFGFGKPEVTSAAS
FSFKSPAASSFGSPGFSGLPASLATGPVRAPVAPAFGGGSSVAGFGSPGSHSHTAFSKPSSDTFGNSSISTSLSA
SSSIIATDNVLFTPRDKLTVEELEQFQSKKFTLGKIPLKPPPLELLNVGAPGSAGSAAGSGMASNDYTQQATQSY
GAYPTQPGQGYSQQSSQPYGQQSYSGYSQSTDTSGYGQSSYSSYGQSQNTGYGTQSTPQGYGSTGGYGSSQSSQS
SYGQQSSYPGYGQQPAPSSTSGSYGSSSQSSSYGQPQSGSYSQQPSYGGQQQSYGQQQSYNPPQGYGQQNQYNSS
SGGGGGGGGGGNYGQDQSSMSSGGGSGGGYGNQDQSGGGGSGGYGQQDRGGRGRGGSGGGGGGGGGGYNRSSGGY
EPRGRGGGGRGGRGGMGGSDRGGFNKFGGPRDQGSRHDSEQDNSDNNTIFVQGLGENVTIESVADYFKQIGIIKTN
KKTGQPMINLYTDRETGKLKGEATVSFDDPPSAKAAIDWFDGKEFSGNPIKVSFATRRADFNRGGGNGRGGRGRG
GPMGRGGYGGGGSGGGGRGGFPSGGGGGGGQQRAGDWKCPNPTCENMNFSWRNECNQCKAPKPDGPGGGPGGSHM
GGNYGDDRRGGRGGAIAGADYKDDDDKGAPGSAGSAAGSGACPVPLQLPPLERLTLDDKKPLNTLISATGLWMSR
TGTIHKIKHHEVSRSKIYIEMACGDHLVVNNSRSSRTARALRHHKYRKTCKRCRVSDEDLNKFLTKANEDQTSVK
VKVVSAPTRTKKAMPKSVARAPKPLENTEAAQAQPSGSKFSPAIPVSTQESVSVPASVSTSISSISTGATASALV
KGNTNPITSMSAPVQASAPALTKSQTDRLEVLLNPKDEISLNSGKPFRELESELLSRRKKDLQQIYAEERENYLG
KLEREITRFFVDRGFLEIKSPILIPLEYIERMGIDNDTELSKQIFRVDKNFCLRPMLAPNLANYLRKLDRALPDP
IKIFEIGPCYRKESDGKEHLEEFTMLAFAQMGSGCTRENLESIITDFLNHLGIDFKIVGDSCMVFGDTLDVMHGD
LELSSAVVGPIPLDREWGIDKPWIGAGFGLERLLKVKHDFKNIKRAARSESYYNGISTNL* (SEQ ID NO:
364)

CG1::MCP
DNA:
ATGGCCATTTGTCAATTCTTCCTTCAAGGCCGGTGCCGCTTTGGAGATCGGTGCTGGAACGAACATCCCGGTGCT
AGGGGTGCAGGAGGAGGACGGCAGCAACCGCAGCAGCAGCCTTCAGGTAATAATAGACGTGGATGGAATACAACT
AGCCAGAGATATTCCAATGTCATCCAGCCATCCAGTTTCTCCAAATCCACACCATGGGGGGGCAGCAGAGATCAA
GAAAAGCCATATTTCAGTTCTTTTGATTCTGGAGCTTCAACTAACAGGAAGGAAGGCTTTGGATTGTCTGAGAAC
CCATTTGCTTCACTTAGTCCTGATGAGCAGAAAGATGAAAAGAAACTTCTGGAAGGAATTGTAAAAGATATGGAG
GTTTGGGAATCATCAGGGCAGTGGATGTTTTCTGTTTATTCACCAGTGAAAAAGAAACCTAATATTTCAGGTTTT
ACAGACATTTCACCAGAGGAATTGAGGCTTGAATACCATAACTTCTTAACCAGCAATAACTTACAGAGTTATCTA
AATTCTGTCCAACGTTTAATAAATCAATGGAGGAACAGGGTAAATGAACTGAAAAGTCTAAATATATCAACTAAA
GTAGCTTTGCTCTCTGATGTAAAGGATGGAGTAAATCAAGCAGCACCTGCATTTGGATTTGGCAGCAGTCAAGCA
GCAACATTTATGTCGCCAGGCTTTCCAGTCAATAACAGCAGCAGTGATAATGCTCAGAACTTTAGTTTTAAAACA
AACTCTGGATTTGCTGCTGCCTCTTCTGGAAGCCCTGCTGGTTTTGGGAGTTCCCCAGCATTTGGAGCTGCAGCC
TCTACCAGTTCAGGTATCTCTACTTCTGCTCCAGCTTTTGGATTTGGGAAGCCTGAAGTCACATCGGCTGCATCA
TTTTCATTCAAAAGCCCTGCAGCTTCCAGTTTTGGATCACCTGGATTTTCAGGACTTCCAGCTTCCTTGGCAACA
GGTCCTGTCAGAGCTCCAGTGGCCCCAGCCTTTGGAGGTGGCAGTTCTGTGGCTGGTTTTGGTAGTCCGGGCTCA
CATTCTCACACTGCTTTTTCTAAGCCATCCAGTGACACTTTTGGAAATAGCAGCATATCCACTTCTCTGTCAGCC
TCAAGCAGCATCATTGCAACAGATAATGTGTTATTCACACCCAGAGATAAACTAACAGTAGAAGAACTGGAACAA
TTTCAATCCAAGAAATTTACTCTGGGAAAAATTCCATTAAAGCCTCCACCTCTGGAACTTCTAAATGTTGCGATC
GCATATCCCTATGATGTGCCGGATTATGCTGGAGCACCAGGAAGTGCTGGTTCTGCTGCTGGTAGTGGAGCTTCT
AACTTTACTCAGTTCGTTCTCGTCGACAATGGCGGAACTGGCGACGTGACTGTCGCCCCAAGCAACTTCGCTAAC
GGGATCGCTGAATGGATCAGCTCTAACTCGCGTTCACAGGCTTACAAAGTAACCTGTAGCGTTCGTCAGAGCTCT
GCGCAGAATCGCAAATACACCATCAAAGTCGAGGTGCCTAAAGGCGCCTGGCGTTCGTACTTAAATATGGAACTA
ACCATTCCAATTTTCGCCACGAATTCCGACTGCGAGCTTATTGTTAAGGCAATGCAAGGTCTCCTAAAAGATGGA
AACCCGATTCCCTCAGCAATCGCAGCAAACTCCGGCATCTACTAA (SEQ ID NO: 365)

Protein:
MAICQFFLQGRCRFGDRCWNEHPGARGAGGGRQQPQQQPSGNNRRGWNTTSQRYSNVIQPSSFSKSTPWGGSRDQ
EKPYFSSFDSGASTNRKEGFGLSENPFASLSPDEQKDEKKLLEGIVKDMEVWESSGQWMFSVYSPVKKKPNISGF
TDISPEELRLEYHNFLTSNNLQSYLNSVQRLINQWRNRVNELKSLNISTKVALLSDVKDGVNQAAPAFGFGSSQA
ATFMSPGFPVNNSSSDNAQNFSFKTNSGFAAASSGSPAGFGSSPAFGAAASTSSGISTSAPAFGFGKPEVTSAAS
FSFKSPAASSFGSPGFSGLPASLATGPVRAPVAPAFGGGSSVAGFGSPGSHSHTAFSKPSSDTFGNSSISTSLSA
SSSIIATDNVLFTPRDKLTVEELEQFQSKKFTLGKIPLKPPPLELLNVAIAYPYDVPDYAGAPGSAGSAAGSGAS
NFTQFVLVDNGGTGDVTVAPSNFANGIAEWISSNSRSQAYKVTCSVRQSSAQNRKYTIKVEVPKGAWRSYLNMEL
TIPIFATNSDCELIVKAMQGLLKDGNPIPSAIAANSGIY* (SEQ ID NO: 366)

CG1::EWSR1::MCP

DNA:
ATGGCCATTTGTCAATTCTTCCTTCAAGGCCGGTGCCGCTTTGGAGATCGGTGCTGGAACGAACATCCCGGTGCT
AGGGGTGCAGGAGGAGGACGGCAGCAACCGCAGCAGCAGCCTTCAGGTAATAATAGACGTGGATGGAATACAACT
AGCCAGAGATATTCCAATGTCATCCAGCCATCCAGTTTCTCCAAATCCACACCATGGGGGGGCAGCAGAGATCAA
GAAAAGCCATATTTCAGTTCTTTTGATTCTGGAGCTTCAACTAACAGGAAGGAAGGCTTTGGATTGTCTGAGAAC
CCATTTGCTTCACTTAGTCCTGATGAGCAGAAAGATGAAAAGAAACTTCTGGAAGGAATTGTAAAAGATATGGAG
GTTTGGGAATCATCAGGGCAGTGGATGTTTTCTGTTTATTCACCAGTGAAAAAGAAACCTAATATTTCAGGTTTT
ACAGACATTTCACCAGAGGAATTGAGGCTTGAATACCATAACTTCTTAACCAGCAATAACTTACAGAGTTATCTA
AATTCTGTCCAACGTTTAATAAATCAATGGAGGAACAGGGTAAATGAACTGAAAAGTCTAAATATATCAACTAAA
GTAGCTTTGCTCTCTGATGTAAAGGATGGAGTAAATCAAGCAGCACCTGCATTTGGATTTGGCAGCAGTCAAGCA
GCAACATTTATGTCGCCAGGCTTTCCAGTCAATAACAGCAGCAGTGATAATGCTCAGAACTTTAGTTTTAAAACA
AACTCTGGATTTGCTGCTGCCTCTTCTGGAAGCCCTGCTGGTTTTGGGAGTTCCCCAGCATTTGGAGCTGCAGCC
TCTACCAGTTCAGGTATCTCTACTTCTGCTCCAGCTTTTGGATTTGGGAAGCCTGAAGTCACATCGGCTGCATCA
TTTTCATTCAAAAGCCCTGCAGCTTCCAGTTTTGGATCACCTGGATTTTCAGGACTTCCAGCTTCCTTGGCAACA
GGTCCTGTCAGAGCTCCAGTGGCCCCAGCCTTTGGAGGTGGCAGTTCTGTGGCTGGTTTTGGTAGTCCGGGCTCA
CATTCTCACACTGCTTTTTCTAAGCCATCCAGTGACACTTTTGGAAATAGCAGCATATCCACTTCTCTGTCAGCC
TCAAGCAGCATCATTGCAACAGATAATGTGTTATTCACACCCAGAGATAAACTAACAGTAGAAGAACTGGAACAA
TTTCAATCCAAGAAATTTACTCTGGGAAAAATTCCATTAAAGCCTCCACCTCTGGAACTTCTAAATGTTATGGCG
TCCACGGATTACAGTACCTATAGCCAAGCTGCAGCGCAGCAGGGCTACAGTGCTTACACCGCCCAGCCCACTCAA
GGATATGCACAGACCACCCAGGCATATGGGCAACAAAGCTATGGAACCTATGGACAGCCCACTGATGTCAGCTAT
ACCCAGGCTCAGACCACTGCAACCTATGGGCAGACCGCCTATGCAACTTCTTATGGACAGCCTCCCACTGGTTAT
ACTACTCCAACTGCCCCCCAGGCATACAGCCAGCCTGTCCAGGGGTATGGCACTGGTGCTTATGATACCACCACT
GCTACAGTCACCACCACCCAGGCCTCCTATGCAGCTCAGTCTGCATATGGCACTCAGCCTGCTTATCCAGCCTAT
GGGCAGCAGCCAGCAGCCACTGCACCTACAAGACCGCAGGATGGAAACAAGCCCACTGAGACTAGTCAACCTCAA
TCTAGCACAGGGGGTTACAACCAGCCCAGCCTAGGATATGGACAGAGTAACTACAGTTATCCCCAGGTACCTGGG
AGCTACCCCATGCAGCCAGTCACTGCACCTCCATCCTACCCTCCTACCAGCTATTCCTCTACACAGCCGACTAGT
TATGATCAGAGCAGTTACTCTCAGCAGAACACCTATGGGCAACCGAGCAGCTATGGACAGCAGAGTAGCTATGGT
CAACAAAGCAGCTATGGGCAGCAGCCTCCCACTAGTTACCCACCCCAAACTGGATCCTACAGCCAAGCTCCAAGT
CAATATAGCCAACAGAGCAGCAGCTACGGGCAGCAGAGTTCATTCCGACAGGACCACCCCAGTAGCATGGGTGTT
TATGGGCAGGAGTCTGGAGGATTTTCCGGACCAGGAGAGAACCGGAGCATGAGTGGCCCTGATAACCGGGGCAGG
GGAAGAGGGGGATTTGATCGTGGAGGCATGAGCAGAGGTGGGCGGGGAGGAGGACGCGGTGGAATGGGCAGCGCT
GGAGAGCGAGGTGGCTTCAATAAGCCTGGTGGACCCATGGATGAAGGACCAGATCTTGATCTAGGCCCACCTGTA
GATCCAGATGAAGACTCTGACAACAGTGCAATTTATGTACAAGGATTAAATGACAGTGTGACTCTAGATGATCTG
GCAGACTTCTTTAAGCAGTGTGGGGTTGTTAAGATGAACAAGAGAACTGGGCAACCCATGATCCACATCTACCTG
GACAAGGAAACAGGAAAGCCCAAAGGCGATGCCACAGTGTCCTATGAAGACCCACCTACTGCCAAGGCTGCCGTG
GAATGGTTTGATGGGAAAGATTTTCAAGGGAGCAAACTTAAAGTCTCCCTTGCTCGGAAGAAGCCTCCAATGAAC
AGTATGCGGGGTGGTCTGCCACCCCGTGAGGGCAGAGGCATGCCACCACCACTCCGTGGAGGTCCAGGAGGCCCA
GGAGGTCCTGGGGGACCCATGGGTCGCATGGGAGGCCGTGGAGGAGATAGAGGAGGCTTCCCTCCAAGAGGACCC
CGGGGTTCCCGAGGGAACCCCTCTGGAGGAGGAAACGTCCAGCACCGAGCTGGAGACTGGCAGTGTCCCAATCCG
GGTTGTGGAAACCAGAACTTCGCCTGGAGAACAGAGTGCAACCAGTGTAAGGCCCCAAAGCCTGAAGGCTTCCTC
CCGCCACCCTTTCCGCCCCCGGGTGGTGATCGTGGCAGAGGTGGCCCTGGTGGCATGCGGGGAGGAAGAGGTGGC
CTCATGGATCGTGGTGGTCCCGGTGGAATGTTCAGAGGTGGCCGTGGTGGAGACAGAGGTGGCTTCCGTGGTGGC
CGGGGCATGGACCGAGGTGGCTTTGGTGGAGGAAGACGAGGTGGCCCTGGGGGGCCCCCTGGACCTTTGATGGAA
CAGGCGATCGCATATCCCTATGATGTGCCGGATTATGCTGGAGCACCAGGAAGTGCTGGTTCTGCTGCTGGTAGT
GGAGCTTCTAACTTTACTCAGTTCGTTCTCGTCGACAATGGCGGAACTGGCGACGTGACTGTCGCCCCAAGCAAC
TTCGCTAACGGGATCGCTGAATGGATCAGCTCTAACTCGCGTTCACAGGCTTACAAAGTAACCTGTAGCGTTCGT
CAGAGCTCTGCGCAGAATCGCAAATACACCATCAAAGTCGAGGTGCCTAAAGGCGCCTGGCGTTCGTACTTAAAT
ATGGAACTAACCATTCCAATTTTCGCCACGAATTCCGACTGCGAGCTTATTGTTAAGGCAATGCAAGGTCTCCTA
AAAGATGGAAACCCGATTCCCTCAGCAATCGCAGCAAACTCCGGCATCTACTAA (SEQ ID NO: 367)

Protein:
MAICQFFLQGRCRFGDRCWNEHPGARGAGGGRQQPQQQPSGNNRRGWNTTSQRYSNVIQPSSFSKSTPWGGSRDQ
EKPYFSSFDSGASTNRKEGFGLSENPFASLSPDEQKDEKKLLEGIVKDMEVWESSGQWMFSVYSPVKKKPNISGF
TDISPEELRLEYHNFLTSNNLQSYLNSVQRLINQWRNRVNELKSLNISTKVALLSDVKDGVNQAAPAFGFGSSQA
ATFMSPGFPVNNSSSDNAQNFSFKTNSGFAAASSGSPAGFGSSPAFGAAASTSSGISTSAPAFGFGKPEVTSAAS
FSFKSPAASSFGSPGFSGLPASLATGPVRAPVAPAFGGGSSVAGFGSPGSHSHTAFSKPSSDTFGNSSISTSLSA
SSSIIATDNVLFTPRDKLTVEELEQFQSKKFTLGKIPLKPPPLELLNVMASTDYSTYSQAAAQQGYSAYTAQPTQ
GYAQTTQAYGQQSYGTYGQPTDVSYTQAQTTATYGQTAYATSYGQPPTGYTTPTAPQAYSQPVQGYGTGAYDTTT

ATVTTTQASYAAQSAYGTQPAYPAYGQQPAATAPTRPQDGNKPTETSQPQSSTGGYNQPSLGYGQSNYSYPQVPG
SYPMQPVTAPPSYPPTSYSSTQPTSYDQSSYSQQNTYGQPSSYGQQSSYGQQSSYGQQPPTSYPPQTGSYSQAPS
QYSQQSSSYGQQSSFRQDHPSSMGVYGQESGGFSGPGENRSMSGPDNRGRGRGGFDRGGMSRGGRGGGRGGMGSA
GERGGFNKPGGPMDEGPDLDLGPPVDPDEDSDNSAIYVQGLNDSVTLDDLADFFKQCGVVKMNKRTGQPMIHIYL
DKETGKPKGDATVSYEDPPTAKAAVEWFDGKDFQGSKLKVSLARKKPPMNSMRGGLPPREGRGMPPPLRGGPGGP
GGPGGPMGRMGGRGGDRGGFPPRGPRGSRGNPSGGGNVQHRAGDWQCPNPGCGNQNFAWRTECNQCKAPKPEGFL
PPPFPPPGGDRGRGGPGGMRGGRGGLMDRGGPGGMFRGGRGGDRGGFRGGRGMDRGGFGGGRRGGPGGPPGPLME
QAIAYPYDVPDYAGAPGSAGSAAGSGASNFTQFVLVDNGGTGDVTVAPSNFANGIAEWISSNSRSQAYKVTCSVR
QSSAQNRKYTIKVEVPKGAWRSYLNMELTIPIFATNSDCELIVKAMQGLLKDGNPIPSAIAANSGIY*    (SEQ
ID NO: 368)

CG1::FUS::PylRS(AF)
DNA:
ATGGCCATTTGTCAATTCTTCCTTCAAGGCCGGTGCCGCTTTGGAGATCGGTGCTGGAACGAACATCCCGGTGCT
AGGGGTGCAGGAGGAGGACGGCAGCAACCGCAGCAGCAGCCTTCAGGTAATAATAGACGTGGATGGAATACAACT
AGCCAGAGATATTCCAATGTCATCCAGCCATCCAGTTTCTCCAAATCCACACCATGGGGGGGCAGCAGAGATCAA
GAAAAGCCATATTTCAGTTCTTTTGATTCTGGAGCTTCAACTAACAGGAAGGAAGGCTTTGGATTGTCTGAGAAC
CCATTTGCTTCACTTAGTCCTGATGAGCAGAAAGATGAAAAGAAACTTCTGGAAGGAATTGTAAAAGATATGGAG
GTTTGGGAATCATCAGGGCAGTGGATGTTTTCTGTTTATTCACCAGTGAAAAAGAAACCTAATATTTCAGGTTTT
ACAGACATTTCACCAGAGGAATTGAGGCTTGAATACCATAACTTCTTAACCAGCAATAACTTACAGAGTTATCTA
AATTCTGTCCAACGTTTAATAAATCAATGGAGGAACAGGGTAAATGAACTGAAAAGTCTAAATATATCAACTAAA
GTAGCTTTGCTCTCTGATGTAAAGGATGGAGTAAATCAAGCAGCACCTGCATTTGGATTTGGCAGCAGTCAAGCA
GCAACATTTATGTCGCCAGGCTTTCCAGTCAATAACAGCAGCAGTGATAATGCTCAGAACTTTAGTTTTAAAACA
AACTCTGGATTTGCTGCTGCCTCTTCTGGAAGCCCTGCTGGTTTTGGGAGTTCCCCAGCATTTGGAGCTGCAGCC
TCTACCAGTTCAGGTATCTCTACTTCTGCTCCAGCTTTTGGATTTGGGAAGCCTGAAGTCACATCGGCTGCATCA
TTTTCATTCAAAAGCCCTGCAGCTTCCAGTTTTGGATCACCTGGATTTTCAGGACTTCCAGCTTCCTTGGCAACA
GGTCCTGTCAGAGCTCCAGTGGCCCCAGCCTTTGGAGGTGGCAGTTCTGTGGCTGGTTTTGGTAGTCCGGGCTCA
CATTCTCACACTGCTTTTTCTAAGCCATCCAGTGACACTTTTGGAAATAGCAGCATATCCACTTCTCTGTCAGCC
TCAAGCAGCATCATTGCAACAGATAATGTGTTATTCACACCCAGAGATAAACTAACAGTAGAAGAACTGGAACAA
TTTCAATCCAAGAAATTTACTCTGGGAAAAATTCCATTAAAGCCTCCACCTCTGGAACTTCTAAATGTTGGAGCA
CCCGGCTCCGCCGGCTCCGCCGCCGGCTCCGGCATGGCCTCAAACGATTATACCCAACAAGCAACCCAAAGCTAT
GGGGCCTACCCCACCCAGCCCGGGCAGGGCTATTCCCAGCAGAGCAGTCAGCCCTACGGACAGCAGAGTTACAGT
GGTTATAGCCAGTCCACGGACACTTCAGGATATGGCCAGAGCAGCTATTCTTCTTATGGCCAGAGCCAGAACACA
GGCTATGGAACTCAGTCAACTCCCCAGGGATATGGCTCGACTGGCGGCTATGGCAGTAGCCAGAGCTCCCAATCG
TCTTACGGGCAGCAGTCCTCCTACCCTGGCTATGGCCAGCAGCCAGCTCCCAGCAGCACCTCGGGAAGTTACGGT
AGCAGTTCTCAGAGCAGCAGCTATGGGCAGCCCCAGAGTGGGAGCTACAGCCAGCAGCCTAGCTATGGTGGACAG
CAGCAAAGCTATGGACAGCAGCAAAGCTATAATCCCCCTCAGGGCTATGGACAGCAGAACCAGTACAACAGCAGC
AGTGGTGGTGGAGGTGGAGGTGGAGGTGGAGGTAACTATGGCCAAGATCAATCCTCCATGAGTAGTGGTGGTGGC
AGTGGTGGCGGTTATGGCAATCAAGACCAGAGTGGTGGAGGTGGCAGCGGTGGCTATGGACAGCAGGACCGTGGA
GGCCGCGGCAGGGGTGGCAGTGGTGGCGGCGGCGGCGGCGGTGGTGGTTACAACCGCAGCAGTGGTGGCTAT
GAACCCAGAGGTCGTGGAGGTGGCCGTGGAGGCAGAGGTGGCATGGGCGGAAGTGACCGTGGTGGCTTCAATAAA
TTTGGTGGCCCTCGGGACCAAGGATCACGTCATGACTCCGAACAGGATAATTCAGACAACAACACCATCTTTGTG
CAAGGCCTGGGTGAGAATGTTACAATTGAGTCTGTGGCTGATTACTTCAAGCAGATTGGTATTATTAAGACAAAC
AAGAAAACGGGACAGCCCATGATTAATTTGTACACAGACAGGGAAACTGGCAAGCTGAAGGGAGAGGCAACGGTC
TCTTTTGATGACCCACCTTCAGCTAAAGCAGCTATTGACTGGTTTGATGGTAAAGAATTCTCCGGAAATCCTATC
AAGGTCTCATTTGCTACTCGCCGGGCAGACTTTAATCGGGGTGGTGGCAATGGTCGTGGAGGCCGAGGGCGAGGA
GGACCCATGGGCCGTGGAGGCTATGGAGGTGGTGGCAGTGGTGGTGGTGGCCGAGGAGGATTTCCCAGTGGAGGT
GGTGGCGGTGGAGGACAGCAGCGAGCTGGTGACTGGAAGTGTCCTAATCCCACCTGTGAGAATATGAACTTCTCT
TGGAGGAATGAATGCAACCAGTGTAAGGCCCCTAAACCAGATGGCCCAGGAGGGGGACCAGGTGGCTCTCACATG
GGGGGTAACTACGGGGATGATCGTCGTGGTGGCAGAGGTGGTGCGATCGCAGGCGCCGATTACAAGGACGATGAT
GACAAGGGAGCACCAGGAAGTGCTGGTTCTGCTGCTGGTAGTGGAGCGTGCCCGGTGCCGCTGCAGCTGCCGCCG
CTGGAACGCCTGACCCTGGATGATAAAAAACCGCTGAATACCCTGATCTCTGCTACTGGTCTGTGGATGAGTCGT
ACCGGAACCATTCATAAAATCAAACACCACGAGGTTAGCCGTTCGAAAATCTATATTGAGATGGCGTGTGGCGAT
CATCTGGTTGTGAACAATAGCCGCTCTTCTCGTACAGCACGTGCACTGCGTCACCACAAATATCGTAAAACCTGT
AAACGTTGCCGTGTGTCCGATGAGGATCTGAACAAATTCCTGACAAAAGCCAATGAGGACCAAACAAGCGTGAAA
GTGAAAGTCGTTAGCGCTCCTACCCGTACTAAAAAAGCAATGCCGAAATCCGTTGCTCGTGCCCCTAAACCACTG
GAAAACACTGAAGCAGCACAGGCACAGCCGTCTGGAAGCAAATTCTCTCCGGCCATTCCTGTTTCTACCCAGGAG

TCCGTTTCTGTTCCAGCAAGTGTGAGCACCAGCATTAGCAGTATTAGCACCGGTGCCACCGCTAGCGCCCTGGTT
AAAGGCAATACCAATCCGATTACAAGCATGTCTGCCCCGGTTCAAGCATCAGCTCCAGCACTGACAAAATCCCAA
ACCGATCGTCTGGAGGTTCTGCTGAATCCGAAAGACGAAATCAGCCTGAATTCCGGCAAACCGTTTCGTGAACTG
GAGAGCGAACTGCTGTCACGTCGTAAAAAAGACCTGCAACAAATCTATGCCGAAGAACGTGAGAACTATCTGGGG
AAACTGGAACGTGAAATCACCCGCTTTTTCGTGGATCGTGGCTTTCTGGAGATCAAATCCCCGATTCTGATTCCT
CTGGAGTATATCGAGCGTATGGGCATCGACAATGATACCGAACTGAGCAAACAAATTTTCCGTGTGGATAAAAAC
TTCTGTCTGCGCCCTATGCTAGCACCAAATCTGGCTAACTATCTGCGCAAACTGGACCGTGCCCTGCCTGATCCT
ATCAAAATCTTCGAGATCGGCCCGTGTTATCGTAAAGAGTCCGACGGTAAAGAACATCTGGAGGAGTTTACCATG
CTGAACTTTTGCCAAATGGGTTCAGGTTGTACTCGTGAGAACCTGGAAAGCATCATCACCGATTTTCTGAACCAC
CTGGGCATTGACTTCAAAATTGTGGGCGACAGCTGTATGGTGTTTGGCGACACCCTGGATGTCATGCACGGCGAC
CTGGAACTGTCTAGTGCCGTTGTGGGCCCAATCCCGCTGGATCGTGAGTGGGGTATCGACAAACCTTGGATCGGT
GCGGGTTTTGGTCTGGAGCGTCTGCTGAAAGTAAAACACGACTTCAAGAACATCAAACGTGCTGCACGTTCCGAG
TCCTATTACAATGGTATTTCTACTAACCTGTAA (SEQ ID NO: 369)

Protein:
MAICQFFLQGRCRFGDRCWNEHPGARGAGGGRQQPQQQPSGNNRRGWNTTSQRYSNVIQPSSFSKSTPWGGSRDQ
EKPYFSSFDSGASTNRKEGFGLSENPFASLSPDEQKDEKKLLEGIVKDMEVWESSGQWMFSVYSPVKKKPNISGF
TDISPEELRLEYHNFLTSNNLQSYLNSVQRLINQWRNRVNELKSLNISTKVALLSDVKDGVNQAAPAFGFGSSQA
ATFMSPGFPVNNSSSDNAQNFSFKTNSGFAAASSGSPAGFGSSPAFGAAASTSSGISTSAPAFGFGKPEVTSAAS
FSFKSPAASSFGSPGFSGLPASLATGPVRAPVAPAFGGGSSVAGFGSPGSHSHTAFSKPSSDTFGNSSISTSLSA
SSSIIATDNVLFTPRDKLTVEELEQFQSKKFTLGKIPLKPPPLELLNVGAPGSAGSAAGSGMASNDYTQQATQSY
GAYPTQPGQGYSQQSSQPYGQQSYSGYSQSTDTSGYGQSSYSSYGQSQNTGYGTQSTPQGYGSTGGYGSSQSSQS
SYGQQSSYPGYGQQPAPSSTSGSYGSSSQSSSYGQPQSGSYSQQPSYGGQQQSYGQQQSYNPPQGYGQQNQYNSS
SGGGGGGGGGGNYGQDQSSMSSGGGSGGGYGNQDQSGGGGSGGYGQQDRGGRGRGGSGGGGGGGGGGYNRSSGGY
EPRGRGGGRGGRGGMGGSDRGGFNKFGGPRDQGSRHDSEQDNSDNNTIFVQGLGENVTIESVADYFKQIGIIKTN
KKTGQPMINLYTDRETGKLKGEATVSFDDPPSAKAAIDWFDGKEFSGNPIKVSFATRRADFNRGGGNGRGGRGRG
GPMGRGGYGGGGSGGGGRGGFPSGGGGGGGQQRAGDWKCPNPTCENMNFSWRNECNQCKAPKPDGPGGGPGGSHM
GGNYGDDRRGGRGGAIAGADYKDDDDKGAPGSAGSAAGSGACPVPLQLPPLERLTLDDKKPLNTLISATGLWMSR
TGTIHKIKHHEVSRSKIYIEMACGDHLVVNNSRSSRTARALRHHKYRKTCKRCRVSDEDLNKFLTKANEDQTSVK
VKVVSAPTRTKKAMPKSVARAPKPLENTEAAQAQPSGSKFSPAIPVSTQESVSVPASVSTSISSISTGATASALV
KGNTNPITSMSAPVQASAPALTKSQTDRLEVLLNPKDEISLNSGKPFRELESELLSRRKKDLQQIYAEERENYLG
KLEREITRFFVDRGFLEIKSPILIPLEYIERMGIDNDTELSKQIFRVDKNFCLRPMLAPNLANYLRKLDRALPDP
IKIFEIGPCYRKESDGKEHLEEFTMLNFCQMGSGCTRENLESIITDFLNHLGIDFKIVGDSCMVFGDTLDVMHGD
LELSSAVVGPIPLDREWGIDKPWIGAGFGLERLLKVKHDFKNIKRAARSESYYNGISTNL* (SEQ ID NO:
370)

CG1::FUS::MCP::PylRS(AF)
DNA:
ATGGCCATTTGTCAATTCTTCCTTCAAGGCCGGTGCCGCTTTGGAGATCGGTGCTGGAACGAACATCCCGGTGCT
AGGGGTGCAGGAGGAGGACGGCAGCAACCGCAGCAGCAGCCTTCAGGTAATAATAGACGTGGATGGAATACAACT
AGCCAGAGATATTCCAATGTCATCCAGCCATCCAGTTTCTCCAAATCCACACCATGGGGGGGCAGCAGAGATCAA
GAAAAGCCATATTTCAGTTCTTTTGATTCTGGAGCTTCAACTAACAGGAAGGAAGGCTTTGGATTGTCTGAGAAC
CCATTTGCTTCACTTAGTCCTGATGAGCAGAAAGATGAAAAGAAACTTCTGGAAGGAATTGTAAAAGATATGGAG
GTTTGGGAATCATCAGGGCAGTGGATGTTTTCTGTTTATTCACCAGTGAAAAAGAAACCTAATATTTCAGGTTTT
ACAGACATTTCACCAGAGGAATTGAGGCTTGAATACCATAACTTCTTAACCAGCAATAACTTACAGAGTTATCTA
AATTCTGTCCAACGTTTAATAAATCAATGGAGGAACAGGGTAAATGAACTGAAAAGTCTAAATATATCAACTAAA
GTAGCTTTGCTCTCTGATGTAAAGGATGGAGTAAATCAAGCAGCACCTGCATTTGGATTTGGCAGCAGTCAAGCA
GCAACATTTATGTCGCCAGGCTTTCCAGTCAATAACAGCAGCAGTGATAATGCTCAGAACTTTAGTTTTAAAACA
AACTCTGGATTTGCTGCTGCCTCTTCTGGAAGCCCTGCTGGTTTTGGGAGTTCCCCAGCATTTGGAGCTGCAGCC
TCTACCAGTTCAGGTATCTCTACTTCTGCTCCAGCTTTTGGATTTGGGAAGCCTGAAGTCACATCGGCTGCATCA
TTTTCATTCAAAAGCCCTGCAGCTTCCAGTTTTGGATCACCTGGATTTTCAGGACTTCCAGCTTCCTTGGCAACA
GGTCCTGTCAGAGCTCCAGTGGCCCCAGCCTTTGGAGGTGGCAGTTCTGTGGCTGGTTTTGGTAGTCCGGGCTCA
CATTCTCACACTGCTTTTTCTAAGCCATCCAGTGACACTTTTGGAAATAGCAGCATATCCACTTCTCTGTCAGCC
TCAAGCAGCATCATTGCAACAGATAATGTGTTATTCACACCCAGAGATAAACTAACAGTAGAAGAACTGGAACAA
TTTCAATCCAAGAAATTTACTCTGGGAAAAATTCCATTAAAGCCTCCACCTCTGGAACTTCTAAATGTTGGAGCA
CCCGGCTCCGCCGGCTCCGCCGCCGGCTCCGGCATGGCCTCAAACGATTATACCCAACAAGCAACCCAAAGCTAT
GGGGCCTACCCCACCCAGCCCGGGCAGGGCTATTCCCAGCAGAGCAGTCAGCCCTACGGACAGCAGAGTTACAGT

GGTTATAGCCAGTCCACGGACACTTCAGGATATGGCCAGAGCAGCTATTCTTCTTATGGCCAGAGCCAGAACACA
GGCTATGGAACTCAGTCAACTCCCCAGGGATATGGCTCGACTGGCGGCTATGGCAGTAGCCAGAGCTCCCAATCG
TCTTACGGGCAGCAGTCCTCCTACCCTGGCTATGGCCAGCAGCCAGCTCCCAGCAGCACCTCGGGAAGTTACGGT
AGCAGTTCTCAGAGCAGCAGCTATGGGCAGCCCCAGAGTGGGAGCTACAGCCAGCAGCCTAGCTATGGTGGACAG
CAGCAAAGCTATGGACAGCAGCAAAGCTATAATCCCCCTCAGGGCTATGGACAGCAGAACCAGTACAACAGCAGC
AGTGGTGGTGGAGGTGGAGGTGGAGGTGGAGGTAACTATGGCCAAGATCAATCCTCCATGAGTAGTGGTGGTGGC
AGTGGTGGCGGTTATGGCAATCAAGACCAGAGTGGTGGAGGTGGCAGCGGTGGCTATGGACAGCAGGACCGTGGA
GGCCGCGGCAGGGGTGGCAGTGGTGGCGGCGGCGGCGGCGGTGGTGGTTACAACCGCAGCAGTGGTGGCTAT
GAACCCAGAGGTCGTGGAGGTGGCCGTGGAGGCAGAGGTGGCATGGGCGGAAGTGACCGTGGTGGCTTCAATAAA
TTTGGTGGCCCTCGGGACCAAGGATCACGTCATGACTCCGAACAGGATAATTCAGACAACAACACCATCTTTGTG
CAAGGCCTGGGTGAGAATGTTACAATTGAGTCTGTGGCTGATTACTTCAAGCAGATTGGTATTATTAAGACAAAC
AAGAAAACGGGACAGCCCATGATTAATTTGTACACAGACAGGGAAACTGGCAAGCTGAAGGGAGAGGCAACGGTC
TCTTTTGATGACCCACCTTCAGCTAAAGCAGCTATTGACTGGTTTGATGGTAAAGAATTCTCCGGAAATCCTATC
AAGGTCTCATTTGCTACTCGCCGGGCAGACTTTAATCGGGGTGGTGGCAATGGTCGTGGAGGCCGAGGGCGAGGA
GGACCCATGGGCCGTGGAGGCTATGGAGGTGGTGGCAGTGGTGGTGGTGGCCGAGGAGGATTTCCCAGTGGAGGT
GGTGGCGGTGGAGGACAGCAGCGAGCTGGTGACTGGAAGTGTCCTAATCCCACCTGTGAGAATATGAACTTCTCT
TGGAGGAATGAATGCAACCAGTGTAAGGCCCCTAAACCAGATGGCCCAGGAGGGGGACCAGGTGGCTCTCACATG
GGGGGTAACTACGGGGATGATCGTCGTGGTGGCAGAGGTGGTGCGATCGCATATCCCTATGATGTGCCGGATTAT
GCTGGAGCACCAGGAAGTGCTGGTTCTGCTGCTGGTAGTGGAGCTTCTAACTTTACTCAGTTCGTTCTCGTCGAC
AATGGCGGAACTGGCGACGTGACTGTCGCCCCAAGCAACTTCGCTAACGGGATCGCTGAATGGATCAGCTCTAAC
TCGCGTTCACAGGCTTACAAAGTAACCTGTAGCGTTCGTCAGAGCTCTGCGCAGAATCGCAAATACACCATCAAA
GTCGAGGTGCCTAAAGGCGCCTGGCGTTCGTACTTAAATATGGAACTAACCATTCCAATTTTCGCCACGAATTCC
GACTGCGAGCTTATTGTTAAGGCAATGCAAGGTCTCCTAAAAGATGGAAACCCGATTCCCTCAGCAATCGCAGCA
AACTCCGGCATCTACGGCGCCGATTACAAGGACGATGATGACAAGGGAGCACCAGGAAGTGCTGGTTCTGCTGCT
GGTAGTGGAGCGTGCCCGGTGCCGCTGCAGCTGCCGCCGCTGGAACGCCTGACCCTGGATGATAAAAAACCGCTG
AATACCCTGATCTCTGCTACTGGTCTGTGGATGAGTCGTACCGGAACCATTCATAAAATCAAACACCACGAGGTT
AGCCGTTCGAAAATCTATATTGAGATGGCGTGTGGCGATCATCTGGTTGTGAACAATAGCCGCTCTTCTCGTACA
GCACGTGCACTGCGTCACCACAAATATCGTAAAACCTGTAAACGTTGCCGTGTGTCCGATGAGGATCTGAACAAA
TTCCTGACAAAAGCCAATGAGGACCAAACAAGCGTGAAAGTGAAAGTCGTTAGCGCTCCTACCCGTACTAAAAAA
GCAATGCCGAAATCCGTTGCTCGTGCCCCTAAACCACTGGAAAACACTGAAGCAGCACAGGCACAGCCGTCTGGA
AGCAAATTCTCTCCGGCCATTCCTGTTTCTACCCAGGAGTCCGTTTCTGTTCCAGCAAGTGTGAGCACCAGCATT
AGCAGTATTAGCACCGGTGCCACCGCTAGCGCCCTGGTTAAAGGCAATACCAATCCGATTACAAGCATGTCTGCC
CCGGTTCAAGCATCAGCTCCAGCACTGACAAAATCCCAAACCGATCGTCTGGAGGTTCTGCTGAATCCGAAAGAC
GAAATCAGCCTGAATTCCGGCAAACCGTTTCGTGAACTGGAGAGCGAACTGCTGTCACGTCGTAAAAAAGACCTG
CAACAAATCTATGCCGAAGAACGTGAGAACTATCTGGGGAAACTGGAACGTGAAATCACCCGCTTTTTCGTGGAT
CGTGGCTTTCTGGAGATCAAATCCCCGATTCTGATTCCTCTGGAGTATATCGAGCGTATGGGCATCGACAATGAT
ACCGAACTGAGCAAACAAATTTTCCGTGTGGATAAAAACTTCTGTCTGCGCCCTATGCTAGCACCAAATCTGGCT
AACTATCTGCGCAAACTGGACCGTGCCCTGCCTGATCCTATCAAAATCTTCGAGATCGGCCCGTGTTATCGTAAA
GAGTCCGACGGTAAAGAACATCTGGAGGAGTTTACCATGCTGAACTTTTGCCAAATGGGTTCAGGTTGTACTCGT
GAGAACCTGGAAAGCATCATCACCGATTTTCTGAACCACCTGGGCATTGACTTCAAAATTGTGGGCGACAGCTGT
ATGGTGTTTGGCGACACCCTGGATGTCATGCACGGCGACCTGGAACTGTCTAGTGCCGTTGTGGGCCCAATCCCG
CTGGATCGTGAGTGGGGTATCGACAAACCTTGGATCGGTGCGGGTTTTGGTCTGGAGCGTCTGCTGAAAGTAAAA
CACGACTTCAAGAACATCAAACGTGCTGCACGTTCCGAGTCCTATTACAATGGTATTTCTACTAACCTGTAA
(SEQ ID NO: 371)

Protein:
MAICQFFLQGRCRFGDRCWNEHPGARGAGGGRQQPQQQPSGNNRRGWNTTSQRYSNVIQPSSFSKSTPWGGSRDQ
EKPYFSSFDSGASTNRKEGFGLSENPFASLSPDEQKDEKKLLEGIVKDMEVWESSGQWMFSVYSPVKKKPNISGF
TDISPEELRLEYHNFLTSNNLQSYLNSVQRLINQWRNRVNELKSLNISTKVALLSDVKDGVNQAAPAFGFGSSQA
ATFMSPGFPVNNSSSDNAQNFSFKTNSGFAAASSGSPAGFGSSPAFGAAASTSSGISTSAPAFGFGKPEVTSAAS
FSFKSPAASSFGSPGFSGLPASLATGPVRAPVAPAFGGGSSVAGFGSPGSHSHTAFSKPSSDTFGNSSISTSLSA
SSSIIATDNVLFTPRDKLTVEELEQFQSKKFTLGKIPLKPPPLELLNVGAPGSAGSAAGSGMASNDYTQQATQSY
GAYPTQPGQGYSQQSSQPYGQQSYSGYSQSTDTSGYGQSSYSSYGQSQNTGYGTQSTPQGYGSTGGYGSSQSSQS
SYGQQSSYPGYGQQPAPSSTSGSYGSSSQSSSYGQPQSGSYSQQPSYGGQQQSYGQQQSYNPPQGYGQQNQYNSS
SGGGGGGGGGNYGQDQSSMSSGGGSGGGYGNQDQSGGGGSGGYGQQDRGGRGRGGSGGGGGGGGGYNRSSGGY
EPRGRGGGRGGRGGMGGSDRGGFNKFGGPRDQGSRHDSEQDNSDNNTIFVQGLGENVTIESVADYFKQIGIIKTN
KKTGQPMINLYTDRETGKLKGEATVSFDDPPSAKAAIDWFDGKEFSGNPIKVSFATRRADFNRGGGNGRGGRGRG
GPMGRGGYGGGGSGGGGRGGFPSGGGGGGGQQRAGDWKCPNPTCENMNFSWRNECNQCKAPKPDGPGGGPGGSHM

GGNYGDDRRGGRGGAIAYPYDVPDYAGAPGSAGSAAGSGASNFTQFVLVDNGGTGDVTVAPSNFANGIAEWISSN
SRSQAYKVTCSVRQSSAQNRKYTIKVEVPKGAWRSYLNMELTIPIFATNSDCELIVKAMQGLLKDGNPIPSAIAA
NSGIYGADYKDDDDKGAPGSAGSAAGSGACPVPLQLPPLERLTLDDKKPLNTLISATGLWMSRTGTIHKIKHHEV
SRSKIYIEMACGDHLVVNNSRSSRTARALRHHKYRKTCKRCRVSDEDLNKFLTKANEDQTSVKVKVVSAPTRTKK
AMPKSVARAPKPLENTEAAQAQPSGSKFSPAIPVSTQESVSVPASVSTSISSISTGATASALVKGNTNPITSMSA
PVQASAPALTKSQTDRLEVLLNPKDEISLNSGKPFRELESELLSRRKKDLQQIYAEERENYLGKLEREITRFFVD
RGFLEIKSPILIPLEYIERMGIDNDTELSKQIFRVDKNFCLRPMLAPNLANYLRKLDRALPDPIKIFEIGPCYRK
ESDGKEHLEEFTMLNFCQMGSGCTRENLESIITDFLNHLGIDFKIVGDSCMVFGDTLDVMHGDLELSSAVVGPIP
LDREWGIDKPWIGAGFGLERLLKVKHDFKNIKRAARSESYYNGISTNL* (SEQ ID NO: 372)

CMP-SaTr::PylRS(AF)
DNA:
ATGGCTGCCCCGAGAGACAATGTCACTTTATTATTCAAGTTATACTGCTTGGCAGTGATGACCCTGATGGCTGCA
GTCTATACCATAGCTTTAAGATACACAAGGACATCAGACAAAGAACTCTACTTTTCAACCACAGCCGTGTGTATC
ACAGAAGTTATAAAGTTATTGCTAAGTGTGGGAATTTTAGCTAAAGAAACTGGTAGTCTGGGTAGATTCAAAGCA
TCTTTAAGAGAAAATGTCTTGGGGAGCCCCAAGGAACTGTTGAAGTTAAGTGTGCCATCGTTAGTGTATGCTGTT
CAGAACAACATGGCTTTCCTAGCTCTTAGCAATCTGGATGCAGCAGTGTACCAGGTGACCTACCAGTTGAAGATT
CCGTGTACTGCTTTATGCACTGTTTTAATGTTAAACCGGACACTCAGCAAATTACAGTGGGTTTCAGTTTTTATG
CTGTGTGCTGGAGTTACGCTTGTACAGTGGAAACCAGCCCAAGCTACAAAAGTGGTGGTGGAACAAAATCCATTA
TTAGGGTTTGGCGCTATAGCTATTGCTGTATTGTGCTCAGGATTTGCAGGAGTATATTTTGAAAAAGTTTTAAAG
AGTTCAGATACTTCTCTTTGGGTGAGAAACATTCAAATGTATCTATCAGGGATTATTGTGACATTAGCTGGCGTC
TACTTGTCAGATGGAGCTGAAATTAAAGAAAAAGGATTTTTCTATGGTTACACATATTATGTCTGGTTTGTCATC
TTTCTTGCAAGTGTTGGTGGCCTCTACACTTCTGTTGTGGTTAAGTACACAGACAATATCATGAAAGGCTTTTCT
GCAGCAGCGGCCATTGTCCTTTCCACCATTGCTTCAGTAATGCTGTTTGGATTACAGATAACACTTACCTTTGCC
CTGGGTACTCTTCTTGTATGTGTTTCCATATATCTCTATGGATTACCCAGACAAGACACTACATCCATCCAACAA
GGAGAAACAGCTTCAAAGGAGAGAGTTATTGGTGTGGGCGCCGATTACAAGGACGATGATGACAAGGGAGCACCA
GGAAGTGCTGGTTCTGCTGCTGGTAGTGGAGCGTGCCCGGTGCCGCTGCAGCTGCCGCCGCTGGAACGCCTGACC
CTGGATGATAAAAAACCGCTGAATACCCTGATCTCTGCTACTGGTCTGTGGATGAGTCGTACCGGAACCATTCAT
AAAATCAAACACCACGAGGTTAGCCGTTCGAAAATCTATATTGAGATGGCGTGTGGCGATCATCTGGTTGTGAAC
AATAGCCGCTCTTCTCGTACAGCACGTGCACTGCGTCACCACAAATATCGTAAAACCTGTAAACGTTGCCGTGTG
TCCGATGAGGATCTGAACAAATTCCTGACAAAAGCCAATGAGGACCAAACAAGCGTGAAAGTGAAAGTCGTTAGC
GCTCCTACCCGTACTAAAAAAGCAATGCCGAAATCCGTTGCTCGTGCCCCTAAACCACTGGAAAACACTGAAGCA
GCACAGGCACAGCCGTCTGGAAGCAAATTCTCTCCGGCCATTCCTGTTTCTACCCAGGAGTCCGTTTCTGTTCCA
GCAAGTGTGAGCACCAGCATTAGCAGTATTAGCACCGGTGCCACCGCTAGCGCCCTGGTTAAAGGCAATACCAAT
CCGATTACAAGCATGTCTGCCCCGGTTCAAGCATCAGCTCCAGCACTGACAAAATCCCAAACCGATCGTCTGGAG
GTTCTGCTGAATCCGAAAGACGAAATCAGCCTGAATTCCGGCAAACCGTTTCGTGAACTGGAGAGCGAACTGCTG
TCACGTCGTAAAAAAGACCTGCAACAAATCTATGCCGAAGAACGTGAGAACTATCTGGGGAAACTGGAACGTGAA
ATCACCCGCTTTTTCGTGGATCGTGGCTTTCTGGAGATCAAATCCCCGATTCTGATTCCTCTGGAGTATATCGAG
CGTATGGGCATCGACAATGATACCGAACTGAGCAAACAAATTTTCCGTGTGGATAAAAACTTCTGTCTGCGCCCT
ATGCTAGCACCAAATCTGGCTAACTATCTGCGCAAACTGGACCGTGCCCTGCCTGATCCTATCAAAATCTTCGAG
ATCGGCCCGTGTTATCGTAAAGAGTCCGACGGTAAAGAACATCTGGAGGAGTTTACCATGCTGAACTTTTGCCAA
ATGGGTTCAGGTTGTACTCGTGAGAACCTGGAAAGCATCATCACCGATTTTCTGAACCACCTGGGCATTGACTTC
AAAATTGTGGGCGACAGCTGTATGGTGTTTGGCGACACCCTGGATGTCATGCACGGCGACCTGGAACTGTCTAGT
GCCGTTGTGGGCCCAATCCCGCTGGATCGTGAGTGGGGTATCGACAAACCTTGGATCGGTGCGGGTTTTGGTCTG
GAGCGTCTGCTGAAAGTAAAACACGACTTCAAGAACATCAAACGTGCTGCACGTTCCGAGTCCTATTACAATGGT
ATTTCTACTAACCTGTAA (SEQ ID NO: 373)

Protein:
MAAPRDNVTLLFKLYCLAVMTLMAAVYTIALRYTRTSDKELYFSTTAVCITEVIKLLLSVGILAKETGSLGRFKA
SLRENVLGSPKELLKLSVPSLVYAVQNNMAFLALSNLDAAVYQVTYQLKIPCTALCTVLMLNRTLSKLQWVSVFM
LCAGVTLVQWKPAQATKVVVEQNPLLGFGAIAIAVLCSGFAGVYFEKVLKSSDTSLWVRNIQMYLSGIIVTLAGV
YLSDGAEIKEKGFFYGYTYYVWFVIFLASVGGLYTSVVVKYTDNIMKGFSAAAAIVLSTIASVMLFGLQITLTFA
LGTLLVCVSIYLYGLPRQDTTSIQQGETASKERVIGVGADYKDDDDKGAPGSAGSAAGSGACPVPLQLPPLERLT
LDDKKPLNTLISATGLWMSRTGTIHKIKHHEVSRSKIYIEMACGDHLVVNNSRSSRTARALRHHKYRKTCKRCRV
SDEDLNKFLTKANEDQTSVKVKVVSAPTRTKKAMPKSVARAPKPLENTEAAQAQPSGSKFSPAIPVSTQESVSVP
ASVSTSISSISTGATASALVKGNTNPITSMSAPVQASAPALTKSQTDRLEVLLNPKDEISLNSGKPFRELESELL
SRRKKDLQQIYAEERENYLGKLEREITRFFVDRGFLEIKSPILIPLEYIERMGIDNDTELSKQIFRVDKNFCLRP

MLAPNLANYLRKLDRALPDPIKIFEIGPCYRKESDGKEHLEEFTMLNFCQMGSGCTRENLESIITDFLNHLGIDF
KIVGDSCMVFGDTLDVMHGDLELSSAVVGPIPLDREWGIDKPWIGAGFGLERLLKVKHDFKNIKRAARSESYYNG
ISTNL* (SEQ ID NO: 374)

CMP-SaTr::PylRS (AA)
DNA:
ATGGCTGCCCCGAGAGACAATGTCACTTTATTATTCAAGTTATACTGCTTGGCAGTGATGACCCTGATGGCTGCA
GTCTATACCATAGCTTTAAGATACACAAGGACATCAGACAAAGAACTCTACTTTTCAACCACAGCCGTGTGTATC
ACAGAAGTTATAAAGTTATTGCTAAGTGTGGGAATTTTAGCTAAAGAAACTGGTAGTCTGGGTAGATTCAAAGCA
TCTTTAAGAGAAAATGTCTTGGGGAGCCCCAAGGAACTGTTGAAGTTAAGTGTGCCATCGTTAGTGTATGCTGTT
CAGAACAACATGGCTTTCCTAGCTCTTAGCAATCTGGATGCAGCAGTGTACCAGGTGACCTACCAGTTGAAGATT
CCGTGTACTGCTTTATGCACTGTTTTAATGTTAAACCGGACACTCAGCAAATTACAGTGGGTTTCAGTTTTTATG
CTGTGTGCTGGAGTTACGCTTGTACAGTGGAAACCAGCCCAAGCTACAAAAGTGGTGGTGGAACAAAATCCATTA
TTAGGGTTTGGCGCTATAGCTATTGCTGTATTGTGCTCAGGATTTGCAGGAGTATATTTTGAAAAAGTTTTAAAG
AGTTCAGATACTTCTCTTTGGGTGAGAAACATTCAAATGTATCTATCAGGGATTATTGTGACATTAGCTGGCGTC
TACTTGTCAGATGGAGCTGAAATTAAAGAAAAAGGATTTTTCTATGGTTACACATATTATGTCTGGTTTGTCATC
TTTCTTGCAAGTGTTGGTGGCCTCTACACTTCTGTTGTGGTTAAGTACACAGACAATATCATGAAAGGCTTTTCT
GCAGCAGCGGCCATTGTCCTTTCCACCATTGCTTCAGTAATGCTGTTTGGATTACAGATAACACTTACCTTTGCC
CTGGGTACTCTTCTTGTATGTGTTTCCATATATCTCTATGGATTACCCAGACAAGACACTACATCCATCCAACAA
GGAGAAACAGCTTCAAAGGAGAGAGTTATTGGTGTGGGCGCCGATTACAAGGACGATGATGACAAGGGAGCACCA
GGAAGTGCTGGTTCTGCTGCTGGTAGTGGAGCGTGCCCGGTGCCGCTGCAGCTGCCGCCGCTGGAACGCCTGACC
CTGGATGACAAAAAACCGCTGAATACCCTGATCTCTGCTACTGGTCTGTGGATGAGTCGTACCGGAACCATTCAT
AAAATCAAACACCACGAGGTTAGCCGTTCGAAAATCTATATTGAGATGGCGTGTGGCGATCATCTGGTTGTGAAC
AATAGCCGCTCTTCTCGTACAGCACGTGCACTGCGTCACCACAAATATCGTAAAACCTGTAAACGTTGCCGTGTG
TCCGATGAGGATCTGAACAAATTCCTGACAAAAGCCAATGAGGACCAAACAAGCGTGAAAGTGAAAGTCGTTAGC
GCTCCTACCCGTACTAAAAAAGCAATGCCGAAATCCGTTGCTCGTGCCCCTAAACCACTGGAAAACACTGAAGCA
GCACAGGCACAGCCGTCTGGAAGCAAATTCTCTCCGGCCATTCCTGTTTCTACCCAGGAGTCCGTTTCTGTTCCA
GCAAGTGTGAGCACCAGCATTAGCAGTATTAGCACCGGTGCCACCGCTAGCGCCCTGGTTAAAGGCAATACCAAT
CCGATTACAAGCATGTCTGCCCCGGTTCAAGCATCAGCTCCAGCACTGACAAAATCCCAAACCGATCGTCTGGAG
GTTCTGCTGAATCCGAAAGACGAAATCAGCCTGAATTCCGGCAAACCGTTTCGTGAACTGGAGAGCGAACTGCTG
TCACGTCGTAAAAAAGACCTGCAACAAATCTATGCCGAAGAACGTGAGAACTATCTGGGGAAACTGGAACGTGAA
ATCACCCGCTTTTTCGTGGATCGTGGCTTTCTGGAGATCAAATCCCCGATTCTGATTCCTCTGGAGTATATCGAG
CGTATGGGCATCGACAATGATACCGAACTGAGCAAACAAATTTTCCGTGTGGATAAAAACTTCTGTCTGCGCCCT
ATGCTGGCACCAAATCTGTATAACTATCTGCGCAAACTGGACCGTGCCCTGCCTGATCCTATCAAAATCTTCGAG
ATCGGCCCGTGTTATCGTAAAGAGTCCGACGGTAAAGAACATCTGGAGGAGTTTACCATGCTGGCCTTTGCCCAA
ATGGGTTCAGGTTGTACTCGTGAGAACCTGGAAAGCATCATCACCGATTTTCTGAACCACCTGGGCATTGACTTC
AAAATTGTGGGCGACAGCTGTATGGTGTATGGCGACACCCTGGATGTCATGCACGGCGACCTGGAACTGTCTAGT
GCCGTTGTTGGACCAATTCCGCTGGACCGTGAGTGGGGTATCGACAAACCGTGGATCGGAGCAGGATTCGGTCTG
GAACGCCTGCTGAAAGTGAAACACGACTTCAAAAACATCAAACGTGCCGCCCGTTCTGAATCGTATTATAACGGG
ATCTCTACGAACCTGTAA (SEQ ID NO: 375)

Protein:
MAAPRDNVTLLFKLYCLAVMTLMAAVYTIALRYTRTSDKELYFSTTAVCITEVIKLLLSVGILAKETGSLGRFKA
SLRENVLGSPKELLKLSVPSLVYAVQNNMAFLALSNLDAAVYQVTYQLKIPCTALCTVLMLNRTLSKLQWVSVFM
LCAGVTLVQWKPAQATKVVVEQNPLLGFGAIAIAVLCSGFAGVYFEKVLKSSDTSLWVRNIQMYLSGIIVTLAGV
YLSDGAEIKEKGFFYGYTYYVWFVIFLASVGGLYTSVVVKYTDNIMKGFSAAAIVLSTIASVMLFGLQITLTFA
LGTLLVCVSIYLYGLPRQDTTSIQQGETASKERVIGVGADYKDDDDKGAPGSAGSAAGSGACPVPLQLPPLERLT
LDDKKPLNTLISATGLWMSRTGTIHKIKHHEVSRSKIYIEMACGDHLVVNNSRSSRTARALRHHKYRKTCKRCRV
SDEDLNKFLTKANEDQTSVKVKVVSAPTRTKKAMPKSVARAPKPLENTEAAQAQPSGSKFSPAIPVSTQESVSVP
ASVSTSISSISTGATASALVKGNTNPITSMSAPVQASAPALTKSQTDRLEVLLNPKDEISLNSGKPFRELESELL
SRRKKDLQQIYAEERENYLGKLEREITRFFVDRGFLEIKSPILIPLEYIERMGIDNDTELSKQIFRVDKNFCLRP
MLAPNLYNYLRKLDRALPDPIKIFEIGPCYRKESDGKEHLEEFTMLAFAQMGSGCTRENLESIITDFLNHLGIDF
KIVGDSCMVYGDTLDVMHGDLELSSAVVGPIPLDREWGIDKPWIGAGFGLERLLKVKHDFKNIKRAARSESYYNG
ISTNL* (SEQ ID NO: 376)

CMP-SaTr::PylRS(AAAF)
DNA:

ATGGCTGCCCCGAGAGACAATGTCACTTTATTATTCAAGTTATACTGCTTGGCAGTGATGACCCTGATGGCTGCA
GTCTATACCATAGCTTTAAGATACACAAGGACATCAGACAAAGAACTCTACTTTTCAACCACAGCCGTGTGTATC
ACAGAAGTTATAAAGTTATTGCTAAGTGTGGGAATTTTAGCTAAAGAAACTGGTAGTCTGGGTAGATTCAAAGCA
TCTTTAAGAGAAAATGTCTTGGGGAGCCCCAAGGAACTGTTGAAGTTAAGTGTGCCATCGTTAGTGTATGCTGTT
CAGAACAACATGGCTTTCCTAGCTCTTAGCAATCTGGATGCAGCAGTGTACCAGGTGACCTACCAGTTGAAGATT
CCGTGTACTGCTTTATGCACTGTTTTAATGTTAAACCGGACACTCAGCAAATTACAGTGGGTTTCAGTTTTTATG
CTGTGTGCTGGAGTTACGCTTGTACAGTGGAAACCAGCCCAAGCTACAAAAGTGGTGGTGGAACAAAATCCATTA
TTAGGGTTTGGCGCTATAGCTATTGCTGTATTGTGCTCAGGATTTGCAGGAGTATATTTTGAAAAAGTTTTAAAG
AGTTCAGATACTTCTCTTTGGGTGAGAAACATTCAAATGTATCTATCAGGGATTATTGTGACATTAGCTGGCGTC
TACTTGTCAGATGGAGCTGAAATTAAAGAAAAAGGATTTTTCTATGGTTACACATATTATGTCTGGTTTGTCATC
TTTCTTGCAAGTGTTGGTGGCCTCTACACTTCTGTTGTGGTTAAGTACACAGACAATATCATGAAAGGCTTTTCT
GCAGCAGCGGCCATTGTCCTTTCCACCATTGCTTCAGTAATGCTGTTTGGATTACAGATAACACTTACCTTTGCC
CTGGGTACTCTTCTTGTATGTGTTTCCATATATCTCTATGGATTACCCAGACAAGACACTACATCCATCCAACAA
GGAGAAACAGCTTCAAAGGAGAGAGTTATTGGTGTGGGCGCCGATTACAAGGACGATGATGACAAGGGAGCACCA
GGAAGTGCTGGTTCTGCTGCTGGTAGTGGAGCGTGCCCGGTGCCGCTGCAGCTGCCGCCGCTGGAACGCCTGACC
CTGGATGATAAAAAACCGCTGAATACCCTGATCTCTGCTACTGGTCTGTGGATGAGTCGTACCGGAACCATTCAT
AAAATCAAACACCACGAGGTTAGCCGTTCGAAAATCTATATTGAGATGGCGTGTGGCGATCATCTGGTTGTGAAC
AATAGCCGCTCTTCTCGTACAGCACGTGCACTGCGTCACCACAAATATCGTAAAACCTGTAAACGTTGCCGTGTG
TCCGATGAGGATCTGAACAAATTCCTGACAAAAGCCAATGAGGACCAAACAAGCGTGAAAGTGAAAGTCGTTAGC
GCTCCTACCCGTACTAAAAAAGCAATGCCGAAATCCGTTGCTCGTGCCCCTAAACCACTGGAAAACACTGAAGCA
GCACAGGCACAGCCGTCTGGAAGCAAATTCTCTCCGGCCATTCCTGTTTCTACCCAGGAGTCCGTTTCTGTTCCA
GCAAGTGTGAGCACCAGCATTAGCAGTATTAGCACCGGTGCCACCGCTAGCGCCCTGGTTAAAGGCAATACCAAT
CCGATTACAAGCATGTCTGCCCCGGTTCAAGCATCAGCTCCAGCACTGACAAAATCCCAAACCGATCGTCTGGAG
GTTCTGCTGAATCCGAAAGACGAAATCAGCCTGAATTCCGGCAAACCGTTTCGTGAACTGGAGAGCGAACTGCTG
TCACGTCGTAAAAAAGACCTGCAACAAATCTATGCCGAAGAACGTGAGAACTATCTGGGGAAACTGGAACGTGAA
ATCACCCGCTTTTTCGTGGATCGTGGCTTTCTGGAGATCAAATCCCCGATTCTGATTCCTCTGGAGTATATCGAG
CGTATGGGCATCGACAATGATACCGAACTGAGCAAACAAATTTTCCGTGTGGATAAAAACTTCTGTCTGCGCCCT
ATGCTAGCACCAAATCTGGCTAACTATCTGCGCAAACTGGACCGTGCCCTGCCTGATCCTATCAAAATCTTCGAG
ATCGGCCCGTGTTATCGTAAAGAGTCCGACGGTAAAGAACATCTGGAGGAGTTTACCATGCTGGCCTTTGCCCAA
ATGGGTTCAGGTTGTACTCGTGAGAACCTGGAAAGCATCATCACCGATTTTCTGAACCACCTGGGCATTGACTTC
AAAATTGTGGGCGACAGCTGTATGGTGTTTGGCGACACCCTGGATGTCATGCACGGCGACCTGGAACTGTCTAGT
GCCGTTGTGGGCCCAATCCCGCTGGATCGTGAGTGGGGTATCGACAAACCTTGGATCGGTGCGGGTTTTGGTCTG
GAGCGTCTGCTGAAAGTAAAACACGACTTCAAGAACATCAAACGTGCTGCACGTTCCGAGTCCTATTACAATGGT
ATTTCTACTAACCTGTAA (SEQ ID NO: 377)

Protein:
MAAPRDNVTLLFKLYCLAVMTLMAAVYTIALRYTRTSDKELYFSTTAVCITEVIKLLLSVGILAKETGSLGRFKA
SLRENVLGSPKELLKLSVPSLVYAVQNNMAFLALSNLDAAVYQVTYQLKIPCTALCTVLMLNRTLSKLQWVSVFM
LCAGVTLVQWKPAQATKVVVEQNPLLGFGAIAIAVLCSGFAGVYFEKVLKSSDTSLWVRNIQMYLSGIIVTLAGV
YLSDGAEIKEKGFFYGYTYYVWFVIFLASVGGLYTSVVVKYTDNIMKGFSAAAAIVLSTIASVMLFGLQITLTFA
LGTLLVCVSIYLYGLPRQDTTSIQQGETASKERVIGVGADYKDDDDKGAPGSAGSAAGSGACPVPLQLPPLERLT
LDDKKPLNTLISATGLWMSRTGTIHKIKHHEVSRSKIYIEMACGDHLVVNNSRSSRTARALRHHKYRKTCKRCRV
SDEDLNKFLTKANEDQTSVKVKVVSAPTRTKKAMPKSVARAPKPLENTEAAQAQPSGSKFSPAIPVSTQESVSVP
ASVSTSISSISTGATASALVKGNTNPITSMSAPVQASAPALTKSQTDRLEVLLNPKDEISLNSGKPFRELESELL
SRRKKDLQQIYAEERENYLGKLEREITRFFVDRGFLEIKSPILIPLEYIERMGIDNDTELSKQIFRVDKNFCLRP
MLAPNLANYLRKLDRALPDPIKIFEIGPCYRKESDGKEHLEEFTMLAFAQMGSGCTRENLESIITDFLNHLGIDF
KIVGDSCMVFGDTLDVMHGDLELSSAVVGPIPLDREWGIDKPWIGAGFGLERLLKVKHDFKNIKRAARSESYYNG
ISTNL* (SEQ ID NO: 378)

CMP-SaTr::FUS::PylRS(AA)
DNA:
ATGGCTGCCCCGAGAGACAATGTCACTTTATTATTCAAGTTATACTGCTTGGCAGTGATGACCCTGATGGCTGCA
GTCTATACCATAGCTTTAAGATACACAAGGACATCAGACAAAGAACTCTACTTTTCAACCACAGCCGTGTGTATC
ACAGAAGTTATAAAGTTATTGCTAAGTGTGGGAATTTTAGCTAAAGAAACTGGTAGTCTGGGTAGATTCAAAGCA
TCTTTAAGAGAAAATGTCTTGGGGAGCCCCAAGGAACTGTTGAAGTTAAGTGTGCCATCGTTAGTGTATGCTGTT
CAGAACAACATGGCTTTCCTAGCTCTTAGCAATCTGGATGCAGCAGTGTACCAGGTGACCTACCAGTTGAAGATT
CCGTGTACTGCTTTATGCACTGTTTTAATGTTAAACCGGACACTCAGCAAATTACAGTGGGTTTCAGTTTTTATG

CTGTGTGCTGGAGTTACGCTTGTACAGTGGAAACCAGCCCAAGCTACAAAAGTGGTGGTGGAACAAAATCCATTA
TTAGGGTTTGGCGCTATAGCTATTGCTGTATTGTGCTCAGGATTTGCAGGAGTATATTTTGAAAAAGTTTTAAAG
AGTTCAGATACTTCTCTTTGGGTGAGAAACATTCAAATGTATCTATCAGGGATTATTGTGACATTAGCTGGCGTC
TACTTGTCAGATGGAGCTGAAATTAAAGAAAAAGGATTTTTCTATGGTTACACATATTATGTCTGGTTTGTCATC
TTTCTTGCAAGTGTTGGTGGCCTCTACACTTCTGTTGTGGTTAAGTACACAGACAATATCATGAAAGGCTTTTCT
GCAGCAGCGGCCATTGTCCTTTCCACCATTGCTTCAGTAATGCTGTTTGGATTACAGATAACACTTACCTTTGCC
CTGGGTACTCTTCTTGTATGTGTTTCCATATATCTCTATGGATTACCCAGACAAGACACTACATCCATCCAACAA
GGAGAAACAGCTTCAAAGGAGAGAGTTATTGGTGTGGGAGCACCCGGCTCCGCCGGCTCCGCCGCCGGCTCCGGC
ATGGCCTCAAACGATTATACCCAACAAGCAACCCAAAGCTATGGGGCCTACCCCACCCAGCCCGGGCAGGGCTAT
TCCCAGCAGAGCAGTCAGCCCTACGGACAGCAGAGTTACAGTGGTTATAGCCAGTCCACGGACACTTCAGGATAT
GGCCAGAGCAGCTATTCTTCTTATGGCCAGAGCCAGAACACAGGCTATGGAACTCAGTCAACTCCCCAGGGATAT
GGCTCGACTGGCGGCTATGGCAGTAGCCAGAGCTCCCAATCGTCTTACGGGCAGCAGTCCTCCTACCCTGGCTAT
GGCCAGCAGCCAGCTCCCAGCAGCACCTCGGGAAGTTACGGTAGCAGTTCTCAGAGCAGCAGCTATGGGCAGCCC
CAGAGTGGGAGCTACAGCCAGCAGCCTAGCTATGGTGGACAGCAGCAAAGCTATGGACAGCAGCAAAGCTATAAT
CCCCCTCAGGGCTATGGACAGCAGAACCAGTACAACAGCAGCAGTGGTGGTGGAGGTGGAGGTGGAGGTGGAGGT
AACTATGGCCAAGATCAATCCTCCATGAGTAGTGGTGGTGGCAGTGGTGGCGGTTATGGCAATCAAGACCAGAGT
GGTGGAGGTGGCAGCGGTGGCTATGGACAGCAGGACCGTGGAGGCCGCGGCAGGGGTGGCAGTGGTGGCGGCGGC
GGCGGCGGCGGTGGTGGTTACAACCGCAGCAGTGGTGGCTATGAACCCAGAGGTCGTGGAGGTGGCCGTGGAGGC
AGAGGTGGCATGGGCGGAAGTGACCGTGGTGGCTTCAATAAATTTGGTGGCCCTCGGGACCAAGGATCACGTCAT
GACTCCGAACAGGATAATTCAGACAACAACACCATCTTTGTGCAAGGCCTGGGTGAGAATGTTACAATTGAGTCT
GTGGCTGATTACTTCAAGCAGATTGGTATTATTAAGACAAACAAGAAAACGGGACAGCCCATGATTAATTTGTAC
ACAGACAGGGAAACTGGCAAGCTGAAGGGAGAGGCAACGGTCTCTTTTGATGACCCACCTTCAGCTAAAGCAGCT
ATTGACTGGTTTGATGGTAAAGAATTCTCCGGAAATCCTATCAAGGTCTCATTTGCTACTCGCCGGGCAGACTTT
AATCGGGGTGGTGGCAATGGTCGTGGAGGCCGAGGGCGAGGAGGACCCATGGGCCGTGGAGGCTATGGAGGTGGT
GGCAGTGGTGGTGGTGGCCGAGGAGGATTTCCCAGTGGAGGTGGTGGCGGTGGAGGACAGCAGCGAGCTGGTGAC
TGGAAGTGTCCTAATCCCACCTGTGAGAATATGAACTTCTCTTGGAGGAATGAATGCAACCAGTGTAAGGCCCCT
AAACCAGATGGCCCAGGAGGGGGACCAGGTGGCTCTCACATGGGGGGTAACTACGGGGATGATCGTCGTGGTGGC
AGAGGTGGTGCGATCGCAGGCGCCGATTACAAGGACGATGATGACAAGGGAGCACCAGGAAGTGCTGGTTCTGCT
GCTGGTAGTGGAGCGTGCCCGGTGCCGCTGCAGCTGCCGCCGCTGGAACGCCTGACCCTGGATGACAAAAAACCG
CTGAATACCCTGATCTCTGCTACTGGTCTGTGGATGAGTCGTACCGGAACCATTCATAAAATCAAACACCACGAG
GTTAGCCGTTCGAAAATCTATATTGAGATGGCGTGTGGCGATCATCTGGTTGTGAACAATAGCCGCTCTTCTCGT
ACAGCACGTGCACTGCGTCACCACAAATATCGTAAAACCTGTAAACGTTGCCGTGTGTCCGATGAGGATCTGAAC
AAATTCCTGACAAAAGCCAATGAGGACCAAACAAGCGTGAAAGTGAAAGTCGTTAGCGCTCCTACCCGTACTAAA
AAAGCAATGCCGAAATCCGTTGCTCGTGCCCCTAAACCACTGGAAAACACTGAAGCAGCACAGGCACAGCCGTCT
GGAAGCAAATTCTCTCCGGCCATTCCTGTTTCTACCCAGGAGTCCGTTTCTGTTCCAGCAAGTGTGAGCACCAGC
ATTAGCAGTATTAGCACCGGTGCCACCGCTAGCGCCCTGGTTAAAGGCAATACCAATCCGATTACAAGCATGTCT
GCCCCGGTTCAAGCATCAGCTCCAGCACTGACAAAATCCCAAACCGATCGTCTGGAGGTTCTGCTGAATCCGAAA
GACGAAATCAGCCTGAATTCCGGCAAACCGTTTCGTGAACTGGAGAGCGAACTGCTGTCACGTCGTAAAAAAGAC
CTGCAACAAATCTATGCCGAAGAACGTGAGAACTATCTGGGGAAACTGGAACGTGAAATCACCCGCTTTTTCGTG
GATCGTGGCTTTCTGGAGATCAAATCCCCGATTCTGATTCCTCTGGAGTATATCGAGCGTATGGGCATCGACAAT
GATACCGAACTGAGCAAACAAATTTTCCGTGTGGATAAAAACTTCTGTCTGCGCCCTATGCTGGCACCAAATCTG
TATAACTATCTGCGCAAACTGGACCGTGCCCTGCCTGATCCTATCAAAATCTTCGAGATCGGCCCGTGTTATCGT
AAAGAGTCCGACGGTAAAGAACATCTGGAGGAGTTTACCATGCTGGCCTTTGCCCAAATGGGTTCAGGTTGTACT
CGTGAGAACCTGGAAAGCATCATCACCGATTTTCTGAACCACCTGGGCATTGACTTCAAAATTGTGGGCGACAGC
TGTATGGTGTATGGCGACACCCTGGATGTCATGCACGGCGACCTGGAACTGTCTAGTGCCGTTGTTGGACCAATT
CCGCTGGACCGTGAGTGGGGTATCGACAAACCGTGGATCGGAGCAGGATTCGGTCTGGAACGCCTGCTGAAAGTG
AAACACGACTTCAAAAACATCAAACGTGCCGCCCGTTCTGAATCGTATTATAACGGGATCTCTACGAACCTGTAA
(SEQ ID NO: 379)

Protein:
MAAPRDNVTLLFKLYCLAVMTLMAAVYTIALRYTRTSDKELYFSTTAVCITEVIKLLLSVGILAKETGSLGRFKA
SLRENVLGSPKELLKLSVPSLVYAVQNNMAFLALSNLDAAVYQVTYQLKIPCTALCTVLMLNRTLSKLQWVSVFM
LCAGVTLVQWKPAQATKVVVEQNPLLGFGAIAIAVLCSGFAGVYFEKVLKSSDTSLWVRNIQMYLSGIIVTLAGV
YLSDGAEIKEKGFFYGYTYYVWFVIFLASVGGLYTSVVVKYTDNIMKGFSAAAAIVLSTIASVMLFGLQITLTFA
LGTLLVCVSIYLYGLPRQDTTSIQQGETASKERVIGVGAPGSAGSAAGSGMASNDYTQQATQSYGAYPTQPGQGY
SQQSSQPYGQQSYSGYSQSTDTSGYGQSSYSSYGQSQNTGYGTQSTPQGYGSTGGYGSSQSSQSSYGQQSSYPGY
GQQPAPSSTSGSYGSSSQSSSYGQPQSGSYSQQPSYGGQQQSYGQQQSYNPPQGYGQQNQYNSSSGGGGGGGGGG
NYGQDQSSMSSGGGSGGGYGNQDQSGGGGSGGYGQQDRGGRGRGGSGGGGGGGGGYNRSSGGYEPRGRGGGRGG

RGGMGGSDRGGFNKFGGPRDQGSRHDSEQDNSDNNTIFVQGLGENVTIESVADYFKQIGIIKTNKKTGQPMINLY
TDRETGKLKGEATVSFDDPPSAKAAIDWFDGKEFSGNPIKVSFATRRADFNRGGGNGRGGRGRGGPMGRGGYGGG
GSGGGGRGGFPSGGGGGGGQQRAGDWKCPNPTCENMNFSWRNECNQCKAPKPDGPGGGPGGSHMGGNYGDDRRGG
RGGAIAGADYKDDDDKGAPGSAGSAAGSGACPVPLQLPPLERLTLDDKKPLNTLISATGLWMSRTGTIHKIKHHE
VSRSKIYIEMACGDHLVVNNSRSSRTARALRHHKYRKTCKRCRVSDEDLNKFLTKANEDQTSVKVKVVSAPTRTK
KAMPKSVARAPKPLENTEAAQAQPSGSKFSPAIPVSTQESVSVPASVSTSISSISTGATASALVKGNTNPITSMS
APVQASAPALTKSQTDRLEVLLNPKDEISLNSGKPFRELESELLSRRKKDLQQIYAEERENYLGKLEREITRFFV
DRGFLEIKSPILIPLEYIERMGIDNDTELSKQIFRVDKNFCLRPMLAPNLYNYLRKLDRALPDPIKIFEIGPCYR
KESDGKEHLEEFTMLAFAQMGSGCTRENLESIITDFLNHLGIDFKIVGDSCMVYGDTLDVMHGDLELSSAVVGPI
PLDREWGIDKPWIGAGFGLERLLKVKHDFKNIKRAARSESYYNGISTNL* (SEQ ID NO: 380)

CMP-SaTr::FUS::PylRS(AAAF)
DNA:
ATGGCTGCCCCGAGAGACAATGTCACTTTATTATTCAAGTTATACTGCTTGGCAGTGATGACCCTGATGGCTGCA
GTCTATACCATAGCTTTAAGATACACAAGGACATCAGACAAAGAACTCTACTTTTCAACCACAGCCGTGTGTATC
ACAGAAGTTATAAAGTTATTGCTAAGTGTGGGAATTTTAGCTAAAGAAACTGGTAGTCTGGGTAGATTCAAAGCA
TCTTTAAGAGAAAATGTCTTGGGGAGCCCCAAGGAACTGTTGAAGTTAAGTGTGCCATCGTTAGTGTATGCTGTT
CAGAACAACATGGCTTTCCTAGCTCTTAGCAATCTGGATGCAGCAGTGTACCAGGTGACCTACCAGTTGAAGATT
CCGTGTACTGCTTTATGCACTGTTTTAATGTTAAACCGGACACTCAGCAAATTACAGTGGGTTTCAGTTTTTATG
CTGTGTGCTGGAGTTACGCTTGTACAGTGGAAACCAGCCCAAGCTACAAAAGTGGTGGTGGAACAAAATCCATTA
TTAGGGTTTGGCGCTATAGCTATTGCTGTATTGTGCTCAGGATTTGCAGGAGTATATTTTGAAAAAGTTTTAAAG
AGTTCAGATACTTCTCTTTGGGTGAGAAACATTCAAATGTATCTATCAGGGATTATTGTGACATTAGCTGGCGTC
TACTTGTCAGATGGAGCTGAAATTAAAGAAAAAGGATTTTTCTATGGTTACACATATTATGTCTGGTTTGTCATC
TTTCTTGCAAGTGTTGGTGGCCTCTACACTTCTGTTGTGGTTAAGTACACAGACAATATCATGAAAGGCTTTTCT
GCAGCAGCGGCCATTGTCCTTTCCACCATTGCTTCAGTAATGCTGTTTGGATTACAGATAACACTTACCTTTGCC
CTGGGTACTCTTCTTGTATGTGTTTCCATATATCTCTATGGATTACCCAGACAAGACACTACATCCATCCAACAA
GGAGAAACAGCTTCAAAGGAGAGAGTTATTGGTGTGGGAGCACCCGGCTCCGCCGGCTCCGCCGCCGGCTCCGGC
ATGGCCTCAAACGATTATACCCAACAAGCAACCCAAAGCTATGGGGCCTACCCCACCCAGCCCGGGCAGGGCTAT
TCCCAGCAGAGCAGTCAGCCCTACGGACAGCAGAGTTACAGTGGTTATAGCCAGTCCACGGACACTTCAGGATAT
GGCCAGAGCAGCTATTCTTCTTATGGCCAGAGCCAGAACACAGGCTATGGAACTCAGTCAACTCCCCAGGGATAT
GGCTCGACTGGCGGCTATGGCAGTAGCCAGAGCTCCCAATCGTCTTACGGGCAGCAGTCCTCCTACCCTGGCTAT
GGCCAGCAGCCAGCTCCCAGCAGCACCTCGGGAAGTTACGGTAGCAGTTCTCAGAGCAGCAGCTATGGGCAGCCC
CAGAGTGGGAGCTACAGCCAGCAGCCTAGCTATGGTGGACAGCAGCAAAGCTATGGACAGCAGCAAAGCTATAAT
CCCCCTCAGGGCTATGGACAGCAGAACCAGTACAACAGCAGCAGTGGTGGTGGAGGTGGAGGTGGAGGTGGAGGT
AACTATGGCCAAGATCAATCCTCCATGAGTAGTGGTGGTGGCAGTGGTGGCGGTTATGGCAATCAAGACCAGAGT
GGTGGAGGTGGCAGCGGTGGCTATGGACAGCAGGACCGTGGAGGCCGCGGCAGGGGTGGCAGTGGTGGCGGCGGC
GGCGGCGGCGGTGGTGGTTACAACCGCAGCAGTGGTGGCTATGAACCCAGAGGTCGTGGAGGTGGCCGTGGAGGC
AGAGGTGGCATGGGCGGAAGTGACCGTGGTGGCTTCAATAAATTTGGTGGCCCTCGGGACCAAGGATCACGTCAT
GACTCCGAACAGGATAATTCAGACAACAACACCATCTTTGTGCAAGGCCTGGGTGAGAATGTTACAATTGAGTCT
GTGGCTGATTACTTCAAGCAGATTGGTATTATTAAGACAAACAAGAAAACGGGACAGCCCATGATTAATTTGTAC
ACAGACAGGGAAACTGGCAAGCTGAAGGGAGAGGCAACGGTCTCTTTTGATGACCCACCTTCAGCTAAAGCAGCT
ATTGACTGGTTTGATGGTAAAGAATTCTCCGGAAATCCTATCAAGGTCTCATTTGCTACTCGCCGGGCAGACTTT
AATCGGGGTGGTGGCAATGGTCGTGGAGGCCGAGGGCGAGGAGGACCCATGGGCCGTGGAGGCTATGGAGGTGGT
GGCAGTGGTGGTGGTGGCCGAGGAGGATTTCCCAGTGGAGGTGGTGGCGGTGGAGGACAGCAGCGAGCTGGTGAC
TGGAAGTGTCCTAATCCCACCTGTGAGAATATGAACTTCTCTTGGAGGAATGAATGCAACCAGTGTAAGGCCCCT
AAACCAGATGGCCCAGGAGGGGGACCAGGTGGCTCTCACATGGGGGGTAACTACGGGGATGATCGTCGTGGTGGC
AGAGGTGGTGCGATCGCAGGCGCCGATTACAAGGACGATGATGACAAGGGAGCACCAGGAAGTGCTGGTTCTGCT
GCTGGTAGTGGAGCGTGCCCGGTGCCGCTGCAGCTGCCGCCGCTGGAACGCCTGACCCTGGATGATAAAAAACCG
CTGAATACCCTGATCTCTGCTACTGGTCTGTGGATGAGTCGTACCGGAACCATTCATAAAATCAAACACCACGAG
GTTAGCCGTTCGAAAATCTATATTGAGATGGCGTGTGGCGATCATCTGGTTGTGAACAATAGCCGCTCTTCTCGT
ACAGCACGTGCACTGCGTCACCACAAATATCGTAAAACCTGTAAACGTTGCCGTGTGTCCGATGAGGATCTGAAC
AAATTCCTGACAAAAGCCAATGAGGACCAAACAAGCGTGAAAGTGAAAGTCGTTAGCGCTCCTACCCGTACTAAA
AAAGCAATGCCGAAATCCGTTGCTCGTGCCCCTAAACCACTGGAAAACACTGAAGCAGCACAGGCACAGCCGTCT
GGAAGCAAATTCTCTCCGGCCATTCCTGTTTCTACCCAGGAGTCCGTTTCTGTTCCAGCAAGTGTGAGCACCAGC
ATTAGCAGTATTAGCACCGGTGCCACCGCTAGCGCCCTGGTTAAAGGCAATACCAATCCGATTACAAGCATGTCT
GCCCCGGTTCAAGCATCAGCTCCAGCACTGACAAAATCCCAAACCGATCGTCTGGAGGTTCTGCTGAATCCGAAA
GACGAAATCAGCCTGAATTCCGGCAAACCGTTTCGTGAACTGGAGAGCGAACTGCTGTCACGTCGTAAAAAAGAC

CTGCAACAAATCTATGCCGAAGAACGTGAGAACTATCTGGGGAAACTGGAACGTGAAATCACCCGCTTTTTCGTG
GATCGTGGCTTTCTGGAGATCAAATCCCCGATTCTGATTCCTCTGGAGTATATCGAGCGTATGGGCATCGACAAT
GATACCGAACTGAGCAAACAAATTTTCCGTGTGGATAAAAACTTCTGTCTGCGCCCTATGCTAGCACCAAATCTG
GCTAACTATCTGCGCAAACTGGACCGTGCCCTGCCTGATCCTATCAAAATCTTCGAGATCGGCCCGTGTTATCGT
AAAGAGTCCGACGGTAAAGAACATCTGGAGGAGTTTACCATGCTGGCCTTTGCCCAAATGGGTTCAGGTTGTACT
CGTGAGAACCTGGAAAGCATCATCACCGATTTTCTGAACCACCTGGGCATTGACTTCAAAATTGTGGGCGACAGC
TGTATGGTGTTTGGCGACACCCTGGATGTCATGCACGGCGACCTGGAACTGTCTAGTGCCGTTGTGGGCCCAATC
CCGCTGGATCGTGAGTGGGGTATCGACAAACCTTGGATCGGTGCGGGTTTTGGTCTGGAGCGTCTGCTGAAAGTA
AAACACGACTTCAAGAACATCAAACGTGCTGCACGTTCCGAGTCCTATTACAATGGTATTTCTACTAACCTGTAA
(SEQ ID NO: 381)

Protein:
MAAPRDNVTLLFKLYCLAVMTLMAAVYTIALRYTRTSDKELYFSTTAVCITEVIKLLLSVGILAKETGSLGRFKA
SLRENVLGSPKELLKLSVPSLVYAVQNNMAFLALSNLDAAVYQVTYQLKIPCTALCTVLMLNRTLSKLQWVSVFM
LCAGVTLVQWKPAQATKVVVEQNPLLGFGAIAIAVLCSGFAGVYFEKVLKSSDTSLWVRNIQMYLSGIIVTLAGV
YLSDGAEIKEKGFFYGYTYYVWFVIFLASVGGLYTSVVVKYTDNIMKGFSAAAAIVLSTIASVMLFGLQITLTFA
LGTLLVCVSIYLYGLPRQDTTSIQQGETASKERVIGVAPGSAGSAAGSGMASNDYTQQATQSYGAYPTQPGQGY
SQQSSQPYGQQSYSGYSQSTDTSGYGQSSYSSYGQSQNTGYGTQSTPQGYGSTGGYGSSQSSQSSYGQQSSYPGY
GQQPAPSSTSGSYGSSSQSSSYGQPQSGSYSQQPSYGGQQQSYGQQQSYNPPQGYGQQNQYNSSSGGGGGGGGGG
NYGQDQSSMSSGGGSGGGYGNQDQSGGGGSGGYGQQDRGGRGRGGSGGGGGGGGGGYNRSSGGYEPRGRGGGRGG
RGGMGGSDRGGFNKFGGPRDQGSRHDSEQDNSDNNTIFVQGLGENVTIESVADYFKQIGIIKTNKKTGQPMINLY
TDRETGKLKGEATVSFDDPPSAKAAIDWFDGKEFSGNPIKVSFATRRADFNRGGGNGRGGRGRGGPMGRGGYGGG
GSGGGGRGGFPSGGGGGGGQQRAGDWKCPNPTCENMNFSWRNECNQCKAPKPDGPGGGPGGSHMGGNYGDDRRGG
RGGAIAGADYKDDDDKGAPGSAGSAAGSGACPVPLQLPPLERLTLDDKKPLNTLISATGLWMSRTGTIHKIKHHE
VSRSKIYIEMACGDHLVVNNSRSSRTARALRHHKYRKTCKRCRVSDEDLNKFLTKANEDQTSVKVKVVSAPTRTK
KAMPKSVARAPKPLENTEAAQAQPSGSKFSPAIPVSTQESVSVPASVSTSISSISTGATASALVKGNTNPITSMS
APVQASAPALTKSQTDRLEVLLNPKDEISLNSGKPFRELESELLSRRKKDLQQIYAEERENYLGKLEREITRFFV
DRGFLEIKSPILIPLEYIERMGIDNDTELSKQIFRVDKNFCLRPMLAPNLANYLRKLDRALPDPIKIFEIGPCYR
KESDGKEHLEEFTMLAFAQMGSGCTRENLESIITDFLNHLGIDFKIVGDSCMVFGDTLDVMHGDLELSSAVVGPI
PLDREWGIDKPWIGAGFGLERLLKVKHDFKNIKRAARSESYYNGISTNL* (SEQ ID NO: 382)

CMP-SaTr::MCP
DNA:
ATGGCTGCCCCGAGAGACAATGTCACTTTATTATTCAAGTTATACTGCTTGGCAGTGATGACCCTGATGGCTGCA
GTCTATACCATAGCTTTAAGATACACAAGGACATCAGACAAAGAACTCTACTTTTCAACCACAGCCGTGTGTATC
ACAGAAGTTATAAAGTTATTGCTAAGTGTGGGAATTTTAGCTAAAGAAACTGGTAGTCTGGGTAGATTCAAAGCA
TCTTTAAGAGAAAATGTCTTGGGGAGCCCCAAGGAACTGTTGAAGTTAAGTGTGCCATCGTTAGTGTATGCTGTT
CAGAACAACATGGCTTTCCTAGCTCTTAGCAATCTGGATGCAGCAGTGTACCAGGTGACCTACCAGTTGAAGATT
CCGTGTACTGCTTTATGCACTGTTTTAATGTTAAACCGGACACTCAGCAAATTACAGTGGGTTTCAGTTTTTATG
CTGTGTGCTGGAGTTACGCTTGTACAGTGGAAACCAGCCCAAGCTACAAAAGTGGTGGTGGAACAAAATCCATTA
TTAGGGTTTGGCGCTATAGCTATTGCTGTATTGTGCTCAGGATTTGCAGGAGTATATTTTGAAAAAGTTTTAAAG
AGTTCAGATACTTCTCTTTGGGTGAGAAACATTCAAATGTATCTATCAGGGATTATTGTGACATTAGCTGGCGTC
TACTTGTCAGATGGAGCTGAAATTAAAGAAAAAGGATTTTTCTATGGTTACACATATTATGTCTGGTTTGTCATC
TTTCTTGCAAGTGTTGGTGGCCTCTACACTTCTGTTGTGGTTAAGTACACAGACAATATCATGAAAGGCTTTTCT
GCAGCAGCGGCCATTGTCCTTTCCACCATTGCTTCAGTAATGCTGTTTGGATTACAGATAACACTTACCTTTGCC
CTGGGTACTCTTCTTGTATGTGTTTCCATATATCTCTATGGATTACCCAGACAAGACACTACATCCATCCAACAA
GGAGAAACAGCTTCAAAGGAGAGAGTTATTGGTGTGGCGATCGCATATCCCTATGATGTGCCGGATTATGCTGGA
GCACCAGGAAGTGCTGGTTCTGCTGCTGGTAGTGGAGCTTCTAACTTTACTCAGTTCGTTCTCGTCGACAATGGC
GGAACTGGCGACGTGACTGTCGCCCCAAGCAACTTCGCTAACGGGATCGCTGAATGGATCAGCTCTAACTCGCGT
TCACAGGCTTACAAAGTAACCTGTAGCGTTCGTCAGAGCTCTGCGCAGAATCGCAAATACACCATCAAAGTCGAG
GTGCCTAAAGGCGCCTGGCGTTCGTACTTAAATATGGAACTAACCATTCCAATTTTCGCCACGAATTCCGACTGC
GAGCTTATTGTTAAGGCAATGCAAGGTCTCCTAAAAGATGGAAACCCGATTCCCTCAGCAATCGCAGCAAACTCC
GGCATCTACTAA (SEQ ID NO: 383)

Protein:
MAAPRDNVTLLFKLYCLAVMTLMAAVYTIALRYTRTSDKELYFSTTAVCITEVIKLLLSVGILAKETGSLGRFKA
SLRENVLGSPKELLKLSVPSLVYAVQNNMAFLALSNLDAAVYQVTYQLKIPCTALCTVLMLNRTLSKLQWVSVFM

LCAGVTLVQWKPAQATKVVVEQNPLLGFGAIAIAVLCSGFAGVYFEKVLKSSDTSLWVRNIQMYLSGIIVTLAGV
YLSDGAEIKEKGFFYGYTYYVWFVIFLASVGGLYTSVVVKYTDNIMKGFSAAAAIVLSTIASVMLFGLQITLTFA
LGTLLVCVSIYLYGLPRQDTTSIQQGETASKERVIGVAIAYPYDVPDYAGAPGSAGSAAGSGASNFTQFVLVDNG
GTGDVTVAPSNFANGIAEWISSNSRSQAYKVTCSVRQSSAQNRKYTIKVEVPKGAWRSYLNMELTIPIFATNSDC
ELIVKAMQGLLKDGNPIPSAIAANSGIY* (SEQ ID NO: 384)

CMP-SaTr::EWSR1::MCP
DNA:
ATGGCTGCCCCGAGAGACAATGTCACTTTATTATTCAAGTTATACTGCTTGGCAGTGATGACCCTGATGGCTGCA
GTCTATACCATAGCTTTAAGATACACAAGGACATCAGACAAAGAACTCTACTTTTCAACCACAGCCGTGTGTATC
ACAGAAGTTATAAAGTTATTGCTAAGTGTGGGAATTTTAGCTAAAGAAACTGGTAGTCTGGGTAGATTCAAAGCA
TCTTTAAGAGAAAATGTCTTGGGGAGCCCCAAGGAACTGTTGAAGTTAAGTGTGCCATCGTTAGTGTATGCTGTT
CAGAACAACATGGCTTTCCTAGCTCTTAGCAATCTGGATGCAGCAGTGTACCAGGTGACCTACCAGTTGAAGATT
CCGTGTACTGCTTTATGCACTGTTTTAATGTTAAACCGGACACTCAGCAAATTACAGTGGGTTTCAGTTTTTATG
CTGTGTGCTGGAGTTACGCTTGTACAGTGGAAACCAGCCCAAGCTACAAAAGTGGTGGTGGAACAAAATCCATTA
TTAGGGTTTGGCGCTATAGCTATTGCTGTATTGTGCTCAGGATTTGCAGGAGTATATTTTGAAAAAGTTTTAAAG
AGTTCAGATACTTCTCTTTGGGTGAGAAACATTCAAATGTATCTATCAGGGATTATTGTGACATTAGCTGGCGTC
TACTTGTCAGATGGAGCTGAAATTAAAGAAAAAGGATTTTTCTATGGTTACACATATTATGTCTGGTTTGTCATC
TTTCTTGCAAGTGTTGGTGGCCTCTACACTTCTGTTGTGGTTAAGTACACAGACAATATCATGAAAGGCTTTTCT
GCAGCAGCGGCCATTGTCCTTTCCACCATTGCTTCAGTAATGCTGTTTGGATTACAGATAACACTTACCTTTGCC
CTGGGTACTCTTCTTGTATGTGTTTCCATATATCTCTATGGATTACCCAGACAAGACACTACATCCATCCAACAA
GGAGAAACAGCTTCAAAGGAGAGAGTTATTGGTGTGATGGCGTCCACGGATTACAGTACCTATAGCCAAGCTGCA
GCGCAGCAGGGCTACAGTGCTTACACCGCCCAGCCCACTCAAGGATATGCACAGACCACCCAGGCATATGGGCAA
CAAAGCTATGGAACCTATGGACAGCCCACTGATGTCAGCTATACCCAGGCTCAGACCACTGCAACCTATGGGCAG
ACCGCCTATGCAACTTCTTATGGACAGCCTCCCACTGGTTATACTACTCCAACTGCCCCCCAGGCATACAGCCAG
CCTGTCCAGGGGTATGGCACTGGTGCTTATGATACCACCACTGCTACAGTCACCACCACCCAGGCCTCCTATGCA
GCTCAGTCTGCATATGGCACTCAGCCTGCTTATCCAGCCTATGGGCAGCAGCCAGCAGCCACTGCACCTACAAGA
CCGCAGGATGGAAACAAGCCCACTGAGACTAGTCAACCTCAATCTAGCACAGGGGGTTACAACCAGCCCAGCCTA
GGATATGGACAGAGTAACTACAGTTATCCCCAGGTACCTGGGAGCTACCCCATGCAGCCAGTCACTGCACCTCCA
TCCTACCCTCCTACCAGCTATTCCTCTACACAGCCGACTAGTTATGATCAGAGCAGTTACTCTCAGCAGAACACC
TATGGGCAACCGAGCAGCTATGGACAGCAGAGTAGCTATGGTCAACAAAGCAGCTATGGGCAGCAGCCTCCCACT
AGTTACCCACCCCAAACTGGATCCTACAGCCAAGCTCCAAGTCAATATAGCCAACAGAGCAGCAGCTACGGGCAG
CAGAGTTCATTCCGACAGGACCACCCCAGTAGCATGGGTGTTTATGGGCAGGAGTCTGGAGGATTTTCCGGACCA
GGAGAGAACCGGAGCATGAGTGGCCCTGATAACCGGGGCAGGGGAAGAGGGGGATTTGATCGTGGAGGCATGAGC
AGAGGTGGGCGGGGAGGAGGACGCGGTGGAATGGGCAGCGCTGGAGAGCGAGGTGGCTTCAATAAGCCTGGTGGA
CCCATGGATGAAGGACCAGATCTTGATCTAGGCCCACCTGTAGATCCAGATGAAGACTCTGACAACAGTGCAATT
TATGTACAAGGATTAAATGACAGTGTGACTCTAGATGATCTGGCAGACTTCTTTAAGCAGTGTGGGGTTGTTAAG
ATGAACAAGAGAACTGGGCAACCCATGATCCACATCTACCTGGACAAGGAAACAGGAAAGCCCAAAGGCGATGCC
ACAGTGTCCTATGAAGACCCACCTACTGCCAAGGCTGCCGTGGAATGGTTTGATGGGAAAGATTTTCAAGGGAGC
AAACTTAAAGTCTCCCTTGCTCGGAAGAAGCCTCCAATGAACAGTATGCGGGGTGGTCTGCCACCCCGTGAGGGC
AGAGGCATGCCACCACCACTCCGTGGAGGTCCAGGAGGCCCAGGAGGTCCTGGGGGACCCATGGGTCGCATGGGA
GGCCGTGGAGGAGATAGAGGAGGCTTCCCTCCAAGAGGACCCCGGGGTTCCCGAGGGAACCCCTCTGGAGGAGGA
AACGTCCAGCACCGAGCTGGAGACTGGCAGTGTCCCAATCCGGGTTGTGGAAACCAGAACTTCGCCTGGAGAACA
GAGTGCAACCAGTGTAAGGCCCCAAAGCCTGAAGGCTTCCTCCCGCCACCCTTTCCGCCCCCGGGTGGTGATCGT
GGCAGAGGTGGCCCTGGTGGCATGCGGGGAGGAAGAGGTGGCCTCATGGATCGTGGTGGTCCCGGTGGAATGTTC
AGAGGTGGCCGTGGTGGAGACAGAGGTGGCTTCCGTGGTGGCCGGGGCATGGACCGAGGTGGCTTTGGTGGAGGA
AGACGAGGTGGCCCTGGGGGGCCCCCTGGACCTTTGATGGAACAGGCGATCGCATATCCCTATGATGTGCCGGAT
TATGCTGGAGCACCAGGAAGTGCTGGTTCTGCTGCTGGTAGTGGAGCTTCTAACTTTACTCAGTTCGTTCTCGTC
GACAATGGCGGAACTGGCGACGTGACTGTCGCCCCAAGCAACTTCGCTAACGGGATCGCTGAATGGATCAGCTCT
AACTCGCGTTCACAGGCTTACAAAGTAACCTGTAGCGTTCGTCAGAGCTCTGCGCAGAATCGCAAATACACCATC
AAAGTCGAGGTGCCTAAAGGCGCCTGGCGTTCGTACTTAAATATGGAACTAACCATTCCAATTTTCGCCACGAAT
TCCGACTGCGAGCTTATTGTTAAGGCAATGCAAGGTCTCCTAAAAGATGGAAACCCGATTCCCTCAGCAATCGCA
GCAAACTCCGGCATCTACTAA (SEQ ID NO: 385)

Protein:
MAAPRDNVTLLFKLYCLAVMTLMAAVYTIALRYTRTSDKELYFSTTAVCITEVIKLLLSVGILAKETGSLGRFKA
SLRENVLGSPKELLKLSVPSLVYAVQNNMAFLALSNLDAAVYQVTYQLKIPCTALCTVLMLNRTLSKLQWVSVFM

LCAGVTLVQWKPAQATKVVVEQNPLLGFGAIAIAVLCSGFAGVYFEKVLKSSDTSLWVRNIQMYLSGIIVTLAGV
YLSDGAEIKEKGFFYGYTYYVWFVIFLASVGGLYTSVVVKYTDNIMKGFSAAAAIVLSTIASVMLFGLQITLTFA
LGTLLVCVSIYLYGLPRQDTTSIQQGETASKERVIGVMASTDYSTYSQAAAQQGYSAYTAQPTQGYAQTTQAYGQ
QSYGTYGQPTDVSYTQAQTTATYGQTAYATSYGQPPTGYTTPTAPQAYSQPVQGYGTGAYDTTTATVTTTQASYA
AQSAYGTQPAYPAYGQQPAATAPTRPQDGNKPTETSQPQSSTGGYNQPSLGYGQSNYSYPQVPGSYPMQPVTAPP
SYPPTSYSSTQPTSYDQSSYSQQNTYGQPSSYGQQSSYGQQSSYGQQPPTSYPPQTGSYSQAPSQYSQQSSSYGQ
QSSFRQDHPSSMGVYGQESGGFSGPGENRSMSGPDNRGRGRGGFDRGGMSRGGRGGGRGGMGSAGERGGFNKPGG
PMDEGPDLDLGPPVDPDEDSDNSAIYVQGLNDSVTLDDLADFFKQCGVVKMNKRTGQPMIHIYLDKETGKPKGDA
TVSYEDPPTAKAAVEWFDGKDFQGSKLKVSLARKKPPMNSMRGGLPPREGRGMPPPLRGGPGGPGGPGGPMGRMG
GRGGDRGGFPPRGPRGSRGNPSGGGNVQHRAGDWQCPNPGCGNQNFAWRTECNQCKAPKPEGFLPPPFPPPGGDR
GRGGPGGMRGGRGGLMDRGGPGGMFRGGRGGDRGGFRGGRGMDRGGFGGGRRGGPGGPPGPLMEQAIAYPYDVPD
YAGAPGSAGSAAGSGASNFTQFVLVDNGGTGDVTVAPSNFANGIAEWISSNSRSQAYKVTCSVRQSSAQNRKYTI
KVEVPKGAWRSYLNMELTIPIFATNSDCELIVKAMQGLLKDGNPIPSAIAANSGIY* (SEQ ID NO: 386)

CMP-SaTr::PylRS(AF)EWSR1::4xλN22
DNA:
ATGGCTGCCCCGAGAGACAATGTCACTTTATTATTCAAGTTATACTGCTTGGCAGTGATGACCCTGATGGCTGCA
GTCTATACCATAGCTTTAAGATACACAAGGACATCAGACAAAGAACTCTACTTTTCAACCACAGCCGTGTGTATC
ACAGAAGTTATAAAGTTATTGCTAAGTGTGGGAATTTTAGCTAAAGAAACTGGTAGTCTGGGTAGATTCAAAGCA
TCTTTAAGAGAAAATGTCTTGGGGAGCCCCAAGGAACTGTTGAAGTTAAGTGTGCCATCGTTAGTGTATGCTGTT
CAGAACAACATGGCTTTCCTAGCTCTTAGCAATCTGGATGCAGCAGTGTACCAGGTGACCTACCAGTTGAAGATT
CCGTGTACTGCTTTATGCACTGTTTTAATGTTAAACCGGACACTCAGCAAATTACAGTGGGTTTCAGTTTTTATG
CTGTGTGCTGGAGTTACGCTTGTACAGTGGAAACCAGCCCAAGCTACAAAAGTGGTGGTGGAACAAAATCCATTA
TTAGGGTTTGGCGCTATAGCTATTGCTGTATTGTGCTCAGGATTTGCAGGAGTATATTTTGAAAAAGTTTTAAAG
AGTTCAGATACTTCTCTTTGGGTGAGAAACATTCAAATGTATCTATCAGGGATTATTGTGACATTAGCTGGCGTC
TACTTGTCAGATGGAGCTGAAATTAAAGAAAAAGGATTTTTCTATGGTTACACATATTATGTCTGGTTTGTCATC
TTTCTTGCAAGTGTTGGTGGCCTCTACACTTCTGTTGTGGTTAAGTACACAGACAATATCATGAAAGGCTTTTCT
GCAGCAGCGGCCATTGTCCTTTCCACCATTGCTTCAGTAATGCTGTTTGGATTACAGATAACACTTACCTTTGCC
CTGGGTACTCTTCTTGTATGTGTTTCCATATATCTCTATGGATTACCCAGACAAGACACTACATCCATCCAACAA
GGAGAAACAGCTTCAAAGGAGAGAGTTATTGGTGTGATGGCGTCCACGGATTACAGTACCTATAGCCAAGCTGCA
GCGCAGCAGGGCTACAGTGCTTACACCGCCCAGCCCACTCAAGGATATGCACAGACCACCCAGGCATATGGGCAA
CAAAGCTATGGAACCTATGGACAGCCCACTGATGTCAGCTATACCCAGGCTCAGACCACTGCAACCTATGGGCAG
ACCGCCTATGCAACTTCTTATGGACAGCCTCCCACTGGTTATACTACTCCAACTGCCCCCCAGGCATACAGCCAG
CCTGTCCAGGGGTATGGCACTGGTGCTTATGATACCACCACTGCTACAGTCACCACCACCCAGGCCTCCTATGCA
GCTCAGTCTGCATATGGCACTCAGCCTGCTTATCCAGCCTATGGGCAGCAGCCAGCAGCCACTGCACCTACAAGA
CCGCAGGATGGAAACAAGCCCACTGAGACTAGTCAACCTCAATCTAGCACAGGGGGTTACAACCAGCCCAGCCTA
GGATATGGACAGAGTAACTACAGTTATCCCCAGGTACCTGGGAGCTACCCCATGCAGCCAGTCACTGCACCTCCA
TCCTACCCTCCTACCAGCTATTCCTCTACACAGCCGACTAGTTATGATCAGAGCAGTTACTCTCAGCAGAACACC
TATGGGCAACCGAGCAGCTATGGACAGCAGAGTAGCTATGGTCAACAAAGCAGCTATGGGCAGCAGCCTCCCACT
AGTTACCCACCCCAAACTGGATCCTACAGCCAAGCTCCAAGTCAATATAGCCAACAGAGCAGCAGCTACGGGCAG
CAGAGTTCATTCCGACAGGACCACCCCAGTAGCATGGGTGTTTATGGGCAGGAGTCTGGAGGATTTTCCGGACCA
GGAGAGAACCGGAGCATGAGTGGCCCTGATAACCGGGGCAGGGGAAGAGGGGGATTTGATCGTGGAGGCATGAGC
AGAGGTGGGCGGGGAGGAGGACGCGGTGGAATGGGCAGCGCTGGAGAGCGAGGTGGCTTCAATAAGCCTGGTGGA
CCCATGGATGAAGGACCAGATCTTGATCTAGGCCCACCTGTAGATCCAGATGAAGACTCTGACAACAGTGCAATT
TATGTACAAGGATTAAATGACAGTGTGACTCTAGATGATCTGGCAGACTTCTTTAAGCAGTGTGGGGTTGTTAAG
ATGAACAAGAGAACTGGGCAACCCATGATCCACATCTACCTGGACAAGGAAACAGGAAAGCCCAAAGGCGATGCC
ACAGTGTCCTATGAAGACCCACCTACTGCCAAGGCTGCCGTGGAATGGTTTGATGGGAAAGATTTTCAAGGGAGC
AAACTTAAAGTCTCCCTTGCTCGGAAGAAGCCTCCAATGAACAGTATGCGGGGTGGTCTGCCACCCCGTGAGGGC
AGAGGCATGCCACCACCACTCCGTGGAGGTCCAGGAGGCCCAGGAGGTCCTGGGGGACCCATGGGTCGCATGGGA
GGCCGTGGAGGAGATAGAGGAGGCTTCCCTCCAAGAGGACCCCGGGGTTCCCGAGGGAACCCCTCTGGAGGAGGA
AACGTCCAGCACCGAGCTGGAGACTGGCAGTGTCCCAATCCGGGTTGTGGAAACCAGAACTTCGCCTGGAGAACA
GAGTGCAACCAGTGTAAGGCCCCAAAGCCTGAAGGCTTCCTCCCGCCACCCTTTCCGCCCCCGGGTGGTGATCGT
GGCAGAGGTGGCCCTGGTGGCATGCGGGGAGGAAGAGGTGGCCTCATGGATCGTGGTGGTCCCGGTGGAATGTTC
AGAGGTGGCCGTGGTGGAGACAGAGGTGGCTTCCGTGGTGGCCGGGGCATGGACCGAGGTGGCTTTGGTGGAGGA
AGACGAGGTGGCCCTGGGGGGCCCCCTGGACCTTTGATGGAACAGGCGATCGCAGGAGCACCAGGAAGTGCTGGT
TCTGCTGCTGGTAGTGGAGAGCAGAAGCTGATCTCAGAGGAGGACCTGCTAGCCACCATGGACGCACAAACACGA
CGACGTGAGCGTCGCGCTGAGAAACAAGCTCAATGGAAAGCTGCAAACCCACCGCTCGACGGAGCCGGAGCTGGC

GCTGGAGCTGGAGCCGGAGCTGGCGGTCTAGCCACCATGGACGCACAAACACGACGACGTGAGCGTCGCGCTGAG
AAACAAGCTCAATGGAAAGCTGCAAACCCACCGCTCGACGGAGCCGGAGCTGGCGCTGGAGCTGGAGCCGGAGCT
GGCGGTCTAGCCACCATGGACGCACAAACACGACGACGTGAGCGTCGCGCTGAGAAACAAGCTCAATGGAAAGCT
GCAAACCCACCGCTCGACGGAGCCGGAGCTGGCGCTGGAGCTGGAGCCGGAGCTGGCGGTCTAGCCACCATGGAC
GCACAAACACGACGACGTGAGCGTCGCGCTGAGAAACAAGCTCAATGGAAAGCTGCAAACCCACCGCTCGAGTCT
AGAGGGCCCGTTTAA (SEQ ID NO: 387)

Protein:
MAAPRDNVTLLFKLYCLAVMTLMAAVYTIALRYTRTSDKELYFSTTAVCITEVIKLLLSVGILAKETGSLGRFKA
SLRENVLGSPKELLKLSVPSLVYAVQNNMAFLALSNLDAAVYQVTYQLKIPCTALCTVLMLNRTLSKLQWVSVFM
LCAGVTLVQWKPAQATKVVVEQNPLLGFGAIAIAVLCSGFAGVYFEKVLKSSDTSLWVRNIQMYLSGIIVTLAGV
YLSDGAEIKEKGFFYGYTYYVWFVIFLASVGGLYTSVVVKYTDNIMKGFSAAAAIVLSTIASVMLFGLQITLTFA
LGTLLVCVSIYLYGLPRQDTTSIQQGETASKERVIGVMASTDYSTYSQAAAQQGYSAYTAQPTQGYAQTTQAYGQ
QSYGTYGQPTDVSYTQAQTTATYGQTAYATSYGQPPTGYTTPTAPQAYSQPVQGYGTGAYDTTTATVTTTQASYA
AQSAYGTQPAYPAYGQQPAATAPTRPQDGNKPTETSQPQSSTGGYNQPSLGYGQSNYSYPQVPGSYPMQPVTAPP
SYPPTSYSSTQPTSYDQSSYSQQNTYGQPSSYGQQSSYGQQSSYGQQPPTSYPPQTGSYSQAPSQYSQQSSSYGQ
QSSFRQDHPSSMGVYGQESGGFSGPGENRSMSGPDNRGRGRGGFDRGGMSRGGRGGGRGGMGSAGERGGFNKPGG
PMDEGPDLDLGPPVDPDEDSDNSAIYVQGLNDSVTLDDLADFFKQCGVVKMNKRTGQPMIHIYLDKETGKPKGDA
TVSYEDPPTAKAAVEWFDGKDFQGSKLKVSLARKKPPMNSMRGGLPPREGRGMPPPLRGGPGGPGGPGGPMGRMG
GRGGDRGGFPPRGPRGSRGNPSGGGNVQHRAGDWQCPNPGCGNQNFAWRTECNQCKAPKPEGFLPPPFPPPGGDR
GRGGPGGMRGGRGGLMDRGGPGGMFRGGRGGDRGGFRGGRGMDRGGFGGGRRGGPGGPPGPLMEQAIAGAPGSAG
SAAGSGEQKLISEEDLLATMDAQTRRRERRAEKQAQWKAANPPLDGAGAGAGAGAGAGGLATMDAQTRRRERRAE
KQAQWKAANPPLDGAGAGAGAGAGAGGLATMDAQTRRRERRAEKQAQWKAANPPLDGAGAGAGAGAGAGGLATMD
AQTRRRERRAEKQAQWKAANPPLESRGPV* (SEQ ID NO: 388)

CMP-SaTr::FUS::PylRS(AF)
DNA:
ATGGCTGCCCCGAGAGACAATGTCACTTTATTATTCAAGTTATACTGCTTGGCAGTGATGACCCTGATGGCTGCA
GTCTATACCATAGCTTTAAGATACACAAGGACATCAGACAAAGAACTCTACTTTTCAACCACAGCCGTGTGTATC
ACAGAAGTTATAAAGTTATTGCTAAGTGTGGGAATTTTAGCTAAAGAAACTGGTAGTCTGGGTAGATTCAAAGCA
TCTTTAAGAGAAAATGTCTTGGGGAGCCCCAAGGAACTGTTGAAGTTAAGTGTGCCATCGTTAGTGTATGCTGTT
CAGAACAACATGGCTTTCCTAGCTCTTAGCAATCTGGATGCAGCAGTGTACCAGGTGACCTACCAGTTGAAGATT
CCGTGTACTGCTTTATGCACTGTTTTAATGTTAAACCGGACACTCAGCAAATTACAGTGGGTTTCAGTTTTTATG
CTGTGTGCTGGAGTTACGCTTGTACAGTGGAAACCAGCCCAAGCTACAAAAGTGGTGGTGGAACAAAATCCATTA
TTAGGGTTTGGCGCTATAGCTATTGCTGTATTGTGCTCAGGATTTGCAGGAGTATATTTTGAAAAAGTTTTAAAG
AGTTCAGATACTTCTCTTTGGGTGAGAAACATTCAAATGTATCTATCAGGGATTATTGTGACATTAGCTGGCGTC
TACTTGTCAGATGGAGCTGAAATTAAAGAAAAAGGATTTTTCTATGGTTACACATATTATGTCTGGTTTGTCATC
TTTCTTGCAAGTGTTGGTGGCCTCTACACTTCTGTTGTGGTTAAGTACACAGACAATATCATGAAAGGCTTTTCT
GCAGCAGCGGCCATTGTCCTTTCCACCATTGCTTCAGTAATGCTGTTTGGATTACAGATAACACTTACCTTTGCC
CTGGGTACTCTTCTTGTATGTGTTTCCATATATCTCTATGGATTACCCAGACAAGACACTACATCCATCCAACAA
GGAGAAACAGCTTCAAAGGAGAGAGTTATTGGTGTGGGAGCACCCGGCTCCGCCGGCTCCGCCGCCGGCTCCGGC
ATGGCCTCAAACGATTATACCCAACAAGCAACCCAAAGCTATGGGGCCTACCCCACCCAGCCCGGGCAGGGCTAT
TCCCAGCAGAGCAGTCAGCCCTACGGACAGCAGAGTTACAGTGGTTATAGCCAGTCCACGGACACTTCAGGATAT
GGCCAGAGCAGCTATTCTTCTTATGGCCAGAGCCAGAACACAGGCTATGGAACTCAGTCAACTCCCCAGGGATAT
GGCTCGACTGGCGGCTATGGCAGTAGCCAGAGCTCCCAATCGTCTTACGGGCAGCAGTCCTCCTACCCTGGCTAT
GGCCAGCAGCCAGCTCCCAGCAGCACCTCGGGAAGTTACGGTAGCAGTTCTCAGAGCAGCAGCTATGGGCAGCCC
CAGAGTGGGAGCTACAGCCAGCAGCCTAGCTATGGTGGACAGCAGCAAAGCTATGGACAGCAGCAAAGCTATAAT
CCCCCTCAGGGCTATGGACAGCAGAACCAGTACAACAGCAGCAGTGGTGGTGGAGGTGGAGGTGGAGGTGGAGGT
AACTATGGCCAAGATCAATCCTCCATGAGTAGTGGTGGTGGCAGTGGTGGCGGTTATGGCAATCAAGACCAGAGT
GGTGGAGGTGGCAGCGGTGGCTATGGACAGCAGGACCGTGGAGGCCGCGGCAGGGGTGGCAGTGGTGGCGGCGGC
GGCGGCGGCGGTGGTGGTTACAACCGCAGCAGTGGTGGCTATGAACCCAGAGGTCGTGGAGGTGGCCGTGGAGGC
AGAGGTGGCATGGGCGGAAGTGACCGTGGTGGCTTCAATAAATTTGGTGGCCCTCGGGACCAAGGATCACGTCAT
GACTCCGAACAGGATAATTCAGACAACAACACCATCTTTGTGCAAGGCCTGGGTGAGAATGTTACAATTGAGTCT
GTGGCTGATTACTTCAAGCAGATTGGTATTATTAAGACAAACAAGAAAACGGGACAGCCCATGATTAATTTGTAC
ACAGACAGGGAAACTGGCAAGCTGAAGGGAGAGGCAACGGTCTCTTTTGATGACCCACCTTCAGCTAAAGCAGCT
ATTGACTGGTTTGATGGTAAAGAATTCTCCGGAAATCCTATCAAGGTCTCATTTGCTACTCGCCGGGCAGACTTT
AATCGGGGTGGTGGCAATGGTCGTGGAGGCCGAGGGCGAGGAGGACCCATGGGCCGTGGAGGCTATGGAGGTGGT

GGCAGTGGTGGTGGTGGCCGAGGAGGATTTCCCAGTGGAGGTGGTGGCGGTGGAGGACAGCAGCGAGCTGGTGAC
TGGAAGTGTCCTAATCCCACCTGTGAGAATATGAACTTCTCTTGGAGGAATGAATGCAACCAGTGTAAGGCCCCT
AAACCAGATGGCCCAGGAGGGGGACCAGGTGGCTCTCACATGGGGGGTAACTACGGGGATGATCGTCGTGGTGGC
AGAGGTGGTGCGATCGCAGGCGCCGATTACAAGGACGATGATGACAAGGGAGCACCAGGAAGTGCTGGTTCTGCT
GCTGGTAGTGGAGCGTGCCCGGTGCCGCTGCAGCTGCCGCCGCTGGAACGCCTGACCCTGGATGATAAAAAACCG
CTGAATACCCTGATCTCTGCTACTGGTCTGTGGATGAGTCGTACCGGAACCATTCATAAAATCAAACACCACGAG
GTTAGCCGTTCGAAAATCTATATTGAGATGGCGTGTGGCGATCATCTGGTTGTGAACAATAGCCGCTCTTCTCGT
ACAGCACGTGCACTGCGTCACCACAAATATCGTAAAACCTGTAAACGTTGCCGTGTGTCCGATGAGGATCTGAAC
AAATTCCTGACAAAAGCCAATGAGGACCAAACAAGCGTGAAAGTGAAAGTCGTTAGCGCTCCTACCCGTACTAAA
AAAGCAATGCCGAAATCCGTTGCTCGTGCCCCTAAACCACTGGAAAACACTGAAGCAGCACAGGCACAGCCGTCT
GGAAGCAAATTCTCTCCGGCCATTCCTGTTTCTACCCAGGAGTCCGTTTCTGTTCCAGCAAGTGTGAGCACCAGC
ATTAGCAGTATTAGCACCGGTGCCACCGCTAGCGCCCTGGTTAAAGGCAATACCAATCCGATTACAAGCATGTCT
GCCCCGGTTCAAGCATCAGCTCCAGCACTGACAAAATCCCAAACCGATCGTCTGGAGGTTCTGCTGAATCCGAAA
GACGAAATCAGCCTGAATTCCGGCAAACCGTTTCGTGAACTGGAGAGCGAACTGCTGTCACGTCGTAAAAAAGAC
CTGCAACAAATCTATGCCGAAGAACGTGAGAACTATCTGGGGAAACTGGAACGTGAAATCACCCGCTTTTTCGTG
GATCGTGGCTTTCTGGAGATCAAATCCCCGATTCTGATTCCTCTGGAGTATATCGAGCGTATGGGCATCGACAAT
GATACCGAACTGAGCAAACAAATTTTCCGTGTGGATAAAAACTTCTGTCTGCGCCCTATGCTAGCACCAAATCTG
GCTAACTATCTGCGCAAACTGGACCGTGCCCTGCCTGATCCTATCAAAATCTTCGAGATCGGCCCGTGTTATCGT
AAAGAGTCCGACGGTAAAGAACATCTGGAGGAGTTTACCATGCTGAACTTTTGCCAAATGGGTTCAGGTTGTACT
CGTGAGAACCTGGAAAGCATCATCACCGATTTTCTGAACCACCTGGGCATTGACTTCAAAATTGTGGGCGACAGC
TGTATGGTGTTTGGCGACACCCTGGATGTCATGCACGGCGACCTGGAACTGTCTAGTGCCGTTGTGGGCCCAATC
CCGCTGGATCGTGAGTGGGGTATCGACAAACCTTGGATCGGTGCGGGTTTTGGTCTGGAGCGTCTGCTGAAAGTA
AAACACGACTTCAAGAACATCAAACGTGCTGCACGTTCCGAGTCCTATTACAATGGTATTTCTACTAACCTGTAA
(SEQ ID NO: 389)

Protein:
MAAPRDNVTLLFKLYCLAVMTLMAAVYTIALRYTRTSDKELYFSTTAVCITEVIKLLLSVGILAKETGSLGRFKA
SLRENVLGSPKELLKLSVPSLVYAVQNNMAFLALSNLDAAVYQVTYQLKIPCTALCTVLMLNRTLSKLQWVSVFM
LCAGVTLVQWKPAQATKVVVEQNPLLGFGAIAIAVLCSGFAGVYFEKVLKSSDTSLWVRNIQMYLSGIIVTLAGV
YLSDGAEIKEKGFFYGYTYYVWFVIFLASVGGLYTSVVVKYTDNIMKGFSAAAAIVLSTIASVMLFGLQITLTFA
LGTLLVCVSIYLYGLPRQDTTSIQQGETASKERVIGVAPGSAGSAAGSGMASNDYTQQATQSYGAYPTQPGQGY
SQQSSQPYGQQSYSGYSQSTDTSGYGQSSYSSYGQSQNTGYGTQSTPQGYGSTGGYGSSQSSQSSYGQQSSYPGY
GQQPAPSSTSGSYGSSSQSSSYGQPQSGSYSQQPSYGGQQQSYGQQQSYNPPQGYGQQNQYNSSSGGGGGGGGGG
NYGQDQSSMSSGGGSGGGYGNQDQSGGGGSGGYGQQDRGGRGRGGSGGGGGGGGGGYNRSSGGYEPRGRGGGRGG
RGGMGGSDRGGFNKFGGPRDQGSRHDSEQDNSDNNTIFVQGLGENVTIESVADYFKQIGIIKTNKKTGQPMINLY
TDRETGKLKGEATVSFDDPPSAKAAIDWFDGKEFSGNPIKVSFATRRADFNRGGGNGRGGRGRGGPMGRGGYGGG
GSGGGGRGGFPSGGGGGGGQQRAGDWKCPNPTCENMNFSWRNECNQCKAPKPDGPGGGPGGSHMGGNYGDDRRGG
RGGAIAGADYKDDDDKGAPGSAGSAAGSGACPVPLQLPPLERLTLDDKKPLNTLISATGLWMSRTGTIHKIKHHE
VSRSKIYIEMACGDHLVVNNSRSSRTARALRHHKYRKTCKRCRVSDEDLNKFLTKANEDQTSVKVKVVSAPTRTK
KAMPKSVARAPKPLENTEAAQAQPSGSKFSPAIPVSTQESVSVPASVSTSISSISTGATASALVKGNTNPITSMS
APVQASAPALTKSQTDRLEVLLNPKDEISLNSGKPFRELESELLSRRKKDLQQIYAEERENYLGKLEREITRFFV
DRGFLEIKSPILIPLEYIERMGIDNDTELSKQIFRVDKNFCLRPMLAPNLANYLRKLDRALPDPIKIFEIGPCYR
KESDGKEHLEEFTMLNFCQMGSGCTRENLESIITDFLNHLGIDFKIVGDSCMVFGDTLDVMHGDLELSSAVVGPI
PLDREWGIDKPWIGAGFGLERLLKVKHDFKNIKRAARSESYYNGISTNL* (SEQ ID NO: 390)

P450 2C1₁₋₂₇::PylRS(AF)
DNA:
ATGGACCCCGTGGTCGTGCTGGGCCTGTGCCTGTCATGCCTGCTGCTGCTGAGCCTGTGGAAGCAGAGCTACGGC
GGAGGCGCGATCGCAGGCAAGCCTATTCCCAACCCCCTGCTGGGCCTGGATAGCACCGGAGCACCAGGAAGTGCT
GGTTCTGCTGCTGGTAGTGGAGCGTGCCCGGTGCCGCTGCAGCTGCCGCCGCTGGAACGCCTGACCCTGGATGAT
AAAAAACCGCTGAATACCCTGATCTCTGCTACTGGTCTGTGGATGAGTCGTACCGGAACCATTCATAAAATCAAA
CACCACGAGGTTAGCCGTTCGAAAATCTATATTGAGATGGCGTGTGGCGATCATCTGGTTGTGAACAATAGCCGC
TCTTCTCGTACAGCACGTGCACTGCGTCACCACAAATATCGTAAAACCTGTAAACGTTGCCGTGTGTCCGATGAG
GATCTGAACAAATTCCTGACAAAAGCCAATGAGGACCAAACAAGCGTGAAAGTGAAAGTCGTTAGCGCTCCTACC
CGTACTAAAAAAGCAATGCCGAAATCCGTTGCTCGTGCCCCTAAACCACTGGAAAACACTGAAGCAGCACAGGCA
CAGCCGTCTGGAAGCAAATTCTCTCCGGCCATTCCTGTTTCTACCCAGGAGTCCGTTTCTGTTCCAGCAAGTGTG
AGCACCAGCATTAGCAGTATTAGCACCGGTGCCACCGCTAGCGCCCTGGTTAAAGGCAATACCAATCCGATTACA

AGCATGTCTGCCCCGGTTCAAGCATCAGCTCCAGCACTGACAAAATCCCAAACCGATCGTCTGGAGGTTCTGCTG
AATCCGAAAGACGAAATCAGCCTGAATTCCGGCAAACCGTTTCGTGAACTGGAGAGCGAACTGCTGTCACGTCGT
AAAAAAGACCTGCAACAAATCTATGCCGAAGAACGTGAGAACTATCTGGGGAAACTGGAACGTGAAATCACCCGC
TTTTTCGTGGATCGTGGCTTTCTGGAGATCAAATCCCCGATTCTGATTCCTCTGGAGTATATCGAGCGTATGGGC
ATCGACAATGATACCGAACTGAGCAAACAAATTTTCCGTGTGGATAAAAACTTCTGTCTGCGCCCTATGCTAGCA
CCAAATCTGGCTAACTATCTGCGCAAACTGGACCGTGCCCTGCCTGATCCTATCAAAATCTTCGAGATCGGCCCG
TGTTATCGTAAAGAGTCCGACGGTAAAGAACATCTGGAGGAGTTTACCATGCTGAACTTTTGCCAAATGGGTTCA
GGTTGTACTCGTGAGAACCTGGAAAGCATCATCACCGATTTTCTGAACCACCTGGGCATTGACTTCAAAATTGTG
GGCGACAGCTGTATGGTGTTTGGCGACACCCTGGATGTCATGCACGGCGACCTGGAACTGTCTAGTGCCGTTGTG
GGCCCAATCCCGCTGGATCGTGAGTGGGGTATCGACAAACCTTGGATCGGTGCGGGTTTTGGTCTGGAGCGTCTG
CTGAAAGTAAAACACGACTTCAAGAACATCAAACGTGCTGCACGTTCCGAGTCCTATTACAATGGTATTTCTACT
AACCTGTAA (SEQ ID NO: 391)

Protein:
MDPVVVLGLCLSCLLLLSLWKQSYGGGAIAGKPIPNPLLGLDSTGAPGSAGSAAGSGACPVPLQLPPLERLTLDD
KKPLNTLISATGLWMSRTGTIHKIKHHEVSRSKIYIEMACGDHLVVNNSRSSRTARALRHHKYRKTCKRCRVSDE
DLNKFLTKANEDQTSVKVKVVSAPTRTKKAMPKSVARAPKPLENTEAAQAQPSGSKFSPAIPVSTQESVSVPASV
STSISSISTGATASALVKGNTNPITSMSAPVQASAPALTKSQTDRLEVLLNPKDEISLNSGKPFRELESELLSRR
KKDLQQIYAEERENYLGKLEREITRFFVDRGFLEIKSPILIPLEYIERMGIDNDTELSKQIFRVDKNFCLRPMLA
PNLANYLRKLDRALPDPIKIFEIGPCYRKESDGKEHLEEFTMLNFCQMGSGCTRENLESIITDFLNHLGIDFKIV
GDSCMVFGDTLDVMHGDLELSSAVVGPIPLDREWGIDKPWIGAGFGLERLLKVKHDFKNIKRAARSESYYNGIST
NL* (SEQ ID NO: 392)

P450 2C1₁₋₂₇::MCP
DNA:
ATGGACCCCGTGGTCGTGCTGGGCCTGTGCCTGTCATGCCTGCTGCTGCTGAGCCTGTGGAAGCAGAGCTACGGC
GGAGGCGCGATCGCATATCCCTATGATGTGCCGGATTATGCTGGAGCACCAGGAAGTGCTGGTTCTGCTGCTGGT
AGTGGAGCTTCTAACTTTACTCAGTTCGTTCTCGTCGACAATGGCGGAACTGGCGACGTGACTGTCGCCCCAAGC
AACTTCGCTAACGGGATCGCTGAATGGATCAGCTCTAACTCGCGTTCACAGGCTTACAAAGTAACCTGTAGCGTT
CGTCAGAGCTCTGCGCAGAATCGCAAATACACCATCAAAGTCGAGGTGCCTAAAGGCGCCTGGCGTTCGTACTTA
AATATGGAACTAACCATTCCAATTTTCGCCACGAATTCCGACTGCGAGCTTATTGTTAAGGCAATGCAAGGTCTC
CTAAAAGATGGAAACCCGATTCCCTCAGCAATCGCAGCAAACTCCGGCATCTACTAA (SEQ ID NO: 393)

Protein:
MDPVVVLGLCLSCLLLLSLWKQSYGGGAIAYPYDVPDYAGAPGSAGSAAGSGASNFTQFVLVDNGGTGDVTVAPS
NFANGIAEWISSNSRSQAYKVTCSVRQSSAQNRKYTIKVEVPKGAWRSYLNMELTIPIFATNSDCELIVKAMQGL
LKDGNPIPSAIAANSGIY* (SEQ ID NO: 394)

P450 2C1₁₋₂₇::FUS::PylRS(AF)
DNA:
ATGGACCCCGTGGTCGTGCTGGGCCTGTGCCTGTCATGCCTGCTGCTGCTGAGCCTGTGGAAGCAGAGCTACGGC
GGAGGCATGGCCTCAAACGATTATACCCAACAAGCAACCCAAAGCTATGGGGCCTACCCCACCCAGCCCGGGCAG
GGCTATTCCCAGCAGAGCAGTCAGCCCTACGGACAGCAGAGTTACAGTGGTTATAGCCAGTCCACGGACACTTCA
GGATATGGCCAGAGCAGCTATTCTTCTTATGGCCAGAGCCAGAACACAGGCTATGGAACTCAGTCAACTCCCCAG
GGATATGGCTCGACTGGCGGCTATGGCAGTAGCCAGAGCTCCCAATCGTCTTACGGGCAGCAGTCCTCCTACCCT
GGCTATGGCCAGCAGCCAGCTCCCAGCAGCACCTCGGGAAGTTACGGTAGCAGTTCTCAGAGCAGCAGCTATGGG
CAGCCCCAGAGTGGGAGCTACAGCCAGCAGCCTAGCTATGGTGGACAGCAGCAAAGCTATGGACAGCAGCAAAGC
TATAATCCCCCTCAGGGCTATGGACAGCAGAACCAGTACAACAGCAGCAGTGGTGGTGGAGGTGGAGGTGGAGGT
GGAGGTAACTATGGCCAAGATCAATCCTCCATGAGTAGTGGTGGTGGCAGTGGTGGCGGTTATGGCAATCAAGAC
CAGAGTGGTGGAGGTGGCAGCGGTGGCTATGGACAGCAGGACCGTGGAGGCCGCGGCAGGGGTGGCAGTGGTGGC
GGCGGCGGCGGCGGCGGTGGTGGTTACAACCGCAGCAGTGGTGGCTATGAACCCAGAGGTCGTGGAGGTGGCCGT
GGAGGCAGAGGTGGCATGGGCGGAAGTGACCGTGGTGGCTTCAATAAATTTGGTGGCCCTCGGGACCAAGGATCA
CGTCATGACTCCGAACAGGATAATTCAGACAACAACACCATCTTTGTGCAAGGCCTGGGTGAGAATGTTACAATT
GAGTCTGTGGCTGATTACTTCAAGCAGATTGGTATTATTAAGACAAACAAGAAAACGGGACAGCCCATGATTAAT
TTGTACACAGACAGGGAAACTGGCAAGCTGAAGGGAGAGGCAACGGTCTCTTTTGATGACCCACCTTCAGCTAAA
GCAGCTATTGACTGGTTTGATGGTAAAGAATTCTCCGGAAATCCTATCAAGGTCTCATTTGCTACTCGCCGGGCA
GACTTTAATCGGGGTGGTGGCAATGGTCGTGGAGGCCGAGGGCGAGGAGGACCCATGGGCCGTGGAGGCTATGGA

GGTGGTGGCAGTGGTGGTGGTGGCCGAGGAGGATTTCCCAGTGGAGGTGGTGGCGGTGGAGGACAGCAGCGAGCT
GGTGACTGGAAGTGTCCTAATCCCACCTGTGAGAATATGAACTTCTCTTGGAGGAATGAATGCAACCAGTGTAAG
GCCCCTAAACCAGATGGCCCAGGAGGGGGACCAGGTGGCTCTCACATGGGGGGTAACTACGGGGATGATCGTCGT
GGTGGCAGAGGAGGCGCGATCGCAGGCAAGCCTATTCCCAACCCCCTGCTGGGCCTGGATAGCACCGGAGCACCA
GGAAGTGCTGGTTCTGCTGCTGGTAGTGGAGCGTGCCCGGTGCCGCTGCAGCTGCCGCCGCTGGAACGCCTGACC
CTGGATGATAAAAAACCGCTGAATACCCTGATCTCTGCTACTGGTCTGTGGATGAGTCGTACCGGAACCATTCAT
AAAATCAAACACCACGAGGTTAGCCGTTCGAAAATCTATATTGAGATGGCGTGTGGCGATCATCTGGTTGTGAAC
AATAGCCGCTCTTCTCGTACAGCACGTGCACTGCGTCACCACAAATATCGTAAAACCTGTAAACGTTGCCGTGTG
TCCGATGAGGATCTGAACAAATTCCTGACAAAAGCCAATGAGGACCAAACAAGCGTGAAAGTGAAAGTCGTTAGC
GCTCCTACCCGTACTAAAAAAGCAATGCCGAAATCCGTTGCTCGTGCCCCTAAACCACTGGAAAACACTGAAGCA
GCACAGGCACAGCCGTCTGGAAGCAAATTCTCTCCGGCCATTCCTGTTTCTACCCAGGAGTCCGTTTCTGTTCCA
GCAAGTGTGAGCACCAGCATTAGCAGTATTAGCACCGGTGCCACCGCTAGCGCCCTGGTTAAAGGCAATACCAAT
CCGATTACAAGCATGTCTGCCCCGGTTCAAGCATCAGCTCCAGCACTGACAAAATCCCAAACCGATCGTCTGGAG
GTTCTGCTGAATCCGAAAGACGAAATCAGCCTGAATTCCGGCAAACCGTTTCGTGAACTGGAGAGCGAACTGCTG
TCACGTCGTAAAAAAGACCTGCAACAAATCTATGCCGAAGAACGTGAGAACTATCTGGGGAAACTGGAACGTGAA
ATCACCCGCTTTTTCGTGGATCGTGGCTTTCTGGAGATCAAATCCCCGATTCTGATTCCTCTGGAGTATATCGAG
CGTATGGGCATCGACAATGATACCGAACTGAGCAAACAAATTTTCCGTGTGGATAAAAACTTCTGTCTGCGCCCT
ATGCTAGCACCAAATCTGGCTAACTATCTGCGCAAACTGGACCGTGCCCTGCCTGATCCTATCAAAATCTTCGAG
ATCGGCCCGTGTTATCGTAAAGAGTCCGACGGTAAAGAACATCTGGAGGAGTTTACCATGCTGAACTTTTGCCAA
ATGGGTTCAGGTTGTACTCGTGAGAACCTGGAAAGCATCATCACCGATTTTCTGAACCACCTGGGCATTGACTTC
AAAATTGTGGGCGACAGCTGTATGGTGTTTGGCGACACCCTGGATGTCATGCACGGCGACCTGGAACTGTCTAGT
GCCGTTGTGGGCCCAATCCCGCTGGATCGTGAGTGGGGTATCGACAAACCTTGGATCGGTGCGGGTTTTGGTCTG
GAGCGTCTGCTGAAAGTAAAACACGACTTCAAGAACATCAAACGTGCTGCACGTTCCGAGTCCTATTACAATGGT
ATTTCTACTAACCTGTAA (SEQ ID NO: 395)

Protein:
MDPVVVLGLCLSCLLLLSLWKQSYGGGMASNDYTQQATQSYGAYPTQPGQGYSQQSSQPYGQQSYSGYSQSTDTS
GYGQSSYSSYGQSQNTGYGTQSTPQGYGSTGGYGSSQSSQSSYGQQSSYPGYGQQPAPSSTSGSYGSSSQSSSYG
QPQSGSYSQQPSYGGQQQSYGQQQSYNPPQGYGQQNQYNSSSGGGGGGGGGNYGQDQSSMSSGGGSGGGYGNQD
QSGGGGSGGYGQQDRGGRGRGGSGGGGGGGGGGYNRSSGGYEPRGRGGGRGGRGGMGGSDRGGFNKFGGPRDQGS
RHDSEQDNSDNNTIFVQGLGENVTIESVADYFKQIGIIKTNKKTGQPMINLYTDRETGKLKGEATVSFDDPPSAK
AAIDWFDGKEFSGNPIKVSFATRRADFNRGGGNGRGGRGRGGPMGRGGYGGGGSGGGGRGGFPSGGGGGGGQQRA
GDWKCPNPTCENMNFSWRNECNQCKAPKPDGPGGGPGGSHMGGNYGDDRRGGRGGAIAGKPIPNPLLGLDSTGAP
GSAGSAAGSGACPVPLQLPPLERLTLDDKKPLNTLISATGLWMSRTGTIHKIKHHEVSRSKIYIEMACGDHLVVN
NSRSSRTARALRHHKYRKTCKRCRVSDEDLNKFLTKANEDQTSVKVKVVSAPTRTKKAMPKSVARAPKPLENTEA
AQAQPSGSKFSPAIPVSTQESVSVPASVSTSISSISTGATASALVKGNTNPITSMSAPVQASAPALTKSQTDRLE
VLLNPKDEISLNSGKPFRELESELLSRRKKDLQQIYAEERENYLGKLEREITRFFVDRGFLEIKSPILIPLEYIE
RMGIDNDTELSKQIFRVDKNFCLRPMLAPNLANYLRKLDRALPDPIKIFEIGPCYRKESDGKEHLEEFTMLNFCQ
MGSGCTRENLESIITDFLNHLGIDFKIVGDSCMVFGDTLDVMHGDLELSSAVVGPIPLDREWGIDKPWIGAGFGL
ERLLKVKHDFKNIKRAARSESYYNGISTNL* (SEQ ID NO: 396)

P450 2C1₁₋₂₇::EWSR1::MCP
DNA:
ATGGACCCCGTGGTCGTGCTGGGCCTGTGCCTGTCATGCCTGCTGCTGCTGAGCCTGTGGAAGCAGAGCTACGGC
GGAGGCATGGCGTCCACGGATTACAGTACCTATAGCCAAGCTGCAGCGCAGCAGGGCTACAGTGCTTACACCGCC
CAGCCCACTCAAGGATATGCACAGACCACCCAGGCATATGGGCAACAAAGCTATGGAACCTATGGACAGCCCACT
GATGTCAGCTATACCCAGGCTCAGACCACTGCAACCTATGGGCAGACCGCCTATGCAACTTCTTATGGACAGCCT
CCCACTGGTTATACTACTCCAACTGCCCCCCAGGCATACAGCCAGCCTGTCCAGGGGTATGGCACTGGTGCTTAT
GATACCACCACTGCTACAGTCACCACCACCCAGGCCTCCTATGCAGCTCAGTCTGCATATGGCACTCAGCCTGCT
TATCCAGCCTATGGGCAGCAGCCAGCAGCCACTGCACCTACAAGACCGCAGGATGGAAACAAGCCCACTGAGACT
AGTCAACCTCAATCTAGCACAGGGGGTTACAACCAGCCCAGCCTAGGATATGGACAGAGTAACTACAGTTATCCC
CAGGTACCTGGGAGCTACCCCATGCAGCCAGTCACTGCACCTCCATCCTACCCTCCTACCAGCTATTCCTCTACA
CAGCCGACTAGTTATGATCAGAGCAGTTACTCTCAGCAGAACACCTATGGGCAACCGAGCAGCTATGGACAGCAG
AGTAGCTATGGTCAACAAAGCAGCTATGGGCAGCAGCCTCCCACTAGTTACCCACCCCAAACTGGATCCTACAGC
CAAGCTCCAAGTCAATATAGCCAACAGAGCAGCAGCTACGGGCAGCAGAGTTCATTCCGACAGGACCACCCCAGT
AGCATGGGTGTTTATGGGCAGGAGTCTGGAGGATTTTCCGGACCAGGAGAGAACCGGAGCATGAGTGGCCCTGAT
AACCGGGGCAGGGGAAGAGGGGGATTTGATCGTGGAGGCATGAGCAGAGGTGGGCGGGGAGGAGGACGCGGTGGA

ATGGGCAGCGCTGGAGAGCGAGGTGGCTTCAATAAGCCTGGTGGACCCATGGATGAAGGACCAGATCTTGATCTA
GGCCCACCTGTAGATCCAGATGAAGACTCTGACAACAGTGCAATTTATGTACAAGGATTAAATGACAGTGTGACT
CTAGATGATCTGGCAGACTTCTTTAAGCAGTGTGGGGTTGTTAAGATGAACAAGAGAACTGGGCAACCCATGATC
CACATCTACCTGGACAAGGAAACAGGAAAGCCCAAAGGCGATGCCACAGTGTCCTATGAAGACCCACCTACTGCC
AAGGCTGCCGTGGAATGGTTTGATGGGAAAGATTTTCAAGGGAGCAAACTTAAAGTCTCCCTTGCTCGGAAGAAG
CCTCCAATGAACAGTATGCGGGGTGGTCTGCCACCCCGTGAGGGCAGAGGCATGCCACCACCACTCCGTGGAGGT
CCAGGAGGCCCAGGAGGTCCTGGGGGACCCATGGGTCGCATGGGAGGCCGTGGAGGAGATAGAGGAGGCTTCCCT
CCAAGAGGACCCCGGGGTTCCCGAGGGAACCCCTCTGGAGGAGGAAACGTCCAGCACCGAGCTGGAGACTGGCAG
TGTCCCAATCCGGGTTGTGGAAACCAGAACTTCGCCTGGAGAACAGAGTGCAACCAGTGTAAGGCCCCAAAGCCT
GAAGGCTTCCTCCCGCCACCCTTTCCGCCCCCGGGTGGTGATCGTGGCAGAGGTGGCCCTGGTGGCATGCGGGGA
GGAAGAGGTGGCCTCATGGATCGTGGTGGTCCCGGTGGAATGTTCAGAGGTGGCCGTGGTGGAGACAGAGGTGGC
TTCCGTGGTGGCCGGGGCATGGACCGAGGTGGCTTTGGTGGAGGAAGACGAGGTGGCCCTGGGGGGCCCCCTGGA
CCTTTGATGGAACAGGCGATCGCATATCCCTATGATGTGCCGGATTATGCTGGAGCACCAGGAAGTGCTGGTTCT
GCTGCTGGTAGTGGAGCTTCTAACTTTACTCAGTTCGTTCTCGTCGACAATGGCGGAACTGGCGACGTGACTGTC
GCCCCAAGCAACTTCGCTAACGGGATCGCTGAATGGATCAGCTCTAACTCGCGTTCACAGGCTTACAAAGTAACC
TGTAGCGTTCGTCAGAGCTCTGCGCAGAATCGCAAATACACCATCAAAGTCGAGGTGCCTAAAGGCGCCTGGCGT
TCGTACTTAAATATGGAACTAACCATTCCAATTTTCGCCACGAATTCCGACTGCGAGCTTATTGTTAAGGCAATG
CAAGGTCTCCTAAAAGATGGAAACCCGATTCCCTCAGCAATCGCAGCAAACTCCGGCATCTACTAA (SEQ ID
NO: 397)

Protein:
MDPVVVLGLCLSCLLLLSLWKQSYGGGMASTDYSTYSQAAAQQGYSAYTAQPTQGYAQTTQAYGQQSYGTYGQPT
DVSYTQAQTTATYGQTAYATSYGQPPTGYTTPTAPQAYSQPVQGYGTGAYDTTTATVTTTQASYAAQSAYGTQPA
YPAYGQQPAATAPTRPQDGNKPTETSQPQSSTGGYNQPSLGYGQSNYSYPQVPGSYPMQPVTAPPSYPPTSYSST
QPTSYDQSSYSQQNTYGQPSSYGQQSSYGQQSSYGQQPPTSYPPQTGSYSQAPSQYSQQSSSYGQQSSFRQDHPS
SMGVYGQESGGFSGPGENRSMSGPDNRGRGRGGFDRGGMSRGGRGGGRGGMGSAGERGGFNKPGGPMDEGPDLDL
GPPVDPDEDSDNSAIYVQGLNDSVTLDDLADFFKQCGVVKMNKRTGQPMIHIYLDKETGKPKGDATVSYEDPPTA
KAAVEWFDGKDFQGSKLKVSLARKKPPMNSMRGGLPPREGRGMPPPLRGGPGGPGGPGGPMGRMGGRGGDRGGFP
PRGPRGSRGNPSGGGNVQHRAGDWQCPNPGCGNQNFAWRTECNQCKAPKPEGFLPPPFPPPGGDRGRGGPGGMRG
GRGGLMDRGGPGGMFRGGRGGDRGGFRGGRGMDRGGFGGGRRGGPGGPPGPLMEQAIAYPYDVPDYAGAPGSAGS
AAGSGASNFTQFVLVDNGGTGDVTVAPSNFANGIAEWISSNSRSQAYKVTCSVRQSSAQNRKYTIKVEVPKGAWR
SYLNMELTIPIFATNSDCELIVKAMQGLLKDGNPIPSAIAANSGIY* (SEQ ID NO: 398)

P450 2C1₁₋₂₇::FUS::MCP::PylRS(AF)
DNA:
ATGGACCCCGTGGTCGTGCTGGGCCTGTGCCTGTCATGCCTGCTGCTGCTGAGCCTGTGGAAGCAGAGCTACGGC
GGAGGCATGGCCTCAAACGATTATACCCAACAAGCAACCCAAAGCTATGGGGCCTACCCCACCCAGCCCGGGCAG
GGCTATTCCCAGCAGAGCAGTCAGCCCTACGGACAGCAGAGTTACAGTGGTTATAGCCAGTCCACGGACACTTCA
GGATATGGCCAGAGCAGCTATTCTTCTTATGGCCAGAGCCAGAACACAGGCTATGGAACTCAGTCAACTCCCCAG
GGATATGGCTCGACTGGCGGCTATGGCAGTAGCCAGAGCTCCCAATCGTCTTACGGGCAGCAGTCCTCCTACCCT
GGCTATGGCCAGCAGCCAGCTCCCAGCAGCACCTCGGGAAGTTACGGTAGCAGTTCTCAGAGCAGCAGCTATGGG
CAGCCCCAGAGTGGGAGCTACAGCCAGCAGCCTAGCTATGGTGGACAGCAGCAAAGCTATGGACAGCAGCAAAGC
TATAATCCCCCTCAGGGCTATGGACAGCAGAACCAGTACAACAGCAGCAGTGGTGGTGGAGGTGGAGGTGGAGGT
GGAGGTAACTATGGCCAAGATCAATCCTCCATGAGTAGTGGTGGTGGCAGTGGTGGCGGTTATGGCAATCAAGAC
CAGAGTGGTGGAGGTGGCAGCGGTGGCTATGGACAGCAGGACCGTGGAGGCCGCGGCAGGGGTGGCAGTGGTGGC
GGCGGCGGCGGCGGCGGTGGTGGTTACAACCGCAGCAGTGGTGGCTATGAACCCAGAGGTCGTGGAGGTGGCCGT
GGAGGCAGAGGTGGCATGGGCGGAAGTGACCGTGGTGGCTTCAATAAATTTGGTGGCCCTCGGGACCAAGGATCA
CGTCATGACTCCGAACAGGATAATTCAGACAACAACACCATCTTTGTGCAAGGCCTGGGTGAGAATGTTACAATT
GAGTCTGTGGCTGATTACTTCAAGCAGATTGGTATTATTAAGACAAACAAGAAAACGGGACAGCCCATGATTAAT
TTGTACACAGACAGGGAAACTGGCAAGCTGAAGGGAGAGGCAACGGTCTCTTTTGATGACCCACCTTCAGCTAAA
GCAGCTATTGACTGGTTTGATGGTAAAGAATTCTCCGGAAATCCTATCAAGGTCTCATTTGCTACTCGCCGGGCA
GACTTTAATCGGGGTGGTGGCAATGGTCGTGGAGGCCGAGGGCGAGGAGGACCCATGGGCCGTGGAGGCTATGGA
GGTGGTGGCAGTGGTGGTGGTGGCCGAGGAGGATTTCCCAGTGGAGGTGGTGGCGGTGGAGGACAGCAGCGAGCT
GGTGACTGGAAGTGTCCTAATCCCACCTGTGAGAATATGAACTTCTCTTGGAGGAATGAATGCAACCAGTGTAAG
GCCCCTAAACCAGATGGCCCAGGAGGGGGACCAGGTGGCTCTCACATGGGGGGTAACTACGGGGATGATCGTCGT
GGTGGCAGAGGAGGCGCGATCGCATATCCCTATGATGTGCCGGATTATGCTGGAGCACCAGGAAGTGCTGGTTCT
GCTGCTGGTAGTGGAGCTTCTAACTTTACTCAGTTCGTTCTCGTCGACAATGGCGGAACTGGCGACGTGACTGTC

GCCCCAAGCAACTTCGCTAACGGGATCGCTGAATGGATCAGCTCTAACTCGCGTTCACAGGCTTACAAAGTAACC
TGTAGCGTTCGTCAGAGCTCTGCGCAGAATCGCAAATACACCATCAAAGTCGAGGTGCCTAAAGGCGCCTGGCGT
TCGTACTTAAATATGGAACTAACCATTCCAATTTTCGCCACGAATTCCGACTGCGAGCTTATTGTTAAGGCAATG
CAAGGTCTCCTAAAAGATGGAAACCCGATTCCCTCAGCAATCGCAGCAAACTCCGGCATCTACGGCGCCGATTAC
AAGGACGATGATGACAAGGGAGCACCAGGAAGTGCTGGTTCTGCTGCTGGTAGTGGAGCGTGCCCGGTGCCGCTG
CAGCTGCCGCCGCTGGAACGCCTGACCCTGGATGATAAAAAACCGCTGAATACCCTGATCTCTGCTACTGGTCTG
TGGATGAGTCGTACCGGAACCATTCATAAAATCAAACACCACGAGGTTAGCCGTTCGAAAATCTATATTGAGATG
GCGTGTGGCGATCATCTGGTTGTGAACAATAGCCGCTCTTCTCGTACAGCACGTGCACTGCGTCACCACAAATAT
CGTAAAACCTGTAAACGTTGCCGTGTGTCCGATGAGGATCTGAACAAATTCCTGACAAAAGCCAATGAGGACCAA
ACAAGCGTGAAAGTGAAAGTCGTTAGCGCTCCTACCCGTACTAAAAAAGCAATGCCGAAATCCGTTGCTCGTGCC
CCTAAACCACTGGAAAACACTGAAGCAGCACAGGCACAGCCGTCTGGAAGCAAATTCTCTCCGGCCATTCCTGTT
TCTACCCAGGAGTCCGTTTCTGTTCCAGCAAGTGTGAGCACCAGCATTAGCAGTATTAGCACCGGTGCCACCGCT
AGCGCCCTGGTTAAAGGCAATACCAATCCGATTACAAGCATGTCTGCCCCGGTTCAAGCATCAGCTCCAGCACTG
ACAAAATCCCAAACCGATCGTCTGGAGGTTCTGCTGAATCCGAAAGACGAAATCAGCCTGAATTCCGGCAAACCG
TTTCGTGAACTGGAGAGCGAACTGCTGTCACGTCGTAAAAAAGACCTGCAACAAATCTATGCCGAAGAACGTGAG
AACTATCTGGGGAAACTGGAACGTGAAATCACCCGCTTTTTCGTGGATCGTGGCTTTCTGGAGATCAAATCCCCG
ATTCTGATTCCTCTGGAGTATATCGAGCGTATGGGCATCGACAATGATACCGAACTGAGCAAACAAATTTTCCGT
GTGGATAAAAACTTCTGTCTGCGCCCTATGCTAGCACCAAATCTGGCTAACTATCTGCGCAAACTGGACCGTGCC
CTGCCTGATCCTATCAAAATCTTCGAGATCGGCCCGTGTTATCGTAAAGAGTCCGACGGTAAAGAACATCTGGAG
GAGTTTACCATGCTGAACTTTTGCCAAATGGGTTCAGGTTGTACTCGTGAGAACCTGGAAAGCATCATCACCGAT
TTTCTGAACCACCTGGGCATTGACTTCAAAATTGTGGGCGACAGCTGTATGGTGTTTGGCGACACCCTGGATGTC
ATGCACGGCGACCTGGAACTGTCTAGTGCCGTTGTGGGCCCAATCCCGCTGGATCGTGAGTGGGGTATCGACAAA
CCTTGGATCGGTGCGGGTTTTGGTCTGGAGCGTCTGCTGAAAGTAAAACACGACTTCAAGAACATCAAACGTGCT
GCACGTTCCGAGTCCTATTACAATGGTATTTCTACTAACCTGTAA (SEQ ID NO: 399)

Protein:
MDPVVVLGLCLSCLLLLSLWKQSYGGGMASNDYTQQATQSYGAYPTQPGQGYSQQSSQPYGQQSYSGYSQSTDTS
GYGQSSYSSYGQSQNTGYGTQSTPQGYGSTGGYGSSQSSQSSYGQQSSYPGYGQQPAPSSTSGSYGSSSQSSSYG
QPQSGSYSQQPSYGGQQQSYGQQQSYNPPQGYGQQNQYNSSSGGGGGGGGGNYGQDQSSMSSGGGSGGGYGNQD
QSGGGGSGGYGQQDRGGRGRGGSGGGGGGGGGGYNRSSGGYEPRGRGGGRGGRGGMGGSDRGGFNKFGGPRDQGS
RHDSEQDNSDNNTIFVQGLGENVTIESVADYFKQIGIIKTNKKTGQPMINLYTDRETGKLKGEATVSFDDPPSAK
AAIDWFDGKEFSGNPIKVSFATRRADFNRGGGNGRGGRGRGGPMGRGGYGGGGSGGGGRGGFPSGGGGGGGQQRA
GDWKCPNPTCENMNFSWRNECNQCKAPKPDGPGGGPGGSHMGGNYGDDRRGGRGGAIAYPYDVPDYAGAPGSAGS
AAGSGASNFTQFVLVDNGGTGDVTVAPSNFANGIAEWISSNSRSQAYKVTCSVRQSSAQNRKYTIKVEVPKGAWR
SYLNMELTIPIFATNSDCELIVKAMQGLLKDGNPIPSAIAANSGIYGADYKDDDDKGAPGSAGSAAGSGACPVPL
QLPPLERLTLDDKKPLNTLISATGLWMSRTGTIHKIKHHEVSRSKIYIEMACGDHLVVNNSRSSRTARALRHHKY
RKTCKRCRVSDEDLNKFLTKANEDQTSVKVKVVSAPTRTKKAMPKSVARAPKPLENTEAAQAQPSGSKFSPAIPV
STQESVSVPASVSTSISSISTGATASALVKGNTNPITSMSAPVQASAPALTKSQTDRLEVLLNPKDEISLNSGKP
FRELESELLSRRKKDLQQIYAEERENYLGKLEREITRFFVDRGFLEIKSPILIPLEYIERMGIDNDTELSKQIFR
VDKNFCLRPMLAPNLANYLRKLDRALPDPIKIFEIGPCYRKESDGKEHLEEFTMLNFCQMGSGCTRENLESIITD
FLNHLGIDFKIVGDSCMVFGDTLDVMHGDLELSSAVVGPIPLDREWGIDKPWIGAGFGLERLLKVKHDFKNIKRA
ARSESYYNGISTNL* (SEQ ID NO: 400)

P450 2C1₁₋₂₉::FUS::MCP::PylRS(AF)
DNA:
ATGGACCCCGTGGTCGTGCTGGGCCTGTGCCTGTCATGCCTGCTGCTGCTGAGCCTGTGGAAGCAGAGCTACGGC
GGAGGCAAGCTGATGGCCTCAAACGATTATACCCAACAAGCAACCCAAAGCTATGGGGCCTACCCCACCCAGCCC
GGGCAGGGCTATTCCCAGCAGAGCAGTCAGCCCTACGGACAGCAGAGTTACAGTGGTTATAGCCAGTCCACGGAC
ACTTCAGGATATGGCCAGAGCAGCTATTCTTCTTATGGCCAGAGCCAGAACACAGGCTATGGAACTCAGTCAACT
CCCCAGGGATATGGCTCGACTGGCGGCTATGGCAGTAGCCAGAGCTCCCAATCGTCTTACGGGCAGCAGTCCTCC
TACCCTGGCTATGGCCAGCAGCCAGCTCCCAGCAGCACCTCGGGAAGTTACGGTAGCAGTTCTCAGAGCAGCAGC
TATGGGCAGCCCCAGAGTGGGAGCTACAGCCAGCAGCCTAGCTATGGTGGACAGCAGCAAAGCTATGGACAGCAG
CAAAGCTATAATCCCCCTCAGGGCTATGGACAGCAGAACCAGTACAACAGCAGCAGTGGTGGTGGAGGTGGAGGT
GGAGGTGGAGGTAACTATGGCCAAGATCAATCCTCCATGAGTAGTGGTGGTGGCAGTGGTGGCGGTTATGGCAAT
CAAGACCAGAGTGGTGGAGGTGGCAGCGGTGGCTATGGACAGCAGGACCGTGGAGGCCGCGGCAGGGGTGGCAGT
GGTGGCGGCGGCGGCGGCGGCGGTGGTGGTTACAACCGCAGCAGTGGTGGCTATGAACCCAGAGGTCGTGGAGGT
GGCCGTGGAGGCAGAGGTGGCATGGGCGGAAGTGACCGTGGTGGCTTCAATAAATTTGGTGGCCCTCGGGACCAA

GGATCACGTCATGACTCCGAACAGGATAATTCAGACAACAACACCATCTTTGTGCAAGGCCTGGGTGAGAATGTT
ACAATTGAGTCTGTGGCTGATTACTTCAAGCAGATTGGTATTATTAAGACAAACAAGAAAACGGGACAGCCCATG
ATTAATTTGTACACAGACAGGGAAACTGGCAAGCTGAAGGGAGAGGCAACGGTCTCTTTTGATGACCCACCTTCA
GCTAAAGCAGCTATTGACTGGTTTGATGGTAAAGAATTCTCCGGAAATCCTATCAAGGTCTCATTTGCTACTCGC
CGGGCAGACTTTAATCGGGGTGGTGGCAATGGTCGTGGAGGCCGAGGGCGAGGAGGACCCATGGGCCGTGGAGGC
TATGGAGGTGGTGGCAGTGGTGGTGGTGGCCGAGGAGGATTTCCCAGTGGAGGTGGTGGCGGTGGAGGACAGCAG
CGAGCTGGTGACTGGAAGTGTCCTAATCCCACCTGTGAGAATATGAACTTCTCTTGGAGGAATGAATGCAACCAG
TGTAAGGCCCCTAAACCAGATGGCCCAGGAGGGGGACCAGGTGGCTCTCACATGGGGGGTAACTACGGGGATGAT
CGTCGTGGTGGCAGAGGAGGCGCGATCGCATATCCCTATGATGTGCCGGATTATGCTGGAGCACCAGGAAGTGCT
GGTTCTGCTGCTGGTAGTGGAGCTTCTAACTTTACTCAGTTCGTTCTCGTCGACAATGGCGGAACTGGCGACGTG
ACTGTCGCCCCAAGCAACTTCGCTAACGGGATCGCTGAATGGATCAGCTCTAACTCGCGTTCACAGGCTTACAAA
GTAACCTGTAGCGTTCGTCAGAGCTCTGCGCAGAATCGCAAATACACCATCAAAGTCGAGGTGCCTAAAGGCGCC
TGGCGTTCGTACTTAAATATGGAACTAACCATTCCAATTTTCGCCACGAATTCCGACTGCGAGCTTATTGTTAAG
GCAATGCAAGGTCTCCTAAAAGATGGAAACCCGATTCCCTCAGCAATCGCAGCAAACTCCGGCATCTACGGCGCC
GATTACAAGGACGATGATGACAAGGGAGCACCAGGAAGTGCTGGTTCTGCTGCTGGTAGTGGAGCGTGCCCGGTG
CCGCTGCAGCTGCCGCCGCTGGAACGCCTGACCCTGGATGATAAAAAACCGCTGAATACCCTGATCTCTGCTACT
GGTCTGTGGATGAGTCGTACCGGAACCATTCATAAAATCAAACACCACGAGGTTAGCCGTTCGAAAATCTATATT
GAGATGGCGTGTGGCGATCATCTGGTTGTGAACAATAGCCGCTCTTCTCGTACAGCACGTGCACTGCGTCACCAC
AAATATCGTAAAACCTGTAAACGTTGCCGTGTGTCCGATGAGGATCTGAACAAATTCCTGACAAAAGCCAATGAG
GACCAAACAAGCGTGAAAGTGAAAGTCGTTAGCGCTCCTACCCGTACTAAAAAAGCAATGCCGAAATCCGTTGCT
CGTGCCCCTAAACCACTGGAAAACACTGAAGCAGCACAGGCACAGCCGTCTGGAAGCAAATTCTCTCCGGCCATT
CCTGTTTCTACCCAGGAGTCCGTTTCTGTTCCAGCAAGTGTGAGCACCAGCATTAGCAGTATTAGCACCGGTGCC
ACCGCTAGCGCCCTGGTTAAAGGCAATACCAATCCGATTACAAGCATGTCTGCCCCGGTTCAAGCATCAGCTCCA
GCACTGACAAAATCCCAAACCGATCGTCTGGAGGTTCTGCTGAATCCGAAAGACGAAATCAGCCTGAATTCCGGC
AAACCGTTTCGTGAACTGGAGAGCGAACTGCTGTCACGTCGTAAAAAAGACCTGCAACAAATCTATGCCGAAGAA
CGTGAGAACTATCTGGGGAAACTGGAACGTGAAATCACCCGCTTTTTCGTGGATCGTGGCTTTCTGGAGATCAAA
TCCCCGATTCTGATTCCTCTGGAGTATATCGAGCGTATGGGCATCGACAATGATACCGAACTGAGCAAACAAATT
TTCCGTGTGGATAAAAACTTCTGTCTGCGCCCTATGCTAGCACCAAATCTGGCTAACTATCTGCGCAAACTGGAC
CGTGCCCTGCCTGATCCTATCAAAATCTTCGAGATCGGCCCGTGTTATCGTAAAGAGTCCGACGGTAAAGAACAT
CTGGAGGAGTTTACCATGCTGAACTTTTGCCAAATGGGTTCAGGTTGTACTCGTGAGAACCTGGAAAGCATCATC
ACCGATTTTCTGAACCACCTGGGCATTGACTTCAAAATTGTGGGCGACAGCTGTATGGTGTTTGGCGACACCCTG
GATGTCATGCACGGCGACCTGGAACTGTCTAGTGCCGTTGTGGGCCCAATCCCGCTGGATCGTGAGTGGGGTATC
GACAAACCTTGGATCGGTGCGGGTTTTGGTCTGGAGCGTCTGCTGAAAGTAAAACACGACTTCAAGAACATCAAA
CGTGCTGCACGTTCCGAGTCCTATTACAATGGTATTTCTACTAACCTGTAA (SEQ ID NO: 401)

Protein:
MDPVVVLGLCLSCLLLLSLWKQSYGGGKLMASNDYTQQATQSYGAYPTQPGQGYSQQSSQPYGQQSYSGYSQSTD
TSGYGQSSYSSYGQSQNTGYGTQSTPQGYGSTGGYGSSQSSQSSYGQQSSYPGYGQQPAPSSTSGSYGSSSQSSS
YGQPQSGSYSQQPSYGGQQQSYGQQQSYNPPQGYGQQNQYNSSSGGGGGGGGGNYGQDQSSMSSGGGSGGGYGN
QDQSGGGGSGGYGQQDRGGRGRGGSGGGGGGGGGGYNRSSGGYEPRGRGGGRGGRGGMGGSDRGGFNKFGGPRDQ
GSRHDSEQDNSDNNTIFVQGLGENVTIESVADYFKQIGIIKTNKKTGQPMINLYTDRETGKLKGEATVSFDDPPS
AKAAIDWFDGKEFSGNPIKVSFATRRADFNRGGGNGRGGRGRGGPMGRGGYGGGGSGGGGRGGFPSGGGGGGGQQ
RAGDWKCPNPTCENMNFSWRNECNQCKAPKPDGPGGGPGGSHMGGNYGDDRRGGRGGAIAYPYDVPDYAGAPGSA
GSAAGSGASNFTQFVLVDNGGTGDVTVAPSNFANGIAEWISSNSRSQAYKVTCSVRQSSAQNRKYTIKVEVPKGA
WRSYLNMELTIPIFATNSDCELIVKAMQGLLKDGNPIPSAIAANSGIYGADYKDDDDKGAPGSAGSAAGSGACPV
PLQLPPLERLTLDDKKPLNTLISATGLWMSRTGTIHKIKHHEVSRSKIYIEMACGDHLVVNNSRSSRTARALRHH
KYRKTCKRCRVSDEDLNKFLTKANEDQTSVKVKVVSAPTRTKKAMPKSVARAPKPLENTEAAQAQPSGSKFSPAI
PVSTQESVSVPASVSTSISSISTGATASALVKGNTNPITSMSAPVQASAPALTKSQTDRLEVLLNPKDEISLNSG
KPFRELESELLSRRKKDLQQIYAEERENYLGKLEREITRFFVDRGFLEIKSPILIPLEYIERMGIDNDTELSKQI
FRVDKNFCLRPMLAPNLANYLRKLDRALPDPIKIFEIGPCYRKESDGKEHLEEFTMLNFCQMGSGCTRENLESII
TDFLNHLGIDFKIVGDSCMVFGDTLDVMHGDLELSSAVVGPIPLDREWGIDKPWIGAGFGLERLLKVKHDFKNIK
RAARSESYYNGISTNL* (SEQ ID NO: 402)

EB1::PylRS(AF)
DNA:
ATGGCAGTGAACGTATACTCAACGTCAGTGACCAGTGATAACCTAAGTCGACATGACATGCTGGCCTGGATCAAT
GAGTCTCTGCAGTTGAATCTGACAAAGATCGAACAGTTGTGCTCAGGGGCTGCGTATTGTCAGTTTATGGACATG

CTGTTCCCTGGCTCCATTGCCTTGAAGAAAGTGAAATTCCAAGCTAAGCTAGAACACGAGTACATCCAGAACTTC
AAAATACTACAAGCAGGTTTTAAGAGAATGGGTGTTGACAAAATAATTCCTGTGGACAAATTAGTAAAAGGAAAG
TTTCAGGACAATTTTGAATTCGTTCAGTGGTTCAAGAAGTTTTTCGATGCAAACTATGATGGAAAAGACTATGAC
CCTGTGGCTGCCAGACAAGGTCAAGAAACTGCAGTGGCTCCTTCCCTTGTTGCTCCAGCTCTGAATAAACCGAAG
AAACCTCTCACTTCTAGCAGTGCAGCTCCCCAGAGGCCCATCTCAACACAGAGAACCGCTGCGGCTCCTAAGGCT
GGCCCTGGTGTGGTGCGAAAGAACCCTGGTGTGGGCAACGGAGATGACGAGGCAGCTGAGTTGATGCAGCAGGTC
AACGTATTGAAACTTACTGTTGAAGACTTGGAGAAAGAGAGGGATTTCTACTTCGGAAAGCTACGGAACATTGAA
TTGATTTGCCAGGAGAACGAGGGGGAAAACGACCCTGTATTGCAGAGGATTGTAGACATTCTGTATGCCACAGAT
GAAGGCTTTGTGATACCTGATGAAGGGGGCCCACAGGAGGAGCAAGAAGAGTATGGCGCCGATTACAAGGACGAT
GATGACAAGGGAGCACCAGGAAGTGCTGGTTCTGCTGCTGGTAGTGGAGCGTGCCCGGTGCCGCTGCAGCTGCCG
CCGCTGGAACGCCTGACCCTGGATGATAAAAAACCGCTGAATACCCTGATCTCTGCTACTGGTCTGTGGATGAGT
CGTACCGGAACCATTCATAAAATCAAACACCACGAGGTTAGCCGTTCGAAAATCTATATTGAGATGGCGTGTGGC
GATCATCTGGTTGTGAACAATAGCCGCTCTTCTCGTACAGCACGTGCACTGCGTCACCACAAATATCGTAAAACC
TGTAAACGTTGCCGTGTGTCCGATGAGGATCTGAACAAATTCCTGACAAAAGCCAATGAGGACCAAACAAGCGTG
AAAGTGAAAGTCGTTAGCGCTCCTACCCGTACTAAAAAAGCAATGCCGAAATCCGTTGCTCGTGCCCCTAAACCA
CTGGAAAACACTGAAGCAGCACAGGCACAGCCGTCTGGAAGCAAATTCTCTCCGGCCATTCCTGTTTCTACCCAG
GAGTCCGTTTCTGTTCCAGCAAGTGTGAGCACCAGCATTAGCAGTATTAGCACCGGTGCCACCGCTAGCGCCCTG
GTTAAAGGCAATACCAATCCGATTACAAGCATGTCTGCCCCGGTTCAAGCATCAGCTCCAGCACTGACAAAATCC
CAAACCGATCGTCTGGAGGTTCTGCTGAATCCGAAAGACGAAATCAGCCTGAATTCCGGCAAACCGTTTCGTGAA
CTGGAGAGCGAACTGCTGTCACGTCGTAAAAAAGACCTGCAACAAATCTATGCCGAAGAACGTGAGAACTATCTG
GGAAAACTGGAACGTGAAATCACCCGCTTTTTCGTGGATCGTGGCTTTCTGGAGATCAAATCCCCGATTCTGATT
CCTCTGGAGTATATCGAGCGTATGGGCATCGACAATGATACCGAACTGAGCAAACAAATTTTCCGTGTGGATAAA
AACTTCTGTCTGCGCCCTATGCTAGCACCAAATCTGGCTAACTATCTGCGCAAACTGGACCGTGCCCTGCCTGAT
CCTATCAAAATCTTCGAGATCGGCCCGTGTTATCGTAAAGAGTCCGACGGTAAAGAACATCTGGAGGAGTTTACC
ATGCTGAACTTTTGCCAAATGGGTTCAGGTTGTACTCGTGAGAACCTGGAAAGCATCATCACCGATTTTCTGAAC
CACCTGGGCATTGACTTCAAAATTGTGGGCGACAGCTGTATGGTGTTTGGCGACACCCTGGATGTCATGCACGGC
GACCTGGAACTGTCTAGTGCCGTTGTGGGCCCAATCCCGCTGGATCGTGAGTGGGGTATCGACAAACCTTGGATC
GGTGCGGGTTTTGGTCTGGAGCGTCTGCTGAAAGTAAAACACGACTTCAAGAACATCAAACGTGCTGCACGTTCC
GAGTCCTATTACAATGGTATTTCTACTAACCTGTAA (SEQ ID NO: 403)

Protein:
MAVNVYSTSVTSDNLSRHDMLAWINESLQLNLTKIEQLCSGAAYCQFMDMLFPGSIALKKVKFQAKLEHEYIQNF
KILQAGFKRMGVDKIIPVDKLVKGKFQDNFEFVQWFKKFFDANYDGKDYDPVAARQGQETAVAPSLVAPALNKPK
KPLTSSSAAPQRPISTQRTAAAPKAGPGVVRKNPGVGNGDDEAAELMQQVNVLKLTVEDLEKERDFYFGKLRNIE
LICQENEGENDPVLQRIVDILYATDEGFVIPDEGGPQEEQEEYGADYKDDDDKGAPGSAGSAAGSGACPVPLQLP
PLERLTLDDKKPLNTLISATGLWMSRTGTIHKIKHHEVSRSKIYIEMACGDHLVVNNSRSSRTARALRHHKYRKT
CKRCRVSDEDLNKFLTKANEDQTSVKVKVVSAPTRTKKAMPKSVARAPKPLENTEAAQAQPSGSKFSPAIPVSTQ
ESVSVPASVSTSISSISTGATASALVKGNTNPITSMSAPVQASAPALTKSQTDRLEVLLNPKDEISLNSGKPFRE
LESELLSRRKKDLQQIYAEERENYLGKLEREITRFFVDRGFLEIKSPILIPLEYIERMGIDNDTELSKQIFRVDK
NFCLRPMLAPNLANYLRKLDRALPDPIKIFEIGPCYRKESDGKEHLEEFTMLNFCQMGSGCTRENLESIITDFLN
HLGIDFKIVGDSCMVFGDTLDVMHGDLELSSAVVGPIPLDREWGIDKPWIGAGFGLERLLKVKHDFKNIKRAARS
ESYYNGISTNL* (SEQ ID NO: 404)

EB1::PylRS(AA)
DNA:
ATGGCAGTGAACGTATACTCAACGTCAGTGACCAGTGATAACCTAAGTCGACATGACATGCTGGCCTGGATCAAT
GAGTCTCTGCAGTTGAATCTGACAAAGATCGAACAGTTGTGCTCAGGGGCTGCGTATTGTCAGTTTATGGACATG
CTGTTCCCTGGCTCCATTGCCTTGAAGAAAGTGAAATTCCAAGCTAAGCTAGAACACGAGTACATCCAGAACTTC
AAAATACTACAAGCAGGTTTTAAGAGAATGGGTGTTGACAAAATAATTCCTGTGGACAAATTAGTAAAAGGAAAG
TTTCAGGACAATTTTGAATTCGTTCAGTGGTTCAAGAAGTTTTTCGATGCAAACTATGATGGAAAAGACTATGAC
CCTGTGGCTGCCAGACAAGGTCAAGAAACTGCAGTGGCTCCTTCCCTTGTTGCTCCAGCTCTGAATAAACCGAAG
AAACCTCTCACTTCTAGCAGTGCAGCTCCCCAGAGGCCCATCTCAACACAGAGAACCGCTGCGGCTCCTAAGGCT
GGCCCTGGTGTGGTGCGAAAGAACCCTGGTGTGGGCAACGGAGATGACGAGGCAGCTGAGTTGATGCAGCAGGTC
AACGTATTGAAACTTACTGTTGAAGACTTGGAGAAAGAGAGGGATTTCTACTTCGGAAAGCTACGGAACATTGAA
TTGATTTGCCAGGAGAACGAGGGGGAAAACGACCCTGTATTGCAGAGGATTGTAGACATTCTGTATGCCACAGAT
GAAGGCTTTGTGATACCTGATGAAGGGGGCCCACAGGAGGAGCAAGAAGAGTATGGCGCCGATTACAAGGACGAT
GATGACAAGGGAGCACCAGGAAGTGCTGGTTCTGCTGCTGGTAGTGGAGCGTGCCCGGTGCCGCTGCAGCTGCCG

CCGCTGGAACGCCTGACCCTGGATGACAAAAAACCGCTGAATACCCTGATCTCTGCTACTGGTCTGTGGATGAGT
CGTACCGGAACCATTCATAAAATCAAACACCACGAGGTTAGCCGTTCGAAAATCTATATTGAGATGGCGTGTGGC
GATCATCTGGTTGTGAACAATAGCCGCTCTTCTCGTACAGCACGTGCACTGCGTCACCACAAATATCGTAAAACC
TGTAAACGTTGCCGTGTGTCCGATGAGGATCTGAACAAATTCCTGACAAAAGCCAATGAGGACCAAACAAGCGTG
AAAGTGAAAGTCGTTAGCGCTCCTACCCGTACTAAAAAAGCAATGCCGAAATCCGTTGCTCGTGCCCCTAAACCA
CTGGAAAACACTGAAGCAGCACAGGCACAGCCGTCTGGAAGCAAATTCTCTCCGGCCATTCCTGTTTCTACCCAG
GAGTCCGTTTCTGTTCCAGCAAGTGTGAGCACCAGCATTAGCAGTATTAGCACCGGTGCCACCGCTAGCGCCCTG
GTTAAAGGCAATACCAATCCGATTACAAGCATGTCTGCCCCGGTTCAAGCATCAGCTCCAGCACTGACAAAATCC
CAAACCGATCGTCTGGAGGTTCTGCTGAATCCGAAAGACGAAATCAGCCTGAATTCCGGCAAACCGTTTCGTGAA
CTGGAGAGCGAACTGCTGTCACGTCGTAAAAAAGACCTGCAACAAATCTATGCCGAAGAACGTGAGAACTATCTG
GGGAAACTGGAACGTGAAATCACCCGCTTTTTCGTGGATCGTGGCTTTCTGGAGATCAAATCCCCGATTCTGATT
CCTCTGGAGTATATCGAGCGTATGGGCATCGACAATGATACCGAACTGAGCAAACAAATTTTCCGTGTGGATAAA
AACTTCTGTCTGCGCCCTATGCTGGCACCAAATCTGTATAACTATCTGCGCAAACTGGACCGTGCCCTGCCTGAT
CCTATCAAAATCTTCGAGATCGGCCCGTGTTATCGTAAAGAGTCCGACGGTAAAGAACATCTGGAGGAGTTTACC
ATGCTGGCCTTTGCCCAAATGGGTTCAGGTTGTACTCGTGAGAACCTGGAAAGCATCATCACCGATTTTCTGAAC
CACCTGGGCATTGACTTCAAAATTGTGGGCGACAGCTGTATGGTGTATGGCGACACCCTGGATGTCATGCACGGC
GACCTGGAACTGTCTAGTGCCGTTGTTGGACCAATTCCGCTGGACCGTGAGTGGGGTATCGACAAACCGTGGATC
GGAGCAGGATTCGGTCTGGAACGCCTGCTGAAAGTGAAACACGACTTCAAAAACATCAAACGTGCCGCCCGTTCT
GAATCGTATTATAACGGGATCTCTACGAACCTGTAA (SEQ ID NO: 405)

Protein:
MAVNVYSTSVTSDNLSRHDMLAWINESLQLNLTKIEQLCSGAAYCQFMDMLFPGSIALKKVKFQAKLEHEYIQNF
KILQAGFKRMGVDKIIPVDKLVKGKFQDNFEFVQWFKKFFDANYDGKDYDPVAARQGQETAVAPSLVAPALNKPK
KPLTSSSAAPQRPISTQRTAAAPKAGPGVVRKNPGVGNGDDEAAELMQQVNVLKLTVEDLEKERDFYFGKLRNIE
LICQENEGENDPVLQRIVDILYATDEGFVIPDEGGPQEEQEEYGADYKDDDDKGAPGSAGSAAGSGACPVPLQLP
PLERLTLDDKKPLNTLISATGLWMSRTGTIHKIKHHEVSRSKIYIEMACGDHLVVNNSRSSRTARALRHHKYRKT
CKRCRVSDEDLNKFLTKANEDQTSVKVKVVSAPTRTKKAMPKSVARAPKPLENTEAAQAQPSGSKFSPAIPVSTQ
ESVSVPASVSTSISSISTGATASALVKGNTNPITSMSAPVQASAPALTKSQTDRLEVLLNPKDEISLNSGKPFRE
LESELLSRRKKDLQQIYAEERENYLGKLEREITRFFVDRGFLEIKSPILIPLEYIERMGIDNDTELSKQIFRVDK
NFCLRPMLAPNLYNYLRKLDRALPDPIKIFEIGPCYRKESDGKEHLEEFTMLAFAQMGSGCTRENLESIITDFLN
HLGIDFKIVGDSCMVYGDTLDVMHGDLELSSAVVGPIPLDREWGIDKPWIGAGFGLERLLKVKHDFKNIKRAARS
ESYYNGISTNL* (SEQ ID NO: 406)

EB1::PylRS(AAAF)
DNA:
ATGGCAGTGAACGTATACTCAACGTCAGTGACCAGTGATAACCTAAGTCGACATGACATGCTGGCCTGGATCAAT
GAGTCTCTGCAGTTGAATCTGACAAAGATCGAACAGTTGTGCTCAGGGGCTGCGTATTGTCAGTTTATGGACATG
CTGTTCCCTGGCTCCATTGCCTTGAAGAAAGTGAAATTCCAAGCTAAGCTAGAACACGAGTACATCCAGAACTTC
AAAATACTACAAGCAGGTTTTAAGAGAATGGGTGTTGACAAAATAATTCCTGTGGACAAATTAGTAAAAGGAAAG
TTTCAGGACAATTTTGAATTCGTTCAGTGGTTCAAGAAGTTTTTCGATGCAAACTATGATGGAAAAGACTATGAC
CCTGTGGCTGCCAGACAAGGTCAAGAAACTGCAGTGGCTCCTTCCCTTGTTGCTCCAGCTCTGAATAAACCGAAG
AAACCTCTCACTTCTAGCAGTGCAGCTCCCCAGAGGCCCATCTCAACACAGAGAACCGCTGCGGCTCCTAAGGCT
GGCCCTGGTGTGGTGCGAAAGAACCCTGGTGTGGGCAACGGAGATGACGAGGCAGCTGAGTTGATGCAGCAGGTC
AACGTATTGAAACTTACTGTTGAAGACTTGGAGAAAGAGAGGGATTTCTACTTCGGAAAGCTACGGAACATTGAA
TTGATTTGCCAGGAGAACGAGGGGGAAAACGACCCTGTATTGCAGAGGATTGTAGACATTCTGTATGCCACAGAT
GAAGGCTTTGTGATACCTGATGAAGGGGGCCCACAGGAGGAGCAAGAAGAGTATGGCGCCGATTACAAGGACGAT
GATGACAAGGGAGCACCAGGAAGTGCTGGTTCTGCTGCTGGTAGTGGAGCGTGCCCGGTGCCGCTGCAGCTGCCG
CCGCTGGAACGCCTGACCCTGGATGATAAAAAACCGCTGAATACCCTGATCTCTGCTACTGGTCTGTGGATGAGT
CGTACCGGAACCATTCATAAAATCAAACACCACGAGGTTAGCCGTTCGAAAATCTATATTGAGATGGCGTGTGGC
GATCATCTGGTTGTGAACAATAGCCGCTCTTCTCGTACAGCACGTGCACTGCGTCACCACAAATATCGTAAAACC
TGTAAACGTTGCCGTGTGTCCGATGAGGATCTGAACAAATTCCTGACAAAAGCCAATGAGGACCAAACAAGCGTG
AAAGTGAAAGTCGTTAGCGCTCCTACCCGTACTAAAAAAGCAATGCCGAAATCCGTTGCTCGTGCCCCTAAACCA
CTGGAAAACACTGAAGCAGCACAGGCACAGCCGTCTGGAAGCAAATTCTCTCCGGCCATTCCTGTTTCTACCCAG
GAGTCCGTTTCTGTTCCAGCAAGTGTGAGCACCAGCATTAGCAGTATTAGCACCGGTGCCACCGCTAGCGCCCTG
GTTAAAGGCAATACCAATCCGATTACAAGCATGTCTGCCCCGGTTCAAGCATCAGCTCCAGCACTGACAAAATCC
CAAACCGATCGTCTGGAGGTTCTGCTGAATCCGAAAGACGAAATCAGCCTGAATTCCGGCAAACCGTTTCGTGAA
CTGGAGAGCGAACTGCTGTCACGTCGTAAAAAAGACCTGCAACAAATCTATGCCGAAGAACGTGAGAACTATCTG
GGGAAACTGGAACGTGAAATCACCCGCTTTTTCGTGGATCGTGGCTTTCTGGAGATCAAATCCCCGATTCTGATT

CCTCTGGAGTATATCGAGCGTATGGGCATCGACAATGATACCGAACTGAGCAAACAAATTTTCCGTGTGGATAAA
AACTTCTGTCTGCGCCCTATGCTAGCACCAAATCTGGCTAACTATCTGCGCAAACTGGACCGTGCCCTGCCTGAT
CCTATCAAAATCTTCGAGATCGGCCCGTGTTATCGTAAAGAGTCCGACGGTAAAGAACATCTGGAGGAGTTTACC
ATGCTGGCCTTTGCCCAAATGGGTTCAGGTTGTACTCGTGAGAACCTGGAAAGCATCATCACCGATTTTCTGAAC
CACCTGGGCATTGACTTCAAAATTGTGGGCGACAGCTGTATGGTGTTTGGCGACACCCTGGATGTCATGCACGGC
GACCTGGAACTGTCTAGTGCCGTTGTGGGCCCAATCCCGCTGGATCGTGAGTGGGGTATCGACAAACCTTGGATC
GGTGCGGGTTTTGGTCTGGAGCGTCTGCTGAAAGTAAAACACGACTTCAAGAACATCAAACGTGCTGCACGTTCC
GAGTCCTATTACAATGGTATTTCTACTAACCTGTAA (SEQ ID NO: 407)

Protein:
MAVNVYSTSVTSDNLSRHDMLAWINESLQLNLTKIEQLCSGAAYCQFMDMLFPGSIALKKVKFQAKLEHEYIQNF
KILQAGFKRMGVDKIIPVDKLVKGKFQDNFEFVQWFKKFFDANYDGKDYDPVAARQGQETAVAPSLVAPALNKPK
KPLTSSSAAPQRPISTQRTAAAPKAGPGVVRKNPGVGNGDDEAAELMQQVNVLKLTVEDLEKERDFYFGKLRNIE
LICQENEGENDPVLQRIVDILYATDEGFVIPDEGGPQEEQEEYGADYKDDDDKGAPGSAGSAAGSGACPVPLQLP
PLERLTLDDKKPLNTLISATGLWMSRTGTIHKIKHHEVSRSKIYIEMACGDHLVVNNSRSSRTARALRHHKYRKT
CKRCRVSDEDLNKFLTKANEDQTSVKVKVVSAPTRTKKAMPKSVARAPKPLENTEAAQAQPSGSKFSPAIPVSTQ
ESVSVPASVSTSISSISTGATASALVKGNTNPITSMSAPVQASAPALTKSQTDRLEVLLNPKDEISLNSGKPFRE
LESELLSRRKKDLQQIYAEERENYLGKLEREITRFFVDRGFLEIKSPILIPLEYIERMGIDNDTELSKQIFRVDK
NFCLRPMLAPNLANYLRKLDRALPDPIKIFEIGPCYRKESDGKEHLEEFTMLAFAQMGSGCTRENLESIITDFLN
HLGIDFKIVGDSCMVFGDTLDVMHGDLELSSAVVGPIPLDREWGIDKPWIGAGFGLERLLKVKHDFKNIKRAARS
ESYYNGISTNL* (SEQ ID NO: 408)

EB1::FUS::PylRS(AA)
DNA:
ATGGCAGTGAACGTATACTCAACGTCAGTGACCAGTGATAACCTAAGTCGACATGACATGCTGGCCTGGATCAAT
GAGTCTCTGCAGTTGAATCTGACAAAGATCGAACAGTTGTGCTCAGGGGCTGCGTATTGTCAGTTTATGGACATG
CTGTTCCCTGGCTCCATTGCCTTGAAGAAAGTGAAATTCCAAGCTAAGCTAGAACACGAGTACATCCAGAACTTC
AAAATACTACAAGCAGGTTTTAAGAGAATGGGTGTTGACAAAATAATTCCTGTGGACAAATTAGTAAAAGGAAAG
TTTCAGGACAATTTTGAATTCGTTCAGTGGTTCAAGAAGTTTTTCGATGCAAACTATGATGGAAAAGACTATGAC
CCTGTGGCTGCCAGACAAGGTCAAGAAACTGCAGTGGCTCCTTCCCTTGTTGCTCCAGCTCTGAATAAACCGAAG
AAACCTCTCACTTCTAGCAGTGCAGCTCCCCAGAGGCCCATCTCAACACAGAGAACCGCTGCGGCTCCTAAGGCT
GGCCCTGGTGTGGTGCGAAAGAACCCTGGTGTGGGCAACGGAGATGACGAGGCAGCTGAGTTGATGCAGCAGGTC
AACGTATTGAAACTTACTGTTGAAGACTTGGAGAAAGAGAGGGATTTCTACTTCGGAAAGCTACGGAACATTGAA
TTGATTTGCCAGGAGAACGAGGGGGAAAACGACCCTGTATTGCAGAGGATTGTAGACATTCTGTATGCCACAGAT
GAAGGCTTTGTGATACCTGATGAAGGGGGCCCACAGGAGGAGCAAGAAGAGTATGGAGCACCCGGCTCCGCCGGC
TCCGCCGCCGGCTCCGGCATGGCCTCAAACGATTATACCCAACAAGCAACCCAAAGCTATGGGGCCTACCCCACC
CAGCCCGGGCAGGGCTATTCCCAGCAGAGCAGTCAGCCCTACGGACAGCAGAGTTACAGTGGTTATAGCCAGTCC
ACGGACACTTCAGGATATGGCCAGAGCAGCTATTCTTCTTATGGCCAGAGCCAGAACACAGGCTATGGAACTCAG
TCAACTCCCCAGGGATATGGCTCGACTGGCGGCTATGGCAGTAGCCAGAGCTCCCAATCGTCTTACGGGCAGCAG
TCCTCCTACCCTGGCTATGGCCAGCAGCCAGCTCCCAGCAGCACCTCGGGAAGTTACGGTAGCAGTTCTCAGAGC
AGCAGCTATGGGCAGCCCCAGAGTGGGAGCTACAGCCAGCAGCCTAGCTATGGTGGACAGCAGCAAAGCTATGGA
CAGCAGCAAAGCTATAATCCCCCTCAGGGCTATGGACAGCAGAACCAGTACAACAGCAGCAGTGGTGGTGGAGGT
GGAGGTGGAGGTGGAGGTAACTATGGCCAAGATCAATCCTCCATGAGTAGTGGTGGTGGCAGTGGTGGCGGTTAT
GGCAATCAAGACCAGAGTGGTGGAGGTGGCAGCGGTGGCTATGGACAGCAGGACCGTGGAGGCCGCGGCAGGGGT
GGCAGTGGTGGCGGCGGCGGCGGCGGCGGTGGTGGTTACAACCGCAGCAGTGGTGGCTATGAACCCAGAGGTCGT
GGAGGTGGCCGTGGAGGCAGAGGTGGCATGGGCGGAAGTGACCGTGGTGGCTTCAATAAATTTGGTGGCCCTCGG
GACCAAGGATCACGTCATGACTCCGAACAGGATAATTCAGACAACAACACCATCTTTGTGCAAGGCCTGGGTGAG
AATGTTACAATTGAGTCTGTGGCTGATTACTTCAAGCAGATTGGTATTATTAAGACAAACAAGAAAACGGGACAG
CCCATGATTAATTTGTACACAGACAGGGAAACTGGCAAGCTGAAGGGAGAGGCAACGGTCTCTTTTGATGACCCA
CCTTCAGCTAAAGCAGCTATTGACTGGTTTGATGGTAAAGAATTCTCCGGAAATCCTATCAAGGTCTCATTTGCT
ACTCGCCGGGCAGACTTTAATCGGGGTGGTGGCAATGGTCGTGGAGGCCGAGGGCGAGGAGGACCCATGGGCCGT
GGAGGCTATGGAGGTGGTGGCAGTGGTGGTGGTGGCCGAGGAGGATTTCCCAGTGGAGGTGGTGGCGGTGGAGGA
CAGCAGCGAGCTGGTGACTGGAAGTGTCCTAATCCCACCTGTGAGAATATGAACTTCTCTTGGAGGAATGAATGC
AACCAGTGTAAGGCCCCTAAACCAGATGGCCCAGGAGGGGGACCAGGTGGCTCTCACATGGGGGGTAACTACGGG
GATGATCGTCGTGGTGGCAGAGGTGGTGCGATCGCAGGCGCCGATTACAAGGACGATGATGACAAGGGAGCACCA
GGAAGTGCTGGTTCTGCTGCTGGTAGTGGAGCGTGCCCGGTGCCGCTGCAGCTGCCGCCGCTGGAACGCCTGACC
CTGGATGACAAAAAACCGCTGAATACCCTGATCTCTGCTACTGGTCTGTGGATGAGTCGTACCGGAACCATTCAT
AAAATCAAACACCACGAGGTTAGCCGTTCGAAAATCTATATTGAGATGGCGTGTGGCGATCATCTGGTTGTGAAC

AATAGCCGCTCTTCTCGTACAGCACGTGCACTGCGTCACCACAAATATCGTAAAACCTGTAAACGTTGCCGTGTG
TCCGATGAGGATCTGAACAAATTCCTGACAAAAGCCAATGAGGACCAAACAAGCGTGAAAGTGAAAGTCGTTAGC
GCTCCTACCCGTACTAAAAAAGCAATGCCGAAATCCGTTGCTCGTGCCCCTAAACCACTGGAAAACACTGAAGCA
GCACAGGCACAGCCGTCTGGAAGCAAATTCTCTCCGGCCATTCCTGTTTCTACCCAGGAGTCCGTTTCTGTTCCA
GCAAGTGTGAGCACCAGCATTAGCAGTATTAGCACCGGTGCCACCGCTAGCGCCCTGGTTAAAGGCAATACCAAT
CCGATTACAAGCATGTCTGCCCCGGTTCAAGCATCAGCTCCAGCACTGACAAAATCCCAAACCGATCGTCTGGAG
GTTCTGCTGAATCCGAAAGACGAAATCAGCCTGAATTCCGGCAAACCGTTTCGTGAACTGGAGAGCGAACTGCTG
TCACGTCGTAAAAAAGACCTGCAACAAATCTATGCCGAAGAACGTGAGAACTATCTGGGGAAACTGGAACGTGAA
ATCACCCGCTTTTTCGTGGATCGTGGCTTTCTGGAGATCAAATCCCCGATTCTGATTCCTCTGGAGTATATCGAG
CGTATGGGCATCGACAATGATACCGAACTGAGCAAACAAATTTTCCGTGTGGATAAAAACTTCTGTCTGCGCCCT
ATGCTGGCACCAAATCTGTATAACTATCTGCGCAAACTGGACCGTGCCCTGCCTGATCCTATCAAAATCTTCGAG
ATCGGCCCGTGTTATCGTAAAGAGTCCGACGGTAAAGAACATCTGGAGGAGTTTACCATGCTGGCCTTTGCCCAA
ATGGGTTCAGGTTGTACTCGTGAGAACCTGGAAAGCATCATCACCGATTTTCTGAACCACCTGGGCATTGACTTC
AAAATTGTGGGCGACAGCTGTATGGTGTATGGCGACACCCTGGATGTCATGCACGGCGACCTGGAACTGTCTAGT
GCCGTTGTTGGACCAATTCCGCTGGACCGTGAGTGGGGTATCGACAAACCGTGGATCGGAGCAGGATTCGGTCTG
GAACGCCTGCTGAAAGTGAAACACGACTTCAAAAACATCAAACGTGCCGCCCGTTCTGAATCGTATTATAACGGG
ATCTCTACGAACCTGTAA (SEQ ID NO: 409)

Protein:
MAVNVYSTSVTSDNLSRHDMLAWINESLQLNLTKIEQLCSGAAYCQFMDMLFPGSIALKKVKFQAKLEHEYIQNF
KILQAGFKRMGVDKIIPVDKLVKGKFQDNFEFVQWFKKFFDANYDGKDYDPVAARQGQETAVAPSLVAPALNKPK
KPLTSSSAAPQRPISTQRTAAAPKAGPGVVRKNPGVGNGDDEAAELMQQVNVLKLTVEDLEKERDFYFGKLRNIE
LICQENEGENDPVLQRIVDILYATDEGFVIPDEGGPQEEQEEYGAPGSAGSAAGSGMASNDYTQQATQSYGAYPT
QPGQGYSQQSSQPYGQQSYSGYSQSTDTSGYGQSSYSSYGQSQNTGYGTQSTPQGYGSTGGYGSSQSSQSSYGQQ
SSYPGYGQQPAPSSTSGSYGSSSQSSSYGQPQSGSYSQQPSYGGQQQSYGQQQSYNPPQGYGQQNQYNSSSGGGG
GGGGGGNYGQDQSSMSSGGGSGGGYGNQDQSGGGGSGGYGQQDRGGRGRGGSGGGGGGGGGGYNRSSGGYEPRGR
GGGRGGRGGMGGSDRGGFNKFGGPRDQGSRHDSEQDNSDNNTIFVQGLGENVTIESVADYFKQIGIIKTNKKTGQ
PMINLYTDRETGKLKGEATVSFDDPPSAKAAIDWFDGKEFSGNPIKVSFATRRADFNRGGGNGRGGRGRGGPMGR
GGYGGGGSGGGGRGGFPSGGGGGGGQQRAGDWKCPNPTCENMNFSWRNECNQCKAPKPDGPGGGPGGSHMGGNYG
DDRRGGRGGAIAGADYKDDDDKGAPGSAGSAAGSGACPVPLQLPPLERLTLDDKKPLNTLISATGLWMSRTGTIH
KIKHHEVSRSKIYIEMACGDHLVVNNSRSSRTARALRHHKYRKTCKRCRVSDEDLNKFLTKANEDQTSVKVKVVS
APTRTKKAMPKSVARAPKPLENTEAAQAQPSGSKFSPAIPVSTQESVSVPASVSTSISSISTGATASALVKGNTN
PITSMSAPVQASAPALTKSQTDRLEVLLNPKDEISLNSGKPFRELESELLSRRKKDLQQIYAEERENYLGKLERE
ITRFFVDRGFLEIKSPILIPLEYIERMGIDNDTELSKQIFRVDKNFCLRPMLAPNLYNYLRKLDRALPDPIKIFE
IGPCYRKESDGKEHLEEFTMLAFAQMGSGCTRENLESIITDFLNHLGIDFKIVGDSCMVYGDTLDVMHGDLELSS
AVVGPIPLDREWGIDKPWIGAGFGLERLLKVKHDFKNIKRAARSESYYNGISTNL* (SEQ ID NO: 410)

EB1::FUS::PylRS(AAAF)
DNA:
ATGGCAGTGAACGTATACTCAACGTCAGTGACCAGTGATAACCTAAGTCGACATGACATGCTGGCCTGGATCAAT
GAGTCTCTGCAGTTGAATCTGACAAAGATCGAACAGTTGTGCTCAGGGGCTGCGTATTGTCAGTTTATGGACATG
CTGTTCCCTGGCTCCATTGCCTTGAAGAAAGTGAAATTCCAAGCTAAGCTAGAACACGAGTACATCCAGAACTTC
AAAATACTACAAGCAGGTTTTAAGAGAATGGGTGTTGACAAAATAATTCCTGTGGACAAATTAGTAAAAGGAAAG
TTTCAGGACAATTTTGAATTCGTTCAGTGGTTCAAGAAGTTTTTCGATGCAAACTATGATGGAAAAGACTATGAC
CCTGTGGCTGCCAGACAAGGTCAAGAAACTGCAGTGGCTCCTTCCCTTGTTGCTCCAGCTCTGAATAAACCGAAG
AAACCTCTCACTTCTAGCAGTGCAGCTCCCCAGAGGCCCATCTCAACACAGAGAACCGCTGCGGCTCCTAAGGCT
GGCCCTGGTGTGGTGCGAAAGAACCCTGGTGTGGGCAACGGAGATGACGAGGCAGCTGAGTTGATGCAGCAGGTC
AACGTATTGAAACTTACTGTTGAAGACTTGGAGAAAGAGAGGGATTTCTACTTCGGAAAGCTACGGAACATTGAA
TTGATTTGCCAGGAGAACGAGGGGGAAAACGACCCTGTATTGCAGAGGATTGTAGACATTCTGTATGCCACAGAT
GAAGGCTTTGTGATACCTGATGAAGGGGGCCCACAGGAGGAGCAAGAAGAGTATGGAGCACCCGGCTCCGCCGGC
TCCGCCGCCGGCTCCGGCATGGCCTCAAACGATTATACCCAACAAGCAACCCAAAGCTATGGGGCCTACCCCACC
CAGCCCGGGCAGGGCTATTCCCAGCAGAGCAGTCAGCCCTACGGACAGCAGAGTTACAGTGGTTATAGCCAGTCC
ACGGACACTTCAGGATATGGCCAGAGCAGCTATTCTTCTTATGGCCAGAGCCAGAACACAGGCTATGGAACTCAG
TCAACTCCCCAGGGATATGGCTCGACTGGCGGCTATGGCAGTAGCCAGAGCTCCCAATCGTCTTACGGGCAGCAG
TCCTCCTACCCTGGCTATGGCCAGCAGCCAGCTCCCAGCAGCACCTCGGGAAGTTACGGTAGCAGTTCTCAGAGC
AGCAGCTATGGGCAGCCCCAGAGTGGGAGCTACAGCCAGCAGCCTAGCTATGGTGGACAGCAGCAAAGCTATGGA
CAGCAGCAAAGCTATAATCCCCCTCAGGGCTATGGACAGCAGAACCAGTACAACAGCAGCAGTGGTGGTGGAGGT
GGAGGTGGAGGTGGAGGTAACTATGGCCAAGATCAATCCTCCATGAGTAGTGGTGGTGGCAGTGGTGGCGGTTAT

GGCAATCAAGACCAGAGTGGTGGAGGTGGCAGCGGTGGCTATGGACAGCAGGACCGTGGAGGCCGCGGCAGGGGT
GGCAGTGGTGGCGGCGGCGGCGGCGGTGGTGGTTACAACCGCAGCAGTGGTGGCTATGAACCCAGAGGTCGT
GGAGGTGGCCGTGGAGGCAGAGGTGGCATGGGCGGAAGTGACCGTGGTGGCTTCAATAAATTTGGTGGCCCTCGG
GACCAAGGATCACGTCATGACTCCGAACAGGATAATTCAGACAACAACACCATCTTTGTGCAAGGCCTGGGTGAG
AATGTTACAATTGAGTCTGTGGCTGATTACTTCAAGCAGATTGGTATTATTAAGACAAACAAGAAAACGGGACAG
CCCATGATTAATTTGTACACAGACAGGGAAACTGGCAAGCTGAAGGGAGAGGCAACGGTCTCTTTTGATGACCCA
CCTTCAGCTAAAGCAGCTATTGACTGGTTTGATGGTAAAGAATTCTCCGGAAATCCTATCAAGGTCTCATTTGCT
ACTCGCCGGGCAGACTTTAATCGGGGTGGTGGCAATGGTCGTGGAGGCCGAGGGCGAGGAGGACCCATGGGCCGT
GGAGGCTATGGAGGTGGTGGCAGTGGTGGTGGTGGCCGAGGAGGATTTCCCAGTGGAGGTGGTGGCGGTGGAGGA
CAGCAGCGAGCTGGTGACTGGAAGTGTCCTAATCCCACCTGTGAGAATATGAACTTCTCTTGGAGGAATGAATGC
AACCAGTGTAAGGCCCCTAAACCAGATGGCCCAGGAGGGGGACCAGGTGGCTCTCACATGGGGGGTAACTACGGG
GATGATCGTCGTGGTGGCAGAGGTGGTGCGATCGCAGGCGCCGATTACAAGGACGATGATGACAAGGGAGCACCA
GGAAGTGCTGGTTCTGCTGCTGGTAGTGGAGCGTGCCCGGTGCCGCTGCAGCTGCCGCCGCTGGAACGCCTGACC
CTGGATGATAAAAAACCGCTGAATACCCTGATCTCTGCTACTGGTCTGTGGATGAGTCGTACCGGAACCATTCAT
AAAATCAAACACCACGAGGTTAGCCGTTCGAAAATCTATATTGAGATGGCGTGTGGCGATCATCTGGTTGTGAAC
AATAGCCGCTCTTCTCGTACAGCACGTGCACTGCGTCACCACAAATATCGTAAAACCTGTAAACGTTGCCGTGTG
TCCGATGAGGATCTGAACAAATTCCTGACAAAAGCCAATGAGGACCAAACAAGCGTGAAAGTGAAAGTCGTTAGC
GCTCCTACCCGTACTAAAAAAGCAATGCCGAAATCCGTTGCTCGTGCCCCTAAACCACTGGAAAACACTGAAGCA
GCACAGGCACAGCCGTCTGGAAGCAAATTCTCTCCGGCCATTCCTGTTTCTACCCAGGAGTCCGTTTCTGTTCCA
GCAAGTGTGAGCACCAGCATTAGCAGTATTAGCACCGGTGCCACCGCTAGCGCCCTGGTTAAAGGCAATACCAAT
CCGATTACAAGCATGTCTGCCCCGGTTCAAGCATCAGCTCCAGCACTGACAAAATCCCAAACCGATCGTCTGGAG
GTTCTGCTGAATCCGAAAGACGAAATCAGCCTGAATTCCGGCAAACCGTTTCGTGAACTGGAGAGCGAACTGCTG
TCACGTCGTAAAAAAGACCTGCAACAAATCTATGCCGAAGAACGTGAGAACTATCTGGGGAAACTGGAACGTGAA
ATCACCCGCTTTTTCGTGGATCGTGGCTTTCTGGAGATCAAATCCCCGATTCTGATTCCTCTGGAGTATATCGAG
CGTATGGGCATCGACAATGATACCGAACTGAGCAAACAAATTTTCCGTGTGGATAAAAACTTCTGTCTGCGCCCT
ATGCTAGCACCAAATCTGGCTAACTATCTGCGCAAACTGGACCGTGCCCTGCCTGATCCTATCAAAATCTTCGAG
ATCGGCCCGTGTTATCGTAAAGAGTCCGACGGTAAAGAACATCTGGAGGAGTTTACCATGCTGGCCTTTGCCCAA
ATGGGTTCAGGTTGTACTCGTGAGAACCTGGAAAGCATCATCACCGATTTTCTGAACCACCTGGGCATTGACTTC
AAAATTGTGGGCGACAGCTGTATGGTGTTTGGCGACACCCTGGATGTCATGCACGGCGACCTGGAACTGTCTAGT
GCCGTTGTGGGCCCAATCCCGCTGGATCGTGAGTGGGGTATCGACAAACCTTGGATCGGTGCGGGTTTTGGTCTG
GAGCGTCTGCTGAAAGTAAAACACGACTTCAAGAACATCAAACGTGCTGCACGTTCCGAGTCCTATTACAATGGT
ATTTCTACTAACCTGTAA (SEQ ID NO: 411)

Protein:
MAVNVYSTSVTSDNLSRHDMLAWINESLQLNLTKIEQLCSGAAYCQFMDMLFPGSIALKKVKFQAKLEHEYIQNF
KILQAGFKRMGVDKIIPVDKLVKGKFQDNFEFVQWFKKFFDANYDGKDYDPVAARQGQETAVAPSLVAPALNKPK
KPLTSSSAAPQRPISTQRTAAAPKAGPGVVRKNPGVGNGDDEAAELMQQVNVLKLTVEDLEKERDFYFGKLRNIE
LICQENEGENDPVLQRIVDILYATDEGFVIPDEGGPQEEQEEYGAPGSAGSAAGSGMASNDYTQQATQSYGAYPT
QPGQGYSQQSSQPYGQQSYSGYSQSTDTSGYGQSSYSSYGQSQNTGYGTQSTPQGYGSTGGYGSSQSSQSSYGQQ
SSYPGYGQQPAPSSTSGSYGSSSQSSSYGQPQSGSYSQQPSYGGQQQSYGQQQSYNPPQGYGQQNQYNSSSGGGG
GGGGGGNYGQDQSSMSSGGGSGGGYGNQDQSGGGGSGGYGQQDRGGRGRGGSGGGGGGGGGYNRSSGGYEPRGR
GGGRGGRGGMGGSDRGGFNKFGGPRDQGSRHDSEQDNSDNNTIFVQGLGENVTIESVADYFKQIGIIKTNKKTGQ
PMINLYTDRETGKLKGEATVSFDDPPSAKAAIDWFDGKEFSGNPIKVSFATRRADFNRGGGNGRGGRGRGGPMGR
GGYGGGGSGGGGRGGFPSGGGGGGGQQRAGDWKCPNPTCENMNFSWRNECNQCKAPKPDGPGGGPGGSHMGGNYG
DDRRGGRGGAIAGADYKDDDDKGAPGSAGSAAGSGACPVPLQLPPLERLTLDDKKPLNTLISATGLWMSRTGTIH
KIKHHEVSRSKIYIEMACGDHLVVNNSRSSRTARALRHHKYRKTCKRCRVSDEDLNKFLTKANEDQTSVKVKVVS
APTRTKKAMPKSVARAPKPLENTEAAQAQPSGSKFSPAIPVSTQESVSVPASVSTSISSISTGATASALVKGNTN
PITSMSAPVQASAPALTKSQTDRLEVLLNPKDEISLNSGKPFRELESELLSRRKKDLQQIYAEERENYLGKLERE
ITRFFVDRGFLEIKSPILIPLEYIERMGIDNDTELSKQIFRVDKNFCLRPMLAPNLANYLRKLDRALPDPIKIFE
IGPCYRKESDGKEHLEEFTMLAFAQMGSGCTRENLESIITDFLNHLGIDFKIVGDSCMVFGDTLDVMHGDLELSS
AVVGPIPLDREWGIDKPWIGAGFGLERLLKVKHDFKNIKRAARSESYYNGISTNL* (SEQ ID NO: 412)

EB1::MCP
DNA:
ATGGCAGTGAACGTATACTCAACGTCAGTGACCAGTGATAACCTAAGTCGACATGACATGCTGGCCTGGATCAAT
GAGTCTCTGCAGTTGAATCTGACAAAGATCGAACAGTTGTGCTCAGGGGCTGCGTATTGTCAGTTTATGGACATG
CTGTTCCCTGGCTCCATTGCCTTGAAGAAAGTGAAATTCCAAGCTAAGCTAGAACACGAGTACATCCAGAACTTC
AAAATACTACAAGCAGGTTTTAAGAGAATGGGTGTTGACAAAATAATTCCTGTGGACAAATTAGTAAAAGGAAAG

TTTCAGGACAATTTTGAATTCGTTCAGTGGTTCAAGAAGTTTTTCGATGCAAACTATGATGGAAAAGACTATGAC
CCTGTGGCTGCCAGACAAGGTCAAGAAACTGCAGTGGCTCCTTCCCTTGTTGCTCCAGCTCTGAATAAACCGAAG
AAACCTCTCACTTCTAGCAGTGCAGCTCCCCAGAGGCCCATCTCAACACAGAGAACCGCTGCGGCTCCTAAGGCT
GGCCCTGGTGTGGTGCGAAAGAACCCTGGTGTGGGCAACGGAGATGACGAGGCAGCTGAGTTGATGCAGCAGGTC
AACGTATTGAAACTTACTGTTGAAGACTTGGAGAAAGAGAGGGATTTCTACTTCGGAAAGCTACGGAACATTGAA
TTGATTTGCCAGGAGAACGAGGGGGAAAACGACCCTGTATTGCAGAGGATTGTAGACATTCTGTATGCCACAGAT
GAAGGCTTTGTGATACCTGATGAAGGGGGCCCACAGGAGGAGCAAGAAGAGTATGCGATCGCATATCCCTATGAT
GTGCCGGATTATGCTGGAGCACCAGGAAGTGCTGGTTCTGCTGCTGGTAGTGGAGCTTCTAACTTTACTCAGTTC
GTTCTCGTCGACAATGGCGGAACTGGCGACGTGACTGTCGCCCCAAGCAACTTCGCTAACGGGATCGCTGAATGG
ATCAGCTCTAACTCGCGTTCACAGGCTTACAAAGTAACCTGTAGCGTTCGTCAGAGCTCTGCGCAGAATCGCAAA
TACACCATCAAAGTCGAGGTGCCTAAAGGCGCCTGGCGTTCGTACTTAAATATGGAACTAACCATTCCAATTTTC
GCCACGAATTCCGACTGCGAGCTTATTGTTAAGGCAATGCAAGGTCTCCTAAAAGATGGAAACCCGATTCCCTCA
GCAATCGCAGCAAACTCCGGCATCTACTAA (SEQ ID NO: 413)

Protein:
MAVNVYSTSVTSDNLSRHDMLAWINESLQLNLTKIEQLCSGAAYCQFMDMLFPGSIALKKVKFQAKLEHEYIQNF
KILQAGFKRMGVDKIIPVDKLVKGKFQDNFEFVQWFKKFFDANYDGKDYDPVAARQGQETAVAPSLVAPALNKPK
KPLTSSSAAPQRPISTQRTAAAPKAGPGVVRKNPGVGNGDDEAAELMQQVNVLKLTVEDLEKERDFYFGKLRNIE
LICQENEGENDPVLQRIVDILYATDEGFVIPDEGGPQEEQEEYAIAYPYDVPDYAGAPGSAGSAAGSGASNFTQF
VLVDNGGTGDVTVAPSNFANGIAEWISSNSRSQAYKVTCSVRQSSAQNRKYTIKVEVPKGAWRSYLNMELTIPIF
ATNSDCELIVKAMQGLLKDGNPIPSAIAANSGIY* (SEQ ID NO: 414)

EB1::EWSR1::MCP
DNA:
ATGGCAGTGAACGTATACTCAACGTCAGTGACCAGTGATAACCTAAGTCGACATGACATGCTGGCCTGGATCAAT
GAGTCTCTGCAGTTGAATCTGACAAAGATCGAACAGTTGTGCTCAGGGGCTGCGTATTGTCAGTTTATGGACATG
CTGTTCCCTGGCTCCATTGCCTTGAAGAAAGTGAAATTCCAAGCTAAGCTAGAACACGAGTACATCCAGAACTTC
AAAATACTACAAGCAGGTTTTAAGAGAATGGGTGTTGACAAAATAATTCCTGTGGACAAATTAGTAAAAGGAAAG
TTTCAGGACAATTTTGAATTCGTTCAGTGGTTCAAGAAGTTTTTCGATGCAAACTATGATGGAAAAGACTATGAC
CCTGTGGCTGCCAGACAAGGTCAAGAAACTGCAGTGGCTCCTTCCCTTGTTGCTCCAGCTCTGAATAAACCGAAG
AAACCTCTCACTTCTAGCAGTGCAGCTCCCCAGAGGCCCATCTCAACACAGAGAACCGCTGCGGCTCCTAAGGCT
GGCCCTGGTGTGGTGCGAAAGAACCCTGGTGTGGGCAACGGAGATGACGAGGCAGCTGAGTTGATGCAGCAGGTC
AACGTATTGAAACTTACTGTTGAAGACTTGGAGAAAGAGAGGGATTTCTACTTCGGAAAGCTACGGAACATTGAA
TTGATTTGCCAGGAGAACGAGGGGGAAAACGACCCTGTATTGCAGAGGATTGTAGACATTCTGTATGCCACAGAT
GAAGGCTTTGTGATACCTGATGAAGGGGGCCCACAGGAGGAGCAAGAAGAGTATATGGCGTCCACGGATTACAGT
ACCTATAGCCAAGCTGCAGCGCAGCAGGGCTACAGTGCTTACACCGCCCAGCCCACTCAAGGATATGCACAGACC
ACCCAGGCATATGGGCAACAAAGCTATGGAACCTATGGACAGCCCACTGATGTCAGCTATACCCAGGCTCAGACC
ACTGCAACCTATGGGCAGACCGCCTATGCAACTTCTTATGGACAGCCTCCCACTGGTTATACTACTCCAACTGCC
CCCCAGGCATACAGCCAGCCTGTCCAGGGGTATGGCACTGGTGCTTATGATACCACCACTGCTACAGTCACCACC
ACCCAGGCCTCCTATGCAGCTCAGTCTGCATATGGCACTCAGCCTGCTTATCCAGCCTATGGGCAGCAGCCAGCA
GCCACTGCACCTACAAGACCGCAGGATGGAAACAAGCCCACTGAGACTAGTCAACCTCAATCTAGCACAGGGGGT
TACAACCAGCCCAGCCTAGGATATGGACAGAGTAACTACAGTTATCCCCAGGTACCTGGGAGCTACCCCATGCAG
CCAGTCACTGCACCTCCATCCTACCCTCCTACCAGCTATTCCTCTACACAGCCGACTAGTTATGATCAGAGCAGT
TACTCTCAGCAGAACACCTATGGGCAACCGAGCAGCTATGGACAGCAGAGTAGCTATGGTCAACAAAGCAGCTAT
GGGCAGCAGCCTCCCACTAGTTACCCACCCCAAACTGGATCCTACAGCCAAGCTCCAAGTCAATATAGCCAACAG
AGCAGCAGCTACGGGCAGCAGAGTTCATTCCGACAGGACCACCCCAGTAGCATGGGTGTTTATGGGCAGGAGTCT
GGAGGATTTTCCGGACCAGGAGAGAACCGGAGCATGAGTGGCCCTGATAACCGGGGCAGGGGAAGAGGGGGATTT
GATCGTGGAGGCATGAGCAGAGGTGGGCGGGGAGGAGGACGCGGTGGAATGGGCAGCGCTGGAGAGCGAGGTGGC
TTCAATAAGCCTGGTGGACCCATGGATGAAGGACCAGATCTTGATCTAGGCCCACCTGTAGATCCAGATGAAGAC
TCTGACAACAGTGCAATTTATGTACAAGGATTAAATGACAGTGTGACTCTAGATGATCTGGCAGACTTCTTTAAG
CAGTGTGGGGTTGTTAAGATGAACAAGAGAACTGGGCAACCCATGATCCACATCTACCTGGACAAGGAAACAGGA
AAGCCCAAAGGCGATGCCACAGTGTCCTATGAAGACCCACCTACTGCCAAGGCTGCCGTGGAATGGTTTGATGGG
AAAGATTTTCAAGGGAGCAAACTTAAAGTCTCCCTTGCTCGGAAGAAGCCTCCAATGAACAGTATGCGGGGTGGT
CTGCCACCCCGTGAGGGCAGAGGCATGCCACCACCACTCCGTGGAGGTCCAGGAGGCCCAGGAGGTCCTGGGGGA
CCCATGGGTCGCATGGGAGGCCGTGGAGGAGATAGAGGAGGCTTCCCTCCAAGAGGACCCCGGGGTTCCCGAGGG
AACCCCTCTGGAGGAGGAAACGTCCAGCACCGAGCTGGAGACTGGCAGTGTCCCAATCCGGGTTGTGGAAACCAG
AACTTCGCCTGGAGAACAGAGTGCAACCAGTGTAAGGCCCCAAAGCCTGAAGGCTTCCTCCCGCCACCCTTTCCG

CCCCCGGGTGGTGATCGTGGCAGAGGTGGCCCTGGTGGCATGCGGGGAGGAAGAGGTGGCCTCATGGATCGTGGT
GGTCCCGGTGGAATGTTCAGAGGTGGCCGTGGTGGAGACAGAGGTGGCTTCCGTGGTGGCCGGGGCATGGACCGA
GGTGGCTTTGGTGGAGGAAGACGAGGTGGCCCTGGGGGGCCCCCTGGACCTTTGATGGAACAGGCGATCGCATAT
CCCTATGATGTGCCGGATTATGCTGGAGCACCAGGAAGTGCTGGTTCTGCTGCTGGTAGTGGAGCTTCTAACTTT
ACTCAGTTCGTTCTCGTCGACAATGGCGGAACTGGCGACGTGACTGTCGCCCCAAGCAACTTCGCTAACGGGATC
GCTGAATGGATCAGCTCTAACTCGCGTTCACAGGCTTACAAAGTAACCTGTAGCGTTCGTCAGAGCTCTGCGCAG
AATCGCAAATACACCATCAAAGTCGAGGTGCCTAAAGGCGCCTGGCGTTCGTACTTAAATATGGAACTAACCATT
CCAATTTTCGCCACGAATTCCGACTGCGAGCTTATTGTTAAGGCAATGCAAGGTCTCCTAAAAGATGGAAACCCG
ATTCCCTCAGCAATCGCAGCAAACTCCGGCATCTACTAA (SEQ ID NO: 415)

Protein:
MAVNVYSTSVTSDNLSRHDMLAWINESLQLNLTKIEQLCSGAAYCQFMDMLFPGSIALKKVKFQAKLEHEYIQNF
KILQAGFKRMGVDKIIPVDKLVKGKFQDNFEFVQWFKKFFDANYDGKDYDPVAARQGQETAVAPSLVAPALNKPK
KPLTSSSAAPQRPISTQRTAAAPKAGPGVVRKNPGVGNGDDEAAELMQQVNVLKLTVEDLEKERDFYFGKLRNIE
LICQENEGENDPVLQRIVDILYATDEGFVIPDEGGPQEEQEEYMASTDYSTYSQAAAQQGYSAYTAQPTQGYAQT
TQAYGQQSYGTYGQPTDVSYTQAQTTATYGQTAYATSYGQPPTGYTTPTAPQAYSQPVQGYGTGAYDTTTATVTT
TQASYAAQSAYGTQPAYPAYGQQPAATAPTRPQDGNKPTETSQPQSSTGGYNQPSLGYGQSNYSYPQVPGSYPMQ
PVTAPPSYPPTSYSSTQPTSYDQSSYSQQNTYGQPSSYGQQSSYGQQSSYGQQPPTSYPPQTGSYSQAPSQYSQQ
SSSYGQQSSFRQDHPSSMGVYGQESGGFSGPGENRSMSGPDNRGRGRGGFDRGGMSRGGRGGGRGGMGSAGERGG
FNKPGGPMDEGPDLDLGPPVDPDEDSDNSAIYVQGLNDSVTLDDLADFFKQCGVVKMNKRTGQPMIHIYLDKETG
KPKGDATVSYEDPPTAKAAVEWFDGKDFQGSKLKVSLARKKPPMNSMRGGLPPREGRGMPPPLRGGPGGPGGPGG
PMGRMGGRGGDRGGFPPRGPRGSRGNPSGGGNVQHRAGDWQCPNPGCGNQNFAWRTECNQCKAPKPEGFLPPPFP
PPGGDRGRGGPGGMRGGRGGLMDRGGPGGMFRGGRGGDRGGFRGGRGMDRGGFGGGRRGGPGGPPGPLMEQAIAY
PYDVPDYAGAPGSAGSAAGSGASNFTQFVLVDNGGTGDVTVAPSNFANGIAEWISSNSRSQAYKVTCSVRQSSAQ
NRKYTIKVEVPKGAWRSYLNMELTIPIFATNSDCELIVKAMQGLLKDGNPIPSAIAANSGIY* (SEQ ID NO:
416)

EB1::EWSR1::4xλN22
DNA:
ATGGCAGTGAACGTATACTCAACGTCAGTGACCAGTGATAACCTAAGTCGACATGACATGCTGGCCTGGATCAAT
GAGTCTCTGCAGTTGAATCTGACAAAGATCGAACAGTTGTGCTCAGGGGCTGCGTATTGTCAGTTTATGGACATG
CTGTTCCCTGGCTCCATTGCCTTGAAGAAAGTGAAATTCCAAGCTAAGCTAGAACACGAGTACATCCAGAACTTC
AAAATACTACAAGCAGGTTTTAAGAGAATGGGTGTTGACAAAATAATTCCTGTGGACAAATTAGTAAAAGGAAAG
TTTCAGGACAATTTTGAATTCGTTCAGTGGTTCAAGAAGTTTTTCGATGCAAACTATGATGGAAAAGACTATGAC
CCTGTGGCTGCCAGACAAGGTCAAGAAACTGCAGTGGCTCCTTCCCTTGTTGCTCCAGCTCTGAATAAACCGAAG
AAACCTCTCACTTCTAGCAGTGCAGCTCCCCAGAGGCCCATCTCAACACAGAGAACCGCTGCGGCTCCTAAGGCT
GGCCCTGGTGTGGTGCGAAAGAACCCTGGTGTGGGCAACGGAGATGACGAGGCAGCTGAGTTGATGCAGCAGGTC
AACGTATTGAAACTTACTGTTGAAGACTTGGAGAAAGAGAGGGATTTCTACTTCGGAAAGCTACGGAACATTGAA
TTGATTTGCCAGGAGAACGAGGGGGAAAACGACCCTGTATTGCAGAGGATTGTAGACATTCTGTATGCCACAGAT
GAAGGCTTTGTGATACCTGATGAAGGGGGCCCACAGGAGGAGCAAGAAGAGTATATGGCGTCCACGGATTACAGT
ACCTATAGCCAAGCTGCAGCGCAGCAGGGCTACAGTGCTTACACCGCCCAGCCCACTCAAGGATATGCACAGACC
ACCCAGGCATATGGGCAACAAAGCTATGGAACCTATGGACAGCCCACTGATGTCAGCTATACCCAGGCTCAGACC
ACTGCAACCTATGGGCAGACCGCCTATGCAACTTCTTATGGACAGCCTCCCACTGGTTATACTACTCCAACTGCC
CCCCAGGCATACAGCCAGCCTGTCCAGGGGTATGGCACTGGTGCTTATGATACCACCACTGCTACAGTCACCACC
ACCCAGGCCTCCTATGCAGCTCAGTCTGCATATGGCACTCAGCCTGCTTATCCAGCCTATGGGCAGCAGCCAGCA
GCCACTGCACCTACAAGACCGCAGGATGGAAACAAGCCCACTGAGACTAGTCAACCTCAATCTAGCACAGGGGGT
TACAACCAGCCCAGCCTAGGATATGGACAGAGTAACTACAGTTATCCCCAGGTACCTGGGAGCTACCCCATGCAG
CCAGTCACTGCACCTCCATCCTACCCTCCTACCAGCTATTCCTCTACACAGCCGACTAGTTATGATCAGAGCAGT
TACTCTCAGCAGAACACCTATGGGCAACCGAGCAGCTATGGACAGCAGAGTAGCTATGGTCAACAAAGCAGCTAT
GGGCAGCAGCCTCCCACTAGTTACCCACCCCAAACTGGATCCTACAGCCAAGCTCCAAGTCAATATAGCCAACAG
AGCAGCAGCTACGGGCAGCAGAGTTCATTCCGACAGGACCACCCCAGTAGCATGGGTGTTTATGGGCAGGAGTCT
GGAGGATTTTCCGGACCAGGAGAGAACCGGAGCATGAGTGGCCCTGATAACCGGGGCAGGGGAAGAGGGGGATTT
GATCGTGGAGGCATGAGCAGAGGTGGGCGGGGAGGAGGACGCGGTGGAATGGGCAGCGCTGGAGAGCGAGGTGGC
TTCAATAAGCCTGGTGGACCCATGGATGAAGGACCAGATCTTGATCTAGGCCCACCTGTAGATCCAGATGAAGAC
TCTGACAACAGTGCAATTTATGTACAAGGATTAAATGACAGTGTGACTCTAGATGATCTGGCAGACTTCTTTAAG
CAGTGTGGGGTTGTTAAGATGAACAAGAGAACTGGGCAACCCATGATCCACATCTACCTGGACAAGGAAACAGGA
AAGCCCAAAGGCGATGCCACAGTGTCCTATGAAGACCCACCTACTGCCAAGGCTGCCGTGGAATGGTTTGATGGG

AAAGATTTTCAAGGGAGCAAACTTAAAGTCTCCCTTGCTCGGAAGAAGCCTCCAATGAACAGTATGCGGGGTGGT
CTGCCACCCCGTGAGGGCAGAGGCATGCCACCACCACTCCGTGGAGGTCCAGGAGGCCCAGGAGGTCCTGGGGGA
CCCATGGGTCGCATGGGAGGCCGTGGAGGAGATAGAGGAGGCTTCCCTCCAAGAGGACCCCGGGGTTCCCGAGGG
AACCCCTCTGGAGGAGGAAACGTCCAGCACCGAGCTGGAGACTGGCAGTGTCCCAATCCGGGTTGTGGAAACCAG
AACTTCGCCTGGAGAACAGAGTGCAACCAGTGTAAGGCCCCAAAGCCTGAAGGCTTCCTCCCGCCACCCTTTCCG
CCCCCGGGTGGTGATCGTGGCAGAGGTGGCCCTGGTGGCATGCGGGGAGGAAGAGGTGGCCTCATGGATCGTGGT
GGTCCCGGTGGAATGTTCAGAGGTGGCCGTGGTGGAGACAGAGGTGGCTTCCGTGGTGGCCGGGGCATGGACCGA
GGTGGCTTTGGTGGAGGAAGACGAGGTGGCCCTGGGGGGCCCCCTGGACCTTTGATGGAACAGGCGATCGCAGGA
GCACCAGGAAGTGCTGGTTCTGCTGCTGGTAGTGGAGAGCAGAAGCTGATCTCAGAGGAGGACCTGCTAGCCACC
ATGGACGCACAAACACGACGACGTGAGCGTCGCGCTGAGAAACAAGCTCAATGGAAAGCTGCAAACCCACCGCTC
GACGGAGCCGGAGCTGGCGCTGGAGCTGGAGCCGGAGCTGGCGGTCTAGCCACCATGGACGCACAAACACGACGA
CGTGAGCGTCGCGCTGAGAAACAAGCTCAATGGAAAGCTGCAAACCCACCGCTCGACGGAGCCGGAGCTGGCGCT
GGAGCTGGAGCCGGAGCTGGCGGTCTAGCCACCATGGACGCACAAACACGACGACGTGAGCGTCGCGCTGAGAAA
CAAGCTCAATGGAAAGCTGCAAACCCACCGCTCGACGGAGCCGGAGCTGGCGCTGGAGCTGGAGCCGGAGCTGGC
GGTCTAGCCACCATGGACGCACAAACACGACGACGTGAGCGTCGCGCTGAGAAACAAGCTCAATGGAAAGCTGCA
AACCCACCGCTCGAGTCTAGAGGGCCCGTTTAA (SEQ ID NO: 417)

Protein:
MAVNVYSTSVTSDNLSRHDMLAWINESLQLNLTKIEQLCSGAAYCQFMDMLFPGSIALKKVKFQAKLEHEYIQNF
KILQAGFKRMGVDKIIPVDKLVKGKFQDNFEFVQWFKKFFDANYDGKDYDPVAARQGQETAVAPSLVAPALNKPK
KPLTSSSAAPQRPISTQRTAAAPKAGPGVVRKNPGVGNGDDEAAELMQQVNVLKLTVEDLEKERDFYFGKLRNIE
LICQENEGENDPVLQRIVDILYATDEGFVIPDEGGPQEEQEEYMASTDYSTYSQAAAQQGYSAYTAQPTQGYAQT
TQAYGQQSYGTYGQPTDVSYTQAQTTATYGQTAYATSYGQPPTGYTTPTAPQAYSQPVQGYGTGAYDTTTATVTT
TQASYAAQSAYGTQPAYPAYGQQPAATAPTRPQDGNKPTETSQPQSSTGGYNQPSLGYGQSNYSYPQVPGSYPMQ
PVTAPPSYPPTSYSSTQPTSYDQSSYSQQNTYGQPSSYGQQSSYGQQSSYGQQPPTSYPPQTGSYSQAPSQYSQQ
SSSYGQQSSFRQDHPSSMGVYGQESGGFSGPGENRSMSGPDNRGRGRGGFDRGGMSRGGRGGGRGGMGSAGERGG
FNKPGGPMDEGPDLDLGPPVDPDEDSDNSAIYVQGLNDSVTLDDLADFFKQCGVVKMNKRTGQPMIHIYLDKETG
KPKGDATVSYEDPPTAKAAVEWFDGKDFQGSKLKVSLARKKPPMNSMRGGLPPREGRGMPPPLRGGPGGPGGPGG
PMGRMGGRGGDRGGFPPRGPRGSRGNPSGGGNVQHRAGDWQCPNPGCGNQNFAWRTECNQCKAPKPEGFLPPPFP
PPGGDRGRGGPGGMRGGRGGLMDRGGPGGMFRGGRGGDRGGFRGGRGMDRGGFGGGRRGGPGGPPGPLMEQAIAG
APGSAGSAAGSGEQKLISEEDLLATMDAQTRRRERRAEKQAQWKAANPPLDGAGAGAGAGAGGLATMDAQTRR
RERRAEKQAQWKAANPPLDGAGAGAGAGAGGLATMDAQTRRRERRAEKQAQWKAANPPLDGAGAGAGAGAG
GLATMDAQTRRRERRAEKQAQWKAANPPLESRGPV* (SEQ ID NO: 418)

EB1::FUS::PylRS(AF)
DNA:
ATGGCAGTGAACGTATACTCAACGTCAGTGACCAGTGATAACCTAAGTCGACATGACATGCTGGCCTGGATCAAT
GAGTCTCTGCAGTTGAATCTGACAAAGATCGAACAGTTGTGCTCAGGGGCTGCGTATTGTCAGTTTATGGACATG
CTGTTCCCTGGCTCCATTGCCTTGAAGAAAGTGAAATTCCAAGCTAAGCTAGAACACGAGTACATCCAGAACTTC
AAAATACTACAAGCAGGTTTTAAGAGAATGGGTGTTGACAAAATAATTCCTGTGGACAAATTAGTAAAAGGAAAG
TTTCAGGACAATTTTGAATTCGTTCAGTGGTTCAAGAAGTTTTTCGATGCAAACTATGATGGAAAAGACTATGAC
CCTGTGGCTGCCAGACAAGGTCAAGAAACTGCAGTGGCTCCTTCCCTTGTTGCTCCAGCTCTGAATAAACCGAAG
AAACCTCTCACTTCTAGCAGTGCAGCTCCCCAGAGGCCCATCTCAACACAGAGAACCGCTGCGGCTCCTAAGGCT
GGCCCTGGTGTGGTGCGAAAGAACCCTGGTGTGGGCAACGGAGATGACGAGGCAGCTGAGTTGATGCAGCAGGTC
AACGTATTGAAACTTACTGTTGAAGACTTGGAGAAAGAGAGGGATTTCTACTTCGGAAAGCTACGGAACATTGAA
TTGATTTGCCAGGAGAACGAGGGGGAAAACGACCCTGTATTGCAGAGGATTGTAGACATTCTGTATGCCACAGAT
GAAGGCTTTGTGATACCTGATGAAGGGGGCCCACAGGAGGAGCAAGAAGAGTATGGAGCACCCGGCTCCGCCGGC
TCCGCCGCCGGCTCCGGCATGGCCTCAAACGATTATACCCAACAAGCAACCCAAAGCTATGGGGCCTACCCCACC
CAGCCCGGGCAGGGCTATTCCCAGCAGAGCAGTCAGCCCTACGGACAGCAGAGTTACAGTGGTTATAGCCAGTCC
ACGGACACTTCAGGATATGGCCAGAGCAGCTATTCTTCTTATGGCCAGAGCCAGAACACAGGCTATGGAACTCAG
TCAACTCCCCAGGGATATGGCTCGACTGGCGGCTATGGCAGTAGCCAGAGCTCCCAATCGTCTTACGGGCAGCAG
TCCTCCTACCCTGGCTATGGCCAGCAGCCAGCTCCCAGCAGCACCTCGGGAAGTTACGGTAGCAGTTCTCAGAGC
AGCAGCTATGGGCAGCCCCAGAGTGGGAGCTACAGCCAGCAGCCTAGCTATGGTGGACAGCAGCAAAGCTATGGA
CAGCAGCAAAGCTATAATCCCCCTCAGGGCTATGGACAGCAGAACCAGTACAACAGCAGCAGTGGTGGTGGAGGT
GGAGGTGGAGGTGGAGGTAACTATGGCCAAGATCAATCCTCCATGAGTAGTGGTGGTGGCAGTGGTGGCGGTTAT
GGCAATCAAGACCAGAGTGGTGGAGGTGGCAGCGGTGGCTATGGACAGCAGGACCGTGGAGGCCGCGGCAGGGGT
GGCAGTGGTGGCGGCGGCGGCGGCGGCGGTGGTGGTTACAACCGCAGCAGTGGTGGCTATGAACCCAGAGGTCGT

GGAGGTGGCCGTGGAGGCAGAGGTGGCATGGGCGGAAGTGACCGTGGTGGCTTCAATAAATTTGGTGGCCCTCGG
GACCAAGGATCACGTCATGACTCCGAACAGGATAATTCAGACAACAACACCATCTTTGTGCAAGGCCTGGGTGAG
AATGTTACAATTGAGTCTGTGGCTGATTACTTCAAGCAGATTGGTATTATTAAGACAAACAAGAAAACGGGACAG
CCCATGATTAATTTGTACACAGACAGGGAAACTGGCAAGCTGAAGGGAGAGGCAACGGTCTCTTTTGATGACCCA
CCTTCAGCTAAAGCAGCTATTGACTGGTTTGATGGTAAAGAATTCTCCGGAAATCCTATCAAGGTCTCATTTGCT
ACTCGCCGGGCAGACTTTAATCGGGGTGGTGGCAATGGTCGTGGAGGCCGAGGGCGAGGAGGACCCATGGGCCGT
GGAGGCTATGGAGGTGGTGGCAGTGGTGGTGGTGGCCGAGGAGGATTTCCCAGTGGAGGTGGTGGCGGTGGAGGA
CAGCAGCGAGCTGGTGACTGGAAGTGTCCTAATCCCACCTGTGAGAATATGAACTTCTCTTGGAGGAATGAATGC
AACCAGTGTAAGGCCCCTAAACCAGATGGCCCAGGAGGGGGACCAGGTGGCTCTCACATGGGGGGTAACTACGGG
GATGATCGTCGTGGTGGCAGAGGTGGTGCGATCGCAGGCGCCGATTACAAGGACGATGATGACAAGGGAGCACCA
GGAAGTGCTGGTTCTGCTGCTGGTAGTGGAGCGTGCCCGGTGCCGCTGCAGCTGCCGCCGCTGGAACGCCTGACC
CTGGATGATAAAAAACCGCTGAATACCCTGATCTCTGCTACTGGTCTGTGGATGAGTCGTACCGGAACCATTCAT
AAAATCAAACACCACGAGGTTAGCCGTTCGAAAATCTATATTGAGATGGCGTGTGGCGATCATCTGGTTGTGAAC
AATAGCCGCTCTTCTCGTACAGCACGTGCACTGCGTCACCACAAATATCGTAAAACCTGTAAACGTTGCCGTGTG
TCCGATGAGGATCTGAACAAATTCCTGACAAAAGCCAATGAGGACCAAACAAGCGTGAAAGTGAAAGTCGTTAGC
GCTCCTACCCGTACTAAAAAAGCAATGCCGAAATCCGTTGCTCGTGCCCCTAAACCACTGGAAAACACTGAAGCA
GCACAGGCACAGCCGTCTGGAAGCAAATTCTCTCCGGCCATTCCTGTTTCTACCCAGGAGTCCGTTTCTGTTCCA
GCAAGTGTGAGCACCAGCATTAGCAGTATTAGCACCGGTGCCACCGCTAGCGCCCTGGTTAAAGGCAATACCAAT
CCGATTACAAGCATGTCTGCCCCGGTTCAAGCATCAGCTCCAGCACTGACAAAATCCCAAACCGATCGTCTGGAG
GTTCTGCTGAATCCGAAAGACGAAATCAGCCTGAATTCCGGCAAACCGTTTCGTGAACTGGAGAGCGAACTGCTG
TCACGTCGTAAAAAAGACCTGCAACAAATCTATGCCGAAGAACGTGAGAACTATCTGGGGAAACTGGAACGTGAA
ATCACCCGCTTTTTCGTGGATCGTGGCTTTCTGGAGATCAAATCCCCGATTCTGATTCCTCTGGAGTATATCGAG
CGTATGGGCATCGACAATGATACCGAACTGAGCAAACAAATTTTCCGTGTGGATAAAAACTTCTGTCTGCGCCCT
ATGCTAGCACCAAATCTGGCTAACTATCTGCGCAAACTGGACCGTGCCCTGCCTGATCCTATCAAAATCTTCGAG
ATCGGCCCGTGTTATCGTAAAGAGTCCGACGGTAAAGAACATCTGGAGGAGTTTACCATGCTGAACTTTTGCCAA
ATGGGTTCAGGTTGTACTCGTGAGAACCTGGAAAGCATCATCACCGATTTTCTGAACCACCTGGGCATTGACTTC
AAAATTGTGGGCGACAGCTGTATGGTGTTTGGCGACACCCTGGATGTCATGCACGGCGACCTGGAACTGTCTAGT
GCCGTTGTGGGCCCAATCCCGCTGGATCGTGAGTGGGGTATCGACAAACCTTGGATCGGTGCGGGTTTTGGTCTG
GAGCGTCTGCTGAAAGTAAAACACGACTTCAAGAACATCAAACGTGCTGCACGTTCCGAGTCCTATTACAATGGT
ATTTCTACTAACCTGTAA (SEQ ID NO: 419)

Protein:
MAVNVYSTSVTSDNLSRHDMLAWINESLQLNLTKIEQLCSGAAYCQFMDMLFPGSIALKKVKFQAKLEHEYIQNF
KILQAGFKRMGVDKIIPVDKLVKGKFQDNFEFVQWFKKFFDANYDGKDYDPVAARQGQETAVAPSLVAPALNKPK
KPLTSSSAAPQRPISTQRTAAAPKAGPGVVRKNPGVGNGDDEAAELMQQVNVLKLTVEDLEKERDFYFGKLRNIE
LICQENEGENDPVLQRIVDILYATDEGFVIPDEGGPQEEQEEYGAPGSAGSAAGSGMASNDYTQQATQSYGAYPT
QPGQGYSQQSSQPYGQQSYSGYSQSTDTSGYGQSSYSSYGQSQNTGYGTQSTPQGYGSTGGYGSSQSSQSSYGQQ
SSYPGYGQQPAPSSTSGSYGSSSQSSSYGQPQSGSYSQQPSYGGQQQSYGQQQSYNPPQGYGQQNQYNSSSGGGG
GGGGGGNYGQDQSSMSSGGGSGGGYGNQDQSGGGGSGGYGQQDRGGRGRGGSGGGGGGGGGYNRSSGGYEPRGR
GGGRGGRGGMGGSDRGGFNKFGGPRDQGSRHDSEQDNSDNNTIFVQGLGENVTIESVADYFKQIGIIKTNKKTGQ
PMINLYTDRETGKLKGEATVSFDDPPSAKAAIDWFDGKEFSGNPIKVSFATRRADFNRGGGNGRGGRGRGGPMGR
GGYGGGGSGGGGRGGFPSGGGGGGGQQRAGDWKCPNPTCENMNFSWRNECNQCKAPKPDGPGGGPGGSHMGGNYG
DDRRGGRGGAIAGADYKDDDDKGAPGSAGSAAGSGACPVPLQLPPLERLTLDDKKPLNTLISATGLWMSRTGTIH
KIKHHEVSRSKIYIEMACGDHLVVNNSRSSRTARALRHHKYRKTCKRCRVSDEDLNKFLTKANEDQTSVKVKVVS
APTRTKKAMPKSVARAPKPLENTEAAQAQPSGSKFSPAIPVSTQESVSVPASVSTSISSISTGATASALVKGNTN
PITSMSAPVQASAPALTKSQTDRLEVLLNPKDEISLNSGKPFRELESELLSRRKKDLQQIYAEERENYLGKLERE
ITRFFVDRGFLEIKSPILIPLEYIERMGIDNDTELSKQIFRVDKNFCLRPMLAPNLANYLRKLDRALPDPIKIFE
IGPCYRKESDGKEHLEEFTMLNFCQMGSGCTRENLESIITDFLNHLGIDFKIVGDSCMVFGDTLDVMHGDLELSS
AVVGPIPLDREWGIDKPWIGAGFGLERLLKVKHDFKNIKRAARSESYYNGISTNL* (SEQ ID NO: 420)

EB1::FUS::MCP::PylRS(AF)
DNA:
ATGGCAGTGAACGTATACTCAACGTCAGTGACCAGTGATAACCTAAGTCGACATGACATGCTGGCCTGGATCAAT
GAGTCTCTGCAGTTGAATCTGACAAAGATCGAACAGTTGTGCTCAGGGGCTGCGTATTGTCAGTTTATGGACATG
CTGTTCCCTGGCTCCATTGCCTTGAAGAAAGTGAAATTCCAAGCTAAGCTAGAACACGAGTACATCCAGAACTTC
AAAATACTACAAGCAGGTTTTAAGAGAATGGGTGTTGACAAAATAATTCCTGTGGACAAATTAGTAAAAGGAAAG
TTTCAGGACAATTTTGAATTCGTTCAGTGGTTCAAGAAGTTTTTCGATGCAAACTATGATGGAAAAGACTATGAC
CCTGTGGCTGCCAGACAAGGTCAAGAAACTGCAGTGGCTCCTTCCCTTGTTGCTCCAGCTCTGAATAAACCGAAG

AAACCTCTCACTTCTAGCAGTGCAGCTCCCCAGAGGCCCATCTCAACACAGAGAACCGCTGCGGCTCCTAAGGCT
GGCCCTGGTGTGGTGCGAAAGAACCCTGGTGTGGGCAACGGAGATGACGAGGCAGCTGAGTTGATGCAGCAGGTC
AACGTATTGAAACTTACTGTTGAAGACTTGGAGAAAGAGAGGGATTTCTACTTCGGAAAGCTACGGAACATTGAA
TTGATTTGCCAGGAGAACGAGGGGGAAAACGACCCTGTATTGCAGAGGATTGTAGACATTCTGTATGCCACAGAT
GAAGGCTTTGTGATACCTGATGAAGGGGGCCCACAGGAGGAGCAAGAAGAGTATGGAGCACCCGGCTCCGCCGGC
TCCGCCGCCGGCTCCGGCATGGCCTCAAACGATTATACCCAACAAGCAACCCAAAGCTATGGGGCCTACCCCACC
CAGCCCGGGCAGGGCTATTCCCAGCAGAGCAGTCAGCCCTACGGACAGCAGAGTTACAGTGGTTATAGCCAGTCC
ACGGACACTTCAGGATATGGCCAGAGCAGCTATTCTTCTTATGGCCAGAGCCAGAACACAGGCTATGGAACTCAG
TCAACTCCCCAGGGATATGGCTCGACTGGCGGCTATGGCAGTAGCCAGAGCTCCCAATCGTCTTACGGGCAGCAG
TCCTCCTACCCTGGCTATGGCCAGCAGCCAGCTCCCAGCAGCACCTCGGGAAGTTACGGTAGCAGTTCTCAGAGC
AGCAGCTATGGGCAGCCCCAGAGTGGGAGCTACAGCCAGCAGCCTAGCTATGGTGGACAGCAGCAAAGCTATGGA
CAGCAGCAAAGCTATAATCCCCCTCAGGGCTATGGACAGCAGAACCAGTACAACAGCAGCAGTGGTGGTGGAGGT
GGAGGTGGAGGTGGAGGTAACTATGGCCAAGATCAATCCTCCATGAGTAGTGGTGGTGGCAGTGGTGGCGGTTAT
GGCAATCAAGACCAGAGTGGTGGAGGTGGCAGCGGTGGCTATGGACAGCAGGACCGTGGAGGCCGCGGCAGGGGT
GGCAGTGGTGGCGGCGGCGGCGGCGGCGGTGGTGGTTACAACCGCAGCAGTGGTGGCTATGAACCCAGAGGTCGT
GGAGGTGGCCGTGGAGGCAGAGGTGGCATGGGCGGAAGTGACCGTGGTGGCTTCAATAAATTTGGTGGCCCTCGG
GACCAAGGATCACGTCATGACTCCGAACAGGATAATTCAGACAACAACACCATCTTTGTGCAAGGCCTGGGTGAG
AATGTTACAATTGAGTCTGTGGCTGATTACTTCAAGCAGATTGGTATTATTAAGACAAACAAGAAAACGGGACAG
CCCATGATTAATTTGTACACAGACAGGGAAACTGGCAAGCTGAAGGGAGAGGCAACGGTCTCTTTTGATGACCCA
CCTTCAGCTAAAGCAGCTATTGACTGGTTTGATGGTAAAGAATTCTCCGGAAATCCTATCAAGGTCTCATTTGCT
ACTCGCCGGGCAGACTTTAATCGGGGTGGTGGCAATGGTCGTGGAGGCCGAGGGCGAGGAGGACCCATGGGCCGT
GGAGGCTATGGAGGTGGTGGCAGTGGTGGTGGTGGCCGAGGAGGATTTCCCAGTGGAGGTGGTGGCGGTGGAGGA
CAGCAGCGAGCTGGTGACTGGAAGTGTCCTAATCCCACCTGTGAGAATATGAACTTCTCTTGGAGGAATGAATGC
AACCAGTGTAAGGCCCCTAAACCAGATGGCCCAGGAGGGGGACCAGGTGGCTCTCACATGGGGGGTAACTACGGG
GATGATCGTCGTGGTGGCAGAGGTGGTGCGATCGCATATCCCTATGATGTGCCGGATTATGCTGGAGCACCAGGA
AGTGCTGGTTCTGCTGCTGGTAGTGGAGCTTCTAACTTTACTCAGTTCGTTCTCGTCGACAATGGCGGAACTGGC
GACGTGACTGTCGCCCCAAGCAACTTCGCTAACGGGATCGCTGAATGGATCAGCTCTAACTCGCGTTCACAGGCT
TACAAAGTAACCTGTAGCGTTCGTCAGAGCTCTGCGCAGAATCGCAAATACACCATCAAAGTCGAGGTGCCTAAA
GGCGCCTGGCGTTCGTACTTAAATATGGAACTAACCATTCCAATTTTCGCCACGAATTCCGACTGCGAGCTTATT
GTTAAGGCAATGCAAGGTCTCCTAAAAGATGGAAACCCGATTCCCTCAGCAATCGCAGCAAACTCCGGCATCTAC
GGCGCCGATTACAAGGACGATGATGACAAGGGAGCACCAGGAAGTGCTGGTTCTGCTGCTGGTAGTGGAGCGTGC
CCGGTGCCGCTGCAGCTGCCGCCGCTGGAACGCCTGACCCTGGATGATAAAAAACCGCTGAATACCCTGATCTCT
GCTACTGGTCTGTGGATGAGTCGTACCGGAACCATTCATAAAATCAAACACCACGAGGTTAGCCGTTCGAAAATC
TATATTGAGATGGCGTGTGGCGATCATCTGGTTGTGAACAATAGCCGCTCTTCTCGTACAGCACGTGCACTGCGT
CACCACAAATATCGTAAAACCTGTAAACGTTGCCGTGTGTCCGATGAGGATCTGAACAAATTCCTGACAAAAGCC
AATGAGGACCAAACAAGCGTGAAAGTGAAAGTCGTTAGCGCTCCTACCCGTACTAAAAAAGCAATGCCGAAATCC
GTTGCTCGTGCCCCTAAACCACTGGAAAACACTGAAGCAGCACAGGCACAGCCGTCTGGAAGCAAATTCTCTCCG
GCCATTCCTGTTTCTACCCAGGAGTCCGTTTCTGTTCCAGCAAGTGTGAGCACCAGCATTAGCAGTATTAGCACC
GGTGCCACCGCTAGCGCCCTGGTTAAAGGCAATACCAATCCGATTACAAGCATGTCTGCCCCGGTTCAAGCATCA
GCTCCAGCACTGACAAAATCCCAAACCGATCGTCTGGAGGTTCTGCTGAATCCGAAAGACGAAATCAGCCTGAAT
TCCGGCAAACCGTTTCGTGAACTGGAGAGCGAACTGCTGTCACGTCGTAAAAAAGACCTGCAACAAATCTATGCC
GAAGAACGTGAGAACTATCTGGGGAAACTGGAACGTGAAATCACCCGCTTTTTCGTGGATCGTGGCTTTCTGGAG
ATCAAATCCCCGATTCTGATTCCTCTGGAGTATATCGAGCGTATGGGCATCGACAATGATACCGAACTGAGCAAA
CAAATTTTCCGTGTGGATAAAAACTTCTGTCTGCGCCCTATGCTAGCACCAAATCTGGCTAACTATCTGCGCAAA
CTGGACCGTGCCCTGCCTGATCCTATCAAAATCTTCGAGATCGGCCCGTGTTATCGTAAAGAGTCCGACGGTAAA
GAACATCTGGAGGAGTTTACCATGCTGAACTTTTGCCAAATGGGTTCAGGTTGTACTCGTGAGAACCTGGAAAGC
ATCATCACCGATTTTCTGAACCACCTGGGCATTGACTTCAAAATTGTGGGCGACAGCTGTATGGTGTTTGGCGAC
ACCCTGGATGTCATGCACGGCGACCTGGAACTGTCTAGTGCCGTTGTGGGCCCAATCCCGCTGGATCGTGAGTGG
GGTATCGACAAACCTTGGATCGGTGCGGGTTTTGGTCTGGAGCGTCTGCTGAAAGTAAAACACGACTTCAAGAAC
ATCAAACGTGCTGCACGTTCCGAGTCCTATTACAATGGTATTTCTACTAACCTGTAA (SEQ ID NO: 421)

Protein:
MAVNVYSTSVTSDNLSRHDMLAWINESLQLNLTKIEQLCSGAAYCQFMDMLFPGSIALKKVKFQAKLEHEYIQNF
KILQAGFKRMGVDKIIPVDKLVKGKFQDNFEFVQWFKKFFDANYDGKDYDPVAARQGQETAVAPSLVAPALNKPK
KPLTSSSAAPQRPISTQRTAAAPKAGPGVVRKNPGVGNGDDEAAELMQQVNVLKLTVEDLEKERDFYFGKLRNIE
LICQENEGENDPVLQRIVDILYATDEGFVIPDEGGPQEEQEEYGAPGSAGSAAGSGMASNDYTQQATQSYGAYPT
QPGQGYSQQSSQPYGQQSYSGYSQSTDTSGYGQSSYSSYGQSQNTGYGTQSTPQGYGSTGGYGSSQSSQSSYGQQ
SSYPGYGQQPAPSSTSGSYGSSSQSSSYGQPQSGSYSQQPSYGGQQQSYGQQQSYNPPQGYGQQNQYNSSSGGGG

GGGGGGNYGQDQSSMSSGGGSGGGYGNQDQSGGGGSGGYGQQDRGGRGRGGSGGGGGGGGGYNRSSGGYEPRGR
GGGRGGRGGMGGSDRGGFNKFGGPRDQGSRHDSEQDNSDNNTIFVQGLGENVTIESVADYFKQIGIIKTNKKTGQ
PMINLYTDRETGKLKGEATVSFDDPPSAKAAIDWFDGKEFSGNPIKVSFATRRADFNRGGGNGRGGRGRGGPMGR
GGYGGGGSGGGGRGGFPSGGGGGGGQQRAGDWKCPNPTCENMNFSWRNECNQCKAPKPDGPGGGPGGSHMGGNYG
DDRRGGRGGAIAYPYDVPDYAGAPGSAGSAAGSGASNFTQFVLVDNGGTGDVTVAPSNFANGIAEWISSNSRSQA
YKVTCSVRQSSAQNRKYTIKVEVPKGAWRSYLNMELTIPIFATNSDCELIVKAMQGLLKDGNPIPSAIAANSGIY
GADYKDDDDKGAPGSAGSAAGSGACPVPLQLPPLERLTLDDKKPLNTLISATGLWMSRTGTIHKIKHHEVSRSKI
YIEMACGDHLVVNNSRSSRTARALRHHKYRKTCKRCRVSDEDLNKFLTKANEDQTSVKVKVVSAPTRTKKAMPKS
VARAPKPLENTEAAQAQPSGSKFSPAIPVSTQESVSVPASVSTSISSISTGATASALVKGNTNPITSMSAPVQAS
APALTKSQTDRLEVLLNPKDEISLNSGKPFRELESELLSRRKKDLQQIYAEERENYLGKLEREITRFFVDRGFLE
IKSPILIPLEYIERMGIDNDTELSKQIFRVDKNFCLRPMLAPNLANYLRKLDRALPDPIKIFEIGPCYRKESDGK
EHLEEFTMLNFCQMGSGCTRENLESIITDFLNHLGIDFKIVGDSCMVFGDTLDVMHGDLELSSAVVGPIPLDREW
GIDKPWIGAGFGLERLLKVKHDFKNIKRAARSESYYNGISTNL* (SEQ ID NO: 422)

TOM20::FUS::PCP::PylRS(AF)
DNA:
ATGGTGGGTCGGAACAGCGCCATCGCCGCCGGTGTATGCGGGGCCCTTTTCATTGGGTACTGCATCTACTTCGAC
CGCAAAAGACGAAGTGACCCCAACTTCAAGAACAGGCTTCGAGAACGAAGAAAGAAACAGAAGCTTGCCAAGGAG
AGAGCTGGGCTTTCCAAGTTACCTGACCTTAAAGATGCTGAAGCTGTTCAGAAATTCTTCATGGCCTCAAACGAT
TATACCCAACAAGCAACCCAAAGCTATGGGGCCTACCCCACCCAGCCCGGGCAGGGCTATTCCCAGCAGAGCAGT
CAGCCCTACGGACAGCAGAGTTACAGTGGTTATAGCCAGTCCACGGACACTTCAGGATATGGCCAGAGCAGCTAT
TCTTCTTATGGCCAGAGCCAGAACACAGGCTATGGAACTCAGTCAACTCCCCAGGGATATGGCTCGACTGGCGGC
TATGGCAGTAGCCAGAGCTCCCAATCGTCTTACGGGCAGCAGTCCTCCTACCCTGGCTATGGCCAGCAGCCAGCT
CCCAGCAGCACCTCGGGAAGTTACGGTAGCAGTTCTCAGAGCAGCAGCTATGGGCAGCCCCAGAGTGGGAGCTAC
AGCCAGCAGCCTAGCTATGGTGGACAGCAGCAAAGCTATGGACAGCAGCAAAGCTATAATCCCCCTCAGGGCTAT
GGACAGCAGAACCAGTACAACAGCAGCAGTGGTGGTGGAGGTGGAGGTGGAGGTGGAGGTAACTATGGCCAAGAT
CAATCCTCCATGAGTAGTGGTGGTGGCAGTGGTGGCGGTTATGGCAATCAAGACCAGAGTGGTGGAGGTGGCAGC
GGTGGCTATGGACAGCAGGACCGTGGAGGCCGCGGCAGGGGTGGCAGTGGTGGCGGCGGCGGCGGCGGCGGTGGT
GGTTACAACCGCAGCAGTGGTGGCTATGAACCCAGAGGTCGTGGAGGTGGCCGTGGAGGCAGAGGTGGCATGGGC
GGAAGTGACCGTGGTGGCTTCAATAAATTTGGTGGCCCTCGGGACCAAGGATCACGTCATGACTCCGAACAGGAT
AATTCAGACAACAACACCATCTTTGTGCAAGGCCTGGGTGAGAATGTTACAATTGAGTCTGTGGCTGATTACTTC
AAGCAGATTGGTATTATTAAGACAAACAAGAAAACGGGACAGCCCATGATTAATTTGTACACAGACAGGGAAACT
GGCAAGCTGAAGGGAGAGGCAACGGTCTCTTTTGATGACCCACCTTCAGCTAAAGCAGCTATTGACTGGTTTGAT
GGTAAAGAATTCTCCGGAAATCCTATCAAGGTCTCATTTGCTACTCGCCGGGCAGACTTTAATCGGGGTGGTGGC
AATGGTCGTGGAGGCCGAGGGCGAGGAGGACCCATGGGCCGTGGAGGCTATGGAGGTGGTGGCAGTGGTGGTGGT
GGCCGAGGAGGATTTCCCAGTGGAGGTGGTGGCGGTGGAGGACAGCAGCGAGCTGGTGACTGGAAGTGTCCTAAT
CCCACCTGTGAGAATATGAACTTCTCTTGGAGGAATGAATGCAACCAGTGTAAGGCCCCTAAACCAGATGGCCCA
GGAGGGGGACCAGGTGGCTCTCACATGGGGGGTAACTACGGGGATGATCGTCGTGGTGGCAGAGGAGGCGCGATC
GCAGGCAAGCCTATTCCCAACCCCCTGCTGGGCCTGGATAGCACCGGAGCACCAGGAAGTGCTGGTTCTGCTGCT
GGTAGTGGAGCATCGATAGAGCAGAAGCTGATCTCAGAGGAGGACCTGATCGAAGGCCGCCATATGCTAGCCTCC
AAAACCATCGTTCTTTCGGTCGGCGAGGCTACTCGCACTCTGACTGAGATCCAGTCCACCGCAGACCGTCAGATC
TTCGAAGAGAAGGTCGGGCCTCTGGTGGGTCGGCTGCGCCTCACGGCTTCGCTCCGTCAAAACGGAGCCAAGACC
GCGTATCGCGTCAACCTAAAACTGGATCAGGCGGACGTCGTTGATTCCGGACTTCCGAAAGTGCGCTACACTCAG
GTATGGTCGCACGACGTGACAATCGTTGCGAATAGCACCGAGGCCTCGCGCAAATCGTTGTACGATTTGACCAAG
TCCCTCGTCGCGACCTCGCAGGTCGAAGATCTTGTCGTCAACCTTGTGCCGCTGGGCCGTGCGGATCCGCTAGCC
GGTGCTCCTGGTTCAGCAGGAAGCGCAGCAGGATCAGGTGCGTGCCCGGTGCCGCTGCAGCTGCCGCCGCTGGAA
CGCCTGACCCTGGATGATAAAAAACCGCTGAATACCCTGATCTCTGCTACTGGTCTGTGGATGAGTCGTACCGGA
ACCATTCATAAAATCAAACACCACGAGGTTAGCCGTTCGAAAATCTATATTGAGATGGCGTGTGGCGATCATCTG
GTTGTGAACAATAGCCGCTCTTCTCGTACAGCACGTGCACTGCGTCACCACAAATATCGTAAAACCTGTAAACGT
TGCCGTGTGTCCGATGAGGATCTGAACAAATTCCTGACAAAAGCCAATGAGGACCAAACAAGCGTGAAAGTGAAA
GTCGTTAGCGCTCCTACCCGTACTAAAAAAGCAATGCCGAAATCCGTTGCTCGTGCCCCTAAACCACTGGAAAAC
ACTGAAGCAGCACAGGCACAGCCGTCTGGAAGCAAATTCTCTCCGGCCATTCCTGTTTCTACCCAGGAGTCCGTT
TCTGTTCCAGCAAGTGTGAGCACCAGCATTAGCAGTATTAGCACCGGTGCCACCGCTAGCGCCCTGGTTAAAGGC
AATACCAATCCGATTACAAGCATGTCTGCCCCGGTTCAAGCATCAGCTCCAGCACTGACAAAATCCCAAACCGAT
CGTCTGGAGGTTCTGCTGAATCCGAAAGACGAAATCAGCCTGAATTCCGGCAAACCGTTTCGTGAACTGGAGAGC
GAACTGCTGTCACGTCGTAAAAAAGACCTGCAACAAATCTATGCCGAAGAACGTGAGAACTATCTGGGGAAACTG
GAACGTGAAATCACCCGCTTTTTCGTGGATCGTGGCTTTCTGGAGATCAAATCCCCGATTCTGATTCCTCTGGAG

TATATCGAGCGTATGGGCATCGACAATGATACCGAACTGAGCAAACAAATTTTCCGTGTGGATAAAAACTTCTGT
CTGCGCCCTATGCTAGCACCAAATCTGGCTAACTATCTGCGCAAACTGGACCGTGCCCTGCCTGATCCTATCAAA
ATCTTCGAGATCGGCCCGTGTTATCGTAAAGAGTCCGACGGTAAAGAACATCTGGAGGAGTTTACCATGCTGAAC
TTTTGCCAAATGGGTTCAGGTTGTACTCGTGAGAACCTGGAAAGCATCATCACCGATTTTCTGAACCACCTGGGC
ATTGACTTCAAAATTGTGGGCGACAGCTGTATGGTGTTTGGCGACACCCTGGATGTCATGCACGGCGACCTGGAA
CTGTCTAGTGCCGTTGTGGGCCCAATCCCGCTGGATCGTGAGTGGGGTATCGACAAACCTTGGATCGGTGCGGGT
TTTGGTCTGGAGCGTCTGCTGAAAGTAAAACACGACTTCAAGAACATCAAACGTGCTGCACGTTCCGAGTCCTAT
TACAATGGTATTTCTACTAACCTGTAA (SEQ ID NO: 423)

Protein:
MVGRNSAIAAGVCGALFIGYCIYFDRKRRSDPNFKNRLRERRKKQKLAKERAGLSKLPDLKDAEAVQKFFMASND
YTQQATQSYGAYPTQPGQGYSQQSSQPYGQQSYSGYSQSTDTSGYGQSSYSSYGQSQNTGYGTQSTPQGYGSTGG
YGSSQSSQSSYGQQSSYPGYGQQPAPSSTSGSYGSSSQSSSYGQPQSGSYSQQPSYGGQQQSYGQQQSYNPPQGY
GQQNQYNSSSGGGGGGGGGGNYGQDQSSMSSGGGSGGGYGNQDQSGGGGSGGYGQQDRGGRGRGGSGGGGGGGGG
GYNRSSGGYEPRGRGGGRGGRGGMGGSDRGGFNKFGGPRDQGSRHDSEQDNSDNNTIFVQGLGENVTIESVADYF
KQIGIIKTNKKTGQPMINLYTDRETGKLKGEATVSFDDPPSAKAAIDWFDGKEFSGNPIKVSFATRRADFNRGGG
NGRGGRGRGGPMGRGGYGGGGSGGGGRGGFPSGGGGGGGQQRAGDWKCPNPTCENMNFSWRNECNQCKAPKPDGP
GGGPGGSHMGGNYGDDRRGGRGGAIAGKPIPNPLLGLDSTGAPGSAGSAAGSGASIEQKLISEEDLIEGRHMLAS
KTIVLSVGEATRTLTEIQSTADRQIFEEKVGPLVGRLRLTASLRQNGAKTAYRVNLKLDQADVVDSGLPKVRYTQ
VWSHDVTIVANSTEASRKSLYDLTKSLVATSQVEDLVVNLVPLGRADPLAGAPGSAGSAAGSGACPVPLQLPPLE
RLTLDDKKPLNTLISATGLWMSRTGTIHKIKHHEVSRSKIYIEMACGDHLVVNNSRSSRTARALRHHKYRKTCKR
CRVSDEDLNKFLTKANEDQTSVKVKVVSAPTRTKKAMPKSVARAPKPLENTEAAQAQPSGSKFSPAIPVSTQESV
SVPASVSTSISSISTGATASALVKGNTNPITSMSAPVQASAPALTKSQTDRLEVLLNPKDEISLNSGKPFRELES
ELLSRRKKDLQQIYAEERENYLGKLEREITRFFVDRGFLEIKSPILIPLEYIERMGIDNDTELSKQIFRVDKNFC
LRPMLAPNLANYLRKLDRALPDPIKIFEIGPCYRKESDGKEHLEEFTMLNFCQMGSGCTRENLESIITDFLNHLG
IDFKIVGDSCMVFGDTLDVMHGDLELSSAVVGPIPLDREWGIDKPWIGAGFGLERLLKVKHDFKNIKRAARSESY
YNGISTNL* (SEQ ID NO: 424)

TOM20::FUS::2xPCP::PylRS(AF)
DNA:
ATGGTGGGTCGGAACAGCGCCATCGCCGCCGGTGTATGCGGGGCCCTTTTCATTGGGTACTGCATCTACTTCGAC
CGCAAAAGACGAAGTGACCCCAACTTCAAGAACAGGCTTCGAGAACGAAGAAAGAAACAGAAGCTTGCCAAGGAG
AGAGCTGGGCTTTCCAAGTTACCTGACCTTAAAGATGCTGAAGCTGTTCAGAAATTCTTCATGGCCTCAAACGAT
TATACCCAACAAGCAACCCAAAGCTATGGGGCCTACCCCACCCAGCCCGGGCAGGGCTATTCCCAGCAGAGCAGT
CAGCCCTACGGACAGCAGAGTTACAGTGGTTATAGCCAGTCCACGGACACTTCAGGATATGGCCAGAGCAGCTAT
TCTTCTTATGGCCAGAGCCAGAACACAGGCTATGGAACTCAGTCAACTCCCCAGGGATATGGCTCGACTGGCGGC
TATGGCAGTAGCCAGAGCTCCCAATCGTCTTACGGGCAGCAGTCCTCCTACCCTGGCTATGGCCAGCAGCCAGCT
CCCAGCAGCACCTCGGGAAGTTACGGTAGCAGTTCTCAGAGCAGCAGCTATGGGCAGCCCCAGAGTGGGAGCTAC
AGCCAGCAGCCTAGCTATGGTGGACAGCAGCAAAGCTATGGACAGCAGCAAAGCTATAATCCCCCTCAGGGCTAT
GGACAGCAGAACCAGTACAACAGCAGCAGTGGTGGTGGAGGTGGAGGTGGAGGTGGAGGTAACTATGGCCAAGAT
CAATCCTCCATGAGTAGTGGTGGTGGCAGTGGTGGCGGTTATGGCAATCAAGACCAGAGTGGTGGAGGTGGCAGC
GGTGGCTATGGACAGCAGGACCGTGGAGGCCGCGGCAGGGGTGGCAGTGGTGGCGGCGGCGGCGGCGGCGGTGGT
GGTTACAACCGCAGCAGTGGTGGCTATGAACCCAGAGGTCGTGGAGGTGGCCGTGGAGGCAGAGGTGGCATGGGC
GGAAGTGACCGTGGTGGCTTCAATAAATTTGGTGGCCCTCGGGACCAAGGATCACGTCATGACTCCGAACAGGAT
AATTCAGACAACAACACCATCTTTGTGCAAGGCCTGGGTGAGAATGTTACAATTGAGTCTGTGGCTGATTACTTC
AAGCAGATTGGTATTATTAAGACAAACAAGAAAACGGGACAGCCCATGATTAATTTGTACACAGACAGGGAAACT
GGCAAGCTGAAGGGAGAGGCAACGGTCTCTTTTGATGACCCACCTTCAGCTAAAGCAGCTATTGACTGGTTTGAT
GGTAAAGAATTCTCCGGAAATCCTATCAAGGTCTCATTTGCTACTCGCCGGGCAGACTTTAATCGGGGTGGTGGC
AATGGTCGTGGAGGCCGAGGGCGAGGAGGACCCATGGGCCGTGGAGGCTATGGAGGTGGTGGCAGTGGTGGTGGT
GGCCGAGGAGGATTTCCCAGTGGAGGTGGTGGCGGTGGAGGACAGCAGCGAGCTGGTGACTGGAAGTGTCCTAAT
CCCACCTGTGAGAATATGAACTTCTCTTGGAGGAATGAATGCAACCAGTGTAAGGCCCCTAAACCAGATGGCCCA
GGAGGGGGACCAGGTGGCTCTCACATGGGGGGTAACTACGGGGATGATCGTCGTGGTGGCAGAGGAGGCGCGATC
GCAGGCAAGCCTATTCCCAACCCCCTGCTGGGCCTGGATAGCACCGGAGCACCAGGAAGTGCTGGTTCTGCTGCT
GGTAGTGGAGCATCGATAGAGCAGAAGCTGATCTCAGAGGAGGACCTGATCGAAGGCCGCCATATGCTAGCCTCC
AAAACCATCGTTCTTTCGGTCGGCGAGGCTACTCGCACTCTGACTGAGATCCAGTCCACCGCAGACCGTCAGATC
TTCGAAGAGAAGGTCGGGCCTCTGGTGGGTCGGCTGCGCCTCACGGCTTCGCTCCGTCAAAACGGAGCCAAGACC
GCGTATCGCGTCAACCTAAAACTGGATCAGGCGGACGTCGTTGATTCCGGACTTCCGAAAGTGCGCTACACTCAG
GTATGGTCGCACGACGTGACAATCGTTGCGAATAGCACCGAGGCCTCGCGCAAATCGTTGTACGATTTGACCAAG

TCCCTCGTCGCGACCTCGCAGGTCGAAGATCTTGTCGTCAACCTTGTGCCGCTGGGCCGTGCGGATCCGCTAGCC
TCCAAAACCATCGTTCTTTCGGTCGGCGAGGCTACTCGCACTCTGACTGAGATCCAGTCCACCGCAGACCGTCAG
ATCTTCGAAGAGAAGGTCGGGCCTCTGGTGGGTCGGCTGCGCCTCACGGCTTCGCTCCGTCAAAACGGAGCCAAG
ACCGCGTATCGCGTCAACCTAAAACTGGATCAGGCGGACGTCGTTGATTCCGGACTTCCGAAAGTGCGCTACACT
CAGGTATGGTCGCACGACGTGACAATCGTTGCGAATAGCACCGAGGCCTCGCGCAAATCGTTGTACGATTTGACC
AAGTCCCTCGTCGCGACCTCGCAGGTCGAAGATCTTGTCGTCAACCTTGTGCCGCTGGGCCGTCCACCGGTCGCC
ACCGGTGCTCCTGGTTCAGCAGGAAGCGCAGCAGGATCAGGTGCGTGCCCGGTGCCGCTGCAGCTGCCGCCGCTG
GAACGCCTGACCCTGGATGATAAAAAACCGCTGAATACCCTGATCTCTGCTACTGGTCTGTGGATGAGTCGTACC
GGAACCATTCATAAAATCAAACACCACGAGGTTAGCCGTTCGAAAATCTATATTGAGATGGCGTGTGGCGATCAT
CTGGTTGTGAACAATAGCCGCTCTTCTCGTACAGCACGTGCACTGCGTCACCACAAATATCGTAAAACCTGTAAA
CGTTGCCGTGTGTCCGATGAGGATCTGAACAAATTCCTGACAAAAGCCAATGAGGACCAAACAAGCGTGAAAGTG
AAAGTCGTTAGCGCTCCTACCCGTACTAAAAAAGCAATGCCGAAATCCGTTGCTCGTGCCCCTAAACCACTGGAA
AACACTGAAGCAGCACAGGCACAGCCGTCTGGAAGCAAATTCTCTCCGGCCATTCCTGTTTCTACCCAGGAGTCC
GTTTCTGTTCCAGCAAGTGTGAGCACCAGCATTAGCAGTATTAGCACCGGTGCCACCGCTAGCGCCCTGGTTAAA
GGCAATACCAATCCGATTACAAGCATGTCTGCCCCGGTTCAAGCATCAGCTCCAGCACTGACAAAATCCCAAACC
GATCGTCTGGAGGTTCTGCTGAATCCGAAAGACGAAATCAGCCTGAATTCCGGCAAACCGTTTCGTGAACTGGAG
AGCGAACTGCTGTCACGTCGTAAAAAAGACCTGCAACAAATCTATGCCGAAGAACGTGAGAACTATCTGGGGAAA
CTGGAACGTGAAATCACCCGCTTTTTCGTGGATCGTGGCTTTCTGGAGATCAAATCCCCGATTCTGATTCCTCTG
GAGTATATCGAGCGTATGGGCATCGACAATGATACCGAACTGAGCAAACAAATTTTCCGTGTGGATAAAAACTTC
TGTCTGCGCCCTATGCTAGCACCAAATCTGGCTAACTATCTGCGCAAACTGGACCGTGCCCTGCCTGATCCTATC
AAAATCTTCGAGATCGGCCCGTGTTATCGTAAAGAGTCCGACGGTAAAGAACATCTGGAGGAGTTTACCATGCTG
AACTTTTGCCAAATGGGTTCAGGTTGTACTCGTGAGAACCTGGAAAGCATCATCACCGATTTTCTGAACCACCTG
GGCATTGACTTCAAAATTGTGGGCGACAGCTGTATGGTGTTTGGCGACACCCTGGATGTCATGCACGGCGACCTG
GAACTGTCTAGTGCCGTTGTGGGCCCAATCCCGCTGGATCGTGAGTGGGGTATCGACAAACCTTGGATCGGTGCG
GGTTTTGGTCTGGAGCGTCTGCTGAAAGTAAAACACGACTTCAAGAACATCAAACGTGCTGCACGTTCCGAGTCC
TATTACAATGGTATTTCTACTAACCTGTAA (SEQ ID NO: 425)

Protein:
MVGRNSAIAAGVCGALFIGYCIYFDRKRRSDPNFKNRLRERRKKQKLAKERAGLSKLPDLKDAEAVQKFFMASND
YTQQATQSYGAYPTQPGQGYSQQSSQPYGQQSYSGYSQSTDTSGYGQSSYSSYGQSQNTGYGTQSTPQGYGSTGG
YGSSQSSQSSYGQQSSYPGYGQQPAPSSTSGSYGSSSQSSSYGQPQSGSYSQQPSYGGQQQSYGQQQSYNPPQGY
GQQNQYNSSSGGGGGGGGGGNYGQDQSSMSSGGGSGGGYGNQDQSGGGGSGGYGQQDRGGRGRGGSGGGGGGGGG
GYNRSSGGYEPRGRGGGRGGRGGMGGSDRGGFNKFGGPRDQGSRHDSEQDNSDNNTIFVQGLGENVTIESVADYF
KQIGIIKTNKKTGQPMINLYTDRETGKLKGEATVSFDDPPSAKAAIDWFDGKEFSGNPIKVSFATRRADFNRGGG
NGRGGRGRGGPMGRGGYGGGGSGGGGRGGFPSGGGGGGGQQRAGDWKCPNPTCENMNFSWRNECNQCKAPKPDGP
GGGPGGSHMGGNYGDDRRGGRGGAIAGKPIPNPLLGLDSTGAPGSAGSAAGSGASIEQKLISEEDLIEGRHMLAS
KTIVLSVGEATRTLTEIQSTADRQIFEEKVGPLVGRLRLTASLRQNGAKTAYRVNLKLDQADVVDSGLPKVRYTQ
VWSHDVTIVANSTEASRKSLYDLTKSLVATSQVEDLVVNLVPLGRADPLASKTIVLSVGEATRTLTEIQSTADRQ
IFEEKVGPLVGRLRLTASLRQNGAKTAYRVNLKLDQADVVDSGLPKVRYTQVWSHDVTIVANSTEASRKSLYDLT
KSLVATSQVEDLVVNLVPLGRPPVATGAPGSAGSAAGSGACPVPLQLPPLERLTLDDKKPLNTLISATGLWMSRT
GTIHKIKHHEVSRSKIYIEMACGDHLVVNNSRSSRTARALRHHKYRKTCKRCRVSDEDLNKFLTKANEDQTSVKV
KVVSAPTRTKKAMPKSVARAPKPLENTEAAQAQPSGSKFSPAIPVSTQESVSVPASVSTSISSISTGATASALVK
GNTNPITSMSAPVQASAPALTKSQTDRLEVLLNPKDEISLNSGKPFRELESELLSRRKKDLQQIYAEERENYLGK
LEREITRFFVDRGFLEIKSPILIPLEYIERMGIDNDTELSKQIFRVDKNFCLRPMLAPNLANYLRKLDRALPDPI
KIFEIGPCYRKESDGKEHLEEFTMLNFCQMGSGCTRENLESIITDFLNHLGIDFKIVGDSCMVFGDTLDVMHGDL
ELSSAVVGPIPLDREWGIDKPWIGAGFGLERLLKVKHDFKNIKRAARSESYYNGISTNL* (SEQ ID NO:
426)

TOM20::FUS::4xλN22::PylRS(AF)
DNA:
ATGGTGGGTCGGAACAGCGCCATCGCCGCCGGTGTATGCGGGGCCCTTTTCATTGGGTACTGCATCTACTTCGAC
CGCAAAAGACGAAGTGACCCCAACTTCAAGAACAGGCTTCGAGAACGAAGAAAGAAACAGAAGCTTGCCAAGGAG
AGAGCTGGGCTTTCCAAGTTACCTGACCTTAAAGATGCTGAAGCTGTTCAGAAATTCTTCATGGCCTCAAACGAT
TATACCCAACAAGCAACCCAAAGCTATGGGGCCTACCCCACCCAGCCCGGGCAGGGCTATTCCCAGCAGAGCAGT
CAGCCCTACGGACAGCAGAGTTACAGTGGTTATAGCCAGTCCACGGACACTTCAGGATATGGCCAGAGCAGCTAT
TCTTCTTATGGCCAGAGCCAGAACACAGGCTATGGAACTCAGTCAACTCCCCAGGGATATGGCTCGACTGGCGGC
TATGGCAGTAGCCAGAGCTCCCAATCGTCTTACGGGCAGCAGTCCTCCTACCCTGGCTATGGCCAGCAGCCAGCT

CCCAGCAGCACCTCGGGAAGTTACGGTAGCAGTTCTCAGAGCAGCAGCTATGGGCAGCCCCAGAGTGGGAGCTAC
AGCCAGCAGCCTAGCTATGGTGGACAGCAGCAAAGCTATGGACAGCAGCAAAGCTATAATCCCCCTCAGGGCTAT
GGACAGCAGAACCAGTACAACAGCAGCAGTGGTGGTGGAGGTGGAGGTGGAGGTGGAGGTAACTATGGCCAAGAT
CAATCCTCCATGAGTAGTGGTGGTGGCAGTGGTGGCGGTTATGGCAATCAAGACCAGAGTGGTGGAGGTGGCAGC
GGTGGCTATGGACAGCAGGACCGTGGAGGCCGCGGCAGGGGTGGCAGTGGTGGCGGCGGCGGCGGCGGTGGT
GGTTACAACCGCAGCAGTGGTGGCTATGAACCCAGAGGTCGTGGAGGTGGCCGTGGAGGCAGAGGTGGCATGGGC
GGAAGTGACCGTGGTGGCTTCAATAAATTTGGTGGCCCTCGGGACCAAGGATCACGTCATGACTCCGAACAGGAT
AATTCAGACAACAACACCATCTTTGTGCAAGGCCTGGGTGAGAATGTTACAATTGAGTCTGTGGCTGATTACTTC
AAGCAGATTGGTATTATTAAGACAAACAAGAAAACGGGACAGCCCATGATTAATTTGTACACAGACAGGGAAACT
GGCAAGCTGAAGGGAGAGGCAACGGTCTCTTTTGATGACCCACCTTCAGCTAAAGCAGCTATTGACTGGTTTGAT
GGTAAAGAATTCTCCGGAAATCCTATCAAGGTCTCATTTGCTACTCGCCGGGCAGACTTTAATCGGGGTGGTGGC
AATGGTCGTGGAGGCCGAGGGCGAGGAGGACCCATGGGCCGTGGAGGCTATGGAGGTGGTGGCAGTGGTGGTGGT
GGCCGAGGAGGATTTCCCAGTGGAGGTGGTGGCGGTGGAGGACAGCAGCGAGCTGGTGACTGGAAGTGTCCTAAT
CCCACCTGTGAGAATATGAACTTCTCTTGGAGGAATGAATGCAACCAGTGTAAGGCCCCTAAACCAGATGGCCCA
GGAGGGGGACCAGGTGGCTCTCACATGGGGGGTAACTACGGGGATGATCGTCGTGGTGGCAGAGGAGGCGCGATC
GCAGGCAAGCCTATTCCCAACCCCCTGCTGGGCCTGGATAGCACCGGAGCACCAGGAAGTGCTGGTTCTGCTGCT
GGTAGTGGAGCATCGATAGAGCAGAAGCTGATCTCAGAGGAGGACCTGCTAGCCACCATGGACGCACAAACACGA
CGACGTGAGCGTCGCGCTGAGAAACAAGCTCAATGGAAAGCTGCAAACCCACCGCTCGACGGAGCCGGAGCTGGC
GCTGGAGCTGGAGCCGGAGCTGGCGGTCTAGCCACCATGGACGCACAAACACGACGACGTGAGCGTCGCGCTGAG
AAACAAGCTCAATGGAAAGCTGCAAACCCACCGCTCGACGGAGCCGGAGCTGGCGCTGGAGCTGGAGCCGGAGCT
GGCGGTCTAGCCACCATGGACGCACAAACACGACGACGTGAGCGTCGCGCTGAGAAACAAGCTCAATGGAAAGCT
GCAAACCCACCGCTCGACGGAGCCGGAGCTGGCGCTGGAGCTGGAGCCGGAGCTGGCGGTCTAGCCACCATGGAC
GCACAAACACGACGACGTGAGCGTCGCGCTGAGAAACAAGCTCAATGGAAAGCTGCAAACCCACCGCTCGAGTCT
AGAGGGCCCGTTGGTGCTCCTGGTTCAGCAGGAAGCGCAGCAGGATCAGGTGCGTGCCCGGTGCCGCTGCAGCTG
CCGCCGCTGGAACGCCTGACCCTGGATGATAAAAAACCGCTGAATACCCTGATCTCTGCTACTGGTCTGTGGATG
AGTCGTACCGGAACCATTCATAAAATCAAACACCACGAGGTTAGCCGTTCGAAAATCTATATTGAGATGGCGTGT
GGCGATCATCTGGTTGTGAACAATAGCCGCTCTTCTCGTACAGCACGTGCACTGCGTCACCACAAATATCGTAAA
ACCTGTAAACGTTGCCGTGTGTCCGATGAGGATCTGAACAAATTCCTGACAAAAGCCAATGAGGACCAAACAAGC
GTGAAAGTGAAAGTCGTTAGCGCTCCTACCCGTACTAAAAAAGCAATGCCGAAATCCGTTGCTCGTGCCCCTAAA
CCACTGGAAAACACTGAAGCAGCACAGGCACAGCCGTCTGGAAGCAAATTCTCTCCGGCCATTCCTGTTTCTACC
CAGGAGTCCGTTTCTGTTCCAGCAAGTGTGAGCACCAGCATTAGCAGTATTAGCACCGGTGCCACCGCTAGCGCC
CTGGTTAAAGGCAATACCAATCCGATTACAAGCATGTCTGCCCCGGTTCAAGCATCAGCTCCAGCACTGACAAAA
TCCCAAACCGATCGTCTGGAGGTTCTGCTGAATCCGAAAGACGAAATCAGCCTGAATTCCGGCAAACCGTTTCGT
GAACTGGAGAGCGAACTGCTGTCACGTCGTAAAAAAGACCTGCAACAAATCTATGCCGAAGAACGTGAGAACTAT
CTGGGGAAACTGGAACGTGAAATCACCCGCTTTTTCGTGGATCGTGGCTTTCTGGAGATCAAATCCCCGATTCTG
ATTCCTCTGGAGTATATCGAGCGTATGGGCATCGACAATGATACCGAACTGAGCAAACAAATTTTCCGTGTGGAT
AAAAACTTCTGTCTGCGCCCTATGCTAGCACCAAATCTGGCTAACTATCTGCGCAAACTGGACCGTGCCCTGCCT
GATCCTATCAAAATCTTCGAGATCGGCCCGTGTTATCGTAAAGAGTCCGACGGTAAAGAACATCTGGAGGAGTTT
ACCATGCTGAACTTTTGCCAAATGGGTTCAGGTTGTACTCGTGAGAACCTGGAAAGCATCATCACCGATTTTCTG
AACCACCTGGGCATTGACTTCAAAATTGTGGGCGACAGCTGTATGGTGTTTGGCGACACCCTGGATGTCATGCAC
GGCGACCTGGAACTGTCTAGTGCCGTTGTGGGCCCAATCCCGCTGGATCGTGAGTGGGGTATCGACAAACCTTGG
ATCGGTGCGGGTTTTGGTCTGGAGCGTCTGCTGAAAGTAAAACACGACTTCAAGAACATCAAACGTGCTGCACGT
TCCGAGTCCTATTACAATGGTATTTCTACTAACCTGTAA (SEQ ID NO: 427)

Protein:
MVGRNSAIAAGVCGALFIGYCIYFDRKRRSDPNFKNRLRERRKKQKLAKERAGLSKLPDLKDAEAVQKFFMASND
YTQQATQSYGAYPTQPGQGYSQQSSQPYGQQSYSGYSQSTDTSGYGQSSYSSYGQSQNTGYGTQSTPQGYGSTGG
YGSSQSSQSSYGQQSSYPGYGQQPAPSSTSGSYGSSSQSSSYGQPQSGSYSQQPSYGGQQQSYGQQQSYNPPQGY
GQQNQYNSSSGGGGGGGGGGNYGQDQSSMSSGGGSGGGYGNQDQSGGGGSGGYGQQDRGGRGRGGSGGGGGGGG
GYNRSSGGYEPRGRGGGRGGRGGMGGSDRGGFNKFGGPRDQGSRHDSEQDNSDNNTIFVQGLGENVTIESVADYF
KQIGIIKTNKKTGQPMINLYTDRETGKLKGEATVSFDDPPSAKAAIDWFDGKEFSGNPIKVSFATRRADFNRGGG
NGRGGRGRGGPMGRGGYGGGSGGGGRGGFPSGGGGGGQQRAGDWKCPNPTCENMNFSWRNECNQCKAPKPDGP
GGGPGGSHMGGNYGDDRRGGRGGAIAGKPIPNPLLGLDSTGAPGSAGSAAGSGASIEQKLISEEDLLATMDAQTR
RRERRAEKQAQWKAANPPLDGAGAGAGAGAGAGGLATMDAQTRRRERRAEKQAQWKAANPPLDGAGAGAGAGAGA
GGLATMDAQTRRRERRAEKQAQWKAANPPLDGAGAGAGAGAGAGGLATMDAQTRRRERRAEKQAQWKAANPPLES
RGPVGAPGSAGSAAGSGACPVPLQLPPLERLTLDDKKPLNTLISATGLWMSRTGTIHKIKHHEVSRSKIYIEMAC
GDHLVVNNSRSSRTARALRHHKYRKTCKRCRVSDEDLNKFLTKANEDQTSVKVKVVSAPTRTKKAMPKSVARAPK
PLENTEAAQAQPSGSKFSPAIPVSTQESVSVPASVSTSISSISTGATASALVKGNTNPITSMSAPVQASAPALTK

SQTDRLEVLLNPKDEISLNSGKPFRELESELLSRRKKDLQQIYAEERENYLGKLEREITRFFVDRGFLEIKSPIL
IPLEYIERMGIDNDTELSKQIFRVDKNFCLRPMLAPNLANYLRKLDRALPDPIKIFEIGPCYRKESDGKEHLEEF
TMLNFCQMGSGCTRENLESIITDFLNHLGIDFKIVGDSCMVFGDTLDVMHGDLELSSAVVGPIPLDREWGIDKPW
IGAGFGLERLLKVKHDFKNIKRAARSESYYNGISTNL* (SEQ ID NO: 428)

LCK::FUS::2xPCP::CbzRS
DNA:
ATGGGCTGCGTGTGCAGCAGCAACCCCGAGGGTACCGAGCTCGCCTCAAACGATTATACCCAACAAGCAACCCAA
AGCTATGGGGCCTACCCCACCCAGCCCGGGCAGGGCTATTCCCAGCAGAGCAGTCAGCCCTACGGACAGCAGAGT
TACAGTGGTTATAGCCAGTCCACGGACACTTCAGGATATGGCCAGAGCAGCTATTCTTCTTATGGCCAGAGCCAG
AACACAGGCTATGGAACTCAGTCAACTCCCCAGGGATATGGCTCGACTGGCGGCTATGGCAGTAGCCAGAGCTCC
CAATCGTCTTACGGGCAGCAGTCCTCCTACCCTGGCTATGGCCAGCAGCCAGCTCCCAGCAGCACCTCGGGAAGT
TACGGTAGCAGTTCTCAGAGCAGCAGCTATGGGCAGCCCCAGAGTGGGAGCTACAGCCAGCAGCCTAGCTATGGT
GGACAGCAGCAAAGCTATGGACAGCAGCAAAGCTATAATCCCCCTCAGGGCTATGGACAGCAGAACCAGTACAAC
AGCAGCAGTGGTGGTGGAGGTGGAGGTGGAGGTGGAGGTAACTATGGCCAAGATCAATCCTCCATGAGTAGTGGT
GGTGGCAGTGGTGGCGGTTATGGCAATCAAGACCAGAGTGGTGGAGGTGGCAGCGGTGGCTATGGACAGCAGGAC
CGTGGAGGCCGCGGCAGGGGTGGCAGTGGTGGCGGCGGCGGCGGCGGTGGTGGTTACAACCGCAGCAGTGGT
GGCTATGAACCCAGAGGTCGTGGAGGTGGCCGTGGAGGCAGAGGTGGCATGGGCGGAAGTGACCGTGGTGGCTTC
AATAAATTTGGTGGCCCTCGGGACCAAGGATCACGTCATGACTCCGAACAGGATAATTCAGACAACAACACCATC
TTTGTGCAAGGCCTGGGTGAGAATGTTACAATTGAGTCTGTGGCTGATTACTTCAAGCAGATTGGTATTATTAAG
ACAAACAAGAAAACGGGACAGCCCATGATTAATTTGTACACAGACAGGGAAACTGGCAAGCTGAAGGGAGAGGCA
ACGGTCTCTTTTGATGACCCACCTTCAGCTAAAGCAGCTATTGACTGGTTTGATGGTAAAGAATTCTCCGGAAAT
CCTATCAAGGTCTCATTTGCTACTCGCCGGGCAGACTTTAATCGGGGTGGTGGCAATGGTCGTGGAGGCCGAGGG
CGAGGAGGACCCATGGGCCGTGGAGGCTATGGAGGTGGTGGCAGTGGTGGTGGTGGCCGAGGAGGATTTCCCAGT
GGAGGTGGTGGCGGTGGAGGACAGCAGCGAGCTGGTGACTGGAAGTGTCCTAATCCCACCTGTGAGAATATGAAC
TTCTCTTGGAGGAATGAATGCAACCAGTGTAAGGCCCCTAAACCAGATGGCCCAGGAGGGGGACCAGGTGGCTCT
CACATGGGGGGTAACTACGGGGATGATCGTCGTGGTGGCAGAGGAGGCGCGATCGCAGGCGCCGATTACAAGGAC
GATGATGACAAGGGAGCACCAGGAAGTGCTGGTTCTGCTGCTGGTAGTGGAGCATCGATAGAGCAGAAGCTGATC
TCAGAGGAGGACCTGATCGAAGGCCGCCATATGCTAGCCTCCAAAACCATCGTTCTTTCGGTCGGCGAGGCTACT
CGCACTCTGACTGAGATCCAGTCCACCGCAGACCGTCAGATCTTCGAAGAGAAGGTCGGGCCTCTGGTGGGTCGG
CTGCGCCTCACGGCTTCGCTCCGTCAAAACGGAGCCAAGACCGCGTATCGCGTCAACCTAAAACTGGATCAGGCG
GACGTCGTTGATTCCGGACTTCCGAAAGTGCGCTACACTCAGGTATGGTCGCACGACGTGACAATCGTTGCGAAT
AGCACCGAGGCCTCGCGCAAATCGTTGTACGATTTGACCAAGTCCCTCGTCGCGACCTCGCAGGTCGAAGATCTT
GTCGTCAACCTTGTGCCGCTGGGCCGTGCGGATCCGCTAGCCTCCAAAACCATCGTTCTTTCGGTCGGCGAGGCT
ACTCGCACTCTGACTGAGATCCAGTCCACCGCAGACCGTCAGATCTTCGAAGAGAAGGTCGGGCCTCTGGTGGGT
CGGCTGCGCCTCACGGCTTCGCTCCGTCAAAACGGAGCCAAGACCGCGTATCGCGTCAACCTAAAACTGGATCAG
GCGGACGTCGTTGATTCCGGACTTCCGAAAGTGCGCTACACTCAGGTATGGTCGCACGACGTGACAATCGTTGCG
AATAGCACCGAGGCCTCGCGCAAATCGTTGTACGATTTGACCAAGTCCCTCGTCGCGACCTCGCAGGTCGAAGAT
CTTGTCGTCAACCTTGTGCCGCTGGGCCGTCCACCGGTCGCCACCGGTGCTCCTGGTTCAGCAGGAAGCGCAGCA
GGATCAGGTGCGTGCCCGGTGCCGCTGCAGCTGCCGCCGCTGGAACGCCTGACCCTGGATGACAAAAAACCGCTG
AATACCCTGATCTCTGCTACTGGTCTGTGGATGAGTCGTACCGGAACCATTCATAAAATCAAACACCACGAGGTT
AGCCGTTCGAAAATCTATATTGAGATGGCGTGTGGCGATCATCTGGTTGTGAACAATAGCCGCTCTTCTCGTACA
GCACGTGCACTGCGTCACCACAAATATCGTAAAACCTGTAAACGTTGCCGTGTGTCCGATGAGGATCTGAACAAA
TTCCTGACAAAAGCCAATGAGGACCAAACAAGCGTGAAAGTGAAAGTCGTTAGCGCTCCTACCCGTACTAAAAAA
GCAATGCCGAAATCCGTTGCTCGTGCCCCTAAACCACTGGAAAACACTGAAGCAGCACAGGCACAGCCGTCTGGA
AGCAAATTCTCTCCGGCCATTCCTGTTTCTACCCAGGAGTCCGTTTCTGTTCCAGCAAGTGTGAGCACCAGCATT
AGCAGTATTAGCACCGGTGCCACCGCTAGCGCCCTGGTTAAAGGCAATACCAATCCGATTACAAGCATGTCTGCC
CCGGTTCAAGCATCAGCTCCAGCACTGACAAAATCCCAAACCGATCGTCTGGAGGTTCTGCTGAATCCGAAAGAC
GAAATCAGCCTGAATTCCGGCAAACCGTTTCGTGAACTGGAGAGCGAACTGCTGTCACGTCGTAAAAAAGACCTG
CAACAAATCTATGCCGAAGAACGTGAGAACTATCTGGGGAAACTGGAACGTGAAATCACCCGCTTTTTCGTGGAT
CGTGGCTTTCTGGAGATCAAATCCCCGATTCTGATTCCTCTGGAGTATATCGAGCGTATGGGCATCGACAATGAT
ACCGAACTGAGCAAACAAATTTTCCGTGTGGATAAAAACTTCTGTCTGCGCCCTATGCTTGCACCAAATCTGATG
AACTATGGACGCAAACTGGACCGTGCCCTGCCTGATCCTATCAAAATCTTCGAGATCGGCCCGTGTTATCGTAAA
GAGTCCGACGGTAAAGAACATCTGGAGGAGTTTACCATGCTGAACTTTACACAAATGGGTTCAGGTTGTACTCGT
GAGAACCTGGAAAGCATCATCACCGATTTTCTGAACCACCTGGGCATTGACTTCAAAATTGTGGGCGACAGCTGT
ATGGTGTATGGCGACACCCTGGATGTCATGCACGGCGACCTGGAACTGTCTAGTGCCGTTGTTGGACCAATTCCG
CTGGACCGTGAGTGGGGTATCGACAAACCGTGGATCGGAGCAGGATTCGGTCTGGAACGCCTGCTGAAAGTGAAA

CACGACTTCAAAAACATCAAACGTGCCGCCCGTTCTGAATCGTATTATAACGGGATCTCTACGAACCTGTAA
(SEQ ID NO: 429)

Protein:
MGCVCSSNPEGTELASNDYTQQATQSYGAYPTQPGQGYSQQSSQPYGQQSYSGYSQSTDTSGYGQSSYSSYGQSQ
NTGYGTQSTPQGYGSTGGYGSSQSSQSSYGQQSSYPGYGQQPAPSSTSGSYGSSSQSSSYGQPQSGSYSQQPSYG
GQQQSYGQQQSYNPPQGYGQQNQYNSSSGGGGGGGGGGNYGQDQSSMSSGGGSGGGYGNQDQSGGGGSGGYGQQD
RGGRGRGGSGGGGGGGGGGYNRSSGGYEPRGRGGGRGGRGGMGGSDRGGFNKFGGPRDQGSRHDSEQDNSDNNTI
FVQGLGENVTIESVADYFKQIGIIKTNKKTGQPMINLYTDRETGKLKGEATVSFDDPPSAKAAIDWFDGKEFSGN
PIKVSFATRRADFNRGGGNGRGGRGRGGPMGRGGYGGGGSGGGGRGGFPSGGGGGGGQQRAGDWKCPNPTCENMN
FSWRNECNQCKAPKPDGPGGGPGGSHMGGNYGDDRRGGRGGAIAGADYKDDDDKGAPGSAGSAAGSGASIEQKLI
SEEDLIEGRHMLASKTIVLSVGEATRTLTEIQSTADRQIFEEKVGPLVGRLRLTASLRQNGAKTAYRVNLKLDQA
DVVDSGLPKVRYTQVWSHDVTIVANSTEASRKSLYDLTKSLVATSQVEDLVVNLVPLGRADPLASKTIVLSVGEA
TRTLTEIQSTADRQIFEEKVGPLVGRLRLTASLRQNGAKTAYRVNLKLDQADVVDSGLPKVRYTQVWSHDVTIVA
NSTEASRKSLYDLTKSLVATSQVEDLVVNLVPLGRPPVATGAPGSAGSAAGSGACPVPLQLPPLERLTLDDKKPL
NTLISATGLWMSRTGTIHKIKHHEVSRSKIYIEMACGDHLVVNNSRSSRTARALRHHKYRKTCKRCRVSDEDLNK
FLTKANEDQTSVKVKVVSAPTRTKKAMPKSVARAPKPLENTEAAQAQPSGSKFSPAIPVSTQESVSVPASVSTSI
SSISTGATASALVKGNTNPITSMSAPVQASAPALTKSQTDRLEVLLNPKDEISLNSGKPFRELESELLSRRKKDL
QQIYAEERENYLGKLEREITRFFVDRGFLEIKSPILIPLEYIERMGIDNDTELSKQIFRVDKNFCLRPMLAPNLM
NYGRKLDRALPDPIKIFEIGPCYRKESDGKEHLEEFTMLNFTQMGSGCTRENLESIITDFLNHLGIDFKIVGDSC
MVYGDTLDVMHGDLELSSAVVGPIPLDREWGIDKPWIGAGFGLERLLKVKHDFKNIKRAARSESYYNGISTNL*
(SEQ ID NO: 430)

LCK::FUS::PCP::CbzRS
DNA:
ATGGGCTGCGTGTGCAGCAGCAACCCCGAGGGTACCGAGCTCGCCTCAAACGATTATACCCAACAAGCAACCCAA
AGCTATGGGGCCTACCCCACCCAGCCCGGGCAGGGCTATTCCCAGCAGAGCAGTCAGCCCTACGGACAGCAGAGT
TACAGTGGTTATAGCCAGTCCACGGACACTTCAGGATATGGCCAGAGCAGCTATTCTTCTTATGGCCAGAGCCAG
AACACAGGCTATGGAACTCAGTCAACTCCCCAGGGATATGGCTCGACTGGCGGCTATGGCAGTAGCCAGAGCTCC
CAATCGTCTTACGGGCAGCAGTCCTCCTACCCTGGCTATGGCCAGCAGCCAGCTCCCAGCAGCACCTCGGGAAGT
TACGGTAGCAGTTCTCAGAGCAGCAGCTATGGGCAGCCCCAGAGTGGGAGCTACAGCCAGCAGCCTAGCTATGGT
GGACAGCAGCAAAGCTATGGACAGCAGCAAAGCTATAATCCCCCTCAGGGCTATGGACAGCAGAACCAGTACAAC
AGCAGCAGTGGTGGTGGAGGTGGAGGTGGAGGTGGAGGTAACTATGGCCAAGATCAATCCTCCATGAGTAGTGGT
GGTGGCAGTGGTGGCGGTTATGGCAATCAAGACCAGAGTGGTGGAGGTGGCAGCGGTGGCTATGGACAGCAGGAC
CGTGGAGGCCGCGGCAGGGGTGGCAGTGGTGGCGGCGGCGGCGGCGGTGGTGGTTACAACCGCAGCAGTGGT
GGCTATGAACCCAGAGGTCGTGGAGGTGGCCGTGGAGGCAGAGGTGGCATGGGCGGAAGTGACCGTGGTGGCTTC
AATAAATTTGGTGGCCCTCGGGACCAAGGATCACGTCATGACTCCGAACAGGATAATTCAGACAACAACACCATC
TTTGTGCAAGGCCTGGGTGAGAATGTTACAATTGAGTCTGTGGCTGATTACTTCAAGCAGATTGGTATTATTAAG
ACAAACAAGAAAACGGGACAGCCCATGATTAATTTGTACACAGACAGGGAAACTGGCAAGCTGAAGGGAGAGGCA
ACGGTCTCTTTTGATGACCCACCTTCAGCTAAAGCAGCTATTGACTGGTTTGATGGTAAAGAATTCTCCGGAAAT
CCTATCAAGGTCTCATTTGCTACTCGCCGGGCAGACTTTAATCGGGGTGGTGGCAATGGTCGTGGAGGCCGAGGG
CGAGGAGGACCCATGGGCCGTGGAGGCTATGGAGGTGGTGGCAGTGGTGGTGGTGGCCGAGGAGGATTTCCCAGT
GGAGGTGGTGGCGGTGGAGGACAGCAGCGAGCTGGTGACTGGAAGTGTCCTAATCCCACCTGTGAGAATATGAAC
TTCTCTTGGAGGAATGAATGCAACCAGTGTAAGGCCCCTAAACCAGATGGCCCAGGAGGGGGACCAGGTGGCTCT
CACATGGGGGGTAACTACGGGGATGATCGTCGTGGTGGCAGAGGAGGCGCGATCGCAGGCGCCGATTACAAGGAC
GATGATGACAAGGGAGCACCAGGAAGTGCTGGTTCTGCTGCTGGTAGTGGAGCATCGATAGAGCAGAAGCTGATC
TCAGAGGAGGACCTGATCGAAGGCCGCCATATGCTAGCCTCCAAAACCATCGTTCTTTCGGTCGGCGAGGCTACT
CGCACTCTGACTGAGATCCAGTCCACCGCAGACCGTCAGATCTTCGAAGAGAAGGTCGGGCCTCTGGTGGGTCGG
CTGCGCCTCACGGCTTCGCTCCGTCAAAACGGAGCCAAGACCGCGTATCGCGTCAACCTAAAACTGGATCAGGCG
GACGTCGTTGATTCCGGACTTCCGAAAGTGCGCTACACTCAGGTATGGTCGCACGACGTGACAATCGTTGCGAAT
AGCACCGAGGCCTCGCGCAAATCGTTGTACGATTTGACCAAGTCCCTCGTCGCGACCTCGCAGGTCGAAGATCTT
GTCGTCAACCTTGTGCCGCTGGGCCGTGCGGATCCGCTAGCCGGTGCTCCTGGTTCAGCAGGAAGCGCAGCAGGA
TCAGGTGCGTGCCCGGTGCCGCTGCAGCTGCCGCCGCTGGAACGCCTGACCCTGGATGACAAAAAACCGCTGAAT
ACCCTGATCTCTGCTACTGGTCTGTGGATGAGTCGTACCGGAACCATTCATAAAATCAAACACCACGAGGTTAGC
CGTTCGAAAATCTATATTGAGATGGCGTGTGGCGATCATCTGGTTGTGAACAATAGCCGCTCTTCTCGTACAGCA
CGTGCACTGCGTCACCACAAATATCGTAAAACCTGTAAACGTTGCCGTGTGTCCGATGAGGATCTGAACAAATTC
CTGACAAAAGCCAATGAGGACCAAACAAGCGTGAAAGTGAAAGTCGTTAGCGCTCCTACCCGTACTAAAAAAGCA
ATGCCGAAATCCGTTGCTCGTGCCCCTAAACCACTGGAAAACACTGAAGCAGCACAGGCACAGCCGTCTGGAAGC

AAATTCTCTCCGGCCATTCCTGTTTCTACCCAGGAGTCCGTTTCTGTTCCAGCAAGTGTGAGCACCAGCATTAGC
AGTATTAGCACCGGTGCCACCGCTAGCGCCCTGGTTAAAGGCAATACCAATCCGATTACAAGCATGTCTGCCCCG
GTTCAAGCATCAGCTCCAGCACTGACAAAATCCCAAACCGATCGTCTGGAGGTTCTGCTGAATCCGAAAGACGAA
ATCAGCCTGAATTCCGGCAAACCGTTTCGTGAACTGGAGAGCGAACTGCTGTCACGTCGTAAAAAAGACCTGCAA
CAAATCTATGCCGAAGAACGTGAGAACTATCTGGGGAAACTGGAACGTGAAATCACCCGCTTTTTCGTGGATCGT
GGCTTTCTGGAGATCAAATCCCCGATTCTGATTCCTCTGGAGTATATCGAGCGTATGGGCATCGACAATGATACC
GAACTGAGCAAACAAATTTTCCGTGTGGATAAAAACTTCTGTCTGCGCCCTATGCTTGCACCAAATCTGATGAAC
TATGGACGCAAACTGGACCGTGCCCTGCCTGATCCTATCAAAATCTTCGAGATCGGCCCGTGTTATCGTAAAGAG
TCCGACGGTAAAGAACATCTGGAGGAGTTTACCATGCTGAACTTTACACAAATGGGTTCAGGTTGTACTCGTGAG
AACCTGGAAAGCATCATCACCGATTTTCTGAACCACCTGGGCATTGACTTCAAAATTGTGGGCGACAGCTGTATG
GTGTATGGCGACACCCTGGATGTCATGCACGGCGACCTGGAACTGTCTAGTGCCGTTGTTGGACCAATTCCGCTG
GACCGTGAGTGGGGTATCGACAAACCGTGGATCGGAGCAGGATTCGGTCTGGAACGCCTGCTGAAAGTGAAACAC
GACTTCAAAAACATCAAACGTGCCGCCCGTTCTGAATCGTATTATAACGGGATCTCTACGAACCTGTAA   (SEQ
ID NO: 431)

Protein:
MGCVCSSNPEGTELASNDYTQQATQSYGAYPTQPGQGYSQQSSQPYGQQSYSGYSQSTDTSGYGQSSYSSYGQSQ
NTGYGTQSTPQGYGSTGGYGSSQSSQSSYGQQSSYPGYGQQPAPSSTSGSYGSSSQSSSYGQPQSGSYSQQPSYG
GQQQSYGQQQSYNPPQGYGQQNQYNSSSGGGGGGGGGNYGQDQSSMSSGGGSGGGYGNQDQSGGGGSGGYGQQD
RGGRGRGGSGGGGGGGGGYNRSSGGYEPRGRGGGRGGRGGMGGSDRGGFNKFGGPRDQGSRHDSEQDNSDNNTI
FVQGLGENVTIESVADYFKQIGIIKTNKKTGQPMINLYTDRETGKLKGEATVSFDDPPSAKAAIDWFDGKEFSGN
PIKVSFATRRADFNRGGGNGRGGRGRGGPMGRGGYGGGGSGGGGRGGFPSGGGGGGGQQRAGDWKCPNPTCENMN
FSWRNECNQCKAPKPDGPGGGPGGSHMGGNYGDDRRGGRGGAIAGADYKDDDDKGAPGSAGSAAGSGASIEQKLI
SEEDLIEGRHMLASKTIVLSVGEATRTLTEIQSTADRQIFEEKVGPLVGRLRLTASLRQNGAKTAYRVNLKLDQA
DVVDSGLPKVRYTQVWSHDVTIVANSTEASRKSLYDLTKSLVATSQVEDLVVNLVPLGRADPLAGAPGSAGSAAG
SGACPVPLQLPPLERLTLDDKKPLNTLISATGLWMSRTGTIHKIKHHEVSRSKIYIEMACGDHLVVNNSRSSRTA
RALRHHKYRKTCKRCRVSDEDLNKFLTKANEDQTSVKVKVVSAPTRTKKAMPKSVARAPKPLENTEAAQAQPSGS
KFSPAIPVSTQESVSVPASVSTSISSISTGATASALVKGNTNPITSMSAPVQASAPALTKSQTDRLEVLLNPKDE
ISLNSGKPFRELESELLSRRKKDLQQIYAEERENYLGKLEREITRFFVDRGFLEIKSPILIPLEYIERMGIDNDT
ELSKQIFRVDKNFCLRPMLAPNLMNYGRKLDRALPDPIKIFEIGPCYRKESDGKEHLEEFTMLNFTQMGSGCTRE
NLESIITDFLNHLGIDFKIVGDSCMVYGDTLDVMHGDLELSSAVVGPIPLDREWGIDKPWIGAGFGLERLLKVKH
DFKNIKRAARSESYYNGISTNL* (SEQ ID NO: 432)

TOM20::FUS::CbzRS
DNA:
ATGGTGGGTCGGAACAGCGCCATCGCCGCCGGTGTATGCGGGGCCCTTTTCATTGGGTACTGCATCTACTTCGAC
CGCAAAAGACGAAGTGACCCCAACTTCAAGAACAGGCTTCGAGAACGAAGAAAGAAACAGAAGCTTGCCAAGGAG
AGAGCTGGGCTTTCCAAGTTACCTGACCTTAAAGATGCTGAAGCTGTTCAGAAATTCTTCATGGCCTCAAACGAT
TATACCCAACAAGCAACCCAAAGCTATGGGGCCTACCCCACCCAGCCCGGGCAGGGCTATTCCCAGCAGAGCAGT
CAGCCCTACGGACAGCAGAGTTACAGTGGTTATAGCCAGTCCACGGACACTTCAGGATATGGCCAGAGCAGCTAT
TCTTCTTATGGCCAGAGCCAGAACACAGGCTATGGAACTCAGTCAACTCCCCAGGGATATGGCTCGACTGGCGGC
TATGGCAGTAGCCAGAGCTCCCAATCGTCTTACGGGCAGCAGTCCTCCTACCCTGGCTATGGCCAGCAGCCAGCT
CCCAGCAGCACCTCGGGAAGTTACGGTAGCAGTTCTCAGAGCAGCAGCTATGGGCAGCCCCAGAGTGGGAGCTAC
AGCCAGCAGCCTAGCTATGGTGGACAGCAGCAAAGCTATGGACAGCAGCAAAGCTATAATCCCCCTCAGGGCTAT
GGACAGCAGAACCAGTACAACAGCAGCAGTGGTGGTGGAGGTGGAGGTGGAGGTGGAGGTAACTATGGCCAAGAT
CAATCCTCCATGAGTAGTGGTGGTGGCAGTGGTGGCGGTTATGGCAATCAAGACCAGAGTGGTGGAGGTGGCAGC
GGTGGCTATGGACAGCAGGACCGTGGAGGCCGCGGCAGGGGTGGCAGTGGTGGCGGCGGCGGCGGCGGCGGTGGT
GGTTACAACCGCAGCAGTGGTGGCTATGAACCCAGAGGTCGTGGAGGTGGCCGTGGAGGCAGAGGTGGCATGGGC
GGAAGTGACCGTGGTGGCTTCAATAAATTTGGTGGCCCTCGGGACCAAGGATCACGTCATGACTCCGAACAGGAT
AATTCAGACAACAACACCATCTTTGTGCAAGGCCTGGGTGAGAATGTTACAATTGAGTCTGTGGCTGATTACTTC
AAGCAGATTGGTATTATTAAGACAAACAAGAAAACGGGACAGCCCATGATTAATTTGTACACAGACAGGGAAACT
GGCAAGCTGAAGGGAGAGGCAACGGTCTCTTTTGATGACCCACCTTCAGCTAAAGCAGCTATTGACTGGTTTGAT
GGTAAAGAATTCTCCGGAAATCCTATCAAGGTCTCATTTGCTACTCGCCGGGCAGACTTTAATCGGGGTGGTGGC
AATGGTCGTGGAGGCCGAGGGCGAGGAGGACCCATGGGCCGTGGAGGCTATGGAGGTGGTGGCAGTGGTGGTGGT
GGCCGAGGAGGATTTCCCAGTGGAGGTGGTGGCGGTGGAGGACAGCAGCGAGCTGGTGACTGGAAGTGTCCTAAT
CCCACCTGTGAGAATATGAACTTCTCTTGGAGGAATGAATGCAACCAGTGTAAGGCCCCTAAACCAGATGGCCCA
GGAGGGGGACCAGGTGGCTCTCACATGGGGGGTAACTACGGGGATGATCGTCGTGGTGGCAGAGGAGGCGCGATC
GCAGGCAAGCCTATTCCCAACCCCCTGCTGGGCCTGGATAGCACCGGAGCACCAGGAAGTGCTGGTTCTGCTGCT

GGTAGTGGAGCATCGATAGAGCAGAAGCTGATCTCAGAGGAGGACCTGATCGAAGGCCGCCATATGCTAGCCTCC
AAAACCATCGTTCTTTCGGTCGGCGAGGCTACTCGCACTCTGACTGAGATCCAGTCCACCGCAGACCGTCAGATC
TTCGAAGAGAAGGTCGGGCCTCTGGTGGGTCGGCTGCGCCTCACGGCTTCGCTCCGTCAAAACGGAGCCAAGACC
GCGTATCGCGTCAACCTAAAACTGGATCAGGCGGACGTCGTTGATTCCGGACTTCCGAAAGTGCGCTACACTCAG
GTATGGTCGCACGACGTGACAATCGTTGCGAATAGCACCGAGGCCTCGCGCAAATCGTTGTACGATTTGACCAAG
TCCCTCGTCGCGACCTCGCAGGTCGAAGATCTTGTCGTCAACCTTGTGCCGCTGGGCCGTGCGGATCCGCTAGCC
GGTGCTCCTGGTTCAGCAGGAAGCGCAGCAGGATCAGGTGCGTGCCCGGTGCCGCTGCAGCTGCCGCCGCTGGAA
CGCCTGACCCTGGATGACAAAAAACCGCTGAATACCCTGATCTCTGCTACTGGTCTGTGGATGAGTCGTACCGGA
ACCATTCATAAAATCAAACACCACGAGGTTAGCCGTTCGAAAATCTATATTGAGATGGCGTGTGGCGATCATCTG
GTTGTGAACAATAGCCGCTCTTCTCGTACAGCACGTGCACTGCGTCACCACAAATATCGTAAAACCTGTAAACGT
TGCCGTGTGTCCGATGAGGATCTGAACAAATTCCTGACAAAAGCCAATGAGGACCAAACAAGCGTGAAAGTGAAA
GTCGTTAGCGCTCCTACCCGTACTAAAAAAGCAATGCCGAAATCCGTTGCTCGTGCCCCTAAACCACTGGAAAAC
ACTGAAGCAGCACAGGCACAGCCGTCTGGAAGCAAATTCTCTCCGGCCATTCCTGTTTCTACCCAGGAGTCCGTT
TCTGTTCCAGCAAGTGTGAGCACCAGCATTAGCAGTATTAGCACCGGTGCCACCGCTAGCGCCCTGGTTAAAGGC
AATACCAATCCGATTACAAGCATGTCTGCCCCGGTTCAAGCATCAGCTCCAGCACTGACAAAATCCCAAACCGAT
CGTCTGGAGGTTCTGCTGAATCCGAAAGACGAAATCAGCCTGAATTCCGGCAAACCGTTTCGTGAACTGGAGAGC
GAACTGCTGTCACGTCGTAAAAAAGACCTGCAACAAATCTATGCCGAAGAACGTGAGAACTATCTGGGGAAACTG
GAACGTGAAATCACCCGCTTTTTCGTGGATCGTGGCTTTCTGGAGATCAAATCCCCGATTCTGATTCCTCTGGAG
TATATCGAGCGTATGGGCATCGACAATGATACCGAACTGAGCAAACAAATTTTCCGTGTGGATAAAAACTTCTGT
CTGCGCCCTATGCTTGCACCAAATCTGATGAACTATGGACGCAAACTGGACCGTGCCCTGCCTGATCCTATCAAA
ATCTTCGAGATCGGCCCGTGTTATCGTAAAGAGTCCGACGGTAAAGAACATCTGGAGGAGTTTACCATGCTGAAC
TTTACACAAATGGGTTCAGGTTGTACTCGTGAGAACCTGGAAAGCATCATCACCGATTTTCTGAACCACCTGGGC
ATTGACTTCAAAATTGTGGGCGACAGCTGTATGGTGTATGGCGACACCCTGGATGTCATGCACGGCGACCTGGAA
CTGTCTAGTGCCGTTGTTGGACCAATTCCGCTGGACCGTGAGTGGGGTATCGACAAACCGTGGATCGGAGCAGGA
TTCGGTCTGGAACGCCTGCTGAAAGTGAAACACGACTTCAAAAACATCAAACGTGCCGCCCGTTCTGAATCGTAT
TATAACGGGATCTCTACGAACCTGTAA (SEQ ID NO: 433)

Protein:
MVGRNSAIAAGVCGALFIGYCIYFDRKRRSDPNFKNRLRERRKKQKLAKERAGLSKLPDLKDAEAVQKFFMASND
YTQQATQSYGAYPTQPGQGYSQQSSQPYGQQSYSGYSQSTDTSGYGQSSYSSYGQSQNTGYGTQSTPQGYGSTGG
YGSSQSSQSSYGQQSSYPGYGQQPAPSSTSGSYGSSSQSSSYGQPQSGSYSQQPSYGGQQQSYGQQQSYNPPQGY
GQQNQYNSSSGGGGGGGGGGNYGQDQSSMSSGGGSGGGYGNQDQSGGGGSGGYGQQDRGGRGRGGSGGGGGGGG
GYNRSSGGYEPRGRGGGRGGRGGMGGSDRGGFNKFGGPRDQGSRHDSEQDNSDNNTIFVQGLGENVTIESVADYF
KQIGIIKTNKKTGQPMINLYTDRETGKLKGEATVSFDDPPSAKAAIDWFDGKEFSGNPIKVSFATRRADFNRGGG
NGRGGRGRGGPMGRGGYGGGGSGGGGRGGFPSGGGGGGGQQRAGDWKCPNPTCENMNFSWRNECNQCKAPKPDGP
GGGPGGSHMGGNYGDDRRGGRGGAIAGKPIPNPLLGLDSTGAPGSAGSAAGSGASIEQKLISEEDLIEGRHMLAS
KTIVLSVGEATRTLTEIQSTADRQIFEEKVGPLVGRLRLTASLRQNGAKTAYRVNLKLDQADVVDSGLPKVRYTQ
VWSHDVTIVANSTEASRKSLYDLTKSLVATSQVEDLVVNLVPLGRADPLAGAPGSAGSAAGSGACPVPLQLPPLE
RLTLDDKKPLNTLISATGLWMSRTGTIHKIKHHEVSRSKIYIEMACGDHLVVNNSRSSRTARALRHHKYRKTCKR
CRVSDEDLNKFLTKANEDQTSVKVKVVSAPTRTKKAMPKSVARAPKPLENTEAAQAQPSGSKFSPAIPVSTQESV
SVPASVSTSISSISTGATASALVKGNTNPITSMSAPVQASAPALTKSQTDRLEVLLNPKDEISLNSGKPFRELES
ELLSRRKKDLQQIYAEERENYLGKLEREITRFFVDRGFLEIKSPILIPLEYIERMGIDNDTELSKQIFRVDKNFC
LRPMLAPNLMNYGRKLDRALPDPIKIFEIGPCYRKESDGKEHLEEFTMLNFTQMGSGCTRENLESIITDFLNHLG
IDFKIVGDSCMVYGDTLDVMHGDLELSSAVVGPIPLDREWGIDKPWIGAGFGLERLLKVKHDFKNIKRAARSESY
YNGISTNL* (SEQ ID NO: 434)

TOM20::FUS::2xPCP::CbzRS
DNA:
ATGGTGGGTCGGAACAGCGCCATCGCCGCCGGTGTATGCGGGGCCCTTTTCATTGGGTACTGCATCTACTTCGAC
CGCAAAAGACGAAGTGACCCCAACTTCAAGAACAGGCTTCGAGAACGAAGAAAGAAACAGAAGCTTGCCAAGGAG
AGAGCTGGGCTTTCCAAGTTACCTGACCTTAAAGATGCTGAAGCTGTTCAGAAATTCTTCATGGCCTCAAACGAT
TATACCCAACAAGCAACCCAAAGCTATGGGGCCTACCCCACCCAGCCCGGGCAGGGCTATTCCCAGCAGAGCAGT
CAGCCCTACGGACAGCAGAGTTACAGTGGTTATAGCCAGTCCACGGACACTTCAGGATATGGCCAGAGCAGCTAT
TCTTCTTATGGCCAGAGCCAGAACACAGGCTATGGAACTCAGTCAACTCCCCAGGGATATGGCTCGACTGGCGGC
TATGGCAGTAGCCAGAGCTCCCAATCGTCTTACGGGCAGCAGTCCTCCTACCCTGGCTATGGCCAGCAGCCAGCT
CCCAGCAGCACCTCGGGAAGTTACGGTAGCAGTTCTCAGAGCAGCAGCTATGGGCAGCCCCAGAGTGGGAGCTAC
AGCCAGCAGCCTAGCTATGGTGGACAGCAGCAAAGCTATGGACAGCAGCAAAGCTATAATCCCCCTCAGGGCTAT

GGACAGCAGAACCAGTACAACAGCAGCAGTGGTGGTGGAGGTGGAGGTGGAGGTGGAGGTAACTATGGCCAAGAT
CAATCCTCCATGAGTAGTGGTGGTGGCAGTGGTGGCGGTTATGGCAATCAAGACCAGAGTGGTGGAGGTGGCAGC
GGTGGCTATGGACAGCAGGACCGTGGAGGCCGCGGCAGGGGTGGCAGTGGTGGCGGCGGCGGCGGCGGCGGTGGT
GGTTACAACCGCAGCAGTGGTGGCTATGAACCCAGAGGTCGTGGAGGTGGCCGTGGAGGCAGAGGTGGCATGGGC
GGAAGTGACCGTGGTGGCTTCAATAAATTTGGTGGCCCTCGGGACCAAGGATCACGTCATGACTCCGAACAGGAT
AATTCAGACAACAACACCATCTTTGTGCAAGGCCTGGGTGAGAATGTTACAATTGAGTCTGTGGCTGATTACTTC
AAGCAGATTGGTATTATTAAGACAAACAAGAAAACGGGACAGCCCATGATTAATTTGTACACAGACAGGGAAACT
GGCAAGCTGAAGGGAGAGGCAACGGTCTCTTTTGATGACCCACCTTCAGCTAAAGCAGCTATTGACTGGTTTGAT
GGTAAAGAATTCTCCGGAAATCCTATCAAGGTCTCATTTGCTACTCGCCGGGCAGACTTTAATCGGGGTGGTGGC
AATGGTCGTGGAGGCCGAGGGCGAGGAGGACCCATGGGCCGTGGAGGCTATGGAGGTGGTGGCAGTGGTGGTGGT
GGCCGAGGAGGATTTCCCAGTGGAGGTGGTGGCGGTGGAGGACAGCAGCGAGCTGGTGACTGGAAGTGTCCTAAT
CCCACCTGTGAGAATATGAACTTCTCTTGGAGGAATGAATGCAACCAGTGTAAGGCCCCTAAACCAGATGGCCCA
GGAGGGGGACCAGGTGGCTCTCACATGGGGGGTAACTACGGGGATGATCGTCGTGGTGGCAGAGGAGGCGCGATC
GCAGGCAAGCCTATTCCCAACCCCCTGCTGGGCCTGGATAGCACCGGAGCACCAGGAAGTGCTGGTTCTGCTGCT
GGTAGTGGAGCATCGATAGAGCAGAAGCTGATCTCAGAGGAGGACCTGATCGAAGGCCGCCATATGCTAGCCTCC
AAAACCATCGTTCTTTCGGTCGGCGAGGCTACTCGCACTCTGACTGAGATCCAGTCCACCGCAGACCGTCAGATC
TTCGAAGAGAAGGTCGGGCCTCTGGTGGGTCGGCTGCGCCTCACGGCTTCGCTCCGTCAAAACGGAGCCAAGACC
GCGTATCGCGTCAACCTAAAACTGGATCAGGCGGACGTCGTTGATTCCGGACTTCCGAAAGTGCGCTACACTCAG
GTATGGTCGCACGACGTGACAATCGTTGCGAATAGCACCGAGGCCTCGCGCAAATCGTTGTACGATTTGACCAAG
TCCCTCGTCGCGACCTCGCAGGTCGAAGATCTTGTCGTCAACCTTGTGCCGCTGGGCCGTGCGGATCCGCTAGCC
TCCAAAACCATCGTTCTTTCGGTCGGCGAGGCTACTCGCACTCTGACTGAGATCCAGTCCACCGCAGACCGTCAG
ATCTTCGAAGAGAAGGTCGGGCCTCTGGTGGGTCGGCTGCGCCTCACGGCTTCGCTCCGTCAAAACGGAGCCAAG
ACCGCGTATCGCGTCAACCTAAAACTGGATCAGGCGGACGTCGTTGATTCCGGACTTCCGAAAGTGCGCTACACT
CAGGTATGGTCGCACGACGTGACAATCGTTGCGAATAGCACCGAGGCCTCGCGCAAATCGTTGTACGATTTGACC
AAGTCCCTCGTCGCGACCTCGCAGGTCGAAGATCTTGTCGTCAACCTTGTGCCGCTGGGCCGTCCACCGGTCGCC
ACCGGTGCTCCTGGTTCAGCAGGAAGCGCAGCAGGATCAGGTGCGTGCCCGGTGCCGCTGCAGCTGCCGCCGCTG
GAACGCCTGACCCTGGATGACAAAAAACCGCTGAATACCCTGATCTCTGCTACTGGTCTGTGGATGAGTCGTACC
GGAACCATTCATAAAATCAAACACCACGAGGTTAGCCGTTCGAAAATCTATATTGAGATGGCGTGTGGCGATCAT
CTGGTTGTGAACAATAGCCGCTCTTCTCGTACAGCACGTGCACTGCGTCACCACAAATATCGTAAAACCTGTAAA
CGTTGCCGTGTGTCCGATGAGGATCTGAACAAATTCCTGACAAAAGCCAATGAGGACCAAACAAGCGTGAAAGTG
AAAGTCGTTAGCGCTCCTACCCGTACTAAAAAAGCAATGCCGAAATCCGTTGCTCGTGCCCCTAAACCACTGGAA
AACACTGAAGCAGCACAGGCACAGCCGTCTGGAAGCAAATTCTCTCCGGCCATTCCTGTTTCTACCCAGGAGTCC
GTTTCTGTTCCAGCAAGTGTGAGCACCAGCATTAGCAGTATTAGCACCGGTGCCACCGCTAGCGCCCTGGTTAAA
GGCAATACCAATCCGATTACAAGCATGTCTGCCCCGGTTCAAGCATCAGCTCCAGCACTGACAAAATCCCAAACC
GATCGTCTGGAGGTTCTGCTGAATCCGAAAGACGAAATCAGCCTGAATTCCGGCAAACCGTTTCGTGAACTGGAG
AGCGAACTGCTGTCACGTCGTAAAAAAGACCTGCAACAAATCTATGCCGAAGAACGTGAGAACTATCTGGGGAAA
CTGGAACGTGAAATCACCCGCTTTTTCGTGGATCGTGGCTTTCTGGAGATCAAATCCCCGATTCTGATTCCTCTG
GAGTATATCGAGCGTATGGGCATCGACAATGATACCGAACTGAGCAAACAAATTTTCCGTGTGGATAAAAACTTC
TGTCTGCGCCCTATGCTTGCACCAAATCTGATGAACTATGGACGCAAACTGGACCGTGCCCTGCCTGATCCTATC
AAAATCTTCGAGATCGGCCCGTGTTATCGTAAAGAGTCCGACGGTAAAGAACATCTGGAGGAGTTTACCATGCTG
AACTTTACACAAATGGGTTCAGGTTGTACTCGTGAGAACCTGGAAAGCATCATCACCGATTTTCTGAACCACCTG
GGCATTGACTTCAAAATTGTGGGCGACAGCTGTATGGTGTATGGCGACACCCTGGATGTCATGCACGGCGACCTG
GAACTGTCTAGTGCCGTTGTTGGACCAATTCCGCTGGACCGTGAGTGGGGTATCGACAAACCGTGGATCGGAGCA
GGATTCGGTCTGGAACGCCTGCTGAAAGTGAAACACGACTTCAAAAACATCAAACGTGCCGCCCGTTCTGAATCG
TATTATAACGGGATCTCTACGAACCTGTAA (SEQ ID NO: 435)

Protein:
MVGRNSAIAAGVCGALFIGYCIYFDRKRRSDPNFKNRLRERRKKQKLAKERAGLSKLPDLKDAEAVQKFFMASND
YTQQATQSYGAYPTQPGQGYSQQSSQPYGQQSYSGYSQSTDTSGYGQSSYSSYGQSQNTGYGTQSTPQGYGSTGG
YGSSQSSQSSYGQQSSYPGYGQQPAPSSTSGSYGSSSQSSSYGQPQSGSYSQQPSYGGQQQSYGQQQSYNPPQGY
GQQNQYNSSSGGGGGGGGGGNYGQDQSSMSSGGGSGGGYGNQDQSGGGGSGGYGQQDRGGRGRGGSGGGGGGGGG
GYNRSSGGYEPRGRGGGRGGRGGMGGSDRGGFNKFGGPRDQGSRHDSEQDNSDNNTIFVQGLGENVTIESVADYF
KQIGIIKTNKKTGQPMINLYTDRETGKLKGEATVSFDDPPSAKAAIDWFDGKEFSGNPIKVSFATRRADFNRGGG
NGRGGRGRGGPMGRGGYGGGGSGGGGRGGFPSGGGGGGGQQRAGDWKCPNPTCENMNFSWRNECNQCKAPKPDGP
GGGPGGSHMGGNYGDDRRGGRGGAIAGKPIPNPLLGLDSTGAPGSAGSAAGSGASIEQKLISEEDLIEGRHMLAS
KTIVLSVGEATRTLTEIQSTADRQIFEEKVGPLVGRLRLTASLRQNGAKTAYRVNLKLDQADVVDSGLPKVRYTQ
VWSHDVTIVANSTEASRKSLYDLTKSLVATSQVEDLVVNLVPLGRADPLASKTIVLSVGEATRTLTEIQSTADRQ
IFEEKVGPLVGRLRLTASLRQNGAKTAYRVNLKLDQADVVDSGLPKVRYTQVWSHDVTIVANSTEASRKSLYDLT

KSLVATSQVEDLVVNLVPLGRPPVATGAPGSAGSAAGSGACPVPLQLPPLERLTLDDKKPLNTLISATGLWMSRT
GTIHKIKHHEVSRSKIYIEMACGDHLVVNNSRSSRTARALRHHKYRKTCKRCRVSDEDLNKFLTKANEDQTSVKV
KVVSAPTRTKKAMPKSVARAPKPLENTEAAQAQPSGSKFSPAIPVSTQESVSVPASVSTSISSISTGATASALVK
GNTNPITSMSAPVQASAPALTKSQTDRLEVLLNPKDEISLNSGKPFRELESELLSRRKKDLQQIYAEERENYLGK
LEREITRFFVDRGFLEIKSPILIPLEYIERMGIDNDTELSKQIFRVDKNFCLRPMLAPNLMNYGRKLDRALPDPI
KIFEIGPCYRKESDGKEHLEEFTMLNFTQMGSGCTRENLESIITDFLNHLGIDFKIVGDSCMVYGDTLDVMHGDL
ELSSAVVGPIPLDREWGIDKPWIGAGFGLERLLKVKHDFKNIKRAARSESYYNGISTNL* (SEQ ID NO:
436)

TOM20::FUS::4xλN22::CbzRS
DNA:
ATGGTGGGTCGGAACAGCGCCATCGCCGCCGGTGTATGCGGGGCCCTTTTCATTGGGTACTGCATCTACTTCGAC
CGCAAAAGACGAAGTGACCCCAACTTCAAGAACAGGCTTCGAGAACGAAGAAAGAAACAGAAGCTTGCCAAGGAG
AGAGCTGGGCTTTCCAAGTTACCTGACCTTAAAGATGCTGAAGCTGTTCAGAAATTCTTCATGGCCTCAAACGAT
TATACCCAACAAGCAACCCAAAGCTATGGGGCCTACCCCACCCAGCCCGGGCAGGGCTATTCCCAGCAGAGCAGT
CAGCCCTACGGACAGCAGAGTTACAGTGGTTATAGCCAGTCCACGGACACTTCAGGATATGGCCAGAGCAGCTAT
TCTTCTTATGGCCAGAGCCAGAACACAGGCTATGGAACTCAGTCAACTCCCCAGGGATATGGCTCGACTGGCGGC
TATGGCAGTAGCCAGAGCTCCCAATCGTCTTACGGGCAGCAGTCCTCCTACCCTGGCTATGGCCAGCAGCCAGCT
CCCAGCAGCACCTCGGGAAGTTACGGTAGCAGTTCTCAGAGCAGCAGCTATGGGCAGCCCCAGAGTGGGAGCTAC
AGCCAGCAGCCTAGCTATGGTGGACAGCAGCAAAGCTATGGACAGCAGCAAAGCTATAATCCCCCTCAGGGCTAT
GGACAGCAGAACCAGTACAACAGCAGCAGTGGTGGTGGAGGTGGAGGTGGAGGTGGAGGTAACTATGGCCAAGAT
CAATCCTCCATGAGTAGTGGTGGTGGCAGTGGTGGCGGTTATGGCAATCAAGACCAGAGTGGTGGAGGTGGCAGC
GGTGGCTATGGACAGCAGGACCGTGGAGGCCGCGGCAGGGGTGGCAGTGGTGGCGGCGGCGGCGGCGGCGGTGGT
GGTTACAACCGCAGCAGTGGTGGCTATGAACCCAGAGGTCGTGGAGGTGGCCGTGGAGGCAGAGGTGGCATGGGC
GGAAGTGACCGTGGTGGCTTCAATAAATTTGGTGGCCCTCGGGACCAAGGATCACGTCATGACTCCGAACAGGAT
AATTCAGACAACAACACCATCTTTGTGCAAGGCCTGGGTGAGAATGTTACAATTGAGTCTGTGGCTGATTACTTC
AAGCAGATTGGTATTATTAAGACAAACAAGAAAACGGGACAGCCCATGATTAATTTGTACACAGACAGGGAAACT
GGCAAGCTGAAGGGAGAGGCAACGGTCTCTTTTGATGACCCACCTTCAGCTAAAGCAGCTATTGACTGGTTTGAT
GGTAAAGAATTCTCCGGAAATCCTATCAAGGTCTCATTTGCTACTCGCCGGGCAGACTTTAATCGGGGTGGTGGC
AATGGTCGTGGAGGCCGAGGGCGAGGAGGACCCATGGGCCGTGGAGGCTATGGAGGTGGTGGCAGTGGTGGTGGT
GGCCGAGGAGGATTTCCCAGTGGAGGTGGTGGCGGTGGAGGACAGCAGCGAGCTGGTGACTGGAAGTGTCCTAAT
CCCACCTGTGAGAATATGAACTTCTCTTGGAGGAATGAATGCAACCAGTGTAAGGCCCCTAAACCAGATGGCCCA
GGAGGGGGACCAGGTGGCTCTCACATGGGGGGTAACTACGGGGATGATCGTCGTGGTGGCAGAGGAGGCGCGATC
GCAGGCAAGCCTATTCCCAACCCCCTGCTGGGCCTGGATAGCACCGGAGCACCAGGAAGTGCTGGTTCTGCTGCT
GGTAGTGGAGCATCGATAGAGCAGAAGCTGATCTCAGAGGAGGACCTGCTAGCCACCATGGACGCACAAACACGA
CGACGTGAGCGTCGCGCTGAGAAACAAGCTCAATGGAAAGCTGCAAACCCACCGCTCGACGGAGCCGGAGCTGGC
GCTGGAGCTGGAGCCGGAGCTGGCGGTCTAGCCACCATGGACGCACAAACACGACGACGTGAGCGTCGCGCTGAG
AAACAAGCTCAATGGAAAGCTGCAAACCCACCGCTCGACGGAGCCGGAGCTGGCGCTGGAGCTGGAGCCGGAGCT
GGCGGTCTAGCCACCATGGACGCACAAACACGACGACGTGAGCGTCGCGCTGAGAAACAAGCTCAATGGAAAGCT
GCAAACCCACCGCTCGACGGAGCCGGAGCTGGCGCTGGAGCTGGAGCCGGAGCTGGCGGTCTAGCCACCATGGAC
GCACAAACACGACGACGTGAGCGTCGCGCTGAGAAACAAGCTCAATGGAAAGCTGCAAACCCACCGCTCGAGTCT
AGAGGGCCCGTTGGTGCTCCTGGTTCAGCAGGAAGCGCAGCAGGATCAGGTGCGTGCCCGGTGCCGCTGCAGCTG
CCGCCGCTGGAACGCCTGACCCTGGATGACAAAAAACCGCTGAATACCCTGATCTCTGCTACTGGTCTGTGGATG
AGTCGTACCGGAACCATTCATAAAATCAAACACCACGAGGTTAGCCGTTCGAAAATCTATATTGAGATGGCGTGT
GGCGATCATCTGGTTGTGAACAATAGCCGCTCTTCTCGTACAGCACGTGCACTGCGTCACCACAAATATCGTAAA
ACCTGTAAACGTTGCCGTGTGTCCGATGAGGATCTGAACAAATTCCTGACAAAAGCCAATGAGGACCAAACAAGC
GTGAAAGTGAAAGTCGTTAGCGCTCCTACCCGTACTAAAAAAGCAATGCCGAAATCCGTTGCTCGTGCCCCTAAA
CCACTGGAAAACACTGAAGCAGCACAGGCACAGCCGTCTGGAAGCAAATTCTCTCCGGCCATTCCTGTTTCTACC
CAGGAGTCCGTTTCTGTTCCAGCAAGTGTGAGCACCAGCATTAGCAGTATTAGCACCGGTGCCACCGCTAGCGCC
CTGGTTAAAGGCAATACCAATCCGATTACAAGCATGTCTGCCCCGGTTCAAGCATCAGCTCCAGCACTGACAAAA
TCCCAAACCGATCGTCTGGAGGTTCTGCTGAATCCGAAAGACGAAATCAGCCTGAATTCCGGCAAACCGTTTCGT
GAACTGGAGAGCGAACTGCTGTCACGTCGTAAAAAAGACCTGCAACAAATCTATGCCGAAGAACGTGAGAACTAT
CTGGGGAAACTGGAACGTGAAATCACCCGCTTTTTCGTGGATCGTGGCTTTCTGGAGATCAAATCCCCGATTCTG
ATTCCTCTGGAGTATATCGAGCGTATGGGCATCGACAATGATACCGAACTGAGCAAACAAATTTTCCGTGTGGAT
AAAAACTTCTGTCTGCGCCCTATGCTTGCACCAAATCTGATGAACTATGGACGCAAACTGGACCGTGCCCTGCCT
GATCCTATCAAAATCTTCGAGATCGGCCCGTGTTATCGTAAAGAGTCCGACGGTAAAGAACATCTGGAGGAGTTT
ACCATGCTGAACTTTACACAAATGGGTTCAGGTTGTACTCGTGAGAACCTGGAAAGCATCATCACCGATTTTCTG

AACCACCTGGGCATTGACTTCAAAATTGTGGGCGACAGCTGTATGGTGTATGGCGACACCCTGGATGTCATGCAC
GGCGACCTGGAACTGTCTAGTGCCGTTGTTGGACCAATTCCGCTGGACCGTGAGTGGGGTATCGACAAACCGTGG
ATCGGAGCAGGATTCGGTCTGGAACGCCTGCTGAAAGTGAAACACGACTTCAAAAACATCAAACGTGCCGCCCGT
TCTGAATCGTATTATAACGGGATCTCTACGAACCTGTAA (SEQ ID NO: 437)

Protein:
MVGRNSAIAAGVCGALFIGYCIYFDRKRRSDPNFKNRLRERRKKQKLAKERAGLSKLPDLKDAEAVQKFFMASND
YTQQATQSYGAYPTQPGQGYSQQSSQPYGQQSYSGYSQSTDTSGYGQSSYSSYGQSQNTGYGTQSTPQGYGSTGG
YGSSQSSQSSYGQQSSYPGYGQQPAPSSTSGSYGSSSQSSSYGQPQSGSYSQQPSYGGQQQSYGQQQSYNPPQGY
GQQNQYNSSSGGGGGGGGGGNYGQDQSSMSSGGGSGGGYGNQDQSGGGGSGGYGQQDRGGRGRGGSGGGGGGGGG
GYNRSSGGYEPRGRGGGRGGRGGMGGSDRGGFNKFGGPRDQGSRHDSEQDNSDNNTIFVQGLGENVTIESVADYF
KQIGIIKTNKKTGQPMINLYTDRETGKLKGEATVSFDDPPSAKAAIDWFDGKEFSGNPIKVSFATRRADFNRGGG
NGRGGRGRGGPMGRGGYGGGGSGGGGRGGFPSGGGGGGGQQRAGDWKCPNPTCENMNFSWRNECNQCKAPKPDGP
GGGPGGSHMGGNYGDDRRGGRGGAIAGKPIPNPLLGLDSTGAPGSAGSAAGSGASIEQKLISEEDLLATMDAQTR
RRERRAEKQAQWKAANPPLDGAGAGAGAGAGAGGLATMDAQTRRRERRAEKQAQWKAANPPLDGAGAGAGAGAGA
GGLATMDAQTRRRERRAEKQAQWKAANPPLDGAGAGAGAGAGAGGLATMDAQTRRRERRAEKQAQWKAANPPLES
RGPVGAPGSAGSAAGSGACPVPLQLPPLERLTLDDKKPLNTLISATGLWMSRTGTIHKIKHHEVSRSKIYIEMAC
GDHLVVNNSRSSRTARALRHHKYRKTCKRCRVSDEDLNKFLTKANEDQTSVKVKVVSAPTRTKKAMPKSVARAPK
PLENTEAAQAQPSGSKFSPAIPVSTQESVSVPASVSTSISSISTGATASALVKGNTNPITSMSAPVQASAPALTK
SQTDRLEVLLNPKDEISLNSGKPFRELESELLSRRKKDLQQIYAEERENYLGKLEREITRFFVDRGFLEIKSPIL
IPLEYIERMGIDNDTELSKQIFRVDKNFCLRPMLAPNLMNYGRKLDRALPDPIKIFEIGPCYRKESDGKEHLEEF
TMLNFTQMGSGCTRENLESIITDFLNHLGIDFKIVGDSCMVYGDTLDVMHGDLELSSAVVGPIPLDREWGIDKPW
IGAGFGLERLLKVKHDFKNIKRAARSESYYNGISTNL* (SEQ ID NO: 438)

EBAG9₁₋₂₉::FUS::PCP::PylRS(AF)
DNA:
ATGGCCATCACCCAGTTTCGGTTATTTAAATTTTGTACCTGCCTAGCAACAGTATTCTCATTCCTAAAGAGATTA
ATATGCAGATCTGGAGCACCCGGCTCCGCCGGCTCCGCCGGCTCCGGCATGGCCTCAAACGATTATACCCAA
CAAGCAACCCAAAGCTATGGGGCCTACCCCACCCAGCCCGGGCAGGGCTATTCCCAGCAGAGCAGTCAGCCCTAC
GGACAGCAGAGTTACAGTGGTTATAGCCAGTCCACGGACACTTCAGGATATGGCCAGAGCAGCTATTCTTCTTAT
GGCCAGAGCCAGAACACAGGCTATGGAACTCAGTCAACTCCCCAGGGATATGGCTCGACTGGCGGCTATGGCAGT
AGCCAGAGCTCCCAATCGTCTTACGGGCAGCAGTCCTCCTACCCTGGCTATGGCCAGCAGCCAGCTCCCAGCAGC
ACCTCGGGAAGTTACGGTAGCAGTTCTCAGAGCAGCAGCTATGGGCAGCCCCAGAGTGGGAGCTACAGCCAGCAG
CCTAGCTATGGTGGACAGCAGCAAAGCTATGGACAGCAGCAAAGCTATAATCCCCCTCAGGGCTATGGACAGCAG
AACCAGTACAACAGCAGCAGTGGTGGTGGAGGTGGAGGTGGAGGTGGAGGTAACTATGGCCAAGATCAATCCTCC
ATGAGTAGTGGTGGTGGCAGTGGTGGCGGTTATGGCAATCAAGACCAGAGTGGTGGAGGTGGCAGCGGTGGCTAT
GGACAGCAGGACCGTGGAGGCCGCGGCAGGGGTGGCAGTGGTGGCGGCGGCGGCGGCGGTGGTGGTTACAAC
CGCAGCAGTGGTGGCTATGAACCCAGAGGTCGTGGAGGTGGCCGTGGAGGCAGAGGTGGCATGGGCGGAAGTGAC
CGTGGTGGCTTCAATAAATTTGGTGGCCCTCGGGACCAAGGATCACGTCATGACTCCGAACAGGATAATTCAGAC
AACAACACCATCTTTGTGCAAGGCCTGGGTGAGAATGTTACAATTGAGTCTGTGGCTGATTACTTCAAGCAGATT
GGTATTATTAAGACAAACAAGAAAACGGGACAGCCCATGATTAATTTGTACACAGACAGGGAAACTGGCAAGCTG
AAGGGAGAGGCAACGGTCTCTTTTGATGACCCACCTTCAGCTAAAGCAGCTATTGACTGGTTTGATGGTAAAGAA
TTCTCCGGAAATCCTATCAAGGTCTCATTTGCTACTCGCCGGGCAGACTTTAATCGGGGTGGTGGCAATGGTCGT
GGAGGCCGAGGGCGAGGAGGACCCATGGGCCGTGGAGGCTATGGAGGTGGTGGCAGTGGTGGTGGTGGCCGAGGA
GGATTTCCCAGTGGAGGTGGTGGCGGTGGAGGACAGCAGCGAGCTGGTGACTGGAAGTGTCCTAATCCCACCTGT
GAGAATATGAACTTCTCTTGGAGGAATGAATGCAACCAGTGTAAGGCCCCTAAACCAGATGGCCCAGGAGGGGGA
CCAGGTGGCTCTCACATGGGGGGTAACTACGGGGATGATCGTCGTGGTGGCAGAGGTGGTGCGATCGCAGGCGCC
GATTACAAGGACGATGATGACAAGGGAGCACCAGGAAGTGCTGGTTCTGCTGCTGGTAGTGGAGCATCGATAGAG
CAGAAGCTGATCTCAGAGGAGGACCTGATCGAAGGCCGCCATATGCTAGCCTCCAAAACCATCGTTCTTTCGGTC
GGCGAGGCTACTCGCACTCTGACTGAGATCCAGTCCACCGCAGACCGTCAGATCTTCGAAGAGAAGGTCGGGCCT
CTGGTGGGTCGGCTGCGCCTCACGGCTTCGCTCCGTCAAAACGGAGCCAAGACCGCGTATCGCGTCAACCTAAAA
CTGGATCAGGCGGACGTCGTTGATTCCGGACTTCCGAAAGTGCGCTACACTCAGGTATGGTCGCACGACGTGACA
ATCGTTGCGAATAGCACCGAGGCCTCGCGCAAATCGTTGTACGATTTGACCAAGTCCCTCGTCGCGACCTCGCAG
GTCGAAGATCTTGTCGTCAACCTTGTGCCGCTGGGCCGTGCGGATCCGCTAGCCGGTGCTCCTGGTTCAGCAGGA
AGCGCAGCAGGATCAGGTGCGTGCCCGGTGCCGCTGCAGCTGCCGCCGCTGGAACGCCTGACCCTGGATGATAAA
AAACCGCTGAATACCCTGATCTCTGCTACTGGTCTGTGGATGAGTCGTACCGGAACCATTCATAAAATCAAACAC
CACGAGGTTAGCCGTTCGAAAATCTATATTGAGATGGCGTGTGGCGATCATCTGGTTGTGAACAATAGCCGCTCT

TCTCGTACAGCACGTGCACTGCGTCACCACAAATATCGTAAAACCTGTAAACGTTGCCGTGTGTCCGATGAGGAT
CTGAACAAATTCCTGACAAAAGCCAATGAGGACCAAACAAGCGTGAAAGTGAAAGTCGTTAGCGCTCCTACCCGT
ACTAAAAAAGCAATGCCGAAATCCGTTGCTCGTGCCCCTAAACCACTGGAAAACACTGAAGCAGCACAGGCACAG
CCGTCTGGAAGCAAATTCTCTCCGGCCATTCCTGTTTCTACCCAGGAGTCCGTTTCTGTTCCAGCAAGTGTGAGC
ACCAGCATTAGCAGTATTAGCACCGGTGCCACCGCTAGCGCCCTGGTTAAAGGCAATACCAATCCGATTACAAGC
ATGTCTGCCCCGGTTCAAGCATCAGCTCCAGCACTGACAAAATCCCAAACCGATCGTCTGGAGGTTCTGCTGAAT
CCGAAAGACGAAATCAGCCTGAATTCCGGCAAACCGTTTCGTGAACTGGAGAGCGAACTGCTGTCACGTCGTAAA
AAAGACCTGCAACAAATCTATGCCGAAGAACGTGAGAACTATCTGGGGAAACTGGAACGTGAAATCACCCGCTTT
TTCGTGGATCGTGGCTTTCTGGAGATCAAATCCCCGATTCTGATTCCTCTGGAGTATATCGAGCGTATGGGCATC
GACAATGATACCGAACTGAGCAAACAAATTTTCCGTGTGGATAAAAACTTCTGTCTGCGCCCTATGCTAGCACCA
AATCTGGCTAACTATCTGCGCAAACTGGACCGTGCCCTGCCTGATCCTATCAAAATCTTCGAGATCGGCCCGTGT
TATCGTAAAGAGTCCGACGGTAAAGAACATCTGGAGGAGTTTACCATGCTGAACTTTTGCCAAATGGGTTCAGGT
TGTACTCGTGAGAACCTGGAAAGCATCATCACCGATTTTCTGAACCACCTGGGCATTGACTTCAAAATTGTGGGC
GACAGCTGTATGGTGTTTGGCGACACCCTGGATGTCATGCACGGCGACCTGGAACTGTCTAGTGCCGTTGTGGGC
CCAATCCCGCTGGATCGTGAGTGGGGTATCGACAAACCTTGGATCGGTGCGGGTTTTGGTCTGGAGCGTCTGCTG
AAAGTAAAACACGACTTCAAGAACATCAAACGTGCTGCACGTTCCGAGTCCTATTACAATGGTATTTCTACTAAC
CTGTAA (SEQ ID NO: 439)

Protein:
MAITQFRLFKFCTCLATVFSFLKRLICRSGAPGSAGSAAGSGMASNDYTQQATQSYGAYPTQPGQGYSQQSSQPY
GQQSYSGYSQSTDTSGYGQSSYSSYGQSQNTGYGTQSTPQGYGSTGGYGSSQSSQSSYGQQSSYPGYGQQPAPSS
TSGSYGSSSQSSSYGQPQSGSYSQQPSYGGQQQSYGQQQSYNPPQGYGQQNQYNSSSGGGGGGGGGNYGQDQSS
MSSGGGSGGGYGNQDQSGGGGSGGYGQQDRGGRGRGGSGGGGGGGGGGYNRSSGGYEPRGRGGGRGGRGGMGGSD
RGGFNKFGGPRDQGSRHDSEQDNSDNNTIFVQGLGENVTIESVADYFKQIGIIKTNKKTGQPMINLYTDRETGKL
KGEATVSFDDPPSAKAAIDWFDGKEFSGNPIKVSFATRRADFNRGGGNGRGGRGRGGPMGRGGYGGGGSGGGGRG
GFPSGGGGGGGQQRAGDWKCPNPTCENMNFSWRNECNQCKAPKPDGPGGGPGGSHMGGNYGDDRRGGRGGAIAGA
DYKDDDDKGAPGSAGSAAGSGASIEQKLISEEDLIEGRHMLASKTIVLSVGEATRTLTEIQSTADRQIFEEKVGP
LVGRLRLTASLRQNGAKTAYRVNLKLDQADVVDSGLPKVRYTQVWSHDVTIVANSTEASRKSLYDLTKSLVATSQ
VEDLVVNLVPLGRADPLAGAPGSAGSAAGSGACPVPLQLPPLERLTLDDKKPLNTLISATGLWMSRTGTIHKIKH
HEVSRSKIYIEMACGDHLVVNNSRSSRTARALRHHKYRKTCKRCRVSDEDLNKFLTKANEDQTSVKVKVVSAPTR
TKKAMPKSVARAPKPLENTEAAQAQPSGSKFSPAIPVSTQESVSVPASVSTSISSISTGATASALVKGNTNPITS
MSAPVQASAPALTKSQTDRLEVLLNPKDEISLNSGKPFRELESELLSRRKKDLQQIYAEERENYLGKLEREITRF
FVDRGFLEIKSPILIPLEYIERMGIDNDTELSKQIFRVDKNFCLRPMLAPNLANYLRKLDRALPDPIKIFEIGPC
YRKESDGKEHLEEFTMLNFCQMGSGCTRENLESIITDFLNHLGIDFKIVGDSCMVFGDTLDVMHGDLELSSAVVG
PIPLDREWGIDKPWIGAGFGLERLLKVKHDFKNIKRAARSESYYNGISTNL* (SEQ ID NO: 440)

EBAG9₁₋₂₉::FUS::4xλ_{N22}::IFRS1
DNA:
ATGGCCATCACCCAGTTTCGGTTATTTAAATTTTGTACCTGCCTAGCAACAGTATTCTCATTCCTAAAGAGATTA
ATATGCAGATCTGGAGCACCCGGCTCCGCCGGCTCCGCCGCCGGCTCCGGCATGGCCTCAAACGATTATACCCAA
CAAGCAACCCAAAGCTATGGGGCCTACCCCACCCAGCCCGGGCAGGGCTATTCCCAGCAGAGCAGTCAGCCCTAC
GGACAGCAGAGTTACAGTGGTTATAGCCAGTCCACGGACACTTCAGGATATGGCCAGAGCAGCTATTCTTCTTAT
GGCCAGAGCCAGAACACAGGCTATGGAACTCAGTCAACTCCCCAGGGATATGGCTCGACTGGCGGCTATGGCAGT
AGCCAGAGCTCCCAATCGTCTTACGGGCAGCAGTCCTCCTACCCTGGCTATGGCCAGCAGCCAGCTCCCAGCAGC
ACCTCGGGAAGTTACGGTAGCAGTTCTCAGAGCAGCAGCTATGGGCAGCCCCAGAGTGGGAGCTACAGCCAGCAG
CCTAGCTATGGTGGACAGCAGCAAAGCTATGGACAGCAGCAAAGCTATAATCCCCCTCAGGGCTATGGACAGCAG
AACCAGTACAACAGCAGCAGTGGTGGTGGAGGTGGAGGTGGAGGTGGAGGTAACTATGGCCAAGATCAATCCTCC
ATGAGTAGTGGTGGTGGCAGTGGTGGCGGTTATGGCAATCAAGACCAGAGTGGTGGAGGTGGCAGCGGTGGCTAT
GGACAGCAGGACCGTGGAGGCCGCGGCAGGGGTGGCAGTGGTGGCGGCGGCGGCGGCGGCGGTGGTGGTTACAAC
CGCAGCAGTGGTGGCTATGAACCCAGAGGTCGTGGAGGTGGCCGTGGAGGCAGAGGTGGCATGGGCGGAAGTGAC
CGTGGTGGCTTCAATAAATTTGGTGGCCCTCGGGACCAAGGATCACGTCATGACTCCGAACAGGATAATTCAGAC
AACAACACCATCTTTGTGCAAGGCCTGGGTGAGAATGTTACAATTGAGTCTGTGGCTGATTACTTCAAGCAGATT
GGTATTATTAAGACAAACAAGAAAACGGGACAGCCCATGATTAATTTGTACACAGACAGGGAAACTGGCAAGCTG
AAGGGAGAGGCAACGGTCTCTTTTGATGACCCACCTTCAGCTAAAGCAGCTATTGACTGGTTTGATGGTAAAGAA
TTCTCCGGAAATCCTATCAAGGTCTCATTTGCTACTCGCCGGGCAGACTTTAATCGGGGTGGTGGCAATGGTCGT
GGAGGCCGAGGGCGAGGAGGACCCATGGGCCGTGGAGGCTATGGAGGTGGTGGCAGTGGTGGTGGTGGCCGAGGA
GGATTTCCCAGTGGAGGTGGTGGCGGTGGAGGACAGCAGCGAGCTGGTGACTGGAAGTGTCCTAATCCCACCTGT

GAGAATATGAACTTCTCTTGGAGGAATGAATGCAACCAGTGTAAGGCCCCTAAACCAGATGGCCCAGGAGGGGGA
CCAGGTGGCTCTCACATGGGGGGTAACTACGGGGATGATCGTCGTGGTGGCAGAGGTGGTGCGATCGCAGGCGCC
GATTACAAGGACGATGATGACAAGGGAGCACCAGGAAGTGCTGGTTCTGCTGCTGGTAGTGGAGCATCGATAGAG
CAGAAGCTGATCTCAGAGGAGGACCTGCTAGCCACCATGGACGCACAAACACGACGACGTGAGCGTCGCGCTGAG
AAACAAGCTCAATGGAAAGCTGCAAACCCACCGCTCGACGGAGCCGGAGCTGGCGCTGGAGCTGGAGCCGGAGCT
GGCGGTCTAGCCACCATGGACGCACAAACACGACGACGTGAGCGTCGCGCTGAGAAACAAGCTCAATGGAAAGCT
GCAAACCCACCGCTCGACGGAGCCGGAGCTGGCGCTGGAGCTGGAGCCGGAGCTGGCGGTCTAGCCACCATGGAC
GCACAAACACGACGACGTGAGCGTCGCGCTGAGAAACAAGCTCAATGGAAAGCTGCAAACCCACCGCTCGACGGA
GCCGGAGCTGGCGCTGGAGCTGGAGCCGGAGCTGGCGGTCTAGCCACCATGGACGCACAAACACGACGACGTGAG
CGTCGCGCTGAGAAACAAGCTCAATGGAAAGCTGCAAACCCACCGCTCGAGTCTAGAGGGCCCGTTGGTGCTCCT
GGTTCAGCAGGAAGCGCAGCAGGATCAGGTGCGTGCCCGGTGCCGCTGCAGCTGCCGCCGCTGGAACGCCTGACC
CTGGATGACAAAAAACCGCTGAATACCCTGATCTCTGCTACTGGTCTGTGGATGAGTCGTACCGGAACCATTCAT
AAAATCAAACACCACGAGGTTAGCCGTTCGAAAATCTATATTGAGATGGCGTGTGGCGATCATCTGGTTGTGAAC
AATAGCCGCTCTTCTCGTACAGCACGTGCACTGCGTCACCACAAATATCGTAAAACCTGTAAACGTTGCCGTGTG
TCCGATGAGGATCTGAACAAATTCCTGACAAAAGCCAATGAGGACCAAACAAGCGTGAAAGTGAAAGTCGTTAGC
GCTCCTACCCGTACTAAAAAAGCAATGCCGAAATCCGTTGCTCGTGCCCCTAAACCACTGGAAAACACTGAAGCA
GCACAGGCACAGCCGTCTGGAAGCAAATTCTCTCCGGCCATTCCTGTTTCTACCCAGGAGTCCGTTTCTGTTCCA
GCAAGTGTGAGCACCAGCATTAGCAGTATTAGCACCGGTGCCACCGCTAGCGCCCTGGTTAAAGGCAATACCAAT
CCGATTACAAGCATGTCTGCCCCGGTTCAAGCATCAGCTCCAGCACTGACAAAATCCCAAACCGATCGTCTGGAG
GTTCTGCTGAATCCGAAAGACGAAATCAGCCTGAATTCCGGCAAACCGTTTCGTGAACTGGAGAGCGAACTGCTG
TCACGTCGTAAAAAAGACCTGCAACAAATCTATGCCGAAGAACGTGAGAACTATCTGGGGAAACTGGAACGTGAA
ATCACCCGCTTTTTCGTGGATCGTGGCTTTCTGGAGATCAAATCCCCGATTCTGATTCCTCTGGAGTATATCGAG
CGTATGGGCATCGACAATGATACCGAACTGAGCAAACAAATTTTCCGTGTGGATAAAAACTTCTGTCTGCGCCCT
ATGCTTGCACCAAATATGCTGAACTATAGCCGCAAACTGGACCGTGCCCTGCCTGATCCTATCAAAATCTTCGAG
ATCGGCCCGTGTTATCGTAAAGAGTCCGACGGTAAAGAACATCTGGAGGAGTTTACCATGCTGTCTTTTATGCAA
ATGGGTTCAGGTTGTACTCGTGAGAACCTGGAAAGCATCATCACCGATTTTCTGAACCACCTGGGCATTGACTTC
AAAATTGTGGGCGACAGCTGTATGGTGTATGGCGACACCCTGGATGTCATGCACGGCGACCTGGAACTGTCTAGT
GCCGTTGTTGGACCAATTCCGCTGGACCGTGAGTGGGGTATCGACAAACCGTGGATCGGAGCAGGATTCGGTCTG
GAACGCCTGCTGAAAGTGAAACACGACTTCAAAAACATCAAACGTGCCGCCCGTTCTGAATCGTATTATAACGGG
ATCTCTACGAACCTGTAA (SEQ ID NO: 441)

Protein:
MAITQFRLFKFCTCLATVFSFLKRLICRSGAPGSAGSAAGSGMASNDYTQQATQSYGAYPTQPGQGYSQQSSQPY
GQQSYSGYSQSTDTSGYGQSSYSSYGQSQNTGYGTQSTPQGYGSTGGYGSSQSSQSSYGQQSSYPGYGQQPAPSS
TSGSYGSSSQSSSYGQPQSGSYSQQPSYGGQQQSYGQQQSYNPPQGYGQQNQYNSSSGGGGGGGGGGNYGQDQSS
MSSGGGSGGGYGNQDQSGGGGSGGYGQQDRGGRGRGGSGGGGGGGGGGYNRSSGGYEPRGRGGGRGGRGGMGGSD
RGGFNKFGGPRDQGSRHDSEQDNSDNNTIFVQGLGENVTIESVADYFKQIGIIKTNKKTGQPMINLYTDRETGKL
KGEATVSFDDPPSAKAAIDWFDGKEFSGNPIKVSFATRRADFNRGGGNGRGGRGRGGPMGRGGYGGGGSGGGGRG
GFPSGGGGGGGQQRAGDWKCPNPTCENMNFSWRNECNQCKAPKPDGPGGGPGGSHMGGNYGDDRRGGRGGAIAGA
DYKDDDDKGAPGSAGSAAGSGASIEQKLISEEDLLATMDAQTRRRERRAEKQAQWKAANPPLDGAGAGAGAGAGA
GGLATMDAQTRRRERRAEKQAQWKAANPPLDGAGAGAGAGAGAGGLATMDAQTRRRERRAEKQAQWKAANPPLDG
AGAGAGAGAGAGGLATMDAQTRRRERRAEKQAQWKAANPPLESRGPVGAPGSAGSAAGSGACPVPLQLPPLERLT
LDDKKPLNTLISATGLWMSRTGTIHKIKHHEVSRSKIYIEMACGDHLVVNNSRSSRTARALRHHKYRKTCKRCRV
SDEDLNKFLTKANEDQTSVKVKVVSAPTRTKKAMPKSVARAPKPLENTEAAQAQPSGSKFSPAIPVSTQESVSVP
ASVSTSISSISTGATASALVKGNTNPITSMSAPVQASAPALTKSQTDRLEVLLNPKDEISLNSGKPFRELESELL
SRRKKDLQQIYAEERENYLGKLEREITRFFVDRGFLEIKSPILIPLEYIERMGIDNDTELSKQIFRVDKNFCLRP
MLAPNMLNYSRKLDRALPDPIKIFEIGPCYRKESDGKEHLEEFTMLSFMQMGSGCTRENLESIITDFLNHLGIDF
KIVGDSCMVYGDTLDVMHGDLELSSAVVGPIPLDREWGIDKPWIGAGFGLERLLKVKHDFKNIKRAARSESYYNG
ISTNL* (SEQ ID NO: 442)

KIF16B::EWSR1::Myc::2xPCP
DNA:
ATGGCATCGGTCAAGGTGGCCGTGAGGGTCCGGCCCATGAATCGCAGGGAAAAGGACTTGGAGGCCAAGTTCATT
ATTCAGATGGAGAAAAGCAAAACGACAATCACAAACTTAAAGATACCAGAAGGAGGCACTGGGGACTCAGGAAGA
GAACGGACCAAGACCTTCACCTATGACTTTTCTTTTTATTCTGCTGATACAAAAAGCCCAGATTACGTTTCACAA
GAAATGGTTTTCAAAACCCTCGGCACAGATGTCGTGAAGTCTGCATTTGAAGGTTATAATGCTTGTGTCTTTGCA
TATGGGCAAACTGGATCTGGAAAGTCATACACTATGATGGGAAATTCTGGAGATTCTGGCTTAATACCTCGGATC

TGTGAAGGACTCTTCAGTCGGATAAATGAAACCACCAGATGGGATGAAGCTTCTTTTCGAACTGAAGTCAGCTAC
TTAGAAATTTATAACGAACGTGTGAGAGATCTACTTCGGCGGAAGTCATCTAAAACCTTCAATTTGAGAGTCCGT
GAGCATCCCAAAGAAGGCCCTTATGTTGAGGATTTATCCAAACATTTAGTACAGAATTATGGTGACGTAGAAGAA
CTTATGGATGCGGGCAATATCAACCGGACCACCGCAGCGACTGGGATGAACGACGTCAGTAGCAGGTCTCATGCC
ATCTTCACCATCAAGTTCACTCAGGCTAAATTTGATTCTGAAATGCCATGTGAAACCGTCAGTAAGATCCACTTG
GTTGATCTTGCCGGAAGTGAGCGTGCAGATGCCACCGGAGCCACCGGGGTTAGGCTAAAGGAAGGGGGAAATATT
AACAAGTCCCTCGTGACTCTGGGGAACGTCATTTCTGCCTTAGCTGATTTATCTCAGGATGCTGCAAATACTCTT
GCAAAGAAGAAGCAAGTTTTCGTGCCTTACAGGGATTCTGTGTTGACTTGGTTGTTAAAAGATAGCCTTGGAGGA
AACTCTAAAACTATCATGATTGCCACCATTTCACCTGCTGATGTCAATTATGGAGAAACCCTAAGTACTCTTCGC
TATGCAAATAGAGCCAAAAACATCATCAACAAGCCTACCATTAATGAGGATGCCAACGTCAAACTTATCCGTGAG
CTGCGAGCTGAAATAGCCAGACTGAAAACGCTGCTTGCTCAAGGGAATCAGATTGCCCTCTTAGACTCCCCCACA
ATGGCGTCCACGGATTACAGTACCTATAGCCAAGCTGCAGCGCAGCAGGGCTACAGTGCTTACACCGCCCAGCCC
ACTCAAGGATATGCACAGACCACCCAGGCATATGGGCAACAAAGCTATGGAACCTATGGACAGCCCACTGATGTC
AGCTATACCCAGGCTCAGACCACTGCAACCTATGGGCAGACCGCCTATGCAACTTCTTATGGACAGCCTCCCACT
GGTTATACTACTCCAACTGCCCCCCAGGCATACAGCCAGCCTGTCCAGGGGTATGGCACTGGTGCTTATGATACC
ACCACTGCTACAGTCACCACCACCCAGGCCTCCTATGCAGCTCAGTCTGCATATGGCACTCAGCCTGCTTATCCA
GCCTATGGGCAGCAGCCAGCAGCCACTGCACCTACAAGACCGCAGGATGGAAACAAGCCCACTGAGACTAGTCAA
CCTCAATCTAGCACAGGGGGTTACAACCAGCCCAGCCTAGGATATGGACAGAGTAACTACAGTTATCCCCAGGTA
CCTGGGAGCTACCCCATGCAGCCAGTCACTGCACCTCCATCCTACCCTCCTACCAGCTATTCCTCTACACAGCCG
ACTAGTTATGATCAGAGCAGTTACTCTCAGCAGAACACCTATGGGCAACCGAGCAGCTATGGACAGCAGAGTAGC
TATGGTCAACAAAGCAGCTATGGGCAGCAGCCTCCCACTAGTTACCCACCCCAAACTGGATCCTACAGCCAAGCT
CCAAGTCAATATAGCCAACAGAGCAGCAGCTACGGGCAGCAGAGTTCATTCCGACAGGACCACCCCAGTAGCATG
GGTGTTTATGGGCAGGAGTCTGGAGGATTTTCCGGACCAGGAGAGAACCGGAGCATGAGTGGCCCTGATAACCGG
GGCAGGGGAAGAGGGGGATTTGATCGTGGAGGCATGAGCAGAGGTGGGCGGGGAGGAGGACGCGGTGGAATGGGC
AGCGCTGGAGAGCGAGGTGGCTTCAATAAGCCTGGTGGACCCATGGATGAAGGACCAGATCTTGATCTAGGCCCA
CCTGTAGATCCAGATGAAGACTCTGACAACAGTGCAATTTATGTACAAGGATTAAATGACAGTGTGACTCTAGAT
GATCTGGCAGACTTCTTTAAGCAGTGTGGGGTTGTTAAGATGAACAAGAGAACTGGGCAACCCATGATCCACATC
TACCTGGACAAGGAAACAGGAAAGCCCAAAGGCGATGCCACAGTGTCCTATGAAGACCCACCTACTGCCAAGGCT
GCCGTGGAATGGTTTGATGGGAAAGATTTTCAAGGGAGCAAACTTAAAGTCTCCCTTGCTCGGAAGAAGCCTCCA
ATGAACAGTATGCGGGGTGGTCTGCCACCCCGTGAGGGCAGAGGCATGCCACCACCACTCCGTGGAGGTCCAGGA
GGCCCAGGAGGTCCTGGGGGACCCATGGGTCGCATGGGAGGCCGTGGAGGAGATAGAGGAGGCTTCCCTCCAAGA
GGACCCCGGGGTTCCCGAGGGAACCCCTCTGGAGGAGGAAACGTCCAGCACCGAGCTGGAGACTGGCAGTGTCCC
AATCCGGGTTGTGGAAACCAGAACTTCGCCTGGAGAACAGAGTGCAACCAGTGTAAGGCCCCAAAGCCTGAAGGC
TTCCTCCCGCCACCCTTTCCGCCCCCGGGTGGTGATCGTGGCAGAGGTGGCCCTGGTGGCATGCGGGGAGGAAGA
GGTGGCCTCATGGATCGTGGTGGTCCCGGTGGAATGTTCAGAGGTGGCCGTGGTGGAGACAGAGGTGGCTTCCGT
GGTGGCCGGGGCATGGACCGAGGTGGCTTTGGTGGAGGAAGACGAGGTGGCCCTGGGGGGCCCCCTGGACCTTTG
ATGGAACAGGCGATCGCAGAGCAGAAGCTGATCTCAGAGGAGGACCTGATCGAAGGCCGCCATATGCTAGCCTCC
AAAACCATCGTTCTTTCGGTCGGCGAGGCTACTCGCACTCTGACTGAGATCCAGTCCACCGCAGACCGTCAGATC
TTCGAAGAGAAGGTCGGGCCTCTGGTGGGTCGGCTGCGCCTCACGGCTTCGCTCCGTCAAAACGGAGCCAAGACC
GCGTATCGCGTCAACCTAAAACTGGATCAGGCGGACGTCGTTGATTCCGGACTTCCGAAAGTGCGCTACACTCAG
GTATGGTCGCACGACGTGACAATCGTTGCGAATAGCACCGAGGCCTCGCGCAAATCGTTGTACGATTTGACCAAG
TCCCTCGTCGCGACCTCGCAGGTCGAAGATCTTGTCGTCAACCTTGTGCCGCTGGGCCGTGCGGATCCGCTAGCC
TCCAAAACCATCGTTCTTTCGGTCGGCGAGGCTACTCGCACTCTGACTGAGATCCAGTCCACCGCAGACCGTCAG
ATCTTCGAAGAGAAGGTCGGGCCTCTGGTGGGTCGGCTGCGCCTCACGGCTTCGCTCCGTCAAAACGGAGCCAAG
ACCGCGTATCGCGTCAACCTAAAACTGGATCAGGCGGACGTCGTTGATTCCGGACTTCCGAAAGTGCGCTACACT
CAGGTATGGTCGCACGACGTGACAATCGTTGCGAATAGCACCGAGGCCTCGCGCAAATCGTTGTACGATTTGACC
AAGTCCCTCGTCGCGACCTCGCAGGTCGAAGATCTTGTCGTCAACCTTGTGCCGCTGGGCCGTCCACCGGTCGCC
ACCTAA (SEQ ID NO: 443)

Protein:
MASVKVAVRVRPMNRREKDLEAKFIIQMEKSKTTITNLKIPEGGTGDSGRERTKTFTYDFSFYSADTKSPDYVSQ
EMVFKTLGTDVVKSAFEGYNACVFAYGQTGSGKSYTMMGNSGDSGLIPRICEGLFSRINETTRWDEASFRTEVSY
LEIYNERVRDLLRRKSSKTFNLRVREHPKEGPYVEDLSKHLVQNYGDVEELMDAGNINRTTAATGMNDVSSRSHA
IFTIKFTQAKFDSEMPCETVSKIHLVDLAGSERADATGATGVRLKEGGNINKSLVTLGNVISALADLSQDAANTL
AKKKQVFVPYRDSVLTWLLKDSLGGNSKTIMIATISPADVNYGETLSTLRYANRAKNIINKPTINEDANVKLIRE
LRAEIARLKTLLAQGNQIALLDSPTMASTDYSTYSQAAAQQGYSAYTAQPTQGYAQTTQAYGQQSYGTYGQPTDV
SYTQAQTTATYGQTAYATSYGQPPTGYTTPTAPQAYSQPVQGYGTGAYDTTTATVTTTQASYAAQSAYGTQPAYP
AYGQQPAATAPTRPQDGNKPTETSQPQSSTGGYNQPSLGYGQSNYSYPQVPGSYPMQPVTAPPSYPPTSYSSTQP

TSYDQSSYSQQNTYGQPSSYGQQSSYGQQSSYGQQPPTSYPPQTGSYSQAPSQYSQQSSSYGQQSSFRQDHPSSM
GVYGQESGGFSGPGENRSMSGPDNRGRGGFDRGGMSRGGRGGGRGGMGSAGERGGFNKPGGPMDEGPDLDLGP
PVDPDEDSDNSAIYVQGLNDSVTLDDLADFFKQCGVVKMNKRTGQPMIHIYLDKETGKPKGDATVSYEDPPTAKA
AVEWFDGKDFQGSKLKVSLARKKPPMNSMRGGLPPREGRGMPPPLRGGPGGPGGPGGPMGRMGGRGGDRGGFPPR
GPRGSRGNPSGGGNVQHRAGDWQCPNPGCGNQNFAWRTECNQCKAPKPEGFLPPPFPPPGGDRGRGGPGGMRGGR
GGLMDRGGPGGMFRGGRGGDRGGFRGGRGMDRGGFGGGRRGGPGGPPGPLMEQAIAEQKLISEEDLIEGRHMLAS
KTIVLSVGEATRTLTEIQSTADRQIFEEKVGPLVGRLRLTASLRQNGAKTAYRVNLKLDQADVVDSGLPKVRYTQ
VWSHDVTIVANSTEASRKSLYDLTKSLVATSQVEDLVVNLVPLGRADPLASKTIVLSVGEATRTLTEIQSTADRQ
IFEEKVGPLVGRLRLTASLRQNGAKTAYRVNLKLDQADVVDSGLPKVRYTQVWSHDVTIVANSTEASRKSLYDLT
KSLVATSQVEDLVVNLVPLGRPPVAT* (SEQ ID NO: 444)

KIF16B::EWSR1::HA::2xPCP
DNA:
ATGGCATCGGTCAAGGTGGCCGTGAGGGTCCGGCCCATGAATCGCAGGGAAAAGGACTTGGAGGCCAAGTTCATT
ATTCAGATGGAGAAAAGCAAAACGACAATCACAAACTTAAAGATACCAGAAGGAGGCACTGGGGACTCAGGAAGA
GAACGGACCAAGACCTTCACCTATGACTTTTCTTTTTATTCTGCTGATACAAAAAGCCCAGATTACGTTTCACAA
GAAATGGTTTTCAAAACCCTCGGCACAGATGTCGTGAAGTCTGCATTTGAAGGTTATAATGCTTGTGTCTTTGCA
TATGGGCAAACTGGATCTGGAAAGTCATACACTATGATGGGAAATTCTGGAGATTCTGGCTTAATACCTCGGATC
TGTGAAGGACTCTTCAGTCGGATAAATGAAACCACCAGATGGGATGAAGCTTCTTTTCGAACTGAAGTCAGCTAC
TTAGAAATTTATAACGAACGTGTGAGAGATCTACTTCGGCGGAAGTCATCTAAAACCTTCAATTTGAGAGTCCGT
GAGCATCCCAAAGAAGGCCCTTATGTTGAGGATTTATCCAAACATTTAGTACAGAATTATGGTGACGTAGAAGAA
CTTATGGATGCGGGCAATATCAACCGGACCACCGCAGCGACTGGGATGAACGACGTCAGTAGCAGGTCTCATGCC
ATCTTCACCATCAAGTTCACTCAGGCTAAATTTGATTCTGAAATGCCATGTGAAACCGTCAGTAAGATCCACTTG
GTTGATCTTGCCGGAAGTGAGCGTGCAGATGCCACCGGAGCCACCGGGGTTAGGCTAAAGGAAGGGGGAAATATT
AACAAGTCCCTCGTGACTCTGGGGAACGTCATTTCTGCCTTAGCTGATTTATCTCAGGATGCTGCAAATACTCTT
GCAAAGAAGAAGCAAGTTTTCGTGCCTTACAGGGATTCTGTGTTGACTTGGTTGTTAAAAGATAGCCTTGGAGGA
AACTCTAAAACTATCATGATTGCCACCATTTCACCTGCTGATGTCAATTATGGAGAAACCCTAAGTACTCTTCGC
TATGCAAATAGAGCCAAAAACATCATCAACAAGCCTACCATTAATGAGGATGCCAACGTCAAACTTATCCGTGAG
CTGCGAGCTGAAATAGCCAGACTGAAAACGCTGCTTGCTCAAGGGAATCAGATTGCCCTCTTAGACTCCCCCACA
ATGGCGTCCACGGATTACAGTACCTATAGCCAAGCTGCAGCGCAGCAGGGCTACAGTGCTTACACCGCCCAGCCC
ACTCAAGGATATGCACAGACCACCCAGGCATATGGGCAACAAAGCTATGGAACCTATGGACAGCCCACTGATGTC
AGCTATACCCAGGCTCAGACCACTGCAACCTATGGGCAGACCGCCTATGCAACTTCTTATGGACAGCCTCCCACT
GGTTATACTACTCCAACTGCCCCCCAGGCATACAGCCAGCCTGTCCAGGGGTATGGCACTGGTGCTTATGATACC
ACCACTGCTACAGTCACCACCACCCAGGCCTCCTATGCAGCTCAGTCTGCATATGGCACTCAGCCTGCTTATCCA
GCCTATGGGCAGCAGCCAGCAGCCACTGCACCTACAAGACCGCAGGATGGAAACAAGCCCACTGAGACTAGTCAA
CCTCAATCTAGCACAGGGGGTTACAACCAGCCCAGCCTAGGATATGGACAGAGTAACTACAGTTATCCCCAGGTA
CCTGGGAGCTACCCCATGCAGCCAGTCACTGCACCTCCATCCTACCCTCCTACCAGCTATTCCTCTACACAGCCG
ACTAGTTATGATCAGAGCAGTTACTCTCAGCAGAACACCTATGGGCAACCGAGCAGCTATGGACAGCAGAGTAGC
TATGGTCAACAAAGCAGCTATGGGCAGCAGCCTCCCACTAGTTACCCACCCCAAACTGGATCCTACAGCCAAGCT
CCAAGTCAATATAGCCAACAGAGCAGCAGCTACGGGCAGCAGAGTTCATTCCGACAGGACCACCCCAGTAGCATG
GGTGTTTATGGGCAGGAGTCTGGAGGATTTTCCGGACCAGGAGAGAACCGGAGCATGAGTGGCCCTGATAACCGG
GGCAGGGGAAGAGGGGGATTTGATCGTGGAGGCATGAGCAGAGGTGGGCGGGGAGGAGGACGCGGTGGAATGGGC
AGCGCTGGAGAGCGAGGTGGCTTCAATAAGCCTGGTGGACCCATGGATGAAGGACCAGATCTTGATCTAGGCCCA
CCTGTAGATCCAGATGAAGACTCTGACAACAGTGCAATTTATGTACAAGGATTAAATGACAGTGTGACTCTAGAT
GATCTGGCAGACTTCTTTAAGCAGTGTGGGGTTGTTAAGATGAACAAGAGAACTGGGCAACCCATGATCCACATC
TACCTGGACAAGGAAACAGGAAAGCCCAAAGGCGATGCCACAGTGTCCTATGAAGACCCACCTACTGCCAAGGCT
GCCGTGGAATGGTTTGATGGGAAAGATTTTCAAGGGAGCAAACTTAAAGTCTCCCTTGCTCGGAAGAAGCCTCCA
ATGAACAGTATGCGGGGTGGTCTGCCACCCCGTGAGGGCAGAGGCATGCCACCACCACTCCGTGGAGGTCCAGGA
GGCCCAGGAGGTCCTGGGGGACCCATGGGTCGCATGGGAGGCCGTGGAGGAGATAGAGGAGGCTTCCCTCCAAGA
GGACCCCGGGGTTCCCGAGGGAACCCCTCTGGAGGAGGAAACGTCCAGCACCGAGCTGGAGACTGGCAGTGTCCC
AATCCGGGTTGTGGAAACCAGAACTTCGCCTGGAGAACAGAGTGCAACCAGTGTAAGGCCCCAAAGCCTGAAGGC
TTCCTCCCGCCACCCTTTCCGCCCCCGGGTGGTGATCGTGGCAGAGGTGGCCCTGGTGGCATGCGGGGAGGAAGA
GGTGGCCTCATGGATCGTGGTGGTCCCGGTGGAATGTTCAGAGGTGGCCGTGGTGGAGACAGAGGTGGCTTCCGT
GGTGGCCGGGGCATGGACCGAGGTGGCTTTGGTGGAGGAAGACGAGGTGGCCCTGGGGGGCCCCCTGGACCTTTG
ATGGAACAGGCGATCGCATACCCCTACGACGTGCCCGACTACGCCATCGAAGGCCGCCATATGCTAGCCTCCAAA
ACCATCGTTCTTTCGGTCGGCGAGGCTACTCGCACTCTGACTGAGATCCAGTCCACCGCAGACCGTCAGATCTTC
GAAGAGAAGGTCGGGCCTCTGGTGGGTCGGCTGCGCCTCACGGCTTCGCTCCGTCAAAACGGAGCCAAGACCGCG

TATCGCGTCAACCTAAAACTGGATCAGGCGGACGTCGTTGATTCCGGACTTCCGAAAGTGCGCTACACTCAGGTA
TGGTCGCACGACGTGACAATCGTTGCGAATAGCACCGAGGCCTCGCGCAAATCGTTGTACGATTTGACCAAGTCC
CTCGTCGCGACCTCGCAGGTCGAAGATCTTGTCGTCAACCTTGTGCCGCTGGGCCGTGCGGATCCGCTAGCCTCC
AAAACCATCGTTCTTTCGGTCGGCGAGGCTACTCGCACTCTGACTGAGATCCAGTCCACCGCAGACCGTCAGATC
TTCGAAGAGAAGGTCGGGCCTCTGGTGGGTCGGCTGCGCCTCACGGCTTCGCTCCGTCAAAACGGAGCCAAGACC
GCGTATCGCGTCAACCTAAAACTGGATCAGGCGGACGTCGTTGATTCCGGACTTCCGAAAGTGCGCTACACTCAG
GTATGGTCGCACGACGTGACAATCGTTGCGAATAGCACCGAGGCCTCGCGCAAATCGTTGTACGATTTGACCAAG
TCCCTCGTCGCGACCTCGCAGGTCGAAGATCTTGTCGTCAACCTTGTGCCGCTGGGCCGTCCACCGGTCGCCACC
TAA (SEQ ID NO: 445)

Protein:
MASVKVAVRVRPMNRREKDLEAKFIIQMEKSKTTITNLKIPEGGTGDSGRERTKTFTYDFSFYSADTKSPDYVSQ
EMVFKTLGTDVVKSAFEGYNACVFAYGQTGSGKSYTMMGNSGDSGLIPRICEGLFSRINETTRWDEASFRTEVSY
LEIYNERVRDLLRRKSSKTFNLRVREHPKEGPYVEDLSKHLVQNYGDVEELMDAGNINRTTAATGMNDVSSRSHA
IFTIKFTQAKFDSEMPCETVSKIHLVDLAGSERADATGATGVRLKEGGNINKSLVTLGNVISALADLSQDAANTL
AKKKQVFVPYRDSVLTWLLKDSLGGNSKTIMIATISPADVNYGETLSTLRYANRAKNIINKPTINEDANVKLIRE
LRAEIARLKTLLAQGNQIALLDSPTMASTDYSTYSQAAAQQGYSAYTAQPTQGYAQTTQAYGQQSYGTYGQPTDV
SYTQAQTTATYGQTAYATSYGQPPTGYTTPTAPQAYSQPVQGYGTGAYDTTTATVTTTQASYAAQSAYGTQPAYP
AYGQQPAATAPTRPQDGNKPTETSQPQSSTGGYNQPSLGYGQSNYSYPQVPGSYPMQPVTAPPSYPPTSYSSTQP
TSYDQSSYSQQNTYGQPSSYGQQSSYGQQSSYGQQPPTSYPPQTGSYSQAPSQYSQQSSSYGQQSSFRQDHPSSM
GVYGQESGGFSGPGENRSMSGPDNRGRGRGGFDRGGMSRGGRGGGRGGMGSAGERGGFNKPGGPMDEGPDLDLGP
PVDPDEDSDNSAIYVQGLNDSVTLDDLADFFKQCGVVKMNKRTGQPMIHIYLDKETGKPKGDATVSYEDPPTAKA
AVEWFDGKDFQGSKLKVSLARKKPPMNSMRGGLPPREGRGMPPPLRGGPGGPGGPGGPMGRMGGRGGDRGGFPPR
GPRGSRGNPSGGGNVQHRAGDWQCPNPGCGNQNFAWRTECNQCKAPKPEGFLPPPFPPPGGDRGRGGPGGMRGGR
GGLMDRGGPGGMFRGGRGGDRGGFRGGRGMDRGGFGGGRRGGPGGPPGPLMEQAIAYPYDVPDYAIEGRHMLASK
TIVLSVGEATRTLTEIQSTADRQIFEEKVGPLVGRLRLTASLRQNGAKTAYRVNLKLDQADVVDSGLPKVRYTQV
WSHDVTIVANSTEASRKSLYDLTKSLVATSQVEDLVVNLVPLGRADPLASKTIVLSVGEATRTLTEIQSTADRQI
FEEKVGPLVGRLRLTASLRQNGAKTAYRVNLKLDQADVVDSGLPKVRYTQVWSHDVTIVANSTEASRKSLYDLTK
SLVATSQVEDLVVNLVPLGRPPVAT* (SEQ ID NO: 446)

EBAG9₁₋₂₉::EWSR1::Myc::2xPCP
DNA:
ATGGCCATCACCCAGTTTCGGTTATTTAAATTTTGTACCTGCCTAGCAACAGTATTCTCATTCCTAAAGAGATTA
AATGCAGATCTATGGCGTCCACGGATTACAGTACCTATAGCCAAGCTGCAGCGCAGCAGGGCTACAGTGCTTAC
ACCGCCCAGCCCACTCAAGGATATGCACAGACCACCCAGGCATATGGGCAACAAAGCTATGGAACCTATGGACAG
CCCACTGATGTCAGCTATACCCAGGCTCAGACCACTGCAACCTATGGGCAGACCGCCTATGCAACTTCTTATGGA
CAGCCTCCCACTGGTTATACTACTCCAACTGCCCCCCAGGCATACAGCCAGCCTGTCCAGGGGTATGGCACTGGT
GCTTATGATACCACCACTGCTACAGTCACCACCACCCAGGCCTCCTATGCAGCTCAGTCTGCATATGGCACTCAG
CCTGCTTATCCAGCCTATGGGCAGCAGCCAGCAGCCACTGCACCTACAAGACCGCAGGATGGAAACAAGCCCACT
GAGACTAGTCAACCTCAATCTAGCACAGGGGGTTACAACCAGCCCAGCCTAGGATATGGACAGAGTAACTACAGT
TATCCCCAGGTACCTGGGAGCTACCCCATGCAGCCAGTCACTGCACCTCCATCCTACCCTCCTACCAGCTATTCC
TCTACACAGCCGACTAGTTATGATCAGAGCAGTTACTCTCAGCAGAACACCTATGGGCAACCGAGCAGCTATGGA
CAGCAGAGTAGCTATGGTCAACAAAGCAGCTATGGGCAGCAGCCTCCCACTAGTTACCCACCCCAAACTGGATCC
TACAGCCAAGCTCCAAGTCAATATAGCCAACAGAGCAGCAGCTACGGGCAGCAGAGTTCATTCCGACAGGACCAC
CCCAGTAGCATGGGTGTTTATGGGCAGGAGTCTGGAGGATTTTCCGGACCAGGAGAGAACCGGAGCATGAGTGGC
CCTGATAACCGGGGCAGGGGAAGAGGGGGATTTGATCGTGGAGGCATGAGCAGAGGTGGGCGGGGAGGAGGACGC
GGTGGAATGGGCAGCGCTGGAGAGCGAGGTGGCTTCAATAAGCCTGGTGGACCCATGGATGAAGGACCAGATCTT
GATCTAGGCCCACCTGTAGATCCAGATGAAGACTCTGACAACAGTGCAATTTATGTACAAGGATTAAATGACAGT
GTGACTCTAGATGATCTGGCAGACTTCTTTAAGCAGTGTGGGGTTGTTAAGATGAACAAGAGAACTGGGCAACCC
ATGATCCACATCTACCTGGACAAGGAAACAGGAAAGCCCAAAGGCGATGCCACAGTGTCCTATGAAGACCCACCT
ACTGCCAAGGCTGCCGTGGAATGGTTTGATGGGAAAGATTTTCAAGGGAGCAAACTTAAAGTCTCCCTTGCTCGG
AAGAAGCCTCCAATGAACAGTATGCGGGGTGGTCTGCCACCCCGTGAGGGCAGAGGCATGCCACCACCACTCCGT
GGAGGTCCAGGAGGCCCAGGAGGTCCTGGGGGACCCATGGGTCGCATGGGAGGCCGTGGAGGAGATAGAGGAGGC
TTCCCTCCAAGAGGACCCCGGGGTTCCCGAGGGAACCCCTCTGGAGGAGGAAACGTCCAGCACCGAGCTGGAGAC
TGGCAGTGTCCCAATCCGGGTTGTGGAAACCAGAACTTCGCCTGGAGAACAGAGTGCAACCAGTGTAAGGCCCCA
AAGCCTGAAGGCTTCCTCCCGCCACCCTTTCCGCCCCCGGGTGGTGATCGTGGCAGAGGTGGCCCTGGTGGCATG
CGGGGAGGAAGAGGTGGCCTCATGGATCGTGGTGGTCCCGGTGGAATGTTCAGAGGTGGCCGTGGTGGAGACAGA

GGTGGCTTCCGTGGTGGCCGGGGCATGGACCGAGGTGGCTTTGGTGGAGGAAGACGAGGTGGCCCTGGGGGGCCC
CCTGGACCTTTGATGGAACAGGCGATCGCAGAGCAGAAGCTGATCTCAGAGGAGGACCTGATCGAAGGCCGCCAT
ATGCTAGCCTCCAAAACCATCGTTCTTTCGGTCGGCGAGGCTACTCGCACTCTGACTGAGATCCAGTCCACCGCA
GACCGTCAGATCTTCGAAGAGAAGGTCGGGCCTCTGGTGGGTCGGCTGCGCCTCACGGCTTCGCTCCGTCAAAAC
GGAGCCAAGACCGCGTATCGCGTCAACCTAAAACTGGATCAGGCGGACGTCGTTGATTCCGGACTTCCGAAAGTG
CGCTACACTCAGGTATGGTCGCACGACGTGACAATCGTTGCGAATAGCACCGAGGCCTCGCGCAAATCGTTGTAC
GATTTGACCAAGTCCCTCGTCGCGACCTCGCAGGTCGAAGATCTTGTCGTCAACCTTGTGCCGCTGGGCCGTGCG
GATCCGCTAGCCTCCAAAACCATCGTTCTTTCGGTCGGCGAGGCTACTCGCACTCTGACTGAGATCCAGTCCACC
GCAGACCGTCAGATCTTCGAAGAGAAGGTCGGGCCTCTGGTGGGTCGGCTGCGCCTCACGGCTTCGCTCCGTCAA
AACGGAGCCAAGACCGCGTATCGCGTCAACCTAAAACTGGATCAGGCGGACGTCGTTGATTCCGGACTTCCGAAA
GTGCGCTACACTCAGGTATGGTCGCACGACGTGACAATCGTTGCGAATAGCACCGAGGCCTCGCGCAAATCGTTG
TACGATTTGACCAAGTCCCTCGTCGCGACCTCGCAGGTCGAAGATCTTGTCGTCAACCTTGTGCCGCTGGGCCGT
CCACCGGTCGCCACCTAA (SEQ ID NO: 447)

Protein:
MAITQFRLFKFCTCLATVFSFLKRLICRSMASTDYSTYSQAAAQQGYSAYTAQPTQGYAQTTQAYGQQSYGTYGQ
PTDVSYTQAQTTATYGQTAYATSYGQPPTGYTTPTAPQAYSQPVQGYGTGAYDTTTATVTTTQASYAAQSAYGTQ
PAYPAYGQQPAATAPTRPQDGNKPTETSQPQSSTGGYNQPSLGYGQSNYSYPQVPGSYPMQPVTAPPSYPPTSYS
STQPTSYDQSSYSQQNTYGQPSSYGQQSSYGQQSSYGQQPPTSYPPQTGSYSQAPSQYSQQSSSYGQQSSFRQDH
PSSMGVYGQESGGFSGPGENRSMSGPDNRGRGRGGFDRGGMSRGGRGGGRGGMGSAGERGGFNKPGGPMDEGPDL
DLGPPVDPDEDSDNSAIYVQGLNDSVTLDDLADFFKQCGVVKMNKRTGQPMIHIYLDKETGKPKGDATVSYEDPP
TAKAAVEWFDGKDFQGSKLKVSLARKKPPMNSMRGGLPPREGRGMPPPLRGGPGGPGGPGGPMGRMGGRGGDRGG
FPPRGPRGSRGNPSGGGNVQHRAGDWQCPNPGCGNQNFAWRTECNQCKAPKPEGFLPPPFPPPGGDRGRGGPGGM
RGGRGGLMDRGGPGGMFRGGRGGDRGGFRGGRGMDRGGFGGGRRGGPGGPPGPLMEQAIAEQKLISEEDLIEGRH
MLASKTIVLSVGEATRTLTEIQSTADRQIFEEKVGPLVGRLRLTASLRQNGAKTAYRVNLKLDQADVVDSGLPKV
RYTQVWSHDVTIVANSTEASRKSLYDLTKSLVATSQVEDLVVNLVPLGRADPLASKTIVLSVGEATRTLTEIQST
ADRQIFEEKVGPLVGRLRLTASLRQNGAKTAYRVNLKLDQADVVDSGLPKVRYTQVWSHDVTIVANSTEASRKSL
YDLTKSLVATSQVEDLVVNLVPLGRPPVAT* (SEQ ID NO: 448)

EBAG9₁₋₂₉::EWSR1::HA::2xPCP
DNA:
ATGGCCATCACCCAGTTTCGGTTATTTAAATTTTGTACCTGCCTAGCAACAGTATTCTCATTCCTAAAGAGATTA
ATATGCAGATCTATGGCGTCCACGGATTACAGTACCTATAGCCAAGCTGCAGCGCAGCAGGGCTACAGTGCTTAC
ACCGCCCAGCCCACTCAAGGATATGCACAGACCACCCAGGCATATGGGCAACAAAGCTATGGAACCTATGGACAG
CCCACTGATGTCAGCTATACCCAGGCTCAGACCACTGCAACCTATGGGCAGACCGCCTATGCAACTTCTTATGGA
CAGCCTCCCACTGGTTATACTACTCCAACTGCCCCCCAGGCATACAGCCAGCCTGTCCAGGGGTATGGCACTGGT
GCTTATGATACCACCACTGCTACAGTCACCACCACCCAGGCCTCCTATGCAGCTCAGTCTGCATATGGCACTCAG
CCTGCTTATCCAGCCTATGGGCAGCAGCCAGCAGCCACTGCACCTACAAGACCGCAGGATGGAAACAAGCCCACT
GAGACTAGTCAACCTCAATCTAGCACAGGGGGTTACAACCAGCCCAGCCTAGGATATGGACAGAGTAACTACAGT
TATCCCCAGGTACCTGGGAGCTACCCCATGCAGCCAGTCACTGCACCTCCATCCTACCCTCCTACCAGCTATTCC
TCTACACAGCCGACTAGTTATGATCAGAGCAGTTACTCTCAGCAGAACACCTATGGGCAACCGAGCAGCTATGGA
CAGCAGAGTAGCTATGGTCAACAAAGCAGCTATGGGCAGCAGCCTCCCACTAGTTACCCACCCCAAACTGGATCC
TACAGCCAAGCTCCAAGTCAATATAGCCAACAGAGCAGCAGCTACGGGCAGCAGAGTTCATTCCGACAGGACCAC
CCCAGTAGCATGGGTGTTTATGGGCAGGAGTCTGGAGGATTTTCCGGACCAGGAGAGAACCGGAGCATGAGTGGC
CCTGATAACCGGGGCAGGGGAAGAGGGGGATTTGATCGTGGAGGCATGAGCAGAGGTGGGCGGGGAGGAGGACGC
GGTGGAATGGGCAGCGCTGGAGAGCGAGGTGGCTTCAATAAGCCTGGTGGACCCATGGATGAAGGACCAGATCTT
GATCTAGGCCCACCTGTAGATCCAGATGAAGACTCTGACAACAGTGCAATTTATGTACAAGGATTAAATGACAGT
GTGACTCTAGATGATCTGGCAGACTTCTTTAAGCAGTGTGGGGTTGTTAAGATGAACAAGAGAACTGGGCAACCC
ATGATCCACATCTACCTGGACAAGGAAACAGGAAAGCCCAAAGGCGATGCCACAGTGTCCTATGAAGACCCACCT
ACTGCCAAGGCTGCCGTGGAATGGTTTGATGGGAAAGATTTTCAAGGGAGCAAACTTAAAGTCTCCCTTGCTCGG
AAGAAGCCTCCAATGAACAGTATGCGGGGTGGTCTGCCACCCCGTGAGGGCAGAGGCATGCCACCACCACTCCGT
GGAGGTCCAGGAGGCCCAGGAGGTCCTGGGGGACCCATGGGTCGCATGGGAGGCCGTGGAGGAGATAGAGGAGGC
TTCCCTCCAAGAGGACCCCGGGGTTCCCGAGGGAACCCCTCTGGAGGAGGAAACGTCCAGCACCGAGCTGGAGAC
TGGCAGTGTCCCAATCCGGGTTGTGGAAACCAGAACTTCGCCTGGAGAACAGAGTGCAACCAGTGTAAGGCCCCA
AAGCCTGAAGGCTTCCTCCCGCCACCCTTTCCGCCCCCGGGTGGTGATCGTGGCAGAGGTGGCCCTGGTGGCATG
CGGGGAGGAAGAGGTGGCCTCATGGATCGTGGTGGTCCCGGTGGAATGTTCAGAGGTGGCCGTGGTGGAGACAGA
GGTGGCTTCCGTGGTGGCCGGGGCATGGACCGAGGTGGCTTTGGTGGAGGAAGACGAGGTGGCCCTGGGGGGCCC

CCTGGACCTTTGATGGAACAGGCGATCGCATACCCCTACGACGTGCCCGACTACGCCATCGAAGGCCGCCATATG
CTAGCCTCCAAAACCATCGTTCTTTCGGTCGGCGAGGCTACTCGCACTCTGACTGAGATCCAGTCCACCGCAGAC
CGTCAGATCTTCGAAGAGAAGGTCGGGCCTCTGGTGGGTCGGCTGCGCCTCACGGCTTCGCTCCGTCAAAACGGA
GCCAAGACCGCGTATCGCGTCAACCTAAAACTGGATCAGGCGGACGTCGTTGATTCCGGACTTCCGAAAGTGCGC
TACACTCAGGTATGGTCGCACGACGTGACAATCGTTGCGAATAGCACCGAGGCCTCGCGCAAATCGTTGTACGAT
TTGACCAAGTCCCTCGTCGCGACCTCGCAGGTCGAAGATCTTGTCGTCAACCTTGTGCCGCTGGGCCGTGCGGAT
CCGCTAGCCTCCAAAACCATCGTTCTTTCGGTCGGCGAGGCTACTCGCACTCTGACTGAGATCCAGTCCACCGCA
GACCGTCAGATCTTCGAAGAGAAGGTCGGGCCTCTGGTGGGTCGGCTGCGCCTCACGGCTTCGCTCCGTCAAAAC
GGAGCCAAGACCGCGTATCGCGTCAACCTAAAACTGGATCAGGCGGACGTCGTTGATTCCGGACTTCCGAAAGTG
CGCTACACTCAGGTATGGTCGCACGACGTGACAATCGTTGCGAATAGCACCGAGGCCTCGCGCAAATCGTTGTAC
GATTTGACCAAGTCCCTCGTCGCGACCTCGCAGGTCGAAGATCTTGTCGTCAACCTTGTGCCGCTGGGCCGTCCA
CCGGTCGCCACCTAA (SEQ ID NO: 449)

Protein:
MAITQFRLFKFCTCLATVFSFLKRLICRSMASTDYSTYSQAAAQQGYSAYTAQPTQGYAQTTQAYGQQSYGTYGQ
PTDVSYTQAQTTATYGQTAYATSYGQPPTGYTTPTAPQAYSQPVQGYGTGAYDTTTATVTTTQASYAAQSAYGTQ
PAYPAYGQQPAATAPTRPQDGNKPTETSQPQSSTGGYNQPSLGYGQSNYSYPQVPGSYPMQPVTAPPSYPPTSYS
STQPTSYDQSSYSQQNTYGQPSSYGQQSSYGQQSSYGQQPPTSYPPQTGSYSQAPSQYSQQSSSYGQQSSFRQDH
PSSMGVYGQESGGFSGPGENRSMSGPDNRGRGRGGFDRGGMSRGGRGGGRGGMGSAGERGGFNKPGGPMDEGPDL
DLGPPVDPDEDSDNSAIYVQGLNDSVTLDDLADFFKQCGVVKMNKRTGQPMIHIYLDKETGKPKGDATVSYEDPP
TAKAAVEWFDGKDFQGSKLKVSLARKKPPMNSMRGGLPPREGRGMPPPLRGGPGGPGGPGGPMGRMGGRGGDRGG
FPPRGPRGSRGNPSGGGNVQHRAGDWQCPNPGCGNQNFAWRTECNQCKAPKPEGFLPPPFPPPGGDRGRGGPGGM
RGGRGGLMDRGGPGGMFRGGRGGDRGGFRGGRGMDRGGFGGGRRGGPGGPPGPLMEQAIAYPYDVPDYAIEGRHM
LASKTIVLSVGEATRTLTEIQSTADRQIFEEKVGPLVGRLRLTASLRQNGAKTAYRVNLKLDQADVVDSGLPKVR
YTQVWSHDVTIVANSTEASRKSLYDLTKSLVATSQVEDLVVNLVPLGRADPLASKTIVLSVGEATRTLTEIQSTA
DRQIFEEKVGPLVGRLRLTASLRQNGAKTAYRVNLKLDQADVVDSGLPKVRYTQVWSHDVTIVANSTEASRKSLY
DLTKSLVATSQVEDLVVNLVPLGRPPVAT* (SEQ ID NO: 450)

LCK::CbzRS
DNA:
ATGGGCTGCGTGTGCAGCAGCAACCCCGAGGGTACCGAGCTCGCGTGCCCGGTGCCGCTGCAGCTGCCGCCGCTG
GAACGCCTGACCCTGGATGATAAAAAACCGCTGAATACCCTGATCTCTGCTACTGGTCTGTGGATGAGTCGTACC
GGAACCATTCATAAAATCAAACACCACGAGGTTAGCCGTTCGAAAATCTATATTGAGATGGCGTGTGGCGATCAT
CTGGTTGTGAACAATAGCCGCTCTTCTCGTACAGCACGTGCACTGCGTCACCACAAATATCGTAAAACCTGTAAA
CGTTGCCGTGTGTCCGATGAGGATCTGAACAAATTCCTGACAAAAGCCAATGAGGACCAAACAAGCGTGAAAGTG
AAAGTCGTTAGCGCTCCTACCCGTACTAAAAAAGCAATGCCGAAATCCGTTGCTCGTGCCCCTAAACCACTGGAA
AACACTGAAGCAGCACAGGCACAGCCGTCTGGAAGCAAATTCTCTCCGGCCATTCCTGTTTCTACCCAGGAGTCC
GTTTCTGTTCCAGCAAGTGTGAGCACCAGCATTAGCAGTATTAGCACCGGTGCCACCGCTAGCGCCCTGGTTAAA
GGCAATACCAATCCGATTACAAGCATGTCTGCCCCGGTTCAAGCATCAGCTCCAGCACTGACAAAATCCCAAACC
GATCGTCTGGAGGTTCTGCTGAATCCGAAAGACGAAATCAGCCTGAATTCCGGCAAACCGTTTCGTGAACTGGAG
AGCGAACTGCTGTCACGTCGTAAAAAAGACCTGCAACAAATCTATGCCGAAGAACGTGAGAACTATCTGGGGAAA
CTGGAACGTGAAATCACCCGCTTTTTCGTGGATCGTGGCTTTCTGGAGATCAAATCCCCGATTCTGATTCCTCTG
GAGTATATCGAGCGTATGGGCATCGACAATGATACCGAACTGAGCAAACAAATTTTCCGTGTGGATAAAAACTTC
TGTCTGCGCCCTATGCTTGCACCAAATCTGATGAACTATGGACGCAAACTGGACCGTGCCCTGCCTGATCCTATC
AAAATCTTCGAGATCGGCCCGTGTTATCGTAAAGAGTCCGACGGTAAAGAACATCTGGAGGAGTTTACCATGCTG
AACTTTACACAAATGGGTTCAGGTTGTACTCGTGAGAACCTGGAAAGCATCATCACCGATTTTCTGAACCACCTG
GGCATTGACTTCAAAATTGTGGGCGACAGCTGTATGGTGTATGGCGACACCCTGGATGTCATGCACGGCGACCTG
GAACTGTCTAGTGCCGTTGTTGGACCAATTCCGCTGGACCGTGAGTGGGGTATCGACAAACCGTGGATCGGAGCA
GGATTCGGTCTGGAACGCCTGCTGAAAGTGAAACACGACTTCAAAAACATCAAACGTGCCGCCCGTTCTGAATCG
TATTATAACGGGATCTCTACGAACCTGTAA (SEQ ID NO: 451)

Protein:
MGCVCSSNPEGTELACPVPLQLPPLERLTLDDKKPLNTLISATGLWMSRTGTIHKIKHHEVSRSKIYIEMACGDH
LVVNNSRSSRTARALRHHKYRKTCKRCRVSDEDLNKFLTKANEDQTSVKVKVVSAPTRTKKAMPKSVARAPKPLE
NTEAAQAQPSGSKFSPAIPVSTQESVSVPASVSTSISSISTGATASALVKGNTNPITSMSAPVQASAPALTKSQT
DRLEVLLNPKDEISLNSGKPFRELESELLSRRKKDLQQIYAEERENYLGKLEREITRFFVDRGFLEIKSPILIPL
EYIERMGIDNDTELSKQIFRVDKNFCLRPMLAPNLMNYGRKLDRALPDPIKIFEIGPCYRKESDGKEHLEEFTML

NFTQMGSGCTRENLESIITDFLNHLGIDFKIVGDSCMVYGDTLDVMHGDLELSSAVVGPIPLDREWGIDKPWIGA
GFGLERLLKVKHDFKNIKRAARSESYYNGISTNL* (SEQ ID NO: 452)

LCK::FUS::CbzRS
DNA:
ATGGGCTGCGTGTGCAGCAGCAACCCCGAGGGTACCGAGCTCGCCTCAAACGATTATACCCAACAAGCAACCCAA
AGCTATGGGGCCTACCCCACCCAGCCCGGGCAGGGCTATTCCCAGCAGAGCAGTCAGCCCTACGGACAGCAGAGT
TACAGTGGTTATAGCCAGTCCACGGACACTTCAGGATATGGCCAGAGCAGCTATTCTTCTTATGGCCAGAGCCAG
AACACAGGCTATGGAACTCAGTCAACTCCCCAGGGATATGGCTCGACTGGCGGCTATGGCAGTAGCCAGAGCTCC
CAATCGTCTTACGGGCAGCAGTCCTCCTACCCTGGCTATGGCCAGCAGCCAGCTCCCAGCAGCACCTCGGGAAGT
TACGGTAGCAGTTCTCAGAGCAGCAGCTATGGGCAGCCCCAGAGTGGGAGCTACAGCCAGCAGCCTAGCTATGGT
GGACAGCAGCAAAGCTATGGACAGCAGCAAAGCTATAATCCCCCTCAGGGCTATGGACAGCAGAACCAGTACAAC
AGCAGCAGTGGTGGTGGAGGTGGAGGTGGAGGTGGAGGTAACTATGGCCAAGATCAATCCTCCATGAGTAGTGGT
GGTGGCAGTGGTGGCGGTTATGGCAATCAAGACCAGAGTGGTGGAGGTGGCAGCGGTGGCTATGGACAGCAGGAC
CGTGGAGGCCGCGGCAGGGGTGGCAGTGGTGGCGGCGGCGGCGGCGGTGGTGGTTACAACCGCAGCAGTGGT
GGCTATGAACCCAGAGGTCGTGGAGGTGGCCGTGGAGGCAGAGGTGGCATGGGCGGAAGTGACCGTGGTGGCTTC
AATAAATTTGGTGGCCCTCGGGACCAAGGATCACGTCATGACTCCGAACAGGATAATTCAGACAACAACACCATC
TTTGTGCAAGGCCTGGGTGAGAATGTTACAATTGAGTCTGTGGCTGATTACTTCAAGCAGATTGGTATTATTAAG
ACAAACAAGAAAACGGGACAGCCCATGATTAATTTGTACACAGACAGGGAAACTGGCAAGCTGAAGGGAGAGGCA
ACGGTCTCTTTTGATGACCCACCTTCAGCTAAAGCAGCTATTGACTGGTTTGATGGTAAAGAATTCTCCGGAAAT
CCTATCAAGGTCTCATTTGCTACTCGCCGGGCAGACTTTAATCGGGGTGGTGGCAATGGTCGTGGAGGCCGAGGG
CGAGGAGGACCCATGGGCCGTGGAGGCTATGGAGGTGGTGGCAGTGGTGGTGGTGGCCGAGGAGGATTTCCCAGT
GGAGGTGGTGGCGGTGGAGGACAGCAGCGAGCTGGTGACTGGAAGTGTCCTAATCCCACCTGTGAGAATATGAAC
TTCTCTTGGAGGAATGAATGCAACCAGTGTAAGGCCCCTAAACCAGATGGCCCAGGAGGGGGACCAGGTGGCTCT
CACATGGGGGGTAACTACGGGGATGATCGTCGTGGTGGCAGAGGAGGCGGCGCCCCCGGCTCCGCCGGCTCCGCC
GCCGGCTCCGGCATGGCGTGCCCGGTGCCGCTGCAGCTGCCGCCGCTGGAACGCCTGACCCTGGATGATAAAAAA
CCGCTGAATACCCTGATCTCTGCTACTGGTCTGTGGATGAGTCGTACCGGAACCATTCATAAAATCAAACACCAC
GAGGTTAGCCGTTCGAAAATCTATATTGAGATGGCGTGTGGCGATCATCTGGTTGTGAACAATAGCCGCTCTTCT
CGTACAGCACGTGCACTGCGTCACCACAAATATCGTAAAACCTGTAAACGTTGCCGTGTGTCCGATGAGGATCTG
AACAAATTCCTGACAAAAGCCAATGAGGACCAAACAAGCGTGAAAGTGAAAGTCGTTAGCGCTCCTACCCGTACT
AAAAAAGCAATGCCGAAATCCGTTGCTCGTGCCCCTAAACCACTGGAAAACACTGAAGCAGCACAGGCACAGCCG
TCTGGAAGCAAATTCTCTCCGGCCATTCCTGTTTCTACCCAGGAGTCCGTTTCTGTTCCAGCAAGTGTGAGCACC
AGCATTAGCAGTATTAGCACCGGTGCCACCGCTAGCGCCCTGGTTAAAGGCAATACCAATCCGATTACAAGCATG
TCTGCCCCGGTTCAAGCATCAGCTCCAGCACTGACAAAATCCCAAACCGATCGTCTGGAGGTTCTGCTGAATCCG
AAAGACGAAATCAGCCTGAATTCCGGCAAACCGTTTCGTGAACTGGAGAGCGAACTGCTGTCACGTCGTAAAAAA
GACCTGCAACAAATCTATGCCGAAGAACGTGAGAACTATCTGGGGAAACTGGAACGTGAAATCACCCGCTTTTTC
GTGGATCGTGGCTTTCTGGAGATCAAATCCCCGATTCTGATTCCTCTGGAGTATATCGAGCGTATGGGCATCGAC
AATGATACCGAACTGAGCAAACAAATTTTCCGTGTGGATAAAAACTTCTGTCTGCGCCCTATGCTTGCACCAAAT
CTGATGAACTATGGACGCAAACTGGACCGTGCCCTGCCTGATCCTATCAAAATCTTCGAGATCGGCCCGTGTTAT
CGTAAAGAGTCCGACGGTAAAGAACATCTGGAGGAGTTTACCATGCTGAACTTTACACAAATGGGTTCAGGTTGT
ACTCGTGAGAACCTGGAAAGCATCATCACCGATTTTCTGAACCACCTGGGCATTGACTTCAAAATTGTGGGCGAC
AGCTGTATGGTGTATGGCGACACCCTGGATGTCATGCACGGCGACCTGGAACTGTCTAGTGCCGTTGTTGGACCA
ATTCCGCTGGACCGTGAGTGGGGTATCGACAAACCGTGGATCGGAGCAGGATTCGGTCTGGAACGCCTGCTGAAA
GTGAAACACGACTTCAAAAACATCAAACGTGCCGCCCGTTCTGAATCGTATTATAACGGGATCTCTACGAACCTG
TAA (SEQ ID NO: 453)

Protein:
MGCVCSSNPEGTELASNDYTQQATQSYGAYPTQPGQGYSQQSSQPYGQQSYSGYSQSTDTSGYGQSSYSSYGQSQ
NTGYGTQSTPQGYGSTGGYGSSQSSQSSYGQQSSYPGYGQQPAPSSTSGSYGSSSQSSSYGQPQSGSYSQQPSYG
GQQQSYGQQQSYNPPQGYGQQNQYNSSSGGGGGGGGGNYGQDQSSMSSGGGSGGGYGNQDQSGGGGSGGYGQQD
RGGRGRGGSGGGGGGGGGYNRSSGGYEPRGRGGGRGGRGGMGGSDRGGFNKFGGPRDQGSRHDSEQDNSDNNTI
FVQGLGENVTIESVADYFKQIGIIKTNKKTGQPMINLYTDRETGKLKGEATVSFDDPPSAKAAIDWFDGKEFSGN
PIKVSFATRRADFNRGGGNGRGGRGRGGPMGRGGYGGGGSGGGGRGGFPSGGGGGGGQQRAGDWKCPNPTCENMN
FSWRNECNQCKAPKPDGPGGGPGGSHMGGNYGDDRRGGRGGGAPGSAGSAAGSGMACPVPLQLPPLERLTLDDKK
PLNTLISATGLWMSRTGTIHKIKHHEVSRSKIYIEMACGDHLVVNNSRSSRTARALRHHKYRKTCKRCRVSDEDL
NKFLTKANEDQTSVKVKVVSAPTRTKKAMPKSVARAPKPLENTEAAQAQPSGSKFSPAIPVSTQESVSVPASVST
SISSISTGATASALVKGNTNPITSMSAPVQASAPALTKSQTDRLEVLLNPKDEISLNSGKPFRELESELLSRRKK

DLQQIYAEERENYLGKLEREITRFFVDRGFLEIKSPILIPLEYIERMGIDNDTELSKQIFRVDKNFCLRPMLAPN
LMNYGRKLDRALPDPIKIFEIGPCYRKESDGKEHLEEFTMLNFTQMGSGCTRENLESIITDFLNHLGIDFKIVGD
SCMVYGDTLDVMHGDLELSSAVVGPIPLDREWGIDKPWIGAGFGLERLLKVKHDFKNIKRAARSESYYNGISTNL
* (SEQ ID NO: 454)

TOM20::FUS::SYNZIP1::CpkRS
DNA:
ATGGTGGGTCGGAACAGCGCCATCGCCGCCGGTGTATGCGGGGCCCTTTTCATTGGGTACTGCATCTACTTCGAC
CGCAAAAGACGAAGTGACCCCAACTTCAAGAACAGGCTTCGAGAACGAAGAAAGAAACAGAAGCTTGCCAAGGAG
AGAGCTGGGCTTTCCAAGTTACCTGACCTTAAAGATGCTGAAGCTGTTCAGAAATTCTTCATGGCCTCAAACGAT
TATACCCAACAAGCAACCCAAAGCTATGGGGCCTACCCCACCCAGCCCGGGCAGGGCTATTCCCAGCAGAGCAGT
CAGCCCTACGGACAGCAGAGTTACAGTGGTTATAGCCAGTCCACGGACACTTCAGGATATGGCCAGAGCAGCTAT
TCTTCTTATGGCCAGAGCCAGAACACAGGCTATGGAACTCAGTCAACTCCCCAGGGATATGGCTCGACTGGCGGC
TATGGCAGTAGCCAGAGCTCCCAATCGTCTTACGGGCAGCAGTCCTCCTACCCTGGCTATGGCCAGCAGCCAGCT
CCCAGCAGCACCTCGGGAAGTTACGGTAGCAGTTCTCAGAGCAGCAGCTATGGGCAGCCCCAGAGTGGGAGCTAC
AGCCAGCAGCCTAGCTATGGTGGACAGCAGCAAAGCTATGGACAGCAGCAAAGCTATAATCCCCCTCAGGGCTAT
GGACAGCAGAACCAGTACAACAGCAGCAGTGGTGGTGGAGGTGGAGGTGGAGGTGGAGGTAACTATGGCCAAGAT
CAATCCTCCATGAGTAGTGGTGGTGGCAGTGGTGGCGGTTATGGCAATCAAGACCAGAGTGGTGGAGGTGGCAGC
GGTGGCTATGGACAGCAGGACCGTGGAGGCCGCGGCAGGGGTGGCAGTGGTGGCGGCGGCGGCGGCGGCGGTGGT
GGTTACAACCGCAGCAGTGGTGGCTATGAACCCAGAGGTCGTGGAGGTGGCCGTGGAGGCAGAGGTGGCATGGGC
GGAAGTGACCGTGGTGGCTTCAATAAATTTGGTGGCCCTCGGGACCAAGGATCACGTCATGACTCCGAACAGGAT
AATTCAGACAACAACACCATCTTTGTGCAAGGCCTGGGTGAGAATGTTACAATTGAGTCTGTGGCTGATTACTTC
AAGCAGATTGGTATTATTAAGACAAACAAGAAAACGGGACAGCCCATGATTAATTTGTACACAGACAGGGAAACT
GGCAAGCTGAAGGGAGAGGCAACGGTCTCTTTTGATGACCCACCTTCAGCTAAAGCAGCTATTGACTGGTTTGAT
GGTAAAGAATTCTCCGGAAATCCTATCAAGGTCTCATTTGCTACTCGCCGGGCAGACTTTAATCGGGGTGGTGGC
AATGGTCGTGGAGGCCGAGGGCGAGGAGGACCCATGGGCCGTGGAGGCTATGGAGGTGGTGGCAGTGGTGGTGGT
GGCCGAGGAGGATTTCCCAGTGGAGGTGGTGGCGGTGGAGGACAGCAGCGAGCTGGTGACTGGAAGTGTCCTAAT
CCCACCTGTGAGAATATGAACTTCTCTTGGAGGAATGAATGCAACCAGTGTAAGGCCCCTAAACCAGATGGCCCA
GGAGGGGGACCAGGTGGCTCTCACATGGGGGGTAACTACGGGGATGATCGTCGTGGTGGCAGAGGAGGCGCGATC
GCAAATCTGGTGGCCCAGCTGGAAAACGAGGTGGCCAGCCTGGAAAACGAGAACGAAACCCTGAAGAAAAAGAAC
CTGCACAAGAAGGACCTGATCGCCTACCTGGAAAAGGAAATCGCCAACCTGAGAAAGAAGATCGAGGAAGGCAAG
CCTATTCCCAACCCCCTGCTGGGCCTGGATAGCACCGGAGCACCAGGAAGTGCTGGTTCTGCTGCTGGTAGTGGA
GCGTGCCCGGTGCCGCTGCAGCTGCCGCCGCTGGAACGCCTGACCCTGGATGATAAAAAACCGCTGAATACCCTG
ATCTCTGCTACTGGTCTGTGGATGAGTCGTACCGGAACCATTCATAAAATCAAACACCACGAGGTTAGCCGTTCG
AAAATCTATATTGAGATGGCGTGTGGCGATCATCTGGTTGTGAACAATAGCCGCTCTTCTCGTACAGCACGTGCA
CTGCGTCACCACAAATATCGTAAAACCTGTAAACGTTGCCGTGTGTCCGATGAGGATCTGAACAAATTCCTGACA
AAAGCCAATGAGGACCAAACAAGCGTGAAAGTGAAAGTCGTTAGCGCTCCTACCCGTACTAAAAAAGCAATGCCG
AAATCCGTTGCTCGTGCCCCTAAACCACTGGAAAACACTGAAGCAGCACAGGCACAGCCGTCTGGAAGCAAATTC
TCTCCGGCCATTCCTGTTTCTACCCAGGAGTCCGTTTCTGTTCCAGCAAGTGTGAGCACCAGCATTAGCAGTATT
AGCACCGGTGCCACCGCTAGCGCCCTGGTTAAAGGCAATACCAATCCGATTACAAGCATGTCTGCCCCGGTTCAA
GCATCAGCTCCAGCACTGACAAAATCCCAAACCGATCGTCTGGAGGTTCTGCTGAATCCGAAAGACGAAATCAGC
CTGAATTCCGGCAAACCGTTTCGTGAACTGGAGAGCGAACTGCTGTCACGTCGTAAAAAAGACCTGCAACAAATC
TATGCCGAAGAACGTGAGAACTATCTGGGGAAACTGGAACGTGAAATCACCCGCTTTTTCGTGGATCGTGGCTTT
CTGGAGATCAAATCCCCGATTCTGATTCCTCTGGAGTATATCGAGCGTATGGGCATCGACAATGATACCGAACTG
AGCAAACAAATTTTCCGTGTGGATAAAAACTTCTGTCTGCGCCCTATGCTTTCACCAAATCTGTATAACTATCTG
CGCAAACTGGACCGTGCCCTGCCTGATCCTATCAAAATCTTCGAGATCGGCCCGTGTTATCGTAAAGAGTCCGAC
GGTAAAGAACATCTGGAGGAGTTTACCATGCTGAACTTTTGCCAAATGGGTTCAGGTTGTACTCGTGAGAACCTG
GAAAGCATCATCACCGATTTTCTGAACCACCTGGGCATTGACTTCAAAATTGTGGGCGACAGCTGTATGGTGTAT
GGCGACACCCTGGATGTCATGCACGGCGACCTGGAACTGTCTAGTGCCGTTGTTGGACCAATTCCGCTGGACCGT
GAGTGGGGTATCGACAAACCGTGGATCGGAGCAGGATTCGGTCTGGAACGCCTGCTGAAAGTGAAACACGACTTC
AAAAACATCAAACGTGCCGCCCGTTCTGAATCGTATTATAACGGGATCTCTACGAACCTGTAA (SEQ ID NO:
455)

Protein:
MVGRNSAIAAGVCGALFIGYCIYFDRKRRSDPNFKNRLRERRKKQKLAKERAGLSKLPDLKDAEAVQKFFMASND
YTQQATQSYGAYPTQPGQGYSQQSSQPYGQQSYSGYSQSTDTSGYGQSSYSSYGQSQNTGYGTQSTPQGYGSTGG
YGSSQSSQSSYGQQSSYPGYGQQPAPSSTSGSYGSSSQSSSYGQPQSGSYSQQPSYGGQQQSYGQQQSYNPPQGY

GQQNQYNSSSGGGGGGGGGGNYGQDQSSMSSGGGSGGGYGNQDQSGGGGSGGYGQQDRGGRGRGGSGGGGGGGG
GYNRSSGGYEPRGRGGGRGGRGGMGGSDRGGFNKFGGPRDQGSRHDSEQDNSDNNTIFVQGLGENVTIESVADYF
KQIGIIKTNKKTGQPMINLYTDRETGKLKGEATVSFDDPPSAKAAIDWFDGKEFSGNPIKVSFATRRADFNRGGG
NGRGGRGRGGPMGRGGYGGGGSGGGGRGGFPSGGGGGGGQQRAGDWKCPNPTCENMNFSWRNECNQCKAPKPDGP
GGGPGGSHMGGNYGDDRRGGRGGAIANLVAQLENEVASLENENETLKKKNLHKKDLIAYLEKEIANLRKKIEEGK
PIPNPLLGLDSTGAPGSAGSAAGSGACPVPLQLPPLERLTLDDKKPLNTLISATGLWMSRTGTIHKIKHHEVSRS
KIYIEMACGDHLVVNNSRSSRTARALRHHKYRKTCKRCRVSDEDLNKFLTKANEDQTSVKVKVVSAPTRTKKAMP
KSVARAPKPLENTEAAQAQPSGSKFSPAIPVSTQESVSVPASVSTSISSISTGATASALVKGNTNPITSMSAPVQ
ASAPALTKSQTDRLEVLLNPKDEISLNSGKPFRELESELLSRRKKDLQQIYAEERENYLGKLEREITRFFVDRGF
LEIKSPILIPLEYIERMGIDNDTELSKQIFRVDKNFCLRPMLSPNLYNYLRKLDRALPDPIKIFEIGPCYRKESD
GKEHLEEFTMLNFCQMGSGCTRENLESIITDFLNHLGIDFKIVGDSCMVYGDTLDVMHGDLELSSAVVGPIPLDR
EWGIDKPWIGAGFGLERLLKVKHDFKNIKRAARSESYYNGISTNL* (SEQ ID NO: 456)

KIF16B::FUS::CbzRS
DNA:
ATGGCATCGGTCAAGGTGGCCGTGAGGGTCCGGCCCATGAATCGCAGGGAAAAGGACTTGGAGGCCAAGTTCATT
ATTCAGATGGAGAAAAGCAAAACGACAATCACAAACTTAAAGATACCAGAAGGAGGCACTGGGGACTCAGGAAGA
GAACGGACCAAGACCTTCACCTATGACTTTTCTTTTTATTCTGCTGATACAAAAAGCCCAGATTACGTTTCACAA
GAAATGGTTTTCAAAACCCTCGGCACAGATGTCGTGAAGTCTGCATTTGAAGGTTATAATGCTTGTGTCTTTGCA
TATGGGCAAACTGGATCTGGAAAGTCATACACTATGATGGGAAATTCTGGAGATTCTGGCTTAATACCTCGGATC
TGTGAAGGACTCTTCAGTCGGATAAATGAAACCACCAGATGGGATGAAGCTTCTTTTCGAACTGAAGTCAGCTAC
TTAGAAATTTATAACGAACGTGTGAGAGATCTACTTCGGCGGAAGTCATCTAAAACCTTCAATTTGAGAGTCCGT
GAGCATCCCAAAGAAGGCCCTTATGTTGAGGATTTATCCAAACATTTAGTACAGAATTATGGTGACGTAGAAGAA
CTTATGGATGCGGGCAATATCAACCGGACCACCGCAGCGACTGGGATGAACGACGTCAGTAGCAGGTCTCATGCC
ATCTTCACCATCAAGTTCACTCAGGCTAAATTTGATTCTGAAATGCCATGTGAAACCGTCAGTAAGATCCACTTG
GTTGATCTTGCCGGAAGTGAGCGTGCAGATGCCACCGGAGCCACCGGGGTTAGGCTAAAGGAAGGGGGAAATATT
AACAAGTCCCTCGTGACTCTGGGGAACGTCATTTCTGCCTTAGCTGATTTATCTCAGGATGCTGCAAATACTCTT
GCAAAGAAGAAGCAAGTTTTCGTGCCTTACAGGGATTCTGTGTTGACTTGGTTGTTAAAAGATAGCCTTGGAGGA
AACTCTAAAACTATCATGATTGCCACCATTTCACCTGCTGATGTCAATTATGGAGAAACCCTAAGTACTCTTCGC
TATGCAAATAGAGCCAAAAACATCATCAACAAGCCTACCATTAATGAGGATGCCAACGTCAAACTTATCCGTGAG
CTGCGAGCTGAAATAGCCAGACTGAAAACGCTGCTTGCTCAAGGGAATCAGATTGCCCTCTTAGACTCCCCCACA
TATACAGATATTGAAATGAACAGATTGGGAAAGGGCGCCCCCGGCTCCGCCGGCTCCGCCGCCGGCTCCGGCATG
GCCTCAAACGATTATACCCAACAAGCAACCCAAAGCTATGGGGCCTACCCCACCCAGCCCGGGCAGGGCTATTCC
CAGCAGAGCAGTCAGCCCTACGGACAGCAGAGTTACAGTGGTTATAGCCAGTCCACGGACACTTCAGGATATGGC
CAGAGCAGCTATTCTTCTTATGGCCAGAGCCAGAACACAGGCTATGGAACTCAGTCAACTCCCCAGGGATATGGC
TCGACTGGCGGCTATGGCAGTAGCCAGAGCTCCCAATCGTCTTACGGGCAGCAGTCCTCCTACCCTGGCTATGGC
CAGCAGCCAGCTCCCAGCAGCACCTCGGGAAGTTACGGTAGCAGTTCTCAGAGCAGCAGCTATGGGCAGCCCCAG
AGTGGGAGCTACAGCCAGCAGCCTAGCTATGGTGGACAGCAGCAAAGCTATGGACAGCAGCAAAGCTATAATCCC
CCTCAGGGCTATGGACAGCAGAACCAGTACAACAGCAGCAGTGGTGGTGGAGGTGGAGGTGGAGGTGGAGGTAAC
TATGGCCAAGATCAATCCTCCATGAGTAGTGGTGGTGGCAGTGGTGGCGGTTATGGCAATCAAGACCAGAGTGGT
GGAGGTGGCAGCGGTGGCTATGGACAGCAGGACCGTGGAGGCCGCGGCAGGGGTGGCAGTGGTGGCGGCGGCGGC
GGCGGCGGTGGTGGTTACAACCGCAGCAGTGGTGGCTATGAACCCAGAGGTCGTGGAGGTGGCCGTGGAGGCAGA
GGTGGCATGGGCGGAAGTGACCGTGGTGGCTTCAATAAATTTGGTGGCCCTCGGGACCAAGGATCACGTCATGAC
TCCGAACAGGATAATTCAGACAACAACACCATCTTTGTGCAAGGCCTGGGTGAGAATGTTACAATTGAGTCTGTG
GCTGATTACTTCAAGCAGATTGGTATTATTAAGACAAACAAGAAAACGGGACAGCCCATGATTAATTTGTACACA
GACAGGGAAACTGGCAAGCTGAAGGGAGAGGCAACGGTCTCTTTTGATGACCCACCTTCAGCTAAAGCAGCTATT
GACTGGTTTGATGGTAAAGAATTCTCCGGAAATCCTATCAAGGTCTCATTTGCTACTCGCCGGGCAGACTTTAAT
CGGGGTGGTGGCAATGGTCGTGGAGGCCGAGGGCGAGGAGGACCCATGGGCCGTGGAGGCTATGGAGGTGGTGGC
AGTGGTGGTGGTGGCCGAGGAGGATTTCCCAGTGGAGGTGGTGGCGGTGGAGGACAGCAGCGAGCTGGTGACTGG
AAGTGTCCTAATCCCACCTGTGAGAATATGAACTTCTCTTGGAGGAATGAATGCAACCAGTGTAAGGCCCCTAAA
CCAGATGGCCCAGGAGGGGGACCAGGTGGCTCTCACATGGGGGGTAACTACGGGGATGATCGTCGTGGTGGCAGA
GGAGGCGATTACAAGGATGACGACGATAAGGGTACCGGCGCCCCCGGCTCCGCCGGCTCCGCCGCCGGCTCCGGC
GCGTGCCCGGTGCCGCTGCAGCTGCCGCCGCTGGAACGCCTGACCCTGGATGATAAAAAACCGCTGAATACCCTG
ATCTCTGCTACTGGTCTGTGGATGAGTCGTACCGGAACCATTCATAAAATCAAACACCACGAGGTTAGCCGTTCG
AAAATCTATATTGAGATGGCGTGTGGCGATCATCTGGTTGTGAACAATAGCCGCTCTTCTCGTACAGCACGTGCA
CTGCGTCACCACAAATATCGTAAAACCTGTAAACGTTGCCGTGTGTCCGATGAGGATCTGAACAAATTCCTGACA
AAAGCCAATGAGGACCAAACAAGCGTGAAAGTGAAAGTCGTTAGCGCTCCTACCCGTACTAAAAAAGCAATGCCG

AAATCCGTTGCTCGTGCCCCTAAACCACTGGAAAACACTGAAGCAGCACAGGCACAGCCGTCTGGAAGCAAATTC
TCTCCGGCCATTCCTGTTTCTACCCAGGAGTCCGTTTCTGTTCCAGCAAGTGTGAGCACCAGCATTAGCAGTATT
AGCACCGGTGCCACCGCTAGCGCCCTGGTTAAAGGCAATACCAATCCGATTACAAGCATGTCTGCCCCGGTTCAA
GCATCAGCTCCAGCACTGACAAAATCCCAAACCGATCGTCTGGAGGTTCTGCTGAATCCGAAAGACGAAATCAGC
CTGAATTCCGGCAAACCGTTTCGTGAACTGGAGAGCGAACTGCTGTCACGTCGTAAAAAAGACCTGCAACAAATC
TATGCCGAAGAACGTGAGAACTATCTGGGGAAACTGGAACGTGAAATCACCCGCTTTTTCGTGGATCGTGGCTTT
CTGGAGATCAAATCCCCGATTCTGATTCCTCTGGAGTATATCGAGCGTATGGGCATCGACAATGATACCGAACTG
AGCAAACAAATTTTCCGTGTGGATAAAAACTTCTGTCTGCGCCCTATGCTTGCACCAAATCTGATGAACTATGGA
CGCAAACTGGACCGTGCCCTGCCTGATCCTATCAAAATCTTCGAGATCGGCCCGTGTTATCGTAAAGAGTCCGAC
GGTAAAGAACATCTGGAGGAGTTTACCATGCTGAACTTTACACAAATGGGTTCAGGTTGTACTCGTGAGAACCTG
GAAAGCATCATCACCGATTTTCTGAACCACCTGGGCATTGACTTCAAAATTGTGGGCGACAGCTGTATGGTGTAT
GGCGACACCCTGGATGTCATGCACGGCGACCTGGAACTGTCTAGTGCCGTTGTTGGACCAATTCCGCTGGACCGT
GAGTGGGGTATCGACAAACCGTGGATCGGAGCAGGATTCGGTCTGGAACGCCTGCTGAAAGTGAAACACGACTTC
AAAAACATCAAACGTGCCGCCCGTTCTGAATCGTATTATAACGGGATCTCTACGAACCTGTAA (SEQ ID NO:
457)

Protein:
MASVKVAVRVRPMNRREKDLEAKFIIQMEKSKTTITNLKIPEGGTGDSGRERTKTFTYDFSFYSADTKSPDYVSQ
EMVFKTLGTDVVKSAFEGYNACVFAYGQTGSGKSYTMMGNSGDSGLIPRICEGLFSRINETTRWDEASFRTEVSY
LEIYNERVRDLLRRKSSKTFNLRVREHPKEGPYVEDLSKHLVQNYGDVEELMDAGNINRTTAATGMNDVSSRSHA
IFTIKFTQAKFDSEMPCETVSKIHLVDLAGSERADATGATGVRLKEGGNINKSLVTLGNVISALADLSQDAANTL
AKKKQVFVPYRDSVLTWLLKDSLGGNSKTIMIATISPADVNYGETLSTLRYANRAKNIINKPTINEDANVKLIRE
LRAEIARLKTLLAQGNQIALLDSPTYTDIEMNRLGKGAPGSAGSAAGSGMASNDYTQQATQSYGAYPTQPGQGYS
QQSSQPYGQQSYSGYSQSTDTSGYGQSSYSSYGQSQNTGYGTQSTPQGYGSTGGYGSSQSSQSSYGQQSSYPGYG
QQPAPSSTSGSYGSSSQSSSYGQPQSGSYSQQPSYGGQQQSYGQQQSYNPPQGYGQQNQYNSSSGGGGGGGGGN
YGQDQSSMSSGGGSGGGYGNQDQSGGGGSGGYGQQDRGGRGRGGSGGGGGGGGGGYNRSSGGYEPRGRGGGRGGR
GGMGGSDRGGFNKFGGPRDQGSRHDSEQDNSDNNTIFVQGLGENVTIESVADYFKQIGIIKTNKKTGQPMINLYT
DRETGKLKGEATVSFDDPPSAKAAIDWFDGKEFSGNPIKVSFATRRADFNRGGGNGRGGRGRGGPMGRGGYGGGG
SGGGGRGGFPSGGGGGGGQQRAGDWKCPNPTCENMNFSWRNECNQCKAPKPDGPGGGPGGSHMGGNYGDDRRGGR
GGDYKDDDDKGTGAPGSAGSAAGSGACPVPLQLPPLERLTLDDKKPLNTLISATGLWMSRTGTIHKIKHHEVSRS
KIYIEMACGDHLVVNNSRSSRTARALRHHKYRKTCKRCRVSDEDLNKFLTKANEDQTSVKVKVVSAPTRTKKAMP
KSVARAPKPLENTEAAQAQPSGSKFSPAIPVSTQESVSVPASVSTSISSISTGATASALVKGNTNPITSMSAPVQ
ASAPALTKSQTDRLEVLLNPKDEISLNSGKPFRELESELLSRRKKDLQQIYAEERENYLGKLEREITRFFVDRGF
LEIKSPILIPLEYIERMGIDNDTELSKQIFRVDKNFCLRPMLAPNLMNYGRKLDRALPDPIKIFEIGPCYRKESD
GKEHLEEFTMLNFTQMGSGCTRENLESIITDFLNHLGIDFKIVGDSCMVYGDTLDVMHGDLELSSAVVGPIPLDR
EWGIDKPWIGAGFGLERLLKVKHDFKNIKRAARSESYYNGISTNL* (SEQ ID NO: 458)

EBAG9₁₋₂₉::FUS::CpkRS
DNA:
ATGGCCATCACCCAGTTTCGGTTATTTAAATTTTGTACCTGCCTAGCAACAGTATTCTCATTCCTAAAGAGATTA
ATATGCAGATCTGGAGCACCCGGCTCCGCCGGCTCCGCCGCCGGCTCCGGCATGGCCTCAAACGATTATACCCAA
CAAGCAACCCAAAGCTATGGGGCCTACCCCACCCAGCCCGGGCAGGGCTATTCCCAGCAGAGCAGTCAGCCCTAC
GGACAGCAGAGTTACAGTGGTTATAGCCAGTCCACGGACACTTCAGGATATGGCCAGAGCAGCTATTCTTCTTAT
GGCCAGAGCCAGAACACAGGCTATGGAACTCAGTCAACTCCCCAGGGATATGGCTCGACTGGCGGCTATGGCAGT
AGCCAGAGCTCCCAATCGTCTTACGGGCAGCAGTCCTCCTACCCTGGCTATGGCCAGCAGCCAGCTCCCAGCAGC
ACCTCGGGAAGTTACGGTAGCAGTTCTCAGAGCAGCAGCTATGGGCAGCCCCAGAGTGGGAGCTACAGCCAGCAG
CCTAGCTATGGTGGACAGCAGCAAAGCTATGGACAGCAGCAAAGCTATAATCCCCCTCAGGGCTATGGACAGCAG
AACCAGTACAACAGCAGCAGTGGTGGTGGAGGTGGAGGTGGAGGTGGAGGTAACTATGGCCAAGATCAATCCTCC
ATGAGTAGTGGTGGTGGCAGTGGTGGCGGTTATGGCAATCAAGACCAGAGTGGTGGAGGTGGCAGCGGTGGCTAT
GGACAGCAGGACCGTGGAGGCCGCGGCAGGGGTGGCAGTGGTGGCGGCGGCGGCGGCGGTGGTGGTTACAAC
CGCAGCAGTGGTGGCTATGAACCCAGAGGTCGTGGAGGTGGCCGTGGAGGCAGAGGTGGCATGGGCGGAAGTGAC
CGTGGTGGCTTCAATAAATTTGGTGGCCCTCGGGACCAAGGATCACGTCATGACTCCGAACAGGATAATTCAGAC
AACAACACCATCTTTGTGCAAGGCCTGGGTGAGAATGTTACAATTGAGTCTGTGGCTGATTACTTCAAGCAGATT
GGTATTATTAAGACAAACAAGAAAACGGGACAGCCCATGATTAATTTGTACACAGACAGGGAAACTGGCAAGCTG
AAGGGAGAGGCAACGGTCTCTTTTGATGACCCACCTTCAGCTAAAGCAGCTATTGACTGGTTTGATGGTAAAGAA
TTCTCCGGAAATCCTATCAAGGTCTCATTTGCTACTCGCCGGGCAGACTTTAATCGGGGTGGTGGCAATGGTCGT
GGAGGCCGAGGGCGAGGAGGACCCATGGGCCGTGGAGGCTATGGAGGTGGTGGCAGTGGTGGTGGTGGCCGAGGA

GGATTTCCCAGTGGAGGTGGTGGCGGTGGAGGACAGCAGCGAGCTGGTGACTGGAAGTGTCCTAATCCCACCTGT
GAGAATATGAACTTCTCTTGGAGGAATGAATGCAACCAGTGTAAGGCCCCTAAACCAGATGGCCCAGGAGGGGGA
CCAGGTGGCTCTCACATGGGGGGTAACTACGGGGATGATCGTCGTGGTGGCAGAGGTGGTGCGATCGCAGGCGCC
GATTACAAGGACGATGATGACAAGGGAGCACCAGGAAGTGCTGGTTCTGCTGCTGGTAGTGGAGCGTGCCCGGTG
CCGCTGCAGCTGCCGCCGCTGGAACGCCTGACCCTGGATGATAAAAAACCGCTGAATACCCTGATCTCTGCTACT
GGTCTGTGGATGAGTCGTACCGGAACCATTCATAAAATCAAACACCACGAGGTTAGCCGTTCGAAAATCTATATT
GAGATGGCGTGTGGCGATCATCTGGTTGTGAACAATAGCCGCTCTTCTCGTACAGCACGTGCACTGCGTCACCAC
AAATATCGTAAAACCTGTAAACGTTGCCGTGTGTCCGATGAGGATCTGAACAAATTCCTGACAAAAGCCAATGAG
GACCAAACAAGCGTGAAAGTGAAAGTCGTTAGCGCTCCTACCCGTACTAAAAAAGCAATGCCGAAATCCGTTGCT
CGTGCCCCTAAACCACTGGAAAACACTGAAGCAGCACAGGCACAGCCGTCTGGAAGCAAATTCTCTCCGGCCATT
CCTGTTTCTACCCAGGAGTCCGTTTCTGTTCCAGCAAGTGTGAGCACCAGCATTAGCAGTATTAGCACCGGTGCC
ACCGCTAGCGCCCTGGTTAAAGGCAATACCAATCCGATTACAAGCATGTCTGCCCCGGTTCAAGCATCAGCTCCA
GCACTGACAAAATCCCAAACCGATCGTCTGGAGGTTCTGCTGAATCCGAAAGACGAAATCAGCCTGAATTCCGGC
AAACCGTTTCGTGAACTGGAGAGCGAACTGCTGTCACGTCGTAAAAAAGACCTGCAACAAATCTATGCCGAAGAA
CGTGAGAACTATCTGGGGAAACTGGAACGTGAAATCACCCGCTTTTTCGTGGATCGTGGCTTTCTGGAGATCAAA
TCCCCGATTCTGATTCCTCTGGAGTATATCGAGCGTATGGGCATCGACAATGATACCGAACTGAGCAAACAAATT
TTCCGTGTGGATAAAAACTTCTGTCTGCGCCCTATGCTTTCACCAAATCTGTATAACTATCTGCGCAAACTGGAC
CGTGCCCTGCCTGATCCTATCAAAATCTTCGAGATCGGCCCGTGTTATCGTAAAGAGTCCGACGGTAAAGAACAT
CTGGAGGAGTTTACCATGCTGAACTTTTGCCAAATGGGTTCAGGTTGTACTCGTGAGAACCTGGAAAGCATCATC
ACCGATTTTCTGAACCACCTGGGCATTGACTTCAAAATTGTGGGCGACAGCTGTATGGTGTATGGCGACACCCTG
GATGTCATGCACGGCGACCTGGAACTGTCTAGTGCCGTTGTTGGACCAATTCCGCTGGACCGTGAGTGGGGTATC
GACAAACCGTGGATCGGAGCAGGATTCGGTCTGGAACGCCTGCTGAAAGTGAAACACGACTTCAAAAACATCAAA
CGTGCCGCCCGTTCTGAATCGTATTATAACGGGATCTCTACGAACCTGTAA (SEQ ID NO: 459)

Protein:
MAITQFRLFKFCTCLATVFSFLKRLICRSGAPGSAGSAAGSGMASNDYTQQATQSYGAYPTQPGQGYSQQSSQPY
GQQSYSGYSQSTDTSGYGQSSYSSYGQSQNTGYGTQSTPQGYGSTGGYGSSQSSQSSYGQQSSYPGYGQQPAPSS
TSGSYGSSSQSSSYGQPQSGSYSQQPSYGGQQQSYGQQQSYNPPQGYGQQNQYNSSSGGGGGGGGGNYGQDQSS
MSSGGGSGGGYGNQDQSGGGGSGGYGQQDRGGRGRGGSGGGGGGGGGGYNRSSGGYEPRGRGGGRGGRGGMGGSD
RGGFNKFGGPRDQGSRHDSEQDNSDNNTIFVQGLGENVTIESVADYFKQIGIIKTNKKTGQPMINLYTDRETGKL
KGEATVSFDDPPSAKAAIDWFDGKEFSGNPIKVSFATRRADFNRGGGNGRGGRGRGGPMGRGGYGGGGSGGGGRG
GFPSGGGGGGGGQQRAGDWKCPNPTCENMNFSWRNECNQCKAPKPDGPGGGPGGSHMGGNYGDDRRGGRGGAIAGA
DYKDDDDKGAPGSAGSAAGSGACPVPLQLPPLERLTLDDKKPLNTLISATGLWMSRTGTIHKIKHHEVSRSKIYI
EMACGDHLVVNNSRSSRTARALRHHKYRKTCKRCRVSDEDLNKFLTKANEDQTSVKVKVVSAPTRTKKAMPKSVA
RAPKPLENTEAAQAQPSGSKFSPAIPVSTQESVSVPASVSTSISSISTGATASALVKGNTNPITSMSAPVQASAP
ALTKSQTDRLEVLLNPKDEISLNSGKPFRELESELLSRRKKDLQQIYAEERENYLGKLEREITRFFVDRGFLEIK
SPILIPLEYIERMGIDNDTELSKQIFRVDKNFCLRPMLSPNLYNYLRKLDRALPDPIKIFEIGPCYRKESDGKEH
LEEFTMLNFCQMGSGCTRENLESIITDFLNHLGIDFKIVGDSCMVYGDTLDVMHGDLELSSAVVGPIPLDREWGI
DKPWIGAGFGLERLLKVKHDFKNIKRAARSESYYNGISTNL* (SEQ ID NO: 460)

TOM20::FUS::CbzRS
DNA:
ATGGTGGGTCGGAACAGCGCCATCGCCGCCGGTGTATGCGGGGCCCTTTTCATTGGGTACTGCATCTACTTCGAC
CGCAAAAGACGAAGTGACCCCAACTTCAAGAACAGGCTTCGAGAACGAAGAAAGAAACAGAAGCTTGCCAAGGAG
AGAGCTGGGCTTTCCAAGTTACCTGACCTTAAAGATGCTGAAGCTGTTCAGAAATTCTTCATGGCCTCAAACGAT
TATACCCAACAAGCAACCCAAAGCTATGGGGCCTACCCCACCCAGCCCGGGCAGGGCTATTCCCAGCAGAGCAGT
CAGCCCTACGGACAGCAGAGTTACAGTGGTTATAGCCAGTCCACGGACACTTCAGGATATGGCCAGAGCAGCTAT
TCTTCTTATGGCCAGAGCCAGAACACAGGCTATGGAACTCAGTCAACTCCCCAGGGATATGGCTCGACTGGCGGC
TATGGCAGTAGCCAGAGCTCCCAATCGTCTTACGGGCAGCAGTCCTCCTACCCTGGCTATGGCCAGCAGCCAGCT
CCCAGCAGCACCTCGGGAAGTTACGGTAGCAGTTCTCAGAGCAGCAGCTATGGGCAGCCCCAGAGTGGGAGCTAC
AGCCAGCAGCCTAGCTATGGTGGACAGCAGCAAAGCTATGGACAGCAGCAAAGCTATAATCCCCCTCAGGGCTAT
GGACAGCAGAACCAGTACAACAGCAGCAGTGGTGGTGGAGGTGGAGGTGGAGGTGGAGGTAACTATGGCCAAGAT
CAATCCTCCATGAGTAGTGGTGGTGGCAGTGGTGGCGGTTATGGCAATCAAGACCAGAGTGGTGGAGGTGGCAGC
GGTGGCTATGGACAGCAGGACCGTGGAGGCCGCGGCAGGGGTGGCAGTGGTGGCGGCGGCGGCGGCGGCGGTGGT
GGTTACAACCGCAGCAGTGGTGGCTATGAACCCAGAGGTCGTGGAGGTGGCCGTGGAGGCAGAGGTGGCATGGGC
GGAAGTGACCGTGGTGGCTTCAATAAATTTGGTGGCCCTCGGGACCAAGGATCACGTCATGACTCCGAACAGGAT
AATTCAGACAACAACACCATCTTTGTGCAAGGCCTGGGTGAGAATGTTACAATTGAGTCTGTGGCTGATTACTTC

AAGCAGATTGGTATTATTAAGACAAACAAGAAAACGGGACAGCCCATGATTAATTTGTACACAGACAGGGAAACT
GGCAAGCTGAAGGGAGAGGCAACGGTCTCTTTTGATGACCCACCTTCAGCTAAAGCAGCTATTGACTGGTTTGAT
GGTAAAGAATTCTCCGGAAATCCTATCAAGGTCTCATTTGCTACTCGCCGGGCAGACTTTAATCGGGGTGGTGGC
AATGGTCGTGGAGGCCGAGGGCGAGGAGGACCCATGGGCCGTGGAGGCTATGGAGGTGGTGGCAGTGGTGGTGGT
GGCCGAGGAGGATTTCCCAGTGGAGGTGGTGGCGGTGGAGGACAGCAGCGAGCTGGTGACTGGAAGTGTCCTAAT
CCCACCTGTGAGAATATGAACTTCTCTTGGAGGAATGAATGCAACCAGTGTAAGGCCCCTAAACCAGATGGCCCA
GGAGGGGGACCAGGTGGCTCTCACATGGGGGGTAACTACGGGGATGATCGTCGTGGTGGCAGAGGAGGCGCGATC
GCAGGCAAGCCTATTCCCAACCCCCTGCTGGGCCTGGATAGCACCGGAGCACCAGGAAGTGCTGGTTCTGCTGCT
GGTAGTGGAGCGTGCCCGGTGCCGCTGCAGCTGCCGCCGCTGGAACGCCTGACCCTGGATGATAAAAAACCGCTG
AATACCCTGATCTCTGCTACTGGTCTGTGGATGAGTCGTACCGGAACCATTCATAAAATCAAACACCACGAGGTT
AGCCGTTCGAAAATCTATATTGAGATGGCGTGTGGCGATCATCTGGTTGTGAACAATAGCCGCTCTTCTCGTACA
GCACGTGCACTGCGTCACCACAAATATCGTAAAACCTGTAAACGTTGCCGTGTGTCCGATGAGGATCTGAACAAA
TTCCTGACAAAAGCCAATGAGGACCAAACAAGCGTGAAAGTGAAAGTCGTTAGCGCTCCTACCCGTACTAAAAAA
GCAATGCCGAAATCCGTTGCTCGTGCCCCTAAACCACTGGAAAACACTGAAGCAGCACAGGCACAGCCGTCTGGA
AGCAAATTCTCTCCGGCCATTCCTGTTTCTACCCAGGAGTCCGTTTCTGTTCCAGCAAGTGTGAGCACCAGCATT
AGCAGTATTAGCACCGGTGCCACCGCTAGCGCCCTGGTTAAAGGCAATACCAATCCGATTACAAGCATGTCTGCC
CCGGTTCAAGCATCAGCTCCAGCACTGACAAAATCCCAAACCGATCGTCTGGAGGTTCTGCTGAATCCGAAAGAC
GAAATCAGCCTGAATTCCGGCAAACCGTTTCGTGAACTGGAGAGCGAACTGCTGTCACGTCGTAAAAAAGACCTG
CAACAAATCTATGCCGAAGAACGTGAGAACTATCTGGGGAAACTGGAACGTGAAATCACCCGCTTTTTCGTGGAT
CGTGGCTTTCTGGAGATCAAATCCCCGATTCTGATTCCTCTGGAGTATATCGAGCGTATGGGCATCGACAATGAT
ACCGAACTGAGCAAACAAATTTTCCGTGTGGATAAAAACTTCTGTCTGCGCCCTATGCTTGCACCAAATCTGATG
AACTATGGACGCAAACTGGACCGTGCCCTGCCTGATCCTATCAAAATCTTCGAGATCGGCCCGTGTTATCGTAAA
GAGTCCGACGGTAAAGAACATCTGGAGGAGTTTACCATGCTGAACTTTACACAAATGGGTTCAGGTTGTACTCGT
GAGAACCTGGAAAGCATCATCACCGATTTTCTGAACCACCTGGGCATTGACTTCAAAATTGTGGGCGACAGCTGT
ATGGTGTATGGCGACACCCTGGATGTCATGCACGGCGACCTGGAACTGTCTAGTGCCGTTGTTGGACCAATTCCG
CTGGACCGTGAGTGGGGTATCGACAAACCGTGGATCGGAGCAGGATTCGGTCTGGAACGCCTGCTGAAAGTGAAA
CACGACTTCAAAAACATCAAACGTGCCGCCCGTTCTGAATCGTATTATAACGGGATCTCTACGAACCTGTAA
(SEQ ID NO: 461)

Protein:
MVGRNSAIAAGVCGALFIGYCIYFDRKRRSDPNFKNRLRERRKKQKLAKERAGLSKLPDLKDAEAVQKFFMASND
YTQQATQSYGAYPTQPGQGYSQQSSQPYGQQSYSGYSQSTDTSGYGQSSYSSYGQSQNTGYGTQSTPQGYGSTGG
YGSSQSSQSSYGQQSSYPGYGQQPAPSSTSGSYGSSSQSSSYGQPQSGSYSQQPSYGGQQQSYGQQQSYNPPQGY
GQQNQYNSSSGGGGGGGGGGNYGQDQSSMSSGGGSGGGYGNQDQSGGGGSGGYGQQDRGGRGRGGSGGGGGGGG
GYNRSSGGYEPRGRGGGRGGRGGMGGSDRGGFNKFGGPRDQGSRHDSEQDNSDNNTIFVQGLGENVTIESVADYF
KQIGIIKTNKKTGQPMINLYTDRETGKLKGEATVSFDDPPSAKAAIDWFDGKEFSGNPIKVSFATRRADFNRGGG
NGRGGRGRGGPMGRGGYGGGGSGGGGRGGFPSGGGGGGGQQRAGDWKCPNPTCENMNFSWRNECNQCKAPKPDGP
GGGPGGSHMGGNYGDDRRGGRGGAIAGKPIPNPLLGLDSTGAPGSAGSAAGSGACPVPLQLPPLERLTLDDKKPL
NTLISATGLWMSRTGTIHKIKHHEVSRSKIYIEMACGDHLVVNNSRSSRTARALRHHKYRKTCKRCRVSDEDLNK
FLTKANEDQTSVKVKVVSAPTRTKKAMPKSVARAPKPLENTEAAQAQPSGSKFSPAIPVSTQESVSVPASVSTSI
SSISTGATASALVKGNTNPITSMSAPVQASAPALTKSQTDRLEVLLNPKDEISLNSGKPFRELESELLSRRKKDL
QQIYAEERENYLGKLEREITRFFVDRGFLEIKSPILIPLEYIERMGIDNDTELSKQIFRVDKNFCLRPMLAPNLM
NYGRKLDRALPDPIKIFEIGPCYRKESDGKEHLEEFTMLNFTQMGSGCTRENLESIITDFLNHLGIDFKIVGDSC
MVYGDTLDVMHGDLELSSAVVGPIPLDREWGIDKPWIGAGFGLERLLKVKHDFKNIKRAARSESYYNGISTNL*
(SEQ ID NO: 462)

EBAG9₁₋₂₉::FUS::CbzRS
DNA:
ATGGCCATCACCCAGTTTCGGTTATTTAAATTTTGTACCTGCCTAGCAACAGTATTCTCATTCCTAAAGAGATTA
ATATGCAGATCTGGAGCACCCGGCTCCGCCGGCTCCGCCGCCGGCTCCGGCATGGCCTCAAACGATTATACCCAA
CAAGCAACCCAAAGCTATGGGGCCTACCCCACCCAGCCCGGGCAGGGCTATTCCCAGCAGAGCAGTCAGCCCTAC
GGACAGCAGAGTTACAGTGGTTATAGCCAGTCCACGGACACTTCAGGATATGGCCAGAGCAGCTATTCTTCTTAT
GGCCAGAGCCAGAACACAGGCTATGGAACTCAGTCAACTCCCCAGGGATATGGCTCGACTGGCGGCTATGGCAGT
AGCCAGAGCTCCCAATCGTCTTACGGGCAGCAGTCCTCCTACCCTGGCTATGGCCAGCAGCCAGCTCCCAGCAGC
ACCTCGGGAAGTTACGGTAGCAGTTCTCAGAGCAGCAGCTATGGGCAGCCCCAGAGTGGGAGCTACAGCCAGCAG
CCTAGCTATGGTGGACAGCAGCAAAGCTATGGACAGCAGCAAAGCTATAATCCCCCTCAGGGCTATGGACAGCAG
AACCAGTACAACAGCAGCAGTGGTGGTGGAGGTGGAGGTGGAGGTGGAGGTAACTATGGCCAAGATCAATCCTCC

ATGAGTAGTGGTGGTGGCAGTGGTGGCGGTTATGGCAATCAAGACCAGAGTGGTGGAGGTGGCAGCGGTGGCTAT
GGACAGCAGGACCGTGGAGGCCGCGGCAGGGGTGGCAGTGGTGGCGGCGGCGGCGGCGGTGGTGGTTACAAC
CGCAGCAGTGGTGGCTATGAACCCAGAGGTCGTGGAGGTGGCCGTGGAGGCAGAGGTGGCATGGGCGGAAGTGAC
CGTGGTGGCTTCAATAAATTTGGTGGCCCTCGGGACCAAGGATCACGTCATGACTCCGAACAGGATAATTCAGAC
AACAACACCATCTTTGTGCAAGGCCTGGGTGAGAATGTTACAATTGAGTCTGTGGCTGATTACTTCAAGCAGATT
GGTATTATTAAGACAAACAAGAAAACGGGACAGCCCATGATTAATTTGTACACAGACAGGGAAACTGGCAAGCTG
AAGGGAGAGGCAACGGTCTCTTTTGATGACCCACCTTCAGCTAAAGCAGCTATTGACTGGTTTGATGGTAAAGAA
TTCTCCGGAAATCCTATCAAGGTCTCATTTGCTACTCGCCGGGCAGACTTTAATCGGGGTGGTGGCAATGGTCGT
GGAGGCCGAGGGCGAGGAGGACCCATGGGCCGTGGAGGCTATGGAGGTGGTGGCAGTGGTGGTGGTGGCCGAGGA
GGATTTCCCAGTGGAGGTGGTGGCGGTGGAGGACAGCAGCGAGCTGGTGACTGGAAGTGTCCTAATCCCACCTGT
GAGAATATGAACTTCTCTTGGAGGAATGAATGCAACCAGTGTAAGGCCCCTAAACCAGATGGCCCAGGAGGGGGA
CCAGGTGGCTCTCACATGGGGGGTAACTACGGGGATGATCGTCGTGGTGGCAGAGGTGGTGCGATCGCAGGCGCC
GATTACAAGGACGATGATGACAAGGGAGCACCAGGAAGTGCTGGTTCTGCTGCTGGTAGTGGAGCGTGCCCGGTG
CCGCTGCAGCTGCCGCCGCTGGAACGCCTGACCCTGGATGATAAAAAACCGCTGAATACCCTGATCTCTGCTACT
GGTCTGTGGATGAGTCGTACCGGAACCATTCATAAAATCAAACACCACGAGGTTAGCCGTTCGAAAATCTATATT
GAGATGGCGTGTGGCGATCATCTGGTTGTGAACAATAGCCGCTCTTCTCGTACAGCACGTGCACTGCGTCACCAC
AAATATCGTAAAACCTGTAAACGTTGCCGTGTGTCCGATGAGGATCTGAACAAATTCCTGACAAAAGCCAATGAG
GACCAAACAAGCGTGAAAGTGAAAGTCGTTAGCGCTCCTACCCGTACTAAAAAAGCAATGCCGAAATCCGTTGCT
CGTGCCCCTAAACCACTGGAAAACACTGAAGCAGCACAGGCACAGCCGTCTGGAAGCAAATTCTCTCCGGCCATT
CCTGTTTCTACCCAGGAGTCCGTTTCTGTTCCAGCAAGTGTGAGCACCAGCATTAGCAGTATTAGCACCGGTGCC
ACCGCTAGCGCCCTGGTTAAAGGCAATACCAATCCGATTACAAGCATGTCTGCCCCGGTTCAAGCATCAGCTCCA
GCACTGACAAAATCCCAAACCGATCGTCTGGAGGTTCTGCTGAATCCGAAAGACGAAATCAGCCTGAATTCCGGC
AAACCGTTTCGTGAACTGGAGAGCGAACTGCTGTCACGTCGTAAAAAAGACCTGCAACAAATCTATGCCGAAGAA
CGTGAGAACTATCTGGGGAAACTGGAACGTGAAATCACCCGCTTTTTCGTGGATCGTGGCTTTCTGGAGATCAAA
TCCCCGATTCTGATTCCTCTGGAGTATATCGAGCGTATGGGCATCGACAATGATACCGAACTGAGCAAACAAATT
TTCCGTGTGGATAAAAACTTCTGTCTGCGCCCTATGCTTGCACCAAATCTGATGAACTATGGACGCAAACTGGAC
CGTGCCCTGCCTGATCCTATCAAAATCTTCGAGATCGGCCCGTGTTATCGTAAAGAGTCCGACGGTAAAGAACAT
CTGGAGGAGTTTACCATGCTGAACTTTACACAAATGGGTTCAGGTTGTACTCGTGAGAACCTGGAAAGCATCATC
ACCGATTTTCTGAACCACCTGGGCATTGACTTCAAAATTGTGGGCGACAGCTGTATGGTGTATGGCGACACCCTG
GATGTCATGCACGGCGACCTGGAACTGTCTAGTGCCGTTGTTGGACCAATTCCGCTGGACCGTGAGTGGGGTATC
GACAAACCGTGGATCGGAGCAGGATTCGGTCTGGAACGCCTGCTGAAAGTGAAACACGACTTCAAAAACATCAAA
CGTGCCGCCCGTTCTGAATCGTATTATAACGGGATCTCTACGAACCTGTAA (SEQ ID NO: 463)

Protein:
MAITQFRLFKFCTCLATVFSFLKRLICRSGAPGSAGSAAGSGMASNDYTQQATQSYGAYPTQPGQGYSQQSSQPY
GQQSYSGYSQSTDTSGYGQSSYSSYGQSQNTGYGTQSTPQGYGSTGGYGSSQSSQSSYGQQSSYPGYGQQPAPSS
TSGSYGSSSQSSSYGQPQSGSYSQQPSYGGQQQSYGQQQSYNPPQGYGQQNQYNSSSGGGGGGGGGNYGQDQSS
MSSGGGSGGGYGNQDQSGGGGSGGYGQQDRGGRGRGGSGGGGGGGGGGYNRSSGGYEPRGRGGGRGGRGGMGGSD
RGGFNKFGGPRDQGSRHDSEQDNSDNNTIFVQGLGENVTIESVADYFKQIGIIKTNKKTGQPMINLYTDRETGKL
KGEATVSFDDPPSAKAAIDWFDGKEFSGNPIKVSFATRRADFNRGGGNGRGGRGRGGPMGRGGYGGGGSGGGGRG
GFPSGGGGGGGQQRAGDWKCPNPTCENMNFSWRNECNQCKAPKPDGPGGGPGGSHMGGNYGDDRRGGRGGAIAGA
DYKDDDDKGAPGSAGSAAGSGACPVPLQLPPLERLTLDDKKPLNTLISATGLWMSRTGTIHKIKHHEVSRSKIYI
EMACGDHLVVNNSRSSRTARALRHHKYRKTCKRCRVSDEDLNKFLTKANEDQTSVKVKVVSAPTRTKKAMPKSVA
RAPKPLENTEAAQAQPSGSKFSPAIPVSTQESVSVPASVSTSISSISTGATASALVKGNTNPITSMSAPVQASAP
ALTKSQTDRLEVLLNPKDEISLNSGKPFRELESELLSRRKKDLQQIYAEERENYLGKLEREITRFFVDRGFLEIK
SPILIPLEYIERMGIDNDTELSKQIFRVDKNFCLRPMLAPNLMNYGRKLDRALPDPIKIFEIGPCYRKESDGKEH
LEEFTMLNFTQMGSGCTRENLESIITDFLNHLGIDFKIVGDSCMVYGDTLDVMHGDLELSSAVVGPIPLDREWGI
DKPWIGAGFGLERLLKVKHDFKNIKRAARSESYYNGISTNL* (SEQ ID NO: 464)

TOM20::FUS::SYNZIP1::CbzRS
DNA:
ATGGTGGGTCGGAACAGCGCCATCGCCGCCGGTGTATGCGGGGCCCTTTTCATTGGGTACTGCATCTACTTCGAC
CGCAAAAGACGAAGTGACCCCAACTTCAAGAACAGGCTTCGAGAACGAAGAAAGAAACAGAAGCTTGCCAAGGAG
AGAGCTGGGCTTTCCAAGTTACCTGACCTTAAAGATGCTGAAGCTGTTCAGAAATTCTTCATGGCCTCAAACGAT
TATACCCAACAAGCAACCCAAAGCTATGGGGCCTACCCCACCCAGCCCGGGCAGGGCTATTCCCAGCAGAGCAGT
CAGCCCTACGGACAGCAGAGTTACAGTGGTTATAGCCAGTCCACGGACACTTCAGGATATGGCCAGAGCAGCTAT
TCTTCTTATGGCCAGAGCCAGAACACAGGCTATGGAACTCAGTCAACTCCCCAGGGATATGGCTCGACTGGCGGC

TATGGCAGTAGCCAGAGCTCCCAATCGTCTTACGGGCAGCAGTCCTCCTACCCTGGCTATGGCCAGCAGCCAGCT
CCCAGCAGCACCTCGGGAAGTTACGGTAGCAGTTCTCAGAGCAGCAGCTATGGGCAGCCCCAGAGTGGGAGCTAC
AGCCAGCAGCCTAGCTATGGTGGACAGCAGCAAAGCTATGGACAGCAGCAAAGCTATAATCCCCCTCAGGGCTAT
GGACAGCAGAACCAGTACAACAGCAGCAGTGGTGGTGGAGGTGGAGGTGGAGGTGGAGGTAACTATGGCCAAGAT
CAATCCTCCATGAGTAGTGGTGGTGGCAGTGGTGGCGGTTATGGCAATCAAGACCAGAGTGGTGGAGGTGGCAGC
GGTGGCTATGGACAGCAGGACCGTGGAGGCCGCGGCAGGGGTGGCAGTGGTGGCGGCGGCGGCGGCGGCGGTGGT
GGTTACAACCGCAGCAGTGGTGGCTATGAACCCAGAGGTCGTGGAGGTGGCCGTGGAGGCAGAGGTGGCATGGGC
GGAAGTGACCGTGGTGGCTTCAATAAATTTGGTGGCCCTCGGGACCAAGGATCACGTCATGACTCCGAACAGGAT
AATTCAGACAACAACACCATCTTTGTGCAAGGCCTGGGTGAGAATGTTACAATTGAGTCTGTGGCTGATTACTTC
AAGCAGATTGGTATTATTAAGACAAACAAGAAAACGGGACAGCCCATGATTAATTTGTACACAGACAGGGAAACT
GGCAAGCTGAAGGGAGAGGCAACGGTCTCTTTTGATGACCCACCTTCAGCTAAAGCAGCTATTGACTGGTTTGAT
GGTAAAGAATTCTCCGGAAATCCTATCAAGGTCTCATTTGCTACTCGCCGGGCAGACTTTAATCGGGGTGGTGGC
AATGGTCGTGGAGGCCGAGGGCGAGGAGGACCCATGGGCCGTGGAGGCTATGGAGGTGGTGGCAGTGGTGGTGGT
GGCCGAGGAGGATTTCCCAGTGGAGGTGGTGGCGGTGGAGGACAGCAGCGAGCTGGTGACTGGAAGTGTCCTAAT
CCCACCTGTGAGAATATGAACTTCTCTTGGAGGAATGAATGCAACCAGTGTAAGGCCCCTAAACCAGATGGCCCA
GGAGGGGGACCAGGTGGCTCTCACATGGGGGGTAACTACGGGGATGATCGTCGTGGTGGCAGAGGAGGCGCGATC
GCAAATCTGGTGGCCCAGCTGGAAAACGAGGTGGCCAGCCTGGAAAACGAGAACGAAACCCTGAAGAAAAAGAAC
CTGCACAAGAAGGACCTGATCGCCTACCTGGAAAAGGAAATCGCCAACCTGAGAAAGAAGATCGAGGAAGGCAAG
CCTATTCCCAACCCCCTGCTGGGCCTGGATAGCACCGGAGCACCAGGAAGTGCTGGTTCTGCTGCTGGTAGTGGA
GCGTGCCCGGTGCCGCTGCAGCTGCCGCCGCTGGAACGCCTGACCCTGGATGATAAAAAACCGCTGAATACCCTG
ATCTCTGCTACTGGTCTGTGGATGAGTCGTACCGGAACCATTCATAAAATCAAACACCACGAGGTTAGCCGTTCG
AAAATCTATATTGAGATGGCGTGTGGCGATCATCTGGTTGTGAACAATAGCCGCTCTTCTCGTACAGCACGTGCA
CTGCGTCACCACAAATATCGTAAAACCTGTAAACGTTGCCGTGTGTCCGATGAGGATCTGAACAAATTCCTGACA
AAAGCCAATGAGGACCAAACAAGCGTGAAAGTGAAAGTCGTTAGCGCTCCTACCCGTACTAAAAAAGCAATGCCG
AAATCCGTTGCTCGTGCCCCTAAACCACTGGAAAACACTGAAGCAGCACAGGCACAGCCGTCTGGAAGCAAATTC
TCTCCGGCCATTCCTGTTTCTACCCAGGAGTCCGTTTCTGTTCCAGCAAGTGTGAGCACCAGCATTAGCAGTATT
AGCACCGGTGCCACCGCTAGCGCCCTGGTTAAAGGCAATACCAATCCGATTACAAGCATGTCTGCCCCGGTTCAA
GCATCAGCTCCAGCACTGACAAAATCCCAAACCGATCGTCTGGAGGTTCTGCTGAATCCGAAAGACGAAATCAGC
CTGAATTCCGGCAAACCGTTTCGTGAACTGGAGAGCGAACTGCTGTCACGTCGTAAAAAAGACCTGCAACAAATC
TATGCCGAAGAACGTGAGAACTATCTGGGGAAACTGGAACGTGAAATCACCCGCTTTTTCGTGGATCGTGGCTTT
CTGGAGATCAAATCCCCGATTCTGATTCCTCTGGAGTATATCGAGCGTATGGGCATCGACAATGATACCGAACTG
AGCAAACAAATTTTCCGTGTGGATAAAAACTTCTGTCTGCGCCCTATGCTTGCACCAAATCTGATGAACTATGGA
CGCAAACTGGACCGTGCCCTGCCTGATCCTATCAAAATCTTCGAGATCGGCCCGTGTTATCGTAAAGAGTCCGAC
GGTAAAGAACATCTGGAGGAGTTTACCATGCTGAACTTTACACAAATGGGTTCAGGTTGTACTCGTGAGAACCTG
GAAAGCATCATCACCGATTTTCTGAACCACCTGGGCATTGACTTCAAAATTGTGGGCGACAGCTGTATGGTGTAT
GGCGACACCCTGGATGTCATGCACGGCGACCTGGAACTGTCTAGTGCCGTTGTTGGACCAATTCCGCTGGACCGT
GAGTGGGGTATCGACAAACCGTGGATCGGAGCAGGATTCGGTCTGGAACGCCTGCTGAAAGTGAAACACGACTTC
AAAAACATCAAACGTGCCGCCCGTTCTGAATCGTATTATAACGGGATCTCTACGAACCTGTAA (SEQ ID NO:
465)

Protein:
MVGRNSAIAAGVCGALFIGYCIYFDRKRRSDPNFKNRLRERRKKQKLAKERAGLSKLPDLKDAEAVQKFFMASND
YTQQATQSYGAYPTQPGQGYSQQSSQPYGQQSYSGYSQSTDTSGYGQSSYSSYGQSQNTGYGTQSTPQGYGSTGG
YGSSQSSQSSYGQQSSYPGYGQQPAPSSTSGSYGSSSQSSSYGQPQSGSYSQQPSYGGQQQSYGQQQSYNPPQGY
GQQNQYNSSSGGGGGGGGGNYGQDQSSMSSGGGSGGGYGNQDQSGGGGSGGYGQQDRGGRGRGGSGGGGGGGG
GYNRSSGGYEPRGRGGGRGGRGGMGGSDRGGFNKFGGPRDQGSRHDSEQDNSDNNTIFVQGLGENVTIESVADYF
KQIGIIKTNKKTGQPMINLYTDRETGKLKGEATVSFDDPPSAKAAIDWFDGKEFSGNPIKVSFATRRADFNRGGG
NGRGGRGRGGPMGRGGYGGGGSGGGGRGGFPSGGGGGGGQQRAGDWKCPNPTCENMNFSWRNECNQCKAPKPDGP
GGGPGGSHMGGNYGDDRRGGRGGAIANLVAQLENEVASLENENETLKKKNLHKKDLIAYLEKEIANLRKKIEEGK
PIPNPLLGLDSTGAPGSAGSAAGSGACPVPLQLPPLERLTLDDKKPLNTLISATGLWMSRTGTIHKIKHHEVSRS
KIYIEMACGDHLVVNNSRSSRTARALRHHKYRKTCKRCRVSDEDLNKFLTKANEDQTSVKVKVVSAPTRTKKAMP
KSVARAPKPLENTEAAQAQPSGSKFSPAIPVSTQESVSVPASVSTSISSISTGATASALVKGNTNPITSMSAPVQ
ASAPALTKSQTDRLEVLLNPKDEISLNSGKPFRELESELLSRRKKDLQQIYAEERENYLGKLEREITRFFVDRGF
LEIKSPILIPLEYIERMGIDNDTELSKQIFRVDKNFCLRPMLAPNLMNYGRKLDRALPDPIKIFEIGPCYRKESD
GKEHLEEFTMLNFTQMGSGCTRENLESIITDFLNHLGIDFKIVGDSCMVYGDTLDVMHGDLELSSAVVGPIPLDR
EWGIDKPWIGAGFGLERLLKVKHDFKNIKRAARSESYYNGISTNL* (SEQ ID NO: 466)

KIF16B::FUS::CpkRS
DNA:
ATGGCATCGGTCAAGGTGGCCGTGAGGGTCCGGCCCATGAATCGCAGGGAAAAGGACTTGGAGGCCAAGTTCATT
ATTCAGATGGAGAAAAGCAAAACGACAATCACAAACTTAAAGATACCAGAAGGAGGCACTGGGGACTCAGGAAGA
GAACGGACCAAGACCTTCACCTATGACTTTTCTTTTTATTCTGCTGATACAAAAAGCCCAGATTACGTTTCACAA
GAAATGGTTTTCAAAACCCTCGGCACAGATGTCGTGAAGTCTGCATTTGAAGGTTATAATGCTTGTGTCTTTGCA
TATGGGCAAACTGGATCTGGAAAGTCATACACTATGATGGGAAATTCTGGAGATTCTGGCTTAATACCTCGGATC
TGTGAAGGACTCTTCAGTCGGATAAATGAAACCACCAGATGGGATGAAGCTTCTTTTCGAACTGAAGTCAGCTAC
TTAGAAATTTATAACGAACGTGTGAGAGATCTACTTCGGCGGAAGTCATCTAAAACCTTCAATTTGAGAGTCCGT
GAGCATCCCAAAGAAGGCCCTTATGTTGAGGATTTATCCAAACATTTAGTACAGAATTATGGTGACGTAGAAGAA
CTTATGGATGCGGGCAATATCAACCGGACCACCGCAGCGACTGGGATGAACGACGTCAGTAGCAGGTCTCATGCC
ATCTTCACCATCAAGTTCACTCAGGCTAAATTTGATTCTGAAATGCCATGTGAAACCGTCAGTAAGATCCACTTG
GTTGATCTTGCCGGAAGTGAGCGTGCAGATGCCACCGGAGCCACCGGGGTTAGGCTAAAGGAAGGGGGAAATATT
AACAAGTCCCTCGTGACTCTGGGGAACGTCATTTCTGCCTTAGCTGATTTATCTCAGGATGCTGCAAATACTCTT
GCAAAGAAGAAGCAAGTTTTCGTGCCTTACAGGGATTCTGTGTTGACTTGGTTGTTAAAAGATAGCCTTGGAGGA
AACTCTAAAACTATCATGATTGCCACCATTTCACCTGCTGATGTCAATTATGGAGAAACCCTAAGTACTCTTCGC
TATGCAAATAGAGCCAAAAACATCATCAACAAGCCTACCATTAATGAGGATGCCAACGTCAAACTTATCCGTGAG
CTGCGAGCTGAAATAGCCAGACTGAAAACGCTGCTTGCTCAAGGGAATCAGATTGCCCTCTTAGACTCCCCCACA
TATACAGATATTGAAATGAACAGATTGGGAAAGGGCGCCCCCGGCTCCGCCGGCTCCGCCGCCGGCTCCGGCATG
GCCTCAAACGATTATACCCAACAAGCAACCCAAAGCTATGGGGCCTACCCCACCCAGCCCGGGCAGGGCTATTCC
CAGCAGAGCAGTCAGCCCTACGGACAGCAGAGTTACAGTGGTTATAGCCAGTCCACGGACACTTCAGGATATGGC
CAGAGCAGCTATTCTTCTTATGGCCAGAGCCAGAACACAGGCTATGGAACTCAGTCAACTCCCCAGGGATATGGC
TCGACTGGCGGCTATGGCAGTAGCCAGAGCTCCCAATCGTCTTACGGGCAGCAGTCCTCCTACCCTGGCTATGGC
CAGCAGCCAGCTCCCAGCAGCACCTCGGGAAGTTACGGTAGCAGTTCTCAGAGCAGCAGCTATGGGCAGCCCCAG
AGTGGGAGCTACAGCCAGCAGCCTAGCTATGGTGGACAGCAGCAAAGCTATGGACAGCAGCAAAGCTATAATCCC
CCTCAGGGCTATGGACAGCAGAACCAGTACAACAGCAGCAGTGGTGGTGGAGGTGGAGGTGGAGGTGGAGGTAAC
TATGGCCAAGATCAATCCTCCATGAGTAGTGGTGGTGGCAGTGGTGGCGGTTATGGCAATCAAGACCAGAGTGGT
GGAGGTGGCAGCGGTGGCTATGGACAGCAGGACCGTGGAGGCCGCGGCAGGGGTGGCAGTGGTGGCGGCGGCGGC
GGCGGCGGTGGTGGTTACAACCGCAGCAGTGGTGGCTATGAACCCAGAGGTCGTGGAGGTGGCCGTGGAGGCAGA
GGTGGCATGGGCGGAAGTGACCGTGGTGGCTTCAATAAATTTGGTGGCCCTCGGGACCAAGGATCACGTCATGAC
TCCGAACAGGATAATTCAGACAACAACACCATCTTTGTGCAAGGCCTGGGTGAGAATGTTACAATTGAGTCTGTG
GCTGATTACTTCAAGCAGATTGGTATTATTAAGACAAACAAGAAAACGGGACAGCCCATGATTAATTTGTACACA
GACAGGGAAACTGGCAAGCTGAAGGGAGAGGCAACGGTCTCTTTTGATGACCCACCTTCAGCTAAAGCAGCTATT
GACTGGTTTGATGGTAAAGAATTCTCCGGAAATCCTATCAAGGTCTCATTTGCTACTCGCCGGGCAGACTTTAAT
CGGGGTGGTGGCAATGGTCGTGGAGGCCGAGGGCGAGGAGGACCCATGGGCCGTGGAGGCTATGGAGGTGGTGGC
AGTGGTGGTGGTGGCCGAGGAGGATTTCCCAGTGGAGGTGGTGGCGGTGGAGGACAGCAGCGAGCTGGTGACTGG
AAGTGTCCTAATCCCACCTGTGAGAATATGAACTTCTCTTGGAGGAATGAATGCAACCAGTGTAAGGCCCCTAAA
CCAGATGGCCCAGGAGGGGGACCAGGTGGCTCTCACATGGGGGGTAACTACGGGGATGATCGTCGTGGTGGCAGA
GGAGGCGATTACAAGGATGACGACGATAAGGGTACCGGCGCCCCCGGCTCCGCCGGCTCCGCCGCCGGCTCCGGC
GCGTGCCCGGTGCCGCTGCAGCTGCCGCCGCTGGAACGCCTGACCCTGGATGATAAAAAACCGCTGAATACCCTG
ATCTCTGCTACTGGTCTGTGGATGAGTCGTACCGGAACCATTCATAAAATCAAACACCACGAGGTTAGCCGTTCG
AAAATCTATATTGAGATGGCGTGTGGCGATCATCTGGTTGTGAACAATAGCCGCTCTTCTCGTACAGCACGTGCA
CTGCGTCACCACAAATATCGTAAAACCTGTAAACGTTGCCGTGTGTCCGATGAGGATCTGAACAAATTCCTGACA
AAAGCCAATGAGGACCAAACAAGCGTGAAAGTGAAAGTCGTTAGCGCTCCTACCCGTACTAAAAAAGCAATGCCG
AAATCCGTTGCTCGTGCCCCTAAACCACTGGAAAACACTGAAGCAGCACAGGCACAGCCGTCTGGAAGCAAATTC
TCTCCGGCCATTCCTGTTTCTACCCAGGAGTCCGTTTCTGTTCCAGCAAGTGTGAGCACCAGCATTAGCAGTATT
AGCACCGGTGCCACCGCTAGCGCCCTGGTTAAAGGCAATACCAATCCGATTACAAGCATGTCTGCCCCGGTTCAA
GCATCAGCTCCAGCACTGACAAAATCCCAAACCGATCGTCTGGAGGTTCTGCTGAATCCGAAAGACGAAATCAGC
CTGAATTCCGGCAAACCGTTTCGTGAACTGGAGAGCGAACTGCTGTCACGTCGTAAAAAAGACCTGCAACAAATC
TATGCCGAAGAACGTGAGAACTATCTGGGGAAACTGGAACGTGAAATCACCCGCTTTTTCGTGGATCGTGGCTTT
CTGGAGATCAAATCCCCGATTCTGATTCCTCTGGAGTATATCGAGCGTATGGGCATCGACAATGATACCGAACTG
AGCAAACAAATTTTCCGTGTGGATAAAAACTTCTGTCTGCGCCCTATGCTTTCACCAAATCTGTATAACTATCTG
CGCAAACTGGACCGTGCCCTGCCTGATCCTATCAAAATCTTCGAGATCGGCCCGTGTTATCGTAAAGAGTCCGAC
GGTAAAGAACATCTGGAGGAGTTTACCATGCTGAACTTTTGCCAAATGGGTTCAGGTTGTACTCGTGAGAACCTG
GAAAGCATCATCACCGATTTTCTGAACCACCTGGGCATTGACTTCAAAATTGTGGGCGACAGCTGTATGGTGTAT
GGCGACACCCTGGATGTCATGCACGGCGACCTGGAACTGTCTAGTGCCGTTGTTGGACCAATTCCGCTGGACCGT
GAGTGGGGTATCGACAAACCGTGGATCGGAGCAGGATTCGGTCTGGAACGCCTGCTGAAAGTGAAACACGACTTC

AAAAACATCAAACGTGCCGCCCGTTCTGAATCGTATTATAACGGGATCTCTACGAACCTGTAA (SEQ ID NO: 467)

Protein:
MASVKVAVRVRPMNRREKDLEAKFIIQMEKSKTTITNLKIPEGGTGDSGRERTKTFTYDFSFYSADTKSPDYVSQ
EMVFKTLGTDVVKSAFEGYNACVFAYGQTGSGKSYTMMGNSGDSGLIPRICEGLFSRINETTRWDEASFRTEVSY
LEIYNERVRDLLRRKSSKTFNLRVREHPKEGPYVEDLSKHLVQNYGDVEELMDAGNINRTTAATGMNDVSSRSHA
IFTIKFTQAKFDSEMPCETVSKIHLVDLAGSERADATGATGVRLKEGGNINKSLVTLGNVISALADLSQDAANTL
AKKKQVFVPYRDSVLTWLLKDSLGGNSKTIMIATISPADVNYGETLSTLRYANRAKNIINKPTINEDANVKLIRE
LRAEIARLKTLLAQGNQIALLDSPTYTDIEMNRLGKGAPGSAGSAAGSGMASNDYTQQATQSYGAYPTQPGQGYS
QQSSQPYGQQSYSGYSQSTDTSGYGQSSYSSYGQSQNTGYGTQSTPQGYGSTGGYGSSQSSQSSYGQQSSYPGYG
QQPAPSSTSGSYGSSSQSSSYGQPQSGSYSQQPSYGGQQQSYGQQQSYNPPQGYGQQNQYNSSSGGGGGGGGGGN
YGQDQSSMSSGGGSGGGYGNQDQSGGGGSGGYGQQDRGGRGRGGSGGGGGGGGGGYNRSSGGYEPRGRGGGRGGR
GGMGGSDRGGFNKFGGPRDQGSRHDSEQDNSDNNTIFVQGLGENVTIESVADYFKQIGIIKTNKKTGQPMINLYT
DRETGKLKGEATVSFDDPPSAKAAIDWFDGKEFSGNPIKVSFATRRADFNRGGGNGRGGRGRGGPMGRGGYGGGG
SGGGGRGGFPSGGGGGGGQQRAGDWKCPNPTCENMNFSWRNECNQCKAPKPDGPGGGPGGSHMGGNYGDDRRGGR
GGDYKDDDDKGTGAPGSAGSAAGSGACPVPLQLPPLERLTLDDKKPLNTLISATGLWMSRTGTIHKIKHHEVSRS
KIYIEMACGDHLVVNNSRSSRTARALRHHKYRKTCKRCRVSDEDLNKFLTKANEDQTSVKVKVVSAPTRTKKAMP
KSVARAPKPLENTEAAQAQPSGSKFSPAIPVSTQESVSVPASVSTSISSISTGATASALVKGNTNPITSMSAPVQ
ASAPALTKSQTDRLEVLLNPKDEISLNSGKPFRELESELLSRRKKDLQQIYAEERENYLGKLEREITRFFVDRGF
LEIKSPILIPLEYIERMGIDNDTELSKQIFRVDKNFCLRPMLSPNLYNYLRKLDRALPDPIKIFEIGPCYRKESD
GKEHLEEFTMLNFCQMGSGCTRENLESIITDFLNHLGIDFKIVGDSCMVYGDTLDVMHGDLELSSAVVGPIPLDR
EWGIDKPWIGAGFGLERLLKVKHDFKNIKRAARSESYYNGISTNL* (SEQ ID NO: 468)

LCK::FUS::CpkRS
DNA:
ATGGGCTGCGTGTGCAGCAGCAACCCCGAGGGTACCGAGCTCGCCTCAAACGATTATACCCAACAAGCAACCCAA
AGCTATGGGGCCTACCCCACCCAGCCCGGGCAGGGCTATTCCCAGCAGAGCAGTCAGCCCTACGGACAGCAGAGT
TACAGTGGTTATAGCCAGTCCACGGACACTTCAGGATATGGCCAGAGCAGCTATTCTTCTTATGGCCAGAGCCAG
AACACAGGCTATGGAACTCAGTCAACTCCCCAGGGATATGGCTCGACTGGCGGCTATGGCAGTAGCCAGAGCTCC
CAATCGTCTTACGGGCAGCAGTCCTCCTACCCTGGCTATGGCCAGCAGCCAGCTCCCAGCAGCACCTCGGGAAGT
TACGGTAGCAGTTCTCAGAGCAGCAGCTATGGGCAGCCCCAGAGTGGGAGCTACAGCCAGCAGCCTAGCTATGGT
GGACAGCAGCAAAGCTATGGACAGCAGCAAAGCTATAATCCCCCTCAGGGCTATGGACAGCAGAACCAGTACAAC
AGCAGCAGTGGTGGTGGAGGTGGAGGTGGAGGTGGAGGTAACTATGGCCAAGATCAATCCTCCATGAGTAGTGGT
GGTGGCAGTGGTGGCGGTTATGGCAATCAAGACCAGAGTGGTGGAGGTGGCAGCGGTGGCTATGGACAGCAGGAC
CGTGGAGGCCGCGGCAGGGGTGGCAGTGGTGGCGGCGGCGGCGGCGGTGGTGGTTACAACCGCAGCAGTGGT
GGCTATGAACCCAGAGGTCGTGGAGGTGGCCGTGGAGGCAGAGGTGGCATGGGCGGAAGTGACCGTGGTGGCTTC
AATAAATTTGGTGGCCCTCGGGACCAAGGATCACGTCATGACTCCGAACAGGATAATTCAGACAACAACACCATC
TTTGTGCAAGGCCTGGGTGAGAATGTTACAATTGAGTCTGTGGCTGATTACTTCAAGCAGATTGGTATTATTAAG
ACAAACAAGAAAACGGGACAGCCCATGATTAATTTGTACACAGACAGGGAAACTGGCAAGCTGAAGGGAGAGGCA
ACGGTCTCTTTTGATGACCCACCTTCAGCTAAAGCAGCTATTGACTGGTTTGATGGTAAAGAATTCTCCGGAAAT
CCTATCAAGGTCTCATTTGCTACTCGCCGGGCAGACTTTAATCGGGGTGGTGGCAATGGTCGTGGAGGCCGAGGG
CGAGGAGGACCCATGGGCCGTGGAGGCTATGGAGGTGGTGGCAGTGGTGGTGGTGGCCGAGGAGGATTTCCCAGT
GGAGGTGGTGGCGGTGGAGGACAGCAGCGAGCTGGTGACTGGAAGTGTCCTAATCCCACCTGTGAGAATATGAAC
TTCTCTTGGAGGAATGAATGCAACCAGTGTAAGGCCCCTAAACCAGATGGCCCAGGAGGGGGACCAGGTGGCTCT
CACATGGGGGGTAACTACGGGGATGATCGTCGTGGTGGCAGAGGAGGCGGCGCCCCCGGCTCCGCCGGCTCCGCC
GCCGGCTCCGGCATGGCGTGCCCGGTGCCGCTGCAGCTGCCGCCGCTGGAACGCCTGACCCTGGATGATAAAAAA
CCGCTGAATACCCTGATCTCTGCTACTGGTCTGTGGATGAGTCGTACCGGAACCATTCATAAAATCAAACACCAC
GAGGTTAGCCGTTCGAAAATCTATATTGAGATGGCGTGTGGCGATCATCTGGTTGTGAACAATAGCCGCTCTTCT
CGTACAGCACGTGCACTGCGTCACCACAAATATCGTAAAACCTGTAAACGTTGCCGTGTGTCCGATGAGGATCTG
AACAAATTCCTGACAAAAGCCAATGAGGACCAAACAAGCGTGAAAGTGAAAGTCGTTAGCGCTCCTACCCGTACT
AAAAAAGCAATGCCGAAATCCGTTGCTCGTGCCCCTAAACCACTGGAAAACACTGAAGCAGCACAGGCACAGCCG
TCTGGAAGCAAATTCTCTCCGGCCATTCCTGTTTCTACCCAGGAGTCCGTTTCTGTTCCAGCAAGTGTGAGCACC
AGCATTAGCAGTATTAGCACCGGTGCCACCGCTAGCGCCCTGGTTAAAGGCAATACCAATCCGATTACAAGCATG
TCTGCCCCGGTTCAAGCATCAGCTCCAGCACTGACAAAATCCCAAACCGATCGTCTGGAGGTTCTGCTGAATCCG
AAAGACGAAATCAGCCTGAATTCCGGCAAACCGTTTCGTGAACTGGAGAGCGAACTGCTGTCACGTCGTAAAAAA
GACCTGCAACAAATCTATGCCGAAGAACGTGAGAACTATCTGGGGAAACTGGAACGTGAAATCACCCGCTTTTTC

GTGGATCGTGGCTTTCTGGAGATCAAATCCCCGATTCTGATTCCTCTGGAGTATATCGAGCGTATGGGCATCGAC
AATGATACCGAACTGAGCAAACAAATTTTCCGTGTGGATAAAAACTTCTGTCTGCGCCCTATGCTTTCACCAAAT
CTGTATAACTATCTGCGCAAACTGGACCGTGCCCTGCCTGATCCTATCAAAATCTTCGAGATCGGCCCGTGTTAT
CGTAAAGAGTCCGACGGTAAAGAACATCTGGAGGAGTTTACCATGCTGAACTTTTGCCAAATGGGTTCAGGTTGT
ACTCGTGAGAACCTGGAAAGCATCATCACCGATTTTCTGAACCACCTGGGCATTGACTTCAAAATTGTGGGCGAC
AGCTGTATGGTGTATGGCGACACCCTGGATGTCATGCACGGCGACCTGGAACTGTCTAGTGCCGTTGTTGGACCA
ATTCCGCTGGACCGTGAGTGGGGTATCGACAAACCGTGGATCGGAGCAGGATTCGGTCTGGAACGCCTGCTGAAA
GTGAAACACGACTTCAAAAACATCAAACGTGCCGCCCGTTCTGAATCGTATTATAACGGGATCTCTACGAACCTG
TAA (SEQ ID NO: 469)

Protein:
MGCVCSSNPEGTELASNDYTQQATQSYGAYPTQPGQGYSQQSSQPYGQQSYSGYSQSTDTSGYGQSSYSSYGQSQ
NTGYGTQSTPQGYGSTGGYGSSQSSQSSYGQQSSYPGYGQQPAPSSTSGSYGSSSQSSSYGQPQSGSYSQQPSYG
GQQQSYGQQQSYNPPQGYGQQNQYNSSSGGGGGGGGGGNYGQDQSSMSSGGGSGGGYGNQDQSGGGGSGGYGQQD
RGGRGRGGSGGGGGGGGGGYNRSSGGYEPRGRGGGRGGRGGMGGSDRGGFNKFGGPRDQGSRHDSEQDNSDNNTI
FVQGLGENVTIESVADYFKQIGIIKTNKKTGQPMINLYTDRETGKLKGEATVSFDDPPSAKAAIDWFDGKEFSGN
PIKVSFATRRADFNRGGGNGRGGRGRGGPMGRGGYGGGGSGGGGRGGFPSGGGGGGGQQRAGDWKCPNPTCENMN
FSWRNECNQCKAPKPDGPGGGPGGSHMGGNYGDDRRGGRGGGAPGSAGSAAGSGMACPVPLQLPPLERLTLDDKK
PLNTLISATGLWMSRTGTIHKIKHHEVSRSKIYIEMACGDHLVVNNSRSSRTARALRHHKYRKTCKRCRVSDEDL
NKFLTKANEDQTSVKVKVVSAPTRTKKAMPKSVARAPKPLENTEAAQAQPSGSKFSPAIPVSTQESVSVPASVST
SISSISTGATASALVKGNTNPITSMSAPVQASAPALTKSQTDRLEVLLNPKDEISLNSGKPFRELESELLSRRKK
DLQQIYAEERENYLGKLEREITRFFVDRGFLEIKSPILIPLEYIERMGIDNDTELSKQIFRVDKNFCLRPMLSPN
LYNYLRKLDRALPDPIKIFEIGPCYRKESDGKEHLEEFTMLNFCQMGSGCTRENLESIITDFLNHLGIDFKIVGD
SCMVYGDTLDVMHGDLELSSAVVGPIPLDREWGIDKPWIGAGFGLERLLKVKHDFKNIKRAARSESYYNGISTNL
* (SEQ ID NO: 470)

LCK::CpkRS
DNA:
ATGGGCTGCGTGTGCAGCAGCAACCCCGAGGGTACCGAGCTCGCGTGCCCGGTGCCGCTGCAGCTGCCGCCGCTG
GAACGCCTGACCCTGGATGATAAAAAACCGCTGAATACCCTGATCTCTGCTACTGGTCTGTGGATGAGTCGTACC
GGAACCATTCATAAAATCAAACACCACGAGGTTAGCCGTTCGAAAATCTATATTGAGATGGCGTGTGGCGATCAT
CTGGTTGTGAACAATAGCCGCTCTTCTCGTACAGCACGTGCACTGCGTCACCACAAATATCGTAAAACCTGTAAA
CGTTGCCGTGTGTCCGATGAGGATCTGAACAAATTCCTGACAAAAGCCAATGAGGACCAAACAAGCGTGAAAGTG
AAAGTCGTTAGCGCTCCTACCCGTACTAAAAAAGCAATGCCGAAATCCGTTGCTCGTGCCCCTAAACCACTGGAA
AACACTGAAGCAGCACAGGCACAGCCGTCTGGAAGCAAATTCTCTCCGGCCATTCCTGTTTCTACCCAGGAGTCC
GTTTCTGTTCCAGCAAGTGTGAGCACCAGCATTAGCAGTATTAGCACCGGTGCCACCGCTAGCGCCCTGGTTAAA
GGCAATACCAATCCGATTACAAGCATGTCTGCCCCGGTTCAAGCATCAGCTCCAGCACTGACAAAATCCCAAACC
GATCGTCTGGAGGTTCTGCTGAATCCGAAAGACGAAATCAGCCTGAATTCCGGCAAACCGTTTCGTGAACTGGAG
AGCGAACTGCTGTCACGTCGTAAAAAAGACCTGCAACAAATCTATGCCGAAGAACGTGAGAACTATCTGGGGAAA
CTGGAACGTGAAATCACCCGCTTTTTCGTGGATCGTGGCTTTCTGGAGATCAAATCCCCGATTCTGATTCCTCTG
GAGTATATCGAGCGTATGGGCATCGACAATGATACCGAACTGAGCAAACAAATTTTCCGTGTGGATAAAAACTTC
TGTCTGCGCCCTATGCTTTCACCAAATCTGTATAACTATCTGCGCAAACTGGACCGTGCCCTGCCTGATCCTATC
AAAATCTTCGAGATCGGCCCGTGTTATCGTAAAGAGTCCGACGGTAAAGAACATCTGGAGGAGTTTACCATGCTG
AACTTTTGCCAAATGGGTTCAGGTTGTACTCGTGAGAACCTGGAAAGCATCATCACCGATTTTCTGAACCACCTG
GGCATTGACTTCAAAATTGTGGGCGACAGCTGTATGGTGTATGGCGACACCCTGGATGTCATGCACGGCGACCTG
GAACTGTCTAGTGCCGTTGTTGGACCAATTCCGCTGGACCGTGAGTGGGGTATCGACAAACCGTGGATCGGAGCA
GGATTCGGTCTGGAACGCCTGCTGAAAGTGAAACACGACTTCAAAAACATCAAACGTGCCGCCCGTTCTGAATCG
TATTATAACGGGATCTCTACGAACCTGTAA (SEQ ID NO: 471)

Protein:
MGCVCSSNPEGTELACPVPLQLPPLERLTLDDKKPLNTLISATGLWMSRTGTIHKIKHHEVSRSKIYIEMACGDH
LVVNNSRSSRTARALRHHKYRKTCKRCRVSDEDLNKFLTKANEDQTSVKVKVVSAPTRTKKAMPKSVARAPKPLE
NTEAAQAQPSGSKFSPAIPVSTQESVSVPASVSTSISSISTGATASALVKGNTNPITSMSAPVQASAPALTKSQT
DRLEVLLNPKDEISLNSGKPFRELESELLSRRKKDLQQIYAEERENYLGKLEREITRFFVDRGFLEIKSPILIPL
EYIERMGIDNDTELSKQIFRVDKNFCLRPMLSPNLYNYLRKLDRALPDPIKIFEIGPCYRKESDGKEHLEEFTML
NFCQMGSGCTRENLESIITDFLNHLGIDFKIVGDSCMVYGDTLDVMHGDLELSSAVVGPIPLDREWGIDKPWIGA
GFGLERLLKVKHDFKNIKRAARSESYYNGISTNL* (SEQ ID NO: 472)

TOM20::FUS::SYNZIP3::CbzRS
DNA:
ATGGTGGGTCGGAACAGCGCCATCGCCGCCGGTGTATGCGGGGCCCTTTTCATTGGGTACTGCATCTACTTCGAC
CGCAAAAGACGAAGTGACCCCAACTTCAAGAACAGGCTTCGAGAACGAAGAAAGAAACAGAAGCTTGCCAAGGAG
AGAGCTGGGCTTTCCAAGTTACCTGACCTTAAAGATGCTGAAGCTGTTCAGAAATTCTTCATGGCCTCAAACGAT
TATACCCAACAAGCAACCCAAAGCTATGGGGCCTACCCCACCCAGCCCGGGCAGGGCTATTCCCAGCAGAGCAGT
CAGCCCTACGGACAGCAGAGTTACAGTGGTTATAGCCAGTCCACGGACACTTCAGGATATGGCCAGAGCAGCTAT
TCTTCTTATGGCCAGAGCCAGAACACAGGCTATGGAACTCAGTCAACTCCCCAGGGATATGGCTCGACTGGCGGC
TATGGCAGTAGCCAGAGCTCCCAATCGTCTTACGGGCAGCAGTCCTCCTACCCTGGCTATGGCCAGCAGCCAGCT
CCCAGCAGCACCTCGGGAAGTTACGGTAGCAGTTCTCAGAGCAGCAGCTATGGGCAGCCCCAGAGTGGGAGCTAC
AGCCAGCAGCCTAGCTATGGTGGACAGCAGCAAAGCTATGGACAGCAGCAAAGCTATAATCCCCCTCAGGGCTAT
GGACAGCAGAACCAGTACAACAGCAGCAGTGGTGGTGGAGGTGGAGGTGGAGGTGGAGGTAACTATGGCCAAGAT
CAATCCTCCATGAGTAGTGGTGGTGGCAGTGGTGGCGGTTATGGCAATCAAGACCAGAGTGGTGGAGGTGGCAGC
GGTGGCTATGGACAGCAGGACCGTGGAGGCCGCGGCAGGGGTGGCAGTGGTGGCGGCGGCGGCGGCGGCGGTGGT
GGTTACAACCGCAGCAGTGGTGGCTATGAACCCAGAGGTCGTGGAGGTGGCCGTGGAGGCAGAGGTGGCATGGGC
GGAAGTGACCGTGGTGGCTTCAATAAATTTGGTGGCCCTCGGGACCAAGGATCACGTCATGACTCCGAACAGGAT
AATTCAGACAACAACACCATCTTTGTGCAAGGCCTGGGTGAGAATGTTACAATTGAGTCTGTGGCTGATTACTTC
AAGCAGATTGGTATTATTAAGACAAACAAGAAAACGGGACAGCCCATGATTAATTTGTACACAGACAGGGAAACT
GGCAAGCTGAAGGGAGAGGCAACGGTCTCTTTTGATGACCCACCTTCAGCTAAAGCAGCTATTGACTGGTTTGAT
GGTAAAGAATTCTCCGGAAATCCTATCAAGGTCTCATTTGCTACTCGCCGGGCAGACTTTAATCGGGGTGGTGGC
AATGGTCGTGGAGGCCGAGGGCGAGGAGGACCCATGGGCCGTGGAGGCTATGGAGGTGGTGGCAGTGGTGGTGGT
GGCCGAGGAGGATTTCCCAGTGGAGGTGGTGGCGGTGGAGGACAGCAGCGAGCTGGTGACTGGAAGTGTCCTAAT
CCCACCTGTGAGAATATGAACTTCTCTTGGAGGAATGAATGCAACCAGTGTAAGGCCCCTAAACCAGATGGCCCA
GGAGGGGGACCAGGTGGCTCTCACATGGGGGGTAACTACGGGGATGATCGTCGTGGTGGCAGAGGAGGCGCGATC
GCAAATGAGGTGACCACCCTGGAAAACGACGCCGCCTTCATCGAGAACGAGAACGCCTACCTGGAAAAAGAGATC
GCCAGACTGAGAAAGGAAAAGGCCGCTCTGCGGAACAGACTGGCCCACAAGAAGGGCAAGCCTATTCCCAACCCC
CTGCTGGGCCTGGATAGCACCGGAGCACCAGGAAGTGCTGGTTCTGCTGCTGGTAGTGGAGCGTGCCCGGTGCCG
CTGCAGCTGCCGCCGCTGGAACGCCTGACCCTGGATGATAAAAAACCGCTGAATACCCTGATCTCTGCTACTGGT
CTGTGGATGAGTCGTACCGGAACCATTCATAAAATCAAACACCACGAGGTTAGCCGTTCGAAAATCTATATTGAG
ATGGCGTGTGGCGATCATCTGGTTGTGAACAATAGCCGCTCTTCTCGTACAGCACGTGCACTGCGTCACCACAAA
TATCGTAAAACCTGTAAACGTTGCCGTGTGTCCGATGAGGATCTGAACAAATTCCTGACAAAAGCCAATGAGGAC
CAAACAAGCGTGAAAGTGAAAGTCGTTAGCGCTCCTACCCGTACTAAAAAAGCAATGCCGAAATCCGTTGCTCGT
GCCCCTAAACCACTGGAAAACACTGAAGCAGCACAGGCACAGCCGTCTGGAAGCAAATTCTCTCCGGCCATTCCT
GTTTCTACCCAGGAGTCCGTTTCTGTTCCAGCAAGTGTGAGCACCAGCATTAGCAGTATTAGCACCGGTGCCACC
GCTAGCGCCCTGGTTAAAGGCAATACCAATCCGATTACAAGCATGTCTGCCCCGGTTCAAGCATCAGCTCCAGCA
CTGACAAAATCCCAAACCGATCGTCTGGAGGTTCTGCTGAATCCGAAAGACGAAATCAGCCTGAATTCCGGCAAA
CCGTTTCGTGAACTGGAGAGCGAACTGCTGTCACGTCGTAAAAAAGACCTGCAACAAATCTATGCCGAAGAACGT
GAGAACTATCTGGGGAAACTGGAACGTGAAATCACCCGCTTTTTCGTGGATCGTGGCTTTCTGGAGATCAAATCC
CCGATTCTGATTCCTCTGGAGTATATCGAGCGTATGGGCATCGACAATGATACCGAACTGAGCAAACAAATTTTC
CGTGTGGATAAAAACTTCTGTCTGCGCCCTATGCTTGCACCAAATCTGATGAACTATGGACGCAAACTGGACCGT
GCCCTGCCTGATCCTATCAAAATCTTCGAGATCGGCCCGTGTTATCGTAAAGAGTCCGACGGTAAAGAACATCTG
GAGGAGTTTACCATGCTGAACTTTACACAAATGGGTTCAGGTTGTACTCGTGAGAACCTGGAAAGCATCATCACC
GATTTTCTGAACCACCTGGGCATTGACTTCAAAATTGTGGGCGACAGCTGTATGGTGTATGGCGACACCCTGGAT
GTCATGCACGGCGACCTGGAACTGTCTAGTGCCGTTGTTGGACCAATTCCGCTGGACCGTGAGTGGGGTATCGAC
AAACCGTGGATCGGAGCAGGATTCGGTCTGGAACGCCTGCTGAAAGTGAAACACGACTTCAAAAACATCAAACGT
GCCGCCCGTTCTGAATCGTATTATAACGGGATCTCTACGAACCTGTAA (SEQ ID NO: 473)

Protein:
MVGRNSAIAAGVCGALFIGYCIYFDRKRRSDPNFKNRLRERRKKQKLAKERAGLSKLPDLKDAEAVQKFFMASND
YTQQATQSYGAYPTQPGQGYSQQSSQPYGQQSYSGYSQSTDTSGYGQSSYSSYGQSQNTGYGTQSTPQGYGSTGG
YGSSQSSQSSYGQQSSYPGYGQQPAPSSTSGSYGSSSQSSSYGQPQSGSYSQQPSYGGQQQSYGQQQSYNPPQGY
GQQNQYNSSSGGGGGGGGGNYGQDQSSMSSGGGSGGGYGNQDQSGGGGSGGYGQQDRGGRGRGGSGGGGGGGG
GYNRSSGGYEPRGRGGGRGGRGGMGGSDRGGFNKFGGPRDQGSRHDSEQDNSDNNTIFVQGLGENVTIESVADYF
KQIGIIKTNKKTGQPMINLYTDRETGKLKGEATVSFDDPPSAKAAIDWFDGKEFSGNPIKVSFATRRADFNRGGG
NGRGGRGRGGPMGRGGYGGGGSGGGGRGGFPSGGGGGGGQQRAGDWKCPNPTCENMNFSWRNECNQCKAPKPDGP
GGGPGGSHMGGNYGDDRRGGRGGAIANEVTTLENDAAFIENENAYLEKEIARLRKEKAALRNRLAHKKGKPIPNP

LLGLDSTGAPGSAGSAAGSGACPVPLQLPPLERLTLDDKKPLNTLISATGLWMSRTGTIHKIKHHEVSRSKIYIE
MACGDHLVVNNSRSSRTARALRHHKYRKTCKRCRVSDEDLNKFLTKANEDQTSVKVKVVSAPTRTKKAMPKSVAR
APKPLENTEAAQAQPSGSKFSPAIPVSTQESVSVPASVSTSISSISTGATASALVKGNTNPITSMSAPVQASAPA
LTKSQTDRLEVLLNPKDEISLNSGKPFRELESELLSRRKKDLQQIYAEERENYLGKLEREITRFFVDRGFLEIKS
PILIPLEYIERMGIDNDTELSKQIFRVDKNFCLRPMLAPNLMNYGRKLDRALPDPIKIFEIGPCYRKESDGKEHL
EEFTMLNFTQMGSGCTRENLESIITDFLNHLGIDFKIVGDSCMVYGDTLDVMHGDLELSSAVVGPIPLDREWGID
KPWIGAGFGLERLLKVKHDFKNIKRAARSESYYNGISTNL* (SEQ ID NO: 474)

TOM20::FUS::SYNZIP3::CpkRS
DNA:
ATGGTGGGTCGGAACAGCGCCATCGCCGCCGGTGTATGCGGGGCCCTTTTCATTGGGTACTGCATCTACTTCGAC
CGCAAAAGACGAAGTGACCCCAACTTCAAGAACAGGCTTCGAGAACGAAGAAAGAAACAGAAGCTTGCCAAGGAG
AGAGCTGGGCTTTCCAAGTTACCTGACCTTAAAGATGCTGAAGCTGTTCAGAAATTCTTCATGGCCTCAAACGAT
TATACCCAACAAGCAACCCAAAGCTATGGGGCCTACCCCACCCAGCCCGGGCAGGGCTATTCCCAGCAGAGCAGT
CAGCCCTACGGACAGCAGAGTTACAGTGGTTATAGCCAGTCCACGGACACTTCAGGATATGGCCAGAGCAGCTAT
TCTTCTTATGGCCAGAGCCAGAACACAGGCTATGGAACTCAGTCAACTCCCCAGGGATATGGCTCGACTGGCGGC
TATGGCAGTAGCCAGAGCTCCCAATCGTCTTACGGGCAGCAGTCCTCCTACCCTGGCTATGGCCAGCAGCCAGCT
CCCAGCAGCACCTCGGGAAGTTACGGTAGCAGTTCTCAGAGCAGCAGCTATGGGCAGCCCCAGAGTGGGAGCTAC
AGCCAGCAGCCTAGCTATGGTGGACAGCAGCAAAGCTATGGACAGCAGCAAAGCTATAATCCCCCTCAGGGCTAT
GGACAGCAGAACCAGTACAACAGCAGCAGTGGTGGTGGAGGTGGAGGTGGAGGTGGAGGTAACTATGGCCAAGAT
CAATCCTCCATGAGTAGTGGTGGTGGCAGTGGTGGCGGTTATGGCAATCAAGACCAGAGTGGTGGAGGTGGCAGC
GGTGGCTATGGACAGCAGGACCGTGGAGGCCGCGGCAGGGGTGGCAGTGGTGGCGGCGGCGGCGGCGGCGGTGGT
GGTTACAACCGCAGCAGTGGTGGCTATGAACCCAGAGGTCGTGGAGGTGGCCGTGGAGGCAGAGGTGGCATGGGC
GGAAGTGACCGTGGTGGCTTCAATAAATTTGGTGGCCCTCGGGACCAAGGATCACGTCATGACTCCGAACAGGAT
AATTCAGACAACAACACCATCTTTGTGCAAGGCCTGGGTGAGAATGTTACAATTGAGTCTGTGGCTGATTACTTC
AAGCAGATTGGTATTATTAAGACAAACAAGAAAACGGGACAGCCCATGATTAATTTGTACACAGACAGGGAAACT
GGCAAGCTGAAGGGAGAGGCAACGGTCTCTTTTGATGACCCACCTTCAGCTAAAGCAGCTATTGACTGGTTTGAT
GGTAAAGAATTCTCCGGAAATCCTATCAAGGTCTCATTTGCTACTCGCCGGGCAGACTTTAATCGGGGTGGTGGC
AATGGTCGTGGAGGCCGAGGGCGAGGAGGACCCATGGGCCGTGGAGGCTATGGAGGTGGTGGCAGTGGTGGTGGT
GGCCGAGGAGGATTTCCCAGTGGAGGTGGTGGCGGTGGAGGACAGCAGCGAGCTGGTGACTGGAAGTGTCCTAAT
CCCACCTGTGAGAATATGAACTTCTCTTGGAGGAATGAATGCAACCAGTGTAAGGCCCCTAAACCAGATGGCCCA
GGAGGGGGACCAGGTGGCTCTCACATGGGGGGTAACTACGGGGATGATCGTCGTGGTGGCAGAGGAGGCGCGATC
GCAAATGAGGTGACCACCCTGGAAAACGACGCCGCCTTCATCGAGAACGAGAACGCCTACCTGGAAAAAGAGATC
GCCAGACTGAGAAAGGAAAAGGCCGCTCTGCGGAACAGACTGGCCCACAAGAAGGGCAAGCCTATTCCCAACCCC
CTGCTGGGCCTGGATAGCACCGGAGCACCAGGAAGTGCTGGTTCTGCTGCTGGTAGTGGAGCGTGCCCGGTGCCG
CTGCAGCTGCCGCCGCTGGAACGCCTGACCCTGGATGATAAAAAACCGCTGAATACCCTGATCTCTGCTACTGGT
CTGTGGATGAGTCGTACCGGAACCATTCATAAAATCAAACACCACGAGGTTAGCCGTTCGAAAATCTATATTGAG
ATGGCGTGTGGCGATCATCTGGTTGTGAACAATAGCCGCTCTTCTCGTACAGCACGTGCACTGCGTCACCACAAA
TATCGTAAAACCTGTAAACGTTGCCGTGTGTCCGATGAGGATCTGAACAAATTCCTGACAAAAGCCAATGAGGAC
CAAACAAGCGTGAAAGTGAAAGTCGTTAGCGCTCCTACCCGTACTAAAAAAGCAATGCCGAAATCCGTTGCTCGT
GCCCCTAAACCACTGGAAAACACTGAAGCAGCACAGGCACAGCCGTCTGGAAGCAAATTCTCTCCGGCCATTCCT
GTTTCTACCCAGGAGTCCGTTTCTGTTCCAGCAAGTGTGAGCACCAGCATTAGCAGTATTAGCACCGGTGCCACC
GCTAGCGCCCTGGTTAAAGGCAATACCAATCCGATTACAAGCATGTCTGCCCCGGTTCAAGCATCAGCTCCAGCA
CTGACAAAATCCCAAACCGATCGTCTGGAGGTTCTGCTGAATCCGAAAGACGAAATCAGCCTGAATTCCGGCAAA
CCGTTTCGTGAACTGGAGAGCGAACTGCTGTCACGTCGTAAAAAAGACCTGCAACAAATCTATGCCGAAGAACGT
GAGAACTATCTGGGGAAACTGGAACGTGAAATCACCCGCTTTTTCGTGGATCGTGGCTTTCTGGAGATCAAATCC
CCGATTCTGATTCCTCTGGAGTATATCGAGCGTATGGGCATCGACAATGATACCGAACTGAGCAAACAAATTTTC
CGTGTGGATAAAAACTTCTGTCTGCGCCCTATGCTTTCACCAAATCTGTATAACTATCTGCGCAAACTGGACCGT
GCCCTGCCTGATCCTATCAAAATCTTCGAGATCGGCCCGTGTTATCGTAAAGAGTCCGACGGTAAAGAACATCTG
GAGGAGTTTACCATGCTGAACTTTTGCCAAATGGGTTCAGGTTGTACTCGTGAGAACCTGGAAAGCATCATCACC
GATTTTCTGAACCACCTGGGCATTGACTTCAAAATTGTGGGCGACAGCTGTATGGTGTATGGCGACACCCTGGAT
GTCATGCACGGCGACCTGGAACTGTCTAGTGCCGTTGTTGGACCAATTCCGCTGGACCGTGAGTGGGGTATCGAC
AAACCGTGGATCGGAGCAGGATTCGGTCTGGAACGCCTGCTGAAAGTGAAACACGACTTCAAAAACATCAAACGT
GCCGCCCGTTCTGAATCGTATTATAACGGGATCTCTACGAACCTGTAA (SEQ ID NO: 475)

Protein:

MVGRNSAIAAGVCGALFIGYCIYFDRKRRSDPNFKNRLRERRKKQKLAKERAGLSKLPDLKDAEAVQKFFMASND
YTQQATQSYGAYPTQPGQGYSQQSSQPYGQQSYSGYSQSTDTSGYGQSSYSSYGQSQNTGYGTQSTPQGYGSTGG
YGSSQSSQSSYGQQSSYPGYGQQPAPSSTSGSYGSSSQSSSYGQPQSGSYSQQPSYGGQQQSYGQQQSYNPPQGY
GQQNQYNSSSGGGGGGGGGGNYGQDQSSMSSGGGSGGGYGNQDQSGGGGSGGYGQQDRGGRGRGGSGGGGGGGG
GYNRSSGGYEPRGRGGGRGGRGGMGGSDRGGFNKFGGPRDQGSRHDSEQDNSDNNTIFVQGLGENVTIESVADYF
KQIGIIKTNKKTGQPMINLYTDRETGKLKGEATVSFDDPPSAKAAIDWFDGKEFSGNPIKVSFATRRADFNRGGG
NGRGGRGRGGPMGRGGYGGGGSGGGGRGGFPSGGGGGGGQQRAGDWKCPNPTCENMNFSWRNECNQCKAPKPDGP
GGGPGGSHMGGNYGDDRRGGRGGAIANEVTTLENDAAFIENENAYLEKEIARLRKEKAALRNRLAHKKGKPIPNP
LLGLDSTGAPGSAGSAAGSGACPVPLQLPPLERLTLDDKKPLNTLISATGLWMSRTGTIHKIKHHEVSRSKIYIE
MACGDHLVVNNSRSSRTARALRHHKYRKTCKRCRVSDEDLNKFLTKANEDQTSVKVKVVSAPTRTKKAMPKSVAR
APKPLENTEAAQAQPSGSKFSPAIPVSTQESVSVPASVSTSISSISTGATASALVKGNTNPITSMSAPVQASAPA
LTKSQTDRLEVLLNPKDEISLNSGKPFRELESELLSRRKKDLQQIYAEERENYLGKLEREITRFFVDRGFLEIKS
PILIPLEYIERMGIDNDTELSKQIFRVDKNFCLRPMLSPNLYNYLRKLDRALPDPIKIFEIGPCYRKESDGKEHL
EEFTMLNFCQMGSGCTRENLESIITDFLNHLGIDFKIVGDSCMVYGDTLDVMHGDLELSSAVVGPIPLDREWGID
KPWIGAGFGLERLLKVKHDFKNIKRAARSESYYNGISTNL* (SEQ ID NO: 476)

TOM20::EWSR1::PylRS(AA)::FUS::PylRS(AA)
DNA:
ATGGTGGGTCGGAACAGCGCCATCGCCGCCGGTGTATGCGGGGCCCTTTTCATTGGGTACTGCATCTACTTCGAC
CGCAAAAGACGAAGTGACCCCAACTTCAAGAACAGGCTTCGAGAACGAAGAAAGAAACAGAAGCTTGCCAAGGAG
AGAGCTGGGCTTTCCAAGTTACCTGACCTTAAAGATGCTGAAGCTGTTCAGAAGTTCTTCATGGCGTCCACGGAT
TACAGTACCTATAGCCAAGCTGCAGCGCAGCAGGGCTACAGTGCTTACACCGCCCAGCCCACTCAAGGATATGCA
CAGACCACCCAGGCATATGGGCAACAAAGCTATGGAACCTATGGACAGCCCACTGATGTCAGCTATACCCAGGCT
CAGACCACTGCAACCTATGGGCAGACCGCCTATGCAACTTCTTATGGACAGCCTCCCACTGGTTATACTACTCCA
ACTGCCCCCCAGGCATACAGCCAGCCTGTCCAGGGGTATGGCACTGGTGCTTATGATACCACCACTGCTACAGTC
ACCACCACCCAGGCCTCCTATGCAGCTCAGTCTGCATATGGCACTCAGCCTGCTTATCCAGCCTATGGGCAGCAG
CCAGCAGCCACTGCACCTACAAGACCGCAGGATGGAAACAAGCCCACTGAGACTAGTCAACCTCAATCTAGCACA
GGGGGTTACAACCAGCCCAGCCTAGGATATGGACAGAGTAACTACAGTTATCCCCAGGTACCTGGGAGCTACCCC
ATGCAGCCAGTCACTGCACCTCCATCCTACCCTCCTACCAGCTATTCCTCTACACAGCCGACTAGTTATGATCAG
AGCAGTTACTCTCAGCAGAACACCTATGGGCAACCGAGCAGCTATGGACAGCAGAGTAGCTATGGTCAACAAAGC
AGCTATGGGCAGCAGCCTCCCACTAGTTACCCACCCCAAACTGGATCCTACAGCCAAGCTCCAAGTCAATATAGC
CAACAGAGCAGCAGCTACGGGCAGCAGAGTTCATTCCGACAGGACCACCCCAGTAGCATGGGTGTTTATGGGCAG
GAGTCTGGAGGATTTTCCGGACCAGGAGAGAACCGGAGCATGAGTGGCCCTGATAACCGGGGCAGGGGAAGAGGG
GGATTTGATCGTGGAGGCATGAGCAGAGGTGGGCGGGGAGGAGGACGCGGTGGAATGGGCAGCGCTGGAGAGCGA
GGTGGCTTCAATAAGCCTGGTGGACCCATGGATGAAGGACCAGATCTTGATCTAGGCCCACCTGTAGATCCAGAT
GAAGACTCTGACAACAGTGCAATTTATGTACAAGGATTAAATGACAGTGTGACTCTAGATGATCTGGCAGACTTC
TTTAAGCAGTGTGGGGTTGTTAAGATGAACAAGAGAACTGGGCAACCCATGATCCACATCTACCTGGACAAGGAA
ACAGGAAAGCCCAAAGGCGATGCCACAGTGTCCTATGAAGACCCACCTACTGCCAAGGCTGCCGTGGAATGGTTT
GATGGGAAAGATTTTCAAGGGAGCAAACTTAAAGTCTCCCTTGCTCGGAAGAAGCCTCCAATGAACAGTATGCGG
GGTGGTCTGCCACCCCGTGAGGGCAGAGGCATGCCACCACCACTCCGTGGAGGTCCAGGAGGCCCAGGAGGTCCT
GGGGGACCCATGGGTCGCATGGGAGGCCGTGGAGGAGATAGAGGAGGCTTCCCTCCAAGAGGACCCCGGGGTTCC
CGAGGGAACCCCTCTGGAGGAGGAAACGTCCAGCACCGAGCTGGAGACTGGCAGTGTCCCAATCCGGGTTGTGGA
AACCAGAACTTCGCCTGGAGAACAGAGTGCAACCAGTGTAAGGCCCCAAAGCCTGAAGGCTTCCTCCCGCCACCC
TTTCCGCCCCCGGGTGGTGATCGTGGCAGAGGTGGCCCTGGTGGCATGCGGGGAGGAAGAGGTGGCCTCATGGAT
CGTGGTGGTCCCGGTGGAATGTTCAGAGGTGGCCGTGGTGGAGACAGAGGTGGCTTCCGTGGTGGCCGGGGCATG
GACCGAGGTGGCTTTGGTGGAGGAAGACGAGGTGGCCCTGGGGGGCCCCCTGGACCTTTGATGGAACAGGCGATC
GCATATCCCTATGATGTGCCGGATTATGCTGGAGCACCAGGAAGTGCTGGTTCTGCTGCTGGTAGTGGAGCTTCT
AACTTTACTCAGTTCGTTCTCGTCGACAATGGCGGAACTGGCGACGTGACTGTCGCCCCAAGCAACTTCGCTAAC
GGGATCGCTGAATGGATCAGCTCTAACTCGCGTTCACAGGCTTACAAAGTAACCTGTAGCGTTCGTCAGAGCTCT
GCGCAGAATCGCAAATACACCATCAAAGTCGAGGTGCCTAAAGGCGCCTGGCGTTCGTACTTAAATATGGAACTA
ACCATTCCAATTTTCGCCACGAATTCCGACTGCGAGCTTATTGTTAAGGCAATGCAAGGTCTCCTAAAAGATGGA
AACCCGATTCCCTCAGCAATCGCAGCAAACTCCGGCATCTACGGGCTAAGCCCCGACCGCGTTAGAGCCGTATCC
CACTGGTCTTCCGCGTGCCCGGTGCCGCTGCAGCTGCCGCCGCTGGAACGCCTGACCCTGGATGATAAAAAACCG
CTGAATACCCTGATCTCTGCTACTGGTCTGTGGATGAGTCGTACCGGAACCATTCATAAAATCAAACACCACGAG
GTTAGCCGTTCGAAAATCTATATTGAGATGGCGTGTGGCGATCATCTGGTTGTGAACAATAGCCGCTCTTCTCGT
ACAGCACGTGCACTGCGTCACCACAAATATCGTAAAACCTGTAAACGTTGCCGTGTGTCCGATGAGGATCTGAAC
AAATTCCTGACAAAAGCCAATGAGGACCAAACAAGCGTGAAAGTGAAAGTCGTTAGCGCTCCTACCCGTACTAAA

AAAGCAATGCCGAAATCCGTTGCTCGTGCCCCTAAACCACTGGAAAACACTGAAGCAGCACAGGCACAGCCGTCT
GGAAGCAAATTCTCTCCGGCCATTCCTGTTTCTACCCAGGAGTCCGTTTCTGTTCCAGCAAGTGTGAGCACCAGC
ATTAGCAGTATTAGCACCGGTGCCACCGCTAGCGCCCTGGTTAAAGGCAATACCAATCCGATTACAAGCATGTCT
GCCCCGGTTCAAGCATCAGCTCCAGCACTGACAAAATCCCAAACCGATCGTCTGGAGGTTCTGCTGAATCCGAAA
GACGAAATCAGCCTGAATTCCGGCAAACCGTTTCGTGAACTGGAGAGCGAACTGCTGTCACGTCGTAAAAAAGAC
CTGCAACAAATCTATGCCGAAGAACGTGAGAACTATCTGGGGAAACTGGAACGTGAAATCACCCGCTTTTTCGTG
GATCGTGGCTTTCTGGAGATCAAATCCCCGATTCTGATTCCTCTGGAGTATATCGAGCGTATGGGCATCGACAAT
GATACCGAACTGAGCAAACAAATTTTCCGTGTGGATAAAAACTTCTGTCTGCGCCCTATGCTGGCACCAAATCTG
TATAACTATCTGCGCAAACTGGACCGTGCCCTGCCTGATCCTATCAAAATCTTCGAGATCGGCCCGTGTTATCGT
AAAGAGTCCGACGGTAAAGAACATCTGGAGGAGTTTACCATGCTGGCCTTTGCCCAAATGGGTTCAGGTTGTACT
CGTGAGAACCTGGAAAGCATCATCACCGATTTTCTGAACCACCTGGGCATTGACTTCAAAATTGTGGGCGACAGC
TGTATGGTGTATGGCGACACCCTGGATGTCATGCACGGCGACCTGGAACTGTCTAGTGCCGTTGTTGGACCAATT
CCGCTGGACCGTGAGTGGGGTATCGACAAACCGTGGATCGGAGCAGGATTCGGTCTGGAACGCCTGCTGAAAGTG
AAACACGACTTCAAAAACATCAAACGTGCCGCCCGTTCTGAATCGTATTATAACGGGATCTCTACGAACCTGGCC
TCAAACGATTATACCCAACAAGCAACCCAAAGCTATGGGGCCTACCCCACCCAGCCCGGGCAGGGCTATTCCCAG
CAGAGCAGTCAGCCCTACGGACAGCAGAGTTACAGTGGTTATAGCCAGTCCACGGACACTTCAGGATATGGCCAG
AGCAGCTATTCTTCTTATGGCCAGAGCCAGAACACAGGCTATGGAACTCAGTCAACTCCCCAGGGATATGGCTCG
ACTGGCGGCTATGGCAGTAGCCAGAGCTCCCAATCGTCTTACGGGCAGCAGTCCTCCTACCCTGGCTATGGCCAG
CAGCCAGCTCCCAGCAGCACCTCGGGAAGTTACGGTAGCAGTTCTCAGAGCAGCAGCTATGGGCAGCCCCAGAGT
GGGAGCTACAGCCAGCAGCCTAGCTATGGTGGACAGCAGCAAAGCTATGGACAGCAGCAAAGCTATAATCCCCCT
CAGGGCTATGGACAGCAGAACCAGTACAACAGCAGCAGTGGTGGTGGAGGTGGAGGTGGAGGTGGAGGTAACTAT
GGCCAAGATCAATCCTCCATGAGTAGTGGTGGTGGCAGTGGTGGCGGTTATGGCAATCAAGACCAGAGTGGTGGA
GGTGGCAGCGGTGGCTATGGACAGCAGGACCGTGGAGGCCGCGGCAGGGGTGGCAGTGGTGGCGGCGGCGGCGGC
GGCGGTGGTGGTTACAACCGCAGCAGTGGTGGCTATGAACCCAGAGGTCGTGGAGGTGGCCGTGGAGGCAGAGGT
GGCATGGGCGGAAGTGACCGTGGTGGCTTCAATAAATTTGGTGGCCCTCGGGACCAAGGATCACGTCATGACTCC
GAACAGGATAATTCAGACAACAACACCATCTTTGTGCAAGGCCTGGGTGAGAATGTTACAATTGAGTCTGTGGCT
GATTACTTCAAGCAGATTGGTATTATTAAGACAAACAAGAAAACGGGACAGCCCATGATTAATTTGTACACAGAC
AGGGAAACTGGCAAGCTGAAGGGAGAGGCAACGGTCTCTTTTGATGACCCACCTTCAGCTAAAGCAGCTATTGAC
TGGTTTGATGGTAAAGAATTCTCCGGAAATCCTATCAAGGTCTCATTTGCTACTCGCCGGGCAGACTTTAATCGG
GGTGGTGGCAATGGTCGTGGAGGCCGAGGGCGAGGAGGACCCATGGGCCGTGGAGGCTATGGAGGTGGTGGCAGT
GGTGGTGGTGGCCGAGGAGGATTTCCCAGTGGAGGTGGTGGCGGTGGAGGACAGCAGCGAGCTGGTGACTGGAAG
TGTCCTAATCCCACCTGTGAGAATATGAACTTCTCTTGGAGGAATGAATGCAACCAGTGTAAGGCCCCTAAACCA
GATGGCCCAGGAGGGGGACCAGGTGGCTCTCACATGGGGGGTAACTACGGGGATGATCGTCGTGGTGGCAGAGGA
GGCGATAAAAAACCGCTGAATACCCTGATCTCTGCTACTGGTCTGTGGATGAGTCGTACCGGAACCATTCATAAA
ATCAAACACCACGAGGTTAGCCGTTCGAAAATCTATATTGAGATGGCGTGTGGCGATCATCTGGTTGTGAACAAT
AGCCGCTCTTCTCGTACAGCACGTGCACTGCGTCACCACAAATATCGTAAAACCTGTAAACGTTGCCGTGTGTCC
GATGAGGATCTGAACAAATTCCTGACAAAAGCCAATGAGGACCAAACAAGCGTGAAAGTGAAAGTCGTTAGCGCT
CCTACCCGTACTAAAAAAGCAATGCCGAAATCCGTTGCTCGTGCCCCTAAACCACTGGAAAACACTGAAGCAGCA
CAGGCACAGCCGTCTGGAAGCAAATTCTCTCCGGCCATTCCTGTTTCTACCCAGGAGTCCGTTTCTGTTCCAGCA
AGTGTGAGCACCAGCATTAGCAGTATTAGCACCGGTGCCACCGCTAGCGCCCTGGTTAAAGGCAATACCAATCCG
ATTACAAGCATGTCTGCCCCGGTTCAAGCATCAGCTCCAGCACTGACAAAATCCCAAACCGATCGTCTGGAGGTT
CTGCTGAATCCGAAAGACGAAATCAGCCTGAATTCCGGCAAACCGTTTCGTGAACTGGAGAGCGAACTGCTGTCA
CGTCGTAAAAAAGACCTGCAACAAATCTATGCCGAAGAACGTGAGAACTATCTGGGGAAACTGGAACGTGAAATC
ACCCGCTTTTTCGTGGATCGTGGCTTTCTGGAGATCAAATCCCCGATTCTGATTCCTCTGGAGTATATCGAGCGT
ATGGGCATCGACAATGATACCGAACTGAGCAAACAAATTTTCCGTGTGGATAAAAACTTCTGTCTGCGCCCTATG
CTGGCACCAAATCTGTATAACTATCTGCGCAAACTGGACCGTGCCCTGCCTGATCCTATCAAAATCTTCGAGATC
GGCCCGTGTTATCGTAAAGAGTCCGACGGTAAAGAACATCTGGAGGAGTTTACCATGCTGGCCTTTGCCCAAATG
GGTTCAGGTTGTACTCGTGAGAACCTGGAAAGCATCATCACCGATTTTCTGAACCACCTGGGCATTGACTTCAAA
ATTGTGGGCGACAGCTGTATGGTGTATGGCGACACCCTGGATGTCATGCACGGCGACCTGGAACTGTCTAGTGCC
GTTGTTGGACCAATTCCGCTGGACCGTGAGTGGGGTATCGACAAACCGTGGATCGGAGCAGGATTCGGTCTGGAA
CGCCTGCTGAAAGTGAAACACGACTTCAAAAACATCAAACGTGCCGCCCGTTCTGAATCGTATTATAACGGGATC
TCTACGAACCTGTAA (SEQ ID NO: 477)

Protein:
MVGRNSAIAAGVCGALFIGYCIYFDRKRRSDPNFKNRLRERRKKQKLAKERAGLSKLPDLKDAEAVQKFFMASTD
YSTYSQAAAQQGYSAYTAQPTQGYAQTTQAYGQQSYGTYGQPTDVSYTQAQTTATYGQTAYATSYGQPPTGYTTP
TAPQAYSQPVQGYGTGAYDTTTATVTTTQASYAAQSAYGTQPAYPAYGQQPAATAPTRPQDGNKPTETSQPQSST
GGYNQPSLGYGQSNYSYPQVPGSYPMQPVTAPPSYPPTSYSSTQPTSYDQSSYSQQNTYGQPSSYGQQSSYGQQS

SYGQQPPTSYPPQTGSYSQAPSQYSQQSSSYGQQSSFRQDHPSSMGVYGQESGGFSGPGENRSMSGPDNRGRGRG
GFDRGGMSRGGRGGGRGGMGSAGERGGFNKPGGPMDEGPDLDLGPPVDPDEDSDNSAIYVQGLNDSVTLDDLADF
FKQCGVVKMNKRTGQPMIHIYLDKETGKPKGDATVSYEDPPTAKAAVEWFDGKDFQGSKLKVSLARKKPPMNSMR
GGLPPREGRGMPPPLRGGPGGPGGPGGPMGRMGGRGGDRGGFPPRGPRGSRGNPSGGGNVQHRAGDWQCPNPGCG
NQNFAWRTECNQCKAPKPEGFLPPPFPPPGGDRGRGGPGGMRGGRGGLMDRGGPGGMFRGGRGGDRGGFRGGRGM
DRGGFGGGRRGGPGGPPGPLMEQAIAYPYDVPDYAGAPGSAGSAAGSGASNFTQFVLVDNGGTGDVTVAPSNFAN
GIAEWISSNSRSQAYKVTCSVRQSSAQNRKYTIKVEVPKGAWRSYLNMELTIPIFATNSDCELIVKAMQGLLKDG
NPIPSAIAANSGIYGLSPDRVRAVSHWSSACPVPLQLPPLERLTLDDKKPLNTLISATGLWMSRTGTIHKIKHHE
VSRSKIYIEMACGDHLVVNNSRSSRTARALRHHKYRKTCKRCRVSDEDLNKFLTKANEDQTSVKVKVVSAPTRTK
KAMPKSVARAPKPLENTEAAQAQPSGSKFSPAIPVSTQESVSVPASVSTSISSISTGATASALVKGNTNPITSMS
APVQASAPALTKSQTDRLEVLLNPKDEISLNSGKPFRELESELLSRRKKDLQQIYAEERENYLGKLEREITRFFV
DRGFLEIKSPILIPLEYIERMGIDNDTELSKQIFRVDKNFCLRPMLAPNLYNYLRKLDRALPDPIKIFEIGPCYR
KESDGKEHLEEFTMLAFAQMGSGCTRENLESIITDFLNHLGIDFKIVGDSCMVYGDTLDVMHGDLELSSAVVGPI
PLDREWGIDKPWIGAGFGLERLLKVKHDFKNIKRAARSESYYNGISTNLASNDYTQQATQSYGAYPTQPGQGYSQ
QSSQPYGQQSYSGYSQSTDTSGYGQSSYSSYGQSQNTGYGTQSTPQGYGSTGGYGSSQSSQSSYGQQSSYPGYGQ
QPAPSSTSGSYGSSSQSSSYGQPQSGSYSQQPSYGGQQQSYGQQQSYNPPQGYGQQNQYNSSSGGGGGGGGGGNY
GQDQSSMSSGGGSGGGYGNQDQSGGGGSGGYGQQDRGGRGRGGSGGGGGGGGGGYNRSSGGYEPRGRGGGRGGRG
GMGGSDRGGFNKFGGPRDQGSRHDSEQDNSDNNTIFVQGLGENVTIESVADYFKQIGIIKTNKKTGQPMINLYTD
RETGKLKGEATVSFDDPPSAKAAIDWFDGKEFSGNPIKVSFATRRADFNRGGGNGRGGRGRGGPMGRGGYGGGGS
GGGGRGGFPSGGGGGGGQQRAGDWKCPNPTCENMNFSWRNECNQCKAPKPDGPGGGPGGSHMGGNYGDDRRGGRG
GDKKPLNTLISATGLWMSRTGTIHKIKHHEVSRSKIYIEMACGDHLVVNNSRSSRTARALRHHKYRKTCKRCRVS
DEDLNKFLTKANEDQTSVKVKVVSAPTRTKKAMPKSVARAPKPLENTEAAQAQPSGSKFSPAIPVSTQESVSVPA
SVSTSISSISTGATASALVKGNTNPITSMSAPVQASAPALTKSQTDRLEVLLNPKDEISLNSGKPFRELESELLS
RRKKDLQQIYAEERENYLGKLEREITRFFVDRGFLEIKSPILIPLEYIERMGIDNDTELSKQIFRVDKNFCLRPM
LAPNLYNYLRKLDRALPDPIKIFEIGPCYRKESDGKEHLEEFTMLAFAQMGSGCTRENLESIITDFLNHLGIDFK
IVGDSCMVYGDTLDVMHGDLELSSAVVGPIPLDREWGIDKPWIGAGFGLERLLKVKHDFKNIKRAARSESYYNGI
STNL* (SEQ ID NO: 478)

LCK::PylRS(AF)::FUS::PylRS(AF)
DNA:
ATGGGCTGCGTGTGCAGCAGCAACCCCGAGGGTACCGAGCTCGCGTGCCCGGTGCCGCTGCAGCTGCCGCCGCTG
GAACGCCTGACCCTGGATGATAAAAAACCGCTGAATACCCTGATCTCTGCTACTGGTCTGTGGATGAGTCGTACC
GGAACCATTCATAAAATCAAACACCACGAGGTTAGCCGTTCGAAAATCTATATTGAGATGGCGTGTGGCGATCAT
CTGGTTGTGAACAATAGCCGCTCTTCTCGTACAGCACGTGCACTGCGTCACCACAAATATCGTAAAACCTGTAAA
CGTTGCCGTGTGTCCGATGAGGATCTGAACAAATTCCTGACAAAAGCCAATGAGGACCAAACAAGCGTGAAAGTG
AAAGTCGTTAGCGCTCCTACCCGTACTAAAAAAGCAATGCCGAAATCCGTTGCTCGTGCCCCTAAACCACTGGAA
AACACTGAAGCAGCACAGGCACAGCCGTCTGGAAGCAAATTCTCTCCGGCCATTCCTGTTTCTACCCAGGAGTCC
GTTTCTGTTCCAGCAAGTGTGAGCACCAGCATTAGCAGTATTAGCACCGGTGCCACCGCTAGCGCCCTGGTTAAA
GGCAATACCAATCCGATTACAAGCATGTCTGCCCCGGTTCAAGCATCAGCTCCAGCACTGACAAAATCCCAAACC
GATCGTCTGGAGGTTCTGCTGAATCCGAAAGACGAAATCAGCCTGAATTCCGGCAAACCGTTTCGTGAACTGGAG
AGCGAACTGCTGTCACGTCGTAAAAAAGACCTGCAACAAATCTATGCCGAAGAACGTGAGAACTATCTGGGGAAA
CTGGAACGTGAAATCACCCGCTTTTTCGTGGATCGTGGCTTTCTGGAGATCAAATCCCCGATTCTGATTCCTCTG
GAGTATATCGAGCGTATGGGCATCGACAATGATACCGAACTGAGCAAACAAATTTTCCGTGTGGATAAAAACTTC
TGTCTGCGCCCTATGCTAGCACCAAATCTGGCTAACTATCTGCGCAAACTGGACCGTGCCCTGCCTGATCCTATC
AAAATCTTCGAGATCGGCCCGTGTTATCGTAAAGAGTCCGACGGTAAAGAACATCTGGAGGAGTTTACCATGCTG
AACTTTTGCCAAATGGGTTCAGGTTGTACTCGTGAGAACCTGGAAAGCATCATCACCGATTTTCTGAACCACCTG
GGCATTGACTTCAAAATTGTGGGCGACAGCTGTATGGTGTTTGGCGACACCCTGGATGTCATGCACGGCGACCTG
GAACTGTCTAGTGCCGTTGTGGGCCCAATCCCGCTGGATCGTGAGTGGGGTATCGACAAACCTTGGATCGGTGCG
GGTTTTGGTCTGGAGCGTCTGCTGAAAGTAAAACACGACTTCAAGAACATCAAACGTGCTGCACGTTCCGAGTCC
TATTACAATGGTATTTCTACTAACCTGGCCTCAAACGATTATACCCAACAAGCAACCCAAAGCTATGGGGCCTAC
CCCACCCAGCCCGGGCAGGGCTATTCCCAGCAGAGCAGTCAGCCCTACGGACAGCAGAGTTACAGTGGTTATAGC
CAGTCCACGGACACTTCAGGATATGGCCAGAGCAGCTATTCTTCTTATGGCCAGAGCCAGAACACAGGCTATGGA
ACTCAGTCAACTCCCCAGGGATATGGCTCGACTGGCGGCTATGGCAGTAGCCAGAGCTCCCAATCGTCTTACGGG
CAGCAGTCCTCCTACCCTGGCTATGGCCAGCAGCCAGCTCCCAGCAGCACCTCGGGAAGTTACGGTAGCAGTTCT
CAGAGCAGCAGCTATGGGCAGCCCCAGAGTGGGAGCTACAGCCAGCAGCCTAGCTATGGTGGACAGCAGCAAAGC
TATGGACAGCAGCAAAGCTATAATCCCCCTCAGGGCTATGGACAGCAGAACCAGTACAACAGCAGCAGTGGTGGT
GGAGGTGGAGGTGGAGGTGGAGGTAACTATGGCCAAGATCAATCCTCCATGAGTAGTGGTGGTGGCAGTGGTGGC

GGTTATGGCAATCAAGACCAGAGTGGTGGAGGTGGCAGCGGTGGCTATGGACAGCAGGACCGTGGAGGCCGCGGC
AGGGGTGGCAGTGGTGGCGGCGGCGGCGGCGGTGGTGGTTACAACCGCAGCAGTGGTGGCTATGAACCCAGA
GGTCGTGGAGGTGGCCGTGGAGGCAGAGGTGGCATGGGCGGAAGTGACCGTGGTGGCTTCAATAAATTTGGTGGC
CCTCGGGACCAAGGATCACGTCATGACTCCGAACAGGATAATTCAGACAACAACACCATCTTTGTGCAAGGCCTG
GGTGAGAATGTTACAATTGAGTCTGTGGCTGATTACTTCAAGCAGATTGGTATTATTAAGACAAACAAGAAAACG
GGACAGCCCATGATTAATTTGTACACAGACAGGGAAACTGGCAAGCTGAAGGGAGAGGCAACGGTCTCTTTTGAT
GACCCACCTTCAGCTAAAGCAGCTATTGACTGGTTTGATGGTAAAGAATTCTCCGGAAATCCTATCAAGGTCTCA
TTTGCTACTCGCCGGGCAGACTTTAATCGGGGTGGTGGCAATGGTCGTGGAGGCCGAGGGCGAGGAGGACCCATG
GGCCGTGGAGGCTATGGAGGTGGTGGCAGTGGTGGTGGTGGCCGAGGAGGATTTCCCAGTGGAGGTGGTGGCGGT
GGAGGACAGCAGCGAGCTGGTGACTGGAAGTGTCCTAATCCCACCTGTGAGAATATGAACTTCTCTTGGAGGAAT
GAATGCAACCAGTGTAAGGCCCCTAAACCAGATGGCCCAGGAGGGGGACCAGGTGGCTCTCACATGGGGGGTAAC
TACGGGGATGATCGTCGTGGTGGCAGAGGAGGCGATAAAAAACCGCTGAATACCCTGATCTCTGCTACTGGTCTG
TGGATGAGTCGTACCGGAACCATTCATAAAATCAAACACCACGAGGTTAGCCGTTCGAAAATCTATATTGAGATG
GCGTGTGGCGATCATCTGGTTGTGAACAATAGCCGCTCTTCTCGTACAGCACGTGCACTGCGTCACCACAAATAT
CGTAAAACCTGTAAACGTTGCCGTGTGTCCGATGAGGATCTGAACAAATTCCTGACAAAAGCCAATGAGGACCAA
ACAAGCGTGAAAGTGAAAGTCGTTAGCGCTCCTACCCGTACTAAAAAAGCAATGCCGAAATCCGTTGCTCGTGCC
CCTAAACCACTGGAAAACACTGAAGCAGCACAGGCACAGCCGTCTGGAAGCAAATTCTCTCCGGCCATTCCTGTT
TCTACCCAGGAGTCCGTTTCTGTTCCAGCAAGTGTGAGCACCAGCATTAGCAGTATTAGCACCGGTGCCACCGCT
AGCGCCCTGGTTAAAGGCAATACCAATCCGATTACAAGCATGTCTGCCCCGGTTCAAGCATCAGCTCCAGCACTG
ACAAAATCCCAAACCGATCGTCTGGAGGTTCTGCTGAATCCGAAAGACGAAATCAGCCTGAATTCCGGCAAACCG
TTTCGTGAACTGGAGAGCGAACTGCTGTCACGTCGTAAAAAAGACCTGCAACAAATCTATGCCGAAGAACGTGAG
AACTATCTGGGGAAACTGGAACGTGAAATCACCCGCTTTTTCGTGGATCGTGGCTTTCTGGAGATCAAATCCCCG
ATTCTGATTCCTCTGGAGTATATCGAGCGTATGGGCATCGACAATGATACCGAACTGAGCAAACAAATTTTCCGT
GTGGATAAAAACTTCTGTCTGCGCCCTATGCTAGCACCAAATCTGGCTAACTATCTGCGCAAACTGGACCGTGCC
CTGCCTGATCCTATCAAAATCTTCGAGATCGGCCCGTGTTATCGTAAAGAGTCCGACGGTAAAGAACATCTGGAG
GAGTTTACCATGCTGAACTTTTGCCAAATGGGTTCAGGTTGTACTCGTGAGAACCTGGAAAGCATCATCACCGAT
TTTCTGAACCACCTGGGCATTGACTTCAAAATTGTGGGCGACAGCTGTATGGTGTTTGGCGACACCCTGGATGTC
ATGCACGGCGACCTGGAACTGTCTAGTGCCGTTGTGGGCCCAATCCCGCTGGATCGTGAGTGGGGTATCGACAAA
CCTTGGATCGGTGCGGGTTTTGGTCTGGAGCGTCTGCTGAAAGTAAAACACGACTTCAAGAACATCAAACGTGCT
GCACGTTCCGAGTCCTATTACAATGGTATTTCTACTAACCTGTAA (SEQ ID NO: 479)

Protein:
MGCVCSSNPEGTELACPVPLQLPPLERLTLDDKKPLNTLISATGLWMSRTGTIHKIKHHEVSRSKIYIEMACGDH
LVVNNSRSSRTARALRHHKYRKTCKRCRVSDEDLNKFLTKANEDQTSVKVKVVSAPTRTKKAMPKSVARAPKPLE
NTEAAQAQPSGSKFSPAIPVSTQESVSVPASVSTSISSISTGATASALVKGNTNPITSMSAPVQASAPALTKSQT
DRLEVLLNPKDEISLNSGKPFRELESELLSRRKKDLQQIYAEERENYLGKLEREITRFFVDRGFLEIKSPILIPL
EYIERMGIDNDTELSKQIFRVDKNFCLRPMLAPNLANYLRKLDRALPDPIKIFEIGPCYRKESDGKEHLEEFTML
NFCQMGSGCTRENLESIITDFLNHLGIDFKIVGDSCMVFGDTLDVMHGDLELSSAVVGPIPLDREWGIDKPWIGA
GFGLERLLKVKHDFKNIKRAARSESYYNGISTNLASNDYTQQATQSYGAYPTQPGQGYSQQSSQPYGQQSYSGYS
QSTDTSGYGQSSYSSYGQSQNTGYGTQSTPQGYGSTGGYGSSQSSQSSYGQQSSYPGYGQQPAPSSTSGSYGSSS
QSSSYGQPQSGSYSQQPSYGGQQQSYGQQQSYNPPQGYGQQNQYNSSSGGGGGGGGGGNYGQDQSSMSSGGGSGG
GYGNQDQSGGGGSGGYGQQDRGGRGRGGSGGGGGGGGGGYNRSSGGYEPRGRGGGRGGRGGMGGSDRGGFNKFGG
PRDQGSRHDSEQDNSDNNTIFVQGLGENVTIESVADYFKQIGIIKTNKKTGQPMINLYTDRETGKLKGEATVSFD
DPPSAKAAIDWFDGKEFSGNPIKVSFATRRADFNRGGGNGRGGRGRGGPMGRGGYGGGGSGGGRGGFPSGGGGG
GGQQRAGDWKCPNPTCENMNFSWRNECNQCKAPKPDGPGGGPGGSHMGGNYGDDRRGGRGGDKKPLNTLISATGL
WMSRTGTIHKIKHHEVSRSKIYIEMACGDHLVVNNSRSSRTARALRHHKYRKTCKRCRVSDEDLNKFLTKANEDQ
TSVKVKVVSAPTRTKKAMPKSVARAPKPLENTEAAQAQPSGSKFSPAIPVSTQESVSVPASVSTSISSISTGATA
SALVKGNTNPITSMSAPVQASAPALTKSQTDRLEVLLNPKDEISLNSGKPFRELESELLSRRKKDLQQIYAEERE
NYLGKLEREITRFFVDRGFLEIKSPILIPLEYIERMGIDNDTELSKQIFRVDKNFCLRPMLAPNLANYLRKLDRA
LPDPIKIFEIGPCYRKESDGKEHLEEFTMLNFCQMGSGCTRENLESIITDFLNHLGIDFKIVGDSCMVFGDTLDV
MHGDLELSSAVVGPIPLDREWGIDKPWIGAGFGLERLLKVKHDFKNIKRAARSESYYNGISTNL* (SEQ ID
NO: 480)

LCK::FUS::PylRS(AF)::EWSR1::PylRS(AF)
DNA:
ATGGGCTGCGTGTGCAGCAGCAACCCCGAGGGTACCGAGCTCGCCTCAAACGATTATACCCAACAAGCAACCCAA
AGCTATGGGGCCTACCCCACCCAGCCCGGGCAGGGCTATTCCCAGCAGAGCAGTCAGCCCTACGGACAGCAGAGT

TACAGTGGTTATAGCCAGTCCACGGACACTTCAGGATATGGCCAGAGCAGCTATTCTTCTTATGGCCAGAGCCAG
AACACAGGCTATGGAACTCAGTCAACTCCCCAGGGATATGGCTCGACTGGCGGCTATGGCAGTAGCCAGAGCTCC
CAATCGTCTTACGGGCAGCAGTCCTCCTACCCTGGCTATGGCCAGCAGCCAGCTCCCAGCAGCACCTCGGGAAGT
TACGGTAGCAGTTCTCAGAGCAGCAGCTATGGGCAGCCCCAGAGTGGGAGCTACAGCCAGCAGCCTAGCTATGGT
GGACAGCAGCAAAGCTATGGACAGCAGCAAAGCTATAATCCCCCTCAGGGCTATGGACAGCAGAACCAGTACAAC
AGCAGCAGTGGTGGTGGAGGTGGAGGTGGAGGTGGAGGTAACTATGGCCAAGATCAATCCTCCATGAGTAGTGGT
GGTGGCAGTGGTGGCGGTTATGGCAATCAAGACCAGAGTGGTGGAGGTGGCAGCGGTGGCTATGGACAGCAGGAC
CGTGGAGGCCGCGGCAGGGGTGGCAGTGGTGGCGGCGGCGGCGGCGGTGGTGGTTACAACCGCAGCAGTGGT
GGCTATGAACCCAGAGGTCGTGGAGGTGGCCGTGGAGGCAGAGGTGGCATGGGCGGAAGTGACCGTGGTGGCTTC
AATAAATTTGGTGGCCCTCGGGACCAAGGATCACGTCATGACTCCGAACAGGATAATTCAGACAACAACACCATC
TTTGTGCAAGGCCTGGGTGAGAATGTTACAATTGAGTCTGTGGCTGATTACTTCAAGCAGATTGGTATTATTAAG
ACAAACAAGAAAACGGGACAGCCCATGATTAATTTGTACACAGACAGGGAAACTGGCAAGCTGAAGGGAGAGGCA
ACGGTCTCTTTTGATGACCCACCTTCAGCTAAAGCAGCTATTGACTGGTTTGATGGTAAAGAATTCTCCGGAAAT
CCTATCAAGGTCTCATTTGCTACTCGCCGGGCAGACTTTAATCGGGGTGGTGGCAATGGTCGTGGAGGCCGAGGG
CGAGGAGGACCCATGGGCCGTGGAGGCTATGGAGGTGGTGGCAGTGGTGGTGGTGGCCGAGGAGGATTTCCCAGT
GGAGGTGGTGGCGGTGGAGGACAGCAGCGAGCTGGTGACTGGAAGTGTCCTAATCCCACCTGTGAGAATATGAAC
TTCTCTTGGAGGAATGAATGCAACCAGTGTAAGGCCCCTAAACCAGATGGCCCAGGAGGGGGACCAGGTGGCTCT
CACATGGGGGGTAACTACGGGGATGATCGTCGTGGTGGCAGAGGAGGCGGCGCCCCCGGCCTCCGCCGGCTCCGCC
GCCGGCTCCGGCATGGCGTGCCCGGTGCCGCTGCAGCTGCCGCCGCTGGAACGCCTGACCCTGGATGATAAAAAA
CCGCTGAATACCCTGATCTCTGCTACTGGTCTGTGGATGAGTCGTACCGGAACCATTCATAAAATCAAACACCAC
GAGGTTAGCCGTTCGAAAATCTATATTGAGATGGCGTGTGGCGATCATCTGGTTGTGAACAATAGCCGCTCTTCT
CGTACAGCACGTGCACTGCGTCACCACAAATATCGTAAAACCTGTAAACGTTGCCGTGTGTCCGATGAGGATCTG
AACAAATTCCTGACAAAAGCCAATGAGGACCAAACAAGCGTGAAAGTGAAAGTCGTTAGCGCTCCTACCCGTACT
AAAAAAGCAATGCCGAAATCCGTTGCTCGTGCCCCTAAACCACTGGAAAACACTGAAGCAGCACAGGCACAGCCG
TCTGGAAGCAAATTCTCTCCGGCCATTCCTGTTTCTACCCAGGAGTCCGTTTCTGTTCCAGCAAGTGTGAGCACC
AGCATTAGCAGTATTAGCACCGGTGCCACCGCTAGCGCCCTGGTTAAAGGCAATACCAATCCGATTACAAGCATG
TCTGCCCCGGTTCAAGCATCAGCTCCAGCACTGACAAAATCCCAAACCGATCGTCTGGAGGTTCTGCTGAATCCG
AAAGACGAAATCAGCCTGAATTCCGGCAAACCGTTTCGTGAACTGGAGAGCGAACTGCTGTCACGTCGTAAAAAA
GACCTGCAACAAATCTATGCCGAAGAACGTGAGAACTATCTGGGGAAACTGGAACGTGAAATCACCCGCTTTTTC
GTGGATCGTGGCTTTCTGGAGATCAAATCCCCGATTCTGATTCCTCTGGAGTATATCGAGCGTATGGGCATCGAC
AATGATACCGAACTGAGCAAACAAATTTTCCGTGTGGATAAAAACTTCTGTCTGCGCCCTATGCTAGCACCAAAT
CTGGCTAACTATCTGCGCAAACTGGACCGTGCCCTGCCTGATCCTATCAAAATCTTCGAGATCGGCCCGTGTTAT
CGTAAAGAGTCCGACGGTAAAGAACATCTGGAGGAGTTTACCATGCTGAACTTTTGCCAAATGGGTTCAGGTTGT
ACTCGTGAGAACCTGGAAAGCATCATCACCGATTTTCTGAACCACCTGGGCATTGACTTCAAAATTGTGGGCGAC
AGCTGTATGGTGTTTGGCGACACCCTGGATGTCATGCACGGCGACCTGGAACTGTCTAGTGCCGTTGTGGGCCCA
ATCCCGCTGGATCGTGAGTGGGGTATCGACAAACCTTGGATCGGTGCGGGTTTTGGTCTGGAGCGTCTGCTGAAA
GTAAAACACGACTTCAAGAACATCAAACGTGCTGCACGTTCCGAGTCCTATTACAATGGTATTTCTACTAACCTG
ATGGCGTCCACGGATTACAGTACCTATAGCCAAGCTGCAGCGCAGCAGGGCTACAGTGCTTACACCGCCCAGCCC
ACTCAAGGATATGCACAGACCACCCAGGCATATGGGCAACAAAGCTATGGAACCTATGGACAGCCCACTGATGTC
AGCTATACCCAGGCTCAGACCACTGCAACCTATGGGCAGACCGCCTATGCAACTTCTTATGGACAGCCTCCCACT
GGTTATACTACTCCAACTGCCCCCCAGGCATACAGCCAGCCTGTCCAGGGGTATGGCACTGGTGCTTATGATACC
ACCACTGCTACAGTCACCACCACCCAGGCCTCCTATGCAGCTCAGTCTGCATATGGCACTCAGCCTGCTTATCCA
GCCTATGGGCAGCAGCCAGCAGCCACTGCACCTACAAGACCGCAGGATGGAAACAAGCCCACTGAGACTAGTCAA
CCTCAATCTAGCACAGGGGGTTACAACCAGCCCAGCCTAGGATATGGACAGAGTAACTACAGTTATCCCCAGGTA
CCTGGGAGCTACCCCATGCAGCCAGTCACTGCACCTCCATCCTACCCTCCTACCAGCTATTCCTCTACACAGCCG
ACTAGTTATGATCAGAGCAGTTACTCTCAGCAGAACACCTATGGGCAACCGAGCAGCTATGGACAGCAGAGTAGC
TATGGTCAACAAAGCAGCTATGGGCAGCAGCCTCCCACTAGTTACCCACCCCAAACTGGATCCTACAGCCAAGCT
CCAAGTCAATATAGCCAACAGAGCAGCAGCTACGGGCAGCAGAGTTCATTCCGACAGGACCACCCCAGTAGCATG
GGTGTTTATGGGCAGGAGTCTGGAGGATTTTCCGGACCAGGAGAGAACCGGAGCATGAGTGGCCCTGATAACCGG
GGCAGGGGAAGAGGGGGATTTGATCGTGGAGGCATGAGCAGAGGTGGGCGGGGAGGAGGACGCGGTGGAATGGGC
AGCGCTGGAGAGCGAGGTGGCTTCAATAAGCCTGGTGGACCCATGGATGAAGGACCAGATCTTGATCTAGGCCCA
CCTGTAGATCCAGATGAAGACTCTGACAACAGTGCAATTTATGTACAAGGATTAAATGACAGTGTGACTCTAGAT
GATCTGGCAGACTTCTTTAAGCAGTGTGGGGTTGTTAAGATGAACAAGAGAACTGGGCAACCCATGATCCACATC
TACCTGGACAAGGAAACAGGAAAGCCCAAAGGCGATGCCACAGTGTCCTATGAAGACCCACCTACTGCCAAGGCT
GCCGTGGAATGGTTTGATGGGAAAGATTTTCAAGGGAGCAAACTTAAAGTCTCCCTTGCTCGGAAGAAGCCTCCA
ATGAACAGTATGCGGGGTGGTCTGCCACCCCGTGAGGGCAGAGGCATGCCACCACCACTCCGTGGAGGTCCAGGA
GGCCCAGGAGGTCCTGGGGGACCCATGGGTCGCATGGGAGGCCGTGGAGGAGATAGAGGAGGCTTCCCTCCAAGA
GGACCCCGGGGTTCCCGAGGGAACCCCTCTGGAGGAGGAAACGTCCAGCACCGAGCTGGAGACTGGCAGTGTCCC

AATCCGGGTTGTGGAAACCAGAACTTCGCCTGGAGAACAGAGTGCAACCAGTGTAAGGCCCCAAAGCCTGAAGGC
TTCCTCCCGCCACCCTTTCCGCCCCCGGGTGGTGATCGTGGCAGAGGTGGCCCTGGTGGCATGCGGGGAGGAAGA
GGTGGCCTCATGGATCGTGGTGGTCCCGGTGGAATGTTCAGAGGTGGCCGTGGTGGAGACAGAGGTGGCTTCCGT
GGTGGCCGGGGCATGGACCGAGGTGGCTTTGGTGGAGGAAGACGAGGTGGCCCTGGGGGGCCCCCTGGACCTTTG
ATGGAACAGGATAAAAAACCGCTGAATACCCTGATCTCTGCTACTGGTCTGTGGATGAGTCGTACCGGAACCATT
CATAAAATCAAACACCACGAGGTTAGCCGTTCGAAAATCTATATTGAGATGGCGTGTGGCGATCATCTGGTTGTG
AACAATAGCCGCTCTTCTCGTACAGCACGTGCACTGCGTCACCACAAATATCGTAAAACCTGTAAACGTTGCCGT
GTGTCCGATGAGGATCTGAACAAATTCCTGACAAAAGCCAATGAGGACCAAACAAGCGTGAAAGTGAAAGTCGTT
AGCGCTCCTACCCGTACTAAAAAAGCAATGCCGAAATCCGTTGCTCGTGCCCCTAAACCACTGGAAAACACTGAA
GCAGCACAGGCACAGCCGTCTGGAAGCAAATTCTCTCCGGCCATTCCTGTTTCTACCCAGGAGTCCGTTTCTGTT
CCAGCAAGTGTGAGCACCAGCATTAGCAGTATTAGCACCGGTGCCACCGCTAGCGCCCTGGTTAAAGGCAATACC
AATCCGATTACAAGCATGTCTGCCCCGGTTCAAGCATCAGCTCCAGCACTGACAAAATCCCAAACCGATCGTCTG
GAGGTTCTGCTGAATCCGAAAGACGAAATCAGCCTGAATTCCGGCAAACCGTTTCGTGAACTGGAGAGCGAACTG
CTGTCACGTCGTAAAAAAGACCTGCAACAAATCTATGCCGAAGAACGTGAGAACTATCTGGGGAAACTGGAACGT
GAAATCACCCGCTTTTTCGTGGATCGTGGCTTTCTGGAGATCAAATCCCCGATTCTGATTCCTCTGGAGTATATC
GAGCGTATGGGCATCGACAATGATACCGAACTGAGCAAACAAATTTTCCGTGTGGATAAAAACTTCTGTCTGCGC
CCTATGCTAGCACCAAATCTGGCTAACTATCTGCGCAAACTGGACCGTGCCCTGCCTGATCCTATCAAAATCTTC
GAGATCGGCCCGTGTTATCGTAAAGAGTCCGACGGTAAAGAACATCTGGAGGAGTTTACCATGCTGAACTTTTGC
CAAATGGGTTCAGGTTGTACTCGTGAGAACCTGGAAAGCATCATCACCGATTTTCTGAACCACCTGGGCATTGAC
TTCAAAATTGTGGGCGACAGCTGTATGGTGTTTGGCGACACCCTGGATGTCATGCACGGCGACCTGGAACTGTCT
AGTGCCGTTGTGGGCCCAATCCCGCTGGATCGTGAGTGGGGTATCGACAAACCTTGGATCGGTGCGGGTTTTGGT
CTGGAGCGTCTGCTGAAAGTAAAACACGACTTCAAGAACATCAAACGTGCTGCACGTTCCGAGTCCTATTACAAT
GGTATTTCTACTAACCTGTAA (SEQ ID NO: 481)

Protein:
MGCVCSSNPEGTELASNDYTQQATQSYGAYPTQPGQGYSQQSSQPYGQQSYSGYSQSTDTSGYGQSSYSSYGQSQ
NTGYGTQSTPQGYGSTGGYGSSQSSQSSYGQQSSYPGYGQQPAPSSTSGSYGSSSQSSSYGQPQSGSYSQQPSYG
GQQQSYGQQQSYNPPQGYGQQNQYNSSSGGGGGGGGGNYGQDQSSMSSGGGSGGGYGNQDQSGGGGSGGYGQQD
RGGRGRGGSGGGGGGGGGGYNRSSGGYEPRGRGGGRGGRGGMGGSDRGGFNKFGGPRDQGSRHDSEQDNSDNNTI
FVQGLGENVTIESVADYFKQIGIIKTNKKTGQPMINLYTDRETGKLKGEATVSFDDPPSAKAAIDWFDGKEFSGN
PIKVSFATRRADFNRGGGNGRGGRGRGGPMGRGGYGGGGSGGGGRGGFPSGGGGGGGQQRAGDWKCPNPTCENMN
FSWRNECNQCKAPKPDGPGGGPGGSHMGGNYGDDRRGGRGGGAPGSAGSAAGSGMACPVPLQLPPLERLTLDDKK
PLNTLISATGLWMSRTGTIHKIKHHEVSRSKIYIEMACGDHLVVNNSRSSRTARALRHHKYRKTCKRCRVSDEDL
NKFLTKANEDQTSVKVKVVSAPTRTKKAMPKSVARAPKPLENTEAAQAQPSGSKFSPAIPVSTQESVSVPASVST
SISSISTGATASALVKGNTNPITSMSAPVQASAPALTKSQTDRLEVLLNPKDEISLNSGKPFRELESELLSRRKK
DLQQIYAEERENYLGKLEREITRFFVDRGFLEIKSPILIPLEYIERMGIDNDTELSKQIFRVDKNFCLRPMLAPN
LANYLRKLDRALPDPIKIFEIGPCYRKESDGKEHLEEFTMLNFCQMGSGCTRENLESIITDFLNHLGIDFKIVGD
SCMVFGDTLDVMHGDLELSSAVVGPIPLDREWGIDKPWIGAGFGLERLLKVKHDFKNIKRAARSESYYNGISTNL
MASTDYSTYSQAAAQQGYSAYTAQPTQGYAQTTQAYGQQSYGTYGQPTDVSYTQAQTTATYGQTAYATSYGQPPT
GYTTPTAPQAYSQPVQGYGTGAYDTTTATVTTTQASYAAQSAYGTQPAYPAYGQQPAATAPTRPQDGNKPTETSQ
PQSSTGGYNQPSLGYGQSNYSYPQVPGSYPMQPVTAPPSYPPTSYSSTQPTSYDQSSYSQQNTYGQPSSYGQQSS
YGQQSSYGQQPPTSYPPQTGSYSQAPSQYSQQSSSYGQQSSFRQDHPSSMGVYGQESGGFSGPGENRSMSGPDNR
GRGRGGFDRGGMSRGGRGGGRGGMGSAGERGGFNKPGGPMDEGPDLDLGPPVDPDEDSDNSAIYVQGLNDSVTLD
DLADFFKQCGVVKMNKRTGQPMIHIYLDKETGKPKGDATVSYEDPPTAKAAVEWFDGKDFQGSKLKVSLARKKPP
MNSMRGGLPPREGRGMPPPLRGGPGGPGGPGGPMGRMGGRGGDRGGFPPRGPRGSRGNPSGGGNVQHRAGDWQCP
NPGCGNQNFAWRTECNQCKAPKPEGFLPPPFPPPGGDRGRGGPGGMRGGRGGLMDRGGPGGMFRGGRGGDRGGFR
GGRGMDRGGFGGGRRGGPGGPPGPLMEQDKKPLNTLISATGLWMSRTGTIHKIKHHEVSRSKIYIEMACGDHLVV
NNSRSSRTARALRHHKYRKTCKRCRVSDEDLNKFLTKANEDQTSVKVKVVSAPTRTKKAMPKSVARAPKPLENTE
AAQAQPSGSKFSPAIPVSTQESVSVPASVSTSISSISTGATASALVKGNTNPITSMSAPVQASAPALTKSQTDRL
EVLLNPKDEISLNSGKPFRELESELLSRRKKDLQQIYAEERENYLGKLEREITRFFVDRGFLEIKSPILIPLEYI
ERMGIDNDTELSKQIFRVDKNFCLRPMLAPNLANYLRKLDRALPDPIKIFEIGPCYRKESDGKEHLEEFTMLNFC
QMGSGCTRENLESIITDFLNHLGIDFKIVGDSCMVFGDTLDVMHGDLELSSAVVGPIPLDREWGIDKPWIGAGFG
LERLLKVKHDFKNIKRAARSESYYNGISTNL* (SEQ ID NO: 482)

LCK::FUS::PylRS(AF)::FUS::PylRS(AF)
DNA:

ATGGGCTGCGTGTGCAGCAGCAACCCCGAGGGTACCGAGCTCGCCTCAAACGATTATACCCAACAAGCAACCCAA
AGCTATGGGGCCTACCCCACCCAGCCCGGGCAGGGCTATTCCCAGCAGAGCAGTCAGCCCTACGGACAGCAGAGT
TACAGTGGTTATAGCCAGTCCACGGACACTTCAGGATATGGCCAGAGCAGCTATTCTTCTTATGGCCAGAGCCAG
AACACAGGCTATGGAACTCAGTCAACTCCCCAGGGATATGGCTCGACTGGCGGCTATGGCAGTAGCCAGAGCTCC
CAATCGTCTTACGGGCAGCAGTCCTCCTACCCTGGCTATGGCCAGCAGCCAGCTCCCAGCAGCACCTCGGGAAGT
TACGGTAGCAGTTCTCAGAGCAGCAGCTATGGGCAGCCCCAGAGTGGGAGCTACAGCCAGCAGCCTAGCTATGGT
GGACAGCAGCAAAGCTATGGACAGCAGCAAAGCTATAATCCCCCTCAGGGCTATGGACAGCAGAACCAGTACAAC
AGCAGCAGTGGTGGTGGAGGTGGAGGTGGAGGTGGAGGTAACTATGGCCAAGATCAATCCTCCATGAGTAGTGGT
GGTGGCAGTGGTGGCGGTTATGGCAATCAAGACCAGAGTGGTGGAGGTGGCAGCGGTGGCTATGGACAGCAGGAC
CGTGGAGGCCGCGGCAGGGGTGGCAGTGGTGGCGGCGGCGGCGGCGGTGGTGGTTACAACCGCAGCAGTGGT
GGCTATGAACCCAGAGGTCGTGGAGGTGGCCGTGGAGGCAGAGGTGGCATGGGCGGAAGTGACCGTGGTGGCTTC
AATAAATTTGGTGGCCCTCGGGACCAAGGATCACGTCATGACTCCGAACAGGATAATTCAGACAACAACACCATC
TTTGTGCAAGGCCTGGGTGAGAATGTTACAATTGAGTCTGTGGCTGATTACTTCAAGCAGATTGGTATTATTAAG
ACAAACAAGAAAACGGGACAGCCCATGATTAATTTGTACACAGACAGGGAAACTGGCAAGCTGAAGGGAGAGGCA
ACGGTCTCTTTTGATGACCCACCTTCAGCTAAAGCAGCTATTGACTGGTTTGATGGTAAAGAATTCTCCGGAAAT
CCTATCAAGGTCTCATTTGCTACTCGCCGGGCAGACTTTAATCGGGGTGGTGGCAATGGTCGTGGAGGCCGAGGG
CGAGGAGGACCCATGGGCCGTGGAGGCTATGGAGGTGGTGGCAGTGGTGGTGGTGGCCGAGGAGGATTTCCCAGT
GGAGGTGGTGGCGGTGGAGGACAGCAGCGAGCTGGTGACTGGAAGTGTCCTAATCCCACCTGTGAGAATATGAAC
TTCTCTTGGAGGAATGAATGCAACCAGTGTAAGGCCCCTAAACCAGATGGCCCAGGAGGGGGACCAGGTGGCTCT
CACATGGGGGGTAACTACGGGGATGATCGTCGTGGTGGCAGAGGAGGCGGCGCCCCCGGCTCCGCCGGCTCCGCC
GCCGGCTCCGGCATGGCGTGCCCGGTGCCGCTGCAGCTGCCGCCGCTGGAACGCCTGACCCTGGATGATAAAAAA
CCGCTGAATACCCTGATCTCTGCTACTGGTCTGTGGATGAGTCGTACCGGAACCATTCATAAAATCAAACACCAC
GAGGTTAGCCGTTCGAAAATCTATATTGAGATGGCGTGTGGCGATCATCTGGTTGTGAACAATAGCCGCTCTTCT
CGTACAGCACGTGCACTGCGTCACCACAAATATCGTAAAACCTGTAAACGTTGCCGTGTGTCCGATGAGGATCTG
AACAAATTCCTGACAAAAGCCAATGAGGACCAAACAAGCGTGAAAGTGAAAGTCGTTAGCGCTCCTACCCGTACT
AAAAAAGCAATGCCGAAATCCGTTGCTCGTGCCCCTAAACCACTGGAAAACACTGAAGCAGCACAGGCACAGCCG
TCTGGAAGCAAATTCTCTCCGGCCATTCCTGTTTCTACCCAGGAGTCCGTTTCTGTTCCAGCAAGTGTGAGCACC
AGCATTAGCAGTATTAGCACCGGTGCCACCGCTAGCGCCCTGGTTAAAGGCAATACCAATCCGATTACAAGCATG
TCTGCCCCGGTTCAAGCATCAGCTCCAGCACTGACAAAATCCCAAACCGATCGTCTGGAGGTTCTGCTGAATCCG
AAAGACGAAATCAGCCTGAATTCCGGCAAACCGTTTCGTGAACTGGAGAGCGAACTGCTGTCACGTCGTAAAAAA
GACCTGCAACAAATCTATGCCGAAGAACGTGAGAACTATCTGGGGAAACTGGAACGTGAAATCACCCGCTTTTTC
GTGGATCGTGGCTTTCTGGAGATCAAATCCCCGATTCTGATTCCTCTGGAGTATATCGAGCGTATGGGCATCGAC
AATGATACCGAACTGAGCAAACAAATTTTCCGTGTGGATAAAAACTTCTGTCTGCGCCCTATGCTAGCACCAAAT
CTGGCTAACTATCTGCGCAAACTGGACCGTGCCCTGCCTGATCCTATCAAAATCTTCGAGATCGGCCCGTGTTAT
CGTAAAGAGTCCGACGGTAAAGAACATCTGGAGGAGTTTACCATGCTGAACTTTTGCCAAATGGGTTCAGGTTGT
ACTCGTGAGAACCTGGAAAGCATCATCACCGATTTTCTGAACCACCTGGGCATTGACTTCAAAATTGTGGGCGAC
AGCTGTATGGTGTTTGGCGACACCCTGGATGTCATGCACGGCGACCTGGAACTGTCTAGTGCCGTTGTGGGCCCA
ATCCCGCTGGATCGTGAGTGGGGTATCGACAAACCTTGGATCGGTGCGGGTTTTGGTCTGGAGCGTCTGCTGAAA
GTAAAACACGACTTCAAGAACATCAAACGTGCTGCACGTTCCGAGTCCTATTACAATGGTATTTCTACTAACCTG
GCCTCAAACGATTATACCCAACAAGCAACCCAAAGCTATGGGGCCTACCCCACCCAGCCCGGGCAGGGCTATTCC
CAGCAGAGCAGTCAGCCCTACGGACAGCAGAGTTACAGTGGTTATAGCCAGTCCACGGACACTTCAGGATATGGC
CAGAGCAGCTATTCTTCTTATGGCCAGAGCCAGAACACAGGCTATGGAACTCAGTCAACTCCCCAGGGATATGGC
TCGACTGGCGGCTATGGCAGTAGCCAGAGCTCCCAATCGTCTTACGGGCAGCAGTCCTCCTACCCTGGCTATGGC
CAGCAGCCAGCTCCCAGCAGCACCTCGGGAAGTTACGGTAGCAGTTCTCAGAGCAGCAGCTATGGGCAGCCCCAG
AGTGGGAGCTACAGCCAGCAGCCTAGCTATGGTGGACAGCAGCAAAGCTATGGACAGCAGCAAAGCTATAATCCC
CCTCAGGGCTATGGACAGCAGAACCAGTACAACAGCAGCAGTGGTGGTGGAGGTGGAGGTGGAGGTGGAGGTAAC
TATGGCCAAGATCAATCCTCCATGAGTAGTGGTGGTGGCAGTGGTGGCGGTTATGGCAATCAAGACCAGAGTGGT
GGAGGTGGCAGCGGTGGCTATGGACAGCAGGACCGTGGAGGCCGCGGCAGGGGTGGCAGTGGTGGCGGCGGCGGC
GGCGGCGGTGGTGGTTACAACCGCAGCAGTGGTGGCTATGAACCCAGAGGTCGTGGAGGTGGCCGTGGAGGCAGA
GGTGGCATGGGCGGAAGTGACCGTGGTGGCTTCAATAAATTTGGTGGCCCTCGGGACCAAGGATCACGTCATGAC
TCCGAACAGGATAATTCAGACAACAACACCATCTTTGTGCAAGGCCTGGGTGAGAATGTTACAATTGAGTCTGTG
GCTGATTACTTCAAGCAGATTGGTATTATTAAGACAAACAAGAAAACGGGACAGCCCATGATTAATTTGTACACA
GACAGGGAAACTGGCAAGCTGAAGGGAGAGGCAACGGTCTCTTTTGATGACCCACCTTCAGCTAAAGCAGCTATT
GACTGGTTTGATGGTAAAGAATTCTCCGGAAATCCTATCAAGGTCTCATTTGCTACTCGCCGGGCAGACTTTAAT
CGGGGTGGTGGCAATGGTCGTGGAGGCCGAGGGCGAGGAGGACCCATGGGCCGTGGAGGCTATGGAGGTGGTGGC
AGTGGTGGTGGTGGCCGAGGAGGATTTCCCAGTGGAGGTGGTGGCGGTGGAGGACAGCAGCGAGCTGGTGACTGG
AAGTGTCCTAATCCCACCTGTGAGAATATGAACTTCTCTTGGAGGAATGAATGCAACCAGTGTAAGGCCCCTAAA
CCAGATGGCCCAGGAGGGGGACCAGGTGGCTCTCACATGGGGGGTAACTACGGGGATGATCGTCGTGGTGGCAGA

GGAGGCGATAAAAAACCGCTGAATACCCTGATCTCTGCTACTGGTCTGTGGATGAGTCGTACCGGAACCATTCAT
AAAATCAAACACCACGAGGTTAGCCGTTCGAAAATCTATATTGAGATGGCGTGTGGCGATCATCTGGTTGTGAAC
AATAGCCGCTCTTCTCGTACAGCACGTGCACTGCGTCACCACAAATATCGTAAAACCTGTAAACGTTGCCGTGTG
TCCGATGAGGATCTGAACAAATTCCTGACAAAAGCCAATGAGGACCAAACAAGCGTGAAAGTGAAAGTCGTTAGC
GCTCCTACCCGTACTAAAAAAGCAATGCCGAAATCCGTTGCTCGTGCCCCTAAACCACTGGAAAACACTGAAGCA
GCACAGGCACAGCCGTCTGGAAGCAAATTCTCTCCGGCCATTCCTGTTTCTACCCAGGAGTCCGTTTCTGTTCCA
GCAAGTGTGAGCACCAGCATTAGCAGTATTAGCACCGGTGCCACCGCTAGCGCCCTGGTTAAAGGCAATACCAAT
CCGATTACAAGCATGTCTGCCCCGGTTCAAGCATCAGCTCCAGCACTGACAAAATCCCAAACCGATCGTCTGGAG
GTTCTGCTGAATCCGAAAGACGAAATCAGCCTGAATTCCGGCAAACCGTTTCGTGAACTGGAGAGCGAACTGCTG
TCACGTCGTAAAAAAGACCTGCAACAAATCTATGCCGAAGAACGTGAGAACTATCTGGGGAAACTGGAACGTGAA
ATCACCCGCTTTTTCGTGGATCGTGGCTTTCTGGAGATCAAATCCCCGATTCTGATTCCTCTGGAGTATATCGAG
CGTATGGGCATCGACAATGATACCGAACTGAGCAAACAAATTTTCCGTGTGGATAAAAACTTCTGTCTGCGCCCT
ATGCTAGCACCAAATCTGGCTAACTATCTGCGCAAACTGGACCGTGCCCTGCCTGATCCTATCAAAATCTTCGAG
ATCGGCCCGTGTTATCGTAAAGAGTCCGACGGTAAAGAACATCTGGAGGAGTTTACCATGCTGAACTTTTGCCAA
ATGGGTTCAGGTTGTACTCGTGAGAACCTGGAAAGCATCATCACCGATTTTCTGAACCACCTGGGCATTGACTTC
AAAATTGTGGGCGACAGCTGTATGGTGTTTGGCGACACCCTGGATGTCATGCACGGCGACCTGGAACTGTCTAGT
GCCGTTGTGGGCCCAATCCCGCTGGATCGTGAGTGGGGTATCGACAAACCTTGGATCGGTGCGGGTTTTGGTCTG
GAGCGTCTGCTGAAAGTAAAACACGACTTCAAGAACATCAAACGTGCTGCACGTTCCGAGTCCTATTACAATGGT
ATTTCTACTAACCTGTAA (SEQ ID NO: 483)

Protein:
MGCVCSSNPEGTELASNDYTQQATQSYGAYPTQPGQGYSQQSSQPYGQQSYSGYSQSTDTSGYGQSSYSSYGQSQ
NTGYGTQSTPQGYGSTGGYGSSQSSQSSYGQQSSYPGYGQQPAPSSTSGSYGSSSQSSSYGQPQSGSYSQQPSYG
GQQQSYGQQQSYNPPQGYGQQNQYNSSSGGGGGGGGGGNYGQDQSSMSSGGGSGGGYGNQDQSGGGGSGGYGQQD
RGGRGRGGSGGGGGGGGGGYNRSSGGYEPRGRGGGRGGRGGMGGSDRGGFNKFGGPRDQGSRHDSEQDNSDNNTI
FVQGLGENVTIESVADYFKQIGIIKTNKKTGQPMINLYTDRETGKLKGEATVSFDDPPSAKAAIDWFDGKEFSGN
PIKVSFATRRADFNRGGGNGRGGRGRGGPMGRGGYGGGGSGGGGRGGFPSGGGGGGGQQRAGDWKCPNPTCENMN
FSWRNECNQCKAPKPDGPGGGPGGSHMGGNYGDDRRGGRGGGAPGSAGSAAGSGMACPVPLQLPPLERLTLDDKK
PLNTLISATGLWMSRTGTIHKIKHHEVSRSKIYIEMACGDHLVVNNSRSSRTARALRHHKYRKTCKRCRVSDEDL
NKFLTKANEDQTSVKVKVVSAPTRTKKAMPKSVARAPKPLENTEAAQAQPSGSKFSPAIPVSTQESVSVPASVST
SISSISTGATASALVKGNTNPITSMSAPVQASAPALTKSQTDRLEVLLNPKDEISLNSGKPFRELESELLSRRKK
DLQQIYAEERENYLGKLEREITRFFVDRGFLEIKSPILIPLEYIERMGIDNDTELSKQIFRVDKNFCLRPMLAPN
LANYLRKLDRALPDPIKIFEIGPCYRKESDGKEHLEEFTMLNFCQMGSGCTRENLESIITDFLNHLGIDFKIVGD
SCMVFGDTLDVMHGDLELSSAVVGPIPLDREWGIDKPWIGAGFGLERLLKVKHDFKNIKRAARSESYYNGISTNL
ASNDYTQQATQSYGAYPTQPGQGYSQQSSQPYGQQSYSGYSQSTDTSGYGQSSYSSYGQSQNTGYGTQSTPQGYG
STGGYGSSQSSQSSYGQQSSYPGYGQQPAPSSTSGSYGSSSQSSSYGQPQSGSYSQQPSYGGQQQSYGQQQSYNP
PQGYGQQNQYNSSSGGGGGGGGGGNYGQDQSSMSSGGGSGGGYGNQDQSGGGGSGGYGQQDRGGRGRGGSGGGGG
GGGGGYNRSSGGYEPRGRGGGRGGRGGMGGSDRGGFNKFGGPRDQGSRHDSEQDNSDNNTIFVQGLGENVTIESV
ADYFKQIGIIKTNKKTGQPMINLYTDRETGKLKGEATVSFDDPPSAKAAIDWFDGKEFSGNPIKVSFATRRADFN
RGGGNGRGGRGRGGPMGRGGYGGGGSGGGGRGGFPSGGGGGGGQQRAGDWKCPNPTCENMNFSWRNECNQCKAPK
PDGPGGGPGGSHMGGNYGDDRRGGRGGDKKPLNTLISATGLWMSRTGTIHKIKHHEVSRSKIYIEMACGDHLVVN
NSRSSRTARALRHHKYRKTCKRCRVSDEDLNKFLTKANEDQTSVKVKVVSAPTRTKKAMPKSVARAPKPLENTEA
AQAQPSGSKFSPAIPVSTQESVSVPASVSTSISSISTGATASALVKGNTNPITSMSAPVQASAPALTKSQTDRLE
VLLNPKDEISLNSGKPFRELESELLSRRKKDLQQIYAEERENYLGKLEREITRFFVDRGFLEIKSPILIPLEYIE
RMGIDNDTELSKQIFRVDKNFCLRPMLAPNLANYLRKLDRALPDPIKIFEIGPCYRKESDGKEHLEEFTMLNFCQ
MGSGCTRENLESIITDFLNHLGIDFKIVGDSCMVFGDTLDVMHGDLELSSAVVGPIPLDREWGIDKPWIGAGFGL
ERLLKVKHDFKNIKRAARSESYYNGISTNL* (SEQ ID NO: 484)

TOM20::FUS::PylRS(AF)::EWSR1::PylRS(AF)
DNA:
ATGGTGGGTCGGAACAGCGCCATCGCCGCCGGTGTATGCGGGGCCCTTTTCATTGGGTACTGCATCTACTTCGAC
CGCAAAAGACGAAGTGACCCCAACTTCAAGAACAGGCTTCGAGAACGAAGAAAGAAACAGAAGCTTGCCAAGGAG
AGAGCTGGGCTTTCCAAGTTACCTGACCTTAAAGATGCTGAAGCTGTTCAGAAATTCTTCATGGCCTCAAACGAT
TATACCCAACAAGCAACCCAAAGCTATGGGGCCTACCCCACCCAGCCCGGGCAGGGCTATTCCCAGCAGAGCAGT
CAGCCCTACGGACAGCAGAGTTACAGTGGTTATAGCCAGTCCACGGACACTTCAGGATATGGCCAGAGCAGCTAT
TCTTCTTATGGCCAGAGCCAGAACACAGGCTATGGAACTCAGTCAACTCCCCAGGGATATGGCTCGACTGGCGGC
TATGGCAGTAGCCAGAGCTCCCAATCGTCTTACGGGCAGCAGTCCTCCTACCCTGGCTATGGCCAGCAGCCAGCT

CCCAGCAGCACCTCGGGAAGTTACGGTAGCAGTTCTCAGAGCAGCAGCTATGGGCAGCCCCAGAGTGGGAGCTAC
AGCCAGCAGCCTAGCTATGGTGGACAGCAGCAAAGCTATGGACAGCAGCAAAGCTATAATCCCCCTCAGGGCTAT
GGACAGCAGAACCAGTACAACAGCAGCAGTGGTGGTGGAGGTGGAGGTGGAGGTGGAGGTAACTATGGCCAAGAT
CAATCCTCCATGAGTAGTGGTGGTGGCAGTGGTGGCGGTTATGGCAATCAAGACCAGAGTGGTGGAGGTGGCAGC
GGTGGCTATGGACAGCAGGACCGTGGAGGCCGCGGCAGGGGTGGCAGTGGTGGCGGCGGCGGCGGCGGCGGTGGT
GGTTACAACCGCAGCAGTGGTGGCTATGAACCCAGAGGTCGTGGAGGTGGCCGTGGAGGCAGAGGTGGCATGGGC
GGAAGTGACCGTGGTGGCTTCAATAAATTTGGTGGCCCTCGGGACCAAGGATCACGTCATGACTCCGAACAGGAT
AATTCAGACAACAACACCATCTTTGTGCAAGGCCTGGGTGAGAATGTTACAATTGAGTCTGTGGCTGATTACTTC
AAGCAGATTGGTATTATTAAGACAAACAAGAAAACGGGACAGCCCATGATTAATTTGTACACAGACAGGGAAACT
GGCAAGCTGAAGGGAGAGGCAACGGTCTCTTTTGATGACCCACCTTCAGCTAAAGCAGCTATTGACTGGTTTGAT
GGTAAAGAATTCTCCGGAAATCCTATCAAGGTCTCATTTGCTACTCGCCGGGCAGACTTTAATCGGGGTGGTGGC
AATGGTCGTGGAGGCCGAGGGCGAGGAGGACCCATGGGCCGTGGAGGCTATGGAGGTGGTGGCAGTGGTGGTGGT
GGCCGAGGAGGATTTCCCAGTGGAGGTGGTGGCGGTGGAGGACAGCAGCGAGCTGGTGACTGGAAGTGTCCTAAT
CCCACCTGTGAGAATATGAACTTCTCTTGGAGGAATGAATGCAACCAGTGTAAGGCCCCTAAACCAGATGGCCCA
GGAGGGGGACCAGGTGGCTCTCACATGGGGGGTAACTACGGGGATGATCGTCGTGGTGGCAGAGGAGGCGCGATC
GCAGGCAAGCCTATTCCCAACCCCCTGCTGGGCCTGGATAGCACCGGAGCACCAGGAAGTGCTGGTTCTGCTGCT
GGTAGTGGAGCGTGCCCGGTGCCGCTGCAGCTGCCGCCGCTGGAACGCCTGACCCTGGATGATAAAAAACCGCTG
AATACCCTGATCTCTGCTACTGGTCTGTGGATGAGTCGTACCGGAACCATTCATAAAATCAAACACCACGAGGTT
AGCCGTTCGAAAATCTATATTGAGATGGCGTGTGGCGATCATCTGGTTGTGAACAATAGCCGCTCTTCTCGTACA
GCACGTGCACTGCGTCACCACAAATATCGTAAAACCTGTAAACGTTGCCGTGTGTCCGATGAGGATCTGAACAAA
TTCCTGACAAAAGCCAATGAGGACCAAACAAGCGTGAAAGTGAAAGTCGTTAGCGCTCCTACCCGTACTAAAAAA
GCAATGCCGAAATCCGTTGCTCGTGCCCCTAAACCACTGGAAAACACTGAAGCAGCACAGGCACAGCCGTCTGGA
AGCAAATTCTCTCCGGCCATTCCTGTTTCTACCCAGGAGTCCGTTTCTGTTCCAGCAAGTGTGAGCACCAGCATT
AGCAGTATTAGCACCGGTGCCACCGCTAGCGCCCTGGTTAAAGGCAATACCAATCCGATTACAAGCATGTCTGCC
CCGGTTCAAGCATCAGCTCCAGCACTGACAAAATCCCAAACCGATCGTCTGGAGGTTCTGCTGAATCCGAAAGAC
GAAATCAGCCTGAATTCCGGCAAACCGTTTCGTGAACTGGAGAGCGAACTGCTGTCACGTCGTAAAAAAGACCTG
CAACAAATCTATGCCGAAGAACGTGAGAACTATCTGGGGAAACTGGAACGTGAAATCACCCGCTTTTTCGTGGAT
CGTGGCTTTCTGGAGATCAAATCCCCGATTCTGATTCCTCTGGAGTATATCGAGCGTATGGGCATCGACAATGAT
ACCGAACTGAGCAAACAAATTTTCCGTGTGGATAAAAACTTCTGTCTGCGCCCTATGCTAGCACCAAATCTGGCT
AACTATCTGCGCAAACTGGACCGTGCCCTGCCTGATCCTATCAAAATCTTCGAGATCGGCCCGTGTTATCGTAAA
GAGTCCGACGGTAAAGAACATCTGGAGGAGTTTACCATGCTGAACTTTTGCCAAATGGGTTCAGGTTGTACTCGT
GAGAACCTGGAAAGCATCATCACCGATTTTCTGAACCACCTGGGCATTGACTTCAAAATTGTGGGCGACAGCTGT
ATGGTGTTTGGCGACACCCTGGATGTCATGCACGGCGACCTGGAACTGTCTAGTGCCGTTGTGGGCCCAATCCCG
CTGGATCGTGAGTGGGGTATCGACAAACCTTGGATCGGTGCGGGTTTTGGTCTGGAGCGTCTGCTGAAAGTAAAA
CACGACTTCAAGAACATCAAACGTGCTGCACGTTCCGAGTCCTATTACAATGGTATTTCTACTAACCTGATGGCG
TCCACGGATTACAGTACCTATAGCCAAGCTGCAGCGCAGCAGGGCTACAGTGCTTACACCGCCCAGCCCACTCAA
GGATATGCACAGACCACCCAGGCATATGGGCAACAAAGCTATGGAACCTATGGACAGCCCACTGATGTCAGCTAT
ACCCAGGCTCAGACCACTGCAACCTATGGGCAGACCGCCTATGCAACTTCTTATGGACAGCCTCCCACTGGTTAT
ACTACTCCAACTGCCCCCCAGGCATACAGCCAGCCTGTCCAGGGGTATGGCACTGGTGCTTATGATACCACCACT
GCTACAGTCACCACCACCCAGGCCTCCTATGCAGCTCAGTCTGCATATGGCACTCAGCCTGCTTATCCAGCCTAT
GGGCAGCAGCCAGCAGCCACTGCACCTACAAGACCGCAGGATGGAAACAAGCCCACTGAGACTAGTCAACCTCAA
TCTAGCACAGGGGGTTACAACCAGCCCAGCCTAGGATATGGACAGAGTAACTACAGTTATCCCCAGGTACCTGGG
AGCTACCCCATGCAGCCAGTCACTGCACCTCCATCCTACCCTCCTACCAGCTATTCCTCTACACAGCCGACTAGT
TATGATCAGAGCAGTTACTCTCAGCAGAACACCTATGGGCAACCGAGCAGCTATGGACAGCAGAGTAGCTATGGT
CAACAAAGCAGCTATGGGCAGCAGCCTCCCACTAGTTACCCACCCCAAACTGGATCCTACAGCCAAGCTCCAAGT
CAATATAGCCAACAGAGCAGCAGCTACGGGCAGCAGAGTTCATTCCGACAGGACCACCCCAGTAGCATGGGTGTT
TATGGGCAGGAGTCTGGAGGATTTTCCGGACCAGGAGAGAACCGGAGCATGAGTGGCCCTGATAACCGGGGCAGG
GGAAGAGGGGGATTTGATCGTGGAGGCATGAGCAGAGGTGGGCGGGGAGGAGGACGCGGTGGAATGGGCAGCGCT
GGAGAGCGAGGTGGCTTCAATAAGCCTGGTGGACCCATGGATGAAGGACCAGATCTTGATCTAGGCCCACCTGTA
GATCCAGATGAAGACTCTGACAACAGTGCAATTTATGTACAAGGATTAAATGACAGTGTGACTCTAGATGATCTG
GCAGACTTCTTTAAGCAGTGTGGGGTTGTTAAGATGAACAAGAGAACTGGGCAACCCATGATCCACATCTACCTG
GACAAGGAAACAGGAAAGCCCAAAGGCGATGCCACAGTGTCCTATGAAGACCCACCTACTGCCAAGGCTGCCGTG
GAATGGTTTGATGGGAAAGATTTTCAAGGGAGCAAACTTAAAGTCTCCCTTGCTCGGAAGAAGCCTCCAATGAAC
AGTATGCGGGGTGGTCTGCCACCCCGTGAGGGCAGAGGCATGCCACCACCACTCCGTGGAGGTCCAGGAGGCCCA
GGAGGTCCTGGGGGACCCATGGGTCGCATGGGAGGCCGTGGAGGAGATAGAGGAGGCTTCCCTCCAAGAGGACCC
CGGGGTTCCCGAGGGAACCCCTCTGGAGGAGGAAACGTCCAGCACCGAGCTGGAGACTGGCAGTGTCCCAATCCG
GGTTGTGGAAACCAGAACTTCGCCTGGAGAACAGAGTGCAACCAGTGTAAGGCCCCAAAGCCTGAAGGCTTCCTC
CCGCCACCCTTTCCGCCCCCGGGTGGTGATCGTGGCAGAGGTGGCCCTGGTGGCATGCGGGGAGGAAGAGGTGGC

CTCATGGATCGTGGTGGTCCCGGTGGAATGTTCAGAGGTGGCCGTGGTGGAGACAGAGGTGGCTTCCGTGGTGGC
CGGGGCATGGACCGAGGTGGCTTTGGTGGAGGAAGACGAGGTGGCCCTGGGGGGCCCCCTGGACCTTTGATGGAA
CAGGATAAAAAACCGCTGAATACCCTGATCTCTGCTACTGGTCTGTGGATGAGTCGTACCGGAACCATTCATAAA
ATCAAACACCACGAGGTTAGCCGTTCGAAAATCTATATTGAGATGGCGTGTGGCGATCATCTGGTTGTGAACAAT
AGCCGCTCTTCTCGTACAGCACGTGCACTGCGTCACCACAAATATCGTAAAACCTGTAAACGTTGCCGTGTGTCC
GATGAGGATCTGAACAAATTCCTGACAAAAGCCAATGAGGACCAAACAAGCGTGAAAGTGAAAGTCGTTAGCGCT
CCTACCCGTACTAAAAAAGCAATGCCGAAATCCGTTGCTCGTGCCCCTAAACCACTGGAAAACACTGAAGCAGCA
CAGGCACAGCCGTCTGGAAGCAAATTCTCTCCGGCCATTCCTGTTTCTACCCAGGAGTCCGTTTCTGTTCCAGCA
AGTGTGAGCACCAGCATTAGCAGTATTAGCACCGGTGCCACCGCTAGCGCCCTGGTTAAAGGCAATACCAATCCG
ATTACAAGCATGTCTGCCCCGGTTCAAGCATCAGCTCCAGCACTGACAAAATCCCAAACCGATCGTCTGGAGGTT
CTGCTGAATCCGAAAGACGAAATCAGCCTGAATTCCGGCAAACCGTTTCGTGAACTGGAGAGCGAACTGCTGTCA
CGTCGTAAAAAAGACCTGCAACAAATCTATGCCGAAGAACGTGAGAACTATCTGGGGAAACTGGAACGTGAAATC
ACCCGCTTTTTCGTGGATCGTGGCTTTCTGGAGATCAAATCCCCGATTCTGATTCCTCTGGAGTATATCGAGCGT
ATGGGCATCGACAATGATACCGAACTGAGCAAACAAATTTTCCGTGTGGATAAAAACTTCTGTCTGCGCCCTATG
CTAGCACCAAATCTGGCTAACTATCTGCGCAAACTGGACCGTGCCCTGCCTGATCCTATCAAAATCTTCGAGATC
GGCCCGTGTTATCGTAAAGAGTCCGACGGTAAAGAACATCTGGAGGAGTTTACCATGCTGAACTTTTGCCAAATG
GGTTCAGGTTGTACTCGTGAGAACCTGGAAAGCATCATCACCGATTTTCTGAACCACCTGGGCATTGACTTCAAA
ATTGTGGGCGACAGCTGTATGGTGTTTGGCGACACCCTGGATGTCATGCACGGCGACCTGGAACTGTCTAGTGCC
GTTGTGGGCCCAATCCCGCTGGATCGTGAGTGGGGTATCGACAAACCTTGGATCGGTGCGGGTTTTGGTCTGGAG
CGTCTGCTGAAAGTAAAACACGACTTCAAGAACATCAAACGTGCTGCACGTTCCGAGTCCTATTACAATGGTATT
TCTACTAACCTGTAA (SEQ ID NO: 485)

Protein:
MVGRNSAIAAGVCGALFIGYCIYFDRKRRSDPNFKNRLRERRKKQKLAKERAGLSKLPDLKDAEAVQKFFMASND
YTQQATQSYGAYPTQPGQGYSQQSSQPYGQQSYSGYSQSTDTSGYGQSSYSSYGQSQNTGYGTQSTPQGYGSTGG
YGSSQSSQSSYGQQSSYPGYGQQPAPSSTSGSYGSSSQSSSYGQPQSGSYSQQPSYGGQQQSYGQQQSYNPPQGY
GQQNQYNSSSGGGGGGGGGGNYGQDQSSMSSGGGSGGGYGNQDQSGGGGSGGYGQQDRGGRGRGGSGGGGGGGG
GYNRSSGGYEPRGRGGGRGGRGGMGGSDRGGFNKFGGPRDQGSRHDSEQDNSDNNTIFVQGLGENVTIESVADYF
KQIGIIKTNKKTGQPMINLYTDRETGKLKGEATVSFDDPPSAKAAIDWFDGKEFSGNPIKVSFATRRADFNRGGG
NGRGGRGRGGPMGRGGYGGGGSGGGGRGGFPSGGGGGGGQQRAGDWKCPNPTCENMNFSWRNECNQCKAPKPDGP
GGGPGGSHMGGNYGDDRRGGRGGAIAGKPIPNPLLGLDSTGAPGSAGSAAGSGACPVPLQLPPLERLTLDDKKPL
NTLISATGLWMSRTGTIHKIKHHEVSRSKIYIEMACGDHLVVNNSRSSRTARALRHHKYRKTCKRCRVSDEDLNK
FLTKANEDQTSVKVKVVSAPTRTKKAMPKSVARAPKPLENTEAAQAQPSGSKFSPAIPVSTQESVSVPASVSTSI
SSISTGATASALVKGNTNPITSMSAPVQASAPALTKSQTDRLEVLLNPKDEISLNSGKPFRELESELLSRRKKDL
QQIYAEERENYLGKLEREITRFFVDRGFLEIKSPILIPLEYIERMGIDNDTELSKQIFRVDKNFCLRPMLAPNLA
NYLRKLDRALPDPIKIFEIGPCYRKESDGKEHLEEFTMLNFCQMGSGCTRENLESIITDFLNHLGIDFKIVGDSC
MVFGDTLDVMHGDLELSSAVVGPIPLDREWGIDKPWIGAGFGLERLLKVKHDFKNIKRAARSESYYNGISTNLMA
STDYSTYSQAAAQQGYSAYTAQPTQGYAQTTQAYGQQSYGTYGQPTDVSYTQAQTTATYGQTAYATSYGQPPTGY
TTPTAPQAYSQPVQGYGTGAYDTTTATVTTTQASYAAQSAYGTQPAYPAYGQQPAATAPTRPQDGNKPTETSQPQ
SSTGGYNQPSLGYGQSNYSYPQVPGSYPMQPVTAPPSYPPTSYSSTQPTSYDQSSYSQQNTYGQPSSYGQQSSYG
QQSSYGQQPPTSYPPQTGSYSQAPSQYSQQSSSYGQQSSFRQDHPSSMGVYGQESGGFSGPGENRSMSGPDNRGR
GRGGFDRGGMSRGGRGGGRGGMGSAGERGGFNKPGGPMDEGPDLDLGPPVDPDEDSDNSAIYVQGLNDSVTLDDL
ADFFKQCGVVKMNKRTGQPMIHIYLDKETGKPKGDATVSYEDPPTAKAAVEWFDGKDFQGSKLKVSLARKKPPMN
SMRGGLPPREGRGMPPPLRGGPGGPGGPGGPMGRMGGRGGDRGGFPPRGPRGSRGNPSGGGNVQHRAGDWQCPNP
GCGNQNFAWRTECNQCKAPKPEGFLPPPFPPPGGDRGRGGPGGMRGGRGGLMDRGGPGGMFRGGRGGDRGGFRGG
RGMDRGGFGGGRRGGPGGPPGPLMEQDKKPLNTLISATGLWMSRTGTIHKIKHHEVSRSKIYIEMACGDHLVVNN
SRSSRTARALRHHKYRKTCKRCRVSDEDLNKFLTKANEDQTSVKVKVVSAPTRTKKAMPKSVARAPKPLENTEAA
QAQPSGSKFSPAIPVSTQESVSVPASVSTSISSISTGATASALVKGNTNPITSMSAPVQASAPALTKSQTDRLEV
LLNPKDEISLNSGKPFRELESELLSRRKKDLQQIYAEERENYLGKLEREITRFFVDRGFLEIKSPILIPLEYIER
MGIDNDTELSKQIFRVDKNFCLRPMLAPNLANYLRKLDRALPDPIKIFEIGPCYRKESDGKEHLEEFTMLNFCQM
GSGCTRENLESIITDFLNHLGIDFKIVGDSCMVFGDTLDVMHGDLELSSAVVGPIPLDREWGIDKPWIGAGFGLE
RLLKVKHDFKNIKRAARSESYYNGISTNL* (SEQ ID NO: 486)

TOM20::FUS::PylRS(AF)::FUS::PylRS(AF)
DNA:
ATGGTGGGTCGGAACAGCGCCATCGCCGCCGGTGTATGCGGGGCCCTTTTCATTGGGTACTGCATCTACTTCGAC
CGCAAAAGACGAAGTGACCCCAACTTCAAGAACAGGCTTCGAGAACGAAGAAAGAAACAGAAGCTTGCCAAGGAG
AGAGCTGGGCTTTCCAAGTTACCTGACCTTAAAGATGCTGAAGCTGTTCAGAAATTCTTCATGGCCTCAAACGAT

TATACCCAACAAGCAACCCAAAGCTATGGGGCCTACCCCACCCAGCCCGGGCAGGGCTATTCCCAGCAGAGCAGT
CAGCCCTACGGACAGCAGAGTTACAGTGGTTATAGCCAGTCCACGGACACTTCAGGATATGGCCAGAGCAGCTAT
TCTTCTTATGGCCAGAGCCAGAACACAGGCTATGGAACTCAGTCAACTCCCCAGGGATATGGCTCGACTGGCGGC
TATGGCAGTAGCCAGAGCTCCCAATCGTCTTACGGGCAGCAGTCCTCCTACCCTGGCTATGGCCAGCAGCCAGCT
CCCAGCAGCACCTCGGGAAGTTACGGTAGCAGTTCTCAGAGCAGCAGCTATGGGCAGCCCCAGAGTGGGAGCTAC
AGCCAGCAGCCTAGCTATGGTGGACAGCAGCAAAGCTATGGACAGCAGCAAAGCTATAATCCCCCTCAGGGCTAT
GGACAGCAGAACCAGTACAACAGCAGCAGTGGTGGTGGAGGTGGAGGTGGAGGTGGAGGTAACTATGGCCAAGAT
CAATCCTCCATGAGTAGTGGTGGTGGCAGTGGTGGCGGTTATGGCAATCAAGACCAGAGTGGTGGAGGTGGCAGC
GGTGGCTATGGACAGCAGGACCGTGGAGGCCGCGGCAGGGGTGGCAGTGGTGGCGGCGGCGGCGGCGGCGGTGGT
GGTTACAACCGCAGCAGTGGTGGCTATGAACCCAGAGGTCGTGGAGGTGGCCGTGGAGGCAGAGGTGGCATGGGC
GGAAGTGACCGTGGTGGCTTCAATAAATTTGGTGGCCCTCGGGACCAAGGATCACGTCATGACTCCGAACAGGAT
AATTCAGACAACAACACCATCTTTGTGCAAGGCCTGGGTGAGAATGTTACAATTGAGTCTGTGGCTGATTACTTC
AAGCAGATTGGTATTATTAAGACAAACAAGAAAACGGGACAGCCCATGATTAATTTGTACACAGACAGGGAAACT
GGCAAGCTGAAGGGAGAGGCAACGGTCTCTTTTGATGACCCACCTTCAGCTAAAGCAGCTATTGACTGGTTTGAT
GGTAAAGAATTCTCCGGAAATCCTATCAAGGTCTCATTTGCTACTCGCCGGGCAGACTTTAATCGGGGTGGTGGC
AATGGTCGTGGAGGCCGAGGGCGAGGAGGACCCATGGGCCGTGGAGGCTATGGAGGTGGTGGCAGTGGTGGTGGT
GGCCGAGGAGGATTTCCCAGTGGAGGTGGTGGCGGTGGAGGACAGCAGCGAGCTGGTGACTGGAAGTGTCCTAAT
CCCACCTGTGAGAATATGAACTTCTCTTGGAGGAATGAATGCAACCAGTGTAAGGCCCCTAAACCAGATGGCCCA
GGAGGGGGACCAGGTGGCTCTCACATGGGGGGTAACTACGGGGATGATCGTCGTGGTGGCAGAGGAGGCGCGATC
GCAGGCAAGCCTATTCCCAACCCCCTGCTGGGCCTGGATAGCACCGGAGCACCAGGAAGTGCTGGTTCTGCTGCT
GGTAGTGGAGCGTGCCCGGTGCCGCTGCAGCTGCCGCCGCTGGAACGCCTGACCCTGGATGATAAAAAACCGCTG
AATACCCTGATCTCTGCTACTGGTCTGTGGATGAGTCGTACCGGAACCATTCATAAAATCAAACACCACGAGGTT
AGCCGTTCGAAAATCTATATTGAGATGGCGTGTGGCGATCATCTGGTTGTGAACAATAGCCGCTCTTCTCGTACA
GCACGTGCACTGCGTCACCACAAATATCGTAAAACCTGTAAACGTTGCCGTGTGTCCGATGAGGATCTGAACAAA
TTCCTGACAAAAGCCAATGAGGACCAAACAAGCGTGAAAGTGAAAGTCGTTAGCGCTCCTACCCGTACTAAAAAA
GCAATGCCGAAATCCGTTGCTCGTGCCCCTAAACCACTGGAAAACACTGAAGCAGCACAGGCACAGCCGTCTGGA
AGCAAATTCTCTCCGGCCATTCCTGTTTCTACCCAGGAGTCCGTTTCTGTTCCAGCAAGTGTGAGCACCAGCATT
AGCAGTATTAGCACCGGTGCCACCGCTAGCGCCCTGGTTAAAGGCAATACCAATCCGATTACAAGCATGTCTGCC
CCGGTTCAAGCATCAGCTCCAGCACTGACAAAATCCCAAACCGATCGTCTGGAGGTTCTGCTGAATCCGAAAGAC
GAAATCAGCCTGAATTCCGGCAAACCGTTTCGTGAACTGGAGAGCGAACTGCTGTCACGTCGTAAAAAAGACCTG
CAACAAATCTATGCCGAAGAACGTGAGAACTATCTGGGGAAACTGGAACGTGAAATCACCCGCTTTTTCGTGGAT
CGTGGCTTTCTGGAGATCAAATCCCCGATTCTGATTCCTCTGGAGTATATCGAGCGTATGGGCATCGACAATGAT
ACCGAACTGAGCAAACAAATTTTCCGTGTGGATAAAAACTTCTGTCTGCGCCCTATGCTAGCACCAAATCTGGCT
AACTATCTGCGCAAACTGGACCGTGCCCTGCCTGATCCTATCAAAATCTTCGAGATCGGCCCGTGTTATCGTAAA
GAGTCCGACGGTAAAGAACATCTGGAGGAGTTTACCATGCTGAACTTTTGCCAAATGGGTTCAGGTTGTACTCGT
GAGAACCTGGAAAGCATCATCACCGATTTTCTGAACCACCTGGGCATTGACTTCAAAATTGTGGGCGACAGCTGT
ATGGTGTTTGGCGACACCCTGGATGTCATGCACGGCGACCTGGAACTGTCTAGTGCCGTTGTGGGCCCAATCCCG
CTGGATCGTGAGTGGGGTATCGACAAACCTTGGATCGGTGCGGGTTTTGGTCTGGAGCGTCTGCTGAAAGTAAAA
CACGACTTCAAGAACATCAAACGTGCTGCACGTTCCGAGTCCTATTACAATGGTATTTCTACTAACCTGGCCTCA
AACGATTATACCCAACAAGCAACCCAAAGCTATGGGGCCTACCCCACCCAGCCCGGGCAGGGCTATTCCCAGCAG
AGCAGTCAGCCCTACGGACAGCAGAGTTACAGTGGTTATAGCCAGTCCACGGACACTTCAGGATATGGCCAGAGC
AGCTATTCTTCTTATGGCCAGAGCCAGAACACAGGCTATGGAACTCAGTCAACTCCCCAGGGATATGGCTCGACT
GGCGGCTATGGCAGTAGCCAGAGCTCCCAATCGTCTTACGGGCAGCAGTCCTCCTACCCTGGCTATGGCCAGCAG
CCAGCTCCCAGCAGCACCTCGGGAAGTTACGGTAGCAGTTCTCAGAGCAGCAGCTATGGGCAGCCCCAGAGTGGG
AGCTACAGCCAGCAGCCTAGCTATGGTGGACAGCAGCAAAGCTATGGACAGCAGCAAAGCTATAATCCCCCTCAG
GGCTATGGACAGCAGAACCAGTACAACAGCAGCAGTGGTGGTGGAGGTGGAGGTGGAGGTGGAGGTAACTATGGC
CAAGATCAATCCTCCATGAGTAGTGGTGGTGGCAGTGGTGGCGGTTATGGCAATCAAGACCAGAGTGGTGGAGGT
GGCAGCGGTGGCTATGGACAGCAGGACCGTGGAGGCCGCGGCAGGGGTGGCAGTGGTGGCGGCGGCGGCGGCGGC
GGTGGTGGTTACAACCGCAGCAGTGGTGGCTATGAACCCAGAGGTCGTGGAGGTGGCCGTGGAGGCAGAGGTGGC
ATGGGCGGAAGTGACCGTGGTGGCTTCAATAAATTTGGTGGCCCTCGGGACCAAGGATCACGTCATGACTCCGAA
CAGGATAATTCAGACAACAACACCATCTTTGTGCAAGGCCTGGGTGAGAATGTTACAATTGAGTCTGTGGCTGAT
TACTTCAAGCAGATTGGTATTATTAAGACAAACAAGAAAACGGGACAGCCCATGATTAATTTGTACACAGACAGG
GAAACTGGCAAGCTGAAGGGAGAGGCAACGGTCTCTTTTGATGACCCACCTTCAGCTAAAGCAGCTATTGACTGG
TTTGATGGTAAAGAATTCTCCGGAAATCCTATCAAGGTCTCATTTGCTACTCGCCGGGCAGACTTTAATCGGGGT
GGTGGCAATGGTCGTGGAGGCCGAGGGCGAGGAGGACCCATGGGCCGTGGAGGCTATGGAGGTGGTGGCAGTGGT
GGTGGTGGCCGAGGAGGATTTCCCAGTGGAGGTGGTGGCGGTGGAGGACAGCAGCGAGCTGGTGACTGGAAGTGT
CCTAATCCCACCTGTGAGAATATGAACTTCTCTTGGAGGAATGAATGCAACCAGTGTAAGGCCCCTAAACCAGAT
GGCCCAGGAGGGGGACCAGGTGGCTCTCACATGGGGGGTAACTACGGGGATGATCGTCGTGGTGGCAGAGGAGGC

GATAAAAAACCGCTGAATACCCTGATCTCTGCTACTGGTCTGTGGATGAGTCGTACCGGAACCATTCATAAAATC
AAACACCACGAGGTTAGCCGTTCGAAAATCTATATTGAGATGGCGTGTGGCGATCATCTGGTTGTGAACAATAGC
CGCTCTTCTCGTACAGCACGTGCACTGCGTCACCACAAATATCGTAAAACCTGTAAACGTTGCCGTGTGTCCGAT
GAGGATCTGAACAAATTCCTGACAAAAGCCAATGAGGACCAAACAAGCGTGAAAGTGAAAGTCGTTAGCGCTCCT
ACCCGTACTAAAAAAGCAATGCCGAAATCCGTTGCTCGTGCCCCTAAACCACTGGAAAACACTGAAGCAGCACAG
GCACAGCCGTCTGGAAGCAAATTCTCTCCGGCCATTCCTGTTTCTACCCAGGAGTCCGTTTCTGTTCCAGCAAGT
GTGAGCACCAGCATTAGCAGTATTAGCACCGGTGCCACCGCTAGCGCCCTGGTTAAAGGCAATACCAATCCGATT
ACAAGCATGTCTGCCCCGGTTCAAGCATCAGCTCCAGCACTGACAAAATCCCAAACCGATCGTCTGGAGGTTCTG
CTGAATCCGAAAGACGAAATCAGCCTGAATTCCGGCAAACCGTTTCGTGAACTGGAGAGCGAACTGCTGTCACGT
CGTAAAAAAGACCTGCAACAAATCTATGCCGAAGAACGTGAGAACTATCTGGGGAAACTGGAACGTGAAATCACC
CGCTTTTTCGTGGATCGTGGCTTTCTGGAGATCAAATCCCCGATTCTGATTCCTCTGGAGTATATCGAGCGTATG
GGCATCGACAATGATACCGAACTGAGCAAACAAATTTTCCGTGTGGATAAAAACTTCTGTCTGCGCCCTATGCTA
GCACCAAATCTGGCTAACTATCTGCGCAAACTGGACCGTGCCCTGCCTGATCCTATCAAAATCTTCGAGATCGGC
CCGTGTTATCGTAAAGAGTCCGACGGTAAAGAACATCTGGAGGAGTTTACCATGCTGAACTTTTGCCAAATGGGT
TCAGGTTGTACTCGTGAGAACCTGGAAAGCATCATCACCGATTTTCTGAACCACCTGGGCATTGACTTCAAAATT
GTGGGCGACAGCTGTATGGTGTTTGGCGACACCCTGGATGTCATGCACGGCGACCTGGAACTGTCTAGTGCCGTT
GTGGGCCCAATCCCGCTGGATCGTGAGTGGGGTATCGACAAACCTTGGATCGGTGCGGGTTTTGGTCTGGAGCGT
CTGCTGAAAGTAAAACACGACTTCAAGAACATCAAACGTGCTGCACGTTCCGAGTCCTATTACAATGGTATTTCT
ACTAACCTGTAA (SEQ ID NO: 487)

Protein:
MVGRNSAIAAGVCGALFIGYCIYFDRKRRSDPNFKNRLRERRKKQKLAKERAGLSKLPDLKDAEAVQKFFMASND
YTQQATQSYGAYPTQPGQGYSQQSSQPYGQQSYSGYSQSTDTSGYGQSSYSSYGQSQNTGYGTQSTPQGYGSTGG
YGSSQSSQSSYGQQSSYPGYGQQPAPSSTSGSYGSSSQSSSYGQPQSGSYSQQPSYGGQQQSYGQQQSYNPPQGY
GQQNQYNSSSGGGGGGGGGGNYGQDQSSMSSGGGSGGGYGNQDQSGGGGSGGYGQQDRGGRGRGGSGGGGGGGG
GYNRSSGGYEPRGRGGGRGGRGGMGGSDRGGFNKFGGPRDQGSRHDSEQDNSDNNTIFVQGLGENVTIESVADYF
KQIGIIKTNKKTGQPMINLYTDRETGKLKGEATVSFDDPPSAKAAIDWFDGKEFSGNPIKVSFATRRADFNRGGG
NGRGGRGRGGPMGRGGYGGGGSGGGGRGGFPSGGGGGGGQQRAGDWKCPNPTCENMNFSWRNECNQCKAPKPDGP
GGGPGGSHMGGNYGDDRRGGRGGAIAGKPIPNPLLGLDSTGAPGSAGSAAGSGACPVPLQLPPLERLTLDDKKPL
NTLISATGLWMSRTGTIHKIKHHEVSRSKIYIEMACGDHLVVNNSRSSRTARALRHHKYRKTCKRCRVSDEDLNK
FLTKANEDQTSVKVKVVSAPTRTKKAMPKSVARAPKPLENTEAAQAQPSGSKFSPAIPVSTQESVSVPASVSTSI
SSISTGATASALVKGNTNPITSMSAPVQASAPALTKSQTDRLEVLLNPKDEISLNSGKPFRELESELLSRRKKDL
QQIYAEERENYLGKLEREITRFFVDRGFLEIKSPILIPLEYIERMGIDNDTELSKQIFRVDKNFCLRPMLAPNLA
NYLRKLDRALPDPIKIFEIGPCYRKESDGKEHLEEFTMLNFCQMGSGCTRENLESIITDFLNHLGIDFKIVGDSC
MVFGDTLDVMHGDLELSSAVVGPIPLDREWGIDKPWIGAGFGLERLLKVKHDFKNIKRAARSESYYNGISTNLAS
NDYTQQATQSYGAYPTQPGQGYSQQSSQPYGQQSYSGYSQSTDTSGYGQSSYSSYGQSQNTGYGTQSTPQGYGST
GGYGSSQSSQSSYGQQSSYPGYGQQPAPSSTSGSYGSSSQSSSYGQPQSGSYSQQPSYGGQQQSYGQQQSYNPPQ
GYGQQNQYNSSSGGGGGGGGGGNYGQDQSSMSSGGGSGGGYGNQDQSGGGGSGGYGQQDRGGRGRGGSGGGGGGG
GGGYNRSSGGYEPRGRGGGRGGRGGMGGSDRGGFNKFGGPRDQGSRHDSEQDNSDNNTIFVQGLGENVTIESVAD
YFKQIGIIKTNKKTGQPMINLYTDRETGKLKGEATVSFDDPPSAKAAIDWFDGKEFSGNPIKVSFATRRADFNRG
GGNGRGGRGRGGPMGRGGYGGGGSGGGGRGGFPSGGGGGGGQQRAGDWKCPNPTCENMNFSWRNECNQCKAPKPD
GPGGGPGGSHMGGNYGDDRRGGRGGDKKPLNTLISATGLWMSRTGTIHKIKHHEVSRSKIYIEMACGDHLVVNNS
RSSRTARALRHHKYRKTCKRCRVSDEDLNKFLTKANEDQTSVKVKVVSAPTRTKKAMPKSVARAPKPLENTEAAQ
AQPSGSKFSPAIPVSTQESVSVPASVSTSISSISTGATASALVKGNTNPITSMSAPVQASAPALTKSQTDRLEVL
LNPKDEISLNSGKPFRELESELLSRRKKDLQQIYAEERENYLGKLEREITRFFVDRGFLEIKSPILIPLEYIERM
GIDNDTELSKQIFRVDKNFCLRPMLAPNLANYLRKLDRALPDPIKIFEIGPCYRKESDGKEHLEEFTMLNFCQMG
SGCTRENLESIITDFLNHLGIDFKIVGDSCMVFGDTLDVMHGDLELSSAVVGPIPLDREWGIDKPWIGAGFGLER
LLKVKHDFKNIKRAARSESYYNGISTNL* (SEQ ID NO: 488)

TOM20::EWSR1::4xλN22::PylRS(AA)::FUS::PylRS(AA)
DNA:
ATGGTGGGTCGGAACAGCGCCATCGCCGCCGGTGTATGCGGGGCCCTTTTCATTGGGTACTGCATCTACTTCGAC
CGCAAAAGACGAAGTGACCCCAACTTCAAGAACAGGCTTCGAGAACGAAGAAAGAAACAGAAGCTTGCCAAGGAG
AGAGCTGGGCTTTCCAAGTTACCTGACCTTAAAGATGCTGAAGCTGTTCAGAAGTTCTTCATGGCGTCCACGGAT
TACAGTACCTATAGCCAAGCTGCAGCGCAGCAGGGCTACAGTGCTTACACCGCCCAGCCCACTCAAGGATATGCA
CAGACCACCCAGGCATATGGGCAACAAAGCTATGGAACCTATGGACAGCCCACTGATGTCAGCTATACCCAGGCT
CAGACCACTGCAACCTATGGGCAGACCGCCTATGCAACTTCTTATGGACAGCCTCCCACTGGTTATACTACTCCA

ACTGCCCCCCAGGCATACAGCCAGCCTGTCCAGGGGTATGGCACTGGTGCTTATGATACCACCACTGCTACAGTC
ACCACCACCCAGGCCTCCTATGCAGCTCAGTCTGCATATGGCACTCAGCCTGCTTATCCAGCCTATGGGCAGCAG
CCAGCAGCCACTGCACCTACAAGACCGCAGGATGGAAACAAGCCCACTGAGACTAGTCAACCTCAATCTAGCACA
GGGGGTTACAACCAGCCCAGCCTAGGATATGGACAGAGTAACTACAGTTATCCCCAGGTACCTGGGAGCTACCCC
ATGCAGCCAGTCACTGCACCTCCATCCTACCCTCCTACCAGCTATTCCTCTACACAGCCGACTAGTTATGATCAG
AGCAGTTACTCTCAGCAGAACACCTATGGGCAACCGAGCAGCTATGGACAGCAGAGTAGCTATGGTCAACAAAGC
AGCTATGGGCAGCAGCCTCCCACTAGTTACCCACCCCAAACTGGATCCTACAGCCAAGCTCCAAGTCAATATAGC
CAACAGAGCAGCAGCTACGGGCAGCAGAGTTCATTCCGACAGGACCACCCCAGTAGCATGGGTGTTTATGGGCAG
GAGTCTGGAGGATTTTCCGGACCAGGAGAGAACCGGAGCATGAGTGGCCCTGATAACCGGGGCAGGGGAAGAGGG
GGATTTGATCGTGGAGGCATGAGCAGAGGTGGGCGGGGAGGAGGACGCGGTGGAATGGGCAGCGCTGGAGAGCGA
GGTGGCTTCAATAAGCCTGGTGGACCCATGGATGAAGGACCAGATCTTGATCTAGGCCCACCTGTAGATCCAGAT
GAAGACTCTGACAACAGTGCAATTTATGTACAAGGATTAAATGACAGTGTGACTCTAGATGATCTGGCAGACTTC
TTTAAGCAGTGTGGGGTTGTTAAGATGAACAAGAGAACTGGGCAACCCATGATCCACATCTACCTGGACAAGGAA
ACAGGAAAGCCCAAAGGCGATGCCACAGTGTCCTATGAAGACCCACCTACTGCCAAGGCTGCCGTGGAATGGTTT
GATGGGAAAGATTTTCAAGGGAGCAAACTTAAAGTCTCCCTTGCTCGGAAGAAGCCTCCAATGAACAGTATGCGG
GGTGGTCTGCCACCCCGTGAGGGCAGAGGCATGCCACCACCACTCCGTGGAGGTCCAGGAGGCCCAGGAGGTCCT
GGGGGACCCATGGGTCGCATGGGAGGCCGTGGAGGAGATAGAGGAGGCTTCCCTCCAAGAGGACCCCGGGGTTCC
CGAGGGAACCCCTCTGGAGGAGGAAACGTCCAGCACCGAGCTGGAGACTGGCAGTGTCCCAATCCGGGTTGTGGA
AACCAGAACTTCGCCTGGAGAACAGAGTGCAACCAGTGTAAGGCCCCAAAGCCTGAAGGCTTCCTCCCGCCACCC
TTTCCGCCCCCGGGTGGTGATCGTGGCAGAGGTGGCCCTGGTGGCATGCGGGGAGGAAGAGGTGGCCTCATGGAT
CGTGGTGGTCCCGGTGGAATGTTCAGAGGTGGCCGTGGTGGAGACAGAGGTGGCTTCCGTGGTGGCCGGGGCATG
GACCGAGGTGGCTTTGGTGGAGGAAGACGAGGTGGCCCTGGGGGGCCCCCTGGACCTTTGATGGAACAGGCGATC
GCAGGAGCACCAGGAAGTGCTGGTTCTGCTGCTGGTAGTGGAGAGCAGAAGCTGATCTCAGAGGAGGACCTGCTA
GCCACCATGGACGCACAAACACGACGACGTGAGCGTCGCGCTGAGAAACAAGCTCAATGGAAAGCTGCAAACCCA
CCGCTCGACGGAGCCGGAGCTGGCGCTGGAGCTGGAGCCGGAGCTGGCGGTCTAGCCACCATGGACGCACAAACA
CGACGACGTGAGCGTCGCGCTGAGAAACAAGCTCAATGGAAAGCTGCAAACCCACCGCTCGACGGAGCCGGAGCT
GGCGCTGGAGCTGGAGCCGGAGCTGGCGGTCTAGCCACCATGGACGCACAAACACGACGACGTGAGCGTCGCGCT
GAGAAACAAGCTCAATGGAAAGCTGCAAACCCACCGCTCGACGGAGCCGGAGCTGGCGCTGGAGCTGGAGCCGGA
GCTGGCGGTCTAGCCACCATGGACGCACAAACACGACGACGTGAGCGTCGCGCTGAGAAACAAGCTCAATGGAAA
GCTGCAAACCCACCGCTCGGGCTAAGCCCCGACCGCGTTAGAGCCGTATCCCACTGGTCTTCCGCGTGCCCGGTG
CCGCTGCAGCTGCCGCCGCTGGAACGCCTGACCCTGGATGATAAAAAACCGCTGAATACCCTGATCTCTGCTACT
GGTCTGTGGATGAGTCGTACCGGAACCATTCATAAAATCAAACACCACGAGGTTAGCCGTTCGAAAATCTATATT
GAGATGGCGTGTGGCGATCATCTGGTTGTGAACAATAGCCGCTCTTCTCGTACAGCACGTGCACTGCGTCACCAC
AAATATCGTAAAACCTGTAAACGTTGCCGTGTGTCCGATGAGGATCTGAACAAATTCCTGACAAAAGCCAATGAG
GACCAAACAAGCGTGAAAGTGAAAGTCGTTAGCGCTCCTACCCGTACTAAAAAAGCAATGCCGAAATCCGTTGCT
CGTGCCCCTAAACCACTGGAAAACACTGAAGCAGCACAGGCACAGCCGTCTGGAAGCAAATTCTCTCCGGCCATT
CCTGTTTCTACCCAGGAGTCCGTTTCTGTTCCAGCAAGTGTGAGCACCAGCATTAGCAGTATTAGCACCGGTGCC
ACCGCTAGCGCCCTGGTTAAAGGCAATACCAATCCGATTACAAGCATGTCTGCCCCGGTTCAAGCATCAGCTCCA
GCACTGACAAAATCCCAAACCGATCGTCTGGAGGTTCTGCTGAATCCGAAAGACGAAATCAGCCTGAATTCCGGC
AAACCGTTTCGTGAACTGGAGAGCGAACTGCTGTCACGTCGTAAAAAAGACCTGCAACAAATCTATGCCGAAGAA
CGTGAGAACTATCTGGGGAAACTGGAACGTGAAATCACCCGCTTTTTCGTGGATCGTGGCTTTCTGGAGATCAAA
TCCCCGATTCTGATTCCTCTGGAGTATATCGAGCGTATGGGCATCGACAATGATACCGAACTGAGCAAACAAATT
TTCCGTGTGGATAAAAACTTCTGTCTGCGCCCTATGCTGGCACCAAATCTGTATAACTATCTGCGCAAACTGGAC
CGTGCCCTGCCTGATCCTATCAAAATCTTCGAGATCGGCCCGTGTTATCGTAAAGAGTCCGACGGTAAAGAACAT
CTGGAGGAGTTTACCATGCTGGCCTTTGCCCAAATGGGTTCAGGTTGTACTCGTGAGAACCTGGAAAGCATCATC
ACCGATTTTCTGAACCACCTGGGCATTGACTTCAAAATTGTGGGCGACAGCTGTATGGTGTATGGCGACACCCTG
GATGTCATGCACGGCGACCTGGAACTGTCTAGTGCCGTTGTTGGACCAATTCCGCTGGACCGTGAGTGGGGTATC
GACAAACCGTGGATCGGAGCAGGATTCGGTCTGGAACGCCTGCTGAAAGTGAAACACGACTTCAAAAACATCAAA
CGTGCCGCCCGTTCTGAATCGTATTATAACGGGATCTCTACGAACCTGGCCTCAAACGATTATACCCAACAAGCA
ACCCAAAGCTATGGGGCCTACCCCACCCAGCCCGGGCAGGGCTATTCCCAGCAGAGCAGTCAGCCCTACGGACAG
CAGAGTTACAGTGGTTATAGCCAGTCCACGGACACTTCAGGATATGGCCAGAGCAGCTATTCTTCTTATGGCCAG
AGCCAGAACACAGGCTATGGAACTCAGTCAACTCCCCAGGGATATGGCTCGACTGGCGGCTATGGCAGTAGCCAG
AGCTCCCAATCGTCTTACGGGCAGCAGTCCTCCTACCCTGGCTATGGCCAGCAGCCAGCTCCCAGCAGCACCTCG
GGAAGTTACGGTAGCAGTTCTCAGAGCAGCAGCTATGGGCAGCCCCAGAGTGGGAGCTACAGCCAGCAGCCTAGC
TATGGTGGACAGCAGCAAAGCTATGGACAGCAGCAAAGCTATAATCCCCCTCAGGGCTATGGACAGCAGAACCAG
TACAACAGCAGCAGTGGTGGTGGAGGTGGAGGTGGAGGTGGAGGTAACTATGGCCAAGATCAATCCTCCATGAGT
AGTGGTGGTGGCAGTGGTGGCGGTTATGGCAATCAAGACCAGAGTGGTGGAGGTGGCAGCGGTGGCTATGGACAG
CAGGACCGTGGAGGCCGCGGCAGGGGTGGCAGTGGTGGCGGCGGCGGCGGCGGTGGTGGTTACAACCGCAGC

AGTGGTGGCTATGAACCCAGAGGTCGTGGAGGTGGCCGTGGAGGCAGAGGTGGCATGGGCGGAAGTGACCGTGGT
GGCTTCAATAAATTTGGTGGCCCTCGGGACCAAGGATCACGTCATGACTCCGAACAGGATAATTCAGACAACAAC
ACCATCTTTGTGCAAGGCCTGGGTGAGAATGTTACAATTGAGTCTGTGGCTGATTACTTCAAGCAGATTGGTATT
ATTAAGACAAACAAGAAAACGGGACAGCCCATGATTAATTTGTACACAGACAGGGAAACTGGCAAGCTGAAGGGA
GAGGCAACGGTCTCTTTTGATGACCCACCTTCAGCTAAAGCAGCTATTGACTGGTTTGATGGTAAAGAATTCTCC
GGAAATCCTATCAAGGTCTCATTTGCTACTCGCCGGGCAGACTTTAATCGGGGTGGTGGCAATGGTCGTGGAGGC
CGAGGGCGAGGAGGACCCATGGGCCGTGGAGGCTATGGAGGTGGTGGCAGTGGTGGTGGTGGCCGAGGAGGATTT
CCCAGTGGAGGTGGTGGCGGTGGAGGACAGCAGCGAGCTGGTGACTGGAAGTGTCCTAATCCCACCTGTGAGAAT
ATGAACTTCTCTTGGAGGAATGAATGCAACCAGTGTAAGGCCCCTAAACCAGATGGCCCAGGAGGGGGACCAGGT
GGCTCTCACATGGGGGGTAACTACGGGGATGATCGTCGTGGTGGCAGAGGAGGCGATAAAAAACCGCTGAATACC
CTGATCTCTGCTACTGGTCTGTGGATGAGTCGTACCGGAACCATTCATAAAATCAAACACCACGAGGTTAGCCGT
TCGAAAATCTATATTGAGATGGCGTGTGGCGATCATCTGGTTGTGAACAATAGCCGCTCTTCTCGTACAGCACGT
GCACTGCGTCACCACAAATATCGTAAAACCTGTAAACGTTGCCGTGTGTCCGATGAGGATCTGAACAAATTCCTG
ACAAAAGCCAATGAGGACCAAACAAGCGTGAAAGTGAAAGTCGTTAGCGCTCCTACCCGTACTAAAAAAGCAATG
CCGAAATCCGTTGCTCGTGCCCCTAAACCACTGGAAAACACTGAAGCAGCACAGGCACAGCCGTCTGGAAGCAAA
TTCTCTCCGGCCATTCCTGTTTCTACCCAGGAGTCCGTTTCTGTTCCAGCAAGTGTGAGCACCAGCATTAGCAGT
ATTAGCACCGGTGCCACCGCTAGCGCCCTGGTTAAAGGCAATACCAATCCGATTACAAGCATGTCTGCCCCGGTT
CAAGCATCAGCTCCAGCACTGACAAAATCCCAAACCGATCGTCTGGAGGTTCTGCTGAATCCGAAAGACGAAATC
AGCCTGAATTCCGGCAAACCGTTTCGTGAACTGGAGAGCGAACTGCTGTCACGTCGTAAAAAAGACCTGCAACAA
ATCTATGCCGAAGAACGTGAGAACTATCTGGGGAAACTGGAACGTGAAATCACCCGCTTTTTCGTGGATCGTGGC
TTTCTGGAGATCAAATCCCCGATTCTGATTCCTCTGGAGTATATCGAGCGTATGGGCATCGACAATGATACCGAA
CTGAGCAAACAAATTTTCCGTGTGGATAAAAACTTCTGTCTGCGCCCTATGCTGGCACCAAATCTGTATAACTAT
CTGCGCAAACTGGACCGTGCCCTGCCTGATCCTATCAAAATCTTCGAGATCGGCCCGTGTTATCGTAAAGAGTCC
GACGGTAAAGAACATCTGGAGGAGTTTACCATGCTGGCCTTTGCCCAAATGGGTTCAGGTTGTACTCGTGAGAAC
CTGGAAAGCATCATCACCGATTTTCTGAACCACCTGGGCATTGACTTCAAAATTGTGGGCGACAGCTGTATGGTG
TATGGCGACACCCTGGATGTCATGCACGGCGACCTGGAACTGTCTAGTGCCGTTGTTGGACCAATTCCGCTGGAC
CGTGAGTGGGGTATCGACAAACCGTGGATCGGAGCAGGATTCGGTCTGGAACGCCTGCTGAAAGTGAAACACGAC
TTCAAAAACATCAAACGTGCCGCCCGTTCTGAATCGTATTATAACGGGATCTCTACGAACCTGTAA (SEQ ID
NO: 489)

Protein:
MVGRNSAIAAGVCGALFIGYCIYFDRKRRSDPNFKNRLRERRKKQKLAKERAGLSKLPDLKDAEAVQKFFMASTD
YSTYSQAAAQQGYSAYTAQPTQGYAQTTQAYGQQSYGTYGQPTDVSYTQAQTTATYGQTAYATSYGQPPTGYTTP
TAPQAYSQPVQGYGTGAYDTTTATVTTTQASYAAQSAYGTQPAYPAYGQQPAATAPTRPQDGNKPTETSQPQSST
GGYNQPSLGYGQSNYSYPQVPGSYPMQPVTAPPSYPPTSYSSTQPTSYDQSSYSQQNTYGQPSSYGQQSSYGQQS
SYGQQPPTSYPPQTGSYSQAPSQYSQQSSSYGQQSSFRQDHPSSMGVYGQESGGFSGPGENRSMSGPDNRGRGRG
GFDRGGMSRGGRGGGRGGMGSAGERGGFNKPGGPMDEGPDLDLGPPVDPDEDSDNSAIYVQGLNDSVTLDDLADF
FKQCGVVKMNKRTGQPMIHIYLDKETGKPKGDATVSYEDPPTAKAAVEWFDGKDFQGSKLKVSLARKKPPMNSMR
GGLPPREGRGMPPPLRGGPGGPGGPGGPMGRMGGRGGDRGGFPPRGPRGSRGNPSGGGNVQHRAGDWQCPNPGCG
NQNFAWRTECNQCKAPKPEGFLPPPFPPPGGDRGRGGPGGMRGGRGGLMDRGGPGGMFRGGRGGDRGGFRGGRGM
DRGGFGGGRRGGPGGPPGPLMEQAIAGAPGSAGSAAGSGEQKLISEEDLLATMDAQTRRRERRAEKQAQWKAANP
PLDGAGAGAGAGAGAGGLATMDAQTRRRERRAEKQAQWKAANPPLDGAGAGAGAGAGAGGLATMDAQTRRRERRA
EKQAQWKAANPPLDGAGAGAGAGAGAGGLATMDAQTRRRERRAEKQAQWKAANPPLGLSPDRVRAVSHWSSACPV
PLQLPPLERLTLDDKKPLNTLISATGLWMSRTGTIHKIKHHEVSRSKIYIEMACGDHLVVNNSRSSRTARALRHH
KYRKTCKRCRVSDEDLNKFLTKANEDQTSVKVKVVSAPTRTKKAMPKSVARAPKPLENTEAAQAQPSGSKFSPAI
PVSTQESVSVPASVSTSISSISTGATASALVKGNTNPITSMSAPVQASAPALTKSQTDRLEVLLNPKDEISLNSG
KPFRELESELLSRRKKDLQQIYAEERENYLGKLEREITRFFVDRGFLEIKSPILIPLEYIERMGIDNDTELSKQI
FRVDKNFCLRPMLAPNLYNYLRKLDRALPDPIKIFEIGPCYRKESDGKEHLEEFTMLAFAQMGSGCTRENLESII
TDFLNHLGIDFKIVGDSCMVYGDTLDVMHGDLELSSAVVGPIPLDREWGIDKPWIGAGFGLERLLKVKHDFKNIK
RAARSESYYNGISTNLASNDYTQQATQSYGAYPTQPGQGYSQQSSQPYGQQSYSGYSQSTDTSGYGQSSYSSYGQ
SQNTGYGTQSTPQGYGSTGGYGSSQSSQSSYGQQSSYPGYGQQPAPSSTSGSYGSSSQSSSYGQPQSGSYSQQPS
YGGQQQSYGQQQSYNPPQGYGQQNQYNSSSGGGGGGGGGGNYGQDQSSMSSGGGSGGGYGNQDQSGGGGSGGYGQ
QDRGGRGRGGSGGGGGGGGGGYNRSSGGYEPRGRGGGRGGRGGMGGSDRGGFNKFGGPRDQGSRHDSEQDNSDNN
TIFVQGLGENVTIESVADYFKQIGIIKTNKKTGQPMINLYTDRETGKLKGEATVSFDDPPSAKAAIDWFDGKEFS
GNPIKVSFATRRADFNRGGGNGRGGRGRGGPMGRGGYGGGGSGGGGRGGFPSGGGGGGGQQRAGDWKCPNPTCEN
MNFSWRNECNQCKAPKPDGPGGGPGGSHMGGNYGDDRRGGRGGDKKPLNTLISATGLWMSRTGTIHKIKHHEVSR
SKIYIEMACGDHLVVNNSRSSRTARALRHHKYRKTCKRCRVSDEDLNKFLTKANEDQTSVKVKVVSAPTRTKKAM
PKSVARAPKPLENTEAAQAQPSGSKFSPAIPVSTQESVSVPASVSTSISSISTGATASALVKGNTNPITSMSAPV

QASAPALTKSQTDRLEVLLNPKDEISLNSGKPFRELESELLSRRKKDLQQIYAEERENYLGKLEREITRFFVDRG
FLEIKSPILIPLEYIERMGIDNDTELSKQIFRVDKNFCLRPMLAPNLYNYLRKLDRALPDPIKIFEIGPCYRKES
DGKEHLEEFTMLAFAQMGSGCTRENLESIITDFLNHLGIDFKIVGDSCMVYGDTLDVMHGDLELSSAVVGPIPLD
REWGIDKPWIGAGFGLERLLKVKHDFKNIKRAARSESYYNGISTNL* (SEQ ID NO: 490)

LCK::EWSR1::MCP::PylRS(AA)::FUS::PylRS(AA)
DNA:
ATGGGCTGCGTGTGCAGCAGCAACCCCGAGGGTACCGAGCTCATGGCGTCCACGGATTACAGTACCTATAGCCAA
GCTGCAGCGCAGCAGGGCTACAGTGCTTACACCGCCCAGCCCACTCAAGGATATGCACAGACCACCCAGGCATAT
GGGCAACAAAGCTATGGAACCTATGGACAGCCCACTGATGTCAGCTATACCCAGGCTCAGACCACTGCAACCTAT
GGGCAGACCGCCTATGCAACTTCTTATGGACAGCCTCCCACTGGTTATACTACTCCAACTGCCCCCCAGGCATAC
AGCCAGCCTGTCCAGGGGTATGGCACTGGTGCTTATGATACCACCACTGCTACAGTCACCACCACCCAGGCCTCC
TATGCAGCTCAGTCTGCATATGGCACTCAGCCTGCTTATCCAGCCTATGGGCAGCAGCCAGCAGCCACTGCACCT
ACAAGACCGCAGGATGGAAACAAGCCCACTGAGACTAGTCAACCTCAATCTAGCACAGGGGGTTACAACCAGCCC
AGCCTAGGATATGGACAGAGTAACTACAGTTATCCCCAGGTACCTGGGAGCTACCCCATGCAGCCAGTCACTGCA
CCTCCATCCTACCCTCCTACCAGCTATTCCTCTACACAGCCGACTAGTTATGATCAGAGCAGTTACTCTCAGCAG
AACACCTATGGGCAACCGAGCAGCTATGGACAGCAGAGTAGCTATGGTCAACAAAGCAGCTATGGGCAGCAGCCT
CCCACTAGTTACCCACCCCAAACTGGATCCTACAGCCAAGCTCCAAGTCAATATAGCCAACAGAGCAGCAGCTAC
GGGCAGCAGAGTTCATTCCGACAGGACCACCCCAGTAGCATGGGTGTTTATGGGCAGGAGTCTGGAGGATTTTCC
GGACCAGGAGAGAACCGGAGCATGAGTGGCCCTGATAACCGGGGCAGGGGAAGAGGGGGATTTGATCGTGGAGGC
ATGAGCAGAGGTGGGCGGGGAGGAGGACGCGGTGGAATGGGCAGCGCTGGAGAGCGAGGTGGCTTCAATAAGCCT
GGTGGACCCATGGATGAAGGACCAGATCTTGATCTAGGCCCACCTGTAGATCCAGATGAAGACTCTGACAACAGT
GCAATTTATGTACAAGGATTAAATGACAGTGTGACTCTAGATGATCTGGCAGACTTCTTTAAGCAGTGTGGGGTT
GTTAAGATGAACAAGAGAACTGGGCAACCCATGATCCACATCTACCTGGACAAGGAAACAGGAAAGCCCAAAGGC
GATGCCACAGTGTCCTATGAAGACCCACCTACTGCCAAGGCTGCCGTGGAATGGTTTGATGGGAAAGATTTTCAA
GGGAGCAAACTTAAAGTCTCCCTTGCTCGGAAGAAGCCTCCAATGAACAGTATGCGGGGTGGTCTGCCACCCCGT
GAGGGCAGAGGCATGCCACCACCACTCCGTGGAGGTCCAGGAGGCCCAGGAGGTCCTGGGGGACCCATGGGTCGC
ATGGGAGGCCGTGGAGGAGATAGAGGAGGCTTCCCTCCAAGAGGACCCCGGGGTTCCCGAGGGAACCCCTCTGGA
GGAGGAAACGTCCAGCACCGAGCTGGAGACTGGCAGTGTCCCAATCCGGGTTGTGGAAACCAGAACTTCGCCTGG
AGAACAGAGTGCAACCAGTGTAAGGCCCCAAAGCCTGAAGGCTTCCTCCCGCCACCCTTTCCGCCCCCGGGTGGT
GATCGTGGCAGAGGTGGCCCTGGTGGCATGCGGGGAGGAAGAGGTGGCCTCATGGATCGTGGTGGTCCCGGTGGA
ATGTTCAGAGGTGGCCGTGGTGGAGACAGAGGTGGCTTCCGTGGTGGCCGGGGCATGGACCGAGGTGGCTTTGGT
GGAGGAAGACGAGGTGGCCCTGGGGGGCCCCCTGGACCTTTGATGGAACAGGCGATCGCATATCCCTATGATGTG
CCGGATTATGCTGGAGCACCAGGAAGTGCTGGTTCTGCTGCTGGTAGTGGAGCTTCTAACTTTACTCAGTTCGTT
CTCGTCGACAATGGCGGAACTGGCGACGTGACTGTCGCCCCAAGCAACTTCGCTAACGGGATCGCTGAATGGATC
AGCTCTAACTCGCGTTCACAGGCTTACAAAGTAACCTGTAGCGTTCGTCAGAGCTCTGCGCAGAATCGCAAATAC
ACCATCAAAGTCGAGGTGCCTAAAGGCGCCTGGCGTTCGTACTTAAATATGGAACTAACCATTCCAATTTTCGCC
ACGAATTCCGACTGCGAGCTTATTGTTAAGGCAATGCAAGGTCTCCTAAAAGATGGAAACCCGATTCCCTCAGCA
ATCGCAGCAAACTCCGGCATCTACGGGCTAAGCCCCGACCGCGTTAGAGCCGTATCCCACTGGTCTTCCGCGTGC
CCGGTGCCGCTGCAGCTGCCGCCGCTGGAACGCCTGACCCTGGATGATAAAAAACCGCTGAATACCCTGATCTCT
GCTACTGGTCTGTGGATGAGTCGTACCGGAACCATTCATAAAATCAAACACCACGAGGTTAGCCGTTCGAAAATC
TATATTGAGATGGCGTGTGGCGATCATCTGGTTGTGAACAATAGCCGCTCTTCTCGTACAGCACGTGCACTGCGT
CACCACAAATATCGTAAAACCTGTAAACGTTGCCGTGTGTCCGATGAGGATCTGAACAAATTCCTGACAAAAGCC
AATGAGGACCAAACAAGCGTGAAAGTGAAAGTCGTTAGCGCTCCTACCCGTACTAAAAAAGCAATGCCGAAATCC
GTTGCTCGTGCCCCTAAACCACTGGAAAACACTGAAGCAGCACAGGCACAGCCGTCTGGAAGCAAATTCTCTCCG
GCCATTCCTGTTTCTACCCAGGAGTCCGTTTCTGTTCCAGCAAGTGTGAGCACCAGCATTAGCAGTATTAGCACC
GGTGCCACCGCTAGCGCCCTGGTTAAAGGCAATACCAATCCGATTACAAGCATGTCTGCCCCGGTTCAAGCATCA
GCTCCAGCACTGACAAAATCCCAAACCGATCGTCTGGAGGTTCTGCTGAATCCGAAAGACGAAATCAGCCTGAAT
TCCGGCAAACCGTTTCGTGAACTGGAGAGCGAACTGCTGTCACGTCGTAAAAAAGACCTGCAACAAATCTATGCC
GAAGAACGTGAGAACTATCTGGGGAAACTGGAACGTGAAATCACCCGCTTTTTCGTGGATCGTGGCTTTCTGGAG
ATCAAATCCCCGATTCTGATTCCTCTGGAGTATATCGAGCGTATGGGCATCGACAATGATACCGAACTGAGCAAA
CAAATTTTCCGTGTGGATAAAAACTTCTGTCTGCGCCCTATGCTGGCACCAAATCTGTATAACTATCTGCGCAAA
CTGGACCGTGCCCTGCCTGATCCTATCAAAATCTTCGAGATCGGCCCGTGTTATCGTAAAGAGTCCGACGGTAAA
GAACATCTGGAGGAGTTTACCATGCTGGCCTTTGCCCAAATGGGTTCAGGTTGTACTCGTGAGAACCTGGAAAGC
ATCATCACCGATTTTCTGAACCACCTGGGCATTGACTTCAAAATTGTGGGCGACAGCTGTATGGTGTATGGCGAC
ACCCTGGATGTCATGCACGGCGACCTGGAACTGTCTAGTGCCGTTGTTGGACCAATTCCGCTGGACCGTGAGTGG
GGTATCGACAAACCGTGGATCGGAGCAGGATTCGGTCTGGAACGCCTGCTGAAAGTGAAACACGACTTCAAAAAC

ATCAAACGTGCCGCCCGTTCTGAATCGTATTATAACGGGATCTCTACGAACCTGGCCTCAAACGATTATACCCAA
CAAGCAACCCAAAGCTATGGGGCCTACCCCACCCAGCCCGGGCAGGGCTATTCCCAGCAGAGCAGTCAGCCCTAC
GGACAGCAGAGTTACAGTGGTTATAGCCAGTCCACGGACACTTCAGGATATGGCCAGAGCAGCTATTCTTCTTAT
GGCCAGAGCCAGAACACAGGCTATGGAACTCAGTCAACTCCCCAGGGATATGGCTCGACTGGCGGCTATGGCAGT
AGCCAGAGCTCCCAATCGTCTTACGGGCAGCAGTCCTCCTACCCTGGCTATGGCCAGCAGCCAGCTCCCAGCAGC
ACCTCGGGAAGTTACGGTAGCAGTTCTCAGAGCAGCAGCTATGGGCAGCCCCAGAGTGGGAGCTACAGCCAGCAG
CCTAGCTATGGTGGACAGCAGCAAAGCTATGGACAGCAGCAAAGCTATAATCCCCCTCAGGGCTATGGACAGCAG
AACCAGTACAACAGCAGCAGTGGTGGTGGAGGTGGAGGTGGAGGTGGAGGTAACTATGGCCAAGATCAATCCTCC
ATGAGTAGTGGTGGTGGCAGTGGTGGCGGTTATGGCAATCAAGACCAGAGTGGTGGAGGTGGCAGCGGTGGCTAT
GGACAGCAGGACCGTGGAGGCCGCGGCAGGGGTGGCAGTGGTGGCGGCGGCGGCGGCGGCGGTGGTGGTTACAAC
CGCAGCAGTGGTGGCTATGAACCCAGAGGTCGTGGAGGTGGCCGTGGAGGCAGAGGTGGCATGGGCGGAAGTGAC
CGTGGTGGCTTCAATAAATTTGGTGGCCCTCGGGACCAAGGATCACGTCATGACTCCGAACAGGATAATTCAGAC
AACAACACCATCTTTGTGCAAGGCCTGGGTGAGAATGTTACAATTGAGTCTGTGGCTGATTACTTCAAGCAGATT
GGTATTATTAAGACAAACAAGAAAACGGGACAGCCCATGATTAATTTGTACACAGACAGGGAAACTGGCAAGCTG
AAGGGAGAGGCAACGGTCTCTTTTGATGACCCACCTTCAGCTAAAGCAGCTATTGACTGGTTTGATGGTAAAGAA
TTCTCCGGAAATCCTATCAAGGTCTCATTTGCTACTCGCCGGGCAGACTTTAATCGGGGTGGTGGCAATGGTCGT
GGAGGCCGAGGGCGAGGAGGACCCATGGGCCGTGGAGGCTATGGAGGTGGTGGCAGTGGTGGTGGTGGCCGAGGA
GGATTTCCCAGTGGAGGTGGTGGCGGTGGAGGACAGCAGCGAGCTGGTGACTGGAAGTGTCCTAATCCCACCTGT
GAGAATATGAACTTCTCTTGGAGGAATGAATGCAACCAGTGTAAGGCCCCTAAACCAGATGGCCCAGGAGGGGGA
CCAGGTGGCTCTCACATGGGGGGTAACTACGGGGATGATCGTCGTGGTGGCAGAGGAGGCGATAAAAAACCGCTG
AATACCCTGATCTCTGCTACTGGTCTGTGGATGAGTCGTACCGGAACCATTCATAAAATCAAACACCACGAGGTT
AGCCGTTCGAAAATCTATATTGAGATGGCGTGTGGCGATCATCTGGTTGTGAACAATAGCCGCTCTTCTCGTACA
GCACGTGCACTGCGTCACCACAAATATCGTAAAACCTGTAAACGTTGCCGTGTGTCCGATGAGGATCTGAACAAA
TTCCTGACAAAAGCCAATGAGGACCAAACAAGCGTGAAAGTGAAAGTCGTTAGCGCTCCTACCCGTACTAAAAAA
GCAATGCCGAAATCCGTTGCTCGTGCCCCTAAACCACTGGAAAACACTGAAGCAGCACAGGCACAGCCGTCTGGA
AGCAAATTCTCTCCGGCCATTCCTGTTTCTACCCAGGAGTCCGTTTCTGTTCCAGCAAGTGTGAGCACCAGCATT
AGCAGTATTAGCACCGGTGCCACCGCTAGCGCCCTGGTTAAAGGCAATACCAATCCGATTACAAGCATGTCTGCC
CCGGTTCAAGCATCAGCTCCAGCACTGACAAAATCCCAAACCGATCGTCTGGAGGTTCTGCTGAATCCGAAAGAC
GAAATCAGCCTGAATTCCGGCAAACCGTTTCGTGAACTGGAGAGCGAACTGCTGTCACGTCGTAAAAAAGACCTG
CAACAAATCTATGCCGAAGAACGTGAGAACTATCTGGGGAAACTGGAACGTGAAATCACCCGCTTTTTCGTGGAT
CGTGGCTTTCTGGAGATCAAATCCCCGATTCTGATTCCTCTGGAGTATATCGAGCGTATGGGCATCGACAATGAT
ACCGAACTGAGCAAACAAATTTTCCGTGTGGATAAAAACTTCTGTCTGCGCCCTATGCTGGCACCAAATCTGTAT
AACTATCTGCGCAAACTGGACCGTGCCCTGCCTGATCCTATCAAAATCTTCGAGATCGGCCCGTGTTATCGTAAA
GAGTCCGACGGTAAAGAACATCTGGAGGAGTTTACCATGCTGGCCTTTGCCCAAATGGGTTCAGGTTGTACTCGT
GAGAACCTGGAAAGCATCATCACCGATTTTCTGAACCACCTGGGCATTGACTTCAAAATTGTGGGCGACAGCTGT
ATGGTGTATGGCGACACCCTGGATGTCATGCACGGCGACCTGGAACTGTCTAGTGCCGTTGTTGGACCAATTCCG
CTGGACCGTGAGTGGGGTATCGACAAACCGTGGATCGGAGCAGGATTCGGTCTGGAACGCCTGCTGAAAGTGAAA
CACGACTTCAAAAACATCAAACGTGCCGCCCGTTCTGAATCGTATTATAACGGGATCTCTACGAACCTGTAA
(SEQ ID NO: 491)

Protein:
MGCVCSSNPEGTELMASTDYSTYSQAAAQQGYSAYTAQPTQGYAQTTQAYGQQSYGTYGQPTDVSYTQAQTTATY
GQTAYATSYGQPPTGYTTPTAPQAYSQPVQGYGTGAYDTTTATVTTTQASYAAQSAYGTQPAYPAYGQQPAATAP
TRPQDGNKPTETSQPQSSTGGYNQPSLGYGQSNYSYPQVPGSYPMQPVTAPPSYPPTSYSSTQPTSYDQSSYSQQ
NTYGQPSSYGQQSSYGQQSSYGQQPPTSYPPQTGSYSQAPSQYSQQSSSYGQQSSFRQDHPSSMGVYGQESGGFS
GPGENRSMSGPDNRGRGRGGFDRGGMSRGGRGGGRGGMGSAGERGGFNKPGGPMDEGPDLDLGPPVDPDEDSDNS
AIYVQGLNDSVTLDDLADFFKQCGVVKMNKRTGQPMIHIYLDKETGKPKGDATVSYEDPPTAKAAVEWFDGKDFQ
GSKLKVSLARKKPPMNSMRGGLPPREGRGMPPPLRGGPGGPGGPGGPMGRMGGRGGDRGGFPPRGPRGSRGNPSG
GGNVQHRAGDWQCPNPGCGNQNFAWRTECNQCKAPKPEGFLPPPFPPPGGDRGRGGPGGMRGGRGGLMDRGGPGG
MFRGGRGGDRGGFRGGRGMDRGGFGGGRRGGPGGPPGPLMEQAIAYPYDVPDYAGAPGSAGSAAGSGASNFTQFV
LVDNGGTGDVTVAPSNFANGIAEWISSNSRSQAYKVTCSVRQSSAQNRKYTIKVEVPKGAWRSYLNMELTIPIFA
TNSDCELIVKAMQGLLKDGNPIPSAIAANSGIYGLSPDRVRAVSHWSSACPVPLQLPPLERLTLDDKKPLNTLIS
ATGLWMSRTGTIHKIKHHEVSRSKIYIEMACGDHLVVNNSRSSRTARALRHHKYRKTCKRCRVSDEDLNKFLTKA
NEDQTSVKVKVVSAPTRTKKAMPKSVARAPKPLENTEAAQAQPSGSKFSPAIPVSTQESVSVPASVSTSISSIST
GATASALVKGNTNPITSMSAPVQASAPALTKSQTDRLEVLLNPKDEISLNSGKPFRELESELLSRRKKDLQQIYA
EERENYLGKLEREITRFFVDRGFLEIKSPILIPLEYIERMGIDNDTELSKQIFRVDKNFCLRPMLAPNLYNYLRK
LDRALPDPIKIFEIGPCYRKESDGKEHLEEFTMLAFAQMGSGCTRENLESIITDFLNHLGIDFKIVGDSCMVYGD
TLDVMHGDLELSSAVVGPIPLDREWGIDKPWIGAGFGLERLLKVKHDFKNIKRAARSESYYNGISTNLASNDYTQ

QATQSYGAYPTQPGQGYSQQSSQPYGQQSYSGYSQSTDTSGYGQSSYSSYGQSQNTGYGTQSTPQGYGSTGGYGS
SQSSQSSYGQQSSYPGYGQQPAPSSTSGSYGSSSQSSSYGQPQSGSYSQQPSYGGQQQSYGQQQSYNPPQGYGQQ
NQYNSSSGGGGGGGGGGNYGQDQSSMSSGGGSGGGYGNQDQSGGGGSGGYGQQDRGGRGRGGSGGGGGGGGGYN
RSSGGYEPRGRGGGRGGRGGMGGSDRGGFNKFGGPRDQGSRHDSEQDNSDNNTIFVQGLGENVTIESVADYFKQI
GIIKTNKKTGQPMINLYTDRETGKLKGEATVSFDDPPSAKAAIDWFDGKEFSGNPIKVSFATRRADFNRGGGNGR
GGRGRGGPMGRGGYGGGGSGGGGRGGFPSGGGGGGGQQRAGDWKCPNPTCENMNFSWRNECNQCKAPKPDGPGGG
PGGSHMGGNYGDDRRGGRGGDKKPLNTLISATGLWMSRTGTIHKIKHHEVSRSKIYIEMACGDHLVVNNSRSSRT
ARALRHHKYRKTCKRCRVSDEDLNKFLTKANEDQTSVKVKVVSAPTRTKKAMPKSVARAPKPLENTEAAQAQPSG
SKFSPAIPVSTQESVSVPASVSTSISSISTGATASALVKGNTNPITSMSAPVQASAPALTKSQTDRLEVLLNPKD
EISLNSGKPFRELESELLSRRKKDLQQIYAEERENYLGKLEREITRFFVDRGFLEIKSPILIPLEYIERMGIDND
TELSKQIFRVDKNFCLRPMLAPNLYNYLRKLDRALPDPIKIFEIGPCYRKESDGKEHLEEFTMLAFAQMGSGCTR
ENLESIITDFLNHLGIDFKIVGDSCMVYGDTLDVMHGDLELSSAVVGPIPLDREWGIDKPWIGAGFGLERLLKVK
HDFKNIKRAARSESYYNGISTNL* (SEQ ID NO: 492)

LCK::PylRS(AA)::FUS::PylRS(AA)
DNA:
ATGGGCTGCGTGTGCAGCAGCAACCCCGAGGGTACCGAGCTCGCGTGCCCGGTGCCGCTGCAGCTGCCGCCGCTG
GAACGCCTGACCCTGGATGATAAAAAACCGCTGAATACCCTGATCTCTGCTACTGGTCTGTGGATGAGTCGTACC
GGAACCATTCATAAAATCAAACACCACGAGGTTAGCCGTTCGAAAATCTATATTGAGATGGCGTGTGGCGATCAT
CTGGTTGTGAACAATAGCCGCTCTTCTCGTACAGCACGTGCACTGCGTCACCACAAATATCGTAAAACCTGTAAA
CGTTGCCGTGTGTCCGATGAGGATCTGAACAAATTCCTGACAAAAGCCAATGAGGACCAAACAAGCGTGAAAGTG
AAAGTCGTTAGCGCTCCTACCCGTACTAAAAAAGCAATGCCGAAATCCGTTGCTCGTGCCCCTAAACCACTGGAA
AACACTGAAGCAGCACAGGCACAGCCGTCTGGAAGCAAATTCTCTCCGGCCATTCCTGTTTCTACCCAGGAGTCC
GTTTCTGTTCCAGCAAGTGTGAGCACCAGCATTAGCAGTATTAGCACCGGTGCCACCGCTAGCGCCCTGGTTAAA
GGCAATACCAATCCGATTACAAGCATGTCTGCCCCGGTTCAAGCATCAGCTCCAGCACTGACAAAATCCCAAACC
GATCGTCTGGAGGTTCTGCTGAATCCGAAAGACGAAATCAGCCTGAATTCCGGCAAACCGTTTCGTGAACTGGAG
AGCGAACTGCTGTCACGTCGTAAAAAAGACCTGCAACAAATCTATGCCGAAGAACGTGAGAACTATCTGGGGAAA
CTGGAACGTGAAATCACCCGCTTTTTCGTGGATCGTGGCTTTCTGGAGATCAAATCCCCGATTCTGATTCCTCTG
GAGTATATCGAGCGTATGGGCATCGACAATGATACCGAACTGAGCAAACAAATTTTCCGTGTGGATAAAAACTTC
TGTCTGCGCCCTATGCTGGCACCAAATCTGTATAACTATCTGCGCAAACTGGACCGTGCCCTGCCTGATCCTATC
AAAATCTTCGAGATCGGCCCGTGTTATCGTAAAGAGTCCGACGGTAAAGAACATCTGGAGGAGTTTACCATGCTG
GCCTTTGCCCAAATGGGTTCAGGTTGTACTCGTGAGAACCTGGAAAGCATCATCACCGATTTTCTGAACCACCTG
GGCATTGACTTCAAAATTGTGGGCGACAGCTGTATGGTGTATGGCGACACCCTGGATGTCATGCACGGCGACCTG
GAACTGTCTAGTGCCGTTGTTGGACCAATTCCGCTGGACCGTGAGTGGGGTATCGACAAACCGTGGATCGGAGCA
GGATTCGGTCTGGAACGCCTGCTGAAAGTGAAACACGACTTCAAAAACATCAAACGTGCCGCCCGTTCTGAATCG
TATTATAACGGGATCTCTACGAACCTGGCCTCAAACGATTATACCCAACAAGCAACCCAAAGCTATGGGGCCTAC
CCCACCCAGCCCGGGCAGGGCTATTCCCAGCAGAGCAGTCAGCCCTACGGACAGCAGAGTTACAGTGGTTATAGC
CAGTCCACGGACACTTCAGGATATGGCCAGAGCAGCTATTCTTCTTATGGCCAGAGCCAGAACACAGGCTATGGA
ACTCAGTCAACTCCCCAGGGATATGGCTCGACTGGCGGCTATGGCAGTAGCCAGAGCTCCCAATCGTCTTACGGG
CAGCAGTCCTCCTACCCTGGCTATGGCCAGCAGCCAGCTCCCAGCAGCACCTCGGGAAGTTACGGTAGCAGTTCT
CAGAGCAGCAGCTATGGGCAGCCCCAGAGTGGGAGCTACAGCCAGCAGCCTAGCTATGGTGGACAGCAGCAAAGC
TATGGACAGCAGCAAAGCTATAATCCCCCTCAGGGCTATGGACAGCAGAACCAGTACAACAGCAGCAGTGGTGGT
GGAGGTGGAGGTGGAGGTGGAGGTAACTATGGCCAAGATCAATCCTCCATGAGTAGTGGTGGTGGCAGTGGTGGC
GGTTATGGCAATCAAGACCAGAGTGGTGGAGGTGGCAGCGGTGGCTATGGACAGCAGGACCGTGGAGGCCGCGGC
AGGGGTGGCAGTGGTGGCGGCGGCGGCGGCGGCGGTGGTGGTTACAACCGCAGCAGTGGTGGCTATGAACCCAGA
GGTCGTGGAGGTGGCCGTGGAGGCAGAGGTGGCATGGGCGGAAGTGACCGTGGTGGCTTCAATAAATTTGGTGGC
CCTCGGGACCAAGGATCACGTCATGACTCCGAACAGGATAATTCAGACAACAACACCATCTTTGTGCAAGGCCTG
GGTGAGAATGTTACAATTGAGTCTGTGGCTGATTACTTCAAGCAGATTGGTATTATTAAGACAAACAAGAAAACG
GGACAGCCCATGATTAATTTGTACACAGACAGGGAAACTGGCAAGCTGAAGGGAGAGGCAACGGTCTCTTTTGAT
GACCCACCTTCAGCTAAAGCAGCTATTGACTGGTTTGATGGTAAAGAATTCTCCGGAAATCCTATCAAGGTCTCA
TTTGCTACTCGCCGGGCAGACTTTAATCGGGGTGGTGGCAATGGTCGTGGAGGCCGAGGGCGAGGAGGACCCATG
GGCCGTGGAGGCTATGGAGGTGGTGGCAGTGGTGGTGGTGGCCGAGGAGGATTTCCCAGTGGAGGTGGTGGCGGT
GGAGGACAGCAGCGAGCTGGTGACTGGAAGTGTCCTAATCCCACCTGTGAGAATATGAACTTCTCTTGGAGGAAT
GAATGCAACCAGTGTAAGGCCCCTAAACCAGATGGCCCAGGAGGGGGACCAGGTGGCTCTCACATGGGGGGTAAC
TACGGGGATGATCGTCGTGGTGGCAGAGGAGGCGATAAAAAACCGCTGAATACCCTGATCTCTGCTACTGGTCTG
TGGATGAGTCGTACCGGAACCATTCATAAAATCAAACACCACGAGGTTAGCCGTTCGAAAATCTATATTGAGATG
GCGTGTGGCGATCATCTGGTTGTGAACAATAGCCGCTCTTCTCGTACAGCACGTGCACTGCGTCACCACAAATAT

CGTAAAACCTGTAAACGTTGCCGTGTGTCCGATGAGGATCTGAACAAATTCCTGACAAAAGCCAATGAGGACCAA
ACAAGCGTGAAAGTGAAAGTCGTTAGCGCTCCTACCCGTACTAAAAAAGCAATGCCGAAATCCGTTGCTCGTGCC
CCTAAACCACTGGAAAACACTGAAGCAGCACAGGCACAGCCGTCTGGAAGCAAATTCTCTCCGGCCATTCCTGTT
TCTACCCAGGAGTCCGTTTCTGTTCCAGCAAGTGTGAGCACCAGCATTAGCAGTATTAGCACCGGTGCCACCGCT
AGCGCCCTGGTTAAAGGCAATACCAATCCGATTACAAGCATGTCTGCCCCGGTTCAAGCATCAGCTCCAGCACTG
ACAAAATCCCAAACCGATCGTCTGGAGGTTCTGCTGAATCCGAAAGACGAAATCAGCCTGAATTCCGGCAAACCG
TTTCGTGAACTGGAGAGCGAACTGCTGTCACGTCGTAAAAAAGACCTGCAACAAATCTATGCCGAAGAACGTGAG
AACTATCTGGGGAAACTGGAACGTGAAATCACCCGCTTTTTCGTGGATCGTGGCTTTCTGGAGATCAAATCCCCG
ATTCTGATTCCTCTGGAGTATATCGAGCGTATGGGCATCGACAATGATACCGAACTGAGCAAACAAATTTTCCGT
GTGGATAAAAACTTCTGTCTGCGCCCTATGCTGGCACCAAATCTGTATAACTATCTGCGCAAACTGGACCGTGCC
CTGCCTGATCCTATCAAAATCTTCGAGATCGGCCCGTGTTATCGTAAAGAGTCCGACGGTAAAGAACATCTGGAG
GAGTTTACCATGCTGGCCTTTGCCCAAATGGGTTCAGGTTGTACTCGTGAGAACCTGGAAAGCATCATCACCGAT
TTTCTGAACCACCTGGGCATTGACTTCAAAATTGTGGGCGACAGCTGTATGGTGTATGGCGACACCCTGGATGTC
ATGCACGGCGACCTGGAACTGTCTAGTGCCGTTGTTGGACCAATTCCGCTGGACCGTGAGTGGGGTATCGACAAA
CCGTGGATCGGAGCAGGATTCGGTCTGGAACGCCTGCTGAAAGTGAAACACGACTTCAAAAACATCAAACGTGCC
GCCCGTTCTGAATCGTATTATAACGGGATCTCTACGAACCTGTAA (SEQ ID NO: 493)

Protein:
MGCVCSSNPEGTELACPVPLQLPPLERLTLDDKKPLNTLISATGLWMSRTGTIHKIKHHEVSRSKIYIEMACGDH
LVVNNSRSSRTARALRHHKYRKTCKRCRVSDEDLNKFLTKANEDQTSVKVKVVSAPTRTKKAMPKSVARAPKPLE
NTEAAQAQPSGSKFSPAIPVSTQESVSVPASVSTSISSISTGATASALVKGNTNPITSMSAPVQASAPALTKSQT
DRLEVLLNPKDEISLNSGKPFRELESELLSRRKKDLQQIYAEERENYLGKLEREITRFFVDRGFLEIKSPILIPL
EYIERMGIDNDTELSKQIFRVDKNFCLRPMLAPNLYNYLRKLDRALPDPIKIFEIGPCYRKESDGKEHLEEFTML
AFAQMGSGCTRENLESIITDFLNHLGIDFKIVGDSCMVYGDTLDVMHGDLELSSAVVGPIPLDREWGIDKPWIGA
GFGLERLLKVKHDFKNIKRAARSESYYNGISTNLASNDYTQQATQSYGAYPTQPGQGYSQQSSQPYGQQSYSGYS
QSTDTSGYGQSSYSSYGQSQNTGYGTQSTPQGYGSTGGYGSSQSSQSSYGQQSSYPGYGQQPAPSSTSGSYGSSS
QSSSYGQPQSGSYSQQPSYGGQQQSYGQQQSYNPPQGYGQQNQYNSSSGGGGGGGGGNYGQDQSSMSSGGGSGG
GYGNQDQSGGGGSGGYGQQDRGGRGRGGSGGGGGGGGGYNRSSGGYEPRGRGGGRGGRGGMGGSDRGGFNKFGG
PRDQGSRHDSEQDNSDNNTIFVQGLGENVTIESVADYFKQIGIIKTNKKTGQPMINLYTDRETGKLKGEATVSFD
DPPSAKAAIDWFDGKEFSGNPIKVSFATRRADFNRGGGNGRGGRGRGGPMGRGGYGGGGSGGGGRGGFPSGGGGG
GGQQRAGDWKCPNPTCENMNFSWRNECNQCKAPKPDGPGGGPGGSHMGGNYGDDRRGGRGGDKKPLNTLISATGL
WMSRTGTIHKIKHHEVSRSKIYIEMACGDHLVVNNSRSSRTARALRHHKYRKTCKRCRVSDEDLNKFLTKANEDQ
TSVKVKVVSAPTRTKKAMPKSVARAPKPLENTEAAQAQPSGSKFSPAIPVSTQESVSVPASVSTSISSISTGATA
SALVKGNTNPITSMSAPVQASAPALTKSQTDRLEVLLNPKDEISLNSGKPFRELESELLSRRKKDLQQIYAEERE
NYLGKLEREITRFFVDRGFLEIKSPILIPLEYIERMGIDNDTELSKQIFRVDKNFCLRPMLAPNLYNYLRKLDRA
LPDPIKIFEIGPCYRKESDGKEHLEEFTMLAFAQMGSGCTRENLESIITDFLNHLGIDFKIVGDSCMVYGDTLDV
MHGDLELSSAVVGPIPLDREWGIDKPWIGAGFGLERLLKVKHDFKNIKRAARSESYYNGISTNL* (SEQ ID
NO: 494)

LCK::PylRS(AF)::EWSR1::PylRS(AF)
DNA:
ATGGGCTGCGTGTGCAGCAGCAACCCCGAGGGTACCGAGCTCGCGTGCCCGGTGCCGCTGCAGCTGCCGCCGCTG
GAACGCCTGACCCTGGATGATAAAAAACCGCTGAATACCCTGATCTCTGCTACTGGTCTGTGGATGAGTCGTACC
GGAACCATTCATAAAATCAAACACCACGAGGTTAGCCGTTCGAAAATCTATATTGAGATGGCGTGTGGCGATCAT
CTGGTTGTGAACAATAGCCGCTCTTCTCGTACAGCACGTGCACTGCGTCACCACAAATATCGTAAAACCTGTAAA
CGTTGCCGTGTGTCCGATGAGGATCTGAACAAATTCCTGACAAAAGCCAATGAGGACCAAACAAGCGTGAAAGTG
AAAGTCGTTAGCGCTCCTACCCGTACTAAAAAAGCAATGCCGAAATCCGTTGCTCGTGCCCCTAAACCACTGGAA
AACACTGAAGCAGCACAGGCACAGCCGTCTGGAAGCAAATTCTCTCCGGCCATTCCTGTTTCTACCCAGGAGTCC
GTTTCTGTTCCAGCAAGTGTGAGCACCAGCATTAGCAGTATTAGCACCGGTGCCACCGCTAGCGCCCTGGTTAAA
GGCAATACCAATCCGATTACAAGCATGTCTGCCCCGGTTCAAGCATCAGCTCCAGCACTGACAAAATCCCAAACC
GATCGTCTGGAGGTTCTGCTGAATCCGAAAGACGAAATCAGCCTGAATTCCGGCAAACCGTTTCGTGAACTGGAG
AGCGAACTGCTGTCACGTCGTAAAAAAGACCTGCAACAAATCTATGCCGAAGAACGTGAGAACTATCTGGGGAAA
CTGGAACGTGAAATCACCCGCTTTTTCGTGGATCGTGGCTTTCTGGAGATCAAATCCCCGATTCTGATTCCTCTG
GAGTATATCGAGCGTATGGGCATCGACAATGATACCGAACTGAGCAAACAAATTTTCCGTGTGGATAAAAACTTC
TGTCTGCGCCCTATGCTAGCACCAAATCTGGCTAACTATCTGCGCAAACTGGACCGTGCCCTGCCTGATCCTATC
AAAATCTTCGAGATCGGCCCGTGTTATCGTAAAGAGTCCGACGGTAAAGAACATCTGGAGGAGTTTACCATGCTG
AACTTTTGCCAAATGGGTTCAGGTTGTACTCGTGAGAACCTGGAAAGCATCATCACCGATTTTCTGAACCACCTG

GGCATTGACTTCAAAATTGTGGGCGACAGCTGTATGGTGTTTGGCGACACCCTGGATGTCATGCACGGCGACCTG
GAACTGTCTAGTGCCGTTGTGGGCCCAATCCCGCTGGATCGTGAGTGGGGTATCGACAAACCTTGGATCGGTGCG
GGTTTTGGTCTGGAGCGTCTGCTGAAAGTAAAACACGACTTCAAGAACATCAAACGTGCTGCACGTTCCGAGTCC
TATTACAATGGTATTTCTACTAACCTGATGGCGTCCACGGATTACAGTACCTATAGCCAAGCTGCAGCGCAGCAG
GGCTACAGTGCTTACACCGCCCAGCCCACTCAAGGATATGCACAGACCACCCAGGCATATGGGCAACAAAGCTAT
GGAACCTATGGACAGCCCACTGATGTCAGCTATACCCAGGCTCAGACCACTGCAACCTATGGGCAGACCGCCTAT
GCAACTTCTTATGGACAGCCTCCCACTGGTTATACTACTCCAACTGCCCCCCAGGCATACAGCCAGCCTGTCCAG
GGGTATGGCACTGGTGCTTATGATACCACCACTGCTACAGTCACCACCACCCAGGCCTCCTATGCAGCTCAGTCT
GCATATGGCACTCAGCCTGCTTATCCAGCCTATGGGCAGCAGCCAGCAGCCACTGCACCTACAAGACCGCAGGAT
GGAAACAAGCCCACTGAGACTAGTCAACCTCAATCTAGCACAGGGGGTTACAACCAGCCCAGCCTAGGATATGGA
CAGAGTAACTACAGTTATCCCCAGGTACCTGGGAGCTACCCCATGCAGCCAGTCACTGCACCTCCATCCTACCCT
CCTACCAGCTATTCCTCTACACAGCCGACTAGTTATGATCAGAGCAGTTACTCTCAGCAGAACACCTATGGGCAA
CCGAGCAGCTATGGACAGCAGAGTAGCTATGGTCAACAAAGCAGCTATGGGCAGCAGCCTCCCACTAGTTACCCA
CCCCAAACTGGATCCTACAGCCAAGCTCCAAGTCAATATAGCCAACAGAGCAGCAGCTACGGGCAGCAGAGTTCA
TTCCGACAGGACCACCCCAGTAGCATGGGTGTTTATGGGCAGGAGTCTGGAGGATTTTCCGGACCAGGAGAGAAC
CGGAGCATGAGTGGCCCTGATAACCGGGGCAGGGGAAGAGGGGGATTTGATCGTGGAGGCATGAGCAGAGGTGGG
CGGGGAGGAGGACGCGGTGGAATGGGCAGCGCTGGAGAGCGAGGTGGCTTCAATAAGCCTGGTGGACCCATGGAT
GAAGGACCAGATCTTGATCTAGGCCCACCTGTAGATCCAGATGAAGACTCTGACAACAGTGCAATTTATGTACAA
GGATTAAATGACAGTGTGACTCTAGATGATCTGGCAGACTTCTTTAAGCAGTGTGGGGTTGTTAAGATGAACAAG
AGAACTGGGCAACCCATGATCCACATCTACCTGGACAAGGAAACAGGAAAGCCCAAAGGCGATGCCACAGTGTCC
TATGAAGACCCACCTACTGCCAAGGCTGCCGTGGAATGGTTTGATGGGAAAGATTTTCAAGGGAGCAAACTTAAA
GTCTCCCTTGCTCGGAAGAAGCCTCCAATGAACAGTATGCGGGGTGGTCTGCCACCCCGTGAGGGCAGAGGCATG
CCACCACCACTCCGTGGAGGTCCAGGAGGCCCAGGAGGTCCTGGGGGACCCATGGGTCGCATGGGAGGCCGTGGA
GGAGATAGAGGAGGCTTCCCTCCAAGAGGACCCCGGGGTTCCCGAGGGAACCCCTCTGGAGGAGGAAACGTCCAG
CACCGAGCTGGAGACTGGCAGTGTCCCAATCCGGGTTGTGGAAACCAGAACTTCGCCTGGAGAACAGAGTGCAAC
CAGTGTAAGGCCCCAAAGCCTGAAGGCTTCCTCCCGCCACCCTTTCCGCCCCCGGGTGGTGATCGTGGCAGAGGT
GGCCCTGGTGGCATGCGGGGAGGAAGAGGTGGCCTCATGGATCGTGGTGGTCCCGGTGGAATGTTCAGAGGTGGC
CGTGGTGGAGACAGAGGTGGCTTCCGTGGTGGCCGGGGCATGGACCGAGGTGGCTTTGGTGGAGGAAGACGAGGT
GGCCCTGGGGGGCCCCCTGGACCTTTGATGGAACAGGATAAAAAACCGCTGAATACCCTGATCTCTGCTACTGGT
CTGTGGATGAGTCGTACCGGAACCATTCATAAAATCAAACACCACGAGGTTAGCCGTTCGAAAATCTATATTGAG
ATGGCGTGTGGCGATCATCTGGTTGTGAACAATAGCCGCTCTTCTCGTACAGCACGTGCACTGCGTCACCACAAA
TATCGTAAAACCTGTAAACGTTGCCGTGTGTCCGATGAGGATCTGAACAAATTCCTGACAAAAGCCAATGAGGAC
CAAACAAGCGTGAAAGTGAAAGTCGTTAGCGCTCCTACCCGTACTAAAAAAGCAATGCCGAAATCCGTTGCTCGT
GCCCCTAAACCACTGGAAAACACTGAAGCAGCACAGGCACAGCCGTCTGGAAGCAAATTCTCTCCGGCCATTCCT
GTTTCTACCCAGGAGTCCGTTTCTGTTCCAGCAAGTGTGAGCACCAGCATTAGCAGTATTAGCACCGGTGCCACC
GCTAGCGCCCTGGTTAAAGGCAATACCAATCCGATTACAAGCATGTCTGCCCCGGTTCAAGCATCAGCTCCAGCA
CTGACAAAATCCCAAACCGATCGTCTGGAGGTTCTGCTGAATCCGAAAGACGAAATCAGCCTGAATTCCGGCAAA
CCGTTTCGTGAACTGGAGAGCGAACTGCTGTCACGTCGTAAAAAAGACCTGCAACAAATCTATGCCGAAGAACGT
GAGAACTATCTGGGGAAACTGGAACGTGAAATCACCCGCTTTTTCGTGGATCGTGGCTTTCTGGAGATCAAATCC
CCGATTCTGATTCCTCTGGAGTATATCGAGCGTATGGGCATCGACAATGATACCGAACTGAGCAAACAAATTTTC
CGTGTGGATAAAAACTTCTGTCTGCGCCCTATGCTAGCACCAAATCTGGCTAACTATCTGCGCAAACTGGACCGT
GCCCTGCCTGATCCTATCAAAATCTTCGAGATCGGCCCGTGTTATCGTAAAGAGTCCGACGGTAAAGAACATCTG
GAGGAGTTTACCATGCTGAACTTTTGCCAAATGGGTTCAGGTTGTACTCGTGAGAACCTGGAAAGCATCATCACC
GATTTTCTGAACCACCTGGGCATTGACTTCAAAATTGTGGGCGACAGCTGTATGGTGTTTGGCGACACCCTGGAT
GTCATGCACGGCGACCTGGAACTGTCTAGTGCCGTTGTGGGCCCAATCCCGCTGGATCGTGAGTGGGGTATCGAC
AAACCTTGGATCGGTGCGGGTTTTGGTCTGGAGCGTCTGCTGAAAGTAAAACACGACTTCAAGAACATCAAACGT
GCTGCACGTTCCGAGTCCTATTACAATGGTATTTCTACTAACCTGTAA (SEQ ID NO: 495)

Protein:
MGCVCSSNPEGTELACPVPLQLPPLERLTLDDKKPLNTLISATGLWMSRTGTIHKIKHHEVSRSKIYIEMACGDH
LVVNNSRSSRTARALRHHKYRKTCKRCRVSDEDLNKFLTKANEDQTSVKVKVVSAPTRTKKAMPKSVARAPKPLE
NTEAAQAQPSGSKFSPAIPVSTQESVSVPASVSTSISSISTGATASALVKGNTNPITSMSAPVQASAPALTKSQT
DRLEVLLNPKDEISLNSGKPFRELESELLSRRKKDLQQIYAEERENYLGKLEREITRFFVDRGFLEIKSPILIPL
EYIERMGIDNDTELSKQIFRVDKNFCLRPMLAPNLANYLRKLDRALPDPIKIFEIGPCYRKESDGKEHLEEFTML
NFCQMGSGCTRENLESIITDFLNHLGIDFKIVGDSCMVFGDTLDVMHGDLELSSAVVGPIPLDREWGIDKPWIGA
GFGLERLLKVKHDFKNIKRAARSESYYNGISTNLMASTDYSTYSQAAAQQGYSAYTAQPTQGYAQTTQAYGQQSY
GTYGQPTDVSYTQAQTTATYGQTAYATSYGQPPTGYTTPTAPQAYSQPVQGYGTGAYDTTTATVTTTQASYAAQS
AYGTQPAYPAYGQQPAATAPTRPQDGNKPTETSQPQSSTGGYNQPSLGYGQSNYSYPQVPGSYPMQPVTAPPSYP

PTSYSSTQPTSYDQSSYSQQNTYGQPSSYGQQSSYGQQSSYGQQPPTSYPPQTGSYSQAPSQYSQQSSSYGQQSS
FRQDHPSSMGVYGQESGGFSGPGENRSMSGPDNRGRGRGGFDRGGMSRGGRGGGRGGMGSAGERGGFNKPGGPMD
EGPDLDLGPPVDPDEDSDNSAIYVQGLNDSVTLDDLADFFKQCGVVKMNKRTGQPMIHIYLDKETGKPKGDATVS
YEDPPTAKAAVEWFDGKDFQGSKLKVSLARKKPPMNSMRGGLPPREGRGMPPPLRGGPGGPGGPGGPMGRMGGRG
GDRGGFPPRGPRGSRGNPSGGGNVQHRAGDWQCPNPGCGNQNFAWRTECNQCKAPKPEGFLPPPFPPPGGDRGRG
GPGGMRGGRGGLMDRGGPGGMFRGGRGGDRGGFRGGRGMDRGGFGGGRRGGPGGPPGPLMEQDKKPLNTLISATG
LWMSRTGTIHKIKHHEVSRSKIYIEMACGDHLVVNNSRSSRTARALRHHKYRKTCKRCRVSDEDLNKFLTKANED
QTSVKVKVVSAPTRTKKAMPKSVARAPKPLENTEAAQAQPSGSKFSPAIPVSTQESVSVPASVSTSISSISTGAT
ASALVKGNTNPITSMSAPVQASAPALTKSQTDRLEVLLNPKDEISLNSGKPFRELESELLSRRKKDLQQIYAEER
ENYLGKLEREITRFFVDRGFLEIKSPILIPLEYIERMGIDNDTELSKQIFRVDKNFCLRPMLAPNLANYLRKLDR
ALPDPIKIFEIGPCYRKESDGKEHLEEFTMLNFCQMGSGCTRENLESIITDFLNHLGIDFKIVGDSCMVFGDTLD
VMHGDLELSSAVVGPIPLDREWGIDKPWIGAGFGLERLLKVKHDFKNIKRAARSESYYNGISTNL* (SEQ ID
NO: 496)

TOM20::FUS::MCP::PylRS(AF)::EWSR1::PylRS(AF)
DNA:
ATGGTGGGTCGGAACAGCGCCATCGCCGCCGGTGTATGCGGGGCCCTTTTCATTGGGTACTGCATCTACTTCGAC
CGCAAAAGACGAAGTGACCCCAACTTCAAGAACAGGCTTCGAGAACGAAGAAAGAAACAGAAGCTTGCCAAGGAG
AGAGCTGGGCTTTCCAAGTTACCTGACCTTAAAGATGCTGAAGCTGTTCAGAAATTCTTCATGGCCTCAAACGAT
TATACCCAACAAGCAACCCAAAGCTATGGGGCCTACCCCACCCAGCCCGGGCAGGGCTATTCCCAGCAGAGCAGT
CAGCCCTACGGACAGCAGAGTTACAGTGGTTATAGCCAGTCCACGGACACTTCAGGATATGGCCAGAGCAGCTAT
TCTTCTTATGGCCAGAGCCAGAACACAGGCTATGGAACTCAGTCAACTCCCCAGGGATATGGCTCGACTGGCGGC
TATGGCAGTAGCCAGAGCTCCCAATCGTCTTACGGGCAGCAGTCCTCCTACCCTGGCTATGGCCAGCAGCCAGCT
CCCAGCAGCACCTCGGGAAGTTACGGTAGCAGTTCTCAGAGCAGCAGCTATGGGCAGCCCCAGAGTGGGAGCTAC
AGCCAGCAGCCTAGCTATGGTGGACAGCAGCAAAGCTATGGACAGCAGCAAAGCTATAATCCCCCTCAGGGCTAT
GGACAGCAGAACCAGTACAACAGCAGCAGTGGTGGTGGAGGTGGAGGTGGAGGTGGAGGTAACTATGGCCAAGAT
CAATCCTCCATGAGTAGTGGTGGTGGCAGTGGTGGCGGTTATGGCAATCAAGACCAGAGTGGTGGAGGTGGCAGC
GGTGGCTATGGACAGCAGGACCGTGGAGGCCGCGGCAGGGGTGGCAGTGGTGGCGGCGGCGGCGGCGGCGGTGGT
GGTTACAACCGCAGCAGTGGTGGCTATGAACCCAGAGGTCGTGGAGGTGGCCGTGGAGGCAGAGGTGGCATGGGC
GGAAGTGACCGTGGTGGCTTCAATAAATTTGGTGGCCCTCGGGACCAAGGATCACGTCATGACTCCGAACAGGAT
AATTCAGACAACAACACCATCTTTGTGCAAGGCCTGGGTGAGAATGTTACAATTGAGTCTGTGGCTGATTACTTC
AAGCAGATTGGTATTATTAAGACAAACAAGAAAACGGGACAGCCCATGATTAATTTGTACACAGACAGGGAAACT
GGCAAGCTGAAGGGAGAGGCAACGGTCTCTTTTGATGACCCACCTTCAGCTAAAGCAGCTATTGACTGGTTTGAT
GGTAAAGAATTCTCCGGAAATCCTATCAAGGTCTCATTTGCTACTCGCCGGGCAGACTTTAATCGGGGTGGTGGC
AATGGTCGTGGAGGCCGAGGGCGAGGAGGACCCATGGGCCGTGGAGGCTATGGAGGTGGTGGCAGTGGTGGTGGT
GGCCGAGGAGGATTTCCCAGTGGAGGTGGTGGCGGTGGAGGACAGCAGCGAGCTGGTGACTGGAAGTGTCCTAAT
CCCACCTGTGAGAATATGAACTTCTCTTGGAGGAATGAATGCAACCAGTGTAAGGCCCCTAAACCAGATGGCCCA
GGAGGGGGACCAGGTGGCTCTCACATGGGGGGTAACTACGGGGATGATCGTCGTGGTGGCAGAGGAGGCGCGATC
GCATATCCCTATGATGTGCCGGATTATGCTGGAGCACCAGGAAGTGCTGGTTCTGCTGCTGGTAGTGGAGCTTCT
AACTTTACTCAGTTCGTTCTCGTCGACAATGGCGGAACTGGCGACGTGACTGTCGCCCCAAGCAACTTCGCTAAC
GGGATCGCTGAATGGATCAGCTCTAACTCGCGTTCACAGGCTTACAAAGTAACCTGTAGCGTTCGTCAGAGCTCT
GCGCAGAATCGCAAATACACCATCAAAGTCGAGGTGCCTAAAGGCGCCTGGCGTTCGTACTTAAATATGGAACTA
ACCATTCCAATTTTCGCCACGAATTCCGACTGCGAGCTTATTGTTAAGGCAATGCAAGGTCTCCTAAAAGATGGA
AACCCGATTCCCTCAGCAATCGCAGCAAACTCCGGCATCTACGGCGCCGATTACAAGGACGATGATGACAAGGGA
GCACCAGGAAGTGCTGGTTCTGCTGCTGGTAGTGGAGCGTGCCCGGTGCCGCTGCAGCTGCCGCCGCTGGAACGC
CTGACCCTGGATGATAAAAAACCGCTGAATACCCTGATCTCTGCTACTGGTCTGTGGATGAGTCGTACCGGAACC
ATTCATAAAATCAAACACCACGAGGTTAGCCGTTCGAAAATCTATATTGAGATGGCGTGTGGCGATCATCTGGTT
GTGAACAATAGCCGCTCTTCTCGTACAGCACGTGCACTGCGTCACCACAAATATCGTAAAACCTGTAAACGTTGC
CGTGTGTCCGATGAGGATCTGAACAAATTCCTGACAAAAGCCAATGAGGACCAAACAAGCGTGAAAGTGAAAGTC
GTTAGCGCTCCTACCCGTACTAAAAAAGCAATGCCGAAATCCGTTGCTCGTGCCCCTAAACCACTGGAAAACACT
GAAGCAGCACAGGCACAGCCGTCTGGAAGCAAATTCTCTCCGGCCATTCCTGTTTCTACCCAGGAGTCCGTTTCT
GTTCCAGCAAGTGTGAGCACCAGCATTAGCAGTATTAGCACCGGTGCCACCGCTAGCGCCCTGGTTAAAGGCAAT
ACCAATCCGATTACAAGCATGTCTGCCCCGGTTCAAGCATCAGCTCCAGCACTGACAAAATCCCAAACCGATCGT
CTGGAGGTTCTGCTGAATCCGAAAGACGAAATCAGCCTGAATTCCGGCAAACCGTTTCGTGAACTGGAGAGCGAA
CTGCTGTCACGTCGTAAAAAAGACCTGCAACAAATCTATGCCGAAGAACGTGAGAACTATCTGGGGAAACTGGAA
CGTGAAATCACCCGCTTTTTCGTGGATCGTGGCTTTCTGGAGATCAAATCCCCGATTCTGATTCCTCTGGAGTAT
ATCGAGCGTATGGGCATCGACAATGATACCGAACTGAGCAAACAAATTTTCCGTGTGGATAAAAACTTCTGTCTG

CGCCCTATGCTAGCACCAAATCTGGCTAACTATCTGCGCAAACTGGACCGTGCCCTGCCTGATCCTATCAAAATC
TTCGAGATCGGCCCGTGTTATCGTAAAGAGTCCGACGGTAAAGAACATCTGGAGGAGTTTACCATGCTGAACTTT
TGCCAAATGGGTTCAGGTTGTACTCGTGAGAACCTGGAAAGCATCATCACCGATTTTCTGAACCACCTGGGCATT
GACTTCAAAATTGTGGGCGACAGCTGTATGGTGTTTGGCGACACCCTGGATGTCATGCACGGCGACCTGGAACTG
TCTAGTGCCGTTGTGGGCCCAATCCCGCTGGATCGTGAGTGGGGTATCGACAAACCTTGGATCGGTGCGGGTTTT
GGTCTGGAGCGTCTGCTGAAAGTAAAACACGACTTCAAGAACATCAAACGTGCTGCACGTTCCGAGTCCTATTAC
AATGGTATTTCTACTAACCTGATGGCGTCCACGGATTACAGTACCTATAGCCAAGCTGCAGCGCAGCAGGGCTAC
AGTGCTTACACCGCCCAGCCCACTCAAGGATATGCACAGACCACCCAGGCATATGGGCAACAAAGCTATGGAACC
TATGGACAGCCCACTGATGTCAGCTATACCCAGGCTCAGACCACTGCAACCTATGGGCAGACCGCCTATGCAACT
TCTTATGGACAGCCTCCCACTGGTTATACTACTCCAACTGCCCCCCAGGCATACAGCCAGCCTGTCCAGGGGTAT
GGCACTGGTGCTTATGATACCACCACTGCTACAGTCACCACCACCCAGGCCTCCTATGCAGCTCAGTCTGCATAT
GGCACTCAGCCTGCTTATCCAGCCTATGGGCAGCAGCCAGCAGCCACTGCACCTACAAGACCGCAGGATGGAAAC
AAGCCCACTGAGACTAGTCAACCTCAATCTAGCACAGGGGGTTACAACCAGCCCAGCCTAGGATATGGACAGAGT
AACTACAGTTATCCCCAGGTACCTGGGAGCTACCCCATGCAGCCAGTCACTGCACCTCCATCCTACCCTCCTACC
AGCTATTCCTCTACACAGCCGACTAGTTATGATCAGAGCAGTTACTCTCAGCAGAACACCTATGGGCAACCGAGC
AGCTATGGACAGCAGAGTAGCTATGGTCAACAAAGCAGCTATGGGCAGCAGCCTCCCACTAGTTACCCACCCCAA
ACTGGATCCTACAGCCAAGCTCCAAGTCAATATAGCCAACAGAGCAGCAGCTACGGGCAGCAGAGTTCATTCCGA
CAGGACCACCCCAGTAGCATGGGTGTTTATGGGCAGGAGTCTGGAGGATTTTCCGGACCAGGAGAGAACCGGAGC
ATGAGTGGCCCTGATAACCGGGGCAGGGGAAGAGGGGGATTTGATCGTGGAGGCATGAGCAGAGGTGGGCGGGGA
GGAGGACGCGGTGGAATGGGCAGCGCTGGAGAGCGAGGTGGCTTCAATAAGCCTGGTGGACCCATGGATGAAGGA
CCAGATCTTGATCTAGGCCCACCTGTAGATCCAGATGAAGACTCTGACAACAGTGCAATTTATGTACAAGGATTA
AATGACAGTGTGACTCTAGATGATCTGGCAGACTTCTTTAAGCAGTGTGGGGTTGTTAAGATGAACAAGAGAACT
GGGCAACCCATGATCCACATCTACCTGGACAAGGAAACAGGAAAGCCCAAAGGCGATGCCACAGTGTCCTATGAA
GACCCACCTACTGCCAAGGCTGCCGTGGAATGGTTTGATGGGAAAGATTTTCAAGGGAGCAAACTTAAAGTCTCC
CTTGCTCGGAAGAAGCCTCCAATGAACAGTATGCGGGGTGGTCTGCCACCCCGTGAGGGCAGAGGCATGCCACCA
CCACTCCGTGGAGGTCCAGGAGGCCCAGGAGGTCCTGGGGGACCCATGGGTCGCATGGGAGGCCGTGGAGGAGAT
AGAGGAGGCTTCCCTCCAAGAGGACCCCGGGGTTCCCGAGGGAACCCCTCTGGAGGAGGAAACGTCCAGCACCGA
GCTGGAGACTGGCAGTGTCCCAATCCGGGTTGTGGAAACCAGAACTTCGCCTGGAGAACAGAGTGCAACCAGTGT
AAGGCCCCAAAGCCTGAAGGCTTCCTCCCGCCACCCTTTCCGCCCCCGGGTGGTGATCGTGGCAGAGGTGGCCCT
GGTGGCATGCGGGGAGGAAGAGGTGGCCTCATGGATCGTGGTGGTCCCGGTGGAATGTTCAGAGGTGGCCGTGGT
GGAGACAGAGGTGGCTTCCGTGGTGGCCGGGGCATGGACCGAGGTGGCTTTGGTGGAGGAAGACGAGGTGGCCCT
GGGGGGCCCCCTGGACCTTTGATGGAACAGGATAAAAAACCGCTGAATACCCTGATCTCTGCTACTGGTCTGTGG
ATGAGTCGTACCGGAACCATTCATAAAATCAAACACCACGAGGTTAGCCGTTCGAAAATCTATATTGAGATGGCG
TGTGGCGATCATCTGGTTGTGAACAATAGCCGCTCTTCTCGTACAGCACGTGCACTGCGTCACCACAAATATCGT
AAAACCTGTAAACGTTGCCGTGTGTCCGATGAGGATCTGAACAAATTCCTGACAAAAGCCAATGAGGACCAAACA
AGCGTGAAAGTGAAAGTCGTTAGCGCTCCTACCCGTACTAAAAAAGCAATGCCGAAATCCGTTGCTCGTGCCCCT
AAACCACTGGAAAACACTGAAGCAGCACAGGCACAGCCGTCTGGAAGCAAATTCTCTCCGGCCATTCCTGTTTCT
ACCCAGGAGTCCGTTTCTGTTCCAGCAAGTGTGAGCACCAGCATTAGCAGTATTAGCACCGGTGCCACCGCTAGC
GCCCTGGTTAAAGGCAATACCAATCCGATTACAAGCATGTCTGCCCCGGTTCAAGCATCAGCTCCAGCACTGACA
AAATCCCAAACCGATCGTCTGGAGGTTCTGCTGAATCCGAAAGACGAAATCAGCCTGAATTCCGGCAAACCGTTT
CGTGAACTGGAGAGCGAACTGCTGTCACGTCGTAAAAAAGACCTGCAACAAATCTATGCCGAAGAACGTGAGAAC
TATCTGGGGAAACTGGAACGTGAAATCACCCGCTTTTTCGTGGATCGTGGCTTTCTGGAGATCAAATCCCCGATT
CTGATTCCTCTGGAGTATATCGAGCGTATGGGCATCGACAATGATACCGAACTGAGCAAACAAATTTTCCGTGTG
GATAAAAACTTCTGTCTGCGCCCTATGCTAGCACCAAATCTGGCTAACTATCTGCGCAAACTGGACCGTGCCCTG
CCTGATCCTATCAAAATCTTCGAGATCGGCCCGTGTTATCGTAAAGAGTCCGACGGTAAAGAACATCTGGAGGAG
TTTACCATGCTGAACTTTTGCCAAATGGGTTCAGGTTGTACTCGTGAGAACCTGGAAAGCATCATCACCGATTTT
CTGAACCACCTGGGCATTGACTTCAAAATTGTGGGCGACAGCTGTATGGTGTTTGGCGACACCCTGGATGTCATG
CACGGCGACCTGGAACTGTCTAGTGCCGTTGTGGGCCCAATCCCGCTGGATCGTGAGTGGGGTATCGACAAACCT
TGGATCGGTGCGGGTTTTGGTCTGGAGCGTCTGCTGAAAGTAAAACACGACTTCAAGAACATCAAACGTGCTGCA
CGTTCCGAGTCCTATTACAATGGTATTTCTACTAACCTGTAA (SEQ ID NO: 497)

Protein:
MVGRNSAIAAGVCGALFIGYCIYFDRKRRSDPNFKNRLRERRKKQKLAKERAGLSKLPDLKDAEAVQKFFMASND
YTQQATQSYGAYPTQPGQGYSQQSSQPYGQQSYSGYSQSTDTSGYGQSSYSSYGQSQNTGYGTQSTPQGYGSTGG
YGSSQSSQSSYGQQSSYPGYGQQPAPSSTSGSYGSSSQSSSYGQPQSGSYSQQPSYGGQQQSYGQQQSYNPPQGY
GQQNQYNSSSGGGGGGGGGGNYGQDQSSMSSGGGSGGGYGNQDQSGGGGSGGYGQQDRGGRGRGGSGGGGGGGGG
GYNRSSGGYEPRGRGGGRGGRGGMGGSDRGGFNKFGGPRDQGSRHDSEQDNSDNNTIFVQGLGENVTIESVADYF
KQIGIIKTNKKTGQPMINLYTDRETGKLKGEATVSFDDPPSAKAAIDWFDGKEFSGNPIKVSFATRRADFNRGGG

NGRGGRGRGGPMGRGGYGGGGSGGGGRGGFPSGGGGGGGQQRAGDWKCPNPTCENMNFSWRNECNQCKAPKPDGP
GGGPGGSHMGGNYGDDRRGGRGGAIAYPYDVPDYAGAPGSAGSAAGSGASNFTQFVLVDNGGTGDVTVAPSNFAN
GIAEWISSNSRSQAYKVTCSVRQSSAQNRKYTIKVEVPKGAWRSYLNMELTIPIFATNSDCELIVKAMQGLLKDG
NPIPSAIAANSGIYGADYKDDDDKGAPGSAGSAAGSGACPVPLQLPPLERLTLDDKKPLNTLISATGLWMSRTGT
IHKIKHHEVSRSKIYIEMACGDHLVVNNSRSSRTARALRHHKYRKTCKRCRVSDEDLNKFLTKANEDQTSVKVKV
VSAPTRTKKAMPKSVARAPKPLENTEAAQAQPSGSKFSPAIPVSTQESVSVPASVSTSISSISTGATASALVKGN
TNPITSMSAPVQASAPALTKSQTDRLEVLLNPKDEISLNSGKPFRELESELLSRRKKDLQQIYAEERENYLGKLE
REITRFFVDRGFLEIKSPILIPLEYIERMGIDNDTELSKQIFRVDKNFCLRPMLAPNLANYLRKLDRALPDPIKI
FEIGPCYRKESDGKEHLEEFTMLNFCQMGSGCTRENLESIITDFLNHLGIDFKIVGDSCMVFGDTLDVMHGDLEL
SSAVVGPIPLDREWGIDKPWIGAGFGLERLLKVKHDFKNIKRAARSESYYNGISTNLMASTDYSTYSQAAAQQGY
SAYTAQPTQGYAQTTQAYGQQSYGTYGQPTDVSYTQAQTTATYGQTAYATSYGQPPTGYTTPTAPQAYSQPVQGY
GTGAYDTTTATVTTTQASYAAQSAYGTQPAYPAYGQQPAATAPTRPQDGNKPTETSQPQSSTGGYNQPSLGYGQS
NYSYPQVPGSYPMQPVTAPPSYPPTSYSSTQPTSYDQSSYSQQNTYGQPSSYGQQSSYGQQSSYGQQPPTSYPPQ
TGSYSQAPSQYSQQSSSYGQQSSFRQDHPSSMGVYGQESGGFSGPGENRSMSGPDNRGRGRGGFDRGGMSRGGRG
GGRGGMGSAGERGGFNKPGGPMDEGPDLDLGPPVDPDEDSDNSAIYVQGLNDSVTLDDLADFFKQCGVVKMNKRT
GQPMIHIYLDKETGKPKGDATVSYEDPPTAKAAVEWFDGKDFQGSKLKVSLARKKPPMNSMRGGLPPREGRGMPP
PLRGGPGGPGGPGGPMGRMGGRGGDRGGFPPRGPRGSRGNPSGGGNVQHRAGDWQCPNPGCGNQNFAWRTECNQC
KAPKPEGFLPPPFPPPGGDRGRGGPGGMRGGRGGLMDRGGPGGMFRGGRGGDRGGFRGGRGMDRGGFGGGRRGGP
GGPPGPLMEQDKKPLNTLISATGLWMSRTGTIHKIKHHEVSRSKIYIEMACGDHLVVNNSRSSRTARALRHHKYR
KTCKRCRVSDEDLNKFLTKANEDQTSVKVKVVSAPTRTKKAMPKSVARAPKPLENTEAAQAQPSGSKFSPAIPVS
TQESVSVPASVSTSISSISTGATASALVKGNTNPITSMSAPVQASAPALTKSQTDRLEVLLNPKDEISLNSGKPF
RELESELLSRRKKDLQQIYAEERENYLGKLEREITRFFVDRGFLEIKSPILIPLEYIERMGIDNDTELSKQIFRV
DKNFCLRPMLAPNLANYLRKLDRALPDPIKIFEIGPCYRKESDGKEHLEEFTMLNFCQMGSGCTRENLESIITDF
LNHLGIDFKIVGDSCMVFGDTLDVMHGDLELSSAVVGPIPLDREWGIDKPWIGAGFGLERLLKVKHDFKNIKRAA
RSESYYNGISTNL* (SEQ ID NO: 498)

TOM20::FUS::4xλN22::PylRS(AF)::EWSR1::PylRS(AF)
DNA:
ATGGTGGGTCGGAACAGCGCCATCGCCGCCGGTGTATGCGGGGCCCTTTTCATTGGGTACTGCATCTACTTCGAC
CGCAAAAGACGAAGTGACCCCAACTTCAAGAACAGGCTTCGAGAACGAAGAAAGAAACAGAAGCTTGCCAAGGAG
AGAGCTGGGCTTTCCAAGTTACCTGACCTTAAAGATGCTGAAGCTGTTCAGAAATTCTTCATGGCCTCAAACGAT
TATACCCAACAAGCAACCCAAAGCTATGGGGCCTACCCCACCCAGCCCGGGCAGGGCTATTCCCAGCAGAGCAGT
CAGCCCTACGGACAGCAGAGTTACAGTGGTTATAGCCAGTCCACGGACACTTCAGGATATGGCCAGAGCAGCTAT
TCTTCTTATGGCCAGAGCCAGAACACAGGCTATGGAACTCAGTCAACTCCCCAGGGATATGGCTCGACTGGCGGC
TATGGCAGTAGCCAGAGCTCCCAATCGTCTTACGGGCAGCAGTCCTCCTACCCTGGCTATGGCCAGCAGCCAGCT
CCCAGCAGCACCTCGGGAAGTTACGGTAGCAGTTCTCAGAGCAGCAGCTATGGGCAGCCCCAGAGTGGGAGCTAC
AGCCAGCAGCCTAGCTATGGTGGACAGCAGCAAAGCTATGGACAGCAGCAAAGCTATAATCCCCCTCAGGGCTAT
GGACAGCAGAACCAGTACAACAGCAGCAGTGGTGGTGGAGGTGGAGGTGGAGGTGGAGGTAACTATGGCCAAGAT
CAATCCTCCATGAGTAGTGGTGGTGGCAGTGGTGGCGGTTATGGCAATCAAGACCAGAGTGGTGGAGGTGGCAGC
GGTGGCTATGGACAGCAGGACCGTGGAGGCCGCGGCAGGGGTGGCAGTGGTGGCGGCGGCGGCGGCGGCGGTGGT
GGTTACAACCGCAGCAGTGGTGGCTATGAACCCAGAGGTCGTGGAGGTGGCCGTGGAGGCAGAGGTGGCATGGGC
GGAAGTGACCGTGGTGGCTTCAATAAATTTGGTGGCCCTCGGGACCAAGGATCACGTCATGACTCCGAACAGGAT
AATTCAGACAACAACACCATCTTTGTGCAAGGCCTGGGTGAGAATGTTACAATTGAGTCTGTGGCTGATTACTTC
AAGCAGATTGGTATTATTAAGACAAACAAGAAAACGGGACAGCCCATGATTAATTTGTACACAGACAGGGAAACT
GGCAAGCTGAAGGGAGAGGCAACGGTCTCTTTTGATGACCCACCTTCAGCTAAAGCAGCTATTGACTGGTTTGAT
GGTAAAGAATTCTCCGGAAATCCTATCAAGGTCTCATTTGCTACTCGCCGGGCAGACTTTAATCGGGGTGGTGGC
AATGGTCGTGGAGGCCGAGGGCGAGGAGGACCCATGGGCCGTGGAGGCTATGGAGGTGGTGGCAGTGGTGGTGGT
GGCCGAGGAGGATTTCCCAGTGGAGGTGGTGGCGGTGGAGGACAGCAGCGAGCTGGTGACTGGAAGTGTCCTAAT
CCCACCTGTGAGAATATGAACTTCTCTTGGAGGAATGAATGCAACCAGTGTAAGGCCCCTAAACCAGATGGCCCA
GGAGGGGGACCAGGTGGCTCTCACATGGGGGGTAACTACGGGGATGATCGTCGTGGTGGCAGAGGAGGCGCGATC
GCAGGCAAGCCTATTCCCAACCCCCTGCTGGGCCTGGATAGCACCGGAGCACCAGGAAGTGCTGGTTCTGCTGCT
GGTAGTGGAGCATCGATAGAGCAGAAGCTGATCTCAGAGGAGGACCTGCTAGCCACCATGGACGCACAAACACGA
CGACGTGAGCGTCGCGCTGAGAAACAAGCTCAATGGAAAGCTGCAAACCCACCGCTCGACGGAGCCGGAGCTGGC
GCTGGAGCTGGAGCCGGAGCTGGCGGTCTAGCCACCATGGACGCACAAACACGACGACGTGAGCGTCGCGCTGAG
AAACAAGCTCAATGGAAAGCTGCAAACCCACCGCTCGACGGAGCCGGAGCTGGCGCTGGAGCTGGAGCCGGAGCT
GGCGGTCTAGCCACCATGGACGCACAAACACGACGACGTGAGCGTCGCGCTGAGAAACAAGCTCAATGGAAAGCT
GCAAACCCACCGCTCGACGGAGCCGGAGCTGGCGCTGGAGCTGGAGCCGGAGCTGGCGGTCTAGCCACCATGGAC

GCACAAACACGACGACGTGAGCGTCGCGCTGAGAAACAAGCTCAATGGAAAGCTGCAAACCCACCGCTCGAGTCT
AGAGGGCCCGTTGGTGCTCCTGGTTCAGCAGGAAGCGCAGCAGGATCAGGTGCGTGCCCGGTGCCGCTGCAGCTG
CCGCCGCTGGAACGCCTGACCCTGGATGACAAAAAACCGCTGAATACCCTGATCTCTGCTACTGGTCTGTGGATG
AGTCGTACCGGAACCATTCATAAAATCAAACACCACGAGGTTAGCCGTTCGAAAATCTATATTGAGATGGCGTGT
GGCGATCATCTGGTTGTGAACAATAGCCGCTCTTCTCGTACAGCACGTGCACTGCGTCACCACAAATATCGTAAA
ACCTGTAAACGTTGCCGTGTGTCCGATGAGGATCTGAACAAATTCCTGACAAAAGCCAATGAGGACCAAACAAGC
GTGAAAGTGAAAGTCGTTAGCGCTCCTACCCGTACTAAAAAAGCAATGCCGAAATCCGTTGCTCGTGCCCCTAAA
CCACTGGAAAACACTGAAGCAGCACAGGCACAGCCGTCTGGAAGCAAATTCTCTCCGGCCATTCCTGTTTCTACC
CAGGAGTCCGTTTCTGTTCCAGCAAGTGTGAGCACCAGCATTAGCAGTATTAGCACCGGTGCCACCGCTAGCGCC
CTGGTTAAAGGCAATACCAATCCGATTACAAGCATGTCTGCCCCGGTTCAAGCATCAGCTCCAGCACTGACAAAA
TCCCAAACCGATCGTCTGGAGGTTCTGCTGAATCCGAAAGACGAAATCAGCCTGAATTCCGGCAAACCGTTTCGT
GAACTGGAGAGCGAACTGCTGTCACGTCGTAAAAAAGACCTGCAACAAATCTATGCCGAAGAACGTGAGAACTAT
CTGGGGAAACTGGAACGTGAAATCACCCGCTTTTTCGTGGATCGTGGCTTTCTGGAGATCAAATCCCCGATTCTG
ATTCCTCTGGAGTATATCGAGCGTATGGGCATCGACAATGATACCGAACTGAGCAAACAAATTTTCCGTGTGGAT
AAAAACTTCTGTCTGCGCCCTATGCTAGCACCAAATCTGGCTAACTATCTGCGCAAACTGGACCGTGCCCTGCCT
GATCCTATCAAAATCTTCGAGATCGGCCCGTGTTATCGTAAAGAGTCCGACGGTAAAGAACATCTGGAGGAGTTT
ACCATGCTGAACTTTTGCCAAATGGGTTCAGGTTGTACTCGTGAGAACCTGGAAAGCATCATCACCGATTTTCTG
AACCACCTGGGCATTGACTTCAAAATTGTGGGCGACAGCTGTATGGTGTTTGGCGACACCCTGGATGTCATGCAC
GGCGACCTGGAACTGTCTAGTGCCGTTGTGGGCCCAATCCCGCTGGATCGTGAGTGGGGTATCGACAAACCTTGG
ATCGGTGCGGGTTTTGGTCTGGAGCGTCTGCTGAAAGTAAAACACGACTTCAAGAACATCAAACGTGCTGCACGT
TCCGAGTCCTATTACAATGGTATTTCTACTAACCTGATGGCGTCCACGGATTACAGTACCTATAGCCAAGCTGCA
GCGCAGCAGGGCTACAGTGCTTACACCGCCCAGCCCACTCAAGGATATGCACAGACCACCCAGGCATATGGGCAA
CAAAGCTATGGAACCTATGGACAGCCCACTGATGTCAGCTATACCCAGGCTCAGACCACTGCAACCTATGGGCAG
ACCGCCTATGCAACTTCTTATGGACAGCCTCCCACTGGTTATACTACTCCAACTGCCCCCCAGGCATACAGCCAG
CCTGTCCAGGGGTATGGCACTGGTGCTTATGATACCACCACTGCTACAGTCACCACCACCCAGGCCTCCTATGCA
GCTCAGTCTGCATATGGCACTCAGCCTGCTTATCCAGCCTATGGGCAGCAGCCAGCAGCCACTGCACCTACAAGA
CCGCAGGATGGAAACAAGCCCACTGAGACTAGTCAACCTCAATCTAGCACAGGGGGTTACAACCAGCCCAGCCTA
GGATATGGACAGAGTAACTACAGTTATCCCCAGGTACCTGGGAGCTACCCCATGCAGCCAGTCACTGCACCTCCA
TCCTACCCTCCTACCAGCTATTCCTCTACACAGCCGACTAGTTATGATCAGAGCAGTTACTCTCAGCAGAACACC
TATGGGCAACCGAGCAGCTATGGACAGCAGAGTAGCTATGGTCAACAAAGCAGCTATGGGCAGCAGCCTCCCACT
AGTTACCCACCCCAAACTGGATCCTACAGCCAAGCTCCAAGTCAATATAGCCAACAGAGCAGCAGCTACGGGCAG
CAGAGTTCATTCCGACAGGACCACCCCAGTAGCATGGGTGTTTATGGGCAGGAGTCTGGAGGATTTTCCGGACCA
GGAGAGAACCGGAGCATGAGTGGCCCTGATAACCGGGGCAGGGGAAGAGGGGGATTTGATCGTGGAGGCATGAGC
AGAGGTGGGCGGGGAGGAGGACGCGGTGGAATGGGCAGCGCTGGAGAGCGAGGTGGCTTCAATAAGCCTGGTGGA
CCCATGGATGAAGGACCAGATCTTGATCTAGGCCCACCTGTAGATCCAGATGAAGACTCTGACAACAGTGCAATT
TATGTACAAGGATTAAATGACAGTGTGACTCTAGATGATCTGGCAGACTTCTTTAAGCAGTGTGGGGTTGTTAAG
ATGAACAAGAGAACTGGGCAACCCATGATCCACATCTACCTGGACAAGGAAACAGGAAAGCCCAAAGGCGATGCC
ACAGTGTCCTATGAAGACCCACCTACTGCCAAGGCTGCCGTGGAATGGTTTGATGGGAAAGATTTTCAAGGGAGC
AAACTTAAAGTCTCCCTTGCTCGGAAGAAGCCTCCAATGAACAGTATGCGGGGTGGTCTGCCACCCCGTGAGGGC
AGAGGCATGCCACCACCACTCCGTGGAGGTCCAGGAGGCCCAGGAGGTCCTGGGGGACCCATGGGTCGCATGGGA
GGCCGTGGAGGAGATAGAGGAGGCTTCCCTCCAAGAGGACCCCGGGGTTCCCGAGGGAACCCCTCTGGAGGAGGA
AACGTCCAGCACCGAGCTGGAGACTGGCAGTGTCCCAATCCGGGTTGTGGAAACCAGAACTTCGCCTGGAGAACA
GAGTGCAACCAGTGTAAGGCCCCAAAGCCTGAAGGCTTCCTCCCGCCACCCTTTCCGCCCCCGGGTGGTGATCGT
GGCAGAGGTGGCCCTGGTGGCATGCGGGGAGGAAGAGGTGGCCTCATGGATCGTGGTGGTCCCGGTGGAATGTTC
AGAGGTGGCCGTGGTGGAGACAGAGGTGGCTTCCGTGGTGGCCGGGGCATGGACCGAGGTGGCTTTGGTGGAGGA
AGACGAGGTGGCCCTGGGGGGCCCCCTGGACCTTTGATGGAACAGGATAAAAAACCGCTGAATACCCTGATCTCT
GCTACTGGTCTGTGGATGAGTCGTACCGGAACCATTCATAAAATCAAACACCACGAGGTTAGCCGTTCGAAAATC
TATATTGAGATGGCGTGTGGCGATCATCTGGTTGTGAACAATAGCCGCTCTTCTCGTACAGCACGTGCACTGCGT
CACCACAAATATCGTAAAACCTGTAAACGTTGCCGTGTGTCCGATGAGGATCTGAACAAATTCCTGACAAAAGCC
AATGAGGACCAAACAAGCGTGAAAGTGAAAGTCGTTAGCGCTCCTACCCGTACTAAAAAAGCAATGCCGAAATCC
GTTGCTCGTGCCCCTAAACCACTGGAAAACACTGAAGCAGCACAGGCACAGCCGTCTGGAAGCAAATTCTCTCCG
GCCATTCCTGTTTCTACCCAGGAGTCCGTTTCTGTTCCAGCAAGTGTGAGCACCAGCATTAGCAGTATTAGCACC
GGTGCCACCGCTAGCGCCCTGGTTAAAGGCAATACCAATCCGATTACAAGCATGTCTGCCCCGGTTCAAGCATCA
GCTCCAGCACTGACAAAATCCCAAACCGATCGTCTGGAGGTTCTGCTGAATCCGAAAGACGAAATCAGCCTGAAT
TCCGGCAAACCGTTTCGTGAACTGGAGAGCGAACTGCTGTCACGTCGTAAAAAAGACCTGCAACAAATCTATGCC
GAAGAACGTGAGAACTATCTGGGGAAACTGGAACGTGAAATCACCCGCTTTTTCGTGGATCGTGGCTTTCTGGAG
ATCAAATCCCCGATTCTGATTCCTCTGGAGTATATCGAGCGTATGGGCATCGACAATGATACCGAACTGAGCAAA
CAAATTTTCCGTGTGGATAAAAACTTCTGTCTGCGCCCTATGCTAGCACCAAATCTGGCTAACTATCTGCGCAAA

CTGGACCGTGCCCTGCCTGATCCTATCAAAATCTTCGAGATCGGCCCGTGTTATCGTAAAGAGTCCGACGGTAAA
GAACATCTGGAGGAGTTTACCATGCTGAACTTTTGCCAAATGGGTTCAGGTTGTACTCGTGAGAACCTGGAAAGC
ATCATCACCGATTTTCTGAACCACCTGGGCATTGACTTCAAAATTGTGGGCGACAGCTGTATGGTGTTTGGCGAC
ACCCTGGATGTCATGCACGGCGACCTGGAACTGTCTAGTGCCGTTGTGGGCCCAATCCCGCTGGATCGTGAGTGG
GGTATCGACAAACCTTGGATCGGTGCGGGTTTTGGTCTGGAGCGTCTGCTGAAAGTAAAACACGACTTCAAGAAC
ATCAAACGTGCTGCACGTTCCGAGTCCTATTACAATGGTATTTCTACTAACCTGTAA (SEQ ID NO: 499)

Protein:
MVGRNSAIAAGVCGALFIGYCIYFDRKRRSDPNFKNRLRERRKKQKLAKERAGLSKLPDLKDAEAVQKFFMASND
YTQQATQSYGAYPTQPGQGYSQQSSQPYGQQSYSGYSQSTDTSGYGQSSYSSYGQSQNTGYGTQSTPQGYGSTGG
YGSSQSSQSSYGQQSSYPGYGQQPAPSSTSGSYGSSSQSSSYGQPQSGSYSQQPSYGGQQQSYGQQQSYNPPQGY
GQQNQYNSSSGGGGGGGGGGNYGQDQSSMSSGGGSGGGYGNQDQSGGGGSGGYGQQDRGGRGRGGSGGGGGGGGG
GYNRSSGGYEPRGRGGGRGGRGGMGGSDRGGFNKFGGPRDQGSRHDSEQDNSDNNTIFVQGLGENVTIESVADYF
KQIGIIKTNKKTGQPMINLYTDRETGKLKGEATVSFDDPPSAKAAIDWFDGKEFSGNPIKVSFATRRADFNRGGG
NGRGGRGRGGPMGRGGYGGGGSGGGGRGGFPSGGGGGGGQQRAGDWKCPNPTCENMNFSWRNECNQCKAPKPDGP
GGGPGGSHMGGNYGDDRRGGRGGAIAGKPIPNPLLGLDSTGAPGSAGSAAGSGASIEQKLISEEDLLATMDAQTR
RRERRAEKQAQWKAANPPLDGAGAGAGAGAGAGGLATMDAQTRRRERRAEKQAQWKAANPPLDGAGAGAGAGAGA
GGLATMDAQTRRRERRAEKQAQWKAANPPLDGAGAGAGAGAGAGGLATMDAQTRRRERRAEKQAQWKAANPPLES
RGPVGAPGSAGSAAGSGACPVPLQLPPLERLTLDDKKPLNTLISATGLWMSRTGTIHKIKHHEVSRSKIYIEMAC
GDHLVVNNSRSSRTARALRHHKYRKTCKRCRVSDEDLNKFLTKANEDQTSVKVKVVSAPTRTKKAMPKSVARAPK
PLENTEAAQAQPSGSKFSPAIPVSTQESVSVPASVSTSISSISTGATASALVKGNTNPITSMSAPVQASAPALTK
SQTDRLEVLLNPKDEISLNSGKPFRELESELLSRRKKDLQQIYAEERENYLGKLEREITRFFVDRGFLEIKSPIL
IPLEYIERMGIDNDTELSKQIFRVDKNFCLRPMLAPNLANYLRKLDRALPDPIKIFEIGPCYRKESDGKEHLEEF
TMLNFCQMGSGCTRENLESIITDFLNHLGIDFKIVGDSCMVFGDTLDVMHGDLELSSAVVGPIPLDREWGIDKPW
IGAGFGLERLLKVKHDFKNIKRAARSESYYNGISTNLMASTDYSTYSQAAAQQGYSAYTAQPTQGYAQTTQAYGQ
QSYGTYGQPTDVSYTQAQTTATYGQTAYATSYGQPPTGYTTPTAPQAYSQPVQGYGTGAYDTTTATVTTTQASYA
AQSAYGTQPAYPAYGQQPAATAPTRPQDGNKPTETSQPQSSTGGYNQPSLGYGQSNYSYPQVPGSYPMQPVTAPP
SYPPTSYSSTQPTSYDQSSYSQQNTYGQPSSYGQQSSYGQQSSYGQQPPTSYPPQTGSYSQAPSQYSQQSSSYGQ
QSSFRQDHPSSMGVYGQESGGFSGPGENRSMSGPDNRGRGRGGFDRGGMSRGGRGGGRGGMGSAGERGGFNKPGG
PMDEGPDLDLGPPVDPDEDSDNSAIYVQGLNDSVTLDDLADFFKQCGVVKMNKRTGQPMIHIYLDKETGKPKGDA
TVSYEDPPTAKAAVEWFDGKDFQGSKLKVSLARKKPPMNSMRGGLPPREGRGMPPPLRGGPGGPGGPGGPMGRMG
GRGGDRGGFPPRGPRGSRGNPSGGGNVQHRAGDWQCPNPGCGNQNFAWRTECNQCKAPKPEGFLPPPFPPPGGDR
GRGGPGGMRGGRGGLMDRGGPGGMFRGGRGGDRGGFRGGRGMDRGGFGGGRRGGPGGPPGPLMEQDKKPLNTLIS
ATGLWMSRTGTIHKIKHHEVSRSKIYIEMACGDHLVVNNSRSSRTARALRHHKYRKTCKRCRVSDEDLNKFLTKA
NEDQTSVKVKVVSAPTRTKKAMPKSVARAPKPLENTEAAQAQPSGSKFSPAIPVSTQESVSVPASVSTSISSIST
GATASALVKGNTNPITSMSAPVQASAPALTKSQTDRLEVLLNPKDEISLNSGKPFRELESELLSRRKKDLQQIYA
EERENYLGKLEREITRFFVDRGFLEIKSPILIPLEYIERMGIDNDTELSKQIFRVDKNFCLRPMLAPNLANYLRK
LDRALPDPIKIFEIGPCYRKESDGKEHLEEFTMLNFCQMGSGCTRENLESIITDFLNHLGIDFKIVGDSCMVFGD
TLDVMHGDLELSSAVVGPIPLDREWGIDKPWIGAGFGLERLLKVKHDFKNIKRAARSESYYNGISTNL* (SEQ
ID NO: 500)

TOM20::FUS::SYNZIP1::MCP::PylRS(AF)::EWSR1::PylRS(AF)
DNA:
ATGGTGGGTCGGAACAGCGCCATCGCCGCCGGTGTATGCGGGGCCCTTTTCATTGGGTACTGCATCTACTTCGAC
CGCAAAAGACGAAGTGACCCCAACTTCAAGAACAGGCTTCGAGAACGAAGAAAGAAACAGAAGCTTGCCAAGGAG
AGAGCTGGGCTTTCCAAGTTACCTGACCTTAAAGATGCTGAAGCTGTTCAGAAATTCTTCATGGCCTCAAACGAT
TATACCCAACAAGCAACCCAAAGCTATGGGGCCTACCCCACCCAGCCCGGGCAGGGCTATTCCCAGCAGAGCAGT
CAGCCCTACGGACAGCAGAGTTACAGTGGTTATAGCCAGTCCACGGACACTTCAGGATATGGCCAGAGCAGCTAT
TCTTCTTATGGCCAGAGCCAGAACACAGGCTATGGAACTCAGTCAACTCCCCAGGGATATGGCTCGACTGGCGGC
TATGGCAGTAGCCAGAGCTCCCAATCGTCTTACGGGCAGCAGTCCTCCTACCCTGGCTATGGCCAGCAGCCAGCT
CCCAGCAGCACCTCGGGAAGTTACGGTAGCAGTTCTCAGAGCAGCAGCTATGGGCAGCCCCAGAGTGGGAGCTAC
AGCCAGCAGCCTAGCTATGGTGGACAGCAGCAAAGCTATGGACAGCAGCAAAGCTATAATCCCCCTCAGGGCTAT
GGACAGCAGAACCAGTACAACAGCAGCAGTGGTGGTGGAGGTGGAGGTGGAGGTGGAGGTAACTATGGCCAAGAT
CAATCCTCCATGAGTAGTGGTGGTGGCAGTGGTGGCGGTTATGGCAATCAAGACCAGAGTGGTGGAGGTGGCAGC
GGTGGCTATGGACAGCAGGACCGTGGAGGCCGCGGCAGGGGTGGCAGTGGTGGCGGCGGCGGCGGCGGCGGTGGT
GGTTACAACCGCAGCAGTGGTGGCTATGAACCCAGAGGTCGTGGAGGTGGCCGTGGAGGCAGAGGTGGCATGGGC
GGAAGTGACCGTGGTGGCTTCAATAAATTTGGTGGCCCTCGGGACCAAGGATCACGTCATGACTCCGAACAGGAT

AATTCAGACAACAACACCATCTTTGTGCAAGGCCTGGGTGAGAATGTTACAATTGAGTCTGTGGCTGATTACTTC
AAGCAGATTGGTATTATTAAGACAAACAAGAAAACGGGACAGCCCATGATTAATTTGTACACAGACAGGGAAACT
GGCAAGCTGAAGGGAGAGGCAACGGTCTCTTTTGATGACCCACCTTCAGCTAAAGCAGCTATTGACTGGTTTGAT
GGTAAAGAATTCTCCGGAAATCCTATCAAGGTCTCATTTGCTACTCGCCGGGCAGACTTTAATCGGGGTGGTGGC
AATGGTCGTGGAGGCCGAGGGCGAGGAGGACCCATGGGCCGTGGAGGCTATGGAGGTGGTGGCAGTGGTGGTGGT
GGCCGAGGAGGATTTCCCAGTGGAGGTGGTGGCGGTGGAGGACAGCAGCGAGCTGGTGACTGGAAGTGTCCTAAT
CCCACCTGTGAGAATATGAACTTCTCTTGGAGGAATGAATGCAACCAGTGTAAGGCCCCTAAACCAGATGGCCCA
GGAGGGGGACCAGGTGGCTCTCACATGGGGGGTAACTACGGGGATGATCGTCGTGGTGGCAGAGGAGGCGCGATC
GCAAATCTGGTGGCCCAGCTGGAAAACGAGGTGGCCAGCCTGGAAAACGAGAACGAAACCCTGAAGAAAAAGAAC
CTGCACAAGAAGGACCTGATCGCCTACCTGGAAAAGGAAATCGCCAACCTGAGAAAGAAGATCGAGGAAGCATCG
ATATATCCCTATGATGTGCCGGATTATGCTGGAGCACCAGGAAGTGCTGGTTCTGCTGCTGGTAGTGGAGCTTCT
AACTTTACTCAGTTCGTTCTCGTCGACAATGGCGGAACTGGCGACGTGACTGTCGCCCCAAGCAACTTCGCTAAC
GGGATCGCTGAATGGATCAGCTCTAACTCGCGTTCACAGGCTTACAAAGTAACCTGTAGCGTTCGTCAGAGCTCT
GCGCAGAATCGCAAATACACCATCAAAGTCGAGGTGCCTAAAGGCGCCTGGCGTTCGTACTTAAATATGGAACTA
ACCATTCCAATTTTCGCCACGAATTCCGACTGCGAGCTTATTGTTAAGGCAATGCAAGGTCTCCTAAAAGATGGA
AACCCGATTCCCTCAGCAATCGCAGCAAACTCCGGCATCTACGGCGCCGATTACAAGGACGATGATGACAAGGGA
GCACCAGGAAGTGCTGGTTCTGCTGCTGGTAGTGGAGCGTGCCCGGTGCCGCTGCAGCTGCCGCCGCTGGAACGC
CTGACCCTGGATGATAAAAAACCGCTGAATACCCTGATCTCTGCTACTGGTCTGTGGATGAGTCGTACCGGAACC
ATTCATAAAATCAAACACCACGAGGTTAGCCGTTCGAAAATCTATATTGAGATGGCGTGTGGCGATCATCTGGTT
GTGAACAATAGCCGCTCTTCTCGTACAGCACGTGCACTGCGTCACCACAAATATCGTAAAACCTGTAAACGTTGC
CGTGTGTCCGATGAGGATCTGAACAAATTCCTGACAAAAGCCAATGAGGACCAAACAAGCGTGAAAGTGAAAGTC
GTTAGCGCTCCTACCCGTACTAAAAAAGCAATGCCGAAATCCGTTGCTCGTGCCCCTAAACCACTGGAAAACACT
GAAGCAGCACAGGCACAGCCGTCTGGAAGCAAATTCTCTCCGGCCATTCCTGTTTCTACCCAGGAGTCCGTTTCT
GTTCCAGCAAGTGTGAGCACCAGCATTAGCAGTATTAGCACCGGTGCCACCGCTAGCGCCCTGGTTAAAGGCAAT
ACCAATCCGATTACAAGCATGTCTGCCCCGGTTCAAGCATCAGCTCCAGCACTGACAAAATCCCAAACCGATCGT
CTGGAGGTTCTGCTGAATCCGAAAGACGAAATCAGCCTGAATTCCGGCAAACCGTTTCGTGAACTGGAGAGCGAA
CTGCTGTCACGTCGTAAAAAAGACCTGCAACAAATCTATGCCGAAGAACGTGAGAACTATCTGGGGAAACTGGAA
CGTGAAATCACCCGCTTTTTCGTGGATCGTGGCTTTCTGGAGATCAAATCCCCGATTCTGATTCCTCTGGAGTAT
ATCGAGCGTATGGGCATCGACAATGATACCGAACTGAGCAAACAAATTTTCCGTGTGGATAAAAACTTCTGTCTG
CGCCCTATGCTAGCACCAAATCTGGCTAACTATCTGCGCAAACTGGACCGTGCCCTGCCTGATCCTATCAAAATC
TTCGAGATCGGCCCGTGTTATCGTAAAGAGTCCGACGGTAAAGAACATCTGGAGGAGTTTACCATGCTGAACTTT
TGCCAAATGGGTTCAGGTTGTACTCGTGAGAACCTGGAAAGCATCATCACCGATTTTCTGAACCACCTGGGCATT
GACTTCAAAATTGTGGGCGACAGCTGTATGGTGTTTGGCGACACCCTGGATGTCATGCACGGCGACCTGGAACTG
TCTAGTGCCGTTGTGGGCCCAATCCCGCTGGATCGTGAGTGGGGTATCGACAAACCTTGGATCGGTGCGGGTTTT
GGTCTGGAGCGTCTGCTGAAAGTAAAACACGACTTCAAGAACATCAAACGTGCTGCACGTTCCGAGTCCTATTAC
AATGGTATTTCTACTAACCTGATGGCGTCCACGGATTACAGTACCTATAGCCAAGCTGCAGCGCAGCAGGGCTAC
AGTGCTTACACCGCCCAGCCCACTCAAGGATATGCACAGACCACCCAGGCATATGGGCAACAAAGCTATGGAACC
TATGGACAGCCCACTGATGTCAGCTATACCCAGGCTCAGACCACTGCAACCTATGGGCAGACCGCCTATGCAACT
TCTTATGGACAGCCTCCCACTGGTTATACTACTCCAACTGCCCCCCAGGCATACAGCCAGCCTGTCCAGGGGTAT
GGCACTGGTGCTTATGATACCACCACTGCTACAGTCACCACCACCCAGGCCTCCTATGCAGCTCAGTCTGCATAT
GGCACTCAGCCTGCTTATCCAGCCTATGGGCAGCAGCCAGCAGCCACTGCACCTACAAGACCGCAGGATGGAAAC
AAGCCCACTGAGACTAGTCAACCTCAATCTAGCACAGGGGGTTACAACCAGCCCAGCCTAGGATATGGACAGAGT
AACTACAGTTATCCCCAGGTACCTGGGAGCTACCCCATGCAGCCAGTCACTGCACCTCCATCCTACCCTCCTACC
AGCTATTCCTCTACACAGCCGACTAGTTATGATCAGAGCAGTTACTCTCAGCAGAACACCTATGGGCAACCGAGC
AGCTATGGACAGCAGAGTAGCTATGGTCAACAAAGCAGCTATGGGCAGCAGCCTCCCACTAGTTACCCACCCCAA
ACTGGATCCTACAGCCAAGCTCCAAGTCAATATAGCCAACAGAGCAGCAGCTACGGGCAGCAGAGTTCATTCCGA
CAGGACCACCCCAGTAGCATGGGTGTTTATGGGCAGGAGTCTGGAGGATTTTCCGGACCAGGAGAGAACCGGAGC
ATGAGTGGCCCTGATAACCGGGGCAGGGGAAGAGGGGGATTTGATCGTGGAGGCATGAGCAGAGGTGGGCGGGGA
GGAGGACGCGGTGGAATGGGCAGCGCTGGAGAGCGAGGTGGCTTCAATAAGCCTGGTGGACCCATGGATGAAGGA
CCAGATCTTGATCTAGGCCCACCTGTAGATCCAGATGAAGACTCTGACAACAGTGCAATTTATGTACAAGGATTA
AATGACAGTGTGACTCTAGATGATCTGGCAGACTTCTTTAAGCAGTGTGGGGTTGTTAAGATGAACAAGAGAACT
GGGCAACCCATGATCCACATCTACCTGGACAAGGAAACAGGAAAGCCCAAAGGCGATGCCACAGTGTCCTATGAA
GACCCACCTACTGCCAAGGCTGCCGTGGAATGGTTTGATGGGAAAGATTTTCAAGGGAGCAAACTTAAAGTCTCC
CTTGCTCGGAAGAAGCCTCCAATGAACAGTATGCGGGGTGGTCTGCCACCCCGTGAGGGCAGAGGCATGCCACCA
CCACTCCGTGGAGGTCCAGGAGGCCCAGGAGGTCCTGGGGGACCCATGGGTCGCATGGGAGGCCGTGGAGGAGAT
AGAGGAGGCTTCCCTCCAAGAGGACCCCGGGGTTCCCGAGGGAACCCCTCTGGAGGAGGAAACGTCCAGCACCGA
GCTGGAGACTGGCAGTGTCCCAATCCGGGTTGTGGAAACCAGAACTTCGCCTGGAGAACAGAGTGCAACCAGTGT
AAGGCCCCAAAGCCTGAAGGCTTCCTCCCGCCACCCTTTCCGCCCCCGGGTGGTGATCGTGGCAGAGGTGGCCCT

GGTGGCATGCGGGGAGGAAGAGGTGGCCTCATGGATCGTGGTGGTCCCGGTGGAATGTTCAGAGGTGGCCGTGGT
GGAGACAGAGGTGGCTTCCGTGGTGGCCGGGGCATGGACCGAGGTGGCTTTGGTGGAGGAAGACGAGGTGGCCCT
GGGGGGCCCCCTGGACCTTTGATGGAACAGGATAAAAAACCGCTGAATACCCTGATCTCTGCTACTGGTCTGTGG
ATGAGTCGTACCGGAACCATTCATAAAATCAAACACCACGAGGTTAGCCGTTCGAAAATCTATATTGAGATGGCG
TGTGGCGATCATCTGGTTGTGAACAATAGCCGCTCTTCTCGTACAGCACGTGCACTGCGTCACCACAAATATCGT
AAAACCTGTAAACGTTGCCGTGTGTCCGATGAGGATCTGAACAAATTCCTGACAAAAGCCAATGAGGACCAAACA
AGCGTGAAAGTGAAAGTCGTTAGCGCTCCTACCCGTACTAAAAAAGCAATGCCGAAATCCGTTGCTCGTGCCCCT
AAACCACTGGAAAACACTGAAGCAGCACAGGCACAGCCGTCTGGAAGCAAATTCTCTCCGGCCATTCCTGTTTCT
ACCCAGGAGTCCGTTTCTGTTCCAGCAAGTGTGAGCACCAGCATTAGCAGTATTAGCACCGGTGCCACCGCTAGC
GCCCTGGTTAAAGGCAATACCAATCCGATTACAAGCATGTCTGCCCCGGTTCAAGCATCAGCTCCAGCACTGACA
AAATCCCAAACCGATCGTCTGGAGGTTCTGCTGAATCCGAAAGACGAAATCAGCCTGAATTCCGGCAAACCGTTT
CGTGAACTGGAGAGCGAACTGCTGTCACGTCGTAAAAAAGACCTGCAACAAATCTATGCCGAAGAACGTGAGAAC
TATCTGGGGAAACTGGAACGTGAAATCACCCGCTTTTTCGTGGATCGTGGCTTTCTGGAGATCAAATCCCCGATT
CTGATTCCTCTGGAGTATATCGAGCGTATGGGCATCGACAATGATACCGAACTGAGCAAACAAATTTTCCGTGTG
GATAAAAACTTCTGTCTGCGCCCTATGCTAGCACCAAATCTGGCTAACTATCTGCGCAAACTGGACCGTGCCCTG
CCTGATCCTATCAAAATCTTCGAGATCGGCCCGTGTTATCGTAAAGAGTCCGACGGTAAAGAACATCTGGAGGAG
TTTACCATGCTGAACTTTTGCCAAATGGGTTCAGGTTGTACTCGTGAGAACCTGGAAAGCATCATCACCGATTTT
CTGAACCACCTGGGCATTGACTTCAAAATTGTGGGCGACAGCTGTATGGTGTTTGGCGACACCCTGGATGTCATG
CACGGCGACCTGGAACTGTCTAGTGCCGTTGTGGGCCCAATCCCGCTGGATCGTGAGTGGGGTATCGACAAACCT
TGGATCGGTGCGGGTTTTGGTCTGGAGCGTCTGCTGAAAGTAAAACACGACTTCAAGAACATCAAACGTGCTGCA
CGTTCCGAGTCCTATTACAATGGTATTTCTACTAACCTGTAA (SEQ ID NO: 501)

Protein:
MVGRNSAIAAGVCGALFIGYCIYFDRKRRSDPNFKNRLRERRKKQKLAKERAGLSKLPDLKDAEAVQKFFMASND
YTQQATQSYGAYPTQPGQGYSQQSSQPYGQQSYSGYSQSTDTSGYGQSSYSSYGQSQNTGYGTQSTPQGYGSTGG
YGSSQSSQSSYGQQSSYPGYGQQPAPSSTSGSYGSSSQSSSYGQPQSGSYSQQPSYGGQQQSYGQQQSYNPPQGY
GQQNQYNSSSGGGGGGGGGGNYGQDQSSMSSGGGSGGGYGNQDQSGGGGSGGYGQQDRGGRGRGGSGGGGGGGGG
GYNRSSGGYEPRGRGGGRGGRGGMGGSDRGGFNKFGGPRDQGSRHDSEQDNSDNNTIFVQGLGENVTIESVADYF
KQIGIIKTNKKTGQPMINLYTDRETGKLKGEATVSFDDPPSAKAAIDWFDGKEFSGNPIKVSFATRRADFNRGGG
NGRGGRGRGGPMGRGGYGGGGSGGGGRGGFPSGGGGGGGQQRAGDWKCPNPTCENMNFSWRNECNQCKAPKPDGP
GGGPGGSHMGGNYGDDRRGGRGGAIANLVAQLENEVASLENENETLKKKNLHKKDLIAYLEKEIANLRKKIEEAS
IYPYDVPDYAGAPGSAGSAAGSGASNFTQFVLVDNGGTGDVTVAPSNFANGIAEWISSNSRSQAYKVTCSVRQSS
AQNRKYTIKVEVPKGAWRSYLNMELTIPIFATNSDCELIVKAMQGLLKDGNPIPSAIAANSGIYGADYKDDDDKG
APGSAGSAAGSGACPVPLQLPPLERLTLDDKKPLNTLISATGLWMSRTGTIHKIKHHEVSRSKIYIEMACGDHLV
VNNSRSSRTARALRHHKYRKTCKRCRVSDEDLNKFLTKANEDQTSVKVKVVSAPTRTKKAMPKSVARAPKPLENT
EAAQAQPSGSKFSPAIPVSTQESVSVPASVSTSISSISTGATASALVKGNTNPITSMSAPVQASAPALTKSQTDR
LEVLLNPKDEISLNSGKPFRELESELLSRRKKDLQQIYAEERENYLGKLEREITRFFVDRGFLEIKSPILIPLEY
IERMGIDNDTELSKQIFRVDKNFCLRPMLAPNLANYLRKLDRALPDPIKIFEIGPCYRKESDGKEHLEEFTMLNF
CQMGSGCTRENLESIITDFLNHLGIDFKIVGDSCMVFGDTLDVMHGDLELSSAVVGPIPLDREWGIDKPWIGAGF
GLERLLKVKHDFKNIKRAARSESYYNGISTNLMASTDYSTYSQAAAQQGYSAYTAQPTQGYAQTTQAYGQQSYGT
YGQPTDVSYTQAQTTATYGQTAYATSYGQPPTGYTTPTAPQAYSQPVQGYGTGAYDTTTATVTTTQASYAAQSAY
GTQPAYPAYGQQPAATAPTRPQDGNKPTETSQPQSSTGGYNQPSLGYGQSNYSYPQVPGSYPMQPVTAPPSYPPT
SYSSTQPTSYDQSSYSQQNTYGQPSSYGQQSSYGQQSSYGQQPPTSYPPQTGSYSQAPSQYSQQSSSYGQQSSFR
QDHPSSMGVYGQESGGFSGPGENRSMSGPDNRGRGRGGFDRGGMSRGGRGGGRGGMGSAGERGGFNKPGGPMDEG
PDLDLGPPVDPDEDSDNSAIYVQGLNDSVTLDDLADFFKQCGVVKMNKRTGQPMIHIYLDKETGKPKGDATVSYE
DPPTAKAAVEWFDGKDFQGSKLKVSLARKKPPMNSMRGGLPPREGRGMPPPLRGGPGGPGGPGGPMGRMGGRGGD
RGGFPPRGPRGSRGNPSGGGNVQHRAGDWQCPNPGCGNQNFAWRTECNQCKAPKPEGFLPPPFPPPGGDRGRGGP
GGMRGGRGGLMDRGGPGGMFRGGRGGDRGGFRGGRGMDRGGFGGGRRGGPGGPPGPLMEQDKKPLNTLISATGLW
MSRTGTIHKIKHHEVSRSKIYIEMACGDHLVVNNSRSSRTARALRHHKYRKTCKRCRVSDEDLNKFLTKANEDQT
SVKVKVVSAPTRTKKAMPKSVARAPKPLENTEAAQAQPSGSKFSPAIPVSTQESVSVPASVSTSISSISTGATAS
ALVKGNTNPITSMSAPVQASAPALTKSQTDRLEVLLNPKDEISLNSGKPFRELESELLSRRKKDLQQIYAEEREN
YLGKLEREITRFFVDRGFLEIKSPILIPLEYIERMGIDNDTELSKQIFRVDKNFCLRPMLAPNLANYLRKLDRAL
PDPIKIFEIGPCYRKESDGKEHLEEFTMLNFCQMGSGCTRENLESIITDFLNHLGIDFKIVGDSCMVFGDTLDVM
HGDLELSSAVVGPIPLDREWGIDKPWIGAGFGLERLLKVKHDFKNIKRAARSESYYNGISTNL* (SEQ ID
NO: 502)

TOM20::FUS::SYNZIP2::MCP::PylRS(AF)::EWSR1::PylRS(AF)
DNA:

ATGGTGGGTCGGAACAGCGCCATCGCCGCCGGTGTATGCGGGGCCCTTTTCATTGGGTACTGCATCTACTTCGAC
CGCAAAAGACGAAGTGACCCCAACTTCAAGAACAGGCTTCGAGAACGAAGAAAGAAACAGAAGCTTGCCAAGGAG
AGAGCTGGGCTTTCCAAGTTACCTGACCTTAAAGATGCTGAAGCTGTTCAGAAATTCTTCATGGCCTCAAACGAT
TATACCCAACAAGCAACCCAAAGCTATGGGGCCTACCCCACCCAGCCCGGGCAGGGCTATTCCCAGCAGAGCAGT
CAGCCCTACGGACAGCAGAGTTACAGTGGTTATAGCCAGTCCACGGACACTTCAGGATATGGCCAGAGCAGCTAT
TCTTCTTATGGCCAGAGCCAGAACACAGGCTATGGAACTCAGTCAACTCCCCAGGGATATGGCTCGACTGGCGGC
TATGGCAGTAGCCAGAGCTCCCAATCGTCTTACGGGCAGCAGTCCTCCTACCCTGGCTATGGCCAGCAGCCAGCT
CCCAGCAGCACCTCGGGAAGTTACGGTAGCAGTTCTCAGAGCAGCAGCTATGGGCAGCCCCAGAGTGGGAGCTAC
AGCCAGCAGCCTAGCTATGGTGGACAGCAGCAAAGCTATGGACAGCAGCAAAGCTATAATCCCCCTCAGGGCTAT
GGACAGCAGAACCAGTACAACAGCAGCAGTGGTGGTGGAGGTGGAGGTGGAGGTGGAGGTAACTATGGCCAAGAT
CAATCCTCCATGAGTAGTGGTGGTGGCAGTGGTGGCGGTTATGGCAATCAAGACCAGAGTGGTGGAGGTGGCAGC
GGTGGCTATGGACAGCAGGACCGTGGAGGCCGCGGCAGGGGTGGCAGTGGTGGCGGCGGCGGCGGCGGCGGTGGT
GGTTACAACCGCAGCAGTGGTGGCTATGAACCCAGAGGTCGTGGAGGTGGCCGTGGAGGCAGAGGTGGCATGGGC
GGAAGTGACCGTGGTGGCTTCAATAAATTTGGTGGCCCTCGGGACCAAGGATCACGTCATGACTCCGAACAGGAT
AATTCAGACAACAACACCATCTTTGTGCAAGGCCTGGGTGAGAATGTTACAATTGAGTCTGTGGCTGATTACTTC
AAGCAGATTGGTATTATTAAGACAAACAAGAAAACGGGACAGCCCATGATTAATTTGTACACAGACAGGGAAACT
GGCAAGCTGAAGGGAGAGGCAACGGTCTCTTTTGATGACCCACCTTCAGCTAAAGCAGCTATTGACTGGTTTGAT
GGTAAAGAATTCTCCGGAAATCCTATCAAGGTCTCATTTGCTACTCGCCGGGCAGACTTTAATCGGGGTGGTGGC
AATGGTCGTGGAGGCCGAGGGCGAGGAGGACCCATGGGCCGTGGAGGCTATGGAGGTGGTGGCAGTGGTGGTGGT
GGCCGAGGAGGATTTCCCAGTGGAGGTGGTGGCGGTGGAGGACAGCAGCGAGCTGGTGACTGGAAGTGTCCTAAT
CCCACCTGTGAGAATATGAACTTCTCTTGGAGGAATGAATGCAACCAGTGTAAGGCCCCTAAACCAGATGGCCCA
GGAGGGGGACCAGGTGGCTCTCACATGGGGGGTAACTACGGGGATGATCGTCGTGGTGGCAGAGGAGGCGCGATC
GCAGCTAGAAACGCCTACCTGAGAAAGAAAATCGCCAGACTGAAGAAGGACAACCTGCAGCTGGAAAGAGACGAG
CAGAACCTGGAAAAGATCATCGCCAACCTCAGAGATGAGATCGCCAGACTGGAAAACGAGGTGGCCAGCCACGAG
CAGGCATCGATATATCCCTATGATGTGCCGGATTATGCTGGAGCACCAGGAAGTGCTGGTTCTGCTGCTGGTAGT
GGAGCTTCTAACTTTACTCAGTTCGTTCTCGTCGACAATGGCGGAACTGGCGACGTGACTGTCGCCCCAAGCAAC
TTCGCTAACGGGATCGCTGAATGGATCAGCTCTAACTCGCGTTCACAGGCTTACAAAGTAACCTGTAGCGTTCGT
CAGAGCTCTGCGCAGAATCGCAAATACACCATCAAAGTCGAGGTGCCTAAAGGCGCCTGGCGTTCGTACTTAAAT
ATGGAACTAACCATTCCAATTTTCGCCACGAATTCCGACTGCGAGCTTATTGTTAAGGCAATGCAAGGTCTCCTA
AAAGATGGAAACCCGATTCCCTCAGCAATCGCAGCAAACTCCGGCATCTACGGCGCCGATTACAAGGACGATGAT
GACAAGGGAGCACCAGGAAGTGCTGGTTCTGCTGCTGGTAGTGGAGCGTGCCCGGTGCCGCTGCAGCTGCCGCCG
CTGGAACGCCTGACCCTGGATGATAAAAAACCGCTGAATACCCTGATCTCTGCTACTGGTCTGTGGATGAGTCGT
ACCGGAACCATTCATAAAATCAAACACCACGAGGTTAGCCGTTCGAAAATCTATATTGAGATGGCGTGTGGCGAT
CATCTGGTTGTGAACAATAGCCGCTCTTCTCGTACAGCACGTGCACTGCGTCACCACAAATATCGTAAAACCTGT
AAACGTTGCCGTGTGTCCGATGAGGATCTGAACAAATTCCTGACAAAAGCCAATGAGGACCAAACAAGCGTGAAA
GTGAAAGTCGTTAGCGCTCCTACCCGTACTAAAAAAGCAATGCCGAAATCCGTTGCTCGTGCCCCTAAACCACTG
GAAAACACTGAAGCAGCACAGGCACAGCCGTCTGGAAGCAAATTCTCTCCGGCCATTCCTGTTTCTACCCAGGAG
TCCGTTTCTGTTCCAGCAAGTGTGAGCACCAGCATTAGCAGTATTAGCACCGGTGCCACCGCTAGCGCCCTGGTT
AAAGGCAATACCAATCCGATTACAAGCATGTCTGCCCCGGTTCAAGCATCAGCTCCAGCACTGACAAAATCCCAA
ACCGATCGTCTGGAGGTTCTGCTGAATCCGAAAGACGAAATCAGCCTGAATTCCGGCAAACCGTTTCGTGAACTG
GAGAGCGAACTGCTGTCACGTCGTAAAAAAGACCTGCAACAAATCTATGCCGAAGAACGTGAGAACTATCTGGGG
AAACTGGAACGTGAAATCACCCGCTTTTTCGTGGATCGTGGCTTTCTGGAGATCAAATCCCCGATTCTGATTCCT
CTGGAGTATATCGAGCGTATGGGCATCGACAATGATACCGAACTGAGCAAACAAATTTTCCGTGTGGATAAAAAC
TTCTGTCTGCGCCCTATGCTAGCACCAAATCTGGCTAACTATCTGCGCAAACTGGACCGTGCCCTGCCTGATCCT
ATCAAAATCTTCGAGATCGGCCCGTGTTATCGTAAAGAGTCCGACGGTAAAGAACATCTGGAGGAGTTTACCATG
CTGAACTTTTGCCAAATGGGTTCAGGTTGTACTCGTGAGAACCTGGAAAGCATCATCACCGATTTTCTGAACCAC
CTGGGCATTGACTTCAAAATTGTGGGCGACAGCTGTATGGTGTTTGGCGACACCCTGGATGTCATGCACGGCGAC
CTGGAACTGTCTAGTGCCGTTGTGGGCCCAATCCCGCTGGATCGTGAGTGGGGTATCGACAAACCTTGGATCGGT
GCGGGTTTTGGTCTGGAGCGTCTGCTGAAAGTAAAACACGACTTCAAGAACATCAAACGTGCTGCACGTTCCGAG
TCCTATTACAATGGTATTTCTACTAACCTGATGGCGTCCACGGATTACAGTACCTATAGCCAAGCTGCAGCGCAG
CAGGGCTACAGTGCTTACACCGCCCAGCCCACTCAAGGATATGCACAGACCACCCAGGCATATGGGCAACAAAGC
TATGGAACCTATGGACAGCCCACTGATGTCAGCTATACCCAGGCTCAGACCACTGCAACCTATGGGCAGACCGCC
TATGCAACTTCTTATGGACAGCCTCCCACTGGTTATACTACTCCAACTGCCCCCCAGGCATACAGCCAGCCTGTC
CAGGGGTATGGCACTGGTGCTTATGATACCACCACTGCTACAGTCACCACCACCCAGGCCTCCTATGCAGCTCAG
TCTGCATATGGCACTCAGCCTGCTTATCCAGCCTATGGGCAGCAGCCAGCAGCCACTGCACCTACAAGACCGCAG
GATGGAAACAAGCCCACTGAGACTAGTCAACCTCAATCTAGCACAGGGGGTTACAACCAGCCCAGCCTAGGATAT
GGACAGAGTAACTACAGTTATCCCCAGGTACCTGGGAGCTACCCCATGCAGCCAGTCACTGCACCTCCATCCTAC
CCTCCTACCAGCTATTCCTCTACACAGCCGACTAGTTATGATCAGAGCAGTTACTCTCAGCAGAACACCTATGGG

CAACCGAGCAGCTATGGACAGCAGAGTAGCTATGGTCAACAAAGCAGCTATGGGCAGCAGCCTCCCACTAGTTAC
CCACCCCAAACTGGATCCTACAGCCAAGCTCCAAGTCAATATAGCCAACAGAGCAGCAGCTACGGGCAGCAGAGT
TCATTCCGACAGGACCACCCCAGTAGCATGGGTGTTTATGGGCAGGAGTCTGGAGGATTTTCCGGACCAGGAGAG
AACCGGAGCATGAGTGGCCCTGATAACCGGGGCAGGGGAAGAGGGGGATTTGATCGTGGAGGCATGAGCAGAGGT
GGGCGGGGAGGAGGACGCGGTGGAATGGGCAGCGCTGGAGAGCGAGGTGGCTTCAATAAGCCTGGTGGACCCATG
GATGAAGGACCAGATCTTGATCTAGGCCCACCTGTAGATCCAGATGAAGACTCTGACAACAGTGCAATTTATGTA
CAAGGATTAAATGACAGTGTGACTCTAGATGATCTGGCAGACTTCTTTAAGCAGTGTGGGGTTGTTAAGATGAAC
AAGAGAACTGGGCAACCCATGATCCACATCTACCTGGACAAGGAAACAGGAAAGCCCAAAGGCGATGCCACAGTG
TCCTATGAAGACCCACCTACTGCCAAGGCTGCCGTGGAATGGTTTGATGGGAAAGATTTTCAAGGGAGCAAACTT
AAAGTCTCCCTTGCTCGGAAGAAGCCTCCAATGAACAGTATGCGGGGTGGTCTGCCACCCCGTGAGGGCAGAGGC
ATGCCACCACCACTCCGTGGAGGTCCAGGAGGCCCAGGAGGTCCTGGGGGACCCATGGGTCGCATGGGAGGCCGT
GGAGGAGATAGAGGAGGCTTCCCTCCAAGAGGACCCCGGGGTTCCCGAGGGAACCCCTCTGGAGGAGGAAACGTC
CAGCACCGAGCTGGAGACTGGCAGTGTCCCAATCCGGGTTGTGGAAACCAGAACTTCGCCTGGAGAACAGAGTGC
AACCAGTGTAAGGCCCCAAAGCCTGAAGGCTTCCTCCCGCCACCCTTTCCGCCCCCGGGTGGTGATCGTGGCAGA
GGTGGCCCTGGTGGCATGCGGGGAGGAAGAGGTGGCCTCATGGATCGTGGTGGTCCCGGTGGAATGTTCAGAGGT
GGCCGTGGTGGAGACAGAGGTGGCTTCCGTGGTGGCCGGGGCATGGACCGAGGTGGCTTTGGTGGAGGAAGACGA
GGTGGCCCTGGGGGGCCCCCTGGACCTTTGATGGAACAGGATAAAAAACCGCTGAATACCCTGATCTCTGCTACT
GGTCTGTGGATGAGTCGTACCGGAACCATTCATAAAATCAAACACCACGAGGTTAGCCGTTCGAAAATCTATATT
GAGATGGCGTGTGGCGATCATCTGGTTGTGAACAATAGCCGCTCTTCTCGTACAGCACGTGCACTGCGTCACCAC
AAATATCGTAAAACCTGTAAACGTTGCCGTGTGTCCGATGAGGATCTGAACAAATTCCTGACAAAAGCCAATGAG
GACCAAACAAGCGTGAAAGTGAAAGTCGTTAGCGCTCCTACCCGTACTAAAAAAGCAATGCCGAAATCCGTTGCT
CGTGCCCCTAAACCACTGGAAAACACTGAAGCAGCACAGGCACAGCCGTCTGGAAGCAAATTCTCTCCGGCCATT
CCTGTTTCTACCCAGGAGTCCGTTTCTGTTCCAGCAAGTGTGAGCACCAGCATTAGCAGTATTAGCACCGGTGCC
ACCGCTAGCGCCCTGGTTAAAGGCAATACCAATCCGATTACAAGCATGTCTGCCCCGGTTCAAGCATCAGCTCCA
GCACTGACAAAATCCCAAACCGATCGTCTGGAGGTTCTGCTGAATCCGAAAGACGAAATCAGCCTGAATTCCGGC
AAACCGTTTCGTGAACTGGAGAGCGAACTGCTGTCACGTCGTAAAAAAGACCTGCAACAAATCTATGCCGAAGAA
CGTGAGAACTATCTGGGGAAACTGGAACGTGAAATCACCCGCTTTTTCGTGGATCGTGGCTTTCTGGAGATCAAA
TCCCCGATTCTGATTCCTCTGGAGTATATCGAGCGTATGGGCATCGACAATGATACCGAACTGAGCAAACAAATT
TTCCGTGTGGATAAAAACTTCTGTCTGCGCCCTATGCTAGCACCAAATCTGGCTAACTATCTGCGCAAACTGGAC
CGTGCCCTGCCTGATCCTATCAAAATCTTCGAGATCGGCCCGTGTTATCGTAAAGAGTCCGACGGTAAAGAACAT
CTGGAGGAGTTTACCATGCTGAACTTTTGCCAAATGGGTTCAGGTTGTACTCGTGAGAACCTGGAAAGCATCATC
ACCGATTTTCTGAACCACCTGGGCATTGACTTCAAAATTGTGGGCGACAGCTGTATGGTGTTTGGCGACACCCTG
GATGTCATGCACGGCGACCTGGAACTGTCTAGTGCCGTTGTGGGCCCAATCCCGCTGGATCGTGAGTGGGGTATC
GACAAACCTTGGATCGGTGCGGGTTTTGGTCTGGAGCGTCTGCTGAAAGTAAAACACGACTTCAAGAACATCAAA
CGTGCTGCACGTTCCGAGTCCTATTACAATGGTATTTCTACTAACCTGTAA (SEQ ID NO: 503)

Protein:
MVGRNSAIAAGVCGALFIGYCIYFDRKRRSDPNFKNRLRERRKKQKLAKERAGLSKLPDLKDAEAVQKFFMASND
YTQQATQSYGAYPTQPGQGYSQQSSQPYGQQSYSGYSQSTDTSGYGQSSYSSYGQSQNTGYGTQSTPQGYGSTGG
YGSSQSSQSSYGQQSSYPGYGQQPAPSSTSGSYGSSSQSSSYGQPQSGSYSQQPSYGGQQQSYGQQQSYNPPQGY
GQQNQYNSSSGGGGGGGGGGNYGQDQSSMSSGGGSGGGYGNQDQSGGGGSGGYGQQDRGGRGRGGSGGGGGGGGG
GYNRSSGGYEPRGRGGGRGGRGGMGGSDRGGFNKFGGPRDQGSRHDSEQDNSDNNTIFVQGLGENVTIESVADYF
KQIGIIKTNKKTGQPMINLYTDRETGKLKGEATVSFDDPPSAKAAIDWFDGKEFSGNPIKVSFATRRADFNRGGG
NGRGGRGRGGPMGRGGYGGGGSGGGGRGGFPSGGGGGGGQQRAGDWKCPNPTCENMNFSWRNECNQCKAPKPDGP
GGGPGGSHMGGNYGDDRRGGRGGAIAARNAYLRKKIARLKKDNLQLERDEQNLEKIIANLRDEIARLENEVASHE
QASIYPYDVPDYAGAPGSAGSAAGSGASNFTQFVLVDNGGTGDVTVAPSNFANGIAEWISSNSRSQAYKVTCSVR
QSSAQNRKYTIKVEVPKGAWRSYLNMELTIPIFATNSDCELIVKAMQGLLKDGNPIPSAIAANSGIYGADYKDDD
DKGAPGSAGSAAGSGACPVPLQLPPLERLTLDDKKPLNTLISATGLWMSRTGTIHKIKHHEVSRSKIYIEMACGD
HLVVNNSRSSRTARALRHHKYRKTCKRCRVSDEDLNKFLTKANEDQTSVKVKVVSAPTRTKKAMPKSVARAPKPL
ENTEAAQAQPSGSKFSPAIPVSTQESVSVPASVSTSISSISTGATASALVKGNTNPITSMSAPVQASAPALTKSQ
TDRLEVLLNPKDEISLNSGKPFRELESELLSRRKKDLQQIYAEERENYLGKLEREITRFFVDRGFLEIKSPILIP
LEYIERMGIDNDTELSKQIFRVDKNFCLRPMLAPNLANYLRKLDRALPDPIKIFEIGPCYRKESDGKEHLEEFTM
LNFCQMGSGCTRENLESIITDFLNHLGIDFKIVGDSCMVFGDTLDVMHGDLELSSAVVGPIPLDREWGIDKPWIG
AGFGLERLLKVKHDFKNIKRAARSESYYNGISTNLMASTDYSTYSQAAAQQGYSAYTAQPTQGYAQTTQAYGQQS
YGTYGQPTDVSYTQAQTTATYGQTAYATSYGQPPTGYTTPTAPQAYSQPVQGYGTGAYDTTTATVTTTQASYAAQ
SAYGTQPAYPAYGQQPAATAPTRPQDGNKPTETSQPQSSTGGYNQPSLGYGQSNYSYPQVPGSYPMQPVTAPPSY
PPTSYSSTQPTSYDQSSYSQQNTYGQPSSYGQQSSYGQQSSYGQQPPTSYPPQTGSYSQAPSQYSQQSSSYGQQS
SFRQDHPSSMGVYGQESGGFSGPGENRSMSGPDNRGRGRGGFDRGGMSRGGRGGGRGGMGSAGERGGFNKPGGPM

DEGPDLDLGPPVDPDEDSDNSAIYVQGLNDSVTLDDLADFFKQCGVVKMNKRTGQPMIHIYLDKETGKPKGDATV
SYEDPPTAKAAVEWFDGKDFQGSKLKVSLARKKPPMNSMRGGLPPREGRGMPPPLRGGPGGPGGPGGPMGRMGGR
GGDRGGFPPRGPRGSRGNPSGGGNVQHRAGDWQCPNPGCGNQNFAWRTECNQCKAPKPEGFLPPPFPPPGGDRGR
GGPGGMRGGRGGLMDRGGPGGMFRGGRGGDRGGFRGGRGMDRGGFGGGRRGGPGGPPGPLMEQDKKPLNTLISAT
GLWMSRTGTIHKIKHHEVSRSKIYIEMACGDHLVVNNSRSSRTARALRHHKYRKTCKRCRVSDEDLNKFLTKANE
DQTSVKVKVVSAPTRTKKAMPKSVARAPKPLENTEAAQAQPSGSKFSPAIPVSTQESVSVPASVSTSISSISTGA
TASALVKGNTNPITSMSAPVQASAPALTKSQTDRLEVLLNPKDEISLNSGKPFRELESELLSRRKKDLQQIYAEE
RENYLGKLEREITRFFVDRGFLEIKSPILIPLEYIERMGIDNDTELSKQIFRVDKNFCLRPMLAPNLANYLRKLD
RALPDPIKIFEIGPCYRKESDGKEHLEEFTMLNFCQMGSGCTRENLESIITDFLNHLGIDFKIVGDSCMVFGDTL
DVMHGDLELSSAVVGPIPLDREWGIDKPWIGAGFGLERLLKVKHDFKNIKRAARSESYYNGISTNL* (SEQ ID
NO: 504)

LCK::FUS::SYNZIP1::MCP::PylRS(AF)::EWSR1::PylRS(AF)
DNA:
ATGGGCTGCGTGTGCAGCAGCAACCCCGAGGGTACCGAGCTCGCCTCAAACGATTATACCCAACAAGCAACCCAA
AGCTATGGGGCCTACCCCACCCAGCCCGGGCAGGGCTATTCCCAGCAGAGCAGTCAGCCCTACGGACAGCAGAGT
TACAGTGGTTATAGCCAGTCCACGGACACTTCAGGATATGGCCAGAGCAGCTATTCTTCTTATGGCCAGAGCCAG
AACACAGGCTATGGAACTCAGTCAACTCCCCAGGGATATGGCTCGACTGGCGGCTATGGCAGTAGCCAGAGCTCC
CAATCGTCTTACGGGCAGCAGTCCTCCTACCCTGGCTATGGCCAGCAGCCAGCTCCCAGCAGCACCTCGGGAAGT
TACGGTAGCAGTTCTCAGAGCAGCAGCTATGGGCAGCCCCAGAGTGGGAGCTACAGCCAGCAGCCTAGCTATGGT
GGACAGCAGCAAAGCTATGGACAGCAGCAAAGCTATAATCCCCCTCAGGGCTATGGACAGCAGAACCAGTACAAC
AGCAGCAGTGGTGGTGGAGGTGGAGGTGGAGGTGGAGGTAACTATGGCCAAGATCAATCCTCCATGAGTAGTGGT
GGTGGCAGTGGTGGCGGTTATGGCAATCAAGACCAGAGTGGTGGAGGTGGCAGCGGTGGCTATGGACAGCAGGAC
CGTGGAGGCCGCGGCAGGGGTGGCAGTGGTGGCGGCGGCGGCGGCGGTGGTGGTTACAACCGCAGCAGTGGT
GGCTATGAACCCAGAGGTCGTGGAGGTGGCCGTGGAGGCAGAGGTGGCATGGGCGGAAGTGACCGTGGTGGCTTC
AATAAATTTGGTGGCCCTCGGGACCAAGGATCACGTCATGACTCCGAACAGGATAATTCAGACAACAACACCATC
TTTGTGCAAGGCCTGGGTGAGAATGTTACAATTGAGTCTGTGGCTGATTACTTCAAGCAGATTGGTATTATTAAG
ACAAACAAGAAAACGGGACAGCCCATGATTAATTTGTACACAGACAGGGAAACTGGCAAGCTGAAGGGAGAGGCA
ACGGTCTCTTTTGATGACCCACCTTCAGCTAAAGCAGCTATTGACTGGTTTGATGGTAAAGAATTCTCCGGAAAT
CCTATCAAGGTCTCATTTGCTACTCGCCGGGCAGACTTTAATCGGGGTGGTGGCAATGGTCGTGGAGGCCGAGGG
CGAGGAGGACCCATGGGCCGTGGAGGCTATGGAGGTGGTGGCAGTGGTGGTGGTGGCCGAGGAGGATTTCCCAGT
GGAGGTGGTGGCGGTGGAGGACAGCAGCGAGCTGGTGACTGGAAGTGTCCTAATCCCACCTGTGAGAATATGAAC
TTCTCTTGGAGGAATGAATGCAACCAGTGTAAGGCCCCTAAACCAGATGGCCCAGGAGGGGGACCAGGTGGCTCT
CACATGGGGGGTAACTACGGGGATGATCGTCGTGGTGGCAGAGGAGGCGCGATCGCAAATCTGGTGGCCCAGCTG
GAAAACGAGGTGGCCAGCCTGGAAAACGAGAACGAAACCCTGAAGAAAAAGAACCTGCACAAGAAGGACCTGATC
GCCTACCTGGAAAAGGAAATCGCCAACCTGAGAAAGAAGATCGAGGAAGCATCGATATATCCCTATGATGTGCCG
GATTATGCTGGAGCACCAGGAAGTGCTGGTTCTGCTGCTGGTAGTGGAGCTTCTAACTTTACTCAGTTCGTTCTC
GTCGACAATGGCGGAACTGGCGACGTGACTGTCGCCCCAAGCAACTTCGCTAACGGGATCGCTGAATGGATCAGC
TCTAACTCGCGTTCACAGGCTTACAAAGTAACCTGTAGCGTTCGTCAGAGCTCTGCGCAGAATCGCAAATACACC
ATCAAAGTCGAGGTGCCTAAAGGCGCCTGGCGTTCGTACTTAAATATGGAACTAACCATTCCAATTTTCGCCACG
AATTCCGACTGCGAGCTTATTGTTAAGGCAATGCAAGGTCTCCTAAAAGATGGAAACCCGATTCCCTCAGCAATC
GCAGCAAACTCCGGCATCTACGGCGCCGATTACAAGGACGATGATGACAAGGGAGCACCAGGAAGTGCTGGTTCT
GCTGCTGGTAGTGGAGCGTGCCCGGTGCCGCTGCAGCTGCCGCCGCTGGAACGCCTGACCCTGGATGATAAAAAA
CCGCTGAATACCCTGATCTCTGCTACTGGTCTGTGGATGAGTCGTACCGGAACCATTCATAAAATCAAACACCAC
GAGGTTAGCCGTTCGAAAATCTATATTGAGATGGCGTGTGGCGATCATCTGGTTGTGAACAATAGCCGCTCTTCT
CGTACAGCACGTGCACTGCGTCACCACAAATATCGTAAAACCTGTAAACGTTGCCGTGTGTCCGATGAGGATCTG
AACAAATTCCTGACAAAAGCCAATGAGGACCAAACAAGCGTGAAAGTGAAAGTCGTTAGCGCTCCTACCCGTACT
AAAAAAGCAATGCCGAAATCCGTTGCTCGTGCCCCTAAACCACTGGAAAACACTGAAGCAGCACAGGCACAGCCG
TCTGGAAGCAAATTCTCTCCGGCCATTCCTGTTTCTACCCAGGAGTCCGTTTCTGTTCCAGCAAGTGTGAGCACC
AGCATTAGCAGTATTAGCACCGGTGCCACCGCTAGCGCCCTGGTTAAAGGCAATACCAATCCGATTACAAGCATG
TCTGCCCCGGTTCAAGCATCAGCTCCAGCACTGACAAAATCCCAAACCGATCGTCTGGAGGTTCTGCTGAATCCG
AAAGACGAAATCAGCCTGAATTCCGGCAAACCGTTTCGTGAACTGGAGAGCGAACTGCTGTCACGTCGTAAAAAA
GACCTGCAACAAATCTATGCCGAAGAACGTGAGAACTATCTGGGGAAACTGGAACGTGAAATCACCCGCTTTTTC
GTGGATCGTGGCTTTCTGGAGATCAAATCCCCGATTCTGATTCCTCTGGAGTATATCGAGCGTATGGGCATCGAC
AATGATACCGAACTGAGCAAACAAATTTTCCGTGTGGATAAAAACTTCTGTCTGCGCCCTATGCTAGCACCAAAT
CTGGCTAACTATCTGCGCAAACTGGACCGTGCCCTGCCTGATCCTATCAAAATCTTCGAGATCGGCCCGTGTTAT
CGTAAAGAGTCCGACGGTAAAGAACATCTGGAGGAGTTTACCATGCTGAACTTTTGCCAAATGGGTTCAGGTTGT

ACTCGTGAGAACCTGGAAAGCATCATCACCGATTTTCTGAACCACCTGGGCATTGACTTCAAAATTGTGGGCGAC
AGCTGTATGGTGTTTGGCGACACCCTGGATGTCATGCACGGCGACCTGGAACTGTCTAGTGCCGTTGTGGGCCCA
ATCCCGCTGGATCGTGAGTGGGGTATCGACAAACCTTGGATCGGTGCGGGTTTTGGTCTGGAGCGTCTGCTGAAA
GTAAAACACGACTTCAAGAACATCAAACGTGCTGCACGTTCCGAGTCCTATTACAATGGTATTTCTACTAACCTG
ATGGCGTCCACGGATTACAGTACCTATAGCCAAGCTGCAGCGCAGCAGGGCTACAGTGCTTACACCGCCCAGCCC
ACTCAAGGATATGCACAGACCACCCAGGCATATGGGCAACAAAGCTATGGAACCTATGGACAGCCCACTGATGTC
AGCTATACCCAGGCTCAGACCACTGCAACCTATGGGCAGACCGCCTATGCAACTTCTTATGGACAGCCTCCCACT
GGTTATACTACTCCAACTGCCCCCCAGGCATACAGCCAGCCTGTCCAGGGGTATGGCACTGGTGCTTATGATACC
ACCACTGCTACAGTCACCACCACCCAGGCCTCCTATGCAGCTCAGTCTGCATATGGCACTCAGCCTGCTTATCCA
GCCTATGGGCAGCAGCCAGCAGCCACTGCACCTACAAGACCGCAGGATGGAAACAAGCCCACTGAGACTAGTCAA
CCTCAATCTAGCACAGGGGGTTACAACCAGCCCAGCCTAGGATATGGACAGAGTAACTACAGTTATCCCCAGGTA
CCTGGGAGCTACCCCATGCAGCCAGTCACTGCACCTCCATCCTACCCTCCTACCAGCTATTCCTCTACACAGCCG
ACTAGTTATGATCAGAGCAGTTACTCTCAGCAGAACACCTATGGGCAACCGAGCAGCTATGGACAGCAGAGTAGC
TATGGTCAACAAAGCAGCTATGGGCAGCAGCCTCCCACTAGTTACCCACCCCAAACTGGATCCTACAGCCAAGCT
CCAAGTCAATATAGCCAACAGAGCAGCAGCTACGGGCAGCAGAGTTCATTCCGACAGGACCACCCCAGTAGCATG
GGTGTTTATGGGCAGGAGTCTGGAGGATTTTCCGGACCAGGAGAGAACCGGAGCATGAGTGGCCCTGATAACCGG
GGCAGGGGAAGAGGGGGATTTGATCGTGGAGGCATGAGCAGAGGTGGGCGGGGAGGAGGACGCGGTGGAATGGGC
AGCGCTGGAGAGCGAGGTGGCTTCAATAAGCCTGGTGGACCCATGGATGAAGGACCAGATCTTGATCTAGGCCCA
CCTGTAGATCCAGATGAAGACTCTGACAACAGTGCAATTTATGTACAAGGATTAAATGACAGTGTGACTCTAGAT
GATCTGGCAGACTTCTTTAAGCAGTGTGGGGTTGTTAAGATGAACAAGAGAACTGGGCAACCCATGATCCACATC
TACCTGGACAAGGAAACAGGAAAGCCCAAAGGCGATGCCACAGTGTCCTATGAAGACCCACCTACTGCCAAGGCT
GCCGTGGAATGGTTTGATGGGAAAGATTTTCAAGGGAGCAAACTTAAAGTCTCCCTTGCTCGGAAGAAGCCTCCA
ATGAACAGTATGCGGGGTGGTCTGCCACCCCGTGAGGGCAGAGGCATGCCACCACCACTCCGTGGAGGTCCAGGA
GGCCCAGGAGGTCCTGGGGGACCCATGGGTCGCATGGGAGGCCGTGGAGGAGATAGAGGAGGCTTCCCTCCAAGA
GGACCCCGGGGTTCCCGAGGGAACCCCTCTGGAGGAGGAAACGTCCAGCACCGAGCTGGAGACTGGCAGTGTCCC
AATCCGGGTTGTGGAAACCAGAACTTCGCCTGGAGAACAGAGTGCAACCAGTGTAAGGCCCCAAAGCCTGAAGGC
TTCCTCCCGCCACCCTTTCCGCCCCCGGGTGGTGATCGTGGCAGAGGTGGCCCTGGTGGCATGCGGGGAGGAAGA
GGTGGCCTCATGGATCGTGGTGGTCCCGGTGGAATGTTCAGAGGTGGCCGTGGTGGAGACAGAGGTGGCTTCCGT
GGTGGCCGGGGCATGGACCGAGGTGGCTTTGGTGGAGGAAGACGAGGTGGCCCTGGGGGGCCCCCTGGACCTTTG
ATGGAACAGGATAAAAAACCGCTGAATACCCTGATCTCTGCTACTGGTCTGTGGATGAGTCGTACCGGAACCATT
CATAAAATCAAACACCACGAGGTTAGCCGTTCGAAAATCTATATTGAGATGGCGTGTGGCGATCATCTGGTTGTG
AACAATAGCCGCTCTTCTCGTACAGCACGTGCACTGCGTCACCACAAATATCGTAAAACCTGTAAACGTTGCCGT
GTGTCCGATGAGGATCTGAACAAATTCCTGACAAAAGCCAATGAGGACCAAACAAGCGTGAAAGTGAAAGTCGTT
AGCGCTCCTACCCGTACTAAAAAAGCAATGCCGAAATCCGTTGCTCGTGCCCCTAAACCACTGGAAAACACTGAA
GCAGCACAGGCACAGCCGTCTGGAAGCAAATTCTCTCCGGCCATTCCTGTTTCTACCCAGGAGTCCGTTTCTGTT
CCAGCAAGTGTGAGCACCAGCATTAGCAGTATTAGCACCGGTGCCACCGCTAGCGCCCTGGTTAAAGGCAATACC
AATCCGATTACAAGCATGTCTGCCCCGGTTCAAGCATCAGCTCCAGCACTGACAAAATCCCAAACCGATCGTCTG
GAGGTTCTGCTGAATCCGAAAGACGAAATCAGCCTGAATTCCGGCAAACCGTTTCGTGAACTGGAGAGCGAACTG
CTGTCACGTCGTAAAAAAGACCTGCAACAAATCTATGCCGAAGAACGTGAGAACTATCTGGGGAAACTGGAACGT
GAAATCACCCGCTTTTTCGTGGATCGTGGCTTTCTGGAGATCAAATCCCCGATTCTGATTCCTCTGGAGTATATC
GAGCGTATGGGCATCGACAATGATACCGAACTGAGCAAACAAATTTTCCGTGTGGATAAAAACTTCTGTCTGCGC
CCTATGCTAGCACCAAATCTGGCTAACTATCTGCGCAAACTGGACCGTGCCCTGCCTGATCCTATCAAAATCTTC
GAGATCGGCCCGTGTTATCGTAAAGAGTCCGACGGTAAAGAACATCTGGAGGAGTTTACCATGCTGAACTTTTGC
CAAATGGGTTCAGGTTGTACTCGTGAGAACCTGGAAAGCATCATCACCGATTTTCTGAACCACCTGGGCATTGAC
TTCAAAATTGTGGGCGACAGCTGTATGGTGTTTGGCGACACCCTGGATGTCATGCACGGCGACCTGGAACTGTCT
AGTGCCGTTGTGGGCCCAATCCCGCTGGATCGTGAGTGGGGTATCGACAAACCTTGGATCGGTGCGGGTTTTGGT
CTGGAGCGTCTGCTGAAAGTAAAACACGACTTCAAGAACATCAAACGTGCTGCACGTTCCGAGTCCTATTACAAT
GGTATTTCTACTAACCTGTAA (SEQ ID NO: 505)

Protein:
MGCVCSSNPEGTELASNDYTQQATQSYGAYPTQPGQGYSQQSSQPYGQQSYSGYSQSTDTSGYGQSSYSSYGQSQ
NTGYGTQSTPQGYGSTGGYGSSQSSQSSYGQQSSYPGYGQQPAPSSTSGSYGSSSQSSSYGQPQSGSYSQQPSYG
GQQQSYGQQQSYNPPQGYGQQNQYNSSSGGGGGGGGGNYGQDQSSMSSGGGSGGGYGNQDQSGGGGSGGYGQQD
RGGRGRGGSGGGGGGGGGGYNRSSGGYEPRGRGGGRGGRGGMGGSDRGGFNKFGGPRDQGSRHDSEQDNSDNNTI
FVQGLGENVTIESVADYFKQIGIIKTNKKTGQPMINLYTDRETGKLKGEATVSFDDPPSAKAAIDWFDGKEFSGN
PIKVSFATRRADFNRGGGNGRGGRGRGGPMGRGGYGGGGSGGGGRGGFPSGGGGGGGQQRAGDWKCPNPTCENMN
FSWRNECNQCKAPKPDGPGGGPGGSHMGGNYGDDRRGGRGGAIANLVAQLENEVASLENENETLKKKNLHKKDLI
AYLEKEIANLRKKIEEASIYPYDVPDYAGAPGSAGSAAGSGASNFTQFVLVDNGGTGDVTVAPSNFANGIAEWIS

SNSRSQAYKVTCSVRQSSAQNRKYTIKVEVPKGAWRSYLNMELTIPIFATNSDCELIVKAMQGLLKDGNPIPSAI
AANSGIYGADYKDDDDKGAPGSAGSAAGSGACPVPLQLPPLERLTLDDKKPLNTLISATGLWMSRTGTIHKIKHH
EVSRSKIYIEMACGDHLVVNNSRSSRTARALRHHKYRKTCKRCRVSDEDLNKFLTKANEDQTSVKVKVVSAPTRT
KKAMPKSVARAPKPLENTEAAQAQPSGSKFSPAIPVSTQESVSVPASVSTSISSISTGATASALVKGNTNPITSM
SAPVQASAPALTKSQTDRLEVLLNPKDEISLNSGKPFRELESELLSRRKKDLQQIYAEERENYLGKLEREITRFF
VDRGFLEIKSPILIPLEYIERMGIDNDTELSKQIFRVDKNFCLRPMLAPNLANYLRKLDRALPDPIKIFEIGPCY
RKESDGKEHLEEFTMLNFCQMGSGCTRENLESIITDFLNHLGIDFKIVGDSCMVFGDTLDVMHGDLELSSAVVGP
IPLDREWGIDKPWIGAGFGLERLLKVKHDFKNIKRAARSESYYNGISTNLMASTDYSTYSQAAAQQGYSAYTAQP
TQGYAQTTQAYGQQSYGTYGQPTDVSYTQAQTTATYGQTAYATSYGQPPTGYTTPTAPQAYSQPVQGYGTGAYDT
TTATVTTTQASYAAQSAYGTQPAYPAYGQQPAATAPTRPQDGNKPTETSQPQSSTGGYNQPSLGYGQSNYSYPQV
PGSYPMQPVTAPPSYPPTSYSSTQPTSYDQSSYSQQNTYGQPSSYGQQSSYGQQSSYGQQPPTSYPPQTGSYSQA
PSQYSQQSSSYGQQSSFRQDHPSSMGVYGQESGGFSGPGENRSMSGPDNRGRGRGGFDRGGMSRGGRGGGRGGMG
SAGERGGFNKPGGPMDEGPDLDLGPPVDPDEDSDNSAIYVQGLNDSVTLDDLADFFKQCGVVKMNKRTGQPMIHI
YLDKETGKPKGDATVSYEDPPTAKAAVEWFDGKDFQGSKLKVSLARKKPPMNSMRGGLPPREGRGMPPPLRGGPG
GPGGPGGPMGRMGGRGGDRGGFPPRGPRGSRGNPSGGGNVQHRAGDWQCPNPGCGNQNFAWRTECNQCKAPKPEG
FLPPPFPPPGGDRGRGGPGGMRGGRGGLMDRGGPGGMFRGGRGGDRGGFRGGRGMDRGGFGGGRRGGPGGPPGPL
MEQDKKPLNTLISATGLWMSRTGTIHKIKHHEVSRSKIYIEMACGDHLVVNNSRSSRTARALRHHKYRKTCKRCR
VSDEDLNKFLTKANEDQTSVKVKVVSAPTRTKKAMPKSVARAPKPLENTEAAQAQPSGSKFSPAIPVSTQESVSV
PASVSTSISSISTGATASALVKGNTNPITSMSAPVQASAPALTKSQTDRLEVLLNPKDEISLNSGKPFRELESEL
LSRRKKDLQQIYAEERENYLGKLEREITRFFVDRGFLEIKSPILIPLEYIERMGIDNDTELSKQIFRVDKNFCLR
PMLAPNLANYLRKLDRALPDPIKIFEIGPCYRKESDGKEHLEEFTMLNFCQMGSGCTRENLESIITDFLNHLGID
FKIVGDSCMVFGDTLDVMHGDLELSSAVVGPIPLDREWGIDKPWIGAGFGLERLLKVKHDFKNIKRAARSESYYN
GISTNL* (SEQ ID NO: 506)

LCK::FUS::SYNZIP2::MCP::PylRS(AF)::EWSR1::PylRS(AF)
DNA:
ATGGGCTGCGTGTGCAGCAGCAACCCCGAGGGTACCGAGCTCGCCTCAAACGATTATACCCAACAAGCAACCCAA
AGCTATGGGGCCTACCCCACCCAGCCCGGGCAGGGCTATTCCCAGCAGAGCAGTCAGCCCTACGGACAGCAGAGT
TACAGTGGTTATAGCCAGTCCACGGACACTTCAGGATATGGCCAGAGCAGCTATTCTTCTTATGGCCAGAGCCAG
AACACAGGCTATGGAACTCAGTCAACTCCCCAGGGATATGGCTCGACTGGCGGCTATGGCAGTAGCCAGAGCTCC
CAATCGTCTTACGGGCAGCAGTCCTCCTACCCTGGCTATGGCCAGCAGCCAGCTCCCAGCAGCACCTCGGGAAGT
TACGGTAGCAGTTCTCAGAGCAGCAGCTATGGGCAGCCCCAGAGTGGGAGCTACAGCCAGCAGCCTAGCTATGGT
GGACAGCAGCAAAGCTATGGACAGCAGCAAAGCTATAATCCCCCTCAGGGCTATGGACAGCAGAACCAGTACAAC
AGCAGCAGTGGTGGTGGAGGTGGAGGTGGAGGTGGAGGTAACTATGGCCAAGATCAATCCTCCATGAGTAGTGGT
GGTGGCAGTGGTGGCGGTTATGGCAATCAAGACCAGAGTGGTGGAGGTGGCAGCGGTGGCTATGGACAGCAGGAC
CGTGGAGGCCGCGGCAGGGGTGGCAGTGGTGGCGGCGGCGGCGGCGGCGGTGGTGGTTACAACCGCAGCAGTGGT
GGCTATGAACCCAGAGGTCGTGGAGGTGGCCGTGGAGGCAGAGGTGGCATGGGCGGAAGTGACCGTGGTGGCTTC
AATAAATTTGGTGGCCCTCGGGACCAAGGATCACGTCATGACTCCGAACAGGATAATTCAGACAACAACACCATC
TTTGTGCAAGGCCTGGGTGAGAATGTTACAATTGAGTCTGTGGCTGATTACTTCAAGCAGATTGGTATTATTAAG
ACAAACAAGAAAACGGGACAGCCCATGATTAATTTGTACACAGACAGGGAAACTGGCAAGCTGAAGGGAGAGGCA
ACGGTCTCTTTTGATGACCCACCTTCAGCTAAAGCAGCTATTGACTGGTTTGATGGTAAAGAATTCTCCGGAAAT
CCTATCAAGGTCTCATTTGCTACTCGCCGGGCAGACTTTAATCGGGGTGGTGGCAATGGTCGTGGAGGCCGAGGG
CGAGGAGGACCCATGGGCCGTGGAGGCTATGGAGGTGGTGGCAGTGGTGGTGGTGGCCGAGGAGGATTTCCCAGT
GGAGGTGGTGGCGGTGGAGGACAGCAGCGAGCTGGTGACTGGAAGTGTCCTAATCCCACCTGTGAGAATATGAAC
TTCTCTTGGAGGAATGAATGCAACCAGTGTAAGGCCCCTAAACCAGATGGCCCAGGAGGGGGACCAGGTGGCTCT
CACATGGGGGGTAACTACGGGGATGATCGTCGTGGTGGCAGAGGAGGCGCGATCGCAGCTAGAAACGCCTACCTG
AGAAAGAAAATCGCCAGACTGAAGAAGGACAACCTGCAGCTGGAAAGAGACGAGCAGAACCTGGAAAAGATCATC
GCCAACCTCAGAGATGAGATCGCCAGACTGGAAAACGAGGTGGCCAGCCACGAGCAGGCATCGATATATCCCTAT
GATGTGCCGGATTATGCTGGAGCACCAGGAAGTGCTGGTTCTGCTGCTGGTAGTGGAGCTTCTAACTTTACTCAG
TTCGTTCTCGTCGACAATGGCGGAACTGGCGACGTGACTGTCGCCCCAAGCAACTTCGCTAACGGGATCGCTGAA
TGGATCAGCTCTAACTCGCGTTCACAGGCTTACAAAGTAACCTGTAGCGTTCGTCAGAGCTCTGCGCAGAATCGC
AAATACACCATCAAAGTCGAGGTGCCTAAAGGCGCCTGGCGTTCGTACTTAAATATGGAACTAACCATTCCAATT
TTCGCCACGAATTCCGACTGCGAGCTTATTGTTAAGGCAATGCAAGGTCTCCTAAAAGATGGAAACCCGATTCCC
TCAGCAATCGCAGCAAACTCCGGCATCTACGGCGCCGATTACAAGGACGATGATGACAAGGGAGCACCAGGAAGT
GCTGGTTCTGCTGCTGGTAGTGGAGCGTGCCCGGTGCCGCTGCAGCTGCCGCCGCTGGAACGCCTGACCCTGGAT
GATAAAAAACCGCTGAATACCCTGATCTCTGCTACTGGTCTGTGGATGAGTCGTACCGGAACCATTCATAAAATC
AAACACCACGAGGTTAGCCGTTCGAAAATCTATATTGAGATGGCGTGTGGCGATCATCTGGTTGTGAACAATAGC
CGCTCTTCTCGTACAGCACGTGCACTGCGTCACCACAAATATCGTAAAACCTGTAAACGTTGCCGTGTGTCCGAT

GAGGATCTGAACAAATTCCTGACAAAAGCCAATGAGGACCAAACAAGCGTGAAAGTGAAAGTCGTTAGCGCTCCT
ACCCGTACTAAAAAAGCAATGCCGAAATCCGTTGCTCGTGCCCCTAAACCACTGGAAAACACTGAAGCAGCACAG
GCACAGCCGTCTGGAAGCAAATTCTCTCCGGCCATTCCTGTTTCTACCCAGGAGTCCGTTTCTGTTCCAGCAAGT
GTGAGCACCAGCATTAGCAGTATTAGCACCGGTGCCACCGCTAGCGCCCTGGTTAAAGGCAATACCAATCCGATT
ACAAGCATGTCTGCCCCGGTTCAAGCATCAGCTCCAGCACTGACAAAATCCCAAACCGATCGTCTGGAGGTTCTG
CTGAATCCGAAAGACGAAATCAGCCTGAATTCCGGCAAACCGTTTCGTGAACTGGAGAGCGAACTGCTGTCACGT
CGTAAAAAAGACCTGCAACAAATCTATGCCGAAGAACGTGAGAACTATCTGGGGAAACTGGAACGTGAAATCACC
CGCTTTTTCGTGGATCGTGGCTTTCTGGAGATCAAATCCCCGATTCTGATTCCTCTGGAGTATATCGAGCGTATG
GGCATCGACAATGATACCGAACTGAGCAAACAAATTTTCCGTGTGGATAAAAACTTCTGTCTGCGCCCTATGCTA
GCACCAAATCTGGCTAACTATCTGCGCAAACTGGACCGTGCCCTGCCTGATCCTATCAAAATCTTCGAGATCGGC
CCGTGTTATCGTAAAGAGTCCGACGGTAAAGAACATCTGGAGGAGTTTACCATGCTGAACTTTTGCCAAATGGGT
TCAGGTTGTACTCGTGAGAACCTGGAAAGCATCATCACCGATTTTCTGAACCACCTGGGCATTGACTTCAAAATT
GTGGGCGACAGCTGTATGGTGTTTGGCGACACCCTGGATGTCATGCACGGCGACCTGGAACTGTCTAGTGCCGTT
GTGGGCCCAATCCCGCTGGATCGTGAGTGGGGTATCGACAAACCTTGGATCGGTGCGGGTTTTGGTCTGGAGCGT
CTGCTGAAAGTAAAACACGACTTCAAGAACATCAAACGTGCTGCACGTTCCGAGTCCTATTACAATGGTATTTCT
ACTAACCTGATGGCGTCCACGGATTACAGTACCTATAGCCAAGCTGCAGCGCAGCAGGGCTACAGTGCTTACACC
GCCCAGCCCACTCAAGGATATGCACAGACCACCCAGGCATATGGGCAACAAAGCTATGGAACCTATGGACAGCCC
ACTGATGTCAGCTATACCCAGGCTCAGACCACTGCAACCTATGGGCAGACCGCCTATGCAACTTCTTATGGACAG
CCTCCCACTGGTTATACTACTCCAACTGCCCCCCAGGCATACAGCCAGCCTGTCCAGGGGTATGGCACTGGTGCT
TATGATACCACCACTGCTACAGTCACCACCACCCAGGCCTCCTATGCAGCTCAGTCTGCATATGGCACTCAGCCT
GCTTATCCAGCCTATGGGCAGCAGCCAGCAGCCACTGCACCTACAAGACCGCAGGATGGAAACAAGCCCACTGAG
ACTAGTCAACCTCAATCTAGCACAGGGGGTTACAACCAGCCCAGCCTAGGATATGGACAGAGTAACTACAGTTAT
CCCCAGGTACCTGGGAGCTACCCCATGCAGCCAGTCACTGCACCTCCATCCTACCCTCCTACCAGCTATTCCTCT
ACACAGCCGACTAGTTATGATCAGAGCAGTTACTCTCAGCAGAACACCTATGGGCAACCGAGCAGCTATGGACAG
CAGAGTAGCTATGGTCAACAAAGCAGCTATGGGCAGCAGCCTCCCACTAGTTACCCACCCCAAACTGGATCCTAC
AGCCAAGCTCCAAGTCAATATAGCCAACAGAGCAGCAGCTACGGGCAGCAGAGTTCATTCCGACAGGACCACCCC
AGTAGCATGGGTGTTTATGGGCAGGAGTCTGGAGGATTTTCCGGACCAGGAGAGAACCGGAGCATGAGTGGCCCT
GATAACCGGGGCAGGGGAAGAGGGGGATTTGATCGTGGAGGCATGAGCAGAGGTGGGCGGGGAGGAGGACGCGGT
GGAATGGGCAGCGCTGGAGAGCGAGGTGGCTTCAATAAGCCTGGTGGACCCATGGATGAAGGACCAGATCTTGAT
CTAGGCCCACCTGTAGATCCAGATGAAGACTCTGACAACAGTGCAATTTATGTACAAGGATTAAATGACAGTGTG
ACTCTAGATGATCTGGCAGACTTCTTTAAGCAGTGTGGGGTTGTTAAGATGAACAAGAGAACTGGGCAACCCATG
ATCCACATCTACCTGGACAAGGAAACAGGAAAGCCCAAAGGCGATGCCACAGTGTCCTATGAAGACCCACCTACT
GCCAAGGCTGCCGTGGAATGGTTTGATGGGAAAGATTTTCAAGGGAGCAAACTTAAAGTCTCCCTTGCTCGGAAG
AAGCCTCCAATGAACAGTATGCGGGGTGGTCTGCCACCCCGTGAGGGCAGAGGCATGCCACCACCACTCCGTGGA
GGTCCAGGAGGCCCAGGAGGTCCTGGGGGACCCATGGGTCGCATGGGAGGCCGTGGAGGAGATAGAGGAGGCTTC
CCTCCAAGAGGACCCCGGGGTTCCCGAGGGAACCCCTCTGGAGGAGGAAACGTCCAGCACCGAGCTGGAGACTGG
CAGTGTCCCAATCCGGGTTGTGGAAACCAGAACTTCGCCTGGAGAACAGAGTGCAACCAGTGTAAGGCCCCAAAG
CCTGAAGGCTTCCTCCCGCCACCCTTTCCGCCCCCGGGTGGTGATCGTGGCAGAGGTGGCCCTGGTGGCATGCGG
GGAGGAAGAGGTGGCCTCATGGATCGTGGTGGTCCCGGTGGAATGTTCAGAGGTGGCCGTGGTGGAGACAGAGGT
GGCTTCCGTGGTGGCCGGGGCATGGACCGAGGTGGCTTTGGTGGAGGAAGACGAGGTGGCCCTGGGGGGCCCCCT
GGACCTTTGATGGAACAGGATAAAAAACCGCTGAATACCCTGATCTCTGCTACTGGTCTGTGGATGAGTCGTACC
GGAACCATTCATAAAATCAAACACCACGAGGTTAGCCGTTCGAAAATCTATATTGAGATGGCGTGTGGCGATCAT
CTGGTTGTGAACAATAGCCGCTCTTCTCGTACAGCACGTGCACTGCGTCACCACAAATATCGTAAAACCTGTAAA
CGTTGCCGTGTGTCCGATGAGGATCTGAACAAATTCCTGACAAAAGCCAATGAGGACCAAACAAGCGTGAAAGTG
AAAGTCGTTAGCGCTCCTACCCGTACTAAAAAAGCAATGCCGAAATCCGTTGCTCGTGCCCCTAAACCACTGGAA
AACACTGAAGCAGCACAGGCACAGCCGTCTGGAAGCAAATTCTCTCCGGCCATTCCTGTTTCTACCCAGGAGTCC
GTTTCTGTTCCAGCAAGTGTGAGCACCAGCATTAGCAGTATTAGCACCGGTGCCACCGCTAGCGCCCTGGTTAAA
GGCAATACCAATCCGATTACAAGCATGTCTGCCCCGGTTCAAGCATCAGCTCCAGCACTGACAAAATCCCAAACC
GATCGTCTGGAGGTTCTGCTGAATCCGAAAGACGAAATCAGCCTGAATTCCGGCAAACCGTTTCGTGAACTGGAG
AGCGAACTGCTGTCACGTCGTAAAAAAGACCTGCAACAAATCTATGCCGAAGAACGTGAGAACTATCTGGGGAAA
CTGGAACGTGAAATCACCCGCTTTTTCGTGGATCGTGGCTTTCTGGAGATCAAATCCCCGATTCTGATTCCTCTG
GAGTATATCGAGCGTATGGGCATCGACAATGATACCGAACTGAGCAAACAAATTTTCCGTGTGGATAAAAACTTC
TGTCTGCGCCCTATGCTAGCACCAAATCTGGCTAACTATCTGCGCAAACTGGACCGTGCCCTGCCTGATCCTATC
AAAATCTTCGAGATCGGCCCGTGTTATCGTAAAGAGTCCGACGGTAAAGAACATCTGGAGGAGTTTACCATGCTG
AACTTTTGCCAAATGGGTTCAGGTTGTACTCGTGAGAACCTGGAAAGCATCATCACCGATTTTCTGAACCACCTG
GGCATTGACTTCAAAATTGTGGGCGACAGCTGTATGGTGTTTGGCGACACCCTGGATGTCATGCACGGCGACCTG
GAACTGTCTAGTGCCGTTGTGGGCCCAATCCCGCTGGATCGTGAGTGGGGTATCGACAAACCTTGGATCGGTGCG

GGTTTTGGTCTGGAGCGTCTGCTGAAAGTAAAACACGACTTCAAGAACATCAAACGTGCTGCACGTTCCGAGTCC
TATTACAATGGTATTTCTACTAACCTGTAA (SEQ ID NO: 507)

Protein:
MGCVCSSNPEGTELASNDYTQQATQSYGAYPTQPGQGYSQQSSQPYGQQSYSGYSQSTDTSGYGQSSYSSYGQSQ
NTGYGTQSTPQGYGSTGGYGSSQSSQSSYGQQSSYPGYGQQPAPSSTSGSYGSSSQSSSYGQPQSGSYSQQPSYG
GQQQSYGQQQSYNPPQGYGQQNQYNSSSGGGGGGGGGGNYGQDQSSMSSGGGSGGGYGNQDQSGGGGSGGYGQQD
RGGRGRGGSGGGGGGGGGGYNRSSGGYEPRGRGGGRGGRGGMGGSDRGGFNKFGGPRDQGSRHDSEQDNSDNNTI
FVQGLGENVTIESVADYFKQIGIIKTNKKTGQPMINLYTDRETGKLKGEATVSFDDPPSAKAAIDWFDGKEFSGN
PIKVSFATRRADFNRGGGNGRGGRGRGGPMGRGGYGGGGSGGGGRGGFPSGGGGGGGQQRAGDWKCPNPTCENMN
FSWRNECNQCKAPKPDGPGGGPGGSHMGGNYGDDRRGGRGGAIAARNAYLRKKIARLKKDNLQLERDEQNLEKII
ANLRDEIARLENEVASHEQASIYPYDVPDYAGAPGSAGSAAGSGASNFTQFVLVDNGGTGDVTVAPSNFANGIAE
WISSNSRSQAYKVTCSVRQSSAQNRKYTIKVEVPKGAWRSYLNMELTIPIFATNSDCELIVKAMQGLLKDGNPIP
SAIAANSGIYGADYKDDDDKGAPGSAGSAAGSGACPVPLQLPPLERLTLDDKKPLNTLISATGLWMSRTGTIHKI
KHHEVSRSKIYIEMACGDHLVVNNSRSSRTARALRHHKYRKTCKRCRVSDEDLNKFLTKANEDQTSVKVKVVSAP
TRTKKAMPKSVARAPKPLENTEAAQAQPSGSKFSPAIPVSTQESVSVPASVSTSISSISTGATASALVKGNTNPI
TSMSAPVQASAPALTKSQTDRLEVLLNPKDEISLNSGKPFRELESELLSRRKKDLQQIYAEERENYLGKLEREIT
RFFVDRGFLEIKSPILIPLEYIERMGIDNDTELSKQIFRVDKNFCLRPMLAPNLANYLRKLDRALPDPIKIFEIG
PCYRKESDGKEHLEEFTMLNFCQMGSGCTRENLESIITDFLNHLGIDFKIVGDSCMVFGDTLDVMHGDLELSSAV
VGPIPLDREWGIDKPWIGAGFGLERLLKVKHDFKNIKRAARSESYYNGISTNLMASTDYSTYSQAAAQQGYSAYT
AQPTQGYAQTTQAYGQQSYGTYGQPTDVSYTQAQTTATYGQTAYATSYGQPPTGYTTPTAPQAYSQPVQGYGTGA
YDTTTATVTTTQASYAAQSAYGTQPAYPAYGQQPAATAPTRPQDGNKPTETSQPQSSTGGYNQPSLGYGQSNYSY
PQVPGSYPMQPVTAPPSYPPTSYSSTQPTSYDQSSYSQQNTYGQPSSYGQQSSYGQQSSYGQQPPTSYPPQTGSY
SQAPSQYSQQSSSYGQQSSFRQDHPSSMGVYGQESGGFSGPGENRSMSGPDNRGRGRGGFDRGGMSRGGRGGGRG
GMGSAGERGGFNKPGGPMDEGPDLDLGPPVDPDEDSDNSAIYVQGLNDSVTLDDLADFFKQCGVVKMNKRTGQPM
IHIYLDKETGKPKGDATVSYEDPPTAKAAVEWFDGKDFQGSKLKVSLARKKPPMNSMRGGLPPREGRGMPPPLRG
GPGGPGGPGGPMGRMGGRGGDRGGFPPRGPRGSRGNPSGGGNVQHRAGDWQCPNPGCGNQNFAWRTECNQCKAPK
PEGFLPPPFPPPGGDRGRGGPGGMRGGRGGLMDRGGPGGMFRGGRGGDRGGFRGGRGMDRGGFGGGRRGGPGGPP
GPLMEQDKKPLNTLISATGLWMSRTGTIHKIKHHEVSRSKIYIEMACGDHLVVNNSRSSRTARALRHHKYRKTCK
RCRVSDEDLNKFLTKANEDQTSVKVKVVSAPTRTKKAMPKSVARAPKPLENTEAAQAQPSGSKFSPAIPVSTQES
VSVPASVSTSISSISTGATASALVKGNTNPITSMSAPVQASAPALTKSQTDRLEVLLNPKDEISLNSGKPFRELE
SELLSRRKKDLQQIYAEERENYLGKLEREITRFFVDRGFLEIKSPILIPLEYIERMGIDNDTELSKQIFRVDKNF
CLRPMLAPNLANYLRKLDRALPDPIKIFEIGPCYRKESDGKEHLEEFTMLNFCQMGSGCTRENLESIITDFLNHL
GIDFKIVGDSCMVFGDTLDVMHGDLELSSAVVGPIPLDREWGIDKPWIGAGFGLERLLKVKHDFKNIKRAARSES
YYNGISTNL* (SEQ ID NO: 508)

LCK::PylRS(AA)::EWSR1::PylRS(AA)
DNA:
ATGGGCTGCGTGTGCAGCAGCAACCCCGAGGGTACCGAGCTCGCGTGCCCGGTGCCGCTGCAGCTGCCGCCGCTG
GAACGCCTGACCCTGGATGATAAAAAACCGCTGAATACCCTGATCTCTGCTACTGGTCTGTGGATGAGTCGTACC
GGAACCATTCATAAAATCAAACACCACGAGGTTAGCCGTTCGAAAATCTATATTGAGATGGCGTGTGGCGATCAT
CTGGTTGTGAACAATAGCCGCTCTTCTCGTACAGCACGTGCACTGCGTCACCACAAATATCGTAAAACCTGTAAA
CGTTGCCGTGTGTCCGATGAGGATCTGAACAAATTCCTGACAAAAGCCAATGAGGACCAAACAAGCGTGAAAGTG
AAAGTCGTTAGCGCTCCTACCCGTACTAAAAAAGCAATGCCGAAATCCGTTGCTCGTGCCCCTAAACCACTGGAA
AACACTGAAGCAGCACAGGCACAGCCGTCTGGAAGCAAATTCTCTCCGGCCATTCCTGTTTCTACCCAGGAGTCC
GTTTCTGTTCCAGCAAGTGTGAGCACCAGCATTAGCAGTATTAGCACCGGTGCCACCGCTAGCGCCCTGGTTAAA
GGCAATACCAATCCGATTACAAGCATGTCTGCCCCGGTTCAAGCATCAGCTCCAGCACTGACAAAATCCCAAACC
GATCGTCTGGAGGTTCTGCTGAATCCGAAAGACGAAATCAGCCTGAATTCCGGCAAACCGTTTCGTGAACTGGAG
AGCGAACTGCTGTCACGTCGTAAAAAAGACCTGCAACAAATCTATGCCGAAGAACGTGAGAACTATCTGGGGAAA
CTGGAACGTGAAATCACCCGCTTTTTCGTGGATCGTGGCTTTCTGGAGATCAAATCCCCGATTCTGATTCCTCTG
GAGTATATCGAGCGTATGGGCATCGACAATGATACCGAACTGAGCAAACAAATTTTCCGTGTGGATAAAAACTTC
TGTCTGCGCCCTATGCTGGCACCAAATCTGTATAACTATCTGCGCAAACTGGACCGTGCCCTGCCTGATCCTATC
AAAATCTTCGAGATCGGCCCGTGTTATCGTAAAGAGTCCGACGGTAAAGAACATCTGGAGGAGTTTACCATGCTG
GCCTTTGCCCAAATGGGTTCAGGTTGTACTCGTGAGAACCTGGAAAGCATCATCACCGATTTTCTGAACCACCTG
GGCATTGACTTCAAAATTGTGGGCGACAGCTGTATGGTGTATGGCGACACCCTGGATGTCATGCACGGCGACCTG
GAACTGTCTAGTGCCGTTGTTGGACCAATTCCGCTGGACCGTGAGTGGGGTATCGACAAACCGTGGATCGGAGCA
GGATTCGGTCTGGAACGCCTGCTGAAAGTGAAACACGACTTCAAAAACATCAAACGTGCCGCCCGTTCTGAATCG
TATTATAACGGGATCTCTACGAACCTGATGGCGTCCACGGATTACAGTACCTATAGCCAAGCTGCAGCGCAGCAG

GGCTACAGTGCTTACACCGCCCAGCCCACTCAAGGATATGCACAGACCACCCAGGCATATGGGCAACAAAGCTAT
GGAACCTATGGACAGCCCACTGATGTCAGCTATACCCAGGCTCAGACCACTGCAACCTATGGGCAGACCGCCTAT
GCAACTTCTTATGGACAGCCTCCCACTGGTTATACTACTCCAACTGCCCCCCAGGCATACAGCCAGCCTGTCCAG
GGGTATGGCACTGGTGCTTATGATACCACCACTGCTACAGTCACCACCACCCAGGCCTCCTATGCAGCTCAGTCT
GCATATGGCACTCAGCCTGCTTATCCAGCCTATGGGCAGCAGCCAGCAGCCACTGCACCTACAAGACCGCAGGAT
GGAAACAAGCCCACTGAGACTAGTCAACCTCAATCTAGCACAGGGGGTTACAACCAGCCCAGCCTAGGATATGGA
CAGAGTAACTACAGTTATCCCCAGGTACCTGGGAGCTACCCCATGCAGCCAGTCACTGCACCTCCATCCTACCCT
CCTACCAGCTATTCCTCTACACAGCCGACTAGTTATGATCAGAGCAGTTACTCTCAGCAGAACACCTATGGGCAA
CCGAGCAGCTATGGACAGCAGAGTAGCTATGGTCAACAAAGCAGCTATGGGCAGCAGCCTCCCACTAGTTACCCA
CCCCAAACTGGATCCTACAGCCAAGCTCCAAGTCAATATAGCCAACAGAGCAGCAGCTACGGGCAGCAGAGTTCA
TTCCGACAGGACCACCCCAGTAGCATGGGTGTTTATGGGCAGGAGTCTGGAGGATTTTCCGGACCAGGAGAGAAC
CGGAGCATGAGTGGCCCTGATAACCGGGGCAGGGGAAGAGGGGGATTTGATCGTGGAGGCATGAGCAGAGGTGGG
CGGGGAGGAGGACGCGGTGGAATGGGCAGCGCTGGAGAGCGAGGTGGCTTCAATAAGCCTGGTGGACCCATGGAT
GAAGGACCAGATCTTGATCTAGGCCCACCTGTAGATCCAGATGAAGACTCTGACAACAGTGCAATTTATGTACAA
GGATTAAATGACAGTGTGACTCTAGATGATCTGGCAGACTTCTTTAAGCAGTGTGGGGTTGTTAAGATGAACAAG
AGAACTGGGCAACCCATGATCCACATCTACCTGGACAAGGAAACAGGAAAGCCCAAAGGCGATGCCACAGTGTCC
TATGAAGACCCACCTACTGCCAAGGCTGCCGTGGAATGGTTTGATGGGAAAGATTTTCAAGGGAGCAAACTTAAA
GTCTCCCTTGCTCGGAAGAAGCCTCCAATGAACAGTATGCGGGGTGGTCTGCCACCCCGTGAGGGCAGAGGCATG
CCACCACCACTCCGTGGAGGTCCAGGAGGCCCAGGAGGTCCTGGGGGACCCATGGGTCGCATGGGAGGCCGTGGA
GGAGATAGAGGAGGCTTCCCTCCAAGAGGACCCCGGGGTTCCCGAGGGAACCCCTCTGGAGGAGGAAACGTCCAG
CACCGAGCTGGAGACTGGCAGTGTCCCAATCCGGGTTGTGGAAACCAGAACTTCGCCTGGAGAACAGAGTGCAAC
CAGTGTAAGGCCCCAAAGCCTGAAGGCTTCCTCCCGCCACCCTTTCCGCCCCCGGGTGGTGATCGTGGCAGAGGT
GGCCCTGGTGGCATGCGGGGAGGAAGAGGTGGCCTCATGGATCGTGGTGGTCCCGGTGGAATGTTCAGAGGTGGC
CGTGGTGGAGACAGAGGTGGCTTCCGTGGTGGCCGGGGCATGGACCGAGGTGGCTTTGGTGGAGGAAGACGAGGT
GGCCCTGGGGGGCCCCCTGGACCTTTGATGGAACAGGATAAAAAACCGCTGAATACCCTGATCTCTGCTACTGGT
CTGTGGATGAGTCGTACCGGAACCATTCATAAAATCAAACACCACGAGGTTAGCCGTTCGAAAATCTATATTGAG
ATGGCGTGTGGCGATCATCTGGTTGTGAACAATAGCCGCTCTTCTCGTACAGCACGTGCACTGCGTCACCACAAA
TATCGTAAAACCTGTAAACGTTGCCGTGTGTCCGATGAGGATCTGAACAAATTCCTGACAAAAGCCAATGAGGAC
CAAACAAGCGTGAAAGTGAAAGTCGTTAGCGCTCCTACCCGTACTAAAAAAGCAATGCCGAAATCCGTTGCTCGT
GCCCCTAAACCACTGGAAAACACTGAAGCAGCACAGGCACAGCCGTCTGGAAGCAAATTCTCTCCGGCCATTCCT
GTTTCTACCCAGGAGTCCGTTTCTGTTCCAGCAAGTGTGAGCACCAGCATTAGCAGTATTAGCACCGGTGCCACC
GCTAGCGCCCTGGTTAAAGGCAATACCAATCCGATTACAAGCATGTCTGCCCCGGTTCAAGCATCAGCTCCAGCA
CTGACAAAATCCCAAACCGATCGTCTGGAGGTTCTGCTGAATCCGAAAGACGAAATCAGCCTGAATTCCGGCAAA
CCGTTTCGTGAACTGGAGAGCGAACTGCTGTCACGTCGTAAAAAAGACCTGCAACAAATCTATGCCGAAGAACGT
GAGAACTATCTGGGGAAACTGGAACGTGAAATCACCCGCTTTTTCGTGGATCGTGGCTTTCTGGAGATCAAATCC
CCGATTCTGATTCCTCTGGAGTATATCGAGCGTATGGGCATCGACAATGATACCGAACTGAGCAAACAAATTTTC
CGTGTGGATAAAAACTTCTGTCTGCGCCCTATGCTGGCACCAAATCTGTATAACTATCTGCGCAAACTGGACCGT
GCCCTGCCTGATCCTATCAAAATCTTCGAGATCGGCCCGTGTTATCGTAAAGAGTCCGACGGTAAAGAACATCTG
GAGGAGTTTACCATGCTGGCCTTTGCCCAAATGGGTTCAGGTTGTACTCGTGAGAACCTGGAAAGCATCATCACC
GATTTTCTGAACCACCTGGGCATTGACTTCAAAATTGTGGGCGACAGCTGTATGGTGTATGGCGACACCCTGGAT
GTCATGCACGGCGACCTGGAACTGTCTAGTGCCGTTGTTGGACCAATTCCGCTGGACCGTGAGTGGGGTATCGAC
AAACCGTGGATCGGAGCAGGATTCGGTCTGGAACGCCTGCTGAAAGTGAAACACGACTTCAAAAACATCAAACGT
GCCGCCCGTTCTGAATCGTATTATAACGGGATCTCTACGAACCTGTAA (SEQ ID NO: 509)

Protein:
MGCVCSSNPEGTELACPVPLQLPPLERLTLDDKKPLNTLISATGLWMSRTGTIHKIKHHEVSRSKIYIEMACGDH
LVVNNSRSSRTARALRHHKYRKTCKRCRVSDEDLNKFLTKANEDQTSVKVKVVSAPTRTKKAMPKSVARAPKPLE
NTEAAQAQPSGSKFSPAIPVSTQESVSVPASVSTSISSISTGATASALVKGNTNPITSMSAPVQASAPALTKSQT
DRLEVLLNPKDEISLNSGKPFRELESELLSRRKKDLQQIYAEERENYLGKLEREITRFFVDRGFLEIKSPILIPL
EYIERMGIDNDTELSKQIFRVDKNFCLRPMLAPNLYNYLRKLDRALPDPIKIFEIGPCYRKESDGKEHLEEFTML
AFAQMGSGCTRENLESIITDFLNHLGIDFKIVGDSCMVYGDTLDVMHGDLELSSAVVGPIPLDREWGIDKPWIGA
GFGLERLLKVKHDFKNIKRAARSESYYNGISTNLMASTDYSTYSQAAAQQGYSAYTAQPTQGYAQTTQAYGQQSY
GTYGQPTDVSYTQAQTTATYGQTAYATSYGQPPTGYTTPTAPQAYSQPVQGYGTGAYDTTTATVTTTQASYAAQS
AYGTQPAYPAYGQQPAATAPTRPQDGNKPTETSQPQSSTGGYNQPSLGYGQSNYSYPQVPGSYPMQPVTAPPSYP
PTSYSSTQPTSYDQSSYSQQNTYGQPSSYGQQSSYGQQSSYGQQPPTSYPPQTGSYSQAPSQYSQQSSSYGQQSS
FRQDHPSSMGVYGQESGGFSGPGENRSMSGPDNRGRGRGGFDRGGMSRGGRGGGRGGMGSAGERGGFNKPGGPMD
EGPDLDLGPPVDPDEDSDNSAIYVQGLNDSVTLDDLADFFKQCGVVKMNKRTGQPMIHIYLDKETGKPKGDATVS
YEDPPTAKAAVEWFDGKDFQGSKLKVSLARKKPPMNSMRGGLPPREGRGMPPPLRGGPGGPGGPGGPMGRMGGRG

GDRGGFPPRGPRGSRGNPSGGGNVQHRAGDWQCPNPGCGNQNFAWRTECNQCKAPKPEGFLPPPFPPPGGDRGRG
GPGGMRGGRGGLMDRGGPGGMFRGGRGGDRGGFRGGRGMDRGGFGGGRRGGPGGPPGPLMEQDKKPLNTLISATG
LWMSRTGTIHKIKHHEVSRSKIYIEMACGDHLVVNNSRSSRTARALRHHKYRKTCKRCRVSDEDLNKFLTKANED
QTSVKVKVVSAPTRTKKAMPKSVARAPKPLENTEAAQAQPSGSKFSPAIPVSTQESVSVPASVSTSISSISTGAT
ASALVKGNTNPITSMSAPVQASAPALTKSQTDRLEVLLNPKDEISLNSGKPFRELESELLSRRKKDLQQIYAEER
ENYLGKLEREITRFFVDRGFLEIKSPILIPLEYIERMGIDNDTELSKQIFRVDKNFCLRPMLAPNLYNYLRKLDR
ALPDPIKIFEIGPCYRKESDGKEHLEEFTMLAFAQMGSGCTRENLESIITDFLNHLGIDFKIVGDSCMVYGDTLD
VMHGDLELSSAVVGPIPLDREWGIDKPWIGAGFGLERLLKVKHDFKNIKRAARSESYYNGISTNL* (SEQ ID
NO: 510)

LCK::FUS::PylRS(AA)::EWSR1::PylRS(AA)
DNA:
ATGGGCTGCGTGTGCAGCAGCAACCCCGAGGGTACCGAGCTCGCCTCAAACGATTATACCCAACAAGCAACCCAA
AGCTATGGGGCCTACCCCACCCAGCCCGGGCAGGGCTATTCCCAGCAGAGCAGTCAGCCCTACGGACAGCAGAGT
TACAGTGGTTATAGCCAGTCCACGGACACTTCAGGATATGGCCAGAGCAGCTATTCTTCTTATGGCCAGAGCCAG
AACACAGGCTATGGAACTCAGTCAACTCCCCAGGGATATGGCTCGACTGGCGGCTATGGCAGTAGCCAGAGCTCC
CAATCGTCTTACGGGCAGCAGTCCTCCTACCCTGGCTATGGCCAGCAGCCAGCTCCCAGCAGCACCTCGGGAAGT
TACGGTAGCAGTTCTCAGAGCAGCAGCTATGGGCAGCCCCAGAGTGGGAGCTACAGCCAGCAGCCTAGCTATGGT
GGACAGCAGCAAAGCTATGGACAGCAGCAAAGCTATAATCCCCCTCAGGGCTATGGACAGCAGAACCAGTACAAC
AGCAGCAGTGGTGGTGGAGGTGGAGGTGGAGGTGGAGGTAACTATGGCCAAGATCAATCCTCCATGAGTAGTGGT
GGTGGCAGTGGTGGCGGTTATGGCAATCAAGACCAGAGTGGTGGAGGTGGCAGCGGTGGCTATGGACAGCAGGAC
CGTGGAGGCCGCGGCAGGGGTGGCAGTGGTGGCGGCGGCGGCGGCGGTGGTGGTTACAACCGCAGCAGTGGT
GGCTATGAACCCAGAGGTCGTGGAGGTGGCCGTGGAGGCAGAGGTGGCATGGGCGGAAGTGACCGTGGTGGCTTC
AATAAATTTGGTGGCCCTCGGGACCAAGGATCACGTCATGACTCCGAACAGGATAATTCAGACAACAACACCATC
TTTGTGCAAGGCCTGGGTGAGAATGTTACAATTGAGTCTGTGGCTGATTACTTCAAGCAGATTGGTATTATTAAG
ACAAACAAGAAAACGGGACAGCCCATGATTAATTTGTACACAGACAGGGAAACTGGCAAGCTGAAGGGAGAGGCA
ACGGTCTCTTTTGATGACCCACCTTCAGCTAAAGCAGCTATTGACTGGTTTGATGGTAAAGAATTCTCCGGAAAT
CCTATCAAGGTCTCATTTGCTACTCGCCGGGCAGACTTTAATCGGGGTGGTGGCAATGGTCGTGGAGGCCGAGGG
CGAGGAGGACCCATGGGCCGTGGAGGCTATGGAGGTGGTGGCAGTGGTGGTGGTGGCCGAGGAGGATTTCCCAGT
GGAGGTGGTGGCGGTGGAGGACAGCAGCGAGCTGGTGACTGGAAGTGTCCTAATCCCACCTGTGAGAATATGAAC
TTCTCTTGGAGGAATGAATGCAACCAGTGTAAGGCCCCTAAACCAGATGGCCCAGGAGGGGGACCAGGTGGCTCT
CACATGGGGGGTAACTACGGGGATGATCGTCGTGGTGGCAGAGGAGGCGGCGCCCCCGGCTCCGCCGGCTCCGCC
GCCGGCTCCGGCATGGCGTGCCCGGTGCCGCTGCAGCTGCCGCCGCTGGAACGCCTGACCCTGGATGATAAAAAA
CCGCTGAATACCCTGATCTCTGCTACTGGTCTGTGGATGAGTCGTACCGGAACCATTCATAAAATCAAACACCAC
GAGGTTAGCCGTTCGAAAATCTATATTGAGATGGCGTGTGGCGATCATCTGGTTGTGAACAATAGCCGCTCTTCT
CGTACAGCACGTGCACTGCGTCACCACAAATATCGTAAAACCTGTAAACGTTGCCGTGTGTCCGATGAGGATCTG
AACAAATTCCTGACAAAAGCCAATGAGGACCAAACAAGCGTGAAAGTGAAAGTCGTTAGCGCTCCTACCCGTACT
AAAAAAGCAATGCCGAAATCCGTTGCTCGTGCCCCTAAACCACTGGAAAACACTGAAGCAGCACAGGCACAGCCG
TCTGGAAGCAAATTCTCTCCGGCCATTCCTGTTTCTACCCAGGAGTCCGTTTCTGTTCCAGCAAGTGTGAGCACC
AGCATTAGCAGTATTAGCACCGGTGCCACCGCTAGCGCCCTGGTTAAAGGCAATACCAATCCGATTACAAGCATG
TCTGCCCCGGTTCAAGCATCAGCTCCAGCACTGACAAAATCCCAAACCGATCGTCTGGAGGTTCTGCTGAATCCG
AAAGACGAAATCAGCCTGAATTCCGGCAAACCGTTTCGTGAACTGGAGAGCGAACTGCTGTCACGTCGTAAAAAA
GACCTGCAACAAATCTATGCCGAAGAACGTGAGAACTATCTGGGGAAACTGGAACGTGAAATCACCCGCTTTTTC
GTGGATCGTGGCTTTCTGGAGATCAAATCCCCGATTCTGATTCCTCTGGAGTATATCGAGCGTATGGGCATCGAC
AATGATACCGAACTGAGCAAACAAATTTTCCGTGTGGATAAAAACTTCTGTCTGCGCCCTATGCTGGCACCAAAT
CTGTATAACTATCTGCGCAAACTGGACCGTGCCCTGCCTGATCCTATCAAAATCTTCGAGATCGGCCCGTGTTAT
CGTAAAGAGTCCGACGGTAAAGAACATCTGGAGGAGTTTACCATGCTGGCCTTTGCCCAAATGGGTTCAGGTTGT
ACTCGTGAGAACCTGGAAAGCATCATCACCGATTTTCTGAACCACCTGGGCATTGACTTCAAAATTGTGGGCGAC
AGCTGTATGGTGTATGGCGACACCCTGGATGTCATGCACGGCGACCTGGAACTGTCTAGTGCCGTTGTTGGACCA
ATTCCGCTGGACCGTGAGTGGGGTATCGACAAACCGTGGATCGGAGCAGGATTCGGTCTGGAACGCCTGCTGAAA
GTGAAACACGACTTCAAAAACATCAAACGTGCCGCCCGTTCTGAATCGTATTATAACGGGATCTCTACGAACCTG
ATGGCGTCCACGGATTACAGTACCTATAGCCAAGCTGCAGCGCAGCAGGGCTACAGTGCTTACACCGCCCAGCCC
ACTCAAGGATATGCACAGACCACCCAGGCATATGGGCAACAAAGCTATGGAACCTATGGACAGCCCACTGATGTC
AGCTATACCCAGGCTCAGACCACTGCAACCTATGGGCAGACCGCCTATGCAACTTCTTATGGACAGCCTCCCACT
GGTTATACTACTCCAACTGCCCCCCAGGCATACAGCCAGCCTGTCCAGGGGTATGGCACTGGTGCTTATGATACC
ACCACTGCTACAGTCACCACCACCCAGGCCTCCTATGCAGCTCAGTCTGCATATGGCACTCAGCCTGCTTATCCA
GCCTATGGGCAGCAGCCAGCAGCCACTGCACCTACAAGACCGCAGGATGGAAACAAGCCCACTGAGACTAGTCAA
CCTCAATCTAGCACAGGGGGTTACAACCAGCCCAGCCTAGGATATGGACAGAGTAACTACAGTTATCCCCAGGTA

CCTGGGAGCTACCCCATGCAGCCAGTCACTGCACCTCCATCCTACCCTCCTACCAGCTATTCCTCTACACAGCCG
ACTAGTTATGATCAGAGCAGTTACTCTCAGCAGAACACCTATGGGCAACCGAGCAGCTATGGACAGCAGAGTAGC
TATGGTCAACAAAGCAGCTATGGGCAGCAGCCTCCCACTAGTTACCCACCCCAAACTGGATCCTACAGCCAAGCT
CCAAGTCAATATAGCCAACAGAGCAGCAGCTACGGGCAGCAGAGTTCATTCCGACAGGACCACCCCAGTAGCATG
GGTGTTTATGGGCAGGAGTCTGGAGGATTTTCCGGACCAGGAGAGAACCGGAGCATGAGTGGCCCTGATAACCGG
GGCAGGGGAAGAGGGGGATTTGATCGTGGAGGCATGAGCAGAGGTGGGCGGGGAGGAGGACGCGGTGGAATGGGC
AGCGCTGGAGAGCGAGGTGGCTTCAATAAGCCTGGTGGACCCATGGATGAAGGACCAGATCTTGATCTAGGCCCA
CCTGTAGATCCAGATGAAGACTCTGACAACAGTGCAATTTATGTACAAGGATTAAATGACAGTGTGACTCTAGAT
GATCTGGCAGACTTCTTTAAGCAGTGTGGGGTTGTTAAGATGAACAAGAGAACTGGGCAACCCATGATCCACATC
TACCTGGACAAGGAAACAGGAAAGCCCAAAGGCGATGCCACAGTGTCCTATGAAGACCCACCTACTGCCAAGGCT
GCCGTGGAATGGTTTGATGGGAAAGATTTTCAAGGGAGCAAACTTAAAGTCTCCCTTGCTCGGAAGAAGCCTCCA
ATGAACAGTATGCGGGGTGGTCTGCCACCCCGTGAGGGCAGAGGCATGCCACCACCACTCCGTGGAGGTCCAGGA
GGCCCAGGAGGTCCTGGGGGACCCATGGGTCGCATGGGAGGCCGTGGAGGAGATAGAGGAGGCTTCCCTCCAAGA
GGACCCCGGGGTTCCCGAGGGAACCCCTCTGGAGGAGGAAACGTCCAGCACCGAGCTGGAGACTGGCAGTGTCCC
AATCCGGGTTGTGGAAACCAGAACTTCGCCTGGAGAACAGAGTGCAACCAGTGTAAGGCCCCAAAGCCTGAAGGC
TTCCTCCCGCCACCCTTTCCGCCCCCGGGTGGTGATCGTGGCAGAGGTGGCCCTGGTGGCATGCGGGGAGGAAGA
GGTGGCCTCATGGATCGTGGTGGTCCCGGTGGAATGTTCAGAGGTGGCCGTGGTGGAGACAGAGGTGGCTTCCGT
GGTGGCCGGGGCATGGACCGAGGTGGCTTTGGTGGAGGAAGACGAGGTGGCCCTGGGGGGCCCCCTGGACCTTTG
ATGGAACAGGATAAAAAACCGCTGAATACCCTGATCTCTGCTACTGGTCTGTGGATGAGTCGTACCGGAACCATT
CATAAAATCAAACACCACGAGGTTAGCCGTTCGAAAATCTATATTGAGATGGCGTGTGGCGATCATCTGGTTGTG
AACAATAGCCGCTCTTCTCGTACAGCACGTGCACTGCGTCACCACAAATATCGTAAAACCTGTAAACGTTGCCGT
GTGTCCGATGAGGATCTGAACAAATTCCTGACAAAAGCCAATGAGGACCAAACAAGCGTGAAAGTGAAAGTCGTT
AGCGCTCCTACCCGTACTAAAAAAGCAATGCCGAAATCCGTTGCTCGTGCCCCTAAACCACTGGAAAACACTGAA
GCAGCACAGGCACAGCCGTCTGGAAGCAAATTCTCTCCGGCCATTCCTGTTTCTACCCAGGAGTCCGTTTCTGTT
CCAGCAAGTGTGAGCACCAGCATTAGCAGTATTAGCACCGGTGCCACCGCTAGCGCCCTGGTTAAAGGCAATACC
AATCCGATTACAAGCATGTCTGCCCCGGTTCAAGCATCAGCTCCAGCACTGACAAAATCCCAAACCGATCGTCTG
GAGGTTCTGCTGAATCCGAAAGACGAAATCAGCCTGAATTCCGGCAAACCGTTTCGTGAACTGGAGAGCGAACTG
CTGTCACGTCGTAAAAAAGACCTGCAACAAATCTATGCCGAAGAACGTGAGAACTATCTGGGGAAACTGGAACGT
GAAATCACCCGCTTTTTCGTGGATCGTGGCTTTCTGGAGATCAAATCCCCGATTCTGATTCCTCTGGAGTATATC
GAGCGTATGGGCATCGACAATGATACCGAACTGAGCAAACAAATTTTCCGTGTGGATAAAAACTTCTGTCTGCGC
CCTATGCTGGCACCAAATCTGTATAACTATCTGCGCAAACTGGACCGTGCCCTGCCTGATCCTATCAAAATCTTC
GAGATCGGCCCGTGTTATCGTAAAGAGTCCGACGGTAAAGAACATCTGGAGGAGTTTACCATGCTGGCCTTTGCC
CAAATGGGTTCAGGTTGTACTCGTGAGAACCTGGAAAGCATCATCACCGATTTTCTGAACCACCTGGGCATTGAC
TTCAAAATTGTGGGCGACAGCTGTATGGTGTATGGCGACACCCTGGATGTCATGCACGGCGACCTGGAACTGTCT
AGTGCCGTTGTTGGACCAATTCCGCTGGACCGTGAGTGGGGTATCGACAAACCGTGGATCGGAGCAGGATTCGGT
CTGGAACGCCTGCTGAAAGTGAAACACGACTTCAAAAACATCAAACGTGCCGCCCGTTCTGAATCGTATTATAAC
GGGATCTCTACGAACCTGTAA (SEQ ID NO: 511)

Protein:
MGCVCSSNPEGTELASNDYTQQATQSYGAYPTQPGQGYSQQSSQPYGQQSYSGYSQSTDTSGYGQSSYSSYGQSQ
NTGYGTQSTPQGYGSTGGYGSSQSSQSSYGQQSSYPGYGQQPAPSSTSGSYGSSSQSSSYGQPQSGSYSQQPSYG
GQQQSYGQQQSYNPPQGYGQQNQYNSSSGGGGGGGGGGNYGQDQSSMSSGGGSGGGYGNQDQSGGGGSGGYGQQD
RGGRGRGGSGGGGGGGGGGYNRSSGGYEPRGRGGGRGGRGGMGGSDRGGFNKFGGPRDQGSRHDSEQDNSDNNTI
FVQGLGENVTIESVADYFKQIGIIKTNKKTGQPMINLYTDRETGKLKGEATVSFDDPPSAKAAIDWFDGKEFSGN
PIKVSFATRRADFNRGGGNGRGGRGRGGPMGRGGYGGGGSGGGGRGGFPSGGGGGGGQQRAGDWKCPNPTCENMN
FSWRNECNQCKAPKPDGPGGGPGGSHMGGNYGDDRRGGRGGGAPGSAGSAAGSGMACPVPLQLPPLERLTLDDKK
PLNTLISATGLWMSRTGTIHKIKHHEVSRSKIYIEMACGDHLVVNNSRSSRTARALRHHKYRKTCKRCRVSDEDL
NKFLTKANEDQTSVKVKVVSAPTRTKKAMPKSVARAPKPLENTEAAQAQPSGSKFSPAIPVSTQESVSVPASVST
SISSISTGATASALVKGNTNPITSMSAPVQASAPALTKSQTDRLEVLLNPKDEISLNSGKPFRELESELLSRRKK
DLQQIYAEERENYLGKLEREITRFFVDRGFLEIKSPILIPLEYIERMGIDNDTELSKQIFRVDKNFCLRPMLAPN
LYNYLRKLDRALPDPIKIFEIGPCYRKESDGKEHLEEFTMLAFAQMGSGCTRENLESIITDFLNHLGIDFKIVGD
SCMVYGDTLDVMHGDLELSSAVVGPIPLDREWGIDKPWIGAGFGLERLLKVKHDFKNIKRAARSESYYNGISTNL
MASTDYSTYSQAAAQQGYSAYTAQPTQGYAQTTQAYGQQSYGTYGQPTDVSYTQAQTTATYGQTAYATSYGQPPT
GYTTPTAPQAYSQPVQGYGTGAYDTTTATVTTTQASYAAQSAYGTQPAYPAYGQQPAATAPTRPQDGNKPTETSQ
PQSSTGGYNQPSLGYGQSNYSYPQVPGSYPMQPVTAPPSYPPTSYSSTQPTSYDQSSYSQQNTYGQPSSYGQQSS
YGQQSSYGQQPPTSYPPQTGSYSQAPSQYSQQSSSYGQQSSFRQDHPSSMGVYGQESGGFSGPGENRSMSGPDNR
GRGRGGFDRGGMSRGGRGGGRGGMGSAGERGGFNKPGGPMDEGPDLDLGPPVDPDEDSDNSAIYVQGLNDSVTLD
DLADFFKQCGVVKMNKRTGQPMIHIYLDKETGKPKGDATVSYEDPPTAKAAVEWFDGKDFQGSKLKVSLARKKPP

MNSMRGGLPPREGRGMPPPLRGGPGGPGGPGGPMGRMGGRGGDRGGFPPRGPRGSRGNPSGGGNVQHRAGDWQCP
NPGCGNQNFAWRTECNQCKAPKPEGFLPPPFPPPGGDRGRGGPGGMRGGRGGLMDRGGPGGMFRGGRGGDRGGFR
GGRGMDRGGFGGGRRGGPGGPPGPLMEQDKKPLNTLISATGLWMSRTGTIHKIKHHEVSRSKIYIEMACGDHLVV
NNSRSSRTARALRHHKYRKTCKRCRVSDEDLNKFLTKANEDQTSVKVKVVSAPTRTKKAMPKSVARAPKPLENTE
AAQAQPSGSKFSPAIPVSTQESVSVPASVSTSISSISTGATASALVKGNTNPITSMSAPVQASAPALTKSQTDRL
EVLLNPKDEISLNSGKPFRELESELLSRRKKDLQQIYAEERENYLGKLEREITRFFVDRGFLEIKSPILIPLEYI
ERMGIDNDTELSKQIFRVDKNFCLRPMLAPNLYNYLRKLDRALPDPIKIFEIGPCYRKESDGKEHLEEFTMLAFA
QMGSGCTRENLESIITDFLNHLGIDFKIVGDSCMVYGDTLDVMHGDLELSSAVVGPIPLDREWGIDKPWIGAGFG
LERLLKVKHDFKNIKRAARSESYYNGISTNL* (SEQ ID NO: 512)

TOM20::FUS::PylRS(AA)::EWSR1::PylRS(AA)
DNA:
ATGGTGGGTCGGAACAGCGCCATCGCCGCCGGTGTATGCGGGGCCCTTTTCATTGGGTACTGCATCTACTTCGAC
CGCAAAAGACGAAGTGACCCCAACTTCAAGAACAGGCTTCGAGAACGAAGAAAGAAACAGAAGCTTGCCAAGGAG
AGAGCTGGGCTTTCCAAGTTACCTGACCTTAAAGATGCTGAAGCTGTTCAGAAATTCTTCATGGCCTCAAACGAT
TATACCCAACAAGCAACCCAAAGCTATGGGGCCTACCCCACCCAGCCCGGGCAGGGCTATTCCCAGCAGAGCAGT
CAGCCCTACGGACAGCAGAGTTACAGTGGTTATAGCCAGTCCACGGACACTTCAGGATATGGCCAGAGCAGCTAT
TCTTCTTATGGCCAGAGCCAGAACACAGGCTATGGAACTCAGTCAACTCCCCAGGGATATGGCTCGACTGGCGGC
TATGGCAGTAGCCAGAGCTCCCAATCGTCTTACGGGCAGCAGTCCTCCTACCCTGGCTATGGCCAGCAGCCAGCT
CCCAGCAGCACCTCGGGAAGTTACGGTAGCAGTTCTCAGAGCAGCAGCTATGGGCAGCCCCAGAGTGGGAGCTAC
AGCCAGCAGCCTAGCTATGGTGGACAGCAGCAAAGCTATGGACAGCAGCAAAGCTATAATCCCCCTCAGGGCTAT
GGACAGCAGAACCAGTACAACAGCAGCAGTGGTGGTGGAGGTGGAGGTGGAGGTGGAGGTAACTATGGCCAAGAT
CAATCCTCCATGAGTAGTGGTGGTGGCAGTGGTGGCGGTTATGGCAATCAAGACCAGAGTGGTGGAGGTGGCAGC
GGTGGCTATGGACAGCAGGACCGTGGAGGCCGCGGCAGGGGTGGCAGTGGTGGCGGCGGCGGCGGCGGCGGTGGT
GGTTACAACCGCAGCAGTGGTGGCTATGAACCCAGAGGTCGTGGAGGTGGCCGTGGAGGCAGAGGTGGCATGGGC
GGAAGTGACCGTGGTGGCTTCAATAAATTTGGTGGCCCTCGGGACCAAGGATCACGTCATGACTCCGAACAGGAT
AATTCAGACAACAACACCATCTTTGTGCAAGGCCTGGGTGAGAATGTTACAATTGAGTCTGTGGCTGATTACTTC
AAGCAGATTGGTATTATTAAGACAAACAAGAAAACGGGACAGCCCATGATTAATTTGTACACAGACAGGGAAACT
GGCAAGCTGAAGGGAGAGGCAACGGTCTCTTTTGATGACCCACCTTCAGCTAAAGCAGCTATTGACTGGTTTGAT
GGTAAAGAATTCTCCGGAAATCCTATCAAGGTCTCATTTGCTACTCGCCGGGCAGACTTTAATCGGGGTGGTGGC
AATGGTCGTGGAGGCCGAGGGCGAGGAGGACCCATGGGCCGTGGAGGCTATGGAGGTGGTGGCAGTGGTGGTGGT
GGCCGAGGAGGATTTCCCAGTGGAGGTGGTGGCGGTGGAGGACAGCAGCGAGCTGGTGACTGGAAGTGTCCTAAT
CCCACCTGTGAGAATATGAACTTCTCTTGGAGGAATGAATGCAACCAGTGTAAGGCCCCTAAACCAGATGGCCCA
GGAGGGGGACCAGGTGGCTCTCACATGGGGGGTAACTACGGGGATGATCGTCGTGGTGGCAGAGGAGGCGCGATC
GCAGGCAAGCCTATTCCCAACCCCCTGCTGGGCCTGGATAGCACCGGAGCACCAGGAAGTGCTGGTTCTGCTGCT
GGTAGTGGAGCGTGCCCGGTGCCGCTGCAGCTGCCGCCGCTGGAACGCCTGACCCTGGATGATAAAAAACCGCTG
AATACCCTGATCTCTGCTACTGGTCTGTGGATGAGTCGTACCGGAACCATTCATAAAATCAAACACCACGAGGTT
AGCCGTTCGAAAATCTATATTGAGATGGCGTGTGGCGATCATCTGGTTGTGAACAATAGCCGCTCTTCTCGTACA
GCACGTGCACTGCGTCACCACAAATATCGTAAAACCTGTAAACGTTGCCGTGTGTCCGATGAGGATCTGAACAAA
TTCCTGACAAAAGCCAATGAGGACCAAACAAGCGTGAAAGTGAAAGTCGTTAGCGCTCCTACCCGTACTAAAAAA
GCAATGCCGAAATCCGTTGCTCGTGCCCCTAAACCACTGGAAAACACTGAAGCAGCACAGGCACAGCCGTCTGGA
AGCAAATTCTCTCCGGCCATTCCTGTTTCTACCCAGGAGTCCGTTTCTGTTCCAGCAAGTGTGAGCACCAGCATT
AGCAGTATTAGCACCGGTGCCACCGCTAGCGCCCTGGTTAAAGGCAATACCAATCCGATTACAAGCATGTCTGCC
CCGGTTCAAGCATCAGCTCCAGCACTGACAAAATCCCAAACCGATCGTCTGGAGGTTCTGCTGAATCCGAAAGAC
GAAATCAGCCTGAATTCCGGCAAACCGTTTCGTGAACTGGAGAGCGAACTGCTGTCACGTCGTAAAAAAGACCTG
CAACAAATCTATGCCGAAGAACGTGAGAACTATCTGGGGAAACTGGAACGTGAAATCACCCGCTTTTTCGTGGAT
CGTGGCTTTCTGGAGATCAAATCCCCGATTCTGATTCCTCTGGAGTATATCGAGCGTATGGGCATCGACAATGAT
ACCGAACTGAGCAAACAAATTTTCCGTGTGGATAAAAACTTCTGTCTGCGCCCTATGCTGGCACCAAATCTGTAT
AACTATCTGCGCAAACTGGACCGTGCCCTGCCTGATCCTATCAAAATCTTCGAGATCGGCCCGTGTTATCGTAAA
GAGTCCGACGGTAAAGAACATCTGGAGGAGTTTACCATGCTGGCCTTTGCCCAAATGGGTTCAGGTTGTACTCGT
GAGAACCTGGAAAGCATCATCACCGATTTTCTGAACCACCTGGGCATTGACTTCAAAATTGTGGGCGACAGCTGT
ATGGTGTATGGCGACACCCTGGATGTCATGCACGGCGACCTGGAACTGTCTAGTGCCGTTGTTGGACCAATTCCG
CTGGACCGTGAGTGGGGTATCGACAAACCGTGGATCGGAGCAGGATTCGGTCTGGAACGCCTGCTGAAAGTGAAA
CACGACTTCAAAAACATCAAACGTGCCGCCCGTTCTGAATCGTATTATAACGGGATCTCTACGAACCTGATGGCG
TCCACGGATTACAGTACCTATAGCCAAGCTGCAGCGCAGCAGGGCTACAGTGCTTACACCGCCCAGCCCACTCAA
GGATATGCACAGACCACCCAGGCATATGGGCAACAAAGCTATGGAACCTATGGACAGCCCACTGATGTCAGCTAT
ACCCAGGCTCAGACCACTGCAACCTATGGGCAGACCGCCTATGCAACTTCTTATGGACAGCCTCCCACTGGTTAT
ACTACTCCAACTGCCCCCCAGGCATACAGCCAGCCTGTCCAGGGGTATGGCACTGGTGCTTATGATACCACCACT

GCTACAGTCACCACCACCCAGGCCTCCTATGCAGCTCAGTCTGCATATGGCACTCAGCCTGCTTATCCAGCCTAT
GGGCAGCAGCCAGCAGCCACTGCACCTACAAGACCGCAGGATGGAAACAAGCCCACTGAGACTAGTCAACCTCAA
TCTAGCACAGGGGGTTACAACCAGCCCAGCCTAGGATATGGACAGAGTAACTACAGTTATCCCCAGGTACCTGGG
AGCTACCCCATGCAGCCAGTCACTGCACCTCCATCCTACCCTCCTACCAGCTATTCCTCTACACAGCCGACTAGT
TATGATCAGAGCAGTTACTCTCAGCAGAACACCTATGGGCAACCGAGCAGCTATGGACAGCAGAGTAGCTATGGT
CAACAAAGCAGCTATGGGCAGCAGCCTCCCACTAGTTACCCACCCCAAACTGGATCCTACAGCCAAGCTCCAAGT
CAATATAGCCAACAGAGCAGCAGCTACGGGCAGCAGAGTTCATTCCGACAGGACCACCCCAGTAGCATGGGTGTT
TATGGGCAGGAGTCTGGAGGATTTTCCGGACCAGGAGAGAACCGGAGCATGAGTGGCCCTGATAACCGGGGCAGG
GGAAGAGGGGGATTTGATCGTGGAGGCATGAGCAGAGGTGGGCGGGGAGGAGGACGCGGTGGAATGGGCAGCGCT
GGAGAGCGAGGTGGCTTCAATAAGCCTGGTGGACCCATGGATGAAGGACCAGATCTTGATCTAGGCCCACCTGTA
GATCCAGATGAAGACTCTGACAACAGTGCAATTTATGTACAAGGATTAAATGACAGTGTGACTCTAGATGATCTG
GCAGACTTCTTTAAGCAGTGTGGGGTTGTTAAGATGAACAAGAGAACTGGGCAACCCATGATCCACATCTACCTG
GACAAGGAAACAGGAAAGCCCAAAGGCGATGCCACAGTGTCCTATGAAGACCCACCTACTGCCAAGGCTGCCGTG
GAATGGTTTGATGGGAAAGATTTTCAAGGGAGCAAACTTAAAGTCTCCCTTGCTCGGAAGAAGCCTCCAATGAAC
AGTATGCGGGGTGGTCTGCCACCCCGTGAGGGCAGAGGCATGCCACCACCACTCCGTGGAGGTCCAGGAGGCCCA
GGAGGTCCTGGGGGACCCATGGGTCGCATGGGAGGCCGTGGAGGAGATAGAGGAGGCTTCCCTCCAAGAGGACCC
CGGGGTTCCCGAGGGAACCCCTCTGGAGGAGGAAACGTCCAGCACCGAGCTGGAGACTGGCAGTGTCCCAATCCG
GGTTGTGGAAACCAGAACTTCGCCTGGAGAACAGAGTGCAACCAGTGTAAGGCCCCAAAGCCTGAAGGCTTCCTC
CCGCCACCCTTTCCGCCCCCGGGTGGTGATCGTGGCAGAGGTGGCCCTGGTGGCATGCGGGGAGGAAGAGGTGGC
CTCATGGATCGTGGTGGTCCCGGTGGAATGTTCAGAGGTGGCCGTGGTGGAGACAGAGGTGGCTTCCGTGGTGGC
CGGGGCATGGACCGAGGTGGCTTTGGTGGAGGAAGACGAGGTGGCCCTGGGGGGCCCCCTGGACCTTTGATGGAA
CAGGATAAAAAACCGCTGAATACCCTGATCTCTGCTACTGGTCTGTGGATGAGTCGTACCGGAACCATTCATAAA
ATCAAACACCACGAGGTTAGCCGTTCGAAAATCTATATTGAGATGGCGTGTGGCGATCATCTGGTTGTGAACAAT
AGCCGCTCTTCTCGTACAGCACGTGCACTGCGTCACCACAAATATCGTAAAACCTGTAAACGTTGCCGTGTGTCC
GATGAGGATCTGAACAAATTCCTGACAAAAGCCAATGAGGACCAAACAAGCGTGAAAGTGAAAGTCGTTAGCGCT
CCTACCCGTACTAAAAAAGCAATGCCGAAATCCGTTGCTCGTGCCCCTAAACCACTGGAAAACACTGAAGCAGCA
CAGGCACAGCCGTCTGGAAGCAAATTCTCTCCGGCCATTCCTGTTTCTACCCAGGAGTCCGTTTCTGTTCCAGCA
AGTGTGAGCACCAGCATTAGCAGTATTAGCACCGGTGCCACCGCTAGCGCCCTGGTTAAAGGCAATACCAATCCG
ATTACAAGCATGTCTGCCCCGGTTCAAGCATCAGCTCCAGCACTGACAAAATCCCAAACCGATCGTCTGGAGGTT
CTGCTGAATCCGAAAGACGAAATCAGCCTGAATTCCGGCAAACCGTTTCGTGAACTGGAGAGCGAACTGCTGTCA
CGTCGTAAAAAAGACCTGCAACAAATCTATGCCGAAGAACGTGAGAACTATCTGGGGAAACTGGAACGTGAAATC
ACCCGCTTTTTCGTGGATCGTGGCTTTCTGGAGATCAAATCCCCGATTCTGATTCCTCTGGAGTATATCGAGCGT
ATGGGCATCGACAATGATACCGAACTGAGCAAACAAATTTTCCGTGTGGATAAAAACTTCTGTCTGCGCCCTATG
CTGGCACCAAATCTGTATAACTATCTGCGCAAACTGGACCGTGCCCTGCCTGATCCTATCAAAATCTTCGAGATC
GGCCCGTGTTATCGTAAAGAGTCCGACGGTAAAGAACATCTGGAGGAGTTTACCATGCTGGCCTTTGCCCAAATG
GGTTCAGGTTGTACTCGTGAGAACCTGGAAAGCATCATCACCGATTTTCTGAACCACCTGGGCATTGACTTCAAA
ATTGTGGGCGACAGCTGTATGGTGTATGGCGACACCCTGGATGTCATGCACGGCGACCTGGAACTGTCTAGTGCC
GTTGTTGGACCAATTCCGCTGGACCGTGAGTGGGGTATCGACAAACCGTGGATCGGAGCAGGATTCGGTCTGGAA
CGCCTGCTGAAAGTGAAACACGACTTCAAAAACATCAAACGTGCCGCCCGTTCTGAATCGTATTATAACGGGATC
TCTACGAACCTGTAA (SEQ ID NO: 513)

Protein:
MVGRNSAIAAGVCGALFIGYCIYFDRKRRSDPNFKNRLRERRKKQKLAKERAGLSKLPDLKDAEAVQKFFMASND
YTQQATQSYGAYPTQPGQGYSQQSSQPYGQQSYSGYSQSTDTSGYGQSSYSSYGQSQNTGYGTQSTPQGYGSTGG
YGSSQSSQSSYGQQSSYPGYGQQPAPSSTSGSYGSSSQSSSYGQPQSGSYSQQPSYGGQQQSYGQQQSYNPPQGY
GQQNQYNSSSGGGGGGGGGGNYGQDQSSMSSGGGSGGGYGNQDQSGGGGSGGYGQQDRGGRGRGGSGGGGGGGG
GYNRSSGGYEPRGRGGGRGGRGGMGGSDRGGFNKFGGPRDQGSRHDSEQDNSDNNTIFVQGLGENVTIESVADYF
KQIGIIKTNKKTGQPMINLYTDRETGKLKGEATVSFDDPPSAKAAIDWFDGKEFSGNPIKVSFATRRADFNRGGG
NGRGGRGRGGPMGRGGYGGGGSGGGGRGGFPSGGGGGGGQQRAGDWKCPNPTCENMNFSWRNECNQCKAPKPDGP
GGGPGGSHMGGNYGDDRRGGRGGAIAGKPIPNPLLGLDSTGAPGSAGSAAGSGACPVPLQLPPLERLTLDDKKPL
NTLISATGLWMSRTGTIHKIKHHEVSRSKIYIEMACGDHLVVNNSRSSRTARALRHHKYRKTCKRCRVSDEDLNK
FLTKANEDQTSVKVKVVSAPTRTKKAMPKSVARAPKPLENTEAAQAQPSGSKFSPAIPVSTQESVSVPASVSTSI
SSISTGATASALVKGNTNPITSMSAPVQASAPALTKSQTDRLEVLLNPKDEISLNSGKPFRELESELLSRRKKDL
QQIYAEERENYLGKLEREITRFFVDRGFLEIKSPILIPLEYIERMGIDNDTELSKQIFRVDKNFCLRPMLAPNLY
NYLRKLDRALPDPIKIFEIGPCYRKESDGKEHLEEFTMLAFAQMGSGCTRENLESIITDFLNHLGIDFKIVGDSC
MVYGDTLDVMHGDLELSSAVVGPIPLDREWGIDKPWIGAGFGLERLLKVKHDFKNIKRAARSESYYNGISTNLMA
STDYSTYSQAAAQQGYSAYTAQPTQGYAQTTQAYGQQSYGTYGQPTDVSYTQAQTTATYGQTAYATSYGQPPTGY
TTPTAPQAYSQPVQGYGTGAYDTTTATVTTTQASYAAQSAYGTQPAYPAYGQQPAATAPTRPQDGNKPTETSQPQ

SSTGGYNQPSLGYGQSNYSYPQVPGSYPMQPVTAPPSYPPTSYSSTQPTSYDQSSYSQQNTYGQPSSYGQQSSYG
QQSSYGQQPPTSYPPQTGSYSQAPSQYSQQSSSYGQQSSFRQDHPSSMGVYGQESGGFSGPGENRSMSGPDNRGR
GRGGFDRGGMSRGGRGGGRGGMGSAGERGGFNKPGGPMDEGPDLDLGPPVDPDEDSDNSAIYVQGLNDSVTLDDL
ADFFKQCGVVKMNKRTGQPMIHIYLDKETGKPKGDATVSYEDPPTAKAAVEWFDGKDFQGSKLKVSLARKKPPMN
SMRGGLPPREGRGMPPPLRGGPGGPGGPGGPMGRMGGRGGDRGGFPPRGPRGSRGNPSGGGNVQHRAGDWQCPNP
GCGNQNFAWRTECNQCKAPKPEGFLPPPFPPPGGDRGRGGPGGMRGGRGGLMDRGGPGGMFRGGRGGDRGGFRGG
RGMDRGGFGGGRRGGPGGPPGPLMEQDKKPLNTLISATGLWMSRTGTIHKIKHHEVSRSKIYIEMACGDHLVVNN
SRSSRTARALRHHKYRKTCKRCRVSDEDLNKFLTKANEDQTSVKVKVVSAPTRTKKAMPKSVARAPKPLENTEAA
QAQPSGSKFSPAIPVSTQESVSVPASVSTSISSISTGATASALVKGNTNPITSMSAPVQASAPALTKSQTDRLEV
LLNPKDEISLNSGKPFRELESELLSRRKKDLQQIYAEERENYLGKLEREITRFFVDRGFLEIKSPILIPLEYIER
MGIDNDTELSKQIFRVDKNFCLRPMLAPNLYNYLRKLDRALPDPIKIFEIGPCYRKESDGKEHLEEFTMLAFAQM
GSGCTRENLESIITDFLNHLGIDFKIVGDSCMVYGDTLDVMHGDLELSSAVVGPIPLDREWGIDKPWIGAGFGLE
RLLKVKHDFKNIKRAARSESYYNGISTNL* (SEQ ID NO: 514)

TOM20::FUS::MCP::PylRS(AA)::EWSR1::PylRS(AA)
DNA:
ATGGTGGGTCGGAACAGCGCCATCGCCGCCGGTGTATGCGGGGCCCTTTTCATTGGGTACTGCATCTACTTCGAC
CGCAAAAGACGAAGTGACCCCAACTTCAAGAACAGGCTTCGAGAACGAAGAAAGAAACAGAAGCTTGCCAAGGAG
AGAGCTGGGCTTTCCAAGTTACCTGACCTTAAAGATGCTGAAGCTGTTCAGAAATTCTTCATGGCCTCAAACGAT
TATACCCAACAAGCAACCCAAAGCTATGGGGCCTACCCCACCCAGCCCGGGCAGGGCTATTCCCAGCAGAGCAGT
CAGCCCTACGGACAGCAGAGTTACAGTGGTTATAGCCAGTCCACGGACACTTCAGGATATGGCCAGAGCAGCTAT
TCTTCTTATGGCCAGAGCCAGAACACAGGCTATGGAACTCAGTCAACTCCCCAGGGATATGGCTCGACTGGCGGC
TATGGCAGTAGCCAGAGCTCCCAATCGTCTTACGGGCAGCAGTCCTCCTACCCTGGCTATGGCCAGCAGCCAGCT
CCCAGCAGCACCTCGGGAAGTTACGGTAGCAGTTCTCAGAGCAGCAGCTATGGGCAGCCCCAGAGTGGGAGCTAC
AGCCAGCAGCCTAGCTATGGTGGACAGCAGCAAAGCTATGGACAGCAGCAAAGCTATAATCCCCCTCAGGGCTAT
GGACAGCAGAACCAGTACAACAGCAGCAGTGGTGGTGGAGGTGGAGGTGGAGGTGGAGGTAACTATGGCCAAGAT
CAATCCTCCATGAGTAGTGGTGGTGGCAGTGGTGGCGGTTATGGCAATCAAGACCAGAGTGGTGGAGGTGGCAGC
GGTGGCTATGGACAGCAGGACCGTGGAGGCCGCGGCAGGGGTGGCAGTGGTGGCGGCGGCGGCGGCGGCGGTGGT
GGTTACAACCGCAGCAGTGGTGGCTATGAACCCAGAGGTCGTGGAGGTGGCCGTGGAGGCAGAGGTGGCATGGGC
GGAAGTGACCGTGGTGGCTTCAATAAATTTGGTGGCCCTCGGGACCAAGGATCACGTCATGACTCCGAACAGGAT
AATTCAGACAACAACACCATCTTTGTGCAAGGCCTGGGTGAGAATGTTACAATTGAGTCTGTGGCTGATTACTTC
AAGCAGATTGGTATTATTAAGACAAACAAGAAAACGGGACAGCCCATGATTAATTTGTACACAGACAGGGAAACT
GGCAAGCTGAAGGGAGAGGCAACGGTCTCTTTTGATGACCCACCTTCAGCTAAAGCAGCTATTGACTGGTTTGAT
GGTAAAGAATTCTCCGGAAATCCTATCAAGGTCTCATTTGCTACTCGCCGGGCAGACTTTAATCGGGGTGGTGGC
AATGGTCGTGGAGGCCGAGGGCGAGGAGGACCCATGGGCCGTGGAGGCTATGGAGGTGGTGGCAGTGGTGGTGGT
GGCCGAGGAGGATTTCCCAGTGGAGGTGGTGGCGGTGGAGGACAGCAGCGAGCTGGTGACTGGAAGTGTCCTAAT
CCCACCTGTGAGAATATGAACTTCTCTTGGAGGAATGAATGCAACCAGTGTAAGGCCCCTAAACCAGATGGCCCA
GGAGGGGGACCAGGTGGCTCTCACATGGGGGGTAACTACGGGGATGATCGTCGTGGTGGCAGAGGAGGCGCGATC
GCATATCCCTATGATGTGCCGGATTATGCTGGAGCACCAGGAAGTGCTGGTTCTGCTGCTGGTAGTGGAGCTTCT
AACTTTACTCAGTTCGTTCTCGTCGACAATGGCGGAACTGGCGACGTGACTGTCGCCCCAAGCAACTTCGCTAAC
GGGATCGCTGAATGGATCAGCTCTAACTCGCGTTCACAGGCTTACAAAGTAACCTGTAGCGTTCGTCAGAGCTCT
GCGCAGAATCGCAAATACACCATCAAAGTCGAGGTGCCTAAAGGCGCCTGGCGTTCGTACTTAAATATGGAACTA
ACCATTCCAATTTTCGCCACGAATTCCGACTGCGAGCTTATTGTTAAGGCAATGCAAGGTCTCCTAAAAGATGGA
AACCCGATTCCCTCAGCAATCGCAGCAAACTCCGGCATCTACGGCGCCGATTACAAGGACGATGATGACAAGGGA
GCACCAGGAAGTGCTGGTTCTGCTGCTGGTAGTGGAGCGTGCCCGGTGCCGCTGCAGCTGCCGCCGCTGGAACGC
CTGACCCTGGATGATAAAAAACCGCTGAATACCCTGATCTCTGCTACTGGTCTGTGGATGAGTCGTACCGGAACC
ATTCATAAAATCAAACACCACGAGGTTAGCCGTTCGAAAATCTATATTGAGATGGCGTGTGGCGATCATCTGGTT
GTGAACAATAGCCGCTCTTCTCGTACAGCACGTGCACTGCGTCACCACAAATATCGTAAAACCTGTAAACGTTGC
CGTGTGTCCGATGAGGATCTGAACAAATTCCTGACAAAAGCCAATGAGGACCAAACAAGCGTGAAAGTGAAAGTC
GTTAGCGCTCCTACCCGTACTAAAAAAGCAATGCCGAAATCCGTTGCTCGTGCCCCTAAACCACTGGAAAACACT
GAAGCAGCACAGGCACAGCCGTCTGGAAGCAAATTCTCTCCGGCCATTCCTGTTTCTACCCAGGAGTCCGTTTCT
GTTCCAGCAAGTGTGAGCACCAGCATTAGCAGTATTAGCACCGGTGCCACCGCTAGCGCCCTGGTTAAAGGCAAT
ACCAATCCGATTACAAGCATGTCTGCCCCGGTTCAAGCATCAGCTCCAGCACTGACAAAATCCCAAACCGATCGT
CTGGAGGTTCTGCTGAATCCGAAAGACGAAATCAGCCTGAATTCCGGCAAACCGTTTCGTGAACTGGAGAGCGAA
CTGCTGTCACGTCGTAAAAAAGACCTGCAACAAATCTATGCCGAAGAACGTGAGAACTATCTGGGGAAACTGGAA
CGTGAAATCACCCGCTTTTTCGTGGATCGTGGCTTTCTGGAGATCAAATCCCCGATTCTGATTCCTCTGGAGTAT
ATCGAGCGTATGGGCATCGACAATGATACCGAACTGAGCAAACAAATTTTCCGTGTGGATAAAAACTTCTGTCTG

CGCCCTATGCTGGCACCAAATCTGTATAACTATCTGCGCAAACTGGACCGTGCCCTGCCTGATCCTATCAAAATC
TTCGAGATCGGCCCGTGTTATCGTAAAGAGTCCGACGGTAAAGAACATCTGGAGGAGTTTACCATGCTGGCCTTT
GCCCAAATGGGTTCAGGTTGTACTCGTGAGAACCTGGAAAGCATCATCACCGATTTTCTGAACCACCTGGGCATT
GACTTCAAAATTGTGGGCGACAGCTGTATGGTGTATGGCGACACCCTGGATGTCATGCACGGCGACCTGGAACTG
TCTAGTGCCGTTGTTGGACCAATTCCGCTGGACCGTGAGTGGGGTATCGACAAACCGTGGATCGGAGCAGGATTC
GGTCTGGAACGCCTGCTGAAAGTGAAACACGACTTCAAAAACATCAAACGTGCCGCCCGTTCTGAATCGTATTAT
AACGGGATCTCTACGAACCTGATGGCGTCCACGGATTACAGTACCTATAGCCAAGCTGCAGCGCAGCAGGGCTAC
AGTGCTTACACCGCCCAGCCCACTCAAGGATATGCACAGACCACCCAGGCATATGGGCAACAAAGCTATGGAACC
TATGGACAGCCCACTGATGTCAGCTATACCCAGGCTCAGACCACTGCAACCTATGGGCAGACCGCCTATGCAACT
TCTTATGGACAGCCTCCCACTGGTTATACTACTCCAACTGCCCCCCAGGCATACAGCCAGCCTGTCCAGGGGTAT
GGCACTGGTGCTTATGATACCACCACTGCTACAGTCACCACCACCCAGGCCTCCTATGCAGCTCAGTCTGCATAT
GGCACTCAGCCTGCTTATCCAGCCTATGGGCAGCAGCCAGCAGCCACTGCACCTACAAGACCGCAGGATGGAAAC
AAGCCCACTGAGACTAGTCAACCTCAATCTAGCACAGGGGGTTACAACCAGCCCAGCCTAGGATATGGACAGAGT
AACTACAGTTATCCCCAGGTACCTGGGAGCTACCCCATGCAGCCAGTCACTGCACCTCCATCCTACCCTCCTACC
AGCTATTCCTCTACACAGCCGACTAGTTATGATCAGAGCAGTTACTCTCAGCAGAACACCTATGGGCAACCGAGC
AGCTATGGACAGCAGAGTAGCTATGGTCAACAAAGCAGCTATGGGCAGCAGCCTCCCACTAGTTACCCACCCCAA
ACTGGATCCTACAGCCAAGCTCCAAGTCAATATAGCCAACAGAGCAGCAGCTACGGGCAGCAGAGTTCATTCCGA
CAGGACCACCCCAGTAGCATGGGTGTTTATGGGCAGGAGTCTGGAGGATTTTCCGGACCAGGAGAGAACCGGAGC
ATGAGTGGCCCTGATAACCGGGGCAGGGGAAGAGGGGGATTTGATCGTGGAGGCATGAGCAGAGGTGGGCGGGGA
GGAGGACGCGGTGGAATGGGCAGCGCTGGAGAGCGAGGTGGCTTCAATAAGCCTGGTGGACCCATGGATGAAGGA
CCAGATCTTGATCTAGGCCCACCTGTAGATCCAGATGAAGACTCTGACAACAGTGCAATTTATGTACAAGGATTA
AATGACAGTGTGACTCTAGATGATCTGGCAGACTTCTTTAAGCAGTGTGGGGTTGTTAAGATGAACAAGAGAACT
GGGCAACCCATGATCCACATCTACCTGGACAAGGAAACAGGAAAGCCCAAAGGCGATGCCACAGTGTCCTATGAA
GACCCACCTACTGCCAAGGCTGCCGTGGAATGGTTTGATGGGAAAGATTTTCAAGGGAGCAAACTTAAAGTCTCC
CTTGCTCGGAAGAAGCCTCCAATGAACAGTATGCGGGGTGGTCTGCCACCCCGTGAGGGCAGAGGCATGCCACCA
CCACTCCGTGGAGGTCCAGGAGGCCCAGGAGGTCCTGGGGGACCCATGGGTCGCATGGGAGGCCGTGGAGGAGAT
AGAGGAGGCTTCCCTCCAAGAGGACCCCGGGGTTCCCGAGGGAACCCCTCTGGAGGAGGAAACGTCCAGCACCGA
GCTGGAGACTGGCAGTGTCCCAATCCGGGTTGTGGAAACCAGAACTTCGCCTGGAGAACAGAGTGCAACCAGTGT
AAGGCCCCAAAGCCTGAAGGCTTCCTCCCGCCACCCTTTCCGCCCCCGGGTGGTGATCGTGGCAGAGGTGGCCCT
GGTGGCATGCGGGGAGGAAGAGGTGGCCTCATGGATCGTGGTGGTCCCGGTGGAATGTTCAGAGGTGGCCGTGGT
GGAGACAGAGGTGGCTTCCGTGGTGGCCGGGGCATGGACCGAGGTGGCTTTGGTGGAGGAAGACGAGGTGGCCCT
GGGGGGCCCCCTGGACCTTTGATGGAACAGGATAAAAAACCGCTGAATACCCTGATCTCTGCTACTGGTCTGTGG
ATGAGTCGTACCGGAACCATTCATAAAATCAAACACCACGAGGTTAGCCGTTCGAAAATCTATATTGAGATGGCG
TGTGGCGATCATCTGGTTGTGAACAATAGCCGCTCTTCTCGTACAGCACGTGCACTGCGTCACCACAAATATCGT
AAAACCTGTAAACGTTGCCGTGTGTCCGATGAGGATCTGAACAAATTCCTGACAAAAGCCAATGAGGACCAAACA
AGCGTGAAAGTGAAAGTCGTTAGCGCTCCTACCCGTACTAAAAAAGCAATGCCGAAATCCGTTGCTCGTGCCCCT
AAACCACTGGAAAACACTGAAGCAGCACAGGCACAGCCGTCTGGAAGCAAATTCTCTCCGGCCATTCCTGTTTCT
ACCCAGGAGTCCGTTTCTGTTCCAGCAAGTGTGAGCACCAGCATTAGCAGTATTAGCACCGGTGCCACCGCTAGC
GCCCTGGTTAAAGGCAATACCAATCCGATTACAAGCATGTCTGCCCCGGTTCAAGCATCAGCTCCAGCACTGACA
AAATCCCAAACCGATCGTCTGGAGGTTCTGCTGAATCCGAAAGACGAAATCAGCCTGAATTCCGGCAAACCGTTT
CGTGAACTGGAGAGCGAACTGCTGTCACGTCGTAAAAAAGACCTGCAACAAATCTATGCCGAAGAACGTGAGAAC
TATCTGGGGAAACTGGAACGTGAAATCACCCGCTTTTTCGTGGATCGTGGCTTTCTGGAGATCAAATCCCCGATT
CTGATTCCTCTGGAGTATATCGAGCGTATGGGCATCGACAATGATACCGAACTGAGCAAACAAATTTTCCGTGTG
GATAAAAACTTCTGTCTGCGCCCTATGCTGGCACCAAATCTGTATAACTATCTGCGCAAACTGGACCGTGCCCTG
CCTGATCCTATCAAAATCTTCGAGATCGGCCCGTGTTATCGTAAAGAGTCCGACGGTAAAGAACATCTGGAGGAG
TTTACCATGCTGGCCTTTGCCCAAATGGGTTCAGGTTGTACTCGTGAGAACCTGGAAAGCATCATCACCGATTTT
CTGAACCACCTGGGCATTGACTTCAAAATTGTGGGCGACAGCTGTATGGTGTATGGCGACACCCTGGATGTCATG
CACGGCGACCTGGAACTGTCTAGTGCCGTTGTTGGACCAATTCCGCTGGACCGTGAGTGGGGTATCGACAAACCG
TGGATCGGAGCAGGATTCGGTCTGGAACGCCTGCTGAAAGTGAAACACGACTTCAAAAACATCAAACGTGCCGCC
CGTTCTGAATCGTATTATAACGGGATCTCTACGAACCTGTAA (SEQ ID NO: 515)

Protein:
MVGRNSAIAAGVCGALFIGYCIYFDRKRRSDPNFKNRLRERRKKQKLAKERAGLSKLPDLKDAEAVQKFFMASND
YTQQATQSYGAYPTQPGQGYSQQSSQPYGQQSYSGYSQSTDTSGYGQSSYSSYGQSQNTGYGTQSTPQGYGSTGG
YGSSQSSQSSYGQQSSYPGYGQQPAPSSTSGSYGSSSQSSSYGQPQSGSYSQQPSYGGQQQSYGQQQSYNPPQGY
GQQNQYNSSSGGGGGGGGGGNYGQDQSSMSSGGGSGGGYGNQDQSGGGGSGGYGQQDRGGRGRGGSGGGGGGGGG
GYNRSSGGYEPRGRGGGRGGRGGMGGSDRGGFNKFGGPRDQGSRHDSEQDNSDNNTIFVQGLGENVTIESVADYF
KQIGIIKTNKKTGQPMINLYTDRETGKLKGEATVSFDDPPSAKAAIDWFDGKEFSGNPIKVSFATRRADFNRGGG

NGRGGRGRGGPMGRGGYGGGGSGGGGRGGFPSGGGGGGGQQRAGDWKCPNPTCENMNFSWRNECNQCKAPKPDGP
GGGPGGSHMGGNYGDDRRGGRGGAIAYPYDVPDYAGAPGSAGSAAGSGASNFTQFVLVDNGGTGDVTVAPSNFAN
GIAEWISSNSRSQAYKVTCSVRQSSAQNRKYTIKVEVPKGAWRSYLNMELTIPIFATNSDCELIVKAMQGLLKDG
NPIPSAIAANSGIYGADYKDDDDKGAPGSAGSAAGSGACPVPLQLPPLERLTLDDKKPLNTLISATGLWMSRTGT
IHKIKHHEVSRSKIYIEMACGDHLVVNNSRSSRTARALRHHKYRKTCKRCRVSDEDLNKFLTKANEDQTSVKVKV
VSAPTRTKKAMPKSVARAPKPLENTEAAQAQPSGSKFSPAIPVSTQESVSVPASVSTSISSISTGATASALVKGN
TNPITSMSAPVQASAPALTKSQTDRLEVLLNPKDEISLNSGKPFRELESELLSRRKKDLQQIYAEERENYLGKLE
REITRFFVDRGFLEIKSPILIPLEYIERMGIDNDTELSKQIFRVDKNFCLRPMLAPNLYNYLRKLDRALPDPIKI
FEIGPCYRKESDGKEHLEEFTMLAFAQMGSGCTRENLESIITDFLNHLGIDFKIVGDSCMVYGDTLDVMHGDLEL
SSAVVGPIPLDREWGIDKPWIGAGFGLERLLKVKHDFKNIKRAARSESYYNGISTNLMASTDYSTYSQAAAQQGY
SAYTAQPTQGYAQTTQAYGQQSYGTYGQPTDVSYTQAQTTATYGQTAYATSYGQPPTGYTTPTAPQAYSQPVQGY
GTGAYDTTTATVTTTQASYAAQSAYGTQPAYPAYGQQPAATAPTRPQDGNKPTETSQPQSSTGGYNQPSLGYGQS
NYSYPQVPGSYPMQPVTAPPSYPPTSYSSTQPTSYDQSSYSQQNTYGQPSSYGQQSSYGQQSSYGQQPPTSYPPQ
TGSYSQAPSQYSQQSSSYGQQSSFRQDHPSSMGVYGQESGGFSGPGENRSMSGPDNRGRGRGGFDRGGMSRGGRG
GGRGGMGSAGERGGFNKPGGPMDEGPDLDLGPPVDPDEDSDNSAIYVQGLNDSVTLDDLADFFKQCGVVKMNKRT
GQPMIHIYLDKETGKPKGDATVSYEDPPTAKAAVEWFDGKDFQGSKLKVSLARKKPPMNSMRGGLPPREGRGMPP
PLRGGPGGPGGPGGPMGRMGGRGGDRGGFPPRGPRGSRGNPSGGGNVQHRAGDWQCPNPGCGNQNFAWRTECNQC
KAPKPEGFLPPPFPPPGGDRGRGGPGGMRGGRGGLMDRGGPGGMFRGGRGGDRGGFRGGRGMDRGGFGGGRRGGP
GGPPGPLMEQDKKPLNTLISATGLWMSRTGTIHKIKHHEVSRSKIYIEMACGDHLVVNNSRSSRTARALRHHKYR
KTCKRCRVSDEDLNKFLTKANEDQTSVKVKVVSAPTRTKKAMPKSVARAPKPLENTEAAQAQPSGSKFSPAIPVS
TQESVSVPASVSTSISSISTGATASALVKGNTNPITSMSAPVQASAPALTKSQTDRLEVLLNPKDEISLNSGKPF
RELESELLSRRKKDLQQIYAEERENYLGKLEREITRFFVDRGFLEIKSPILIPLEYIERMGIDNDTELSKQIFRV
DKNFCLRPMLAPNLYNYLRKLDRALPDPIKIFEIGPCYRKESDGKEHLEEFTMLAFAQMGSGCTRENLESIITDF
LNHLGIDFKIVGDSCMVYGDTLDVMHGDLELSSAVVGPIPLDREWGIDKPWIGAGFGLERLLKVKHDFKNIKRAA
RSESYYNGISTNL* (SEQ ID NO: 516)

TOM20::FUS::4xλN22::PylRS(AA)::EWSR1::PylRS(AA)
DNA:
ATGGTGGGTCGGAACAGCGCCATCGCCGCCGGTGTATGCGGGGCCCTTTTCATTGGGTACTGCATCTACTTCGAC
CGCAAAAGACGAAGTGACCCCAACTTCAAGAACAGGCTTCGAGAACGAAGAAAGAAACAGAAGCTTGCCAAGGAG
AGAGCTGGGCTTTCCAAGTTACCTGACCTTAAAGATGCTGAAGCTGTTCAGAAATTCTTCATGGCCTCAAACGAT
TATACCCAACAAGCAACCCAAAGCTATGGGGCCTACCCCACCCAGCCCGGGCAGGGCTATTCCCAGCAGAGCAGT
CAGCCCTACGGACAGCAGAGTTACAGTGGTTATAGCCAGTCCACGGACACTTCAGGATATGGCCAGAGCAGCTAT
TCTTCTTATGGCCAGAGCCAGAACACAGGCTATGGAACTCAGTCAACTCCCCAGGGATATGGCTCGACTGGCGGC
TATGGCAGTAGCCAGAGCTCCCAATCGTCTTACGGGCAGCAGTCCTCCTACCCTGGCTATGGCCAGCAGCCAGCT
CCCAGCAGCACCTCGGGAAGTTACGGTAGCAGTTCTCAGAGCAGCAGCTATGGGCAGCCCCAGAGTGGGAGCTAC
AGCCAGCAGCCTAGCTATGGTGGACAGCAGCAAAGCTATGGACAGCAGCAAAGCTATAATCCCCCTCAGGGCTAT
GGACAGCAGAACCAGTACAACAGCAGCAGTGGTGGTGGAGGTGGAGGTGGAGGTGGAGGTAACTATGGCCAAGAT
CAATCCTCCATGAGTAGTGGTGGTGGCAGTGGTGGCGGTTATGGCAATCAAGACCAGAGTGGTGGAGGTGGCAGC
GGTGGCTATGGACAGCAGGACCGTGGAGGCCGCGGCAGGGGTGGCAGTGGTGGCGGCGGCGGCGGCGGCGGTGGT
GGTTACAACCGCAGCAGTGGTGGCTATGAACCCAGAGGTCGTGGAGGTGGCCGTGGAGGCAGAGGTGGCATGGGC
GGAAGTGACCGTGGTGGCTTCAATAAATTTGGTGGCCCTCGGGACCAAGGATCACGTCATGACTCCGAACAGGAT
AATTCAGACAACAACACCATCTTTGTGCAAGGCCTGGGTGAGAATGTTACAATTGAGTCTGTGGCTGATTACTTC
AAGCAGATTGGTATTATTAAGACAAACAAGAAAACGGGACAGCCCATGATTAATTTGTACACAGACAGGGAAACT
GGCAAGCTGAAGGGAGAGGCAACGGTCTCTTTTGATGACCCACCTTCAGCTAAAGCAGCTATTGACTGGTTTGAT
GGTAAAGAATTCTCCGGAAATCCTATCAAGGTCTCATTTGCTACTCGCCGGGCAGACTTTAATCGGGGTGGTGGC
AATGGTCGTGGAGGCCGAGGGCGAGGAGGACCCATGGGCCGTGGAGGCTATGGAGGTGGTGGCAGTGGTGGTGGT
GGCCGAGGAGGATTTCCCAGTGGAGGTGGTGGCGGTGGAGGACAGCAGCGAGCTGGTGACTGGAAGTGTCCTAAT
CCCACCTGTGAGAATATGAACTTCTCTTGGAGGAATGAATGCAACCAGTGTAAGGCCCCTAAACCAGATGGCCCA
GGAGGGGGACCAGGTGGCTCTCACATGGGGGGTAACTACGGGGATGATCGTCGTGGTGGCAGAGGAGGCGCGATC
GCAGGCAAGCCTATTCCCAACCCCCTGCTGGGCCTGGATAGCACCGGAGCACCAGGAAGTGCTGGTTCTGCTGCT
GGTAGTGGAGCATCGATAGAGCAGAAGCTGATCTCAGAGGAGGACCTGCTAGCCACCATGGACGCACAAACACGA
CGACGTGAGCGTCGCGCTGAGAAACAAGCTCAATGGAAAGCTGCAAACCCACCGCTCGACGGAGCCGGAGCTGGC
GCTGGAGCTGGAGCCGGAGCTGGCGGTCTAGCCACCATGGACGCACAAACACGACGACGTGAGCGTCGCGCTGAG
AAACAAGCTCAATGGAAAGCTGCAAACCCACCGCTCGACGGAGCCGGAGCTGGCGCTGGAGCTGGAGCCGGAGCT
GGCGGTCTAGCCACCATGGACGCACAAACACGACGACGTGAGCGTCGCGCTGAGAAACAAGCTCAATGGAAAGCT
GCAAACCCACCGCTCGACGGAGCCGGAGCTGGCGCTGGAGCTGGAGCCGGAGCTGGCGGTCTAGCCACCATGGAC

GCACAAACACGACGACGTGAGCGTCGCGCTGAGAAACAAGCTCAATGGAAAGCTGCAAACCCACCGCTCGAGTCT
AGAGGGCCCGTTGGTGCTCCTGGTTCAGCAGGAAGCGCAGCAGGATCAGGTGCGTGCCCGGTGCCGCTGCAGCTG
CCGCCGCTGGAACGCCTGACCCTGGATGACAAAAAACCGCTGAATACCCTGATCTCTGCTACTGGTCTGTGGATG
AGTCGTACCGGAACCATTCATAAAATCAAACACCACGAGGTTAGCCGTTCGAAAATCTATATTGAGATGGCGTGT
GGCGATCATCTGGTTGTGAACAATAGCCGCTCTTCTCGTACAGCACGTGCACTGCGTCACCACAAATATCGTAAA
ACCTGTAAACGTTGCCGTGTGTCCGATGAGGATCTGAACAAATTCCTGACAAAAGCCAATGAGGACCAAACAAGC
GTGAAAGTGAAAGTCGTTAGCGCTCCTACCCGTACTAAAAAAGCAATGCCGAAATCCGTTGCTCGTGCCCCTAAA
CCACTGGAAAACACTGAAGCAGCACAGGCACAGCCGTCTGGAAGCAAATTCTCTCCGGCCATTCCTGTTTCTACC
CAGGAGTCCGTTTCTGTTCCAGCAAGTGTGAGCACCAGCATTAGCAGTATTAGCACCGGTGCCACCGCTAGCGCC
CTGGTTAAAGGCAATACCAATCCGATTACAAGCATGTCTGCCCCGGTTCAAGCATCAGCTCCAGCACTGACAAAA
TCCCAAACCGATCGTCTGGAGGTTCTGCTGAATCCGAAAGACGAAATCAGCCTGAATTCCGGCAAACCGTTTCGT
GAACTGGAGAGCGAACTGCTGTCACGTCGTAAAAAAGACCTGCAACAAATCTATGCCGAAGAACGTGAGAACTAT
CTGGGGAAACTGGAACGTGAAATCACCCGCTTTTTCGTGGATCGTGGCTTTCTGGAGATCAAATCCCCGATTCTG
ATTCCTCTGGAGTATATCGAGCGTATGGGCATCGACAATGATACCGAACTGAGCAAACAAATTTTCCGTGTGGAT
AAAAACTTCTGTCTGCGCCCTATGCTGGCACCAAATCTGTATAACTATCTGCGCAAACTGGACCGTGCCCTGCCT
GATCCTATCAAAATCTTCGAGATCGGCCCGTGTTATCGTAAAGAGTCCGACGGTAAAGAACATCTGGAGGAGTTT
ACCATGCTGGCCTTTGCCCAAATGGGTTCAGGTTGTACTCGTGAGAACCTGGAAAGCATCATCACCGATTTTCTG
AACCACCTGGGCATTGACTTCAAAATTGTGGGCGACAGCTGTATGGTGTATGGCGACACCCTGGATGTCATGCAC
GGCGACCTGGAACTGTCTAGTGCCGTTGTTGGACCAATTCCGCTGGACCGTGAGTGGGGTATCGACAAACCGTGG
ATCGGAGCAGGATTCGGTCTGGAACGCCTGCTGAAAGTGAAACACGACTTCAAAAACATCAAACGTGCCGCCCGT
TCTGAATCGTATTATAACGGGATCTCTACGAACCTGATGGCGTCCACGGATTACAGTACCTATAGCCAAGCTGCA
GCGCAGCAGGGCTACAGTGCTTACACCGCCCAGCCCACTCAAGGATATGCACAGACCACCCAGGCATATGGGCAA
CAAAGCTATGGAACCTATGGACAGCCCACTGATGTCAGCTATACCCAGGCTCAGACCACTGCAACCTATGGGCAG
ACCGCCTATGCAACTTCTTATGGACAGCCTCCCACTGGTTATACTACTCCAACTGCCCCCCAGGCATACAGCCAG
CCTGTCCAGGGGTATGGCACTGGTGCTTATGATACCACCACTGCTACAGTCACCACCACCCAGGCCTCCTATGCA
GCTCAGTCTGCATATGGCACTCAGCCTGCTTATCCAGCCTATGGGCAGCAGCCAGCAGCCACTGCACCTACAAGA
CCGCAGGATGGAAACAAGCCCACTGAGACTAGTCAACCTCAATCTAGCACAGGGGGTTACAACCAGCCCAGCCTA
GGATATGGACAGAGTAACTACAGTTATCCCCAGGTACCTGGGAGCTACCCCATGCAGCCAGTCACTGCACCTCCA
TCCTACCCTCCTACCAGCTATTCCTCTACACAGCCGACTAGTTATGATCAGAGCAGTTACTCTCAGCAGAACACC
TATGGGCAACCGAGCAGCTATGGACAGCAGAGTAGCTATGGTCAACAAAGCAGCTATGGGCAGCAGCCTCCCACT
AGTTACCCACCCCAAACTGGATCCTACAGCCAAGCTCCAAGTCAATATAGCCAACAGAGCAGCAGCTACGGGCAG
CAGAGTTCATTCCGACAGGACCACCCCAGTAGCATGGGTGTTTATGGGCAGGAGTCTGGAGGATTTTCCGGACCA
GGAGAGAACCGGAGCATGAGTGGCCCTGATAACCGGGGCAGGGGAAGAGGGGGATTTGATCGTGGAGGCATGAGC
AGAGGTGGGCGGGGAGGAGGACGCGGTGGAATGGGCAGCGCTGGAGAGCGAGGTGGCTTCAATAAGCCTGGTGGA
CCCATGGATGAAGGACCAGATCTTGATCTAGGCCCACCTGTAGATCCAGATGAAGACTCTGACAACAGTGCAATT
TATGTACAAGGATTAAATGACAGTGTGACTCTAGATGATCTGGCAGACTTCTTTAAGCAGTGTGGGGTTGTTAAG
ATGAACAAGAGAACTGGGCAACCCATGATCCACATCTACCTGGACAAGGAAACAGGAAAGCCCAAAGGCGATGCC
ACAGTGTCCTATGAAGACCCACCTACTGCCAAGGCTGCCGTGGAATGGTTTGATGGGAAAGATTTTCAAGGGAGC
AAACTTAAAGTCTCCCTTGCTCGGAAGAAGCCTCCAATGAACAGTATGCGGGGTGGTCTGCCACCCCGTGAGGGC
AGAGGCATGCCACCACCACTCCGTGGAGGTCCAGGAGGCCCAGGAGGTCCTGGGGGACCCATGGGTCGCATGGGA
GGCCGTGGAGGAGATAGAGGAGGCTTCCCTCCAAGAGGACCCCGGGGTTCCCGAGGGAACCCCTCTGGAGGAGGA
AACGTCCAGCACCGAGCTGGAGACTGGCAGTGTCCCAATCCGGGTTGTGGAAACCAGAACTTCGCCTGGAGAACA
GAGTGCAACCAGTGTAAGGCCCCAAAGCCTGAAGGCTTCCTCCCGCCACCCTTTCCGCCCCCGGGTGGTGATCGT
GGCAGAGGTGGCCCTGGTGGCATGCGGGGAGGAAGAGGTGGCCTCATGGATCGTGGTGGTCCCGGTGGAATGTTC
AGAGGTGGCCGTGGTGGAGACAGAGGTGGCTTCCGTGGTGGCCGGGGCATGGACCGAGGTGGCTTTGGTGGAGGA
AGACGAGGTGGCCCTGGGGGGCCCCCTGGACCTTTGATGGAACAGGATAAAAAACCGCTGAATACCCTGATCTCT
GCTACTGGTCTGTGGATGAGTCGTACCGGAACCATTCATAAAATCAAACACCACGAGGTTAGCCGTTCGAAAATC
TATATTGAGATGGCGTGTGGCGATCATCTGGTTGTGAACAATAGCCGCTCTTCTCGTACAGCACGTGCACTGCGT
CACCACAAATATCGTAAAACCTGTAAACGTTGCCGTGTGTCCGATGAGGATCTGAACAAATTCCTGACAAAAGCC
AATGAGGACCAAACAAGCGTGAAAGTGAAAGTCGTTAGCGCTCCTACCCGTACTAAAAAAGCAATGCCGAAATCC
GTTGCTCGTGCCCCTAAACCACTGGAAAACACTGAAGCAGCACAGGCACAGCCGTCTGGAAGCAAATTCTCTCCG
GCCATTCCTGTTTCTACCCAGGAGTCCGTTTCTGTTCCAGCAAGTGTGAGCACCAGCATTAGCAGTATTAGCACC
GGTGCCACCGCTAGCGCCCTGGTTAAAGGCAATACCAATCCGATTACAAGCATGTCTGCCCCGGTTCAAGCATCA
GCTCCAGCACTGACAAAATCCCAAACCGATCGTCTGGAGGTTCTGCTGAATCCGAAAGACGAAATCAGCCTGAAT
TCCGGCAAACCGTTTCGTGAACTGGAGAGCGAACTGCTGTCACGTCGTAAAAAAGACCTGCAACAAATCTATGCC
GAAGAACGTGAGAACTATCTGGGGAAACTGGAACGTGAAATCACCCGCTTTTTCGTGGATCGTGGCTTTCTGGAG
ATCAAATCCCCGATTCTGATTCCTCTGGAGTATATCGAGCGTATGGGCATCGACAATGATACCGAACTGAGCAAA
CAAATTTTCCGTGTGGATAAAAACTTCTGTCTGCGCCCTATGCTGGCACCAAATCTGTATAACTATCTGCGCAAA

CTGGACCGTGCCCTGCCTGATCCTATCAAAATCTTCGAGATCGGCCCGTGTTATCGTAAAGAGTCCGACGGTAAA
GAACATCTGGAGGAGTTTACCATGCTGGCCTTTGCCCAAATGGGTTCAGGTTGTACTCGTGAGAACCTGGAAAGC
ATCATCACCGATTTTCTGAACCACCTGGGCATTGACTTCAAAATTGTGGGCGACAGCTGTATGGTGTATGGCGAC
ACCCTGGATGTCATGCACGGCGACCTGGAACTGTCTAGTGCCGTTGTTGGACCAATTCCGCTGGACCGTGAGTGG
GGTATCGACAAACCGTGGATCGGAGCAGGATTCGGTCTGGAACGCCTGCTGAAAGTGAAACACGACTTCAAAAAC
ATCAAACGTGCCGCCCGTTCTGAATCGTATTATAACGGGATCTCTACGAACCTGTAA (SEQ ID NO: 517)

Protein:
MVGRNSAIAAGVCGALFIGYCIYFDRKRRSDPNFKNRLRERRKKQKLAKERAGLSKLPDLKDAEAVQKFFMASND
YTQQATQSYGAYPTQPGQGYSQQSSQPYGQQSYSGYSQSTDTSGYGQSSYSSYGQSQNTGYGTQSTPQGYGSTGG
YGSSQSSQSSYGQQSSYPGYGQQPAPSSTSGSYGSSSQSSSYGQPQSGSYSQQPSYGGQQQSYGQQQSYNPPQGY
GQQNQYNSSSGGGGGGGGGGNYGQDQSSMSSGGGSGGGYGNQDQSGGGGSGGYGQQDRGGRGRGGSGGGGGGGGG
GYNRSSGGYEPRGRGGGRGGRGGMGGSDRGGFNKFGGPRDQGSRHDSEQDNSDNNTIFVQGLGENVTIESVADYF
KQIGIIKTNKKTGQPMINLYTDRETGKLKGEATVSFDDPPSAKAAIDWFDGKEFSGNPIKVSFATRRADFNRGGG
NGRGGRGRGGPMGRGGYGGGGSGGGGRGGFPSGGGGGGGQQRAGDWKCPNPTCENMNFSWRNECNQCKAPKPDGP
GGGPGGSHMGGNYGDDRRGGRGGAIAGKPIPNPLLGLDSTGAPGSAGSAAGSGASIEQKLISEEDLLATMDAQTR
RRERRAEKQAQWKAANPPLDGAGAGAGAGAGAGGLATMDAQTRRRERRAEKQAQWKAANPPLDGAGAGAGAGAGA
GGLATMDAQTRRRERRAEKQAQWKAANPPLDGAGAGAGAGAGAGGLATMDAQTRRRERRAEKQAQWKAANPPLES
RGPVGAPGSAGSAAGSGACPVPLQLPPLERLTLDDKKPLNTLISATGLWMSRTGTIHKIKHHEVSRSKIYIEMAC
GDHLVVNNSRSSRTARALRHHKYRKTCKRCRVSDEDLNKFLTKANEDQTSVKVKVVSAPTRTKKAMPKSVARAPK
PLENTEAAQAQPSGSKFSPAIPVSTQESVSVPASVSTSISSISTGATASALVKGNTNPITSMSAPVQASAPALTK
SQTDRLEVLLNPKDEISLNSGKPFRELESELLSRRKKDLQQIYAEERENYLGKLEREITRFFVDRGFLEIKSPIL
IPLEYIERMGIDNDTELSKQIFRVDKNFCLRPMLAPNLYNYLRKLDRALPDPIKIFEIGPCYRKESDGKEHLEEF
TMLAFAQMGSGCTRENLESIITDFLNHLGIDFKIVGDSCMVYGDTLDVMHGDLELSSAVVGPIPLDREWGIDKPW
IGAGFGLERLLKVKHDFKNIKRAARSESYYNGISTNLMASTDYSTYSQAAAQQGYSAYTAQPTQGYAQTTQAYGQ
QSYGTYGQPTDVSYTQAQTTATYGQTAYATSYGQPPTGYTTPTAPQAYSQPVQGYGTGAYDTTTATVTTTQASYA
AQSAYGTQPAYPAYGQQPAATAPTRPQDGNKPTETSQPQSSTGGYNQPSLGYGQSNYSYPQVPGSYPMQPVTAPP
SYPPTSYSSTQPTSYDQSSYSQQNTYGQPSSYGQQSSYGQQSSYGQQPPTSYPPQTGSYSQAPSQYSQQSSSYGQ
QSSFRQDHPSSMGVYGQESGGFSGPGENRSMSGPDNRGRGRGGFDRGGMSRGGRGGGRGGMGSAGERGGFNKPGG
PMDEGPDLDLGPPVDPDEDSDNSAIYVQGLNDSVTLDDLADFFKQCGVVKMNKRTGQPMIHIYLDKETGKPKGDA
TVSYEDPPTAKAAVEWFDGKDFQGSKLKVSLARKKPPMNSMRGGLPPREGRGMPPPLRGGPGGPGGPGGPMGRMG
GRGGDRGGFPPRGPRGSRGNPSGGGNVQHRAGDWQCPNPGCGNQNFAWRTECNQCKAPKPEGFLPPPFPPPGGDR
GRGGPGGMRGGRGGLMDRGGPGGMFRGGRGGDRGGFRGGRGMDRGGFGGGRRGGPGGPPGPLMEQDKKPLNTLIS
ATGLWMSRTGTIHKIKHHEVSRSKIYIEMACGDHLVVNNSRSSRTARALRHHKYRKTCKRCRVSDEDLNKFLTKA
NEDQTSVKVKVVSAPTRTKKAMPKSVARAPKPLENTEAAQAQPSGSKFSPAIPVSTQESVSVPASVSTSISSIST
GATASALVKGNTNPITSMSAPVQASAPALTKSQTDRLEVLLNPKDEISLNSGKPFRELESELLSRRKKDLQQIYA
EERENYLGKLEREITRFFVDRGFLEIKSPILIPLEYIERMGIDNDTELSKQIFRVDKNFCLRPMLAPNLYNYLRK
LDRALPDPIKIFEIGPCYRKESDGKEHLEEFTMLAFAQMGSGCTRENLESIITDFLNHLGIDFKIVGDSCMVYGD
TLDVMHGDLELSSAVVGPIPLDREWGIDKPWIGAGFGLERLLKVKHDFKNIKRAARSESYYNGISTNL* (SEQ
ID NO:518)

LCK::EWSR1::MCP::PylRS(AF)::FUS::PylRS(AF)
DNA:
ATGGGCTGCGTGTGCAGCAGCAACCCCGAGGGTACCGAGCTCATGGCGTCCACGGATTACAGTACCTATAGCCAA
GCTGCAGCGCAGCAGGGCTACAGTGCTTACACCGCCCAGCCCACTCAAGGATATGCACAGACCACCCAGGCATAT
GGGCAACAAAGCTATGGAACCTATGGACAGCCCACTGATGTCAGCTATACCCAGGCTCAGACCACTGCAACCTAT
GGGCAGACCGCCTATGCAACTTCTTATGGACAGCCTCCCACTGGTTATACTACTCCAACTGCCCCCCAGGCATAC
AGCCAGCCTGTCCAGGGGTATGGCACTGGTGCTTATGATACCACCACTGCTACAGTCACCACCACCCAGGCCTCC
TATGCAGCTCAGTCTGCATATGGCACTCAGCCTGCTTATCCAGCCTATGGGCAGCAGCCAGCAGCCACTGCACCT
ACAAGACCGCAGGATGGAAACAAGCCCACTGAGACTAGTCAACCTCAATCTAGCACAGGGGGTTACAACCAGCCC
AGCCTAGGATATGGACAGAGTAACTACAGTTATCCCCAGGTACCTGGGAGCTACCCCATGCAGCCAGTCACTGCA
CCTCCATCCTACCCTCCTACCAGCTATTCCTCTACACAGCCGACTAGTTATGATCAGAGCAGTTACTCTCAGCAG
AACACCTATGGGCAACCGAGCAGCTATGGACAGCAGAGTAGCTATGGTCAACAAAGCAGCTATGGGCAGCAGCCT
CCCACTAGTTACCCACCCCAAACTGGATCCTACAGCCAAGCTCCAAGTCAATATAGCCAACAGAGCAGCAGCTAC
GGGCAGCAGAGTTCATTCCGACAGGACCACCCCAGTAGCATGGGTGTTTATGGGCAGGAGTCTGGAGGATTTTCC
GGACCAGGAGAGAACCGGAGCATGAGTGGCCCTGATAACCGGGGCAGGGGAAGAGGGGGATTTGATCGTGGAGGC
ATGAGCAGAGGTGGGCGGGGAGGAGGACGCGGTGGAATGGGCAGCGCTGGAGAGCGAGGTGGCTTCAATAAGCCT
GGTGGACCCATGGATGAAGGACCAGATCTTGATCTAGGCCCACCTGTAGATCCAGATGAAGACTCTGACAACAGT

GCAATTTATGTACAAGGATTAAATGACAGTGTGACTCTAGATGATCTGGCAGACTTCTTTAAGCAGTGTGGGGTT
GTTAAGATGAACAAGAGAACTGGGCAACCCATGATCCACATCTACCTGGACAAGGAAACAGGAAAGCCCAAAGGC
GATGCCACAGTGTCCTATGAAGACCCACCTACTGCCAAGGCTGCCGTGGAATGGTTTGATGGGAAAGATTTTCAA
GGGAGCAAACTTAAAGTCTCCCTTGCTCGGAAGAAGCCTCCAATGAACAGTATGCGGGGTGGTCTGCCACCCCGT
GAGGGCAGAGGCATGCCACCACCACTCCGTGGAGGTCCAGGAGGCCCAGGAGGTCCTGGGGGACCCATGGGTCGC
ATGGGAGGCCGTGGAGGAGATAGAGGAGGCTTCCCTCCAAGAGGACCCCGGGGTTCCCGAGGGAACCCCTCTGGA
GGAGGAAACGTCCAGCACCGAGCTGGAGACTGGCAGTGTCCCAATCCGGGTTGTGGAAACCAGAACTTCGCCTGG
AGAACAGAGTGCAACCAGTGTAAGGCCCCAAAGCCTGAAGGCTTCCTCCCGCCACCCTTTCCGCCCCCGGGTGGT
GATCGTGGCAGAGGTGGCCCTGGTGGCATGCGGGGAGGAAGAGGTGGCCTCATGGATCGTGGTGGTCCCGGTGGA
ATGTTCAGAGGTGGCCGTGGTGGAGACAGAGGTGGCTTCCGTGGTGGCCGGGGCATGGACCGAGGTGGCTTTGGT
GGAGGAAGACGAGGTGGCCCTGGGGGGCCCCCTGGACCTTTGATGGAACAGGCGATCGCATATCCCTATGATGTG
CCGGATTATGCTGGAGCACCAGGAAGTGCTGGTTCTGCTGCTGGTAGTGGAGCTTCTAACTTTACTCAGTTCGTT
CTCGTCGACAATGGCGGAACTGGCGACGTGACTGTCGCCCCAAGCAACTTCGCTAACGGGATCGCTGAATGGATC
AGCTCTAACTCGCGTTCACAGGCTTACAAAGTAACCTGTAGCGTTCGTCAGAGCTCTGCGCAGAATCGCAAATAC
ACCATCAAAGTCGAGGTGCCTAAAGGCGCCTGGCGTTCGTACTTAAATATGGAACTAACCATTCCAATTTTCGCC
ACGAATTCCGACTGCGAGCTTATTGTTAAGGCAATGCAAGGTCTCCTAAAAGATGGAAACCCGATTCCCTCAGCA
ATCGCAGCAAACTCCGGCATCTACGGGCTAAGCTATACAGATATTGAAATGAACAGATTGGGAAAGGCGTGCCCG
GTGCCGCTGCAGCTGCCGCCGCTGGAACGCCTGACCCTGGATGATAAAAAACCGCTGAATACCCTGATCTCTGCT
ACTGGTCTGTGGATGAGTCGTACCGGAACCATTCATAAAATCAAACACCACGAGGTTAGCCGTTCGAAAATCTAT
ATTGAGATGGCGTGTGGCGATCATCTGGTTGTGAACAATAGCCGCTCTTCTCGTACAGCACGTGCACTGCGTCAC
CACAAATATCGTAAAACCTGTAAACGTTGCCGTGTGTCCGATGAGGATCTGAACAAATTCCTGACAAAAGCCAAT
GAGGACCAAACAAGCGTGAAAGTGAAAGTCGTTAGCGCTCCTACCCGTACTAAAAAAGCAATGCCGAAATCCGTT
GCTCGTGCCCCTAAACCACTGGAAAACACTGAAGCAGCACAGGCACAGCCGTCTGGAAGCAAATTCTCTCCGGCC
ATTCCTGTTTCTACCCAGGAGTCCGTTTCTGTTCCAGCAAGTGTGAGCACCAGCATTAGCAGTATTAGCACCGGT
GCCACCGCTAGCGCCCTGGTTAAAGGCAATACCAATCCGATTACAAGCATGTCTGCCCCGGTTCAAGCATCAGCT
CCAGCACTGACAAAATCCCAAACCGATCGTCTGGAGGTTCTGCTGAATCCGAAAGACGAAATCAGCCTGAATTCC
GGCAAACCGTTTCGTGAACTGGAGAGCGAACTGCTGTCACGTCGTAAAAAAGACCTGCAACAAATCTATGCCGAA
GAACGTGAGAACTATCTGGGGAAACTGGAACGTGAAATCACCCGCTTTTTCGTGGATCGTGGCTTTCTGGAGATC
AAATCCCCGATTCTGATTCCTCTGGAGTATATCGAGCGTATGGGCATCGACAATGATACCGAACTGAGCAAACAA
ATTTTCCGTGTGGATAAAAACTTCTGTCTGCGCCCTATGCTAGCACCAAATCTGGCTAACTATCTGCGCAAACTG
GACCGTGCCCTGCCTGATCCTATCAAAATCTTCGAGATCGGCCCGTGTTATCGTAAAGAGTCCGACGGTAAAGAA
CATCTGGAGGAGTTTACCATGCTGAACTTTTGCCAAATGGGTTCAGGTTGTACTCGTGAGAACCTGGAAAGCATC
ATCACCGATTTTCTGAACCACCTGGGCATTGACTTCAAAATTGTGGGCGACAGCTGTATGGTGTTTGGCGACACC
CTGGATGTCATGCACGGCGACCTGGAACTGTCTAGTGCCGTTGTGGGCCCAATCCCGCTGGATCGTGAGTGGGGT
ATCGACAAACCTTGGATCGGTGCGGGTTTTGGTCTGGAGCGTCTGCTGAAAGTAAAACACGACTTCAAGAACATC
AAACGTGCTGCACGTTCCGAGTCCTATTACAATGGTATTTCTACTAACCTGGCCTCAAACGATTATACCCAACAA
GCAACCCAAAGCTATGGGGCCTACCCCACCCAGCCCGGGCAGGGCTATTCCCAGCAGAGCAGTCAGCCCTACGGA
CAGCAGAGTTACAGTGGTTATAGCCAGTCCACGGACACTTCAGGATATGGCCAGAGCAGCTATTCTTCTTATGGC
CAGAGCCAGAACACAGGCTATGGAACTCAGTCAACTCCCCAGGGATATGGCTCGACTGGCGGCTATGGCAGTAGC
CAGAGCTCCCAATCGTCTTACGGGCAGCAGTCCTCCTACCCTGGCTATGGCCAGCAGCCAGCTCCCAGCAGCACC
TCGGGAAGTTACGGTAGCAGTTCTCAGAGCAGCAGCTATGGGCAGCCCCAGAGTGGGAGCTACAGCCAGCAGCCT
AGCTATGGTGGACAGCAGCAAAGCTATGGACAGCAGCAAAGCTATAATCCCCCTCAGGGCTATGGACAGCAGAAC
CAGTACAACAGCAGCAGTGGTGGTGGAGGTGGAGGTGGAGGTGGAGGTAACTATGGCCAAGATCAATCCTCCATG
AGTAGTGGTGGTGGCAGTGGTGGCGGTTATGGCAATCAAGACCAGAGTGGTGGAGGTGGCAGCGGTGGCTATGGA
CAGCAGGACCGTGGAGGCCGCGGCAGGGGTGGCAGTGGTGGCGGCGGCGGCGGCGGCGGTGGTGGTTACAACCGC
AGCAGTGGTGGCTATGAACCCAGAGGTCGTGGAGGTGGCCGTGGAGGCAGAGGTGGCATGGGCGGAAGTGACCGT
GGTGGCTTCAATAAATTTGGTGGCCCTCGGGACCAAGGATCACGTCATGACTCCGAACAGGATAATTCAGACAAC
AACACCATCTTTGTGCAAGGCCTGGGTGAGAATGTTACAATTGAGTCTGTGGCTGATTACTTCAAGCAGATTGGT
ATTATTAAGACAAACAAGAAAACGGGACAGCCCATGATTAATTTGTACACAGACAGGGAAACTGGCAAGCTGAAG
GGAGAGGCAACGGTCTCTTTTGATGACCCACCTTCAGCTAAAGCAGCTATTGACTGGTTTGATGGTAAAGAATTC
TCCGGAAATCCTATCAAGGTCTCATTTGCTACTCGCCGGGCAGACTTTAATCGGGGTGGTGGCAATGGTCGTGGA
GGCCGAGGGCGAGGAGGACCCATGGGCCGTGGAGGCTATGGAGGTGGTGGCAGTGGTGGTGGTGGCCGAGGAGGA
TTTCCCAGTGGAGGTGGTGGCGGTGGAGGACAGCAGCGAGCTGGTGACTGGAAGTGTCCTAATCCCACCTGTGAG
AATATGAACTTCTCTTGGAGGAATGAATGCAACCAGTGTAAGGCCCCTAAACCAGATGGCCCAGGAGGGGGACCA
GGTGGCTCTCACATGGGGGGTAACTACGGGGATGATCGTCGTGGTGGCAGAGGAGGCGATAAAAAACCGCTGAAT
ACCCTGATCTCTGCTACTGGTCTGTGGATGAGTCGTACCGGAACCATTCATAAAATCAAACACCACGAGGTTAGC
CGTTCGAAAATCTATATTGAGATGGCGTGTGGCGATCATCTGGTTGTGAACAATAGCCGCTCTTCTCGTACAGCA
CGTGCACTGCGTCACCACAAATATCGTAAAACCTGTAAACGTTGCCGTGTGTCCGATGAGGATCTGAACAAATTC

CTGACAAAAGCCAATGAGGACCAAACAAGCGTGAAAGTGAAAGTCGTTAGCGCTCCTACCCGTACTAAAAAAGCA
ATGCCGAAATCCGTTGCTCGTGCCCCTAAACCACTGGAAAACACTGAAGCAGCACAGGCACAGCCGTCTGGAAGC
AAATTCTCTCCGGCCATTCCTGTTTCTACCCAGGAGTCCGTTTCTGTTCCAGCAAGTGTGAGCACCAGCATTAGC
AGTATTAGCACCGGTGCCACCGCTAGCGCCCTGGTTAAAGGCAATACCAATCCGATTACAAGCATGTCTGCCCCG
GTTCAAGCATCAGCTCCAGCACTGACAAAATCCCAAACCGATCGTCTGGAGGTTCTGCTGAATCCGAAAGACGAA
ATCAGCCTGAATTCCGGCAAACCGTTTCGTGAACTGGAGAGCGAACTGCTGTCACGTCGTAAAAAAGACCTGCAA
CAAATCTATGCCGAAGAACGTGAGAACTATCTGGGGAAACTGGAACGTGAAATCACCCGCTTTTTCGTGGATCGT
GGCTTTCTGGAGATCAAATCCCCGATTCTGATTCCTCTGGAGTATATCGAGCGTATGGGCATCGACAATGATACC
GAACTGAGCAAACAAATTTTCCGTGTGGATAAAAACTTCTGTCTGCGCCCTATGCTAGCACCAAATCTGGCTAAC
TATCTGCGCAAACTGGACCGTGCCCTGCCTGATCCTATCAAAATCTTCGAGATCGGCCCGTGTTATCGTAAAGAG
TCCGACGGTAAAGAACATCTGGAGGAGTTTACCATGCTGAACTTTTGCCAAATGGGTTCAGGTTGTACTCGTGAG
AACCTGGAAAGCATCATCACCGATTTTCTGAACCACCTGGGCATTGACTTCAAAATTGTGGGCGACAGCTGTATG
GTGTTTGGCGACACCCTGGATGTCATGCACGGCGACCTGGAACTGTCTAGTGCCGTTGTGGGCCCAATCCCGCTG
GATCGTGAGTGGGGTATCGACAAACCTTGGATCGGTGCGGGTTTTGGTCTGGAGCGTCTGCTGAAAGTAAAACAC
GACTTCAAGAACATCAAACGTGCTGCACGTTCCGAGTCCTATTACAATGGTATTTCTACTAACCTGTAA (SEQ
ID NO: 519)

Protein:
MGCVCSSNPEGTELMASTDYSTYSQAAAQQGYSAYTAQPTQGYAQTTQAYGQQSYGTYGQPTDVSYTQAQTTATY
GQTAYATSYGQPPTGYTTPTAPQAYSQPVQGYGTGAYDTTTATVTTTQASYAAQSAYGTQPAYPAYGQQPAATAP
TRPQDGNKPTETSQPQSSTGGYNQPSLGYGQSNYSYPQVPGSYPMQPVTAPPSYPPTSYSSTQPTSYDQSSYSQQ
NTYGQPSSYGQQSSYGQQSSYGQQPPTSYPPQTGSYSQAPSQYSQQSSSYGQQSSFRQDHPSSMGVYGQESGGFS
GPGENRSMSGPDNRGRGRGGFDRGGMSRGGRGGGRGGMGSAGERGGFNKPGGPMDEGPDLDLGPPVDPDEDSDNS
AIYVQGLNDSVTLDDLADFFKQCGVVKMNKRTGQPMIHIYLDKETGKPKGDATVSYEDPPTAKAAVEWFDGKDFQ
GSKLKVSLARKKPPMNSMRGGLPPREGRGMPPPLRGGPGGPGGPGGPMGRMGGRGGDRGGFPPRGPRGSRGNPSG
GGNVQHRAGDWQCPNPGCGNQNFAWRTECNQCKAPKPEGFLPPPFPPPGGDRGRGGPGGMRGGRGGLMDRGGPGG
MFRGGRGGDRGGFRGGRGMDRGGFGGGRRGGPGGPPGPLMEQAIAYPYDVPDYAGAPGSAGSAAGSGASNFTQFV
LVDNGGTGDVTVAPSNFANGIAEWISSNSRSQAYKVTCSVRQSSAQNRKYTIKVEVPKGAWRSYLNMELTIPIFA
TNSDCELIVKAMQGLLKDGNPIPSAIAANSGIYGLSYTDIEMNRLGKACPVPLQLPPLERLTLDDKKPLNTLISA
TGLWMSRTGTIHKIKHHEVSRSKIYIEMACGDHLVVNNSRSSRTARALRHHKYRKTCKRCRVSDEDLNKFLTKAN
EDQTSVKVKVVSAPTRTKKAMPKSVARAPKPLENTEAAQAQPSGSKFSPAIPVSTQESVSVPASVSTSISSISTG
ATASALVKGNTNPITSMSAPVQASAPALTKSQTDRLEVLLNPKDEISLNSGKPFRELESELLSRRKKDLQQIYAE
ERENYLGKLEREITRFFVDRGFLEIKSPILIPLEYIERMGIDNDTELSKQIFRVDKNFCLRPMLAPNLANYLRKL
DRALPDPIKIFEIGPCYRKESDGKEHLEEFTMLNFCQMGSGCTRENLESIITDFLNHLGIDFKIVGDSCMVFGDT
LDVMHGDLELSSAVVGPIPLDREWGIDKPWIGAGFGLERLLKVKHDFKNIKRAARSESYYNGISTNLASNDYTQQ
ATQSYGAYPTQPGQGYSQQSSQPYGQQSYSGYSQSTDTSGYGQSSYSSYGQSQNTGYGTQSTPQGYGSTGGYGSS
QSSQSSYGQQSSYPGYGQQPAPSSTSGSYGSSSQSSSYGQPQSGSYSQQPSYGGQQQSYGQQQSYNPPQGYGQQN
QYNSSSGGGGGGGGGGNYGQDQSSMSSGGGSGGGYGNQDQSGGGGSGGYGQQDRGGRGRGGSGGGGGGGGGGYNR
SSGGYEPRGRGGGRGGRGGMGGSDRGGFNKFGGPRDQGSRHDSEQDNSDNNTIFVQGLGENVTIESVADYFKQIG
IIKTNKKTGQPMINLYTDRETGKLKGEATVSFDDPPSAKAAIDWFDGKEFSGNPIKVSFATRRADFNRGGGNGRG
GRGRGGPMGRGGYGGGGSGGGGRGGFPSGGGGGGGQQRAGDWKCPNPTCENMNFSWRNECNQCKAPKPDGPGGGP
GGSHMGGNYGDDRRGGRGGDKKPLNTLISATGLWMSRTGTIHKIKHHEVSRSKIYIEMACGDHLVVNNSRSSRTA
RALRHHKYRKTCKRCRVSDEDLNKFLTKANEDQTSVKVKVVSAPTRTKKAMPKSVARAPKPLENTEAAQAQPSGS
KFSPAIPVSTQESVSVPASVSTSISSISTGATASALVKGNTNPITSMSAPVQASAPALTKSQTDRLEVLLNPKDE
ISLNSGKPFRELESELLSRRKKDLQQIYAEERENYLGKLEREITRFFVDRGFLEIKSPILIPLEYIERMGIDNDT
ELSKQIFRVDKNFCLRPMLAPNLANYLRKLDRALPDPIKIFEIGPCYRKESDGKEHLEEFTMLNFCQMGSGCTRE
NLESIITDFLNHLGIDFKIVGDSCMVFGDTLDVMHGDLELSSAVVGPIPLDREWGIDKPWIGAGFGLERLLKVKH
DFKNIKRAARSESYYNGISTNL* (SEQ ID NO: 520)

TOM20::EWSR1::4xλN22::PylRS(AF)::FUS::PylRS(AF)
DNA:
ATGGTGGGTCGGAACAGCGCCATCGCCGCCGGTGTATGCGGGGCCCTTTTCATTGGGTACTGCATCTACTTCGAC
CGCAAAAGACGAAGTGACCCCAACTTCAAGAACAGGCTTCGAGAACGAAGAAAGAAACAGAAGCTTGCCAAGGAG
AGAGCTGGGCTTTCCAAGTTACCTGACCTTAAAGATGCTGAAGCTGTTCAGAAGTTCTTCATGGCGTCCACGGAT
TACAGTACCTATAGCCAAGCTGCAGCGCAGCAGGGCTACAGTGCTTACACCGCCCAGCCCACTCAAGGATATGCA
CAGACCACCCAGGCATATGGGCAACAAAGCTATGGAACCTATGGACAGCCCACTGATGTCAGCTATACCCAGGCT
CAGACCACTGCAACCTATGGGCAGACCGCCTATGCAACTTCTTATGGACAGCCTCCCACTGGTTATACTACTCCA

ACTGCCCCCCAGGCATACAGCCAGCCTGTCCAGGGGTATGGCACTGGTGCTTATGATACCACCACTGCTACAGTC
ACCACCACCCAGGCCTCCTATGCAGCTCAGTCTGCATATGGCACTCAGCCTGCTTATCCAGCCTATGGGCAGCAG
CCAGCAGCCACTGCACCTACAAGACCGCAGGATGGAAACAAGCCCACTGAGACTAGTCAACCTCAATCTAGCACA
GGGGGTTACAACCAGCCCAGCCTAGGATATGGACAGAGTAACTACAGTTATCCCCAGGTACCTGGGAGCTACCCC
ATGCAGCCAGTCACTGCACCTCCATCCTACCCTCCTACCAGCTATTCCTCTACACAGCCGACTAGTTATGATCAG
AGCAGTTACTCTCAGCAGAACACCTATGGGCAACCGAGCAGCTATGGACAGCAGAGTAGCTATGGTCAACAAAGC
AGCTATGGGCAGCAGCCTCCCACTAGTTACCCACCCCAAACTGGATCCTACAGCCAAGCTCCAAGTCAATATAGC
CAACAGAGCAGCAGCTACGGGCAGCAGAGTTCATTCCGACAGGACCACCCCAGTAGCATGGGTGTTTATGGGCAG
GAGTCTGGAGGATTTTCCGGACCAGGAGAGAACCGGAGCATGAGTGGCCCTGATAACCGGGGCAGGGGAAGAGGG
GGATTTGATCGTGGAGGCATGAGCAGAGGTGGGCGGGGAGGAGGACGCGGTGGAATGGGCAGCGCTGGAGAGCGA
GGTGGCTTCAATAAGCCTGGTGGACCCATGGATGAAGGACCAGATCTTGATCTAGGCCCACCTGTAGATCCAGAT
GAAGACTCTGACAACAGTGCAATTTATGTACAAGGATTAAATGACAGTGTGACTCTAGATGATCTGGCAGACTTC
TTTAAGCAGTGTGGGGTTGTTAAGATGAACAAGAGAACTGGGCAACCCATGATCCACATCTACCTGGACAAGGAA
ACAGGAAAGCCCAAAGGCGATGCCACAGTGTCCTATGAAGACCCACCTACTGCCAAGGCTGCCGTGGAATGGTTT
GATGGGAAAGATTTTCAAGGGAGCAAACTTAAAGTCTCCCTTGCTCGGAAGAAGCCTCCAATGAACAGTATGCGG
GGTGGTCTGCCACCCCGTGAGGGCAGAGGCATGCCACCACCACTCCGTGGAGGTCCAGGAGGCCCAGGAGGTCCT
GGGGGACCCATGGGTCGCATGGGAGGCCGTGGAGGAGATAGAGGAGGCTTCCCTCCAAGAGGACCCCGGGGTTCC
CGAGGGAACCCCTCTGGAGGAGGAAACGTCCAGCACCGAGCTGGAGACTGGCAGTGTCCCAATCCGGGTTGTGGA
AACCAGAACTTCGCCTGGAGAACAGAGTGCAACCAGTGTAAGGCCCCAAAGCCTGAAGGCTTCCTCCCGCCACCC
TTTCCGCCCCCGGGTGGTGATCGTGGCAGAGGTGGCCCTGGTGGCATGCGGGGAGGAAGAGGTGGCCTCATGGAT
CGTGGTGGTCCCGGTGGAATGTTCAGAGGTGGCCGTGGTGGAGACAGAGGTGGCTTCCGTGGTGGCCGGGGCATG
GACCGAGGTGGCTTTGGTGGAGGAAGACGAGGTGGCCCTGGGGGGCCCCCTGGACCTTTGATGGAACAGGCGATC
GCAGGAGCACCAGGAAGTGCTGGTTCTGCTGCTGGTAGTGGAGAGCAGAAGCTGATCTCAGAGGAGGACCTGCTA
GCCACCATGGACGCACAAACACGACGACGTGAGCGTCGCGCTGAGAAACAAGCTCAATGGAAAGCTGCAAACCCA
CCGCTCGACGGAGCCGGAGCTGGCGCTGGAGCTGGAGCCGGAGCTGGCGGTCTAGCCACCATGGACGCACAAACA
CGACGACGTGAGCGTCGCGCTGAGAAACAAGCTCAATGGAAAGCTGCAAACCCACCGCTCGACGGAGCCGGAGCT
GGCGCTGGAGCTGGAGCCGGAGCTGGCGGTCTAGCCACCATGGACGCACAAACACGACGACGTGAGCGTCGCGCT
GAGAAACAAGCTCAATGGAAAGCTGCAAACCCACCGCTCGACGGAGCCGGAGCTGGCGCTGGAGCTGGAGCCGGA
GCTGGCGGTCTAGCCACCATGGACGCACAAACACGACGACGTGAGCGTCGCGCTGAGAAACAAGCTCAATGGAAA
GCTGCAAACCCACCGCTCGGGCTAAGCTATACAGATATTGAAATGAACAGATTGGGAAAGGCGTGCCCGGTGCCG
CTGCAGCTGCCGCCGCTGGAACGCCTGACCCTGGATGATAAAAAACCGCTGAATACCCTGATCTCTGCTACTGGT
CTGTGGATGAGTCGTACCGGAACCATTCATAAAATCAAACACCACGAGGTTAGCCGTTCGAAAATCTATATTGAG
ATGGCGTGTGGCGATCATCTGGTTGTGAACAATAGCCGCTCTTCTCGTACAGCACGTGCACTGCGTCACCACAAA
TATCGTAAAACCTGTAAACGTTGCCGTGTGTCCGATGAGGATCTGAACAAATTCCTGACAAAAGCCAATGAGGAC
CAAACAAGCGTGAAAGTGAAAGTCGTTAGCGCTCCTACCCGTACTAAAAAAGCAATGCCGAAATCCGTTGCTCGT
GCCCCTAAACCACTGGAAAACACTGAAGCAGCACAGGCACAGCCGTCTGGAAGCAAATTCTCTCCGGCCATTCCT
GTTTCTACCCAGGAGTCCGTTTCTGTTCCAGCAAGTGTGAGCACCAGCATTAGCAGTATTAGCACCGGTGCCACC
GCTAGCGCCCTGGTTAAAGGCAATACCAATCCGATTACAAGCATGTCTGCCCCGGTTCAAGCATCAGCTCCAGCA
CTGACAAAATCCCAAACCGATCGTCTGGAGGTTCTGCTGAATCCGAAAGACGAAATCAGCCTGAATTCCGGCAAA
CCGTTTCGTGAACTGGAGAGCGAACTGCTGTCACGTCGTAAAAAAGACCTGCAACAAATCTATGCCGAAGAACGT
GAGAACTATCTGGGGAAACTGGAACGTGAAATCACCCGCTTTTTCGTGGATCGTGGCTTTCTGGAGATCAAATCC
CCGATTCTGATTCCTCTGGAGTATATCGAGCGTATGGGCATCGACAATGATACCGAACTGAGCAAACAAATTTTC
CGTGTGGATAAAAACTTCTGTCTGCGCCCTATGCTAGCACCAAATCTGGCTAACTATCTGCGCAAACTGGACCGT
GCCCTGCCTGATCCTATCAAAATCTTCGAGATCGGCCCGTGTTATCGTAAAGAGTCCGACGGTAAAGAACATCTG
GAGGAGTTTACCATGCTGAACTTTTGCCAAATGGGTTCAGGTTGTACTCGTGAGAACCTGGAAAGCATCATCACC
GATTTTCTGAACCACCTGGGCATTGACTTCAAAATTGTGGGCGACAGCTGTATGGTGTTTGGCGACACCCTGGAT
GTCATGCACGGCGACCTGGAACTGTCTAGTGCCGTTGTGGGCCCAATCCCGCTGGATCGTGAGTGGGGTATCGAC
AAACCTTGGATCGGTGCGGGTTTTGGTCTGGAGCGTCTGCTGAAAGTAAAACACGACTTCAAGAACATCAAACGT
GCTGCACGTTCCGAGTCCTATTACAATGGTATTTCTACTAACCTGGCCTCAAACGATTATACCCAACAAGCAACC
CAAAGCTATGGGGCCTACCCCACCCAGCCCGGGCAGGGCTATTCCCAGCAGAGCAGTCAGCCCTACGGACAGCAG
AGTTACAGTGGTTATAGCCAGTCCACGGACACTTCAGGATATGGCCAGAGCAGCTATTCTTCTTATGGCCAGAGC
CAGAACACAGGCTATGGAACTCAGTCAACTCCCCAGGGATATGGCTCGACTGGCGGCTATGGCAGTAGCCAGAGC
TCCCAATCGTCTTACGGGCAGCAGTCCTCCTACCCTGGCTATGGCCAGCAGCCAGCTCCCAGCAGCACCTCGGGA
AGTTACGGTAGCAGTTCTCAGAGCAGCAGCTATGGGCAGCCCCAGAGTGGGAGCTACAGCCAGCAGCCTAGCTAT
GGTGGACAGCAGCAAAGCTATGGACAGCAGCAAAGCTATAATCCCCCTCAGGGCTATGGACAGCAGAACCAGTAC
AACAGCAGCAGTGGTGGTGGAGGTGGAGGTGGAGGTGGAGGTAACTATGGCCAAGATCAATCCTCCATGAGTAGT
GGTGGTGGCAGTGGTGGCGGTTATGGCAATCAAGACCAGAGTGGTGGAGGTGGCAGCGGTGGCTATGGACAGCAG
GACCGTGGAGGCCGCGGCAGGGGTGGCAGTGGTGGCGGCGGCGGCGGCGGTGGTGGTTACAACCGCAGCAGT

GGTGGCTATGAACCCAGAGGTCGTGGAGGTGGCCGTGGAGGCAGAGGTGGCATGGGCGGAAGTGACCGTGGTGGC
TTCAATAAATTTGGTGGCCCTCGGGACCAAGGATCACGTCATGACTCCGAACAGGATAATTCAGACAACAACACC
ATCTTTGTGCAAGGCCTGGGTGAGAATGTTACAATTGAGTCTGTGGCTGATTACTTCAAGCAGATTGGTATTATT
AAGACAAACAAGAAAACGGGACAGCCCATGATTAATTTGTACACAGACAGGGAAACTGGCAAGCTGAAGGGAGAG
GCAACGGTCTCTTTTGATGACCCACCTTCAGCTAAAGCAGCTATTGACTGGTTTGATGGTAAAGAATTCTCCGGA
AATCCTATCAAGGTCTCATTTGCTACTCGCCGGGCAGACTTTAATCGGGGTGGTGGCAATGGTCGTGGAGGCCGA
GGGCGAGGAGGACCCATGGGCCGTGGAGGCTATGGAGGTGGTGGCAGTGGTGGTGGTGGCCGAGGAGGATTTCCC
AGTGGAGGTGGTGGCGGTGGAGGACAGCAGCGAGCTGGTGACTGGAAGTGTCCTAATCCCACCTGTGAGAATATG
AACTTCTCTTGGAGGAATGAATGCAACCAGTGTAAGGCCCCTAAACCAGATGGCCCAGGAGGGGGACCAGGTGGC
TCTCACATGGGGGGTAACTACGGGGATGATCGTCGTGGTGGCAGAGGAGGCGATAAAAAACCGCTGAATACCCTG
ATCTCTGCTACTGGTCTGTGGATGAGTCGTACCGGAACCATTCATAAAATCAAACACCACGAGGTTAGCCGTTCG
AAAATCTATATTGAGATGGCGTGTGGCGATCATCTGGTTGTGAACAATAGCCGCTCTTCTCGTACAGCACGTGCA
CTGCGTCACCACAAATATCGTAAAACCTGTAAACGTTGCCGTGTGTCCGATGAGGATCTGAACAAATTCCTGACA
AAAGCCAATGAGGACCAAACAAGCGTGAAAGTGAAAGTCGTTAGCGCTCCTACCCGTACTAAAAAAGCAATGCCG
AAATCCGTTGCTCGTGCCCCTAAACCACTGGAAAACACTGAAGCAGCACAGGCACAGCCGTCTGGAAGCAAATTC
TCTCCGGCCATTCCTGTTTCTACCCAGGAGTCCGTTTCTGTTCCAGCAAGTGTGAGCACCAGCATTAGCAGTATT
AGCACCGGTGCCACCGCTAGCGCCCTGGTTAAAGGCAATACCAATCCGATTACAAGCATGTCTGCCCCGGTTCAA
GCATCAGCTCCAGCACTGACAAAATCCCAAACCGATCGTCTGGAGGTTCTGCTGAATCCGAAAGACGAAATCAGC
CTGAATTCCGGCAAACCGTTTCGTGAACTGGAGAGCGAACTGCTGTCACGTCGTAAAAAAGACCTGCAACAAATC
TATGCCGAAGAACGTGAGAACTATCTGGGGAAACTGGAACGTGAAATCACCCGCTTTTTCGTGGATCGTGGCTTT
CTGGAGATCAAATCCCCGATTCTGATTCCTCTGGAGTATATCGAGCGTATGGGCATCGACAATGATACCGAACTG
AGCAAACAAATTTTCCGTGTGGATAAAAACTTCTGTCTGCGCCCTATGCTAGCACCAAATCTGGCTAACTATCTG
CGCAAACTGGACCGTGCCCTGCCTGATCCTATCAAAATCTTCGAGATCGGCCCGTGTTATCGTAAAGAGTCCGAC
GGTAAAGAACATCTGGAGGAGTTTACCATGCTGAACTTTTGCCAAATGGGTTCAGGTTGTACTCGTGAGAACCTG
GAAAGCATCATCACCGATTTTCTGAACCACCTGGGCATTGACTTCAAAATTGTGGGCGACAGCTGTATGGTGTTT
GGCGACACCCTGGATGTCATGCACGGCGACCTGGAACTGTCTAGTGCCGTTGTGGGCCCAATCCCGCTGGATCGT
GAGTGGGGTATCGACAAACCTTGGATCGGTGCGGGTTTTGGTCTGGAGCGTCTGCTGAAAGTAAAACACGACTTC
AAGAACATCAAACGTGCTGCACGTTCCGAGTCCTATTACAATGGTATTTCTACTAACCTGTAA (SEQ ID NO:
521)

Protein:
MVGRNSAIAAGVCGALFIGYCIYFDRKRRSDPNFKNRLRERRKKQKLAKERAGLSKLPDLKDAEAVQKFFMASTD
YSTYSQAAAQQGYSAYTAQPTQGYAQTTQAYGQQSYGTYGQPTDVSYTQAQTTATYGQTAYATSYGQPPTGYTTP
TAPQAYSQPVQGYGTGAYDTTTATVTTTQASYAAQSAYGTQPAYPAYGQQPAATAPTRPQDGNKPTETSQPQSST
GGYNQPSLGYGQSNYSYPQVPGSYPMQPVTAPPSYPPTSYSSTQPTSYDQSSYSQQNTYGQPSSYGQQSSYGQQS
SYGQQPPTSYPPQTGSYSQAPSQYSQQSSSYGQQSSFRQDHPSSMGVYGQESGGFSGPGENRSMSGPDNRGRGRG
GFDRGGMSRGGRGGGRGGMGSAGERGGFNKPGGPMDEGPDLDLGPPVDPDEDSDNSAIYVQGLNDSVTLDDLADF
FKQCGVVKMNKRTGQPMIHIYLDKETGKPKGDATVSYEDPPTAKAAVEWFDGKDFQGSKLKVSLARKKPPMNSMR
GGLPPREGRGMPPPLRGGPGGPGGPGGPMGRMGGRGGDRGGFPPRGPRGSRGNPSGGGNVQHRAGDWQCPNPGCG
NQNFAWRTECNQCKAPKPEGFLPPPFPPPGGDRGRGGPGGMRGGRGGLMDRGGPGGMFRGGRGGDRGGFRGGRGM
DRGGFGGGRRGGPGGPPGPLMEQAIAGAPGSAGSAAGSGEQKLISEEDLLATMDAQTRRRERRAEKQAQWKAANP
PLDGAGAGAGAGAGAGGLATMDAQTRRRERRAEKQAQWKAANPPLDGAGAGAGAGAGAGGLATMDAQTRRRERRA
EKQAQWKAANPPLDGAGAGAGAGAGAGGLATMDAQTRRRERRAEKQAQWKAANPPLGLSYTDIEMNRLGKACPVP
LQLPPLERLTLDDKKPLNTLISATGLWMSRTGTIHKIKHHEVSRSKIYIEMACGDHLVVNNSRSSRTARALRHHK
YRKTCKRCRVSDEDLNKFLTKANEDQTSVKVKVVSAPTRTKKAMPKSVARAPKPLENTEAAQAQPSGSKFSPAIP
VSTQESVSVPASVSTSISSISTGATASALVKGNTNPITSMSAPVQASAPALTKSQTDRLEVLLNPKDEISLNSGK
PFRELESELLSRRKKDLQQIYAEERENYLGKLEREITRFFVDRGFLEIKSPILIPLEYIERMGIDNDTELSKQIF
RVDKNFCLRPMLAPNLANYLRKLDRALPDPIKIFEIGPCYRKESDGKEHLEEFTMLNFCQMGSGCTRENLESIIT
DFLNHLGIDFKIVGDSCMVFGDTLDVMHGDLELSSAVVGPIPLDREWGIDKPWIGAGFGLERLLKVKHDFKNIKR
AARSESYYNGISTNLASNDYTQQATQSYGAYPTQPGQGYSQQSSQPYGQQSYSGYSQSTDTSGYGQSSYSSYGQS
QNTGYGTQSTPQGYGSTGGYGSSQSSQSSYGQQSSYPGYGQQPAPSSTSGSYGSSSQSSSYGQPQSGSYSQQPSY
GGQQQSYGQQQSYNPPQGYGQQNQYNSSSGGGGGGGGGGNYGQDQSSMSSGGGSGGGYGNQDQSGGGGSGGYGQQ
DRGGRGRGGSGGGGGGGGGGYNRSSGGYEPRGRGGGRGGRGGMGGSDRGGFNKFGGPRDQGSRHDSEQDNSDNNT
IFVQGLGENVTIESVADYFKQIGIIKTNKKTGQPMINLYTDRETGKLKGEATVSFDDPPSAKAAIDWFDGKEFSG
NPIKVSFATRRADFNRGGGNGRGGRGRGGPMGRGGYGGGGSGGGGRGGFPSGGGGGGGQQRAGDWKCPNPTCENM
NFSWRNECNQCKAPKPDGPGGGPGGSHMGGNYGDDRRGGRGGDKKPLNTLISATGLWMSRTGTIHKIKHHEVSRS
KIYIEMACGDHLVVNNSRSSRTARALRHHKYRKTCKRCRVSDEDLNKFLTKANEDQTSVKVKVVSAPTRTKKAMP
KSVARAPKPLENTEAAQAQPSGSKFSPAIPVSTQESVSVPASVSTSISSISTGATASALVKGNTNPITSMSAPVQ

ASAPALTKSQTDRLEVLLNPKDEISLNSGKPFRELESELLSRRKKDLQQIYAEERENYLGKLEREITRFFVDRGF
LEIKSPILIPLEYIERMGIDNDTELSKQIFRVDKNFCLRPMLAPNLANYLRKLDRALPDPIKIFEIGPCYRKESD
GKEHLEEFTMLNFCQMGSGCTRENLESIITDFLNHLGIDFKIVGDSCMVFGDTLDVMHGDLELSSAVVGPIPLDR
EWGIDKPWIGAGFGLERLLKVKHDFKNIKRAARSESYYNGISTNL* (SEQ ID NO: 522)

TOM20::EWSR1::MCP::PylRS(AF)::FUS::PylRS(AF)
DNA:
ATGGTGGGTCGGAACAGCGCCATCGCCGCCGGTGTATGCGGGGCCCTTTTCATTGGGTACTGCATCTACTTCGAC
CGCAAAAGACGAAGTGACCCCAACTTCAAGAACAGGCTTCGAGAACGAAGAAAGAAACAGAAGCTTGCCAAGGAG
AGAGCTGGGCTTTCCAAGTTACCTGACCTTAAAGATGCTGAAGCTGTTCAGAAGTTCTTCATGGCGTCCACGGAT
TACAGTACCTATAGCCAAGCTGCAGCGCAGCAGGGCTACAGTGCTTACACCGCCCAGCCCACTCAAGGATATGCA
CAGACCACCCAGGCATATGGGCAACAAAGCTATGGAACCTATGGACAGCCCACTGATGTCAGCTATACCCAGGCT
CAGACCACTGCAACCTATGGGCAGACCGCCTATGCAACTTCTTATGGACAGCCTCCCACTGGTTATACTACTCCA
ACTGCCCCCCAGGCATACAGCCAGCCTGTCCAGGGGTATGGCACTGGTGCTTATGATACCACCACTGCTACAGTC
ACCACCACCCAGGCCTCCTATGCAGCTCAGTCTGCATATGGCACTCAGCCTGCTTATCCAGCCTATGGGCAGCAG
CCAGCAGCCACTGCACCTACAAGACCGCAGGATGGAAACAAGCCCACTGAGACTAGTCAACCTCAATCTAGCACA
GGGGGTTACAACCAGCCCAGCCTAGGATATGGACAGAGTAACTACAGTTATCCCCAGGTACCTGGGAGCTACCCC
ATGCAGCCAGTCACTGCACCTCCATCCTACCCTCCTACCAGCTATTCCTCTACACAGCCGACTAGTTATGATCAG
AGCAGTTACTCTCAGCAGAACACCTATGGGCAACCGAGCAGCTATGGACAGCAGAGTAGCTATGGTCAACAAAGC
AGCTATGGGCAGCAGCCTCCCACTAGTTACCCACCCCAAACTGGATCCTACAGCCAAGCTCCAAGTCAATATAGC
CAACAGAGCAGCAGCTACGGGCAGCAGAGTTCATTCCGACAGGACCACCCCAGTAGCATGGGTGTTTATGGGCAG
GAGTCTGGAGGATTTTCCGGACCAGGAGAGAACCGGAGCATGAGTGGCCCTGATAACCGGGGCAGGGGAAGAGGG
GGATTTGATCGTGGAGGCATGAGCAGAGGTGGGCGGGGAGGAGGACGCGGTGGAATGGGCAGCGCTGGAGAGCGA
GGTGGCTTCAATAAGCCTGGTGGACCCATGGATGAAGGACCAGATCTTGATCTAGGCCCACCTGTAGATCCAGAT
GAAGACTCTGACAACAGTGCAATTTATGTACAAGGATTAAATGACAGTGTGACTCTAGATGATCTGGCAGACTTC
TTTAAGCAGTGTGGGGTTGTTAAGATGAACAAGAGAACTGGGCAACCCATGATCCACATCTACCTGGACAAGGAA
ACAGGAAAGCCCAAAGGCGATGCCACAGTGTCCTATGAAGACCCACCTACTGCCAAGGCTGCCGTGGAATGGTTT
GATGGGAAAGATTTTCAAGGGAGCAAACTTAAAGTCTCCCTTGCTCGGAAGAAGCCTCCAATGAACAGTATGCGG
GGTGGTCTGCCACCCCGTGAGGGCAGAGGCATGCCACCACCACTCCGTGGAGGTCCAGGAGGCCCAGGAGGTCCT
GGGGGACCCATGGGTCGCATGGGAGGCCGTGGAGGAGATAGAGGAGGCTTCCCTCCAAGAGGACCCCGGGGTTCC
CGAGGGAACCCCTCTGGAGGAGGAAACGTCCAGCACCGAGCTGGAGACTGGCAGTGTCCCAATCCGGGTTGTGGA
AACCAGAACTTCGCCTGGAGAACAGAGTGCAACCAGTGTAAGGCCCCAAAGCCTGAAGGCTTCCTCCCGCCACCC
TTTCCGCCCCCGGGTGGTGATCGTGGCAGAGGTGGCCCTGGTGGCATGCGGGGAGGAAGAGGTGGCCTCATGGAT
CGTGGTGGTCCCGGTGGAATGTTCAGAGGTGGCCGTGGTGGAGACAGAGGTGGCTTCCGTGGTGGCCGGGGCATG
GACCGAGGTGGCTTTGGTGGAGGAAGACGAGGTGGCCCTGGGGGGCCCCCTGGACCTTTGATGGAACAGGCGATC
GCATATCCCTATGATGTGCCGGATTATGCTGGAGCACCAGGAAGTGCTGGTTCTGCTGCTGGTAGTGGAGCTTCT
AACTTTACTCAGTTCGTTCTCGTCGACAATGGCGGAACTGGCGACGTGACTGTCGCCCCAAGCAACTTCGCTAAC
GGGATCGCTGAATGGATCAGCTCTAACTCGCGTTCACAGGCTTACAAAGTAACCTGTAGCGTTCGTCAGAGCTCT
GCGCAGAATCGCAAATACACCATCAAAGTCGAGGTGCCTAAAGGCGCCTGGCGTTCGTACTTAAATATGGAACTA
ACCATTCCAATTTTCGCCACGAATTCCGACTGCGAGCTTATTGTTAAGGCAATGCAAGGTCTCCTAAAAGATGGA
AACCCGATTCCCTCAGCAATCGCAGCAAACTCCGGCATCTACGGGCTAAGCTATACAGATATTGAAATGAACAGA
TTGGGAAAGGCGTGCCCGGTGCCGCTGCAGCTGCCGCCGCTGGAACGCCTGACCCTGGATGATAAAAAACCGCTG
AATACCCTGATCTCTGCTACTGGTCTGTGGATGAGTCGTACCGGAACCATTCATAAAATCAAACACCACGAGGTT
AGCCGTTCGAAAATCTATATTGAGATGGCGTGTGGCGATCATCTGGTTGTGAACAATAGCCGCTCTTCTCGTACA
GCACGTGCACTGCGTCACCACAAATATCGTAAAACCTGTAAACGTTGCCGTGTGTCCGATGAGGATCTGAACAAA
TTCCTGACAAAAGCCAATGAGGACCAAACAAGCGTGAAAGTGAAAGTCGTTAGCGCTCCTACCCGTACTAAAAAA
GCAATGCCGAAATCCGTTGCTCGTGCCCCTAAACCACTGGAAAACACTGAAGCAGCACAGGCACAGCCGTCTGGA
AGCAAATTCTCTCCGGCCATTCCTGTTTCTACCCAGGAGTCCGTTTCTGTTCCAGCAAGTGTGAGCACCAGCATT
AGCAGTATTAGCACCGGTGCCACCGCTAGCGCCCTGGTTAAAGGCAATACCAATCCGATTACAAGCATGTCTGCC
CCGGTTCAAGCATCAGCTCCAGCACTGACAAAATCCCAAACCGATCGTCTGGAGGTTCTGCTGAATCCGAAAGAC
GAAATCAGCCTGAATTCCGGCAAACCGTTTCGTGAACTGGAGAGCGAACTGCTGTCACGTCGTAAAAAAGACCTG
CAACAAATCTATGCCGAAGAACGTGAGAACTATCTGGGGAAACTGGAACGTGAAATCACCCGCTTTTTCGTGGAT
CGTGGCTTTCTGGAGATCAAATCCCCGATTCTGATTCCTCTGGAGTATATCGAGCGTATGGGCATCGACAATGAT
ACCGAACTGAGCAAACAAATTTTCCGTGTGGATAAAAACTTCTGTCTGCGCCCTATGCTAGCACCAAATCTGGCT
AACTATCTGCGCAAACTGGACCGTGCCCTGCCTGATCCTATCAAAATCTTCGAGATCGGCCCGTGTTATCGTAAA
GAGTCCGACGGTAAAGAACATCTGGAGGAGTTTACCATGCTGAACTTTTGCCAAATGGGTTCAGGTTGTACTCGT
GAGAACCTGGAAAGCATCATCACCGATTTTCTGAACCACCTGGGCATTGACTTCAAAATTGTGGGCGACAGCTGT

ATGGTGTTTGGCGACACCCTGGATGTCATGCACGGCGACCTGGAACTGTCTAGTGCCGTTGTGGGCCCAATCCCG
CTGGATCGTGAGTGGGGTATCGACAAACCTTGGATCGGTGCGGGTTTTGGTCTGGAGCGTCTGCTGAAAGTAAAA
CACGACTTCAAGAACATCAAACGTGCTGCACGTTCCGAGTCCTATTACAATGGTATTTCTACTAACCTGGCCTCA
AACGATTATACCCAACAAGCAACCCAAAGCTATGGGGCCTACCCCACCCAGCCCGGGCAGGGCTATTCCCAGCAG
AGCAGTCAGCCCTACGGACAGCAGAGTTACAGTGGTTATAGCCAGTCCACGGACACTTCAGGATATGGCCAGAGC
AGCTATTCTTCTTATGGCCAGAGCCAGAACACAGGCTATGGAACTCAGTCAACTCCCCAGGGATATGGCTCGACT
GGCGGCTATGGCAGTAGCCAGAGCTCCCAATCGTCTTACGGGCAGCAGTCCTCCTACCCTGGCTATGGCCAGCAG
CCAGCTCCCAGCAGCACCTCGGGAAGTTACGGTAGCAGTTCTCAGAGCAGCAGCTATGGGCAGCCCCAGAGTGGG
AGCTACAGCCAGCAGCCTAGCTATGGTGGACAGCAGCAAAGCTATGGACAGCAGCAAAGCTATAATCCCCCTCAG
GGCTATGGACAGCAGAACCAGTACAACAGCAGCAGTGGTGGTGGAGGTGGAGGTGGAGGTGGAGGTAACTATGGC
CAAGATCAATCCTCCATGAGTAGTGGTGGTGGCAGTGGTGGCGGTTATGGCAATCAAGACCAGAGTGGTGGAGGT
GGCAGCGGTGGCTATGGACAGCAGGACCGTGGAGGCCGCGGCAGGGGTGGCAGTGGTGGCGGCGGCGGCGGCGGC
GGTGGTGGTTACAACCGCAGCAGTGGTGGCTATGAACCCAGAGGTCGTGGAGGTGGCCGTGGAGGCAGAGGTGGC
ATGGGCGGAAGTGACCGTGGTGGCTTCAATAAATTTGGTGGCCCTCGGGACCAAGGATCACGTCATGACTCCGAA
CAGGATAATTCAGACAACAACACCATCTTTGTGCAAGGCCTGGGTGAGAATGTTACAATTGAGTCTGTGGCTGAT
TACTTCAAGCAGATTGGTATTATTAAGACAAACAAGAAAACGGGACAGCCCATGATTAATTTGTACACAGACAGG
GAAACTGGCAAGCTGAAGGGAGAGGCAACGGTCTCTTTTGATGACCCACCTTCAGCTAAAGCAGCTATTGACTGG
TTTGATGGTAAAGAATTCTCCGGAAATCCTATCAAGGTCTCATTTGCTACTCGCCGGGCAGACTTTAATCGGGGT
GGTGGCAATGGTCGTGGAGGCCGAGGGCGAGGAGGACCCATGGGCCGTGGAGGCTATGGAGGTGGTGGCAGTGGT
GGTGGTGGCCGAGGAGGATTTCCCAGTGGAGGTGGTGGCGGTGGAGGACAGCAGCGAGCTGGTGACTGGAAGTGT
CCTAATCCCACCTGTGAGAATATGAACTTCTCTTGGAGGAATGAATGCAACCAGTGTAAGGCCCCTAAACCAGAT
GGCCCAGGAGGGGGACCAGGTGGCTCTCACATGGGGGGTAACTACGGGGATGATCGTCGTGGTGGCAGAGGAGGC
GATAAAAAACCGCTGAATACCCTGATCTCTGCTACTGGTCTGTGGATGAGTCGTACCGGAACCATTCATAAAATC
AAACACCACGAGGTTAGCCGTTCGAAAATCTATATTGAGATGGCGTGTGGCGATCATCTGGTTGTGAACAATAGC
CGCTCTTCTCGTACAGCACGTGCACTGCGTCACCACAAATATCGTAAAACCTGTAAACGTTGCCGTGTGTCCGAT
GAGGATCTGAACAAATTCCTGACAAAAGCCAATGAGGACCAAACAAGCGTGAAAGTGAAAGTCGTTAGCGCTCCT
ACCCGTACTAAAAAAGCAATGCCGAAATCCGTTGCTCGTGCCCCTAAACCACTGGAAAACACTGAAGCAGCACAG
GCACAGCCGTCTGGAAGCAAATTCTCTCCGGCCATTCCTGTTTCTACCCAGGAGTCCGTTTCTGTTCCAGCAAGT
GTGAGCACCAGCATTAGCAGTATTAGCACCGGTGCCACCGCTAGCGCCCTGGTTAAAGGCAATACCAATCCGATT
ACAAGCATGTCTGCCCCGGTTCAAGCATCAGCTCCAGCACTGACAAAATCCCAAACCGATCGTCTGGAGGTTCTG
CTGAATCCGAAAGACGAAATCAGCCTGAATTCCGGCAAACCGTTTCGTGAACTGGAGAGCGAACTGCTGTCACGT
CGTAAAAAAGACCTGCAACAAATCTATGCCGAAGAACGTGAGAACTATCTGGGGAAACTGGAACGTGAAATCACC
CGCTTTTTCGTGGATCGTGGCTTTCTGGAGATCAAATCCCCGATTCTGATTCCTCTGGAGTATATCGAGCGTATG
GGCATCGACAATGATACCGAACTGAGCAAACAAATTTTCCGTGTGGATAAAAACTTCTGTCTGCGCCCTATGCTA
GCACCAAATCTGGCTAACTATCTGCGCAAACTGGACCGTGCCCTGCCTGATCCTATCAAAATCTTCGAGATCGGC
CCGTGTTATCGTAAAGAGTCCGACGGTAAAGAACATCTGGAGGAGTTTACCATGCTGAACTTTTGCCAAATGGGT
TCAGGTTGTACTCGTGAGAACCTGGAAAGCATCATCACCGATTTTCTGAACCACCTGGGCATTGACTTCAAAATT
GTGGGCGACAGCTGTATGGTGTTTGGCGACACCCTGGATGTCATGCACGGCGACCTGGAACTGTCTAGTGCCGTT
GTGGGCCCAATCCCGCTGGATCGTGAGTGGGGTATCGACAAACCTTGGATCGGTGCGGGTTTTGGTCTGGAGCGT
CTGCTGAAAGTAAAACACGACTTCAAGAACATCAAACGTGCTGCACGTTCCGAGTCCTATTACAATGGTATTTCT
ACTAACCTGTAA (SEQ ID NO: 523)

Protein:
MVGRNSAIAAGVCGALFIGYCIYFDRKRRSDPNFKNRLRERRKKQKLAKERAGLSKLPDLKDAEAVQKFFMASTD
YSTYSQAAAQQGYSAYTAQPTQGYAQTTQAYGQQSYGTYGQPTDVSYTQAQTTATYGQTAYATSYGQPPTGYTTP
TAPQAYSQPVQGYGTGAYDTTTATVTTTQASYAAQSAYGTQPAYPAYGQQPAATAPTRPQDGNKPTETSQPQSST
GGYNQPSLGYGQSNYSYPQVPGSYPMQPVTAPPSYPPTSYSSTQPTSYDQSSYSQQNTYGQPSSYGQQSSYGQQS
SYGQQPPTSYPPQTGSYSQAPSQYSQQSSSYGQQSSFRQDHPSSMGVYGQESGGFSGPGENRSMSGPDNRGRGRG
GFDRGGMSRGGRGGGRGGMGSAGERGGFNKPGGPMDEGPDLDLGPPVDPDEDSDNSAIYVQGLNDSVTLDDLADF
FKQCGVVKMNKRTGQPMIHIYLDKETGKPKGDATVSYEDPPTAKAAVEWFDGKDFQGSKLKVSLARKKPPMNSMR
GGLPPREGRGMPPPLRGGPGGPGGPGGPMGRMGGRGGDRGGFPPRGPRGSRGNPSGGGNVQHRAGDWQCPNPGCG
NQNFAWRTECNQCKAPKPEGFLPPPFPPPGGDRGRGGPGGMRGGRGGLMDRGGPGGMFRGGRGGDRGGFRGGRGM
DRGGFGGGRRGGPGGPPGPLMEQAIAYPYDVPDYAGAPGSAGSAAGSGASNFTQFVLVDNGGTGDVTVAPSNFAN
GIAEWISSNSRSQAYKVTCSVRQSSAQNRKYTIKVEVPKGAWRSYLNMELTIPIFATNSDCELIVKAMQGLLKDG
NPIPSAIAANSGIYGLSYTDIEMNRLGKACPVPLQLPPLERLTLDDKKPLNTLISATGLWMSRTGTIHKIKHHEV
SRSKIYIEMACGDHLVVNNSRSSRTARALRHHKYRKTCKRCRVSDEDLNKFLTKANEDQTSVKVKVVSAPTRTKK
AMPKSVARAPKPLENTEAAQAQPSGSKFSPAIPVSTQESVSVPASVSTSISSISTGATASALVKGNTNPITSMSA
PVQASAPALTKSQTDRLEVLLNPKDEISLNSGKPFRELESELLSRRKKDLQQIYAEERENYLGKLEREITRFFVD

RGFLEIKSPILIPLEYIERMGIDNDTELSKQIFRVDKNFCLRPMLAPNLANYLRKLDRALPDPIKIFEIGPCYRK
ESDGKEHLEEFTMLNFCQMGSGCTRENLESIITDFLNHLGIDFKIVGDSCMVFGDTLDVMHGDLELSSAVVGPIP
LDREWGIDKPWIGAGFGLERLLKVKHDFKNIKRAARSESYYNGISTNLASNDYTQQATQSYGAYPTQPGQGYSQQ
SSQPYGQQSYSGYSQSTDTSGYGQSSYSSYGQSQNTGYGTQSTPQGYGSTGGYGSSQSSQSSYGQQSSYPGYGQQ
PAPSSTSGSYGSSSQSSSYGQPQSGSYSQQPSYGGQQQSYGQQQSYNPPQGYGQQNQYNSSSGGGGGGGGGGNYG
QDQSSMSSGGGSGGGYGNQDQSGGGGSGGYGQQDRGGRGRGGSGGGGGGGGGGYNRSSGGYEPRGRGGGRGGRGG
MGGSDRGGFNKFGGPRDQGSRHDSEQDNSDNNTIFVQGLGENVTIESVADYFKQIGIIKTNKKTGQPMINLYTDR
ETGKLKGEATVSFDDPPSAKAAIDWFDGKEFSGNPIKVSFATRRADFNRGGGNGRGGRGRGGPMGRGGYGGGGSG
GGGRGGFPSGGGGGGGQQRAGDWKCPNPTCENMNFSWRNECNQCKAPKPDGPGGGPGGSHMGGNYGDDRRGGRGG
DKKPLNTLISATGLWMSRTGTIHKIKHHEVSRSKIYIEMACGDHLVVNNSRSSRTARALRHHKYRKTCKRCRVSD
EDLNKFLTKANEDQTSVKVKVVSAPTRTKKAMPKSVARAPKPLENTEAAQAQPSGSKFSPAIPVSTQESVSVPAS
VSTSISSISTGATASALVKGNTNPITSMSAPVQASAPALTKSQTDRLEVLLNPKDEISLNSGKPFRELESELLSR
RKKDLQQIYAEERENYLGKLEREITRFFVDRGFLEIKSPILIPLEYIERMGIDNDTELSKQIFRVDKNFCLRPML
APNLANYLRKLDRALPDPIKIFEIGPCYRKESDGKEHLEEFTMLNFCQMGSGCTRENLESIITDFLNHLGIDFKI
VGDSCMVFGDTLDVMHGDLELSSAVVGPIPLDREWGIDKPWIGAGFGLERLLKVKHDFKNIKRAARSESYYNGIS
TNL* (SEQ ID NO: 524)

LCK::PylRS(AA)::FUS::PylRS(AA)
DNA:
ATGGGCTGCGTGTGCAGCAGCAACCCCGAGGGTACCGAGCTCGCGTGCCCGGTGCCGCTGCAGCTGCCGCCGCTG
GAACGCCTGACCCTGGATGATAAAAAACCGCTGAATACCCTGATCTCTGCTACTGGTCTGTGGATGAGTCGTACC
GGAACCATTCATAAAATCAAACACCACGAGGTTAGCCGTTCGAAAATCTATATTGAGATGGCGTGTGGCGATCAT
CTGGTTGTGAACAATAGCCGCTCTTCTCGTACAGCACGTGCACTGCGTCACCACAAATATCGTAAAACCTGTAAA
CGTTGCCGTGTGTCCGATGAGGATCTGAACAAATTCCTGACAAAAGCCAATGAGGACCAAACAAGCGTGAAAGTG
AAAGTCGTTAGCGCTCCTACCCGTACTAAAAAAGCAATGCCGAAATCCGTTGCTCGTGCCCCTAAACCACTGGAA
AACACTGAAGCAGCACAGGCACAGCCGTCTGGAAGCAAATTCTCTCCGGCCATTCCTGTTTCTACCCAGGAGTCC
GTTTCTGTTCCAGCAAGTGTGAGCACCAGCATTAGCAGTATTAGCACCGGTGCCACCGCTAGCGCCCTGGTTAAA
GGCAATACCAATCCGATTACAAGCATGTCTGCCCCGGTTCAAGCATCAGCTCCAGCACTGACAAAATCCCAAACC
GATCGTCTGGAGGTTCTGCTGAATCCGAAAGACGAAATCAGCCTGAATTCCGGCAAACCGTTTCGTGAACTGGAG
AGCGAACTGCTGTCACGTCGTAAAAAAGACCTGCAACAAATCTATGCCGAAGAACGTGAGAACTATCTGGGGAAA
CTGGAACGTGAAATCACCCGCTTTTTCGTGGATCGTGGCTTTCTGGAGATCAAATCCCCGATTCTGATTCCTCTG
GAGTATATCGAGCGTATGGGCATCGACAATGATACCGAACTGAGCAAACAAATTTTCCGTGTGGATAAAAACTTC
TGTCTGCGCCCTATGCTGGCACCAAATCTGTATAACTATCTGCGCAAACTGGACCGTGCCCTGCCTGATCCTATC
AAAATCTTCGAGATCGGCCCGTGTTATCGTAAAGAGTCCGACGGTAAAGAACATCTGGAGGAGTTTACCATGCTG
GCCTTTGCCCAAATGGGTTCAGGTTGTACTCGTGAGAACCTGGAAAGCATCATCACCGATTTTCTGAACCACCTG
GGCATTGACTTCAAAATTGTGGGCGACAGCTGTATGGTGTATGGCGACACCCTGGATGTCATGCACGGCGACCTG
GAACTGTCTAGTGCCGTTGTTGGACCAATTCCGCTGGACCGTGAGTGGGGTATCGACAAACCGTGGATCGGAGCA
GGATTCGGTCTGGAACGCCTGCTGAAAGTGAAACACGACTTCAAAAACATCAAACGTGCCGCCCGTTCTGAATCG
TATTATAACGGGATCTCTACGAACCTGGCCTCAAACGATTATACCCAACAAGCAACCCAAAGCTATGGGGCCTAC
CCCACCCAGCCCGGGCAGGGCTATTCCCAGCAGAGCAGTCAGCCCTACGGACAGCAGAGTTACAGTGGTTATAGC
CAGTCCACGGACACTTCAGGATATGGCCAGAGCAGCTATTCTTCTTATGGCCAGAGCCAGAACACAGGCTATGGA
ACTCAGTCAACTCCCCAGGGATATGGCTCGACTGGCGGCTATGGCAGTAGCCAGAGCTCCCAATCGTCTTACGGG
CAGCAGTCCTCCTACCCTGGCTATGGCCAGCAGCCAGCTCCCAGCAGCACCTCGGGAAGTTACGGTAGCAGTTCT
CAGAGCAGCAGCTATGGGCAGCCCCAGAGTGGGAGCTACAGCCAGCAGCCTAGCTATGGTGGACAGCAGCAAAGC
TATGGACAGCAGCAAAGCTATAATCCCCCTCAGGGCTATGGACAGCAGAACCAGTACAACAGCAGCAGTGGTGGT
GGAGGTGGAGGTGGAGGTGGAGGTAACTATGGCCAAGATCAATCCTCCATGAGTAGTGGTGGTGGCAGTGGTGGC
GGTTATGGCAATCAAGACCAGAGTGGTGGAGGTGGCAGCGGTGGCTATGGACAGCAGGACCGTGGAGGCCGCGGC
AGGGGTGGCAGTGGTGGCGGCGGCGGCGGCGGCGGTGGTGGTTACAACCGCAGCAGTGGTGGCTATGAACCCAGA
GGTCGTGGAGGTGGCCGTGGAGGCAGAGGTGGCATGGGCGGAAGTGACCGTGGTGGCTTCAATAAATTTGGTGGC
CCTCGGGACCAAGGATCACGTCATGACTCCGAACAGGATAATTCAGACAACAACACCATCTTTGTGCAAGGCCTG
GGTGAGAATGTTACAATTGAGTCTGTGGCTGATTACTTCAAGCAGATTGGTATTATTAAGACAAACAAGAAAACG
GGACAGCCCATGATTAATTTGTACACAGACAGGGAAACTGGCAAGCTGAAGGGAGAGGCAACGGTCTCTTTTGAT
GACCCACCTTCAGCTAAAGCAGCTATTGACTGGTTTGATGGTAAAGAATTCTCCGGAAATCCTATCAAGGTCTCA
TTTGCTACTCGCCGGGCAGACTTTAATCGGGGTGGTGGCAATGGTCGTGGAGGCCGAGGGCGAGGAGGACCCATG
GGCCGTGGAGGCTATGGAGGTGGTGGCAGTGGTGGTGGTGGCCGAGGAGGATTTCCCAGTGGAGGTGGTGGCGGT
GGAGGACAGCAGCGAGCTGGTGACTGGAAGTGTCCTAATCCCACCTGTGAGAATATGAACTTCTCTTGGAGGAAT
GAATGCAACCAGTGTAAGGCCCCTAAACCAGATGGCCCAGGAGGGGGACCAGGTGGCTCTCACATGGGGGGTAAC

TACGGGGATGATCGTCGTGGTGGCAGAGGAGGCGATAAAAAACCGCTGAATACCCTGATCTCTGCTACTGGTCTG
TGGATGAGTCGTACCGGAACCATTCATAAAATCAAACACCACGAGGTTAGCCGTTCGAAAATCTATATTGAGATG
GCGTGTGGCGATCATCTGGTTGTGAACAATAGCCGCTCTTCTCGTACAGCACGTGCACTGCGTCACCACAAATAT
CGTAAAACCTGTAAACGTTGCCGTGTGTCCGATGAGGATCTGAACAAATTCCTGACAAAAGCCAATGAGGACCAA
ACAAGCGTGAAAGTGAAAGTCGTTAGCGCTCCTACCCGTACTAAAAAAGCAATGCCGAAATCCGTTGCTCGTGCC
CCTAAACCACTGGAAAACACTGAAGCAGCACAGGCACAGCCGTCTGGAAGCAAATTCTCTCCGGCCATTCCTGTT
TCTACCCAGGAGTCCGTTTCTGTTCCAGCAAGTGTGAGCACCAGCATTAGCAGTATTAGCACCGGTGCCACCGCT
AGCGCCCTGGTTAAAGGCAATACCAATCCGATTACAAGCATGTCTGCCCCGGTTCAAGCATCAGCTCCAGCACTG
ACAAAATCCCAAACCGATCGTCTGGAGGTTCTGCTGAATCCGAAAGACGAAATCAGCCTGAATTCCGGCAAACCG
TTTCGTGAACTGGAGAGCGAACTGCTGTCACGTCGTAAAAAAGACCTGCAACAAATCTATGCCGAAGAACGTGAG
AACTATCTGGGGAAACTGGAACGTGAAATCACCCGCTTTTTCGTGGATCGTGGCTTTCTGGAGATCAAATCCCCG
ATTCTGATTCCTCTGGAGTATATCGAGCGTATGGGCATCGACAATGATACCGAACTGAGCAAACAAATTTTCCGT
GTGGATAAAAACTTCTGTCTGCGCCCTATGCTGGCACCAAATCTGTATAACTATCTGCGCAAACTGGACCGTGCC
CTGCCTGATCCTATCAAAATCTTCGAGATCGGCCCGTGTTATCGTAAAGAGTCCGACGGTAAAGAACATCTGGAG
GAGTTTACCATGCTGGCCTTTGCCCAAATGGGTTCAGGTTGTACTCGTGAGAACCTGGAAAGCATCATCACCGAT
TTTCTGAACCACCTGGGCATTGACTTCAAAATTGTGGGCGACAGCTGTATGGTGTATGGCGACACCCTGGATGTC
ATGCACGGCGACCTGGAACTGTCTAGTGCCGTTGTTGGACCAATTCCGCTGGACCGTGAGTGGGGTATCGACAAA
CCGTGGATCGGAGCAGGATTCGGTCTGGAACGCCTGCTGAAAGTGAAACACGACTTCAAAAACATCAAACGTGCC
GCCCGTTCTGAATCGTATTATAACGGGATCTCTACGAACCTGTAA (SEQ ID NO: 525)

Protein:
MGCVCSSNPEGTELACPVPLQLPPLERLTLDDKKPLNTLISATGLWMSRTGTIHKIKHHEVSRSKIYIEMACGDH
LVVNNSRSSRTARALRHHKYRKTCKRCRVSDEDLNKFLTKANEDQTSVKVKVVSAPTRTKKAMPKSVARAPKPLE
NTEAAQAQPSGSKFSPAIPVSTQESVSVPASVSTSISSISTGATASALVKGNTNPITSMSAPVQASAPALTKSQT
DRLEVLLNPKDEISLNSGKPFRELESELLSRRKKDLQQIYAEERENYLGKLEREITRFFVDRGFLEIKSPILIPL
EYIERMGIDNDTELSKQIFRVDKNFCLRPMLAPNLYNYLRKLDRALPDPIKIFEIGPCYRKESDGKEHLEEFTML
AFAQMGSGCTRENLESIITDFLNHLGIDFKIVGDSCMVYGDTLDVMHGDLELSSAVVGPIPLDREWGIDKPWIGA
GFGLERLLKVKHDFKNIKRAARSESYYNGISTNLASNDYTQQATQSYGAYPTQPGQGYSQQSSQPYGQQSYSGYS
QSTDTSGYGQSSYSSYGQSQNTGYGTQSTPQGYGSTGGYGSSQSSQSSYGQQSSYPGYGQQPAPSSTSGSYGSSS
QSSSYGQPQSGSYSQQPSYGGQQQSYGQQQSYNPPQGYGQQNQYNSSSGGGGGGGGGNYGQDQSSMSSGGGSGG
GYGNQDQSGGGGSGGYGQQDRGGRGRGGSGGGGGGGGGGYNRSSGGYEPRGRGGGRGGRGGMGGSDRGGFNKFGG
PRDQGSRHDSEQDNSDNNTIFVQGLGENVTIESVADYFKQIGIIKTNKKTGQPMINLYTDRETGKLKGEATVSFD
DPPSAKAAIDWFDGKEFSGNPIKVSFATRRADFNRGGGNGRGGRGRGGPMGRGGYGGGGSGGGGRGGFPSGGGGG
GGQQRAGDWKCPNPTCENMNFSWRNECNQCKAPKPDGPGGGPGGSHMGGNYGDDRRGGRGGDKKPLNTLISATGL
WMSRTGTIHKIKHHEVSRSKIYIEMACGDHLVVNNSRSSRTARALRHHKYRKTCKRCRVSDEDLNKFLTKANEDQ
TSVKVKVVSAPTRTKKAMPKSVARAPKPLENTEAAQAQPSGSKFSPAIPVSTQESVSVPASVSTSISSISTGATA
SALVKGNTNPITSMSAPVQASAPALTKSQTDRLEVLLNPKDEISLNSGKPFRELESELLSRRKKDLQQIYAEERE
NYLGKLEREITRFFVDRGFLEIKSPILIPLEYIERMGIDNDTELSKQIFRVDKNFCLRPMLAPNLYNYLRKLDRA
LPDPIKIFEIGPCYRKESDGKEHLEEFTMLAFAQMGSGCTRENLESIITDFLNHLGIDFKIVGDSCMVYGDTLDV
MHGDLELSSAVVGPIPLDREWGIDKPWIGAGFGLERLLKVKHDFKNIKRAARSESYYNGISTNL* (SEQ ID
NO: 526)

EBAG9₁₋₂₉::EWSR1::SYNZIP4::4xλ_{N22}
DNA:
ATGGCCATCACCCAGTTTCGGTTATTTAAATTTTGTACCTGCCTAGCAACAGTATTCTCATTCCTAAAGAGATTA
ATATGCAGATCTATGGCGTCCACGGATTACAGTACCTATAGCCAAGCTGCAGCGCAGCAGGGCTACAGTGCTTAC
ACCGCCCAGCCCACTCAAGGATATGCACAGACCACCCAGGCATATGGGCAACAAAGCTATGGAACCTATGGACAG
CCCACTGATGTCAGCTATACCCAGGCTCAGACCACTGCAACCTATGGGCAGACCGCCTATGCAACTTCTTATGGA
CAGCCTCCCACTGGTTATACTACTCCAACTGCCCCCCAGGCATACAGCCAGCCTGTCCAGGGGTATGGCACTGGT
GCTTATGATACCACCACTGCTACAGTCACCACCACCCAGGCCTCCTATGCAGCTCAGTCTGCATATGGCACTCAG
CCTGCTTATCCAGCCTATGGGCAGCAGCCAGCAGCCACTGCACCTACAAGACCGCAGGATGGAAACAAGCCCACT
GAGACTAGTCAACCTCAATCTAGCACAGGGGGTTACAACCAGCCCAGCCTAGGATATGGACAGAGTAACTACAGT
TATCCCCAGGTACCTGGGAGCTACCCCATGCAGCCAGTCACTGCACCTCCATCCTACCCTCCTACCAGCTATTCC
TCTACACAGCCGACTAGTTATGATCAGAGCAGTTACTCTCAGCAGAACACCTATGGGCAACCGAGCAGCTATGGA
CAGCAGAGTAGCTATGGTCAACAAAGCAGCTATGGGCAGCAGCCTCCCACTAGTTACCCACCCCAAACTGGATCC
TACAGCCAAGCTCCAAGTCAATATAGCCAACAGAGCAGCAGCTACGGGCAGCAGAGTTCATTCCGACAGGACCAC
CCCAGTAGCATGGGTGTTTATGGGCAGGAGTCTGGAGGATTTTCCGGACCAGGAGAGAACCGGAGCATGAGTGGC

CCTGATAACCGGGGCAGGGGAAGAGGGGGATTTGATCGTGGAGGCATGAGCAGAGGTGGGCGGGGAGGAGGACGC
GGTGGAATGGGCAGCGCTGGAGAGCGAGGTGGCTTCAATAAGCCTGGTGGACCCATGGATGAAGGACCAGATCTT
GATCTAGGCCCACCTGTAGATCCAGATGAAGACTCTGACAACAGTGCAATTTATGTACAAGGATTAAATGACAGT
GTGACTCTAGATGATCTGGCAGACTTCTTTAAGCAGTGTGGGGTTGTTAAGATGAACAAGAGAACTGGGCAACCC
ATGATCCACATCTACCTGGACAAGGAAACAGGAAAGCCCAAAGGCGATGCCACAGTGTCCTATGAAGACCCACCT
ACTGCCAAGGCTGCCGTGGAATGGTTTGATGGGAAAGATTTTCAAGGGAGCAAACTTAAAGTCTCCCTTGCTCGG
AAGAAGCCTCCAATGAACAGTATGCGGGGTGGTCTGCCACCCCGTGAGGGCAGAGGCATGCCACCACCACTCCGT
GGAGGTCCAGGAGGCCCAGGAGGTCCTGGGGGACCCATGGGTCGCATGGGAGGCCGTGGAGGAGATAGAGGAGGC
TTCCCTCCAAGAGGACCCCGGGGTTCCCGAGGGAACCCCTCTGGAGGAGGAAACGTCCAGCACCGAGCTGGAGAC
TGGCAGTGTCCCAATCCGGGTTGTGGAAACCAGAACTTCGCCTGGAGAACAGAGTGCAACCAGTGTAAGGCCCCA
AAGCCTGAAGGCTTCCTCCCGCCACCCTTTCCGCCCCCGGGTGGTGATCGTGGCAGAGGTGGCCCTGGTGGCATG
CGGGGAGGAAGAGGTGGCCTCATGGATCGTGGTGGTCCCGGTGGAATGTTCAGAGGTGGCCGTGGTGGAGACAGA
GGTGGCTTCCGTGGTGGCCGGGGCATGGACCGAGGTGGCTTTGGTGGAGGAAGACGAGGTGGCCCTGGGGGGCCC
CCTGGACCTTTGATGGAACAGGCGATCGCACAAAAGGTGGCTGAACTGAAAAATAGAGTGGCCGTGAAGCTGAAC
CGGAACGAGCAGCTGAAGAACAAGGTGGAAGAGCTGAAGAACAGAAACGCCTACCTGAAGAATGAGCTGGCCACC
CTGGAAAACGAGGTGGCCAGACTGGAAAACGACGTGGCCGAGGGAGCACCAGGAAGTGCTGGTTCTGCTGCTGGT
AGTGGAGAGCAGAAGCTGATCTCAGAGGAGGACCTGCTAGCCACCATGGACGCACAAACACGACGACGTGAGCGT
CGCGCTGAGAAACAAGCTCAATGGAAAGCTGCAAACCCACCGCTCGACGGAGCCGGAGCTGGCGCTGGAGCTGGA
GCCGGAGCTGGCGGTCTAGCCACCATGGACGCACAAACACGACGACGTGAGCGTCGCGCTGAGAAACAAGCTCAA
TGGAAAGCTGCAAACCCACCGCTCGACGGAGCCGGAGCTGGCGCTGGAGCTGGAGCCGGAGCTGGCGGTCTAGCC
ACCATGGACGCACAAACACGACGACGTGAGCGTCGCGCTGAGAAACAAGCTCAATGGAAAGCTGCAAACCCACCG
CTCGACGGAGCCGGAGCTGGCGCTGGAGCTGGAGCCGGAGCTGGCGGTCTAGCCACCATGGACGCACAAACACGA
CGACGTGAGCGTCGCGCTGAGAAACAAGCTCAATGGAAAGCTGCAAACCCACCGCTCGAGTCTAGAGGGCCCGTT
TAA (SEQ ID NO: 527)

Protein:
MAITQFRLFKFCTCLATVFSFLKRLICRSMASTDYSTYSQAAAQQGYSAYTAQPTQGYAQTTQAYGQQSYGTYGQ
PTDVSYTQAQTTATYGQTAYATSYGQPPTGYTTPTAPQAYSQPVQGYGTGAYDTTTATVTTTQASYAAQSAYGTQ
PAYPAYGQQPAATAPTRPQDGNKPTETSQPQSSTGGYNQPSLGYGQSNYSYPQVPGSYPMQPVTAPPSYPPTSYS
STQPTSYDQSSYSQQNTYGQPSSYGQQSSYGQQSSYGQQPPTSYPPQTGSYSQAPSQYSQQSSSYGQQSSFRQDH
PSSMGVYGQESGGFSGPGENRSMSGPDNRGRGRGGFDRGGMSRGGRGGGRGGMGSAGERGGFNKPGGPMDEGPDL
DLGPPVDPDEDSDNSAIYVQGLNDSVTLDDLADFFKQCGVVKMNKRTGQPMIHIYLDKETGKPKGDATVSYEDPP
TAKAAVEWFDGKDFQGSKLKVSLARKKPPMNSMRGGLPPREGRGMPPPLRGGPGGPGGPGGPMGRMGGRGGDRGG
FPPRGPRGSRGNPSGGGNVQHRAGDWQCPNPGCGNQNFAWRTECNQCKAPKPEGFLPPPFPPPGGDRGRGGPGGM
RGGRGGLMDRGGPGGMFRGGRGGDRGGFRGGRGMDRGGFGGGRRGGPGGPPGPLMEQAIAQKVAELKNRVAVKLN
RNEQLKNKVEELKNRNAYLKNELATLENEVARLENDVAEGAPGSAGSAAGSGEQKLISEEDLLATMDAQTRRRER
RAEKQAQWKAANPPLDGAGAGAGAGAGAGGLATMDAQTRRRERRAEKQAQWKAANPPLDGAGAGAGAGAGAGGLA
TMDAQTRRRERRAEKQAQWKAANPPLDGAGAGAGAGAGAGGLATMDAQTRRRERRAEKQAQWKAANPPLESRGPV
* (SEQ ID NO: 528)

KIF16B::FUS::SYNZIP1::PylRS(AF)
DNA:
ATGGCATCGGTCAAGGTGGCCGTGAGGGTCCGGCCCATGAATCGCAGGGAAAAGGACTTGGAGGCCAAGTTCATT
ATTCAGATGGAGAAAAGCAAAACGACAATCACAAACTTAAAGATACCAGAAGGAGGCACTGGGGACTCAGGAAGA
GAACGGACCAAGACCTTCACCTATGACTTTTCTTTTTATTCTGCTGATACAAAAAGCCCAGATTACGTTTCACAA
GAAATGGTTTTCAAAACCCTCGGCACAGATGTCGTGAAGTCTGCATTTGAAGGTTATAATGCTTGTGTCTTTGCA
TATGGGCAAACTGGATCTGGAAAGTCATACACTATGATGGGAAATTCTGGAGATTCTGGCTTAATACCTCGGATC
TGTGAAGGACTCTTCAGTCGGATAAATGAAACCACCAGATGGGATGAAGCTTCTTTTCGAACTGAAGTCAGCTAC
TTAGAAATTTATAACGAACGTGTGAGAGATCTACTTCGGCGGAAGTCATCTAAAACCTTCAATTTGAGAGTCCGT
GAGCATCCCAAAGAAGGCCCTTATGTTGAGGATTTATCCAAACATTTAGTACAGAATTATGGTGACGTAGAAGAA
CTTATGGATGCGGGCAATATCAACCGGACCACCGCAGCGACTGGGATGAACGACGTCAGTAGCAGGTCTCATGCC
ATCTTCACCATCAAGTTCACTCAGGCTAAATTTGATTCTGAAATGCCATGTGAAACCGTCAGTAAGATCCACTTG
GTTGATCTTGCCGGAAGTGAGCGTGCAGATGCCACCGGAGCCACCGGGGTTAGGCTAAAGGAAGGGGGAAATATT
AACAAGTCCCTCGTGACTCTGGGGAACGTCATTTCTGCCTTAGCTGATTTATCTCAGGATGCTGCAAATACTCTT
GCAAAGAAGAAGCAAGTTTTCGTGCCTTACAGGGATTCTGTGTTGACTTGGTTGTTAAAAGATAGCCTTGGAGGA
AACTCTAAAACTATCATGATTGCCACCATTTCACCTGCTGATGTCAATTATGGAGAAACCCTAAGTACTCTTCGC
TATGCAAATAGAGCCAAAAACATCATCAACAAGCCTACCATTAATGAGGATGCCAACGTCAAACTTATCCGTGAG

CTGCGAGCTGAAATAGCCAGACTGAAAACGCTGCTTGCTCAAGGGAATCAGATTGCCCTCTTAGACTCCCCCACA
TATACAGATATTGAAATGAACAGATTGGGAAAGGGCGCCCCCGGCTCCGCCGGCTCCGCCGCCGGCTCCGGCATG
GCCTCAAACGATTATACCCAACAAGCAACCCAAAGCTATGGGGCCTACCCCACCCAGCCCGGGCAGGGCTATTCC
CAGCAGAGCAGTCAGCCCTACGGACAGCAGAGTTACAGTGGTTATAGCCAGTCCACGGACACTTCAGGATATGGC
CAGAGCAGCTATTCTTCTTATGGCCAGAGCCAGAACACAGGCTATGGAACTCAGTCAACTCCCCAGGGATATGGC
TCGACTGGCGGCTATGGCAGTAGCCAGAGCTCCCAATCGTCTTACGGGCAGCAGTCCTCCTACCCTGGCTATGGC
CAGCAGCCAGCTCCCAGCAGCACCTCGGGAAGTTACGGTAGCAGTTCTCAGAGCAGCAGCTATGGGCAGCCCCAG
AGTGGGAGCTACAGCCAGCAGCCTAGCTATGGTGGACAGCAGCAAAGCTATGGACAGCAGCAAAGCTATAATCCC
CCTCAGGGCTATGGACAGCAGAACCAGTACAACAGCAGCAGTGGTGGTGGAGGTGGAGGTGGAGGTGGAGGTAAC
TATGGCCAAGATCAATCCTCCATGAGTAGTGGTGGTGGCAGTGGTGGCGGTTATGGCAATCAAGACCAGAGTGGT
GGAGGTGGCAGCGGTGGCTATGGACAGCAGGACCGTGGAGGCCGCGGCAGGGGTGGCAGTGGTGGCGGCGGCGGC
GGCGGCGGTGGTGGTTACAACCGCAGCAGTGGTGGCTATGAACCCAGAGGTCGTGGAGGTGGCCGTGGAGGCAGA
GGTGGCATGGGCGGAAGTGACCGTGGTGGCTTCAATAAATTTGGTGGCCCTCGGGACCAAGGATCACGTCATGAC
TCCGAACAGGATAATTCAGACAACAACACCATCTTTGTGCAAGGCCTGGGTGAGAATGTTACAATTGAGTCTGTG
GCTGATTACTTCAAGCAGATTGGTATTATTAAGACAAACAAGAAAACGGGACAGCCCATGATTAATTTGTACACA
GACAGGGAAACTGGCAAGCTGAAGGGAGAGGCAACGGTCTCTTTTGATGACCCACCTTCAGCTAAAGCAGCTATT
GACTGGTTTGATGGTAAAGAATTCTCCGGAAATCCTATCAAGGTCTCATTTGCTACTCGCCGGGCAGACTTTAAT
CGGGGTGGTGGCAATGGTCGTGGAGGCCGAGGGCGAGGAGGACCCATGGGCCGTGGAGGCTATGGAGGTGGTGGC
AGTGGTGGTGGTGGCCGAGGAGGATTTCCCAGTGGAGGTGGTGGCGGTGGAGGACAGCAGCGAGCTGGTGACTGG
AAGTGTCCTAATCCCACCTGTGAGAATATGAACTTCTCTTGGAGGAATGAATGCAACCAGTGTAAGGCCCCTAAA
CCAGATGGCCCAGGAGGGGGACCAGGTGGCTCTCACATGGGGGGTAACTACGGGGATGATCGTCGTGGTGGCAGA
GGTGGTGCGATCGCAAATCTGGTGGCCCAGCTGGAAAACGAGGTGGCCAGCCTGGAAAACGAGAACGAAACCCTG
AAGAAAAAGAACCTGCACAAGAAGGACCTGATCGCCTACCTGGAAAAGGAAATCGCCAACCTGAGAAAGAAGATC
GAGGAAGGAGCACCAGGAAGTGCTGGTTCTGCTGCTGGTAGTGGAGCGTGCCCGGTGCCGCTGCAGCTGCCGCCG
CTGGAACGCCTGACCCTGGATGATAAAAAACCGCTGAATACCCTGATCTCTGCTACTGGTCTGTGGATGAGTCGT
ACCGGAACCATTCATAAAATCAAACACCACGAGGTTAGCCGTTCGAAAATCTATATTGAGATGGCGTGTGGCGAT
CATCTGGTTGTGAACAATAGCCGCTCTTCTCGTACAGCACGTGCACTGCGTCACCACAAATATCGTAAAACCTGT
AAACGTTGCCGTGTGTCCGATGAGGATCTGAACAAATTCCTGACAAAAGCCAATGAGGACCAAACAAGCGTGAAA
GTGAAAGTCGTTAGCGCTCCTACCCGTACTAAAAAAGCAATGCCGAAATCCGTTGCTCGTGCCCCTAAACCACTG
GAAAACACTGAAGCAGCACAGGCACAGCCGTCTGGAAGCAAATTCTCTCCGGCCATTCCTGTTTCTACCCAGGAG
TCCGTTTCTGTTCCAGCAAGTGTGAGCACCAGCATTAGCAGTATTAGCACCGGTGCCACCGCTAGCGCCCTGGTT
AAAGGCAATACCAATCCGATTACAAGCATGTCTGCCCCGGTTCAAGCATCAGCTCCAGCACTGACAAAATCCCAA
ACCGATCGTCTGGAGGTTCTGCTGAATCCGAAAGACGAAATCAGCCTGAATTCCGGCAAACCGTTTCGTGAACTG
GAGAGCGAACTGCTGTCACGTCGTAAAAAAGACCTGCAACAAATCTATGCCGAAGAACGTGAGAACTATCTGGGG
AAACTGGAACGTGAAATCACCCGCTTTTTCGTGGATCGTGGCTTTCTGGAGATCAAATCCCCGATTCTGATTCCT
CTGGAGTATATCGAGCGTATGGGCATCGACAATGATACCGAACTGAGCAAACAAATTTTCCGTGTGGATAAAAAC
TTCTGTCTGCGCCCTATGCTAGCACCAAATCTGGCTAACTATCTGCGCAAACTGGACCGTGCCCTGCCTGATCCT
ATCAAAATCTTCGAGATCGGCCCGTGTTATCGTAAAGAGTCCGACGGTAAAGAACATCTGGAGGAGTTTACCATG
CTGAACTTTTGCCAAATGGGTTCAGGTTGTACTCGTGAGAACCTGGAAAGCATCATCACCGATTTTCTGAACCAC
CTGGGCATTGACTTCAAAATTGTGGGCGACAGCTGTATGGTGTTTGGCGACACCCTGGATGTCATGCACGGCGAC
CTGGAACTGTCTAGTGCCGTTGTGGGCCCAATCCCGCTGGATCGTGAGTGGGGTATCGACAAACCTTGGATCGGT
GCGGGTTTTGGTCTGGAGCGTCTGCTGAAAGTAAAACACGACTTCAAGAACATCAAACGTGCTGCACGTTCCGAG
TCCTATTACAATGGTATTTCTACTAACCTGTAA (SEQ ID NO: 529)

Protein:
MASVKVAVRVRPMNRREKDLEAKFIIQMEKSKTTITNLKIPEGGTGDSGRERTKTFTYDFSFYSADTKSPDYVSQ
EMVFKTLGTDVVKSAFEGYNACVFAYGQTGSGKSYTMMGNSGDSGLIPRICEGLFSRINETTRWDEASFRTEVSY
LEIYNERVRDLLRRKSSKTFNLRVREHPKEGPYVEDLSKHLVQNYGDVEELMDAGNINRTTAATGMNDVSSRSHA
IFTIKFTQAKFDSEMPCETVSKIHLVDLAGSERADATGATGVRLKEGGNINKSLVTLGNVISALADLSQDAANTL
AKKKQVFVPYRDSVLTWLLKDSLGGNSKTIMIATISPADVNYGETLSTLRYANRAKNIINKPTINEDANVKLIRE
LRAEIARLKTLLAQGNQIALLDSPTYTDIEMNRLGKGAPGSAGSAAGSGMASNDYTQQATQSYGAYPTQPGQGYS
QQSSQPYGQQSYSGYSQSTDTSGYGQSSYSSYGQSQNTGYGTQSTPQGYGSTGGYGSSQSSQSSYGQQSSYPGYG
QQPAPSSTSGSYGSSSQSSSYGQPQSGSYSQQPSYGGQQQSYGQQQSYNPPQGYGQQNQYNSSSGGGGGGGGGN
YGQDQSSMSSGGGSGGGYGNQDQSGGGGSGGYGQQDRGGRGRGGSGGGGGGGGGGYNRSSGGYEPRGRGGGRGGR
GGMGGSDRGGFNKFGGPRDQGSRHDSEQDNSDNNTIFVQGLGENVTIESVADYFKQIGIIKTNKKTGQPMINLYT
DRETGKLKGEATVSFDDPPSAKAAIDWFDGKEFSGNPIKVSFATRRADFNRGGGNGRGGRGRGGPMGRGGYGGGG
SGGGGRGGFPSGGGGGGGQQRAGDWKCPNPTCENMNFSWRNECNQCKAPKPDGPGGGPGGSHMGGNYGDDRRGGR
GGAIANLVAQLENEVASLENENETLKKKNLHKKDLIAYLEKEIANLRKKIEEGAPGSAGSAAGSGACPVPLQLPP

LERLTLDDKKPLNTLISATGLWMSRTGTIHKIKHHEVSRSKIYIEMACGDHLVVNNSRSSRTARALRHHKYRKTC
KRCRVSDEDLNKFLTKANEDQTSVKVKVVSAPTRTKKAMPKSVARAPKPLENTEAAQAQPSGSKFSPAIPVSTQE
SVSVPASVSTSISSISTGATASALVKGNTNPITSMSAPVQASAPALTKSQTDRLEVLLNPKDEISLNSGKPFREL
ESELLSRRKKDLQQIYAEERENYLGKLEREITRFFVDRGFLEIKSPILIPLEYIERMGIDNDTELSKQIFRVDKN
FCLRPMLAPNLANYLRKLDRALPDPIKIFEIGPCYRKESDGKEHLEEFTMLNFCQMGSGCTRENLESIITDFLNH
LGIDFKIVGDSCMVFGDTLDVMHGDLELSSAVVGPIPLDREWGIDKPWIGAGFGLERLLKVKHDFKNIKRAARSE
SYYNGISTNL* (SEQ ID NO: 530)

KIF16B::FUS::SYNZIP1::PylRS(AA)
DNA:
ATGGCATCGGTCAAGGTGGCCGTGAGGGTCCGGCCCATGAATCGCAGGGAAAAGGACTTGGAGGCCAAGTTCATT
ATTCAGATGGAGAAAAGCAAAACGACAATCACAAACTTAAAGATACCAGAAGGAGGCACTGGGGACTCAGGAAGA
GAACGGACCAAGACCTTCACCTATGACTTTTCTTTTTATTCTGCTGATACAAAAAGCCCAGATTACGTTTCACAA
GAAATGGTTTTCAAAACCCTCGGCACAGATGTCGTGAAGTCTGCATTTGAAGGTTATAATGCTTGTGTCTTTGCA
TATGGGCAAACTGGATCTGGAAAGTCATACACTATGATGGGAAATTCTGGAGATTCTGGCTTAATACCTCGGATC
TGTGAAGGACTCTTCAGTCGGATAAATGAAACCACCAGATGGGATGAAGCTTCTTTTCGAACTGAAGTCAGCTAC
TTAGAAATTTATAACGAACGTGTGAGAGATCTACTTCGGCGGAAGTCATCTAAAACCTTCAATTTGAGAGTCCGT
GAGCATCCCAAAGAAGGCCCTTATGTTGAGGATTTATCCAAACATTTAGTACAGAATTATGGTGACGTAGAAGAA
CTTATGGATGCGGGCAATATCAACCGGACCACCGCAGCGACTGGGATGAACGACGTCAGTAGCAGGTCTCATGCC
ATCTTCACCATCAAGTTCACTCAGGCTAAATTTGATTCTGAAATGCCATGTGAAACCGTCAGTAAGATCCACTTG
GTTGATCTTGCCGGAAGTGAGCGTGCAGATGCCACCGGAGCCACCGGGGTTAGGCTAAAGGAAGGGGGAAATATT
AACAAGTCCCTCGTGACTCTGGGGAACGTCATTTCTGCCTTAGCTGATTTATCTCAGGATGCTGCAAATACTCTT
GCAAAGAAGAAGCAAGTTTTCGTGCCTTACAGGGATTCTGTGTTGACTTGGTTGTTAAAAGATAGCCTTGGAGGA
AACTCTAAAACTATCATGATTGCCACCATTTCACCTGCTGATGTCAATTATGGAGAAACCCTAAGTACTCTTCGC
TATGCAAATAGAGCCAAAAACATCATCAACAAGCCTACCATTAATGAGGATGCCAACGTCAAACTTATCCGTGAG
CTGCGAGCTGAAATAGCCAGACTGAAAACGCTGCTTGCTCAAGGGAATCAGATTGCCCTCTTAGACTCCCCCACA
TATACAGATATTGAAATGAACAGATTGGGAAAGGGCGCCCCCGGCTCCGCCGGCTCCGCCGCCGGCTCCGGCATG
GCCTCAAACGATTATACCCAACAAGCAACCCAAAGCTATGGGGCCTACCCCACCCAGCCCGGGCAGGGCTATTCC
CAGCAGAGCAGTCAGCCCTACGGACAGCAGAGTTACAGTGGTTATAGCCAGTCCACGGACACTTCAGGATATGGC
CAGAGCAGCTATTCTTCTTATGGCCAGAGCCAGAACACAGGCTATGGAACTCAGTCAACTCCCCAGGGATATGGC
TCGACTGGCGGCTATGGCAGTAGCCAGAGCTCCCAATCGTCTTACGGGCAGCAGTCCTCCTACCCTGGCTATGGC
CAGCAGCCAGCTCCCAGCAGCACCTCGGGAAGTTACGGTAGCAGTTCTCAGAGCAGCAGCTATGGGCAGCCCCAG
AGTGGGAGCTACAGCCAGCAGCCTAGCTATGGTGGACAGCAGCAAAGCTATGGACAGCAGCAAAGCTATAATCCC
CCTCAGGGCTATGGACAGCAGAACCAGTACAACAGCAGCAGTGGTGGTGGAGGTGGAGGTGGAGGTGGAGGTAAC
TATGGCCAAGATCAATCCTCCATGAGTAGTGGTGGTGGCAGTGGTGGCGGTTATGGCAATCAAGACCAGAGTGGT
GGAGGTGGCAGCGGTGGCTATGGACAGCAGGACCGTGGAGGCCGCGGCAGGGGTGGCAGTGGTGGCGGCGGCGGC
GGCGGCGGTGGTGGTTACAACCGCAGCAGTGGTGGCTATGAACCCAGAGGTCGTGGAGGTGGCCGTGGAGGCAGA
GGTGGCATGGGCGGAAGTGACCGTGGTGGCTTCAATAAATTTGGTGGCCCTCGGGACCAAGGATCACGTCATGAC
TCCGAACAGGATAATTCAGACAACAACACCATCTTTGTGCAAGGCCTGGGTGAGAATGTTACAATTGAGTCTGTG
GCTGATTACTTCAAGCAGATTGGTATTATTAAGACAAACAAGAAAACGGGACAGCCCATGATTAATTTGTACACA
GACAGGGAAACTGGCAAGCTGAAGGGAGAGGCAACGGTCTCTTTTGATGACCCACCTTCAGCTAAAGCAGCTATT
GACTGGTTTGATGGTAAAGAATTCTCCGGAAATCCTATCAAGGTCTCATTTGCTACTCGCCGGGCAGACTTTAAT
CGGGGTGGTGGCAATGGTCGTGGAGGCCGAGGGCGAGGAGGACCCATGGGCCGTGGAGGCTATGGAGGTGGTGGC
AGTGGTGGTGGTGGCCGAGGAGGATTTCCCAGTGGAGGTGGTGGCGGTGGAGGACAGCAGCGAGCTGGTGACTGG
AAGTGTCCTAATCCCACCTGTGAGAATATGAACTTCTCTTGGAGGAATGAATGCAACCAGTGTAAGGCCCCTAAA
CCAGATGGCCCAGGAGGGGGACCAGGTGGCTCTCACATGGGGGGTAACTACGGGGATGATCGTCGTGGTGGCAGA
GGTGGTGCGATCGCAAATCTGGTGGCCCAGCTGGAAAACGAGGTGGCCAGCCTGGAAAACGAGAACGAAACCCTG
AAGAAAAAGAACCTGCACAAGAAGGACCTGATCGCCTACCTGGAAAAGGAAATCGCCAACCTGAGAAAGAAGATC
GAGGAAGGAGCACCAGGAAGTGCTGGTTCTGCTGCTGGTAGTGGAGCGTGCCCGGTGCCGCTGCAGCTGCCGCCG
CTGGAACGCCTGACCCTGGATGATAAAAAACCGCTGAATACCCTGATCTCTGCTACTGGTCTGTGGATGAGTCGT
ACCGGAACCATTCATAAAATCAAACACCACGAGGTTAGCCGTTCGAAAATCTATATTGAGATGGCGTGTGGCGAT
CATCTGGTTGTGAACAATAGCCGCTCTTCTCGTACAGCACGTGCACTGCGTCACCACAAATATCGTAAAACCTGT
AAACGTTGCCGTGTGTCCGATGAGGATCTGAACAAATTCCTGACAAAAGCCAATGAGGACCAAACAAGCGTGAAA
GTGAAAGTCGTTAGCGCTCCTACCCGTACTAAAAAAGCAATGCCGAAATCCGTTGCTCGTGCCCCTAAACCACTG
GAAAACACTGAAGCAGCACAGGCACAGCCGTCTGGAAGCAAATTCTCTCCGGCCATTCCTGTTTCTACCCAGGAG
TCCGTTTCTGTTCCAGCAAGTGTGAGCACCAGCATTAGCAGTATTAGCACCGGTGCCACCGCTAGCGCCCTGGTT
AAAGGCAATACCAATCCGATTACAAGCATGTCTGCCCCGGTTCAAGCATCAGCTCCAGCACTGACAAAATCCCAA

ACCGATCGTCTGGAGGTTCTGCTGAATCCGAAAGACGAAATCAGCCTGAATTCCGGCAAACCGTTTCGTGAACTG
GAGAGCGAACTGCTGTCACGTCGTAAAAAAGACCTGCAACAAATCTATGCCGAAGAACGTGAGAACTATCTGGGG
AAACTGGAACGTGAAATCACCCGCTTTTTCGTGGATCGTGGCTTTCTGGAGATCAAATCCCCGATTCTGATTCCT
CTGGAGTATATCGAGCGTATGGGCATCGACAATGATACCGAACTGAGCAAACAAATTTTCCGTGTGGATAAAAAC
TTCTGTCTGCGCCCTATGCTGGCACCAAATCTGTATAACTATCTGCGCAAACTGGACCGTGCCCTGCCTGATCCT
ATCAAAATCTTCGAGATCGGCCCGTGTTATCGTAAAGAGTCCGACGGTAAAGAACATCTGGAGGAGTTTACCATG
CTGGCCTTTGCCCAAATGGGTTCAGGTTGTACTCGTGAGAACCTGGAAAGCATCATCACCGATTTTCTGAACCAC
CTGGGCATTGACTTCAAAATTGTGGGCGACAGCTGTATGGTGTATGGCGACACCCTGGATGTCATGCACGGCGAC
CTGGAACTGTCTAGTGCCGTTGTTGGACCAATTCCGCTGGACCGTGAGTGGGGTATCGACAAACCGTGGATCGGA
GCAGGATTCGGTCTGGAACGCCTGCTGAAAGTGAAACACGACTTCAAAAACATCAAACGTGCCGCCCGTTCTGAA
TCGTATTATAACGGGATCTCTACGAACCTGTAA (SEQ ID NO: 531)

Protein:
MASVKVAVRVRPMNRREKDLEAKFIIQMEKSKTTITNLKIPEGGTGDSGRERTKTFTYDFSFYSADTKSPDYVSQ
EMVFKTLGTDVVKSAFEGYNACVFAYGQTGSGKSYTMMGNSGDSGLIPRICEGLFSRINETTRWDEASFRTEVSY
LEIYNERVRDLLRRKSSKTFNLRVREHPKEGPYVEDLSKHLVQNYGDVEELMDAGNINRTTAATGMNDVSSRSHA
IFTIKFTQAKFDSEMPCETVSKIHLVDLAGSERADATGATGVRLKEGGNINKSLVTLGNVISALADLSQDAANTL
AKKKQVFVPYRDSVLTWLLKDSLGGNSKTIMIATISPADVNYGETLSTLRYANRAKNIINKPTINEDANVKLIRE
LRAEIARLKTLLAQGNQIALLDSPTYTDIEMNRLGKGAPGSAGSAAGSGMASNDYTQQATQSYGAYPTQPGQGYS
QQSSQPYGQQSYSGYSQSTDTSGYGQSSYSSYGQSQNTGYGTQSTPQGYGSTGGYGSSQSSQSSYGQQSSYPGYG
QQPAPSSTSGSYGSSSQSSSYGQPQSGSYSQQPSYGGQQQSYGQQQSYNPPQGYGQQNQYNSSSGGGGGGGGGGN
YGQDQSSMSSGGGSGGGYGNQDQSGGGGSGGYGQQDRGGRGRGGSGGGGGGGGGGYNRSSGGYEPRGRGGGRGGR
GGMGGSDRGGFNKFGGPRDQGSRHDSEQDNSDNNTIFVQGLGENVTIESVADYFKQIGIIKTNKKTGQPMINLYT
DRETGKLKGEATVSFDDPPSAKAAIDWFDGKEFSGNPIKVSFATRRADFNRGGGNGRGGRGRGGPMGRGGYGGGG
SGGGGRGGFPSGGGGGGGQQRAGDWKCPNPTCENMNFSWRNECNQCKAPKPDGPGGGPGGSHMGGNYGDDRRGGR
GGAIANLVAQLENEVASLENENETLKKKNLHKKDLIAYLEKEIANLRKKIEEGAPGSAGSAAGSGACPVPLQLPP
LERLTLDDKKPLNTLISATGLWMSRTGTIHKIKHHEVSRSKIYIEMACGDHLVVNNSRSSRTARALRHHKYRKTC
KRCRVSDEDLNKFLTKANEDQTSVKVKVVSAPTRTKKAMPKSVARAPKPLENTEAAQAQPSGSKFSPAIPVSTQE
SVSVPASVSTSISSISTGATASALVKGNTNPITSMSAPVQASAPALTKSQTDRLEVLLNPKDEISLNSGKPFREL
ESELLSRRKKDLQQIYAEERENYLGKLEREITRFFVDRGFLEIKSPILIPLEYIERMGIDNDTELSKQIFRVDKN
FCLRPMLAPNLYNYLRKLDRALPDPIKIFEIGPCYRKESDGKEHLEEFTMLAFAQMGSGCTRENLESIITDFLNH
LGIDFKIVGDSCMVYGDTLDVMHGDLELSSAVVGPIPLDREWGIDKPWIGAGFGLERLLKVKHDFKNIKRAARSE
SYYNGISTNL* (SEQ ID NO: 532)

EBAG9₁₋₂₉::EWSR1::SYNZIP2::MCP
DNA:
ATGGCCATCACCCAGTTTCGGTTATTTAAATTTTGTACCTGCCTAGCAACAGTATTCTCATTCCTAAAGAGATT
AATATGCAGATCTATGGCGTCCACGGATTACAGTACCTATAGCCAAGCTGCAGCGCAGCAGGGCTACAGTGCTTAC
ACCGCCCAGCCCACTCAAGGATATGCACAGACCACCCAGGCATATGGGCAACAAAGCTATGGAACCTATGGACAG
CCCACTGATGTCAGCTATACCCAGGCTCAGACCACTGCAACCTATGGGCAGACCGCCTATGCAACTTCTTATGGA
CAGCCTCCCACTGGTTATACTACTCCAACTGCCCCCCAGGCATACAGCCAGCCTGTCCAGGGGTATGGCACTGGT
GCTTATGATACCACCACTGCTACAGTCACCACCACCCAGGCCTCCTATGCAGCTCAGTCTGCATATGGCACTCAG
CCTGCTTATCCAGCCTATGGGCAGCAGCCAGCAGCCACTGCACCTACAAGACCGCAGGATGGAAACAAGCCCACT
GAGACTAGTCAACCTCAATCTAGCACAGGGGGTTACAACCAGCCCAGCCTAGGATATGGACAGAGTAACTACAGT
TATCCCCAGGTACCTGGGAGCTACCCCATGCAGCCAGTCACTGCACCTCCATCCTACCCTCCTACCAGCTATTCC
TCTACACAGCCGACTAGTTATGATCAGAGCAGTTACTCTCAGCAGAACACCTATGGGCAACCGAGCAGCTATGGA
CAGCAGAGTAGCTATGGTCAACAAAGCAGCTATGGGCAGCAGCCTCCCACTAGTTACCCACCCCAAACTGGATCC
TACAGCCAAGCTCCAAGTCAATATAGCCAACAGAGCAGCAGCTACGGGCAGCAGAGTTCATTCCGACAGGACCAC
CCCAGTAGCATGGGTGTTTATGGGCAGGAGTCTGGAGGATTTTCCGGACCAGGAGAGAACCGGAGCATGAGTGGC
CCTGATAACCGGGGCAGGGGAAGAGGGGGATTTGATCGTGGAGGCATGAGCAGAGGTGGGCGGGGAGGAGGACGC
GGTGGAATGGGCAGCGCTGGAGAGCGAGGTGGCTTCAATAAGCCTGGTGGACCCATGGATGAAGGACCAGATCTT
GATCTAGGCCCACCTGTAGATCCAGATGAAGACTCTGACAACAGTGCAATTTATGTACAAGGATTAAATGACAGT
GTGACTCTAGATGATCTGGCAGACTTCTTTAAGCAGTGTGGGGTTGTTAAGATGAACAAGAGAACTGGGCAACCC
ATGATCCACATCTACCTGGACAAGGAAACAGGAAAGCCCAAAGGCGATGCCACAGTGTCCTATGAAGACCCACCT
ACTGCCAAGGCTGCCGTGGAATGGTTTGATGGGAAAGATTTTCAAGGGAGCAAACTTAAAGTCTCCCTTGCTCGG
AAGAAGCCTCCAATGAACAGTATGCGGGGTGGTCTGCCACCCCGTGAGGGCAGAGGCATGCCACCACCACTCCGT
GGAGGTCCAGGAGGCCCAGGAGGTCCTGGGGGACCCATGGGTCGCATGGGAGGCCGTGGAGGAGATAGAGGAGGC

TTCCCTCCAAGAGGACCCCGGGGTTCCCGAGGGAACCCCTCTGGAGGAGGAAACGTCCAGCACCGAGCTGGAGAC
TGGCAGTGTCCCAATCCGGGTTGTGGAAACCAGAACTTCGCCTGGAGAACAGAGTGCAACCAGTGTAAGGCCCCA
AAGCCTGAAGGCTTCCTCCCGCCACCCTTTCCGCCCCCGGGTGGTGATCGTGGCAGAGGTGGCCCTGGTGGCATG
CGGGGAGGAAGAGGTGGCCTCATGGATCGTGGTGGTCCCGGTGGAATGTTCAGAGGTGGCCGTGGTGGAGACAGA
GGTGGCTTCCGTGGTGGCCGGGGCATGGACCGAGGTGGCTTTGGTGGAGGAAGACGAGGTGGCCCTGGGGGGCCC
CCTGGACCTTTGATGGAACAGGCGATCGCAGCTAGAAACGCCTACCTGAGAAAGAAAATCGCCAGACTGAAGAAG
GACAACCTGCAGCTGGAAAGAGACGAGCAGAACCTGGAAAAGATCATCGCCAACCTCAGAGATGAGATCGCCAGA
CTGGAAAACGAGGTGGCCAGCCACGAGCAGTATCCCTATGATGTGCCGGATTATGCTGGAGCACCAGGAAGTGCT
GGTTCTGCTGCTGGTAGTGGAGCTTCTAACTTTACTCAGTTCGTTCTCGTCGACAATGGCGGAACTGGCGACGTG
ACTGTCGCCCCAAGCAACTTCGCTAACGGGATCGCTGAATGGATCAGCTCTAACTCGCGTTCACAGGCTTACAAA
GTAACCTGTAGCGTTCGTCAGAGCTCTGCGCAGAATCGCAAATACACCATCAAAGTCGAGGTGCCTAAAGGCGCC
TGGCGTTCGTACTTAAATATGGAACTAACCATTCCAATTTTCGCCACGAATTCCGACTGCGAGCTTATTGTTAAG
GCAATGCAAGGTCTCCTAAAAGATGGAAACCCGATTCCCTCAGCAATCGCAGCAAACTCCGGCATCTACTAA
(SEQ ID NO: 533)

Protein:
MAITQFRLFKFCTCLATVFSFLKRLICRSMASTDYSTYSQAAAQQGYSAYTAQPTQGYAQTTQAYGQQSYGTYGQ
PTDVSYTQAQTTATYGQTAYATSYGQPPTGYTTPTAPQAYSQPVQGYGTGAYDTTTATVTTTQASYAAQSAYGTQ
PAYPAYGQQPAATAPTRPQDGNKPTETSQPQSSTGGYNQPSLGYGQSNYSYPQVPGSYPMQPVTAPPSYPPTSYS
STQPTSYDQSSYSQQNTYGQPSSYGQQSSYGQQSSYGQQPPTSYPPQTGSYSQAPSQYSQQSSSYGQQSSFRQDH
PSSMGVYGQESGGFSGPGENRSMSGPDNRGRGRGGFDRGGMSRGGRGGGRGGMGSAGERGGFNKPGGPMDEGPDL
DLGPPVDPDEDSDNSAIYVQGLNDSVTLDDLADFFKQCGVVKMNKRTGQPMIHIYLDKETGKPKGDATVSYEDPP
TAKAAVEWFDGKDFQGSKLKVSLARKKPPMNSMRGGLPPREGRGMPPPLRGGPGGPGGPGGPMGRMGGRGGDRGG
FPPRGPRGSRGNPSGGGNVQHRAGDWQCPNPGCGNQNFAWRTECNQCKAPKPEGFLPPPFPPPGGDRGRGGPGGM
RGGRGGLMDRGGPGGMFRGGRGGDRGGFRGGRGMDRGGFGGGRRGGPGGPPGPLMEQAIAARNAYLRKKIARLKK
DNLQLERDEQNLEKIIANLRDEIARLENEVASHEQYPYDVPDYAGAPGSAGSAAGSGASNFTQFVLVDNGGTGDV
TVAPSNFANGIAEWISSNSRSQAYKVTCSVRQSSAQNRKYTIKVEVPKGAWRSYLNMELTIPIFATNSDCELIVK
AMQGLLKDGNPIPSAIAANSGIY* (SEQ ID NO: 534)

TOM20::EWSR1::SYNZIP2::MCP
DNA:
ATGGTGGGTCGGAACAGCGCCATCGCCGCCGGTGTATGCGGGGCCCTTTTCATTGGGTACTGCATCTACTTCGAC
CGCAAAAGACGAAGTGACCCCAACTTCAAGAACAGGCTTCGAGAACGAAGAAAGAAACAGAAGCTTGCCAAGGAG
AGAGCTGGGCTTTCCAAGTTACCTGACCTTAAAGATGCTGAAGCTGTTCAGAAGTTCTTCATGGCGTCCACGGAT
TACAGTACCTATAGCCAAGCTGCAGCGCAGCAGGGCTACAGTGCTTACACCGCCCAGCCCACTCAAGGATATGCA
CAGACCACCCAGGCATATGGGCAACAAAGCTATGGAACCTATGGACAGCCCACTGATGTCAGCTATACCCAGGCT
CAGACCACTGCAACCTATGGGCAGACCGCCTATGCAACTTCTTATGGACAGCCTCCCACTGGTTATACTACTCCA
ACTGCCCCCCAGGCATACAGCCAGCCTGTCCAGGGGTATGGCACTGGTGCTTATGATACCACCACTGCTACAGTC
ACCACCACCCAGGCCTCCTATGCAGCTCAGTCTGCATATGGCACTCAGCCTGCTTATCCAGCCTATGGGCAGCAG
CCAGCAGCCACTGCACCTACAAGACCGCAGGATGGAAACAAGCCCACTGAGACTAGTCAACCTCAATCTAGCACA
GGGGGTTACAACCAGCCCAGCCTAGGATATGGACAGAGTAACTACAGTTATCCCCAGGTACCTGGGAGCTACCCC
ATGCAGCCAGTCACTGCACCTCCATCCTACCCTCCTACCAGCTATTCCTCTACACAGCCGACTAGTTATGATCAG
AGCAGTTACTCTCAGCAGAACACCTATGGGCAACCGAGCAGCTATGGACAGCAGAGTAGCTATGGTCAACAAAGC
AGCTATGGGCAGCAGCCTCCCACTAGTTACCCACCCCAAACTGGATCCTACAGCCAAGCTCCAAGTCAATATAGC
CAACAGAGCAGCAGCTACGGGCAGCAGAGTTCATTCCGACAGGACCACCCCAGTAGCATGGGTGTTTATGGGCAG
GAGTCTGGAGGATTTTCCGGACCAGGAGAGAACCGGAGCATGAGTGGCCCTGATAACCGGGGCAGGGGAAGAGGG
GGATTTGATCGTGGAGGCATGAGCAGAGGTGGGCGGGGAGGAGGACGCGGTGGAATGGGCAGCGCTGGAGAGCGA
GGTGGCTTCAATAAGCCTGGTGGACCCATGGATGAAGGACCAGATCTTGATCTAGGCCCACCTGTAGATCCAGAT
GAAGACTCTGACAACAGTGCAATTTATGTACAAGGATTAAATGACAGTGTGACTCTAGATGATCTGGCAGACTTC
TTTAAGCAGTGTGGGGTTGTTAAGATGAACAAGAGAACTGGGCAACCCATGATCCACATCTACCTGGACAAGGAA
ACAGGAAAGCCCAAAGGCGATGCCACAGTGTCCTATGAAGACCCACCTACTGCCAAGGCTGCCGTGGAATGGTTT
GATGGGAAAGATTTTCAAGGGAGCAAACTTAAAGTCTCCCTTGCTCGGAAGAAGCCTCCAATGAACAGTATGCGG
GGTGGTCTGCCACCCCGTGAGGGCAGAGGCATGCCACCACCACTCCGTGGAGGTCCAGGAGGCCCAGGAGGTCCT
GGGGGACCCATGGGTCGCATGGGAGGCCGTGGAGGAGATAGAGGAGGCTTCCCTCCAAGAGGACCCCGGGGTTCC
CGAGGGAACCCCTCTGGAGGAGGAAACGTCCAGCACCGAGCTGGAGACTGGCAGTGTCCCAATCCGGGTTGTGGA
AACCAGAACTTCGCCTGGAGAACAGAGTGCAACCAGTGTAAGGCCCCAAAGCCTGAAGGCTTCCTCCCGCCACCC
TTTCCGCCCCCGGGTGGTGATCGTGGCAGAGGTGGCCCTGGTGGCATGCGGGGAGGAAGAGGTGGCCTCATGGAT

CGTGGTGGTCCCGGTGGAATGTTCAGAGGTGGCCGTGGTGGAGACAGAGGTGGCTTCCGTGGTGGCCGGGGCATG
GACCGAGGTGGCTTTGGTGGAGGAAGACGAGGTGGCCCTGGGGGGCCCCCTGGACCTTTGATGGAACAGGCGATC
GCAGCTAGAAACGCCTACCTGAGAAAGAAAATCGCCAGACTGAAGAAGGACAACCTGCAGCTGGAAAGAGACGAG
CAGAACCTGGAAAAGATCATCGCCAACCTCAGAGATGAGATCGCCAGACTGGAAAACGAGGTGGCCAGCCACGAG
CAGTATCCCTATGATGTGCCGGATTATGCTGGAGCACCAGGAAGTGCTGGTTCTGCTGCTGGTAGTGGAGCTTCT
AACTTTACTCAGTTCGTTCTCGTCGACAATGGCGGAACTGGCGACGTGACTGTCGCCCCAAGCAACTTCGCTAAC
GGGATCGCTGAATGGATCAGCTCTAACTCGCGTTCACAGGCTTACAAAGTAACCTGTAGCGTTCGTCAGAGCTCT
GCGCAGAATCGCAAATACACCATCAAAGTCGAGGTGCCTAAAGGCGCCTGGCGTTCGTACTTAAATATGGAACTA
ACCATTCCAATTTTCGCCACGAATTCCGACTGCGAGCTTATTGTTAAGGCAATGCAAGGTCTCCTAAAAGATGGA
AACCCGATTCCCTCAGCAATCGCAGCAAACTCCGGCATCTACTAA (SEQ ID NO: 535)

Protein:
MVGRNSAIAAGVCGALFIGYCIYFDRKRRSDPNFKNRLRERRKKQKLAKERAGLSKLPDLKDAEAVQKFFMASTD
YSTYSQAAAQQGYSAYTAQPTQGYAQTTQAYGQQSYGTYGQPTDVSYTQAQTTATYGQTAYATSYGQPPTGYTTP
TAPQAYSQPVQGYGTGAYDTTTATVTTTQASYAAQSAYGTQPAYPAYGQQPAATAPTRPQDGNKPTETSQPQSST
GGYNQPSLGYGQSNYSYPQVPGSYPMQPVTAPPSYPPTSYSSTQPTSYDQSSYSQQNTYGQPSSYGQQSSYGQQS
SYGQQPPTSYPPQTGSYSQAPSQYSQQSSSYGQQSSFRQDHPSSMGVYGQESGGFSGPGENRSMSGPDNRGRGRG
GFDRGGMSRGGRGGGRGGMGSAGERGGFNKPGGPMDEGPDLDLGPPVDPDEDSDNSAIYVQGLNDSVTLDDLADF
FKQCGVVKMNKRTGQPMIHIYLDKETGKPKGDATVSYEDPPTAKAAVEWFDGKDFQGSKLKVSLARKKPPMNSMR
GGLPPREGRGMPPPLRGGPGGPGGPGGPMGRMGGRGGDRGGFPPRGPRGSRGNPSGGGNVQHRAGDWQCPNPGCG
NQNFAWRTECNQCKAPKPEGFLPPPFPPPGGDRGRGGPGGMRGGRGGLMDRGGPGGMFRGGRGGDRGGFRGGRGM
DRGGFGGGRRGGPGGPPGPLMEQAIAARNAYLRKKIARLKKDNLQLERDEQNLEKIIANLRDEIARLENEVASHE
QYPYDVPDYAGAPGSAGSAAGSGASNFTQFVLVDNGGTGDVTVAPSNFANGIAEWISSNSRSQAYKVTCSVRQSS
AQNRKYTIKVEVPKGAWRSYLNMELTIPIFATNSDCELIVKAMQGLLKDGNPIPSAIAANSGIY* (SEQ ID
NO: 536)

TOM20::FUS::SYNZIP4::4xλN22::PylRS(AA)
DNA:
ATGGTGGGTCGGAACAGCGCCATCGCCGCCGGTGTATGCGGGGCCCTTTTCATTGGGTACTGCATCTACTTCGAC
CGCAAAAGACGAAGTGACCCCAACTTCAAGAACAGGCTTCGAGAACGAAGAAAGAAACAGAAGCTTGCCAAGGAG
AGAGCTGGGCTTTCCAAGTTACCTGACCTTAAAGATGCTGAAGCTGTTCAGAAATTCTTCATGGCCTCAAACGAT
TATACCCAACAAGCAACCCAAAGCTATGGGGCCTACCCCACCCAGCCCGGGCAGGGCTATTCCCAGCAGAGCAGT
CAGCCCTACGGACAGCAGAGTTACAGTGGTTATAGCCAGTCCACGGACACTTCAGGATATGGCCAGAGCAGCTAT
TCTTCTTATGGCCAGAGCCAGAACACAGGCTATGGAACTCAGTCAACTCCCCAGGGATATGGCTCGACTGGCGGC
TATGGCAGTAGCCAGAGCTCCCAATCGTCTTACGGGCAGCAGTCCTCCTACCCTGGCTATGGCCAGCAGCCAGCT
CCCAGCAGCACCTCGGGAAGTTACGGTAGCAGTTCTCAGAGCAGCAGCTATGGGCAGCCCCAGAGTGGGAGCTAC
AGCCAGCAGCCTAGCTATGGTGGACAGCAGCAAAGCTATGGACAGCAGCAAAGCTATAATCCCCCTCAGGGCTAT
GGACAGCAGAACCAGTACAACAGCAGCAGTGGTGGTGGAGGTGGAGGTGGAGGTGGAGGTAACTATGGCCAAGAT
CAATCCTCCATGAGTAGTGGTGGTGGCAGTGGTGGCGGTTATGGCAATCAAGACCAGAGTGGTGGAGGTGGCAGC
GGTGGCTATGGACAGCAGGACCGTGGAGGCCGCGGCAGGGGTGGCAGTGGTGGCGGCGGCGGCGGCGGCGGTGGT
GGTTACAACCGCAGCAGTGGTGGCTATGAACCCAGAGGTCGTGGAGGTGGCCGTGGAGGCAGAGGTGGCATGGGC
GGAAGTGACCGTGGTGGCTTCAATAAATTTGGTGGCCCTCGGGACCAAGGATCACGTCATGACTCCGAACAGGAT
AATTCAGACAACAACACCATCTTTGTGCAAGGCCTGGGTGAGAATGTTACAATTGAGTCTGTGGCTGATTACTTC
AAGCAGATTGGTATTATTAAGACAAACAAGAAAACGGGACAGCCCATGATTAATTTGTACACAGACAGGGAAACT
GGCAAGCTGAAGGGAGAGGCAACGGTCTCTTTTGATGACCCACCTTCAGCTAAAGCAGCTATTGACTGGTTTGAT
GGTAAAGAATTCTCCGGAAATCCTATCAAGGTCTCATTTGCTACTCGCCGGGCAGACTTTAATCGGGGTGGTGGC
AATGGTCGTGGAGGCCGAGGGCGAGGAGGACCCATGGGCCGTGGAGGCTATGGAGGTGGTGGCAGTGGTGGTGGT
GGCCGAGGAGGATTTCCCAGTGGAGGTGGTGGCGGTGGAGGACAGCAGCGAGCTGGTGACTGGAAGTGTCCTAAT
CCCACCTGTGAGAATATGAACTTCTCTTGGAGGAATGAATGCAACCAGTGTAAGGCCCCTAAACCAGATGGCCCA
GGAGGGGGACCAGGTGGCTCTCACATGGGGGGTAACTACGGGGATGATCGTCGTGGTGGCAGAGGAGGCGCGATC
GCACAAAAGGTGGCTGAACTGAAAAATAGAGTGGCCGTGAAGCTGAACCGGAACGAGCAGCTGAAGAACAAGGTG
GAAGAGCTGAAGAACAGAAACGCCTACCTGAAGAATGAGCTGGCCACCCTGGAAAACGAGGTGGCCAGACTGGAA
AACGACGTGGCCGAGTTAATCGCAGGAGCACCAGGAAGTGCTGGTTCTGCTGCTGGTAGTGGAGCATCGATAGAG
CAGAAGCTGATCTCAGAGGAGGACCTGCTAGCCACCATGGACGCACAAACACGACGACGTGAGCGTCGCGCTGAG
AAACAAGCTCAATGGAAAGCTGCAAACCCACCGCTCGACGGAGCCGGAGCTGGCGCTGGAGCTGGAGCCGGAGCT
GGCGGTCTAGCCACCATGGACGCACAAACACGACGACGTGAGCGTCGCGCTGAGAAACAAGCTCAATGGAAAGCT
GCAAACCCACCGCTCGACGGAGCCGGAGCTGGCGCTGGAGCTGGAGCCGGAGCTGGCGGTCTAGCCACCATGGAC

GCACAAACACGACGACGTGAGCGTCGCGCTGAGAAACAAGCTCAATGGAAAGCTGCAAACCCACCGCTCGACGGA
GCCGGAGCTGGCGCTGGAGCTGGAGCCGGAGCTGGCGGTCTAGCCACCATGGACGCACAAACACGACGACGTGAG
CGTCGCGCTGAGAAACAAGCTCAATGGAAAGCTGCAAACCCACCGCTCGAGTCTAGAGGGCCCGTTGGTGCTCCT
GGTTCAGCAGGAAGCGCAGCAGGATCAGGTGCGTGCCCGGTGCCGCTGCAGCTGCCGCCGCTGGAACGCCTGACC
CTGGATGACAAAAAACCGCTGAATACCCTGATCTCTGCTACTGGTCTGTGGATGAGTCGTACCGGAACCATTCAT
AAAATCAAACACCACGAGGTTAGCCGTTCGAAAATCTATATTGAGATGGCGTGTGGCGATCATCTGGTTGTGAAC
AATAGCCGCTCTTCTCGTACAGCACGTGCACTGCGTCACCACAAATATCGTAAAACCTGTAAACGTTGCCGTGTG
TCCGATGAGGATCTGAACAAATTCCTGACAAAAGCCAATGAGGACCAAACAAGCGTGAAAGTGAAAGTCGTTAGC
GCTCCTACCCGTACTAAAAAAGCAATGCCGAAATCCGTTGCTCGTGCCCCTAAACCACTGGAAAACACTGAAGCA
GCACAGGCACAGCCGTCTGGAAGCAAATTCTCTCCGGCCATTCCTGTTTCTACCCAGGAGTCCGTTTCTGTTCCA
GCAAGTGTGAGCACCAGCATTAGCAGTATTAGCACCGGTGCCACCGCTAGCGCCCTGGTTAAAGGCAATACCAAT
CCGATTACAAGCATGTCTGCCCCGGTTCAAGCATCAGCTCCAGCACTGACAAAATCCCAAACCGATCGTCTGGAG
GTTCTGCTGAATCCGAAAGACGAAATCAGCCTGAATTCCGGCAAACCGTTTCGTGAACTGGAGAGCGAACTGCTG
TCACGTCGTAAAAAAGACCTGCAACAAATCTATGCCGAAGAACGTGAGAACTATCTGGGGAAACTGGAACGTGAA
ATCACCCGCTTTTTCGTGGATCGTGGCTTTCTGGAGATCAAATCCCCGATTCTGATTCCTCTGGAGTATATCGAG
CGTATGGGCATCGACAATGATACCGAACTGAGCAAACAAATTTTCCGTGTGGATAAAAACTTCTGTCTGCGCCCT
ATGCTGGCACCAAATCTGTATAACTATCTGCGCAAACTGGACCGTGCCCTGCCTGATCCTATCAAAATCTTCGAG
ATCGGCCCGTGTTATCGTAAAGAGTCCGACGGTAAAGAACATCTGGAGGAGTTTACCATGCTGGCCTTTGCCCAA
ATGGGTTCAGGTTGTACTCGTGAGAACCTGGAAAGCATCATCACCGATTTTCTGAACCACCTGGGCATTGACTTC
AAAATTGTGGGCGACAGCTGTATGGTGTATGGCGACACCCTGGATGTCATGCACGGCGACCTGGAACTGTCTAGT
GCCGTTGTTGGACCAATTCCGCTGGACCGTGAGTGGGGTATCGACAAACCGTGGATCGGAGCAGGATTCGGTCTG
GAACGCCTGCTGAAAGTGAAACACGACTTCAAAAACATCAAACGTGCCGCCCGTTCTGAATCGTATTATAACGGG
ATCTCTACGAACCTGTAA (SEQ ID NO: 537)

Protein:
MVGRNSAIAAGVCGALFIGYCIYFDRKRRSDPNFKNRLRERRKKQKLAKERAGLSKLPDLKDAEAVQKFFMASND
YTQQATQSYGAYPTQPGQGYSQQSSQPYGQQSYSGYSQSTDTSGYGQSSYSSYGQSQNTGYGTQSTPQGYGSTGG
YGSSQSSQSSYGQQSSYPGYGQQPAPSSTSGSYGSSSQSSSYGQPQSGSYSQQPSYGGQQQSYGQQQSYNPPQGY
GQQNQYNSSSGGGGGGGGGGNYGQDQSSMSSGGGSGGGYGNQDQSGGGGSGGYGQQDRGGRGRGGSGGGGGGGGG
GYNRSSGGYEPRGRGGGRGGRGGMGGSDRGGFNKFGGPRDQGSRHDSEQDNSDNNTIFVQGLGENVTIESVADYF
KQIGIIKTNKKTGQPMINLYTDRETGKLKGEATVSFDDPPSAKAAIDWFDGKEFSGNPIKVSFATRRADFNRGGG
NGRGGRGRGGPMGRGGYGGGGSGGGGRGGFPSGGGGGGGQQRAGDWKCPNPTCENMNFSWRNECNQCKAPKPDGP
GGGPGGSHMGGNYGDDRRGGRGGAIAQKVAELKNRVAVKLNRNEQLKNKVEELKNRNAYLKNELATLENEVARLE
NDVAELIAGAPGSAGSAAGSGASIEQKLISEEDLLATMDAQTRRRERRAEKQAQWKAANPPLDGAGAGAGAGAGA
GGLATMDAQTRRRERRAEKQAQWKAANPPLDGAGAGAGAGAGAGGLATMDAQTRRRERRAEKQAQWKAANPPLDG
AGAGAGAGAGAGGLATMDAQTRRRERRAEKQAQWKAANPPLESRGPVGAPGSAGSAAGSGACPVPLQLPPLERLT
LDDKKPLNTLISATGLWMSRTGTIHKIKHHEVSRSKIYIEMACGDHLVVNNSRSSRTARALRHHKYRKTCKRCRV
SDEDLNKFLTKANEDQTSVKVKVVSAPTRTKKAMPKSVARAPKPLENTEAAQAQPSGSKFSPAIPVSTQESVSVP
ASVSTSISSISTGATASALVKGNTNPITSMSAPVQASAPALTKSQTDRLEVLLNPKDEISLNSGKPFRELESELL
SRRKKDLQQIYAEERENYLGKLEREITRFFVDRGFLEIKSPILIPLEYIERMGIDNDTELSKQIFRVDKNFCLRP
MLAPNLYNYLRKLDRALPDPIKIFEIGPCYRKESDGKEHLEEFTMLAFAQMGSGCTRENLESIITDFLNHLGIDF
KIVGDSCMVYGDTLDVMHGDLELSSAVVGPIPLDREWGIDKPWIGAGFGLERLLKVKHDFKNIKRAARSESYYNG
ISTNL* (SEQ ID NO: 538)

KIF16B::EWSR1::SYNZIP4::4xλN22
DNA:
ATGGCATCGGTCAAGGTGGCCGTGAGGGTCCGGCCCATGAATCGCAGGGAAAAGGACTTGGAGGCCAAGTTCATT
ATTCAGATGGAGAAAAGCAAAACGACAATCACAAACTTAAAGATACCAGAAGGAGGCACTGGGGACTCAGGAAGA
GAACGGACCAAGACCTTCACCTATGACTTTTCTTTTTATTCTGCTGATACAAAAAGCCCAGATTACGTTTCACAA
GAAATGGTTTTCAAAACCCTCGGCACAGATGTCGTGAAGTCTGCATTTGAAGGTTATAATGCTTGTGTCTTTGCA
TATGGGCAAACTGGATCTGGAAAGTCATACACTATGATGGGAAATTCTGGAGATTCTGGCTTAATACCTCGGATC
TGTGAAGGACTCTTCAGTCGGATAAATGAAACCACCAGATGGGATGAAGCTTCTTTTCGAACTGAAGTCAGCTAC
TTAGAAATTTATAACGAACGTGTGAGAGATCTACTTCGGCGGAAGTCATCTAAAACCTTCAATTTGAGAGTCCGT
GAGCATCCCAAAGAAGGCCCTTATGTTGAGGATTTATCCAAACATTTAGTACAGAATTATGGTGACGTAGAAGAA
CTTATGGATGCGGGCAATATCAACCGGACCACCGCAGCGACTGGGATGAACGACGTCAGTAGCAGGTCTCATGCC
ATCTTCACCATCAAGTTCACTCAGGCTAAATTTGATTCTGAAATGCCATGTGAAACCGTCAGTAAGATCCACTTG
GTTGATCTTGCCGGAAGTGAGCGTGCAGATGCCACCGGAGCCACCGGGGTTAGGCTAAAGGAAGGGGGAAATATT

AACAAGTCCCTCGTGACTCTGGGGAACGTCATTTCTGCCTTAGCTGATTTATCTCAGGATGCTGCAAATACTCTT
GCAAAGAAGAAGCAAGTTTTCGTGCCTTACAGGGATTCTGTGTTGACTTGGTTGTTAAAAGATAGCCTTGGAGGA
AACTCTAAAACTATCATGATTGCCACCATTTCACCTGCTGATGTCAATTATGGAGAAACCCTAAGTACTCTTCGC
TATGCAAATAGAGCCAAAAACATCATCAACAAGCCTACCATTAATGAGGATGCCAACGTCAAACTTATCCGTGAG
CTGCGAGCTGAAATAGCCAGACTGAAAACGCTGCTTGCTCAAGGGAATCAGATTGCCCTCTTAGACTCCCCCACA
ATGGCGTCCACGGATTACAGTACCTATAGCCAAGCTGCAGCGCAGCAGGGCTACAGTGCTTACACCGCCCAGCCC
ACTCAAGGATATGCACAGACCACCCAGGCATATGGGCAACAAAGCTATGGAACCTATGGACAGCCCACTGATGTC
AGCTATACCCAGGCTCAGACCACTGCAACCTATGGGCAGACCGCCTATGCAACTTCTTATGGACAGCCTCCCACT
GGTTATACTACTCCAACTGCCCCCCAGGCATACAGCCAGCCTGTCCAGGGGTATGGCACTGGTGCTTATGATACC
ACCACTGCTACAGTCACCACCACCCAGGCCTCCTATGCAGCTCAGTCTGCATATGGCACTCAGCCTGCTTATCCA
GCCTATGGGCAGCAGCCAGCAGCCACTGCACCTACAAGACCGCAGGATGGAAACAAGCCCACTGAGACTAGTCAA
CCTCAATCTAGCACAGGGGGTTACAACCAGCCCAGCCTAGGATATGGACAGAGTAACTACAGTTATCCCCAGGTA
CCTGGGAGCTACCCCATGCAGCCAGTCACTGCACCTCCATCCTACCCTCCTACCAGCTATTCCTCTACACAGCCG
ACTAGTTATGATCAGAGCAGTTACTCTCAGCAGAACACCTATGGGCAACCGAGCAGCTATGGACAGCAGAGTAGC
TATGGTCAACAAAGCAGCTATGGGCAGCAGCCTCCCACTAGTTACCCACCCCAAACTGGATCCTACAGCCAAGCT
CCAAGTCAATATAGCCAACAGAGCAGCAGCTACGGGCAGCAGAGTTCATTCCGACAGGACCACCCCAGTAGCATG
GGTGTTTATGGGCAGGAGTCTGGAGGATTTTCCGGACCAGGAGAGAACCGGAGCATGAGTGGCCCTGATAACCGG
GGCAGGGGAAGAGGGGGATTTGATCGTGGAGGCATGAGCAGAGGTGGGCGGGGAGGAGGACGCGGTGGAATGGGC
AGCGCTGGAGAGCGAGGTGGCTTCAATAAGCCTGGTGGACCCATGGATGAAGGACCAGATCTTGATCTAGGCCCA
CCTGTAGATCCAGATGAAGACTCTGACAACAGTGCAATTTATGTACAAGGATTAAATGACAGTGTGACTCTAGAT
GATCTGGCAGACTTCTTTAAGCAGTGTGGGGTTGTTAAGATGAACAAGAGAACTGGGCAACCCATGATCCACATC
TACCTGGACAAGGAAACAGGAAAGCCCAAAGGCGATGCCACAGTGTCCTATGAAGACCCACCTACTGCCAAGGCT
GCCGTGGAATGGTTTGATGGGAAAGATTTTCAAGGGAGCAAACTTAAAGTCTCCCTTGCTCGGAAGAAGCCTCCA
ATGAACAGTATGCGGGGTGGTCTGCCACCCCGTGAGGGCAGAGGCATGCCACCACCACTCCGTGGAGGTCCAGGA
GGCCCAGGAGGTCCTGGGGGACCCATGGGTCGCATGGGAGGCCGTGGAGGAGATAGAGGAGGCTTCCCTCCAAGA
GGACCCCGGGGTTCCCGAGGGAACCCCTCTGGAGGAGGAAACGTCCAGCACCGAGCTGGAGACTGGCAGTGTCCC
AATCCGGGTTGTGGAAACCAGAACTTCGCCTGGAGAACAGAGTGCAACCAGTGTAAGGCCCCAAAGCCTGAAGGC
TTCCTCCCGCCACCCTTTCCGCCCCCGGGTGGTGATCGTGGCAGAGGTGGCCCTGGTGGCATGCGGGGAGGAAGA
GGTGGCCTCATGGATCGTGGTGGTCCCGGTGGAATGTTCAGAGGTGGCCGTGGTGGAGACAGAGGTGGCTTCCGT
GGTGGCCGGGGCATGGACCGAGGTGGCTTTGGTGGAGGAAGACGAGGTGGCCCTGGGGGGCCCCCTGGACCTTTG
ATGGAACAGGCGATCGCACAAAAGGTGGCTGAACTGAAAAATAGAGTGGCCGTGAAGCTGAACCGGAACGAGCAG
CTGAAGAACAAGGTGGAAGAGCTGAAGAACAGAAACGCCTACCTGAAGAATGAGCTGGCCACCCTGGAAAACGAG
GTGGCCAGACTGGAAAACGACGTGGCCGAGGGAGCACCAGGAAGTGCTGGTTCTGCTGCTGGTAGTGGAGAGCAG
AAGCTGATCTCAGAGGAGGACCTGCTAGCCACCATGGACGCACAAACACGACGACGTGAGCGTCGCGCTGAGAAA
CAAGCTCAATGGAAAGCTGCAAACCCACCGCTCGACGGAGCCGGAGCTGGCGCTGGAGCTGGAGCCGGAGCTGGC
GGTCTAGCCACCATGGACGCACAAACACGACGACGTGAGCGTCGCGCTGAGAAACAAGCTCAATGGAAAGCTGCA
AACCCACCGCTCGACGGAGCCGGAGCTGGCGCTGGAGCTGGAGCCGGAGCTGGCGGTCTAGCCACCATGGACGCA
CAAACACGACGACGTGAGCGTCGCGCTGAGAAACAAGCTCAATGGAAAGCTGCAAACCCACCGCTCGACGGAGCC
GGAGCTGGCGCTGGAGCTGGAGCCGGAGCTGGCGGTCTAGCCACCATGGACGCACAAACACGACGACGTGAGCGT
CGCGCTGAGAAACAAGCTCAATGGAAAGCTGCAAACCCACCGCTCGAGTCTAGAGGGCCCGTTTAA (SEQ ID
NO: 539)

Protein:
MASVKVAVRVRPMNRREKDLEAKFIIQMEKSKTTITNLKIPEGGTGDSGRERTKTFTYDFSFYSADTKSPDYVSQ
EMVFKTLGTDVVKSAFEGYNACVFAYGQTGSGKSYTMMGNSGDSGLIPRICEGLFSRINETTRWDEASFRTEVSY
LEIYNERVRDLLRRKSSKTFNLRVREHPKEGPYVEDLSKHLVQNYGDVEELMDAGNINRTTAATGMNDVSSRSHA
IFTIKFTQAKFDSEMPCETVSKIHLVDLAGSERADATGATGVRLKEGGNINKSLVTLGNVISALADLSQDAANTL
AKKKQVFVPYRDSVLTWLLKDSLGGNSKTIMIATISPADVNYGETLSTLRYANRAKNIINKPTINEDANVKLIRE
LRAEIARLKTLLAQGNQIALLDSPTMASTDYSTYSQAAAQQGYSAYTAQPTQGYAQTTQAYGQQSYGTYGQPTDV
SYTQAQTTATYGQTAYATSYGQPPTGYTTPTAPQAYSQPVQGYGTGAYDTTTATVTTTQASYAAQSAYGTQPAYP
AYGQQPAATAPTRPQDGNKPTETSQPQSSTGGYNQPSLGYGQSNYSYPQVPGSYPMQPVTAPPSYPPTSYSSTQP
TSYDQSSYSQQNTYGQPSSYGQQSSYGQQSSYGQQPPTSYPPQTGSYSQAPSQYSQQSSSYGQQSSFRQDHPSSM
GVYGQESGGFSGPGENRSMSGPDNRGRGRGGFDRGGMSRGGRGGGRGGMGSAGERGGFNKPGGPMDEGPDLDLGP
PVDPDEDSDNSAIYVQGLNDSVTLDDLADFFKQCGVVKMNKRTGQPMIHIYLDKETGKPKGDATVSYEDPPTAKA
AVEWFDGKDFQGSKLKVSLARKKPPMNSMRGGLPPREGRGMPPPLRGGPGGPGGPGGPMGRMGGRGGDRGGFPPR
GPRGSRGNPSGGGNVQHRAGDWQCPNPGCGNQNFAWRTECNQCKAPKPEGFLPPPFPPPGGDRGRGGPGGMRGGR
GGLMDRGGPGGMFRGGRGGDRGGFRGGRGMDRGGFGGGRRGGPGGPPGPLMEQAIAQKVAELKNRVAVKLNRNEQ
LKNKVEELKNRNAYLKNELATLENEVARLENDVAEGAPGSAGSAAGSGEQKLISEEDLLATMDAQTRRRERRAEK

QAQWKAANPPLDGAGAGAGAGAGAGGLATMDAQTRRRERRAEKQAQWKAANPPLDGAGAGAGAGAGAGGLATMDA
QTRRRERRAEKQAQWKAANPPLDGAGAGAGAGAGAGGLATMDAQTRRRERRAEKQAQWKAANPPLESRGPV*
(SEQ ID NO: 540)

TOM20::EWSR1::SYNZP4::4xλN22
DNA:
ATGGTGGGTCGGAACAGCGCCATCGCCGCCGGTGTATGCGGGGCCCTTTTCATTGGGTACTGCATCTACTTCGAC
CGCAAAAGACGAAGTGACCCCAACTTCAAGAACAGGCTTCGAGAACGAAGAAAGAAACAGAAGCTTGCCAAGGAG
AGAGCTGGGCTTTCCAAGTTACCTGACCTTAAAGATGCTGAAGCTGTTCAGAAGTTCTTCATGGCGTCCACGGAT
TACAGTACCTATAGCCAAGCTGCAGCGCAGCAGGGCTACAGTGCTTACACCGCCCAGCCCACTCAAGGATATGCA
CAGACCACCCAGGCATATGGGCAACAAAGCTATGGAACCTATGGACAGCCCACTGATGTCAGCTATACCCAGGCT
CAGACCACTGCAACCTATGGGCAGACCGCCTATGCAACTTCTTATGGACAGCCTCCCACTGGTTATACTACTCCA
ACTGCCCCCCAGGCATACAGCCAGCCTGTCCAGGGGTATGGCACTGGTGCTTATGATACCACCACTGCTACAGTC
ACCACCACCCAGGCCTCCTATGCAGCTCAGTCTGCATATGGCACTCAGCCTGCTTATCCAGCCTATGGGCAGCAG
CCAGCAGCCACTGCACCTACAAGACCGCAGGATGGAAACAAGCCCACTGAGACTAGTCAACCTCAATCTAGCACA
GGGGGTTACAACCAGCCCAGCCTAGGATATGGACAGAGTAACTACAGTTATCCCCAGGTACCTGGGAGCTACCCC
ATGCAGCCAGTCACTGCACCTCCATCCTACCCTCCTACCAGCTATTCCTCTACACAGCCGACTAGTTATGATCAG
AGCAGTTACTCTCAGCAGAACACCTATGGGCAACCGAGCAGCTATGGACAGCAGAGTAGCTATGGTCAACAAAGC
AGCTATGGGCAGCAGCCTCCCACTAGTTACCCACCCCAAACTGGATCCTACAGCCAAGCTCCAAGTCAATATAGC
CAACAGAGCAGCAGCTACGGGCAGCAGAGTTCATTCCGACAGGACCACCCCAGTAGCATGGGTGTTTATGGGCAG
GAGTCTGGAGGATTTTCCGGACCAGGAGAGAACCGGAGCATGAGTGGCCCTGATAACCGGGGCAGGGGAAGAGGG
GGATTTGATCGTGGAGGCATGAGCAGAGGTGGGCGGGGAGGAGGACGCGGTGGAATGGGCAGCGCTGGAGAGCGA
GGTGGCTTCAATAAGCCTGGTGGACCCATGGATGAAGGACCAGATCTTGATCTAGGCCCACCTGTAGATCCAGAT
GAAGACTCTGACAACAGTGCAATTTATGTACAAGGATTAAATGACAGTGTGACTCTAGATGATCTGGCAGACTTC
TTTAAGCAGTGTGGGGTTGTTAAGATGAACAAGAGAACTGGGCAACCCATGATCCACATCTACCTGGACAAGGAA
ACAGGAAAGCCCAAAGGCGATGCCACAGTGTCCTATGAAGACCCACCTACTGCCAAGGCTGCCGTGGAATGGTTT
GATGGGAAAGATTTTCAAGGGAGCAAACTTAAAGTCTCCCTTGCTCGGAAGAAGCCTCCAATGAACAGTATGCGG
GGTGGTCTGCCACCCCGTGAGGGCAGAGGCATGCCACCACCACTCCGTGGAGGTCCAGGAGGCCCAGGAGGTCCT
GGGGGACCCATGGGTCGCATGGGAGGCCGTGGAGGAGATAGAGGAGGCTTCCCTCCAAGAGGACCCCGGGGTTCC
CGAGGGAACCCCTCTGGAGGAGGAAACGTCCAGCACCGAGCTGGAGACTGGCAGTGTCCCAATCCGGGTTGTGGA
AACCAGAACTTCGCCTGGAGAACAGAGTGCAACCAGTGTAAGGCCCCAAAGCCTGAAGGCTTCCTCCCGCCACCC
TTTCCGCCCCCGGGTGGTGATCGTGGCAGAGGTGGCCCTGGTGGCATGCGGGGAGGAAGAGGTGGCCTCATGGAT
CGTGGTGGTCCCGGTGGAATGTTCAGAGGTGGCCGTGGTGGAGACAGAGGTGGCTTCCGTGGTGGCCGGGGCATG
GACCGAGGTGGCTTTGGTGGAGGAAGACGAGGTGGCCCTGGGGGGCCCCCTGGACCTTTGATGGAACAGGCGATC
GCACAAAAGGTGGCTGAACTGAAAAATAGAGTGGCCGTGAAGCTGAACCGGAACGAGCAGCTGAAGAACAAGGTG
GAAGAGCTGAAGAACAGAAACGCCTACCTGAAGAATGAGCTGGCCACCCTGGAAAACGAGGTGGCCAGACTGGAA
AACGACGTGGCCGAGGGAGCACCAGGAAGTGCTGGTTCTGCTGCTGGTAGTGGAGAGCAGAAGCTGATCTCAGAG
GAGGACCTGCTAGCCACCATGGACGCACAAACACGACGACGTGAGCGTCGCGCTGAGAAACAAGCTCAATGGAAA
GCTGCAAACCCACCGCTCGACGGAGCCGGAGCTGGCGCTGGAGCTGGAGCCGGAGCTGGCGGTCTAGCCACCATG
GACGCACAAACACGACGACGTGAGCGTCGCGCTGAGAAACAAGCTCAATGGAAAGCTGCAAACCCACCGCTCGAC
GGAGCCGGAGCTGGCGCTGGAGCTGGAGCCGGAGCTGGCGGTCTAGCCACCATGGACGCACAAACACGACGACGT
GAGCGTCGCGCTGAGAAACAAGCTCAATGGAAAGCTGCAAACCCACCGCTCGACGGAGCCGGAGCTGGCGCTGGA
GCTGGAGCCGGAGCTGGCGGTCTAGCCACCATGGACGCACAAACACGACGACGTGAGCGTCGCGCTGAGAAACAA
GCTCAATGGAAAGCTGCAAACCCACCGCTCGAGTCTAGAGGGCCCGTTTAA (SEQ ID NO: 541)

Protein:
MVGRNSAIAAGVCGALFIGYCIYFDRKRRSDPNFKNRLRERRKKQKLAKERAGLSKLPDLKDAEAVQKFFMASTD
YSTYSQAAAQQGYSAYTAQPTQGYAQTTQAYGQQSYGTYGQPTDVSYTQAQTTATYGQTAYATSYGQPPTGYTTP
TAPQAYSQPVQGYGTGAYDTTTATVTTTQASYAAQSAYGTQPAYPAYGQQPAATAPTRPQDGNKPTETSQPQSST
GGYNQPSLGYGQSNYSYPQVPGSYPMQPVTAPPSYPPTSYSSTQPTSYDQSSYSQQNTYGQPSSYGQQSSYGQQS
SYGQQPPTSYPPQTGSYSQAPSQYSQQSSSYGQQSSFRQDHPSSMGVYGQESGGFSGPGENRSMSGPDNRGRGRG
GFDRGGMSRGGRGGGRGGMGSAGERGGFNKPGGPMDEGPDLDLGPPVDPDEDSDNSAIYVQGLNDSVTLDDLADF
FKQCGVVKMNKRTGQPMIHIYLDKETGKPKGDATVSYEDPPTAKAAVEWFDGKDFQGSKLKVSLARKKPPMNSMR
GGLPPREGRGMPPPLRGGPGGPGGPGGPMGRMGGRGGDRGGFPPRGPRGSRGNPSGGGNVQHRAGDWQCPNPGCG
NQNFAWRTECNQCKAPKPEGFLPPPFPPPGGDRGRGGPGGMRGGRGGLMDRGGPGGMFRGGRGGDRGGFRGGRGM
DRGGFGGGRRGGPGGPPGPLMEQAIAQKVAELKNRVAVKLNRNEQLKNKVEELKNRNAYLKNELATLENEVARLE
NDVAEGAPGSAGSAAGSGEQKLISEEDLLATMDAQTRRRERRAEKQAQWKAANPPLDGAGAGAGAGAGAGGLATM

DAQTRRRERRAEKQAQWKAANPPLDGAGAGAGAGAGAGGLATMDAQTRRRERRAEKQAQWKAANPPLDGAGAGAG
AGAGAGGLATMDAQTRRRERRAEKQAQWKAANPPLESRGPV* (SEQ ID NO: 542)

TOM20::FUS::SYNZIP1::PylRS(AF)
DNA:
ATGGTGGGTCGGAACAGCGCCATCGCCGCCGGTGTATGCGGGGCCCTTTTCATTGGGTACTGCATCTACTTCGAC
CGCAAAAGACGAAGTGACCCCAACTTCAAGAACAGGCTTCGAGAACGAAGAAAGAAACAGAAGCTTGCCAAGGAG
AGAGCTGGGCTTTCCAAGTTACCTGACCTTAAAGATGCTGAAGCTGTTCAGAAATTCTTCATGGCCTCAAACGAT
TATACCCAACAAGCAACCCAAAGCTATGGGGCCTACCCCACCCAGCCCGGGCAGGGCTATTCCCAGCAGAGCAGT
CAGCCCTACGGACAGCAGAGTTACAGTGGTTATAGCCAGTCCACGGACACTTCAGGATATGGCCAGAGCAGCTAT
TCTTCTTATGGCCAGAGCCAGAACACAGGCTATGGAACTCAGTCAACTCCCCAGGGATATGGCTCGACTGGCGGC
TATGGCAGTAGCCAGAGCTCCCAATCGTCTTACGGGCAGCAGTCCTCCTACCCTGGCTATGGCCAGCAGCCAGCT
CCCAGCAGCACCTCGGGAAGTTACGGTAGCAGTTCTCAGAGCAGCAGCTATGGGCAGCCCCAGAGTGGGAGCTAC
AGCCAGCAGCCTAGCTATGGTGGACAGCAGCAAAGCTATGGACAGCAGCAAAGCTATAATCCCCCTCAGGGCTAT
GGACAGCAGAACCAGTACAACAGCAGCAGTGGTGGTGGAGGTGGAGGTGGAGGTGGAGGTAACTATGGCCAAGAT
CAATCCTCCATGAGTAGTGGTGGTGGCAGTGGTGGCGGTTATGGCAATCAAGACCAGAGTGGTGGAGGTGGCAGC
GGTGGCTATGGACAGCAGGACCGTGGAGGCCGCGGCAGGGGTGGCAGTGGTGGCGGCGGCGGCGGCGGTGGT
GGTTACAACCGCAGCAGTGGTGGCTATGAACCCAGAGGTCGTGGAGGTGGCCGTGGAGGCAGAGGTGGCATGGGC
GGAAGTGACCGTGGTGGCTTCAATAAATTTGGTGGCCCTCGGGACCAAGGATCACGTCATGACTCCGAACAGGAT
AATTCAGACAACAACACCATCTTTGTGCAAGGCCTGGGTGAGAATGTTACAATTGAGTCTGTGGCTGATTACTTC
AAGCAGATTGGTATTATTAAGACAAACAAGAAAACGGGACAGCCCATGATTAATTTGTACACAGACAGGGAAACT
GGCAAGCTGAAGGGAGAGGCAACGGTCTCTTTTGATGACCCACCTTCAGCTAAAGCAGCTATTGACTGGTTTGAT
GGTAAAGAATTCTCCGGAAATCCTATCAAGGTCTCATTTGCTACTCGCCGGGCAGACTTTAATCGGGGTGGTGGC
AATGGTCGTGGAGGCCGAGGGCGAGGAGGACCCATGGGCCGTGGAGGCTATGGAGGTGGTGGCAGTGGTGGTGGT
GGCCGAGGAGGATTTCCCAGTGGAGGTGGTGGCGGTGGAGGACAGCAGCGAGCTGGTGACTGGAAGTGTCCTAAT
CCCACCTGTGAGAATATGAACTTCTCTTGGAGGAATGAATGCAACCAGTGTAAGGCCCCTAAACCAGATGGCCCA
GGAGGGGGACCAGGTGGCTCTCACATGGGGGGTAACTACGGGGATGATCGTCGTGGTGGCAGAGGAGGCGCGATC
GCAAATCTGGTGGCCCAGCTGGAAAACGAGGTGGCCAGCCTGGAAAACGAGAACGAAACCCTGAAGAAAAAGAAC
CTGCACAAGAAGGACCTGATCGCCTACCTGGAAAAGGAAATCGCCAACCTGAGAAAGAAGATCGAGGAAGGCAAG
CCTATTCCCAACCCCCTGCTGGGCCTGGATAGCACCGGAGCACCAGGAAGTGCTGGTTCTGCTGCTGGTAGTGGA
GCGTGCCCGGTGCCGCTGCAGCTGCCGCCGCTGGAACGCCTGACCCTGGATGATAAAAAACCGCTGAATACCCTG
ATCTCTGCTACTGGTCTGTGGATGAGTCGTACCGGAACCATTCATAAAATCAAACACCACGAGGTTAGCCGTTCG
AAAATCTATATTGAGATGGCGTGTGGCGATCATCTGGTTGTGAACAATAGCCGCTCTTCTCGTACAGCACGTGCA
CTGCGTCACCACAAATATCGTAAAACCTGTAAACGTTGCCGTGTGTCCGATGAGGATCTGAACAAATTCCTGACA
AAAGCCAATGAGGACCAAACAAGCGTGAAAGTGAAAGTCGTTAGCGCTCCTACCCGTACTAAAAAAGCAATGCCG
AAATCCGTTGCTCGTGCCCCTAAACCACTGGAAAACACTGAAGCAGCACAGGCACAGCCGTCTGGAAGCAAATTC
TCTCCGGCCATTCCTGTTTCTACCCAGGAGTCCGTTTCTGTTCCAGCAAGTGTGAGCACCAGCATTAGCAGTATT
AGCACCGGTGCCACCGCTAGCGCCCTGGTTAAAGGCAATACCAATCCGATTACAAGCATGTCTGCCCCGGTTCAA
GCATCAGCTCCAGCACTGACAAAATCCCAAACCGATCGTCTGGAGGTTCTGCTGAATCCGAAAGACGAAATCAGC
CTGAATTCCGGCAAACCGTTTCGTGAACTGGAGAGCGAACTGCTGTCACGTCGTAAAAAAGACCTGCAACAAATC
TATGCCGAAGAACGTGAGAACTATCTGGGGAAACTGGAACGTGAAATCACCCGCTTTTTCGTGGATCGTGGCTTT
CTGGAGATCAAATCCCCGATTCTGATTCCTCTGGAGTATATCGAGCGTATGGGCATCGACAATGATACCGAACTG
AGCAAACAAATTTTCCGTGTGGATAAAAACTTCTGTCTGCGCCCTATGCTAGCACCAAATCTGGCTAACTATCTG
CGCAAACTGGACCGTGCCCTGCCTGATCCTATCAAAATCTTCGAGATCGGCCCGTGTTATCGTAAAGAGTCCGAC
GGTAAAGAACATCTGGAGGAGTTTACCATGCTGAACTTTTGCCAAATGGGTTCAGGTTGTACTCGTGAGAACCTG
GAAAGCATCATCACCGATTTTCTGAACCACCTGGGCATTGACTTCAAAATTGTGGGCGACAGCTGTATGGTGTTT
GGCGACACCCTGGATGTCATGCACGGCGACCTGGAACTGTCTAGTGCCGTTGTGGGCCCAATCCCGCTGGATCGT
GAGTGGGGTATCGACAAACCTTGGATCGGTGCGGGTTTTGGTCTGGAGCGTCTGCTGAAAGTAAAACACGACTTC
AAGAACATCAAACGTGCTGCACGTTCCGAGTCCTATTACAATGGTATTTCTACTAACCTGTAA (SEQ ID NO:
543)

Protein:
MVGRNSAIAAGVCGALFIGYCIYFDRKRRSDPNFKNRLRERRKKQKLAKERAGLSKLPDLKDAEAVQKFFMASND
YTQQATQSYGAYPTQPGQGYSQQSSQPYGQQSYSGYSQSTDTSGYGQSSYSSYGQSQNTGYGTQSTPQGYGSTGG
YGSSQSSQSSYGQQSSYPGYGQQPAPSSTSGSYGSSSQSSSYGQPQSGSYSQQPSYGGQQQSYGQQQSYNPPQGY
GQQNQYNSSSGGGGGGGGGNYGQDQSSMSSGGGSGGGYGNQDQSGGGGSGGYGQQDRGGRGRGGSGGGGGGGGG
GYNRSSGGYEPRGRGGGRGGRGGMGGSDRGGFNKFGGPRDQGSRHDSEQDNSDNNTIFVQGLGENVTIESVADYF

KQIGIIKTNKKTGQPMINLYTDRETGKLKGEATVSFDDPPSAKAAIDWFDGKEFSGNPIKVSFATRRADFNRGGG
NGRGGRGRGGPMGRGGYGGGGSGGGGRGGFPSGGGGGGGQQRAGDWKCPNPTCENMNFSWRNECNQCKAPKPDGP
GGGPGGSHMGGNYGDDRRGGRGGAIANLVAQLENEVASLENENETLKKKNLHKKDLIAYLEKEIANLRKKIEEGK
PIPNPLLGLDSTGAPGSAGSAAGSGACPVPLQLPPLERLTLDDKKPLNTLISATGLWMSRTGTIHKIKHHEVSRS
KIYIEMACGDHLVVNNSRSSRTARALRHHKYRKTCKRCRVSDEDLNKFLTKANEDQTSVKVKVVSAPTRTKKAMP
KSVARAPKPLENTEAAQAQPSGSKFSPAIPVSTQESVSVPASVSTSISSISTGATASALVKGNTNPITSMSAPVQ
ASAPALTKSQTDRLEVLLNPKDEISLNSGKPFRELESELLSRRKKDLQQIYAEERENYLGKLEREITRFFVDRGF
LEIKSPILIPLEYIERMGIDNDTELSKQIFRVDKNFCLRPMLAPNLANYLRKLDRALPDPIKIFEIGPCYRKESD
GKEHLEEFTMLNFCQMGSGCTRENLESIITDFLNHLGIDFKIVGDSCMVFGDTLDVMHGDLELSSAVVGPIPLDR
EWGIDKPWIGAGFGLERLLKVKHDFKNIKRAARSESYYNGISTNL* (SEQ ID NO: 544)

TOM20::FUS::SYNZIP::3::PylRS(AF)
DNA:
ATGGTGGGTCGGAACAGCGCCATCGCCGCCGGTGTATGCGGGGCCCTTTTCATTGGGTACTGCATCTACTTCGAC
CGCAAAAGACGAAGTGACCCCAACTTCAAGAACAGGCTTCGAGAACGAAGAAAGAAACAGAAGCTTGCCAAGGAG
AGAGCTGGGCTTTCCAAGTTACCTGACCTTAAAGATGCTGAAGCTGTTCAGAAATTCTTCATGGCCTCAAACGAT
TATACCCAACAAGCAACCCAAAGCTATGGGGCCTACCCCACCCAGCCCGGGCAGGGCTATTCCCAGCAGAGCAGT
CAGCCCTACGGACAGCAGAGTTACAGTGGTTATAGCCAGTCCACGGACACTTCAGGATATGGCCAGAGCAGCTAT
TCTTCTTATGGCCAGAGCCAGAACACAGGCTATGGAACTCAGTCAACTCCCCAGGGATATGGCTCGACTGGCGGC
TATGGCAGTAGCCAGAGCTCCCAATCGTCTTACGGGCAGCAGTCCTCCTACCCTGGCTATGGCCAGCAGCCAGCT
CCCAGCAGCACCTCGGGAAGTTACGGTAGCAGTTCTCAGAGCAGCAGCTATGGGCAGCCCCAGAGTGGGAGCTAC
AGCCAGCAGCCTAGCTATGGTGGACAGCAGCAAAGCTATGGACAGCAGCAAAGCTATAATCCCCCTCAGGGCTAT
GGACAGCAGAACCAGTACAACAGCAGCAGTGGTGGTGGAGGTGGAGGTGGAGGTGGAGGTAACTATGGCCAAGAT
CAATCCTCCATGAGTAGTGGTGGTGGCAGTGGTGGCGGTTATGGCAATCAAGACCAGAGTGGTGGAGGTGGCAGC
GGTGGCTATGGACAGCAGGACCGTGGAGGCCGCGGCAGGGGTGGCAGTGGTGGCGGCGGCGGCGGCGGTGGT
GGTTACAACCGCAGCAGTGGTGGCTATGAACCCAGAGGTCGTGGAGGTGGCCGTGGAGGCAGAGGTGGCATGGGC
GGAAGTGACCGTGGTGGCTTCAATAAATTTGGTGGCCCTCGGGACCAAGGATCACGTCATGACTCCGAACAGGAT
AATTCAGACAACAACACCATCTTTGTGCAAGGCCTGGGTGAGAATGTTACAATTGAGTCTGTGGCTGATTACTTC
AAGCAGATTGGTATTATTAAGACAAACAAGAAAACGGGACAGCCCATGATTAATTTGTACACAGACAGGGAAACT
GGCAAGCTGAAGGGAGAGGCAACGGTCTCTTTTGATGACCCACCTTCAGCTAAAGCAGCTATTGACTGGTTTGAT
GGTAAAGAATTCTCCGGAAATCCTATCAAGGTCTCATTTGCTACTCGCCGGGCAGACTTTAATCGGGGTGGTGGC
AATGGTCGTGGAGGCCGAGGGCGAGGAGGACCCATGGGCCGTGGAGGCTATGGAGGTGGTGGCAGTGGTGGTGGT
GGCCGAGGAGGATTTCCCAGTGGAGGTGGTGGCGGTGGAGGACAGCAGCGAGCTGGTGACTGGAAGTGTCCTAAT
CCCACCTGTGAGAATATGAACTTCTCTTGGAGGAATGAATGCAACCAGTGTAAGGCCCCTAAACCAGATGGCCCA
GGAGGGGGACCAGGTGGCTCTCACATGGGGGGTAACTACGGGGATGATCGTCGTGGTGGCAGAGGAGGCGCGATC
GCAAATGAGGTGACCACCCTGGAAAACGACGCCGCCTTCATCGAGAACGAGAACGCCTACCTGGAAAAAGAGATC
GCCAGACTGAGAAAGGAAAAGGCCGCTCTGCGGAACAGACTGGCCCACAAGAAGGGCAAGCCTATTCCCAACCCC
CTGCTGGGCCTGGATAGCACCGGAGCACCAGGAAGTGCTGGTTCTGCTGCTGGTAGTGGAGCGTGCCCGGTGCCG
CTGCAGCTGCCGCCGCTGGAACGCCTGACCCTGGATGATAAAAAACCGCTGAATACCCTGATCTCTGCTACTGGT
CTGTGGATGAGTCGTACCGGAACCATTCATAAAATCAAACACCACGAGGTTAGCCGTTCGAAAATCTATATTGAG
ATGGCGTGTGGCGATCATCTGGTTGTGAACAATAGCCGCTCTTCTCGTACAGCACGTGCACTGCGTCACCACAAA
TATCGTAAAACCTGTAAACGTTGCCGTGTGTCCGATGAGGATCTGAACAAATTCCTGACAAAAGCCAATGAGGAC
CAAACAAGCGTGAAAGTGAAAGTCGTTAGCGCTCCTACCCGTACTAAAAAAGCAATGCCGAAATCCGTTGCTCGT
GCCCCTAAACCACTGGAAAACACTGAAGCAGCACAGGCACAGCCGTCTGGAAGCAAATTCTCTCCGGCCATTCCT
GTTTCTACCCAGGAGTCCGTTTCTGTTCCAGCAAGTGTGAGCACCAGCATTAGCAGTATTAGCACCGGTGCCACC
GCTAGCGCCCTGGTTAAAGGCAATACCAATCCGATTACAAGCATGTCTGCCCCGGTTCAAGCATCAGCTCCAGCA
CTGACAAAATCCCAAACCGATCGTCTGGAGGTTCTGCTGAATCCGAAAGACGAAATCAGCCTGAATTCCGGCAAA
CCGTTTCGTGAACTGGAGAGCGAACTGCTGTCACGTCGTAAAAAAGACCTGCAACAAATCTATGCCGAAGAACGT
GAGAACTATCTGGGGAAACTGGAACGTGAAATCACCCGCTTTTTCGTGGATCGTGGCTTTCTGGAGATCAAATCC
CCGATTCTGATTCCTCTGGAGTATATCGAGCGTATGGGCATCGACAATGATACCGAACTGAGCAAACAAATTTTC
CGTGTGGATAAAAACTTCTGTCTGCGCCCTATGCTAGCACCAAATCTGGCTAACTATCTGCGCAAACTGGACCGT
GCCCTGCCTGATCCTATCAAAATCTTCGAGATCGGCCCGTGTTATCGTAAAGAGTCCGACGGTAAAGAACATCTG
GAGGAGTTTACCATGCTGAACTTTTGCCAAATGGGTTCAGGTTGTACTCGTGAGAACCTGGAAAGCATCATCACC
GATTTTCTGAACCACCTGGGCATTGACTTCAAAATTGTGGGCGACAGCTGTATGGTGTTTGGCGACACCCTGGAT
GTCATGCACGGCGACCTGGAACTGTCTAGTGCCGTTGTGGGCCCAATCCCGCTGGATCGTGAGTGGGGTATCGAC
AAACCTTGGATCGGTGCGGGTTTTGGTCTGGAGCGTCTGCTGAAAGTAAAACACGACTTCAAGAACATCAAACGT
GCTGCACGTTCCGAGTCCTATTACAATGGTATTTCTACTAACCTGTAA (SEQ ID NO: 545)

Protein:
MVGRNSAIAAGVCGALFIGYCIYFDRKRRSDPNFKNRLRERRKKQKLAKERAGLSKLPDLKDAEAVQKFFMASND
YTQQATQSYGAYPTQPGQGYSQQSSQPYGQQSYSGYSQSTDTSGYGQSSYSSYGQSQNTGYGTQSTPQGYGSTGG
YGSSQSSQSSYGQQSSYPGYGQQPAPSSTSGSYGSSSQSSSYGQPQSGSYSQQPSYGGQQQSYGQQQSYNPPQGY
GQQNQYNSSSGGGGGGGGGNYGQDQSSMSSGGGSGGGYGNQDQSGGGGSGGYGQQDRGGRGRGGSGGGGGGGG
GYNRSSGGYEPRGRGGGRGGRGGMGGSDRGGFNKFGGPRDQGSRHDSEQDNSDNNTIFVQGLGENVTIESVADYF
KQIGIIKTNKKTGQPMINLYTDRETGKLKGEATVSFDDPPSAKAAIDWFDGKEFSGNPIKVSFATRRADFNRGGG
NGRGGRGRGGPMGRGGYGGGGSGGGGRGGFPSGGGGGGGQQRAGDWKCPNPTCENMNFSWRNECNQCKAPKPDGP
GGGPGGSHMGGNYGDDRRGGRGGAIANEVTTLENDAAFIENENAYLEKEIARLRKEKAALRNRLAHKKGKPIPNP
LLGLDSTGAPGSAGSAAGSGACPVPLQLPPLERLTLDDKKPLNTLISATGLWMSRTGTIHKIKHHEVSRSKIYIE
MACGDHLVVNNSRSSRTARALRHHKYRKTCKRCRVSDEDLNKFLTKANEDQTSVKVKVVSAPTRTKKAMPKSVAR
APKPLENTEAAQAQPSGSKFSPAIPVSTQESVSVPASVSTSISSISTGATASALVKGNTNPITSMSAPVQASAPA
LTKSQTDRLEVLLNPKDEISLNSGKPFRELESELLSRRKKDLQQIYAEERENYLGKLEREITRFFVDRGFLEIKS
PILIPLEYIERMGIDNDTELSKQIFRVDKNFCLRPMLAPNLANYLRKLDRALPDPIKIFEIGPCYRKESDGKEHL
EEFTMLNFCQMGSGCTRENLESIITDFLNHLGIDFKIVGDSCMVFGDTLDVMHGDLELSSAVVGPIPLDREWGID
KPWIGAGFGLERLLKVKHDFKNIKRAARSESYYNGISTNL* (SEQ ID NO: 546)

EBAG9₁₋₂₉::FUS::SYNZIP1::PylRS(AF)
DNA:
ATGGCCATCACCCAGTTTCGGTTATTTAAATTTTGTACCTGCCTAGCAACAGTATTCTCATTCCTAAAGAGATTA
ATATGCAGATCTGGAGCACCCGGCTCCGCCGGCTCCGCCGCCGGCTCCGGCATGGCCTCAAACGATTATACCCAA
CAAGCAACCCAAAGCTATGGGGCCTACCCCACCCAGCCCGGGCAGGGCTATTCCCAGCAGAGCAGTCAGCCCTAC
GGACAGCAGAGTTACAGTGGTTATAGCCAGTCCACGGACACTTCAGGATATGGCCAGAGCAGCTATTCTTCTTAT
GGCCAGAGCCAGAACACAGGCTATGGAACTCAGTCAACTCCCCAGGGATATGGCTCGACTGGCGGCTATGGCAGT
AGCCAGAGCTCCCAATCGTCTTACGGGCAGCAGTCCTCCTACCCTGGCTATGGCCAGCAGCCAGCTCCCAGCAGC
ACCTCGGGAAGTTACGGTAGCAGTTCTCAGAGCAGCAGCTATGGGCAGCCCCAGAGTGGGAGCTACAGCCAGCAG
CCTAGCTATGGTGGACAGCAGCAAAGCTATGGACAGCAGCAAAGCTATAATCCCCCTCAGGGCTATGGACAGCAG
AACCAGTACAACAGCAGCAGTGGTGGTGGAGGTGGAGGTGGAGGTGGAGGTAACTATGGCCAAGATCAATCCTCC
ATGAGTAGTGGTGGTGGCAGTGGTGGCGGTTATGGCAATCAAGACCAGAGTGGTGGAGGTGGCAGCGGTGGCTAT
GGACAGCAGGACCGTGGAGGCCGCGGCAGGGGTGGCAGTGGTGGCGGCGGCGGCGGCGGTGGTGGTTACAAC
CGCAGCAGTGGTGGCTATGAACCCAGAGGTCGTGGAGGTGGCCGTGGAGGCAGAGGTGGCATGGGCGGAAGTGAC
CGTGGTGGCTTCAATAAATTTGGTGGCCCTCGGGACCAAGGATCACGTCATGACTCCGAACAGGATAATTCAGAC
AACAACACCATCTTTGTGCAAGGCCTGGGTGAGAATGTTACAATTGAGTCTGTGGCTGATTACTTCAAGCAGATT
GGTATTATTAAGACAAACAAGAAAACGGGACAGCCCATGATTAATTTGTACACAGACAGGGAAACTGGCAAGCTG
AAGGGAGAGGCAACGGTCTCTTTTGATGACCCACCTTCAGCTAAAGCAGCTATTGACTGGTTTGATGGTAAAGAA
TTCTCCGGAAATCCTATCAAGGTCTCATTTGCTACTCGCCGGGCAGACTTTAATCGGGGTGGTGGCAATGGTCGT
GGAGGCCGAGGGCGAGGAGGACCCATGGGCCGTGGAGGCTATGGAGGTGGTGGCAGTGGTGGTGGTGGCCGAGGA
GGATTTCCCAGTGGAGGTGGTGGCGGTGGAGGACAGCAGCGAGCTGGTGACTGGAAGTGTCCTAATCCCACCTGT
GAGAATATGAACTTCTCTTGGAGGAATGAATGCAACCAGTGTAAGGCCCCTAAACCAGATGGCCCAGGAGGGGGA
CCAGGTGGCTCTCACATGGGGGGTAACTACGGGGATGATCGTCGTGGTGGCAGAGGTGGTGCGATCGCAAATCTG
GTGGCCCAGCTGGAAAACGAGGTGGCCAGCCTGGAAAACGAGAACGAAACCCTGAAGAAAAAGAACCTGCACAAG
AAGGACCTGATCGCCTACCTGGAAAAGGAAATCGCCAACCTGAGAAAGAAGATCGAGGAAGGCGCCGATTACAAG
GACGATGATGACAAGGGAGCACCAGGAAGTGCTGGTTCTGCTGCTGGTAGTGGAGCGTGCCCGGTGCCGCTGCAG
CTGCCGCCGCTGGAACGCCTGACCCTGGATGATAAAAAACCGCTGAATACCCTGATCTCTGCTACTGGTCTGTGG
ATGAGTCGTACCGGAACCATTCATAAAATCAAACACCACGAGGTTAGCCGTTCGAAAATCTATATTGAGATGGCG
TGTGGCGATCATCTGGTTGTGAACAATAGCCGCTCTTCTCGTACAGCACGTGCACTGCGTCACCACAAATATCGT
AAAACCTGTAAACGTTGCCGTGTGTCCGATGAGGATCTGAACAAATTCCTGACAAAAGCCAATGAGGACCAAACA
AGCGTGAAAGTGAAAGTCGTTAGCGCTCCTACCCGTACTAAAAAAGCAATGCCGAAATCCGTTGCTCGTGCCCCT
AAACCACTGGAAAACACTGAAGCAGCACAGGCACAGCCGTCTGGAAGCAAATTCTCTCCGGCCATTCCTGTTTCT
ACCCAGGAGTCCGTTTCTGTTCCAGCAAGTGTGAGCACCAGCATTAGCAGTATTAGCACCGGTGCCACCGCTAGC
GCCCTGGTTAAAGGCAATACCAATCCGATTACAAGCATGTCTGCCCCGGTTCAAGCATCAGCTCCAGCACTGACA
AAATCCCAAACCGATCGTCTGGAGGTTCTGCTGAATCCGAAAGACGAAATCAGCCTGAATTCCGGCAAACCGTTT
CGTGAACTGGAGAGCGAACTGCTGTCACGTCGTAAAAAAGACCTGCAACAAATCTATGCCGAAGAACGTGAGAAC
TATCTGGGGAAACTGGAACGTGAAATCACCCGCTTTTTCGTGGATCGTGGCTTTCTGGAGATCAAATCCCCGATT
CTGATTCCTCTGGAGTATATCGAGCGTATGGGCATCGACAATGATACCGAACTGAGCAAACAAATTTTCCGTGTG
GATAAAAACTTCTGTCTGCGCCCTATGCTAGCACCAAATCTGGCTAACTATCTGCGCAAACTGGACCGTGCCCTG

CCTGATCCTATCAAAATCTTCGAGATCGGCCCGTGTTATCGTAAAGAGTCCGACGGTAAAGAACATCTGGAGGAG
TTTACCATGCTGAACTTTTGCCAAATGGGTTCAGGTTGTACTCGTGAGAACCTGGAAAGCATCATCACCGATTTT
CTGAACCACCTGGGCATTGACTTCAAAATTGTGGGCGACAGCTGTATGGTGTTTGGCGACACCCTGGATGTCATG
CACGGCGACCTGGAACTGTCTAGTGCCGTTGTGGGCCCAATCCCGCTGGATCGTGAGTGGGGTATCGACAAACCT
TGGATCGGTGCGGGTTTTGGTCTGGAGCGTCTGCTGAAAGTAAAACACGACTTCAAGAACATCAAACGTGCTGCA
CGTTCCGAGTCCTATTACAATGGTATTTCTACTAACCTGTAA (SEQ ID NO: 547)

Protein:
MAITQFRLFKFCTCLATVFSFLKRLICRSGAPGSAGSAAGSGMASNDYTQQATQSYGAYPTQPGQGYSQQSSQPY
GQQSYSGYSQSTDTSGYGQSSYSSYGQSQNTGYGTQSTPQGYGSTGGYGSSQSSQSSYGQQSSYPGYGQQPAPSS
TSGSYGSSSQSSSYGQPQSGSYSQQPSYGGQQQSYGQQQSYNPPQGYGQQNQYNSSSGGGGGGGGGGNYGQDQSS
MSSGGGSGGGYGNQDQSGGGGSGGYGQQDRGGRGRGGSGGGGGGGGGGYNRSSGGYEPRGRGGGRGGRGGMGGSD
RGGFNKFGGPRDQGSRHDSEQDNSDNNTIFVQGLGENVTIESVADYFKQIGIIKTNKKTGQPMINLYTDRETGKL
KGEATVSFDDPPSAKAAIDWFDGKEFSGNPIKVSFATRRADFNRGGGNGRGGRGRGGPMGRGGYGGGGSGGGGRG
GFPSGGGGGGGGQQRAGDWKCPNPTCENMNFSWRNECNQCKAPKPDGPGGGPGGSHMGGNYGDDRRGGRGGAIANL
VAQLENEVASLENENETLKKKNLHKKDLIAYLEKEIANLRKKIEEGADYKDDDDKGAPGSAGSAAGSGACPVPLQ
LPPLERLTLDDKKPLNTLISATGLWMSRTGTIHKIKHHEVSRSKIYIEMACGDHLVVNNSRSSRTARALRHHKYR
KTCKRCRVSDEDLNKFLTKANEDQTSVKVKVVSAPTRTKKAMPKSVARAPKPLENTEAAQAQPSGSKFSPAIPVS
TQESVSVPASVSTSISSISTGATASALVKGNTNPITSMSAPVQASAPALTKSQTDRLEVLLNPKDEISLNSGKPF
RELESELLSRRKKDLQQIYAEERENYLGKLEREITRFFVDRGFLEIKSPILIPLEYIERMGIDNDTELSKQIFRV
DKNFCLRPMLAPNLANYLRKLDRALPDPIKIFEIGPCYRKESDGKEHLEEFTMLNFCQMGSGCTRENLESIITDF
LNHLGIDFKIVGDSCMVFGDTLDVMHGDLELSSAVVGPIPLDREWGIDKPWIGAGFGLERLLKVKHDFKNIKRAA
RSESYYNGISTNL* (SEQ ID NO: 548)

EBAG9₁₋₂₉::FUS::SYNZIP3::PylRS(AF)
DNA:
ATGGCCATCACCCAGTTTCGGTTATTTAAATTTTGTACCTGCCTAGCAACAGTATTCTCATTCCTAAAGAGATTA
ATATGCAGATCTGGAGCACCCGGCTCCGCCGGCTCCGCCGCCGGCTCCGGCATGGCCTCAAACGATTATACCCAA
CAAGCAACCCAAAGCTATGGGGCCTACCCCACCCAGCCCGGGCAGGGCTATTCCCAGCAGAGCAGTCAGCCCTAC
GGACAGCAGAGTTACAGTGGTTATAGCCAGTCCACGGACACTTCAGGATATGGCCAGAGCAGCTATTCTTCTTAT
GGCCAGAGCCAGAACACAGGCTATGGAACTCAGTCAACTCCCCAGGGATATGGCTCGACTGGCGGCTATGGCAGT
AGCCAGAGCTCCCAATCGTCTTACGGGCAGCAGTCCTCCTACCCTGGCTATGGCCAGCAGCCAGCTCCCAGCAGC
ACCTCGGGAAGTTACGGTAGCAGTTCTCAGAGCAGCAGCTATGGGCAGCCCCAGAGTGGGAGCTACAGCCAGCAG
CCTAGCTATGGTGGACAGCAGCAAAGCTATGGACAGCAGCAAAGCTATAATCCCCCTCAGGGCTATGGACAGCAG
AACCAGTACAACAGCAGCAGTGGTGGTGGAGGTGGAGGTGGAGGTGGAGGTAACTATGGCCAAGATCAATCCTCC
ATGAGTAGTGGTGGTGGCAGTGGTGGCGGTTATGGCAATCAAGACCAGAGTGGTGGAGGTGGCAGCGGTGGCTAT
GGACAGCAGGACCGTGGAGGCCGCGGCAGGGGTGGCAGTGGTGGCGGCGGCGGCGGCGGCGGTGGTGGTTACAAC
CGCAGCAGTGGTGGCTATGAACCCAGAGGTCGTGGAGGTGGCCGTGGAGGCAGAGGTGGCATGGGCGGAAGTGAC
CGTGGTGGCTTCAATAAATTTGGTGGCCCTCGGGACCAAGGATCACGTCATGACTCCGAACAGGATAATTCAGAC
AACAACACCATCTTTGTGCAAGGCCTGGGTGAGAATGTTACAATTGAGTCTGTGGCTGATTACTTCAAGCAGATT
GGTATTATTAAGACAAACAAGAAAACGGGACAGCCCATGATTAATTTGTACACAGACAGGGAAACTGGCAAGCTG
AAGGGAGAGGCAACGGTCTCTTTTGATGACCCACCTTCAGCTAAAGCAGCTATTGACTGGTTTGATGGTAAAGAA
TTCTCCGGAAATCCTATCAAGGTCTCATTTGCTACTCGCCGGGCAGACTTTAATCGGGGTGGTGGCAATGGTCGT
GGAGGCCGAGGGCGAGGAGGACCCATGGGCCGTGGAGGCTATGGAGGTGGTGGCAGTGGTGGTGGTGGCCGAGGA
GGATTTCCCAGTGGAGGTGGTGGCGGTGGAGGACAGCAGCGAGCTGGTGACTGGAAGTGTCCTAATCCCACCTGT
GAGAATATGAACTTCTCTTGGAGGAATGAATGCAACCAGTGTAAGGCCCCTAAACCAGATGGCCCAGGAGGGGGA
CCAGGTGGCTCTCACATGGGGGGTAACTACGGGGATGATCGTCGTGGTGGCAGAGGTGGTGCGATCGCAAATGAG
GTGACCACCCTGGAAAACGACGCCGCCTTCATCGAGAACGAGAACGCCTACCTGGAAAAAGAGATCGCCAGACTG
AGAAAGGAAAAGGCCGCTCTGCGGAACAGACTGGCCCACAAGAAGGGCGCCGATTACAAGGACGATGATGACAAG
GGAGCACCAGGAAGTGCTGGTTCTGCTGCTGGTAGTGGAGCGTGCCCGGTGCCGCTGCAGCTGCCGCCGCTGGAA
CGCCTGACCCTGGATGATAAAAAACCGCTGAATACCCTGATCTCTGCTACTGGTCTGTGGATGAGTCGTACCGGA
ACCATTCATAAAATCAAACACCACGAGGTTAGCCGTTCGAAAATCTATATTGAGATGGCGTGTGGCGATCATCTG
GTTGTGAACAATAGCCGCTCTTCTCGTACAGCACGTGCACTGCGTCACCACAAATATCGTAAAACCTGTAAACGT
TGCCGTGTGTCCGATGAGGATCTGAACAAATTCCTGACAAAAGCCAATGAGGACCAAACAAGCGTGAAAGTGAAA
GTCGTTAGCGCTCCTACCCGTACTAAAAAAGCAATGCCGAAATCCGTTGCTCGTGCCCCTAAACCACTGGAAAAC
ACTGAAGCAGCACAGGCACAGCCGTCTGGAAGCAAATTCTCTCCGGCCATTCCTGTTTCTACCCAGGAGTCCGTT
TCTGTTCCAGCAAGTGTGAGCACCAGCATTAGCAGTATTAGCACCGGTGCCACCGCTAGCGCCCTGGTTAAAGGC

AATACCAATCCGATTACAAGCATGTCTGCCCCGGTTCAAGCATCAGCTCCAGCACTGACAAAATCCCAAACCGAT
CGTCTGGAGGTTCTGCTGAATCCGAAAGACGAAATCAGCCTGAATTCCGGCAAACCGTTTCGTGAACTGGAGAGC
GAACTGCTGTCACGTCGTAAAAAAGACCTGCAACAAATCTATGCCGAAGAACGTGAGAACTATCTGGGGAAACTG
GAACGTGAAATCACCCGCTTTTTCGTGGATCGTGGCTTTCTGGAGATCAAATCCCCGATTCTGATTCCTCTGGAG
TATATCGAGCGTATGGGCATCGACAATGATACCGAACTGAGCAAACAAATTTTCCGTGTGGATAAAAACTTCTGT
CTGCGCCCTATGCTAGCACCAAATCTGGCTAACTATCTGCGCAAACTGGACCGTGCCCTGCCTGATCCTATCAAA
ATCTTCGAGATCGGCCCGTGTTATCGTAAAGAGTCCGACGGTAAAGAACATCTGGAGGAGTTTACCATGCTGAAC
TTTTGCCAAATGGGTTCAGGTTGTACTCGTGAGAACCTGGAAAGCATCATCACCGATTTTCTGAACCACCTGGGC
ATTGACTTCAAAATTGTGGGCGACAGCTGTATGGTGTTTGGCGACACCCTGGATGTCATGCACGGCGACCTGGAA
CTGTCTAGTGCCGTTGTGGGCCCAATCCCGCTGGATCGTGAGTGGGGTATCGACAAACCTTGGATCGGTGCGGGT
TTTGGTCTGGAGCGTCTGCTGAAAGTAAAACACGACTTCAAGAACATCAAACGTGCTGCACGTTCCGAGTCCTAT
TACAATGGTATTTCTACTAACCTGTAA (SEQ ID NO: 549)

Protein:
MAITQFRLFKFCTCLATVFSFLKRLICRSGAPGSAGSAAGSGMASNDYTQQATQSYGAYPTQPGQGYSQQSSQPY
GQQSYSGYSQSTDTSGYGQSSYSSYGQSQNTGYGTQSTPQGYGSTGGYGSSQSSQSSYGQQSSYPGYGQQPAPSS
TSGSYGSSSQSSSYGQPQSGSYSQQPSYGGQQQSYGQQQSYNPPQGYGQQNQYNSSSGGGGGGGGGNYGQDQSS
MSSGGGSGGGYGNQDQSGGGGSGGYGQQDRGGRGRGGSGGGGGGGGGGYNRSSGGYEPRGRGGGRGGRGGMGGSD
RGGFNKFGGPRDQGSRHDSEQDNSDNNTIFVQGLGENVTIESVADYFKQIGIIKTNKKTGQPMINLYTDRETGKL
KGEATVSFDDPPSAKAAIDWFDGKEFSGNPIKVSFATRRADFNRGGGNGRGGRGRGGPMGRGGYGGGGSGGGGRG
GFPSGGGGGGGQQRAGDWKCPNPTCENMNFSWRNECNQCKAPKPDGPGGGPGGSHMGGNYGDDRRGGRGGAIANE
VTTLENDAAFIENENAYLEKEIARLRKEKAALRNRLAHKKGADYKDDDDKGAPGSAGSAAGSGACPVPLQLPPLE
RLTLDDKKPLNTLISATGLWMSRTGTIHKIKHHEVSRSKIYIEMACGDHLVVNNSRSSRTARALRHHKYRKTCKR
CRVSDEDLNKFLTKANEDQTSVKVKVVSAPTRTKKAMPKSVARAPKPLENTEAAQAQPSGSKFSPAIPVSTQESV
SVPASVSTSISSISTGATASALVKGNTNPITSMSAPVQASAPALTKSQTDRLEVLLNPKDEISLNSGKPFRELES
ELLSRRKKDLQQIYAEERENYLGKLEREITRFFVDRGFLEIKSPILIPLEYIERMGIDNDTELSKQIFRVDKNFC
LRPMLAPNLANYLRKLDRALPDPIKIFEIGPCYRKESDGKEHLEEFTMLNFCQMGSGCTRENLESIITDFLNHLG
IDFKIVGDSCMVFGDTLDVMHGDLELSSAVVGPIPLDREWGIDKPWIGAGFGLERLLKVKHDFKNIKRAARSESY
YNGISTNL* (SEQ ID NO: 550)

TOM20::FUS::SYNZIP1::PylRS(AA)
DNA:
ATGGTGGGTCGGAACAGCGCCATCGCCGCCGGTGTATGCGGGGCCCTTTTCATTGGGTACTGCATCTACTTCGAC
CGCAAAAGACGAAGTGACCCCAACTTCAAGAACAGGCTTCGAGAACGAAGAAAGAAACAGAAGCTTGCCAAGGAG
AGAGCTGGGCTTTCCAAGTTACCTGACCTTAAAGATGCTGAAGCTGTTCAGAAATTCTTCATGGCCTCAAACGAT
TATACCCAACAAGCAACCCAAAGCTATGGGGCCTACCCCACCCAGCCCGGGCAGGGCTATTCCCAGCAGAGCAGT
CAGCCCTACGGACAGCAGAGTTACAGTGGTTATAGCCAGTCCACGGACACTTCAGGATATGGCCAGAGCAGCTAT
TCTTCTTATGGCCAGAGCCAGAACACAGGCTATGGAACTCAGTCAACTCCCCAGGGATATGGCTCGACTGGCGGC
TATGGCAGTAGCCAGAGCTCCCAATCGTCTTACGGGCAGCAGTCCTCCTACCCTGGCTATGGCCAGCAGCCAGCT
CCCAGCAGCACCTCGGGAAGTTACGGTAGCAGTTCTCAGAGCAGCAGCTATGGGCAGCCCCAGAGTGGGAGCTAC
AGCCAGCAGCCTAGCTATGGTGGACAGCAGCAAAGCTATGGACAGCAGCAAAGCTATAATCCCCCTCAGGGCTAT
GGACAGCAGAACCAGTACAACAGCAGCAGTGGTGGTGGAGGTGGAGGTGGAGGTGGAGGTAACTATGGCCAAGAT
CAATCCTCCATGAGTAGTGGTGGTGGCAGTGGTGGCGGTTATGGCAATCAAGACCAGAGTGGTGGAGGTGGCAGC
GGTGGCTATGGACAGCAGGACCGTGGAGGCCGCGGCAGGGGTGGCAGTGGTGGCGGCGGCGGCGGCGGCGGTGGT
GGTTACAACCGCAGCAGTGGTGGCTATGAACCCAGAGGTCGTGGAGGTGGCCGTGGAGGCAGAGGTGGCATGGGC
GGAAGTGACCGTGGTGGCTTCAATAAATTTGGTGGCCCTCGGGACCAAGGATCACGTCATGACTCCGAACAGGAT
AATTCAGACAACAACACCATCTTTGTGCAAGGCCTGGGTGAGAATGTTACAATTGAGTCTGTGGCTGATTACTTC
AAGCAGATTGGTATTATTAAGACAAACAAGAAAACGGGACAGCCCATGATTAATTTGTACACAGACAGGGAAACT
GGCAAGCTGAAGGGAGAGGCAACGGTCTCTTTTGATGACCCACCTTCAGCTAAAGCAGCTATTGACTGGTTTGAT
GGTAAAGAATTCTCCGGAAATCCTATCAAGGTCTCATTTGCTACTCGCCGGGCAGACTTTAATCGGGGTGGTGGC
AATGGTCGTGGAGGCCGAGGGCGAGGAGGACCCATGGGCCGTGGAGGCTATGGAGGTGGTGGCAGTGGTGGTGGT
GGCCGAGGAGGATTTCCCAGTGGAGGTGGTGGCGGTGGAGGACAGCAGCGAGCTGGTGACTGGAAGTGTCCTAAT
CCCACCTGTGAGAATATGAACTTCTCTTGGAGGAATGAATGCAACCAGTGTAAGGCCCCTAAACCAGATGGCCCA
GGAGGGGGACCAGGTGGCTCTCACATGGGGGGTAACTACGGGGATGATCGTCGTGGTGGCAGAGGAGGCGCGATC
GCAAATCTGGTGGCCCAGCTGGAAAACGAGGTGGCCAGCCTGGAAAACGAGAACGAAACCCTGAAGAAAAAGAAC
CTGCACAAGAAGGACCTGATCGCCTACCTGGAAAAGGAAATCGCCAACCTGAGAAAGAAGATCGAGGAAGGCAAG
CCTATTCCCAACCCCCTGCTGGGCCTGGATAGCACCGGAGCACCAGGAAGTGCTGGTTCTGCTGCTGGTAGTGGA

GCGTGCCCGGTGCCGCTGCAGCTGCCGCCGCTGGAACGCCTGACCCTGGATGACAAAAAACCGCTGAATACCCTG
ATCTCTGCTACTGGTCTGTGGATGAGTCGTACCGGAACCATTCATAAAATCAAACACCACGAGGTTAGCCGTTCG
AAAATCTATATTGAGATGGCGTGTGGCGATCATCTGGTTGTGAACAATAGCCGCTCTTCTCGTACAGCACGTGCA
CTGCGTCACCACAAATATCGTAAAACCTGTAAACGTTGCCGTGTGTCCGATGAGGATCTGAACAAATTCCTGACA
AAAGCCAATGAGGACCAAACAAGCGTGAAAGTGAAAGTCGTTAGCGCTCCTACCCGTACTAAAAAAGCAATGCCG
AAATCCGTTGCTCGTGCCCCTAAACCACTGGAAAACACTGAAGCAGCACAGGCACAGCCGTCTGGAAGCAAATTC
TCTCCGGCCATTCCTGTTTCTACCCAGGAGTCCGTTTCTGTTCCAGCAAGTGTGAGCACCAGCATTAGCAGTATT
AGCACCGGTGCCACCGCTAGCGCCCTGGTTAAAGGCAATACCAATCCGATTACAAGCATGTCTGCCCCGGTTCAA
GCATCAGCTCCAGCACTGACAAAATCCCAAACCGATCGTCTGGAGGTTCTGCTGAATCCGAAAGACGAAATCAGC
CTGAATTCCGGCAAACCGTTTCGTGAACTGGAGAGCGAACTGCTGTCACGTCGTAAAAAAGACCTGCAACAAATC
TATGCCGAAGAACGTGAGAACTATCTGGGGAAACTGGAACGTGAAATCACCCGCTTTTTCGTGGATCGTGGCTTT
CTGGAGATCAAATCCCCGATTCTGATTCCTCTGGAGTATATCGAGCGTATGGGCATCGACAATGATACCGAACTG
AGCAAACAAATTTTCCGTGTGGATAAAAACTTCTGTCTGCGCCCTATGCTGGCACCAAATCTGTATAACTATCTG
CGCAAACTGGACCGTGCCCTGCCTGATCCTATCAAAATCTTCGAGATCGGCCCGTGTTATCGTAAAGAGTCCGAC
GGTAAAGAACATCTGGAGGAGTTTACCATGCTGGCCTTTGCCCAAATGGGTTCAGGTTGTACTCGTGAGAACCTG
GAAAGCATCATCACCGATTTTCTGAACCACCTGGGCATTGACTTCAAAATTGTGGGCGACAGCTGTATGGTGTAT
GGCGACACCCTGGATGTCATGCACGGCGACCTGGAACTGTCTAGTGCCGTTGTTGGACCAATTCCGCTGGACCGT
GAGTGGGGTATCGACAAACCGTGGATCGGAGCAGGATTCGGTCTGGAACGCCTGCTGAAAGTGAAACACGACTTC
AAAAACATCAAACGTGCCGCCCGTTCTGAATCGTATTATAACGGGATCTCTACGAACCTGTAA (SEQ ID NO:
551)

Protein:
MVGRNSAIAAGVCGALFIGYCIYFDRKRRSDPNFKNRLRERRKKQKLAKERAGLSKLPDLKDAEAVQKFFMASND
YTQQATQSYGAYPTQPGQGYSQQSSQPYGQQSYSGYSQSTDTSGYGQSSYSSYGQSQNTGYGTQSTPQGYGSTGG
YGSSQSSQSSYGQQSSYPGYGQQPAPSSTSGSYGSSSQSSSYGQPQSGSYSQQPSYGGQQQSYGQQQSYNPPQGY
GQQNQYNSSSGGGGGGGGGGNYGQDQSSMSSGGGSGGGYGNQDQSGGGGSGGYGQQDRGGRGRGGSGGGGGGGGG
GYNRSSGGYEPRGRGGGRGGRGGMGGSDRGGFNKFGGPRDQGSRHDSEQDNSDNNTIFVQGLGENVTIESVADYF
KQIGIIKTNKKTGQPMINLYTDRETGKLKGEATVSFDDPPSAKAAIDWFDGKEFSGNPIKVSFATRRADFNRGGG
NGRGGRGRGGPMGRGGYGGGGSGGGGRGGFPSGGGGGGGQQRAGDWKCPNPTCENMNFSWRNECNQCKAPKPDGP
GGGPGGSHMGGNYGDDRRGGRGGAIANLVAQLENEVASLENENETLKKKNLHKKDLIAYLEKEIANLRKKIEEGK
PIPNPLLGLDSTGAPGSAGSAAGSGACPVPLQLPPLERLTLDDKKPLNTLISATGLWMSRTGTIHKIKHHEVSRS
KIYIEMACGDHLVVNNSRSSRTARALRHHKYRKTCKRCRVSDEDLNKFLTKANEDQTSVKVKVVSAPTRTKKAMP
KSVARAPKPLENTEAAQAQPSGSKFSPAIPVSTQESVSVPASVSTSISSISTGATASALVKGNTNPITSMSAPVQ
ASAPALTKSQTDRLEVLLNPKDEISLNSGKPFRELESELLSRRKKDLQQIYAEERENYLGKLEREITRFFVDRGF
LEIKSPILIPLEYIERMGIDNDTELSKQIFRVDKNFCLRPMLAPNLYNYLRKLDRALPDPIKIFEIGPCYRKESD
GKEHLEEFTMLAFAQMGSGCTRENLESIITDFLNHLGIDFKIVGDSCMVYGDTLDVMHGDLELSSAVVGPIPLDR
EWGIDKPWIGAGFGLERLLKVKHDFKNIKRAARSESYYNGISTNL* (SEQ ID NO: 552)

TOM20::FUS::SYNZIP3::PylRS(AA)
DNA:
ATGGTGGGTCGGAACAGCGCCATCGCCGCCGGTGTATGCGGGGCCCTTTTCATTGGGTACTGCATCTACTTCGAC
CGCAAAAGACGAAGTGACCCCAACTTCAAGAACAGGCTTCGAGAACGAAGAAAGAAACAGAAGCTTGCCAAGGAG
AGAGCTGGGCTTTCCAAGTTACCTGACCTTAAAGATGCTGAAGCTGTTCAGAAATTCTTCATGGCCTCAAACGAT
TATACCCAACAAGCAACCCAAAGCTATGGGGCCTACCCCACCCAGCCCGGGCAGGGCTATTCCCAGCAGAGCAGT
CAGCCCTACGGACAGCAGAGTTACAGTGGTTATAGCCAGTCCACGGACACTTCAGGATATGGCCAGAGCAGCTAT
TCTTCTTATGGCCAGAGCCAGAACACAGGCTATGGAACTCAGTCAACTCCCCAGGGATATGGCTCGACTGGCGGC
TATGGCAGTAGCCAGAGCTCCCAATCGTCTTACGGGCAGCAGTCCTCCTACCCTGGCTATGGCCAGCAGCCAGCT
CCCAGCAGCACCTCGGGAAGTTACGGTAGCAGTTCTCAGAGCAGCAGCTATGGGCAGCCCCAGAGTGGGAGCTAC
AGCCAGCAGCCTAGCTATGGTGGACAGCAGCAAAGCTATGGACAGCAGCAAAGCTATAATCCCCCTCAGGGCTAT
GGACAGCAGAACCAGTACAACAGCAGCAGTGGTGGTGGAGGTGGAGGTGGAGGTGGAGGTAACTATGGCCAAGAT
CAATCCTCCATGAGTAGTGGTGGTGGCAGTGGTGGCGGTTATGGCAATCAAGACCAGAGTGGTGGAGGTGGCAGC
GGTGGCTATGGACAGCAGGACCGTGGAGGCCGCGGCAGGGGTGGCAGTGGTGGCGGCGGCGGCGGCGGCGGTGGT
GGTTACAACCGCAGCAGTGGTGGCTATGAACCCAGAGGTCGTGGAGGTGGCCGTGGAGGCAGAGGTGGCATGGGC
GGAAGTGACCGTGGTGGCTTCAATAAATTTGGTGGCCCTCGGGACCAAGGATCACGTCATGACTCCGAACAGGAT
AATTCAGACAACAACACCATCTTTGTGCAAGGCCTGGGTGAGAATGTTACAATTGAGTCTGTGGCTGATTACTTC
AAGCAGATTGGTATTATTAAGACAAACAAGAAAACGGGACAGCCCATGATTAATTTGTACACAGACAGGGAAACT
GGCAAGCTGAAGGGAGAGGCAACGGTCTCTTTTGATGACCCACCTTCAGCTAAAGCAGCTATTGACTGGTTTGAT

GGTAAAGAATTCTCCGGAAATCCTATCAAGGTCTCATTTGCTACTCGCCGGGCAGACTTTAATCGGGGTGGTGGC
AATGGTCGTGGAGGCCGAGGGCGAGGAGGACCCATGGGCCGTGGAGGCTATGGAGGTGGTGGCAGTGGTGGTGGT
GGCCGAGGAGGATTTCCCAGTGGAGGTGGTGGCGGTGGAGGACAGCAGCGAGCTGGTGACTGGAAGTGTCCTAAT
CCCACCTGTGAGAATATGAACTTCTCTTGGAGGAATGAATGCAACCAGTGTAAGGCCCCTAAACCAGATGGCCCA
GGAGGGGGACCAGGTGGCTCTCACATGGGGGGTAACTACGGGGATGATCGTCGTGGTGGCAGAGGAGGCGCGATC
GCAAATGAGGTGACCACCCTGGAAAACGACGCCGCCTTCATCGAGAACGAGAACGCCTACCTGGAAAAAGAGATC
GCCAGACTGAGAAAGGAAAAGGCCGCTCTGCGGAACAGACTGGCCCACAAGAAGGGCAAGCCTATTCCCAACCCC
CTGCTGGGCCTGGATAGCACCGGAGCACCAGGAAGTGCTGGTTCTGCTGCTGGTAGTGGAGCGTGCCCGGTGCCG
CTGCAGCTGCCGCCGCTGGAACGCCTGACCCTGGATGACAAAAAACCGCTGAATACCCTGATCTCTGCTACTGGT
CTGTGGATGAGTCGTACCGGAACCATTCATAAAATCAAACACCACGAGGTTAGCCGTTCGAAAATCTATATTGAG
ATGGCGTGTGGCGATCATCTGGTTGTGAACAATAGCCGCTCTTCTCGTACAGCACGTGCACTGCGTCACCACAAA
TATCGTAAAACCTGTAAACGTTGCCGTGTGTCCGATGAGGATCTGAACAAATTCCTGACAAAAGCCAATGAGGAC
CAAACAAGCGTGAAAGTGAAAGTCGTTAGCGCTCCTACCCGTACTAAAAAAGCAATGCCGAAATCCGTTGCTCGT
GCCCCTAAACCACTGGAAAACACTGAAGCAGCACAGGCACAGCCGTCTGGAAGCAAATTCTCTCCGGCCATTCCT
GTTTCTACCCAGGAGTCCGTTTCTGTTCCAGCAAGTGTGAGCACCAGCATTAGCAGTATTAGCACCGGTGCCACC
GCTAGCGCCCTGGTTAAAGGCAATACCAATCCGATTACAAGCATGTCTGCCCCGGTTCAAGCATCAGCTCCAGCA
CTGACAAAATCCCAAACCGATCGTCTGGAGGTTCTGCTGAATCCGAAAGACGAAATCAGCCTGAATTCCGGCAAA
CCGTTTCGTGAACTGGAGAGCGAACTGCTGTCACGTCGTAAAAAAGACCTGCAACAAATCTATGCCGAAGAACGT
GAGAACTATCTGGGGAAACTGGAACGTGAAATCACCCGCTTTTTCGTGGATCGTGGCTTTCTGGAGATCAAATCC
CCGATTCTGATTCCTCTGGAGTATATCGAGCGTATGGGCATCGACAATGATACCGAACTGAGCAAACAAATTTTC
CGTGTGGATAAAAACTTCTGTCTGCGCCCTATGCTGGCACCAAATCTGTATAACTATCTGCGCAAACTGGACCGT
GCCCTGCCTGATCCTATCAAAATCTTCGAGATCGGCCCGTGTTATCGTAAAGAGTCCGACGGTAAAGAACATCTG
GAGGAGTTTACCATGCTGGCCTTTGCCCAAATGGGTTCAGGTTGTACTCGTGAGAACCTGGAAAGCATCATCACC
GATTTTCTGAACCACCTGGGCATTGACTTCAAAATTGTGGGCGACAGCTGTATGGTGTATGGCGACACCCTGGAT
GTCATGCACGGCGACCTGGAACTGTCTAGTGCCGTTGTTGGACCAATTCCGCTGGACCGTGAGTGGGGTATCGAC
AAACCGTGGATCGGAGCAGGATTCGGTCTGGAACGCCTGCTGAAAGTGAAACACGACTTCAAAAACATCAAACGT
GCCGCCCGTTCTGAATCGTATTATAACGGGATCTCTACGAACCTGTAA (SEQ ID NO: 553)

Protein:
MVGRNSAIAAGVCGALFIGYCIYFDRKRRSDPNFKNRLRERRKKQKLAKERAGLSKLPDLKDAEAVQKFFMASND
YTQQATQSYGAYPTQPGQGYSQQSSQPYGQQSYSGYSQSTDTSGYGQSSYSSYGQSQNTGYGTQSTPQGYGSTGG
YGSSQSSQSSYGQQSSYPGYGQQPAPSSTSGSYGSSSQSSSYGQPQSGSYSQQPSYGGQQQSYGQQQSYNPPQGY
GQQNQYNSSSGGGGGGGGGGNYGQDQSSMSSGGGSGGGYGNQDQSGGGGSGGYGQQDRGGRGRGGSGGGGGGGGG
GYNRSSGGYEPRGRGGGRGGRGGMGGSDRGGFNKFGGPRDQGSRHDSEQDNSDNNTIFVQGLGENVTIESVADYF
KQIGIIKTNKKTGQPMINLYTDRETGKLKGEATVSFDDPPSAKAAIDWFDGKEFSGNPIKVSFATRRADFNRGGG
NGRGGRGRGGPMGRGGYGGGGSGGGGRGGFPSGGGGGGGQQRAGDWKCPNPTCENMNFSWRNECNQCKAPKPDGP
GGGPGGSHMGGNYGDDRRGGRGGAIANEVTTLENDAAFIENENAYLEKEIARLRKEKAALRNRLAHKKGKPIPNP
LLGLDSTGAPGSAGSAAGSGACPVPLQLPPLERLTLDDKKPLNTLISATGLWMSRTGTIHKIKHHEVSRSKIYIE
MACGDHLVVNNSRSSRTARALRHHKYRKTCKRCRVSDEDLNKFLTKANEDQTSVKVKVVSAPTRTKKAMPKSVAR
APKPLENTEAAQAQPSGSKFSPAIPVSTQESVSVPASVSTSISSISTGATASALVKGNTNPITSMSAPVQASAPA
LTKSQTDRLEVLLNPKDEISLNSGKPFRELESELLSRRKKDLQQIYAEERENYLGKLEREITRFFVDRGFLEIKS
PILIPLEYIERMGIDNDTELSKQIFRVDKNFCLRPMLAPNLYNYLRKLDRALPDPIKIFEIGPCYRKESDGKEHL
EEFTMLAFAQMGSGCTRENLESIITDFLNHLGIDFKIVGDSCMVYGDTLDVMHGDLELSSAVVGPIPLDREWGID
KPWIGAGFGLERLLKVKHDFKNIKRAARSESYYNGISTNL* (SEQ ID NO: 554)

TOM20::FUS::SYNZIP3::PylRS(AAAF)
DNA:
ATGGTGGGTCGGAACAGCGCCATCGCCGCCGGTGTATGCGGGGCCCTTTTCATTGGGTACTGCATCTACTTCGAC
CGCAAAAGACGAAGTGACCCCAACTTCAAGAACAGGCTTCGAGAACGAAGAAAGAAACAGAAGCTTGCCAAGGAG
AGAGCTGGGCTTTCCAAGTTACCTGACCTTAAAGATGCTGAAGCTGTTCAGAAATTCTTCATGGCCTCAAACGAT
TATACCCAACAAGCAACCCAAAGCTATGGGGCCTACCCCACCCAGCCCGGGCAGGGCTATTCCCAGCAGAGCAGT
CAGCCCTACGGACAGCAGAGTTACAGTGGTTATAGCCAGTCCACGGACACTTCAGGATATGGCCAGAGCAGCTAT
TCTTCTTATGGCCAGAGCCAGAACACAGGCTATGGAACTCAGTCAACTCCCCAGGGATATGGCTCGACTGGCGGC
TATGGCAGTAGCCAGAGCTCCCAATCGTCTTACGGGCAGCAGTCCTCCTACCCTGGCTATGGCCAGCAGCCAGCT
CCCAGCAGCACCTCGGGAAGTTACGGTAGCAGTTCTCAGAGCAGCAGCTATGGGCAGCCCCAGAGTGGGAGCTAC
AGCCAGCAGCCTAGCTATGGTGGACAGCAGCAAAGCTATGGACAGCAGCAAAGCTATAATCCCCCTCAGGGCTAT
GGACAGCAGAACCAGTACAACAGCAGCAGTGGTGGTGGAGGTGGAGGTGGAGGTGGAGGTAACTATGGCCAAGAT

CAATCCTCCATGAGTAGTGGTGGTGGCAGTGGTGGCGGTTATGGCAATCAAGACCAGAGTGGTGGAGGTGGCAGC
GGTGGCTATGGACAGCAGGACCGTGGAGGCCGCGGCAGGGGTGGCAGTGGTGGCGGCGGCGGCGGCGGCGGTGGT
GGTTACAACCGCAGCAGTGGTGGCTATGAACCCAGAGGTCGTGGAGGTGGCCGTGGAGGCAGAGGTGGCATGGGC
GGAAGTGACCGTGGTGGCTTCAATAAATTTGGTGGCCCTCGGGACCAAGGATCACGTCATGACTCCGAACAGGAT
AATTCAGACAACAACACCATCTTTGTGCAAGGCCTGGGTGAGAATGTTACAATTGAGTCTGTGGCTGATTACTTC
AAGCAGATTGGTATTATTAAGACAAACAAGAAAACGGGACAGCCCATGATTAATTTGTACACAGACAGGGAAACT
GGCAAGCTGAAGGGAGAGGCAACGGTCTCTTTTGATGACCCACCTTCAGCTAAAGCAGCTATTGACTGGTTTGAT
GGTAAAGAATTCTCCGGAAATCCTATCAAGGTCTCATTTGCTACTCGCCGGGCAGACTTTAATCGGGGTGGTGGC
AATGGTCGTGGAGGCCGAGGGCGAGGAGGACCCATGGGCCGTGGAGGCTATGGAGGTGGTGGCAGTGGTGGTGGT
GGCCGAGGAGGATTTCCCAGTGGAGGTGGTGGCGGTGGAGGACAGCAGCGAGCTGGTGACTGGAAGTGTCCTAAT
CCCACCTGTGAGAATATGAACTTCTCTTGGAGGAATGAATGCAACCAGTGTAAGGCCCCTAAACCAGATGGCCCA
GGAGGGGGACCAGGTGGCTCTCACATGGGGGGTAACTACGGGGATGATCGTCGTGGTGGCAGAGGAGGCGCGATC
GCAAATGAGGTGACCACCCTGGAAAACGACGCCGCCTTCATCGAGAACGAGAACGCCTACCTGGAAAAAGAGATC
GCCAGACTGAGAAAGGAAAAGGCCGCTCTGCGGAACAGACTGGCCCACAAGAAGGGCAAGCCTATTCCCAACCCC
CTGCTGGGCCTGGATAGCACCGGAGCACCAGGAAGTGCTGGTTCTGCTGCTGGTAGTGGAGCGTGCCCGGTGCCG
CTGCAGCTGCCGCCGCTGGAACGCCTGACCCTGGATGACAAAAAACCGCTGAATACCCTGATCTCTGCTACTGGT
CTGTGGATGAGTCGTACCGGAACCATTCATAAAATCAAACACCACGAGGTTAGCCGTTCGAAAATCTATATTGAG
ATGGCGTGTGGCGATCATCTGGTTGTGAACAATAGCCGCTCTTCTCGTACAGCACGTGCACTGCGTCACCACAAA
TATCGTAAAACCTGTAAACGTTGCCGTGTGTCCGATGAGGATCTGAACAAATTCCTGACAAAAGCCAATGAGGAC
CAAACAAGCGTGAAAGTGAAAGTCGTTAGCGCTCCTACCCGTACTAAAAAAGCAATGCCGAAATCCGTTGCTCGT
GCCCCTAAACCACTGGAAAACACTGAAGCAGCACAGGCACAGCCGTCTGGAAGCAAATTCTCTCCGGCCATTCCT
GTTTCTACCCAGGAGTCCGTTTCTGTTCCAGCAAGTGTGAGCACCAGCATTAGCAGTATTAGCACCGGTGCCACC
GCTAGCGCCCTGGTTAAAGGCAATACCAATCCGATTACAAGCATGTCTGCCCCGGTTCAAGCATCAGCTCCAGCA
CTGACAAAATCCCAAACCGATCGTCTGGAGGTTCTGCTGAATCCGAAAGACGAAATCAGCCTGAATTCCGGCAAA
CCGTTTCGTGAACTGGAGAGCGAACTGCTGTCACGTCGTAAAAAAGACCTGCAACAAATCTATGCCGAAGAACGT
GAGAACTATCTGGGGAAACTGGAACGTGAAATCACCCGCTTTTTCGTGGATCGTGGCTTTCTGGAGATCAAATCC
CCGATTCTGATTCCTCTGGAGTATATCGAGCGTATGGGCATCGACAATGATACCGAACTGAGCAAACAAATTTTC
CGTGTGGATAAAAACTTCTGTCTGCGCCCTATGCTAGCACCAAATCTGGCTAACTATCTGCGCAAACTGGACCGT
GCCCTGCCTGATCCTATCAAAATCTTCGAGATCGGCCCGTGTTATCGTAAAGAGTCCGACGGTAAAGAACATCTG
GAGGAGTTTACCATGCTGGCCTTTGCCCAAATGGGTTCAGGTTGTACTCGTGAGAACCTGGAAAGCATCATCACC
GATTTTCTGAACCACCTGGGCATTGACTTCAAAATTGTGGGCGACAGCTGTATGGTGTTTGGCGACACCCTGGAT
GTCATGCACGGCGACCTGGAACTGTCTAGTGCCGTTGTGGGCCCAATCCCGCTGGATCGTGAGTGGGGTATCGAC
AAACCTTGGATCGGTGCGGGTTTTGGTCTGGAGCGTCTGCTGAAAGTAAAACACGACTTCAAGAACATCAAACGT
GCTGCACGTTCCGAGTCCTATTACAATGGTATTTCTACTAACCTGTAA (SEQ ID NO: 555)

Protein:
MVGRNSAIAAGVCGALFIGYCIYFDRKRRSDPNFKNRLRERRKKQKLAKERAGLSKLPDLKDAEAVQKFFMASND
YTQQATQSYGAYPTQPGQGYSQQSSQPYGQQSYSGYSQSTDTSGYGQSSYSSYGQSQNTGYGTQSTPQGYGSTGG
YGSSQSSQSSYGQQSSYPGYGQQPAPSSTSGSYGSSSQSSSYGQPQSGSYSQQPSYGGQQQSYGQQQSYNPPQGY
GQQNQYNSSSGGGGGGGGGGNYGQDQSSMSSGGGSGGGYGNQDQSGGGGSGGYGQQDRGGRGRGGSGGGGGGGGG
GYNRSSGGYEPRGRGGGRGGRGGMGGSDRGGFNKFGGPRDQGSRHDSEQDNSDNNTIFVQGLGENVTIESVADYF
KQIGIIKTNKKTGQPMINLYTDRETGKLKGEATVSFDDPPSAKAAIDWFDGKEFSGNPIKVSFATRRADFNRGGG
NGRGGRGRGGPMGRGGYGGGGSGGGGRGGFPSGGGGGGGQQRAGDWKCPNPTCENMNFSWRNECNQCKAPKPDGP
GGGPGGSHMGGNYGDDRRGGRGGAIANEVTTLENDAAFIENENAYLEKEIARLRKEKAALRNRLAHKKGKPIPNP
LLGLDSTGAPGSAGSAAGSGACPVPLQLPPLERLTLDDKKPLNTLISATGLWMSRTGTIHKIKHHEVSRSKIYIE
MACGDHLVVNNSRSSRTARALRHHKYRKTCKRCRVSDEDLNKFLTKANEDQTSVKVKVVSAPTRTKKAMPKSVAR
APKPLENTEAAQAQPSGSKFSPAIPVSTQESVSVPASVSTSISSISTGATASALVKGNTNPITSMSAPVQASAPA
LTKSQTDRLEVLLNPKDEISLNSGKPFRELESELLSRRKKDLQQIYAEERENYLGKLEREITRFFVDRGFLEIKS
PILIPLEYIERMGIDNDTELSKQIFRVDKNFCLRPMLAPNLANYLRKLDRALPDPIKIFEIGPCYRKESDGKEHL
EEFTMLAFAQMGSGCTRENLESIITDFLNHLGIDFKIVGDSCMVFGDTLDVMHGDLELSSAVVGPIPLDREWGID
KPWIGAGFGLERLLKVKHDFKNIKRAARSESYYNGISTNL* (SEQ ID NO: 556)

LCK::FUS::SYNZIP3::PylRS(AF)
DNA:
ATGGGCTGCGTGTGCAGCAGCAACCCCGAGGGTACCGAGCTCATGGCCTCAAACGATTATACCCAACAAGCAACC
CAAAGCTATGGGGCCTACCCCACCCAGCCCGGGCAGGGCTATTCCCAGCAGAGCAGTCAGCCCTACGGACAGCAG
AGTTACAGTGGTTATAGCCAGTCCACGGACACTTCAGGATATGGCCAGAGCAGCTATTCTTCTTATGGCCAGAGC

CAGAACACAGGCTATGGAACTCAGTCAACTCCCCAGGGATATGGCTCGACTGGCGGCTATGGCAGTAGCCAGAGC
TCCCAATCGTCTTACGGGCAGCAGTCCTCCTACCCTGGCTATGGCCAGCAGCCAGCTCCCAGCAGCACCTCGGGA
AGTTACGGTAGCAGTTCTCAGAGCAGCAGCTATGGGCAGCCCCAGAGTGGGAGCTACAGCCAGCAGCCTAGCTAT
GGTGGACAGCAGCAAAGCTATGGACAGCAGCAAAGCTATAATCCCCCTCAGGGCTATGGACAGCAGAACCAGTAC
AACAGCAGCAGTGGTGGTGGAGGTGGAGGTGGAGGTGGAGGTAACTATGGCCAAGATCAATCCTCCATGAGTAGT
GGTGGTGGCAGTGGTGGCGGTTATGGCAATCAAGACCAGAGTGGTGGAGGTGGCAGCGGTGGCTATGGACAGCAG
GACCGTGGAGGCCGCGGCAGGGGTGGCAGTGGTGGCGGCGGCGGCGGCGGTGGTGGTTACAACCGCAGCAGT
GGTGGCTATGAACCCAGAGGTCGTGGAGGTGGCCGTGGAGGCAGAGGTGGCATGGGCGGAAGTGACCGTGGTGGC
TTCAATAAATTTGGTGGCCCTCGGGACCAAGGATCACGTCATGACTCCGAACAGGATAATTCAGACAACAACACC
ATCTTTGTGCAAGGCCTGGGTGAGAATGTTACAATTGAGTCTGTGGCTGATTACTTCAAGCAGATTGGTATTATT
AAGACAAACAAGAAAACGGGACAGCCCATGATTAATTTGTACACAGACAGGGAAACTGGCAAGCTGAAGGGAGAG
GCAACGGTCTCTTTTGATGACCCACCTTCAGCTAAAGCAGCTATTGACTGGTTTGATGGTAAAGAATTCTCCGGA
AATCCTATCAAGGTCTCATTTGCTACTCGCCGGGCAGACTTTAATCGGGGTGGTGGCAATGGTCGTGGAGGCCGA
GGGCGAGGAGGACCCATGGGCCGTGGAGGCTATGGAGGTGGTGGCAGTGGTGGTGGTGGCCGAGGAGGATTTCCC
AGTGGAGGTGGTGGCGGTGGAGGACAGCAGCGAGCTGGTGACTGGAAGTGTCCTAATCCCACCTGTGAGAATATG
AACTTCTCTTGGAGGAATGAATGCAACCAGTGTAAGGCCCCTAAACCAGATGGCCCAGGAGGGGGACCAGGTGGC
TCTCACATGGGGGGTAACTACGGGGATGATCGTCGTGGTGGCAGAGGTGGTGCGATCGCAAATGAGGTGACCACC
CTGGAAAACGACGCCGCCTTCATCGAGAACGAGAACGCCTACCTGGAAAAAGAGATCGCCAGACCTGAGAAAGGAA
AAGGCCGCTCTGCGGAACAGACTGGCCCACAAGAAGGGCGCCGATTACAAGGACGATGATGACAAGGGAGCACCA
GGAAGTGCTGGTTCTGCTGCTGGTAGTGGAGCGTGCCCGGTGCCGCTGCAGCTGCCGCCGCTGGAACGCCTGACC
CTGGATGATAAAAAACCGCTGAATACCCTGATCTCTGCTACTGGTCTGTGGATGAGTCGTACCGGAACCATTCAT
AAAATCAAACACCACGAGGTTAGCCGTTCGAAAATCTATATTGAGATGGCGTGTGGCGATCATCTGGTTGTGAAC
AATAGCCGCTCTTCTCGTACAGCACGTGCACTGCGTCACCACAAATATCGTAAAACCTGTAAACGTTGCCGTGTG
TCCGATGAGGATCTGAACAAATTCCTGACAAAAGCCAATGAGGACCAAACAAGCGTGAAAGTGAAAGTCGTTAGC
GCTCCTACCCGTACTAAAAAAGCAATGCCGAAATCCGTTGCTCGTGCCCCTAAACCACTGGAAAACACTGAAGCA
GCACAGGCACAGCCGTCTGGAAGCAAATTCTCTCCGGCCATTCCTGTTTCTACCCAGGAGTCCGTTTCTGTTCCA
GCAAGTGTGAGCACCAGCATTAGCAGTATTAGCACCGGTGCCACCGCTAGCGCCCTGGTTAAAGGCAATACCAAT
CCGATTACAAGCATGTCTGCCCCGGTTCAAGCATCAGCTCCAGCACTGACAAAATCCCAAACCGATCGTCTGGAG
GTTCTGCTGAATCCGAAAGACGAAATCAGCCTGAATTCCGGCAAACCGTTTCGTGAACTGGAGAGCGAACTGCTG
TCACGTCGTAAAAAAGACCTGCAACAAATCTATGCCGAAGAACGTGAGAACTATCTGGGGAAACTGGAACGTGAA
ATCACCCGCTTTTTCGTGGATCGTGGCTTTCTGGAGATCAAATCCCCGATTCTGATTCCTCTGGAGTATATCGAG
CGTATGGGCATCGACAATGATACCGAACTGAGCAAACAAATTTTCCGTGTGGATAAAAACTTCTGTCTGCGCCCT
ATGCTAGCACCAAATCTGGCTAACTATCTGCGCAAACTGGACCGTGCCCTGCCTGATCCTATCAAAATCTTCGAG
ATCGGCCCGTGTTATCGTAAAGAGTCCGACGGTAAAGAACATCTGGAGGAGTTTACCATGCTGAACTTTTGCCAA
ATGGGTTCAGGTTGTACTCGTGAGAACCTGGAAAGCATCATCACCGATTTTCTGAACCACCTGGGCATTGACTTC
AAAATTGTGGGCGACAGCTGTATGGTGTTTGGCGACACCCTGGATGTCATGCACGGCGACCTGGAACTGTCTAGT
GCCGTTGTGGGCCCAATCCCGCTGGATCGTGAGTGGGGTATCGACAAACCTTGGATCGGTGCGGGTTTTGGTCTG
GAGCGTCTGCTGAAAGTAAAACACGACTTCAAGAACATCAAACGTGCTGCACGTTCCGAGTCCTATTACAATGGT
ATTTCTACTAACCTGTAA (SEQ ID NO: 557)

Protein:
MGCVCSSNPEGTELMASNDYTQQATQSYGAYPTQPGQGYSQQSSQPYGQQSYSGYSQSTDTSGYGQSSYSSYGQS
QNTGYGTQSTPQGYGSTGGYGSSQSSQSSYGQQSSYPGYGQQPAPSSTSGSYGSSSQSSSYGQPQSGSYSQQPSY
GGQQQSYGQQQSYNPPQGYGQQNQYNSSSGGGGGGGGGNYGQDQSSMSSGGGSGGGYGNQDQSGGGGSGGYGQQ
DRGGRGRGGSGGGGGGGGGYNRSSGGYEPRGRGGGRGGRGGMGGSDRGGFNKFGGPRDQGSRHDSEQDNSDNNT
IFVQGLGENVTIESVADYFKQIGIIKTNKKTGQPMINLYTDRETGKLKGEATVSFDDPPSAKAAIDWFDGKEFSG
NPIKVSFATRRADFNRGGGNGRGGRGRGGPMGRGGYGGGGSGGGGRGGFPSGGGGGGGQQRAGDWKCPNPTCENM
NFSWRNECNQCKAPKPDGPGGGPGGSHMGGNYGDDRRGGRGGAIANEVTTLENDAAFIENENAYLEKEIARLRKE
KAALRNRLAHKKGADYKDDDDKGAPGSAGSAAGSGACPVPLQLPPLERLTLDDKKPLNTLISATGLWMSRTGTIH
KIKHHEVSRSKIYIEMACGDHLVVNNSRSSRTARALRHHKYRKTCKRCRVSDEDLNKFLTKANEDQTSVKVKVVS
APTRTKKAMPKSVARAPKPLENTEAAQAQPSGSKFSPAIPVSTQESVSVPASVSTSISSISTGATASALVKGNTN
PITSMSAPVQASAPALTKSQTDRLEVLLNPKDEISLNSGKPFRELESELLSRRKKDLQQIYAEERENYLGKLERE
ITRFFVDRGFLEIKSPILIPLEYIERMGIDNDTELSKQIFRVDKNFCLRPMLAPNLANYLRKLDRALPDPIKIFE
IGPCYRKESDGKEHLEEFTMLNFCQMGSGCTRENLESIITDFLNHLGIDFKIVGDSCMVFGDTLDVMHGDLELSS
AVVGPIPLDREWGIDKPWIGAGFGLERLLKVKHDFKNIKRAARSESYYNGISTNL* (SEQ ID NO: 558)

LCK::SYNZIP1::PylRS(AF)

DNA:
ATGGGCTGCGTGTGCAGCAGCAACCCCGAGGGTACCGAGCTCGCGATCGCAAATCTGGTGGCCCAGCTGGAAAAC
GAGGTGGCCAGCCTGGAAAACGAGAACGAAACCCTGAAGAAAAAGAACCTGCACAAGAAGGACCTGATCGCCTAC
CTGGAAAAGGAAATCGCCAACCTGAGAAAGAAGATCGAGGAAGGCAAGCCTATTCCCAACCCCCTGCTGGGCCTG
GATAGCACCGGAGCACCAGGAAGTGCTGGTTCTGCTGCTGGTAGTGGAGCGTGCCCGGTGCCGCTGCAGCTGCCG
CCGCTGGAACGCCTGACCCTGGATGACAAAAAACCGCTGAATACCCTGATCTCTGCTACTGGTCTGTGGATGAGT
CGTACCGGAACCATTCATAAAATCAAACACCACGAGGTTAGCCGTTCGAAAATCTATATTGAGATGGCGTGTGGC
GATCATCTGGTTGTGAACAATAGCCGCTCTTCTCGTACAGCACGTGCACTGCGTCACCACAAATATCGTAAAACC
TGTAAACGTTGCCGTGTGTCCGATGAGGATCTGAACAAATTCCTGACAAAAGCCAATGAGGACCAAACAAGCGTG
AAAGTGAAAGTCGTTAGCGCTCCTACCCGTACTAAAAAAGCAATGCCGAAATCCGTTGCTCGTGCCCCTAAACCA
CTGGAAAACACTGAAGCAGCACAGGCACAGCCGTCTGGAAGCAAATTCTCTCCGGCCATTCCTGTTTCTACCCAG
GAGTCCGTTTCTGTTCCAGCAAGTGTGAGCACCAGCATTAGCAGTATTAGCACCGGTGCCACCGCTAGCGCCCTG
GTTAAAGGCAATACCAATCCGATTACAAGCATGTCTGCCCCGGTTCAAGCATCAGCTCCAGCACTGACAAAATCC
CAAACCGATCGTCTGGAGGTTCTGCTGAATCCGAAAGACGAAATCAGCCTGAATTCCGGCAAACCGTTTCGTGAA
CTGGAGAGCGAACTGCTGTCACGTCGTAAAAAAGACCTGCAACAAATCTATGCCGAAGAACGTGAGAACTATCTG
GGGAAACTGGAACGTGAAATCACCCGCTTTTTCGTGGATCGTGGCTTTCTGGAGATCAAATCCCCGATTCTGATT
CCTCTGGAGTATATCGAGCGTATGGGCATCGACAATGATACCGAACTGAGCAAACAAATTTTCCGTGTGGATAAA
AACTTCTGTCTGCGCCCTATGCTAGCACCAAATCTGGCTAACTATCTGCGCAAACTGGACCGTGCCCTGCCTGAT
CCTATCAAAATCTTCGAGATCGGCCCGTGTTATCGTAAAGAGTCCGACGGTAAAGAACATCTGGAGGAGTTTACC
ATGCTGAACTTTTGCCAAATGGGTTCAGGTTGTACTCGTGAGAACCTGGAAAGCATCATCACCGATTTTCTGAAC
CACCTGGGCATTGACTTCAAAATTGTGGGCGACAGCTGTATGGTGTTTGGCGACACCCTGGATGTCATGCACGGC
GACCTGGAACTGTCTAGTGCCGTTGTGGGCCCAATCCCGCTGGATCGTGAGTGGGGTATCGACAAACCTTGGATC
GGTGCGGGTTTTGGTCTGGAGCGTCTGCTGAAAGTAAAACACGACTTCAAGAACATCAAACGTGCTGCACGTTCC
GAGTCCTATTACAATGGTATTTCTACTAACCTGTAA (SEQ ID NO: 559)

Protein:
MGCVCSSNPEGTELAIANLVAQLENEVASLENENETLKKKNLHKKDLIAYLEKEIANLRKKIEEGKPIPNPLLGL
DSTGAPGSAGSAAGSGACPVPLQLPPLERLTLDDKKPLNTLISATGLWMSRTGTIHKIKHHEVSRSKIYIEMACG
DHLVVNNSRSSRTARALRHHKYRKTCKRCRVSDEDLNKFLTKANEDQTSVKVKVVSAPTRTKKAMPKSVARAPKP
LENTEAAQAQPSGSKFSPAIPVSTQESVSVPASVSTSISSISTGATASALVKGNTNPITSMSAPVQASAPALTKS
QTDRLEVLLNPKDEISLNSGKPFRELESELLSRRKKDLQQIYAEERENYLGKLEREITRFFVDRGFLEIKSPILI
PLEYIERMGIDNDTELSKQIFRVDKNFCLRPMLAPNLANYLRKLDRALPDPIKIFEIGPCYRKESDGKEHLEEFT
MLNFCQMGSGCTRENLESIITDFLNHLGIDFKIVGDSCMVFGDTLDVMHGDLELSSAVVGPIPLDREWGIDKPWI
GAGFGLERLLKVKHDFKNIKRAARSESYYNGISTNL* (SEQ ID NO: 560)

LCK::SYNZIP3::PylRS(AF)
DNA:
ATGGGCTGCGTGTGCAGCAGCAACCCCGAGGGTACCGAGCTCGCGATCGCAAATGAGGTGACCACCCTGGAAAAC
GACGCCGCCTTCATCGAGAACGAGAACGCCTACCTGGAAAAAGAGATCGCCAGACTGAGAAAGGAAAAGGCCGCT
CTGCGGAACAGACTGGCCCACAAGAAGGGCAAGCCTATTCCCAACCCCCTGCTGGGCCTGGATAGCACCGGAGCA
CCAGGAAGTGCTGGTTCTGCTGCTGGTAGTGGAGCGTGCCCGGTGCCGCTGCAGCTGCCGCCGCTGGAACGCCTG
ACCCTGGATGACAAAAAACCGCTGAATACCCTGATCTCTGCTACTGGTCTGTGGATGAGTCGTACCGGAACCATT
CATAAAATCAAACACCACGAGGTTAGCCGTTCGAAAATCTATATTGAGATGGCGTGTGGCGATCATCTGGTTGTG
AACAATAGCCGCTCTTCTCGTACAGCACGTGCACTGCGTCACCACAAATATCGTAAAACCTGTAAACGTTGCCGT
GTGTCCGATGAGGATCTGAACAAATTCCTGACAAAAGCCAATGAGGACCAAACAAGCGTGAAAGTGAAAGTCGTT
AGCGCTCCTACCCGTACTAAAAAAGCAATGCCGAAATCCGTTGCTCGTGCCCCTAAACCACTGGAAAACACTGAA
GCAGCACAGGCACAGCCGTCTGGAAGCAAATTCTCTCCGGCCATTCCTGTTTCTACCCAGGAGTCCGTTTCTGTT
CCAGCAAGTGTGAGCACCAGCATTAGCAGTATTAGCACCGGTGCCACCGCTAGCGCCCTGGTTAAAGGCAATACC
AATCCGATTACAAGCATGTCTGCCCCGGTTCAAGCATCAGCTCCAGCACTGACAAAATCCCAAACCGATCGTCTG
GAGGTTCTGCTGAATCCGAAAGACGAAATCAGCCTGAATTCCGGCAAACCGTTTCGTGAACTGGAGAGCGAACTG
CTGTCACGTCGTAAAAAAGACCTGCAACAAATCTATGCCGAAGAACGTGAGAACTATCTGGGGAAACTGGAACGT
GAAATCACCCGCTTTTTCGTGGATCGTGGCTTTCTGGAGATCAAATCCCCGATTCTGATTCCTCTGGAGTATATC
GAGCGTATGGGCATCGACAATGATACCGAACTGAGCAAACAAATTTTCCGTGTGGATAAAAACTTCTGTCTGCGC
CCTATGCTAGCACCAAATCTGGCTAACTATCTGCGCAAACTGGACCGTGCCCTGCCTGATCCTATCAAAATCTTC
GAGATCGGCCCGTGTTATCGTAAAGAGTCCGACGGTAAAGAACATCTGGAGGAGTTTACCATGCTGAACTTTTGC
CAAATGGGTTCAGGTTGTACTCGTGAGAACCTGGAAAGCATCATCACCGATTTTCTGAACCACCTGGGCATTGAC
TTCAAAATTGTGGGCGACAGCTGTATGGTGTTTGGCGACACCCTGGATGTCATGCACGGCGACCTGGAACTGTCT

AGTGCCGTTGTGGGCCCAATCCCGCTGGATCGTGAGTGGGGTATCGACAAACCTTGGATCGGTGCGGGTTTTGGT
CTGGAGCGTCTGCTGAAAGTAAAACACGACTTCAAGAACATCAAACGTGCTGCACGTTCCGAGTCCTATTACAAT
GGTATTTCTACTAACCTGTAA (SEQ ID NO: 561)

Protein:
MGCVCSSNPEGTELAIANEVTTLENDAAFIENENAYLEKEIARLRKEKAALRNRLAHKKGKPIPNPLLGLDSTGA
PGSAGSAAGSGACPVPLQLPPLERLTLDDKKPLNTLISATGLWMSRTGTIHKIKHHEVSRSKIYIEMACGDHLVV
NNSRSSRTARALRHHKYRKTCKRCRVSDEDLNKFLTKANEDQTSVKVKVVSAPTRTKKAMPKSVARAPKPLENTE
AAQAQPSGSKFSPAIPVSTQESVSVPASVSTSISSISTGATASALVKGNTNPITSMSAPVQASAPALTKSQTDRL
EVLLNPKDEISLNSGKPFRELESELLSRRKKDLQQIYAEERENYLGKLEREITRFFVDRGFLEIKSPILIPLEYI
ERMGIDNDTELSKQIFRVDKNFCLRPMLAPNLANYLRKLDRALPDPIKIFEIGPCYRKESDGKEHLEEFTMLNFC
QMGSGCTRENLESIITDFLNHLGIDFKIVGDSCMVFGDTLDVMHGDLELSSAVVGPIPLDREWGIDKPWIGAGFG
LERLLKVKHDFKNIKRAARSESYYNGISTNL* (SEQ ID NO: 562)

SYNZIP2::MCP
DNA:
ATGGCGATCGCAGCTAGAAACGCCTACCTGAGAAAGAAAATCGCCAGACTGAAGAAGGACAACCTGCAGCTGGAA
AGAGACGAGCAGAACCTGGAAAAGATCATCGCCAACCTCAGAGATGAGATCGCCAGACTGGAAAACGAGGTGGCC
AGCCACGAGCAGTATCCCTATGATGTGCCGGATTATGCTGGAGCACCAGGAAGTGCTGGTTCTGCTGCTGGTAGT
GGAGCTTCTAACTTTACTCAGTTCGTTCTCGTCGACAATGGCGGAACTGGCGACGTGACTGTCGCCCCAAGCAAC
TTCGCTAACGGGATCGCTGAATGGATCAGCTCTAACTCGCGTTCACAGGCTTACAAAGTAACCTGTAGCGTTCGT
CAGAGCTCTGCGCAGAATCGCAAATACACCATCAAAGTCGAGGTGCCTAAAGGCGCCTGGCGTTCGTACTTAAAT
ATGGAACTAACCATTCCAATTTTCGCCACGAATTCCGACTGCGAGCTTATTGTTAAGGCAATGCAAGGTCTCCTA
AAAGATGGAAACCCGATTCCCTCAGCAATCGCAGCAAACTCCGGCATCTACTAA (SEQ ID NO: 563)

Protein:
MAIAARNAYLRKKIARLKKDNLQLERDEQNLEKIIANLRDEIARLENEVASHEQYPYDVPDYAGAPGSAGSAAGS
GASNFTQFVLVDNGGTGDVTVAPSNFANGIAEWISSNSRSQAYKVTCSVRQSSAQNRKYTIKVEVPKGAWRSYLN
MELTIPIFATNSDCELIVKAMQGLLKDGNPIPSAIAANSGIY* (SEQ ID NO: 564)

LCK::EWSR1::SYNZIP2::MCP
DNA:
ATGGGCTGCGTGTGCAGCAGCAACCCCGAGGGTACCGAGCTCATGGCGTCCACGGATTACAGTACCTATAGCCAA
GCTGCAGCGCAGCAGGGCTACAGTGCTTACACCGCCCAGCCCACTCAAGGATATGCACAGACCACCCAGGCATAT
GGGCAACAAAGCTATGGAACCTATGGACAGCCCACTGATGTCAGCTATACCCAGGCTCAGACCACTGCAACCTAT
GGGCAGACCGCCTATGCAACTTCTTATGGACAGCCTCCCACTGGTTATACTACTCCAACTGCCCCCCAGGCATAC
AGCCAGCCTGTCCAGGGGTATGGCACTGGTGCTTATGATACCACCACTGCTACAGTCACCACCACCCAGGCCTCC
TATGCAGCTCAGTCTGCATATGGCACTCAGCCTGCTTATCCAGCCTATGGGCAGCAGCCAGCAGCCACTGCACCT
ACAAGACCGCAGGATGGAAACAAGCCCACTGAGACTAGTCAACCTCAATCTAGCACAGGGGGTTACAACCAGCCC
AGCCTAGGATATGGACAGAGTAACTACAGTTATCCCCAGGTACCTGGGAGCTACCCCATGCAGCCAGTCACTGCA
CCTCCATCCTACCCTCCTACCAGCTATTCCTCTACACAGCCGACTAGTTATGATCAGAGCAGTTACTCTCAGCAG
AACACCTATGGGCAACCGAGCAGCTATGGACAGCAGAGTAGCTATGGTCAACAAAGCAGCTATGGGCAGCAGCCT
CCCACTAGTTACCCACCCCAAACTGGATCCTACAGCCAAGCTCCAAGTCAATATAGCCAACAGAGCAGCAGCTAC
GGGCAGCAGAGTTCATTCCGACAGGACCACCCCAGTAGCATGGGTGTTTATGGGCAGGAGTCTGGAGGATTTTCC
GGACCAGGAGAGAACCGGAGCATGAGTGGCCCTGATAACCGGGGCAGGGGAAGAGGGGGATTTGATCGTGGAGGC
ATGAGCAGAGGTGGGCGGGGAGGAGGACGCGGTGGAATGGGCAGCGCTGGAGAGCGAGGTGGCTTCAATAAGCCT
GGTGGACCCATGGATGAAGGACCAGATCTTGATCTAGGCCCACCTGTAGATCCAGATGAAGACTCTGACAACAGT
GCAATTTATGTACAAGGATTAAATGACAGTGTGACTCTAGATGATCTGGCAGACTTCTTTAAGCAGTGTGGGGTT
GTTAAGATGAACAAGAGAACTGGGCAACCCATGATCCACATCTACCTGGACAAGGAAACAGGAAAGCCCAAAGGC
GATGCCACAGTGTCCTATGAAGACCCACCTACTGCCAAGGCTGCCGTGGAATGGTTTGATGGGAAAGATTTTCAA
GGGAGCAAACTTAAAGTCTCCCTTGCTCGGAAGAAGCCTCCAATGAACAGTATGCGGGGTGGTCTGCCACCCCGT
GAGGGCAGAGGCATGCCACCACCACTCCGTGGAGGTCCAGGAGGCCCAGGAGGTCCTGGGGGACCCATGGGTCGC
ATGGGAGGCCGTGGAGGAGATAGAGGAGGCTTCCCTCCAAGAGGACCCCGGGGTTCCCGAGGGAACCCCTCTGGA
GGAGGAAACGTCCAGCACCGAGCTGGAGACTGGCAGTGTCCCAATCCGGGTTGTGGAAACCAGAACTTCGCCTGG
AGAACAGAGTGCAACCAGTGTAAGGCCCCAAAGCCTGAAGGCTTCCTCCCGCCACCCTTTCCGCCCCCGGGTGGT
GATCGTGGCAGAGGTGGCCCTGGTGGCATGCGGGGAGGAAGAGGTGGCCTCATGGATCGTGGTGGTCCCGGTGGA

ATGTTCAGAGGTGGCCGTGGTGGAGACAGAGGTGGCTTCCGTGGTGGCCGGGGCATGGACCGAGGTGGCTTTGGT
GGAGGAAGACGAGGTGGCCCTGGGGGGCCCCCTGGACCTTTGATGGAACAGGCGATCGCAGCTAGAAACGCCTAC
CTGAGAAAGAAAATCGCCAGACTGAAGAAGGACAACCTGCAGCTGGAAAGAGACGAGCAGAACCTGGAAAAGATC
ATCGCCAACCTCAGAGATGAGATCGCCAGACTGGAAAACGAGGTGGCCAGCCACGAGCAGTATCCCTATGATGTG
CCGGATTATGCTGGAGCACCAGGAAGTGCTGGTTCTGCTGCTGGTAGTGGAGCTTCTAACTTTACTCAGTTCGTT
CTCGTCGACAATGGCGGAACTGGCGACGTGACTGTCGCCCCAAGCAACTTCGCTAACGGGATCGCTGAATGGATC
AGCTCTAACTCGCGTTCACAGGCTTACAAAGTAACCTGTAGCGTTCGTCAGAGCTCTGCGCAGAATCGCAAATAC
ACCATCAAAGTCGAGGTGCCTAAAGGCGCCTGGCGTTCGTACTTAAATATGGAACTAACCATTCCAATTTTCGCC
ACGAATTCCGACTGCGAGCTTATTGTTAAGGCAATGCAAGGTCTCCTAAAAGATGGAAACCCGATTCCCTCAGCA
ATCGCAGCAAACTCCGGCATCTACTAA (SEQ ID NO: 565)

Protein:
MGCVCSSNPEGTELMASTDYSTYSQAAAQQGYSAYTAQPTQGYAQTTQAYGQQSYGTYGQPTDVSYTQAQTTATY
GQTAYATSYGQPPTGYTTPTAPQAYSQPVQGYGTGAYDTTTATVTTTQASYAAQSAYGTQPAYPAYGQQPAATAP
TRPQDGNKPTETSQPQSSTGGYNQPSLGYGQSNYSYPQVPGSYPMQPVTAPPSYPPTSYSSTQPTSYDQSSYSQQ
NTYGQPSSYGQQSSYGQQSSYGQQPPTSYPPQTGSYSQAPSQYSQQSSSYGQQSSFRQDHPSSMGVYGQESGGFS
GPGENRSMSGPDNRGRGRGGFDRGGMSRGGRGGGRGGMGSAGERGGFNKPGGPMDEGPDLDLGPPVDPDEDSDNS
AIYVQGLNDSVTLDDLADFFKQCGVVKMNKRTGQPMIHIYLDKETGKPKGDATVSYEDPPTAKAAVEWFDGKDFQ
GSKLKVSLARKKPPMNSMRGGLPPREGRGMPPPLRGGPGGPGGPGGPMGRMGGRGGDRGGFPPRGPRGSRGNPSG
GGNVQHRAGDWQCPNPGCGNQNFAWRTECNQCKAPKPEGFLPPPFPPPGGDRGRGGPGGMRGGRGGLMDRGGPGG
MFRGGRGGDRGGFRGGRGMDRGGFGGGRRGGPGGPPGPLMEQAIAARNAYLRKKIARLKKDNLQLERDEQNLEKI
IANLRDEIARLENEVASHEQYPYDVPDYAGAPGSAGSAAGSGASNFTQFVLVDNGGTGDVTVAPSNFANGIAEWI
SSNSRSQAYKVTCSVRQSSAQNRKYTIKVEVPKGAWRSYLNMELTIPIFATNSDCELIVKAMQGLLKDGNPIPSA
IAANSGIY* (SEQ ID NO: 566)

LCK::EWSR1::SYNZIP4::4xλN22
DNA:
ATGGGCTGCGTGTGCAGCAGCAACCCCGAGGGTACCGAGCTCATGGCGTCCACGGATTACAGTACCTATAGCCAA
GCTGCAGCGCAGCAGGGCTACAGTGCTTACACCGCCCAGCCCACTCAAGGATATGCACAGACCACCCAGGCATAT
GGGCAACAAAGCTATGGAACCTATGGACAGCCCACTGATGTCAGCTATACCCAGGCTCAGACCACTGCAACCTAT
GGGCAGACCGCCTATGCAACTTCTTATGGACAGCCTCCCACTGGTTATACTACTCCAACTGCCCCCCAGGCATAC
AGCCAGCCTGTCCAGGGGTATGGCACTGGTGCTTATGATACCACCACTGCTACAGTCACCACCACCCAGGCCTCC
TATGCAGCTCAGTCTGCATATGGCACTCAGCCTGCTTATCCAGCCTATGGGCAGCAGCCAGCAGCCACTGCACCT
ACAAGACCGCAGGATGGAAACAAGCCCACTGAGACTAGTCAACCTCAATCTAGCACAGGGGGTTACAACCAGCCC
AGCCTAGGATATGGACAGAGTAACTACAGTTATCCCCAGGTACCTGGGAGCTACCCCATGCAGCCAGTCACTGCA
CCTCCATCCTACCCTCCTACCAGCTATTCCTCTACACAGCCGACTAGTTATGATCAGAGCAGTTACTCTCAGCAG
AACACCTATGGGCAACCGAGCAGCTATGGACAGCAGAGTAGCTATGGTCAACAAAGCAGCTATGGGCAGCAGCCT
CCCACTAGTTACCCACCCCAAACTGGATCCTACAGCCAAGCTCCAAGTCAATATAGCCAACAGAGCAGCAGCTAC
GGGCAGCAGAGTTCATTCCGACAGGACCACCCCAGTAGCATGGGTGTTTATGGGCAGGAGTCTGGAGGATTTTCC
GGACCAGGAGAGAACCGGAGCATGAGTGGCCCTGATAACCGGGGCAGGGGAAGAGGGGGATTTGATCGTGGAGGC
ATGAGCAGAGGTGGGCGGGGAGGAGGACGCGGTGGAATGGGCAGCGCTGGAGAGCGAGGTGGCTTCAATAAGCCT
GGTGGACCCATGGATGAAGGACCAGATCTTGATCTAGGCCCACCTGTAGATCCAGATGAAGACTCTGACAACAGT
GCAATTTATGTACAAGGATTAAATGACAGTGTGACTCTAGATGATCTGGCAGACTTCTTTAAGCAGTGTGGGGTT
GTTAAGATGAACAAGAGAACTGGGCAACCCATGATCCACATCTACCTGGACAAGGAAACAGGAAAGCCCAAAGGC
GATGCCACAGTGTCCTATGAAGACCCACCTACTGCCAAGGCTGCCGTGGAATGGTTTGATGGGAAAGATTTTCAA
GGGAGCAAACTTAAAGTCTCCCTTGCTCGGAAGAAGCCTCCAATGAACAGTATGCGGGGTGGTCTGCCACCCCGT
GAGGGCAGAGGCATGCCACCACCACTCCGTGGAGGTCCAGGAGGCCCAGGAGGTCCTGGGGGACCCATGGGTCGC
ATGGGAGGCCGTGGAGGAGATAGAGGAGGCTTCCCTCCAAGAGGACCCCGGGGTTCCCGAGGGAACCCCTCTGGA
GGAGGAAACGTCCAGCACCGAGCTGGAGACTGGCAGTGTCCCAATCCGGGTTGTGGAAACCAGAACTTCGCCTGG
AGAACAGAGTGCAACCAGTGTAAGGCCCCAAAGCCTGAAGGCTTCCTCCCGCCACCCTTTCCGCCCCCGGGTGGT
GATCGTGGCAGAGGTGGCCCTGGTGGCATGCGGGGAGGAAGAGGTGGCCTCATGGATCGTGGTGGTCCCGGTGGA
ATGTTCAGAGGTGGCCGTGGTGGAGACAGAGGTGGCTTCCGTGGTGGCCGGGGCATGGACCGAGGTGGCTTTGGT
GGAGGAAGACGAGGTGGCCCTGGGGGGCCCCCTGGACCTTTGATGGAACAGGCGATCGCACAAAAGGTGGCTGAA
CTGAAAAATAGAGTGGCCGTGAAGCTGAACCGGAACGAGCAGCTGAAGAACAAGGTGGAAGAGCTGAAGAACAGA
AACGCCTACCTGAAGAATGAGCTGGCCACCCTGGAAAACGAGGTGGCCAGACTGGAAAACGACGTGGCCGAGGGA
GCACCAGGAAGTGCTGGTTCTGCTGCTGGTAGTGGAGAGCAGAAGCTGATCTCAGAGGAGGACCTGCTAGCCACC
ATGGACGCACAAACACGACGACGTGAGCGTCGCGCTGAGAAACAAGCTCAATGGAAAGCTGCAAACCCACCGCTC

GACGGAGCCGGAGCTGGCGCTGGAGCTGGAGCCGGAGCTGGCGGTCTAGCCACCATGGACGCACAAACACGACGA
CGTGAGCGTCGCGCTGAGAAACAAGCTCAATGGAAAGCTGCAAACCCACCGCTCGACGGAGCCGGAGCTGGCGCT
GGAGCTGGAGCCGGAGCTGGCGGTCTAGCCACCATGGACGCACAAACACGACGACGTGAGCGTCGCGCTGAGAAA
CAAGCTCAATGGAAAGCTGCAAACCCACCGCTCGACGGAGCCGGAGCTGGCGCTGGAGCTGGAGCCGGAGCTGGC
GGTCTAGCCACCATGGACGCACAAACACGACGACGTGAGCGTCGCGCTGAGAAACAAGCTCAATGGAAAGCTGCA
AACCCACCGCTCGAGTCTAGAGGGCCCGTTTAA (SEQ ID NO: 567)

Protein:
MGCVCSSNPEGTELMASTDYSTYSQAAAQQGYSAYTAQPTQGYAQTTQAYGQQSYGTYGQPTDVSYTQAQTTATY
GQTAYATSYGQPPTGYTTPTAPQAYSQPVQGYGTGAYDTTTATVTTTQASYAAQSAYGTQPAYPAYGQQPAATAP
TRPQDGNKPTETSQPQSSTGGYNQPSLGYGQSNYSYPQVPGSYPMQPVTAPPSYPPTSYSSTQPTSYDQSSYSQQ
NTYGQPSSYGQQSSYGQQSSYGQQPPTSYPPQTGSYSQAPSQYSQQSSSYGQQSSFRQDHPSSMGVYGQESGGFS
GPGENRSMSGPDNRGRGRGGFDRGGMSRGGRGGGRGGMGSAGERGGFNKPGGPMDEGPDLDLGPPVDPDEDSDNS
AIYVQGLNDSVTLDDLADFFKQCGVVKMNKRTGQPMIHIYLDKETGKPKGDATVSYEDPPTAKAAVEWFDGKDFQ
GSKLKVSLARKKPPMNSMRGGLPPREGRGMPPPLRGGPGGPGGPGGPMGRMGGRGGDRGGFPPRGPRGSRGNPSG
GGNVQHRAGDWQCPNPGCGNQNFAWRTECNQCKAPKPEGFLPPPFPPPGGDRGRGGPGGMRGGRGGLMDRGGPGG
MFRGGRGGDRGGFRGGRGMDRGGFGGGRRGGPGGPPGPLMEQAIAQKVAELKNRVAVKLNRNEQLKNKVEELKNR
NAYLKNELATLENEVARLENDVAEGAPGSAGSAAGSGEQKLISEEDLLATMDAQTRRRERRAEKQAQWKAANPPL
DGAGAGAGAGAGAGGLATMDAQTRRRERRAEKQAQWKAANPPLDGAGAGAGAGAGAGGLATMDAQTRRRERRAEK
QAQWKAANPPLDGAGAGAGAGAGAGGLATMDAQTRRRERRAEKQAQWKAANPPLESRGPV* (SEQ ID NO:
568)

LCK::SYNZIP2::MCP
DNA:
ATGGGCTGCGTGTGCAGCAGCAACCCCGAGGGTACCGAGCTCGCGATCGCAGCTAGAAACGCCTACCTGAGAAAG
AAAATCGCCAGACTGAAGAAGGACAACCTGCAGCTGGAAAGAGACGAGCAGAACCTGGAAAAGATCATCGCCAAC
CTCAGAGATGAGATCGCCAGACTGGAAAACGAGGTGGCCAGCCACGAGCAGTATCCCTATGATGTGCCGGATTAT
GCTGGAGCACCAGGAAGTGCTGGTTCTGCTGCTGGTAGTGGAGCTTCTAACTTTACTCAGTTCGTTCTCGTCGAC
AATGGCGGAACTGGCGACGTGACTGTCGCCCCAAGCAACTTCGCTAACGGGATCGCTGAATGGATCAGCTCTAAC
TCGCGTTCACAGGCTTACAAAGTAACCTGTAGCGTTCGTCAGAGCTCTGCGCAGAATCGCAAATACACCATCAAA
GTCGAGGTGCCTAAAGGCGCCTGGCGTTCGTACTTAAATATGGAACTAACCATTCCAATTTTCGCCACGAATTCC
GACTGCGAGCTTATTGTTAAGGCAATGCAAGGTCTCCTAAAAGATGGAAACCCGATTCCCTCAGCAATCGCAGCA
AACTCCGGCATCTACTAA (SEQ ID NO: 569)

Protein:
MGCVCSSNPEGTELAIAARNAYLRKKIARLKKDNLQLERDEQNLEKIIANLRDEIARLENEVASHEQYPYDVPDY
AGAPGSAGSAAGSGASNFTQFVLVDNGGTGDVTVAPSNFANGIAEWISSNSRSQAYKVTCSVRQSSAQNRKYTIK
VEVPKGAWRSYLNMELTIPIFATNSDCELIVKAMQGLLKDGNPIPSAIAANSGIY* (SEQ ID NO: 570)

EWSR1::SYNZIP2::MCP
DNA:
ATGATGGCGTCCACGGATTACAGTACCTATAGCCAAGCTGCAGCGCAGCAGGGCTACAGTGCTTACACCGCCCAG
CCCACTCAAGGATATGCACAGACCACCCAGGCATATGGGCAACAAAGCTATGGAACCTATGGACAGCCCACTGAT
GTCAGCTATACCCAGGCTCAGACCACTGCAACCTATGGGCAGACCGCCTATGCAACTTCTTATGGACAGCCTCCC
ACTGGTTATACTACTCCAACTGCCCCCCAGGCATACAGCCAGCCTGTCCAGGGGTATGGCACTGGTGCTTATGAT
ACCACCACTGCTACAGTCACCACCACCCAGGCCTCCTATGCAGCTCAGTCTGCATATGGCACTCAGCCTGCTTAT
CCAGCCTATGGGCAGCAGCCAGCAGCCACTGCACCTACAAGACCGCAGGATGGAAACAAGCCCACTGAGACTAGT
CAACCTCAATCTAGCACAGGGGGTTACAACCAGCCCAGCCTAGGATATGGACAGAGTAACTACAGTTATCCCCAG
GTACCTGGGAGCTACCCCATGCAGCCAGTCACTGCACCTCCATCCTACCCTCCTACCAGCTATTCCTCTACACAG
CCGACTAGTTATGATCAGAGCAGTTACTCTCAGCAGAACACCTATGGGCAACCGAGCAGCTATGGACAGCAGAGT
AGCTATGGTCAACAAAGCAGCTATGGGCAGCAGCCTCCCACTAGTTACCCACCCCAAACTGGATCCTACAGCCAA
GCTCCAAGTCAATATAGCCAACAGAGCAGCAGCTACGGGCAGCAGAGTTCATTCCGACAGGACCACCCCAGTAGC
ATGGGTGTTTATGGGCAGGAGTCTGGAGGATTTTCCGGACCAGGAGAGAACCGGAGCATGAGTGGCCCTGATAAC
CGGGGCAGGGGAAGAGGGGGATTTGATCGTGGAGGCATGAGCAGAGGTGGGCGGGGAGGAGGACGCGGTGGAATG
GGCAGCGCTGGAGAGCGAGGTGGCTTCAATAAGCCTGGTGGACCCATGGATGAAGGACCAGATCTTGATCTAGGC
CCACCTGTAGATCCAGATGAAGACTCTGACAACAGTGCAATTTATGTACAAGGATTAAATGACAGTGTGACTCTA

GATGATCTGGCAGACTTCTTTAAGCAGTGTGGGGTTGTTAAGATGAACAAGAGAACTGGGCAACCCATGATCCAC
ATCTACCTGGACAAGGAAACAGGAAAGCCCAAAGGCGATGCCACAGTGTCCTATGAAGACCCACCTACTGCCAAG
GCTGCCGTGGAATGGTTTGATGGGAAAGATTTTCAAGGGAGCAAACTTAAAGTCTCCCTTGCTCGGAAGAAGCCT
CCAATGAACAGTATGCGGGGTGGTCTGCCACCCCGTGAGGGCAGAGGCATGCCACCACCACTCCGTGGAGGTCCA
GGAGGCCCAGGAGGTCCTGGGGGACCCATGGGTCGCATGGGAGGCCGTGGAGGAGATAGAGGAGGCTTCCCTCCA
AGAGGACCCCGGGGTTCCCGAGGGAACCCCTCTGGAGGAGGAAACGTCCAGCACCGAGCTGGAGACTGGCAGTGT
CCCAATCCGGGTTGTGGAAACCAGAACTTCGCCTGGAGAACAGAGTGCAACCAGTGTAAGGCCCCAAAGCCTGAA
GGCTTCCTCCCGCCACCCTTTCCGCCCCCGGGTGGTGATCGTGGCAGAGGTGGCCCTGGTGGCATGCGGGGAGGA
AGAGGTGGCCTCATGGATCGTGGTGGTCCCGGTGGAATGTTCAGAGGTGGCCGTGGTGGAGACAGAGGTGGCTTC
CGTGGTGGCCGGGGCATGGACCGAGGTGGCTTTGGTGGAGGAAGACGAGGTGGCCCTGGGGGGCCCCCTGGACCT
TTGATGGAACAGGCGATCGCAGCTAGAAACGCCTACCTGAGAAAGAAAATCGCCAGACTGAAGAAGGACAACCTG
CAGCTGGAAAGAGACGAGCAGAACCTGGAAAAGATCATCGCCAACCTCAGAGATGAGATCGCCAGACTGGAAAAC
GAGGTGGCCAGCCACGAGCAGTATCCCTATGATGTGCCGGATTATGCTGGAGCACCAGGAAGTGCTGGTTCTGCT
GCTGGTAGTGGAGCTTCTAACTTTACTCAGTTCGTTCTCGTCGACAATGGCGGAACTGGCGACGTGACTGTCGCC
CCAAGCAACTTCGCTAACGGGATCGCTGAATGGATCAGCTCTAACTCGCGTTCACAGGCTTACAAAGTAACCTGT
AGCGTTCGTCAGAGCTCTGCGCAGAATCGCAAATACACCATCAAAGTCGAGGTGCCTAAAGGCGCCTGGCGTTCG
TACTTAAATATGGAACTAACCATTCCAATTTTCGCCACGAATTCCGACTGCGAGCTTATTGTTAAGGCAATGCAA
GGTCTCCTAAAAGATGGAAACCCGATTCCCTCAGCAATCGCAGCAAACTCCGGCATCTACTAA (SEQ ID NO:
571)

Protein:
MMASTDYSTYSQAAAQQGYSAYTAQPTQGYAQTTQAYGQQSYGTYGQPTDVSYTQAQTTATYGQTAYATSYGQPP
TGYTTPTAPQAYSQPVQGYGTGAYDTTTATVTTTQASYAAQSAYGTQPAYPAYGQQPAATAPTRPQDGNKPTETS
QPQSSTGGYNQPSLGYGQSNYSYPQVPGSYPMQPVTAPPSYPPTSYSSTQPTSYDQSSYSQQNTYGQPSSYGQQS
SYGQQSSYGQQPPTSYPPQTGSYSQAPSQYSQQSSSYGQQSSFRQDHPSSMGVYGQESGGFSGPGENRSMSGPDN
RGRGRGGFDRGGMSRGGRGGGRGGMGSAGERGGFNKPGGPMDEGPDLDLGPPVDPDEDSDNSAIYVQGLNDSVTL
DDLADFFKQCGVVKMNKRTGQPMIHIYLDKETGKPKGDATVSYEDPPTAKAAVEWFDGKDFQGSKLKVSLARKKP
PMNSMRGGLPPREGRGMPPPLRGGPGGPGGPGGPMGRMGGRGGDRGGFPPRGPRGSRGNPSGGGNVQHRAGDWQC
PNPGCGNQNFAWRTECNQCKAPKPEGFLPPPFPPPGGDRGRGGPGGMRGGRGGLMDRGGPGGMFRGGRGGDRGGF
RGGRGMDRGGFGGGRRGGPGGPPGPLMEQAIAARNAYLRKKIARLKKDNLQLERDEQNLEKIIANLRDEIARLEN
EVASHEQYPYDVPDYAGAPGSAGSAAGSGASNFTQFVLVDNGGTGDVTVAPSNFANGIAEWISSNSRSQAYKVTC
SVRQSSAQNRKYTIKVEVPKGAWRSYLNMELTIPIFATNSDCELIVKAMQGLLKDGNPIPSAIAANSGIY*
(SEQ ID NO: 572)

LCK::FUS::SYNZIP1::PylRS(AF)
DNA:
ATGGGCTGCGTGTGCAGCAGCAACCCCGAGGGTACCGAGCTCATGGCCTCAAACGATTATACCCAACAAGCAACC
CAAAGCTATGGGGCCTACCCCACCCAGCCCGGGCAGGGCTATTCCCAGCAGAGCAGTCAGCCCTACGGACAGCAG
AGTTACAGTGGTTATAGCCAGTCCACGGACACTTCAGGATATGGCCAGAGCAGCTATTCTTCTTATGGCCAGAGC
CAGAACACAGGCTATGGAACTCAGTCAACTCCCCAGGGATATGGCTCGACTGGCGGCTATGGCAGTAGCCAGAGC
TCCCAATCGTCTTACGGGCAGCAGTCCTCCTACCCTGGCTATGGCCAGCAGCCAGCTCCCAGCAGCACCTCGGGA
AGTTACGGTAGCAGTTCTCAGAGCAGCAGCTATGGGCAGCCCCAGAGTGGGAGCTACAGCCAGCAGCCTAGCTAT
GGTGGACAGCAGCAAAGCTATGGACAGCAGCAAAGCTATAATCCCCCTCAGGGCTATGGACAGCAGAACCAGTAC
AACAGCAGCAGTGGTGGTGGAGGTGGAGGTGGAGGTGGAGGTAACTATGGCCAAGATCAATCCTCCATGAGTAGT
GGTGGTGGCAGTGGTGGCGGTTATGGCAATCAAGACCAGAGTGGTGGAGGTGGCAGCGGTGGCTATGGACAGCAG
GACCGTGGAGGCCGCGGCAGGGGTGGCAGTGGTGGCGGCGGCGGCGGTGGTGGTTACAACCGCAGCAGT
GGTGGCTATGAACCCAGAGGTCGTGGAGGTGGCCGTGGAGGCAGAGGTGGCATGGGCGGAAGTGACCGTGGTGGC
TTCAATAAATTTGGTGGCCCTCGGGACCAAGGATCACGTCATGACTCCGAACAGGATAATTCAGACAACAACACC
ATCTTTGTGCAAGGCCTGGGTGAGAATGTTACAATTGAGTCTGTGGCTGATTACTTCAAGCAGATTGGTATTATT
AAGACAAACAAGAAAACGGGACAGCCCATGATTAATTTGTACACAGACAGGGAAACTGGCAAGCTGAAGGGAGAG
GCAACGGTCTCTTTTGATGACCCACCTTCAGCTAAAGCAGCTATTGACTGGTTTGATGGTAAAGAATTCTCCGGA
AATCCTATCAAGGTCTCATTTGCTACTCGCCGGGCAGACTTTAATCGGGGTGGTGGCAATGGTCGTGGAGGCCGA
GGGCGAGGAGGACCCATGGGCCGTGGAGGCTATGGAGGTGGTGGCAGTGGTGGTGGTGGCCGAGGAGGATTTCCC
AGTGGAGGTGGTGGCGGTGGAGGACAGCAGCGAGCTGGTGACTGGAAGTGTCCTAATCCCACCTGTGAGAATATG
AACTTCTCTTGGAGGAATGAATGCAACCAGTGTAAGGCCCCTAAACCAGATGGCCCAGGAGGGGGACCAGGTGGC
TCTCACATGGGGGGTAACTACGGGGATGATCGTCGTGGTGGCAGAGGAGGCGCGATCGCAAATCTGGTGGCCCAG
CTGGAAAACGAGGTGGCCAGCCTGGAAAACGAGAACGAAACCCTGAAGAAAAAGAACCTGCACAAGAAGGACCTG

ATCGCCTACCTGGAAAAGGAAATCGCCAACCTGAGAAAGAAGATCGAGGAAGGCAAGCCTATTCCCAACCCCCTG
CTGGGCCTGGATAGCACCGGAGCACCAGGAAGTGCTGGTTCTGCTGCTGGTAGTGGAGCGTGCCCGGTGCCGCTG
CAGCTGCCGCCGCTGGAACGCCTGACCCTGGATGACAAAAAACCGCTGAATACCCTGATCTCTGCTACTGGTCTG
TGGATGAGTCGTACCGGAACCATTCATAAAATCAAACACCACGAGGTTAGCCGTTCGAAAATCTATATTGAGATG
GCGTGTGGCGATCATCTGGTTGTGAACAATAGCCGCTCTTCTCGTACAGCACGTGCACTGCGTCACCACAAATAT
CGTAAAACCTGTAAACGTTGCCGTGTGTCCGATGAGGATCTGAACAAATTCCTGACAAAAGCCAATGAGGACCAA
ACAAGCGTGAAAGTGAAAGTCGTTAGCGCTCCTACCCGTACTAAAAAAGCAATGCCGAAATCCGTTGCTCGTGCC
CCTAAACCACTGGAAAACACTGAAGCAGCACAGGCACAGCCGTCTGGAAGCAAATTCTCTCCGGCCATTCCTGTT
TCTACCCAGGAGTCCGTTTCTGTTCCAGCAAGTGTGAGCACCAGCATTAGCAGTATTAGCACCGGTGCCACCGCT
AGCGCCCTGGTTAAAGGCAATACCAATCCGATTACAAGCATGTCTGCCCCGGTTCAAGCATCAGCTCCAGCACTG
ACAAAATCCCAAACCGATCGTCTGGAGGTTCTGCTGAATCCGAAAGACGAAATCAGCCTGAATTCCGGCAAACCG
TTTCGTGAACTGGAGAGCGAACTGCTGTCACGTCGTAAAAAAGACCTGCAACAAATCTATGCCGAAGAACGTGAG
AACTATCTGGGGAAACTGGAACGTGAAATCACCCGCTTTTTCGTGGATCGTGGCTTTCTGGAGATCAAATCCCCG
ATTCTGATTCCTCTGGAGTATATCGAGCGTATGGGCATCGACAATGATACCGAACTGAGCAAACAAATTTTCCGT
GTGGATAAAAACTTCTGTCTGCGCCCTATGCTAGCACCAAATCTGGCTAACTATCTGCGCAAACTGGACCGTGCC
CTGCCTGATCCTATCAAAATCTTCGAGATCGGCCCGTGTTATCGTAAAGAGTCCGACGGTAAAGAACATCTGGAG
GAGTTTACCATGCTGAACTTTTGCCAAATGGGTTCAGGTTGTACTCGTGAGAACCTGGAAAGCATCATCACCGAT
TTTCTGAACCACCTGGGCATTGACTTCAAAATTGTGGGCGACAGCTGTATGGTGTTTGGCGACACCCTGGATGTC
ATGCACGGCGACCTGGAACTGTCTAGTGCCGTTGTGGGCCCAATCCCGCTGGATCGTGAGTGGGGTATCGACAAA
CCTTGGATCGGTGCGGGTTTTGGTCTGGAGCGTCTGCTGAAAGTAAAACACGACTTCAAGAACATCAAACGTGCT
GCACGTTCCGAGTCCTATTACAATGGTATTTCTACTAACCTGTAA (SEQ ID NO: 573)

Protein:
MGCVCSSNPEGTELMASNDYTQQATQSYGAYPTQPGQGYSQQSSQPYGQQSYSGYSQSTDTSGYGQSSYSSYGQS
QNTGYGTQSTPQGYGSTGGYGSSQSSQSSYGQQSSYPGYGQQPAPSSTSGSYGSSSQSSSYGQPQSGSYSQQPSY
GGQQQSYGQQQSYNPPQGYGQQNQYNSSSGGGGGGGGGNYGQDQSSMSSGGGSGGGYGNQDQSGGGGSGGYGQQ
DRGGRGRGGSGGGGGGGGGGYNRSSGGYEPRGRGGGRGGRGGMGGSDRGGFNKFGGPRDQGSRHDSEQDNSDNNT
IFVQGLGENVTIESVADYFKQIGIIKTNKKTGQPMINLYTDRETGKLKGEATVSFDDPPSAKAAIDWFDGKEFSG
NPIKVSFATRRADFNRGGGNGRGGRGRGGPMGRGGYGGGGSGGGGRGGFPSGGGGGGGQQRAGDWKCPNPTCENM
NFSWRNECNQCKAPKPDGPGGGPGGSHMGGNYGDDRRGGRGGAIANLVAQLENEVASLENENETLKKKNLHKKDL
IAYLEKEIANLRKKIEEGKPIPNPLLGLDSTGAPGSAGSAAGSGACPVPLQLPPLERLTLDDKKPLNTLISATGL
WMSRTGTIHKIKHHEVSRSKIYIEMACGDHLVVNNSRSSRTARALRHHKYRKTCKRCRVSDEDLNKFLTKANEDQ
TSVKVKVVSAPTRTKKAMPKSVARAPKPLENTEAAQAQPSGSKFSPAIPVSTQESVSVPASVSTSISSISTGATA
SALVKGNTNPITSMSAPVQASAPALTKSQTDRLEVLLNPKDEISLNSGKPFRELESELLSRRKKDLQQIYAEERE
NYLGKLEREITRFFVDRGFLEIKSPILIPLEYIERMGIDNDTELSKQIFRVDKNFCLRPMLAPNLANYLRKLDRA
LPDPIKIFEIGPCYRKESDGKEHLEEFTMLNFCQMGSGCTRENLESIITDFLNHLGIDFKIVGDSCMVFGDTLDV
MHGDLELSSAVVGPIPLDREWGIDKPWIGAGFGLERLLKVKHDFKNIKRAARSESYYNGISTNL* (SEQ ID
NO: 574)

LCK::FUS::SYNZIP3::PylRS(AF)
DNA:
ATGGGCTGCGTGTGCAGCAGCAACCCCGAGGGTACCGAGCTCATGGCCTCAAACGATTATACCCAACAAGCAACC
CAAAGCTATGGGGCCTACCCCACCCAGCCCGGGCAGGGCTATTCCCAGCAGAGCAGTCAGCCCTACGGACAGCAG
AGTTACAGTGGTTATAGCCAGTCCACGGACACTTCAGGATATGGCCAGAGCAGCTATTCTTCTTATGGCCAGAGC
CAGAACACAGGCTATGGAACTCAGTCAACTCCCCAGGGATATGGCTCGACTGGCGGCTATGGCAGTAGCCAGAGC
TCCCAATCGTCTTACGGGCAGCAGTCCTCCTACCCTGGCTATGGCCAGCAGCCAGCTCCCAGCAGCACCTCGGGA
AGTTACGGTAGCAGTTCTCAGAGCAGCAGCTATGGGCAGCCCCAGAGTGGGAGCTACAGCCAGCAGCCTAGCTAT
GGTGGACAGCAGCAAAGCTATGGACAGCAGCAAAGCTATAATCCCCCTCAGGGCTATGGACAGCAGAACCAGTAC
AACAGCAGCAGTGGTGGTGGAGGTGGAGGTGGAGGTGGAGGTAACTATGGCCAAGATCAATCCTCCATGAGTAGT
GGTGGTGGCAGTGGTGGCGGTTATGGCAATCAAGACCAGAGTGGTGGAGGTGGCAGCGGTGGCTATGGACAGCAG
GACCGTGGAGGCCGCGGCAGGGGTGGCAGTGGTGGCGGCGGCGGCGGCGGTGGTGGTTACAACCGCAGCAGT
GGTGGCTATGAACCCAGAGGTCGTGGAGGTGGCCGTGGAGGCAGAGGTGGCATGGGCGGAAGTGACCGTGGTGGC
TTCAATAAATTTGGTGGCCCTCGGGACCAAGGATCACGTCATGACTCCGAACAGGATAATTCAGACAACAACACC
ATCTTTGTGCAAGGCCTGGGTGAGAATGTTACAATTGAGTCTGTGGCTGATTACTTCAAGCAGATTGGTATTATT
AAGACAAACAAGAAAACGGGACAGCCCATGATTAATTTGTACACAGACAGGGAAACTGGCAAGCTGAAGGGAGAG
GCAACGGTCTCTTTTGATGACCCACCTTCAGCTAAAGCAGCTATTGACTGGTTTGATGGTAAAGAATTCTCCGGA
AATCCTATCAAGGTCTCATTTGCTACTCGCCGGGCAGACTTTAATCGGGGTGGTGGCAATGGTCGTGGAGGCCGA

GGGCGAGGAGGACCCATGGGCCGTGGAGGCTATGGAGGTGGTGGCAGTGGTGGTGGTGGCCGAGGAGGATTTCCC
AGTGGAGGTGGTGGCGGTGGAGGACAGCAGCGAGCTGGTGACTGGAAGTGTCCTAATCCCACCTGTGAGAATATG
AACTTCTCTTGGAGGAATGAATGCAACCAGTGTAAGGCCCCTAAACCAGATGGCCCAGGAGGGGGACCAGGTGGC
TCTCACATGGGGGGTAACTACGGGGATGATCGTCGTGGTGGCAGAGGAGGCGCGATCGCAAATGAGGTGACCACC
CTGGAAAACGACGCCGCCTTCATCGAGAACGAGAACGCCTACCTGGAAAAAGAGATCGCCAGACTGAGAAAGGAA
AAGGCCGCTCTGCGGAACAGACTGGCCCACAAGAAGGGCAAGCCTATTCCCAACCCCCTGCTGGGCCTGGATAGC
ACCGGAGCACCAGGAAGTGCTGGTTCTGCTGCTGGTAGTGGAGCGTGCCCGGTGCCGCTGCAGCTGCCGCCGCTG
GAACGCCTGACCCTGGATGACAAAAAACCGCTGAATACCCTGATCTCTGCTACTGGTCTGTGGATGAGTCGTACC
GGAACCATTCATAAAATCAAACACCACGAGGTTAGCCGTTCGAAAATCTATATTGAGATGGCGTGTGGCGATCAT
CTGGTTGTGAACAATAGCCGCTCTTCTCGTACAGCACGTGCACTGCGTCACCACAAATATCGTAAAACCTGTAAA
CGTTGCCGTGTGTCCGATGAGGATCTGAACAAATTCCTGACAAAAGCCAATGAGGACCAAACAAGCGTGAAAGTG
AAAGTCGTTAGCGCTCCTACCCGTACTAAAAAAGCAATGCCGAAATCCGTTGCTCGTGCCCCTAAACCACTGGAA
AACACTGAAGCAGCACAGGCACAGCCGTCTGGAAGCAAATTCTCTCCGGCCATTCCTGTTTCTACCCAGGAGTCC
GTTTCTGTTCCAGCAAGTGTGAGCACCAGCATTAGCAGTATTAGCACCGGTGCCACCGCTAGCGCCCTGGTTAAA
GGCAATACCAATCCGATTACAAGCATGTCTGCCCCGGTTCAAGCATCAGCTCCAGCACTGACAAAATCCCAAACC
GATCGTCTGGAGGTTCTGCTGAATCCGAAAGACGAAATCAGCCTGAATTCCGGCAAACCGTTTCGTGAACTGGAG
AGCGAACTGCTGTCACGTCGTAAAAAAGACCTGCAACAAATCTATGCCGAAGAACGTGAGAACTATCTGGGGAAA
CTGGAACGTGAAATCACCCGCTTTTTCGTGGATCGTGGCTTTCTGGAGATCAAATCCCCGATTCTGATTCCTCTG
GAGTATATCGAGCGTATGGGCATCGACAATGATACCGAACTGAGCAAACAAATTTTCCGTGTGGATAAAAACTTC
TGTCTGCGCCCTATGCTAGCACCAAATCTGGCTAACTATCTGCGCAAACTGGACCGTGCCCTGCCTGATCCTATC
AAAATCTTCGAGATCGGCCCGTGTTATCGTAAAGAGTCCGACGGTAAAGAACATCTGGAGGAGTTTACCATGCTG
AACTTTTGCCAAATGGGTTCAGGTTGTACTCGTGAGAACCTGGAAAGCATCATCACCGATTTTCTGAACCACCTG
GGCATTGACTTCAAAATTGTGGGCGACAGCTGTATGGTGTTTGGCGACACCCTGGATGTCATGCACGGCGACCTG
GAACTGTCTAGTGCCGTTGTGGGCCCAATCCCGCTGGATCGTGAGTGGGGTATCGACAAACCTTGGATCGGTGCG
GGTTTTGGTCTGGAGCGTCTGCTGAAAGTAAAACACGACTTCAAGAACATCAAACGTGCTGCACGTTCCGAGTCC
TATTACAATGGTATTTCTACTAACCTGTAA (SEQ ID NO: 575)

Protein:
MGCVCSSNPEGTELMASNDYTQQATQSYGAYPTQPGQGYSQQSSQPYGQQSYSGYSQSTDTSGYGQSSYSSYGQS
QNTGYGTQSTPQGYGSTGGYGSSQSSQSSYGQQSSYPGYGQQPAPSSTSGSYGSSSQSSSYGQPQSGSYSQQPSY
GGQQQSYGQQQSYNPPQGYGQQNQYNSSSGGGGGGGGGGNYGQDQSSMSSGGGSGGGYGNQDQSGGGGSGGYGQQ
DRGGRGRGGSGGGGGGGGGGYNRSSGGYEPRGRGGGRGGRGGMGGSDRGGFNKFGGPRDQGSRHDSEQDNSDNNT
IFVQGLGENVTIESVADYFKQIGIIKTNKKTGQPMINLYTDRETGKLKGEATVSFDDPPSAKAAIDWFDGKEFSG
NPIKVSFATRRADFNRGGGNGRGGRGRGGPMGRGGYGGGGSGGGGRGGFPSGGGGGGGQQRAGDWKCPNPTCENM
NFSWRNECNQCKAPKPDGPGGGPGGSHMGGNYGDDRRGGRGGAIANEVTTLENDAAFIENENAYLEKEIARLRKE
KAALRNRLAHKKGKPIPNPLLGLDSTGAPGSAGSAAGSGACPVPLQLPPLERLTLDDKKPLNTLISATGLWMSRT
GTIHKIKHHEVSRSKIYIEMACGDHLVVNNSRSSRTARALRHHKYRKTCKRCRVSDEDLNKFLTKANEDQTSVKV
KVVSAPTRTKKAMPKSVARAPKPLENTEAAQAQPSGSKFSPAIPVSTQESVSVPASVSTSISSISTGATASALVK
GNTNPITSMSAPVQASAPALTKSQTDRLEVLLNPKDEISLNSGKPFRELESELLSRRKKDLQQIYAEERENYLGK
LEREITRFFVDRGFLEIKSPILIPLEYIERMGIDNDTELSKQIFRVDKNFCLRPMLAPNLANYLRKLDRALPDPI
KIFEIGPCYRKESDGKEHLEEFTMLNFCQMGSGCTRENLESIITDFLNHLGIDFKIVGDSCMVFGDTLDVMHGDL
ELSSAVVGPIPLDREWGIDKPWIGAGFGLERLLKVKHDFKNIKRAARSESYYNGISTNL* (SEQ ID NO:
576)

TOM20::EWSR1::SYNZIP4::2xPCP
DNA:
ATGGTGGGTCGGAACAGCGCCATCGCCGCCGGTGTATGCGGGGCCCTTTTCATTGGGTACTGCATCTACTTCGAC
CGCAAAAGACGAAGTGACCCCAACTTCAAGAACAGGCTTCGAGAACGAAGAAAGAAACAGAAGCTTGCCAAGGAG
AGAGCTGGGCTTTCCAAGTTACCTGACCTTAAAGATGCTGAAGCTGTTCAGAAGTTCTTCATGGCGTCCACGGAT
TACAGTACCTATAGCCAAGCTGCAGCGCAGCAGGGCTACAGTGCTTACACCGCCCAGCCCACTCAAGGATATGCA
CAGACCACCCAGGCATATGGGCAACAAAGCTATGGAACCTATGGACAGCCCACTGATGTCAGCTATACCCAGGCT
CAGACCACTGCAACCTATGGGCAGACCGCCTATGCAACTTCTTATGGACAGCCTCCCACTGGTTATACTACTCCA
ACTGCCCCCCAGGCATACAGCCAGCCTGTCCAGGGGTATGGCACTGGTGCTTATGATACCACCACTGCTACAGTC
ACCACCACCCAGGCCTCCTATGCAGCTCAGTCTGCATATGGCACTCAGCCTGCTTATCCAGCCTATGGGCAGCAG
CCAGCAGCCACTGCACCTACAAGACCGCAGGATGGAAACAAGCCCACTGAGACTAGTCAACCTCAATCTAGCACA
GGGGGTTACAACCAGCCCAGCCTAGGATATGGACAGAGTAACTACAGTTATCCCCAGGTACCTGGGAGCTACCCC
ATGCAGCCAGTCACTGCACCTCCATCCTACCCTCCTACCAGCTATTCCTCTACACAGCCGACTAGTTATGATCAG

AGCAGTTACTCTCAGCAGAACACCTATGGGCAACCGAGCAGCTATGGACAGCAGAGTAGCTATGGTCAACAAAGC
AGCTATGGGCAGCAGCCTCCCACTAGTTACCCACCCCAAACTGGATCCTACAGCCAAGCTCCAAGTCAATATAGC
CAACAGAGCAGCAGCTACGGGCAGCAGAGTTCATTCCGACAGGACCACCCCAGTAGCATGGGTGTTTATGGGCAG
GAGTCTGGAGGATTTTCCGGACCAGGAGAGAACCGGAGCATGAGTGGCCCTGATAACCGGGGCAGGGGAAGAGGG
GGATTTGATCGTGGAGGCATGAGCAGAGGTGGGCGGGGAGGAGGACGCGGTGGAATGGGCAGCGCTGGAGAGCGA
GGTGGCTTCAATAAGCCTGGTGGACCCATGGATGAAGGACCAGATCTTGATCTAGGCCCACCTGTAGATCCAGAT
GAAGACTCTGACAACAGTGCAATTTATGTACAAGGATTAAATGACAGTGTGACTCTAGATGATCTGGCAGACTTC
TTTAAGCAGTGTGGGGTTGTTAAGATGAACAAGAGAACTGGGCAACCCATGATCCACATCTACCTGGACAAGGAA
ACAGGAAAGCCCAAAGGCGATGCCACAGTGTCCTATGAAGACCCACCTACTGCCAAGGCTGCCGTGGAATGGTTT
GATGGGAAAGATTTTCAAGGGAGCAAACTTAAAGTCTCCCTTGCTCGGAAGAAGCCTCCAATGAACAGTATGCGG
GGTGGTCTGCCACCCCGTGAGGGCAGAGGCATGCCACCACCACTCCGTGGAGGTCCAGGAGGCCCAGGAGGTCCT
GGGGGACCCATGGGTCGCATGGGAGGCCGTGGAGGAGATAGAGGAGGCTTCCCTCCAAGAGGACCCCGGGGTTCC
CGAGGGAACCCCTCTGGAGGAGGAAACGTCCAGCACCGAGCTGGAGACTGGCAGTGTCCCAATCCGGGTTGTGGA
AACCAGAACTTCGCCTGGAGAACAGAGTGCAACCAGTGTAAGGCCCCAAAGCCTGAAGGCTTCCTCCCGCCACCC
TTTCCGCCCCCGGGTGGTGATCGTGGCAGAGGTGGCCCTGGTGGCATGCGGGGAGGAAGAGGTGGCCTCATGGAT
CGTGGTGGTCCCGGTGGAATGTTCAGAGGTGGCCGTGGTGGAGACAGAGGTGGCTTCCGTGGTGGCCGGGGCATG
GACCGAGGTGGCTTTGGTGGAGGAAGACGAGGTGGCCCTGGGGGGCCCCCTGGACCTTTGATGGAACAGGCGATC
GCACAAAAGGTGGCTGAACTGAAAAATAGAGTGGCCGTGAAGCTGAACCGGAACGAGCAGCTGAAGAACAAGGTG
GAAGAGCTGAAGAACAGAAACGCCTACCTGAAGAATGAGCTGGCCACCCTGGAAAACGAGGTGGCCAGACTGGAA
AACGACGTGGCCGAGTTAATCGCAGAGCAGAAGCTGATCTCAGAGGAGGACCTGATCGAAGGCCGCCATATGCTA
GCCTCCAAAACCATCGTTCTTTCGGTCGGCGAGGCTACTCGCACTCTGACTGAGATCCAGTCCACCGCAGACCGT
CAGATCTTCGAAGAGAAGGTCGGGCCTCTGGTGGGTCGGCTGCGCCTCACGGCTTCGCTCCGTCAAAACGGAGCC
AAGACCGCGTATCGCGTCAACCTAAAACTGGATCAGGCGGACGTCGTTGATTCCGGACTTCCGAAAGTGCGCTAC
ACTCAGGTATGGTCGCACGACGTGACAATCGTTGCGAATAGCACCGAGGCCTCGCGCAAATCGTTGTACGATTTG
ACCAAGTCCCTCGTCGCGACCTCGCAGGTCGAAGATCTTGTCGTCAACCTTGTGCCGCTGGGCCGTGCGGATCCG
CTAGCCTCCAAAACCATCGTTCTTTCGGTCGGCGAGGCTACTCGCACTCTGACTGAGATCCAGTCCACCGCAGAC
CGTCAGATCTTCGAAGAGAAGGTCGGGCCTCTGGTGGGTCGGCTGCGCCTCACGGCTTCGCTCCGTCAAAACGGA
GCCAAGACCGCGTATCGCGTCAACCTAAAACTGGATCAGGCGGACGTCGTTGATTCCGGACTTCCGAAAGTGCGC
TACACTCAGGTATGGTCGCACGACGTGACAATCGTTGCGAATAGCACCGAGGCCTCGCGCAAATCGTTGTACGAT
TTGACCAAGTCCCTCGTCGCGACCTCGCAGGTCGAAGATCTTGTCGTCAACCTTGTGCCGCTGGGCCGTCCACCG
GTCGCCACCTAA (SEQ ID NO: 577)

Protein:
MVGRNSAIAAGVCGALFIGYCIYFDRKRRSDPNFKNRLRERRKKQKLAKERAGLSKLPDLKDAEAVQKFFMASTD
YSTYSQAAAQQGYSAYTAQPTQGYAQTTQAYGQQSYGTYGQPTDVSYTQAQTTATYGQTAYATSYGQPPTGYTTP
TAPQAYSQPVQGYGTGAYDTTTATVTTTQASYAAQSAYGTQPAYPAYGQQPAATAPTRPQDGNKPTETSQPQSST
GGYNQPSLGYGQSNYSYPQVPGSYPMQPVTAPPSYPPTSYSSTQPTSYDQSSYSQQNTYGQPSSYGQQSSYGQQS
SYGQQPPTSYPPQTGSYSQAPSQYSQQSSSYGQQSSFRQDHPSSMGVYGQESGGFSGPGENRSMSGPDNRGRGRG
GFDRGGMSRGGRGGGRGGMGSAGERGGFNKPGGPMDEGPDLDLGPPVDPDEDSDNSAIYVQGLNDSVTLDDLADF
FKQCGVVKMNKRTGQPMIHIYLDKETGKPKGDATVSYEDPPTAKAAVEWFDGKDFQGSKLKVSLARKKPPMNSMR
GGLPPREGRGMPPPLRGGPGGPGGPGGPMGRMGGRGGDRGGFPPRGPRGSRGNPSGGGNVQHRAGDWQCPNPGCG
NQNFAWRTECNQCKAPKPEGFLPPPFPPPGGDRGRGGPGGMRGGRGGLMDRGGPGGMFRGGRGGDRGGFRGGRGM
DRGGFGGGRRGGPGGPPGPLMEQAIAQKVAELKNRVAVKLNRNEQLKNKVEELKNRNAYLKNELATLENEVARLE
NDVAELIAEQKLISEEDLIEGRHMLASKTIVLSVGEATRTLTEIQSTADRQIFEEKVGPLVGRLRLTASLRQNGA
KTAYRVNLKLDQADVVDSGLPKVRYTQVWSHDVTIVANSTEASRKSLYDLTKSLVATSQVEDLVVNLVPLGRADP
LASKTIVLSVGEATRTLTEIQSTADRQIFEEKVGPLVGRLRLTASLRQNGAKTAYRVNLKLDQADVVDSGLPKVR
YTQVWSHDVTIVANSTEASRKSLYDLTKSLVATSQVEDLVVNLVPLGRPPVAT* (SEQ ID NO: 578)

TOM20::EWSR1::SYNZIP2::2xPCP
DNA:
ATGGTGGGTCGGAACAGCGCCATCGCCGCCGGTGTATGCGGGGCCCTTTTCATTGGGTACTGCATCTACTTCGAC
CGCAAAAGACGAAGTGACCCCAACTTCAAGAACAGGCTTCGAGAACGAAGAAAGAAACAGAAGCTTGCCAAGGAG
AGAGCTGGGCTTTCCAAGTTACCTGACCTTAAAGATGCTGAAGCTGTTCAGAAGTTCTTCATGGCGTCCACGGAT
TACAGTACCTATAGCCAAGCTGCAGCGCAGCAGGGCTACAGTGCTTACACCGCCCAGCCCACTCAAGGATATGCA
CAGACCACCCAGGCATATGGGCAACAAAGCTATGGAACCTATGGACAGCCCACTGATGTCAGCTATACCCAGGCT
CAGACCACTGCAACCTATGGGCAGACCGCCTATGCAACTTCTTATGGACAGCCTCCCACTGGTTATACTACTCCA
ACTGCCCCCCAGGCATACAGCCAGCCTGTCCAGGGGTATGGCACTGGTGCTTATGATACCACCACTGCTACAGTC

ACCACCACCCAGGCCTCCTATGCAGCTCAGTCTGCATATGGCACTCAGCCTGCTTATCCAGCCTATGGGCAGCAG
CCAGCAGCCACTGCACCTACAAGACCGCAGGATGGAAACAAGCCCACTGAGACTAGTCAACCTCAATCTAGCACA
GGGGGTTACAACCAGCCCAGCCTAGGATATGGACAGAGTAACTACAGTTATCCCCAGGTACCTGGGAGCTACCCC
ATGCAGCCAGTCACTGCACCTCCATCCTACCCTCCTACCAGCTATTCCTCTACACAGCCGACTAGTTATGATCAG
AGCAGTTACTCTCAGCAGAACACCTATGGGCAACCGAGCAGCTATGGACAGCAGAGTAGCTATGGTCAACAAAGC
AGCTATGGGCAGCAGCCTCCCACTAGTTACCCACCCCAAACTGGATCCTACAGCCAAGCTCCAAGTCAATATAGC
CAACAGAGCAGCAGCTACGGGCAGCAGAGTTCATTCCGACAGGACCACCCCAGTAGCATGGGTGTTTATGGGCAG
GAGTCTGGAGGATTTTCCGGACCAGGAGAGAACCGGAGCATGAGTGGCCCTGATAACCGGGGCAGGGGAAGAGGG
GGATTTGATCGTGGAGGCATGAGCAGAGGTGGGCGGGGAGGAGGACGCGGTGGAATGGGCAGCGCTGGAGAGCGA
GGTGGCTTCAATAAGCCTGGTGGACCCATGGATGAAGGACCAGATCTTGATCTAGGCCCACCTGTAGATCCAGAT
GAAGACTCTGACAACAGTGCAATTTATGTACAAGGATTAAATGACAGTGTGACTCTAGATGATCTGGCAGACTTC
TTTAAGCAGTGTGGGGTTGTTAAGATGAACAAGAGAACTGGGCAACCCATGATCCACATCTACCTGGACAAGGAA
ACAGGAAAGCCCAAAGGCGATGCCACAGTGTCCTATGAAGACCCACCTACTGCCAAGGCTGCCGTGGAATGGTTT
GATGGGAAAGATTTTCAAGGGAGCAAACTTAAAGTCTCCCTTGCTCGGAAGAAGCCTCCAATGAACAGTATGCGG
GGTGGTCTGCCACCCCGTGAGGGCAGAGGCATGCCACCACCACTCCGTGGAGGTCCAGGAGGCCCAGGAGGTCCT
GGGGGACCCATGGGTCGCATGGGAGGCCGTGGAGGAGATAGAGGAGGCTTCCCTCCAAGAGGACCCCGGGGTTCC
CGAGGGAACCCCTCTGGAGGAGGAAACGTCCAGCACCGAGCTGGAGACTGGCAGTGTCCCAATCCGGGTTGTGGA
AACCAGAACTTCGCCTGGAGAACAGAGTGCAACCAGTGTAAGGCCCCAAAGCCTGAAGGCTTCCTCCCGCCACCC
TTTCCGCCCCCGGGTGGTGATCGTGGCAGAGGTGGCCCTGGTGGCATGCGGGGAGGAAGAGGTGGCCTCATGGAT
CGTGGTGGTCCCGGTGGAATGTTCAGAGGTGGCCGTGGTGGAGACAGAGGTGGCTTCCGTGGTGGCCGGGGCATG
GACCGAGGTGGCTTTGGTGGAGGAAGACGAGGTGGCCCTGGGGGGCCCCCTGGACCTTTGATGGAACAGGCGATC
GCAGCTAGAAACGCCTACCTGAGAAAGAAAATCGCCAGACTGAAGAAGGACAACCTGCAGCTGGAAAGAGACGAG
CAGAACCTGGAAAAGATCATCGCCAACCTCAGAGATGAGATCGCCAGACTGGAAAACGAGGTGGCCAGCCACGAG
CAGTTAATCGCATACCCCTACGACGTGCCCGACTACGCCATCGAAGGCCGCCATATGCTAGCCTCCAAAACCATC
GTTCTTTCGGTCGGCGAGGCTACTCGCACTCTGACTGAGATCCAGTCCACCGCAGACCGTCAGATCTTCGAAGAG
AAGGTCGGGCCTCTGGTGGGTCGGCTGCGCCTCACGGCTTCGCTCCGTCAAAACGGAGCCAAGACCGCGTATCGC
GTCAACCTAAAACTGGATCAGGCGGACGTCGTTGATTCCGGACTTCCGAAAGTGCGCTACACTCAGGTATGGTCG
CACGACGTGACAATCGTTGCGAATAGCACCGAGGCCTCGCGCAAATCGTTGTACGATTTGACCAAGTCCCTCGTC
GCGACCTCGCAGGTCGAAGATCTTGTCGTCAACCTTGTGCCGCTGGGCCGTGCGGATCCGCTAGCCTCCAAAACC
ATCGTTCTTTCGGTCGGCGAGGCTACTCGCACTCTGACTGAGATCCAGTCCACCGCAGACCGTCAGATCTTCGAA
GAGAAGGTCGGGCCTCTGGTGGGTCGGCTGCGCCTCACGGCTTCGCTCCGTCAAAACGGAGCCAAGACCGCGTAT
CGCGTCAACCTAAAACTGGATCAGGCGGACGTCGTTGATTCCGGACTTCCGAAAGTGCGCTACACTCAGGTATGG
TCGCACGACGTGACAATCGTTGCGAATAGCACCGAGGCCTCGCGCAAATCGTTGTACGATTTGACCAAGTCCCTC
GTCGCGACCTCGCAGGTCGAAGATCTTGTCGTCAACCTTGTGCCGCTGGGCCGTCCACCGGTCGCCACCTAA
(SEQ ID NO: 579)

Protein:
MVGRNSAIAAGVCGALFIGYCIYFDRKRRSDPNFKNRLRERRKKQKLAKERAGLSKLPDLKDAEAVQKFFMASTD
YSTYSQAAAQQGYSAYTAQPTQGYAQTTQAYGQQSYGTYGQPTDVSYTQAQTTATYGQTAYATSYGQPPTGYTTP
TAPQAYSQPVQGYGTGAYDTTTATVTTTQASYAAQSAYGTQPAYPAYGQQPAATAPTRPQDGNKPTETSQPQSST
GGYNQPSLGYGQSNYSYPQVPGSYPMQPVTAPPSYPPTSYSSTQPTSYDQSSYSQQNTYGQPSSYGQQSSYGQQS
SYGQQPPTSYPPQTGSYSQAPSQYSQQSSSYGQQSSFRQDHPSSMGVYGQESGGFSGPGENRSMSGPDNRGRGRG
GFDRGGMSRGGRGGGRGGMGSAGERGGFNKPGGPMDEGPDLDLGPPVDPDEDSDNSAIYVQGLNDSVTLDDLADF
FKQCGVVKMNKRTGQPMIHIYLDKETGKPKGDATVSYEDPPTAKAAVEWFDGKDFQGSKLKVSLARKKPPMNSMR
GGLPPREGRGMPPPLRGGPGGPGGPGGPMGRMGGRGGDRGGFPPRGPRGSRGNPSGGGNVQHRAGDWQCPNPGCG
NQNFAWRTECNQCKAPKPEGFLPPPFPPPGGDRGRGGPGGMRGGRGGLMDRGGPGGMFRGGRGGDRGGFRGGRGM
DRGGFGGGRRGGPGGPPGPLMEQAIAARNAYLRKKIARLKKDNLQLERDEQNLEKIIANLRDEIARLENEVASHE
QLIAYPYDVPDYAIEGRHMLASKTIVLSVGEATRTLTEIQSTADRQIFEEKVGPLVGRLRLTASLRQNGAKTAYR
VNLKLDQADVVDSGLPKVRYTQVWSHDVTIVANSTEASRKSLYDLTKSLVATSQVEDLVVNLVPLGRADPLASKT
IVLSVGEATRTLTEIQSTADRQIFEEKVGPLVGRLRLTASLRQNGAKTAYRVNLKLDQADVVDSGLPKVRYTQVW
SHDVTIVANSTEASRKSLYDLTKSLVATSQVEDLVVNLVPLGRPPVAT* (SEQ ID NO: 580)

KIF16B::EWSR1::SYNZIP2::MCP
DNA:
ATGGCATCGGTCAAGGTGGCCGTGAGGGTCCGGCCCATGAATCGCAGGGAAAAGGACTTGGAGGCCAAGTTCATT
ATTCAGATGGAGAAAAGCAAAACGACAATCACAAACTTAAAGATACCAGAAGGAGGCACTGGGGACTCAGGAAGA
GAACGGACCAAGACCTTCACCTATGACTTTTCTTTTTATTCTGCTGATACAAAAAGCCCAGATTACGTTTCACAA

GAAATGGTTTTCAAAACCCTCGGCACAGATGTCGTGAAGTCTGCATTTGAAGGTTATAATGCTTGTGTCTTTGCA
TATGGGCAAACTGGATCTGGAAAGTCATACACTATGATGGGAAATTCTGGAGATTCTGGCTTAATACCTCGGATC
TGTGAAGGACTCTTCAGTCGGATAAATGAAACCACCAGATGGGATGAAGCTTCTTTTCGAACTGAAGTCAGCTAC
TTAGAAATTTATAACGAACGTGTGAGAGATCTACTTCGGCGGAAGTCATCTAAAACCTTCAATTTGAGAGTCCGT
GAGCATCCCAAAGAAGGCCCTTATGTTGAGGATTTATCCAAACATTTAGTACAGAATTATGGTGACGTAGAAGAA
CTTATGGATGCGGGCAATATCAACCGGACCACCGCAGCGACTGGGATGAACGACGTCAGTAGCAGGTCTCATGCC
ATCTTCACCATCAAGTTCACTCAGGCTAAATTTGATTCTGAAATGCCATGTGAAACCGTCAGTAAGATCCACTTG
GTTGATCTTGCCGGAAGTGAGCGTGCAGATGCCACCGGAGCCACCGGGGTTAGGCTAAAGGAAGGGGGAAATATT
AACAAGTCCCTCGTGACTCTGGGGAACGTCATTTCTGCCTTAGCTGATTTATCTCAGGATGCTGCAAATACTCTT
GCAAAGAAGAAGCAAGTTTTCGTGCCTTACAGGGATTCTGTGTTGACTTGGTTGTTAAAAGATAGCCTTGGAGGA
AACTCTAAAACTATCATGATTGCCACCATTTCACCTGCTGATGTCAATTATGGAGAAACCCTAAGTACTCTTCGC
TATGCAAATAGAGCCAAAAACATCATCAACAAGCCTACCATTAATGAGGATGCCAACGTCAAACTTATCCGTGAG
CTGCGAGCTGAAATAGCCAGACTGAAAACGCTGCTTGCTCAAGGGAATCAGATTGCCCTCTTAGACTCCCCCACA
ATGGCGTCCACGGATTACAGTACCTATAGCCAAGCTGCAGCGCAGCAGGGCTACAGTGCTTACACCGCCCAGCCC
ACTCAAGGATATGCACAGACCACCCAGGCATATGGGCAACAAAGCTATGGAACCTATGGACAGCCCACTGATGTC
AGCTATACCCAGGCTCAGACCACTGCAACCTATGGGCAGACCGCCTATGCAACTTCTTATGGACAGCCTCCCACT
GGTTATACTACTCCAACTGCCCCCCAGGCATACAGCCAGCCTGTCCAGGGGTATGGCACTGGTGCTTATGATACC
ACCACTGCTACAGTCACCACCACCCAGGCCTCCTATGCAGCTCAGTCTGCATATGGCACTCAGCCTGCTTATCCA
GCCTATGGGCAGCAGCCAGCAGCCACTGCACCTACAAGACCGCAGGATGGAAACAAGCCCACTGAGACTAGTCAA
CCTCAATCTAGCACAGGGGGTTACAACCAGCCCAGCCTAGGATATGGACAGAGTAACTACAGTTATCCCCAGGTA
CCTGGGAGCTACCCCATGCAGCCAGTCACTGCACCTCCATCCTACCCTCCTACCAGCTATTCCTCTACACAGCCG
ACTAGTTATGATCAGAGCAGTTACTCTCAGCAGAACACCTATGGGCAACCGAGCAGCTATGGACAGCAGAGTAGC
TATGGTCAACAAAGCAGCTATGGGCAGCAGCCTCCCACTAGTTACCCACCCCAAACTGGATCCTACAGCCAAGCT
CCAAGTCAATATAGCCAACAGAGCAGCAGCTACGGGCAGCAGAGTTCATTCCGACAGGACCACCCCAGTAGCATG
GGTGTTTATGGGCAGGAGTCTGGAGGATTTTCCGGACCAGGAGAGAACCGGAGCATGAGTGGCCCTGATAACCGG
GGCAGGGGAAGAGGGGGATTTGATCGTGGAGGCATGAGCAGAGGTGGGCGGGGAGGAGGACGCGGTGGAATGGGC
AGCGCTGGAGAGCGAGGTGGCTTCAATAAGCCTGGTGGACCCATGGATGAAGGACCAGATCTTGATCTAGGCCCA
CCTGTAGATCCAGATGAAGACTCTGACAACAGTGCAATTTATGTACAAGGATTAAATGACAGTGTGACTCTAGAT
GATCTGGCAGACTTCTTTAAGCAGTGTGGGGTTGTTAAGATGAACAAGAGAACTGGGCAACCCATGATCCACATC
TACCTGGACAAGGAAACAGGAAAGCCCAAAGGCGATGCCACAGTGTCCTATGAAGACCCACCTACTGCCAAGGCT
GCCGTGGAATGGTTTGATGGGAAAGATTTTCAAGGGAGCAAACTTAAAGTCTCCCTTGCTCGGAAGAAGCCTCCA
ATGAACAGTATGCGGGGTGGTCTGCCACCCCGTGAGGGCAGAGGCATGCCACCACCACTCCGTGGAGGTCCAGGA
GGCCCAGGAGGTCCTGGGGGACCCATGGGTCGCATGGGAGGCCGTGGAGGAGATAGAGGAGGCTTCCCTCCAAGA
GGACCCCGGGGTTCCCGAGGGAACCCCTCTGGAGGAGGAAACGTCCAGCACCGAGCTGGAGACTGGCAGTGTCCC
AATCCGGGTTGTGGAAACCAGAACTTCGCCTGGAGAACAGAGTGCAACCAGTGTAAGGCCCCAAAGCCTGAAGGC
TTCCTCCCGCCACCCTTTCCGCCCCCGGGTGGTGATCGTGGCAGAGGTGGCCCTGGTGGCATGCGGGGAGGAAGA
GGTGGCCTCATGGATCGTGGTGGTCCCGGTGGAATGTTCAGAGGTGGCCGTGGTGGAGACAGAGGTGGCTTCCGT
GGTGGCCGGGGCATGGACCGAGGTGGCTTTGGTGGAGGAAGACGAGGTGGCCCTGGGGGGCCCCCTGGACCTTTG
ATGGAACAGGCGATCGCAGCTAGAAACGCCTACCTGAGAAAGAAAATCGCCAGACTGAAGAAGGACAACCTGCAG
CTGGAAAGAGACGAGCAGAACCTGGAAAAGATCATCGCCAACCTCAGAGATGAGATCGCCAGACTGGAAAACGAG
GTGGCCAGCCACGAGCAGTATCCCTATGATGTGCCGGATTATGCTGGAGCACCAGGAAGTGCTGGTTCTGCTGCT
GGTAGTGGAGCTTCTAACTTTACTCAGTTCGTTCTCGTCGACAATGGCGGAACTGGCGACGTGACTGTCGCCCCA
AGCAACTTCGCTAACGGGATCGCTGAATGGATCAGCTCTAACTCGCGTTCACAGGCTTACAAAGTAACCTGTAGC
GTTCGTCAGAGCTCTGCGCAGAATCGCAAATACACCATCAAAGTCGAGGTGCCTAAAGGCGCCTGGCGTTCGTAC
TTAAATATGGAACTAACCATTCCAATTTTCGCCACGAATTCCGACTGCGAGCTTATTGTTAAGGCAATGCAAGGT
CTCCTAAAAGATGGAAACCCGATTCCCTCAGCAATCGCAGCAAACTCCGGCATCTACTAA (SEQ ID NO:
581)

Protein:
MASVKVAVRVRPMNRREKDLEAKFIIQMEKSKTTITNLKIPEGGTGDSGRERTKTFTYDFSFYSADTKSPDYVSQ
EMVFKTLGTDVVKSAFEGYNACVFAYGQTGSGKSYTMMGNSGDSGLIPRICEGLFSRINETTRWDEASFRTEVSY
LEIYNERVRDLLRRKSSKTFNLRVREHPKEGPYVEDLSKHLVQNYGDVEELMDAGNINRTTAATGMNDVSSRSHA
IFTIKFTQAKFDSEMPCETVSKIHLVDLAGSERADATGATGVRLKEGGNINKSLVTLGNVISALADLSQDAANTL
AKKKQVFVPYRDSVLTWLLKDSLGGNSKTIMIATISPADVNYGETLSTLRYANRAKNIINKPTINEDANVKLIRE
LRAEIARLKTLLAQGNQIALLDSPTMASTDYSTYSQAAAQQGYSAYTAQPTQGYAQTTQAYGQQSYGTYGQPTDV
SYTQAQTTATYGQTAYATSYGQPPTGYTTPTAPQAYSQPVQGYGTGAYDTTTATVTTTQASYAAQSAYGTQPAYP
AYGQQPAATAPTRPQDGNKPTETSQPQSSTGGYNQPSLGYGQSNYSYPQVPGSYPMQPVTAPPSYPPTSYSSTQP
TSYDQSSYSQQNTYGQPSSYGQQSSYGQQSSYGQQPPTSYPPQTGSYSQAPSQYSQQSSSYGQQSSFRQDHPSSM

GVYGQESGGFSGPGENRSMSGPDNRGRGRGGFDRGGMSRGGRGGGRGGMGSAGERGGFNKPGGPMDEGPDLDLGP
PVDPDEDSDNSAIYVQGLNDSVTLDDLADFFKQCGVVKMNKRTGQPMIHIYLDKETGKPKGDATVSYEDPPTAKA
AVEWFDGKDFQGSKLKVSLARKKPPMNSMRGGLPPREGRGMPPPLRGGPGGPGGPGGPMGRMGGRGGDRGGFPPR
GPRGSRGNPSGGGNVQHRAGDWQCPNPGCGNQNFAWRTECNQCKAPKPEGFLPPPFPPPGGDRGRGGPGGMRGGR
GGLMDRGGPGGMFRGGRGGDRGGFRGGRGMDRGGFGGGRRGGPGGPPGPLMEQAIAARNAYLRKKIARLKKDNLQ
LERDEQNLEKIIANLRDEIARLENEVASHEQYPYDVPDYAGAPGSAGSAAGSGASNFTQFVLVDNGGTGDVTVAP
SNFANGIAEWISSNSRSQAYKVTCSVRQSSAQNRKYTIKVEVPKGAWRSYLNMELTIPIFATNSDCELIVKAMQG
LLKDGNPIPSAIAANSGIY* (SEQ ID NO: 582)

LCK::SYNZIP1::PylRS(AF)
DNA:
ATGGGCTGCGTGTGCAGCAGCAACCCCGAGGGTACCGAGCTCGCGATCGCAAATCTGGTGGCCCAGCTGGAAAAC
GAGGTGGCCAGCCTGGAAAACGAGAACGAAACCCTGAAGAAAAAGAACCTGCACAAGAAGGACCTGATCGCCTAC
CTGGAAAAGGAAATCGCCAACCTGAGAAAGAAGATCGAGGAAGGCGCCGATTACAAGGACGATGATGACAAGGGA
GCACCAGGAAGTGCTGGTTCTGCTGCTGGTAGTGGAGCGTGCCCGGTGCCGCTGCAGCTGCCGCCGCTGGAACGC
CTGACCCTGGATGATAAAAAACCGCTGAATACCCTGATCTCTGCTACTGGTCTGTGGATGAGTCGTACCGGAACC
ATTCATAAAATCAAACACCACGAGGTTAGCCGTTCGAAAATCTATATTGAGATGGCGTGTGGCGATCATCTGGTT
GTGAACAATAGCCGCTCTTCTCGTACAGCACGTGCACTGCGTCACCACAAATATCGTAAAACCTGTAAACGTTGC
CGTGTGTCCGATGAGGATCTGAACAAATTCCTGACAAAAGCCAATGAGGACCAAACAAGCGTGAAAGTGAAAGTC
GTTAGCGCTCCTACCCGTACTAAAAAAGCAATGCCGAAATCCGTTGCTCGTGCCCCTAAACCACTGGAAAACACT
GAAGCAGCACAGGCACAGCCGTCTGGAAGCAAATTCTCTCCGGCCATTCCTGTTTCTACCCAGGAGTCCGTTTCT
GTTCCAGCAAGTGTGAGCACCAGCATTAGCAGTATTAGCACCGGTGCCACCGCTAGCGCCCTGGTTAAAGGCAAT
ACCAATCCGATTACAAGCATGTCTGCCCCGGTTCAAGCATCAGCTCCAGCACTGACAAAATCCCAAACCGATCGT
CTGGAGGTTCTGCTGAATCCGAAAGACGAAATCAGCCTGAATTCCGGCAAACCGTTTCGTGAACTGGAGAGCGAA
CTGCTGTCACGTCGTAAAAAAGACCTGCAACAAATCTATGCCGAAGAACGTGAGAACTATCTGGGGAAACTGGAA
CGTGAAATCACCCGCTTTTTCGTGGATCGTGGCTTTCTGGAGATCAAATCCCCGATTCTGATTCCTCTGGAGTAT
ATCGAGCGTATGGGCATCGACAATGATACCGAACTGAGCAAACAAATTTTCCGTGTGGATAAAAACTTCTGTCTG
CGCCCTATGCTAGCACCAAATCTGGCTAACTATCTGCGCAAACTGGACCGTGCCCTGCCTGATCCTATCAAAATC
TTCGAGATCGGCCCGTGTTATCGTAAAGAGTCCGACGGTAAAGAACATCTGGAGGAGTTTACCATGCTGAACTTT
TGCCAAATGGGTTCAGGTTGTACTCGTGAGAACCTGGAAAGCATCATCACCGATTTTCTGAACCACCTGGGCATT
GACTTCAAAATTGTGGGCGACAGCTGTATGGTGTTTGGCGACACCCTGGATGTCATGCACGGCGACCTGGAACTG
TCTAGTGCCGTTGTGGGCCCAATCCCGCTGGATCGTGAGTGGGGTATCGACAAACCTTGGATCGGTGCGGGTTTT
GGTCTGGAGCGTCTGCTGAAAGTAAAACACGACTTCAAGAACATCAAACGTGCTGCACGTTCCGAGTCCTATTAC
AATGGTATTTCTACTAACCTGTAA (SEQ ID NO: 583)

Protein:
MGCVCSSNPEGTELAIANLVAQLENEVASLENENETLKKKNLHKKDLIAYLEKEIANLRKKIEEGADYKDDDDKG
APGSAGSAAGSGACPVPLQLPPLERLTLDDKKPLNTLISATGLWMSRTGTIHKIKHHEVSRSKIYIEMACGDHLV
VNNSRSSRTARALRHHKYRKTCKRCRVSDEDLNKFLTKANEDQTSVKVKVVSAPTRTKKAMPKSVARAPKPLENT
EAAQAQPSGSKFSPAIPVSTQESVSVPASVSTSISSISTGATASALVKGNTNPITSMSAPVQASAPALTKSQTDR
LEVLLNPKDEISLNSGKPFRELESELLSRRKKDLQQIYAEERENYLGKLEREITRFFVDRGFLEIKSPILIPLEY
IERMGIDNDTELSKQIFRVDKNFCLRPMLAPNLANYLRKLDRALPDPIKIFEIGPCYRKESDGKEHLEEFTMLNF
CQMGSGCTRENLESIITDFLNHLGIDFKIVGDSCMVFGDTLDVMHGDLELSSAVVGPIPLDREWGIDKPWIGAGF
GLERLLKVKHDFKNIKRAARSESYYNGISTNL* (SEQ ID NO: 584)

LCK::FUS::SYNZIP1::PylRS(AF)
DNA:
ATGGGCTGCGTGTGCAGCAGCAACCCCGAGGGTACCGAGCTCATGGCCTCAAACGATTATACCCAACAAGCAACC
CAAAGCTATGGGGCCTACCCCACCCAGCCCGGGCAGGGCTATTCCCAGCAGAGCAGTCAGCCCTACGGACAGCAG
AGTTACAGTGGTTATAGCCAGTCCACGGACACTTCAGGATATGGCCAGAGCAGCTATTCTTCTTATGGCCAGAGC
CAGAACACAGGCTATGGAACTCAGTCAACTCCCCAGGGATATGGCTCGACTGGCGGCTATGGCAGTAGCCAGAGC
TCCCAATCGTCTTACGGGCAGCAGTCCTCCTACCCTGGCTATGGCCAGCAGCCAGCTCCCAGCAGCACCTCGGGA
AGTTACGGTAGCAGTTCTCAGAGCAGCAGCTATGGGCAGCCCCAGAGTGGGAGCTACAGCCAGCAGCCTAGCTAT
GGTGGACAGCAGCAAAGCTATGGACAGCAGCAAAGCTATAATCCCCCTCAGGGCTATGGACAGCAGAACCAGTAC
AACAGCAGCAGTGGTGGTGGAGGTGGAGGTGGAGGTGGAGGTAACTATGGCCAAGATCAATCCTCCATGAGTAGT
GGTGGTGGCAGTGGTGGCGGTTATGGCAATCAAGACCAGAGTGGTGGAGGTGGCAGCGGTGGCTATGGACAGCAG

GACCGTGGAGGCCGCGGCAGGGGTGGCAGTGGTGGCGGCGGCGGCGGCGGTGGTGGTTACAACCGCAGCAGT
GGTGGCTATGAACCCAGAGGTCGTGGAGGTGGCCGTGGAGGCAGAGGTGGCATGGGCGGAAGTGACCGTGGTGGC
TTCAATAAATTTGGTGGCCCTCGGGACCAAGGATCACGTCATGACTCCGAACAGGATAATTCAGACAACAACACC
ATCTTTGTGCAAGGCCTGGGTGAGAATGTTACAATTGAGTCTGTGGCTGATTACTTCAAGCAGATTGGTATTATT
AAGACAAACAAGAAAACGGGACAGCCCATGATTAATTTGTACACAGACAGGGAAACTGGCAAGCTGAAGGGAGAG
GCAACGGTCTCTTTTGATGACCCACCTTCAGCTAAAGCAGCTATTGACTGGTTTGATGGTAAAGAATTCTCCGGA
AATCCTATCAAGGTCTCATTTGCTACTCGCCGGGCAGACTTTAATCGGGGTGGTGGCAATGGTCGTGGAGGCCGA
GGGCGAGGAGGACCCATGGGCCGTGGAGGCTATGGAGGTGGTGGCAGTGGTGGTGGTGGCCGAGGAGGATTTCCC
AGTGGAGGTGGTGGCGGTGGAGGACAGCAGCGAGCTGGTGACTGGAAGTGTCCTAATCCCACCTGTGAGAATATG
AACTTCTCTTGGAGGAATGAATGCAACCAGTGTAAGGCCCCTAAACCAGATGGCCCAGGAGGGGGACCAGGTGGC
TCTCACATGGGGGGTAACTACGGGGATGATCGTCGTGGTGGCAGAGGTGGTGCGATCGCAAATCTGGTGGCCCAG
CTGGAAAACGAGGTGGCCAGCCTGGAAAACGAGAACGAAACCCTGAAGAAAAAGAACCTGCACAAGAAGGACCTG
ATCGCCTACCTGGAAAAGGAAATCGCCAACCTGAGAAAGAAGATCGAGGAAGGCGCCGATTACAAGGACGATGAT
GACAAGGGAGCACCAGGAAGTGCTGGTTCTGCTGCTGGTAGTGGAGCGTGCCCGGTGCCGCTGCAGCTGCCGCCG
CTGGAACGCCTGACCCTGGATGATAAAAAACCGCTGAATACCCTGATCTCTGCTACTGGTCTGTGGATGAGTCGT
ACCGGAACCATTCATAAAATCAAACACCACGAGGTTAGCCGTTCGAAAATCTATATTGAGATGGCGTGTGGCGAT
CATCTGGTTGTGAACAATAGCCGCTCTTCTCGTACAGCACGTGCACTGCGTCACCACAAATATCGTAAAACCTGT
AAACGTTGCCGTGTGTCCGATGAGGATCTGAACAAATTCCTGACAAAAGCCAATGAGGACCAAACAAGCGTGAAA
GTGAAAGTCGTTAGCGCTCCTACCCGTACTAAAAAAGCAATGCCGAAATCCGTTGCTCGTGCCCCTAAACCACTG
GAAAACACTGAAGCAGCACAGGCACAGCCGTCTGGAAGCAAATTCTCTCCGGCCATTCCTGTTTCTACCCAGGAG
TCCGTTTCTGTTCCAGCAAGTGTGAGCACCAGCATTAGCAGTATTAGCACCGGTGCCACCGCTAGCGCCCTGGTT
AAAGGCAATACCAATCCGATTACAAGCATGTCTGCCCCGGTTCAAGCATCAGCTCCAGCACTGACAAAATCCCAA
ACCGATCGTCTGGAGGTTCTGCTGAATCCGAAAGACGAAATCAGCCTGAATTCCGGCAAACCGTTTCGTGAACTG
GAGAGCGAACTGCTGTCACGTCGTAAAAAAGACCTGCAACAAATCTATGCCGAAGAACGTGAGAACTATCTGGGG
AAACTGGAACGTGAAATCACCCGCTTTTTCGTGGATCGTGGCTTTCTGGAGATCAAATCCCCGATTCTGATTCCT
CTGGAGTATATCGAGCGTATGGGCATCGACAATGATACCGAACTGAGCAAACAAATTTTCCGTGTGGATAAAAAC
TTCTGTCTGCGCCCTATGCTAGCACCAAATCTGGCTAACTATCTGCGCAAACTGGACCGTGCCCTGCCTGATCCT
ATCAAAATCTTCGAGATCGGCCCGTGTTATCGTAAAGAGTCCGACGGTAAAGAACATCTGGAGGAGTTTACCATG
CTGAACTTTTGCCAAATGGGTTCAGGTTGTACTCGTGAGAACCTGGAAAGCATCATCACCGATTTTCTGAACCAC
CTGGGCATTGACTTCAAAATTGTGGGCGACAGCTGTATGGTGTTTGGCGACACCCTGGATGTCATGCACGGCGAC
CTGGAACTGTCTAGTGCCGTTGTGGGCCCAATCCCGCTGGATCGTGAGTGGGGTATCGACAAACCTTGGATCGGT
GCGGGTTTTGGTCTGGAGCGTCTGCTGAAAGTAAAACACGACTTCAAGAACATCAAACGTGCTGCACGTTCCGAG
TCCTATTACAATGGTATTTCTACTAACCTGTAA (SEQ ID NO: 585)

Protein:
MGCVCSSNPEGTELMASNDYTQQATQSYGAYPTQPGQGYSQQSSQPYGQQSYSGYSQSTDTSGYGQSSYSSYGQS
QNTGYGTQSTPQGYGSTGGYGSSQSSQSSYGQQSSYPGYGQQPAPSSTSGSYGSSSQSSSYGQPQSGSYSQQPSY
GGQQQSYGQQQSYNPPQGYGQQNQYNSSSGGGGGGGGGGNYGQDQSSMSSGGGSGGGYGNQDQSGGGGSGGYGQQ
DRGGRGRGGSGGGGGGGGGGYNRSSGGYEPRGRGGGRGGRGGMGGSDRGGFNKFGGPRDQGSRHDSEQDNSDNNT
IFVQGLGENVTIESVADYFKQIGIIKTNKKTGQPMINLYTDRETGKLKGEATVSFDDPPSAKAAIDWFDGKEFSG
NPIKVSFATRRADFNRGGGNGRGGRGRGGPMGRGGYGGGGSGGGGRGGFPSGGGGGGGQQRAGDWKCPNPTCENM
NFSWRNECNQCKAPKPDGPGGGPGGSHMGGNYGDDRRGGRGGAIANLVAQLENEVASLENENETLKKKNLHKKDL
IAYLEKEIANLRKKIEEGADYKDDDDKGAPGSAGSAAGSGACPVPLQLPPLERLTLDDKKPLNTLISATGLWMSR
TGTIHKIKHHEVSRSKIYIEMACGDHLVVNNSRSSRTARALRHHKYRKTCKRCRVSDEDLNKFLTKANEDQTSVK
VKVVSAPTRTKKAMPKSVARAPKPLENTEAAQAQPSGSKFSPAIPVSTQESVSVPASVSTSISSISTGATASALV
KGNTNPITSMSAPVQASAPALTKSQTDRLEVLLNPKDEISLNSGKPFRELESELLSRRKKDLQQIYAEERENYLG
KLEREITRFFVDRGFLEIKSPILIPLEYIERMGIDNDTELSKQIFRVDKNFCLRPMLAPNLANYLRKLDRALPDP
IKIFEIGPCYRKESDGKEHLEEFTMLNFCQMGSGCTRENLESIITDFLNHLGIDFKIVGDSCMVFGDTLDVMHGD
LELSSAVVGPIPLDREWGIDKPWIGAGFGLERLLKVKHDFKNIKRAARSESYYNGISTNL* (SEQ ID NO:
586)

SYNZIP4::4xλN22
DNA:
ATGGCGATCGCACAAAAGGTGGCTGAACTGAAAAATAGAGTGGCCGTGAAGCTGAACCGGAACGAGCAGCTGAAG
AACAAGGTGGAAGAGCTGAAGAACAGAAACGCCTACCTGAAGAATGAGCTGGCCACCCTGGAAAACGAGGTGGCC
AGACTGGAAAACGACGTGGCCGAGGGAGCACCAGGAAGTGCTGGTTCTGCTGCTGGTAGTGGAGAGCAGAAGCTG
ATCTCAGAGGAGGACCTGCTAGCCACCATGGACGCACAAACACGACGACGTGAGCGTCGCGCTGAGAAACAAGCT

CAATGGAAAGCTGCAAACCCACCGCTCGACGGAGCCGGAGCTGGCGCTGGAGCTGGAGCCGGAGCTGGCGGTCTA
GCCACCATGGACGCACAAACACGACGACGTGAGCGTCGCGCTGAGAAACAAGCTCAATGGAAAGCTGCAAACCCA
CCGCTCGACGGAGCCGGAGCTGGCGCTGGAGCTGGAGCCGGAGCTGGCGGTCTAGCCACCATGGACGCACAAACA
CGACGACGTGAGCGTCGCGCTGAGAAACAAGCTCAATGGAAAGCTGCAAACCCACCGCTCGACGGAGCCGGAGCT
GGCGCTGGAGCTGGAGCCGGAGCTGGCGGTCTAGCCACCATGGACGCACAAACACGACGACGTGAGCGTCGCGCT
GAGAAACAAGCTCAATGGAAAGCTGCAAACCCACCGCTCGAGTCTAGAGGGCCCGTTTAA (SEQ ID NO:
587)

Protein:
MAIAQKVAELKNRVAVKLNRNEQLKNKVEELKNRNAYLKNELATLENEVARLENDVAEGAPGSAGSAAGSGEQKL
ISEEDLLATMDAQTRRRERRAEKQAQWKAANPPLDGAGAGAGAGAGGLATMDAQTRRRERRAEKQAQWKAANP
PLDGAGAGAGAGAGGLATMDAQTRRRERRAEKQAQWKAANPPLDGAGAGAGAGAGGLATMDAQTRRRERRA
EKQAQWKAANPPLESRGPV* (SEQ ID NO: 588)

TOM20::FUS::SYNZIP1::MCP::PylRS(AF)
DNA:
ATGGTGGGTCGGAACAGCGCCATCGCCGCCGGTGTATGCGGGGCCCTTTTCATTGGGTACTGCATCTACTTCGAC
CGCAAAAGACGAAGTGACCCCAACTTCAAGAACAGGCTTCGAGAACGAAGAAAGAAACAGAAGCTTGCCAAGGAG
AGAGCTGGGCTTTCCAAGTTACCTGACCTTAAAGATGCTGAAGCTGTTCAGAAATTCTTCATGGCCTCAAACGAT
TATACCCAACAAGCAACCCAAAGCTATGGGGCCTACCCCACCCAGCCCGGGCAGGGCTATTCCCAGCAGAGCAGT
CAGCCCTACGGACAGCAGAGTTACAGTGGTTATAGCCAGTCCACGGACACTTCAGGATATGGCCAGAGCAGCTAT
TCTTCTTATGGCCAGAGCCAGAACACAGGCTATGGAACTCAGTCAACTCCCCAGGGATATGGCTCGACTGGCGGC
TATGGCAGTAGCCAGAGCTCCCAATCGTCTTACGGGCAGCAGTCCTCCTACCCTGGCTATGGCCAGCAGCCAGCT
CCCAGCAGCACCTCGGGAAGTTACGGTAGCAGTTCTCAGAGCAGCAGCTATGGGCAGCCCCAGAGTGGGAGCTAC
AGCCAGCAGCCTAGCTATGGTGGACAGCAGCAAAGCTATGGACAGCAGCAAAGCTATAATCCCCCTCAGGGCTAT
GGACAGCAGAACCAGTACAACAGCAGCAGTGGTGGTGGAGGTGGAGGTGGAGGTGGAGGTAACTATGGCCAAGAT
CAATCCTCCATGAGTAGTGGTGGTGGCAGTGGTGGCGGTTATGGCAATCAAGACCAGAGTGGTGGAGGTGGCAGC
GGTGGCTATGGACAGCAGGACCGTGGAGGCCGCGGCAGGGGTGGCAGTGGTGGCGGCGGCGGCGGCGGCGGTGGT
GGTTACAACCGCAGCAGTGGTGGCTATGAACCCAGAGGTCGTGGAGGTGGCCGTGGAGGCAGAGGTGGCATGGGC
GGAAGTGACCGTGGTGGCTTCAATAAATTTGGTGGCCCTCGGGACCAAGGATCACGTCATGACTCCGAACAGGAT
AATTCAGACAACAACACCATCTTTGTGCAAGGCCTGGGTGAGAATGTTACAATTGAGTCTGTGGCTGATTACTTC
AAGCAGATTGGTATTATTAAGACAAACAAGAAAACGGGACAGCCCATGATTAATTTGTACACAGACAGGGAAACT
GGCAAGCTGAAGGGAGAGGCAACGGTCTCTTTTGATGACCCACCTTCAGCTAAAGCAGCTATTGACTGGTTTGAT
GGTAAAGAATTCTCCGGAAATCCTATCAAGGTCTCATTTGCTACTCGCCGGGCAGACTTTAATCGGGGTGGTGGC
AATGGTCGTGGAGGCCGAGGGCGAGGAGGACCCATGGGCCGTGGAGGCTATGGAGGTGGTGGCAGTGGTGGTGGT
GGCCGAGGAGGATTTCCCAGTGGAGGTGGTGGCGGTGGAGGACAGCAGCGAGCTGGTGACTGGAAGTGTCCTAAT
CCCACCTGTGAGAATATGAACTTCTCTTGGAGGAATGAATGCAACCAGTGTAAGGCCCCTAAACCAGATGGCCCA
GGAGGGGGACCAGGTGGCTCTCACATGGGGGGTAACTACGGGGATGATCGTCGTGGTGGCAGAGGAGGCGCGATC
GCAAATCTGGTGGCCCAGCTGGAAAACGAGGTGGCCAGCCTGGAAAACGAGAACGAAACCCTGAAGAAAAAGAAC
CTGCACAAGAAGGACCTGATCGCCTACCTGGAAAAGGAAATCGCCAACCTGAGAAAGAAGATCGAGGAAGCATCG
ATATATCCCTATGATGTGCCGGATTATGCTGGAGCACCAGGAAGTGCTGGTTCTGCTGCTGGTAGTGGAGCTTCT
AACTTTACTCAGTTCGTTCTCGTCGACAATGGCGGAACTGGCGACGTGACTGTCGCCCCAAGCAACTTCGCTAAC
GGGATCGCTGAATGGATCAGCTCTAACTCGCGTTCACAGGCTTACAAAGTAACCTGTAGCGTTCGTCAGAGCTCT
GCGCAGAATCGCAAATACACCATCAAAGTCGAGGTGCCTAAAGGCGCCTGGCGTTCGTACTTAAATATGGAACTA
ACCATTCCAATTTTCGCCACGAATTCCGACTGCGAGCTTATTGTTAAGGCAATGCAAGGTCTCCTAAAAGATGGA
AACCCGATTCCCTCAGCAATCGCAGCAAACTCCGGCATCTACGGCGCCGATTACAAGGACGATGATGACAAGGGA
GCACCAGGAAGTGCTGGTTCTGCTGCTGGTAGTGGAGCGTGCCCGGTGCCGCTGCAGCTGCCGCCGCTGGAACGC
CTGACCCTGGATGATAAAAAACCGCTGAATACCCTGATCTCTGCTACTGGTCTGTGGATGAGTCGTACCGGAACC
ATTCATAAAATCAAACACCACGAGGTTAGCCGTTCGAAAATCTATATTGAGATGGCGTGTGGCGATCATCTGGTT
GTGAACAATAGCCGCTCTTCTCGTACAGCACGTGCACTGCGTCACCACAAATATCGTAAAACCTGTAAACGTTGC
CGTGTGTCCGATGAGGATCTGAACAAATTCCTGACAAAAGCCAATGAGGACCAAACAAGCGTGAAAGTGAAAGTC
GTTAGCGCTCCTACCCGTACTAAAAAAGCAATGCCGAAATCCGTTGCTCGTGCCCCTAAACCACTGGAAAACACT
GAAGCAGCACAGGCACAGCCGTCTGGAAGCAAATTCTCTCCGGCCATTCCTGTTTCTACCCAGGAGTCCGTTTCT
GTTCCAGCAAGTGTGAGCACCAGCATTAGCAGTATTAGCACCGGTGCCACCGCTAGCGCCCTGGTTAAAGGCAAT
ACCAATCCGATTACAAGCATGTCTGCCCCGGTTCAAGCATCAGCTCCAGCACTGACAAAATCCCAAACCGATCGT
CTGGAGGTTCTGCTGAATCCGAAAGACGAAATCAGCCTGAATTCCGGCAAACCGTTTCGTGAACTGGAGAGCGAA
CTGCTGTCACGTCGTAAAAAAGACCTGCAACAAATCTATGCCGAAGAACGTGAGAACTATCTGGGGAAACTGGAA

CGTGAAATCACCCGCTTTTTCGTGGATCGTGGCTTTCTGGAGATCAAATCCCCGATTCTGATTCCTCTGGAGTAT
ATCGAGCGTATGGGCATCGACAATGATACCGAACTGAGCAAACAAATTTTCCGTGTGGATAAAAACTTCTGTCTG
CGCCCTATGCTAGCACCAAATCTGGCTAACTATCTGCGCAAACTGGACCGTGCCCTGCCTGATCCTATCAAAATC
TTCGAGATCGGCCCGTGTTATCGTAAAGAGTCCGACGGTAAAGAACATCTGGAGGAGTTTACCATGCTGAACTTT
TGCCAAATGGGTTCAGGTTGTACTCGTGAGAACCTGGAAAGCATCATCACCGATTTTCTGAACCACCTGGGCATT
GACTTCAAAATTGTGGGCGACAGCTGTATGGTGTTTGGCGACACCCTGGATGTCATGCACGGCGACCTGGAACTG
TCTAGTGCCGTTGTGGGCCCAATCCCGCTGGATCGTGAGTGGGGTATCGACAAACCTTGGATCGGTGCGGGTTTT
GGTCTGGAGCGTCTGCTGAAAGTAAAACACGACTTCAAGAACATCAAACGTGCTGCACGTTCCGAGTCCTATTAC
AATGGTATTTCTACTAACCTGTAA (SEQ ID NO: 589)

Protein:
MVGRNSAIAAGVCGALFIGYCIYFDRKRRSDPNFKNRLRERRKKQKLAKERAGLSKLPDLKDAEAVQKFFMASND
YTQQATQSYGAYPTQPGQGYSQQSSQPYGQQSYSGYSQSTDTSGYGQSSYSSYGQSQNTGYGTQSTPQGYGSTGG
YGSSQSSQSSYGQQSSYPGYGQQPAPSSTSGSYGSSSQSSSYGQPQSGSYSQQPSYGGQQQSYGQQQSYNPPQGY
GQQNQYNSSSGGGGGGGGGGNYGQDQSSMSSGGGSGGGYGNQDQSGGGGSGGYGQQDRGGRGRGGSGGGGGGGG
GYNRSSGGYEPRGRGGGRGGRGGMGGSDRGGFNKFGGPRDQGSRHDSEQDNSDNNTIFVQGLGENVTIESVADYF
KQIGIIKTNKKTGQPMINLYTDRETGKLKGEATVSFDDPPSAKAAIDWFDGKEFSGNPIKVSFATRRADFNRGGG
NGRGGRGRGGPMGRGGYGGGGSGGGGRGGFPSGGGGGGGQQRAGDWKCPNPTCENMNFSWRNECNQCKAPKPDGP
GGGPGGSHMGGNYGDDRRGGRGGAIANLVAQLENEVASLENENETLKKKNLHKKDLIAYLEKEIANLRKKIEEAS
IYPYDVPDYAGAPGSAGSAAGSGASNFTQFVLVDNGGTGDVTVAPSNFANGIAEWISSNSRSQAYKVTCSVRQSS
AQNRKYTIKVEVPKGAWRSYLNMELTIPIFATNSDCELIVKAMQGLLKDGNPIPSAIAANSGIYGADYKDDDDKG
APGSAGSAAGSGACPVPLQLPPLERLTLDDKKPLNTLISATGLWMSRTGTIHKIKHHEVSRSKIYIEMACGDHLV
VNNSRSSRTARALRHHKYRKTCKRCRVSDEDLNKFLTKANEDQTSVKVKVVSAPTRTKKAMPKSVARAPKPLENT
EAAQAQPSGSKFSPAIPVSTQESVSVPASVSTSISSISTGATASALVKGNTNPITSMSAPVQASAPALTKSQTDR
LEVLLNPKDEISLNSGKPFRELESELLSRRKKDLQQIYAEERENYLGKLEREITRFFVDRGFLEIKSPILIPLEY
IERMGIDNDTELSKQIFRVDKNFCLRPMLAPNLANYLRKLDRALPDPIKIFEIGPCYRKESDGKEHLEEFTMLNF
CQMGSGCTRENLESIITDFLNHLGIDFKIVGDSCMVFGDTLDVMHGDLELSSAVVGPIPLDREWGIDKPWIGAGF
GLERLLKVKHDFKNIKRAARSESYYNGISTNL* (SEQ ID NO: 590)

TOM20::FUS::SYNZIP2::MCP::PylRS(AF)
DNA:
ATGGTGGGTCGGAACAGCGCCATCGCCGCCGGTGTATGCGGGGCCCTTTTCATTGGGTACTGCATCTACTTCGAC
CGCAAAAGACGAAGTGACCCCAACTTCAAGAACAGGCTTCGAGAACGAAGAAAGAAACAGAAGCTTGCCAAGGAG
AGAGCTGGGCTTTCCAAGTTACCTGACCTTAAAGATGCTGAAGCTGTTCAGAAATTCTTCATGGCCTCAAACGAT
TATACCCAACAAGCAACCCAAAGCTATGGGGCCTACCCCACCCAGCCCGGGCAGGGCTATTCCCAGCAGAGCAGT
CAGCCCTACGGACAGCAGAGTTACAGTGGTTATAGCCAGTCCACGGACACTTCAGGATATGGCCAGAGCAGCTAT
TCTTCTTATGGCCAGAGCCAGAACACAGGCTATGGAACTCAGTCAACTCCCCAGGGATATGGCTCGACTGGCGGC
TATGGCAGTAGCCAGAGCTCCCAATCGTCTTACGGGCAGCAGTCCTCCTACCCTGGCTATGGCCAGCAGCCAGCT
CCCAGCAGCACCTCGGGAAGTTACGGTAGCAGTTCTCAGAGCAGCAGCTATGGGCAGCCCCAGAGTGGGAGCTAC
AGCCAGCAGCCTAGCTATGGTGGACAGCAGCAAAGCTATGGACAGCAGCAAAGCTATAATCCCCCTCAGGGCTAT
GGACAGCAGAACCAGTACAACAGCAGCAGTGGTGGTGGAGGTGGAGGTGGAGGTGGAGGTAACTATGGCCAAGAT
CAATCCTCCATGAGTAGTGGTGGTGGCAGTGGTGGCGGTTATGGCAATCAAGACCAGAGTGGTGGAGGTGGCAGC
GGTGGCTATGGACAGCAGGACCGTGGAGGCCGCGGCAGGGGTGGCAGTGGTGGCGGCGGCGGCGGCGGTGGT
GGTTACAACCGCAGCAGTGGTGGCTATGAACCCAGAGGTCGTGGAGGTGGCCGTGGAGGCAGAGGTGGCATGGGC
GGAAGTGACCGTGGTGGCTTCAATAAATTTGGTGGCCCTCGGGACCAAGGATCACGTCATGACTCCGAACAGGAT
AATTCAGACAACAACACCATCTTTGTGCAAGGCCTGGGTGAGAATGTTACAATTGAGTCTGTGGCTGATTACTTC
AAGCAGATTGGTATTATTAAGACAAACAAGAAAACGGGACAGCCCATGATTAATTTGTACACAGACAGGGAAACT
GGCAAGCTGAAGGGAGAGGCAACGGTCTCTTTTGATGACCCACCTTCAGCTAAAGCAGCTATTGACTGGTTTGAT
GGTAAAGAATTCTCCGGAAATCCTATCAAGGTCTCATTTGCTACTCGCCGGGCAGACTTTAATCGGGGTGGTGGC
AATGGTCGTGGAGGCCGAGGGCGAGGAGGACCCATGGGCCGTGGAGGCTATGGAGGTGGTGGCAGTGGTGGTGGT
GGCCGAGGAGGATTTCCCAGTGGAGGTGGTGGCGGTGGAGGACAGCAGCGAGCTGGTGACTGGAAGTGTCCTAAT
CCCACCTGTGAGAATATGAACTTCTCTTGGAGGAATGAATGCAACCAGTGTAAGGCCCCTAAACCAGATGGCCCA
GGAGGGGGACCAGGTGGCTCTCACATGGGGGGTAACTACGGGGATGATCGTCGTGGTGGCAGAGGAGGCGCGATC
GCAGCTAGAAACGCCTACCTGAGAAAGAAAATCGCCAGACTGAAGAAGGACAACCTGCAGCTGGAAAGAGACGAG
CAGAACCTGGAAAAGATCATCGCCAACCTCAGAGATGAGATCGCCAGACTGGAAAACGAGGTGGCCAGCCACGAG
CAGGCATCGATATATCCCTATGATGTGCCGGATTATGCTGGAGCACCAGGAAGTGCTGGTTCTGCTGCTGGTAGT
GGAGCTTCTAACTTTACTCAGTTCGTTCTCGTCGACAATGGCGGAACTGGCGACGTGACTGTCGCCCCAAGCAAC

TTCGCTAACGGGATCGCTGAATGGATCAGCTCTAACTCGCGTTCACAGGCTTACAAAGTAACCTGTAGCGTTCGT
CAGAGCTCTGCGCAGAATCGCAAATACACCATCAAAGTCGAGGTGCCTAAAGGCGCCTGGCGTTCGTACTTAAAT
ATGGAACTAACCATTCCAATTTTCGCCACGAATTCCGACTGCGAGCTTATTGTTAAGGCAATGCAAGGTCTCCTA
AAAGATGGAAACCCGATTCCCTCAGCAATCGCAGCAAACTCCGGCATCTACGGCGCCGATTACAAGGACGATGAT
GACAAGGGAGCACCAGGAAGTGCTGGTTCTGCTGCTGGTAGTGGAGCGTGCCCGGTGCCGCTGCAGCTGCCGCCG
CTGGAACGCCTGACCCTGGATGATAAAAAACCGCTGAATACCCTGATCTCTGCTACTGGTCTGTGGATGAGTCGT
ACCGGAACCATTCATAAAATCAAACACCACGAGGTTAGCCGTTCGAAAATCTATATTGAGATGGCGTGTGGCGAT
CATCTGGTTGTGAACAATAGCCGCTCTTCTCGTACAGCACGTGCACTGCGTCACCACAAATATCGTAAAACCTGT
AAACGTTGCCGTGTGTCCGATGAGGATCTGAACAAATTCCTGACAAAAGCCAATGAGGACCAAACAAGCGTGAAA
GTGAAAGTCGTTAGCGCTCCTACCCGTACTAAAAAAGCAATGCCGAAATCCGTTGCTCGTGCCCCTAAACCACTG
GAAAACACTGAAGCAGCACAGGCACAGCCGTCTGGAAGCAAATTCTCTCCGGCCATTCCTGTTTCTACCCAGGAG
TCCGTTTCTGTTCCAGCAAGTGTGAGCACCAGCATTAGCAGTATTAGCACCGGTGCCACCGCTAGCGCCCTGGTT
AAAGGCAATACCAATCCGATTACAAGCATGTCTGCCCCGGTTCAAGCATCAGCTCCAGCACTGACAAAATCCCAA
ACCGATCGTCTGGAGGTTCTGCTGAATCCGAAAGACGAAATCAGCCTGAATTCCGGCAAACCGTTTCGTGAACTG
GAGAGCGAACTGCTGTCACGTCGTAAAAAAGACCTGCAACAAATCTATGCCGAAGAACGTGAGAACTATCTGGGG
AAACTGGAACGTGAAATCACCCGCTTTTTCGTGGATCGTGGCTTTCTGGAGATCAAATCCCCGATTCTGATTCCT
CTGGAGTATATCGAGCGTATGGGCATCGACAATGATACCGAACTGAGCAAACAAATTTTCCGTGTGGATAAAAAC
TTCTGTCTGCGCCCTATGCTAGCACCAAATCTGGCTAACTATCTGCGCAAACTGGACCGTGCCCTGCCTGATCCT
ATCAAAATCTTCGAGATCGGCCCGTGTTATCGTAAAGAGTCCGACGGTAAAGAACATCTGGAGGAGTTTACCATG
CTGAACTTTTGCCAAATGGGTTCAGGTTGTACTCGTGAGAACCTGGAAAGCATCATCACCGATTTTCTGAACCAC
CTGGGCATTGACTTCAAAATTGTGGGCGACAGCTGTATGGTGTTTGGCGACACCCTGGATGTCATGCACGGCGAC
CTGGAACTGTCTAGTGCCGTTGTGGGCCCAATCCCGCTGGATCGTGAGTGGGGTATCGACAAACCTTGGATCGGT
GCGGGTTTTGGTCTGGAGCGTCTGCTGAAAGTAAAACACGACTTCAAGAACATCAAACGTGCTGCACGTTCCGAG
TCCTATTACAATGGTATTTCTACTAACCTGTAA (SEQ ID NO: 591)

Protein:
MVGRNSAIAAGVCGALFIGYCIYFDRKRRSDPNFKNRLRERRKKQKLAKERAGLSKLPDLKDAEAVQKFFMASND
YTQQATQSYGAYPTQPGQGYSQQSSQPYGQQSYSGYSQSTDTSGYGQSSYSSYGQSQNTGYGTQSTPQGYGSTGG
YGSSQSSQSSYGQQSSYPGYGQQPAPSSTSGSYGSSSQSSSYGQPQSGSYSQQPSYGGQQQSYGQQQSYNPPQGY
GQQNQYNSSSGGGGGGGGGGNYGQDQSSMSSGGGSGGGYGNQDQSGGGGSGGYGQQDRGGRGRGGSGGGGGGGG
GYNRSSGGYEPRGRGGGRGGRGGMGGSDRGGFNKFGGPRDQGSRHDSEQDNSDNNTIFVQGLGENVTIESVADYF
KQIGIIKTNKKTGQPMINLYTDRETGKLKGEATVSFDDPPSAKAAIDWFDGKEFSGNPIKVSFATRRADFNRGGG
NGRGGRGRGGPMGRGGYGGGGSGGGGRGGFPSGGGGGGGQQRAGDWKCPNPTCENMNFSWRNECNQCKAPKPDGP
GGGPGGSHMGGNYGDDRRGGRGGAIAARNAYLRKKIARLKKDNLQLERDEQNLEKIIANLRDEIARLENEVASHE
QASIYPYDVPDYAGAPGSAGSAAGSGASNFTQFVLVDNGGTGDVTVAPSNFANGIAEWISSNSRSQAYKVTCSVR
QSSAQNRKYTIKVEVPKGAWRSYLNMELTIPIFATNSDCELIVKAMQGLLKDGNPIPSAIAANSGIYGADYKDDD
DKGAPGSAGSAAGSGACPVPLQLPPLERLTLDDKKPLNTLISATGLWMSRTGTIHKIKHHEVSRSKIYIEMACGD
HLVVNNSRSSRTARALRHHKYRKTCKRCRVSDEDLNKFLTKANEDQTSVKVKVVSAPTRTKKAMPKSVARAPKPL
ENTEAAQAQPSGSKFSPAIPVSTQESVSVPASVSTSISSISTGATASALVKGNTNPITSMSAPVQASAPALTKSQ
TDRLEVLLNPKDEISLNSGKPFRELESELLSRRKKDLQQIYAEERENYLGKLEREITRFFVDRGFLEIKSPILIP
LEYIERMGIDNDTELSKQIFRVDKNFCLRPMLAPNLANYLRKLDRALPDPIKIFEIGPCYRKESDGKEHLEEFTM
LNFCQMGSGCTRENLESIITDFLNHLGIDFKIVGDSCMVFGDTLDVMHGDLELSSAVVGPIPLDREWGIDKPWIG
AGFGLERLLKVKHDFKNIKRAARSESYYNGISTNL* (SEQ ID NO: 592)

TOM20::FUS::SYNZIP1::MCP::PylRS(AA)
DNA:
ATGGTGGGTCGGAACAGCGCCATCGCCGCCGGTGTATGCGGGGCCCTTTTCATTGGGTACTGCATCTACTTCGAC
CGCAAAAGACGAAGTGACCCCAACTTCAAGAACAGGCTTCGAGAACGAAGAAAGAAACAGAAGCTTGCCAAGGAG
AGAGCTGGGCTTTCCAAGTTACCTGACCTTAAAGATGCTGAAGCTGTTCAGAAATTCTTCATGGCCTCAAACGAT
TATACCCAACAAGCAACCCAAAGCTATGGGGCCTACCCCACCCAGCCCGGGCAGGGCTATTCCCAGCAGAGCAGT
CAGCCCTACGGACAGCAGAGTTACAGTGGTTATAGCCAGTCCACGGACACTTCAGGATATGGCCAGAGCAGCTAT
TCTTCTTATGGCCAGAGCCAGAACACAGGCTATGGAACTCAGTCAACTCCCCAGGGATATGGCTCGACTGGCGGC
TATGGCAGTAGCCAGAGCTCCCAATCGTCTTACGGGCAGCAGTCCTCCTACCCTGGCTATGGCCAGCAGCCAGCT
CCCAGCAGCACCTCGGGAAGTTACGGTAGCAGTTCTCAGAGCAGCAGCTATGGGCAGCCCCAGAGTGGGAGCTAC
AGCCAGCAGCCTAGCTATGGTGGACAGCAGCAAAGCTATGGACAGCAGCAAAGCTATAATCCCCCTCAGGGCTAT
GGACAGCAGAACCAGTACAACAGCAGCAGTGGTGGTGGAGGTGGAGGTGGAGGTGGAGGTAACTATGGCCAAGAT
CAATCCTCCATGAGTAGTGGTGGTGGCAGTGGTGGCGGTTATGGCAATCAAGACCAGAGTGGTGGAGGTGGCAGC

GGTGGCTATGGACAGCAGGACCGTGGAGGCCGCGGCAGGGGTGGCAGTGGTGGCGGCGGCGGCGGCGGTGGT
GGTTACAACCGCAGCAGTGGTGGCTATGAACCCAGAGGTCGTGGAGGTGGCCGTGGAGGCAGAGGTGGCATGGGC
GGAAGTGACCGTGGTGGCTTCAATAAATTTGGTGGCCCTCGGGACCAAGGATCACGTCATGACTCCGAACAGGAT
AATTCAGACAACAACACCATCTTTGTGCAAGGCCTGGGTGAGAATGTTACAATTGAGTCTGTGGCTGATTACTTC
AAGCAGATTGGTATTATTAAGACAAACAAGAAAACGGGACAGCCCATGATTAATTTGTACACAGACAGGGAAACT
GGCAAGCTGAAGGGAGAGGCAACGGTCTCTTTTGATGACCCACCTTCAGCTAAAGCAGCTATTGACTGGTTTGAT
GGTAAAGAATTCTCCGGAAATCCTATCAAGGTCTCATTTGCTACTCGCCGGGCAGACTTTAATCGGGGTGGTGGC
AATGGTCGTGGAGGCCGAGGGCGAGGAGGACCCATGGGCCGTGGAGGCTATGGAGGTGGTGGCAGTGGTGGTGGT
GGCCGAGGAGGATTTCCCAGTGGAGGTGGTGGCGGTGGAGGACAGCAGCGAGCTGGTGACTGGAAGTGTCCTAAT
CCCACCTGTGAGAATATGAACTTCTCTTGGAGGAATGAATGCAACCAGTGTAAGGCCCCTAAACCAGATGGCCCA
GGAGGGGGACCAGGTGGCTCTCACATGGGGGGTAACTACGGGGATGATCGTCGTGGTGGCAGAGGAGGCGCGATC
GCAAATCTGGTGGCCCAGCTGGAAAACGAGGTGGCCAGCCTGGAAAACGAGAACGAAACCCTGAAGAAAAAGAAC
CTGCACAAGAAGGACCTGATCGCCTACCTGGAAAAGGAAATCGCCAACCTGAGAAAGAAGATCGAGGAAGCATCG
ATATATCCCTATGATGTGCCGGATTATGCTGGAGCACCAGGAAGTGCTGGTTCTGCTGCTGGTAGTGGAGCTTCT
AACTTTACTCAGTTCGTTCTCGTCGACAATGGCGGAACTGGCGACGTGACTGTCGCCCCAAGCAACTTCGCTAAC
GGGATCGCTGAATGGATCAGCTCTAACTCGCGTTCACAGGCTTACAAAGTAACCTGTAGCGTTCGTCAGAGCTCT
GCGCAGAATCGCAAATACACCATCAAAGTCGAGGTGCCTAAAGGCGCCTGGCGTTCGTACTTAAATATGGAACTA
ACCATTCCAATTTTCGCCACGAATTCCGACTGCGAGCTTATTGTTAAGGCAATGCAAGGTCTCCTAAAAGATGGA
AACCCGATTCCCTCAGCAATCGCAGCAAACTCCGGCATCTACGGCGCCGATTACAAGGACGATGATGACAAGGGA
GCACCAGGAAGTGCTGGTTCTGCTGCTGGTAGTGGAGCGTGCCCGGTGCCGCTGCAGCTGCCGCCGCTGGAACGC
CTGACCCTGGATGATAAAAAACCGCTGAATACCCTGATCTCTGCTACTGGTCTGTGGATGAGTCGTACCGGAACC
ATTCATAAAATCAAACACCACGAGGTTAGCCGTTCGAAAATCTATATTGAGATGGCGTGTGGCGATCATCTGGTT
GTGAACAATAGCCGCTCTTCTCGTACAGCACGTGCACTGCGTCACCACAAATATCGTAAAACCTGTAAACGTTGC
CGTGTGTCCGATGAGGATCTGAACAAATTCCTGACAAAAGCCAATGAGGACCAAACAAGCGTGAAAGTGAAAGTC
GTTAGCGCTCCTACCCGTACTAAAAAAGCAATGCCGAAATCCGTTGCTCGTGCCCCTAAACCACTGGAAAACACT
GAAGCAGCACAGGCACAGCCGTCTGGAAGCAAATTCTCTCCGGCCATTCCTGTTTCTACCCAGGAGTCCGTTTCT
GTTCCAGCAAGTGTGAGCACCAGCATTAGCAGTATTAGCACCGGTGCCACCGCTAGCGCCCTGGTTAAAGGCAAT
ACCAATCCGATTACAAGCATGTCTGCCCCGGTTCAAGCATCAGCTCCAGCACTGACAAAATCCCAAACCGATCGT
CTGGAGGTTCTGCTGAATCCGAAAGACGAAATCAGCCTGAATTCCGGCAAACCGTTTCGTGAACTGGAGAGCGAA
CTGCTGTCACGTCGTAAAAAAGACCTGCAACAAATCTATGCCGAAGAACGTGAGAACTATCTGGGGAAACTGGAA
CGTGAAATCACCCGCTTTTTCGTGGATCGTGGCTTTCTGGAGATCAAATCCCCGATTCTGATTCCTCTGGAGTAT
ATCGAGCGTATGGGCATCGACAATGATACCGAACTGAGCAAACAAATTTTCCGTGTGGATAAAAACTTCTGTCTG
CGCCCTATGCTGGCACCAAATCTGTATAACTATCTGCGCAAACTGGACCGTGCCCTGCCTGATCCTATCAAAATC
TTCGAGATCGGCCCGTGTTATCGTAAAGAGTCCGACGGTAAAGAACATCTGGAGGAGTTTACCATGCTGGCCTTT
GCCCAAATGGGTTCAGGTTGTACTCGTGAGAACCTGGAAAGCATCATCACCGATTTTCTGAACCACCTGGGCATT
GACTTCAAAATTGTGGGCGACAGCTGTATGGTGTATGGCGACACCCTGGATGTCATGCACGGCGACCTGGAACTG
TCTAGTGCCGTTGTTGGACCAATTCCGCTGGACCGTGAGTGGGGTATCGACAAACCGTGGATCGGAGCAGGATTC
GGTCTGGAACGCCTGCTGAAAGTGAAACACGACTTCAAAAACATCAAACGTGCCGCCCGTTCTGAATCGTATTAT
AACGGGATCTCTACGAACCTGTAA (SEQ ID NO: 593)

Protein:
MVGRNSAIAAGVCGALFIGYCIYFDRKRRSDPNFKNRLRERRKKQKLAKERAGLSKLPDLKDAEAVQKFFMASND
YTQQATQSYGAYPTQPGQGYSQQSSQPYGQQSYSGYSQSTDTSGYGQSSYSSYGQSQNTGYGTQSTPQGYGSTGG
YGSSQSSQSSYGQQSSYPGYGQQPAPSSTSGSYGSSSQSSSYGQPQSGSYSQQPSYGGQQQSYGQQQSYNPPQGY
GQQNQYNSSSGGGGGGGGGGNYGQDQSSMSSGGGSGGGYGNQDQSGGGGSGGYGQQDRGGRGRGGSGGGGGGGG
GYNRSSGGYEPRGRGGGRGGRGGMGGSDRGGFNKFGGPRDQGSRHDSEQDNSDNNTIFVQGLGENVTIESVADYF
KQIGIIKTNKKTGQPMINLYTDRETGKLKGEATVSFDDPPSAKAAIDWFDGKEFSGNPIKVSFATRRADFNRGGG
NGRGGRGRGGPMGRGGYGGGGSGGGGRGGFPSGGGGGGGQQRAGDWKCPNPTCENMNFSWRNECNQCKAPKPDGP
GGGPGGSHMGGNYGDDRRGGRGGAIANLVAQLENEVASLENENETLKKKNLHKKDLIAYLEKEIANLRKKIEEAS
IYPYDVPDYAGAPGSAGSAAGSGASNFTQFVLVDNGGTGDVTVAPSNFANGIAEWISSNSRSQAYKVTCSVRQSS
AQNRKYTIKVEVPKGAWRSYLNMELTIPIFATNSDCELIVKAMQGLLKDGNPIPSAIAANSGIYGADYKDDDDKG
APGSAGSAAGSGACPVPLQLPPLERLTLDDKKPLNTLISATGLWMSRTGTIHKIKHHEVSRSKIYIEMACGDHLV
VNNSRSSRTARALRHHKYRKTCKRCRVSDEDLNKFLTKANEDQTSVKVKVVSAPTRTKKAMPKSVARAPKPLENT
EAAQAQPSGSKFSPAIPVSTQESVSVPASVSTSISSISTGATASALVKGNTNPITSMSAPVQASAPALTKSQTDR
LEVLLNPKDEISLNSGKPFRELESELLSRRKKDLQQIYAEERENYLGKLEREITRFFVDRGFLEIKSPILIPLEY
IERMGIDNDTELSKQIFRVDKNFCLRPMLAPNLYNYLRKLDRALPDPIKIFEIGPCYRKESDGKEHLEEFTMLAF
AQMGSGCTRENLESIITDFLNHLGIDFKIVGDSCMVYGDTLDVMHGDLELSSAVVGPIPLDREWGIDKPWIGAGF
GLERLLKVKHDFKNIKRAARSESYYNGISTNL* (SEQ ID NO: 594)

TOM20::FUS::SYNZIP2::MCP::PylRS(AA)
DNA:
ATGGTGGGTCGGAACAGCGCCATCGCCGCCGGTGTATGCGGGGCCCTTTTCATTGGGTACTGCATCTACTTCGAC
CGCAAAAGACGAAGTGACCCCAACTTCAAGAACAGGCTTCGAGAACGAAGAAAGAAACAGAAGCTTGCCAAGGAG
AGAGCTGGGCTTTCCAAGTTACCTGACCTTAAAGATGCTGAAGCTGTTCAGAAATTCTTCATGGCCTCAAACGAT
TATACCCAACAAGCAACCCAAAGCTATGGGGCCTACCCCACCCAGCCCGGGCAGGGCTATTCCCAGCAGAGCAGT
CAGCCCTACGGACAGCAGAGTTACAGTGGTTATAGCCAGTCCACGGACACTTCAGGATATGGCCAGAGCAGCTAT
TCTTCTTATGGCCAGAGCCAGAACACAGGCTATGGAACTCAGTCAACTCCCCAGGGATATGGCTCGACTGGCGGC
TATGGCAGTAGCCAGAGCTCCCAATCGTCTTACGGGCAGCAGTCCTCCTACCCTGGCTATGGCCAGCAGCCAGCT
CCCAGCAGCACCTCGGGAAGTTACGGTAGCAGTTCTCAGAGCAGCAGCTATGGGCAGCCCCAGAGTGGGAGCTAC
AGCCAGCAGCCTAGCTATGGTGGACAGCAGCAAAGCTATGGACAGCAGCAAAGCTATAATCCCCCTCAGGGCTAT
GGACAGCAGAACCAGTACAACAGCAGCAGTGGTGGTGGAGGTGGAGGTGGAGGTGGAGGTAACTATGGCCAAGAT
CAATCCTCCATGAGTAGTGGTGGTGGCAGTGGTGGCGGTTATGGCAATCAAGACCAGAGTGGTGGAGGTGGCAGC
GGTGGCTATGGACAGCAGGACCGTGGAGGCCGCGGCAGGGGTGGCAGTGGTGGCGGCGGCGGCGGCGGCGGTGGT
GGTTACAACCGCAGCAGTGGTGGCTATGAACCCAGAGGTCGTGGAGGTGGCCGTGGAGGCAGAGGTGGCATGGGC
GGAAGTGACCGTGGTGGCTTCAATAAATTTGGTGGCCCTCGGGACCAAGGATCACGTCATGACTCCGAACAGGAT
AATTCAGACAACAACACCATCTTTGTGCAAGGCCTGGGTGAGAATGTTACAATTGAGTCTGTGGCTGATTACTTC
AAGCAGATTGGTATTATTAAGACAAACAAGAAAACGGGACAGCCCATGATTAATTTGTACACAGACAGGGAAACT
GGCAAGCTGAAGGGAGAGGCAACGGTCTCTTTTGATGACCCACCTTCAGCTAAAGCAGCTATTGACTGGTTTGAT
GGTAAAGAATTCTCCGGAAATCCTATCAAGGTCTCATTTGCTACTCGCCGGGCAGACTTTAATCGGGGTGGTGGC
AATGGTCGTGGAGGCCGAGGGCGAGGAGGACCCATGGGCCGTGGAGGCTATGGAGGTGGTGGCAGTGGTGGTGGT
GGCCGAGGAGGATTTCCCAGTGGAGGTGGTGGCGGTGGAGGACAGCAGCGAGCTGGTGACTGGAAGTGTCCTAAT
CCCACCTGTGAGAATATGAACTTCTCTTGGAGGAATGAATGCAACCAGTGTAAGGCCCCTAAACCAGATGGCCCA
GGAGGGGGACCAGGTGGCTCTCACATGGGGGGTAACTACGGGGATGATCGTCGTGGTGGCAGAGGAGGCGCGATC
GCAGCTAGAAACGCCTACCTGAGAAAGAAAATCGCCAGACTGAAGAAGGACAACCTGCAGCTGGAAAGAGACGAG
CAGAACCTGGAAAAGATCATCGCCAACCTCAGAGATGAGATCGCCAGACTGGAAAACGAGGTGGCCAGCCACGAG
CAGGCATCGATATATCCCTATGATGTGCCGGATTATGCTGGAGCACCAGGAAGTGCTGGTTCTGCTGCTGGTAGT
GGAGCTTCTAACTTTACTCAGTTCGTTCTCGTCGACAATGGCGGAACTGGCGACGTGACTGTCGCCCCAAGCAAC
TTCGCTAACGGGATCGCTGAATGGATCAGCTCTAACTCGCGTTCACAGGCTTACAAAGTAACCTGTAGCGTTCGT
CAGAGCTCTGCGCAGAATCGCAAATACACCATCAAAGTCGAGGTGCCTAAAGGCGCCTGGCGTTCGTACTTAAAT
ATGGAACTAACCATTCCAATTTTCGCCACGAATTCCGACTGCGAGCTTATTGTTAAGGCAATGCAAGGTCTCCTA
AAAGATGGAAACCCGATTCCCTCAGCAATCGCAGCAAACTCCGGCATCTACGGCGCCGATTACAAGGACGATGAT
GACAAGGGAGCACCAGGAAGTGCTGGTTCTGCTGCTGGTAGTGGAGCGTGCCCGGTGCCGCTGCAGCTGCCGCCG
CTGGAACGCCTGACCCTGGATGATAAAAAACCGCTGAATACCCTGATCTCTGCTACTGGTCTGTGGATGAGTCGT
ACCGGAACCATTCATAAAATCAAACACCACGAGGTTAGCCGTTCGAAAATCTATATTGAGATGGCGTGTGGCGAT
CATCTGGTTGTGAACAATAGCCGCTCTTCTCGTACAGCACGTGCACTGCGTCACCACAAATATCGTAAAACCTGT
AAACGTTGCCGTGTGTCCGATGAGGATCTGAACAAATTCCTGACAAAAGCCAATGAGGACCAAACAAGCGTGAAA
GTGAAAGTCGTTAGCGCTCCTACCCGTACTAAAAAAGCAATGCCGAAATCCGTTGCTCGTGCCCCTAAACCACTG
GAAAACACTGAAGCAGCACAGGCACAGCCGTCTGGAAGCAAATTCTCTCCGGCCATTCCTGTTTCTACCCAGGAG
TCCGTTTCTGTTCCAGCAAGTGTGAGCACCAGCATTAGCAGTATTAGCACCGGTGCCACCGCTAGCGCCCTGGTT
AAAGGCAATACCAATCCGATTACAAGCATGTCTGCCCCGGTTCAAGCATCAGCTCCAGCACTGACAAAATCCCAA
ACCGATCGTCTGGAGGTTCTGCTGAATCCGAAAGACGAAATCAGCCTGAATTCCGGCAAACCGTTTCGTGAACTG
GAGAGCGAACTGCTGTCACGTCGTAAAAAAGACCTGCAACAAATCTATGCCGAAGAACGTGAGAACTATCTGGGG
AAACTGGAACGTGAAATCACCCGCTTTTTCGTGGATCGTGGCTTTCTGGAGATCAAATCCCCGATTCTGATTCCT
CTGGAGTATATCGAGCGTATGGGCATCGACAATGATACCGAACTGAGCAAACAAATTTTCCGTGTGGATAAAAAC
TTCTGTCTGCGCCCTATGCTGGCACCAAATCTGTATAACTATCTGCGCAAACTGGACCGTGCCCTGCCTGATCCT
ATCAAAATCTTCGAGATCGGCCCGTGTTATCGTAAAGAGTCCGACGGTAAAGAACATCTGGAGGAGTTTACCATG
CTGGCCTTTGCCCAAATGGGTTCAGGTTGTACTCGTGAGAACCTGGAAAGCATCATCACCGATTTTCTGAACCAC
CTGGGCATTGACTTCAAAATTGTGGGCGACAGCTGTATGGTGTATGGCGACACCCTGGATGTCATGCACGGCGAC
CTGGAACTGTCTAGTGCCGTTGTTGGACCAATTCCGCTGGACCGTGAGTGGGGTATCGACAAACCGTGGATCGGA
GCAGGATTCGGTCTGGAACGCCTGCTGAAAGTGAAACACGACTTCAAAAACATCAAACGTGCCGCCCGTTCTGAA
TCGTATTATAACGGGATCTCTACGAACCTGTAA (SEQ ID NO: 595)

Protein:
MVGRNSAIAAGVCGALFIGYCIYFDRKRRSDPNFKNRLRERRKKQKLAKERAGLSKLPDLKDAEAVQKFFMASND
YTQQATQSYGAYPTQPGQGYSQQSSQPYGQQSYSGYSQSTDTSGYGQSSYSSYGQSQNTGYGTQSTPQGYGSTGG

YGSSQSSQSSYGQQSSYPGYGQQPAPSSTSGSYGSSSQSSSYGQPQSGSYSQQPSYGGQQQSYGQQQSYNPPQGY
GQQNQYNSSSGGGGGGGGGGNYGQDQSSMSSGGGSGGGYGNQDQSGGGGSGGYGQQDRGGRGRGGSGGGGGGGG
GYNRSSGGYEPRGRGGGRGGRGGMGGSDRGGFNKFGGPRDQGSRHDSEQDNSDNNTIFVQGLGENVTIESVADYF
KQIGIIKTNKKTGQPMINLYTDRETGKLKGEATVSFDDPPSAKAAIDWFDGKEFSGNPIKVSFATRRADFNRGGG
NGRGGRGRGGPMGRGGYGGGGSGGGGRGGFPSGGGGGGGQQRAGDWKCPNPTCENMNFSWRNECNQCKAPKPDGP
GGGPGGSHMGGNYGDDRRGGRGGAIAARNAYLRKKIARLKKDNLQLERDEQNLEKIIANLRDEIARLENEVASHE
QASIYPYDVPDYAGAPGSAGSAAGSGASNFTQFVLVDNGGTGDVTVAPSNFANGIAEWISSNSRSQAYKVTCSVR
QSSAQNRKYTIKVEVPKGAWRSYLNMELTIPIFATNSDCELIVKAMQGLLKDGNPIPSAIAANSGIYGADYKDDD
DKGAPGSAGSAAGSGACPVPLQLPPLERLTLDDKKPLNTLISATGLWMSRTGTIHKIKHHEVSRSKIYIEMACGD
HLVVNNSRSSRTARALRHHKYRKTCKRCRVSDEDLNKFLTKANEDQTSVKVKVVSAPTRTKKAMPKSVARAPKPL
ENTEAAQAQPSGSKFSPAIPVSTQESVSVPASVSTSISSISTGATASALVKGNTNPITSMSAPVQASAPALTKSQ
TDRLEVLLNPKDEISLNSGKPFRELESELLSRRKKDLQQIYAEERENYLGKLEREITRFFVDRGFLEIKSPILIP
LEYIERMGIDNDTELSKQIFRVDKNFCLRPMLAPNLYNYLRKLDRALPDPIKIFEIGPCYRKESDGKEHLEEFTM
LAFAQMGSGCTRENLESIITDFLNHLGIDFKIVGDSCMVYGDTLDVMHGDLELSSAVVGPIPLDREWGIDKPWIG
AGFGLERLLKVKHDFKNIKRAARSESYYNGISTNL* (SEQ ID NO: 596)

TOM20::FUS::SYNZIP::MCP::IFRS1
DNA:
ATGGTGGGTCGGAACAGCGCCATCGCCGCCGGTGTATGCGGGGCCCTTTTCATTGGGTACTGCATCTACTTCGAC
CGCAAAAGACGAAGTGACCCCAACTTCAAGAACAGGCTTCGAGAACGAAGAAAGAAACAGAAGCTTGCCAAGGAG
AGAGCTGGGCTTTCCAAGTTACCTGACCTTAAAGATGCTGAAGCTGTTCAGAAATTCTTCATGGCCTCAAACGAT
TATACCCAACAAGCAACCCAAAGCTATGGGGCCTACCCCACCCAGCCCGGGCAGGGCTATTCCCAGCAGAGCAGT
CAGCCCTACGGACAGCAGAGTTACAGTGGTTATAGCCAGTCCACGGACACTTCAGGATATGGCCAGAGCAGCTAT
TCTTCTTATGGCCAGAGCCAGAACACAGGCTATGGAACTCAGTCAACTCCCCAGGGATATGGCTCGACTGGCGGC
TATGGCAGTAGCCAGAGCTCCCAATCGTCTTACGGGCAGCAGTCCTCCTACCCTGGCTATGGCCAGCAGCCAGCT
CCCAGCAGCACCTCGGGAAGTTACGGTAGCAGTTCTCAGAGCAGCAGCTATGGGCAGCCCCAGAGTGGGAGCTAC
AGCCAGCAGCCTAGCTATGGTGGACAGCAGCAAAGCTATGGACAGCAGCAAAGCTATAATCCCCCTCAGGGCTAT
GGACAGCAGAACCAGTACAACAGCAGCAGTGGTGGTGGAGGTGGAGGTGGAGGTGGAGGTAACTATGGCCAAGAT
CAATCCTCCATGAGTAGTGGTGGTGGCAGTGGTGGCGGTTATGGCAATCAAGACCAGAGTGGTGGAGGTGGCAGC
GGTGGCTATGGACAGCAGGACCGTGGAGGCCGCGGCAGGGGTGGCAGTGGTGGCGGCGGCGGCGGCGGTGGT
GGTTACAACCGCAGCAGTGGTGGCTATGAACCCAGAGGTCGTGGAGGTGGCCGTGGAGGCAGAGGTGGCATGGGC
GGAAGTGACCGTGGTGGCTTCAATAAATTTGGTGGCCCTCGGGACCAAGGATCACGTCATGACTCCGAACAGGAT
AATTCAGACAACAACACCATCTTTGTGCAAGGCCTGGGTGAGAATGTTACAATTGAGTCTGTGGCTGATTACTTC
AAGCAGATTGGTATTATTAAGACAAACAAGAAAACGGGACAGCCCATGATTAATTTGTACACAGACAGGGAAACT
GGCAAGCTGAAGGGAGAGGCAACGGTCTCTTTTGATGACCCACCTTCAGCTAAAGCAGCTATTGACTGGTTTGAT
GGTAAAGAATTCTCCGGAAATCCTATCAAGGTCTCATTTGCTACTCGCCGGGCAGACTTTAATCGGGGTGGTGGC
AATGGTCGTGGAGGCCGAGGGCGAGGAGGACCCATGGGCCGTGGAGGCTATGGAGGTGGTGGCAGTGGTGGTGGT
GGCCGAGGAGGATTTCCCAGTGGAGGTGGTGGCGGTGGAGGACAGCAGCGAGCTGGTGACTGGAAGTGTCCTAAT
CCCACCTGTGAGAATATGAACTTCTCTTGGAGGAATGAATGCAACCAGTGTAAGGCCCCTAAACCAGATGGCCCA
GGAGGGGGACCAGGTGGCTCTCACATGGGGGGTAACTACGGGGATGATCGTCGTGGTGGCAGAGGAGGCGCGATC
GCAAATCTGGTGGCCCAGCTGGAAAACGAGGTGGCCAGCCTGGAAAACGAGAACGAAACCCTGAAGAAAAAGAAC
CTGCACAAGAAGGACCTGATCGCCTACCTGGAAAAGGAAATCGCCAACCTGAGAAAGAAGATCGAGGAAGCATCG
ATATATCCCTATGATGTGCCGGATTATGCTGGAGCACCAGGAAGTGCTGGTTCTGCTGCTGGTAGTGGAGCTTCT
AACTTTACTCAGTTCGTTCTCGTCGACAATGGCGGAACTGGCGACGTGACTGTCGCCCCAAGCAACTTCGCTAAC
GGGATCGCTGAATGGATCAGCTCTAACTCGCGTTCACAGGCTTACAAAGTAACCTGTAGCGTTCGTCAGAGCTCT
GCGCAGAATCGCAAATACACCATCAAAGTCGAGGTGCCTAAAGGCGCCTGGCGTTCGTACTTAAATATGGAACTA
ACCATTCCAATTTTCGCCACGAATTCCGACTGCGAGCTTATTGTTAAGGCAATGCAAGGTCTCCTAAAAGATGGA
AACCCGATTCCCTCAGCAATCGCAGCAAACTCCGGCATCTACGGCGCCGATTACAAGGACGATGATGACAAGGGA
GCACCAGGAAGTGCTGGTTCTGCTGCTGGTAGTGGAGCGTGCCCGGTGCCGCTGCAGCTGCCGCCGCTGGAACGC
CTGACCCTGGATGATAAAAAACCGCTGAATACCCTGATCTCTGCTACTGGTCTGTGGATGAGTCGTACCGGAACC
ATTCATAAAATCAAACACCACGAGGTTAGCCGTTCGAAAATCTATATTGAGATGGCGTGTGGCGATCATCTGGTT
GTGAACAATAGCCGCTCTTCTCGTACAGCACGTGCACTGCGTCACCACAAATATCGTAAAACCTGTAAACGTTGC
CGTGTGTCCGATGAGGATCTGAACAAATTCCTGACAAAAGCCAATGAGGACCAAACAAGCGTGAAAGTGAAAGTC
GTTAGCGCTCCTACCCGTACTAAAAAAGCAATGCCGAAATCCGTTGCTCGTGCCCCTAAACCACTGGAAAACACT
GAAGCAGCACAGGCACAGCCGTCTGGAAGCAAATTCTCTCCGGCCATTCCTGTTTCTACCCAGGAGTCCGTTTCT
GTTCCAGCAAGTGTGAGCACCAGCATTAGCAGTATTAGCACCGGTGCCACCGCTAGCGCCCTGGTTAAAGGCAAT
ACCAATCCGATTACAAGCATGTCTGCCCCGGTTCAAGCATCAGCTCCAGCACTGACAAAATCCCAAACCGATCGT
CTGGAGGTTCTGCTGAATCCGAAAGACGAAATCAGCCTGAATTCCGGCAAACCGTTTCGTGAACTGGAGAGCGAA

CTGCTGTCACGTCGTAAAAAAGACCTGCAACAAATCTATGCCGAAGAACGTGAGAACTATCTGGGGAAACTGGAA
CGTGAAATCACCCGCTTTTTCGTGGATCGTGGCTTTCTGGAGATCAAATCCCCGATTCTGATTCCTCTGGAGTAT
ATCGAGCGTATGGGCATCGACAATGATACCGAACTGAGCAAACAAATTTTCCGTGTGGATAAAAACTTCTGTCTG
CGCCCTATGCTTGCACCAAATATGCTGAACTATAGCCGCAAACTGGACCGTGCCCTGCCTGATCCTATCAAAATC
TTCGAGATCGGCCCGTGTTATCGTAAAGAGTCCGACGGTAAAGAACATCTGGAGGAGTTTACCATGCTGTCTTTT
ATGCAAATGGGTTCAGGTTGTACTCGTGAGAACCTGGAAAGCATCATCACCGATTTTCTGAACCACCTGGGCATT
GACTTCAAAATTGTGGGCGACAGCTGTATGGTGTATGGCGACACCCTGGATGTCATGCACGGCGACCTGGAACTG
TCTAGTGCCGTTGTTGGACCAATTCCGCTGGACCGTGAGTGGGGTATCGACAAACCGTGGATCGGAGCAGGATTC
GGTCTGGAACGCCTGCTGAAAGTGAAACACGACTTCAAAAACATCAAACGTGCCGCCCGTTCTGAATCGTATTAT
AACGGGATCTCTACGAACCTGTAA (SEQ ID NO: 597)

Protein:
MVGRNSAIAAGVCGALFIGYCIYFDRKRRSDPNFKNRLRERRKKQKLAKERAGLSKLPDLKDAEAVQKFFMASND
YTQQATQSYGAYPTQPGQGYSQQSSQPYGQQSYSGYSQSTDTSGYGQSSYSSYGQSQNTGYGTQSTPQGYGSTGG
YGSSQSSQSSYGQQSSYPGYGQQPAPSSTSGSYGSSSQSSSYGQPQSGSYSQQPSYGGQQQSYGQQQSYNPPQGY
GQQNQYNSSSGGGGGGGGGGNYGQDQSSMSSGGGSGGGYGNQDQSGGGGSGGYGQQDRGGRGRGGSGGGGGGGGG
GYNRSSGGYEPRGRGGGRGGRGGMGGSDRGGFNKFGGPRDQGSRHDSEQDNSDNNTIFVQGLGENVTIESVADYF
KQIGIIKTNKKTGQPMINLYTDRETGKLKGEATVSFDDPPSAKAAIDWFDGKEFSGNPIKVSFATRRADFNRGGG
NGRGGRGRGGPMGRGGYGGGGSGGGGRGGFPSGGGGGGGQQRAGDWKCPNPTCENMNFSWRNECNQCKAPKPDGP
GGGPGGSHMGGNYGDDRRGGRGGAIANLVAQLENEVASLENENETLKKKNLHKKDLIAYLEKEIANLRKKIEEAS
IYPYDVPDYAGAPGSAGSAAGSGASNFTQFVLVDNGGTGDVTVAPSNFANGIAEWISSNSRSQAYKVTCSVRQSS
AQNRKYTIKVEVPKGAWRSYLNMELTIPIFATNSDCELIVKAMQGLLKDGNPIPSAIAANSGIYGADYKDDDDKG
APGSAGSAAGSGACPVPLQLPPLERLTLDDKKPLNTLISATGLWMSRTGTIHKIKHHEVSRSKIYIEMACGDHLV
VNNSRSSRTARALRHHKYRKTCKRCRVSDEDLNKFLTKANEDQTSVKVKVVSAPTRTKKAMPKSVARAPKPLENT
EAAQAQPSGSKFSPAIPVSTQESVSVPASVSTSISSISTGATASALVKGNTNPITSMSAPVQASAPALTKSQTDR
LEVLLNPKDEISLNSGKPFRELESELLSRRKKDLQQIYAEERENYLGKLEREITRFFVDRGFLEIKSPILIPLEY
IERMGIDNDTELSKQIFRVDKNFCLRPMLAPNMLNYSRKLDRALPDPIKIFEIGPCYRKESDGKEHLEEFTMLSF
MQMGSGCTRENLESIITDFLNHLGIDFKIVGDSCMVYGDTLDVMHGDLELSSAVVGPIPLDREWGIDKPWIGAGF
GLERLLKVKHDFKNIKRAARSESYYNGISTNL* (SEQ ID NO: 598)

TOM20::FUS::SYNZIP2::MCP::IFRS1
DNA:
ATGGTGGGTCGGAACAGCGCCATCGCCGCCGGTGTATGCGGGGCCCTTTTCATTGGGTACTGCATCTACTTCGAC
CGCAAAAGACGAAGTGACCCCAACTTCAAGAACAGGCTTCGAGAACGAAGAAAGAAACAGAAGCTTGCCAAGGAG
AGAGCTGGGCTTTCCAAGTTACCTGACCTTAAAGATGCTGAAGCTGTTCAGAAATTCTTCATGGCCTCAAACGAT
TATACCCAACAAGCAACCCAAAGCTATGGGGCCTACCCCACCCAGCCCGGGCAGGGCTATTCCCAGCAGAGCAGT
CAGCCCTACGGACAGCAGAGTTACAGTGGTTATAGCCAGTCCACGGACACTTCAGGATATGGCCAGAGCAGCTAT
TCTTCTTATGGCCAGAGCCAGAACACAGGCTATGGAACTCAGTCAACTCCCCAGGGATATGGCTCGACTGGCGGC
TATGGCAGTAGCCAGAGCTCCCAATCGTCTTACGGGCAGCAGTCCTCCTACCCTGGCTATGGCCAGCAGCCAGCT
CCCAGCAGCACCTCGGGAAGTTACGGTAGCAGTTCTCAGAGCAGCAGCTATGGGCAGCCCCAGAGTGGGAGCTAC
AGCCAGCAGCCTAGCTATGGTGGACAGCAGCAAAGCTATGGACAGCAGCAAAGCTATAATCCCCCTCAGGGCTAT
GGACAGCAGAACCAGTACAACAGCAGCAGTGGTGGTGGAGGTGGAGGTGGAGGTGGAGGTAACTATGGCCAAGAT
CAATCCTCCATGAGTAGTGGTGGTGGCAGTGGTGGCGGTTATGGCAATCAAGACCAGAGTGGTGGAGGTGGCAGC
GGTGGCTATGGACAGCAGGACCGTGGAGGCCGCGGCAGGGGTGGCAGTGGTGGCGGCGGCGGCGGCGGCGGTGGT
GGTTACAACCGCAGCAGTGGTGGCTATGAACCCAGAGGTCGTGGAGGTGGCCGTGGAGGCAGAGGTGGCATGGGC
GGAAGTGACCGTGGTGGCTTCAATAAATTTGGTGGCCCTCGGGACCAAGGATCACGTCATGACTCCGAACAGGAT
AATTCAGACAACAACACCATCTTTGTGCAAGGCCTGGGTGAGAATGTTACAATTGAGTCTGTGGCTGATTACTTC
AAGCAGATTGGTATTATTAAGACAAACAAGAAAACGGGACAGCCCATGATTAATTTGTACACAGACAGGGAAACT
GGCAAGCTGAAGGGAGAGGCAACGGTCTCTTTTGATGACCCACCTTCAGCTAAAGCAGCTATTGACTGGTTTGAT
GGTAAAGAATTCTCCGGAAATCCTATCAAGGTCTCATTTGCTACTCGCCGGGCAGACTTTAATCGGGGTGGTGGC
AATGGTCGTGGAGGCCGAGGGCGAGGAGGACCCATGGGCCGTGGAGGCTATGGAGGTGGTGGCAGTGGTGGTGGT
GGCCGAGGAGGATTTCCCAGTGGAGGTGGTGGCGGTGGAGGACAGCAGCGAGCTGGTGACTGGAAGTGTCCTAAT
CCCACCTGTGAGAATATGAACTTCTCTTGGAGGAATGAATGCAACCAGTGTAAGGCCCCTAAACCAGATGGCCCA
GGAGGGGGACCAGGTGGCTCTCACATGGGGGGTAACTACGGGGATGATCGTCGTGGTGGCAGAGGAGGCGCGATC
GCAGCTAGAAACGCCTACCTGAGAAAGAAAATCGCCAGACTGAAGAAGGACAACCTGCAGCTGGAAAGAGACGAG
CAGAACCTGGAAAAGATCATCGCCAACCTCAGAGATGAGATCGCCAGACTGGAAAACGAGGTGGCCAGCCACGAG
CAGGCATCGATATATCCCTATGATGTGCCGGATTATGCTGGAGCACCAGGAAGTGCTGGTTCTGCTGCTGGTAGT

GGAGCTTCTAACTTTACTCAGTTCGTTCTCGTCGACAATGGCGGAACTGGCGACGTGACTGTCGCCCCAAGCAAC
TTCGCTAACGGGATCGCTGAATGGATCAGCTCTAACTCGCGTTCACAGGCTTACAAAGTAACCTGTAGCGTTCGT
CAGAGCTCTGCGCAGAATCGCAAATACACCATCAAAGTCGAGGTGCCTAAAGGCGCCTGGCGTTCGTACTTAAAT
ATGGAACTAACCATTCCAATTTTCGCCACGAATTCCGACTGCGAGCTTATTGTTAAGGCAATGCAAGGTCTCCTA
AAAGATGGAAACCCGATTCCCTCAGCAATCGCAGCAAACTCCGGCATCTACGGCGCCGATTACAAGGACGATGAT
GACAAGGGAGCACCAGGAAGTGCTGGTTCTGCTGCTGGTAGTGGAGCGTGCCCGGTGCCGCTGCAGCTGCCGCCG
CTGGAACGCCTGACCCTGGATGATAAAAAACCGCTGAATACCCTGATCTCTGCTACTGGTCTGTGGATGAGTCGT
ACCGGAACCATTCATAAAATCAAACACCACGAGGTTAGCCGTTCGAAAATCTATATTGAGATGGCGTGTGGCGAT
CATCTGGTTGTGAACAATAGCCGCTCTTCTCGTACAGCACGTGCACTGCGTCACCACAAATATCGTAAAACCTGT
AAACGTTGCCGTGTGTCCGATGAGGATCTGAACAAATTCCTGACAAAAGCCAATGAGGACCAAACAAGCGTGAAA
GTGAAAGTCGTTAGCGCTCCTACCCGTACTAAAAAAGCAATGCCGAAATCCGTTGCTCGTGCCCCTAAACCACTG
GAAAACACTGAAGCAGCACAGGCACAGCCGTCTGGAAGCAAATTCTCTCCGGCCATTCCTGTTTCTACCCAGGAG
TCCGTTTCTGTTCCAGCAAGTGTGAGCACCAGCATTAGCAGTATTAGCACCGGTGCCACCGCTAGCGCCCTGGTT
AAAGGCAATACCAATCCGATTACAAGCATGTCTGCCCCGGTTCAAGCATCAGCTCCAGCACTGACAAAATCCCAA
ACCGATCGTCTGGAGGTTCTGCTGAATCCGAAAGACGAAATCAGCCTGAATTCCGGCAAACCGTTTCGTGAACTG
GAGAGCGAACTGCTGTCACGTCGTAAAAAAGACCTGCAACAAATCTATGCCGAAGAACGTGAGAACTATCTGGGG
AAACTGGAACGTGAAATCACCCGCTTTTTCGTGGATCGTGGCTTTCTGGAGATCAAATCCCCGATTCTGATTCCT
CTGGAGTATATCGAGCGTATGGGCATCGACAATGATACCGAACTGAGCAAACAAATTTTCCGTGTGGATAAAAAC
TTCTGTCTGCGCCCTATGCTTGCACCAAATATGCTGAACTATAGCCGCAAACTGGACCGTGCCCTGCCTGATCCT
ATCAAAATCTTCGAGATCGGCCCGTGTTATCGTAAAGAGTCCGACGGTAAAGAACATCTGGAGGAGTTTACCATG
CTGTCTTTTATGCAAATGGGTTCAGGTTGTACTCGTGAGAACCTGGAAAGCATCATCACCGATTTTCTGAACCAC
CTGGGCATTGACTTCAAAATTGTGGGCGACAGCTGTATGGTGTATGGCGACACCCTGGATGTCATGCACGGCGAC
CTGGAACTGTCTAGTGCCGTTGTTGGACCAATTCCGCTGGACCGTGAGTGGGGTATCGACAAACCGTGGATCGGA
GCAGGATTCGGTCTGGAACGCCTGCTGAAAGTGAAACACGACTTCAAAAACATCAAACGTGCCGCCCGTTCTGAA
TCGTATTATAACGGGATCTCTACGAACCTGTAA (SEQ ID NO:599)

Protein:
MVGRNSAIAAGVCGALFIGYCIYFDRKRRSDPNFKNRLRERRKKQKLAKERAGLSKLPDLKDAEAVQKFFMASND
YTQQATQSYGAYPTQPGQGYSQQSSQPYGQQSYSGYSQSTDTSGYGQSSYSSYGQSQNTGYGTQSTPQGYGSTGG
YGSSQSSQSSYGQQSSYPGYGQQPAPSSTSGSYGSSSQSSSYGQPQSGSYSQQPSYGGQQQSYGQQQSYNPPQGY
GQQNQYNSSSGGGGGGGGGGNYGQDQSSMSSGGGSGGGYGNQDQSGGGGSGGYGQQDRGGRGRGGSGGGGGGGGG
GYNRSSGGYEPRGRGGGRGGRGGMGGSDRGGFNKFGGPRDQGSRHDSEQDNSDNNTIFVQGLGENVTIESVADYF
KQIGIIKTNKKTGQPMINLYTDRETGKLKGEATVSFDDPPSAKAAIDWFDGKEFSGNPIKVSFATRRADFNRGGG
NGRGGRGRGGPMGRGGYGGGGSGGGGRGGFPSGGGGGGGQQRAGDWKCPNPTCENMNFSWRNECNQCKAPKPDGP
GGGPGGSHMGGNYGDDRRGGRGGAIAARNAYLRKKIARLKKDNLQLERDEQNLEKIIANLRDEIARLENEVASHE
QASIYPYDVPDYAGAPGSAGSAAGSGASNFTQFVLVDNGGTGDVTVAPSNFANGIAEWISSNSRSQAYKVTCSVR
QSSAQNRKYTIKVEVPKGAWRSYLNMELTIPIFATNSDCELIVKAMQGLLKDGNPIPSAIAANSGIYGADYKDDD
DKGAPGSAGSAAGSGACPVPLQLPPLERLTLDDKKPLNTLISATGLWMSRTGTIHKIKHHEVSRSKIYIEMACGD
HLVVNNSRSSRTARALRHHKYRKTCKRCRVSDEDLNKFLTKANEDQTSVKVKVVSAPTRTKKAMPKSVARAPKPL
ENTEAAQAQPSGSKFSPAIPVSTQESVSVPASVSTSISSISTGATASALVKGNTNPITSMSAPVQASAPALTKSQ
TDRLEVLLNPKDEISLNSGKPFRELESELLSRRKKDLQQIYAEERENYLGKLEREITRFFVDRGFLEIKSPILIP
LEYIERMGIDNDTELSKQIFRVDKNFCLRPMLAPNMLNYSRKLDRALPDPIKIFEIGPCYRKESDGKEHLEEFTM
LSFMQMGSGCTRENLESIITDFLNHLGIDFKIVGDSCMVYGDTLDVMHGDLELSSAVVGPIPLDREWGIDKPWIG
AGFGLERLLKVKHDFKNIKRAARSESYYNGISTNL* (SEQ ID NO: 600)

TOM20::FUS::SYNZIP3::4xλN22::CbzRS
DNA:
ATGGTGGGTCGGAACAGCGCCATCGCCGCCGGTGTATGCGGGGCCCTTTTCATTGGGTACTGCATCTACTTCGAC
CGCAAAAGACGAAGTGACCCCAACTTCAAGAACAGGCTTCGAGAACGAAGAAAGAAACAGAAGCTTGCCAAGGAG
AGAGCTGGGCTTTCCAAGTTACCTGACCTTAAAGATGCTGAAGCTGTTCAGAAATTCTTCATGGCCTCAAACGAT
TATACCCAACAAGCAACCCAAAGCTATGGGGCCTACCCCACCCAGCCCGGGCAGGGCTATTCCCAGCAGAGCAGT
CAGCCCTACGGACAGCAGAGTTACAGTGGTTATAGCCAGTCCACGGACACTTCAGGATATGGCCAGAGCAGCTAT
TCTTCTTATGGCCAGAGCCAGAACACAGGCTATGGAACTCAGTCAACTCCCCAGGGATATGGCTCGACTGGCGGC
TATGGCAGTAGCCAGAGCTCCCAATCGTCTTACGGGCAGCAGTCCTCCTACCCTGGCTATGGCCAGCAGCCAGCT
CCCAGCAGCACCTCGGGAAGTTACGGTAGCAGTTCTCAGAGCAGCAGCTATGGGCAGCCCCAGAGTGGGAGCTAC
AGCCAGCAGCCTAGCTATGGTGGACAGCAGCAAAGCTATGGACAGCAGCAAAGCTATAATCCCCCTCAGGGCTAT
GGACAGCAGAACCAGTACAACAGCAGCAGTGGTGGTGGAGGTGGAGGTGGAGGTGGAGGTAACTATGGCCAAGAT

CAATCCTCCATGAGTAGTGGTGGTGGCAGTGGTGGCGGTTATGGCAATCAAGACCAGAGTGGTGGAGGTGGCAGC
GGTGGCTATGGACAGCAGGACCGTGGAGGCCGCGGCAGGGGTGGCAGTGGTGGCGGCGGCGGCGGCGGCGGTGGT
GGTTACAACCGCAGCAGTGGTGGCTATGAACCCAGAGGTCGTGGAGGTGGCCGTGGAGGCAGAGGTGGCATGGGC
GGAAGTGACCGTGGTGGCTTCAATAAATTTGGTGGCCCTCGGGACCAAGGATCACGTCATGACTCCGAACAGGAT
AATTCAGACAACAACACCATCTTTGTGCAAGGCCTGGGTGAGAATGTTACAATTGAGTCTGTGGCTGATTACTTC
AAGCAGATTGGTATTATTAAGACAAACAAGAAAACGGGACAGCCCATGATTAATTTGTACACAGACAGGGAAACT
GGCAAGCTGAAGGGAGAGGCAACGGTCTCTTTTGATGACCCACCTTCAGCTAAAGCAGCTATTGACTGGTTTGAT
GGTAAAGAATTCTCCGGAAATCCTATCAAGGTCTCATTTGCTACTCGCCGGGCAGACTTTAATCGGGGTGGTGGC
AATGGTCGTGGAGGCCGAGGGCGAGGAGGACCCATGGGCCGTGGAGGCTATGGAGGTGGTGGCAGTGGTGGTGGT
GGCCGAGGAGGATTTCCCAGTGGAGGTGGTGGCGGTGGAGGACAGCAGCGAGCTGGTGACTGGAAGTGTCCTAAT
CCCACCTGTGAGAATATGAACTTCTCTTGGAGGAATGAATGCAACCAGTGTAAGGCCCCTAAACCAGATGGCCCA
GGAGGGGGACCAGGTGGCTCTCACATGGGGGGTAACTACGGGGATGATCGTCGTGGTGGCAGAGGAGGCGCGATC
GCAAATGAGGTGACCACCCTGGAAAACGACGCCGCCTTCATCGAGAACGAGAACGCCTACCTGGAAAAAGAGATC
GCCAGACTGAGAAAGGAAAAGGCCGCTCTGCGGAACAGACTGGCCCACAAGAAGGGAGCACCAGGAAGTGCTGGT
TCTGCTGCTGGTAGTGGAGCATCGATAGAGCAGAAGCTGATCTCAGAGGAGGACCTGCTAGCCACCATGGACGCA
CAAACACGACGACGTGAGCGTCGCGCTGAGAAACAAGCTCAATGGAAAGCTGCAAACCCACCGCTCGACGGAGCC
GGAGCTGGCGCTGGAGCTGGAGCCGGAGCTGGCGGTCTAGCCACCATGGACGCACAAACACGACGACGTGAGCGT
CGCGCTGAGAAACAAGCTCAATGGAAAGCTGCAAACCCACCGCTCGACGGAGCCGGAGCTGGCGCTGGAGCTGGA
GCCGGAGCTGGCGGTCTAGCCACCATGGACGCACAAACACGACGACGTGAGCGTCGCGCTGAGAAACAAGCTCAA
TGGAAAGCTGCAAACCCACCGCTCGACGGAGCCGGAGCTGGCGCTGGAGCTGGAGCCGGAGCTGGCGGTCTAGCC
ACCATGGACGCACAAACACGACGACGTGAGCGTCGCGCTGAGAAACAAGCTCAATGGAAAGCTGCAAACCCACCG
CTCGAGTCTAGAGGGCCCGTTGGTGCTCCTGGTTCAGCAGGAAGCGCAGCAGGATCAGGTGCGTGCCCGGTGCCG
CTGCAGCTGCCGCCGCTGGAACGCCTGACCCTGGATGACAAAAAACCGCTGAATACCCTGATCTCTGCTACTGGT
CTGTGGATGAGTCGTACCGGAACCATTCATAAAATCAAACACCACGAGGTTAGCCGTTCGAAAATCTATATTGAG
ATGGCGTGTGGCGATCATCTGGTTGTGAACAATAGCCGCTCTTCTCGTACAGCACGTGCACTGCGTCACCACAAA
TATCGTAAAACCTGTAAACGTTGCCGTGTGTCCGATGAGGATCTGAACAAATTCCTGACAAAAGCCAATGAGGAC
CAAACAAGCGTGAAAGTGAAAGTCGTTAGCGCTCCTACCCGTACTAAAAAAGCAATGCCGAAATCCGTTGCTCGT
GCCCCTAAACCACTGGAAAACACTGAAGCAGCACAGGCACAGCCGTCTGGAAGCAAATTCTCTCCGGCCATTCCT
GTTTCTACCCAGGAGTCCGTTTCTGTTCCAGCAAGTGTGAGCACCAGCATTAGCAGTATTAGCACCGGTGCCACC
GCTAGCGCCCTGGTTAAAGGCAATACCAATCCGATTACAAGCATGTCTGCCCCGGTTCAAGCATCAGCTCCAGCA
CTGACAAAATCCCAAACCGATCGTCTGGAGGTTCTGCTGAATCCGAAAGACGAAATCAGCCTGAATTCCGGCAAA
CCGTTTCGTGAACTGGAGAGCGAACTGCTGTCACGTCGTAAAAAAGACCTGCAACAAATCTATGCCGAAGAACGT
GAGAACTATCTGGGGAAACTGGAACGTGAAATCACCCGCTTTTTCGTGGATCGTGGCTTTCTGGAGATCAAATCC
CCGATTCTGATTCCTCTGGAGTATATCGAGCGTATGGGCATCGACAATGATACCGAACTGAGCAAACAAATTTTC
CGTGTGGATAAAAACTTCTGTCTGCGCCCTATGCTTGCACCAAATCTGATGAACTATGGACGCAAACTGGACCGT
GCCCTGCCTGATCCTATCAAAATCTTCGAGATCGGCCCGTGTTATCGTAAAGAGTCCGACGGTAAAGAACATCTG
GAGGAGTTTACCATGCTGAACTTTACACAAATGGGTTCAGGTTGTACTCGTGAGAACCTGGAAAGCATCATCACC
GATTTTCTGAACCACCTGGGCATTGACTTCAAAATTGTGGGCGACAGCTGTATGGTGTATGGCGACACCCTGGAT
GTCATGCACGGCGACCTGGAACTGTCTAGTGCCGTTGTTGGACCAATTCCGCTGGACCGTGAGTGGGGTATCGAC
AAACCGTGGATCGGAGCAGGATTCGGTCTGGAACGCCTGCTGAAAGTGAAACACGACTTCAAAAACATCAAACGT
GCCGCCCGTTCTGAATCGTATTATAACGGGATCTCTACGAACCTGTAA (SEQ ID NO: 601)

Protein:
MVGRNSAIAAGVCGALFIGYCIYFDRKRRSDPNFKNRLRERRKKQKLAKERAGLSKLPDLKDAEAVQKFFMASND
YTQQATQSYGAYPTQPGQGYSQQSSQPYGQQSYSGYSQSTDTSGYGQSSYSSYGQSQNTGYGTQSTPQGYGSTGG
YGSSQSSQSSYGQQSSYPGYGQQPAPSSTSGSYGSSSQSSSYGQPQSGSYSQQPSYGGQQQSYGQQQSYNPPQGY
GQQNQYNSSSGGGGGGGGGGNYGQDQSSMSSGGGSGGGYGNQDQSGGGGSGGYGQQDRGGRGRGGSGGGGGGGGG
GYNRSSGGYEPRGRGGGRGGRGGMGGSDRGGFNKFGGPRDQGSRHDSEQDNSDNNTIFVQGLGENVTIESVADYF
KQIGIIKTNKKTGQPMINLYTDRETGKLKGEATVSFDDPPSAKAAIDWFDGKEFSGNPIKVSFATRRADFNRGGG
NGRGGRGRGGPMGRGGYGGGGSGGGGRGGFPSGGGGGGGQQRAGDWKCPNPTCENMNFSWRNECNQCKAPKPDGP
GGGPGGSHMGGNYGDDRRGGRGGAIANEVTTLENDAAFIENENAYLEKEIARLRKEKAALRNRLAHKKGAPGSAG
SAAGSGASIEQKLISEEDLLATMDAQTRRRERRAEKQAQWKAANPPLDGAGAGAGAGAGGLATMDAQTRRRER
RAEKQAQWKAANPPLDGAGAGAGAGAGGLATMDAQTRRRERRAEKQAQWKAANPPLDGAGAGAGAGAGGLA
TMDAQTRRRERRAEKQAQWKAANPPLESRGPVGAPGSAGSAAGSGACPVPLQLPPLERLTLDDKKPLNTLISATG
LWMSRTGTIHKIKHHEVSRSKIYIEMACGDHLVVNNSRSSRTARALRHHKYRKTCKRCRVSDEDLNKFLTKANED
QTSVKVKVVSAPTRTKKAMPKSVARAPKPLENTEAAQAQPSGSKFSPAIPVSTQESVSVPASVSTSISSISTGAT
ASALVKGNTNPITSMSAPVQASAPALTKSQTDRLEVLLNPKDEISLNSGKPFRELESELLSRRKKDLQQIYAEER
ENYLGKLEREITRFFVDRGFLEIKSPILIPLEYIERMGIDNDTELSKQIFRVDKNFCLRPMLAPNLMNYGRKLDR

ALPDPIKIFEIGPCYRKESDGKEHLEEFTMLNFTQMGSGCTRENLESIITDFLNHLGIDFKIVGDSCMVYGDTLD
VMHGDLELSSAVVGPIPLDREWGIDKPWIGAGFGLERLLKVKHDFKNIKRAARSESYYNGISTNL* (SEQ ID
NO: 602)

EBAG9₁₋₂₉::FUS::SYNZIP3::PCP::PylRS(AF)
DNA:
ATGGCCATCACCCAGTTTCGGTTATTTAAATTTTGTACCTGCCTAGCAACAGTATTCTCATTCCTAAAGAGATTA
ATATGCAGATCTGGAGCACCCGGCTCCGCCGGCTCCGCCGGCTCCGGCATGGCCTCAAACGATTATACCCAA
CAAGCAACCCAAAGCTATGGGGCCTACCCCACCCAGCCCGGGCAGGGCTATTCCCAGCAGAGCAGTCAGCCCTAC
GGACAGCAGAGTTACAGTGGTTATAGCCAGTCCACGGACACTTCAGGATATGGCCAGAGCAGCTATTCTTCTTAT
GGCCAGAGCCAGAACACAGGCTATGGAACTCAGTCAACTCCCCAGGGATATGGCTCGACTGGCGGCTATGGCAGT
AGCCAGAGCTCCCAATCGTCTTACGGGCAGCAGTCCTCCTACCCTGGCTATGGCCAGCAGCCAGCTCCCAGCAGC
ACCTCGGGAAGTTACGGTAGCAGTTCTCAGAGCAGCAGCTATGGGCAGCCCCAGAGTGGGAGCTACAGCCAGCAG
CCTAGCTATGGTGGACAGCAGCAAAGCTATGGACAGCAGCAAAGCTATAATCCCCCTCAGGGCTATGGACAGCAG
AACCAGTACAACAGCAGCAGTGGTGGTGGAGGTGGAGGTGGAGGTAACTATGGCCAAGATCAATCCTCC
ATGAGTAGTGGTGGTGGCAGTGGTGGCGGTTATGGCAATCAAGACCAGAGTGGTGGAGGTGGCAGCGGTGGCTAT
GGACAGCAGGACCGTGGAGGCCGCGGCAGGGGTGGCAGTGGTGGCGGCGGCGGCGGCGGTGGTGGTTACAAC
CGCAGCAGTGGTGGCTATGAACCCAGAGGTCGTGGAGGTGGCCGTGGAGGCAGAGGTGGCATGGGCGGAAGTGAC
CGTGGTGGCTTCAATAAATTTGGTGGCCCTCGGGACCAAGGATCACGTCATGACTCCGAACAGGATAATTCAGAC
AACAACACCATCTTTGTGCAAGGCCTGGGTGAGAATGTTACAATTGAGTCTGTGGCTGATTACTTCAAGCAGATT
GGTATTATTAAGACAAACAAGAAAACGGGACAGCCCATGATTAATTTGTACACAGACAGGGAAACTGGCAAGCTG
AAGGGAGAGGCAACGGTCTCTTTTGATGACCCACCTTCAGCTAAAGCAGCTATTGACTGGTTTGATGGTAAAGAA
TTCTCCGGAAATCCTATCAAGGTCTCATTTGCTACTCGCCGGGCAGACTTTAATCGGGGTGGTGGCAATGGTCGT
GGAGGCCGAGGGCGAGGAGGACCCATGGGCCGTGGAGGCTATGGAGGTGGTGGCAGTGGTGGTGGCCGAGGA
GGATTTCCCAGTGGAGGTGGTGGCGGTGGAGGACAGCAGCGAGCTGGTGACTGGAAGTGTCCTAATCCCACCTGT
GAGAATATGAACTTCTCTTGGAGGAATGAATGCAACCAGTGTAAGGCCCCTAAACCAGATGGCCCAGGAGGGGGA
CCAGGTGGCTCTCACATGGGGGGTAACTACGGGGATGATCGTCGTGGTGGCAGAGGTGGTGCGATCGCAAATGAG
GTGACCACCCTGGAAAACGACGCCGCCTTCATCGAGAACGAGAACGCCTACCTGGAAAAAGAGATCGCCAGACTG
AGAAAGGAAAAGGCCGCTCTGCGGAACAGACTGGCCCACAAGAAGGGAGCACCAGGAAGTGCTGGTTCTGCTGCT
GGTAGTGGAGCATCGATAGAGCAGAAGCTGATCTCAGAGGAGGACCTGATCGAAGGCCGCCATATGCTAGCCTCC
AAAACCATCGTTCTTTCGGTCGGCGAGGCTACTCGCACTCTGACTGAGATCCAGTCCACCGCAGACCGTCAGATC
TTCGAAGAGAAGGTCGGGCCTCTGGTGGGTCGGCTGCGCCTCACGGCTTCGCTCCGTCAAAACGGAGCCAAGACC
GCGTATCGCGTCAACCTAAAACTGGATCAGGCGGACGTCGTTGATTCCGGACTTCCGAAAGTGCGCTACACTCAG
GTATGGTCGCACGACGTGACAATCGTTGCGAATAGCACCGAGGCCTCGCGCAAATCGTTGTACGATTTGACCAAG
TCCCTCGTCGCGACCTCGCAGGTCGAAGATCTTGTCGTCAACCTTGTGCCGCTGGGCCGTGCGGATCCGCTAGCC
GGTGCTCCTGGTTCAGCAGGAAGCGCAGCAGGATCAGGTGCGTGCCCGGTGCCGCTGCAGCTGCCGCCGCTGGAA
CGCCTGACCCTGGATGATAAAAAACCGCTGAATACCCTGATCTCTGCTACTGGTCTGTGGATGAGTCGTACCGGA
ACCATTCATAAAATCAAACACCACGAGGTTAGCCGTTCGAAAATCTATATTGAGATGGCGTGTGGCGATCATCTG
GTTGTGAACAATAGCCGCTCTTCTCGTACAGCACGTGCACTGCGTCACCACAAATATCGTAAAACCTGTAAACGT
TGCCGTGTGTCCGATGAGGATCTGAACAAATTCCTGACAAAAGCCAATGAGGACCAAACAAGCGTGAAAGTGAAA
GTCGTTAGCGCTCCTACCCGTACTAAAAAAGCAATGCCGAAATCCGTTGCTCGTGCCCCTAAACCACTGGAAAAC
ACTGAAGCAGCACAGGCACAGCCGTCTGGAAGCAAATTCTCTCCGGCCATTCCTGTTTCTACCCAGGAGTCCGTT
TCTGTTCCAGCAAGTGTGAGCACCAGCATTAGCAGTATTAGCACCGGTGCCACCGCTAGCGCCCTGGTTAAAGGC
AATACCAATCCGATTACAAGCATGTCTGCCCCGGTTCAAGCATCAGCTCCAGCACTGACAAAATCCCAAACCGAT
CGTCTGGAGGTTCTGCTGAATCCGAAAGACGAAATCAGCCTGAATTCCGGCAAACCGTTTCGTGAACTGGAGAGC
GAACTGCTGTCACGTCGTAAAAAAGACCTGCAACAAATCTATGCCGAAGAACGTGAGAACTATCTGGGGAAACTG
GAACGTGAAATCACCCGCTTTTTCGTGGATCGTGGCTTTCTGGAGATCAAATCCCCGATTCTGATTCCTCTGGAG
TATATCGAGCGTATGGGCATCGACAATGATACCGAACTGAGCAAACAAATTTTCCGTGTGGATAAAAACTTCTGT
CTGCGCCCTATGCTAGCACCAAATCTGGCTAACTATCTGCGCAAACTGGACCGTGCCCTGCCTGATCCTATCAAA
ATCTTCGAGATCGGCCCGTGTTATCGTAAAGAGTCCGACGGTAAAGAACATCTGGAGGAGTTTACCATGCTGAAC
TTTTGCCAAATGGGTTCAGGTTGTACTCGTGAGAACCTGGAAAGCATCATCACCGATTTTCTGAACCACCTGGGC
ATTGACTTCAAAATTGTGGGCGACAGCTGTATGGTGTTTGGCGACACCCTGGATGTCATGCACGGCGACCTGGAA
CTGTCTAGTGCCGTTGTGGGCCCAATCCCGCTGGATCGTGAGTGGGGTATCGACAAACCTTGGATCGGTGCGGGT
TTTGGTCTGGAGCGTCTGCTGAAAGTAAAACACGACTTCAAGAACATCAAACGTGCTGCACGTTCCGAGTCCTAT
TACAATGGTATTTCTACTAACCTTTAA (SEQ ID NO: 603)

Protein:

MAITQFRLFKFCTCLATVFSFLKRLICRSGAPGSAGSAAGSGMASNDYTQQATQSYGAYPTQPGQGYSQQSSQPY
GQQSYSGYSQSTDTSGYGQSSYSSYGQSQNTGYGTQSTPQGYGSTGGYGSSQSSQSSYGQQSSYPGYGQQPAPSS
TSGSYGSSSQSSSYGQPQSGSYSQQPSYGGQQQSYGQQQSYNPPQGYGQQNQYNSSSGGGGGGGGGNYGQDQSS
MSSGGGSGGGYGNQDQSGGGGSGGYGQQDRGGRGRGGSGGGGGGGGGYNRSSGGYEPRGRGGGRGGRGGMGGSD
RGGFNKFGGPRDQGSRHDSEQDNSDNNTIFVQGLGENVTIESVADYFKQIGIIKTNKKTGQPMINLYTDRETGKL
KGEATVSFDDPPSAKAAIDWFDGKEFSGNPIKVSFATRRADFNRGGGNGRGGRGRGGPMGRGGYGGGGSGGGGRG
GFPSGGGGGGGQQRAGDWKCPNPTCENMNFSWRNECNQCKAPKPDGPGGGPGGSHMGGNYGDDRRGGRGGAIANE
VTTLENDAAFIENENAYLEKEIARLRKEKAALRNRLAHKKGAPGSAGSAAGSGASIEQKLISEEDLIEGRHMLAS
KTIVLSVGEATRTLTEIQSTADRQIFEEKVGPLVGRLRLTASLRQNGAKTAYRVNLKLDQADVVDSGLPKVRYTQ
VWSHDVTIVANSTEASRKSLYDLTKSLVATSQVEDLVVNLVPLGRADPLAGAPGSAGSAAGSGACPVPLQLPPLE
RLTLDDKKPLNTLISATGLWMSRTGTIHKIKHHEVSRSKIYIEMACGDHLVVNNSRSSRTARALRHHKYRKTCKR
CRVSDEDLNKFLTKANEDQTSVKVKVVSAPTRTKKAMPKSVARAPKPLENTEAAQAQPSGSKFSPAIPVSTQESV
SVPASVSTSISSISTGATASALVKGNTNPITSMSAPVQASAPALTKSQTDRLEVLLNPKDEISLNSGKPFRELES
ELLSRRKKDLQQIYAEERENYLGKLEREITRFFVDRGFLEIKSPILIPLEYIERMGIDNDTELSKQIFRVDKNFC
LRPMLAPNLANYLRKLDRALPDPIKIFEIGPCYRKESDGKEHLEEFTMLNFCQMGSGCTRENLESIITDFLNHLG
IDFKIVGDSCMVFGDTLDVMHGDLELSSAVVGPIPLDREWGIDKPWIGAGFGLERLLKVKHDFKNIKRAARSESY
YNGISTNL* (SEQ ID NO: 604)

EBAG9₁₋₂₉::FUS::SYNZIP4::PylRS(AF)
DNA:
ATGGCCATCACCCAGTTTCGGTTATTTAAATTTTGTACCTGCCTAGCAACAGTATTCTCATTCCTAAAGAGATTA
ATATGCAGATCTGGAGCACCCGGCTCCGCCGGCTCCGCCGCCGGCTCCGGCATGGCCTCAAACGATTATACCCAA
CAAGCAACCCAAAGCTATGGGGCCTACCCCACCCAGCCCGGGCAGGGCTATTCCCAGCAGAGCAGTCAGCCCTAC
GGACAGCAGAGTTACAGTGGTTATAGCCAGTCCACGGACACTTCAGGATATGGCCAGAGCAGCTATTCTTCTTAT
GGCCAGAGCCAGAACACAGGCTATGGAACTCAGTCAACTCCCCAGGGATATGGCTCGACTGGCGGCTATGGCAGT
AGCCAGAGCTCCCAATCGTCTTACGGGCAGCAGTCCTCCTACCCTGGCTATGGCCAGCAGCCAGCTCCCAGCAGC
ACCTCGGGAAGTTACGGTAGCAGTTCTCAGAGCAGCAGCTATGGGCAGCCCCAGAGTGGGAGCTACAGCCAGCAG
CCTAGCTATGGTGGACAGCAGCAAAGCTATGGACAGCAGCAAAGCTATAATCCCCCTCAGGGCTATGGACAGCAG
AACCAGTACAACAGCAGCAGTGGTGGTGGAGGTGGAGGTGGAGGTGGAGGTAACTATGGCCAAGATCAATCCTCC
ATGAGTAGTGGTGGTGGCAGTGGTGGCGGTTATGGCAATCAAGACCAGAGTGGTGGAGGTGGCAGCGGTGGCTAT
GGACAGCAGGACCGTGGAGGCCGCGGCAGGGGTGGCAGTGGTGGCGGCGGCGGCGGCGGTGGTGGTTACAAC
CGCAGCAGTGGTGGCTATGAACCCAGAGGTCGTGGAGGTGGCCGTGGAGGCAGAGGTGGCATGGGCGGAAGTGAC
CGTGGTGGCTTCAATAAATTTGGTGGCCCTCGGGACCAAGGATCACGTCATGACTCCGAACAGGATAATTCAGAC
AACAACACCATCTTTGTGCAAGGCCTGGGTGAGAATGTTACAATTGAGTCTGTGGCTGATTACTTCAAGCAGATT
GGTATTATTAAGACAAACAAGAAAACGGGACAGCCCATGATTAATTTGTACACAGACAGGGAAACTGGCAAGCTG
AAGGGAGAGGCAACGGTCTCTTTTGATGACCCACCTTCAGCTAAAGCAGCTATTGACTGGTTTGATGGTAAAGAA
TTCTCCGGAAATCCTATCAAGGTCTCATTTGCTACTCGCCGGGCAGACTTTAATCGGGGTGGTGGCAATGGTCGT
GGAGGCCGAGGGCGAGGAGGACCCATGGGCCGTGGAGGCTATGGAGGTGGTGGCAGTGGTGGTGGTGGCCGAGGA
GGATTTCCCAGTGGAGGTGGTGGCGGTGGAGGACAGCAGCGAGCTGGTGACTGGAAGTGTCCTAATCCCACCTGT
GAGAATATGAACTTCTCTTGGAGGAATGAATGCAACCAGTGTAAGGCCCCTAAACCAGATGGCCCAGGAGGGGGA
CCAGGTGGCTCTCACATGGGGGGTAACTACGGGGATGATCGTCGTGGTGGCAGAGGTGGTGCGATCGCACAAAAG
GTGGCTGAACTGAAAAATAGAGTGGCCGTGAAGCTGAACCGGAACGAGCAGCTGAAGAACAAGGTGGAAGAGCTG
AAGAACAGAAACGCCTACCTGAAGAATGAGCTGGCCACCCTGGAAAACGAGGTGGCCAGACTGGAAAACGACGTG
GCCGAGTTAATCGCAGGAGCACCAGGAAGTGCTGGTTCTGCTGCTGGTAGTGGAGCATCGATAGAGCAGAAGCTG
ATCTCAGAGGAGGACCTGATCGAAGGCCGCCATATGCTAGCCTCCAAAACCATCGTTCTTTCGGTCGGCGAGGCT
ACTCGCACTCTGACTGAGATCCAGTCCACCGCAGACCGTCAGATCTTCGAAGAGAAGGTCGGGCCTCTGGTGGGT
CGGCTGCGCCTCACGGCTTCGCTCCGTCAAAACGGAGCCAAGACCGCGTATCGCGTCAACCTAAAACTGGATCAG
GCGGACGTCGTTGATTCCGGACTTCCGAAAGTGCGCTACACTCAGGTATGGTCGCACGACGTGACAATCGTTGCG
AATAGCACCGAGGCCTCGCGCAAATCGTTGTACGATTTGACCAAGTCCCTCGTCGCGACCTCGCAGGTCGAAGAT
CTTGTCGTCAACCTTGTGCCGCTGGGCCGTGCGGATCCGCTAGCCGGTGCTCCTGGTTCAGCAGGAAGCGCAGCA
GGATCAGGTGCGTGCCCGGTGCCGCTGCAGCTGCCGCCGCTGGAACGCCTGACCCTGGATGATAAAAAACCGCTG
AATACCCTGATCTCTGCTACTGGTCTGTGGATGAGTCGTACCGGAACCATTCATAAAATCAAACACCACGAGGTT
AGCCGTTCGAAAATCTATATTGAGATGGCGTGTGGCGATCATCTGGTTGTGAACAATAGCCGCTCTTCTCGTACA
GCACGTGCACTGCGTCACCACAAATATCGTAAAACCTGTAAACGTTGCCGTGTGTCCGATGAGGATCTGAACAAA
TTCCTGACAAAAGCCAATGAGGACCAAACAAGCGTGAAAGTGAAAGTCGTTAGCGCTCCTACCCGTACTAAAAAA
GCAATGCCGAAATCCGTTGCTCGTGCCCCTAAACCACTGGAAAACACTGAAGCAGCACAGGCACAGCCGTCTGGA
AGCAAATTCTCTCCGGCCATTCCTGTTTCTACCCAGGAGTCCGTTTCTGTTCCAGCAAGTGTGAGCACCAGCATT

AGCAGTATTAGCACCGGTGCCACCGCTAGCGCCCTGGTTAAAGGCAATACCAATCCGATTACAAGCATGTCTGCC
CCGGTTCAAGCATCAGCTCCAGCACTGACAAAATCCCAAACCGATCGTCTGGAGGTTCTGCTGAATCCGAAAGAC
GAAATCAGCCTGAATTCCGGCAAACCGTTTCGTGAACTGGAGAGCGAACTGCTGTCACGTCGTAAAAAAGACCTG
CAACAAATCTATGCCGAAGAACGTGAGAACTATCTGGGGAAACTGGAACGTGAAATCACCCGCTTTTTCGTGGAT
CGTGGCTTTCTGGAGATCAAATCCCCGATTCTGATTCCTCTGGAGTATATCGAGCGTATGGGCATCGACAATGAT
ACCGAACTGAGCAAACAAATTTTCCGTGTGGATAAAAACTTCTGTCTGCGCCCTATGCTAGCACCAAATCTGGCT
AACTATCTGCGCAAACTGGACCGTGCCCTGCCTGATCCTATCAAAATCTTCGAGATCGGCCCGTGTTATCGTAAA
GAGTCCGACGGTAAAGAACATCTGGAGGAGTTTACCATGCTGAACTTTTGCCAAATGGGTTCAGGTTGTACTCGT
GAGAACCTGGAAAGCATCATCACCGATTTTCTGAACCACCTGGGCATTGACTTCAAAATTGTGGGCGACAGCTGT
ATGGTGTTTGGCGACACCCTGGATGTCATGCACGGCGACCTGGAACTGTCTAGTGCCGTTGTGGGCCCAATCCCG
CTGGATCGTGAGTGGGGTATCGACAAACCTTGGATCGGTGCGGGTTTTGGTCTGGAGCGTCTGCTGAAAGTAAAA
CACGACTTCAAGAACATCAAACGTGCTGCACGTTCCGAGTCCTATTACAATGGTATTTCTACTAACCTGTAA
(SEQ ID NO: 605)

Protein:
MAITQFRLFKFCTCLATVFSFLKRLICRSGAPGSAGSAAGSGMASNDYTQQATQSYGAYPTQPGQGYSQQSSQPY
GQQSYSGYSQSTDTSGYGQSSYSSYGQSQNTGYGTQSTPQGYGSTGGYGSSQSSQSSYGQQSSYPGYGQQPAPSS
TSGSYGSSSQSSSYGQPQSGSYSQQPSYGGQQQSYGQQQSYNPPQGYGQQNQYNSSSGGGGGGGGGNYGQDQSS
MSSGGGSGGGYGNQDQSGGGGSGGYGQQDRGGRGRGGSGGGGGGGGGGYNRSSGGYEPRGRGGGRGGRGGMGGSD
RGGFNKFGGPRDQGSRHDSEQDNSDNNTIFVQGLGENVTIESVADYFKQIGIIKTNKKTGQPMINLYTDRETGKL
KGEATVSFDDPPSAKAAIDWFDGKEFSGNPIKVSFATRRADFNRGGGNGRGGRGRGGPMGRGGYGGGGSGGGGRG
GFPSGGGGGGGQQRAGDWKCPNPTCENMNFSWRNECNQCKAPKPDGPGGGPGGSHMGGNYGDDRRGGRGGAIAQK
VAELKNRVAVKLNRNEQLKNKVEELKNRNAYLKNELATLENEVARLENDVAELIAGAPGSAGSAAGSGASIEQKL
ISEEDLIEGRHMLASKTIVLSVGEATRTLTEIQSTADRQIFEEKVGPLVGRLRLTASLRQNGAKTAYRVNLKLDQ
ADVVDSGLPKVRYTQVWSHDVTIVANSTEASRKSLYDLTKSLVATSQVEDLVVNLVPLGRADPLAGAPGSAGSAA
GSGACPVPLQLPPLERLTLDDKKPLNTLISATGLWMSRTGTIHKIKHHEVSRSKIYIEMACGDHLVVNNSRSSRT
ARALRHHKYRKTCKRCRVSDEDLNKFLTKANEDQTSVKVKVVSAPTRTKKAMPKSVARAPKPLENTEAAQAQPSG
SKFSPAIPVSTQESVSVPASVSTSISSISTGATASALVKGNTNPITSMSAPVQASAPALTKSQTDRLEVLLNPKD
EISLNSGKPFRELESELLSRRKKDLQQIYAEERENYLGKLEREITRFFVDRGFLEIKSPILIPLEYIERMGIDND
TELSKQIFRVDKNFCLRPMLAPNLANYLRKLDRALPDPIKIFEIGPCYRKESDGKEHLEEFTMLNFCQMGSGCTR
ENLESIITDFLNHLGIDFKIVGDSCMVFGDTLDVMHGDLELSSAVVGPIPLDREWGIDKPWIGAGFGLERLLKVK
HDFKNIKRAARSESYYNGISTNL* (SEQ ID NO: 606)

EBAG9₁₋₂₉::FUS::SYNZIP3::4xλ_{N22}::IFRS1
DNA:
ATGGCCATCACCCAGTTTCGGTTATTTAAATTTTGTACCTGCCTAGCAACAGTATTCTCATTCCTAAAGAGATTA
ATATGCAGATCTGGAGCACCCGGCTCCGCCGGCTCCGCCGCCGGCTCCGGCATGGCCTCAAACGATTATACCCAA
CAAGCAACCCAAAGCTATGGGGCCTACCCCACCCAGCCCGGGCAGGGCTATTCCCAGCAGAGCAGTCAGCCCTAC
GGACAGCAGAGTTACAGTGGTTATAGCCAGTCCACGGACACTTCAGGATATGGCCAGAGCAGCTATTCTTCTTAT
GGCCAGAGCCAGAACACAGGCTATGGAACTCAGTCAACTCCCCAGGGATATGGCTCGACTGGCGGCTATGGCAGT
AGCCAGAGCTCCCAATCGTCTTACGGGCAGCAGTCCTCCTACCCTGGCTATGGCCAGCAGCCAGCTCCCAGCAGC
ACCTCGGGAAGTTACGGTAGCAGTTCTCAGAGCAGCAGCTATGGGCAGCCCCAGAGTGGGAGCTACAGCCAGCAG
CCTAGCTATGGTGGACAGCAGCAAAGCTATGGACAGCAGCAAAGCTATAATCCCCCTCAGGGCTATGGACAGCAG
AACCAGTACAACAGCAGCAGTGGTGGTGGAGGTGGAGGTGGAGGTGGAGGTAACTATGGCCAAGATCAATCCTCC
ATGAGTAGTGGTGGTGGCAGTGGTGGCGGTTATGGCAATCAAGACCAGAGTGGTGGAGGTGGCAGCGGTGGCTAT
GGACAGCAGGACCGTGGAGGCCGCGGCAGGGGTGGCAGTGGTGGCGGCGGCGGCGGCGGTGGTGGTTACAAC
CGCAGCAGTGGTGGCTATGAACCCAGAGGTCGTGGAGGTGGCCGTGGAGGCAGAGGTGGCATGGGCGGAAGTGAC
CGTGGTGGCTTCAATAAATTTGGTGGCCCTCGGGACCAAGGATCACGTCATGACTCCGAACAGGATAATTCAGAC
AACAACACCATCTTTGTGCAAGGCCTGGGTGAGAATGTTACAATTGAGTCTGTGGCTGATTACTTCAAGCAGATT
GGTATTATTAAGACAAACAAGAAAACGGGACAGCCCATGATTAATTTGTACACAGACAGGGAAACTGGCAAGCTG
AAGGGAGAGGCAACGGTCTCTTTTGATGACCCACCTTCAGCTAAAGCAGCTATTGACTGGTTTGATGGTAAAGAA
TTCTCCGGAAATCCTATCAAGGTCTCATTTGCTACTCGCCGGGCAGACTTTAATCGGGGTGGTGGCAATGGTCGT
GGAGGCCGAGGGCGAGGAGGACCCATGGGCCGTGGAGGCTATGGAGGTGGTGGCAGTGGTGGTGGTGGCCGAGGA
GGATTTCCCAGTGGAGGTGGTGGCGGTGGAGGACAGCAGCGAGCTGGTGACTGGAAGTGTCCTAATCCCACCTGT
GAGAATATGAACTTCTCTTGGAGGAATGAATGCAACCAGTGTAAGGCCCCTAAACCAGATGGCCCAGGAGGGGGA
CCAGGTGGCTCTCACATGGGGGGTAACTACGGGGATGATCGTCGTGGTGGCAGAGGTGGTGCGATCGCAAATGAG
GTGACCACCCTGGAAAACGACGCCGCCTTCATCGAGAACGAGAACGCCTACCTGGAAAAAGAGATCGCCAGACTG

AGAAAGGAAAAGGCCGCTCTGCGGAACAGACTGGCCCACAAGAAGGGAGCACCAGGAAGTGCTGGTTCTGCTGCT
GGTAGTGGAGCATCGATAGAGCAGAAGCTGATCTCAGAGGAGGACCTGCTAGCCACCATGGACGCACAAACACGA
CGACGTGAGCGTCGCGCTGAGAAACAAGCTCAATGGAAAGCTGCAAACCCACCGCTCGACGGAGCCGGAGCTGGC
GCTGGAGCTGGAGCCGGAGCTGGCGGTCTAGCCACCATGGACGCACAAACACGACGACGTGAGCGTCGCGCTGAG
AAACAAGCTCAATGGAAAGCTGCAAACCCACCGCTCGACGGAGCCGGAGCTGGCGCTGGAGCTGGAGCCGGAGCT
GGCGGTCTAGCCACCATGGACGCACAAACACGACGACGTGAGCGTCGCGCTGAGAAACAAGCTCAATGGAAAGCT
GCAAACCCACCGCTCGACGGAGCCGGAGCTGGCGCTGGAGCTGGAGCCGGAGCTGGCGGTCTAGCCACCATGGAC
GCACAAACACGACGACGTGAGCGTCGCGCTGAGAAACAAGCTCAATGGAAAGCTGCAAACCCACCGCTCGAGTCT
AGAGGGCCCGTTGGTGCTCCTGGTTCAGCAGGAAGCGCAGCAGGATCAGGTGCGTGCCCGGTGCCGCTGCAGCTG
CCGCCGCTGGAACGCCTGACCCTGGATGACAAAAAACCGCTGAATACCCTGATCTCTGCTACTGGTCTGTGGATG
AGTCGTACCGGAACCATTCATAAAATCAAACACCACGAGGTTAGCCGTTCGAAAATCTATATTGAGATGGCGTGT
GGCGATCATCTGGTTGTGAACAATAGCCGCTCTTCTCGTACAGCACGTGCACTGCGTCACCACAAATATCGTAAA
ACCTGTAAACGTTGCCGTGTGTCCGATGAGGATCTGAACAAATTCCTGACAAAAGCCAATGAGGACCAAACAAGC
GTGAAAGTGAAAGTCGTTAGCGCTCCTACCCGTACTAAAAAAGCAATGCCGAAATCCGTTGCTCGTGCCCCTAAA
CCACTGGAAAACACTGAAGCAGCACAGGCACAGCCGTCTGGAAGCAAATTCTCTCCGGCCATTCCTGTTTCTACC
CAGGAGTCCGTTTCTGTTCCAGCAAGTGTGAGCACCAGCATTAGCAGTATTAGCACCGGTGCCACCGCTAGCGCC
CTGGTTAAAGGCAATACCAATCCGATTACAAGCATGTCTGCCCCGGTTCAAGCATCAGCTCCAGCACTGACAAAA
TCCCAAACCGATCGTCTGGAGGTTCTGCTGAATCCGAAAGACGAAATCAGCCTGAATTCCGGCAAACCGTTTCGT
GAACTGGAGAGCGAACTGCTGTCACGTCGTAAAAAAGACCTGCAACAAATCTATGCCGAAGAACGTGAGAACTAT
CTGGGGAAACTGGAACGTGAAATCACCCGCTTTTTCGTGGATCGTGGCTTTCTGGAGATCAAATCCCCGATTCTG
ATTCCTCTGGAGTATATCGAGCGTATGGGCATCGACAATGATACCGAACTGAGCAAACAAATTTTCCGTGTGGAT
AAAAACTTCTGTCTGCGCCCTATGCTTGCACCAAATATGCTGAACTATAGCCGCAAACTGGACCGTGCCCTGCCT
GATCCTATCAAAATCTTCGAGATCGGCCCGTGTTATCGTAAAGAGTCCGACGGTAAAGAACATCTGGAGGAGTTT
ACCATGCTGTCTTTTATGCAAATGGGTTCAGGTTGTACTCGTGAGAACCTGGAAAGCATCATCACCGATTTTCTG
AACCACCTGGGCATTGACTTCAAAATTGTGGGCGACAGCTGTATGGTGTATGGCGACACCCTGGATGTCATGCAC
GGCGACCTGGAACTGTCTAGTGCCGTTGTTGGACCAATTCCGCTGGACCGTGAGTGGGGTATCGACAAACCGTGG
ATCGGAGCAGGATTCGGTCTGGAACGCCTGCTGAAAGTGAAACACGACTTCAAAAACATCAAACGTGCCGCCCGT
TCTGAATCGTATTATAACGGGATCTCTACGAACCTGTAA (SEQ ID NO: 607)

Protein:
MAITQFRLFKFCTCLATVFSFLKRLICRSGAPGSAGSAAGSGMASNDYTQQATQSYGAYPTQPGQGYSQQSSQPY
GQQSYSGYSQSTDTSGYGQSSYSSYGQSQNTGYGTQSTPQGYGSTGGYGSSQSSQSSYGQQSSYPGYGQQPAPSS
TSGSYGSSSQSSSYGQPQSGSYSQQPSYGGQQQSYGQQQSYNPPQGYGQQNQYNSSSGGGGGGGGGGNYGQDQSS
MSSGGGSGGGYGNQDQSGGGGSGGYGQQDRGGRGRGGSGGGGGGGGGGYNRSSGGYEPRGRGGGRGGRGGMGGSD
RGGFNKFGGPRDQGSRHDSEQDNSDNNTIFVQGLGENVTIESVADYFKQIGIIKTNKKTGQPMINLYTDRETGKL
KGEATVSFDDPPSAKAAIDWFDGKEFSGNPIKVSFATRRADFNRGGGNGRGGRGRGGPMGRGGYGGGGSGGGGRG
GFPSGGGGGGGQQRAGDWKCPNPTCENMNFSWRNECNQCKAPKPDGPGGGPGGSHMGGNYGDDRRGGRGGAIANE
VTTLENDAAFIENENAYLEKEIARLRKEKAALRNRLAHKKGAPGSAGSAAGSGASIEQKLISEEDLLATMDAQTR
RRERRAEKQAQWKAANPPLDGAGAGAGAGAGAGGLATMDAQTRRRERRAEKQAQWKAANPPLDGAGAGAGAGAGA
GGLATMDAQTRRRERRAEKQAQWKAANPPLDGAGAGAGAGAGAGGLATMDAQTRRRERRAEKQAQWKAANPPLES
RGPVGAPGSAGSAAGSGACPVPLQLPPLERLTLDDKKPLNTLISATGLWMSRTGTIHKIKHHEVSRSKIYIEMAC
GDHLVVNNSRSSRTARALRHHKYRKTCKRCRVSDEDLNKFLTKANEDQTSVKVKVVSAPTRTKKAMPKSVARAPK
PLENTEAAQAQPSGSKFSPAIPVSTQESVSVPASVSTSISSISTGATASALVKGNTNPITSMSAPVQASAPALTK
SQTDRLEVLLNPKDEISLNSGKPFRELESELLSRRKKDLQQIYAEERENYLGKLEREITRFFVDRGFLEIKSPIL
IPLEYIERMGIDNDTELSKQIFRVDKNFCLRPMLAPNMLNYSRKLDRALPDPIKIFEIGPCYRKESDGKEHLEEF
TMLSFMQMGSGCTRENLESIITDFLNHLGIDFKIVGDSCMVYGDTLDVMHGDLELSSAVVGPIPLDREWGIDKPW
IGAGFGLERLLKVKHDFKNIKRAARSESYYNGISTNL* (SEQ ID NO: 608)

LCK::FUS::SYNZIP1::MCP::PylRS(AF)
DNA:
ATGGGCTGCGTGTGCAGCAGCAACCCCGAGGGTACCGAGCTCGCCTCAAACGATTATACCCAACAAGCAACCCAA
AGCTATGGGGCCTACCCCACCCAGCCCGGGCAGGGCTATTCCCAGCAGAGCAGTCAGCCCTACGGACAGCAGAGT
TACAGTGGTTATAGCCAGTCCACGGACACTTCAGGATATGGCCAGAGCAGCTATTCTTCTTATGGCCAGAGCCAG
AACACAGGCTATGGAACTCAGTCAACTCCCCAGGGATATGGCTCGACTGGCGGCTATGGCAGTAGCCAGAGCTCC
CAATCGTCTTACGGGCAGCAGTCCTCCTACCCTGGCTATGGCCAGCAGCCAGCTCCCAGCAGCACCTCGGGAAGT
TACGGTAGCAGTTCTCAGAGCAGCAGCTATGGGCAGCCCCAGAGTGGGAGCTACAGCCAGCAGCCTAGCTATGGT
GGACAGCAGCAAAGCTATGGACAGCAGCAAAGCTATAATCCCCCTCAGGGCTATGGACAGCAGAACCAGTACAAC

AGCAGCAGTGGTGGTGGAGGTGGAGGTGGAGGTGGAGGTAACTATGGCCAAGATCAATCCTCCATGAGTAGTGGT
GGTGGCAGTGGTGGCGGTTATGGCAATCAAGACCAGAGTGGTGGAGGTGGCAGCGGTGGCTATGGACAGCAGGAC
CGTGGAGGCCGCGGCAGGGGTGGCAGTGGTGGCGGCGGCGGCGGCGGTGGTGGTTACAACCGCAGCAGTGGT
GGCTATGAACCCAGAGGTCGTGGAGGTGGCCGTGGAGGCAGAGGTGGCATGGGCGGAAGTGACCGTGGTGGCTTC
AATAAATTTGGTGGCCCTCGGGACCAAGGATCACGTCATGACTCCGAACAGGATAATTCAGACAACAACACCATC
TTTGTGCAAGGCCTGGGTGAGAATGTTACAATTGAGTCTGTGGCTGATTACTTCAAGCAGATTGGTATTATTAAG
ACAAACAAGAAAACGGGACAGCCCATGATTAATTTGTACACAGACAGGGAAACTGGCAAGCTGAAGGGAGAGGCA
ACGGTCTCTTTTGATGACCCACCTTCAGCTAAAGCAGCTATTGACTGGTTTGATGGTAAAGAATTCTCCGGAAAT
CCTATCAAGGTCTCATTTGCTACTCGCCGGGCAGACTTTAATCGGGGTGGTGGCAATGGTCGTGGAGGCCGAGGG
CGAGGAGGACCCATGGGCCGTGGAGGCTATGGAGGTGGTGGCAGTGGTGGTGGTGGCCGAGGAGGATTTCCCAGT
GGAGGTGGTGGCGGTGGAGGACAGCAGCGAGCTGGTGACTGGAAGTGTCCTAATCCCACCTGTGAGAATATGAAC
TTCTCTTGGAGGAATGAATGCAACCAGTGTAAGGCCCCTAAACCAGATGGCCCAGGAGGGGGACCAGGTGGCTCT
CACATGGGGGGTAACTACGGGGATGATCGTCGTGGTGGCAGAGGAGGCGCGATCGCAAATCTGGTGGCCCAGCTG
GAAAACGAGGTGGCCAGCCTGGAAAACGAGAACGAAACCCTGAAGAAAAAGAACCTGCACAAGAAGGACCTGATC
GCCTACCTGGAAAAGGAAATCGCCAACCTGAGAAAGAAGATCGAGGAAGCATCGATATATCCCTATGATGTGCCG
GATTATGCTGGAGCACCAGGAAGTGCTGGTTCTGCTGCTGGTAGTGGAGCTTCTAACTTTACTCAGTTCGTTCTC
GTCGACAATGGCGGAACTGGCGACGTGACTGTCGCCCCAAGCAACTTCGCTAACGGGATCGCTGAATGGATCAGC
TCTAACTCGCGTTCACAGGCTTACAAAGTAACCTGTAGCGTTCGTCAGAGCTCTGCGCAGAATCGCAAATACACC
ATCAAAGTCGAGGTGCCTAAAGGCGCCTGGCGTTCGTACTTAAATATGGAACTAACCATTCCAATTTTCGCCACG
AATTCCGACTGCGAGCTTATTGTTAAGGCAATGCAAGGTCTCCTAAAAGATGGAAACCCGATTCCCTCAGCAATC
GCAGCAAACTCCGGCATCTACGGCGCCGATTACAAGGACGATGATGACAAGGGAGCACCAGGAAGTGCTGGTTCT
GCTGCTGGTAGTGGAGCGTGCCCGGTGCCGCTGCAGCTGCCGCCGCTGGAACGCCTGACCCTGGATGATAAAAAA
CCGCTGAATACCCTGATCTCTGCTACTGGTCTGTGGATGAGTCGTACCGGAACCATTCATAAAATCAAACACCAC
GAGGTTAGCCGTTCGAAAATCTATATTGAGATGGCGTGTGGCGATCATCTGGTTGTGAACAATAGCCGCTCTTCT
CGTACAGCACGTGCACTGCGTCACCACAAATATCGTAAAACCTGTAAACGTTGCCGTGTGTCCGATGAGGATCTG
AACAAATTCCTGACAAAAGCCAATGAGGACCAAACAAGCGTGAAAGTGAAAGTCGTTAGCGCTCCTACCCGTACT
AAAAAAGCAATGCCGAAATCCGTTGCTCGTGCCCCTAAACCACTGGAAAACACTGAAGCAGCACAGGCACAGCCG
TCTGGAAGCAAATTCTCTCCGGCCATTCCTGTTTCTACCCAGGAGTCCGTTTCTGTTCCAGCAAGTGTGAGCACC
AGCATTAGCAGTATTAGCACCGGTGCCACCGCTAGCGCCCTGGTTAAAGGCAATACCAATCCGATTACAAGCATG
TCTGCCCCGGTTCAAGCATCAGCTCCAGCACTGACAAAATCCCAAACCGATCGTCTGGAGGTTCTGCTGAATCCG
AAAGACGAAATCAGCCTGAATTCCGGCAAACCGTTTCGTGAACTGGAGAGCGAACTGCTGTCACGTCGTAAAAAA
GACCTGCAACAAATCTATGCCGAAGAACGTGAGAACTATCTGGGGAAACTGGAACGTGAAATCACCCGCTTTTTC
GTGGATCGTGGCTTTCTGGAGATCAAATCCCCGATTCTGATTCCTCTGGAGTATATCGAGCGTATGGGCATCGAC
AATGATACCGAACTGAGCAAACAAATTTTCCGTGTGGATAAAAACTTCTGTCTGCGCCCTATGCTAGCACCAAAT
CTGGCTAACTATCTGCGCAAACTGGACCGTGCCCTGCCTGATCCTATCAAAATCTTCGAGATCGGCCCGTGTTAT
CGTAAAGAGTCCGACGGTAAAGAACATCTGGAGGAGTTTACCATGCTGAACTTTTGCCAAATGGGTTCAGGTTGT
ACTCGTGAGAACCTGGAAAGCATCATCACCGATTTTCTGAACCACCTGGGCATTGACTTCAAAATTGTGGGCGAC
AGCTGTATGGTGTTTGGCGACACCCTGGATGTCATGCACGGCGACCTGGAACTGTCTAGTGCCGTTGTGGGCCCA
ATCCCGCTGGATCGTGAGTGGGGTATCGACAAACCTTGGATCGGTGCGGGTTTTGGTCTGGAGCGTCTGCTGAAA
GTAAAACACGACTTCAAGAACATCAAACGTGCTGCACGTTCCGAGTCCTATTACAATGGTATTTCTACTAACCTG
TAA (SEQ ID NO: 609)

Protein:
MGCVCSSNPEGTELASNDYTQQATQSYGAYPTQPGQGYSQQSSQPYGQQSYSGYSQSTDTSGYGQSSYSSYGQSQ
NTGYGTQSTPQGYGSTGGYGSSQSSQSSYGQQSSYPGYGQQPAPSSTSGSYGSSSQSSSYGQPQSGSYSQQPSYG
GQQQSYGQQQSYNPPQGYGQQNQYNSSSGGGGGGGGGGNYGQDQSSMSSGGGSGGGYGNQDQSGGGGSGGYGQQD
RGGRGRGGSGGGGGGGGGGYNRSSGGYEPRGRGGGRGGRGGMGGSDRGGFNKFGGPRDQGSRHDSEQDNSDNNTI
FVQGLGENVTIESVADYFKQIGIIKTNKKTGQPMINLYTDRETGKLKGEATVSFDDPPSAKAAIDWFDGKEFSGN
PIKVSFATRRADFNRGGGNGRGGRGRGGPMGRGGYGGGGSGGGGRGGFPSGGGGGGGQQRAGDWKCPNPTCENMN
FSWRNECNQCKAPKPDGPGGGPGGSHMGGNYGDDRRGGRGGAIANLVAQLENEVASLENENETLKKKNLHKKDLI
AYLEKEIANLRKKIEEASIYPYDVPDYAGAPGSAGSAAGSGASNFTQFVLVDNGGTGDVTVAPSNFANGIAEWIS
SNSRSQAYKVTCSVRQSSAQNRKYTIKVEVPKGAWRSYLNMELTIPIFATNSDCELIVKAMQGLLKDGNPIPSAI
AANSGIYGADYKDDDDKGAPGSAGSAAGSGACPVPLQLPPLERLTLDDKKPLNTLISATGLWMSRTGTIHKIKHH
EVSRSKIYIEMACGDHLVVNNSRSSRTARALRHHKYRKTCKRCRVSDEDLNKFLTKANEDQTSVKVKVVSAPTRT
KKAMPKSVARAPKPLENTEAAQAQPSGSKFSPAIPVSTQESVSVPASVSTSISSISTGATASALVKGNTNPITSM
SAPVQASAPALTKSQTDRLEVLLNPKDEISLNSGKPFRELESELLSRRKKDLQQIYAEERENYLGKLEREITRFF
VDRGFLEIKSPILIPLEYIERMGIDNDTELSKQIFRVDKNFCLRPMLAPNLANYLRKLDRALPDPIKIFEIGPCY

RKESDGKEHLEEFTMLNFCQMGSGCTRENLESIITDFLNHLGIDFKIVGDSCMVFGDTLDVMHGDLELSSAVVGP
IPLDREWGIDKPWIGAGFGLERLLKVKHDFKNIKRAARSESYYNGISTNL* (SEQ ID NO: 610)

LCK::FUS::SYNZIP2::MCP::PylRS(AF)
DNA:
ATGGGCTGCGTGTGCAGCAGCAACCCCGAGGGTACCGAGCTCGCCTCAAACGATTATACCCAACAAGCAACCCAA
AGCTATGGGGCCTACCCCACCCAGCCCGGGCAGGGCTATTCCCAGCAGAGCAGTCAGCCCTACGGACAGCAGAGT
TACAGTGGTTATAGCCAGTCCACGGACACTTCAGGATATGGCCAGAGCAGCTATTCTTCTTATGGCCAGAGCCAG
AACACAGGCTATGGAACTCAGTCAACTCCCCAGGGATATGGCTCGACTGGCGGCTATGGCAGTAGCCAGAGCTCC
CAATCGTCTTACGGGCAGCAGTCCTCCTACCCTGGCTATGGCCAGCAGCCAGCTCCCAGCAGCACCTCGGGAAGT
TACGGTAGCAGTTCTCAGAGCAGCAGCTATGGGCAGCCCCAGAGTGGGAGCTACAGCCAGCAGCCTAGCTATGGT
GGACAGCAGCAAAGCTATGGACAGCAGCAAAGCTATAATCCCCCTCAGGGCTATGGACAGCAGAACCAGTACAAC
AGCAGCAGTGGTGGTGGAGGTGGAGGTGGAGGTGGAGGTAACTATGGCCAAGATCAATCCTCCATGAGTAGTGGT
GGTGGCAGTGGTGGCGGTTATGGCAATCAAGACCAGAGTGGTGGAGGTGGCAGCGGTGGCTATGGACAGCAGGAC
CGTGGAGGCCGCGGCAGGGGTGGCAGTGGTGGCGGCGGCGGCGGCGGTGGTGGTTACAACCGCAGCAGTGGT
GGCTATGAACCCAGAGGTCGTGGAGGTGGCCGTGGAGGCAGAGGTGGCATGGGCGGAAGTGACCGTGGTGGCTTC
AATAAATTTGGTGGCCCTCGGGACCAAGGATCACGTCATGACTCCGAACAGGATAATTCAGACAACAACACCATC
TTTGTGCAAGGCCTGGGTGAGAATGTTACAATTGAGTCTGTGGCTGATTACTTCAAGCAGATTGGTATTATTAAG
ACAAACAAGAAAACGGGACAGCCCATGATTAATTTGTACACAGACAGGGAAACTGGCAAGCTGAAGGGAGAGGCA
ACGGTCTCTTTTGATGACCCACCTTCAGCTAAAGCAGCTATTGACTGGTTTGATGGTAAAGAATTCTCCGGAAAT
CCTATCAAGGTCTCATTTGCTACTCGCCGGGCAGACTTTAATCGGGGTGGTGGCAATGGTCGTGGAGGCCGAGGG
CGAGGAGGACCCATGGGCCGTGGAGGCTATGGAGGTGGTGGCAGTGGTGGTGGTGGCCGAGGAGGATTTCCCAGT
GGAGGTGGTGGCGGTGGAGGACAGCAGCGAGCTGGTGACTGGAAGTGTCCTAATCCCACCTGTGAGAATATGAAC
TTCTCTTGGAGGAATGAATGCAACCAGTGTAAGGCCCCTAAACCAGATGGCCCAGGAGGGGGACCAGGTGGCTCT
CACATGGGGGGTAACTACGGGGATGATCGTCGTGGTGGCAGAGGAGGCGCGATCGCAGCTAGAAACGCCTACCTG
AGAAAGAAAATCGCCAGACTGAAGAAGGACAACCTGCAGCTGGAAAGAGACGAGCAGAACCTGGAAAAGATCATC
GCCAACCTCAGAGATGAGATCGCCAGACTGGAAAACGAGGTGGCCAGCCACGAGCAGGCATCGATATATCCCTAT
GATGTGCCGGATTATGCTGGAGCACCAGGAAGTGCTGGTTCTGCTGCTGGTAGTGGAGCTTCTAACTTTACTCAG
TTCGTTCTCGTCGACAATGGCGGAACTGGCGACGTGACTGTCGCCCCAAGCAACTTCGCTAACGGGATCGCTGAA
TGGATCAGCTCTAACTCGCGTTCACAGGCTTACAAAGTAACCTGTAGCGTTCGTCAGAGCTCTGCGCAGAATCGC
AAATACACCATCAAAGTCGAGGTGCCTAAAGGCGCCTGGCGTTCGTACTTAAATATGGAACTAACCATTCCAATT
TTCGCCACGAATTCCGACTGCGAGCTTATTGTTAAGGCAATGCAAGGTCTCCTAAAAGATGGAAACCCGATTCCC
TCAGCAATCGCAGCAAACTCCGGCATCTACGGCGCCGATTACAAGGACGATGATGACAAGGGAGCACCAGGAAGT
GCTGGTTCTGCTGCTGGTAGTGGAGCGTGCCCGGTGCCGCTGCAGCTGCCGCCGCTGGAACGCCTGACCCTGGAT
GATAAAAAACCGCTGAATACCCTGATCTCTGCTACTGGTCTGTGGATGAGTCGTACCGGAACCATTCATAAAATC
AAACACCACGAGGTTAGCCGTTCGAAAATCTATATTGAGATGGCGTGTGGCGATCATCTGGTTGTGAACAATAGC
CGCTCTTCTCGTACAGCACGTGCACTGCGTCACCACAAATATCGTAAAACCTGTAAACGTTGCCGTGTGTCCGAT
GAGGATCTGAACAAATTCCTGACAAAAGCCAATGAGGACCAAACAAGCGTGAAAGTGAAAGTCGTTAGCGCTCCT
ACCCGTACTAAAAAAGCAATGCCGAAATCCGTTGCTCGTGCCCCTAAACCACTGGAAAACACTGAAGCAGCACAG
GCACAGCCGTCTGGAAGCAAATTCTCTCCGGCCATTCCTGTTTCTACCCAGGAGTCCGTTTCTGTTCCAGCAAGT
GTGAGCACCAGCATTAGCAGTATTAGCACCGGTGCCACCGCTAGCGCCCTGGTTAAAGGCAATACCAATCCGATT
ACAAGCATGTCTGCCCCGGTTCAAGCATCAGCTCCAGCACTGACAAAATCCCAAACCGATCGTCTGGAGGTTCTG
CTGAATCCGAAAGACGAAATCAGCCTGAATTCCGGCAAACCGTTTCGTGAACTGGAGAGCGAACTGCTGTCACGT
CGTAAAAAAGACCTGCAACAAATCTATGCCGAAGAACGTGAGAACTATCTGGGGAAACTGGAACGTGAAATCACC
CGCTTTTTCGTGGATCGTGGCTTTCTGGAGATCAAATCCCCGATTCTGATTCCTCTGGAGTATATCGAGCGTATG
GGCATCGACAATGATACCGAACTGAGCAAACAAATTTTCCGTGTGGATAAAAACTTCTGTCTGCGCCCTATGCTA
GCACCAAATCTGGCTAACTATCTGCGCAAACTGGACCGTGCCCTGCCTGATCCTATCAAAATCTTCGAGATCGGC
CCGTGTTATCGTAAAGAGTCCGACGGTAAAGAACATCTGGAGGAGTTTACCATGCTGAACTTTTGCCAAATGGGT
TCAGGTTGTACTCGTGAGAACCTGGAAAGCATCATCACCGATTTTCTGAACCACCTGGGCATTGACTTCAAAATT
GTGGGCGACAGCTGTATGGTGTTTGGCGACACCCTGGATGTCATGCACGGCGACCTGGAACTGTCTAGTGCCGTT
GTGGGCCCAATCCCGCTGGATCGTGAGTGGGGTATCGACAAACCTTGGATCGGTGCGGGTTTTGGTCTGGAGCGT
CTGCTGAAAGTAAAACACGACTTCAAGAACATCAAACGTGCTGCACGTTCCGAGTCCTATTACAATGGTATTTCT
ACTAACCTGTAA (SEQ ID NO: 611)

Protein:
MGCVCSSNPEGTELASNDYTQQATQSYGAYPTQPGQGYSQQSSQPYGQQSYSGYSQSTDTSGYGQSSYSSYGQSQ
NTGYGTQSTPQGYGSTGGYGSSQSSQSSYGQQSSYPGYGQQPAPSSTSGSYGSSSQSSSYGQPQSGSYSQQPSYG

GQQQSYGQQQSYNPPQGYGQQNQYNSSSGGGGGGGGGGNYGQDQSSMSSGGGSGGGYGNQDQSGGGGSGGYGQQD
RGGRGRGGSGGGGGGGGGGYNRSSGGYEPRGRGGGRGGRGGMGGSDRGGFNKFGGPRDQGSRHDSEQDNSDNNTI
FVQGLGENVTIESVADYFKQIGIIKTNKKTGQPMINLYTDRETGKLKGEATVSFDDPPSAKAAIDWFDGKEFSGN
PIKVSFATRRADFNRGGGNGRGGRGRGGPMGRGGYGGGGSGGGGRGGFPSGGGGGGGQQRAGDWKCPNPTCENMN
FSWRNECNQCKAPKPDGPGGGPGGSHMGGNYGDDRRGGRGGAIAARNAYLRKKIARLKKDNLQLERDEQNLEKII
ANLRDEIARLENEVASHEQASIYPYDVPDYAGAPGSAGSAAGSGASNFTQFVLVDNGGTGDVTVAPSNFANGIAE
WISSNSRSQAYKVTCSVRQSSAQNRKYTIKVEVPKGAWRSYLNMELTIPIFATNSDCELIVKAMQGLLKDGNPIP
SAIAANSGIYGADYKDDDDKGAPGSAGSAAGSGACPVPLQLPPLERLTLDDKKPLNTLISATGLWMSRTGTIHKI
KHHEVSRSKIYIEMACGDHLVVNNSRSSRTARALRHHKYRKTCKRCRVSDEDLNKFLTKANEDQTSVKVKVVSAP
TRTKKAMPKSVARAPKPLENTEAAQAQPSGSKFSPAIPVSTQESVSVPASVSTSISSISTGATASALVKGNTNPI
TSMSAPVQASAPALTKSQTDRLEVLLNPKDEISLNSGKPFRELESELLSRRKKDLQQIYAEERENYLGKLEREIT
RFFVDRGFLEIKSPILIPLEYIERMGIDNDTELSKQIFRVDKNFCLRPMLAPNLANYLRKLDRALPDPIKIFEIG
PCYRKESDGKEHLEEFTMLNFCQMGSGCTRENLESIITDFLNHLGIDFKIVGDSCMVFGDTLDVMHGDLELSSAV
VGPIPLDREWGIDKPWIGAGFGLERLLKVKHDFKNIKRAARSESYYNGISTNL* (SEQ ID NO: 612)

CG1::FUS::SYNZIP1::MCP::PylRS(AF)
DNA:
ATGGCCATTTGTCAATTCTTCCTTCAAGGCCGGTGCCGCTTTGGAGATCGGTGCTGGAACGAACATCCCGGTGCT
AGGGGTGCAGGAGGAGGACGGCAGCAACCGCAGCAGCAGCCTTCAGGTAATAATAGACGTGGATGGAATACAACT
AGCCAGAGATATTCCAATGTCATCCAGCCATCCAGTTTCTCCAAATCCACACCATGGGGGGGCAGCAGAGATCAA
GAAAAGCCATATTTCAGTTCTTTTGATTCTGGAGCTTCAACTAACAGGAAGGAAGGCTTTGGATTGTCTGAGAAC
CCATTTGCTTCACTTAGTCCTGATGAGCAGAAAGATGAAAAGAAACTTCTGGAAGGAATTGTAAAAGATATGGAG
GTTTGGGAATCATCAGGGCAGTGGATGTTTTCTGTTTATTCACCAGTGAAAAAGAAACCTAATATTTCAGGTTTT
ACAGACATTTCACCAGAGGAATTGAGGCTTGAATACCATAACTTCTTAACCAGCAATAACTTACAGAGTTATCTA
AATTCTGTCCAACGTTTAATAAATCAATGGAGGAACAGGGTAAATGAACTGAAAAGTCTAAATATATCAACTAAA
GTAGCTTTGCTCTCTGATGTAAAGGATGGAGTAAATCAAGCAGCACCTGCATTTGGATTTGGCAGCAGTCAAGCA
GCAACATTTATGTCGCCAGGCTTTCCAGTCAATAACAGCAGCAGTGATAATGCTCAGAACTTTAGTTTTAAAACA
AACTCTGGATTTGCTGCTGCCTCTTCTGGAAGCCCTGCTGGTTTTGGGAGTTCCCCAGCATTTGGAGCTGCAGCC
TCTACCAGTTCAGGTATCTCTACTTCTGCTCCAGCTTTTGGATTTGGGAAGCCTGAAGTCACATCGGCTGCATCA
TTTTCATTCAAAAGCCCTGCAGCTTCCAGTTTTGGATCACCTGGATTTTCAGGACTTCCAGCTTCCTTGGCAACA
GGTCCTGTCAGAGCTCCAGTGGCCCCAGCCTTTGGAGGTGGCAGTTCTGTGGCTGGTTTTGGTAGTCCGGGCTCA
CATTCTCACACTGCTTTTTCTAAGCCATCCAGTGACACTTTTGGAAATAGCAGCATATCCACTTCTCTGTCAGCC
TCAAGCAGCATCATTGCAACAGATAATGTGTTATTCACACCCAGAGATAAACTAACAGTAGAAGAACTGGAACAA
TTTCAATCCAAGAAATTTACTCTGGGAAAAATTCCATTAAAGCCTCCACCTCTGGAACTTCTAAATGTTGGAGCA
CCCGGCTCCGCCGGCTCCGCCGCCGGCTCCGGCATGGCCTCAAACGATTATACCCAACAAGCAACCCAAAGCTAT
GGGGCCTACCCCACCCAGCCCGGGCAGGGCTATTCCCAGCAGAGCAGTCAGCCCTACGGACAGCAGAGTTACAGT
GGTTATAGCCAGTCCACGGACACTTCAGGATATGGCCAGAGCAGCTATTCTTCTTATGGCCAGAGCCAGAACACA
GGCTATGGAACTCAGTCAACTCCCCAGGGATATGGCTCGACTGGCGGCTATGGCAGTAGCCAGAGCTCCCAATCG
TCTTACGGGCAGCAGTCCTCCTACCCTGGCTATGGCCAGCAGCCAGCTCCCAGCAGCACCTCGGGAAGTTACGGT
AGCAGTTCTCAGAGCAGCAGCTATGGGCAGCCCCAGAGTGGGAGCTACAGCCAGCAGCCTAGCTATGGTGGACAG
CAGCAAAGCTATGGACAGCAGCAAAGCTATAATCCCCCTCAGGGCTATGGACAGCAGAACCAGTACAACAGCAGC
AGTGGTGGTGGAGGTGGAGGTGGAGGTGGAGGTAACTATGGCCAAGATCAATCCTCCATGAGTAGTGGTGGTGGC
AGTGGTGGCGGTTATGGCAATCAAGACCAGAGTGGTGGAGGTGGCAGCGGTGGCTATGGACAGCAGGACCGTGGA
GGCCGCGGCAGGGGTGGCAGTGGTGGCGGCGGCGGCGGCGGCGGTGGTGGTTACAACCGCAGCAGTGGTGGCTAT
GAACCCAGAGGTCGTGGAGGTGGCCGTGGAGGCAGAGGTGGCATGGGCGGAAGTGACCGTGGTGGCTTCAATAAA
TTTGGTGGCCCTCGGGACCAAGGATCACGTCATGACTCCGAACAGGATAATTCAGACAACAACACCATCTTTGTG
CAAGGCCTGGGTGAGAATGTTACAATTGAGTCTGTGGCTGATTACTTCAAGCAGATTGGTATTATTAAGACAAAC
AAGAAAACGGGACAGCCCATGATTAATTTGTACACAGACAGGGAAACTGGCAAGCTGAAGGGAGAGGCAACGGTC
TCTTTTGATGACCCACCTTCAGCTAAAGCAGCTATTGACTGGTTTGATGGTAAAGAATTCTCCGGAAATCCTATC
AAGGTCTCATTTGCTACTCGCCGGGCAGACTTTAATCGGGGTGGTGGCAATGGTCGTGGAGGCCGAGGGCGAGGA
GGACCCATGGGCCGTGGAGGCTATGGAGGTGGTGGCAGTGGTGGTGGTGGCCGAGGAGGATTTCCCAGTGGAGGT
GGTGGCGGTGGAGGACAGCAGCGAGCTGGTGACTGGAAGTGTCCTAATCCCACCTGTGAGAATATGAACTTCTCT
TGGAGGAATGAATGCAACCAGTGTAAGGCCCCTAAACCAGATGGCCCAGGAGGGGGACCAGGTGGCTCTCACATG
GGGGGTAACTACGGGGATGATCGTCGTGGTGGCAGAGGTGGTGCGATCGCAAATCTGGTGGCCCAGCTGGAAAAC
GAGGTGGCCAGCCTGGAAAACGAGAACGAAACCCTGAAGAAAAAGAACCTGCACAAGAAGGACCTGATCGCCTAC
CTGGAAAAGGAAATCGCCAACCTGAGAAAGAAGATCGAGGAAGCATCGATATATCCCTATGATGTGCCGGATTAT
GCTGGAGCACCAGGAAGTGCTGGTTCTGCTGCTGGTAGTGGAGCTTCTAACTTTACTCAGTTCGTTCTCGTCGAC

AATGGCGGAACTGGCGACGTGACTGTCGCCCCAAGCAACTTCGCTAACGGGATCGCTGAATGGATCAGCTCTAAC
TCGCGTTCACAGGCTTACAAAGTAACCTGTAGCGTTCGTCAGAGCTCTGCGCAGAATCGCAAATACACCATCAAA
GTCGAGGTGCCTAAAGGCGCCTGGCGTTCGTACTTAAATATGGAACTAACCATTCCAATTTTCGCCACGAATTCC
GACTGCGAGCTTATTGTTAAGGCAATGCAAGGTCTCCTAAAAGATGGAAACCCGATTCCCTCAGCAATCGCAGCA
AACTCCGGCATCTACGGCGCCGATTACAAGGACGATGATGACAAGGGAGCACCAGGAAGTGCTGGTTCTGCTGCT
GGTAGTGGAGCGTGCCCGGTGCCGCTGCAGCTGCCGCCGCTGGAACGCCTGACCCTGGATGATAAAAAACCGCTG
AATACCCTGATCTCTGCTACTGGTCTGTGGATGAGTCGTACCGGAACCATTCATAAAATCAAACACCACGAGGTT
AGCCGTTCGAAAATCTATATTGAGATGGCGTGTGGCGATCATCTGGTTGTGAACAATAGCCGCTCTTCTCGTACA
GCACGTGCACTGCGTCACCACAAATATCGTAAAACCTGTAAACGTTGCCGTGTGTCCGATGAGGATCTGAACAAA
TTCCTGACAAAAGCCAATGAGGACCAAACAAGCGTGAAAGTGAAAGTCGTTAGCGCTCCTACCCGTACTAAAAAA
GCAATGCCGAAATCCGTTGCTCGTGCCCCTAAACCACTGGAAAACACTGAAGCAGCACAGGCACAGCCGTCTGGA
AGCAAATTCTCTCCGGCCATTCCTGTTTCTACCCAGGAGTCCGTTTCTGTTCCAGCAAGTGTGAGCACCAGCATT
AGCAGTATTAGCACCGGTGCCACCGCTAGCGCCCTGGTTAAAGGCAATACCAATCCGATTACAAGCATGTCTGCC
CCGGTTCAAGCATCAGCTCCAGCACTGACAAAATCCCAAACCGATCGTCTGGAGGTTCTGCTGAATCCGAAAGAC
GAAATCAGCCTGAATTCCGGCAAACCGTTTCGTGAACTGGAGAGCGAACTGCTGTCACGTCGTAAAAAAGACCTG
CAACAAATCTATGCCGAAGAACGTGAGAACTATCTGGGGAAACTGGAACGTGAAATCACCCGCTTTTTCGTGGAT
CGTGGCTTTCTGGAGATCAAATCCCCGATTCTGATTCCTCTGGAGTATATCGAGCGTATGGGCATCGACAATGAT
ACCGAACTGAGCAAACAAATTTTCCGTGTGGATAAAAACTTCTGTCTGCGCCCTATGCTAGCACCAAATCTGGCT
AACTATCTGCGCAAACTGGACCGTGCCCTGCCTGATCCTATCAAAATCTTCGAGATCGGCCCGTGTTATCGTAAA
GAGTCCGACGGTAAAGAACATCTGGAGGAGTTTACCATGCTGAACTTTTGCCAAATGGGTTCAGGTTGTACTCGT
GAGAACCTGGAAAGCATCATCACCGATTTTCTGAACCACCTGGGCATTGACTTCAAAATTGTGGGCGACAGCTGT
ATGGTGTTTGGCGACACCCTGGATGTCATGCACGGCGACCTGGAACTGTCTAGTGCCGTTGTGGGCCCAATCCCG
CTGGATCGTGAGTGGGGTATCGACAAACCTTGGATCGGTGCGGGTTTTGGTCTGGAGCGTCTGCTGAAAGTAAAA
CACGACTTCAAGAACATCAAACGTGCTGCACGTTCCGAGTCCTATTACAATGGTATTTCTACTAACCTGTAA
(SEQ ID NO: 613)

Protein:
MAICQFFLQGRCRFGDRCWNEHPGARGAGGGRQQPQQQPSGNNRRGWNTTSQRYSNVIQPSSFSKSTPWGGSRDQ
EKPYFSSFDSGASTNRKEGFGLSENPFASLSPDEQKDEKKLLEGIVKDMEVWESSGQWMFSVYSPVKKKPNISGF
TDISPEELRLEYHNFLTSNNLQSYLNSVQRLINQWRNRVNELKSLNISTKVALLSDVKDGVNQAAPAFGFGSSQA
ATFMSPGFPVNNSSSDNAQNFSFKTNSGFAAASSGSPAGFGSSPAFGAAASTSSGISTSAPAFGFGKPEVTSAAS
FSFKSPAASSFGSPGFSGLPASLATGPVRAPVAPAFGGGSSVAGFGSPGSHSHTAFSKPSSDTFGNSSISTSLSA
SSSIIATDNVLFTPRDKLTVEELEQFQSKKFTLGKIPLKPPPLELLNVGAPGSAGSAAGSGMASNDYTQQATQSY
GAYPTQPGQGYSQQSSQPYGQQSYSGYSQSTDTSGYGQSSYSSYGQSQNTGYGTQSTPQGYGSTGGYGSSQSSQS
SYGQQSSYPGYGQQPAPSSTSGSYGSSSQSSSYGQPQSGSYSQQPSYGGQQQSYGQQQSYNPPQGYGQQNQYNSS
SGGGGGGGGGGNYGQDQSSMSSGGGSGGGYGNQDQSGGGGSGGYGQQDRGGRGRGGSGGGGGGGGGGYNRSSGGY
EPRGRGGGGRGGRGGMGGSDRGGFNKFGGPRDQGSRHDSEQDNSDNNTIFVQGLGENVTIESVADYFKQIGIIKTN
KKTGQPMINLYTDRETGKLKGEATVSFDDPPSAKAAIDWFDGKEFSGNPIKVSFATRRADFNRGGGNGRGGRGRG
GPMGRGGYGGGGSGGGGRGGFPSGGGGGGGQQRAGDWKCPNPTCENMNFSWRNECNQCKAPKPDGPGGGPGGSHM
GGNYGDDRRGGRGGAIANLVAQLENEVASLENENETLKKKNLHKKDLIAYLEKEIANLRKKIEEASIYPYDVPDY
AGAPGSAGSAAGSGASNFTQFVLVDNGGTGDVTVAPSNFANGIAEWISSNSRSQAYKVTCSVRQSSAQNRKYTIK
VEVPKGAWRSYLNMELTIPIFATNSDCELIVKAMQGLLKDGNPIPSAIAANSGIYGADYKDDDDKGAPGSAGSAA
GSGACPVPLQLPPLERLTLDDKKPLNTLISATGLWMSRTGTIHKIKHHEVSRSKIYIEMACGDHLVVNNSRSSRT
ARALRHHKYRKTCKRCRVSDEDLNKFLTKANEDQTSVKVKVVSAPTRTKKAMPKSVARAPKPLENTEAAQAQPSG
SKFSPAIPVSTQESVSVPASVSTSISSISTGATASALVKGNTNPITSMSAPVQASAPALTKSQTDRLEVLLNPKD
EISLNSGKPFRELESELLSRRKKDLQQIYAEERENYLGKLEREITRFFVDRGFLEIKSPILIPLEYIERMGIDND
TELSKQIFRVDKNFCLRPMLAPNLANYLRKLDRALPDPIKIFEIGPCYRKESDGKEHLEEFTMLNFCQMGSGCTR
ENLESIITDFLNHLGIDFKIVGDSCMVFGDTLDVMHGDLELSSAVVGPIPLDREWGIDKPWIGAGFGLERLLKVK
HDFKNIKRAARSESYYNGISTNL* (SEQ ID NO: 614)

CG1::FUS::SYNZIP2::MCP::PylRS(AF)
DNA:
ATGGCCATTTGTCAATTCTTCCTTCAAGGCCGGTGCCGCTTTGGAGATCGGTGCTGGAACGAACATCCCGGTGCT
AGGGGTGCAGGAGGAGGACGGCAGCAACCGCAGCAGCAGCCTTCAGGTAATAATAGACGTGGATGGAATACAACT
AGCCAGAGATATTCCAATGTCATCCAGCCATCCAGTTTCTCCAAATCCACACCATGGGGGGGCAGCAGAGATCAA
GAAAAGCCATATTTCAGTTCTTTTGATTCTGGAGCTTCAACTAACAGGAAGGAAGGCTTTGGATTGTCTGAGAAC
CCATTTGCTTCACTTAGTCCTGATGAGCAGAAAGATGAAAAGAAACTTCTGGAAGGAATTGTAAAAGATATGGAG

GTTTGGGAATCATCAGGGCAGTGGATGTTTTCTGTTTATTCACCAGTGAAAAAGAAACCTAATATTTCAGGTTTT
ACAGACATTTCACCAGAGGAATTGAGGCTTGAATACCATAACTTCTTAACCAGCAATAACTTACAGAGTTATCTA
AATTCTGTCCAACGTTTAATAAATCAATGGAGGAACAGGGTAAATGAACTGAAAAGTCTAAATATATCAACTAAA
GTAGCTTTGCTCTCTGATGTAAAGGATGGAGTAAATCAAGCAGCACCTGCATTTGGATTTGGCAGCAGTCAAGCA
GCAACATTTATGTCGCCAGGCTTTCCAGTCAATAACAGCAGCAGTGATAATGCTCAGAACTTTAGTTTTAAAACA
AACTCTGGATTTGCTGCTGCCTCTTCTGGAAGCCCTGCTGGTTTTGGGAGTTCCCCAGCATTTGGAGCTGCAGCC
TCTACCAGTTCAGGTATCTCTACTTCTGCTCCAGCTTTTGGATTTGGGAAGCCTGAAGTCACATCGGCTGCATCA
TTTTCATTCAAAAGCCCTGCAGCTTCCAGTTTTGGATCACCTGGATTTTCAGGACTTCCAGCTTCCTTGGCAACA
GGTCCTGTCAGAGCTCCAGTGGCCCCAGCCTTTGGAGGTGGCAGTTCTGTGGCTGGTTTTGGTAGTCCGGGCTCA
CATTCTCACACTGCTTTTTCTAAGCCATCCAGTGACACTTTTGGAAATAGCAGCATATCCACTTCTCTGTCAGCC
TCAAGCAGCATCATTGCAACAGATAATGTGTTATTCACACCCAGAGATAAACTAACAGTAGAAGAACTGGAACAA
TTTCAATCCAAGAAATTTACTCTGGGAAAAATTCCATTAAAGCCTCCACCTCTGGAACTTCTAAATGTTGGAGCA
CCCGGCTCCGCCGGCTCCGCCGCCGGCTCCGGCATGGCCTCAAACGATTATACCCAACAAGCAACCCAAAGCTAT
GGGGCCTACCCCACCCAGCCCGGGCAGGGCTATTCCCAGCAGAGCAGTCAGCCCTACGGACAGCAGAGTTACAGT
GGTTATAGCCAGTCCACGGACACTTCAGGATATGGCCAGAGCAGCTATTCTTCTTATGGCCAGAGCCAGAACACA
GGCTATGGAACTCAGTCAACTCCCCAGGGATATGGCTCGACTGGCGGCTATGGCAGTAGCCAGAGCTCCCAATCG
TCTTACGGGCAGCAGTCCTCCTACCCTGGCTATGGCCAGCAGCCAGCTCCCAGCAGCACCTCGGGAAGTTACGGT
AGCAGTTCTCAGAGCAGCAGCTATGGGCAGCCCCAGAGTGGGAGCTACAGCCAGCAGCCTAGCTATGGTGGACAG
CAGCAAAGCTATGGACAGCAGCAAAGCTATAATCCCCCTCAGGGCTATGGACAGCAGAACCAGTACAACAGCAGC
AGTGGTGGTGGAGGTGGAGGTGGAGGTAACTATGGCCAAGATCAATCCTCCATGAGTAGTGGTGGTGGC
AGTGGTGGCGGTTATGGCAATCAAGACCAGAGTGGTGGAGGTGGCAGCGGTGGCTATGGACAGCAGGACCGTGGA
GGCCGCGGCAGGGGTGGCAGTGGTGGCGGCGGCGGCGGCGGTGGTGGTTACAACCGCAGCAGTGGTGGCTAT
GAACCCAGAGGTCGTGGAGGTGGCCGTGGAGGCAGAGGTGGCATGGGCGGAAGTGACCGTGGTGGCTTCAATAAA
TTTGGTGGCCCTCGGGACCAAGGATCACGTCATGACTCCGAACAGGATAATTCAGACAACAACACCATCTTTGTG
CAAGGCCTGGGTGAGAATGTTACAATTGAGTCTGTGGCTGATTACTTCAAGCAGATTGGTATTATTAAGACAAAC
AAGAAAACGGGACAGCCCATGATTAATTTGTACACAGACAGGGAAACTGGCAAGCTGAAGGGAGAGGCAACGGTC
TCTTTTGATGACCCACCTTCAGCTAAAGCAGCTATTGACTGGTTTGATGGTAAAGAATTCTCCGGAAATCCTATC
AAGGTCTCATTTGCTACTCGCCGGGCAGACTTTAATCGGGGTGGTGGCAATGGTCGTGGAGGCCGAGGGCGAGGA
GGACCCATGGGCCGTGGAGGCTATGGAGGTGGTGGCAGTGGTGGTGGTGGCCGAGGAGGATTTCCCAGTGGAGGT
GGTGGCGGTGGAGGACAGCAGCGAGCTGGTGACTGGAAGTGTCCTAATCCCACCTGTGAGAATATGAACTTCTCT
TGGAGGAATGAATGCAACCAGTGTAAGGCCCCTAAACCAGATGGCCCAGGAGGGGGACCAGGTGGCTCTCACATG
GGGGGTAACTACGGGGATGATCGTCGTGGTGGCAGAGGTGGTGCGATCGCAGCTAGAAACGCCTACCTGAGAAAG
AAAATCGCCAGACTGAAGAAGGACAACCTGCAGCTGGAAAGAGACGAGCAGAACCTGGAAAAGATCATCGCCAAC
CTCAGAGATGAGATCGCCAGACTGGAAAACGAGGTGGCCAGCCACGAGCAGGCATCGATATATCCCTATGATGTG
CCGGATTATGCTGGAGCACCAGGAAGTGCTGGTTCTGCTGCTGGTAGTGGAGCTTCTAACTTTACTCAGTTCGTT
CTCGTCGACAATGGCGGAACTGGCGACGTGACTGTCGCCCCAAGCAACTTCGCTAACGGGATCGCTGAATGGATC
AGCTCTAACTCGCGTTCACAGGCTTACAAAGTAACCTGTAGCGTTCGTCAGAGCTCTGCGCAGAATCGCAAATAC
ACCATCAAAGTCGAGGTGCCTAAAGGCGCCTGGCGTTCGTACTTAAATATGGAACTAACCATTCCAATTTTCGCC
ACGAATTCCGACTGCGAGCTTATTGTTAAGGCAATGCAAGGTCTCCTAAAAGATGGAAACCCGATTCCCTCAGCA
ATCGCAGCAAACTCCGGCATCTACGGCGCCGATTACAAGGACGATGATGACAAGGGAGCACCAGGAAGTGCTGGT
TCTGCTGCTGGTAGTGGAGCGTGCCCGGTGCCGCTGCAGCTGCCGCCGCTGGAACGCCTGACCCTGGATGATAAA
AAACCGCTGAATACCCTGATCTCTGCTACTGGTCTGTGGATGAGTCGTACCGGAACCATTCATAAAATCAAACAC
CACGAGGTTAGCCGTTCGAAAATCTATATTGAGATGGCGTGTGGCGATCATCTGGTTGTGAACAATAGCCGCTCT
TCTCGTACAGCACGTGCACTGCGTCACCACAAATATCGTAAAACCTGTAAACGTTGCCGTGTGTCCGATGAGGAT
CTGAACAAATTCCTGACAAAAGCCAATGAGGACCAAACAAGCGTGAAAGTGAAAGTCGTTAGCGCTCCTACCCGT
ACTAAAAAAGCAATGCCGAAATCCGTTGCTCGTGCCCCTAAACCACTGGAAAACACTGAAGCAGCACAGGCACAG
CCGTCTGGAAGCAAATTCTCTCCGGCCATTCCTGTTTCTACCCAGGAGTCCGTTTCTGTTCCAGCAAGTGTGAGC
ACCAGCATTAGCAGTATTAGCACCGGTGCCACCGCTAGCGCCCTGGTTAAAGGCAATACCAATCCGATTACAAGC
ATGTCTGCCCCGGTTCAAGCATCAGCTCCAGCACTGACAAAATCCCAAACCGATCGTCTGGAGGTTCTGCTGAAT
CCGAAAGACGAAATCAGCCTGAATTCCGGCAAACCGTTTCGTGAACTGGAGAGCGAACTGCTGTCACGTCGTAAA
AAAGACCTGCAACAAATCTATGCCGAAGAACGTGAGAACTATCTGGGGAAACTGGAACGTGAAATCACCCGCTTT
TTCGTGGATCGTGGCTTTCTGGAGATCAAATCCCCGATTCTGATTCCTCTGGAGTATATCGAGCGTATGGGCATC
GACAATGATACCGAACTGAGCAAACAAATTTTCCGTGTGGATAAAAACTTCTGTCTGCGCCCTATGCTAGCACCA
AATCTGGCTAACTATCTGCGCAAACTGGACCGTGCCCTGCCTGATCCTATCAAAATCTTCGAGATCGGCCCGTGT
TATCGTAAAGAGTCCGACGGTAAAGAACATCTGGAGGAGTTTACCATGCTGAACTTTTGCCAAATGGGTTCAGGT
TGTACTCGTGAGAACCTGGAAAGCATCATCACCGATTTTCTGAACCACCTGGGCATTGACTTCAAAATTGTGGGC
GACAGCTGTATGGTGTTTGGCGACACCCTGGATGTCATGCACGGCGACCTGGAACTGTCTAGTGCCGTTGTGGGC
CCAATCCCGCTGGATCGTGAGTGGGGTATCGACAAACCTTGGATCGGTGCGGGTTTTGGTCTGGAGCGTCTGCTG

AAAGTAAAACACGACTTCAAGAACATCAAACGTGCTGCACGTTCCGAGTCCTATTACAATGGTATTTCTACTAAC
CTGTAA (SEQ ID NO: 615)

Protein:
MAICQFFLQGRCRFGDRCWNEHPGARGAGGGRQQPQQQPSGNNRRGWNTTSQRYSNVIQPSSFSKSTPWGGSRDQ
EKPYFSSFDSGASTNRKEGFGLSENPFASLSPDEQKDEKKLLEGIVKDMEVWESSGQWMFSVYSPVKKKPNISGF
TDISPEELRLEYHNFLTSNNLQSYLNSVQRLINQWRNRVNELKSLNISTKVALLSDVKDGVNQAAPAFGFGSSQA
ATFMSPGFPVNNSSSDNAQNFSFKTNSGFAAASSGSPAGFGSSPAFGAAASTSSGISTSAPAFGFGKPEVTSAAS
FSFKSPAASSFGSPGFSGLPASLATGPVRAPVAPAFGGGSSVAGFGSPGSHSHTAFSKPSSDTFGNSSISTSLSA
SSSIIATDNVLFTPRDKLTVEELEQFQSKKFTLGKIPLKPPPLELLNVGAPGSAGSAAGSGMASNDYTQQATQSY
GAYPTQPGQGYSQQSSQPYGQQSYSGYSQSTDTSGYGQSSYSSYGQSQNTGYGTQSTPQGYGSTGGYGSSQSSQS
SYGQQSSYPGYGQQPAPSSTSGSYGSSSQSSSYGQPQSGSYSQQPSYGGQQQSYGQQQSYNPPQGYGQQNQYNSS
SGGGGGGGGGGNYGQDQSSMSSGGGSGGGYGNQDQSGGGGSGGYGQQDRGGRGRGGSGGGGGGGGGYNRSSGGY
EPRGRGGGGRGGRGGMGGSDRGGFNKFGGPRDQGSRHDSEQDNSDNNTIFVQGLGENVTIESVADYFKQIGIIKTN
KKTGQPMINLYTDRETGKLKGEATVSFDDPPSAKAAIDWFDGKEFSGNPIKVSFATRRADFNRGGGNGRGGRGRG
GPMGRGGYGGGGSGGGGRGGFPSGGGGGGGQQRAGDWKCPNPTCENMNFSWRNECNQCKAPKPDGPGGGPGGSHM
GGNYGDDRRGGRGGAIAARNAYLRKKIARLKKDNLQLERDEQNLEKIIANLRDEIARLENEVASHEQASIYPYDV
PDYAGAPGSAGSAAGSGASNFTQFVLVDNGGTGDVTVAPSNFANGIAEWISSNSRSQAYKVTCSVRQSSAQNRKY
TIKVEVPKGAWRSYLNMELTIPIFATNSDCELIVKAMQGLLKDGNPIPSAIAANSGIYGADYKDDDDKGAPGSAG
SAAGSGACPVPLQLPPLERLTLDDKKPLNTLISATGLWMSRTGTIHKIKHHEVSRSKIYIEMACGDHLVVNNSRS
SRTARALRHHKYRKTCKRCRVSDEDLNKFLTKANEDQTSVKVKVVSAPTRTKKAMPKSVARAPKPLENTEAAQAQ
PSGSKFSPAIPVSTQESVSVPASVSTSISSISTGATASALVKGNTNPITSMSAPVQASAPALTKSQTDRLEVLLN
PKDEISLNSGKPFRELESELLSRRKKDLQQIYAEERENYLGKLEREITRFFVDRGFLEIKSPILIPLEYIERMGI
DNDTELSKQIFRVDKNFCLRPMLAPNLANYLRKLDRALPDPIKIFEIGPCYRKESDGKEHLEEFTMLNFCQMGSG
CTRENLESIITDFLNHLGIDFKIVGDSCMVFGDTLDVMHGDLELSSAVVGPIPLDREWGIDKPWIGAGFGLERLL
KVKHDFKNIKRAARSESYYNGISTNL* (SEQ ID NO: 616)

TOM20::FUS::SYNZIP4::λN22::CbzRS
DNA:
ATGGTGGGTCGGAACAGCGCCATCGCCGCCGGTGTATGCGGGGCCCTTTTCATTGGGTACTGCATCTACTTCGAC
CGCAAAAGACGAAGTGACCCCAACTTCAAGAACAGGCTTCGAGAACGAAGAAAGAAACAGAAGCTTGCCAAGGAG
AGAGCTGGGCTTTCCAAGTTACCTGACCTTAAAGATGCTGAAGCTGTTCAGAAATTCTTCATGGCCTCAAACGAT
TATACCCAACAAGCAACCCAAAGCTATGGGGCCTACCCCACCCAGCCCGGGCAGGGCTATTCCCAGCAGAGCAGT
CAGCCCTACGGACAGCAGAGTTACAGTGGTTATAGCCAGTCCACGGACACTTCAGGATATGGCCAGAGCAGCTAT
TCTTCTTATGGCCAGAGCCAGAACACAGGCTATGGAACTCAGTCAACTCCCCAGGGATATGGCTCGACTGGCGGC
TATGGCAGTAGCCAGAGCTCCCAATCGTCTTACGGGCAGCAGTCCTCCTACCCTGGCTATGGCCAGCAGCCAGCT
CCCAGCAGCACCTCGGGAAGTTACGGTAGCAGTTCTCAGAGCAGCAGCTATGGGCAGCCCCAGAGTGGGAGCTAC
AGCCAGCAGCCTAGCTATGGTGGACAGCAGCAAAGCTATGGACAGCAGCAAAGCTATAATCCCCCTCAGGGCTAT
GGACAGCAGAACCAGTACAACAGCAGCAGTGGTGGTGGAGGTGGAGGTGGAGGTGGAGGTAACTATGGCCAAGAT
CAATCCTCCATGAGTAGTGGTGGTGGCAGTGGTGGCGGTTATGGCAATCAAGACCAGAGTGGTGGAGGTGGCAGC
GGTGGCTATGGACAGCAGGACCGTGGAGGCCGCGGCAGGGGTGGCAGTGGTGGCGGCGGCGGCGGCGGCGGTGGT
GGTTACAACCGCAGCAGTGGTGGCTATGAACCCAGAGGTCGTGGAGGTGGCCGTGGAGGCAGAGGTGGCATGGGC
GGAAGTGACCGTGGTGGCTTCAATAAATTTGGTGGCCCTCGGGACCAAGGATCACGTCATGACTCCGAACAGGAT
AATTCAGACAACAACACCATCTTTGTGCAAGGCCTGGGTGAGAATGTTACAATTGAGTCTGTGGCTGATTACTTC
AAGCAGATTGGTATTATTAAGACAAACAAGAAAACGGGACAGCCCATGATTAATTTGTACACAGACAGGGAAACT
GGCAAGCTGAAGGGAGAGGCAACGGTCTCTTTTGATGACCCACCTTCAGCTAAAGCAGCTATTGACTGGTTTGAT
GGTAAAGAATTCTCCGGAAATCCTATCAAGGTCTCATTTGCTACTCGCCGGGCAGACTTTAATCGGGGTGGTGGC
AATGGTCGTGGAGGCCGAGGGCGAGGAGGACCCATGGGCCGTGGAGGCTATGGAGGTGGTGGCAGTGGTGGTGGT
GGCCGAGGAGGATTTCCCAGTGGAGGTGGTGGCGGTGGAGGACAGCAGCGAGCTGGTGACTGGAAGTGTCCTAAT
CCCACCTGTGAGAATATGAACTTCTCTTGGAGGAATGAATGCAACCAGTGTAAGGCCCCTAAACCAGATGGCCCA
GGAGGGGGACCAGGTGGCTCTCACATGGGGGGTAACTACGGGGATGATCGTCGTGGTGGCAGAGGAGGCGCGATC
GCACAAAAGGTGGCTGAACTGAAAAATAGAGTGGCCGTGAAGCTGAACCGGAACGAGCAGCTGAAGAACAAGGTG
GAAGAGCTGAAGAACAGAAACGCCTACCTGAAGAATGAGCTGGCCACCCTGGAAAACGAGGTGGCCAGACTGGAA
AACGACGTGGCCGAGTTAATCGCAGGAGCACCAGGAAGTGCTGGTTCTGCTGCTGGTAGTGGAGCATCGATAGAG
CAGAAGCTGATCTCAGAGGAGGACCTGCTAGCCACCATGGACGCACAAACACGACGACGTGAGCGTCGCGCTGAG
AAACAAGCTCAATGGAAAGCTGCAAACCCACCGCTCGACGGAGCCGGAGCTGGCGCTGGAGCTGGAGCCGGAGCT
GGCGGTCTAGCCACCATGGACGCACAAACACGACGACGTGAGCGTCGCGCTGAGAAACAAGCTCAATGGAAAGCT
GCAAACCCACCGCTCGACGGAGCCGGAGCTGGCGCTGGAGCTGGAGCCGGAGCTGGCGGTCTAGCCACCATGGAC

GCACAAACACGACGACGTGAGCGTCGCGCTGAGAAACAAGCTCAATGGAAAGCTGCAAACCCACCGCTCGACGGA
GCCGGAGCTGGCGCTGGAGCTGGAGCCGGAGCTGGCGGTCTAGCCACCATGGACGCACAAACACGACGACGTGAG
CGTCGCGCTGAGAAACAAGCTCAATGGAAAGCTGCAAACCCACCGCTCGAGTCTAGAGGGCCCGTTGGTGCTCCT
GGTTCAGCAGGAAGCGCAGCAGGATCAGGTGCGTGCCCGGTGCCGCTGCAGCTGCCGCCGCTGGAACGCCTGACC
CTGGATGACAAAAAACCGCTGAATACCCTGATCTCTGCTACTGGTCTGTGGATGAGTCGTACCGGAACCATTCAT
AAAATCAAACACCACGAGGTTAGCCGTTCGAAAATCTATATTGAGATGGCGTGTGGCGATCATCTGGTTGTGAAC
AATAGCCGCTCTTCTCGTACAGCACGTGCACTGCGTCACCACAAATATCGTAAAACCTGTAAACGTTGCCGTGTG
TCCGATGAGGATCTGAACAAATTCCTGACAAAAGCCAATGAGGACCAAACAAGCGTGAAAGTGAAAGTCGTTAGC
GCTCCTACCCGTACTAAAAAAGCAATGCCGAAATCCGTTGCTCGTGCCCCTAAACCACTGGAAAACACTGAAGCA
GCACAGGCACAGCCGTCTGGAAGCAAATTCTCTCCGGCCATTCCTGTTTCTACCCAGGAGTCCGTTTCTGTTCCA
GCAAGTGTGAGCACCAGCATTAGCAGTATTAGCACCGGTGCCACCGCTAGCGCCCTGGTTAAAGGCAATACCAAT
CCGATTACAAGCATGTCTGCCCCGGTTCAAGCATCAGCTCCAGCACTGACAAAATCCCAAACCGATCGTCTGGAG
GTTCTGCTGAATCCGAAAGACGAAATCAGCCTGAATTCCGGCAAACCGTTTCGTGAACTGGAGAGCGAACTGCTG
TCACGTCGTAAAAAAGACCTGCAACAAATCTATGCCGAAGAACGTGAGAACTATCTGGGGAAACTGGAACGTGAA
ATCACCCGCTTTTTCGTGGATCGTGGCTTTCTGGAGATCAAATCCCCGATTCTGATTCCTCTGGAGTATATCGAG
CGTATGGGCATCGACAATGATACCGAACTGAGCAAACAAATTTTCCGTGTGGATAAAAACTTCTGTCTGCGCCCT
ATGCTTGCACCAAATCTGATGAACTATGGACGCAAACTGGACCGTGCCCTGCCTGATCCTATCAAAATCTTCGAG
ATCGGCCCGTGTTATCGTAAAGAGTCCGACGGTAAAGAACATCTGGAGGAGTTTACCATGCTGAACTTTACACAA
ATGGGTTCAGGTTGTACTCGTGAGAACCTGGAAAGCATCATCACCGATTTTCTGAACCACCTGGGCATTGACTTC
AAAATTGTGGGCGACAGCTGTATGGTGTATGGCGACACCCTGGATGTCATGCACGGCGACCTGGAACTGTCTAGT
GCCGTTGTTGGACCAATTCCGCTGGACCGTGAGTGGGGTATCGACAAACCGTGGATCGGAGCAGGATTCGGTCTG
GAACGCCTGCTGAAAGTGAAACACGACTTCAAAAACATCAAACGTGCCGCCCGTTCTGAATCGTATTATAACGGG
ATCTCTACGAACCTGTAA (SEQ ID NO: 617)

Protein:
MVGRNSAIAAGVCGALFIGYCIYFDRKRRSDPNFKNRLRERRKKQKLAKERAGLSKLPDLKDAEAVQKFFMASND
YTQQATQSYGAYPTQPGQGYSQQSSQPYGQQSYSGYSQSTDTSGYGQSSYSSYGQSQNTGYGTQSTPQGYGSTGG
YGSSQSSQSSYGQQSSYPGYGQQPAPSSTSGSYGSSSQSSSYGQPQSGSYSQQPSYGGQQQSYGQQQSYNPPQGY
GQQNQYNSSSGGGGGGGGGGNYGQDQSSMSSGGGSGGGYGNQDQSGGGGSGGYGQQDRGGRGRGGSGGGGGGGGG
GYNRSSGGYEPRGRGGGRGGRGGMGGSDRGGFNKFGGPRDQGSRHDSEQDNSDNNTIFVQGLGENVTIESVADYF
KQIGIIKTNKKTGQPMINLYTDRETGKLKGEATVSFDDPPSAKAAIDWFDGKEFSGNPIKVSFATRRADFNRGGG
NGRGGRGRGGPMGRGGYGGGGSGGGGRGGFPSGGGGGGGQQRAGDWKCPNPTCENMNFSWRNECNQCKAPKPDGP
GGGPGGSHMGGNYGDDRRGGRGGAIAQKVAELKNRVAVKLNRNEQLKNKVEELKNRNAYLKNELATLENEVARLE
NDVAELIAGAPGSAGSAAGSGASIEQKLISEEDLLATMDAQTRRRERRAEKQAQWKAANPPLDGAGAGAGAGAGA
GGLATMDAQTRRRERRAEKQAQWKAANPPLDGAGAGAGAGAGAGGLATMDAQTRRRERRAEKQAQWKAANPPLDG
AGAGAGAGAGAGGLATMDAQTRRRERRAEKQAQWKAANPPLESRGPVGAPGSAGSAAGSGACPVPLQLPPLERLT
LDDKKPLNTLISATGLWMSRTGTIHKIKHHEVSRSKIYIEMACGDHLVVNNSRSSRTARALRHHKYRKTCKRCRV
SDEDLNKFLTKANEDQTSVKVKVVSAPTRTKKAMPKSVARAPKPLENTEAAQAQPSGSKFSPAIPVSTQESVSVP
ASVSTSISSISTGATASALVKGNTNPITSMSAPVQASAPALTKSQTDRLEVLLNPKDEISLNSGKPFRELESELL
SRRKKDLQQIYAEERENYLGKLEREITRFFVDRGFLEIKSPILIPLEYIERMGIDNDTELSKQIFRVDKNFCLRP
MLAPNLMNYGRKLDRALPDPIKIFEIGPCYRKESDGKEHLEEFTMLNFTQMGSGCTRENLESIITDFLNHLGIDF
KIVGDSCMVYGDTLDVMHGDLELSSAVVGPIPLDREWGIDKPWIGAGFGLERLLKVKHDFKNIKRAARSESYYNG
ISTNL* (SEQ ID NO: 618)

EBAG9₁₋₂₉::FUS::SYNZIP4::4xλ_{N22}::IFRS1
DNA:
ATGGCCATCACCCAGTTTCGGTTATTTAAATTTTGTACCTGCCTAGCAACAGTATTCTCATTCCTAAAGAGATTA
ATATGCAGATCTGGAGCACCCGGCTCCGCCGGCTCCGCCGCCGGCTCCGGCATGGCCTCAAACGATTATACCCAA
CAAGCAACCCAAAGCTATGGGGCCTACCCCACCCAGCCCGGGCAGGGCTATTCCCAGCAGAGCAGTCAGCCCTAC
GGACAGCAGAGTTACAGTGGTTATAGCCAGTCCACGGACACTTCAGGATATGGCCAGAGCAGCTATTCTTCTTAT
GGCCAGAGCCAGAACACAGGCTATGGAACTCAGTCAACTCCCCAGGGATATGGCTCGACTGGCGGCTATGGCAGT
AGCCAGAGCTCCCAATCGTCTTACGGGCAGCAGTCCTCCTACCCTGGCTATGGCCAGCAGCCAGCTCCCAGCAGC
ACCTCGGGAAGTTACGGTAGCAGTTCTCAGAGCAGCAGCTATGGGCAGCCCCAGAGTGGGAGCTACAGCCAGCAG
CCTAGCTATGGTGGACAGCAGCAAAGCTATGGACAGCAGCAAAGCTATAATCCCCCTCAGGGCTATGGACAGCAG
AACCAGTACAACAGCAGCAGTGGTGGTGGAGGTGGAGGTGGAGGTGGAGGTAACTATGGCCAAGATCAATCCTCC
ATGAGTAGTGGTGGTGGCAGTGGTGGCGGTTATGGCAATCAAGACCAGAGTGGTGGAGGTGGCAGCGGTGGCTAT
GGACAGCAGGACCGTGGAGGCCGCGGCAGGGGTGGCAGTGGTGGCGGCGGCGGCGGCGGCGGTGGTGGTTACAAC

CGCAGCAGTGGTGGCTATGAACCCAGAGGTCGTGGAGGTGGCCGTGGAGGCAGAGGTGGCATGGGCGGAAGTGAC
CGTGGTGGCTTCAATAAATTTGGTGGCCCTCGGGACCAAGGATCACGTCATGACTCCGAACAGGATAATTCAGAC
AACAACACCATCTTTGTGCAAGGCCTGGGTGAGAATGTTACAATTGAGTCTGTGGCTGATTACTTCAAGCAGATT
GGTATTATTAAGACAAACAAGAAAACGGGACAGCCCATGATTAATTTGTACACAGACAGGGAAACTGGCAAGCTG
AAGGGAGAGGCAACGGTCTCTTTTGATGACCCACCTTCAGCTAAAGCAGCTATTGACTGGTTTGATGGTAAAGAA
TTCTCCGGAAATCCTATCAAGGTCTCATTTGCTACTCGCCGGGCAGACTTTAATCGGGGTGGTGGCAATGGTCGT
GGAGGCCGAGGGCGAGGAGGACCCATGGGCCGTGGAGGCTATGGAGGTGGTGGCAGTGGTGGTGGTGGCCGAGGA
GGATTTCCCAGTGGAGGTGGTGGCGGTGGAGGACAGCAGCGAGCTGGTGACTGGAAGTGTCCTAATCCCACCTGT
GAGAATATGAACTTCTCTTGGAGGAATGAATGCAACCAGTGTAAGGCCCCTAAACCAGATGGCCCAGGAGGGGGA
CCAGGTGGCTCTCACATGGGGGGTAACTACGGGGATGATCGTCGTGGTGGCAGAGGTGGTGCGATCGCACAAAAG
GTGGCTGAACTGAAAAATAGAGTGGCCGTGAAGCTGAACCGGAACGAGCAGCTGAAGAACAAGGTGGAAGAGCTG
AAGAACAGAAACGCCTACCTGAAGAATGAGCTGGCCACCCTGGAAAACGAGGTGGCCAGACTGGAAAACGACGTG
GCCGAGTTAATCGCAGGAGCACCAGGAAGTGCTGGTTCTGCTGCTGGTAGTGGAGCATCGATAGAGCAGAAGCTG
ATCTCAGAGGAGGACCTGCTAGCCACCATGGACGCACAAACACGACGACGTGAGCGTCGCGCTGAGAAACAAGCT
CAATGGAAAGCTGCAAACCCACCGCTCGACGGAGCCGGAGCTGGCGCTGGAGCTGGAGCCGGAGCTGGCGGTCTA
GCCACCATGGACGCACAAACACGACGACGTGAGCGTCGCGCTGAGAAACAAGCTCAATGGAAAGCTGCAAACCCA
CCGCTCGACGGAGCCGGAGCTGGCGCTGGAGCTGGAGCCGGAGCTGGCGGTCTAGCCACCATGGACGCACAAACA
CGACGACGTGAGCGTCGCGCTGAGAAACAAGCTCAATGGAAAGCTGCAAACCCACCGCTCGACGGAGCCGGAGCT
GGCGCTGGAGCTGGAGCCGGAGCTGGCGGTCTAGCCACCATGGACGCACAAACACGACGACGTGAGCGTCGCGCT
GAGAAACAAGCTCAATGGAAAGCTGCAAACCCACCGCTCGAGTCTAGAGGGCCCGTTGGTGCTCCTGGTTCAGCA
GGAAGCGCAGCAGGATCAGGTGCGTGCCCGGTGCCGCTGCAGCTGCCGCCGCTGGAACGCCTGACCCTGGATGAC
AAAAAACCGCTGAATACCCTGATCTCTGCTACTGGTCTGTGGATGAGTCGTACCGGAACCATTCATAAAATCAAA
CACCACGAGGTTAGCCGTTCGAAAATCTATATTGAGATGGCGTGTGGCGATCATCTGGTTGTGAACAATAGCCGC
TCTTCTCGTACAGCACGTGCACTGCGTCACCACAAATATCGTAAAACCTGTAAACGTTGCCGTGTGTCCGATGAG
GATCTGAACAAATTCCTGACAAAAGCCAATGAGGACCAAACAAGCGTGAAAGTGAAAGTCGTTAGCGCTCCTACC
CGTACTAAAAAAGCAATGCCGAAATCCGTTGCTCGTGCCCCTAAACCACTGGAAAACACTGAAGCAGCACAGGCA
CAGCCGTCTGGAAGCAAATTCTCTCCGGCCATTCCTGTTTCTACCCAGGAGTCCGTTTCTGTTCCAGCAAGTGTG
AGCACCAGCATTAGCAGTATTAGCACCGGTGCCACCGCTAGCGCCCTGGTTAAAGGCAATACCAATCCGATTACA
AGCATGTCTGCCCCGGTTCAAGCATCAGCTCCAGCACTGACAAAATCCCAAACCGATCGTCTGGAGGTTCTGCTG
AATCCGAAAGACGAAATCAGCCTGAATTCCGGCAAACCGTTTCGTGAACTGGAGAGCGAACTGCTGTCACGTCGT
AAAAAAGACCTGCAACAAATCTATGCCGAAGAACGTGAGAACTATCTGGGGAAACTGGAACGTGAAATCACCCGC
TTTTTCGTGGATCGTGGCTTTCTGGAGATCAAATCCCCGATTCTGATTCCTCTGGAGTATATCGAGCGTATGGGC
ATCGACAATGATACCGAACTGAGCAAACAAATTTTCCGTGTGGATAAAAACTTCTGTCTGCGCCCTATGCTTGCA
CCAAATATGCTGAACTATAGCCGCAAACTGGACCGTGCCCTGCCTGATCCTATCAAAATCTTCGAGATCGGCCCG
TGTTATCGTAAAGAGTCCGACGGTAAAGAACATCTGGAGGAGTTTACCATGCTGTCTTTTATGCAAATGGGTTCA
GGTTGTACTCGTGAGAACCTGGAAAGCATCATCACCGATTTTCTGAACCACCTGGGCATTGACTTCAAAATTGTG
GGCGACAGCTGTATGGTGTATGGCGACACCCTGGATGTCATGCACGGCGACCTGGAACTGTCTAGTGCCGTTGTT
GGACCAATTCCGCTGGACCGTGAGTGGGGTATCGACAAACCGTGGATCGGAGCAGGATTCGGTCTGGAACGCCTG
CTGAAAGTGAAACACGACTTCAAAAACATCAAACGTGCCGCCCGTTCTGAATCGTATTATAACGGGATCTCTACG
AACCTGTAA (SEQ ID NO: 619)

Protein:
MAITQFRLFKFCTCLATVFSFLKRLICRSGAPGSAGSAAGSGMASNDYTQQATQSYGAYPTQPGQGYSQQSSQPY
GQQSYSGYSQSTDTSGYGQSSYSSYGQSQNTGYGTQSTPQGYGSTGGYGSSQSSQSSYGQQSSYPGYGQQPAPSS
TSGSYGSSSQSSSYGQPQSGSYSQQPSYGGQQQSYGQQQSYNPPQGYGQQNQYNSSSGGGGGGGGGNYGQDQSS
MSSGGGSGGGYGNQDQSGGGGSGGYGQQDRGGRGRGGSGGGGGGGGGYNRSSGGYEPRGRGGGRGGRGGMGGSD
RGGFNKFGGPRDQGSRHDSEQDNSDNNTIFVQGLGENVTIESVADYFKQIGIIKTNKKTGQPMINLYTDRETGKL
KGEATVSFDDPPSAKAAIDWFDGKEFSGNPIKVSFATRRADFNRGGGNGRGGRGRGGPMGRGGYGGGGSGGGGRG
GFPSGGGGGGGQQRAGDWKCPNPTCENMNFSWRNECNQCKAPKPDGPGGGPGGSHMGGNYGDDRRGGRGGAIAQK
VAELKNRVAVKLNRNEQLKNKVEELKNRNAYLKNELATLENEVARLENDVAELIAGAPGSAGSAAGSGASIEQKL
ISEEDLLATMDAQTRRRERRAEKQAQWKAANPPLDGAGAGAGAGAGGLATMDAQTRRRERRAEKQAQWKAANP
PLDGAGAGAGAGAGGLATMDAQTRRRERRAEKQAQWKAANPPLDGAGAGAGAGAGGLATMDAQTRRRERRA
EKQAQWKAANPPLESRGPVGAPGSAGSAAGSGACPVPLQLPPLERLTLDDKKPLNTLISATGLWMSRTGTIHKIK
HHEVSRSKIYIEMACGDHLVVNNSRSSRTARALRHHKYRKTCKRCRVSDEDLNKFLTKANEDQTSVKVKVVSAPT
RTKKAMPKSVARAPKPLENTEAAQAQPSGSKFSPAIPVSTQESVSVPASVSTSISSISTGATASALVKGNTNPIT
SMSAPVQASAPALTKSQTDRLEVLLNPKDEISLNSGKPFRELESELLSRRKKDLQQIYAEERENYLGKLEREITR
FFVDRGFLEIKSPILIPLEYIERMGIDNDTELSKQIFRVDKNFCLRPMLAPNMLNYSRKLDRALPDPIKIFEIGP

CYRKESDGKEHLEEFTMLSFMQMGSGCTRENLESIITDFLNHLGIDFKIVGDSCMVYGDTLDVMHGDLELSSAVV
GPIPLDREWGIDKPWIGAGFGLERLLKVKHDFKNIKRAARSESYYNGISTNL* (SEQ ID NO: 620)

TOM20::FUS::SYNZIP3::4xλN22::PylRS(AA)
DNA:
ATGGTGGGTCGGAACAGCGCCATCGCCGCCGGTGTATGCGGGGCCCTTTTCATTGGGTACTGCATCTACTTCGAC
CGCAAAAGACGAAGTGACCCCAACTTCAAGAACAGGCTTCGAGAACGAAGAAAGAAACAGAAGCTTGCCAAGGAG
AGAGCTGGGCTTTCCAAGTTACCTGACCTTAAAGATGCTGAAGCTGTTCAGAAATTCTTCATGGCCTCAAACGAT
TATACCCAACAAGCAACCCAAAGCTATGGGGCCTACCCCACCCAGCCCGGGCAGGGCTATTCCCAGCAGAGCAGT
CAGCCCTACGGACAGCAGAGTTACAGTGGTTATAGCCAGTCCACGGACACTTCAGGATATGGCCAGAGCAGCTAT
TCTTCTTATGGCCAGAGCCAGAACACAGGCTATGGAACTCAGTCAACTCCCCAGGGATATGGCTCGACTGGCGGC
TATGGCAGTAGCCAGAGCTCCCAATCGTCTTACGGGCAGCAGTCCTCCTACCCTGGCTATGGCCAGCAGCCAGCT
CCCAGCAGCACCTCGGGAAGTTACGGTAGCAGTTCTCAGAGCAGCAGCTATGGGCAGCCCCAGAGTGGGAGCTAC
AGCCAGCAGCCTAGCTATGGTGGACAGCAGCAAAGCTATGGACAGCAGCAAAGCTATAATCCCCCTCAGGGCTAT
GGACAGCAGAACCAGTACAACAGCAGCAGTGGTGGTGGAGGTGGAGGTGGAGGTGGAGGTAACTATGGCCAAGAT
CAATCCTCCATGAGTAGTGGTGGTGGCAGTGGTGGCGGTTATGGCAATCAAGACCAGAGTGGTGGAGGTGGCAGC
GGTGGCTATGGACAGCAGGACCGTGGAGGCCGCGGCAGGGGTGGCAGTGGTGGCGGCGGCGGCGGCGGCGGTGGT
GGTTACAACCGCAGCAGTGGTGGCTATGAACCCAGAGGTCGTGGAGGTGGCCGTGGAGGCAGAGGTGGCATGGGC
GGAAGTGACCGTGGTGGCTTCAATAAATTTGGTGGCCCTCGGGACCAAGGATCACGTCATGACTCCGAACAGGAT
AATTCAGACAACAACACCATCTTTGTGCAAGGCCTGGGTGAGAATGTTACAATTGAGTCTGTGGCTGATTACTTC
AAGCAGATTGGTATTATTAAGACAAACAAGAAAACGGGACAGCCCATGATTAATTTGTACACAGACAGGGAAACT
GGCAAGCTGAAGGGAGAGGCAACGGTCTCTTTTGATGACCCACCTTCAGCTAAAGCAGCTATTGACTGGTTTGAT
GGTAAAGAATTCTCCGGAAATCCTATCAAGGTCTCATTTGCTACTCGCCGGGCAGACTTTAATCGGGGTGGTGGC
AATGGTCGTGGAGGCCGAGGGCGAGGAGGACCCATGGGCCGTGGAGGCTATGGAGGTGGTGGCAGTGGTGGTGGT
GGCCGAGGAGGATTTCCCAGTGGAGGTGGTGGCGGTGGAGGACAGCAGCGAGCTGGTGACTGGAAGTGTCCTAAT
CCCACCTGTGAGAATATGAACTTCTCTTGGAGGAATGAATGCAACCAGTGTAAGGCCCCTAAACCAGATGGCCCA
GGAGGGGGACCAGGTGGCTCTCACATGGGGGGTAACTACGGGGATGATCGTCGTGGTGGCAGAGGAGGCGCGATC
GCAAATGAGGTGACCACCCTGGAAAACGACGCCGCCTTCATCGAGAACGAGAACGCCTACCTGGAAAAAGAGATC
GCCAGACTGAGAAAGGAAAAGGCCGCTCTGCGGAACAGACTGGCCCACAAGAAGGGAGCACCAGGAAGTGCTGGT
TCTGCTGCTGGTAGTGGAGCATCGATAGAGCAGAAGCTGATCTCAGAGGAGGACCTGCTAGCCACCATGGACGCA
CAAACACGACGACGTGAGCGTCGCGCTGAGAAACAAGCTCAATGGAAAGCTGCAAACCCACCGCTCGACGGAGCC
GGAGCTGGCGCTGGAGCTGGAGCCGGAGCTGGCGGTCTAGCCACCATGGACGCACAAACACGACGACGTGAGCGT
CGCGCTGAGAAACAAGCTCAATGGAAAGCTGCAAACCCACCGCTCGACGGAGCCGGAGCTGGCGCTGGAGCTGGA
GCCGGAGCTGGCGGTCTAGCCACCATGGACGCACAAACACGACGACGTGAGCGTCGCGCTGAGAAACAAGCTCAA
TGGAAAGCTGCAAACCCACCGCTCGACGGAGCCGGAGCTGGCGCTGGAGCTGGAGCCGGAGCTGGCGGTCTAGCC
ACCATGGACGCACAAACACGACGACGTGAGCGTCGCGCTGAGAAACAAGCTCAATGGAAAGCTGCAAACCCACCG
CTCGAGTCTAGAGGGCCCGTTGGTGCTCCTGGTTCAGCAGGAAGCGCAGCAGGATCAGGTGCGTGCCCGGTGCCG
CTGCAGCTGCCGCCGCTGGAACGCCTGACCCTGGATGACAAAAAACCGCTGAATACCCTGATCTCTGCTACTGGT
CTGTGGATGAGTCGTACCGGAACCATTCATAAAATCAAACACCACGAGGTTAGCCGTTCGAAAATCTATATTGAG
ATGGCGTGTGGCGATCATCTGGTTGTGAACAATAGCCGCTCTTCTCGTACAGCACGTGCACTGCGTCACCACAAA
TATCGTAAAACCTGTAAACGTTGCCGTGTGTCCGATGAGGATCTGAACAAATTCCTGACAAAAGCCAATGAGGAC
CAAACAAGCGTGAAAGTGAAAGTCGTTAGCGCTCCTACCCGTACTAAAAAAGCAATGCCGAAATCCGTTGCTCGT
GCCCCTAAACCACTGGAAAACACTGAAGCAGCACAGGCACAGCCGTCTGGAAGCAAATTCTCTCCGGCCATTCCT
GTTTCTACCCAGGAGTCCGTTTCTGTTCCAGCAAGTGTGAGCACCAGCATTAGCAGTATTAGCACCGGTGCCACC
GCTAGCGCCCTGGTTAAAGGCAATACCAATCCGATTACAAGCATGTCTGCCCCGGTTCAAGCATCAGCTCCAGCA
CTGACAAAATCCCAAACCGATCGTCTGGAGGTTCTGCTGAATCCGAAAGACGAAATCAGCCTGAATTCCGGCAAA
CCGTTTCGTGAACTGGAGAGCGAACTGCTGTCACGTCGTAAAAAAGACCTGCAACAAATCTATGCCGAAGAACGT
GAGAACTATCTGGGGAAACTGGAACGTGAAATCACCCGCTTTTTCGTGGATCGTGGCTTTCTGGAGATCAAATCC
CCGATTCTGATTCCTCTGGAGTATATCGAGCGTATGGGCATCGACAATGATACCGAACTGAGCAAACAAATTTTC
CGTGTGGATAAAAACTTCTGTCTGCGCCCTATGCTGGCACCAAATCTGTATAACTATCTGCGCAAACTGGACCGT
GCCCTGCCTGATCCTATCAAAATCTTCGAGATCGGCCCGTGTTATCGTAAAGAGTCCGACGGTAAAGAACATCTG
GAGGAGTTTACCATGCTGGCCTTTGCCCAAATGGGTTCAGGTTGTACTCGTGAGAACCTGGAAAGCATCATCACC
GATTTTCTGAACCACCTGGGCATTGACTTCAAAATTGTGGGCGACAGCTGTATGGTGTATGGCGACACCCTGGAT
GTCATGCACGGCGACCTGGAACTGTCTAGTGCCGTTGTTGGACCAATTCCGCTGGACCGTGAGTGGGGTATCGAC
AAACCGTGGATCGGAGCAGGATTCGGTCTGGAACGCCTGCTGAAAGTGAAACACGACTTCAAAAACATCAAACGT
GCCGCCCGTTCTGAATCGTATTATAACGGGATCTCTACGAACCTGTAA (SEQ ID NO: 621)

Protein:
MVGRNSAIAAGVCGALFIGYCIYFDRKRRSDPNFKNRLRERRKKQKLAKERAGLSKLPDLKDAEAVQKFFMASND
YTQQATQSYGAYPTQPGQGYSQQSSQPYGQQSYSGYSQSTDTSGYGQSSYSSYGQSQNTGYGTQSTPQGYGSTGG
YGSSQSSQSSYGQQSSYPGYGQQPAPSSTSGSYGSSSQSSSYGQPQSGSYSQQPSYGGQQQSYGQQQSYNPPQGY
GQQNQYNSSSGGGGGGGGGGNYGQDQSSMSSGGGSGGGYGNQDQSGGGGSGGYGQQDRGGRGRGGSGGGGGGGG
GYNRSSGGYEPRGRGGGRGGRGGMGGSDRGGFNKFGGPRDQGSRHDSEQDNSDNNTIFVQGLGENVTIESVADYF
KQIGIIKTNKKTGQPMINLYTDRETGKLKGEATVSFDDPPSAKAAIDWFDGKEFSGNPIKVSFATRRADFNRGGG
NGRGGRGRGGPMGRGGYGGGGSGGGGRGGFPSGGGGGGGQQRAGDWKCPNPTCENMNFSWRNECNQCKAPKPDGP
GGGPGGSHMGGNYGDDRRGGRGGAIANEVTTLENDAAFIENENAYLEKEIARLRKEKAALRNRLAHKKGAPGSAG
SAAGSGASIEQKLISEEDLLATMDAQTRRRERRAEKQAQWKAANPPLDGAGAGAGAGAGAGGLATMDAQTRRRER
RAEKQAQWKAANPPLDGAGAGAGAGAGAGGLATMDAQTRRRERRAEKQAQWKAANPPLDGAGAGAGAGAGAGGLA
TMDAQTRRRERRAEKQAQWKAANPPLESRGPVGAPGSAGSAAGSGACPVPLQLPPLERLTLDDKKPLNTLISATG
LWMSRTGTIHKIKHHEVSRSKIYIEMACGDHLVVNNSRSSRTARALRHHKYRKTCKRCRVSDEDLNKFLTKANED
QTSVKVKVVSAPTRTKKAMPKSVARAPKPLENTEAAQAQPSGSKFSPAIPVSTQESVSVPASVSTSISSISTGAT
ASALVKGNTNPITSMSAPVQASAPALTKSQTDRLEVLLNPKDEISLNSGKPFRELESELLSRRKKDLQQIYAEER
ENYLGKLEREITRFFVDRGFLEIKSPILIPLEYIERMGIDNDTELSKQIFRVDKNFCLRPMLAPNLYNYLRKLDR
ALPDPIKIFEIGPCYRKESDGKEHLEEFTMLAFAQMGSGCTRENLESIITDFLNHLGIDFKIVGDSCMVYGDTLD
VMHGDLELSSAVVGPIPLDREWGIDKPWIGAGFGLERLLKVKHDFKNIKRAARSESYYNGISTNL* (SEQ ID
NO: 622)

LCK::FUS::SYNZIP1::MCP::PylRS(AA)
DNA:
ATGGGCTGCGTGTGCAGCAGCAACCCCGAGGGTACCGAGCTCGCCTCAAACGATTATACCCAACAAGCAACCCAA
AGCTATGGGGCCTACCCCACCCAGCCCGGGCAGGGCTATTCCCAGCAGAGCAGTCAGCCCTACGGACAGCAGAGT
TACAGTGGTTATAGCCAGTCCACGGACACTTCAGGATATGGCCAGAGCAGCTATTCTTCTTATGGCCAGAGCCAG
AACACAGGCTATGGAACTCAGTCAACTCCCCAGGGATATGGCTCGACTGGCGGCTATGGCAGTAGCCAGAGCTCC
CAATCGTCTTACGGGCAGCAGTCCTCCTACCCTGGCTATGGCCAGCAGCCAGCTCCCAGCAGCACCTCGGGAAGT
TACGGTAGCAGTTCTCAGAGCAGCAGCTATGGGCAGCCCCAGAGTGGGAGCTACAGCCAGCAGCCTAGCTATGGT
GGACAGCAGCAAAGCTATGGACAGCAGCAAAGCTATAATCCCCCTCAGGGCTATGGACAGCAGAACCAGTACAAC
AGCAGCAGTGGTGGTGGAGGTGGAGGTGGAGGTGGAGGTAACTATGGCCAAGATCAATCCTCCATGAGTAGTGGT
GGTGGCAGTGGTGGCGGTTATGGCAATCAAGACCAGAGTGGTGGAGGTGGCAGCGGTGGCTATGGACAGCAGGAC
CGTGGAGGCCGCGGCAGGGGTGGCAGTGGTGGCGGCGGCGGCGGCGGTGGTGGTTACAACCGCAGCAGTGGT
GGCTATGAACCCAGAGGTCGTGGAGGTGGCCGTGGAGGCAGAGGTGGCATGGGCGGAAGTGACCGTGGTGGCTTC
AATAAATTTGGTGGCCCTCGGGACCAAGGATCACGTCATGACTCCGAACAGGATAATTCAGACAACAACACCATC
TTTGTGCAAGGCCTGGGTGAGAATGTTACAATTGAGTCTGTGGCTGATTACTTCAAGCAGATTGGTATTATTAAG
ACAAACAAGAAAACGGGACAGCCCATGATTAATTTGTACACAGACAGGGAAACTGGCAAGCTGAAGGGAGAGGCA
ACGGTCTCTTTTGATGACCCACCTTCAGCTAAAGCAGCTATTGACTGGTTTGATGGTAAAGAATTCTCCGGAAAT
CCTATCAAGGTCTCATTTGCTACTCGCCGGGCAGACTTTAATCGGGGTGGTGGCAATGGTCGTGGAGGCCGAGGG
CGAGGAGGACCCATGGGCCGTGGAGGCTATGGAGGTGGTGGCAGTGGTGGTGGTGGCCGAGGAGGATTTCCCAGT
GGAGGTGGTGGCGGTGGAGGACAGCAGCGAGCTGGTGACTGGAAGTGTCCTAATCCCACCTGTGAGAATATGAAC
TTCTCTTGGAGGAATGAATGCAACCAGTGTAAGGCCCCTAAACCAGATGGCCCAGGAGGGGGACCAGGTGGCTCT
CACATGGGGGGTAACTACGGGGATGATCGTCGTGGTGGCAGAGGAGGCGCGATCGCAAATCTGGTGGCCCAGCTG
GAAAACGAGGTGGCCAGCCTGGAAAACGAGAACGAAACCCTGAAGAAAAAGAACCTGCACAAGAAGGACCTGATC
GCCTACCTGGAAAAGGAAATCGCCAACCTGAGAAAGAAGATCGAGGAAGCATCGATATATCCCTATGATGTGCCG
GATTATGCTGGAGCACCAGGAAGTGCTGGTTCTGCTGCTGGTAGTGGAGCTTCTAACTTTACTCAGTTCGTTCTC
GTCGACAATGGCGGAACTGGCGACGTGACTGTCGCCCCAAGCAACTTCGCTAACGGGATCGCTGAATGGATCAGC
TCTAACTCGCGTTCACAGGCTTACAAAGTAACCTGTAGCGTTCGTCAGAGCTCTGCGCAGAATCGCAAATACACC
ATCAAAGTCGAGGTGCCTAAAGGCGCCTGGCGTTCGTACTTAAATATGGAACTAACCATTCCAATTTTCGCCACG
AATTCCGACTGCGAGCTTATTGTTAAGGCAATGCAAGGTCTCCTAAAAGATGGAAACCCGATTCCCTCAGCAATC
GCAGCAAACTCCGGCATCTACGGCGCCGATTACAAGGACGATGATGACAAGGGAGCACCAGGAAGTGCTGGTTCT
GCTGCTGGTAGTGGAGCGTGCCCGGTGCCGCTGCAGCTGCCGCCGCTGGAACGCCTGACCCTGGATGATAAAAAA
CCGCTGAATACCCTGATCTCTGCTACTGGTCTGTGGATGAGTCGTACCGGAACCATTCATAAAATCAAACACCAC
GAGGTTAGCCGTTCGAAAATCTATATTGAGATGGCGTGTGGCGATCATCTGGTTGTGAACAATAGCCGCTCTTCT
CGTACAGCACGTGCACTGCGTCACCACAAATATCGTAAAACCTGTAAACGTTGCCGTGTGTCCGATGAGGATCTG
AACAAATTCCTGACAAAAGCCAATGAGGACCAAACAAGCGTGAAAGTGAAAGTCGTTAGCGCTCCTACCCGTACT
AAAAAAGCAATGCCGAAATCCGTTGCTCGTGCCCCTAAACCACTGGAAAACACTGAAGCAGCACAGGCACAGCCG
TCTGGAAGCAAATTCTCTCCGGCCATTCCTGTTTCTACCCAGGAGTCCGTTTCTGTTCCAGCAAGTGTGAGCACC

AGCATTAGCAGTATTAGCACCGGTGCCACCGCTAGCGCCCTGGTTAAAGGCAATACCAATCCGATTACAAGCATG
TCTGCCCCGGTTCAAGCATCAGCTCCAGCACTGACAAAATCCCAAACCGATCGTCTGGAGGTTCTGCTGAATCCG
AAAGACGAAATCAGCCTGAATTCCGGCAAACCGTTTCGTGAACTGGAGAGCGAACTGCTGTCACGTCGTAAAAAA
GACCTGCAACAAATCTATGCCGAAGAACGTGAGAACTATCTGGGGAAACTGGAACGTGAAATCACCCGCTTTTTC
GTGGATCGTGGCTTTCTGGAGATCAAATCCCCGATTCTGATTCCTCTGGAGTATATCGAGCGTATGGGCATCGAC
AATGATACCGAACTGAGCAAACAAATTTTCCGTGTGGATAAAAACTTCTGTCTGCGCCCTATGCTGGCACCAAAT
CTGTATAACTATCTGCGCAAACTGGACCGTGCCCTGCCTGATCCTATCAAAATCTTCGAGATCGGCCCGTGTTAT
CGTAAAGAGTCCGACGGTAAAGAACATCTGGAGGAGTTTACCATGCTGGCCTTTGCCCAAATGGGTTCAGGTTGT
ACTCGTGAGAACCTGGAAAGCATCATCACCGATTTTCTGAACCACCTGGGCATTGACTTCAAAATTGTGGGCGAC
AGCTGTATGGTGTATGGCGACACCCTGGATGTCATGCACGGCGACCTGGAACTGTCTAGTGCCGTTGTTGGACCA
ATTCCGCTGGACCGTGAGTGGGGTATCGACAAACCGTGGATCGGAGCAGGATTCGGTCTGGAACGCCTGCTGAAA
GTGAAACACGACTTCAAAAACATCAAACGTGCCGCCCGTTCTGAATCGTATTATAACGGGATCTCTACGAACCTG
TAA (SEQ ID NO: 623)

Protein:
MGCVCSSNPEGTELASNDYTQQATQSYGAYPTQPGQGYSQQSSQPYGQQSYSGYSQSTDTSGYGQSSYSSYGQSQ
NTGYGTQSTPQGYGSTGGYGSSQSSQSSYGQQSSYPGYGQQPAPSSTSGSYGSSSQSSSYGQPQSGSYSQQPSYG
GQQQSYGQQQSYNPPQGYGQQNQYNSSSGGGGGGGGGGNYGQDQSSMSSGGGSGGGYGNQDQSGGGGSGGYGQQD
RGGRGRGGSGGGGGGGGGGYNRSSGGYEPRGRGGGRGGRGGMGGSDRGGFNKFGGPRDQGSRHDSEQDNSDNNTI
FVQGLGENVTIESVADYFKQIGIIKTNKKTGQPMINLYTDRETGKLKGEATVSFDDPPSAKAAIDWFDGKEFSGN
PIKVSFATRRADFNRGGGNGRGGRGRGGPMGRGGYGGGGSGGGGRGGFPSGGGGGGGQQRAGDWKCPNPTCENMN
FSWRNECNQCKAPKPDGPGGGPGGSHMGGNYGDDRRGGRGGAIANLVAQLENEVASLENENETLKKKNLHKKDLI
AYLEKEIANLRKKIEEASIYPYDVPDYAGAPGSAGSAAGSGASNFTQFVLVDNGGTGDVTVAPSNFANGIAEWIS
SNSRSQAYKVTCSVRQSSAQNRKYTIKVEVPKGAWRSYLNMELTIPIFATNSDCELIVKAMQGLLKDGNPIPSAI
AANSGIYGADYKDDDDKGAPGSAGSAAGSGACPVPLQLPPLERLTLDDKKPLNTLISATGLWMSRTGTIHKIKHH
EVSRSKIYIEMACGDHLVVNNSRSSRTARALRHHKYRKTCKRCRVSDEDLNKFLTKANEDQTSVKVKVVSAPTRT
KKAMPKSVARAPKPLENTEAAQAQPSGSKFSPAIPVSTQESVSVPASVSTSISSISTGATASALVKGNTNPITSM
SAPVQASAPALTKSQTDRLEVLLNPKDEISLNSGKPFRELESELLSRRKKDLQQIYAEERENYLGKLEREITRFF
VDRGFLEIKSPILIPLEYIERMGIDNDTELSKQIFRVDKNFCLRPMLAPNLYNYLRKLDRALPDPIKIFEIGPCY
RKESDGKEHLEEFTMLAFAQMGSGCTRENLESIITDFLNHLGIDFKIVGDSCMVYGDTLDVMHGDLELSSAVVGP
IPLDREWGIDKPWIGAGFGLERLLKVKHDFKNIKRAARSESYYNGISTNL* (SEQ ID NO: 624)

TOM20::EWSR1::SYNZIP4::4xλN22::SYNZIP4::PylRS(AF)
DNA:
ATGGTGGGTCGGAACAGCGCCATCGCCGCCGGTGTATGCGGGGCCCTTTTCATTGGGTACTGCATCTACTTCGAC
CGCAAAAGACGAAGTGACCCCAACTTCAAGAACAGGCTTCGAGAACGAAGAAAGAAACAGAAGCTTGCCAAGGAG
AGAGCTGGGCTTTCCAAGTTACCTGACCTTAAAGATGCTGAAGCTGTTCAGAAGTTCTTCATGGCGTCCACGGAT
TACAGTACCTATAGCCAAGCTGCAGCGCAGCAGGGCTACAGTGCTTACACCGCCCAGCCCACTCAAGGATATGCA
CAGACCACCCAGGCATATGGGCAACAAAGCTATGGAACCTATGGACAGCCCACTGATGTCAGCTATACCCAGGCT
CAGACCACTGCAACCTATGGGCAGACCGCCTATGCAACTTCTTATGGACAGCCTCCCACTGGTTATACTACTCCA
ACTGCCCCCCAGGCATACAGCCAGCCTGTCCAGGGGTATGGCACTGGTGCTTATGATACCACCACTGCTACAGTC
ACCACCACCCAGGCCTCCTATGCAGCTCAGTCTGCATATGGCACTCAGCCTGCTTATCCAGCCTATGGGCAGCAG
CCAGCAGCCACTGCACCTACAAGACCGCAGGATGGAAACAAGCCCACTGAGACTAGTCAACCTCAATCTAGCACA
GGGGGTTACAACCAGCCCAGCCTAGGATATGGACAGAGTAACTACAGTTATCCCCAGGTACCTGGGAGCTACCCC
ATGCAGCCAGTCACTGCACCTCCATCCTACCCTCCTACCAGCTATTCCTCTACACAGCCGACTAGTTATGATCAG
AGCAGTTACTCTCAGCAGAACACCTATGGGCAACCGAGCAGCTATGGACAGCAGAGTAGCTATGGTCAACAAAGC
AGCTATGGGCAGCAGCCTCCCACTAGTTACCCACCCCAAACTGGATCCTACAGCCAAGCTCCAAGTCAATATAGC
CAACAGAGCAGCAGCTACGGGCAGCAGAGTTCATTCCGACAGGACCACCCCAGTAGCATGGGTGTTTATGGGCAG
GAGTCTGGAGGATTTTCCGGACCAGGAGAGAACCGGAGCATGAGTGGCCCTGATAACCGGGGCAGGGGAAGAGGG
GGATTTGATCGTGGAGGCATGAGCAGAGGTGGGCGGGGAGGAGGACGCGGTGGAATGGGCAGCGCTGGAGAGCGA
GGTGGCTTCAATAAGCCTGGTGGACCCATGGATGAAGGACCAGATCTTGATCTAGGCCCACCTGTAGATCCAGAT
GAAGACTCTGACAACAGTGCAATTTATGTACAAGGATTAAATGACAGTGTGACTCTAGATGATCTGGCAGACTTC
TTTAAGCAGTGTGGGGTTGTTAAGATGAACAAGAGAACTGGGCAACCCATGATCCACATCTACCTGGACAAGGAA
ACAGGAAAGCCCAAAGGCGATGCCACAGTGTCCTATGAAGACCCACCTACTGCCAAGGCTGCCGTGGAATGGTTT
GATGGGAAAGATTTTCAAGGGAGCAAACTTAAAGTCTCCCTTGCTCGGAAGAAGCCTCCAATGAACAGTATGCGG
GGTGGTCTGCCACCCCGTGAGGGCAGAGGCATGCCACCACCACTCCGTGGAGGTCCAGGAGGCCCAGGAGGTCCT
GGGGGACCCATGGGTCGCATGGGAGGCCGTGGAGGAGATAGAGGAGGCTTCCCTCCAAGAGGACCCCGGGGTTCC

CGAGGGAACCCCTCTGGAGGAGGAAACGTCCAGCACCGAGCTGGAGACTGGCAGTGTCCCAATCCGGGTTGTGGA
AACCAGAACTTCGCCTGGAGAACAGAGTGCAACCAGTGTAAGGCCCCAAAGCCTGAAGGCTTCCTCCCGCCACCC
TTTCCGCCCCCGGGTGGTGATCGTGGCAGAGGTGGCCCTGGTGGCATGCGGGGAGGAAGAGGTGGCCTCATGGAT
CGTGGTGGTCCCGGTGGAATGTTCAGAGGTGGCCGTGGTGGAGACAGAGGTGGCTTCCGTGGTGGCCGGGGCATG
GACCGAGGTGGCTTTGGTGGAGGAAGACGAGGTGGCCCTGGGGGGCCCCCTGGACCTTTGATGGAACAGGCGATC
GCACAAAAGGTGGCTGAACTGAAAAATAGAGTGGCCGTGAAGCTGAACCGGAACGAGCAGCTGAAGAACAAGGTG
GAAGAGCTGAAGAACAGAAACGCCTACCTGAAGAATGAGCTGGCCACCCTGGAAAACGAGGTGGCCAGACTGGAA
AACGACGTGGCCGAGGGAGCACCAGGAAGTGCTGGTTCTGCTGCTGGTAGTGGAGAGCAGAAGCTGATCTCAGAG
GAGGACCTGCTAGCCACCATGGACGCACAAACACGACGACGTGAGCGTCGCGCTGAGAAACAAGCTCAATGGAAA
GCTGCAAACCCACCGCTCGACGGAGCCGGAGCTGGCGCTGGAGCTGGAGCCGGAGCTGGCGGTCTAGCCACCATG
GACGCACAAACACGACGACGTGAGCGTCGCGCTGAGAAACAAGCTCAATGGAAAGCTGCAAACCCACCGCTCGAC
GGAGCCGGAGCTGGCGCTGGAGCTGGAGCCGGAGCTGGCGGTCTAGCCACCATGGACGCACAAACACGACGACGT
GAGCGTCGCGCTGAGAAACAAGCTCAATGGAAAGCTGCAAACCCACCGCTCGACGGAGCCGGAGCTGGCGCTGGA
GCTGGAGCCGGAGCTGGCGGTCTAGCCACCATGGACGCACAAACACGACGACGTGAGCGTCGCGCTGAGAAACAA
GCTCAATGGAAAGCTGCAAACCCACCGCTCGAGTCTAGAGGGCCCGTTTCTGGGCTAAGCGGTGCTCCGGGGTCA
GCCGGAAGTGCAGCAGGATCAGGTCAAAAGGTGGCTGAACTGAAAAATAGAGTGGCCGTGAAGCTGAACCGGAAC
GAGCAGCTGAAGAACAAGGTGGAAGAGCTGAAGAACAGAAACGCCTACCTGAAGAATGAGCTGGCCACCCTGGAA
AACGAGGTGGCCAGACTGGAAAACGACGTGGCCGAGGGCAAGCCTATTCCCAACCCCCTGCTGGGCCTGGATAGC
ACCGGAGCACCAGGAAGTGCTGGTTCTGCTGCTGGTAGTGGAGCGTGCCCGGTGCCGCTGCAGCTGCCGCCGCTG
GAACGCCTGACCCTGGATGACAAAAAACCGCTGAATACCCTGATCTCTGCTACTGGTCTGTGGATGAGTCGTACC
GGAACCATTCATAAAATCAAACACCACGAGGTTAGCCGTTCGAAAATCTATATTGAGATGGCGTGTGGCGATCAT
CTGGTTGTGAACAATAGCCGCTCTTCTCGTACAGCACGTGCACTGCGTCACCACAAATATCGTAAAACCTGTAAA
CGTTGCCGTGTGTCCGATGAGGATCTGAACAAATTCCTGACAAAAGCCAATGAGGACCAAACAAGCGTGAAAGTG
AAAGTCGTTAGCGCTCCTACCCGTACTAAAAAAGCAATGCCGAAATCCGTTGCTCGTGCCCCTAAACCACTGGAA
AACACTGAAGCAGCACAGGCACAGCCGTCTGGAAGCAAATTCTCTCCGGCCATTCCTGTTTCTACCCAGGAGTCC
GTTTCTGTTCCAGCAAGTGTGAGCACCAGCATTAGCAGTATTAGCACCGGTGCCACCGCTAGCGCCCTGGTTAAA
GGCAATACCAATCCGATTACAAGCATGTCTGCCCCGGTTCAAGCATCAGCTCCAGCACTGACAAAATCCCAAACC
GATCGTCTGGAGGTTCTGCTGAATCCGAAAGACGAAATCAGCCTGAATTCCGGCAAACCGTTTCGTGAACTGGAG
AGCGAACTGCTGTCACGTCGTAAAAAAGACCTGCAACAAATCTATGCCGAAGAACGTGAGAACTATCTGGGGAAA
CTGGAACGTGAAATCACCCGCTTTTTCGTGGATCGTGGCTTTCTGGAGATCAAATCCCCGATTCTGATTCCTCTG
GAGTATATCGAGCGTATGGGCATCGACAATGATACCGAACTGAGCAAACAAATTTTCCGTGTGGATAAAAACTTC
TGTCTGCGCCCTATGCTAGCACCAAATCTGGCTAACTATCTGCGCAAACTGGACCGTGCCCTGCCTGATCCTATC
AAAATCTTCGAGATCGGCCCGTGTTATCGTAAAGAGTCCGACGGTAAAGAACATCTGGAGGAGTTTACCATGCTG
AACTTTTGCCAAATGGGTTCAGGTTGTACTCGTGAGAACCTGGAAAGCATCATCACCGATTTTCTGAACCACCTG
GGCATTGACTTCAAAATTGTGGGCGACAGCTGTATGGTGTTTGGCGACACCCTGGATGTCATGCACGGCGACCTG
GAACTGTCTAGTGCCGTTGTGGGCCCAATCCCGCTGGATCGTGAGTGGGGTATCGACAAACCTTGGATCGGTGCG
GGTTTTGGTCTGGAGCGTCTGCTGAAAGTAAAACACGACTTCAAGAACATCAAACGTGCTGCACGTTCCGAGTCC
TATTACAATGGTATTTCTACTAACCTGTAA (SEQ ID NO: 625)

Protein:
MVGRNSAIAAGVCGALFIGYCIYFDRKRRSDPNFKNRLRERRKKQKLAKERAGLSKLPDLKDAEAVQKFFMASTD
YSTYSQAAAQQGYSAYTAQPTQGYAQTTQAYGQQSYGTYGQPTDVSYTQAQTTATYGQTAYATSYGQPPTGYTTP
TAPQAYSQPVQGYGTGAYDTTTATVTTTQASYAAQSAYGTQPAYPAYGQQPAATAPTRPQDGNKPTETSQPQSST
GGYNQPSLGYGQSNYSYPQVPGSYPMQPVTAPPSYPPTSYSSTQPTSYDQSSYSQQNTYGQPSSYGQQSSYGQQS
SYGQQPPTSYPPQTGSYSQAPSQYSQQSSSYGQQSSFRQDHPSSMGVYGQESGGFSGPGENRSMSGPDNRGRGRG
GFDRGGMSRGGRGGGRGGMGSAGERGGFNKPGGPMDEGPDLDLGPPVDPDEDSDNSAIYVQGLNDSVTLDDLADF
FKQCGVVKMNKRTGQPMIHIYLDKETGKPKGDATVSYEDPPTAKAAVEWFDGKDFQGSKLKVSLARKKPPMNSMR
GGLPPREGRGMPPPLRGGPGGPGGPGGPMGRMGGRGGDRGGFPPRGPRGSRGNPSGGGNVQHRAGDWQCPNPGCG
NQNFAWRTECNQCKAPKPEGFLPPPFPPPGGDRGRGGPGGMRGGRGGLMDRGGPGGMFRGGRGGDRGGFRGGRGM
DRGGFGGGRRGGPGGPPGPLMEQAIAQKVAELKNRVAVKLNRNEQLKNKVEELKNRNAYLKNELATLENEVARLE
NDVAEGAPGSAGSAAGSGEQKLISEEDLLATMDAQTRRRERRAEKQAQWKAANPPLDGAGAGAGAGAGAGGLATM
DAQTRRRERRAEKQAQWKAANPPLDGAGAGAGAGAGAGGLATMDAQTRRRERRAEKQAQWKAANPPLDGAGAGAG
AGAGAGGLATMDAQTRRRERRAEKQAQWKAANPPLESRGPVSGLSGAPGSAGSAAGSGQKVAELKNRVAVKLNRN
EQLKNKVEELKNRNAYLKNELATLENEVARLENDVAEGKPIPNPLLGLDSTGAPGSAGSAAGSGACPVPLQLPPL
ERLTLDDKKPLNTLISATGLWMSRTGTIHKIKHHEVSRSKIYIEMACGDHLVVNNSRSSRTARALRHHKYRKTCK
RCRVSDEDLNKFLTKANEDQTSVKVKVVSAPTRTKKAMPKSVARAPKPLENTEAAQAQPSGSKFSPAIPVSTQES
VSVPASVSTSISSISTGATASALVKGNTNPITSMSAPVQASAPALTKSQTDRLEVLLNPKDEISLNSGKPFRELE
SELLSRRKKDLQQIYAEERENYLGKLEREITRFFVDRGFLEIKSPILIPLEYIERMGIDNDTELSKQIFRVDKNF

CLRPMLAPNLANYLRKLDRALPDPIKIFEIGPCYRKESDGKEHLEEFTMLNFCQMGSGCTRENLESIITDFLNHL
GIDFKIVGDSCMVFGDTLDVMHGDLELSSAVVGPIPLDREWGIDKPWIGAGFGLERLLKVKHDFKNIKRAARSES
YYNGISTNL* (SEQ ID NO: 626)

TOM20::FUS::SYNZIP1::MCP::SYNZIP1::PylRS(AF)
DNA:
ATGGTGGGTCGGAACAGCGCCATCGCCGCCGGTGTATGCGGGGCCCTTTTCATTGGGTACTGCATCTACTTCGAC
CGCAAAAGACGAAGTGACCCCAACTTCAAGAACAGGCTTCGAGAACGAAGAAAGAAACAGAAGCTTGCCAAGGAG
AGAGCTGGGCTTTCCAAGTTACCTGACCTTAAAGATGCTGAAGCTGTTCAGAAATTCTTCATGGCCTCAAACGAT
TATACCCAACAAGCAACCCAAAGCTATGGGGCCTACCCCACCCAGCCCGGGCAGGGCTATTCCCAGCAGAGCAGT
CAGCCCTACGGACAGCAGAGTTACAGTGGTTATAGCCAGTCCACGGACACTTCAGGATATGGCCAGAGCAGCTAT
TCTTCTTATGGCCAGAGCCAGAACACAGGCTATGGAACTCAGTCAACTCCCCAGGGATATGGCTCGACTGGCGGC
TATGGCAGTAGCCAGAGCTCCCAATCGTCTTACGGGCAGCAGTCCTCCTACCCTGGCTATGGCCAGCAGCCAGCT
CCCAGCAGCACCTCGGGAAGTTACGGTAGCAGTTCTCAGAGCAGCAGCTATGGGCAGCCCCAGAGTGGGAGCTAC
AGCCAGCAGCCTAGCTATGGTGGACAGCAGCAAAGCTATGGACAGCAGCAAAGCTATAATCCCCCTCAGGGCTAT
GGACAGCAGAACCAGTACAACAGCAGCAGTGGTGGTGGAGGTGGAGGTGGAGGTGGAGGTAACTATGGCCAAGAT
CAATCCTCCATGAGTAGTGGTGGTGGCAGTGGTGGCGGTTATGGCAATCAAGACCAGAGTGGTGGAGGTGGCAGC
GGTGGCTATGGACAGCAGGACCGTGGAGGCCGCGGCAGGGGTGGCAGTGGTGGCGGCGGCGGCGGCGGCGGTGGT
GGTTACAACCGCAGCAGTGGTGGCTATGAACCCAGAGGTCGTGGAGGTGGCCGTGGAGGCAGAGGTGGCATGGGC
GGAAGTGACCGTGGTGGCTTCAATAAATTTGGTGGCCCTCGGGACCAAGGATCACGTCATGACTCCGAACAGGAT
AATTCAGACAACAACACCATCTTTGTGCAAGGCCTGGGTGAGAATGTTACAATTGAGTCTGTGGCTGATTACTTC
AAGCAGATTGGTATTATTAAGACAAACAAGAAAACGGGACAGCCCATGATTAATTTGTACACAGACAGGGAAACT
GGCAAGCTGAAGGGAGAGGCAACGGTCTCTTTTGATGACCCACCTTCAGCTAAAGCAGCTATTGACTGGTTTGAT
GGTAAAGAATTCTCCGGAAATCCTATCAAGGTCTCATTTGCTACTCGCCGGGCAGACTTTAATCGGGGTGGTGGC
AATGGTCGTGGAGGCCGAGGGCGAGGAGGACCCATGGGCCGTGGAGGCTATGGAGGTGGTGGCAGTGGTGGTGGT
GGCCGAGGAGGATTTCCCAGTGGAGGTGGTGGCGGTGGAGGACAGCAGCGAGCTGGTGACTGGAAGTGTCCTAAT
CCCACCTGTGAGAATATGAACTTCTCTTGGAGGAATGAATGCAACCAGTGTAAGGCCCCTAAACCAGATGGCCCA
GGAGGGGGACCAGGTGGCTCTCACATGGGGGGTAACTACGGGGATGATCGTCGTGGTGGCAGAGGAGGCGCGATC
GCAAATCTGGTGGCCCAGCTGGAAAACGAGGTGGCCAGCCTGGAAAACGAGAACGAAACCCTGAAGAAAAAGAAC
CTGCACAAGAAGGACCTGATCGCCTACCTGGAAAAGGAAATCGCCAACCTGAGAAAGAAGATCGAGGAAGCATCG
ATATATCCCTATGATGTGCCGGATTATGCTGGAGCACCAGGAAGTGCTGGTTCTGCTGCTGGTAGTGGAGCTTCT
AACTTTACTCAGTTCGTTCTCGTCGACAATGGCGGAACTGGCGACGTGACTGTCGCCCCAAGCAACTTCGCTAAC
GGGATCGCTGAATGGATCAGCTCTAACTCGCGTTCACAGGCTTACAAAGTAACCTGTAGCGTTCGTCAGAGCTCT
GCGCAGAATCGCAAATACACCATCAAAGTCGAGGTGCCTAAAGGCGCCTGGCGTTCGTACTTAAATATGGAACTA
ACCATTCCAATTTTCGCCACGAATTCCGACTGCGAGCTTATTGTTAAGGCAATGCAAGGTCTCCTAAAAGATGGA
AACCCGATTCCCTCAGCAATCGCAGCAAACTCCGGCATCTACGGGCTAAGCGGTGCTCCGGGGTCAGCCGGAAGT
GCAGCAGGATCAGGTAATCTGGTGGCCCAGCTGGAAAACGAGGTGGCCAGCCTGGAAAACGAGAACGAAACCCTG
AAGAAAAAGAACCTGCACAAGAAGGACCTGATCGCCTACCTGGAAAAGGAAATCGCCAACCTGAGAAAGAAGATC
GAGGAAGGCAAGCCTATTCCCAACCCCCTGCTGGGCCTGGATAGCACCGGAGCACCAGGAAGTGCTGGTTCTGCT
GCTGGTAGTGGAGCGTGCCCGGTGCCGCTGCAGCTGCCGCCGCTGGAACGCCTGACCCTGGATGACAAAAAACCG
CTGAATACCCTGATCTCTGCTACTGGTCTGTGGATGAGTCGTACCGGAACCATTCATAAAATCAAACACCACGAG
GTTAGCCGTTCGAAAATCTATATTGAGATGGCGTGTGGCGATCATCTGGTTGTGAACAATAGCCGCTCTTCTCGT
ACAGCACGTGCACTGCGTCACCACAAATATCGTAAAACCTGTAAACGTTGCCGTGTGTCCGATGAGGATCTGAAC
AAATTCCTGACAAAAGCCAATGAGGACCAAACAAGCGTGAAAGTGAAAGTCGTTAGCGCTCCTACCCGTACTAAA
AAAGCAATGCCGAAATCCGTTGCTCGTGCCCCTAAACCACTGGAAAACACTGAAGCAGCACAGGCACAGCCGTCT
GGAAGCAAATTCTCTCCGGCCATTCCTGTTTCTACCCAGGAGTCCGTTTCTGTTCCAGCAAGTGTGAGCACCAGC
ATTAGCAGTATTAGCACCGGTGCCACCGCTAGCGCCCTGGTTAAAGGCAATACCAATCCGATTACAAGCATGTCT
GCCCCGGTTCAAGCATCAGCTCCAGCACTGACAAAATCCCAAACCGATCGTCTGGAGGTTCTGCTGAATCCGAAA
GACGAAATCAGCCTGAATTCCGGCAAACCGTTTCGTGAACTGGAGAGCGAACTGCTGTCACGTCGTAAAAAAGAC
CTGCAACAAATCTATGCCGAAGAACGTGAGAACTATCTGGGGAAACTGGAACGTGAAATCACCCGCTTTTTCGTG
GATCGTGGCTTTCTGGAGATCAAATCCCCGATTCTGATTCCTCTGGAGTATATCGAGCGTATGGGCATCGACAAT
GATACCGAACTGAGCAAACAAATTTTCCGTGTGGATAAAAACTTCTGTCTGCGCCCTATGCTAGCACCAAATCTG
GCTAACTATCTGCGCAAACTGGACCGTGCCCTGCCTGATCCTATCAAAATCTTCGAGATCGGCCCGTGTTATCGT
AAAGAGTCCGACGGTAAAGAACATCTGGAGGAGTTTACCATGCTGAACTTTTGCCAAATGGGTTCAGGTTGTACT
CGTGAGAACCTGGAAAGCATCATCACCGATTTTCTGAACCACCTGGGCATTGACTTCAAAATTGTGGGCGACAGC
TGTATGGTGTTTGGCGACACCCTGGATGTCATGCACGGCGACCTGGAACTGTCTAGTGCCGTTGTGGGCCCAATC
CCGCTGGATCGTGAGTGGGGTATCGACAAACCTTGGATCGGTGCGGGTTTTGGTCTGGAGCGTCTGCTGAAAGTA

AAACACGACTTCAAGAACATCAAACGTGCTGCACGTTCCGAGTCCTATTACAATGGTATTTCTACTAACCTGTAA
(SEQ ID NO: 627)

Protein:
MVGRNSAIAAGVCGALFIGYCIYFDRKRRSDPNFKNRLRERRKKQKLAKERAGLSKLPDLKDAEAVQKFFMASND
YTQQATQSYGAYPTQPGQGYSQQSSQPYGQQSYSGYSQSTDTSGYGQSSYSSYGQSQNTGYGTQSTPQGYGSTGG
YGSSQSSQSSYGQQSSYPGYGQQPAPSSTSGSYGSSSQSSSYGQPQSGSYSQQPSYGGQQQSYGQQQSYNPPQGY
GQQNQYNSSSGGGGGGGGGGNYGQDQSSMSSGGGSGGGYGNQDQSGGGGSGGYGQQDRGGRGRGGSGGGGGGGGG
GYNRSSGGYEPRGRGGGRGGRGGMGGSDRGGFNKFGGPRDQGSRHDSEQDNSDNNTIFVQGLGENVTIESVADYF
KQIGIIKTNKKTGQPMINLYTDRETGKLKGEATVSFDDPPSAKAAIDWFDGKEFSGNPIKVSFATRRADFNRGGG
NGRGGRGRGGPMGRGGYGGGGSGGGGRGGFPSGGGGGGGQQRAGDWKCPNPTCENMNFSWRNECNQCKAPKPDGP
GGGPGGSHMGGNYGDDRRGGRGGAIANLVAQLENEVASLENENETLKKKNLHKKDLIAYLEKEIANLRKKIEEAS
IYPYDVPDYAGAPGSAGSAAGSGASNFTQFVLVDNGGTGDVTVAPSNFANGIAEWISSNSRSQAYKVTCSVRQSS
AQNRKYTIKVEVPKGAWRSYLNMELTIPIFATNSDCELIVKAMQGLLKDGNPIPSAIAANSGIYGLSGAPGSAGS
AAGSGNLVAQLENEVASLENENETLKKKNLHKKDLIAYLEKEIANLRKKIEEGKPIPNPLLGLDSTGAPGSAGSA
AGSGACPVPLQLPPLERLTLDDKKPLNTLISATGLWMSRTGTIHKIKHHEVSRSKIYIEMACGDHLVVNNSRSSR
TARALRHHKYRKTCKRCRVSDEDLNKFLTKANEDQTSVKVKVVSAPTRTKKAMPKSVARAPKPLENTEAAQAQPS
GSKFSPAIPVSTQESVSVPASVSTSISSISTGATASALVKGNTNPITSMSAPVQASAPALTKSQTDRLEVLLNPK
DEISLNSGKPFRELESELLSRRKKDLQQIYAEERENYLGKLEREITRFFVDRGFLEIKSPILIPLEYIERMGIDN
DTELSKQIFRVDKNFCLRPMLAPNLANYLRKLDRALPDPIKIFEIGPCYRKESDGKEHLEEFTMLNFCQMGSGCT
RENLESIITDFLNHLGIDFKIVGDSCMVFGDTLDVMHGDLELSSAVVGPIPLDREWGIDKPWIGAGFGLERLLKV
KHDFKNIKRAARSESYYNGISTNL* (SEQ ID NO: 628)

TOM20::EWSR1::SYNZIP4::4xλN22::SYNZIP4::PylRS(AA)
DNA:
ATGGTGGGTCGGAACAGCGCCATCGCCGCCGGTGTATGCGGGGCCCTTTTCATTGGGTACTGCATCTACTTCGAC
CGCAAAAGACGAAGTGACCCCAACTTCAAGAACAGGCTTCGAGAACGAAGAAAGAAACAGAAGCTTGCCAAGGAG
AGAGCTGGGCTTTCCAAGTTACCTGACCTTAAAGATGCTGAAGCTGTTCAGAAGTTCTTCATGGCGTCCACGGAT
TACAGTACCTATAGCCAAGCTGCAGCGCAGCAGGGCTACAGTGCTTACACCGCCCAGCCCACTCAAGGATATGCA
CAGACCACCCAGGCATATGGGCAACAAAGCTATGGAACCTATGGACAGCCCACTGATGTCAGCTATACCCAGGCT
CAGACCACTGCAACCTATGGGCAGACCGCCTATGCAACTTCTTATGGACAGCCTCCCACTGGTTATACTACTCCA
ACTGCCCCCCAGGCATACAGCCAGCCTGTCCAGGGGTATGGCACTGGTGCTTATGATACCACCACTGCTACAGTC
ACCACCACCCAGGCCTCCTATGCAGCTCAGTCTGCATATGGCACTCAGCCTGCTTATCCAGCCTATGGGCAGCAG
CCAGCAGCCACTGCACCTACAAGACCGCAGGATGGAAACAAGCCCACTGAGACTAGTCAACCTCAATCTAGCACA
GGGGGTTACAACCAGCCCAGCCTAGGATATGGACAGAGTAACTACAGTTATCCCCAGGTACCTGGGAGCTACCCC
ATGCAGCCAGTCACTGCACCTCCATCCTACCCTCCTACCAGCTATTCCTCTACACAGCCGACTAGTTATGATCAG
AGCAGTTACTCTCAGCAGAACACCTATGGGCAACCGAGCAGCTATGGACAGCAGAGTAGCTATGGTCAACAAAGC
AGCTATGGGCAGCAGCCTCCCACTAGTTACCCACCCCAAACTGGATCCTACAGCCAAGCTCCAAGTCAATATAGC
CAACAGAGCAGCAGCTACGGGCAGCAGAGTTCATTCCGACAGGACCACCCCAGTAGCATGGGTGTTTATGGGCAG
GAGTCTGGAGGATTTTCCGGACCAGGAGAGAACCGGAGCATGAGTGGCCCTGATAACCGGGGCAGGGGAAGAGGG
GGATTTGATCGTGGAGGCATGAGCAGAGGTGGGCGGGGAGGAGGACGCGGTGGAATGGGCAGCGCTGGAGAGCGA
GGTGGCTTCAATAAGCCTGGTGGACCCATGGATGAAGGACCAGATCTTGATCTAGGCCCACCTGTAGATCCAGAT
GAAGACTCTGACAACAGTGCAATTTATGTACAAGGATTAAATGACAGTGTGACTCTAGATGATCTGGCAGACTTC
TTTAAGCAGTGTGGGGTTGTTAAGATGAACAAGAGAACTGGGCAACCCATGATCCACATCTACCTGGACAAGGAA
ACAGGAAAGCCCAAAGGCGATGCCACAGTGTCCTATGAAGACCCACCTACTGCCAAGGCTGCCGTGGAATGGTTT
GATGGGAAAGATTTTCAAGGGAGCAAACTTAAAGTCTCCCTTGCTCGGAAGAAGCCTCCAATGAACAGTATGCGG
GGTGGTCTGCCACCCCGTGAGGGCAGAGGCATGCCACCACCACTCCGTGGAGGTCCAGGAGGCCCAGGAGGTCCT
GGGGGACCCATGGGTCGCATGGGAGGCCGTGGAGGAGATAGAGGAGGCTTCCCTCCAAGAGGACCCCGGGGTTCC
CGAGGGAACCCCTCTGGAGGAGGAAACGTCCAGCACCGAGCTGGAGACTGGCAGTGTCCCAATCCGGGTTGTGGA
AACCAGAACTTCGCCTGGAGAACAGAGTGCAACCAGTGTAAGGCCCCAAAGCCTGAAGGCTTCCTCCCGCCACCC
TTTCCGCCCCCGGGTGGTGATCGTGGCAGAGGTGGCCCTGGTGGCATGCGGGGAGGAAGAGGTGGCCTCATGGAT
CGTGGTGGTCCCGGTGGAATGTTCAGAGGTGGCCGTGGTGGAGACAGAGGTGGCTTCCGTGGTGGCCGGGGCATG
GACCGAGGTGGCTTTGGTGGAGGAAGACGAGGTGGCCCTGGGGGGCCCCCTGGACCTTTGATGGAACAGGCGATC
GCACAAAAGGTGGCTGAACTGAAAAATAGAGTGGCCGTGAAGCTGAACCGGAACGAGCAGCTGAAGAACAAGGTG
GAAGAGCTGAAGAACAGAAACGCCTACCTGAAGAATGAGCTGGCCACCCTGGAAAACGAGGTGGCCAGACTGGAA
AACGACGTGGCCGAGGGAGCACCAGGAAGTGCTGGTTCTGCTGCTGGTAGTGGAGAGCAGAAGCTGATCTCAGAG
GAGGACCTGCTAGCCACCATGGACGCACAAACACGACGACGTGAGCGTCGCGCTGAGAAACAAGCTCAATGGAAA

GCTGCAAACCCACCGCTCGACGGAGCCGGAGCTGGCGCTGGAGCTGGAGCCGGAGCTGGCGGTCTAGCCACCATG
GACGCACAAACACGACGACGTGAGCGTCGCGCTGAGAAACAAGCTCAATGGAAAGCTGCAAACCCACCGCTCGAC
GGAGCCGGAGCTGGCGCTGGAGCTGGAGCCGGAGCTGGCGGTCTAGCCACCATGGACGCACAAACACGACGACGT
GAGCGTCGCGCTGAGAAACAAGCTCAATGGAAAGCTGCAAACCCACCGCTCGACGGAGCCGGAGCTGGCGCTGGA
GCTGGAGCCGGAGCTGGCGGTCTAGCCACCATGGACGCACAAACACGACGACGTGAGCGTCGCGCTGAGAAACAA
GCTCAATGGAAAGCTGCAAACCCACCGCTCGAGTCTAGAGGGCCCGTTTCTGGGCTAAGCGGTGCTCCGGGGTCA
GCCGGAAGTGCAGCAGGATCAGGTCAAAAGGTGGCTGAACTGAAAAATAGAGTGGCCGTGAAGCTGAACCGGAAC
GAGCAGCTGAAGAACAAGGTGGAAGAGCTGAAGAACAGAAACGCCTACCTGAAGAATGAGCTGGCCACCCTGGAA
AACGAGGTGGCCAGACTGGAAAACGACGTGGCCGAGGGCAAGCCTATTCCCAACCCCCTGCTGGGCCTGGATAGC
ACCGGAGCACCAGGAAGTGCTGGTTCTGCTGCTGGTAGTGGAGCGTGCCCGGTGCCGCTGCAGCTGCCGCCGCTG
GAACGCCTGACCCTGGATGACAAAAAACCGCTGAATACCCTGATCTCTGCTACTGGTCTGTGGATGAGTCGTACC
GGAACCATTCATAAAATCAAACACCACGAGGTTAGCCGTTCGAAAATCTATATTGAGATGGCGTGTGGCGATCAT
CTGGTTGTGAACAATAGCCGCTCTTCTCGTACAGCACGTGCACTGCGTCACCACAAATATCGTAAAACCTGTAAA
CGTTGCCGTGTGTCCGATGAGGATCTGAACAAATTCCTGACAAAAGCCAATGAGGACCAAACAAGCGTGAAAGTG
AAAGTCGTTAGCGCTCCTACCCGTACTAAAAAAGCAATGCCGAAATCCGTTGCTCGTGCCCCTAAACCACTGGAA
AACACTGAAGCAGCACAGGCACAGCCGTCTGGAAGCAAATTCTCTCCGGCCATTCCTGTTTCTACCCAGGAGTCC
GTTTCTGTTCCAGCAAGTGTGAGCACCAGCATTAGCAGTATTAGCACCGGTGCCACCGCTAGCGCCCTGGTTAAA
GGCAATACCAATCCGATTACAAGCATGTCTGCCCCGGTTCAAGCATCAGCTCCAGCACTGACAAAATCCCAAACC
GATCGTCTGGAGGTTCTGCTGAATCCGAAAGACGAAATCAGCCTGAATTCCGGCAAACCGTTTCGTGAACTGGAG
AGCGAACTGCTGTCACGTCGTAAAAAAGACCTGCAACAAATCTATGCCGAAGAACGTGAGAACTATCTGGGGAAA
CTGGAACGTGAAATCACCCGCTTTTTCGTGGATCGTGGCTTTCTGGAGATCAAATCCCCGATTCTGATTCCTCTG
GAGTATATCGAGCGTATGGGCATCGACAATGATACCGAACTGAGCAAACAAATTTTCCGTGTGGATAAAAACTTC
TGTCTGCGCCCTATGCTGGCACCAAATCTGTATAACTATCTGCGCAAACTGGACCGTGCCCTGCCTGATCCTATC
AAAATCTTCGAGATCGGCCCGTGTTATCGTAAAGAGTCCGACGGTAAAGAACATCTGGAGGAGTTTACCATGCTG
GCCTTTGCCCAAATGGGTTCAGGTTGTACTCGTGAGAACCTGGAAAGCATCATCACCGATTTTCTGAACCACCTG
GGCATTGACTTCAAAATTGTGGGCGACAGCTGTATGGTGTATGGCGACACCCTGGATGTCATGCACGGCGACCTG
GAACTGTCTAGTGCCGTTGTTGGACCAATTCCGCTGGACCGTGAGTGGGGTATCGACAAACCGTGGATCGGAGCA
GGATTCGGTCTGGAACGCCTGCTGAAAGTGAAACACGACTTCAAAAACATCAAACGTGCCGCCCGTTCTGAATCG
TATTATAACGGGATCTCTACGAACCTGTAA (SEQ ID NO: 629)

Protein:
MVGRNSAIAAGVCGALFIGYCIYFDRKRRSDPNFKNRLRERRKKQKLAKERAGLSKLPDLKDAEAVQKFFMASTD
YSTYSQAAAQQGYSAYTAQPTQGYAQTTQAYGQQSYGTYGQPTDVSYTQAQTTATYGQTAYATSYGQPPTGYTTP
TAPQAYSQPVQGYGTGAYDTTTATVTTTQASYAAQSAYGTQPAYPAYGQQPAATAPTRPQDGNKPTETSQPQSST
GGYNQPSLGYGQSNYSYPQVPGSYPMQPVTAPPSYPPTSYSSTQPTSYDQSSYSQQNTYGQPSSYGQQSSYGQQS
SYGQQPPTSYPPQTGSYSQAPSQYSQQSSSYGQQSSFRQDHPSSMGVYGQESGGFSGPGENRSMSGPDNRGRGRG
GFDRGGMSRGGRGGGRGGMGSAGERGGFNKPGGPMDEGPDLDLGPPVDPDEDSDNSAIYVQGLNDSVTLDDLADF
FKQCGVVKMNKRTGQPMIHIYLDKETGKPKGDATVSYEDPPTAKAAVEWFDGKDFQGSKLKVSLARKKPPMNSMR
GGLPPREGRGMPPPLRGGPGGPGGPGGPMGRMGGRGGDRGGFPPRGPRGSRGNPSGGGNVQHRAGDWQCPNPGCG
NQNFAWRTECNQCKAPKPEGFLPPPFPPPGGDRGRGGPGGMRGGRGGLMDRGGPGGMFRGGRGGDRGGFRGGRGM
DRGGFGGGRRGGPGGPPGPLMEQAIAQKVAELKNRVAVKLNRNEQLKNKVEELKNRNAYLKNELATLENEVARLE
NDVAEGAPGSAGSAAGSGEQKLISEEDLLATMDAQTRRRERRAEKQAQWKAANPPLDGAGAGAGAGAGAGGLATM
DAQTRRRERRAEKQAQWKAANPPLDGAGAGAGAGAGAGGLATMDAQTRRRERRAEKQAQWKAANPPLDGAGAGAG
AGAGAGGLATMDAQTRRRERRAEKQAQWKAANPPLESRGPVSGLSGAPGSAGSAAGSGQKVAELKNRVAVKLNRN
EQLKNKVEELKNRNAYLKNELATLENEVARLENDVAEGKPIPNPLLGLDSTGAPGSAGSAAGSGACPVPLQLPPL
ERLTLDDKKPLNTLISATGLWMSRTGTIHKIKHHEVSRSKIYIEMACGDHLVVNNSRSSRTARALRHHKYRKTCK
RCRVSDEDLNKFLTKANEDQTSVKVKVVSAPTRTKKAMPKSVARAPKPLENTEAAQAQPSGSKFSPAIPVSTQES
VSVPASVSTSISSISTGATASALVKGNTNPITSMSAPVQASAPALTKSQTDRLEVLLNPKDEISLNSGKPFRELE
SELLSRRKKDLQQIYAEERENYLGKLEREITRFFVDRGFLEIKSPILIPLEYIERMGIDNDTELSKQIFRVDKNF
CLRPMLAPNLYNYLRKLDRALPDPIKIFEIGPCYRKESDGKEHLEEFTMLAFAQMGSGCTRENLESIITDFLNHL
GIDFKIVGDSCMVYGDTLDVMHGDLELSSAVVGPIPLDREWGIDKPWIGAGFGLERLLKVKHDFKNIKRAARSES
YYNGISTNL* (SEQ ID NO: 630)

TOM20::FUS::SYNZIP3::4xλN22::SYNZIP3::PylRS(AA)
DNA:
ATGGTGGGTCGGAACAGCGCCATCGCCGCCGGTGTATGCGGGGCCCTTTTCATTGGGTACTGCATCTACTTCGAC
CGCAAAAGACGAAGTGACCCCAACTTCAAGAACAGGCTTCGAGAACGAAGAAAGAAACAGAAGCTTGCCAAGGAG

AGAGCTGGGCTTTCCAAGTTACCTGACCTTAAAGATGCTGAAGCTGTTCAGAAATTCTTCATGGCCTCAAACGAT
TATACCCAACAAGCAACCCAAAGCTATGGGGCCTACCCCACCCAGCCCGGGCAGGGCTATTCCCAGCAGAGCAGT
CAGCCCTACGGACAGCAGAGTTACAGTGGTTATAGCCAGTCCACGGACACTTCAGGATATGGCCAGAGCAGCTAT
TCTTCTTATGGCCAGAGCCAGAACACAGGCTATGGAACTCAGTCAACTCCCCAGGGATATGGCTCGACTGGCGGC
TATGGCAGTAGCCAGAGCTCCCAATCGTCTTACGGGCAGCAGTCCTCCTACCCTGGCTATGGCCAGCAGCCAGCT
CCCAGCAGCACCTCGGGAAGTTACGGTAGCAGTTCTCAGAGCAGCAGCTATGGGCAGCCCCAGAGTGGGAGCTAC
AGCCAGCAGCCTAGCTATGGTGGACAGCAGCAAAGCTATGGACAGCAGCAAAGCTATAATCCCCCTCAGGGCTAT
GGACAGCAGAACCAGTACAACAGCAGCAGTGGTGGTGGAGGTGGAGGTGGAGGTGGAGGTAACTATGGCCAAGAT
CAATCCTCCATGAGTAGTGGTGGTGGCAGTGGTGGCGGTTATGGCAATCAAGACCAGAGTGGTGGAGGTGGCAGC
GGTGGCTATGGACAGCAGGACCGTGGAGGCCGCGGCAGGGGTGGCAGTGGTGGCGGCGGCGGCGGCGGTGGT
GGTTACAACCGCAGCAGTGGTGGCTATGAACCCAGAGGTCGTGGAGGTGGCCGTGGAGGCAGAGGTGGCATGGGC
GGAAGTGACCGTGGTGGCTTCAATAAATTTGGTGGCCCTCGGGACCAAGGATCACGTCATGACTCCGAACAGGAT
AATTCAGACAACAACACCATCTTTGTGCAAGGCCTGGGTGAGAATGTTACAATTGAGTCTGTGGCTGATTACTTC
AAGCAGATTGGTATTATTAAGACAAACAAGAAAACGGGACAGCCCATGATTAATTTGTACACAGACAGGGAAACT
GGCAAGCTGAAGGGAGAGGCAACGGTCTCTTTTGATGACCCACCTTCAGCTAAAGCAGCTATTGACTGGTTTGAT
GGTAAAGAATTCTCCGGAAATCCTATCAAGGTCTCATTTGCTACTCGCCGGGCAGACTTTAATCGGGGTGGTGGC
AATGGTCGTGGAGGCCGAGGGCGAGGAGGACCCATGGGCCGTGGAGGCTATGGAGGTGGTGGCAGTGGTGGTGGT
GGCCGAGGAGGATTTCCCAGTGGAGGTGGTGGCGGTGGAGGACAGCAGCGAGCTGGTGACTGGAAGTGTCCTAAT
CCCACCTGTGAGAATATGAACTTCTCTTGGAGGAATGAATGCAACCAGTGTAAGGCCCCTAAACCAGATGGCCCA
GGAGGGGGACCAGGTGGCTCTCACATGGGGGGTAACTACGGGGATGATCGTCGTGGTGGCAGAGGAGGCGCGATC
GCAAATGAGGTGACCACCCTGGAAAACGACGCCGCCTTCATCGAGAACGAGAACGCCTACCTGGAAAAAGAGATC
GCCAGACTGAGAAAGGAAAAGGCCGCTCTGCGGAACAGACTGGCCCACAAGAAGGGAGCACCAGGAAGTGCTGGT
TCTGCTGCTGGTAGTGGAGCATCGATAGAGCAGAAGCTGATCTCAGAGGAGGACCTGCTAGCCACCATGGACGCA
CAAACACGACGACGTGAGCGTCGCGCTGAGAAACAAGCTCAATGGAAAGCTGCAAACCCACCGCTCGACGGAGCC
GGAGCTGGCGCTGGAGCTGGAGCCGGAGCTGGCGGTCTAGCCACCATGGACGCACAAACACGACGACGTGAGCGT
CGCGCTGAGAAACAAGCTCAATGGAAAGCTGCAAACCCACCGCTCGACGGAGCCGGAGCTGGCGCTGGAGCTGGA
GCCGGAGCTGGCGGTCTAGCCACCATGGACGCACAAACACGACGACGTGAGCGTCGCGCTGAGAAACAAGCTCAA
TGGAAAGCTGCAAACCCACCGCTCGACGGAGCCGGAGCTGGCGCTGGAGCTGGAGCCGGAGCTGGCGGTCTAGCC
ACCATGGACGCACAAACACGACGACGTGAGCGTCGCGCTGAGAAACAAGCTCAATGGAAAGCTGCAAACCCACCG
CTCGAGTCTAGAGGGCTAAGCGGTGCTCCGGGGTCAGCCGGAAGTGCAGCAGGATCAGGTAATGAGGTGACCACC
CTGGAAAACGACGCCGCCTTCATCGAGAACGAGAACGCCTACCTGGAAAAAGAGATCGCCAGACTGAGAAAGGAA
AAGGCCGCTCTGCGGAACAGACTGGCCCACAAGAAGGGCAAGCCTATTCCCAACCCCCTGCTGGGCCTGGATAGC
ACCGGAGCACCAGGAAGTGCTGGTTCTGCTGCTGGTAGTGGAGCGTGCCCGGTGCCGCTGCAGCTGCCGCCGCTG
GAACGCCTGACCCTGGATGACAAAAAACCGCTGAATACCCTGATCTCTGCTACTGGTCTGTGGATGAGTCGTACC
GGAACCATTCATAAAATCAAACACCACGAGGTTAGCCGTTCGAAAATCTATATTGAGATGGCGTGTGGCGATCAT
CTGGTTGTGAACAATAGCCGCTCTTCTCGTACAGCACGTGCACTGCGTCACCACAAATATCGTAAAACCTGTAAA
CGTTGCCGTGTGTCCGATGAGGATCTGAACAAATTCCTGACAAAAGCCAATGAGGACCAAACAAGCGTGAAAGTG
AAAGTCGTTAGCGCTCCTACCCGTACTAAAAAAGCAATGCCGAAATCCGTTGCTCGTGCCCCTAAACCACTGGAA
AACACTGAAGCAGCACAGGCACAGCCGTCTGGAAGCAAATTCTCTCCGGCCATTCCTGTTTCTACCCAGGAGTCC
GTTTCTGTTCCAGCAAGTGTGAGCACCAGCATTAGCAGTATTAGCACCGGTGCCACCGCTAGCGCCCTGGTTAAA
GGCAATACCAATCCGATTACAAGCATGTCTGCCCCGGTTCAAGCATCAGCTCCAGCACTGACAAAATCCCAAACC
GATCGTCTGGAGGTTCTGCTGAATCCGAAAGACGAAATCAGCCTGAATTCCGGCAAACCGTTTCGTGAACTGGAG
AGCGAACTGCTGTCACGTCGTAAAAAAGACCTGCAACAAATCTATGCCGAAGAACGTGAGAACTATCTGGGGAAA
CTGGAACGTGAAATCACCCGCTTTTTCGTGGATCGTGGCTTTCTGGAGATCAAATCCCCGATTCTGATTCCTCTG
GAGTATATCGAGCGTATGGGCATCGACAATGATACCGAACTGAGCAAACAAATTTTCCGTGTGGATAAAAACTTC
TGTCTGCGCCCTATGCTGGCACCAAATCTGTATAACTATCTGCGCAAACTGGACCGTGCCCTGCCTGATCCTATC
AAAATCTTCGAGATCGGCCCGTGTTATCGTAAAGAGTCCGACGGTAAAGAACATCTGGAGGAGTTTACCATGCTG
GCCTTTGCCCAAATGGGTTCAGGTTGTACTCGTGAGAACCTGGAAAGCATCATCACCGATTTTCTGAACCACCTG
GGCATTGACTTCAAAATTGTGGGCGACAGCTGTATGGTGTATGGCGACACCCTGGATGTCATGCACGGCGACCTG
GAACTGTCTAGTGCCGTTGTTGGACCAATTCCGCTGGACCGTGAGTGGGGTATCGACAAACCGTGGATCGGAGCA
GGATTCGGTCTGGAACGCCTGCTGAAAGTGAAACACGACTTCAAAAACATCAAACGTGCCGCCCGTTCTGAATCG
TATTATAACGGGATCTCTACGAACCTGTAA (SEQ ID NO: 631)

Protein:
MVGRNSAIAAGVCGALFIGYCIYFDRKRRSDPNFKNRLRERRKKQKLAKERAGLSKLPDLKDAEAVQKFFMASND
YTQQATQSYGAYPTQPGQGYSQQSSQPYGQQSYSGYSQSTDTSGYGQSSYSSYGQSQNTGYGTQSTPQGYGSTGG
YGSSQSSQSSYGQQSSYPGYGQQPAPSSTSGSYGSSSQSSSYGQPQSGSYSQQPSYGGQQQSYGQQQSYNPPQGY
GQQNQYNSSSGGGGGGGGGNYGQDQSSMSSGGGSGGGYGNQDQSGGGGSGGYGQQDRGGRGRGGSGGGGGGGG

GYNRSSGGYEPRGRGGGRGGRGGMGGSDRGGFNKFGGPRDQGSRHDSEQDNSDNNTIFVQGLGENVTIESVADYF
KQIGIIKTNKKTGQPMINLYTDRETGKLKGEATVSFDDPPSAKAAIDWFDGKEFSGNPIKVSFATRRADFNRGGG
NGRGGRGRGGPMGRGGYGGGGSGGGGRGGFPSGGGGGGGQQRAGDWKCPNPTCENMNFSWRNECNQCKAPKPDGP
GGGPGGSHMGGNYGDDRRGGRGGAIANEVTTLENDAAFIENENAYLEKEIARLRKEKAALRNRLAHKKGAPGSAG
SAAGSGASIEQKLISEEDLLATMDAQTRRRERRAEKQAQWKAANPPLDGAGAGAGAGAGAGGLATMDAQTRRRER
RAEKQAQWKAANPPLDGAGAGAGAGAGAGGLATMDAQTRRRERRAEKQAQWKAANPPLDGAGAGAGAGAGAGGLA
TMDAQTRRRERRAEKQAQWKAANPPLESRGLSGAPGSAGSAAGSGNEVTTLENDAAFIENENAYLEKEIARLRKE
KAALRNRLAHKKGKPIPNPLLGLDSTGAPGSAGSAAGSGACPVPLQLPPLERLTLDDKKPLNTLISATGLWMSRT
GTIHKIKHHEVSRSKIYIEMACGDHLVVNNSRSSRTARALRHHKYRKTCKRCRVSDEDLNKFLTKANEDQTSVKV
KVVSAPTRTKKAMPKSVARAPKPLENTEAAQAQPSGSKFSPAIPVSTQESVSVPASVSTSISSISTGATASALVK
GNTNPITSMSAPVQASAPALTKSQTDRLEVLLNPKDEISLNSGKPFRELESELLSRRKKDLQQIYAEERENYLGK
LEREITRFFVDRGFLEIKSPILIPLEYIERMGIDNDTELSKQIFRVDKNFCLRPMLAPNLYNYLRKLDRALPDPI
KIFEIGPCYRKESDGKEHLEEFTMLAFAQMGSGCTRENLESIITDFLNHLGIDFKIVGDSCMVYGDTLDVMHGDL
ELSSAVVGPIPLDREWGIDKPWIGAGFGLERLLKVKHDFKNIKRAARSESYYNGISTNL*  (SEQ  ID  NO:
632)

LCK::EWSR1::SYNZIP4::4xλN22::SYNZIP4::PylRS(AF)
DNA:
ATGGGCTGCGTGTGCAGCAGCAACCCCGAGGGTACCGAGCTCATGGCGTCCACGGATTACAGTACCTATAGCCAA
GCTGCAGCGCAGCAGGGCTACAGTGCTTACACCGCCCAGCCCACTCAAGGATATGCACAGACCACCCAGGCATAT
GGGCAACAAAGCTATGGAACCTATGGACAGCCCACTGATGTCAGCTATACCCAGGCTCAGACCACTGCAACCTAT
GGGCAGACCGCCTATGCAACTTCTTATGGACAGCCTCCCACTGGTTATACTACTCCAACTGCCCCCCAGGCATAC
AGCCAGCCTGTCCAGGGGTATGGCACTGGTGCTTATGATACCACCACTGCTACAGTCACCACCACCCAGGCCTCC
TATGCAGCTCAGTCTGCATATGGCACTCAGCCTGCTTATCCAGCCTATGGGCAGCAGCCAGCAGCCACTGCACCT
ACAAGACCGCAGGATGGAAACAAGCCCACTGAGACTAGTCAACCTCAATCTAGCACAGGGGGTTACAACCAGCCC
AGCCTAGGATATGGACAGAGTAACTACAGTTATCCCCAGGTACCTGGGAGCTACCCCATGCAGCCAGTCACTGCA
CCTCCATCCTACCCTCCTACCAGCTATTCCTCTACACAGCCGACTAGTTATGATCAGAGCAGTTACTCTCAGCAG
AACACCTATGGGCAACCGAGCAGCTATGGACAGCAGAGTAGCTATGGTCAACAAAGCAGCTATGGGCAGCAGCCT
CCCACTAGTTACCCACCCCAAACTGGATCCTACAGCCAAGCTCCAAGTCAATATAGCCAACAGAGCAGCAGCTAC
GGGCAGCAGAGTTCATTCCGACAGGACCACCCCAGTAGCATGGGTGTTTATGGGCAGGAGTCTGGAGGATTTTCC
GGACCAGGAGAGAACCGGAGCATGAGTGGCCCTGATAACCGGGGCAGGGGAAGAGGGGGATTTGATCGTGGAGGC
ATGAGCAGAGGTGGGCGGGGAGGAGGACGCGGTGGAATGGGCAGCGCTGGAGAGCGAGGTGGCTTCAATAAGCCT
GGTGGACCCATGGATGAAGGACCAGATCTTGATCTAGGCCCACCTGTAGATCCAGATGAAGACTCTGACAACAGT
GCAATTTATGTACAAGGATTAAATGACAGTGTGACTCTAGATGATCTGGCAGACTTCTTTAAGCAGTGTGGGGTT
GTTAAGATGAACAAGAGAACTGGGCAACCCATGATCCACATCTACCTGGACAAGGAAACAGGAAAGCCCAAAGGC
GATGCCACAGTGTCCTATGAAGACCCACCTACTGCCAAGGCTGCCGTGGAATGGTTTGATGGGAAAGATTTTCAA
GGGAGCAAACTTAAAGTCTCCCTTGCTCGGAAGAAGCCTCCAATGAACAGTATGCGGGGTGGTCTGCCACCCCGT
GAGGGCAGAGGCATGCCACCACCACTCCGTGGAGGTCCAGGAGGCCCAGGAGGTCCTGGGGGACCCATGGGTCGC
ATGGGAGGCCGTGGAGGAGATAGAGGAGGCTTCCCTCCAAGAGGACCCCGGGGTTCCCGAGGGAACCCCTCTGGA
GGAGGAAACGTCCAGCACCGAGCTGGAGACTGGCAGTGTCCCAATCCGGGTTGTGGAAACCAGAACTTCGCCTGG
AGAACAGAGTGCAACCAGTGTAAGGCCCCAAAGCCTGAAGGCTTCCTCCCGCCACCCTTTCCGCCCCCGGGTGGT
GATCGTGGCAGAGGTGGCCCTGGTGGCATGCGGGGAGGAAGAGGTGGCCTCATGGATCGTGGTGGTCCCGGTGGA
ATGTTCAGAGGTGGCCGTGGTGGAGACAGAGGTGGCTTCCGTGGTGGCCGGGGCATGGACCGAGGTGGCTTTGGT
GGAGGAAGACGAGGTGGCCCTGGGGGGCCCCCTGGACCTTTGATGGAACAGGCGATCGCACAAAAGGTGGCTGAA
CTGAAAAATAGAGTGGCCGTGAAGCTGAACCGGAACGAGCAGCTGAAGAACAAGGTGGAAGAGCTGAAGAACAGA
AACGCCTACCTGAAGAATGAGCTGGCCACCCTGGAAAACGAGGTGGCCAGACTGGAAAACGACGTGGCCGAGGGA
GCACCAGGAAGTGCTGGTTCTGCTGCTGGTAGTGGAGAGCAGAAGCTGATCTCAGAGGAGGACCTGCTAGCCACC
ATGGACGCACAAACACGACGACGTGAGCGTCGCGCTGAGAAACAAGCTCAATGGAAAGCTGCAAACCCACCGCTC
GACGGAGCCGGAGCTGGCGCTGGAGCTGGAGCCGGAGCTGGCGGTCTAGCCACCATGGACGCACAAACACGACGA
CGTGAGCGTCGCGCTGAGAAACAAGCTCAATGGAAAGCTGCAAACCCACCGCTCGACGGAGCCGGAGCTGGCGCT
GGAGCTGGAGCCGGAGCTGGCGGTCTAGCCACCATGGACGCACAAACACGACGACGTGAGCGTCGCGCTGAGAAA
CAAGCTCAATGGAAAGCTGCAAACCCACCGCTCGACGGAGCCGGAGCTGGCGCTGGAGCTGGAGCCGGAGCTGGC
GGTCTAGCCACCATGGACGCACAAACACGACGACGTGAGCGTCGCGCTGAGAAACAAGCTCAATGGAAAGCTGCA
AACCCACCGCTCGAGTCTAGAGGGCCCGTTTCTGGGCTAAGCGGTGCTCCGGGGTCAGCCGGAAGTGCAGCAGGA
TCAGGTCAAAAGGTGGCTGAACTGAAAAATAGAGTGGCCGTGAAGCTGAACCGGAACGAGCAGCTGAAGAACAAG
GTGGAAGAGCTGAAGAACAGAAACGCCTACCTGAAGAATGAGCTGGCCACCCTGGAAAACGAGGTGGCCAGACTG
GAAAACGACGTGGCCGAGGGCAAGCCTATTCCCAACCCCCTGCTGGGCCTGGATAGCACCGGAGCACCAGGAAGT

GCTGGTTCTGCTGCTGGTAGTGGAGCGTGCCCGGTGCCGCTGCAGCTGCCGCCGCTGGAACGCCTGACCCTGGAT
GACAAAAAACCGCTGAATACCCTGATCTCTGCTACTGGTCTGTGGATGAGTCGTACCGGAACCATTCATAAAATC
AAACACCACGAGGTTAGCCGTTCGAAAATCTATATTGAGATGGCGTGTGGCGATCATCTGGTTGTGAACAATAGC
CGCTCTTCTCGTACAGCACGTGCACTGCGTCACCACAAATATCGTAAAACCTGTAAACGTTGCCGTGTGTCCGAT
GAGGATCTGAACAAATTCCTGACAAAAGCCAATGAGGACCAAACAAGCGTGAAAGTGAAAGTCGTTAGCGCTCCT
ACCCGTACTAAAAAAGCAATGCCGAAATCCGTTGCTCGTGCCCCTAAACCACTGGAAAACACTGAAGCAGCACAG
GCACAGCCGTCTGGAAGCAAATTCTCTCCGGCCATTCCTGTTTCTACCCAGGAGTCCGTTTCTGTTCCAGCAAGT
GTGAGCACCAGCATTAGCAGTATTAGCACCGGTGCCACCGCTAGCGCCCTGGTTAAAGGCAATACCAATCCGATT
ACAAGCATGTCTGCCCCGGTTCAAGCATCAGCTCCAGCACTGACAAAATCCCAAACCGATCGTCTGGAGGTTCTG
CTGAATCCGAAAGACGAAATCAGCCTGAATTCCGGCAAACCGTTTCGTGAACTGGAGAGCGAACTGCTGTCACGT
CGTAAAAAAGACCTGCAACAAATCTATGCCGAAGAACGTGAGAACTATCTGGGGAAACTGGAACGTGAAATCACC
CGCTTTTTCGTGGATCGTGGCTTTCTGGAGATCAAATCCCCGATTCTGATTCCTCTGGAGTATATCGAGCGTATG
GGCATCGACAATGATACCGAACTGAGCAAACAAATTTTCCGTGTGGATAAAAACTTCTGTCTGCGCCCTATGCTA
GCACCAAATCTGGCTAACTATCTGCGCAAACTGGACCGTGCCCTGCCTGATCCTATCAAAATCTTCGAGATCGGC
CCGTGTTATCGTAAAGAGTCCGACGGTAAAGAACATCTGGAGGAGTTTACCATGCTGAACTTTTGCCAAATGGGT
TCAGGTTGTACTCGTGAGAACCTGGAAAGCATCATCACCGATTTTCTGAACCACCTGGGCATTGACTTCAAAATT
GTGGGCGACAGCTGTATGGTGTTTGGCGACACCCTGGATGTCATGCACGGCGACCTGGAACTGTCTAGTGCCGTT
GTGGGCCCAATCCCGCTGGATCGTGAGTGGGGTATCGACAAACCTTGGATCGGTGCGGGTTTTGGTCTGGAGCGT
CTGCTGAAAGTAAAACACGACTTCAAGAACATCAAACGTGCTGCACGTTCCGAGTCCTATTACAATGGTATTTCT
ACTAACCTGTAA (SEQ ID NO: 633)

Protein:
MGCVCSSNPEGTELMASTDYSTYSQAAAQQGYSAYTAQPTQGYAQTTQAYGQQSYGTYGQPTDVSYTQAQTTATY
GQTAYATSYGQPPTGYTTPTAPQAYSQPVQGYGTGAYDTTTATVTTTQASYAAQSAYGTQPAYPAYGQQPAATAP
TRPQDGNKPTETSQPQSSTGGYNQPSLGYGQSNYSYPQVPGSYPMQPVTAPPSYPPTSYSSTQPTSYDQSSYSQQ
NTYGQPSSYGQQSSYGQQSSYGQQPPTSYPPQTGSYSQAPSQYSQQSSSYGQQSSFRQDHPSSMGVYGQESGGFS
GPGENRSMSGPDNRGRGRGGFDRGGMSRGGRGGGRGGMGSAGERGGFNKPGGPMDEGPDLDLGPPVDPDEDSDNS
AIYVQGLNDSVTLDDLADFFKQCGVVKMNKRTGQPMIHIYLDKETGKPKGDATVSYEDPPTAKAAVEWFDGKDFQ
GSKLKVSLARKKPPMNSMRGGLPPREGRGMPPPLRGGPGGPGGPGGPMGRMGGRGGDRGGFPPRGPRGSRGNPSG
GGNVQHRAGDWQCPNPGCGNQNFAWRTECNQCKAPKPEGFLPPPFPPPGGDRGRGGPGGMRGGRGGLMDRGGPGG
MFRGGRGGDRGGFRGGRGMDRGGFGGGRRGGPGGPPGPLMEQAIAQKVAELKNRVAVKLNRNEQLKNKVEELKNR
NAYLKNELATLENEVARLENDVAEGAPGSAGSAAGSGEQKLISEEDLLATMDAQTRRRERRAEKQAQWKAANPPL
DGAGAGAGAGAGAGGLATMDAQTRRRERRAEKQAQWKAANPPLDGAGAGAGAGAGAGGLATMDAQTRRRERRAEK
QAQWKAANPPLDGAGAGAGAGAGAGGLATMDAQTRRRERRAEKQAQWKAANPPLESRGPVSGLSGAPGSAGSAAG
SGQKVAELKNRVAVKLNRNEQLKNKVEELKNRNAYLKNELATLENEVARLENDVAEGKPIPNPLLGLDSTGAPGS
AGSAAGSGACPVPLQLPPLERLTLDDKKPLNTLISATGLWMSRTGTIHKIKHHEVSRSKIYIEMACGDHLVVNNS
RSSRTARALRHHKYRKTCKRCRVSDEDLNKFLTKANEDQTSVKVKVVSAPTRTKKAMPKSVARAPKPLENTEAAQ
AQPSGSKFSPAIPVSTQESVSVPASVSTSISSISTGATASALVKGNTNPITSMSAPVQASAPALTKSQTDRLEVL
LNPKDEISLNSGKPFRELESELLSRRKKDLQQIYAEERENYLGKLEREITRFFVDRGFLEIKSPILIPLEYIERM
GIDNDTELSKQIFRVDKNFCLRPMLAPNLANYLRKLDRALPDPIKIFEIGPCYRKESDGKEHLEEFTMLNFCQMG
SGCTRENLESIITDFLNHLGIDFKIVGDSCMVFGDTLDVMHGDLELSSAVVGPIPLDREWGIDKPWIGAGFGLER
LLKVKHDFKNIKRAARSESYYNGISTNL* (SEQ ID NO: 634)

LCK::FUS::SYNZIP3::4xλN22::SYNZIP3::PylRS(AF)
DNA:
ATGGGCTGCGTGTGCAGCAGCAACCCCGAGGGTACCGAGCTCGCCTCAAACGATTATACCCAACAAGCAACCCAA
AGCTATGGGGCCTACCCCACCCAGCCCGGGCAGGGCTATTCCCAGCAGAGCAGTCAGCCCTACGGACAGCAGAGT
TACAGTGGTTATAGCCAGTCCACGGACACTTCAGGATATGGCCAGAGCAGCTATTCTTCTTATGGCCAGAGCCAG
AACACAGGCTATGGAACTCAGTCAACTCCCCAGGGATATGGCTCGACTGGCGGCTATGGCAGTAGCCAGAGCTCC
CAATCGTCTTACGGGCAGCAGTCCTCCTACCCTGGCTATGGCCAGCAGCCAGCTCCCAGCAGCACCTCGGGAAGT
TACGGTAGCAGTTCTCAGAGCAGCAGCTATGGGCAGCCCCAGAGTGGGAGCTACAGCCAGCAGCCTAGCTATGGT
GGACAGCAGCAAAGCTATGGACAGCAGCAAAGCTATAATCCCCCTCAGGGCTATGGACAGCAGAACCAGTACAAC
AGCAGCAGTGGTGGTGGAGGTGGAGGTGGAGGTGGAGGTAACTATGGCCAAGATCAATCCTCCATGAGTAGTGGT
GGTGGCAGTGGTGGCGGTTATGGCAATCAAGACCAGAGTGGTGGAGGTGGCAGCGGTGGCTATGGACAGCAGGAC
CGTGGAGGCCGCGGCAGGGGTGGCAGTGGTGGCGGCGGCGGCGGCGGTGGTGGTTACAACCGCAGCAGTGGT
GGCTATGAACCCAGAGGTCGTGGAGGTGGCCGTGGAGGCAGAGGTGGCATGGGCGGAAGTGACCGTGGTGGCTTC
AATAAATTTGGTGGCCCTCGGGACCAAGGATCACGTCATGACTCCGAACAGGATAATTCAGACAACAACACCATC

TTTGTGCAAGGCCTGGGTGAGAATGTTACAATTGAGTCTGTGGCTGATTACTTCAAGCAGATTGGTATTATTAAG
ACAAACAAGAAAACGGGACAGCCCATGATTAATTTGTACACAGACAGGGAAACTGGCAAGCTGAAGGGAGAGGCA
ACGGTCTCTTTTGATGACCCACCTTCAGCTAAAGCAGCTATTGACTGGTTTGATGGTAAAGAATTCTCCGGAAAT
CCTATCAAGGTCTCATTTGCTACTCGCCGGGCAGACTTTAATCGGGGTGGTGGCAATGGTCGTGGAGGCCGAGGG
CGAGGAGGACCCATGGGCCGTGGAGGCTATGGAGGTGGTGGCAGTGGTGGTGGTGGCCGAGGAGGATTTCCCAGT
GGAGGTGGTGGCGGTGGAGGACAGCAGCGAGCTGGTGACTGGAAGTGTCCTAATCCCACCTGTGAGAATATGAAC
TTCTCTTGGAGGAATGAATGCAACCAGTGTAAGGCCCCTAAACCAGATGGCCCAGGAGGGGGACCAGGTGGCTCT
CACATGGGGGGTAACTACGGGGATGATCGTCGTGGTGGCAGAGGAGGCGCGATCGCAAATGAGGTGACCACCCTG
GAAAACGACGCCGCCTTCATCGAGAACGAGAACGCCTACCTGGAAAAAGAGATCGCCAGACTGAGAAAGGAAAAG
GCCGCTCTGCGGAACAGACTGGCCCACAAGAAGGGAGCACCAGGAAGTGCTGGTTCTGCTGCTGGTAGTGGAGCA
TCGATAGAGCAGAAGCTGATCTCAGAGGAGGACCTGCTAGCCACCATGGACGCACAAACACGACGACGTGAGCGT
CGCGCTGAGAAACAAGCTCAATGGAAAGCTGCAAACCCACCGCTCGACGGAGCCGGAGCTGGCGCTGGAGCTGGA
GCCGGAGCTGGCGGTCTAGCCACCATGGACGCACAAACACGACGACGTGAGCGTCGCGCTGAGAAACAAGCTCAA
TGGAAAGCTGCAAACCCACCGCTCGACGGAGCCGGAGCTGGCGCTGGAGCTGGAGCCGGAGCTGGCGGTCTAGCC
ACCATGGACGCACAAACACGACGACGTGAGCGTCGCGCTGAGAAACAAGCTCAATGGAAAGCTGCAAACCCACCG
CTCGACGGAGCCGGAGCTGGCGCTGGAGCTGGAGCCGGAGCTGGCGGTCTAGCCACCATGGACGCACAAACACGA
CGACGTGAGCGTCGCGCTGAGAAACAAGCTCAATGGAAAGCTGCAAACCCACCGCTCGAGTCTAGAGGGCTAAGC
GGTGCTCCGGGGTCAGCCGGAAGTGCAGCAGGATCAGGTAATGAGGTGACCACCCTGGAAAACGACGCCGCCTTC
ATCGAGAACGAGAACGCCTACCTGGAAAAAGAGATCGCCAGACTGAGAAAGGAAAAGGCCGCTCTGCGGAACAGA
CTGGCCCACAAGAAGGGCAAGCCTATTCCCAACCCCCTGCTGGGCCTGGATAGCACCGGAGCACCAGGAAGTGCT
GGTTCTGCTGCTGGTAGTGGAGCGTGCCCGGTGCCGCTGCAGCTGCCGCCGCTGGAACGCCTGACCCTGGATGAC
AAAAAACCGCTGAATACCCTGATCTCTGCTACTGGTCTGTGGATGAGTCGTACCGGAACCATTCATAAAATCAAA
CACCACGAGGTTAGCCGTTCGAAAATCTATATTGAGATGGCGTGTGGCGATCATCTGGTTGTGAACAATAGCCGC
TCTTCTCGTACAGCACGTGCACTGCGTCACCACAAATATCGTAAAACCTGTAAACGTTGCCGTGTGTCCGATGAG
GATCTGAACAAATTCCTGACAAAAGCCAATGAGGACCAAACAAGCGTGAAAGTGAAAGTCGTTAGCGCTCCTACC
CGTACTAAAAAAGCAATGCCGAAATCCGTTGCTCGTGCCCCTAAACCACTGGAAAACACTGAAGCAGCACAGGCA
CAGCCGTCTGGAAGCAAATTCTCTCCGGCCATTCCTGTTTCTACCCAGGAGTCCGTTTCTGTTCCAGCAAGTGTG
AGCACCAGCATTAGCAGTATTAGCACCGGTGCCACCGCTAGCGCCCTGGTTAAAGGCAATACCAATCCGATTACA
AGCATGTCTGCCCCGGTTCAAGCATCAGCTCCAGCACTGACAAAATCCCAAACCGATCGTCTGGAGGTTCTGCTG
AATCCGAAAGACGAAATCAGCCTGAATTCCGGCAAACCGTTTCGTGAACTGGAGAGCGAACTGCTGTCACGTCGT
AAAAAAGACCTGCAACAAATCTATGCCGAAGAACGTGAGAACTATCTGGGGAAACTGGAACGTGAAATCACCCGC
TTTTTCGTGGATCGTGGCTTTCTGGAGATCAAATCCCCGATTCTGATTCCTCTGGAGTATATCGAGCGTATGGGC
ATCGACAATGATACCGAACTGAGCAAACAAATTTTCCGTGTGGATAAAAACTTCTGTCTGCGCCCTATGCTAGCA
CCAAATCTGGCTAACTATCTGCGCAAACTGGACCGTGCCCTGCCTGATCCTATCAAAATCTTCGAGATCGGCCCG
TGTTATCGTAAAGAGTCCGACGGTAAAGAACATCTGGAGGAGTTTACCATGCTGAACTTTTGCCAAATGGGTTCA
GGTTGTACTCGTGAGAACCTGGAAAGCATCATCACCGATTTTCTGAACCACCTGGGCATTGACTTCAAAATTGTG
GGCGACAGCTGTATGGTGTTTGGCGACACCCTGGATGTCATGCACGGCGACCTGGAACTGTCTAGTGCCGTTGTG
GGCCCAATCCCGCTGGATCGTGAGTGGGGTATCGACAAACCTTGGATCGGTGCGGGTTTTGGTCTGGAGCGTCTG
CTGAAAGTAAAACACGACTTCAAGAACATCAAACGTGCTGCACGTTCCGAGTCCTATTACAATGGTATTTCTACT
AACCTGTAA (SEQ ID NO: 635)

Protein:
MGCVCSSNPEGTELASNDYTQQATQSYGAYPTQPGQGYSQQSSQPYGQQSYSGYSQSTDTSGYGQSSYSSYGQSQ
NTGYGTQSTPQGYGSTGGYGSSQSSQSSYGQQSSYPGYGQQPAPSSTSGSYGSSSQSSSYGQPQSGSYSQQPSYG
GQQQSYGQQQSYNPPQGYGQQNQYNSSSGGGGGGGGGGNYGQDQSSMSSGGGSGGGYGNQDQSGGGGSGGYGQQD
RGGRGRGGSGGGGGGGGGYNRSSGGYEPRGRGGGRGGRGGMGGSDRGGFNKFGGPRDQGSRHDSEQDNSDNNTI
FVQGLGENVTIESVADYFKQIGIIKTNKKTGQPMINLYTDRETGKLKGEATVSFDDPPSAKAAIDWFDGKEFSGN
PIKVSFATRRADFNRGGGNGRGGRGRGGPMGRGGYGGGGSGGGGRGGFPSGGGGGGGQQRAGDWKCPNPTCENMN
FSWRNECNQCKAPKPDGPGGGPGGSHMGGNYGDDRRGGRGGAIANEVTTLENDAAFIENENAYLEKEIARLRKEK
AALRNRLAHKKGAPGSAGSAAGSGASIEQKLISEEDLLATMDAQTRRRERRAEKQAQWKAANPPLDGAGAGAGAG
AGAGGLATMDAQTRRRERRAEKQAQWKAANPPLDGAGAGAGAGAGAGGLATMDAQTRRRERRAEKQAQWKAANPP
LDGAGAGAGAGAGAGGLATMDAQTRRRERRAEKQAQWKAANPPLESRGLSGAPGSAGSAAGSGNEVTTLENDAAF
IENENAYLEKEIARLRKEKAALRNRLAHKKGKPIPNPLLGLDSTGAPGSAGSAAGSGACPVPLQLPPLERLTLDD
KKPLNTLISATGLWMSRTGTIHKIKHHEVSRSKIYIEMACGDHLVVNNSRSSRTARALRHHKYRKTCKRCRVSDE
DLNKFLTKANEDQTSVKVKVVSAPTRTKKAMPKSVARAPKPLENTEAAQAQPSGSKFSPAIPVSTQESVSVPASV
STSISSISTGATASALVKGNTNPITSMSAPVQASAPALTKSQTDRLEVLLNPKDEISLNSGKPFRELESELLSRR
KKDLQQIYAEERENYLGKLEREITRFFVDRGFLEIKSPILIPLEYIERMGIDNDTELSKQIFRVDKNFCLRPMLA
PNLANYLRKLDRALPDPIKIFEIGPCYRKESDGKEHLEEFTMLNFCQMGSGCTRENLESIITDFLNHLGIDFKIV

GDSCMVFGDTLDVMHGDLELSSAVVGPIPLDREWGIDKPWIGAGFGLERLLKVKHDFKNIKRAARSESYYNGIST
NL* (SEQ ID NO: 636)

TOM20::FUS::SYNZIP3::4xλN22::SYNZIP3::PylRS(AF)
DNA:
ATGGTGGGTCGGAACAGCGCCATCGCCGCCGGTGTATGCGGGGCCCTTTTCATTGGGTACTGCATCTACTTCGAC
CGCAAAAGACGAAGTGACCCCAACTTCAAGAACAGGCTTCGAGAACGAAGAAAGAAACAGAAGCTTGCCAAGGAG
AGAGCTGGGCTTTCCAAGTTACCTGACCTTAAAGATGCTGAAGCTGTTCAGAAATTCTTCATGGCCTCAAACGAT
TATACCCAACAAGCAACCCAAAGCTATGGGGCCTACCCCACCCAGCCCGGGCAGGGCTATTCCCAGCAGAGCAGT
CAGCCCTACGGACAGCAGAGTTACAGTGGTTATAGCCAGTCCACGGACACTTCAGGATATGGCCAGAGCAGCTAT
TCTTCTTATGGCCAGAGCCAGAACACAGGCTATGGAACTCAGTCAACTCCCCAGGGATATGGCTCGACTGGCGGC
TATGGCAGTAGCCAGAGCTCCCAATCGTCTTACGGGCAGCAGTCCTCCTACCCTGGCTATGGCCAGCAGCCAGCT
CCCAGCAGCACCTCGGGAAGTTACGGTAGCAGTTCTCAGAGCAGCAGCTATGGGCAGCCCCAGAGTGGGAGCTAC
AGCCAGCAGCCTAGCTATGGTGGACAGCAGCAAAGCTATGGACAGCAGCAAAGCTATAATCCCCCTCAGGGCTAT
GGACAGCAGAACCAGTACAACAGCAGCAGTGGTGGTGGAGGTGGAGGTGGAGGTGGAGGTAACTATGGCCAAGAT
CAATCCTCCATGAGTAGTGGTGGTGGCAGTGGTGGCGGTTATGGCAATCAAGACCAGAGTGGTGGAGGTGGCAGC
GGTGGCTATGGACAGCAGGACCGTGGAGGCCGCGGCAGGGGTGGCAGTGGTGGCGGCGGCGGCGGCGGCGGTGGT
GGTTACAACCGCAGCAGTGGTGGCTATGAACCCAGAGGTCGTGGAGGTGGCCGTGGAGGCAGAGGTGGCATGGGC
GGAAGTGACCGTGGTGGCTTCAATAAATTTGGTGGCCCTCGGGACCAAGGATCACGTCATGACTCCGAACAGGAT
AATTCAGACAACAACACCATCTTTGTGCAAGGCCTGGGTGAGAATGTTACAATTGAGTCTGTGGCTGATTACTTC
AAGCAGATTGGTATTATTAAGACAAACAAGAAAACGGGACAGCCCATGATTAATTTGTACACAGACAGGGAAACT
GGCAAGCTGAAGGGAGAGGCAACGGTCTCTTTTGATGACCCACCTTCAGCTAAAGCAGCTATTGACTGGTTTGAT
GGTAAAGAATTCTCCGGAAATCCTATCAAGGTCTCATTTGCTACTCGCCGGGCAGACTTTAATCGGGGTGGTGGC
AATGGTCGTGGAGGCCGAGGGCGAGGAGGACCCATGGGCCGTGGAGGCTATGGAGGTGGTGGCAGTGGTGGTGGT
GGCCGAGGAGGATTTCCCAGTGGAGGTGGTGGCGGTGGAGGACAGCAGCGAGCTGGTGACTGGAAGTGTCCTAAT
CCCACCTGTGAGAATATGAACTTCTCTTGGAGGAATGAATGCAACCAGTGTAAGGCCCCTAAACCAGATGGCCCA
GGAGGGGGACCAGGTGGCTCTCACATGGGGGGTAACTACGGGGATGATCGTCGTGGTGGCAGAGGAGGCGCGATC
GCAAATGAGGTGACCACCCTGGAAAACGACGCCGCCTTCATCGAGAACGAGAACGCCTACCTGGAAAAAGAGATC
GCCAGACTGAGAAAGGAAAAGGCCGCTCTGCGGAACAGACTGGCCCACAAGAAGGGAGCACCAGGAAGTGCTGGT
TCTGCTGCTGGTAGTGGAGCATCGATAGAGCAGAAGCTGATCTCAGAGGAGGACCTGCTAGCCACCATGGACGCA
CAAACACGACGACGTGAGCGTCGCGCTGAGAAACAAGCTCAATGGAAAGCTGCAAACCCACCGCTCGACGGAGCC
GGAGCTGGCGCTGGAGCTGGAGCCGGAGCTGGCGGTCTAGCCACCATGGACGCACAAACACGACGACGTGAGCGT
CGCGCTGAGAAACAAGCTCAATGGAAAGCTGCAAACCCACCGCTCGACGGAGCCGGAGCTGGCGCTGGAGCTGGA
GCCGGAGCTGGCGGTCTAGCCACCATGGACGCACAAACACGACGACGTGAGCGTCGCGCTGAGAAACAAGCTCAA
TGGAAAGCTGCAAACCCACCGCTCGACGGAGCCGGAGCTGGCGCTGGAGCTGGAGCCGGAGCTGGCGGTCTAGCC
ACCATGGACGCACAAACACGACGACGTGAGCGTCGCGCTGAGAAACAAGCTCAATGGAAAGCTGCAAACCCACCG
CTCGAGTCTAGAGGGCTAAGCGGTGCTCCGGGGTCAGCCGGAAGTGCAGCAGGATCAGGTAATGAGGTGACCACC
CTGGAAAACGACGCCGCCTTCATCGAGAACGAGAACGCCTACCTGGAAAAAGAGATCGCCAGACTGAGAAAGGAA
AAGGCCGCTCTGCGGAACAGACTGGCCCACAAGAAGGGCAAGCCTATTCCCAACCCCCTGCTGGGCCTGGATAGC
ACCGGAGCACCAGGAAGTGCTGGTTCTGCTGCTGGTAGTGGAGCGTGCCCGGTGCCGCTGCAGCTGCCGCCGCTG
GAACGCCTGACCCTGGATGACAAAAAACCGCTGAATACCCTGATCTCTGCTACTGGTCTGTGGATGAGTCGTACC
GGAACCATTCATAAAATCAAACACCACGAGGTTAGCCGTTCGAAAATCTATATTGAGATGGCGTGTGGCGATCAT
CTGGTTGTGAACAATAGCCGCTCTTCTCGTACAGCACGTGCACTGCGTCACCACAAATATCGTAAAACCTGTAAA
CGTTGCCGTGTGTCCGATGAGGATCTGAACAAATTCCTGACAAAAGCCAATGAGGACCAAACAAGCGTGAAAGTG
AAAGTCGTTAGCGCTCCTACCCGTACTAAAAAAGCAATGCCGAAATCCGTTGCTCGTGCCCCTAAACCACTGGAA
AACACTGAAGCAGCACAGGCACAGCCGTCTGGAAGCAAATTCTCTCCGGCCATTCCTGTTTCTACCCAGGAGTCC
GTTTCTGTTCCAGCAAGTGTGAGCACCAGCATTAGCAGTATTAGCACCGGTGCCACCGCTAGCGCCCTGGTTAAA
GGCAATACCAATCCGATTACAAGCATGTCTGCCCCGGTTCAAGCATCAGCTCCAGCACTGACAAAATCCCAAACC
GATCGTCTGGAGGTTCTGCTGAATCCGAAAGACGAAATCAGCCTGAATTCCGGCAAACCGTTTCGTGAACTGGAG
AGCGAACTGCTGTCACGTCGTAAAAAAGACCTGCAACAAATCTATGCCGAAGAACGTGAGAACTATCTGGGGAAA
CTGGAACGTGAAATCACCCGCTTTTTCGTGGATCGTGGCTTTCTGGAGATCAAATCCCCGATTCTGATTCCTCTG
GAGTATATCGAGCGTATGGGCATCGACAATGATACCGAACTGAGCAAACAAATTTTCCGTGTGGATAAAAACTTC
TGTCTGCGCCCTATGCTAGCACCAAATCTGGCTAACTATCTGCGCAAACTGGACCGTGCCCTGCCTGATCCTATC
AAAATCTTCGAGATCGGCCCGTGTTATCGTAAAGAGTCCGACGGTAAAGAACATCTGGAGGAGTTTACCATGCTG
AACTTTTGCCAAATGGGTTCAGGTTGTACTCGTGAGAACCTGGAAAGCATCATCACCGATTTTCTGAACCACCTG
GGCATTGACTTCAAAATTGTGGGCGACAGCTGTATGGTGTTTGGCGACACCCTGGATGTCATGCACGGCGACCTG
GAACTGTCTAGTGCCGTTGTGGGCCCAATCCCGCTGGATCGTGAGTGGGGTATCGACAAACCTTGGATCGGTGCG

GGTTTTGGTCTGGAGCGTCTGCTGAAAGTAAAACACGACTTCAAGAACATCAAACGTGCTGCACGTTCCGAGTCC
TATTACAATGGTATTTCTACTAACCTGTAA (SEQ ID NO: 637)

Protein:
MVGRNSAIAAGVCGALFIGYCIYFDRKRRSDPNFKNRLRERRKKQKLAKERAGLSKLPDLKDAEAVQKFFMASND
YTQQATQSYGAYPTQPGQGYSQQSSQPYGQQSYSGYSQSTDTSGYGQSSYSSYGQSQNTGYGTQSTPQGYGSTGG
YGSSQSSQSSYGQQSSYPGYGQQPAPSSTSGSYGSSSQSSSYGQPQSGSYSQQPSYGGQQQSYGQQQSYNPPQGY
GQQNQYNSSSGGGGGGGGGGNYGQDQSSMSSGGGSGGGYGNQDQSGGGGSGGYGQQDRGGRGRGGSGGGGGGGG
GYNRSSGGYEPRGRGGGRGGRGGMGGSDRGGFNKFGGPRDQGSRHDSEQDNSDNNTIFVQGLGENVTIESVADYF
KQIGIIKTNKKTGQPMINLYTDRETGKLKGEATVSFDDPPSAKAAIDWFDGKEFSGNPIKVSFATRRADFNRGGG
NGRGGRGRGGPMGRGGYGGGGSGGGGRGGFPSGGGGGGGQQRAGDWKCPNPTCENMNFSWRNECNQCKAPKPDGP
GGGPGGSHMGGNYGDDRRGGRGGAIANEVTTLENDAAFIENENAYLEKEIARLRKEKAALRNRLAHKKGAPGSAG
SAAGSGASIEQKLISEEDLLATMDAQTRRRERRAEKQAQWKAANPPLDGAGAGAGAGAGAGGLATMDAQTRRRER
RAEKQAQWKAANPPLDGAGAGAGAGAGAGGLATMDAQTRRRERRAEKQAQWKAANPPLDGAGAGAGAGAGAGGLA
TMDAQTRRRERRAEKQAQWKAANPPLESRGLSGAPGSAGSAAGSGNEVTTLENDAAFIENENAYLEKEIARLRKE
KAALRNRLAHKKGKPIPNPLLGLDSTGAPGSAGSAAGSGACPVPLQLPPLERLTLDDKKPLNTLISATGLWMSRT
GTIHKIKHHEVSRSKIYIEMACGDHLVVNNSRSSRTARALRHHKYRKTCKRCRVSDEDLNKFLTKANEDQTSVKV
KVVSAPTRTKKAMPKSVARAPKPLENTEAAQAQPSGSKFSPAIPVSTQESVSVPASVSTSISSISTGATASALVK
GNTNPITSMSAPVQASAPALTKSQTDRLEVLLNPKDEISLNSGKPFRELESELLSRRKKDLQQIYAEERENYLGK
LEREITRFFVDRGFLEIKSPILIPLEYIERMGIDNDTELSKQIFRVDKNFCLRPMLAPNLANYLRKLDRALPDPI
KIFEIGPCYRKESDGKEHLEEFTMLNFCQMGSGCTRENLESIITDFLNHLGIDFKIVGDSCMVFGDTLDVMHGDL
ELSSAVVGPIPLDREWGIDKPWIGAGFGLERLLKVKHDFKNIKRAARSESYYNGISTNL* (SEQ ID NO:
638)

TOM20::EWSR1::SYNZIP2::MCP::SYNZIP2::PylRS(AF)
DNA:
ATGGTGGGTCGGAACAGCGCCATCGCCGCCGGTGTATGCGGGGCCCTTTTCATTGGGTACTGCATCTACTTCGAC
CGCAAAAGACGAAGTGACCCCAACTTCAAGAACAGGCTTCGAGAACGAAGAAAGAAACAGAAGCTTGCCAAGGAG
AGAGCTGGGCTTTCCAAGTTACCTGACCTTAAAGATGCTGAAGCTGTTCAGAAGTTCTTCATGGCGTCCACGGAT
TACAGTACCTATAGCCAAGCTGCAGCGCAGCAGGGCTACAGTGCTTACACCGCCCAGCCCACTCAAGGATATGCA
CAGACCACCCAGGCATATGGGCAACAAAGCTATGGAACCTATGGACAGCCCACTGATGTCAGCTATACCCAGGCT
CAGACCACTGCAACCTATGGGCAGACCGCCTATGCAACTTCTTATGGACAGCCTCCCACTGGTTATACTACTCCA
ACTGCCCCCCAGGCATACAGCCAGCCTGTCCAGGGGTATGGCACTGGTGCTTATGATACCACCACTGCTACAGTC
ACCACCACCCAGGCCTCCTATGCAGCTCAGTCTGCATATGGCACTCAGCCTGCTTATCCAGCCTATGGGCAGCAG
CCAGCAGCCACTGCACCTACAAGACCGCAGGATGGAAACAAGCCCACTGAGACTAGTCAACCTCAATCTAGCACA
GGGGGTTACAACCAGCCCAGCCTAGGATATGGACAGAGTAACTACAGTTATCCCCAGGTACCTGGGAGCTACCCC
ATGCAGCCAGTCACTGCACCTCCATCCTACCCTCCTACCAGCTATTCCTCTACACAGCCGACTAGTTATGATCAG
AGCAGTTACTCTCAGCAGAACACCTATGGGCAACCGAGCAGCTATGGACAGCAGAGTAGCTATGGTCAACAAAGC
AGCTATGGGCAGCAGCCTCCCACTAGTTACCCACCCCAAACTGGATCCTACAGCCAAGCTCCAAGTCAATATAGC
CAACAGAGCAGCAGCTACGGGCAGCAGAGTTCATTCCGACAGGACCACCCCAGTAGCATGGGTGTTTATGGGCAG
GAGTCTGGAGGATTTTCCGGACCAGGAGAGAACCGGAGCATGAGTGGCCCTGATAACCGGGGCAGGGGAAGAGGG
GGATTTGATCGTGGAGGCATGAGCAGAGGTGGGCGGGGAGGAGGACGCGGTGGAATGGGCAGCGCTGGAGAGCGA
GGTGGCTTCAATAAGCCTGGTGGACCCATGGATGAAGGACCAGATCTTGATCTAGGCCCACCTGTAGATCCAGAT
GAAGACTCTGACAACAGTGCAATTTATGTACAAGGATTAAATGACAGTGTGACTCTAGATGATCTGGCAGACTTC
TTTAAGCAGTGTGGGGTTGTTAAGATGAACAAGAGAACTGGGCAACCCATGATCCACATCTACCTGGACAAGGAA
ACAGGAAAGCCCAAAGGCGATGCCACAGTGTCCTATGAAGACCCACCTACTGCCAAGGCTGCCGTGGAATGGTTT
GATGGGAAAGATTTTCAAGGGAGCAAACTTAAAGTCTCCCTTGCTCGGAAGAAGCCTCCAATGAACAGTATGCGG
GGTGGTCTGCCACCCCGTGAGGGCAGAGGCATGCCACCACCACTCCGTGGAGGTCCAGGAGGCCCAGGAGGTCCT
GGGGGACCCATGGGTCGCATGGGAGGCCGTGGAGGAGATAGAGGAGGCTTCCCTCCAAGAGGACCCCGGGGTTCC
CGAGGGAACCCCTCTGGAGGAGGAAACGTCCAGCACCGAGCTGGAGACTGGCAGTGTCCCAATCCGGGTTGTGGA
AACCAGAACTTCGCCTGGAGAACAGAGTGCAACCAGTGTAAGGCCCCAAAGCCTGAAGGCTTCCTCCCGCCACCC
TTTCCGCCCCCGGGTGGTGATCGTGGCAGAGGTGGCCCTGGTGGCATGCGGGGAGGAAGAGGTGGCCTCATGGAT
CGTGGTGGTCCCGGTGGAATGTTCAGAGGTGGCCGTGGTGGAGACAGAGGTGGCTTCCGTGGTGGCCGGGGCATG
GACCGAGGTGGCTTTGGTGGAGGAAGACGAGGTGGCCCTGGGGGGCCCCCTGGACCTTTGATGGAACAGGCGATC
GCAGCTAGAAACGCCTACCTGAGAAAGAAAATCGCCAGACTGAAGAAGGACAACCTGCAGCTGGAAAGAGACGAG
CAGAACCTGGAAAAGATCATCGCCAACCTCAGAGATGAGATCGCCAGACTGGAAAACGAGGTGGCCAGCCACGAG
CAGTATCCCTATGATGTGCCGGATTATGCTGGAGCACCAGGAAGTGCTGGTTCTGCTGCTGGTAGTGGAGCTTCT

AACTTTACTCAGTTCGTTCTCGTCGACAATGGCGGAACTGGCGACGTGACTGTCGCCCCAAGCAACTTCGCTAAC
GGGATCGCTGAATGGATCAGCTCTAACTCGCGTTCACAGGCTTACAAAGTAACCTGTAGCGTTCGTCAGAGCTCT
GCGCAGAATCGCAAATACACCATCAAAGTCGAGGTGCCTAAAGGCGCCTGGCGTTCGTACTTAAATATGGAACTA
ACCATTCCAATTTTCGCCACGAATTCCGACTGCGAGCTTATTGTTAAGGCAATGCAAGGTCTCCTAAAAGATGGA
AACCCGATTCCCTCAGCAATCGCAGCAAACTCCGGCATCTACGGGCTAAGCGGTGCTCCGGGGTCAGCCGGAAGT
GCAGCAGGATCAGGTGCTAGAAACGCCTACCTGAGAAAGAAAATCGCCAGACTGAAGAAGGACAACCTGCAGCTG
GAAAGAGACGAGCAGAACCTGGAAAAGATCATCGCCAACCTCAGAGATGAGATCGCCAGACTGGAAAACGAGGTG
GCCAGCCACGAGCAGGGCAAGCCTATTCCCAACCCCCTGCTGGGCCTGGATAGCACCGGAGCACCAGGAAGTGCT
GGTTCTGCTGCTGGTAGTGGAGCGTGCCCGGTGCCGCTGCAGCTGCCGCCGCTGGAACGCCTGACCCTGGATGAC
AAAAAACCGCTGAATACCCTGATCTCTGCTACTGGTCTGTGGATGAGTCGTACCGGAACCATTCATAAAATCAAA
CACCACGAGGTTAGCCGTTCGAAAATCTATATTGAGATGGCGTGTGGCGATCATCTGGTTGTGAACAATAGCCGC
TCTTCTCGTACAGCACGTGCACTGCGTCACCACAAATATCGTAAAACCTGTAAACGTTGCCGTGTGTCCGATGAG
GATCTGAACAAATTCCTGACAAAAGCCAATGAGGACCAAACAAGCGTGAAAGTGAAAGTCGTTAGCGCTCCTACC
CGTACTAAAAAAGCAATGCCGAAATCCGTTGCTCGTGCCCCTAAACCACTGGAAAACACTGAAGCAGCACAGGCA
CAGCCGTCTGGAAGCAAATTCTCTCCGGCCATTCCTGTTTCTACCCAGGAGTCCGTTTCTGTTCCAGCAAGTGTG
AGCACCAGCATTAGCAGTATTAGCACCGGTGCCACCGCTAGCGCCCTGGTTAAAGGCAATACCAATCCGATTACA
AGCATGTCTGCCCCGGTTCAAGCATCAGCTCCAGCACTGACAAAATCCCAAACCGATCGTCTGGAGGTTCTGCTG
AATCCGAAAGACGAAATCAGCCTGAATTCCGGCAAACCGTTTCGTGAACTGGAGAGCGAACTGCTGTCACGTCGT
AAAAAAGACCTGCAACAAATCTATGCCGAAGAACGTGAGAACTATCTGGGGAAACTGGAACGTGAAATCACCCGC
TTTTTCGTGGATCGTGGCTTTCTGGAGATCAAATCCCCGATTCTGATTCCTCTGGAGTATATCGAGCGTATGGGC
ATCGACAATGATACCGAACTGAGCAAACAAATTTTCCGTGTGGATAAAAACTTCTGTCTGCGCCCTATGCTAGCA
CCAAATCTGGCTAACTATCTGCGCAAACTGGACCGTGCCCTGCCTGATCCTATCAAAATCTTCGAGATCGGCCCG
TGTTATCGTAAAGAGTCCGACGGTAAAGAACATCTGGAGGAGTTTACCATGCTGAACTTTTGCCAAATGGGTTCA
GGTTGTACTCGTGAGAACCTGGAAAGCATCATCACCGATTTTCTGAACCACCTGGGCATTGACTTCAAAATTGTG
GGCGACAGCTGTATGGTGTTTGGCGACACCCTGGATGTCATGCACGGCGACCTGGAACTGTCTAGTGCCGTTGTG
GGCCCAATCCCGCTGGATCGTGAGTGGGGTATCGACAAACCTTGGATCGGTGCGGGTTTTGGTCTGGAGCGTCTG
CTGAAAGTAAAACACGACTTCAAGAACATCAAACGTGCTGCACGTTCCGAGTCCTATTACAATGGTATTTCTACT
AACCTGTAA (SEQ ID NO: 639)

Protein:
MVGRNSAIAAGVCGALFIGYCIYFDRKRRSDPNFKNRLRERRKKQKLAKERAGLSKLPDLKDAEAVQKFFMASTD
YSTYSQAAAQQGYSAYTAQPTQGYAQTTQAYGQQSYGTYGQPTDVSYTQAQTTATYGQTAYATSYGQPPTGYTTP
TAPQAYSQPVQGYGTGAYDTTTATVTTTQASYAAQSAYGTQPAYPAYGQQPAATAPTRPQDGNKPTETSQPQSST
GGYNQPSLGYGQSNYSYPQVPGSYPMQPVTAPPSYPPTSYSSTQPTSYDQSSYSQQNTYGQPSSYGQQSSYGQQS
SYGQQPPTSYPPQTGSYSQAPSQYSQQSSSYGQQSSFRQDHPSSMGVYGQESGGFSGPGENRSMSGPDNRGRGRG
GFDRGGMSRGGRGGGRGGMGSAGERGGFNKPGGPMDEGPDLDLGPPVDPDEDSDNSAIYVQGLNDSVTLDDLADF
FKQCGVVKMNKRTGQPMIHIYLDKETGKPKGDATVSYEDPPTAKAAVEWFDGKDFQGSKLKVSLARKKPPMNSMR
GGLPPREGRGMPPPLRGGPGGPGGPGGPMGRMGGRGGDRGGFPPRGPRGSRGNPSGGGNVQHRAGDWQCPNPGCG
NQNFAWRTECNQCKAPKPEGFLPPPFPPPGGDRGRGGPGGMRGGRGGLMDRGGPGGMFRGGRGGDRGGFRGGRGM
DRGGFGGGRRGGPGGPPGPLMEQAIAARNAYLRKKIARLKKDNLQLERDEQNLEKIIANLRDEIARLENEVASHE
QYPYDVPDYAGAPGSAGSAAGSGASNFTQFVLVDNGGTGDVTVAPSNFANGIAEWISSNSRSQAYKVTCSVRQSS
AQNRKYTIKVEVPKGAWRSYLNMELTIPIFATNSDCELIVKAMQGLLKDGNPIPSAIAANSGIYGLSGAPGSAGS
AAGSGARNAYLRKKIARLKKDNLQLERDEQNLEKIIANLRDEIARLENEVASHEQGKPIPNPLLGLDSTGAPGSA
GSAAGSGACPVPLQLPPLERLTLDDKKPLNTLISATGLWMSRTGTIHKIKHHEVSRSKIYIEMACGDHLVVNNSR
SSRTARALRHHKYRKTCKRCRVSDEDLNKFLTKANEDQTSVKVKVVSAPTRTKKAMPKSVARAPKPLENTEAAQA
QPSGSKFSPAIPVSTQESVSVPASVSTSISSISTGATASALVKGNTNPITSMSAPVQASAPALTKSQTDRLEVLL
NPKDEISLNSGKPFRELESELLSRRKKDLQQIYAEERENYLGKLEREITRFFVDRGFLEIKSPILIPLEYIERMG
IDNDTELSKQIFRVDKNFCLRPMLAPNLANYLRKLDRALPDPIKIFEIGPCYRKESDGKEHLEEFTMLNFCQMGS
GCTRENLESIITDFLNHLGIDFKIVGDSCMVFGDTLDVMHGDLELSSAVVGPIPLDREWGIDKPWIGAGFGLERL
LKVKHDFKNIKRAARSESYYNGISTNL* (SEQ ID NO: 640)

LCK::OMeRS
DNA:
ATGGGCTGCGTGTGCAGCAGCAACCCCGAGGGTACCGAGCTCGCGTGCCCGGTGCCGCTGCAGCTGCCGCCGCTG
GAACGCCTGACCCTGGATGATAAAAAACCGCTGAATACCCTGATCTCTGCTACTGGTCTGTGGATGAGTCGTACC
GGAACCATTCATAAAATCAAACACCACGAGGTTAGCCGTTCGAAAATCTATATTGAGATGGCGTGTGGCGATCAT
CTGGTTGTGAACAATAGCCGCTCTTCTCGTACAGCACGTGCACTGCGTCACCACAAATATCGTAAAACCTGTAAA

CGTTGCCGTGTGTCCGATGAGGATCTGAACAAATTCCTGACAAAAGCCAATGAGGACCAAACAAGCGTGAAAGTG
AAAGTCGTTAGCGCTCCTACCCGTACTAAAAAAGCAATGCCGAAATCCGTTGCTCGTGCCCCTAAACCACTGGAA
AACACTGAAGCAGCACAGGCACAGCCGTCTGGAAGCAAATTCTCTCCGGCCATTCCTGTTTCTACCCAGGAGTCC
GTTTCTGTTCCAGCAAGTGTGAGCACCAGCATTAGCAGTATTAGCACCGGTGCCACCGCTAGCGCCCTGGTTAAA
GGCAATACCAATCCGATTACAAGCATGTCTGCCCCGGTTCAAGCATCAGCTCCAGCACTGACAAAATCCCAAACC
GATCGTCTGGAGGTTCTGCTGAATCCGAAAGACGAAATCAGCCTGAATTCCGGCAAACCGTTTCGTGAACTGGAG
AGCGAACTGCTGTCACGTCGTAAAAAAGACCTGCAACAAATCTATGCCGAAGAACGTGAGAACTATCTGGGGAAA
CTGGAACGTGAAATCACCCGCTTTTTCGTGGATCGTGGCTTTCTGGAGATCAAATCCCCGATTCTGATTCCTCTG
GAGTATATCGAGCGTATGGGCATCGACAATGATACCGAACTGAGCAAACAAATTTTCCGTGTGGATAAAAACTTC
TGTCTGCGCCCTATGCTAACACCAAATCTGTATAACTATCTGCGCAAACTGGACCGTGCCCTGCCTGATCCTATC
AAAATCTTCGAGATCGGCCCGTGTTATCGTAAAGAGTCCGACGGTAAAGAACATCTGGAGGAGTTTACCATGCTG
GTCTTTTGGCAAATGGGTTCAGGTTGTACTCGTGAGAACCTGGAAAGCATCATCACCGATTTTCTGAACCACCTG
GGCATTGACTTCAAAATTGTGGGCGACAGCTGTATGGTGTTTGGCGACACCCTGGATGTCATGCACGGCGACCTG
GAACTGTCTAGTGCCCTTGTGGGCCCAATCCCGCTGGATCGTGAGTGGGGTATCGACAAACCTTGGATCGGTGCG
GGTTTTGGTCTGGAGCGTCTGCTGAAAGTAAAACACGACTTCAAGAACATCAAACGTGCTGCACGTTCCGAGTCC
TATTACAATGGTATTTCTACTAACCTGTAA (SEQ ID NO: 641)

Protein:
MGCVCSSNPEGTELACPVPLQLPPLERLTLDDKKPLNTLISATGLWMSRTGTIHKIKHHEVSRSKIYIEMACGDH
LVVNNSRSSRTARALRHHKYRKTCKRCRVSDEDLNKFLTKANEDQTSVKVKVVSAPTRTKKAMPKSVARAPKPLE
NTEAAQAQPSGSKFSPAIPVSTQESVSVPASVSTSISSISTGATASALVKGNTNPITSMSAPVQASAPALTKSQT
DRLEVLLNPKDEISLNSGKPFRELESELLSRRKKDLQQIYAEERENYLGKLEREITRFFVDRGFLEIKSPILIPL
EYIERMGIDNDTELSKQIFRVDKNFCLRPMLTPNLYNYLRKLDRALPDPIKIFEIGPCYRKESDGKEHLEEFTML
VFWQMGSGCTRENLESIITDFLNHLGIDFKIVGDSCMVFGDTLDVMHGDLELSSALVGPIPLDREWGIDKPWIGA
GFGLERLLKVKHDFKNIKRAARSESYYNGISTNL* (SEQ ID NO: 642)

TOM20::FUS::OMeRS
DNA:
ATGGTGGGTCGGAACAGCGCCATCGCCGCCGGTGTATGCGGGGCCCTTTTCATTGGGTACTGCATCTACTTCGAC
CGCAAAAGACGAAGTGACCCCAACTTCAAGAACAGGCTTCGAGAACGAAGAAAGAAACAGAAGCTTGCCAAGGAG
AGAGCTGGGCTTTCCAAGTTACCTGACCTTAAAGATGCTGAAGCTGTTCAGAAATTCTTCATGGCCTCAAACGAT
TATACCCAACAAGCAACCCAAAGCTATGGGGCCTACCCCACCCAGCCCGGGCAGGGCTATTCCCAGCAGAGCAGT
CAGCCCTACGGACAGCAGAGTTACAGTGGTTATAGCCAGTCCACGGACACTTCAGGATATGGCCAGAGCAGCTAT
TCTTCTTATGGCCAGAGCCAGAACACAGGCTATGGAACTCAGTCAACTCCCCAGGGATATGGCTCGACTGGCGGC
TATGGCAGTAGCCAGAGCTCCCAATCGTCTTACGGGCAGCAGTCCTCCTACCCTGGCTATGGCCAGCAGCCAGCT
CCCAGCAGCACCTCGGGAAGTTACGGTAGCAGTTCTCAGAGCAGCAGCTATGGGCAGCCCCAGAGTGGGAGCTAC
AGCCAGCAGCCTAGCTATGGTGGACAGCAGCAAAGCTATGGACAGCAGCAAAGCTATAATCCCCCTCAGGGCTAT
GGACAGCAGAACCAGTACAACAGCAGCAGTGGTGGTGGAGGTGGAGGTGGAGGTGGAGGTAACTATGGCCAAGAT
CAATCCTCCATGAGTAGTGGTGGTGGCAGTGGTGGCGGTTATGGCAATCAAGACCAGAGTGGTGGAGGTGGCAGC
GGTGGCTATGGACAGCAGGACCGTGGAGGCCGCGGCAGGGGTGGCAGTGGTGGCGGCGGCGGCGGCGGTGGT
GGTTACAACCGCAGCAGTGGTGGCTATGAACCCAGAGGTCGTGGAGGTGGCCGTGGAGGCAGAGGTGGCATGGGC
GGAAGTGACCGTGGTGGCTTCAATAAATTTGGTGGCCCTCGGGACCAAGGATCACGTCATGACTCCGAACAGGAT
AATTCAGACAACAACACCATCTTTGTGCAAGGCCTGGGTGAGAATGTTACAATTGAGTCTGTGGCTGATTACTTC
AAGCAGATTGGTATTATTAAGACAAACAAGAAAACGGGACAGCCCATGATTAATTTGTACACAGACAGGGAAACT
GGCAAGCTGAAGGGAGAGGCAACGGTCTCTTTTGATGACCCACCTTCAGCTAAAGCAGCTATTGACTGGTTTGAT
GGTAAAGAATTCTCCGGAAATCCTATCAAGGTCTCATTTGCTACTCGCCGGGCAGACTTTAATCGGGGTGGTGGC
AATGGTCGTGGAGGCCGAGGGCGAGGAGGACCCATGGGCCGTGGAGGCTATGGAGGTGGTGGCAGTGGTGGTGGT
GGCCGAGGAGGATTTCCCAGTGGAGGTGGTGGCGGTGGAGGACAGCAGCGAGCTGGTGACTGGAAGTGTCCTAAT
CCCACCTGTGAGAATATGAACTTCTCTTGGAGGAATGAATGCAACCAGTGTAAGGCCCCTAAACCAGATGGCCCA
GGAGGGGGACCAGGTGGCTCTCACATGGGGGGTAACTACGGGGATGATCGTCGTGGTGGCAGAGGAGGCGCGATC
GCAGGCAAGCCTATTCCCAACCCCCTGCTGGGCCTGGATAGCACCGGAGCACCAGGAAGTGCTGGTTCTGCTGCT
GGTAGTGGAGCGTGCCCGGTGCCGCTGCAGCTGCCGCCGCTGGAACGCCTGACCCTGGATGATAAAAAACCGCTG
AATACCCTGATCTCTGCTACTGGTCTGTGGATGAGTCGTACCGGAACCATTCATAAAATCAAACACCACGAGGTT
AGCCGTTCGAAAATCTATATTGAGATGGCGTGTGGCGATCATCTGGTTGTGAACAATAGCCGCTCTTCTCGTACA
GCACGTGCACTGCGTCACCACAAATATCGTAAAACCTGTAAACGTTGCCGTGTGTCCGATGAGGATCTGAACAAA
TTCCTGACAAAAGCCAATGAGGACCAAACAAGCGTGAAAGTGAAAGTCGTTAGCGCTCCTACCCGTACTAAAAAA
GCAATGCCGAAATCCGTTGCTCGTGCCCCTAAACCACTGGAAAACACTGAAGCAGCACAGGCACAGCCGTCTGGA
AGCAAATTCTCTCCGGCCATTCCTGTTTCTACCCAGGAGTCCGTTTCTGTTCCAGCAAGTGTGAGCACCAGCATT

AGCAGTATTAGCACCGGTGCCACCGCTAGCGCCCTGGTTAAAGGCAATACCAATCCGATTACAAGCATGTCTGCC
CCGGTTCAAGCATCAGCTCCAGCACTGACAAAATCCCAAACCGATCGTCTGGAGGTTCTGCTGAATCCGAAAGAC
GAAATCAGCCTGAATTCCGGCAAACCGTTTCGTGAACTGGAGAGCGAACTGCTGTCACGTCGTAAAAAAGACCTG
CAACAAATCTATGCCGAAGAACGTGAGAACTATCTGGGGAAACTGGAACGTGAAATCACCCGCTTTTTCGTGGAT
CGTGGCTTTCTGGAGATCAAATCCCCGATTCTGATTCCTCTGGAGTATATCGAGCGTATGGGCATCGACAATGAT
ACCGAACTGAGCAAACAAATTTTCCGTGTGGATAAAAACTTCTGTCTGCGCCCTATGCTAACACCAAATCTGTAT
AACTATCTGCGCAAACTGGACCGTGCCCTGCCTGATCCTATCAAAATCTTCGAGATCGGCCCGTGTTATCGTAAA
GAGTCCGACGGTAAAGAACATCTGGAGGAGTTTACCATGCTGGTCTTTTGGCAAATGGGTTCAGGTTGTACTCGT
GAGAACCTGGAAAGCATCATCACCGATTTTCTGAACCACCTGGGCATTGACTTCAAAATTGTGGGCGACAGCTGT
ATGGTGTTTGGCGACACCCTGGATGTCATGCACGGCGACCTGGAACTGTCTAGTGCCCTTGTGGGCCCAATCCCG
CTGGATCGTGAGTGGGGTATCGACAAACCTTGGATCGGTGCGGGTTTTGGTCTGGAGCGTCTGCTGAAAGTAAAA
CACGACTTCAAGAACATCAAACGTGCTGCACGTTCCGAGTCCTATTACAATGGTATTTCTACTAACCTGTAA
(SEQ ID NO: 643)

Protein:
MVGRNSAIAAGVCGALFIGYCIYFDRKRRSDPNFKNRLRERRKKQKLAKERAGLSKLPDLKDAEAVQKFFMASND
YTQQATQSYGAYPTQPGQGYSQQSSQPYGQQSYSGYSQSTDTSGYGQSSYSSYGQSQNTGYGTQSTPQGYGSTGG
YGSSQSSQSSYGQQSSYPGYGQQPAPSSTSGSYGSSSQSSSYGQPQSGSYSQQPSYGGQQQSYGQQQSYNPPQGY
GQQNQYNSSSGGGGGGGGGGNYGQDQSSMSSGGGSGGGYGNQDQSGGGGSGGYGQQDRGGRGRGGSGGGGGGGGG
GYNRSSGGYEPRGRGGGRGGRGGMGGSDRGGFNKFGGPRDQGSRHDSEQDNSDNNTIFVQGLGENVTIESVADYF
KQIGIIKTNKKTGQPMINLYTDRETGKLKGEATVSFDDPPSAKAAIDWFDGKEFSGNPIKVSFATRRADFNRGGG
NGRGGRGRGGPMGRGGYGGGGSGGGGRGGFPSGGGGGGGQQRAGDWKCPNPTCENMNFSWRNECNQCKAPKPDGP
GGGPGGSHMGGNYGDDRRGGRGGAIAGKPIPNPLLGLDSTGAPGSAGSAAGSGACPVPLQLPPLERLTLDDKKPL
NTLISATGLWMSRTGTIHKIKHHEVSRSKIYIEMACGDHLVVNNSRSSRTARALRHHKYRKTCKRCRVSDEDLNK
FLTKANEDQTSVKVKVVSAPTRTKKAMPKSVARAPKPLENTEAAQAQPSGSKFSPAIPVSTQESVSVPASVSTSI
SSISTGATASALVKGNTNPITSMSAPVQASAPALTKSQTDRLEVLLNPKDEISLNSGKPFRELESELLSRRKKDL
QQIYAEERENYLGKLEREITRFFVDRGFLEIKSPILIPLEYIERMGIDNDTELSKQIFRVDKNFCLRPMLTPNLY
NYLRKLDRALPDPIKIFEIGPCYRKESDGKEHLEEFTMLVFWQMGSGCTRENLESIITDFLNHLGIDFKIVGDSC
MVFGDTLDVMHGDLELSSALVGPIPLDREWGIDKPWIGAGFGLERLLKVKHDFKNIKRAARSESYYNGISTNL*
(SEQ ID NO: 644)

KIF16B::FUS::OMeRS
DNA:
ATGGCATCGGTCAAGGTGGCCGTGAGGGTCCGGCCCATGAATCGCAGGGAAAAGGACTTGGAGGCCAAGTTCATT
ATTCAGATGGAGAAAAGCAAAACGACAATCACAAACTTAAAGATACCAGAAGGAGGCACTGGGGACTCAGGAAGA
GAACGGACCAAGACCTTCACCTATGACTTTTCTTTTTATTCTGCTGATACAAAAAGCCCAGATTACGTTTCACAA
GAAATGGTTTTCAAAACCCTCGGCACAGATGTCGTGAAGTCTGCATTTGAAGGTTATAATGCTTGTGTCTTTGCA
TATGGGCAAACTGGATCTGGAAAGTCATACACTATGATGGGAAATTCTGGAGATTCTGGCTTAATACCTCGGATC
TGTGAAGGACTCTTCAGTCGGATAAATGAAACCACCAGATGGGATGAAGCTTCTTTTCGAACTGAAGTCAGCTAC
TTAGAAATTTATAACGAACGTGTGAGAGATCTACTTCGGCGGAAGTCATCTAAAACCTTCAATTTGAGAGTCCGT
GAGCATCCCAAAGAAGGCCCTTATGTTGAGGATTTATCCAAACATTTAGTACAGAATTATGGTGACGTAGAAGAA
CTTATGGATGCGGGCAATATCAACCGGACCACCGCAGCGACTGGGATGAACGACGTCAGTAGCAGGTCTCATGCC
ATCTTCACCATCAAGTTCACTCAGGCTAAATTTGATTCTGAAATGCCATGTGAAACCGTCAGTAAGATCCACTTG
GTTGATCTTGCCGGAAGTGAGCGTGCAGATGCCACCGGAGCCACCGGGGTTAGGCTAAAGGAAGGGGGAAATATT
AACAAGTCCCTCGTGACTCTGGGGAACGTCATTTCTGCCTTAGCTGATTTATCTCAGGATGCTGCAAATACTCTT
GCAAAGAAGAAGCAAGTTTTCGTGCCTTACAGGGATTCTGTGTTGACTTGGTTGTTAAAAGATAGCCTTGGAGGA
AACTCTAAAACTATCATGATTGCCACCATTTCACCTGCTGATGTCAATTATGGAGAAACCCTAAGTACTCTTCGC
TATGCAAATAGAGCCAAAAACATCATCAACAAGCCTACCATTAATGAGGATGCCAACGTCAAACTTATCCGTGAG
CTGCGAGCTGAAATAGCCAGACTGAAAACGCTGCTTGCTCAAGGGAATCAGATTGCCCTCTTAGACTCCCCCACA
TATACAGATATTGAAATGAACAGATTGGGAAAGGGCGCCCCCGGCTCCGCCGGCTCCGCCGCCGGCTCCGGCATG
GCCTCAAACGATTATACCCAACAAGCAACCCAAAGCTATGGGGCCTACCCCACCCAGCCCGGGCAGGGCTATTCC
CAGCAGAGCAGTCAGCCCTACGGACAGCAGAGTTACAGTGGTTATAGCCAGTCCACGGACACTTCAGGATATGGC
CAGAGCAGCTATTCTTCTTATGGCCAGAGCCAGAACACAGGCTATGGAACTCAGTCAACTCCCCAGGGATATGGC
TCGACTGGCGGCTATGGCAGTAGCCAGAGCTCCCAATCGTCTTACGGGCAGCAGTCCTCCTACCCTGGCTATGGC
CAGCAGCCAGCTCCCAGCAGCACCTCGGGAAGTTACGGTAGCAGTTCTCAGAGCAGCAGCTATGGGCAGCCCCAG
AGTGGGAGCTACAGCCAGCAGCCTAGCTATGGTGGACAGCAGCAAAGCTATGGACAGCAGCAAAGCTATAATCCC
CCTCAGGGCTATGGACAGCAGAACCAGTACAACAGCAGCAGTGGTGGTGGAGGTGGAGGTGGAGGTGGAGGTAAC

TATGGCCAAGATCAATCCTCCATGAGTAGTGGTGGTGGCAGTGGTGGCGGTTATGGCAATCAAGACCAGAGTGGT
GGAGGTGGCAGCGGTGGCTATGGACAGCAGGACCGTGGAGGCCGCGGCAGGGGTGGCAGTGGTGGCGGCGGCGGC
GGCGGCGGTGGTGGTTACAACCGCAGCAGTGGTGGCTATGAACCCAGAGGTCGTGGAGGTGGCCGTGGAGGCAGA
GGTGGCATGGGCGGAAGTGACCGTGGTGGCTTCAATAAATTTGGTGGCCCTCGGGACCAAGGATCACGTCATGAC
TCCGAACAGGATAATTCAGACAACAACACCATCTTTGTGCAAGGCCTGGGTGAGAATGTTACAATTGAGTCTGTG
GCTGATTACTTCAAGCAGATTGGTATTATTAAGACAAACAAGAAAACGGGACAGCCCATGATTAATTTGTACACA
GACAGGGAAACTGGCAAGCTGAAGGGAGAGGCAACGGTCTCTTTTGATGACCCACCTTCAGCTAAAGCAGCTATT
GACTGGTTTGATGGTAAAGAATTCTCCGGAAATCCTATCAAGGTCTCATTTGCTACTCGCCGGGCAGACTTTAAT
CGGGGTGGTGGCAATGGTCGTGGAGGCCGAGGGCGAGGAGGACCCATGGGCCGTGGAGGCTATGGAGGTGGTGGC
AGTGGTGGTGGTGGCCGAGGAGGATTTCCCAGTGGAGGTGGTGGCGGTGGAGGACAGCAGCGAGCTGGTGACTGG
AAGTGTCCTAATCCCACCTGTGAGAATATGAACTTCTCTTGGAGGAATGAATGCAACCAGTGTAAGGCCCCTAAA
CCAGATGGCCCAGGAGGGGGACCAGGTGGCTCTCACATGGGGGGTAACTACGGGGATGATCGTCGTGGTGGCAGA
GGAGGCGATTACAAGGATGACGACGATAAGGGTACCGGCGCCCCCGGCTCCGCCGGCTCCGCCGCCGGCTCCGGC
GCGTGCCCGGTGCCGCTGCAGCTGCCGCCGCTGGAACGCCTGACCCTGGATGATAAAAAACCGCTGAATACCCTG
ATCTCTGCTACTGGTCTGTGGATGAGTCGTACCGGAACCATTCATAAAATCAAACACCACGAGGTTAGCCGTTCG
AAAATCTATATTGAGATGGCGTGTGGCGATCATCTGGTTGTGAACAATAGCCGCTCTTCTCGTACAGCACGTGCA
CTGCGTCACCACAAATATCGTAAAACCTGTAAACGTTGCCGTGTGTCCGATGAGGATCTGAACAAATTCCTGACA
AAAGCCAATGAGGACCAAACAAGCGTGAAAGTGAAAGTCGTTAGCGCTCCTACCCGTACTAAAAAAGCAATGCCG
AAATCCGTTGCTCGTGCCCCTAAACCACTGGAAAACACTGAAGCAGCACAGGCACAGCCGTCTGGAAGCAAATTC
TCTCCGGCCATTCCTGTTTCTACCCAGGAGTCCGTTTCTGTTCCAGCAAGTGTGAGCACCAGCATTAGCAGTATT
AGCACCGGTGCCACCGCTAGCGCCCTGGTTAAAGGCAATACCAATCCGATTACAAGCATGTCTGCCCCGGTTCAA
GCATCAGCTCCAGCACTGACAAAATCCCAAACCGATCGTCTGGAGGTTCTGCTGAATCCGAAAGACGAAATCAGC
CTGAATTCCGGCAAACCGTTTCGTGAACTGGAGAGCGAACTGCTGTCACGTCGTAAAAAAGACCTGCAACAAATC
TATGCCGAAGAACGTGAGAACTATCTGGGGAAACTGGAACGTGAAATCACCCGCTTTTTCGTGGATCGTGGCTTT
CTGGAGATCAAATCCCCGATTCTGATTCCTCTGGAGTATATCGAGCGTATGGGCATCGACAATGATACCGAACTG
AGCAAACAAATTTTCCGTGTGGATAAAAACTTCTGTCTGCGCCCTATGCTAACACCAAATCTGTATAACTATCTG
CGCAAACTGGACCGTGCCCTGCCTGATCCTATCAAAATCTTCGAGATCGGCCCGTGTTATCGTAAAGAGTCCGAC
GGTAAAGAACATCTGGAGGAGTTTACCATGCTGGTCTTTTGGCAAATGGGTTCAGGTTGTACTCGTGAGAACCTG
GAAAGCATCATCACCGATTTTCTGAACCACCTGGGCATTGACTTCAAAATTGTGGGCGACAGCTGTATGGTGTTT
GGCGACACCCTGGATGTCATGCACGGCGACCTGGAACTGTCTAGTGCCCTTGTGGGCCCAATCCCGCTGGATCGT
GAGTGGGGTATCGACAAACCTTGGATCGGTGCGGGTTTTGGTCTGGAGCGTCTGCTGAAAGTAAAACACGACTTC
AAGAACATCAAACGTGCTGCACGTTCCGAGTCCTATTACAATGGTATTTCTACTAACCTGTAA (SEQ ID NO:
645)

Protein:
MASVKVAVRVRPMNRREKDLEAKFIIQMEKSKTTITNLKIPEGGTGDSGRERTKTFTYDFSFYSADTKSPDYVSQ
EMVFKTLGTDVVKSAFEGYNACVFAYGQTGSGKSYTMMGNSGDSGLIPRICEGLFSRINETTRWDEASFRTEVSY
LEIYNERVRDLLRRKSSKTFNLRVREHPKEGPYVEDLSKHLVQNYGDVEELMDAGNINRTTAATGMNDVSSRSHA
IFTIKFTQAKFDSEMPCETVSKIHLVDLAGSERADATGATGVRLKEGGNINKSLVTLGNVISALADLSQDAANTL
AKKKQVFVPYRDSVLTWLLKDSLGGNSKTIMIATISPADVNYGETLSTLRYANRAKNIINKPTINEDANVKLIRE
LRAEIARLKTLLAQGNQIALLDSPTYTDIEMNRLGKGAPGSAGSAAGSGMASNDYTQQATQSYGAYPTQPGQGYS
QQSSQPYGQQSYSGYSQSTDTSGYGQSSYSSYGQSQNTGYGTQSTPQGYGSTGGYGSSQSSQSSYGQQSSYPGYG
QQPAPSSTSGSYGSSSQSSSYGQPQSGSYSQQPSYGGQQQSYGQQQSYNPPQGYGQQNQYNSSSGGGGGGGGGGN
YGQDQSSMSSGGGSGGGYGNQDQSGGGGSGGYGQQDRGGRGRGGSGGGGGGGGGGYNRSSGGYEPRGRGGGRGGR
GGMGGSDRGGFNKFGGPRDQGSRHDSEQDNSDNNTIFVQGLGENVTIESVADYFKQIGIIKTNKKTGQPMINLYT
DRETGKLKGEATVSFDDPPSAKAAIDWFDGKEFSGNPIKVSFATRRADFNRGGGNGRGGRGRGGPMGRGGYGGGG
SGGGGRGGFPSGGGGGGGQQRAGDWKCPNPTCENMNFSWRNECNQCKAPKPDGPGGGPGGSHMGGNYGDDRRGGR
GGDYKDDDDKGTGAPGSAGSAAGSGACPVPLQLPPLERLTLDDKKPLNTLISATGLWMSRTGTIHKIKHHEVSRS
KIYIEMACGDHLVVNNSRSSRTARALRHHKYRKTCKRCRVSDEDLNKFLTKANEDQTSVKVKVVSAPTRTKKAMP
KSVARAPKPLENTEAAQAQPSGSKFSPAIPVSTQESVSVPASVSTSISSISTGATASALVKGNTNPITSMSAPVQ
ASAPALTKSQTDRLEVLLNPKDEISLNSGKPFRELESELLSRRKKDLQQIYAEERENYLGKLEREITRFFVDRGF
LEIKSPILIPLEYIERMGIDNDTELSKQIFRVDKNFCLRPMLTPNLYNYLRKLDRALPDPIKIFEIGPCYRKESD
GKEHLEEFTMLVFWQMGSGCTRENLESIITDFLNHLGIDFKIVGDSCMVFGDTLDVMHGDLELSSALVGPIPLDR
EWGIDKPWIGAGFGLERLLKVKHDFKNIKRAARSESYYNGISTNL* (SEQ ID NO: 646)

LCK::FUS::OMeRS
DNA:

ATGGGCTGCGTGTGCAGCAGCAACCCCGAGGGTACCGAGCTCGCCTCAAACGATTATACCCAACAAGCAACCCAA
AGCTATGGGGCCTACCCCACCCAGCCCGGGCAGGGCTATTCCCAGCAGAGCAGTCAGCCCTACGGACAGCAGAGT
TACAGTGGTTATAGCCAGTCCACGGACACTTCAGGATATGGCCAGAGCAGCTATTCTTCTTATGGCCAGAGCCAG
AACACAGGCTATGGAACTCAGTCAACTCCCCAGGGATATGGCTCGACTGGCGGCTATGGCAGTAGCCAGAGCTCC
CAATCGTCTTACGGGCAGCAGTCCTCCTACCCTGGCTATGGCCAGCAGCCAGCTCCCAGCAGCACCTCGGGAAGT
TACGGTAGCAGTTCTCAGAGCAGCAGCTATGGGCAGCCCCAGAGTGGGAGCTACAGCCAGCAGCCTAGCTATGGT
GGACAGCAGCAAAGCTATGGACAGCAGCAAAGCTATAATCCCCCTCAGGGCTATGGACAGCAGAACCAGTACAAC
AGCAGCAGTGGTGGTGGAGGTGGAGGTGGAGGTGGAGGTAACTATGGCCAAGATCAATCCTCCATGAGTAGTGGT
GGTGGCAGTGGTGGCGGTTATGGCAATCAAGACCAGAGTGGTGGAGGTGGCAGCGGTGGCTATGGACAGCAGGAC
CGTGGAGGCCGCGGCAGGGGTGGCAGTGGTGGCGGCGGCGGCGGCGGTGGTGGTTACAACCGCAGCAGTGGT
GGCTATGAACCCAGAGGTCGTGGAGGTGGCCGTGGAGGCAGAGGTGGCATGGGCGGAAGTGACCGTGGTGGCTTC
AATAAATTTGGTGGCCCTCGGGACCAAGGATCACGTCATGACTCCGAACAGGATAATTCAGACAACAACACCATC
TTTGTGCAAGGCCTGGGTGAGAATGTTACAATTGAGTCTGTGGCTGATTACTTCAAGCAGATTGGTATTATTAAG
ACAAACAAGAAAACGGGACAGCCCATGATTAATTTGTACACAGACAGGGAAACTGGCAAGCTGAAGGGAGAGGCA
ACGGTCTCTTTTGATGACCCACCTTCAGCTAAAGCAGCTATTGACTGGTTTGATGGTAAAGAATTCTCCGGAAAT
CCTATCAAGGTCTCATTTGCTACTCGCCGGGCAGACTTTAATCGGGGTGGTGGCAATGGTCGTGGAGGCCGAGGG
CGAGGAGGACCCATGGGCCGTGGAGGCTATGGAGGTGGTGGCAGTGGTGGTGGTGGCCGAGGAGGATTTCCCAGT
GGAGGTGGTGGCGGTGGAGGACAGCAGCGAGCTGGTGACTGGAAGTGTCCTAATCCCACCTGTGAGAATATGAAC
TTCTCTTGGAGGAATGAATGCAACCAGTGTAAGGCCCCTAAACCAGATGGCCCAGGAGGGGGACCAGGTGGCTCT
CACATGGGGGGTAACTACGGGGATGATCGTCGTGGTGGCAGAGGAGGCGGCGCCCCCGGCTCCGCCGGCTCCGCC
GCCGGCTCCGGCATGGCGTGCCCGGTGCCGCTGCAGCTGCCGCCGCTGGAACGCCTGACCCTGGATGATAAAAAA
CCGCTGAATACCCTGATCTCTGCTACTGGTCTGTGGATGAGTCGTACCGGAACCATTCATAAAATCAAACACCAC
GAGGTTAGCCGTTCGAAAATCTATATTGAGATGGCGTGTGGCGATCATCTGGTTGTGAACAATAGCCGCTCTTCT
CGTACAGCACGTGCACTGCGTCACCACAAATATCGTAAAACCTGTAAACGTTGCCGTGTGTCCGATGAGGATCTG
AACAAATTCCTGACAAAAGCCAATGAGGACCAAACAAGCGTGAAAGTGAAAGTCGTTAGCGCTCCTACCCGTACT
AAAAAAGCAATGCCGAAATCCGTTGCTCGTGCCCCTAAACCACTGGAAAACACTGAAGCAGCACAGGCACAGCCG
TCTGGAAGCAAATTCTCTCCGGCCATTCCTGTTTCTACCCAGGAGTCCGTTTCTGTTCCAGCAAGTGTGAGCACC
AGCATTAGCAGTATTAGCACCGGTGCCACCGCTAGCGCCCTGGTTAAAGGCAATACCAATCCGATTACAAGCATG
TCTGCCCCGGTTCAAGCATCAGCTCCAGCACTGACAAAATCCCAAACCGATCGTCTGGAGGTTCTGCTGAATCCG
AAAGACGAAATCAGCCTGAATTCCGGCAAACCGTTTCGTGAACTGGAGAGCGAACTGCTGTCACGTCGTAAAAAA
GACCTGCAACAAATCTATGCCGAAGAACGTGAGAACTATCTGGGGAAACTGGAACGTGAAATCACCCGCTTTTTC
GTGGATCGTGGCTTTCTGGAGATCAAATCCCCGATTCTGATTCCTCTGGAGTATATCGAGCGTATGGGCATCGAC
AATGATACCGAACTGAGCAAACAAATTTTCCGTGTGGATAAAAACTTCTGTCTGCGCCCTATGCTAACACCAAAT
CTGTATAACTATCTGCGCAAACTGGACCGTGCCCTGCCTGATCCTATCAAAATCTTCGAGATCGGCCCGTGTTAT
CGTAAAGAGTCCGACGGTAAAGAACATCTGGAGGAGTTTACCATGCTGGTCTTTTGGCAAATGGGTTCAGGTTGT
ACTCGTGAGAACCTGGAAAGCATCATCACCGATTTTCTGAACCACCTGGGCATTGACTTCAAAATTGTGGGCGAC
AGCTGTATGGTGTTTGGCGACACCCTGGATGTCATGCACGGCGACCTGGAACTGTCTAGTGCCCTTGTGGGCCCA
ATCCCGCTGGATCGTGAGTGGGGTATCGACAAACCTTGGATCGGTGCGGGTTTTGGTCTGGAGCGTCTGCTGAAA
GTAAAACACGACTTCAAGAACATCAAACGTGCTGCACGTTCCGAGTCCTATTACAATGGTATTTCTACTAACCTG
TAA (SEQ ID NO: 647)

Protein:
MGCVCSSNPEGTELASNDYTQQATQSYGAYPTQPGQGYSQQSSQPYGQQSYSGYSQSTDTSGYGQSSYSSYGQSQ
NTGYGTQSTPQGYGSTGGYGSSQSSQSSYGQQSSYPGYGQQPAPSSTSGSYGSSSQSSSYGQPQSGSYSQQPSYG
GQQQSYGQQQSYNPPQGYGQQNQYNSSSGGGGGGGGGNYGQDQSSMSSGGGSGGGYGNQDQSGGGGSGGYGQQD
RGGRGRGGSGGGGGGGGGGYNRSSGGYEPRGRGGGRGGRGGMGGSDRGGFNKFGGPRDQGSRHDSEQDNSDNNTI
FVQGLGENVTIESVADYFKQIGIIKTNKKTGQPMINLYTDRETGKLKGEATVSFDDPPSAKAAIDWFDGKEFSGN
PIKVSFATRRADFNRGGGNGRGGRGRGGPMGRGGYGGGGSGGGGRGGFPSGGGGGGGQQRAGDWKCPNPTCENMN
FSWRNECNQCKAPKPDGPGGGPGGSHMGGNYGDDRRGGRGGGAPGSAGSAAGSGMACPVPLQLPPLERLTLDDKK
PLNTLISATGLWMSRTGTIHKIKHHEVSRSKIYIEMACGDHLVVNNSRSSRTARALRHHKYRKTCKRCRVSDEDL
NKFLTKANEDQTSVKVKVVSAPTRTKKAMPKSVARAPKPLENTEAAQAQPSGSKFSPAIPVSTQESVSVPASVST
SISSISTGATASALVKGNTNPITSMSAPVQASAPALTKSQTDRLEVLLNPKDEISLNSGKPFRELESELLSRRKK
DLQQIYAEERENYLGKLEREITRFFVDRGFLEIKSPILIPLEYIERMGIDNDTELSKQIFRVDKNFCLRPMLTPN
LYNYLRKLDRALPDPIKIFEIGPCYRKESDGKEHLEEFTMLVFWQMGSGCTRENLESIITDFLNHLGIDFKIVGD
SCMVFGDTLDVMHGDLELSSALVGPIPLDREWGIDKPWIGAGFGLERLLKVKHDFKNIKRAARSESYYNGISTNL
* (SEQ ID NO: 648)

EBAG9₁₋₂₉:FUS::OMeRS
DNA:
ATGGCCATCACCCAGTTTCGGTTATTTAAATTTTGTACCTGCCTAGCAACAGTATTCTCATTCCTAAAGAGATTA
ATATGCAGATCTGGAGCACCCGGCTCCGCCGGCTCCGCCGCCGGCTCCGGCATGGCCTCAAACGATTATACCCAA
CAAGCAACCCAAAGCTATGGGGCCTACCCCACCCAGCCCGGGCAGGGCTATTCCCAGCAGAGCAGTCAGCCCTAC
GGACAGCAGAGTTACAGTGGTTATAGCCAGTCCACGGACACTTCAGGATATGGCCAGAGCAGCTATTCTTCTTAT
GGCCAGAGCCAGAACACAGGCTATGGAACTCAGTCAACTCCCCAGGGATATGGCTCGACTGGCGGCTATGGCAGT
AGCCAGAGCTCCCAATCGTCTTACGGGCAGCAGTCCTCCTACCCTGGCTATGGCCAGCAGCCAGCTCCCAGCAGC
ACCTCGGGAAGTTACGGTAGCAGTTCTCAGAGCAGCAGCTATGGGCAGCCCCAGAGTGGGAGCTACAGCCAGCAG
CCTAGCTATGGTGGACAGCAGCAAAGCTATGGACAGCAGCAAAGCTATAATCCCCCTCAGGGCTATGGACAGCAG
AACCAGTACAACAGCAGCAGTGGTGGTGGAGGTGGAGGTGGAGGTGGAGGTAACTATGGCCAAGATCAATCCTCC
ATGAGTAGTGGTGGTGGCAGTGGTGGCGGTTATGGCAATCAAGACCAGAGTGGTGGAGGTGGCAGCGGTGGCTAT
GGACAGCAGGACCGTGGAGGCCGCGGCAGGGGTGGCAGTGGTGGCGGCGGCGGCGGCGGTGGTGGTTACAAC
CGCAGCAGTGGTGGCTATGAACCCAGAGGTCGTGGAGGTGGCCGTGGAGGCAGAGGTGGCATGGGCGGAAGTGAC
CGTGGTGGCTTCAATAAATTTGGTGGCCCTCGGGACCAAGGATCACGTCATGACTCCGAACAGGATAATTCAGAC
AACAACACCATCTTTGTGCAAGGCCTGGGTGAGAATGTTACAATTGAGTCTGTGGCTGATTACTTCAAGCAGATT
GGTATTATTAAGACAAACAAGAAAACGGGACAGCCCATGATTAATTTGTACACAGACAGGGAAACTGGCAAGCTG
AAGGGAGAGGCAACGGTCTCTTTTGATGACCCACCTTCAGCTAAAGCAGCTATTGACTGGTTTGATGGTAAAGAA
TTCTCCGGAAATCCTATCAAGGTCTCATTTGCTACTCGCCGGGCAGACTTTAATCGGGGTGGTGGCAATGGTCGT
GGAGGCCGAGGGCGAGGAGGACCCATGGGCCGTGGAGGCTATGGAGGTGGTGGCAGTGGTGGTGGTGGCCGAGGA
GGATTTCCCAGTGGAGGTGGTGGCGGTGGAGGACAGCAGCGAGCTGGTGACTGGAAGTGTCCTAATCCCACCTGT
GAGAATATGAACTTCTCTTGGAGGAATGAATGCAACCAGTGTAAGGCCCCTAAACCAGATGGCCCAGGAGGGGGA
CCAGGTGGCTCTCACATGGGGGGTAACTACGGGGATGATCGTCGTGGTGGCAGAGGTGGTGCGATCGCAGGCGCC
GATTACAAGGACGATGATGACAAGGGAGCACCAGGAAGTGCTGGTTCTGCTGCTGGTAGTGGAGCGTGCCCGGTG
CCGCTGCAGCTGCCGCCGCTGGAACGCCTGACCCTGGATGATAAAAAACCGCTGAATACCCTGATCTCTGCTACT
GGTCTGTGGATGAGTCGTACCGGAACCATTCATAAAATCAAACACCACGAGGTTAGCCGTTCGAAAATCTATATT
GAGATGGCGTGTGGCGATCATCTGGTTGTGAACAATAGCCGCTCTTCTCGTACAGCACGTGCACTGCGTCACCAC
AAATATCGTAAAACCTGTAAACGTTGCCGTGTGTCCGATGAGGATCTGAACAAATTCCTGACAAAAGCCAATGAG
GACCAAACAAGCGTGAAAGTGAAAGTCGTTAGCGCTCCTACCCGTACTAAAAAAGCAATGCCGAAATCCGTTGCT
CGTGCCCCTAAACCACTGGAAAACACTGAAGCAGCACAGGCACAGCCGTCTGGAAGCAAATTCTCTCCGGCCATT
CCTGTTTCTACCCAGGAGTCCGTTTCTGTTCCAGCAAGTGTGAGCACCAGCATTAGCAGTATTAGCACCGGTGCC
ACCGCTAGCGCCCTGGTTAAAGGCAATACCAATCCGATTACAAGCATGTCTGCCCCGGTTCAAGCATCAGCTCCA
GCACTGACAAAATCCCAAACCGATCGTCTGGAGGTTCTGCTGAATCCGAAAGACGAAATCAGCCTGAATTCCGGC
AAACCGTTTCGTGAACTGGAGAGCGAACTGCTGTCACGTCGTAAAAAAGACCTGCAGCAAATCTATGCCGAAGAA
CGTGAGAACTATCTGGGGAAACTGGAACGTGAAATCACCCGCTTTTTCGTGGATCGTGGCTTTCTGGAGATCAAA
TCCCCGATTCTGATTCCTCTGGAGTATATCGAGCGTATGGGCATCGACAATGATACCGAACTGAGCAAACAAATT
TTCCGTGTGGATAAAAACTTCTGTCTGCGCCCTATGCTAACACCAAATCTGTATAACTATCTGCGCAAACTGGAC
CGTGCCCTGCCTGATCCTATCAAAATCTTCGAGATCGGCCCGTGTTATCGTAAAGAGTCCGACGGTAAAGAACAT
CTGGAGGAGTTTACCATGCTGGTCTTTTGGCAAATGGGTTCAGGTTGTACTCGTGAGAACCTGGAAAGCATCATC
ACCGATTTTCTGAACCACCTGGGCATTGACTTCAAAATTGTGGGCGACAGCTGTATGGTGTTTGGCGACACCCTG
GATGTCATGCACGGCGACCTGGAACTGTCTAGTGCCCTTGTGGGCCCAATCCCGCTGGATCGTGAGTGGGGTATC
GACAAACCTTGGATCGGTGCGGGTTTTGGTCTGGAGCGTCTGCTGAAAGTAAAACACGACTTCAAGAACATCAAA
CGTGCTGCACGTTCCGAGTCCTATTACAATGGTATTTCTACTAACCTGTAA (SEQ ID NO: 649)

Protein:
MAITQFRLFKFCTCLATVFSFLKRLICRSGAPGSAGSAAGSGMASNDYTQQATQSYGAYPTQPGQGYSQQSSQPY
GQQSYSGYSQSTDTSGYGQSSYSSYGQSQNTGYGTQSTPQGYGSTGGYGSSQSSQSSYGQQSSYPGYGQQPAPSS
TSGSYGSSSQSSSYGQPQSGSYSQQPSYGGQQQSYGQQQSYNPPQGYGQQNQYNSSSGGGGGGGGGNYGQDQSS
MSSGGGSGGGYGNQDQSGGGGSGGYGQQDRGGRGRGGSGGGGGGGGGYNRSSGGYEPRGRGGGRGGRGGMGGSD
RGGFNKFGGPRDQGSRHDSEQDNSDNNTIFVQGLGENVTIESVADYFKQIGIIKTNKKTGQPMINLYTDRETGKL
KGEATVSFDDPPSAKAAIDWFDGKEFSGNPIKVSFATRRADFNRGGGNGRGGRGRGGPMGRGGYGGGGSGGGGRG
GFPSGGGGGGGQQRAGDWKCPNPTCENMNFSWRNECNQCKAPKPDGPGGGPGGSHMGGNYGDDRRGGRGGAIAGA
DYKDDDDKGAPGSAGSAAGSGACPVPLQLPPLERLTLDDKKPLNTLISATGLWMSRTGTIHKIKHHEVSRSKIYI
EMACGDHLVVNNSRSSRTARALRHHKYRKTCKRCRVSDEDLNKFLTKANEDQTSVKVKVVSAPTRTKKAMPKSVA
RAPKPLENTEAAQAQPSGSKFSPAIPVSTQESVSVPASVSTSISSISTGATASALVKGNTNPITSMSAPVQASAP
ALTKSQTDRLEVLLNPKDEISLNSGKPFRELESELLSRRKKDLQQIYAEERENYLGKLEREITRFFVDRGFLEIK
SPILIPLEYIERMGIDNDTELSKQIFRVDKNFCLRPMLTPNLYNYLRKLDRALPDPIKIFEIGPCYRKESDGKEH

LEEFTMLVFWQMGSGCTRENLESIITDFLNHLGIDFKIVGDSCMVFGDTLDVMHGDLELSSALVGPIPLDREWGI
DKPWIGAGFGLERLLKVKHDFKNIKRAARSESYYNGISTNL* (SEQ ID NO: 650)

TOM20::FUS::SYNZIP1::OMeRS
DNA:
ATGGTGGGTCGGAACAGCGCCATCGCCGCCGGTGTATGCGGGGCCCTTTTCATTGGGTACTGCATCTACTTCGAC
CGCAAAAGACGAAGTGACCCCAACTTCAAGAACAGGCTTCGAGAACGAAGAAAGAAACAGAAGCTTGCCAAGGAG
AGAGCTGGGCTTTCCAAGTTACCTGACCTTAAAGATGCTGAAGCTGTTCAGAAATTCTTCATGGCCTCAAACGAT
TATACCCAACAAGCAACCCAAAGCTATGGGGCCTACCCCACCCAGCCCGGGCAGGGCTATTCCCAGCAGAGCAGT
CAGCCCTACGGACAGCAGAGTTACAGTGGTTATAGCCAGTCCACGGACACTTCAGGATATGGCCAGAGCAGCTAT
TCTTCTTATGGCCAGAGCCAGAACACAGGCTATGGAACTCAGTCAACTCCCCAGGGATATGGCTCGACTGGCGGC
TATGGCAGTAGCCAGAGCTCCCAATCGTCTTACGGGCAGCAGTCCTCCTACCCTGGCTATGGCCAGCAGCCAGCT
CCCAGCAGCACCTCGGGAAGTTACGGTAGCAGTTCTCAGAGCAGCAGCTATGGGCAGCCCCAGAGTGGGAGCTAC
AGCCAGCAGCCTAGCTATGGTGGACAGCAGCAAAGCTATGGACAGCAGCAAAGCTATAATCCCCCTCAGGGCTAT
GGACAGCAGAACCAGTACAACAGCAGCAGTGGTGGTGGAGGTGGAGGTGGAGGTGGAGGTAACTATGGCCAAGAT
CAATCCTCCATGAGTAGTGGTGGTGGCAGTGGTGGCGGTTATGGCAATCAAGACCAGAGTGGTGGAGGTGGCAGC
GGTGGCTATGGACAGCAGGACCGTGGAGGCCGCGGCAGGGGTGGCAGTGGTGGCGGCGGCGGCGGCGGCGGTGGT
GGTTACAACCGCAGCAGTGGTGGCTATGAACCCAGAGGTCGTGGAGGTGGCCGTGGAGGCAGAGGTGGCATGGGC
GGAAGTGACCGTGGTGGCTTCAATAAATTTGGTGGCCCTCGGGACCAAGGATCACGTCATGACTCCGAACAGGAT
AATTCAGACAACAACACCATCTTTGTGCAAGGCCTGGGTGAGAATGTTACAATTGAGTCTGTGGCTGATTACTTC
AAGCAGATTGGTATTATTAAGACAAACAAGAAAACGGGACAGCCCATGATTAATTTGTACACAGACAGGGAAACT
GGCAAGCTGAAGGGAGAGGCAACGGTCTCTTTTGATGACCCACCTTCAGCTAAAGCAGCTATTGACTGGTTTGAT
GGTAAAGAATTCTCCGGAAATCCTATCAAGGTCTCATTTGCTACTCGCCGGGCAGACTTTAATCGGGGTGGTGGC
AATGGTCGTGGAGGCCGAGGGCGAGGAGGACCCATGGGCCGTGGAGGCTATGGAGGTGGTGGCAGTGGTGGTGGT
GGCCGAGGAGGATTTCCCAGTGGAGGTGGTGGCGGTGGAGGACAGCAGCGAGCTGGTGACTGGAAGTGTCCTAAT
CCCACCTGTGAGAATATGAACTTCTCTTGGAGGAATGAATGCAACCAGTGTAAGGCCCCTAAACCAGATGGCCCA
GGAGGGGGACCAGGTGGCTCTCACATGGGGGGTAACTACGGGGATGATCGTCGTGGTGGCAGAGGAGGCGCGATC
GCAAATCTGGTGGCCCAGCTGGAAAACGAGGTGGCCAGCCTGGAAAACGAGAACGAAACCCTGAAGAAAAAGAAC
CTGCACAAGAAGGACCTGATCGCCTACCTGGAAAAGGAAATCGCCAACCTGAGAAAGAAGATCGAGGAAGGCAAG
CCTATTCCCAACCCCCTGCTGGGCCTGGATAGCACCGGAGCACCAGGAAGTGCTGGTTCTGCTGCTGGTAGTGGA
GCGTGCCCGGTGCCGCTGCAGCTGCCGCCGCTGGAACGCCTGACCCTGGATGATAAAAAACCGCTGAATACCCTG
ATCTCTGCTACTGGTCTGTGGATGAGTCGTACCGGAACCATTCATAAAATCAAACACCACGAGGTTAGCCGTTCG
AAAATCTATATTGAGATGGCGTGTGGCGATCATCTGGTTGTGAACAATAGCCGCTCTTCTCGTACAGCACGTGCA
CTGCGTCACCACAAATATCGTAAAACCTGTAAACGTTGCCGTGTGTCCGATGAGGATCTGAACAAATTCCTGACA
AAAGCCAATGAGGACCAAACAAGCGTGAAAGTGAAAGTCGTTAGCGCTCCTACCCGTACTAAAAAAGCAATGCCG
AAATCCGTTGCTCGTGCCCCTAAACCACTGGAAAACACTGAAGCAGCACAGGCACAGCCGTCTGGAAGCAAATTC
TCTCCGGCCATTCCTGTTTCTACCCAGGAGTCCGTTTCTGTTCCAGCAAGTGTGAGCACCAGCATTAGCAGTATT
AGCACCGGTGCCACCGCTAGCGCCCTGGTTAAAGGCAATACCAATCCGATTACAAGCATGTCTGCCCCGGTTCAA
GCATCAGCTCCAGCACTGACAAAATCCCAAACCGATCGTCTGGAGGTTCTGCTGAATCCGAAAGACGAAATCAGC
CTGAATTCCGGCAAACCGTTTCGTGAACTGGAGAGCGAACTGCTGTCACGTCGTAAAAAAGACCTGCAACAAATC
TATGCCGAAGAACGTGAGAACTATCTGGGGAAACTGGAACGTGAAATCACCCGCTTTTTCGTGGATCGTGGCTTT
CTGGAGATCAAATCCCCGATTCTGATTCCTCTGGAGTATATCGAGCGTATGGGCATCGACAATGATACCGAACTG
AGCAAACAAATTTTCCGTGTGGATAAAAACTTCTGTCTGCGCCCTATGCTAACACCAAATCTGTATAACTATCTG
CGCAAACTGGACCGTGCCCTGCCTGATCCTATCAAAATCTTCGAGATCGGCCCGTGTTATCGTAAAGAGTCCGAC
GGTAAAGAACATCTGGAGGAGTTTACCATGCTGGTCTTTTGGCAAATGGGTTCAGGTTGTACTCGTGAGAACCTG
GAAAGCATCATCACCGATTTTCTGAACCACCTGGGCATTGACTTCAAAATTGTGGGCGACAGCTGTATGGTGTTT
GGCGACACCCTGGATGTCATGCACGGCGACCTGGAACTGTCTAGTGCCCTTGTGGGCCCAATCCCGCTGGATCGT
GAGTGGGGTATCGACAAACCTTGGATCGGTGCGGGTTTTGGTCTGGAGCGTCTGCTGAAAGTAAAACACGACTTC
AAGAACATCAAACGTGCTGCACGTTCCGAGTCCTATTACAATGGTATTTCTACTAACCTGTAA (SEQ ID NO:
651)

Protein:
MVGRNSAIAAGVCGALFIGYCIYFDRKRRSDPNFKNRLRERRKKQKLAKERAGLSKLPDLKDAEAVQKFFMASND
YTQQATQSYGAYPTQPGQGYSQQSSQPYGQQSYSGYSQSTDTSGYGQSSYSSYGQSQNTGYGTQSTPQGYGSTGG
YGSSQSSQSSYGQQSSYPGYGQQPAPSSTSGSYGSSSQSSSYGQPQSGSYSQQPSYGGQQQSYGQQQSYNPPQGY
GQQNQYNSSSGGGGGGGGGNYGQDQSSMSSGGGSGGGYGNQDQSGGGGSGGYGQQDRGGRGRGGSGGGGGGGGG
GYNRSSGGYEPRGRGGGRGGRGGMGGSDRGGFNKFGGPRDQGSRHDSEQDNSDNNTIFVQGLGENVTIESVADYF
KQIGIIKTNKKTGQPMINLYTDRETGKLKGEATVSFDDPPSAKAAIDWFDGKEFSGNPIKVSFATRRADFNRGGG

NGRGGRGRGGPMGRGGYGGGGSGGGGRGGFPSGGGGGGGQQRAGDWKCPNPTCENMNFSWRNECNQCKAPKPDGP
GGGPGGSHMGGNYGDDRRGGRGGAIANLVAQLENEVASLENENETLKKKNLHKKDLIAYLEKEIANLRKKIEEGK
PIPNPLLGLDSTGAPGSAGSAAGSGACPVPLQLPPLERLTLDDKKPLNTLISATGLWMSRTGTIHKIKHHEVSRS
KIYIEMACGDHLVVNNSRSSRTARALRHHKYRKTCKRCRVSDEDLNKFLTKANEDQTSVKVKVVSAPTRTKKAMP
KSVARAPKPLENTEAAQAQPSGSKFSPAIPVSTQESVSVPASVSTSISSISTGATASALVKGNTNPITSMSAPVQ
ASAPALTKSQTDRLEVLLNPKDEISLNSGKPFRELESELLSRRKKDLQQIYAEERENYLGKLEREITRFFVDRGF
LEIKSPILIPLEYIERMGIDNDTELSKQIFRVDKNFCLRPMLTPNLYNYLRKLDRALPDPIKIFEIGPCYRKESD
GKEHLEEFTMLVFWQMGSGCTRENLESIITDFLNHLGIDFKIVGDSCMVFGDTLDVMHGDLELSSALVGPIPLDR
EWGIDKPWIGAGFGLERLLKVKHDFKNIKRAARSESYYNGISTNL* (SEQ ID NO: 652)

TOM20::FUS::SYNZIP3::OMeRS
DNA:
ATGGTGGGTCGGAACAGCGCCATCGCCGCCGGTGTATGCGGGGCCCTTTTCATTGGGTACTGCATCTACTTCGAC
CGCAAAAGACGAAGTGACCCCAACTTCAAGAACAGGCTTCGAGAACGAAGAAAGAAACAGAAGCTTGCCAAGGAG
AGAGCTGGGCTTTCCAAGTTACCTGACCTTAAAGATGCTGAAGCTGTTCAGAAATTCTTCATGGCCTCAAACGAT
TATACCCAACAAGCAACCCAAAGCTATGGGGCCTACCCCACCCAGCCCGGGCAGGGCTATTCCCAGCAGAGCAGT
CAGCCCTACGGACAGCAGAGTTACAGTGGTTATAGCCAGTCCACGGACACTTCAGGATATGGCCAGAGCAGCTAT
TCTTCTTATGGCCAGAGCCAGAACACAGGCTATGGAACTCAGTCAACTCCCCAGGGATATGGCTCGACTGGCGGC
TATGGCAGTAGCCAGAGCTCCCAATCGTCTTACGGGCAGCAGTCCTCCTACCCTGGCTATGGCCAGCAGCCAGCT
CCCAGCAGCACCTCGGGAAGTTACGGTAGCAGTTCTCAGAGCAGCAGCTATGGGCAGCCCCAGAGTGGGAGCTAC
AGCCAGCAGCCTAGCTATGGTGGACAGCAGCAAAGCTATGGACAGCAGCAAAGCTATAATCCCCCTCAGGGCTAT
GGACAGCAGAACCAGTACAACAGCAGCAGTGGTGGTGGAGGTGGAGGTGGAGGTGGAGGTAACTATGGCCAAGAT
CAATCCTCCATGAGTAGTGGTGGTGGCAGTGGTGGCGGTTATGGCAATCAAGACCAGAGTGGTGGAGGTGGCAGC
GGTGGCTATGGACAGCAGGACCGTGGAGGCCGCGGCAGGGGTGGCAGTGGTGGCGGCGGCGGCGGCGGCGGTGGT
GGTTACAACCGCAGCAGTGGTGGCTATGAACCCAGAGGTCGTGGAGGTGGCCGTGGAGGCAGAGGTGGCATGGGC
GGAAGTGACCGTGGTGGCTTCAATAAATTTGGTGGCCCTCGGGACCAAGGATCACGTCATGACTCCGAACAGGAT
AATTCAGACAACAACACCATCTTTGTGCAAGGCCTGGGTGAGAATGTTACAATTGAGTCTGTGGCTGATTACTTC
AAGCAGATTGGTATTATTAAGACAAACAAGAAAACGGGACAGCCCATGATTAATTTGTACACAGACAGGGAAACT
GGCAAGCTGAAGGGAGAGGCAACGGTCTCTTTTGATGACCCACCTTCAGCTAAAGCAGCTATTGACTGGTTTGAT
GGTAAAGAATTCTCCGGAAATCCTATCAAGGTCTCATTTGCTACTCGCCGGGCAGACTTTAATCGGGGTGGTGGC
AATGGTCGTGGAGGCCGAGGGCGAGGAGGACCCATGGGCCGTGGAGGCTATGGAGGTGGTGGCAGTGGTGGTGGT
GGCCGAGGAGGATTTCCCAGTGGAGGTGGTGGCGGTGGAGGACAGCAGCGAGCTGGTGACTGGAAGTGTCCTAAT
CCCACCTGTGAGAATATGAACTTCTCTTGGAGGAATGAATGCAACCAGTGTAAGGCCCCTAAACCAGATGGCCCA
GGAGGGGGACCAGGTGGCTCTCACATGGGGGGTAACTACGGGGATGATCGTCGTGGTGGCAGAGGAGGCGCGATC
GCAAATGAGGTGACCACCCTGGAAAACGACGCCGCCTTCATCGAGAACGAGAACGCCTACCTGGAAAAAGAGATC
GCCAGACTGAGAAAGGAAAAGGCCGCTCTGCGGAACAGACTGGCCCACAAGAAGGGCAAGCCTATTCCCAACCCC
CTGCTGGGCCTGGATAGCACCGGAGCACCAGGAAGTGCTGGTTCTGCTGCTGGTAGTGGAGCGTGCCCGGTGCCG
CTGCAGCTGCCGCCGCTGGAACGCCTGACCCTGGATGATAAAAAACCGCTGAATACCCTGATCTCTGCTACTGGT
CTGTGGATGAGTCGTACCGGAACCATTCATAAAATCAAACACCACGAGGTTAGCCGTTCGAAAATCTATATTGAG
ATGGCGTGTGGCGATCATCTGGTTGTGAACAATAGCCGCTCTTCTCGTACAGCACGTGCACTGCGTCACCACAAA
TATCGTAAAACCTGTAAACGTTGCCGTGTGTCCGATGAGGATCTGAACAAATTCCTGACAAAAGCCAATGAGGAC
CAAACAAGCGTGAAAGTGAAAGTCGTTAGCGCTCCTACCCGTACTAAAAAAGCAATGCCGAAATCCGTTGCTCGT
GCCCCTAAACCACTGGAAAACACTGAAGCAGCACAGGCACAGCCGTCTGGAAGCAAATTCTCTCCGGCCATTCCT
GTTTCTACCCAGGAGTCCGTTTCTGTTCCAGCAAGTGTGAGCACCAGCATTAGCAGTATTAGCACCGGTGCCACC
GCTAGCGCCCTGGTTAAAGGCAATACCAATCCGATTACAAGCATGTCTGCCCCGGTTCAAGCATCAGCTCCAGCA
CTGACAAAATCCCAAACCGATCGTCTGGAGGTTCTGCTGAATCCGAAAGACGAAATCAGCCTGAATTCCGGCAAA
CCGTTTCGTGAACTGGAGAGCGAACTGCTGTCACGTCGTAAAAAAGACCTGCAACAAATCTATGCCGAAGAACGT
GAGAACTATCTGGGGAAACTGGAACGTGAAATCACCCGCTTTTTCGTGGATCGTGGCTTTCTGGAGATCAAATCC
CCGATTCTGATTCCTCTGGAGTATATCGAGCGTATGGGCATCGACAATGATACCGAACTGAGCAAACAAATTTTC
CGTGTGGATAAAAACTTCTGTCTGCGCCCTATGCTAACACCAAATCTGTATAACTATCTGCGCAAACTGGACCGT
GCCCTGCCTGATCCTATCAAAATCTTCGAGATCGGCCCGTGTTATCGTAAAGAGTCCGACGGTAAAGAACATCTG
GAGGAGTTTACCATGCTGGTCTTTTGGCAAATGGGTTCAGGTTGTACTCGTGAGAACCTGGAAAGCATCATCACC
GATTTTCTGAACCACCTGGGCATTGACTTCAAAATTGTGGGCGACAGCTGTATGGTGTTTGGCGACACCCTGGAT
GTCATGCACGGCGACCTGGAACTGTCTAGTGCCCTTGTGGGCCCAATCCCGCTGGATCGTGAGTGGGGTATCGAC
AAACCTTGGATCGGTGCGGGTTTTGGTCTGGAGCGTCTGCTGAAAGTAAAACACGACTTCAAGAACATCAAACGT
GCTGCACGTTCCGAGTCCTATTACAATGGTATTTCTACTAACCTGTAA (SEQ ID NO: 653)

Protein:
MVGRNSAIAAGVCGALFIGYCIYFDRKRRSDPNFKNRLRERRKKQKLAKERAGLSKLPDLKDAEAVQKFFMASND
YTQQATQSYGAYPTQPGQGYSQQSSQPYGQQSYSGYSQSTDTSGYGQSSYSSYGQSQNTGYGTQSTPQGYGSTGG
YGSSQSSQSSYGQQSSYPGYGQQPAPSSTSGSYGSSSQSSSYGQPQSGSYSQQPSYGGQQQSYGQQQSYNPPQGY
GQQNQYNSSSGGGGGGGGGGNYGQDQSSMSSGGGSGGGYGNQDQSGGGGSGGYGQQDRGGRGRGGSGGGGGGGGG
GYNRSSGGYEPRGRGGGRGGRGGMGGSDRGGFNKFGGPRDQGSRHDSEQDNSDNNTIFVQGLGENVTIESVADYF
KQIGIIKTNKKTGQPMINLYTDRETGKLKGEATVSFDDPPSAKAAIDWFDGKEFSGNPIKVSFATRRADFNRGGG
NGRGGRGRGGPMGRGGYGGGGSGGGGRGGFPSGGGGGGGQQRAGDWKCPNPTCENMNFSWRNECNQCKAPKPDGP
GGGPGGSHMGGNYGDDRRGGRGGAIANEVTTLENDAAFIENENAYLEKEIARLRKEKAALRNRLAHKKGKPIPNP
LLGLDSTGAPGSAGSAAGSGACPVPLQLPPLERLTLDDKKPLNTLISATGLWMSRTGTIHKIKHHEVSRSKIYIE
MACGDHLVVNNSRSSRTARALRHHKYRKTCKRCRVSDEDLNKFLTKANEDQTSVKVKVVSAPTRTKKAMPKSVAR
APKPLENTEAAQAQPSGSKFSPAIPVSTQESVSVPASVSTSISSISTGATASALVKGNTNPITSMSAPVQASAPA
LTKSQTDRLEVLLNPKDEISLNSGKPFRELESELLSRRKKDLQQIYAEERENYLGKLEREITRFFVDRGFLEIKS
PILIPLEYIERMGIDNDTELSKQIFRVDKNFCLRPMLTPNLYNYLRKLDRALPDPIKIFEIGPCYRKESDGKEHL
EEFTMLVFWQMGSGCTRENLESIITDFLNHLGIDFKIVGDSCMVFGDTLDVMHGDLELSSALVGPIPLDREWGID
KPWIGAGFGLERLLKVKHDFKNIKRAARSESYYNGISTNL* (SEQ ID NO: 654)

SLP3::FUS::PylRS(AF)
DNA:
ATGGATTCTAGGGTGTCTTCACCTGAGAAGCAAGATAAAGAGAATTTCGTGGGTGTCAACAATAAACGGCTTGGT
GTATGTGGCTGGATCCTGTTTTCCCTCTCTTTCCTGTTGGTGATCATTACCTTCCCCATCTCCATATGGATGTGC
TTGAAGATCATTAAGGAGTATGAACGTGGAGCACCCGGCTCCGCCGGCTCCGCCGCCGGCTCCGGCATGGCCTCA
AACGATTATACCCAACAAGCAACCCAAAGCTATGGGGCCTACCCCACCCAGCCCGGGCAGGGCTATTCCCAGCAG
AGCAGTCAGCCCTACGGACAGCAGAGTTACAGTGGTTATAGCCAGTCCACGGACACTTCAGGATATGGCCAGAGC
AGCTATTCTTCTTATGGCCAGAGCCAGAACACAGGCTATGGAACTCAGTCAACTCCCCAGGGATATGGCTCGACT
GGCGGCTATGGCAGTAGCCAGAGCTCCCAATCGTCTTACGGGCAGCAGTCCTCCTACCCTGGCTATGGCCAGCAG
CCAGCTCCCAGCAGCACCTCGGGAAGTTACGGTAGCAGTTCTCAGAGCAGCAGCTATGGGCAGCCCCAGAGTGGG
AGCTACAGCCAGCAGCCTAGCTATGGTGGACAGCAGCAAAGCTATGGACAGCAGCAAAGCTATAATCCCCCTCAG
GGCTATGGACAGCAGAACCAGTACAACAGCAGCAGTGGTGGTGGAGGTGGAGGTGGAGGTGGAGGTAACTATGGC
CAAGATCAATCCTCCATGAGTAGTGGTGGTGGCAGTGGTGGCGGTTATGGCAATCAAGACCAGAGTGGTGGAGGT
GGCAGCGGTGGCTATGGACAGCAGGACCGTGGAGGCCGCGGCAGGGGTGGCAGTGGTGGCGGCGGCGGCGGCGGC
GGTGGTGGTTACAACCGCAGCAGTGGTGGCTATGAACCCAGAGGTCGTGGAGGTGGCCGTGGAGGCAGAGGTGGC
ATGGGCGGAAGTGACCGTGGTGGCTTCAATAAATTTGGTGGCCCTCGGGACCAAGGATCACGTCATGACTCCGAA
CAGGATAATTCAGACAACAACACCATCTTTGTGCAAGGCCTGGGTGAGAATGTTACAATTGAGTCTGTGGCTGAT
TACTTCAAGCAGATTGGTATTATTAAGACAAACAAGAAAACGGGACAGCCCATGATTAATTTGTACACAGACAGG
GAAACTGGCAAGCTGAAGGGAGAGGCAACGGTCTCTTTTGATGACCCACCTTCAGCTAAAGCAGCTATTGACTGG
TTTGATGGTAAAGAATTCTCCGGAAATCCTATCAAGGTCTCATTTGCTACTCGCCGGGCAGACTTTAATCGGGGT
GGTGGCAATGGTCGTGGAGGCCGAGGGCGAGGAGGACCCATGGGCCGTGGAGGCTATGGAGGTGGTGGCAGTGGT
GGTGGTGGCCGAGGAGGATTTCCCAGTGGAGGTGGTGGCGGTGGAGGACAGCAGCGAGCTGGTGACTGGAAGTGT
CCTAATCCCACCTGTGAGAATATGAACTTCTCTTGGAGGAATGAATGCAACCAGTGTAAGGCCCCTAAACCAGAT
GGCCCAGGAGGGGGACCAGGTGGCTCTCACATGGGGGGTAACTACGGGGATGATCGTCGTGGTGGCAGAGGTGGT
GCGATCGCAGGCGCCGATTACAAGGACGATGATGACAAGGGAGCACCAGGAAGTGCTGGTTCTGCTGCTGGTAGT
GGAGCGTGCCCGGTGCCGCTGCAGCTGCCGCCGCTGGAACGCCTGACCCTGGATGATAAAAAACCGCTGAATACC
CTGATCTCTGCTACTGGTCTGTGGATGAGTCGTACCGGAACCATTCATAAAATCAAACACCACGAGGTTAGCCGT
TCGAAAATCTATATTGAGATGGCGTGTGGCGATCATCTGGTTGTGAACAATAGCCGCTCTTCTCGTACAGCACGT
GCACTGCGTCACCACAAATATCGTAAAACCTGTAAACGTTGCCGTGTGTCCGATGAGGATCTGAACAAATTCCTG
ACAAAAGCCAATGAGGACCAAACAAGCGTGAAAGTGAAAGTCGTTAGCGCTCCTACCCGTACTAAAAAAGCAATG
CCGAAATCCGTTGCTCGTGCCCCTAAACCACTGGAAAACACTGAAGCAGCACAGGCACAGCCGTCTGGAAGCAAA
TTCTCTCCGGCCATTCCTGTTTCTACCCAGGAGTCCGTTTCTGTTCCAGCAAGTGTGAGCACCAGCATTAGCAGT
ATTAGCACCGGTGCCACCGCTAGCGCCCTGGTTAAAGGCAATACCAATCCGATTACAAGCATGTCTGCCCCGGTT
CAAGCATCAGCTCCAGCACTGACAAAATCCCAAACCGATCGTCTGGAGGTTCTGCTGAATCCGAAAGACGAAATC
AGCCTGAATTCCGGCAAACCGTTTCGTGAACTGGAGAGCGAACTGCTGTCACGTCGTAAAAAAGACCTGCAACAA
ATCTATGCCGAAGAACGTGAGAACTATCTGGGGAAACTGGAACGTGAAATCACCCGCTTTTTCGTGGATCGTGGC
TTTCTGGAGATCAAATCCCCGATTCTGATTCCTCTGGAGTATATCGAGCGTATGGGCATCGACAATGATACCGAA
CTGAGCAAACAAATTTTCCGTGTGGATAAAAACTTCTGTCTGCGCCCTATGCTAGCACCAAATCTGGCTAACTAT
CTGCGCAAACTGGACCGTGCCCTGCCTGATCCTATCAAAATCTTCGAGATCGGCCCGTGTTATCGTAAAGAGTCC
GACGGTAAAGAACATCTGGAGGAGTTTACCATGCTGAACTTTTGCCAAATGGGTTCAGGTTGTACTCGTGAGAAC

CTGGAAAGCATCATCACCGATTTTCTGAACCACCTGGGCATTGACTTCAAAATTGTGGGCGACAGCTGTATGGTG
TTTGGCGACACCCTGGATGTCATGCACGGCGACCTGGAACTGTCTAGTGCCGTTGTGGGCCCAATCCCGCTGGAT
CGTGAGTGGGGTATCGACAAACCTTGGATCGGTGCGGGTTTTGGTCTGGAGCGTCTGCTGAAAGTAAAACACGAC
TTCAAGAACATCAAACGTGCTGCACGTTCCGAGTCCTATTACAATGGTATTTCTACTAACCTGTAA (SEQ ID
NO: 655)

Protein:
MDSRVSSPEKQDKENFVGVNNKRLGVCGWILFSLSFLLVIITFPISIWMCLKIIKEYERGAPGSAGSAAGSGMAS
NDYTQQATQSYGAYPTQPGQGYSQQSSQPYGQQSYSGYSQSTDTSGYGQSSYSSYGQSQNTGYGTQSTPQGYGST
GGYGSSQSSQSSYGQQSSYPGYGQQPAPSSTSGSYGSSSQSSSYGQPQSGSYSQQPSYGGQQQSYGQQQSYNPPQ
GYGQQNQYNSSSGGGGGGGGGGNYGQDQSSMSSGGGSGGGYGNQDQSGGGGSGGYGQQDRGGRGRGGSGGGGGGG
GGGYNRSSGGYEPRGRGGGRGGRGGMGGSDRGGFNKFGGPRDQGSRHDSEQDNSDNNTIFVQGLGENVTIESVAD
YFKQIGIIKTNKKTGQPMINLYTDRETGKLKGEATVSFDDPPSAKAAIDWFDGKEFSGNPIKVSFATRRADFNRG
GGNGRGGRGRGGPMGRGGYGGGGSGGGGRGGFPSGGGGGGGQQRAGDWKCPNPTCENMNFSWRNECNQCKAPKPD
GPGGGPGGSHMGGNYGDDRRGGRGGAIAGADYKDDDDKGAPGSAGSAAGSGACPVPLQLPPLERLTLDDKKPLNT
LISATGLWMSRTGTIHKIKHHEVSRSKIYIEMACGDHLVVNNSRSSRTARALRHHKYRKTCKRCRVSDEDLNKFL
TKANEDQTSVKVKVVSAPTRTKKAMPKSVARAPKPLENTEAAQAQPSGSKFSPAIPVSTQESVSVPASVSTSISS
ISTGATASALVKGNTNPITSMSAPVQASAPALTKSQTDRLEVLLNPKDEISLNSGKPFRELESELLSRRKKDLQQ
IYAEERENYLGKLEREITRFFVDRGFLEIKSPILIPLEYIERMGIDNDTELSKQIFRVDKNFCLRPMLAPNLANY
LRKLDRALPDPIKIFEIGPCYRKESDGKEHLEEFTMLNFCQMGSGCTRENLESIITDFLNHLGIDFKIVGDSCMV
FGDTLDVMHGDLELSSAVVGPIPLDREWGIDKPWIGAGFGLERLLKVKHDFKNIKRAARSESYYNGISTNL*
(SEQ ID NO: 656)

SLP3::MCP
DNA:
ATGGATTCTAGGGTGTCTTCACCTGAGAAGCAAGATAAAGAGAATTTCGTGGGTGTCAACAATAAACGGCTTGGT
GTATGTGGCTGGATCCTGTTTTCCCTCTCTTTCCTGTTGGTGATCATTACCTTCCCCATCTCCATATGGATGTGC
TTGAAGATCATTAAGGAGTATGAACGTGCGATCGCATATCCCTATGATGTGCCGGATTATGCTGGAGCACCAGGA
AGTGCTGGTTCTGCTGCTGGTAGTGGAGCTTCTAACTTTACTCAGTTCGTTCTCGTCGACAATGGCGGAACTGGC
GACGTGACTGTCGCCCCAAGCAACTTCGCTAACGGGATCGCTGAATGGATCAGCTCTAACTCGCGTTCACAGGCT
TACAAAGTAACCTGTAGCGTTCGTCAGAGCTCTGCGCAGAATCGCAAATACACCATCAAAGTCGAGGTGCCTAAA
GGCGCCTGGCGTTCGTACTTAAATATGGAACTAACCATTCCAATTTTCGCCACGAATTCCGACTGCGAGCTTATT
GTTAAGGCAATGCAAGGTCTCCTAAAAGATGGAAACCCGATTCCCTCAGCAATCGCAGCAAACTCCGGCATCTAC
TAA (SEQ ID NO: 657)

Protein:
MDSRVSSPEKQDKENFVGVNNKRLGVCGWILFSLSFLLVIITFPISIWMCLKIIKEYERAIAYPYDVPDYAGAPG
SAGSAAGSGASNFTQFVLVDNGGTGDVTVAPSNFANGIAEWISSNSRSQAYKVTCSVRQSSAQNRKYTIKVEVPK
GAWRSYLNMELTIPIFATNSDCELIVKAMQGLLKDGNPIPSAIAANSGIY* (SEQ ID NO: 658)

SLP3::EWSR1::MCP
DNA:
ATGGATTCTAGGGTGTCTTCACCTGAGAAGCAAGATAAAGAGAATTTCGTGGGTGTCAACAATAAACGGCTTGGT
GTATGTGGCTGGATCCTGTTTTCCCTCTCTTTCCTGTTGGTGATCATTACCTTCCCCATCTCCATATGGATGTGC
TTGAAGATCATTAAGGAGTATGAACGTATGGCGTCCACGGATTACAGTACCTATAGCCAAGCTGCAGCGCAGCAG
GGCTACAGTGCTTACACCGCCCAGCCCACTCAAGGATATGCACAGACCACCCAGGCATATGGGCAACAAAGCTAT
GGAACCTATGGACAGCCCACTGATGTCAGCTATACCCAGGCTCAGACCACTGCAACCTATGGGCAGACCGCCTAT
GCAACTTCTTATGGACAGCCTCCCACTGGTTATACTACTCCAACTGCCCCCCAGGCATACAGCCAGCCTGTCCAG
GGGTATGGCACTGGTGCTTATGATACCACCACTGCTACAGTCACCACCACCCAGGCCTCCTATGCAGCTCAGTCT
GCATATGGCACTCAGCCTGCTTATCCAGCCTATGGGCAGCAGCCAGCAGCCACTGCACCTACAAGACCGCAGGAT
GGAAACAAGCCCACTGAGACTAGTCAACCTCAATCTAGCACAGGGGGTTACAACCAGCCCAGCCTAGGATATGGA
CAGAGTAACTACAGTTATCCCCAGGTACCTGGGAGCTACCCCATGCAGCCAGTCACTGCACCTCCATCCTACCCT
CCTACCAGCTATTCCTCTACACAGCCGACTAGTTATGATCAGAGCAGTTACTCTCAGCAGAACACCTATGGGCAA
CCGAGCAGCTATGGACAGCAGAGTAGCTATGGTCAACAAAGCAGCTATGGGCAGCAGCCTCCCACTAGTTACCCA
CCCCAAACTGGATCCTACAGCCAAGCTCCAAGTCAATATAGCCAACAGAGCAGCAGCTACGGGCAGCAGAGTTCA
TTCCGACAGGACCACCCCAGTAGCATGGGTGTTTATGGGCAGGAGTCTGGAGGATTTTCCGGACCAGGAGAGAAC

CGGAGCATGAGTGGCCCTGATAACCGGGGCAGGGGAAGAGGGGGATTTGATCGTGGAGGCATGAGCAGAGGTGGG
CGGGGAGGAGGACGCGGTGGAATGGGCAGCGCTGGAGAGCGAGGTGGCTTCAATAAGCCTGGTGGACCCATGGAT
GAAGGACCAGATCTTGATCTAGGCCCACCTGTAGATCCAGATGAAGACTCTGACAACAGTGCAATTTATGTACAA
GGATTAAATGACAGTGTGACTCTAGATGATCTGGCAGACTTCTTTAAGCAGTGTGGGGTTGTTAAGATGAACAAG
AGAACTGGGCAACCCATGATCCACATCTACCTGGACAAGGAAACAGGAAAGCCCAAAGGCGATGCCACAGTGTCC
TATGAAGACCCACCTACTGCCAAGGCTGCCGTGGAATGGTTTGATGGGAAAGATTTTCAAGGGAGCAAACTTAAA
GTCTCCCTTGCTCGGAAGAAGCCTCCAATGAACAGTATGCGGGGTGGTCTGCCACCCCGTGAGGGCAGAGGCATG
CCACCACCACTCCGTGGAGGTCCAGGAGGCCCAGGAGGTCCTGGGGGACCCATGGGTCGCATGGGAGGCCGTGGA
GGAGATAGAGGAGGCTTCCCTCCAAGAGGACCCCGGGGTTCCCGAGGGAACCCCTCTGGAGGAGGAAACGTCCAG
CACCGAGCTGGAGACTGGCAGTGTCCCAATCCGGGTTGTGGAAACCAGAACTTCGCCTGGAGAACAGAGTGCAAC
CAGTGTAAGGCCCCAAAGCCTGAAGGCTTCCTCCCGCCACCCTTTCCGCCCCCGGGTGGTGATCGTGGCAGAGGT
GGCCCTGGTGGCATGCGGGGAGGAAGAGGTGGCCTCATGGATCGTGGTGGTCCCGGTGGAATGTTCAGAGGTGGC
CGTGGTGGAGACAGAGGTGGCTTCCGTGGTGGCCGGGGCATGGACCGAGGTGGCTTTGGTGGAGGAAGACGAGGT
GGCCCTGGGGGGCCCCCTGGACCTTTGATGGAACAGGCGATCGCATATCCCTATGATGTGCCGGATTATGCTGGA
GCACCAGGAAGTGCTGGTTCTGCTGCTGGTAGTGGAGCTTCTAACTTTACTCAGTTCGTTCTCGTCGACAATGGC
GGAACTGGCGACGTGACTGTCGCCCCAAGCAACTTCGCTAACGGGATCGCTGAATGGATCAGCTCTAACTCGCGT
TCACAGGCTTACAAAGTAACCTGTAGCGTTCGTCAGAGCTCTGCGCAGAATCGCAAATACACCATCAAAGTCGAG
GTGCCTAAAGGCGCCTGGCGTTCGTACTTAAATATGGAACTAACCATTCCAATTTTCGCCACGAATTCCGACTGC
GAGCTTATTGTTAAGGCAATGCAAGGTCTCCTAAAAGATGGAAACCCGATTCCCTCAGCAATCGCAGCAAACTCC
GGCATCTACTAA (SEQ ID NO: 659)

Protein:
MDSRVSSPEKQDKENFVGVNNKRLGVCGWILFSLSFLLVIITFPISIWMCLKIIKEYERMASTDYSTYSQAAAQQ
GYSAYTAQPTQGYAQTTQAYGQQSYGTYGQPTDVSYTQAQTTATYGQTAYATSYGQPPTGYTTPTAPQAYSQPVQ
GYGTGAYDTTTATVTTTQASYAAQSAYGTQPAYPAYGQQPAATAPTRPQDGNKPTETSQPQSSTGGYNQPSLGYG
QSNYSYPQVPGSYPMQPVTAPPSYPPTSYSSTQPTSYDQSSYSQQNTYGQPSSYGQQSSYGQQSSYGQQPPTSYP
PQTGSYSQAPSQYSQQSSSYGQQSSFRQDHPSSMGVYGQESGGFSGPGENRSMSGPDNRGRGRGGFDRGGMSRGG
RGGGRGGMGSAGERGGFNKPGGPMDEGPDLDLGPPVDPDEDSDNSAIYVQGLNDSVTLDDLADFFKQCGVVKMNK
RTGQPMIHIYLDKETGKPKGDATVSYEDPPTAKAAVEWFDGKDFQGSKLKVSLARKKPPMNSMRGGLPPREGRGM
PPPLRGGPGGPGGPGGPMGRMGGRGGDRGGFPPRGPRGSRGNPSGGGNVQHRAGDWQCPNPGCGNQNFAWRTECN
QCKAPKPEGFLPPPFPPPGGDRGRGGPGGMRGGRGGLMDRGGPGGMFRGGRGGDRGGFRGGRGMDRGGFGGGRRG
GPGGPPGPLMEQAIAYPYDVPDYAGAPGSAGSAAGSGASNFTQFVLVDNGGTGDVTVAPSNFANGIAEWISSNSR
SQAYKVTCSVRQSSAQNRKYTIKVEVPKGAWRSYLNMELTIPIFATNSDCELIVKAMQGLLKDGNPIPSAIAANS
GIY* (SEQ ID NO: 660)

SLP3::EWSR1::4xλN22
DNA:
ATGGATTCTAGGGTGTCTTCACCTGAGAAGCAAGATAAAGAGAATTTCGTGGGTGTCAACAATAAACGGCTTGGT
GTATGTGGCTGGATCCTGTTTTCCCTCTCTTTCCTGTTGGTGATCATTACCTTCCCCATCTCCATATGGATGTGC
TTGAAGATCATTAAGGAGTATGAACGTATGGCGTCCACGGATTACAGTACCTATAGCCAAGCTGCAGCGCAGCAG
GGCTACAGTGCTTACACCGCCCAGCCCACTCAAGGATATGCACAGACCACCCAGGCATATGGGCAACAAAGCTAT
GGAACCTATGGACAGCCCACTGATGTCAGCTATACCCAGGCTCAGACCACTGCAACCTATGGGCAGACCGCCTAT
GCAACTTCTTATGGACAGCCTCCCACTGGTTATACTACTCCAACTGCCCCCCAGGCATACAGCCAGCCTGTCCAG
GGGTATGGCACTGGTGCTTATGATACCACCACTGCTACAGTCACCACCACCCAGGCCTCCTATGCAGCTCAGTCT
GCATATGGCACTCAGCCTGCTTATCCAGCCTATGGGCAGCAGCCAGCAGCCACTGCACCTACAAGACCGCAGGAT
GGAAACAAGCCCACTGAGACTAGTCAACCTCAATCTAGCACAGGGGGTTACAACCAGCCCAGCCTAGGATATGGA
CAGAGTAACTACAGTTATCCCCAGGTACCTGGGAGCTACCCCATGCAGCCAGTCACTGCACCTCCATCCTACCCT
CCTACCAGCTATTCCTCTACACAGCCGACTAGTTATGATCAGAGCAGTTACTCTCAGCAGAACACCTATGGGCAA
CCGAGCAGCTATGGACAGCAGAGTAGCTATGGTCAACAAAGCAGCTATGGGCAGCAGCCTCCCACTAGTTACCCA
CCCCAAACTGGATCCTACAGCCAAGCTCCAAGTCAATATAGCCAACAGAGCAGCAGCTACGGGCAGCAGAGTTCA
TTCCGACAGGACCACCCCAGTAGCATGGGTGTTTATGGGCAGGAGTCTGGAGGATTTTCCGGACCAGGAGAGAAC
CGGAGCATGAGTGGCCCTGATAACCGGGGCAGGGGAAGAGGGGGATTTGATCGTGGAGGCATGAGCAGAGGTGGG
CGGGGAGGAGGACGCGGTGGAATGGGCAGCGCTGGAGAGCGAGGTGGCTTCAATAAGCCTGGTGGACCCATGGAT
GAAGGACCAGATCTTGATCTAGGCCCACCTGTAGATCCAGATGAAGACTCTGACAACAGTGCAATTTATGTACAA
GGATTAAATGACAGTGTGACTCTAGATGATCTGGCAGACTTCTTTAAGCAGTGTGGGGTTGTTAAGATGAACAAG
AGAACTGGGCAACCCATGATCCACATCTACCTGGACAAGGAAACAGGAAAGCCCAAAGGCGATGCCACAGTGTCC
TATGAAGACCCACCTACTGCCAAGGCTGCCGTGGAATGGTTTGATGGGAAAGATTTTCAAGGGAGCAAACTTAAA

GTCTCCCTTGCTCGGAAGAAGCCTCCAATGAACAGTATGCGGGGTGGTCTGCCACCCCGTGAGGGCAGAGGCATG
CCACCACCACTCCGTGGAGGTCCAGGAGGCCCAGGAGGTCCTGGGGGACCCATGGGTCGCATGGGAGGCCGTGGA
GGAGATAGAGGAGGCTTCCCTCCAAGAGGACCCCGGGGTTCCCGAGGGAACCCCTCTGGAGGAGGAAACGTCCAG
CACCGAGCTGGAGACTGGCAGTGTCCCAATCCGGGTTGTGGAAACCAGAACTTCGCCTGGAGAACAGAGTGCAAC
CAGTGTAAGGCCCCAAAGCCTGAAGGCTTCCTCCCGCCACCCTTTCCGCCCCCGGGTGGTGATCGTGGCAGAGGT
GGCCCTGGTGGCATGCGGGGAGGAAGAGGTGGCCTCATGGATCGTGGTGGTCCCGGTGGAATGTTCAGAGGTGGC
CGTGGTGGAGACAGAGGTGGCTTCCGTGGTGGCCGGGGCATGGACCGAGGTGGCTTTGGTGGAGGAAGACGAGGT
GGCCCTGGGGGGCCCCCTGGACCTTTGATGGAACAGGCGATCGCAGGAGCACCAGGAAGTGCTGGTTCTGCTGCT
GGTAGTGGAGAGCAGAAGCTGATCTCAGAGGAGGACCTGCTAGCCACCATGGACGCACAAACACGACGACGTGAG
CGTCGCGCTGAGAAACAAGCTCAATGGAAAGCTGCAAACCCACCGCTCGACGGAGCCGGAGCTGGCGCTGGAGCT
GGAGCCGGAGCTGGCGGTCTAGCCACCATGGACGCACAAACACGACGACGTGAGCGTCGCGCTGAGAAACAAGCT
CAATGGAAAGCTGCAAACCCACCGCTCGACGGAGCCGGAGCTGGCGCTGGAGCTGGAGCCGGAGCTGGCGGTCTA
GCCACCATGGACGCACAAACACGACGACGTGAGCGTCGCGCTGAGAAACAAGCTCAATGGAAAGCTGCAAACCCA
CCGCTCGACGGAGCCGGAGCTGGCGCTGGAGCTGGAGCCGGAGCTGGCGGTCTAGCCACCATGGACGCACAAACA
CGACGACGTGAGCGTCGCGCTGAGAAACAAGCTCAATGGAAAGCTGCAAACCCACCGCTCGAGTCTAGAGGGCCC
GTTTAA (SEQ ID NO: 661)

Protein:
MDSRVSSPEKQDKENFVGVNNKRLGVCGWILFSLSFLLVIITFPISIWMCLKIIKEYERMASTDYSTYSQAAAQQ
GYSAYTAQPTQGYAQTTQAYGQQSYGTYGQPTDVSYTQAQTTATYGQTAYATSYGQPPTGYTTPTAPQAYSQPVQ
GYGTGAYDTTTATVTTTQASYAAQSAYGTQPAYPAYGQQPAATAPTRPQDGNKPTETSQPQSSTGGYNQPSLGYG
QSNYSYPQVPGSYPMQPVTAPPSYPPTSYSSTQPTSYDQSSYSQQNTYGQPSSYGQQSSYGQQSSYGQQPPTSYP
PQTGSYSQAPSQYSQQSSSYGQQSSFRQDHPSSMGVYGQESGGFSGPGENRSMSGPDNRGRGRGGFDRGGMSRGG
RGGGRGGMGSAGERGGFNKPGGPMDEGPDLDLGPPVDPDEDSDNSAIYVQGLNDSVTLDDLADFFKQCGVVKMNK
RTGQPMIHIYLDKETGKPKGDATVSYEDPPTAKAAVEWFDGKDFQGSKLKVSLARKKPPMNSMRGGLPPREGRGM
PPPLRGGPGGPGGPGGPMGRMGGRGGDRGGFPPRGPRGSRGNPSGGGNVQHRAGDWQCPNPGCGNQNFAWRTECN
QCKAPKPEGFLPPPFPPPGGDRGRGGPGGMRGGRGGLMDRGGPGGMFRGGRGGDRGGFRGGRGMDRGGFGGGRRG
GPGGPPGPLMEQAIAGAPGSAGSAAGSGEQKLISEEDLLATMDAQTRRRERRAEKQAQWKAANPPLDGAGAGAGA
GAGAGGLATMDAQTRRRERRAEKQAQWKAANPPLDGAGAGAGAGAGAGGLATMDAQTRRRERRAEKQAQWKAANP
PLDGAGAGAGAGAGAGGLATMDAQTRRRERRAEKQAQWKAANPPLESRGPV* (SEQ ID NO: 662)

SLP3::PylRS(AF)
DNA:
ATGGATTCTAGGGTGTCTTCACCTGAGAAGCAAGATAAAGAGAATTTCGTGGGTGTCAACAATAAACGGCTTGGT
GTATGTGGCTGGATCCTGTTTTCCCTCTCTTTCCTGTTGGTGATCATTACCTTCCCCATCTCCATATGGATGTGC
TTGAAGATCATTAAGGAGTATGAACGTGGCGCCGATTACAAGGACGATGATGACAAGGGAGCACCAGGAAGTGCT
GGTTCTGCTGCTGGTAGTGGAGCGTGCCCGGTGCCGCTGCAGCTGCCGCCGCTGGAACGCCTGACCCTGGATGAT
AAAAAACCGCTGAATACCCTGATCTCTGCTACTGGTCTGTGGATGAGTCGTACCGGAACCATTCATAAAATCAAA
CACCACGAGGTTAGCCGTTCGAAAATCTATATTGAGATGGCGTGTGGCGATCATCTGGTTGTGAACAATAGCCGC
TCTTCTCGTACAGCACGTGCACTGCGTCACCACAAATATCGTAAAACCTGTAAACGTTGCCGTGTGTCCGATGAG
GATCTGAACAAATTCCTGACAAAAGCCAATGAGGACCAAACAAGCGTGAAAGTGAAAGTCGTTAGCGCTCCTACC
CGTACTAAAAAAGCAATGCCGAAATCCGTTGCTCGTGCCCCTAAACCACTGGAAAACACTGAAGCAGCACAGGCA
CAGCCGTCTGGAAGCAAATTCTCTCCGGCCATTCCTGTTTCTACCCAGGAGTCCGTTTCTGTTCCAGCAAGTGTG
AGCACCAGCATTAGCAGTATTAGCACCGGTGCCACCGCTAGCGCCCTGGTTAAAGGCAATACCAATCCGATTACA
AGCATGTCTGCCCCGGTTCAAGCATCAGCTCCAGCACTGACAAAATCCCAAACCGATCGTCTGGAGGTTCTGCTG
AATCCGAAAGACGAAATCAGCCTGAATTCCGGCAAACCGTTTCGTGAACTGGAGAGCGAACTGCTGTCACGTCGT
AAAAAAGACCTGCAACAAATCTATGCCGAAGAACGTGAGAACTATCTGGGGAAACTGGAACGTGAAATCACCCGC
TTTTTCGTGGATCGTGGCTTTCTGGAGATCAAATCCCCGATTCTGATTCCTCTGGAGTATATCGAGCGTATGGGC
ATCGACAATGATACCGAACTGAGCAAACAAATTTTCCGTGTGGATAAAAACTTCTGTCTGCGCCCTATGCTAGCA
CCAAATCTGGCTAACTATCTGCGCAAACTGGACCGTGCCCTGCCTGATCCTATCAAAATCTTCGAGATCGGCCCG
TGTTATCGTAAAGAGTCCGACGGTAAAGAACATCTGGAGGAGTTTACCATGCTGAACTTTTGCCAAATGGGTTCA
GGTTGTACTCGTGAGAACCTGGAAAGCATCATCACCGATTTTCTGAACCACCTGGGCATTGACTTCAAAATTGTG
GGCGACAGCTGTATGGTGTTTGGCGACACCCTGGATGTCATGCACGGCGACCTGGAACTGTCTAGTGCCGTTGTG
GGCCCAATCCCGCTGGATCGTGAGTGGGGTATCGACAAACCTTGGATCGGTGCGGGTTTTGGTCTGGAGCGTCTG
CTGAAAGTAAAACACGACTTCAAGAACATCAAACGTGCTGCACGTTCCGAGTCCTATTACAATGGTATTTCTACT
AACCTGTAA (SEQ ID NO: 663)

Protein:
MDSRVSSPEKQDKENFVGVNNKRLGVCGWILFSLSFLLVIITFPISIWMCLKIIKEYERGADYKDDDDKGAPGSA
GSAAGSGACPVPLQLPPLERLTLDDKKPLNTLISATGLWMSRTGTIHKIKHHEVSRSKIYIEMACGDHLVVNNSR
SSRTARALRHHKYRKTCKRCRVSDEDLNKFLTKANEDQTSVKVKVVSAPTRTKKAMPKSVARAPKPLENTEAAQA
QPSGSKFSPAIPVSTQESVSVPASVSTSISSISTGATASALVKGNTNPITSMSAPVQASAPALTKSQTDRLEVLL
NPKDEISLNSGKPFRELESELLSRRKKDLQQIYAEERENYLGKLEREITRFFVDRGFLEIKSPILIPLEYIERMG
IDNDTELSKQIFRVDKNFCLRPMLAPNLANYLRKLDRALPDPIKIFEIGPCYRKESDGKEHLEEFTMLNFCQMGS
GCTRENLESIITDFLNHLGIDFKIVGDSCMVFGDTLDVMHGDLELSSAVVGPIPLDREWGIDKPWIGAGFGLERL
LKVKHDFKNIKRAARSESYYNGISTNL* (SEQ ID NO: 664)

TOM20::FUS::MCP::PylRS(AF)
DNA:
ATGGTGGGTCGGAACAGCGCCATCGCCGCCGGTGTATGCGGGGCCCTTTTCATTGGGTACTGCATCTACTTCGAC
CGCAAAAGACGAAGTGACCCCAACTTCAAGAACAGGCTTCGAGAACGAAGAAAGAAACAGAAGCTTGCCAAGGAG
AGAGCTGGGCTTTCCAAGTTACCTGACCTTAAAGATGCTGAAGCTGTTCAGAAATTCTTCATGGCCTCAAACGAT
TATACCCAACAAGCAACCCAAAGCTATGGGGCCTACCCCACCCAGCCCGGGCAGGGCTATTCCCAGCAGAGCAGT
CAGCCCTACGGACAGCAGAGTTACAGTGGTTATAGCCAGTCCACGGACACTTCAGGATATGGCCAGAGCAGCTAT
TCTTCTTATGGCCAGAGCCAGAACACAGGCTATGGAACTCAGTCAACTCCCCAGGGATATGGCTCGACTGGCGGC
TATGGCAGTAGCCAGAGCTCCCAATCGTCTTACGGGCAGCAGTCCTCCTACCCTGGCTATGGCCAGCAGCCAGCT
CCCAGCAGCACCTCGGGAAGTTACGGTAGCAGTTCTCAGAGCAGCAGCTATGGGCAGCCCCAGAGTGGGAGCTAC
AGCCAGCAGCCTAGCTATGGTGGACAGCAGCAAAGCTATGGACAGCAGCAAAGCTATAATCCCCCTCAGGGCTAT
GGACAGCAGAACCAGTACAACAGCAGCAGTGGTGGTGGAGGTGGAGGTGGAGGTGGAGGTAACTATGGCCAAGAT
CAATCCTCCATGAGTAGTGGTGGTGGCAGTGGTGGCGGTTATGGCAATCAAGACCAGAGTGGTGGAGGTGGCAGC
GGTGGCTATGGACAGCAGGACCGTGGAGGCCGCGGCAGGGGTGGCAGTGGTGGCGGCGGCGGCGGCGGCGGTGGT
GGTTACAACCGCAGCAGTGGTGGCTATGAACCCAGAGGTCGTGGAGGTGGCCGTGGAGGCAGAGGTGGCATGGGC
GGAAGTGACCGTGGTGGCTTCAATAAATTTGGTGGCCCTCGGGACCAAGGATCACGTCATGACTCCGAACAGGAT
AATTCAGACAACAACACCATCTTTGTGCAAGGCCTGGGTGAGAATGTTACAATTGAGTCTGTGGCTGATTACTTC
AAGCAGATTGGTATTATTAAGACAAACAAGAAAACGGGACAGCCCATGATTAATTTGTACACAGACAGGGAAACT
GGCAAGCTGAAGGGAGAGGCAACGGTCTCTTTTGATGACCCACCTTCAGCTAAAGCAGCTATTGACTGGTTTGAT
GGTAAAGAATTCTCCGGAAATCCTATCAAGGTCTCATTTGCTACTCGCCGGGCAGACTTTAATCGGGGTGGTGGC
AATGGTCGTGGAGGCCGAGGGCGAGGAGGACCCATGGGCCGTGGAGGCTATGGAGGTGGTGGCAGTGGTGGTGGT
GGCCGAGGAGGATTTCCCAGTGGAGGTGGTGGCGGTGGAGGACAGCAGCGAGCTGGTGACTGGAAGTGTCCTAAT
CCCACCTGTGAGAATATGAACTTCTCTTGGAGGAATGAATGCAACCAGTGTAAGGCCCCTAAACCAGATGGCCCA
GGAGGGGGACCAGGTGGCTCTCACATGGGGGGTAACTACGGGGATGATCGTCGTGGTGGCAGAGGAGGCGCGATC
GCATATCCCTATGATGTGCCGGATTATGCTGGAGCACCAGGAAGTGCTGGTTCTGCTGCTGGTAGTGGAGCTTCT
AACTTTACTCAGTTCGTTCTCGTCGACAATGGCGGAACTGGCGACGTGACTGTCGCCCCAAGCAACTTCGCTAAC
GGGATCGCTGAATGGATCAGCTCTAACTCGCGTTCACAGGCTTACAAAGTAACCTGTAGCGTTCGTCAGAGCTCT
GCGCAGAATCGCAAATACACCATCAAAGTCGAGGTGCCTAAAGGCGCCTGGCGTTCGTACTTAAATATGGAACTA
ACCATTCCAATTTTCGCCACGAATTCCGACTGCGAGCTTATTGTTAAGGCAATGCAAGGTCTCCTAAAAGATGGA
AACCCGATTCCCTCAGCAATCGCAGCAAACTCCGGCATCTACGGCGCCGATTACAAGGACGATGATGACAAGGGA
GCACCAGGAAGTGCTGGTTCTGCTGCTGGTAGTGGAGCGTGCCCGGTGCCGCTGCAGCTGCCGCCGCTGGAACGC
CTGACCCTGGATGATAAAAAACCGCTGAATACCCTGATCTCTGCTACTGGTCTGTGGATGAGTCGTACCGGAACC
ATTCATAAAATCAAACACCACGAGGTTAGCCGTTCGAAAATCTATATTGAGATGGCGTGTGGCGATCATCTGGTT
GTGAACAATAGCCGCTCTTCTCGTACAGCACGTGCACTGCGTCACCACAAATATCGTAAAACCTGTAAACGTTGC
CGTGTGTCCGATGAGGATCTGAACAAATTCCTGACAAAAGCCAATGAGGACCAAACAAGCGTGAAAGTGAAAGTC
GTTAGCGCTCCTACCCGTACTAAAAAAGCAATGCCGAAATCCGTTGCTCGTGCCCCTAAACCACTGGAAAACACT
GAAGCAGCACAGGCACAGCCGTCTGGAAGCAAATTCTCTCCGGCCATTCCTGTTTCTACCCAGGAGTCCGTTTCT
GTTCCAGCAAGTGTGAGCACCAGCATTAGCAGTATTAGCACCGGTGCCACCGCTAGCGCCCTGGTTAAAGGCAAT
ACCAATCCGATTACAAGCATGTCTGCCCCGGTTCAAGCATCAGCTCCAGCACTGACAAAATCCCAAACCGATCGT
CTGGAGGTTCTGCTGAATCCGAAAGACGAAATCAGCCTGAATTCCGGCAAACCGTTTCGTGAACTGGAGAGCGAA
CTGCTGTCACGTCGTAAAAAAGACCTGCAACAAATCTATGCCGAAGAACGTGAGAACTATCTGGGGAAACTGGAA
CGTGAAATCACCCGCTTTTTCGTGGATCGTGGCTTTCTGGAGATCAAATCCCCGATTCTGATTCCTCTGGAGTAT
ATCGAGCGTATGGGCATCGACAATGATACCGAACTGAGCAAACAAATTTTCCGTGTGGATAAAAACTTCTGTCTG
CGCCCTATGCTAGCACCAAATCTGGCTAACTATCTGCGCAAACTGGACCGTGCCCTGCCTGATCCTATCAAAATC
TTCGAGATCGGCCCGTGTTATCGTAAAGAGTCCGACGGTAAAGAACATCTGGAGGAGTTTACCATGCTGAACTTT
TGCCAAATGGGTTCAGGTTGTACTCGTGAGAACCTGGAAAGCATCATCACCGATTTTCTGAACCACCTGGGCATT
GACTTCAAAATTGTGGGCGACAGCTGTATGGTGTTTGGCGACACCCTGGATGTCATGCACGGCGACCTGGAACTG

TCTAGTGCCGTTGTGGGCCCAATCCCGCTGGATCGTGAGTGGGGTATCGACAAACCTTGGATCGGTGCGGGTTTT
GGTCTGGAGCGTCTGCTGAAAGTAAAACACGACTTCAAGAACATCAAACGTGCTGCACGTTCCGAGTCCTATTAC
AATGGTATTTCTACTAACCTGTAA (SEQ ID NO: 665)

Protein:
MVGRNSAIAAGVCGALFIGYCIYFDRKRRSDPNFKNRLRERRKKQKLAKERAGLSKLPDLKDAEAVQKFFMASND
YTQQATQSYGAYPTQPGQGYSQQSSQPYGQQSYSGYSQSTDTSGYGQSSYSSYGQSQNTGYGTQSTPQGYGSTGG
YGSSQSSQSSYGQQSSYPGYGQQPAPSSTSGSYGSSSQSSSYGQPQSGSYSQQPSYGGQQQSYGQQQSYNPPQGY
GQQNQYNSSSGGGGGGGGGGNYGQDQSSMSSGGGSGGGYGNQDQSGGGGSGGYGQQDRGGRGRGGSGGGGGGGG
GYNRSSGGYEPRGRGGGRGGRGGMGGSDRGGFNKFGGPRDQGSRHDSEQDNSDNNTIFVQGLGENVTIESVADYF
KQIGIIKTNKKTGQPMINLYTDRETGKLKGEATVSFDDPPSAKAAIDWFDGKEFSGNPIKVSFATRRADFNRGGG
NGRGGRGRGGPMGRGGYGGGGSGGGGRGGFPSGGGGGGGQQRAGDWKCPNPTCENMNFSWRNECNQCKAPKPDGP
GGGPGGSHMGGNYGDDRRGGRGGAIAYPYDVPDYAGAPGSAGSAAGSGASNFTQFVLVDNGGTGDVTVAPSNFAN
GIAEWISSNSRSQAYKVTCSVRQSSAQNRKYTIKVEVPKGAWRSYLNMELTIPIFATNSDCELIVKAMQGLLKDG
NPIPSAIAANSGIYGADYKDDDDKGAPGSAGSAAGSGACPVPLQLPPLERLTLDDKKPLNTLISATGLWMSRTGT
IHKIKHHEVSRSKIYIEMACGDHLVVNNSRSSRTARALRHHKYRKTCKRCRVSDEDLNKFLTKANEDQTSVKVKV
VSAPTRTKKAMPKSVARAPKPLENTEAAQAQPSGSKFSPAIPVSTQESVSVPASVSTSISSISTGATASALVKGN
TNPITSMSAPVQASAPALTKSQTDRLEVLLNPKDEISLNSGKPFRELESELLSRRKKDLQQIYAEERENYLGKLE
REITRFFVDRGFLEIKSPILIPLEYIERMGIDNDTELSKQIFRVDKNFCLRPMLAPNLANYLRKLDRALPDPIKI
FEIGPCYRKESDGKEHLEEFTMLNFCQMGSGCTRENLESIITDFLNHLGIDFKIVGDSCMVFGDTLDVMHGDLEL
SSAVVGPIPLDREWGIDKPWIGAGFGLERLLKVKHDFKNIKRAARSESYYNGISTNL* (SEQ ID NO: 666)

KIF16B::1xLAF-1::PylRS(AF)
DNA:
ATGGCATCGGTCAAGGTGGCCGTGAGGGTCCGGCCCATGAATCGCAGGGAAAAGGACTTGGAGGCCAAGTTCATT
ATTCAGATGGAGAAAAGCAAAACGACAATCACAAACTTAAAGATACCAGAAGGAGGCACTGGGGACTCAGGAAGA
GAACGGACCAAGACCTTCACCTATGACTTTTCTTTTTATTCTGCTGATACAAAAAGCCCAGATTACGTTTCACAA
GAAATGGTTTTCAAAACCCTCGGCACAGATGTCGTGAAGTCTGCATTTGAAGGTTATAATGCTTGTGTCTTTGCA
TATGGGCAAACTGGATCTGGAAAGTCATACACTATGATGGGAAATTCTGGAGATTCTGGCTTAATACCTCGGATC
TGTGAAGGACTCTTCAGTCGGATAAATGAAACCACCAGATGGGATGAAGCTTCTTTTCGAACTGAAGTCAGCTAC
TTAGAAATTTATAACGAACGTGTGAGAGATCTACTTCGGCGGAAGTCATCTAAAACCTTCAATTTGAGAGTCCGT
GAGCATCCCAAAGAAGGCCCTTATGTTGAGGATTTATCCAAACATTTAGTACAGAATTATGGTGACGTAGAAGAA
CTTATGGATGCGGGCAATATCAACCGGACCACCGCAGCGACTGGGATGAACGACGTCAGTAGCAGGTCTCATGCC
ATCTTCACCATCAAGTTCACTCAGGCTAAATTTGATTCTGAAATGCCATGTGAAACCGTCAGTAAGATCCACTTG
GTTGATCTTGCCGGAAGTGAGCGTGCAGATGCCACCGGAGCCACCGGGGTTAGGCTAAAGGAAGGGGGAAATATT
AACAAGTCCCTCGTGACTCTGGGGAACGTCATTTCTGCCTTAGCTGATTTATCTCAGGATGCTGCAAATACTCTT
GCAAAGAAGAAGCAAGTTTTCGTGCCTTACAGGGATTCTGTGTTGACTTGGTTGTTAAAAGATAGCCTTGGAGGA
AACTCTAAAACTATCATGATTGCCACCATTTCACCTGCTGATGTCAATTATGGAGAAACCCTAAGTACTCTTCGC
TATGCAAATAGAGCCAAAAACATCATCAACAAGCCTACCATTAATGAGGATGCCAACGTCAAACTTATCCGTGAG
CTGCGAGCTGAAATAGCCAGACTGAAAACGCTGCTTGCTCAAGGGAATCAGATTGCCCTCTTAGACTCCCCCACA
GACTACAAGGACGACGATGATAAGATGGAAAGCAACCAGAGCAACAACGGCGGCTCTGGCAACGCCGCTCTGAAC
AGAGGCGGCAGATACGTGCCCCCCCACCTGAGAGGAGGCGACGGCGGCGCCGCCGCCGCTGCATCTGCCGGCGGA
GATGACAGAAGAGGCGGAGCCGGAGGCGGCGGCTATAGACGGGGAGGCGGAAACAGCGGCGGCGGAGGCGGAGGC
GGCTACGACAGAGGCTACAACGACAACCGGGACGACCGGGACAACAGAGGCGGCAGCGGCGGATACGGCAGAGAT
CGAAACTACGAGGACAGAGGCTACAATGGCGGAGGCGGAGGCGGCGGCAACCGGGGCTACAACAACAACAGAGGA
GGCGGCGGCGGCGGCTACAACCGCCAGGACAGAGGCGATGGCGGATCTAGCAATTTCAGCAGAGGCGGCTACAAC
AACCGGGACGAGGGCAGCGACAACAGAGGCAGCGGAAGAAGCTACAACAATGACCGGAGAGATAATGGCGGAGAT
GGCTCCGGAGCGTGCCCGGTGCCGCTGCAGCTGCCGCCGCTGGAACGCCTGACCCTGGATGATAAAAAACCGCTG
AATACCCTGATCTCTGCTACTGGTCTGTGGATGAGTCGTACCGGAACCATTCATAAAATCAAACACCACGAGGTT
AGCCGTTCGAAAATCTATATTGAGATGGCGTGTGGCGATCATCTGGTTGTGAACAATAGCCGCTCTTCTCGTACA
GCACGTGCACTGCGTCACCACAAATATCGTAAAACCTGTAAACGTTGCCGTGTGTCCGATGAGGATCTGAACAAA
TTCCTGACAAAAGCCAATGAGGACCAAACAAGCGTGAAAGTGAAAGTCGTTAGCGCTCCTACCCGTACTAAAAAA
GCAATGCCGAAATCCGTTGCTCGTGCCCCTAAACCACTGGAAAACACTGAAGCAGCACAGGCACAGCCGTCTGGA
AGCAAATTCTCTCCGGCCATTCCTGTTTCTACCCAGGAGTCCGTTTCTGTTCCAGCAAGTGTGAGCACCAGCATT
AGCAGTATTAGCACCGGTGCCACCGCTAGCGCCCTGGTTAAAGGCAATACCAATCCGATTACAAGCATGTCTGCC
CCGGTTCAAGCATCAGCTCCAGCACTGACAAAATCCCAAACCGATCGTCTGGAGGTTCTGCTGAATCCGAAAGAC
GAAATCAGCCTGAATTCCGGCAAACCGTTTCGTGAACTGGAGAGCGAACTGCTGTCACGTCGTAAAAAAGACCTG

CAACAAATCTATGCCGAAGAACGTGAGAACTATCTGGGGAAACTGGAACGTGAAATCACCCGCTTTTTCGTGGAT
CGTGGCTTTCTGGAGATCAAATCCCCGATTCTGATTCCTCTGGAGTATATCGAGCGTATGGGCATCGACAATGAT
ACCGAACTGAGCAAACAAATTTTCCGTGTGGATAAAAACTTCTGTCTGCGCCCTATGCTAGCACCAAATCTGGCT
AACTATCTGCGCAAACTGGACCGTGCCCTGCCTGATCCTATCAAAATCTTCGAGATCGGCCCGTGTTATCGTAAA
GAGTCCGACGGTAAAGAACATCTGGAGGAGTTTACCATGCTGAACTTTTGCCAAATGGGTTCAGGTTGTACTCGT
GAGAACCTGGAAAGCATCATCACCGATTTTCTGAACCACCTGGGCATTGACTTCAAAATTGTGGGCGACAGCTGT
ATGGTGTTTGGCGACACCCTGGATGTCATGCACGGCGACCTGGAACTGTCTAGTGCCGTTGTGGGCCCAATCCCG
CTGGATCGTGAGTGGGGTATCGACAAACCTTGGATCGGTGCGGGTTTTGGTCTGGAGCGTCTGCTGAAAGTAAAA
CACGACTTCAAGAACATCAAACGTGCTGCACGTTCCGAGTCCTATTACAATGGTATTTCTACTAACCTGTAA
(SEQ ID NO: 667)

Protein:
MASVKVAVRVRPMNRREKDLEAKFIIQMEKSKTTITNLKIPEGGTGDSGRERTKTFTYDFSFYSADTKSPDYVSQ
EMVFKTLGTDVVKSAFEGYNACVFAYGQTGSGKSYTMMGNSGDSGLIPRICEGLFSRINETTRWDEASFRTEVSY
LEIYNERVRDLLRRKSSKTFNLRVREHPKEGPYVEDLSKHLVQNYGDVEELMDAGNINRTTAATGMNDVSSRSHA
IFTIKFTQAKFDSEMPCETVSKIHLVDLAGSERADATGATGVRLKEGGNINKSLVTLGNVISALADLSQDAANTL
AKKKQVFVPYRDSVLTWLLKDSLGGNSKTIMIATISPADVNYGETLSTLRYANRAKNIINKPTINEDANVKLIRE
LRAEIARLKTLLAQGNQIALLDSPTDYKDDDDKMESNQSNNGGSGNAALNRGGRYVPPHLRGGDGGAAAAASAGG
DDRRGGAGGGGYRRGGGNSGGGGGGGYDRGYNDNRDDRDNRGGSGGYGRDRNYEDRGYNGGGGGGGNRGYNNNRG
GGGGGYNRQDRGDGGSSNFSRGGYNNRDEGSDNRGSGRSYNNDRRDNGGDGSGACPVPLQLPPLERLTLDDKKPL
NTLISATGLWMSRTGTIHKIKHHEVSRSKIYIEMACGDHLVVNNSRSSRTARALRHHKYRKTCKRCRVSDEDLNK
FLTKANEDQTSVKVKVVSAPTRTKKAMPKSVARAPKPLENTEAAQAQPSGSKFSPAIPVSTQESVSVPASVSTSI
SSISTGATASALVKGNTNPITSMSAPVQASAPALTKSQTDRLEVLLNPKDEISLNSGKPFRELESELLSRRKKDL
QQIYAEERENYLGKLEREITRFFVDRGFLEIKSPILIPLEYIERMGIDNDTELSKQIFRVDKNFCLRPMLAPNLA
NYLRKLDRALPDPIKIFEIGPCYRKESDGKEHLEEFTMLNFCQMGSGCTRENLESIITDFLNHLGIDFKIVGDSC
MVFGDTLDVMHGDLELSSAVVGPIPLDREWGIDKPWIGAGFGLERLLKVKHDFKNIKRAARSESYYNGISTNL*
(SEQ ID NO: 668)

KIF16B::1xLAF-1::MCP
DNA:
ATGGCATCGGTCAAGGTGGCCGTGAGGGTCCGGCCCATGAATCGCAGGGAAAAGGACTTGGAGGCCAAGTTCATT
ATTCAGATGGAGAAAAGCAAAACGACAATCACAAACTTAAAGATACCAGAAGGAGGCACTGGGGACTCAGGAAGA
GAACGGACCAAGACCTTCACCTATGACTTTTCTTTTTATTCTGCTGATACAAAAAGCCCAGATTACGTTTCACAA
GAAATGGTTTTCAAAACCCTCGGCACAGATGTCGTGAAGTCTGCATTTGAAGGTTATAATGCTTGTGTCTTTGCA
TATGGGCAAACTGGATCTGGAAAGTCATACACTATGATGGGAAATTCTGGAGATTCTGGCTTAATACCTCGGATC
TGTGAAGGACTCTTCAGTCGGATAAATGAAACCACCAGATGGGATGAAGCTTCTTTTCGAACTGAAGTCAGCTAC
TTAGAAATTTATAACGAACGTGTGAGAGATCTACTTCGGCGGAAGTCATCTAAAACCTTCAATTTGAGAGTCCGT
GAGCATCCCAAAGAAGGCCCTTATGTTGAGGATTTATCCAAACATTTAGTACAGAATTATGGTGACGTAGAAGAA
CTTATGGATGCGGGCAATATCAACCGGACCACCGCAGCGACTGGGATGAACGACGTCAGTAGCAGGTCTCATGCC
ATCTTCACCATCAAGTTCACTCAGGCTAAATTTGATTCTGAAATGCCATGTGAAACCGTCAGTAAGATCCACTTG
GTTGATCTTGCCGGAAGTGAGCGTGCAGATGCCACCGGAGCCACCGGGGTTAGGCTAAAGGAAGGGGGAAATATT
AACAAGTCCCTCGTGACTCTGGGGAACGTCATTTCTGCCTTAGCTGATTTATCTCAGGATGCTGCAAATACTCTT
GCAAAGAAGAAGCAAGTTTTCGTGCCTTACAGGGATTCTGTGTTGACTTGGTTGTTAAAAGATAGCCTTGGAGGA
AACTCTAAAACTATCATGATTGCCACCATTTCACCTGCTGATGTCAATTATGGAGAAACCCTAAGTACTCTTCGC
TATGCAAATAGAGCCAAAAACATCATCAACAAGCCTACCATTAATGAGGATGCCAACGTCAAACTTATCCGTGAG
CTGCGAGCTGAAATAGCCAGACTGAAAACGCTGCTTGCTCAAGGGAATCAGATTGCCCTCTTAGACTCCCCCACA
GACTACAAGGACGACGATGATAAGATGGAAAGCAACCAGAGCAACAACGGCGGCTCTGGCAACGCCGCTCTGAAC
AGAGGCGGCAGATACGTGCCCCCCCACCTGAGAGGAGGCGACGGCGGCGCCGCCGCCGCTGCATCTGCCGGCGGA
GATGACAGAAGAGGCGGAGCCGGAGGCGGCGGCTATAGACGGGGAGGCGGAAACAGCGGCGGCGGAGGCGGAGGC
GGCTACGACAGAGGCTACAACGACAACCGGGACGACCGGGACAACAGAGGCGGCAGCGGCGGATACGGCAGAGAT
CGAAACTACGAGGACAGAGGCTACAATGGCGGAGGCGGAGGCGGCGGCAACCGGGGCTACAACAACAACAGAGGA
GGCGGCGGCGGCGGCTACAACCGCCAGGACAGAGGCGATGGCGGATCTAGCAATTTCAGCAGAGGCGGCTACAAC
AACCGGGACGAGGGCAGCGACAACAGAGGCAGCGGAAGAAGCTACAACAATGACCGGAGAGATAATGGCGGAGAT
GGCTCCGGATATCCCTATGATGTGCCGGATTATGCTGGAGCACCAGGAAGTGCTGGTTCTGCTGCTGGTAGTGGA
GCTTCTAACTTTACTCAGTTCGTTCTCGTCGACAATGGCGGAACTGGCGACGTGACTGTCGCCCCAAGCAACTTC
GCTAACGGGATCGCTGAATGGATCAGCTCTAACTCGCGTTCACAGGCTTACAAAGTAACCTGTAGCGTTCGTCAG
AGCTCTGCGCAGAATCGCAAATACACCATCAAAGTCGAGGTGCCTAAAGGCGCCTGGCGTTCGTACTTAAATATG

GAACTAACCATTCCAATTTTCGCCACGAATTCCGACTGCGAGCTTATTGTTAAGGCAATGCAAGGTCTCCTAAAA
GATGGAAACCCGATTCCCTCAGCAATCGCAGCAAACTCCGGCATCTACTAA (SEQ ID NO: 669)

Protein:
MASVKVAVRVRPMNRREKDLEAKFIIQMEKSKTTITNLKIPEGGTGDSGRERTKTFTYDFSFYSADTKSPDYVSQ
EMVFKTLGTDVVKSAFEGYNACVFAYGQTGSGKSYTMMGNSGDSGLIPRICEGLFSRINETTRWDEASFRTEVSY
LEIYNERVRDLLRRKSSKTFNLRVREHPKEGPYVEDLSKHLVQNYGDVEELMDAGNINRTTAATGMNDVSSRSHA
IFTIKFTQAKFDSEMPCETVSKIHLVDLAGSERADATGATGVRLKEGGNINKSLVTLGNVISALADLSQDAANTL
AKKKQVFVPYRDSVLTWLLKDSLGGNSKTIMIATISPADVNYGETLSTLRYANRAKNIINKPTINEDANVKLIRE
LRAEIARLKTLLAQGNQIALLDSPTDYKDDDDKMESNQSNNGGSGNAALNRGGRYVPPHLRGGDGGAAAAASAGG
DDRRGGAGGGGYRRGGGNSGGGGGGGYDRGYNDNRDDRDNRGGSGGYGRDRNYEDRGYNGGGGGGGNRGYNNNRG
GGGGGYNRQDRGDGGSSNFSRGGYNNRDEGSDNRGSGRSYNNDRRDNGGDGSGYPYDVPDYAGAPGSAGSAAGSG
ASNFTQFVLVDNGGTGDVTVAPSNFANGIAEWISSNSRSQAYKVTCSVRQSSAQNRKYTIKVEVPKGAWRSYLNM
ELTIPIFATNSDCELIVKAMQGLLKDGNPIPSAIAANSGIY* (SEQ ID NO: 670)

KIF16B::1xLAF-1::2xPCP
DNA:
ATGGCATCGGTCAAGGTGGCCGTGAGGGTCCGGCCCATGAATCGCAGGGAAAAGGACTTGGAGGCCAAGTTCATT
ATTCAGATGGAGAAAAGCAAAACGACAATCACAAACTTAAAGATACCAGAAGGAGGCACTGGGGACTCAGGAAGA
GAACGGACCAAGACCTTCACCTATGACTTTTCTTTTTATTCTGCTGATACAAAAAGCCCAGATTACGTTTCACAA
GAAATGGTTTTCAAAACCCTCGGCACAGATGTCGTGAAGTCTGCATTTGAAGGTTATAATGCTTGTGTCTTTGCA
TATGGGCAAACTGGATCTGGAAAGTCATACACTATGATGGGAAATTCTGGAGATTCTGGCTTAATACCTCGGATC
TGTGAAGGACTCTTCAGTCGGATAAATGAAACCACCAGATGGGATGAAGCTTCTTTTCGAACTGAAGTCAGCTAC
TTAGAAATTTATAACGAACGTGTGAGAGATCTACTTCGGCGGAAGTCATCTAAAACCTTCAATTTGAGAGTCCGT
GAGCATCCCAAAGAAGGCCCTTATGTTGAGGATTTATCCAAACATTTAGTACAGAATTATGGTGACGTAGAAGAA
CTTATGGATGCGGGCAATATCAACCGGACCACCGCAGCGACTGGGATGAACGACGTCAGTAGCAGGTCTCATGCC
ATCTTCACCATCAAGTTCACTCAGGCTAAATTTGATTCTGAAATGCCATGTGAAACCGTCAGTAAGATCCACTTG
GTTGATCTTGCCGGAAGTGAGCGTGCAGATGCCACCGGAGCCACCGGGGTTAGGCTAAAGGAAGGGGGAAATATT
AACAAGTCCCTCGTGACTCTGGGGAACGTCATTTCTGCCTTAGCTGATTTATCTCAGGATGCTGCAAATACTCTT
GCAAAGAAGAAGCAAGTTTTCGTGCCTTACAGGGATTCTGTGTTGACTTGGTTGTTAAAAGATAGCCTTGGAGGA
AACTCTAAAACTATCATGATTGCCACCATTTCACCTGCTGATGTCAATTATGGAGAAACCCTAAGTACTCTTCGC
TATGCAAATAGAGCCAAAAACATCATCAACAAGCCTACCATTAATGAGGATGCCAACGTCAAACTTATCCGTGAG
CTGCGAGCTGAAATAGCCAGACTGAAAACGCTGCTTGCTCAAGGGAATCAGATTGCCCTCTTAGACTCCCCCACA
GACTACAAGGACGACGATGATAAGATGGAAAGCAACCAGAGCAACAACGGCGGCTCTGGCAACGCCGCTCTGAAC
AGAGGCGGCAGATACGTGCCCCCCCACCTGAGAGGAGGCGACGGCGGCGCCGCCGCCGCTGCATCTGCCGGCGGA
GATGACAGAAGAGGCGGAGCCGGAGGCGGCGGCTATAGACGGGGAGGCGGAAACAGCGGCGGCGGAGGCGGAGGC
GGCTACGACAGAGGCTACAACGACAACCGGGACGACCGGGACAACAGAGGCGGCAGCGGCGGATACGGCAGAGAT
CGAAACTACGAGGACAGAGGCTACAATGGCGGAGGCGGAGGCGGCGGCAACCGGGGCTACAACAACAACAGAGGA
GGCGGCGGCGGCGGCTACAACCGCCAGGACAGAGGCGATGGCGGATCTAGCAATTTCAGCAGAGGCGGCTACAAC
AACCGGGACGAGGGCAGCGACAACAGAGGCAGCGGAAGAAGCTACAACAATGACCGGAGAGATAATGGCGGAGAT
GGCTCCGGCGAGCAGAAGCTGATCTCAGAGGAGGACCTGATCGAAGGCCGCCATATGCTAGCCTCCAAAACCATC
GTTCTTTCGGTCGGCGAGGCTACTCGCACTCTGACTGAGATCCAGTCCACCGCAGACCGTCAGATCTTCGAAGAG
AAGGTCGGGCCTCTGGTGGGTCGGCTGCGCCTCACGGCTTCGCTCCGTCAAAACGGAGCCAAGACCGCGTATCGC
GTCAACCTAAAACTGGATCAGGCGGACGTCGTTGATTCCGGACTTCCGAAAGTGCGCTACACTCAGGTATGGTCG
CACGACGTGACAATCGTTGCGAATAGCACCGAGGCCTCGCGCAAATCGTTGTACGATTTGACCAAGTCCCTCGTC
GCGACCTCGCAGGTCGAAGATCTTGTCGTCAACCTTGTGCCGCTGGGCCGTGCGGATCCGCTAGCCTCCAAAACC
ATCGTTCTTTCGGTCGGCGAGGCTACTCGCACTCTGACTGAGATCCAGTCCACCGCAGACCGTCAGATCTTCGAA
GAGAAGGTCGGGCCTCTGGTGGGTCGGCTGCGCCTCACGGCTTCGCTCCGTCAAAACGGAGCCAAGACCGCGTAT
CGCGTCAACCTAAAACTGGATCAGGCGGACGTCGTTGATTCCGGACTTCCGAAAGTGCGCTACACTCAGGTATGG
TCGCACGACGTGACAATCGTTGCGAATAGCACCGAGGCCTCGCGCAAATCGTTGTACGATTTGACCAAGTCCCTC
GTCGCGACCTCGCAGGTCGAAGATCTTGTCGTCAACCTTGTGCCGCTGGGCCGTCCACCGGTCGCCACCTAA
(SEQ ID NO: 671)

Protein:
MASVKVAVRVRPMNRREKDLEAKFIIQMEKSKTTITNLKIPEGGTGDSGRERTKTFTYDFSFYSADTKSPDYVSQ
EMVFKTLGTDVVKSAFEGYNACVFAYGQTGSGKSYTMMGNSGDSGLIPRICEGLFSRINETTRWDEASFRTEVSY
LEIYNERVRDLLRRKSSKTFNLRVREHPKEGPYVEDLSKHLVQNYGDVEELMDAGNINRTTAATGMNDVSSRSHA

IFTIKFTQAKFDSEMPCETVSKIHLVDLAGSERADATGATGVRLKEGGNINKSLVTLGNVISALADLSQDAANTL
AKKKQVFVPYRDSVLTWLLKDSLGGNSKTIMIATISPADVNYGETLSTLRYANRAKNIINKPTINEDANVKLIRE
LRAEIARLKTLLAQGNQIALLDSPTDYKDDDDKMESNQSNNGGSGNAALNRGGRYVPPHLRGGDGGAAAAASAGG
DDRRGGAGGGGYRRGGGNSGGGGGGGYDRGYNDNRDDRDNRGGSGGYGRDRNYEDRGYNGGGGGGGNRGYNNNRG
GGGGGYNRQDRGDGGSSNFSRGGYNNRDEGSDNRGSGRSYNNDRRDNGGDGSGEQKLISEEDLIEGRHMLASKTI
VLSVGEATRTLTEIQSTADRQIFEEKVGPLVGRLRLTASLRQNGAKTAYRVNLKLDQADVVDSGLPKVRYTQVWS
HDVTIVANSTEASRKSLYDLTKSLVATSQVEDLVVNLVPLGRADPLASKTIVLSVGEATRTLTEIQSTADRQIFE
EKVGPLVGRLRLTASLRQNGAKTAYRVNLKLDQADVVDSGLPKVRYTQVWSHDVTIVANSTEASRKSLYDLTKSL
VATSQVEDLVVNLVPLGRPPVAT* (SEQ ID NO: 672)

KIF16B::2xLAF-1::2xPCP
DNA:
ATGGCATCGGTCAAGGTGGCCGTGAGGGTCCGGCCCATGAATCGCAGGGAAAAGGACTTGGAGGCCAAGTTCATT
ATTCAGATGGAGAAAAGCAAAACGACAATCACAAACTTAAAGATACCAGAAGGAGGCACTGGGGACTCAGGAAGA
GAACGGACCAAGACCTTCACCTATGACTTTTCTTTTTATTCTGCTGATACAAAAAGCCCAGATTACGTTTCACAA
GAAATGGTTTTCAAAACCCTCGGCACAGATGTCGTGAAGTCTGCATTTGAAGGTTATAATGCTTGTGTCTTTGCA
TATGGGCAAACTGGATCTGGAAAGTCATACACTATGATGGGAAATTCTGGAGATTCTGGCTTAATACCTCGGATC
TGTGAAGGACTCTTCAGTCGGATAAATGAAACCACCAGATGGGATGAAGCTTCTTTTCGAACTGAAGTCAGCTAC
TTAGAAATTTATAACGAACGTGTGAGAGATCTACTTCGGCGGAAGTCATCTAAAACCTTCAATTTGAGAGTCCGT
GAGCATCCCAAAGAAGGCCCTTATGTTGAGGATTTATCCAAACATTTAGTACAGAATTATGGTGACGTAGAAGAA
CTTATGGATGCGGGCAATATCAACCGGACCACCGCAGCGACTGGGATGAACGACGTCAGTAGCAGGTCTCATGCC
ATCTTCACCATCAAGTTCACTCAGGCTAAATTTGATTCTGAAATGCCATGTGAAACCGTCAGTAAGATCCACTTG
GTTGATCTTGCCGGAAGTGAGCGTGCAGATGCCACCGGAGCCACCGGGGTTAGGCTAAAGGAAGGGGGAAATATT
AACAAGTCCCTCGTGACTCTGGGGAACGTCATTTCTGCCTTAGCTGATTTATCTCAGGATGCTGCAAATACTCTT
GCAAAGAAGAAGCAAGTTTTCGTGCCTTACAGGGATTCTGTGTTGACTTGGTTGTTAAAAGATAGCCTTGGAGGA
AACTCTAAAACTATCATGATTGCCACCATTTCACCTGCTGATGTCAATTATGGAGAAACCCTAAGTACTCTTCGC
TATGCAAATAGAGCCAAAAACATCATCAACAAGCCTACCATTAATGAGGATGCCAACGTCAAACTTATCCGTGAG
CTGCGAGCTGAAATAGCCAGACTGAAAACGCTGCTTGCTCAAGGGAATCAGATTGCCCTCTTAGACTCCCCCACA
GACTACAAGGACGACGATGATAAGATGGAAAGCAACCAGAGCAACAACGGCGGCTCTGGCAACGCCGCTCTGAAC
AGAGGCGGCAGATACGTGCCCCCCCACCTGAGAGGAGGCGACGGCGGCGCCGCCGCCGCTGCATCTGCCGGCGGA
GATGACAGAAGAGGCGGAGCCGGAGGCGGCGGCTATAGACGGGGAGGCGGAAACAGCGGCGGCGGAGGCGGAGGC
GGCTACGACAGAGGCTACAACGACAACCGGGACGACCGGGACAACAGAGGCGGCAGCGGCGGATACGGCAGAGAT
CGAAACTACGAGGACAGAGGCTACAATGGCGGAGGCGGAGGCGGCGGCAACCGGGGCTACAACAACAACAGAGGA
GGCGGCGGCGGCGGCTACAACCGCCAGGACAGAGGCGATGGCGGATCTAGCAATTTCAGCAGAGGCGGCTACAAC
AACCGGGACGAGGGCAGCGACAACAGAGGCAGCGGAAGAAGCTACAACAATGACCGGAGAGATAATGGCGGAGAT
GGCTCCGGCGGAATGGAAAGCAACCAGAGCAACAACGGCGGCTCTGGCAACGCCGCTCTGAACAGAGGCGGCAGA
TACGTGCCCCCCCACCTGAGAGGAGGCGACGGCGGCGCCGCCGCCGCTGCATCTGCCGGCGGAGATGACAGAAGA
GGCGGAGCCGGAGGCGGCGGCTATAGACGGGGAGGCGGAAACAGCGGCGGCGGAGGCGGAGGCGGCTACGACAGA
GGCTACAACGACAACCGGGACGACCGGGACAACAGAGGCGGCAGCGGCGGATACGGCAGAGATCGAAACTACGAG
GACAGAGGCTACAATGGCGGAGGCGGAGGCGGCGGCAACCGGGGCTACAACAACAACAGAGGAGGCGGCGGCGGC
GGCTACAACCGCCAGGACAGAGGCGATGGCGGATCTAGCAATTTCAGCAGAGGCGGCTACAACAACCGGGACGAG
GGCAGCGACAACAGAGGCAGCGGAAGAAGCTACAACAATGACCGGAGAGATAATGGCGGAGATGGCTCCGGCGAG
CAGAAGCTGATCTCAGAGGAGGACCTGATCGAAGGCCGCCATATGCTAGCCTCCAAAACCATCGTTCTTTCGGTC
GGCGAGGCTACTCGCACTCTGACTGAGATCCAGTCCACCGCAGACCGTCAGATCTTCGAAGAGAAGGTCGGGCCT
CTGGTGGGTCGGCTGCGCCTCACGGCTTCGCTCCGTCAAAACGGAGCCAAGACCGCGTATCGCGTCAACCTAAAA
CTGGATCAGGCGGACGTCGTTGATTCCGGACTTCCGAAAGTGCGCTACACTCAGGTATGGTCGCACGACGTGACA
ATCGTTGCGAATAGCACCGAGGCCTCGCGCAAATCGTTGTACGATTTGACCAAGTCCCTCGTCGCGACCTCGCAG
GTCGAAGATCTTGTCGTCAACCTTGTGCCGCTGGGCCGTGCGGATCCGCTAGCCTCCAAAACCATCGTTCTTTCG
GTCGGCGAGGCTACTCGCACTCTGACTGAGATCCAGTCCACCGCAGACCGTCAGATCTTCGAAGAGAAGGTCGGG
CCTCTGGTGGGTCGGCTGCGCCTCACGGCTTCGCTCCGTCAAAACGGAGCCAAGACCGCGTATCGCGTCAACCTA
AAACTGGATCAGGCGGACGTCGTTGATTCCGGACTTCCGAAAGTGCGCTACACTCAGGTATGGTCGCACGACGTG
ACAATCGTTGCGAATAGCACCGAGGCCTCGCGCAAATCGTTGTACGATTTGACCAAGTCCCTCGTCGCGACCTCG
CAGGTCGAAGATCTTGTCGTCAACCTTGTGCCGCTGGGCCGTCCACCGGTCGCCACCTAA (SEQ ID NO:
673)

Protein:

MASVKVAVRVRPMNRREKDLEAKFIIQMEKSKTTITNLKIPEGGTGDSGRERTKTFTYDFSFYSADTKSPDYVSQ
EMVFKTLGTDVVKSAFEGYNACVFAYGQTGSGKSYTMMGNSGDSGLIPRICEGLFSRINETTRWDEASFRTEVSY
LEIYNERVRDLLRRKSSKTFNLRVREHPKEGPYVEDLSKHLVQNYGDVEELMDAGNINRTTAATGMNDVSSRSHA
IFTIKFTQAKFDSEMPCETVSKIHLVDLAGSERADATGATGVRLKEGGNINKSLVTLGNVISALADLSQDAANTL
AKKKQVFVPYRDSVLTWLLKDSLGGNSKTIMIATISPADVNYGETLSTLRYANRAKNIINKPTINEDANVKLIRE
LRAEIARLKTLLAQGNQIALLDSPTDYKDDDDKMESNQSNNGGSGNAALNRGGRYVPPHLRGGDGGAAAAASAGG
DDRRGGAGGGGYRRGGGNSGGGGGGGYDRGYNDNRDDRDNRGGSGGYGRDRNYEDRGYNGGGGGGGNRGYNNNRG
GGGGGYNRQDRGDGGSSNFSRGGYNNRDEGSDNRGSGRSYNNDRRDNGGDGSGGMESNQSNNGGSGNAALNRGGR
YVPPHLRGGDGGAAAAASAGGDDRRGGAGGGGYRRGGGNSGGGGGGGYDRGYNDNRDDRDNRGGSGGYGRDRNYE
DRGYNGGGGGGGNRGYNNNRGGGGGGYNRQDRGDGGSSNFSRGGYNNRDEGSDNRGSGRSYNNDRRDNGGDGSGE
QKLISEEDLIEGRHMLASKTIVLSVGEATRTLTEIQSTADRQIFEEKVGPLVGRLRLTASLRQNGAKTAYRVNLK
LDQADVVDSGLPKVRYTQVWSHDVTIVANSTEASRKSLYDLTKSLVATSQVEDLVVNLVPLGRADPLASKTIVLS
VGEATRTLTEIQSTADRQIFEEKVGPLVGRLRLTASLRQNGAKTAYRVNLKLDQADVVDSGLPKVRYTQVWSHDV
TIVANSTEASRKSLYDLTKSLVATSQVEDLVVNLVPLGRPPVAT* (SEQ ID NO: 674)

KIF16B::2xLAF-1::PylRS(AF)
DNA:
ATGGCATCGGTCAAGGTGGCCGTGAGGGTCCGGCCCATGAATCGCAGGGAAAAGGACTTGGAGGCCAAGTTCATT
ATTCAGATGGAGAAAAGCAAAACGACAATCACAAACTTAAAGATACCAGAAGGAGGCACTGGGGACTCAGGAAGA
GAACGGACCAAGACCTTCACCTATGACTTTTCTTTTTATTCTGCTGATACAAAAAGCCCAGATTACGTTTCACAA
GAAATGGTTTTCAAAACCCTCGGCACAGATGTCGTGAAGTCTGCATTTGAAGGTTATAATGCTTGTGTCTTTGCA
TATGGGCAAACTGGATCTGGAAAGTCATACACTATGATGGGAAATTCTGGAGATTCTGGCTTAATACCTCGGATC
TGTGAAGGACTCTTCAGTCGGATAAATGAAACCACCAGATGGGATGAAGCTTCTTTTCGAACTGAAGTCAGCTAC
TTAGAAATTTATAACGAACGTGTGAGAGATCTACTTCGGCGGAAGTCATCTAAAACCTTCAATTTGAGAGTCCGT
GAGCATCCCAAAGAAGGCCCTTATGTTGAGGATTTATCCAAACATTTAGTACAGAATTATGGTGACGTAGAAGAA
CTTATGGATGCGGGCAATATCAACCGGACCACCGCAGCGACTGGGATGAACGACGTCAGTAGCAGGTCTCATGCC
ATCTTCACCATCAAGTTCACTCAGGCTAAATTTGATTCTGAAATGCCATGTGAAACCGTCAGTAAGATCCACTTG
GTTGATCTTGCCGGAAGTGAGCGTGCAGATGCCACCGGAGCCACCGGGGTTAGGCTAAAGGAAGGGGGAAATATT
AACAAGTCCCTCGTGACTCTGGGGAACGTCATTTCTGCCTTAGCTGATTTATCTCAGGATGCTGCAAATACTCTT
GCAAAGAAGAAGCAAGTTTTCGTGCCTTACAGGGATTCTGTGTTGACTTGGTTGTTAAAAGATAGCCTTGGAGGA
AACTCTAAAACTATCATGATTGCCACCATTTCACCTGCTGATGTCAATTATGGAGAAACCCTAAGTACTCTTCGC
TATGCAAATAGAGCCAAAAACATCATCAACAAGCCTACCATTAATGAGGATGCCAACGTCAAACTTATCCGTGAG
CTGCGAGCTGAAATAGCCAGACTGAAAACGCTGCTTGCTCAAGGGAATCAGATTGCCCTCTTAGACTCCCCCACA
GACTACAAGGACGACGATGATAAGATGGAAAGCAACCAGAGCAACAACGGCGGCTCTGGCAACGCCGCTCTGAAC
AGAGGCGGCAGATACGTGCCCCCCCACCTGAGAGGAGGCGACGGCGGCGCCGCCGCCGCTGCATCTGCCGGCGGA
GATGACAGAAGAGGCGGAGCCGGAGGCGGCGGCTATAGACGGGGAGGCGGAAACAGCGGCGGCGGAGGCGGAGGC
GGCTACGACAGAGGCTACAACGACAACCGGGACGACCGGGACAACAGAGGCGGCAGCGGCGGATACGGCAGAGAT
CGAAACTACGAGGACAGAGGCTACAATGGCGGAGGCGGAGGCGGCGGCAACCGGGGCTACAACAACAACAGAGGA
GGCGGCGGCGGCGGCTACAACCGCCAGGACAGAGGCGATGGCGGATCTAGCAATTTCAGCAGAGGCGGCTACAAC
AACCGGGACGAGGGCAGCGACAACAGAGGCAGCGGAAGAAGCTACAACAATGACCGGAGAGATAATGGCGGAGAT
GGCTCCGGCGGAATGGAAAGCAACCAGAGCAACAACGGCGGCTCTGGCAACGCCGCTCTGAACAGAGGCGGCAGA
TACGTGCCCCCCCACCTGAGAGGAGGCGACGGCGGCGCCGCCGCCGCTGCATCTGCCGGCGGAGATGACAGAAGA
GGCGGAGCCGGAGGCGGCGGCTATAGACGGGGAGGCGGAAACAGCGGCGGCGGAGGCGGAGGCGGCTACGACAGA
GGCTACAACGACAACCGGGACGACCGGGACAACAGAGGCGGCAGCGGCGGATACGGCAGAGATCGAAACTACGAG
GACAGAGGCTACAATGGCGGAGGCGGAGGCGGCGGCAACCGGGGCTACAACAACAACAGAGGAGGCGGCGGCGGC
GGCTACAACCGCCAGGACAGAGGCGATGGCGGATCTAGCAATTTCAGCAGAGGCGGCTACAACAACCGGGACGAG
GGCAGCGACAACAGAGGCAGCGGAAGAAGCTACAACAATGACCGGAGAGATAATGGCGGAGATGGCTCCGGAGCG
TGCCCGGTGCCGCTGCAGCTGCCGCCGCTGGAACGCCTGACCCTGGATGATAAAAAACCGCTGAATACCCTGATC
TCTGCTACTGGTCTGTGGATGAGTCGTACCGGAACCATTCATAAAATCAAACACCACGAGGTTAGCCGTTCGAAA
ATCTATATTGAGATGGCGTGTGGCGATCATCTGGTTGTGAACAATAGCCGCTCTTCTCGTACAGCACGTGCACTG
CGTCACCACAAATATCGTAAAACCTGTAAACGTTGCCGTGTGTCCGATGAGGATCTGAACAAATTCCTGACAAAA
GCCAATGAGGACCAAACAAGCGTGAAAGTGAAAGTCGTTAGCGCTCCTACCCGTACTAAAAAAGCAATGCCGAAA
TCCGTTGCTCGTGCCCCTAAACCACTGGAAAACACTGAAGCAGCACAGGCACAGCCGTCTGGAAGCAAATTCTCT
CCGGCCATTCCTGTTTCTACCCAGGAGTCCGTTTCTGTTCCAGCAAGTGTGAGCACCAGCATTAGCAGTATTAGC
ACCGGTGCCACCGCTAGCGCCCTGGTTAAAGGCAATACCAATCCGATTACAAGCATGTCTGCCCCGGTTCAAGCA
TCAGCTCCAGCACTGACAAAATCCCAAACCGATCGTCTGGAGGTTCTGCTGAATCCGAAAGACGAAATCAGCCTG
AATTCCGGCAAACCGTTTCGTGAACTGGAGAGCGAACTGCTGTCACGTCGTAAAAAAGACCTGCAACAAATCTAT

GCCGAAGAACGTGAGAACTATCTGGGGAAACTGGAACGTGAAATCACCCGCTTTTTCGTGGATCGTGGCTTTCTG
GAGATCAAATCCCCGATTCTGATTCCTCTGGAGTATATCGAGCGTATGGGCATCGACAATGATACCGAACTGAGC
AAACAAATTTTCCGTGTGGATAAAAACTTCTGTCTGCGCCCTATGCTAGCACCAAATCTGGCTAACTATCTGCGC
AAACTGGACCGTGCCCTGCCTGATCCTATCAAAATCTTCGAGATCGGCCCGTGTTATCGTAAAGAGTCCGACGGT
AAAGAACATCTGGAGGAGTTTACCATGCTGAACTTTTGCCAAATGGGTTCAGGTTGTACTCGTGAGAACCTGGAA
AGCATCATCACCGATTTTCTGAACCACCTGGGCATTGACTTCAAAATTGTGGGCGACAGCTGTATGGTGTTTGGC
GACACCCTGGATGTCATGCACGGCGACCTGGAACTGTCTAGTGCCGTTGTGGGCCCAATCCCGCTGGATCGTGAG
TGGGGTATCGACAAACCTTGGATCGGTGCGGGTTTTGGTCTGGAGCGTCTGCTGAAAGTAAAACACGACTTCAAG
AACATCAAACGTGCTGCACGTTCCGAGTCCTATTACAATGGTATTTCTACTAACCTGTAA (SEQ ID NO:
675)

Protein:
MASVKVAVRVRPMNRREKDLEAKFIIQMEKSKTTITNLKIPEGGTGDSGRERTKTFTYDFSFYSADTKSPDYVSQ
EMVFKTLGTDVVKSAFEGYNACVFAYGQTGSGKSYTMMGNSGDSGLIPRICEGLFSRINETTRWDEASFRTEVSY
LEIYNERVRDLLRRKSSKTFNLRVREHPKEGPYVEDLSKHLVQNYGDVEELMDAGNINRTTAATGMNDVSSRSHA
IFTIKFTQAKFDSEMPCETVSKIHLVDLAGSERADATGATGVRLKEGGNINKSLVTLGNVISALADLSQDAANTL
AKKKQVFVPYRDSVLTWLLKDSLGGNSKTIMIATISPADVNYGETLSTLRYANRAKNIINKPTINEDANVKLIRE
LRAEIARLKTLLAQGNQIALLDSPTDYKDDDDKMESNQSNNGGSGNAALNRGGRYVPPHLRGGDGGAAAAASAGG
DDRRGGAGGGGYRRGGGNSGGGGGGGYDRGYNDNRDDRDNRGGSGGYGRDRNYEDRGYNGGGGGGGNRGYNNNRG
GGGGGYNRQDRGDGGSSNFSRGGYNNRDEGSDNRGSGRSYNNDRRDNGGDGSGGMESNQSNNGGSGNAALNRGGR
YVPPHLRGGDGGAAAAASAGGDDRRGGAGGGGYRRGGGNSGGGGGGGYDRGYNDNRDDRDNRGGSGGYGRDRNYE
DRGYNGGGGGGGNRGYNNNRGGGGGGYNRQDRGDGGSSNFSRGGYNNRDEGSDNRGSGRSYNNDRRDNGGDGSGA
CPVPLQLPPLERLTLDDKKPLNTLISATGLWMSRTGTIHKIKHHEVSRSKIYIEMACGDHLVVNNSRSSRTARAL
RHHKYRKTCKRCRVSDEDLNKFLTKANEDQTSVKVKVVSAPTRTKKAMPKSVARAPKPLENTEAAQAQPSGSKFS
PAIPVSTQESVSVPASVSTSISSISTGATASALVKGNTNPITSMSAPVQASAPALTKSQTDRLEVLLNPKDEISL
NSGKPFRELESELLSRRKKDLQQIYAEERENYLGKLEREITRFFVDRGFLEIKSPILIPLEYIERMGIDNDTELS
KQIFRVDKNFCLRPMLAPNLANYLRKLDRALPDPIKIFEIGPCYRKESDGKEHLEEFTMLNFCQMGSGCTRENLE
SIITDFLNHLGIDFKIVGDSCMVFGDTLDVMHGDLELSSAVVGPIPLDREWGIDKPWIGAGFGLERLLKVKHDFK
NIKRAARSESYYNGISTNL* (SEQ ID NO: 676)

KIF16B::2xLAF-1::MCP
DNA:
ATGGCATCGGTCAAGGTGGCCGTGAGGGTCCGGCCCATGAATCGCAGGGAAAAGGACTTGGAGGCCAAGTTCATT
ATTCAGATGGAGAAAAGCAAAACGACAATCACAAACTTAAAGATACCAGAAGGAGGCACTGGGGACTCAGGAAGA
GAACGGACCAAGACCTTCACCTATGACTTTTCTTTTTATTCTGCTGATACAAAAAGCCCAGATTACGTTTCACAA
GAAATGGTTTTCAAAACCCTCGGCACAGATGTCGTGAAGTCTGCATTTGAAGGTTATAATGCTTGTGTCTTTGCA
TATGGGCAAACTGGATCTGGAAAGTCATACACTATGATGGGAAATTCTGGAGATTCTGGCTTAATACCTCGGATC
TGTGAAGGACTCTTCAGTCGGATAAATGAAACCACCAGATGGGATGAAGCTTCTTTTCGAACTGAAGTCAGCTAC
TTAGAAATTTATAACGAACGTGTGAGAGATCTACTTCGGCGGAAGTCATCTAAAACCTTCAATTTGAGAGTCCGT
GAGCATCCCAAAGAAGGCCCTTATGTTGAGGATTTATCCAAACATTTAGTACAGAATTATGGTGACGTAGAAGAA
CTTATGGATGCGGGCAATATCAACCGGACCACCGCAGCGACTGGGATGAACGACGTCAGTAGCAGGTCTCATGCC
ATCTTCACCATCAAGTTCACTCAGGCTAAATTTGATTCTGAAATGCCATGTGAAACCGTCAGTAAGATCCACTTG
GTTGATCTTGCCGGAAGTGAGCGTGCAGATGCCACCGGAGCCACCGGGGTTAGGCTAAAGGAAGGGGGAAATATT
AACAAGTCCCTCGTGACTCTGGGGAACGTCATTTCTGCCTTAGCTGATTTATCTCAGGATGCTGCAAATACTCTT
GCAAAGAAGAAGCAAGTTTTCGTGCCTTACAGGGATTCTGTGTTGACTTGGTTGTTAAAAGATAGCCTTGGAGGA
AACTCTAAAACTATCATGATTGCCACCATTTCACCTGCTGATGTCAATTATGGAGAAACCCTAAGTACTCTTCGC
TATGCAAATAGAGCCAAAAACATCATCAACAAGCCTACCATTAATGAGGATGCCAACGTCAAACTTATCCGTGAG
CTGCGAGCTGAAATAGCCAGACTGAAAACGCTGCTTGCTCAAGGGAATCAGATTGCCCTCTTAGACTCCCCCACA
GACTACAAGGACGACGATGATAAGATGGAAAGCAACCAGAGCAACAACGGCGGCTCTGGCAACGCCGCTCTGAAC
AGAGGCGGCAGATACGTGCCCCCCCACCTGAGAGGAGGCGACGGCGGCGCCGCCGCCGCTGCATCTGCCGGCGGA
GATGACAGAAGAGGCGGAGCCGGAGGCGGCGGCTATAGACGGGGAGGCGGAAACAGCGGCGGCGGAGGCGGAGGC
GGCTACGACAGAGGCTACAACGACAACCGGGACGACCGGGACAACAGAGGCGGCAGCGGCGGATACGGCAGAGAT
CGAAACTACGAGGACAGAGGCTACAATGGCGGAGGCGGAGGCGGCGGCAACCGGGGCTACAACAACAACAGAGGA
GGCGGCGGCGGCGGCTACAACCGCCAGGACAGAGGCGATGGCGGATCTAGCAATTTCAGCAGAGGCGGCTACAAC
AACCGGGACGAGGGCAGCGACAACAGAGGCAGCGGAAGAAGCTACAACAATGACCGGAGAGATAATGGCGGAGAT
GGCTCCGGCGGAATGGAAAGCAACCAGAGCAACAACGGCGGCTCTGGCAACGCCGCTCTGAACAGAGGCGGCAGA
TACGTGCCCCCCCACCTGAGAGGAGGCGACGGCGGCGCCGCCGCCGCTGCATCTGCCGGCGGAGATGACAGAAGA

GGCGGAGCCGGAGGCGGCGGCTATAGACGGGGAGGCGGAAACAGCGGCGGCGGAGGCGGAGGCGGCTACGACAGA
GGCTACAACGACAACCGGGACGACCGGGACAACAGAGGCGGCAGCGGCGGATACGGCAGAGATCGAAACTACGAG
GACAGAGGCTACAATGGCGGAGGCGGAGGCGGCGGCAACCGGGGCTACAACAACAACAGAGGAGGCGGCGGCGGC
GGCTACAACCGCCAGGACAGAGGCGATGGCGGATCTAGCAATTTCAGCAGAGGCGGCTACAACAACCGGGACGAG
GGCAGCGACAACAGAGGCAGCGGAAGAAGCTACAACAATGACCGGAGAGATAATGGCGGAGATGGCTCCGGATAT
CCCTATGATGTGCCGGATTATGCTGGAGCACCAGGAAGTGCTGGTTCTGCTGCTGGTAGTGGAGCTTCTAACTTT
ACTCAGTTCGTTCTCGTCGACAATGGCGGAACTGGCGACGTGACTGTCGCCCCAAGCAACTTCGCTAACGGGATC
GCTGAATGGATCAGCTCTAACTCGCGTTCACAGGCTTACAAAGTAACCTGTAGCGTTCGTCAGAGCTCTGCGCAG
AATCGCAAATACACCATCAAAGTCGAGGTGCCTAAAGGCGCCTGGCGTTCGTACTTAAATATGGAACTAACCATT
CCAATTTTCGCCACGAATTCCGACTGCGAGCTTATTGTTAAGGCAATGCAAGGTCTCCTAAAAGATGGAAACCCG
ATTCCCTCAGCAATCGCAGCAAACTCCGGCATCTACTAA (SEQ ID NO: 677)

Protein:
MASVKVAVRVRPMNRREKDLEAKFIIQMEKSKTTITNLKIPEGGTGDSGRERTKTFTYDFSFYSADTKSPDYVSQ
EMVFKTLGTDVVKSAFEGYNACVFAYGQTGSGKSYTMMGNSGDSGLIPRICEGLFSRINETTRWDEASFRTEVSY
LEIYNERVRDLLRRKSSKTFNLRVREHPKEGPYVEDLSKHLVQNYGDVEELMDAGNINRTTAATGMNDVSSRSHA
IFTIKFTQAKFDSEMPCETVSKIHLVDLAGSERADATGATGVRLKEGGNINKSLVTLGNVISALADLSQDAANTL
AKKKQVFVPYRDSVLTWLLKDSLGGNSKTIMIATISPADVNYGETLSTLRYANRAKNIINKPTINEDANVKLIRE
LRAEIARLKTLLAQGNQIALLDSPTDYKDDDDKMESNQSNNGGSGNAALNRGGRYVPPHLRGGDGGAAAAASAGG
DDRRGGAGGGGYRRGGGNSGGGGGGGYDRGYNDNRDDRDNRGGSGGYGRDRNYEDRGYNGGGGGGGNRGYNNNRG
GGGGGYNRQDRGDGGSSNFSRGGYNNRDEGSDNRGSGRSYNNDRRDNGGDGSGGMESNQSNNGGSGNAALNRGGR
YVPPHLRGGDGGAAAAASAGGDDRRGGAGGGGYRRGGGNSGGGGGGGYDRGYNDNRDDRDNRGGSGGYGRDRNYE
DRGYNGGGGGGGNRGYNNNRGGGGGGYNRQDRGDGGSSNFSRGGYNNRDEGSDNRGSGRSYNNDRRDNGGDGSGY
PYDVPDYAGAPGSAGSAAGSGASNFTQFVLVDNGGTGDVTVAPSNFANGIAEWISSNSRSQAYKVTCSVRQSSAQ
NRKYTIKVEVPKGAWRSYLNMELTIPIFATNSDCELIVKAMQGLLKDGNPIPSAIAANSGIY* (SEQ ID NO:
678)

3.    Epitope tags:

VSV-G: Vesicular stomatitis virus glycoprotein epitope tag

DNA:

TATACAGATATTGAAATGAACAGATTGGGAAAG (SEQ ID NO:679)

Protein:

YTDIEMNRLGK (SEQ ID NO:680)

HA: Human influence hemagglutinin epitope tag

DNA:

TACCCCTACGACGTGCCCGACTACGCC (SEQ ID NO: 681)

Protein:

YPYDVPDYA (SEQ ID NO: 682)

Myc: Human c-Myc proto-oncogene epitope tag

DNA:

GAGCAGAAGCTGATCTCAGAGGAGGACCTG (SEQ ID NO: 683)
Protein:
EQKLISEEDL (SEQ ID NO: 684)

The invention also relates to the following particular embodiments:
E1: An assembler fusion protein (AFP) comprising:
   (a) at least one first polypeptide segment acting as assembler (AP) that is selected from:
      (a1) a polypeptide segment derived from an intracellular targeting polypeptide (IC-TP segment), wherein said intracellular targeting polypeptide targets, and thus becomes locally enriched at, an intracellular structural element within or directly adjacent to the cytoplasm; and
      (a2) a polypeptide segment derived from a phase separation polypeptide (PSP segment), wherein said phase separation polypeptide has the ability to undergo self-association in the cytoplasm of a cell so as to create sites of high local concentration in the cytoplasm, and
   (b) at least one second polypeptide segment acting as an effector (EP) that is selected from:
      b1) an RNA-targeting polypeptide (RNA-TP) segment, and
      b2) an orthogonal aminoacyl tRNA synthetase (O-RS) segment;
   wherein said polypeptide segments are functionally linked in said AFP.
E2: An assembler fusion protein (AFP) combination comprising at least two AFPs of embodiment 1.
E3: A fusion protein (RNA-TP/O-RS fusion protein) comprising:
   (i) at least one RNA-targeting polypeptide (RNA-TP) segment; and
   (ii) at least one orthogonal aminoacyl tRNA synthetase (O-RS) segment,
   wherein said polypeptide segments are functionally linked in said RNA-TP/O-RS fusion protein.
E4: A nucleic acid molecule, or a combination of two or more nucleic acid molecules, comprising:
   (i) a nucleotide sequence that encodes at least one AFP of embodiment 1, or at least one AFP combination of embodiment 2, or
   (ii) a nucleic acid sequence complementary to the nucleotide sequence of (i).
   (iii) both of (i) and (ii).
E5: A nucleic acid molecule, or a combination of two or more nucleic acid molecules, comprising:
   (i) a nucleotide sequence that encodes at least one RNA-TP/O-RS fusion protein of embodiment 3, or
   (ii) a nucleic acid sequence complementary to (i), or
   (iii) both of (i) and (ii).
E6: An expression cassette comprising the nucleotide sequence of the nucleic acid molecule, or the combination of nucleic acid molecules, of embodiment 4 or embodiment 5.
E7: An expression vector comprising at least one expression cassette of embodiment 6.
E8: A cell comprising at least one nucleic acid molecule, or combination of nucleic acid molecules, of embodiment 4 or embodiment 5, at least one expression cassette of embodiment 6, or at least one expression vector of embodiment 7.
E9: The cell of embodiment 8 comprising a nucleotide sequence that encodes, or is complementary to a nucleotide sequence encoding, at least one AFP of embodiment 1 comprising (i) at least one EP selected from RNA-TP segments and (ii) at least one EP selected from O-RS segments.
E10: The cell of embodiment 8 comprising a nucleotide sequence that encodes, or is complementary to a nucleotide sequence encoding, a combination of at least two AFPs of embodiment 1, wherein one of the at least two AFPs comprises at least one RNA-TP segment and another one of the at least two AFPs comprises at least one O-RS segment.
E11: The cell of embodiment 8 comprising a nucleotide sequence that encodes, or is complementary to a nucleotide sequence encoding at least one RNA-TP/O-RS fusion protein of embodiment 3.
E12: A method for preparing a polypeptide of interest (POI) comprising in its amino acid sequence one or more non-canonical amino acid (ncAA) residues, wherein the method comprises expressing the POI in a cell of embodiment 9 or embodiment 10 in the presence of said one or more ncAAs, wherein the cell comprises:
   (i) a POI-encoding nucleotide sequence (CS^{POI}) wherein said one or more ncAA residues of the POI are encoded by selector codon(s),
   (ii) a targeting nucleotide sequence (TN) that is functionally linked to the CS^{POI} and is able to interact with an RNA-TP segment of at least one of the AFPs in the cell;
   (iii) one or more orthogonal tRNA^{ncAA} (O-tRNA^{ncAA}) molecules which carry the anticodon(s) complementary to the selector codon(s) of the CS^{POI}, and wherein said O-tRNA^{ncAA} molecules together with one or more O-RS segments of at least one of the AFPs in the cell form one or more orthogonal O-RS/O-tRNA^{ncAA} pairs which allow for the introduction of said one or more ncAA residues into the amino acid sequence of the POI;
   and wherein the method optionally further comprises recovering the expressed POI.
E13: A method for preparing a polypeptide of interest (POI) comprising in its amino acid sequence one or more non-canonical amino acid (ncAA) residues, wherein the method comprises expressing the POI in a cell of embodiment 11 in the presence of said one or more ncAAs, wherein the cell comprises:
   (iv) a POI-encoding nucleotide sequence (CS^{POI}) wherein said one or more ncAA residues of the POI are encoded by selector codon(s),
   (v) a targeting nucleotide sequence (TN) that is functionally linked to the CS^{POI} and is able to interact with an RNA-TP segment of at least one of the RNA-TP/O-RS fusion proteins in the cell;
   (vi) one or more orthogonal tRNA^{ncAA} (O-tRNA^{ncAA}) molecules which carry the anticodon(s) complementary to the selector codon(s) of the CS^{POI}, and wherein said O-tRNA^{ncAA} molecules together with one or more O-RS segments of the RNA-TP/O-RS fusion proteins in the cell form one or more orthogonal O-RS/O-tRNA^{ncAA} pairs which allow for the introduction of said one or more ncAA residues into the amino acid sequence of the POI;
   and wherein the method optionally further comprises recovering the expressed POI.
E14: A nucleic acid molecule comprising:
   (i) a nucleotide sequence (CS^{POI}) that encodes a polypeptide of interest (POI), said POI comprising one or more non-canonical amino acid (ncAA) residues which are encoded in the CS^{POI} by selector codons, and
   (ii) a targeting nucleotide sequence (TN), wherein an RNA molecule comprising said TN is able to interact via said TN with an RNA-targeting polypeptide (RNA-TP).
E15: A kit for preparing a polypeptide of interest (POI) having at least one non-canonical amino acid (ncAA) residue, the kit comprising:
   - at least one ncAA, or salt thereof, corresponding to the at least one ncAA residue of the POI, and
   - at least one expression vector of embodiment 7.

## Claims

1. An assembler fusion protein (AFP) comprising:
(a) at least one first polypeptide segment acting as assembler (AP) that is selected from:
(a1) a polypeptide segment of an intracellular targeting polypeptide (IC-TP segment), wherein said segment of said intracellular targeting polypeptide targets, and thus becomes locally enriched at, an intracellular structural element within or directly adjacent to the cytoplasm; and
(a2) a polypeptide segment of a phase separation polypeptide (PSP segment), wherein said segment of said phase separation polypeptide has the ability to undergo self-association in the cytoplasm of a cell so as to create sites of high local concentration in the cytoplasm, and
(b) at least one second polypeptide segment acting as an effector (EP) that is selected from:
b1) an RNA-targeting polypeptide (RNA-TP) segment, and
b2) an orthogonal aminoacyl tRNA synthetase (O-RS) segment;
wherein said polypeptide segments are functionally linked in said AFP;
and
wherein said (IC-TP) is selected from
- KIF16B₁₋₄₀₀ comprising the amino acid sequence of SEQ ID NO:20,
- KIF13A_{1-411,Δ390} comprising the amino acid sequence of SEQ ID NO:22,
- TOMM20₁₋₇₀ comprising the amino acid sequence of SEQ ID NO:24,
- LcK comprising the amino acid sequence of SEQ ID NO:26,
- FRB-CD28 comprising the amino acid sequence of SEQ ID NO:28,
- FUS-CD28 comprising the amino acid sequence of SEQ ID NO:30,
- EB1 comprising the amino acid sequence of SEQ ID NO:302,
- CG1 comprising the amino acid sequence of SEQ ID NO:304,
- EBAG9 comprising the amino acid sequence of SEQ ID NO:292,
- EBAG9₁₋₂₉ comprising amino acid sequence of SEQ ID NO:294,
- CMP Sia Tr comprising the amino acid sequence of SEQ ID NO:296,
- P450 2C1₁₋₂₇ comprising the amino acid sequence of SEQ ID NO:298,
- P450 2C1₁₋₂₉ comprising the amino acid sequence of SEQ ID NO:300, and
- SLP-3₁₋₅₉ comprising the amino acid sequence of SEQ ID NO:310;
- or a sequence having a degree of sequence identity of at least 60% to anyone of the above sequences;
and/or
wherein the PSP is selected from
- SPD5 comprising the amino acid sequence of SEQ ID NO:32,
- FUS comprising the amino acid sequence of SEQ ID NO:34,
- EWSR1 comprising the amino acid sequence of SEQ ID NO:36, and
- ATP-dependent RNA helicase LAF1 (RGG domain) comprising the amino acid sequence of SEQ ID NO:308,
- or a sequence having a degree of sequence identity of at least 60% to anyone of the above sequences;
and/or
wherein said effector (EP) is selected from:
an RNA-targeting polypeptide (RNA-TP) selected from
- MCP comprising the amino acid sequence of SEQ ID NO:14,
- λ_{N22} comprising the amino acid sequence of SEQ ID NO:16, and
- PCP comprising the amino acid sequence of SEQ ID NO:306,
- or a sequence having a degree of sequence identity of at least 60% to anyone of the above sequences;
or
from an O-RS polypeptide (O-RS) is selected from:
- PyIRS^{AF} comprising the amino acid sequence of SEQ ID NO:8;
- PylRS^{AA} comprising the amino acid sequence of SEQ ID NO:10;
- PylRS^{AAAF} comprising the amino acid sequence of SEQ ID NO:12;
- IFRS1 comprising the amino acid sequence of SEQ ID NO:224;
- CbzRS comprising the amino acid sequence of SEQ ID NO:226;
- CpkRS comprising the amino acid sequence of SEQ ID NO:228; and
- OMeRS comprising the amino acid sequence of SEQ ID NO:236,
- or a sequence having a degree of sequence identity of at least 60% to anyone of the above sequences.

2. The AFP of claim 1,
wherein said AFP is selected from at least one of the following groups (A) to (D) of fusion proteins
**Group (A):**
Fusion proteins having one of the following structures:
(1) [IC-TP]ₘ - [O-RS]_{y};
(2) [IC-TP]ₘ - [RNA-TP]ₓ;
(3) [IC-TP]ₘ - [PSP]ₙ - [O-RS]_{y};
(4) [IC-TP]ₘ - [PSP]ₙ - [RNA-TP]ₓ;
(5) [IC-TP]ₘ - [O-RS₁]_{y} - [PSP]ₙ - [O-RS₂]_{y};
(6) [IC-TP]ₘ - [PSP₁]ₙ - [O-RS]_{y} - [PSP₂]ₙ;
(7) [IC-TP]ₘ - [RNA-TP₁]ₓ - [PSP]ₙ - [RNA-TP₂]ₓ;
(8) [IC-TP]ₘ - [PSP₁]ₙ - [O-RS₁]_{y} - [PSP₂]ₙ - [O-RS₂]_{y};
(9) [IC-TP]ₘ - [PSP₁]ₙ - [RNA-TP₁]ₓ - [PSP₂]ₙ - [RNA-TP₂]ₓ;
wherein
m = 1 or 2;
n independently of each other = 1, 2 or 3;
y independently of each other = 1 or 2;
x independently of each other = 1, 2, 3, 4, 5 or 6;
O-RS₁ and O-RS₂ are identical or different;
PSP₁ and PSP₂ are identical or different; and
RNA-TP₁ and RNA-TP₂ are identical or different;
**Group (B):**
Fusion proteins having one of the following structures:
(1) [PSP]ₙ - [O-RS]_{y};
(2) [PSP]ₙ - [RNA-TP]ₓ;
(3) [O-RS₁]_{y} - [PSP]ₙ - [O-RS₂]_{y};
(4) [PSP₁]ₙ - [O-RS]_{y} - [PSP₂]ₙ;
(5) [RNA-TP₁]ₓ - [PSP]ₙ - [RNA-TP₂]ₓ;
(6) [PSP₁]ₙ - [O-RS₁]_{y} - [PSP₂]ₙ - [O-RS₂]_{y};
(7) [PSP₁]ₙ - [RNA-TP₁]ₓ - [PSP₂]ₙ - [RNA-TP₂]ₓ;
wherein
n independently of each other = 1, 2 or 3;
y independently of each other = 1 or 2;
x independently of each other = 1, 2, 3, 4, 5 or 6;
O-RS₁ and O-RS₂ are identical or different;
PSP₁ and PSP₂ are identical or different; and
RNA-TP₁ and RNA-TP₂ are identical or different;
**Group (C):**
Fusion proteins having one of the following structures:
(1) [IC-TP]ₘ - [O-RS]_{y} - [RNA-TP]ₓ;
(2) [IC-TP]ₘ - [RNA-TP]ₓ- [O-RS]_{y};
(3) [IC-TP]ₘ - [PSP]ₙ - [O-RS]_{y} - [RNA-TP]ₓ;
(4) [IC-TP]ₘ - [PSP]ₙ - [RNA-TP]ₓ- [O-RS]_{y};
(5) [IC-TP]ₘ - [O-RS]_{y} - [PSP]ₙ - [RNA-TP]ₓ;
(6) [IC-TP]ₘ - [RNA-TP]ₓ - [PSP]ₙ - [O-RS]_{y};
(7) [IC-TP]ₘ - [PSP₁]ₙ - [O-RS]_{y} - [PSP₂]ₙ - [RNA-TP]ₓ;
(8) [IC-TP]ₘ - [PSP₁]ₙ - [RNA-TP]ₓ - [O-RS₁]_{y} - [PSP₂]ₙ - [O-RS₂]_{y};
(9) [IC-TP]ₘ - [PSP₁]ₙ - [O-RS₁]_{y} - [PSP₂]ₙ - [O-RS₂]_{y} - [RNA-TP]ₓ;
wherein
m = 1 or 2;
n independently of each other = 1, 2 or 3;
y independently of each other = 1 or 2;
x independently of each other = 1, 2, 3, 4, 5 or 6;
O-RS₁ and O-RS₂ are identical or different;
PSP₁ and PSP₂ are identical or different; and
RNA-TP₁ and RNA-TP₂ are identical or different;
**Group (D):**
Fusion proteins having one of the following structures:
(1) [PSP]ₙ - [O-RS]_{y} - [RNA-TP]ₓ;
(2) [PSP]ₙ - [RNA-TP]ₓ- [O-RS]_{y};
(3) [O-RS]_{y} - [PSP]ₙ - [RNA-TP]ₓ;
(4) [RNA-TP]ₓ - [PSP]ₙ - [O-RS]_{y};
(5) [PSP₁]ₙ - [O-RS]_{y} - [PSP₂]ₙ - [RNA-TP]ₓ;
(6) [PSP₁]ₙ - [RNA-TP]ₓ - [O-RS₁]_{y} - [PSP₂]ₙ - [O-RS₂]_{y};
(7) [PSP₁]ₙ - [O-RS₁]_{y} - [PSP₂]ₙ - [O-RS₂]_{y} - [RNA-TP]ₓ;
wherein
n independently of each other = 1, 2 or 3;
y independently of each other = 1 or 2;
x independently of each other = 1, 2, 3, 4, 5 or 6;
O-RS₁ and O-RS₂ are identical or different;
PSP₁ and PSP₂ are identical or different; and
RNA-TP₁ and RNA-TP₂ are identical or different;

3. An assembler fusion protein (AFP) combination comprising at least two AFPs, wherein each AFP of the combination comprises:
(a) at least one first polypeptide segment acting as assembler (AP) that is selected from:
(a1) a polypeptide segment of an intracellular targeting polypeptide (IC-TP segment), wherein said segment of said intracellular targeting polypeptide targets, and thus becomes locally enriched at, an intracellular structural element within or directly adjacent to the cytoplasm; and
(a2) a polypeptide segment of a phase separation polypeptide (PSP segment), wherein said segment of said phase separation polypeptide has the ability to undergo self-association in the cytoplasm of a cell so as to create sites of high local concentration in the cytoplasm, and
(b) at least one second polypeptide segment acting as an effector (EP) that is selected from:
b1) an RNA-targeting polypeptide (RNA-TP) segment, and
b2) an orthogonal aminoacyl tRNA synthetase (O-RS) segment;
wherein at least a first AFP comprises an EP selected from an RNA-TP segment, and
wherein at least a second AFP comprises an EP selected from an O-RS segment; and
wherein said polypeptide segments are functionally linked in each of said AFP; and
wherein said IC-TP, PSP, RNA-TP and O-RS are as defined in claim 1;
and/or
wherein said at least first AFP and said at least second AFP are selected from at least one of the groups (A) to (D) of fusion proteins as defined in claim 2.

4. The AFP combination of claim 3, wherein at least one AFP of said combination includes a first SYNZIP element and at least another AFP of said combination includes a second SYNZIP element, wherein said first and said second SYNZIP element act together by forming a heterodimer structure.

5. The AFP combination of claim 4, wherein said first and said second SYNZIP elements act together as heterospecific coiled-coil SYNZIP pairs, wherein said SYNZIP pairs are selected from SYNZIP 1:2; SYNZIP 3:4 and SYNZIP 5:6.

6. The AFP combination of claim 4 or 5, wherein said least a first AFP and said at least a second AFP are selected from at least one of the groups (E) and (F) of fusion proteins
**Group (E):**
Fusion proteins having one of the following structures:
(1) [PSP]ₙ- [SYNZIP] - [O-RS]_{y};
(2) [PSP]ₙ- [SYNZIP]- [RNA-TP]ₓ;
(3) [IC-TP]ₘ- [SYNZIP] - [O-RS]_{y};
(4) [IC-TP]ₘ - [SYNZIP]- [RNA-TP]ₓ;
(5) [IC-TP]ₘ - [PSP]ₙ- [SYNZIP] - [O-RS]_{y};
(6) [IC-TP]ₘ - [PSP]ₙ - [SYNZIP] - [RNA-TP]ₓ;
wherein
m =1 or 2;
n =1, 2 or 3;
y = 1 or 2; and
x = 1, 2, 3, 4, 5 or 6;
or
**Group (F):**
Fusion proteins having one of the following structures:
(1) [IC-TP]ₘ - [O-RS]_{y}- [SYNZIP] - [RNA-TP]ₓ;
(2) [IC-TP]ₘ - [RNA-TP]ₓ- [SYNZIP]- [O-RS]_{y};
(3) [IC-TP]ₘ - [PSP]ₙ- [SYNZIP] - [O-RS]_{y} - [RNA-TP]ₓ;
(4) [IC-TP]ₘ - [PSP]ₙ - [SYNZIP]- [RNA-TP]ₓ- [O-RS]_{y};
(5) [IC-TP]ₘ - [PSP]ₙ- [SYNZIPₐ] - [O-RS]_{y} - [SYNZIP_{b}] - [RNA-TP]ₓ;
(6) [IC-TP]ₘ - [PSP]ₙ - [SYNZIPₐ]- [RNA-TP]ₓ- [SYNZIP_{b}] - [O-RS]_{y};
(7) [IC-TP]ₘ - [PSP₁]ₙ - [SYNZIP]- [RNA-TP]ₓ- [O-RS_{1]y} - [PSP₂]ₙ - [O-RS₂]_{y};
wherein
m =1 or 2;
n =1, 2 or 3;
y = 1 or 2;
x = 1, 2, 3, 4, 5 or 6;
SYNZIPₐ and SYNZIP_{b} are identical or different;
PSP₁ and PSP₂ are identical or different; and
O-RS₁ and O-RS₂ are identical or different.

7. A fusion protein (RNA-TP/O-RS fusion protein) comprising:
(i) at least one RNA-targeting polypeptide (RNA-TP) segment; and
(ii) at least one orthogonal aminoacyl tRNA synthetase (O-RS) segment,
wherein said polypeptide segments are functionally linked in said RNA-TP/O-RS fusion protein; wherein
wherein said RNA-TP and O-RS are as defined in claim 1;
and/or
wherein said AFP ia selected from one fusion protein of the groups (C) and (D) of fusion proteins as defined in claim 2.

8. A nucleic acid molecule, or a combination of two or more nucleic acid molecules, comprising:
(i) a nucleotide sequence that encodes at least one AFP of claim 1 or 2, or at least one AFP combination of anyone of claims 3 to 6, or
(ii) a nucleic acid sequence complementary to the nucleotide sequence of (i).
(iii) both of (i) and (ii).

9. A nucleic acid molecule, or a combination of two or more nucleic acid molecules, comprising:
(i) a nucleotide sequence that encodes at least one RNA-TP/O-RS fusion protein of claim 7, or
(ii) a nucleic acid sequence complementary to (i), or
(iii) both of (i) and (ii).

10. An expression cassette comprising the nucleotide sequence of the nucleic acid molecule, or the combination of nucleic acid molecules, of claim 8 or claim 9.

11. An expression vector comprising at least one expression cassette of claim 10.

12. A cell comprising at least one nucleic acid molecule, or combination of nucleic acid molecules, of claim 8 or claim 9, at least one expression cassette of claim 10, or at least one expression vector of claim 11.

13. The cell of claim 12 comprising a nucleotide sequence that encodes, or is complementary to a nucleotide sequence encoding, at least one AFP of claim 1 or 2 comprising (i) at least one EP selected from RNA-TP segments and (ii) at least one EP selected from O-RS segments; or
comprising a nucleotide sequence that encodes, or is complementary to a nucleotide sequence encoding, a combination of at least two AFPs of claim 1 or 2, wherein one of the at least two AFPs comprises at least one RNA-TP segment and another one of the at least two AFPs comprises at least one O-RS segment; or
comprising a nucleotide sequence that encodes, or is complementary to a nucleotide sequence encoding at least one RNA-TP/O-RS fusion protein of claim 7.

14. A kit for preparing a polypeptide of interest (POI) having at least one non-canonical amino acid (ncAA) residue, the kit comprising:
- at least one ncAA, or salt thereof, corresponding to the at least one ncAA residue of the POI, and
- at least one expression vector of claim 11.
